(19) Europäisches Patentamt
European Patent Office
Office européen des brevets

(11) **EP 2 447 719 B1**

(12) **EUROPEAN PATENT SPECIFICATION**

(45) Date of publication and mention
of the grant of the patent:
**24.08.2016 Bulletin 2016/34**

(51) Int Cl.:
***C07K 16/30*** (2006.01)   ***G01N 33/574*** (2006.01)

(21) Application number: **12152158.7**

(22) Date of filing: **26.02.2008**

(54) **Proteins**

Proteine

Protéines

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.02.2007   US 903510 P**
**26.02.2007   US 903509 P**
**05.06.2007   PCT/EP2007/055537**
**25.02.2008   PCT/GB2008/050124**

(43) Date of publication of application:
**02.05.2012   Bulletin 2012/18**

(62) Document number(s) of the earlier application(s) in accordance with Art. 76 EPC:
**08709645.9 / 2 121 745**

(73) Proprietor: **Oxford BioTherapeutics Ltd**
**Milton Park**
**Abingdon**
**Oxfordshire OX14 4RZ (GB)**

(72) Inventors:
• **Rohlff, Christian**
**Abingdon, Oxfordshire OX14 4RY (GB)**
• **Stamps, Alasdair**
**Abingdon, Oxfordshire OX14 4RY (GB)**

(74) Representative: **Blance, Stephen John**
**Oxford BioTherapeutics Ltd**
**94A Innovation Drive**
**Milton Park**
**Abingdon OX14 4RZ (GB)**

(56) References cited:
**WO-A1-2004/074481     WO-A2-2007/005502**

• **BÜHRING HANS-JÖRG ET AL: "CDCP1 identifies a broad spectrum of normal and malignant stem/progenitor cell subsets of hematopoietic and nonhematopoietic origin", STEM CELLS, ALPHAMED PRESS, DAYTON, OH, US, vol. 22, no. 3, 1 January 2004 (2004-01-01), pages 334-343, XP002495598, ISSN: 1066-5099, DOI: 10.1634/STEMCELLS.22-3-334**
• **PERRY ET AL: "Expression of the CUB domain containing protein 1 (CDCP1) gene in colorectal tumour cells", FEBS LETTERS, ELSEVIER, AMSTERDAM, NL, vol. 581, no. 6, 20 February 2007 (2007-02-20), pages 1137-1142, XP005932065, ISSN: 0014-5793, DOI: 10.1016/J.FEBSLET.2007.02.025**

Remarks:
The file contains technical information submitted after the application was filed and not included in this specification

EP 2 447 719 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Oxford Genome Sciences (UK) Ltd
Rohlff, Christian
Stamps, Alasdair

<120> PROTEINS

<130> OGL-P148PC

<140> PCT/GB 08/050125
<141> 2008-02-25

<150> US 60/903,510
<151> 2007-02-26

<150> US 60/903,509
<151> 2007-02-26

<150> PCT/EP07/055537
<151> 2007-06-05

<160> 1008

<170> PatentIn version 3.3

<210> 1
<211> 987
<212> PRT
<213> Homo Sapiens

<220>
<221> MISC_FEATURE
<222> (1)..(987)
<223> Designated OGTA002 = Swissprot Accession No. P54760, Ephrin type-B receptor 4

<400> 1

```
Met Glu Leu Arg Val Leu Leu Cys Trp Ala Ser Leu Ala Ala Ala Leu
1               5                   10                  15

Glu Glu Thr Leu Leu Asn Thr Lys Leu Glu Thr Ala Asp Leu Lys Trp
            20                  25                  30

Val Thr Phe Pro Gln Val Asp Gly Gln Trp Glu Glu Leu Ser Gly Leu
            35                  40                  45

Asp Glu Glu Gln His Ser Val Arg Thr Tyr Glu Val Cys Asp Val Gln
    50                  55                  60

Arg Ala Pro Gly Gln Ala His Trp Leu Arg Thr Gly Trp Val Pro Arg
65                  70                  75                  80

Arg Gly Ala Val His Val Tyr Ala Thr Leu Arg Phe Thr Met Leu Glu
                85                  90                  95

Cys Leu Ser Leu Pro Arg Ala Gly Arg Ser Cys Lys Glu Thr Phe Thr
```

|     |     |     | 100 |     |     |     | 105 |     |     |     | 110 |     |     |     |
| --- | --- | --- | --- | --- | --- | --- | --- | --- | --- | --- | --- | --- | --- | --- |

Val Phe Tyr Tyr Glu Ser Asp Ala Asp Thr Ala Thr Ala Leu Thr Pro
115         120             125

Ala Trp Met Glu Asn Pro Tyr Ile Lys Val Asp Thr Val Ala Ala Glu
130         135             140

His Leu Thr Arg Lys Arg Pro Gly Ala Glu Ala Thr Gly Lys Val Asn
145         150             155             160

Val Lys Thr Leu Arg Leu Gly Pro Leu Ser Lys Ala Gly Phe Tyr Leu
165             170             175

Ala Phe Gln Asp Gln Gly Ala Cys Met Ala Leu Leu Ser Leu His Leu
180             185             190

Phe Tyr Lys Lys Cys Ala Gln Leu Thr Val Asn Leu Thr Arg Phe Pro
195             200             205

Glu Thr Val Pro Arg Glu Leu Val Val Pro Val Ala Gly Ser Cys Val
210             215             220

Val Asp Ala Val Pro Ala Pro Gly Pro Ser Pro Ser Leu Tyr Cys Arg
225             230             235             240

Glu Asp Gly Gln Trp Ala Glu Gln Pro Val Thr Gly Cys Ser Cys Ala
245             250             255

Pro Gly Phe Glu Ala Ala Glu Gly Asn Thr Lys Cys Arg Ala Cys Ala
260             265             270

Gln Gly Thr Phe Lys Pro Leu Ser Gly Glu Gly Ser Cys Gln Pro Cys
275             280             285

Pro Ala Asn Ser His Ser Asn Thr Ile Gly Ser Ala Val Cys Gln Cys
290             295             300

Arg Val Gly Tyr Phe Arg Ala Arg Thr Asp Pro Arg Gly Ala Pro Cys
305             310             315             320

Thr Thr Pro Pro Ser Ala Pro Arg Ser Val Val Ser Arg Leu Asn Gly
325             330             335

Ser Ser Leu His Leu Glu Trp Ser Ala Pro Leu Glu Ser Gly Gly Arg
340             345             350

Glu Asp Leu Thr Tyr Ala Leu Arg Cys Arg Glu Cys Arg Pro Gly Gly
355             360             365

```
Ser Cys Ala Pro Cys Gly Gly Asp Leu Thr Phe Asp Pro Gly Pro Arg
    370             375             380

Asp Leu Val Glu Pro Trp Val Val Val Arg Gly Leu Arg Pro Asp Phe
385             390             395             400

Thr Tyr Thr Phe Glu Val Thr Ala Leu Asn Gly Val Ser Ser Leu Ala
            405             410             415

Thr Gly Pro Val Pro Phe Glu Pro Val Asn Val Thr Thr Asp Arg Glu
            420             425             430

Val Pro Pro Ala Val Ser Asp Ile Arg Val Thr Arg Ser Ser Pro Ser
        435             440             445

Ser Leu Ser Leu Ala Trp Ala Val Pro Arg Ala Pro Ser Gly Ala Val
    450             455             460

Leu Asp Tyr Glu Val Lys Tyr His Glu Lys Gly Ala Glu Gly Pro Ser
465             470             475             480

Ser Val Arg Phe Leu Lys Thr Ser Glu Asn Arg Ala Glu Leu Arg Gly
            485             490             495

Leu Lys Arg Gly Ala Ser Tyr Leu Val Gln Val Arg Ala Arg Ser Glu
            500             505             510

Ala Gly Tyr Gly Pro Phe Gly Gln Glu His His Ser Gln Thr Gln Leu
            515             520             525

Asp Glu Ser Glu Gly Trp Arg Glu Gln Leu Ala Leu Ile Ala Gly Thr
    530             535             540

Ala Val Val Gly Val Val Leu Val Leu Val Val Ile Val Val Ala Val
545             550             555             560

Leu Cys Leu Arg Lys Gln Ser Asn Gly Arg Glu Ala Glu Tyr Ser Asp
            565             570             575

Lys His Gly Gln Tyr Leu Ile Gly His Gly Thr Lys Val Tyr Ile Asp
            580             585             590

Pro Phe Thr Tyr Glu Asp Pro Asn Glu Ala Val Arg Glu Phe Ala Lys
    595             600             605

Glu Ile Asp Val Ser Tyr Val Lys Ile Glu Glu Val Ile Gly Ala Gly
    610             615             620
```

```
Glu Phe Gly Glu Val Cys Arg Gly Arg Leu Lys Ala Pro Gly Lys Lys
625             630         635             640

Glu Ser Cys Val Ala Ile Lys Thr Leu Lys Gly Gly Tyr Thr Glu Arg
            645             650             655

Gln Arg Arg Glu Phe Leu Ser Glu Ala Ser Ile Met Gly Gln Phe Glu
        660             665             670

His Pro Asn Ile Ile Arg Leu Glu Gly Val Val Thr Asn Ser Met Pro
        675             680             685

Val Met Ile Leu Thr Glu Phe Met Glu Asn Gly Ala Leu Asp Ser Phe
    690             695             700

Leu Arg Leu Asn Asp Gly Gln Phe Thr Val Ile Gln Leu Val Gly Met
705             710             715             720

Leu Arg Gly Ile Ala Ser Gly Met Arg Tyr Leu Ala Glu Met Ser Tyr
            725             730             735

Val His Arg Asp Leu Ala Ala Arg Asn Ile Leu Val Asn Ser Asn Leu
        740             745             750

Val Cys Lys Val Ser Asp Phe Gly Leu Ser Arg Phe Leu Glu Glu Asn
    755             760             765

Ser Ser Asp Pro Thr Tyr Thr Ser Ser Leu Gly Gly Lys Ile Pro Ile
    770             775             780

Arg Trp Thr Ala Pro Glu Ala Ile Ala Phe Arg Lys Phe Thr Ser Ala
785             790             795             800

Ser Asp Ala Trp Ser Tyr Gly Ile Val Met Trp Glu Val Met Ser Phe
            805             810             815

Gly Glu Arg Pro Tyr Trp Asp Met Ser Asn Gln Asp Val Ile Asn Ala
            820             825             830

Ile Glu Gln Asp Tyr Arg Leu Pro Pro Pro Asp Cys Pro Thr Ser
        835             840             845

Leu His Gln Leu Met Leu Asp Cys Trp Gln Lys Asp Arg Asn Ala Arg
        850             855             860

Pro Arg Phe Pro Gln Val Val Ser Ala Leu Asp Lys Met Ile Arg Asn
865             870             875             880
```

141

```
Pro Ala Ser Leu Lys Ile Val Ala Arg Glu Asn Gly Gly Ala Ser His
                885             890             895

Pro Leu Leu Asp Gln Arg Gln Pro His Tyr Ser Ala Phe Gly Ser Val
            900             905             910

Gly Glu Trp Leu Arg Ala Ile Lys Met Gly Arg Tyr Glu Glu Ser Phe
            915             920             925

Ala Ala Ala Gly Phe Gly Ser Phe Glu Leu Val Ser Gln Ile Ser Ala
    930             935             940

Glu Asp Leu Leu Arg Ile Gly Val Thr Leu Ala Gly His Gln Lys Lys
945             950             955             960

Ile Leu Ala Ser Val Gln His Met Lys Ser Gln Ala Lys Pro Gly Thr
            965             970             975

Pro Gly Gly Thr Gly Gly Pro Ala Pro Gln Tyr
            980             985
```

<210> 2
<211> 220
<212> PRT
<213> Homo Sapiens

<220>
<221> MISC_FEATURE
<222> (1)..(220)
<223> OGTA009 = Swiss Prot Accession No. 015551, Claudin-3

<400> 2

```
Met Ser Met Gly Leu Glu Ile Thr Gly Thr Ala Leu Ala Val Leu Gly
1               5               10                  15

Trp Leu Gly Thr Ile Val Cys Cys Ala Leu Pro Met Trp Arg Val Ser
            20              25                  30

Ala Phe Ile Gly Ser Asn Ile Ile Thr Ser Gln Asn Ile Trp Glu Gly
            35              40                  45

Leu Trp Met Asn Cys Val Val Gln Ser Thr Gly Gln Met Gln Cys Lys
        50              55              60

Val Tyr Asp Ser Leu Leu Ala Leu Pro Gln Asp Leu Gln Ala Ala Arg
65                  70                  75                  80

Ala Leu Ile Val Val Ala Ile Leu Leu Ala Ala Phe Gly Leu Leu Val
                85                  90                  95

Ala Leu Val Gly Ala Gln Cys Thr Asn Cys Val Gln Asp Asp Thr Ala
            100             105             110

Lys Ala Lys Ile Thr Ile Val Ala Gly Val Leu Phe Leu Leu Ala Ala
        115             120             125

Leu Leu Thr Leu Val Pro Val Ser Trp Ser Ala Asn Thr Ile Ile Arg
    130             135             140

Asp Phe Tyr Asn Pro Val Val Pro Glu Ala Gln Lys Arg Glu Met Gly
145             150             155             160

Ala Gly Leu Tyr Val Gly Trp Ala Ala Ala Ala Leu Gln Leu Leu Gly
            165             170             175

Gly Ala Leu Leu Cys Cys Ser Cys Pro Pro Arg Glu Lys Lys Tyr Thr
        180             185             190

Ala Thr Lys Val Val Tyr Ser Ala Pro Arg Ser Thr Gly Pro Gly Ala
        195             200             205

Ser Leu Gly Thr Gly Tyr Asp Arg Lys Asp Tyr Val
    210             215             220
```

<210> 3
<211> 764
<212> PRT
<213> Homo Sapiens

<220>

<221> MISC_FEATURE
<222> (1)..(764)
<223> OGTA016 = Swiss Prot Accession No. P01833, Polymeric-immunoglobulin receptor

<400> 3

```
Met Leu Leu Phe Val Leu Thr Cys Leu Leu Ala Val Phe Pro Ala Ile
1               5                   10                  15


Ser Thr Lys Ser Pro Ile Phe Gly Pro Glu Glu Val Asn Ser Val Glu
            20                  25                  30


Gly Asn Ser Val Ser Ile Thr Cys Tyr Tyr Pro Pro Thr Ser Val Asn
            35                  40                  45


Arg His Thr Arg Lys Tyr Trp Cys Arg Gln Gly Ala Arg Gly Gly Cys
        50                  55                  60


Ile Thr Leu Ile Ser Ser Glu Gly Tyr Val Ser Ser Lys Tyr Ala Gly
65                  70                  75                  80
```

Arg Ala Asn Leu Thr Asn Phe Pro Glu Asn Gly Thr Phe Val Val Asn
              85              90                    95

Ile Ala Gln Leu Ser Gln Asp Asp Ser Gly Arg Tyr Lys Cys Gly Leu
             100             105             110

Gly Ile Asn Ser Arg Gly Leu Ser Phe Asp Val Ser Leu Glu Val Ser
             115             120             125

Gln Gly Pro Gly Leu Leu Asn Asp Thr Lys Val Tyr Thr Val Asp Leu
        130             135             140

Gly Arg Thr Val Thr Ile Asn Cys Pro Phe Lys Thr Glu Asn Ala Gln
145             150             155             160

Lys Arg Lys Ser Leu Tyr Lys Gln Ile Gly Leu Tyr Pro Val Leu Val
             165             170             175

Ile Asp Ser Ser Gly Tyr Val Asn Pro Asn Tyr Thr Gly Arg Ile Arg
             180             185             190

Leu Asp Ile Gln Gly Thr Gly Gln Leu Leu Phe Ser Val Val Ile Asn
             195             200             205

Gln Leu Arg Leu Ser Asp Ala Gly Gln Tyr Leu Cys Gln Ala Gly Asp
        210             215             220

Asp Ser Asn Ser Asn Lys Lys Asn Ala Asp Leu Gln Val Leu Lys Pro
225             230             235             240

Glu Pro Glu Leu Val Tyr Glu Asp Leu Arg Gly Ser Val Thr Phe His
             245             250             255

Cys Ala Leu Gly Pro Glu Val Ala Asn Val Ala Lys Phe Leu Cys Arg
             260             265             270

Gln Ser Ser Gly Glu Asn Cys Asp Val Val Val Asn Thr Leu Gly Lys
        275             280             285

Arg Ala Pro Ala Phe Glu Gly Arg Ile Leu Leu Asn Pro Gln Asp Lys
        290             295             300

Asp Gly Ser Phe Ser Val Val Ile Thr Gly Leu Arg Lys Glu Asp Ala
305             310             315             320

Gly Arg Tyr Leu Cys Gly Ala His Ser Asp Gly Gln Leu Gln Glu Gly
             325             330             335

Ser Pro Ile Gln Ala Trp Gln Leu Phe Val Asn Glu Glu Ser Thr Ile
340             345                 350

Pro Arg Ser Pro Thr Val Val Lys Gly Val Ala Gly Ser Ser Val Ala
355             360                 365

Val Leu Cys Pro Tyr Asn Arg Lys Glu Ser Lys Ser Ile Lys Tyr Trp
370             375                 380

Cys Leu Trp Glu Gly Ala Gln Asn Gly Arg Cys Pro Leu Leu Val Asp
385             390                 395                 400

Ser Glu Gly Trp Val Lys Ala Gln Tyr Glu Gly Arg Leu Ser Leu Leu
405                 410                 415

Glu Glu Pro Gly Asn Gly Thr Phe Thr Val Ile Leu Asn Gln Leu Thr
420             425                 430

Ser Arg Asp Ala Gly Phe Tyr Trp Cys Leu Thr Asn Gly Asp Thr Leu
435             440                 445

Trp Arg Thr Thr Val Glu Ile Lys Ile Ile Glu Gly Glu Pro Asn Leu
450             455                 460

Lys Val Pro Gly Asn Val Thr Ala Val Leu Gly Glu Thr Leu Lys Val
465             470                 475                 480

Pro Cys His Phe Pro Cys Lys Phe Ser Ser Tyr Glu Lys Tyr Trp Cys
485             490                 495

Lys Trp Asn Asn Thr Gly Cys Gln Ala Leu Pro Ser Gln Asp Glu Gly
500             505                 510

Pro Ser Lys Ala Phe Val Asn Cys Asp Glu Asn Ser Arg Leu Val Ser
515             520                 525

Leu Thr Leu Asn Leu Val Thr Arg Ala Asp Glu Gly Trp Tyr Trp Cys
530             535                 540

Gly Val Lys Gln Gly His Phe Tyr Gly Glu Thr Ala Ala Val Tyr Val
545             550                 555                 560

Ala Val Glu Glu Arg Lys Ala Ala Gly Ser Arg Asp Val Ser Leu Ala
565             570                 575

Lys Ala Asp Ala Ala Pro Asp Glu Lys Val Leu Asp Ser Gly Phe Arg
580             585                 590

Glu Ile Glu Asn Lys Ala Ile Gln Asp Pro Arg Leu Phe Ala Glu Glu

**146**

```
                595                     600                      605


        Lys Ala Val Ala Asp Thr Arg Asp Gln Ala Asp Gly Ser Arg Ala Ser
            610                 615                 620

        Val Asp Ser Gly Ser Ser Glu Glu Gln Gly Gly Ser Ser Arg Ala Leu
        625                 630                 635                 640

        Val Ser Thr Leu Val Pro Leu Gly Leu Val Leu Ala Val Gly Ala Val
                        645                 650                 655

        Ala Val Gly Val Ala Arg Ala Arg His Arg Lys Asn Val Asp Arg Val
                        660                 665                 670

        Ser Ile Arg Ser Tyr Arg Thr Asp Ile Ser Met Ser Asp Phe Glu Asn
                    675                 680                 685

        Ser Arg Glu Phe Gly Ala Asn Asp Asn Met Gly Ala Ser Ser Ile Thr
            690                 695                 700

        Gln Glu Thr Ser Leu Gly Gly Lys Glu Glu Phe Val Ala Thr Thr Glu
        705                 710                 715                 720

        Ser Thr Thr Glu Thr Lys Glu Pro Lys Lys Ala Lys Arg Ser Ser Lys
                        725                 730                 735

        Glu Glu Ala Glu Met Ala Tyr Lys Asp Phe Leu Leu Gln Ser Ser Thr
                    740                 745                 750

        Val Ala Ala Glu Ala Gln Asp Gly Pro Gln Glu Ala
                    755                 760
```

<210> 4
<211> 865
<212> PRT
<213> Homo Sapiens

<220>
<221> MISC_FEATURE
<222> (1)..(865)
<223> OGTA028 = Swiss Prot Accession No. Q5T7N2, FLJ10884

<400> 4

Met Ser Asp Val Ser Thr Ser Val Gln Ser Lys Phe Ala Arg Leu Ala
1                   5                   10                  15

Lys Lys Lys Glu Asn Ile Thr Tyr Met Lys Arg Glu Gln Leu Thr Glu
                20                  25                  30

Thr Asp Lys Asp Ile Ala Pro Val Leu Asp Leu Lys Cys Lys Asp Val

```
                35                    40                        45


        Ser Ala Ile Met Asn Lys Phe Lys Val Leu Met Glu Ile Gln Asp Leu
            50                  55                  60


        Met Phe Glu Glu Met Arg Glu Thr Leu Lys Asn Asp Leu Lys Ala Val
        65              70                  75                      80


        Leu Gly Gly Lys Ala Thr Ile Pro Glu Val Lys Asn Ser Glu Asn Ser
                        85                  90                  95


        Ser Ser Arg Thr Glu Phe Gln Gln Ile Ile Asn Leu Ala Leu Gln Lys
                    100             105             110


        Thr Gly Met Val Gly Lys Ile Glu Gly Glu Asn Ser Lys Ile Gly Asp
                115             120             125


        Asp Asn Glu Asn Leu Thr Phe Lys Leu Glu Val Asn Glu Leu Ser Gly
            130             135             140


        Lys Leu Asp Asn Thr Asn Glu Tyr Asn Ser Asn Asp Gly Lys Lys Leu
        145             150             155                 160


        Pro Gln Gly Glu Ser Arg Ser Tyr Glu Val Met Gly Ser Met Glu Glu
                    165             170             175


        Thr Leu Cys Asn Ile Asp Asp Arg Asp Gly Asn Arg Asn Val His Leu
                    180             185             190


        Glu Phe Thr Glu Arg Glu Ser Arg Lys Asp Gly Glu Asp Glu Phe Val
                195             200             205


        Lys Glu Met Arg Glu Glu Arg Lys Phe Gln Lys Leu Lys Asn Lys Glu
                210             215             220


        Glu Val Leu Lys Ala Ser Arg Glu Glu Lys Val Leu Met Asp Glu Gly
        225             230             235                 240


        Ala Val Leu Thr Leu Val Ala Asp Leu Ser Ser Ala Thr Leu Asp Ile
                        245             250             255


        Ser Lys Gln Trp Ser Asn Val Phe Asn Ile Leu Arg Glu Asn Asp Phe
                    260             265             270


        Glu Pro Lys Phe Leu Cys Glu Val Lys Leu Ala Phe Lys Cys Asp Gly
                    275             280             285


        Glu Ile Lys Thr Phe Ser Asp Leu Gln Ser Leu Arg Lys Phe Ala Ser
            290             295             300
```

149

Gln Lys Ser Ser Val Lys Glu Leu Leu Lys Asp Val Leu Pro Gln Lys
305                 310                 315                 320

Glu Glu Ile Asn Gln Gly Gly Arg Lys Tyr Gly Ile Gln Glu Lys Arg
                325                 330                 335

Asp Lys Thr Leu Ile Asp Ser Lys His Arg Ala Gly Glu Ile Thr Ser
            340                 345                 350

Asp Gly Leu Ser Phe Leu Phe Leu Lys Glu Val Lys Val Ala Lys Pro
            355                 360                 365

Glu Glu Met Lys Asn Leu Glu Thr Gln Glu Glu Glu Phe Ser Glu Leu
        370                 375                 380

Glu Glu Leu Asp Glu Glu Ala Ser Gly Met Glu Asp Asp Glu Asp Thr
385                 390                 395                 400

Ser Gly Leu Glu Glu Glu Glu Glu Glu Pro Ser Gly Leu Glu Glu Glu
            405                 410                 415

Glu Glu Glu Glu Ala Ser Gly Leu Glu Glu Asp Glu Ala Ser Gly Leu
        420                 425                 430

Glu Glu Glu Glu Glu Gln Thr Ser Glu Gln Asp Ser Thr Phe Gln Gly
            435                 440                 445

His Thr Leu Val Asp Ala Lys His Glu Val Glu Ile Thr Ser Asp Gly
        450                 455                 460

Met Glu Thr Thr Phe Ile Asp Ser Val Glu Asp Ser Glu Ser Glu Glu
465                 470                 475                 480

Glu Glu Glu Gly Lys Ser Ser Glu Thr Gly Lys Val Lys Thr Thr Ser
            485                 490                 495

Leu Thr Glu Lys Lys Ala Ser Arg Arg Gln Lys Glu Ile Pro Phe Ser
        500                 505                 510

Tyr Leu Val Gly Asp Ser Gly Lys Lys Lys Leu Val Lys His Gln Val
        515                 520                 525

Val His Lys Thr Gln Glu Glu Glu Glu Thr Ala Val Pro Thr Ser Gln
        530                 535                 540

Gly Thr Gly Thr Pro Cys Leu Thr Leu Cys Leu Ala Ser Pro Ser Lys
545                 550                 555                 560

Ser Leu Glu Met Ser His Asp Glu His Lys Lys His Ser His Thr Asn
565 570 575

Leu Ser Ile Ser Thr Gly Val Thr Lys Leu Lys Lys Thr Glu Glu Lys
580 585 590

Lys His Arg Thr Leu His Thr Glu Glu Leu Thr Ser Lys Glu Ala Asp
595 600 605

Leu Thr Glu Glu Thr Glu Glu Asn Leu Arg Ser Ser Val Ile Asn Ser
610 615 620

Ile Arg Glu Ile Lys Glu Glu Ile Gly Asn Leu Lys Ser Ser His Ser
625 630 635 640

Gly Val Leu Glu Ile Glu Asn Ser Val Asp Asp Leu Ser Ser Arg Met
645 650 655

Asp Ile Leu Glu Glu Arg Ile Asp Ser Leu Glu Asp Gln Ile Glu Glu
660 665 670

Phe Ser Lys Asp Thr Met Gln Met Thr Lys Gln Ile Ile Ser Lys Glu
675 680 685

Arg Gln Arg Asp Ile Glu Glu Arg Ser Arg Ser Cys Asn Ile Arg Leu
690 695 700

Ile Gly Ile Pro Glu Lys Glu Ser Tyr Glu Asn Arg Ala Glu Asp Ile
705 710 715 720

Ile Lys Glu Ile Ile Asp Glu Asn Phe Ala Glu Leu Lys Lys Gly Ser
725 730 735

Ser Leu Glu Ile Val Ser Ala Cys Arg Val Pro Ser Lys Ile Asp Glu
740 745 750

Lys Arg Leu Thr Pro Arg His Ile Leu Val Lys Phe Trp Asn Ser Ser
755 760 765

Asp Lys Glu Lys Ile Ile Arg Ala Ser Arg Glu Arg Arg Glu Ile Thr
770 775 780

Tyr Gln Gly Thr Arg Ile Arg Leu Thr Ala Asp Leu Ser Leu Asp Thr
785 790 795 800

Leu Asp Ala Arg Ser Lys Trp Ser Asn Val Phe Lys Val Leu Leu Glu
805 810 815

```
Lys Gly Phe Asn Pro Arg Ile Leu Tyr Pro Ala Lys Met Ala Phe Asp
        820                 825             830

Phe Arg Gly Lys Thr Lys Val Phe Leu Ser Ile Glu Glu Phe Arg Asp
        835                 840             845

Tyr Val Leu His Met Pro Thr Leu Arg Glu Leu Leu Gly Asn Asn Ile
        850                 855             860

Pro
865
```

<210> 5
<211> 323
<212> PRT
<213> Homo Sapiens

<220>
<221> MISC_FEATURE
<222> (1)..(323)
<223> OGTA037 = Swiss Prot Accession No. Q9HA72, Protein FAM26B

<400> 5

Met Ala Ala Leu Ile Ala Glu Asn Phe Arg Phe Leu Ser Leu Phe Phe
1           5               10              15

Lys Ser Lys Asp Val Met Ile Phe Asn Gly Leu Val Ala Leu Gly Thr
            20              25              30

Val Gly Ser Gln Glu Leu Phe Ser Val Val Ala Phe His Cys Pro Cys
            35              40              45

Ser Pro Ala Arg Asn Tyr Leu Tyr Gly Leu Ala Ala Ile Gly Val Pro
    50              55              60

Ala Leu Val Leu Phe Ile Ile Gly Ile Ile Leu Asn Asn His Thr Trp
65              70              75              80

Asn Leu Val Ala Glu Cys Gln His Arg Arg Thr Lys Asn Cys Ser Ala
            85              90              95

Ala Pro Thr Phe Leu Leu Leu Ser Ser Ile Leu Gly Arg Ala Ala Val
            100             105             110

Ala Pro Val Thr Trp Ser Val Ile Ser Leu Leu Arg Gly Glu Ala Tyr
            115             120             125

Val Cys Ala Leu Ser Glu Phe Val Asp Pro Ser Ser Leu Thr Ala Arg
            130             135             140

```
Glu Glu His Phe Pro Ser Ala His Ala Thr Glu Ile Leu Ala Arg Phe
145             150             155             160

Pro Cys Lys Glu Asn Pro Asp Asn Leu Ser Asp Phe Arg Glu Glu Val
                165             170             175

Ser Arg Arg Leu Arg Tyr Glu Ser Gln Leu Phe Gly Trp Leu Leu Ile
            180             185             190

Gly Val Val Ala Ile Leu Val Phe Leu Thr Lys Cys Leu Lys His Tyr
        195             200             205

Cys Ser Pro Leu Ser Tyr Arg Gln Glu Ala Tyr Trp Ala Gln Tyr Arg
    210             215             220

Ala Asn Glu Asp Gln Leu Phe Gln Arg Thr Ala Glu Val His Ser Arg
225             230             235             240

Val Leu Ala Ala Asn Asn Val Arg Arg Phe Phe Gly Phe Val Ala Leu
            245             250             255

Asn Lys Asp Asp Glu Glu Leu Ile Ala Asn Phe Pro Val Glu Gly Thr
            260             265             270

Gln Pro Arg Pro Gln Trp Asn Ala Ile Thr Gly Val Tyr Leu Tyr Arg
        275             280             285

Glu Asn Gln Gly Leu Pro Leu Tyr Ser Arg Leu His Lys Trp Ala Gln
    290             295             300

Gly Leu Ala Gly Asn Gly Ala Ala Pro Asp Asn Val Glu Met Ala Leu
305             310             315             320

Leu Pro Ser
```

<210> 6
<211> 918
<212> PRT
<213> Homo Sapiens

<220>
<221> MISC_FEATURE
<222> (1)..(918)
<223> OGTA041 = Swiss Prot Accession No. P40189, Interleukin-6 receptor subunit beta

<400> 6

```
Met Leu Thr Leu Gln Thr Trp Val Val Gln Ala Leu Phe Ile Phe Leu
1               5               10              15
```

```
Thr Thr Glu Ser Thr Gly Glu Leu Leu Asp Pro Cys Gly Tyr Ile Ser
            20                  25                  30

Pro Glu Ser Pro Val Val Gln Leu His Ser Asn Phe Thr Ala Val Cys
            35                  40                  45

Val Leu Lys Glu Lys Cys Met Asp Tyr Phe His Val Asn Ala Asn Tyr
            50                  55                  60

Ile Val Trp Lys Thr Asn His Phe Thr Ile Pro Lys Glu Gln Tyr Thr
65                  70                  75                  80

Ile Ile Asn Arg Thr Ala Ser Ser Val Thr Phe Thr Asp Ile Ala Ser
            85                  90                  95

Leu Asn Ile Gln Leu Thr Cys Asn Ile Leu Thr Phe Gly Gln Leu Glu
            100                 105                 110

Gln Asn Val Tyr Gly Ile Thr Ile Ile Ser Gly Leu Pro Pro Glu Lys
            115                 120                 125

Pro Lys Asn Leu Ser Cys Ile Val Asn Glu Gly Lys Lys Met Arg Cys
            130                 135                 140

Glu Trp Asp Gly Gly Arg Glu Thr His Leu Glu Thr Asn Phe Thr Leu
145                 150                 155                 160

Lys Ser Glu Trp Ala Thr His Lys Phe Ala Asp Cys Lys Ala Lys Arg
                    165                 170                 175

Asp Thr Pro Thr Ser Cys Thr Val Asp Tyr Ser Thr Val Tyr Phe Val
            180                 185                 190

Asn Ile Glu Val Trp Val Glu Ala Glu Asn Ala Leu Gly Lys Val Thr
            195                 200                 205

Ser Asp His Ile Asn Phe Asp Pro Val Tyr Lys Val Lys Pro Asn Pro
            210                 215                 220

Pro His Asn Leu Ser Val Ile Asn Ser Glu Glu Leu Ser Ser Ile Leu
225                 230                 235                 240

Lys Leu Thr Trp Thr Asn Pro Ser Ile Lys Ser Val Ile Ile Leu Lys
                    245                 250                 255

Tyr Asn Ile Gln Tyr Arg Thr Lys Asp Ala Ser Thr Trp Ser Gln Ile
            260                 265                 270
```

155

```
Pro Pro Glu Asp Thr Ala Ser Thr Arg Ser Ser Phe Thr Val Gln Asp
    275             280             285

Leu Lys Pro Phe Thr Glu Tyr Val Phe Arg Ile Arg Cys Met Lys Glu
    290             295             300

Asp Gly Lys Gly Tyr Trp Ser Asp Trp Ser Glu Glu Ala Ser Gly Ile
305             310             315             320

Thr Tyr Glu Asp Arg Pro Ser Lys Ala Pro Ser Phe Trp Tyr Lys Ile
                325             330             335

Asp Pro Ser His Thr Gln Gly Tyr Arg Thr Val Gln Leu Val Trp Lys
            340             345             350

Thr Leu Pro Pro Phe Glu Ala Asn Gly Lys Ile Leu Asp Tyr Glu Val
            355             360             365

Thr Leu Thr Arg Trp Lys Ser His Leu Gln Asn Tyr Thr Val Asn Ala
    370             375             380

Thr Lys Leu Thr Val Asn Leu Thr Asn Asp Arg Tyr Leu Ala Thr Leu
385             390             395             400

Thr Val Arg Asn Leu Val Gly Lys Ser Asp Ala Ala Val Leu Thr Ile
            405             410             415

Pro Ala Cys Asp Phe Gln Ala Thr His Pro Val Met Asp Leu Lys Ala
            420             425             430

Phe Pro Lys Asp Asn Met Leu Trp Val Glu Trp Thr Thr Pro Arg Glu
            435             440             445

Ser Val Lys Lys Tyr Ile Leu Glu Trp Cys Val Leu Ser Asp Lys Ala
    450             455             460

Pro Cys Ile Thr Asp Trp Gln Gln Glu Asp Gly Thr Val His Arg Thr
465             470             475             480

Tyr Leu Arg Gly Asn Leu Ala Glu Ser Lys Cys Tyr Leu Ile Thr Val
                485             490             495

Thr Pro Val Tyr Ala Asp Gly Pro Gly Ser Pro Glu Ser Ile Lys Ala
            500             505             510

Tyr Leu Lys Gln Ala Pro Pro Ser Lys Gly Pro Thr Val Arg Thr Lys
            515             520             525

Lys Val Gly Lys Asn Glu Ala Val Leu Glu Trp Asp Gln Leu Pro Val
```

530                         535                         540

```
Asp Val Gln Asn Gly Phe Ile Arg Asn Tyr Thr Ile Phe Tyr Arg Thr
545             550             555             560

Ile Ile Gly Asn Glu Thr Ala Val Asn Val Asp Ser Ser His Thr Glu
            565             570             575

Tyr Thr Leu Ser Ser Leu Thr Ser Asp Thr Leu Tyr Met Val Arg Met
            580             585             590

Ala Ala Tyr Thr Asp Glu Gly Gly Lys Asp Gly Pro Glu Phe Thr Phe
            595             600             605

Thr Thr Pro Lys Phe Ala Gln Gly Glu Ile Glu Ala Ile Val Val Pro
        610             615             620

Val Cys Leu Ala Phe Leu Leu Thr Thr Leu Leu Gly Val Leu Phe Cys
625             630             635             640

Phe Asn Lys Arg Asp Leu Ile Lys Lys His Ile Trp Pro Asn Val Pro
            645             650             655

Asp Pro Ser Lys Ser His Ile Ala Gln Trp Ser Pro His Thr Pro Pro
            660             665             670

Arg His Asn Phe Asn Ser Lys Asp Gln Met Tyr Ser Asp Gly Asn Phe
            675             680             685

Thr Asp Val Ser Val Val Glu Ile Glu Ala Asn Asp Lys Lys Pro Phe
        690             695             700

Pro Glu Asp Leu Lys Ser Leu Asp Leu Phe Lys Lys Glu Lys Ile Asn
705             710             715             720

Thr Glu Gly His Ser Ser Gly Ile Gly Gly Ser Ser Cys Met Ser Ser
            725             730             735

Ser Arg Pro Ser Ile Ser Ser Ser Asp Glu Asn Glu Ser Ser Gln Asn
            740             745             750

Thr Ser Ser Thr Val Gln Tyr Ser Thr Val Val His Ser Gly Tyr Arg
            755             760             765

His Gln Val Pro Ser Val Gln Val Phe Ser Arg Ser Glu Ser Thr Gln
        770             775             780

Pro Leu Leu Asp Ser Glu Glu Arg Pro Glu Asp Leu Gln Leu Val Asp
785             790             795             800
```

157

```
His Val Asp Gly Gly Asp Gly Ile Leu Pro Arg Gln Gln Tyr Phe Lys
            805                 810                 815


Gln Asn Cys Ser Gln His Glu Ser Ser Pro Asp Ile Ser His Phe Glu
            820                 825                 830


Arg Ser Lys Gln Val Ser Ser Val Asn Glu Glu Asp Phe Val Arg Leu
            835                 840                 845


Lys Gln Gln Ile Ser Asp His Ile Ser Gln Ser Cys Gly Ser Gly Gln
    850                 855                 860


Met Lys Met Phe Gln Glu Val Ser Ala Ala Asp Ala Phe Gly Pro Gly
865                 870                 875                 880


Thr Glu Gly Gln Val Glu Arg Phe Glu Thr Val Gly Met Glu Ala Ala
                885                 890                 895


Thr Asp Glu Gly Met Pro Lys Ser Tyr Leu Pro Gln Thr Val Arg Gln
                900                 905                 910


Gly Gly Tyr Met Pro Gln
            915
```

<210> 7
<211> 1042
<212> PRT
<213> Homo Sapiens

<220>
<221> MISC_FEATURE
<222> (1)..(1042)
<223> OGTA053 = Swiss Prot Accession No. Q9Y4D2, Sn1-specific diacylglycerol lipase alpha

<400> 7

```
Met Pro Gly Ile Val Val Phe Arg Arg Arg Trp Ser Val Gly Ser Asp
1               5                   10                  15

Asp Leu Val Leu Pro Ala Ile Phe Leu Phe Leu Leu His Thr Thr Trp
            20                  25                  30

Phe Val Ile Leu Ser Val Val Leu Phe Gly Leu Val Tyr Asn Pro His
            35                  40                  45

Glu Ala Cys Ser Leu Asn Leu Val Asp His Gly Arg Gly Tyr Leu Gly
    50                  55                  60

Ile Leu Leu Ser Cys Met Ile Ala Glu Met Ala Ile Ile Trp Leu Ser
```

```
         65                    70                    75                    80


Met Arg Gly Gly Ile Leu Tyr Thr Glu Pro Arg Asp Ser Met Gln Tyr
                85                    90                    95


Val Leu Tyr Val Arg Leu Ala Ile Leu Val Ile Glu Phe Ile Tyr Ala
            100                   105                   110


Ile Val Gly Ile Val Trp Leu Thr Gln Tyr Tyr Thr Ser Cys Asn Asp
            115                   120                   125


Leu Thr Ala Lys Asn Val Thr Leu Gly Met Val Val Cys Asn Trp Val
            130                   135                   140


Val Ile Leu Ser Val Cys Ile Thr Val Leu Cys Val Phe Asp Pro Thr
145                   150                   155                   160


Gly Arg Thr Phe Val Lys Leu Arg Ala Thr Lys Arg Arg Gln Arg Asn
                165                   170                   175


Leu Arg Thr Tyr Asn Leu Arg His Arg Leu Glu Glu Gly Gln Ala Thr
                180                   185                   190


Ser Trp Ser Arg Arg Leu Lys Val Phe Leu Cys Cys Thr Arg Thr Lys
                195                   200                   205


Asp Ser Gln Ser Asp Ala Tyr Ser Glu Ile Ala Tyr Leu Phe Ala Glu
            210                   215                   220


Phe Phe Arg Asp Leu Asp Ile Val Pro Ser Asp Ile Ile Ala Gly Leu
225                   230                   235                   240


Val Leu Leu Arg Gln Arg Gln Arg Ala Lys Arg Asn Ala Val Leu Asp
                245                   250                   255


Glu Ala Asn Asn Asp Ile Leu Ala Phe Leu Ser Gly Met Pro Val Thr
                260                   265                   270


Arg Asn Thr Lys Tyr Leu Asp Leu Lys Asn Ser Gln Glu Met Leu Arg
                275                   280                   285


Tyr Lys Glu Val Cys Tyr Tyr Met Leu Phe Ala Leu Ala Ala Tyr Gly
            290                   295                   300


Trp Pro Met Tyr Leu Met Arg Lys Pro Ala Cys Gly Leu Cys Gln Leu
305                   310                   315                   320


Ala Arg Ser Cys Ser Cys Cys Leu Cys Pro Ala Arg Pro Arg Phe Ala
                325                   330                   335
```

```
Pro Gly Val Thr Ile Glu Glu Asp Asn Cys Cys Gly Cys Asn Ala Ile
            340             345             350

Ala Ile Arg Arg His Phe Leu Asp Glu Asn Met Thr Ala Val Asp Ile
            355             360             365

Val Tyr Thr Ser Cys His Asp Ala Val Tyr Glu Thr Pro Phe Tyr Val
            370             375             380

Ala Val Asp His Asp Lys Lys Lys Val Val Ile Ser Ile Arg Gly Thr
385             390             395             400

Leu Ser Pro Lys Asp Ala Leu Thr Asp Leu Thr Gly Asp Ala Glu Arg
            405             410             415

Leu Pro Val Glu Gly His His Gly Thr Trp Leu Gly His Lys Gly Met
            420             425             430

Val Leu Ser Ala Glu Tyr Ile Lys Lys Lys Leu Glu Gln Glu Met Val
            435             440             445

Leu Ser Gln Ala Phe Gly Arg Asp Leu Gly Arg Gly Thr Lys His Tyr
            450             455             460

Gly Leu Ile Val Val Gly His Ser Leu Gly Ala Gly Thr Ala Ala Ile
465             470             475             480

Leu Ser Phe Leu Leu Arg Pro Gln Tyr Pro Thr Leu Lys Cys Phe Ala
            485             490             495

Tyr Ser Pro Pro Gly Gly Leu Leu Ser Glu Asp Ala Met Glu Tyr Ser
            500             505             510

Lys Glu Phe Val Thr Ala Val Val Leu Gly Lys Asp Leu Val Pro Arg
            515             520             525

Ile Gly Leu Ser Gln Leu Glu Gly Phe Arg Arg Gln Leu Leu Asp Val
            530             535             540

Leu Gln Arg Ser Thr Lys Pro Lys Trp Arg Ile Ile Val Gly Ala Thr
545             550             555             560

Lys Cys Ile Pro Lys Ser Glu Leu Pro Glu Glu Val Glu Val Thr Thr
            565             570             575

Leu Ala Ser Thr Arg Leu Trp Thr His Pro Ser Asp Leu Thr Ile Ala
            580             585             590
```

161

Leu Ser Ala Ser Thr Pro Leu Tyr Pro Pro Gly Arg Ile Ile His Val
        595                 600             605

Val His Asn His Pro Ala Glu Gln Cys Cys Cys Cys Glu Gln Glu Glu
        610                 615             620

Pro Thr Tyr Phe Ala Ile Trp Gly Asp Asn Lys Ala Phe Asn Glu Val
625                 630             635                 640

Ile Ile Ser Pro Ala Met Leu His Glu His Leu Pro Tyr Val Val Met
            645             650                 655

Glu Gly Leu Asn Lys Val Leu Glu Asn Tyr Asn Lys Gly Lys Thr Ala
        660                 665             670

Leu Leu Ser Ala Ala Lys Val Met Val Ser Pro Thr Glu Val Asp Leu
        675             680                 685

Thr Pro Glu Leu Ile Phe Gln Gln Gln Pro Leu Pro Thr Gly Pro Pro
    690                 695             700

Met Pro Thr Gly Leu Ala Leu Glu Leu Pro Thr Ala Asp His Arg Asn
705                 710             715                 720

Ser Ser Val Arg Ser Lys Ser Gln Ser Glu Met Ser Leu Glu Gly Phe
                725             730             735

Ser Glu Gly Arg Leu Leu Ser Pro Val Val Ala Ala Ala Ala Arg Gln
            740             745             750

Asp Pro Val Glu Leu Leu Leu Leu Ser Thr Gln Glu Arg Leu Ala Ala
        755             760             765

Glu Leu Gln Ala Arg Arg Ala Pro Leu Ala Thr Met Glu Ser Leu Ser
    770             775             780

Asp Thr Glu Ser Leu Tyr Ser Phe Asp Ser Arg Arg Ser Ser Gly Phe
785                 790             795                 800

Arg Ser Ile Arg Gly Ser Pro Ser Leu His Ala Val Leu Glu Arg Asp
            805             810             815

Glu Gly His Leu Phe Tyr Ile Asp Pro Ala Ile Pro Glu Glu Asn Pro
        820             825             830

Ser Leu Ser Ser Arg Thr Glu Leu Leu Ala Ala Asp Ser Leu Ser Lys
        835             840             845

```
His Ser Gln Asp Thr Gln Pro Leu Glu Ala Ala Leu Gly Ser Gly Gly
    850                 855             860

Val Thr Pro Glu Arg Pro Pro Ser Ala Ala Ala Asn Asp Glu Glu Glu
865             870             875                     880

Glu Val Gly Gly Gly Gly Gly Gly Pro Ala Ser Arg Gly Glu Leu Ala
                885             890                 895

Leu His Asn Gly Arg Leu Gly Asp Ser Pro Ser Pro Gln Val Leu Glu
            900             905             910

Phe Ala Glu Phe Ile Asp Ser Leu Phe Asn Leu Asp Ser Lys Ser Ser
        915             920             925

Ser Phe Gln Asp Leu Tyr Cys Met Val Val Pro Glu Ser Pro Thr Ser
    930             935             940

Asp Tyr Ala Glu Gly Pro Lys Ser Pro Ser Gln Gln Glu Ile Leu Leu
945             950             955                     960

Arg Ala Gln Phe Glu Pro Asn Leu Val Pro Lys Pro Pro Arg Leu Phe
            965             970             975

Ala Gly Ser Ala Asp Pro Ser Ser Gly Ile Ser Leu Ser Pro Ser Phe
            980             985             990

Pro Leu Ser Ser Ser Gly Glu Leu  Met Asp Leu Thr Pro  Thr Gly Leu
    995             1000            1005

Ser Ser  Gln Glu Cys Leu Ala  Ala Asp Lys Ile Arg  Thr Ser Thr
    1010            1015            1020

Pro Thr  Gly His Gly Ala Ser  Pro Ala Lys Gln Asp  Glu Leu Val
    1025            1030            1035

Ile Ser  Ala Arg
    1040
```

<210> 8
<211> 125
<212> PRT
<213> Homo Sapiens

<220>
<221> MISC_FEATURE
<222> (1)..(125)
<223> OGTA054 = Swiss Prot Accession No. P13164, Interferon-induced transmembrane protein 1

<400> 8

```
Met His Lys Glu Glu His Glu Val Ala Val Leu Gly Ala Pro Pro Ser
1               5                   10                  15

Thr Ile Leu Pro Arg Ser Thr Val Ile Asn Ile His Ser Glu Thr Ser
            20                  25                  30

Val Pro Asp His Val Val Trp Ser Leu Phe Asn Thr Leu Phe Leu Asn
            35                  40                  45

Trp Cys Cys Leu Gly Phe Ile Ala Phe Ala Tyr Ser Val Lys Ser Arg
        50                  55                  60

Asp Arg Lys Met Val Gly Asp Val Thr Gly Ala Gln Ala Tyr Ala Ser
65                  70                  75                  80

Thr Ala Lys Cys Leu Asn Ile Trp Ala Leu Ile Leu Gly Ile Leu Met
                85                  90                  95

Thr Ile Gly Phe Ile Leu Leu Leu Val Phe Gly Ser Val Thr Val Tyr
            100                 105                 110

His Ile Met Leu Gln Ile Ile Gln Glu Lys Arg Gly Tyr
            115                 120                 125
```

<210> 9
<211> 166
<212> PRT
<213> Homo Sapiens

<220>
<221> MISC_FEATURE
<222> (1)..(166)
<223> OGTA066 = Swiss Prot Accession No. Q8TD06, Anterior gradient protein 3 homolog

<400> 9

```
Met Met Leu His Ser Ala Leu Gly Leu Cys Leu Leu Leu Val Thr Val
1               5                   10                  15

Ser Ser Asn Leu Ala Ile Ala Ile Lys Lys Glu Lys Arg Pro Pro Gln
            20                  25                  30

Thr Leu Ser Arg Gly Trp Gly Asp Asp Ile Thr Trp Val Gln Thr Tyr
            35                  40                  45

Glu Glu Gly Leu Phe Tyr Ala Gln Lys Ser Lys Lys Pro Leu Met Val
        50                  55                  60

Ile His His Leu Glu Asp Cys Gln Tyr Ser Gln Ala Leu Lys Lys Val
65                  70                  75                  80

Phe Ala Gln Asn Glu Glu Ile Gln Glu Met Ala Gln Asn Lys Phe Ile
                85                  90                  95

Met Leu Asn Leu Met His Glu Thr Thr Asp Lys Asn Leu Ser Pro Asp
            100                 105                 110

Gly Gln Tyr Val Pro Arg Ile Met Phe Val Asp Pro Ser Leu Thr Val
            115                 120                 125

Arg Ala Asp Ile Ala Gly Arg Tyr Ser Asn Arg Leu Tyr Thr Tyr Glu
    130                 135                 140

Pro Arg Asp Leu Pro Leu Leu Ile Glu Asn Met Lys Lys Ala Leu Arg
145                 150                 155                 160

Leu Ile Gln Ser Glu Leu
                165
```

<210> 10
<211> 407
<212> PRT
<213> Homo Sapiens

<220>
<221> MISC_FEATURE
<222> (1)..(407)
<223> OGTA072 = Swiss Prot Accession No. Q16186, Adhesion-regulating molecule 1

<400> 10

Met Thr Thr Ser Gly Ala Leu Phe Pro Ser Leu Val Pro Gly Ser Arg
1               5                   10                  15

Gly Ala Ser Asn Lys Tyr Leu Val Glu Phe Arg Ala Gly Lys Met Ser
            20                  25                  30

Leu Lys Gly Thr Thr Val Thr Pro Asp Lys Arg Lys Gly Leu Val Tyr
        35                  40                  45

Ile Gln Gln Thr Asp Asp Ser Leu Ile His Phe Cys Trp Lys Asp Arg
    50                  55                  60

Thr Ser Gly Asn Val Glu Asp Asp Leu Ile Ile Phe Pro Asp Asp Cys
65                  70                  75                  80

Glu Phe Lys Arg Val Pro Gln Cys Pro Ser Gly Arg Val Tyr Val Leu
                85                  90                  95

Lys Phe Lys Ala Gly Ser Lys Arg Leu Phe Phe Trp Met Gln Glu Pro

100        105        110

```
Lys Thr Asp Gln Asp Glu Glu His Cys Arg Lys Val Asn Glu Tyr Leu
        115             120             125

Asn Asn Pro Pro Met Pro Gly Ala Leu Gly Ala Ser Gly Ser Ser Gly
        130             135             140

His Glu Leu Ser Ala Leu Gly Gly Glu Gly Gly Leu Gln Ser Leu Leu
145             150             155             160

Gly Asn Met Ser His Ser Gln Leu Met Gln Leu Ile Gly Pro Ala Gly
                165             170             175

Leu Gly Gly Leu Gly Gly Leu Gly Ala Leu Thr Gly Pro Gly Leu Ala
        180             185             190

Ser Leu Leu Gly Ser Ser Gly Pro Pro Gly Ser Ser Ser Ser Ser Ser
        195             200             205

Ser Arg Ser Gln Ser Ala Ala Val Thr Pro Ser Ser Thr Thr Ser Ser
        210             215             220

Thr Arg Ala Thr Pro Ala Pro Ser Ala Pro Ala Ala Ala Ser Ala Thr
225             230             235             240

Ser Pro Ser Pro Ala Pro Ser Ser Gly Asn Gly Ala Ser Thr Ala Ala
                245             250             255

Ser Pro Thr Gln Pro Ile Gln Leu Ser Asp Leu Gln Ser Ile Leu Ala
        260             265             270

Thr Met Asn Val Pro Ala Gly Pro Ala Gly Gly Gln Gln Val Asp Leu
        275             280             285

Ala Ser Val Leu Thr Pro Glu Ile Met Ala Pro Ile Leu Ala Asn Ala
        290             295             300

Asp Val Gln Glu Arg Leu Leu Pro Tyr Leu Pro Ser Gly Glu Ser Leu
305             310             315             320

Pro Gln Thr Ala Asp Glu Ile Gln Asn Thr Leu Thr Ser Pro Gln Phe
                325             330             335

Gln Gln Ala Leu Gly Met Phe Ser Ala Ala Leu Ala Ser Gly Gln Leu
        340             345             350

Gly Pro Leu Met Cys Gln Phe Gly Leu Pro Ala Glu Ala Val Glu Ala
        355             360             365
```

```
        Ala Asn Lys Gly Asp Val Glu Ala Phe Ala Lys Ala Met Gln Asn Asn
            370             375             380

        Ala Lys Pro Glu Gln Lys Glu Gly Asp Thr Lys Asp Lys Lys Asp Glu
        385             390             395             400

        Glu Glu Asp Met Ser Leu Asp
                        405
```

<210> 11
<211> 1587
<212> PRT
<213> Homo Sapiens

<220>
<221> MISC_FEATURE
<222> (1)..(1587)
<223> OGTA074 = Swiss Prot Accession No. 000508, Latent TGF-beta binding protein-4

<400> 11

```
        Met Gly Asp Val Lys Ala Leu Leu Phe Val Ala Ala Ala Arg Ala Arg
        1               5               10              15

        Arg Leu Gly Gly Ala Ala Ala Ser Glu Ser Leu Ala Val Ser Glu Ala
                    20              25              30

        Phe Cys Arg Val Arg Ser Cys Gln Pro Lys Lys Cys Ala Gly Pro Gln
                    35              40              45

        Arg Cys Leu Asn Pro Val Pro Ala Val Pro Ser Pro Ser Pro Ser Val
                    50              55              60

        Arg Lys Arg Gln Val Ser Leu Asn Trp Gln Pro Leu Thr Leu Gln Glu
        65              70              75              80

        Ala Arg Ala Leu Leu Lys Arg Arg Arg Pro Arg Gly Pro Gly Gly Arg
                        85              90              95

        Gly Leu Leu Arg Arg Arg Pro Pro Gln Arg Ala Pro Ala Gly Lys Ala
                    100             105             110

        Pro Val Leu Cys Pro Leu Ile Cys His Asn Gly Gly Val Cys Val Lys
                    115             120             125

        Pro Asp Arg Cys Leu Cys Pro Pro Asp Phe Ala Gly Lys Phe Cys Gln
                    130             135             140

        Leu His Ser Ser Gly Ala Arg Pro Pro Ala Pro Ala Val Pro Gly Leu
```

145     150     155     160

Thr Arg Ser Val Tyr Thr Met Pro Leu Ala Asn His Arg Asp Asp Glu
     165    170     175

His Gly Val Ala Ser Met Val Ser Val His Val Glu His Pro Gln Glu
     180    185     190

Ala Ser Val Val Val His Gln Val Glu Arg Val Ser Gly Pro Trp Glu
    195    200    205

Glu Ala Asp Ala Glu Ala Val Ala Arg Ala Glu Ala Ala Ala Arg Ala
  210    215    220

Glu Ala Ala Ala Pro Tyr Thr Val Leu Ala Gln Ser Ala Pro Arg Glu
225    230    235    240

Asp Gly Tyr Ser Asp Ala Ser Gly Phe Gly Tyr Cys Phe Arg Glu Leu
    245    250    255

Arg Gly Gly Glu Cys Ala Ser Pro Leu Pro Gly Leu Arg Thr Gln Glu
    260    265    270

Val Cys Cys Arg Gly Ala Gly Leu Ala Trp Gly Val His Asp Cys Gln
  275    280    285

Leu Cys Ser Glu Arg Leu Gly Asn Ser Glu Arg Val Ser Ala Pro Asp
  290    295    300

Gly Pro Cys Pro Thr Gly Phe Glu Arg Val Asn Gly Ser Cys Glu Asp
305    310    315    320

Val Asp Glu Cys Ala Thr Gly Gly Arg Cys Gln His Gly Glu Cys Ala
    325    330    335

Asn Thr Arg Gly Gly Tyr Thr Cys Val Cys Pro Asp Gly Phe Leu Leu
    340    345    350

Asp Ser Ser Arg Ser Ser Cys Ile Ser Gln His Val Ile Ser Glu Ala
    355    360    365

Lys Gly Pro Cys Phe Arg Val Leu Arg Asp Gly Gly Cys Ser Leu Pro
  370    375    380

Ile Leu Arg Asn Ile Thr Lys Gln Ile Cys Cys Cys Ser Arg Val Gly
385    390    395    400

Lys Ala Trp Gly Arg Gly Cys Gln Leu Cys Pro Pro Phe Gly Ser Glu
    405    410    415

```
Gly Phe Arg Glu Ile Cys Pro Ala Gly Pro Gly Tyr His Tyr Ser Ala
            420             425             430

Ser Asp Leu Arg Tyr Asn Thr Arg Pro Leu Gly Gln Glu Pro Pro Arg
            435             440             445

Val Ser Leu Ser Gln Pro Arg Thr Leu Pro Ala Thr Ser Arg Pro Ser
    450             455             460

Ala Gly Phe Leu Pro Thr His Arg Leu Glu Pro Arg Pro Glu Pro Arg
465             470             475             480

Pro Asp Pro Arg Pro Gly Pro Glu Leu Pro Leu Pro Ser Ile Pro Ala
            485             490             495

Trp Thr Gly Pro Glu Ile Pro Glu Ser Gly Pro Ser Ser Gly Met Cys
            500             505             510

Gln Arg Asn Pro Gln Val Cys Gly Pro Gly Arg Cys Ile Ser Arg Pro
            515             520             525

Ser Gly Tyr Thr Cys Ala Cys Asp Ser Gly Phe Arg Leu Ser Pro Gln
    530             535             540

Gly Thr Arg Cys Ile Asp Val Asp Glu Cys Arg Arg Val Pro Pro Pro
545             550             555             560

Cys Ala Pro Gly Arg Cys Glu Asn Ser Pro Gly Ser Phe Arg Cys Val
            565             570             575

Cys Gly Pro Gly Phe Arg Ala Gly Pro Arg Ala Ala Glu Cys Leu Asp
            580             585             590

Val Asp Glu Cys His Arg Val Pro Pro Pro Cys Asp Leu Gly Arg Cys
    595             600             605

Glu Asn Thr Pro Gly Ser Phe Leu Cys Val Cys Pro Ala Gly Tyr Gln
    610             615             620

Ala Ala Pro His Gly Ala Ser Cys Gln Asp Val Asp Glu Cys Thr Gln
625             630             635             640

Ser Pro Gly Leu Cys Gly Arg Gly Ala Cys Lys Asn Leu Pro Gly Ser
            645             650             655

Phe Arg Cys Val Cys Pro Ala Gly Phe Arg Gly Ser Ala Cys Glu Glu
            660             665             670
```

```
Asp Val Asp Glu Cys Ala Gln Glu Pro Pro Pro Cys Gly Pro Gly Arg
    675             680             685

Cys Asp Asn Thr Ala Gly Ser Phe His Cys Ala Cys Pro Ala Gly Phe
    690             695             700

Arg Ser Arg Gly Pro Gly Ala Pro Cys Gln Asp Val Asp Glu Cys Ala
705             710             715             720

Arg Ser Pro Pro Pro Cys Thr Tyr Gly Arg Cys Glu Asn Thr Glu Gly
            725             730             735

Ser Phe Gln Cys Val Cys Pro Met Gly Phe Gln Pro Asn Thr Ala Gly
        740             745             750

Ser Glu Cys Glu Asp Val Asp Glu Cys Glu Asn His Leu Ala Cys Pro
        755             760             765

Gly Gln Glu Cys Val Asn Ser Pro Gly Ser Phe Gln Cys Arg Thr Cys
    770             775             780

Pro Ser Gly His His Leu His Arg Gly Arg Cys Thr Asp Val Asp Glu
785             790             795             800

Cys Ser Ser Gly Ala Pro Pro Cys Gly Pro His Gly His Cys Thr Asn
            805             810             815

Thr Glu Gly Ser Phe Arg Cys Ser Cys Ala Pro Gly Tyr Arg Ala Pro
            820             825             830

Ser Gly Arg Pro Gly Pro Cys Ala Asp Val Asn Glu Cys Leu Glu Gly
    835             840             845

Asp Phe Cys Phe Pro His Gly Glu Cys Leu Asn Thr Asp Gly Ser Phe
850             855             860

Ala Cys Thr Cys Ala Pro Gly Tyr Arg Pro Gly Pro Arg Gly Ala Ser
865             870             875             880

Cys Leu Asp Val Asp Glu Cys Ser Glu Glu Asp Leu Cys Gln Ser Gly
            885             890             895

Ile Cys Thr Asn Thr Asp Gly Ser Phe Glu Cys Ile Cys Pro Pro Gly
        900             905             910

His Arg Ala Gly Pro Asp Leu Ala Ser Cys Leu Asp Val Asp Glu Cys
    915             920             925
```

171

```
Arg Glu Arg Gly Pro Ala Leu Cys Gly Ser Gln Arg Cys Glu Asn Ser
    930                 935                 940

Pro Gly Ser Tyr Arg Cys Val Arg Asp Cys Asp Pro Gly Tyr His Ala
945                 950                 955                 960

Gly Pro Glu Gly Thr Cys Asp Asp Val Asp Glu Cys Gln Glu Tyr Gly
                965                 970                 975

Pro Glu Ile Cys Gly Ala Gln Arg Cys Glu Asn Thr Pro Gly Ser Tyr
                980                 985                 990

Arg Cys Thr Pro Ala Cys Asp Pro  Gly Tyr Gln Pro Thr  Pro Gly Gly
            995                 1000                1005

Gly Cys  Gln Asp Val Asp Glu  Cys Arg Asn Arg Ser  Phe Cys Gly
    1010                1015                1020

Ala His  Ala Val Cys Gln Asn  Leu Pro Gly Ser Phe  Gln Cys Leu
    1025                1030                1035

Cys Asp  Gln Gly Tyr Glu Gly  Ala Arg Asp Gly Arg  His Cys Val
    1040                1045                1050

Asp Val  Asn Glu Cys Glu Thr  Leu Gln Gly Val Cys  Gly Ala Ala
    1055                1060                1065

Leu Cys  Glu Asn Val Glu Gly  Ser Phe Leu Cys Val  Cys Pro Asn
    1070                1075                1080

Ser Pro  Glu Glu Phe Asp Pro  Met Thr Gly Arg Cys  Val Pro Pro
    1085                1090                1095

Arg Thr  Ser Ala Gly Thr Phe  Pro Gly Ser Gln Pro  Gln Ala Pro
    1100                1105                1110

Ala Ser  Pro Val Leu Pro Ala  Arg Pro Pro Pro Pro  Pro Leu Pro
    1115                1120                1125

Arg Arg  Pro Ser Thr Pro Arg  Gln Gly Pro Val Gly  Ser Gly Arg
    1130                1135                1140

Arg Glu  Cys Tyr Phe Asp Thr  Ala Ala Pro Asp Ala  Cys Asp Asn
    1145                1150                1155

Ile Leu  Ala Arg Asn Val Thr  Trp Gln Glu Cys Cys  Cys Thr Val
    1160                1165                1170

Gly Glu  Gly Trp Gly Ser Gly  Cys Arg Ile Gln Gln  Cys Pro Gly
```

172

1175                          1180                          1185

Thr Glu  Thr Ala Glu Tyr Gln  Ser Leu Cys Pro His  Gly Arg Gly
    1190                 1195             1200

Tyr Leu  Ala Pro Ser Gly Asp  Leu Ser Leu Arg Arg  Asp Val Asp
    1205                 1210             1215

Glu Cys  Gln Leu Phe Arg Asp  Gln Val Cys Lys Ser  Gly Val Cys
    1220                 1225             1230

Val Asn  Thr Ala Pro Gly Tyr  Ser Cys Tyr Cys Ser  Asn Gly Tyr
    1235                 1240             1245

Tyr Tyr  His Thr Gln Arg Leu  Glu Cys Ile Asp Asn  Asp Glu Cys
    1250                 1255             1260

Ala Asp  Glu Glu Pro Ala Cys  Glu Gly Gly Arg Cys  Val Asn Thr
    1265                 1270             1275

Val Gly  Ser Tyr His Cys Thr  Cys Glu Pro Pro Leu  Val Leu Asp
    1280                 1285             1290

Gly Ser  Gln Arg Arg Cys Val  Ser Asn Glu Ser Gln  Ser Leu Asp
    1295                 1300             1305

Asp Asn  Leu Gly Val Cys Trp  Gln Glu Val Gly Ala  Asp Leu Val
    1310                 1315             1320

Cys Ser  His Pro Arg Leu Asp  Arg Gln Ala Thr Tyr  Thr Glu Cys
    1325                 1330             1335

Cys Cys  Leu Tyr Gly Glu Ala  Trp Gly Met Asp Cys  Ala Leu Cys
    1340                 1345             1350

Pro Ala  Gln Asp Ser Asp Asp  Phe Glu Ala Leu Cys  Asn Val Leu
    1355                 1360             1365

Arg Pro  Pro Ala Tyr Ser Pro  Pro Arg Pro Gly Gly  Phe Gly Leu
    1370                 1375             1380

Pro Tyr  Glu Tyr Gly Pro Asp  Leu Gly Pro Pro Tyr  Gln Gly Leu
    1385                 1390             1395

Pro Tyr  Gly Pro Glu Leu Tyr  Pro Pro Pro Ala Leu  Pro Tyr Asp
    1400                 1405             1410

Pro Tyr  Pro Pro Pro Pro Gly  Pro Phe Ala Arg Arg  Glu Ala Pro
    1415                 1420             1425

```
        Tyr Gly  Ala Pro Arg Phe Asp  Met Pro Asp Phe Glu  Asp Asp Gly
            1430             1435             1440


        Gly Pro  Tyr Gly Glu Ser Glu  Ala Pro Ala Pro Pro  Gly Pro Gly
            1445             1450             1455


        Thr Arg  Trp Pro Tyr Arg Ser  Arg Asp Thr Arg Arg  Ser Phe Pro
            1460             1465             1470


        Glu Pro  Glu Glu Pro Pro Glu  Gly Gly Ser Tyr Ala  Gly Ser Leu
            1475             1480             1485


        Ala Glu  Pro Tyr Glu Glu Leu  Glu Ala Glu Glu Cys  Gly Ile Leu
            1490             1495             1500


        Asp Gly  Cys Thr Asn Gly Arg  Cys Val Arg Val Pro  Glu Gly Phe
            1505             1510             1515


        Thr Cys  Arg Cys Phe Asp Gly  Tyr Arg Leu Asp Met  Thr Arg Met
            1520             1525             1530


        Ala Cys  Val Asp Ile Asn Glu  Cys Asp Glu Ala Glu  Ala Ala Ser
            1535             1540             1545


        Pro Leu  Cys Val Asn Ala Arg  Cys Leu Asn Thr Asp  Gly Ser Phe
            1550             1555             1560


        Arg Cys  Ile Cys Arg Pro Gly  Phe Ala Pro Thr His  Gln Pro His
            1565             1570             1575


        His Cys  Ala Pro Ala Arg Pro  Arg Ala
            1580             1585
```

<210> 12
<211> 1722
<212> PRT
<213> Homo Sapiens

<220>
<221> MISC_FEATURE
<222> (1)..(1722)
<223> OGTA076 = Swiss Prot Accession No. 060449, Lymphocyte antigen 75

<400> 12

```
Met Arg Thr Gly Trp Ala Thr Pro Arg Arg Pro Ala Gly Leu Leu Met
1               5               10              15


Leu Leu Phe Trp Phe Phe Asp Leu Ala Glu Pro Ser Gly Arg Ala Ala
            20              25              30
```

```
Asn Asp Pro Phe Thr Ile Val His Gly Asn Thr Gly Lys Cys Ile Lys
        35                  40                  45

Pro Val Tyr Gly Trp Ile Val Ala Asp Asp Cys Asp Glu Thr Glu Asp
        50                  55                  60

Lys Leu Trp Lys Trp Val Ser Gln His Arg Leu Phe His Leu His Ser
65                  70                  75                  80

Gln Lys Cys Leu Gly Leu Asp Ile Thr Lys Ser Val Asn Glu Leu Arg
                85                  90                  95

Met Phe Ser Cys Asp Ser Ser Ala Met Leu Trp Trp Lys Cys Glu His
            100                 105                 110

His Ser Leu Tyr Gly Ala Ala Arg Tyr Arg Leu Ala Leu Lys Asp Gly
            115                 120                 125

His Gly Thr Ala Ile Ser Asn Ala Ser Asp Val Trp Lys Lys Gly Gly
        130                 135                 140

Ser Glu Glu Ser Leu Cys Asp Gln Pro Tyr His Glu Ile Tyr Thr Arg
145                 150                 155                 160

Asp Gly Asn Ser Tyr Gly Arg Pro Cys Glu Phe Pro Phe Leu Ile Asp
                165                 170                 175

Gly Thr Trp His His Asp Cys Ile Leu Asp Glu Asp His Ser Gly Pro
            180                 185                 190

Trp Cys Ala Thr Thr Leu Asn Tyr Glu Tyr Asp Arg Lys Trp Gly Ile
        195                 200                 205

Cys Leu Lys Pro Glu Asn Gly Cys Glu Asp Asn Trp Glu Lys Asn Glu
210                 215                 220

Gln Phe Gly Ser Cys Tyr Gln Phe Asn Thr Gln Thr Ala Leu Ser Trp
225                 230                 235                 240

Lys Glu Ala Tyr Val Ser Cys Gln Asn Gln Gly Ala Asp Leu Leu Ser
            245                 250                 255

Ile Asn Ser Ala Ala Glu Leu Thr Tyr Leu Lys Glu Lys Glu Gly Ile
            260                 265                 270

Ala Lys Ile Phe Trp Ile Gly Leu Asn Gln Leu Tyr Ser Ala Arg Gly
        275                 280                 285
```

```
Trp Glu Trp Ser Asp His Lys Pro Leu Asn Phe Leu Asn Trp Asp Pro
    290             295         300

Asp Arg Pro Ser Ala Pro Thr Ile Gly Gly Ser Ser Cys Ala Arg Met
305             310             315                     320

Asp Ala Glu Ser Gly Leu Trp Gln Ser Phe Ser Cys Glu Ala Gln Leu
            325             330                     335

Pro Tyr Val Cys Arg Lys Pro Leu Asn Asn Thr Val Glu Leu Thr Asp
        340             345             350

Val Trp Thr Tyr Ser Asp Thr Arg Cys Asp Ala Gly Trp Leu Pro Asn
        355             360             365

Asn Gly Phe Cys Tyr Leu Leu Val Asn Glu Ser Asn Ser Trp Asp Lys
    370             375             380

Ala His Ala Lys Cys Lys Ala Phe Ser Ser Asp Leu Ile Ser Ile His
385             390             395                     400

Ser Leu Ala Asp Val Glu Val Val Val Thr Lys Leu His Asn Glu Asp
            405             410                     415

Ile Lys Glu Glu Val Trp Ile Gly Leu Lys Asn Ile Asn Ile Pro Thr
        420             425             430

Leu Phe Gln Trp Ser Asp Gly Thr Glu Val Thr Leu Thr Tyr Trp Asp
        435             440             445

Glu Asn Glu Pro Asn Val Pro Tyr Asn Lys Thr Pro Asn Cys Val Ser
    450             455             460

Tyr Leu Gly Glu Leu Gly Gln Trp Lys Val Gln Ser Cys Glu Glu Lys
465             470             475                     480

Leu Lys Tyr Val Cys Lys Arg Lys Gly Glu Lys Leu Asn Asp Ala Ser
            485             490                     495

Ser Asp Lys Met Cys Pro Pro Asp Glu Gly Trp Lys Arg His Gly Glu
        500             505             510

Thr Cys Tyr Lys Ile Tyr Glu Asp Glu Val Pro Phe Gly Thr Asn Cys
        515             520             525

Asn Leu Thr Ile Thr Ser Arg Phe Glu Gln Glu Tyr Leu Asn Asp Leu
    530             535             540
```

```
Met Lys Lys Tyr Asp Lys Ser Leu Arg Lys Tyr Phe Trp Thr Gly Leu
545             550             555                 560

Arg Asp Val Asp Ser Cys Gly Glu Tyr Asn Trp Ala Thr Val Gly Gly
                565             570                 575

Arg Arg Arg Ala Val Thr Phe Ser Asn Trp Asn Phe Leu Glu Pro Ala
            580             585             590

Ser Pro Gly Gly Cys Val Ala Met Ser Thr Gly Lys Ser Val Gly Lys
            595             600             605

Trp Glu Val Lys Asp Cys Arg Ser Phe Lys Ala Leu Ser Ile Cys Lys
    610             615             620

Lys Met Ser Gly Pro Leu Gly Pro Glu Glu Ala Ser Pro Lys Pro Asp
625             630             635                 640

Asp Pro Cys Pro Glu Gly Trp Gln Ser Phe Pro Ala Ser Leu Ser Cys
            645             650             655

Tyr Lys Val Phe His Ala Glu Arg Ile Val Arg Lys Arg Asn Trp Glu
            660             665             670

Glu Ala Glu Arg Phe Cys Gln Ala Leu Gly Ala His Leu Ser Ser Phe
            675             680             685

Ser His Val Asp Glu Ile Lys Glu Phe Leu His Phe Leu Thr Asp Gln
    690             695             700

Phe Ser Gly Gln His Trp Leu Trp Ile Gly Leu Asn Lys Arg Ser Pro
705             710             715                 720

Asp Leu Gln Gly Ser Trp Gln Trp Ser Asp Arg Thr Pro Val Ser Thr
            725             730             735

Ile Ile Met Pro Asn Glu Phe Gln Gln Asp Tyr Asp Ile Arg Asp Cys
            740             745             750

Ala Ala Val Lys Val Phe His Arg Pro Trp Arg Arg Gly Trp His Phe
            755             760             765

Tyr Asp Asp Arg Glu Phe Ile Tyr Leu Arg Pro Phe Ala Cys Asp Thr
    770             775             780

Lys Leu Glu Trp Val Cys Gln Ile Pro Lys Gly Arg Thr Pro Lys Thr
785             790             795                 800

Pro Asp Trp Tyr Asn Pro Asp Arg Ala Gly Ile His Gly Pro Pro Leu
```

                    805                          810                          815

Ile Ile Glu Gly Ser Glu Tyr Trp Phe Val Ala Asp Leu His Leu Asn
            820                  825                  830

Tyr Glu Glu Ala Val Leu Tyr Cys Ala Ser Asn His Ser Phe Leu Ala
        835                  840                  845

Thr Ile Thr Ser Phe Val Gly Leu Lys Ala Ile Lys Asn Lys Ile Ala
    850                  855                  860

Asn Ile Ser Gly Asp Gly Gln Lys Trp Trp Ile Arg Ile Ser Glu Trp
865                  870                  875                  880

Pro Ile Asp Asp His Phe Thr Tyr Ser Arg Tyr Pro Trp His Arg Phe
            885                  890                  895

Pro Val Thr Phe Gly Glu Glu Cys Leu Tyr Met Ser Ala Lys Thr Trp
        900                  905                  910

Leu Ile Asp Leu Gly Lys Pro Thr Asp Cys Ser Thr Lys Leu Pro Phe
        915                  920                  925

Ile Cys Glu Lys Tyr Asn Val Ser Ser Leu Glu Lys Tyr Ser Pro Asp
    930                  935                  940

Ser Ala Ala Lys Val Gln Cys Ser Glu Gln Trp Ile Pro Phe Gln Asn
945                  950                  955                  960

Lys Cys Phe Leu Lys Ile Lys Pro Val Ser Leu Thr Phe Ser Gln Ala
            965                  970                  975

Ser Asp Thr Cys His Ser Tyr Gly Gly Thr Leu Pro Ser Val Leu Ser
        980                  985                  990

Gln Ile Glu Gln Asp Phe Ile Thr  Ser Leu Leu Pro Asp  Met Glu Ala
        995                  1000                  1005

Thr Leu  Trp Ile Gly Leu Arg  Trp Thr Ala Tyr Glu  Lys Ile Asn
    1010                  1015                  1020

Lys Trp  Thr Asp Asn Arg Glu  Leu Thr Tyr Ser Asn  Phe His Pro
    1025                  1030                  1035

Leu Leu  Val Ser Gly Arg Leu  Arg Ile Pro Glu Asn  Phe Phe Glu
    1040                  1045                  1050

Glu Glu  Ser Arg Tyr His Cys  Ala Leu Ile Leu Asn  Leu Gln Lys
    1055                  1060                  1065

```
Ser Pro Phe Thr Gly Thr Trp Asn Phe Thr Ser Cys Ser Glu Arg
    1070              1075              1080

His Phe Val Ser Leu Cys Gln Lys Tyr Ser Glu Val Lys Ser Arg
    1085              1090              1095

Gln Thr Leu Gln Asn Ala Ser Glu Thr Val Lys Tyr Leu Asn Asn
    1100              1105              1110

Leu Tyr Lys Ile Ile Pro Lys Thr Leu Thr Trp His Ser Ala Lys
    1115              1120              1125

Arg Glu Cys Leu Lys Ser Asn Met Gln Leu Val Ser Ile Thr Asp
    1130              1135              1140

Pro Tyr Gln Gln Ala Phe Leu Ser Val Gln Ala Leu Leu His Asn
    1145              1150              1155

Ser Ser Leu Trp Ile Gly Leu Phe Ser Gln Asp Asp Glu Leu Asn
    1160              1165              1170

Phe Gly Trp Ser Asp Gly Lys Arg Leu His Phe Ser Arg Trp Ala
    1175              1180              1185

Glu Thr Asn Gly Gln Leu Glu Asp Cys Val Val Leu Asp Thr Asp
    1190              1195              1200

Gly Phe Trp Lys Thr Val Asp Cys Asn Asp Asn Gln Pro Gly Ala
    1205              1210              1215

Ile Cys Tyr Tyr Ser Gly Asn Glu Thr Glu Lys Glu Val Lys Pro
    1220              1225              1230

Val Asp Ser Val Lys Cys Pro Ser Pro Val Leu Asn Thr Pro Trp
    1235              1240              1245

Ile Pro Phe Gln Asn Cys Cys Tyr Asn Phe Ile Ile Thr Lys Asn
    1250              1255              1260

Arg His Met Ala Thr Thr Gln Asp Glu Val His Thr Lys Cys Gln
    1265              1270              1275

Lys Leu Asn Pro Lys Ser His Ile Leu Ser Ile Arg Asp Glu Lys
    1280              1285              1290

Glu Asn Asn Phe Val Leu Glu Gln Leu Leu Tyr Phe Asn Tyr Met
    1295              1300              1305
```

```
Ala Ser Trp Val Met Leu Gly Ile Thr Tyr Arg Asn Asn Ser Leu
    1310                1315            1320

Met Trp Phe Asp Lys Thr Pro Leu Ser Tyr Thr His Trp Arg Ala
    1325                1330            1335

Gly Arg Pro Thr Ile Lys Asn Glu Lys Phe Leu Ala Gly Leu Ser
    1340                1345            1350

Thr Asp Gly Phe Trp Asp Ile Gln Thr Phe Lys Val Ile Glu Glu
    1355                1360            1365

Ala Val Tyr Phe His Gln His Ser Ile Leu Ala Cys Lys Ile Glu
    1370                1375            1380

Met Val Asp Tyr Lys Glu Glu His Asn Thr Thr Leu Pro Gln Phe
    1385                1390            1395

Met Pro Tyr Glu Asp Gly Ile Tyr Ser Val Ile Gln Lys Lys Val
    1400                1405            1410

Thr Trp Tyr Glu Ala Leu Asn Met Cys Ser Gln Ser Gly Gly His
    1415                1420            1425

Leu Ala Ser Val His Asn Gln Asn Gly Gln Leu Phe Leu Glu Asp
    1430                1435            1440

Ile Val Lys Arg Asp Gly Phe Pro Leu Trp Val Gly Leu Ser Ser
    1445                1450            1455

His Asp Gly Ser Glu Ser Ser Phe Glu Trp Ser Asp Gly Ser Thr
    1460                1465            1470

Phe Asp Tyr Ile Pro Trp Lys Gly Gln Thr Ser Pro Gly Asn Cys
    1475                1480            1485

Val Leu Leu Asp Pro Lys Gly Thr Trp Lys His Glu Lys Cys Asn
    1490                1495            1500

Ser Val Lys Asp Gly Ala Ile Cys Tyr Lys Pro Thr Lys Ser Lys
    1505                1510            1515

Lys Leu Ser Arg Leu Thr Tyr Ser Ser Arg Cys Pro Ala Ala Lys
    1520                1525            1530

Glu Asn Gly Ser Arg Trp Ile Gln Tyr Lys Gly His Cys Tyr Lys
    1535                1540            1545
```

181

```
Ser Asp Gln Ala Leu His Ser Phe Ser Glu Ala Lys Lys Leu Cys
    1550              1555              1560

Ser Lys His Asp His Ser Ala Thr Ile Val Ser Ile Lys Asp Glu
    1565              1570              1575

Asp Glu Asn Lys Phe Val Ser Arg Leu Met Arg Glu Asn Asn Asn
    1580              1585              1590

Ile Thr Met Arg Val Trp Leu Gly Leu Ser Gln His Ser Val Asp
    1595              1600              1605

Gln Ser Trp Ser Trp Leu Asp Gly Ser Glu Val Thr Phe Val Lys
    1610              1615              1620

Trp Glu Asn Lys Ser Lys Ser Gly Val Gly Arg Cys Ser Met Leu
    1625              1630              1635

Ile Ala Ser Asn Glu Thr Trp Lys Lys Val Glu Cys Glu His Gly
    1640              1645              1650

Phe Gly Arg Val Val Cys Lys Val Pro Leu Gly Pro Asp Tyr Thr
    1655              1660              1665

Ala Ile Ala Ile Ile Val Ala Thr Leu Ser Ile Leu Val Leu Met
    1670              1675              1680

Gly Gly Leu Ile Trp Phe Leu Phe Gln Arg His Arg Leu His Leu
    1685              1690              1695

Ala Gly Phe Ser Ser Val Arg Tyr Ala Gln Gly Val Asn Glu Asp
    1700              1705              1710

Glu Ile Met Leu Pro Ser Phe His Asp
    1715              1720
```

<210> 13
<211> 355
<212> PRT
<213> Homo Sapiens

<220>
<221> MISC_FEATURE
<222> (1)..(355)
<223> OGTA085 = Swiss Prot Accession No. Q14318, 38 kDa FK506-binding protein homologue

<400> 13

```
Met Gly Gln Pro Pro Ala Glu Glu Ala Glu Gln Pro Gly Ala Leu Ala
1               5                   10                  15
```

```
        Arg Glu Phe Leu Ala Ala Met Glu Pro Glu Pro Ala Pro Ala Pro Ala
                    20              25              30

        Pro Glu Glu Trp Leu Asp Ile Leu Gly Asn Gly Leu Leu Arg Lys Lys
                    35              40              45

        Thr Leu Val Pro Gly Pro Pro Gly Ser Ser Arg Pro Val Lys Gly Gln
                    50              55              60

        Val Val Thr Val His Leu Gln Thr Ser Leu Glu Asn Gly Thr Arg Val
        65              70              75              80

        Gln Glu Glu Pro Glu Leu Val Phe Thr Leu Gly Asp Cys Asp Val Ile
                    85              90              95

        Gln Ala Leu Asp Leu Ser Val Pro Leu Met Asp Val Gly Glu Thr Ala
                    100             105             110

        Met Val Thr Ala Asp Ser Lys Tyr Cys Tyr Gly Pro Gln Gly Arg Ser
                    115             120             125

        Pro Tyr Ile Pro Pro His Ala Ala Leu Cys Leu Glu Val Thr Leu Lys
        130             135             140

        Thr Ala Val Asp Gly Pro Asp Leu Glu Met Leu Thr Gly Gln Glu Arg
        145             150             155             160

        Val Ala Leu Ala Asn Arg Lys Arg Glu Cys Gly Asn Ala His Tyr Gln
                    165             170             175

        Arg Ala Asp Phe Val Leu Ala Ala Asn Ser Tyr Asp Leu Ala Ile Lys
                    180             185             190

        Ala Ile Thr Ser Ser Ala Lys Val Asp Met Thr Phe Glu Glu Glu Ala
                    195             200             205

        Gln Leu Leu Gln Leu Lys Val Lys Cys Leu Asn Asn Leu Ala Ala Ser
                    210             215             220

        Gln Leu Lys Leu Asp His Tyr Arg Ala Ala Leu Arg Ser Cys Ser Leu
        225             230             235             240

        Val Leu Glu His Gln Pro Asp Asn Ile Lys Ala Leu Phe Arg Lys Gly
                    245             250             255

        Lys Val Leu Ala Gln Gln Gly Glu Tyr Ser Glu Ala Ile Pro Ile Leu
                    260             265             270
```

```
Arg Ala Ala Leu Lys Leu Glu Pro Ser Asn Lys Thr Ile His Ala Glu
        275                 280             285

Leu Ser Lys Leu Val Lys Lys His Ala Ala Gln Arg Ser Thr Glu Thr
        290                 295             300

Ala Leu Tyr Arg Lys Met Leu Gly Asn Pro Ser Arg Leu Pro Ala Lys
305                 310             315                 320

Cys Pro Gly Lys Gly Ala Trp Ser Ile Pro Trp Lys Trp Leu Phe Gly
                325             330             335

Ala Thr Ala Val Ala Leu Gly Gly Val Ala Leu Ser Val Val Ile Ala
        340             345             350

Ala Arg Asn
        355
```

<210> 14
<211> 587
<212> PRT
<213> Homo Sapiens

<220>
<221> MISC_FEATURE
<222> (1)..(587)
<223> OGTA087 = Swiss Prot Accession No. 094935, KIAA0851 protein

<400> 14

```
Met Ala Thr Ala Ala Tyr Glu Gln Leu Lys Leu His Ile Thr Pro Glu
1               5               10              15

Lys Phe Tyr Val Glu Ala Cys Asp Asp Gly Ala Asp Asp Val Leu Thr
            20              25              30

Ile Asp Arg Val Ser Thr Glu Val Thr Leu Ala Val Lys Lys Asp Val
        35              40              45

Pro Pro Ser Ala Val Thr Arg Pro Ile Phe Gly Ile Leu Gly Thr Ile
    50              55              60

His Leu Val Ala Gly Asn Tyr Leu Ile Val Ile Thr Lys Lys Ile Lys
65              70              75              80

Val Gly Glu Phe Phe Ser His Val Val Trp Lys Ala Thr Asp Phe Asp
            85              90              95

Val Leu Ser Tyr Lys Lys Thr Met Leu His Leu Thr Asp Ile Gln Leu
            100             105             110
```

Gln Asp Asn Lys Thr Phe Leu Ala Met Leu Asn His Val Leu Asn Val
        115                 120                 125

Asp Gly Phe Tyr Phe Ser Thr Thr Tyr Asp Leu Thr His Thr Leu Gln
        130                 135                 140

Arg Leu Ser Asn Thr Ser Pro Glu Phe Gln Glu Met Ser Leu Leu Glu
145                 150                 155                 160

Arg Ala Asp Gln Arg Phe Val Trp Asn Gly His Leu Leu Arg Glu Leu
                165                 170                 175

Ser Ala Gln Pro Glu Val His Arg Phe Ala Leu Pro Val Leu His Gly
                180                 185                 190

Phe Ile Thr Met His Ser Cys Ser Ile Asn Gly Lys Tyr Phe Asp Trp
        195                 200                 205

Ile Leu Ile Ser Arg Arg Ser Cys Phe Arg Ala Gly Val Arg Tyr Tyr
        210                 215                 220

Val Arg Gly Ile Asp Ser Glu Gly His Ala Ala Asn Phe Val Glu Thr
225                 230                 235                 240

Glu Gln Ile Val His Tyr Asn Gly Ser Lys Ala Ser Phe Val Gln Thr
                245                 250                 255

Arg Gly Ser Ile Pro Val Phe Trp Ser Gln Arg Pro Asn Leu Lys Tyr
                260                 265                 270

Lys Pro Leu Pro Gln Ile Ser Lys Val Ala Asn His Met Asp Gly Phe
        275                 280                 285

Gln Arg His Phe Asp Ser Gln Val Ile Ile Tyr Gly Lys Gln Val Ile
        290                 295                 300

Ile Asn Leu Ile Asn Gln Lys Gly Ser Glu Lys Pro Leu Glu Gln Thr
305                 310                 315                 320

Phe Ala Thr Met Val Ser Ser Leu Gly Ser Gly Met Met Arg Tyr Ile
                325                 330                 335

Ala Phe Asp Phe His Lys Glu Cys Lys Asn Met Arg Trp Asp Arg Leu
        340                 345                 350

Ser Ile Leu Leu Asp Gln Val Ala Glu Met Gln Asp Glu Leu Ser Tyr
        355                 360                 365

Phe Leu Val Asp Ser Ala Gly Gln Val Val Ala Asn Gln Glu Gly Val

370                    375                        380

Phe Arg Ser Asn Cys Met Asp Cys Leu Asp Arg Thr Asn Val Ile Gln
385                 390             395                    400

Ser Leu Leu Ala Arg Arg Ser Leu Gln Ala Gln Leu Gln Arg Leu Gly
                405             410                415

Val Leu His Val Gly Gln Lys Leu Glu Glu Gln Asp Glu Phe Glu Lys
            420             425             430

Ile Tyr Lys Asn Ala Trp Ala Asp Asn Ala Asn Ala Cys Ala Lys Gln
        435             440             445

Tyr Ala Gly Thr Gly Ala Leu Lys Thr Asp Phe Thr Arg Thr Gly Lys
    450             455             460

Arg Thr His Leu Gly Leu Ile Met Asp Gly Trp Asn Ser Met Ile Arg
465             470             475                    480

Tyr Tyr Lys Asn Asn Phe Ser Asp Gly Phe Arg Gln Asp Ser Ile Asp
            485             490             495

Leu Phe Leu Gly Asn Tyr Ser Val Asp Glu Leu Glu Ser His Ser Pro
            500             505             510

Leu Ser Val Pro Arg Asp Trp Lys Phe Leu Ala Leu Pro Ile Ile Met
    515             520             525

Val Val Ala Phe Ser Met Cys Ile Ile Cys Leu Leu Met Ala Gly Asp
    530             535             540

Thr Trp Thr Glu Thr Leu Ala Tyr Val Leu Phe Trp Gly Val Ala Ser
545             550             555                    560

Ile Gly Thr Phe Phe Ile Ile Leu Tyr Asn Gly Lys Asp Phe Val Asp
            565             570             575

Ala Pro Arg Leu Val Gln Lys Glu Lys Ile Asp
            580             585

<210> 15
<211> 338
<212> PRT
<213> Homo Sapiens

<220>
<221> MISC_FEATURE

187

<222> (1)..(338)
<223> OGTA088 = Swiss Prot Accession No. 015126, Secretory carrier-associated membrane protein 1

<400> 15

```
Met Ser Asp Phe Asp Ser Asn Pro Phe Ala Asp Pro Asp Leu Asn Asn
1               5               10              15

Pro Phe Lys Asp Pro Ser Val Thr Gln Val Thr Arg Asn Val Pro Pro
        20              25              30

Gly Leu Asp Glu Tyr Asn Pro Phe Ser Asp Ser Arg Thr Pro Pro Pro
        35              40              45

Gly Gly Val Lys Met Pro Asn Val Pro Asn Thr Gln Pro Ala Ile Met
    50              55              60

Lys Pro Thr Glu Glu His Pro Ala Tyr Thr Gln Ile Ala Lys Glu His
65              70              75              80

Ala Leu Ala Gln Ala Glu Leu Leu Lys Arg Gln Glu Glu Leu Glu Arg
            85              90              95

Lys Ala Ala Glu Leu Asp Arg Arg Glu Arg Glu Met Gln Asn Leu Ser
            100             105             110

Gln His Gly Arg Lys Asn Asn Trp Pro Pro Leu Pro Ser Asn Phe Pro
        115             120             125

Val Gly Pro Cys Phe Tyr Gln Asp Phe Ser Val Asp Ile Pro Val Glu
    130             135             140

Phe Gln Lys Thr Val Lys Leu Met Tyr Tyr Leu Trp Met Phe His Ala
145             150             155             160

Val Thr Leu Phe Leu Asn Ile Phe Gly Cys Leu Ala Trp Phe Cys Val
            165             170             175

Asp Ser Ala Arg Ala Val Asp Phe Gly Leu Ser Ile Leu Trp Phe Leu
            180             185             190

Leu Phe Thr Pro Cys Ser Phe Val Cys Trp Tyr Arg Pro Leu Tyr Gly
        195             200             205

Ala Phe Arg Ser Asp Ser Ser Phe Arg Phe Phe Val Phe Phe Phe Val
        210             215             220

Tyr Ile Cys Gln Phe Ala Val His Val Leu Gln Ala Ala Gly Phe His
225             230             235             240

Asn Trp Gly Asn Cys Gly Trp Ile Ser Ser Leu Thr Gly Leu Asn Gln
```

EP 2 447 719 B1

245                          250                          255

Asn Ile Pro Val Gly Ile Met Met Ile Ile Ile Ala Ala Leu Phe Thr
            260               265               270

Ala Ser Ala Val Ile Ser Leu Val Met Phe Lys Lys Val His Gly Leu
            275               280               285

Tyr Arg Thr Thr Gly Ala Ser Phe Glu Lys Ala Gln Gln Glu Phe Ala
    290               295               300

Thr Gly Val Met Ser Asn Lys Thr Val Gln Thr Ala Ala Ala Asn Ala
305               310               315               320

Ala Ser Thr Ala Ala Ser Ser Ala Ala Gln Asn Ala Phe Lys Gly Asn
            325               330               335

Gln Ile

<210> 16
<211> 1058
<212> PRT
<213> Homo Sapiens

<220>
<221> MISC_FEATURE
<222> (1)..(1058)
<223> OGTA089 = Swiss Prot Accession No. Q5T225, Polycystic kidney disease 1-like

<400> 16

Met Glu Lys Arg Leu Gly Val Lys Pro Asn Pro Ala Ser Trp Ile Leu
1             5               10               15

Ser Gly Tyr Tyr Trp Gln Thr Ser Ala Lys Trp Leu Arg Ser Leu Tyr
            20               25               30

Leu Phe Tyr Thr Cys Phe Cys Phe Ser Val Leu Trp Leu Ser Thr Asp
            35               40               45

Ala Ser Glu Ser Arg Cys Gln Gln Gly Lys Thr Gln Phe Gly Val Gly
    50               55               60

Leu Arg Ser Gly Gly Glu Asn His Leu Trp Leu Leu Glu Gly Thr Pro
65               70               75               80

Ser Leu Gln Ser Cys Trp Ala Ala Cys Cys Gln Asp Ser Ala Cys His
            85               90               95

**190**

Val Phe Trp Trp Leu Glu Gly Met Cys Ile Gln Ala Asp Cys Ser Arg
                100                 105                 110

Pro Gln Ser Cys Arg Ala Phe Arg Thr His Ser Ser Asn Ser Met Leu
        115                 120                 125

Val Phe Leu Lys Lys Phe Gln Thr Ala Asp Asp Leu Gly Phe Leu Pro
    130                 135                 140

Glu Asp Asp Val Pro His Leu Leu Gly Leu Gly Trp Asn Trp Ala Ser
145                 150                 155                 160

Trp Arg Gln Ser Pro Pro Arg Ala Ala Leu Arg Pro Ala Val Ser Ser
                165                 170                 175

Ser Asp Gln Gln Ser Leu Ile Arg Lys Leu Gln Lys Arg Gly Ser Pro
                180                 185                 190

Ser Asp Val Val Thr Pro Ile Val Thr Gln His Ser Lys Val Asn Asp
                195                 200                 205

Ser Asn Glu Leu Gly Gly Leu Thr Thr Ser Gly Ser Ala Glu Val His
    210                 215                 220

Lys Ala Ile Thr Ile Ser Ser Pro Leu Thr Thr Asp Leu Thr Ala Glu
225                 230                 235                 240

Leu Ser Gly Gly Pro Lys Asn Val Ser Val Gln Pro Glu Ile Ser Glu
                245                 250                 255

Gly Leu Ala Thr Thr Pro Ser Thr Gln Gln Val Lys Ser Ser Glu Lys
        260                 265                 270

Thr Gln Ile Ala Val Pro Gln Pro Val Ala Pro Ser Tyr Ser Tyr Ala
        275                 280                 285

Thr Pro Thr Pro Gln Ala Ser Phe Gln Ser Thr Ser Ala Pro Tyr Pro
    290                 295                 300

Val Ile Lys Glu Leu Val Val Ser Ala Gly Glu Ser Val Gln Ile Thr
305                 310                 315                 320

Leu Pro Lys Asn Glu Val Gln Leu Asn Ala Tyr Val Leu Gln Glu Pro
                325                 330                 335

Pro Lys Gly Glu Thr Tyr Thr Tyr Asp Trp Gln Leu Ile Thr His Pro
        340                 345                 350

Arg Asp Tyr Ser Gly Glu Met Glu Gly Lys His Ser Gln Ile Leu Lys

191

355                          360                          365

Leu Ser Lys Leu Thr Pro Gly Leu Tyr Glu Phe Lys Val Ile Val Glu
    370                 375                 380

Gly Gln Asn Ala His Gly Glu Gly Tyr Val Asn Val Thr Val Lys Pro
385                 390                 395                 400

Glu Pro Arg Lys Asn Arg Pro Pro Ile Ala Ile Val Ser Pro Gln Phe
            405                 410                 415

Gln Glu Ile Ser Leu Pro Thr Thr Ser Thr Val Ile Asp Gly Ser Gln
            420                 425                 430

Ser Thr Asp Asp Asp Lys Ile Val Gln Tyr His Trp Glu Glu Leu Lys
        435                 440                 445

Gly Pro Leu Arg Glu Glu Lys Ile Ser Glu Asp Thr Ala Ile Leu Lys
    450                 455                 460

Leu Ser Lys Leu Val Pro Gly Asn Tyr Thr Phe Ser Leu Thr Val Val
465                 470                 475                 480

Asp Ser Asp Gly Ala Thr Asn Ser Thr Thr Ala Asn Leu Thr Val Asn
            485                 490                 495

Lys Ala Val Asp Tyr Pro Pro Val Ala Asn Ala Gly Pro Asn Gln Val
        500                 505                 510

Ile Thr Leu Pro Gln Asn Ser Ile Thr Leu Phe Gly Asn Gln Ser Thr
        515                 520                 525

Asp Asp His Gly Ile Thr Ser Tyr Glu Trp Ser Leu Ser Pro Ser Ser
    530                 535                 540

Lys Gly Lys Val Val Glu Met Gln Gly Val Arg Thr Pro Thr Leu Gln
545                 550                 555                 560

Leu Ser Ala Met Gln Glu Gly Asp Tyr Thr Tyr Gln Leu Thr Val Thr
            565                 570                 575

Asp Thr Ile Gly Gln Gln Ala Thr Ala Gln Val Thr Val Ile Val Gln
            580                 585                 590

Pro Glu Asn Asn Lys Pro Pro Gln Ala Asp Ala Gly Pro Asp Lys Glu
        595                 600                 605

Leu Thr Leu Pro Val Asp Ser Thr Thr Leu Asp Gly Ser Lys Ser Ser
    610                 615                 620

```
Asp Asp Gln Lys Ile Ile Ser Tyr Leu Trp Glu Lys Thr Gln Gly Pro
625             630             635             640

Asp Gly Val Gln Leu Glu Asn Ala Asn Ser Ser Val Ala Thr Val Thr
                645             650             655

Gly Leu Gln Val Gly Thr Tyr Val Phe Thr Leu Thr Val Lys Asp Glu
                660             665             670

Arg Asn Leu Gln Ser Gln Ser Ser Val Asn Val Ile Val Lys Glu Glu
                675             680             685

Ile Asn Lys Pro Pro Ile Ala Lys Ile Thr Gly Asn Val Val Ile Thr
690             695             700

Leu Pro Thr Ser Thr Ala Glu Leu Asp Gly Ser Lys Ser Ser Asp Asp
705             710             715             720

Lys Gly Ile Val Ser Tyr Leu Trp Thr Arg Asp Glu Gly Ser Pro Ala
                725             730             735

Ala Gly Glu Val Leu Asn His Ser Asp His His Pro Ile Leu Phe Leu
                740             745             750

Ser Asn Leu Val Glu Gly Thr Tyr Thr Phe His Leu Lys Val Thr Asp
                755             760             765

Ala Lys Gly Glu Ser Asp Thr Asp Arg Thr Thr Val Glu Val Lys Pro
        770             775             780

Asp Pro Arg Lys Asn Asn Leu Val Glu Ile Ile Leu Asp Ile Asn Val
785             790             795             800

Ser Gln Leu Thr Glu Arg Leu Lys Gly Met Phe Ile Arg Gln Ile Gly
                805             810             815

Val Leu Leu Gly Val Leu Asp Ser Asp Ile Ile Val Gln Lys Ile Gln
                820             825             830

Pro Tyr Thr Glu Gln Ser Thr Lys Met Val Phe Phe Val Gln Asn Glu
        835             840             845

Pro Pro His Gln Ile Phe Lys Gly His Glu Val Ala Ala Met Leu Lys
        850             855             860

Ser Glu Leu Arg Lys Gln Lys Ala Asp Phe Leu Ile Phe Arg Ala Leu
865             870             875             880
```

```
Glu Val Asn Thr Val Thr Cys Gln Leu Asn Cys Ser Asp His Gly His
                885                 890                 895


Cys Asp Ser Phe Thr Lys Arg Cys Ile Cys Asp Pro Phe Trp Met Glu
            900                 905                 910


Asn Phe Ile Lys Val Gln Leu Arg Asp Gly Asp Ser Asn Cys Ala Leu
        915                 920                 925


Thr Ile Leu Ile Phe Leu Ala Glu Trp Ser Val Leu Tyr Val Ile Ile
    930                 935                 940


Ala Thr Phe Val Ile Val Val Ala Leu Gly Ile Leu Ser Trp Thr Val
945                 950                 955                 960


Ile Cys Cys Cys Lys Arg Gln Lys Gly Lys Pro Lys Arg Lys Ser Lys
                965                 970                 975


Tyr Lys Ile Leu Asp Ala Thr Asp Gln Glu Ser Leu Glu Leu Lys Pro
        980                 985                 990


Thr Ser Arg Ala Gly Ile Lys Gln  Lys Gly Leu Leu Leu  Ser Ser Ser
    995                 1000                1005


Leu Met  His Ser Glu Ser Glu  Leu Asp Ser Asp Asp  Ala Ile Phe
    1010                1015                1020


Thr Trp  Pro Asp Arg Glu Lys  Gly Lys Leu Leu His  Gly Gln Asn
    1025                1030                1035


Gly Ser  Val Pro Asn Gly Gln  Thr Pro Leu Lys Ala  Arg Ser Pro
    1040                1045                1050


Arg Glu  Glu Ile Leu
    1055
```

<210> 17
<211> 2080
<212> PRT
<213> Homo Sapiens

<220>
<221> MISC_FEATURE
<222> (1)..(2080)
<223> OGTA091 = Swiss Prot Accession No. 075923, Dysferlin

<400> 17

```
Met Leu Arg Val Phe Ile Leu Tyr Ala Glu Asn Val His Thr Pro Asp
1                 5                   10                  15
```

Thr Asp Ile Ser Asp Ala Tyr Cys Ser Ala Val Phe Ala Gly Val Lys
20 25 30

Lys Arg Thr Lys Val Ile Lys Asn Ser Val Asn Pro Val Trp Asn Glu
35 40 45

Gly Phe Glu Trp Asp Leu Lys Gly Ile Pro Leu Asp Gln Gly Ser Glu
50 55 60

Leu His Val Val Val Lys Asp His Glu Thr Met Gly Arg Asn Arg Phe
65 70 75 80

Leu Gly Glu Ala Lys Val Pro Leu Arg Glu Val Leu Ala Thr Pro Ser
85 90 95

Leu Ser Ala Ser Phe Asn Ala Pro Leu Leu Asp Thr Lys Lys Gln Pro
100 105 110

Thr Gly Ala Ser Leu Val Leu Gln Val Ser Tyr Thr Pro Leu Pro Gly
115 120 125

Ala Val Pro Leu Phe Pro Pro Pro Thr Pro Leu Glu Pro Ser Pro Thr
130 135 140

Leu Pro Asp Leu Asp Val Val Ala Asp Thr Gly Gly Glu Glu Asp Thr
145 150 155 160

Glu Asp Gln Gly Leu Thr Gly Asp Glu Ala Glu Pro Phe Leu Asp Gln
165 170 175

Ser Gly Gly Pro Gly Ala Pro Thr Thr Pro Arg Lys Leu Pro Ser Arg
180 185 190

Pro Pro Pro His Tyr Pro Gly Ile Lys Arg Lys Arg Ser Ala Pro Thr
195 200 205

Ser Arg Lys Leu Leu Ser Asp Lys Pro Gln Asp Phe Gln Ile Arg Val
210 215 220

Gln Val Ile Glu Gly Arg Gln Leu Pro Gly Val Asn Ile Lys Pro Val
225 230 235 240

Val Lys Val Thr Ala Ala Gly Gln Thr Lys Arg Thr Arg Ile His Lys
245 250 255

Gly Asn Ser Pro Leu Phe Asn Glu Thr Leu Phe Phe Asn Leu Phe Asp
260 265 270

Ser Pro Gly Glu Leu Phe Asp Glu Pro Ile Phe Ile Thr Val Val Asp
        275                 280                 285

Ser Arg Ser Leu Arg Thr Asp Ala Leu Leu Gly Glu Phe Arg Met Asp
    290                 295                 300

Val Gly Thr Ile Tyr Arg Glu Pro Arg His Ala Tyr Leu Arg Lys Trp
305                 310                 315                 320

Leu Leu Leu Ser Asp Pro Asp Asp Phe Ser Ala Gly Ala Arg Gly Tyr
                325                 330                 335

Leu Lys Thr Ser Leu Cys Val Leu Gly Pro Gly Asp Glu Ala Pro Leu
            340                 345                 350

Glu Arg Lys Asp Pro Ser Glu Asp Lys Glu Asp Ile Glu Ser Asn Leu
            355                 360                 365

Leu Arg Pro Thr Gly Val Ala Leu Arg Gly Ala His Phe Cys Leu Lys
    370                 375                 380

Val Phe Arg Ala Glu Asp Leu Pro Gln Met Asp Asp Ala Val Met Asp
385                 390                 395                 400

Asn Val Lys Gln Ile Phe Gly Phe Glu Ser Asn Lys Lys Asn Leu Val
                405                 410                 415

Asp Pro Phe Val Glu Val Ser Phe Ala Gly Lys Met Leu Cys Ser Lys
                420                 425                 430

Ile Leu Glu Lys Thr Ala Asn Pro Gln Trp Asn Gln Asn Ile Thr Leu
        435                 440                 445

Pro Ala Met Phe Pro Ser Met Cys Glu Lys Met Arg Ile Arg Ile Ile
        450                 455                 460

Asp Trp Asp Arg Leu Thr His Asn Asp Ile Val Ala Thr Thr Tyr Leu
465                 470                 475                 480

Ser Met Ser Lys Ile Ser Ala Pro Gly Gly Glu Ile Glu Glu Glu Pro
                485                 490                 495

Ala Gly Ala Val Lys Pro Ser Lys Ala Ser Asp Leu Asp Asp Tyr Leu
            500                 505                 510

Gly Phe Leu Pro Thr Phe Gly Pro Cys Tyr Ile Asn Leu Tyr Gly Ser
            515                 520                 525

Pro Arg Glu Phe Thr Gly Phe Pro Asp Pro Tyr Thr Glu Leu Asn Thr

530                     535                          540

Gly Lys Gly Glu Gly Val Ala Tyr Arg Gly Arg Leu Leu Leu Ser Leu
545                550               555               560

Glu Thr Lys Leu Val Glu His Ser Glu Gln Lys Val Glu Asp Leu Pro
                565               570               575

Ala Asp Asp Ile Leu Arg Val Glu Lys Tyr Leu Arg Arg Arg Lys Tyr
                580               585               590

Ser Leu Phe Ala Ala Phe Tyr Ser Ala Thr Met Leu Gln Asp Val Asp
            595               600               605

Asp Ala Ile Gln Phe Glu Val Ser Ile Gly Asn Tyr Gly Asn Lys Phe
610               615               620

Asp Met Thr Cys Leu Pro Leu Ala Ser Thr Thr Gln Tyr Ser Arg Ala
625               630               635               640

Val Phe Asp Gly Cys His Tyr Tyr Tyr Leu Pro Trp Gly Asn Val Lys
            645               650               655

Pro Val Val Val Leu Ser Ser Tyr Trp Glu Asp Ile Ser His Arg Ile
            660               665               670

Glu Thr Gln Asn Gln Leu Leu Gly Ile Ala Asp Arg Leu Glu Ala Gly
            675               680               685

Leu Glu Gln Val His Leu Ala Leu Lys Ala Gln Cys Ser Thr Glu Asp
            690               695               700

Val Asp Ser Leu Val Ala Gln Leu Thr Asp Glu Leu Ile Ala Gly Cys
705               710               715               720

Ser Gln Pro Leu Gly Asp Ile His Glu Thr Pro Ser Ala Thr His Leu
            725               730               735

Asp Gln Tyr Leu Tyr Gln Leu Arg Thr His His Leu Ser Gln Ile Thr
            740               745               750

Glu Ala Ala Leu Ala Leu Lys Leu Gly His Ser Glu Leu Pro Ala Ala
            755               760               765

Leu Glu Gln Ala Glu Asp Trp Leu Leu Arg Leu Arg Ala Leu Ala Glu
            770               775               780

Glu Pro Gln Asn Ser Leu Pro Asp Ile Val Ile Trp Met Leu Gln Gly
785               790               795               800

Asp Lys Arg Val Ala Tyr Gln Arg Val Pro Ala His Gln Val Leu Phe
              805                 810                 815

Ser Arg Arg Gly Ala Asn Tyr Cys Gly Lys Asn Cys Gly Lys Leu Gln
              820                 825                 830

Thr Ile Phe Leu Lys Tyr Pro Met Glu Lys Val Pro Gly Ala Arg Met
              835                 840                 845

Pro Val Gln Ile Arg Val Lys Leu Trp Phe Gly Leu Ser Val Asp Glu
    850                 855                 860

Lys Glu Phe Asn Gln Phe Ala Glu Gly Lys Leu Ser Val Phe Ala Glu
865                 870                 875                 880

Thr Tyr Glu Asn Glu Thr Lys Leu Ala Leu Val Gly Asn Trp Gly Thr
              885                 890                 895

Thr Gly Leu Thr Tyr Pro Lys Phe Ser Asp Val Thr Gly Lys Ile Lys
              900                 905                 910

Leu Pro Lys Asp Ser Phe Arg Pro Ser Ala Gly Trp Thr Trp Ala Gly
              915                 920                 925

Asp Trp Phe Val Cys Pro Glu Lys Thr Leu Leu His Asp Met Asp Ala
    930                 935                 940

Gly His Leu Ser Phe Val Glu Glu Val Phe Glu Asn Gln Thr Arg Leu
945                 950                 955                 960

Pro Gly Gly Gln Trp Ile Tyr Met Ser Asp Asn Tyr Thr Asp Val Asn
              965                 970                 975

Gly Glu Lys Val Leu Pro Lys Asp Asp Ile Glu Cys Pro Leu Gly Trp
              980                 985                 990

Lys Trp Glu Asp Glu Glu Trp Ser Thr Asp Leu Asn Arg Ala Val Asp
              995                 1000                1005

Glu Gln Gly Trp Glu Tyr Ser Ile Thr Ile Pro Pro Glu Arg Lys
        1010                1015                1020

Pro Lys His Trp Val Pro Ala Glu Lys Met Tyr Tyr Thr His Arg
        1025                1030                1035

Arg Arg Arg Trp Val Arg Leu Arg Arg Arg Asp Leu Ser Gln Met
        1040                1045                1050

```
Glu Ala Leu Lys Arg His Arg Gln Ala Glu Ala Glu Gly Glu Gly
    1055                1060            1065

Trp Glu Tyr Ala Ser Leu Phe Gly Trp Lys Phe His Leu Glu Tyr
    1070                1075            1080

Arg Lys Thr Asp Ala Phe Arg Arg Arg Arg Trp Arg Arg Arg Met
    1085                1090            1095

Glu Pro Leu Glu Lys Thr Gly Pro Ala Ala Val Phe Ala Leu Glu
    1100                1105            1110

Gly Ala Leu Gly Gly Val Met Asp Asp Lys Ser Glu Asp Ser Met
    1115                1120            1125

Ser Val Ser Thr Leu Ser Phe Gly Val Asn Arg Pro Thr Ile Ser
    1130                1135            1140

Cys Ile Phe Asp Tyr Gly Asn Arg Tyr His Leu Arg Cys Tyr Met
    1145                1150            1155

Tyr Gln Ala Arg Asp Leu Ala Ala Met Asp Lys Asp Ser Phe Ser
    1160                1165            1170

Asp Pro Tyr Ala Ile Val Ser Phe Leu His Gln Ser Gln Lys Thr
    1175                1180            1185

Val Val Val Lys Asn Thr Leu Asn Pro Thr Trp Asp Gln Thr Leu
    1190                1195            1200

Ile Phe Tyr Glu Ile Glu Ile Phe Gly Glu Pro Ala Thr Val Ala
    1205                1210            1215

Glu Gln Pro Pro Ser Ile Val Val Glu Leu Tyr Asp His Asp Thr
    1220                1225            1230

Tyr Gly Ala Asp Glu Phe Met Gly Arg Cys Ile Cys Gln Pro Ser
    1235                1240            1245

Leu Glu Arg Met Pro Arg Leu Ala Trp Phe Pro Leu Thr Arg Gly
    1250                1255            1260

Ser Gln Pro Ser Gly Glu Leu Leu Ala Ser Phe Glu Leu Ile Gln
    1265                1270            1275

Arg Glu Lys Pro Ala Ile His His Ile Pro Gly Phe Glu Val Gln
    1280                1285            1290
```

199

```
Glu Thr Ser Arg Ile Leu Asp Glu Ser Glu Asp Thr Asp Leu Pro
    1295                 1300             1305

Tyr Pro Pro Pro Gln Arg Glu Ala Asn Ile Tyr Met Val Pro Gln
    1310             1315             1320

Asn Ile Lys Pro Ala Leu Gln Arg Thr Ala Ile Glu Ile Leu Ala
    1325             1330             1335

Trp Gly Leu Arg Asn Met Lys Ser Tyr Gln Leu Ala Asn Ile Ser
    1340             1345             1350

Ser Pro Ser Leu Val Val Glu Cys Gly Gly Gln Thr Val Gln Ser
    1355             1360             1365

Cys Val Ile Arg Asn Leu Arg Lys Asn Pro Asn Phe Asp Ile Cys
    1370             1375             1380

Thr Leu Phe Met Glu Val Met Leu Pro Arg Glu Glu Leu Tyr Cys
    1385             1390             1395

Pro Pro Ile Thr Val Lys Val Ile Asp Asn Arg Gln Phe Gly Arg
    1400             1405             1410

Arg Pro Val Val Gly Gln Cys Thr Ile Arg Ser Leu Glu Ser Phe
    1415             1420             1425

Leu Cys Asp Pro Tyr Ser Ala Glu Ser Pro Ser Pro Gln Gly Gly
    1430             1435             1440

Pro Asp Asp Val Ser Leu Leu Ser Pro Gly Glu Asp Val Leu Ile
    1445             1450             1455

Asp Ile Asp Asp Lys Glu Pro Leu Ile Pro Ile Gln Glu Glu Glu
    1460             1465             1470

Phe Ile Asp Trp Trp Ser Lys Phe Phe Ala Ser Ile Gly Glu Arg
    1475             1480             1485

Glu Lys Cys Gly Ser Tyr Leu Glu Lys Asp Phe Asp Thr Leu Lys
    1490             1495             1500

Val Tyr Asp Thr Gln Leu Glu Asn Val Glu Ala Phe Glu Gly Leu
    1505             1510             1515

Ser Asp Phe Cys Asn Thr Phe Lys Leu Tyr Arg Gly Lys Thr Gln
    1520             1525             1530

Glu Glu Thr Glu Asp Pro Ser Val Ile Gly Glu Phe Lys Gly Leu
```

```
                1535                    1540                    1545



      Phe Lys Ile Tyr Pro Leu Pro Glu Asp Pro Ala Ile Pro Met Pro
          1550          1555          1560

      Pro Arg Gln Phe His Gln Leu Ala Ala Gln Gly Pro Gln Glu Cys
          1565          1570          1575

      Leu Val Arg Ile Tyr Ile Val Arg Ala Phe Gly Leu Gln Pro Lys
          1580          1585          1590

      Asp Pro Asn Gly Lys Cys Asp Pro Tyr Ile Lys Ile Ser Ile Gly
          1595          1600          1605

      Lys Lys Ser Val Ser Asp Gln Asp Asn Tyr Ile Pro Cys Thr Leu
          1610          1615          1620

      Glu Pro Val Phe Gly Lys Met Phe Glu Leu Thr Cys Thr Leu Pro
          1625          1630          1635

      Leu Glu Lys Asp Leu Lys Ile Thr Leu Tyr Asp Tyr Asp Leu Leu
          1640          1645          1650

      Ser Lys Asp Glu Lys Ile Gly Glu Thr Val Val Asp Leu Glu Asn
          1655          1660          1665

      Arg Leu Leu Ser Lys Phe Gly Ala Arg Cys Gly Leu Pro Gln Thr
          1670          1675          1680

      Tyr Cys Val Ser Gly Pro Asn Gln Trp Arg Asp Gln Leu Arg Pro
          1685          1690          1695

      Ser Gln Leu Leu His Leu Phe Cys Gln Gln His Arg Val Lys Ala
          1700          1705          1710

      Pro Val Tyr Arg Thr Asp Arg Val Met Phe Gln Asp Lys Glu Tyr
          1715          1720          1725

      Ser Ile Glu Glu Ile Glu Ala Gly Arg Ile Pro Asn Pro His Leu
          1730          1735          1740

      Gly Pro Val Glu Glu Arg Leu Ala Leu His Val Leu Gln Gln Gln
          1745          1750          1755

      Gly Leu Val Pro Glu His Val Glu Ser Arg Pro Leu Tyr Ser Pro
          1760          1765          1770

      Leu Gln Pro Asp Ile Glu Gln Gly Lys Leu Gln Met Trp Val Asp
          1775          1780          1785
```

```
Leu Phe Pro Lys Ala Leu Gly Arg Pro Gly Pro Pro Phe Asn Ile
    1790            1795            1800

Thr Pro Arg Arg Ala Arg Arg Phe Phe Leu Arg Cys Ile Ile Trp
    1805            1810            1815

Asn Thr Arg Asp Val Ile Leu Asp Asp Leu Ser Leu Thr Gly Glu
    1820            1825            1830

Lys Met Ser Asp Ile Tyr Val Lys Gly Trp Met Ile Gly Phe Glu
    1835            1840            1845

Glu His Lys Gln Lys Thr Asp Val His Tyr Arg Ser Leu Gly Gly
    1850            1855            1860

Glu Gly Asn Phe Asn Trp Arg Phe Ile Phe Pro Phe Asp Tyr Leu
    1865            1870            1875

Pro Ala Glu Gln Val Cys Thr Ile Ala Lys Lys Asp Ala Phe Trp
    1880            1885            1890

Arg Leu Asp Lys Thr Glu Ser Lys Ile Pro Ala Arg Val Val Phe
    1895            1900            1905

Gln Ile Trp Asp Asn Asp Lys Phe Ser Phe Asp Asp Phe Leu Gly
    1910            1915            1920

Ser Leu Gln Leu Asp Leu Asn Arg Met Pro Lys Pro Ala Lys Thr
    1925            1930            1935

Ala Lys Lys Cys Ser Leu Asp Gln Leu Asp Asp Ala Phe His Pro
    1940            1945            1950

Glu Trp Phe Val Ser Leu Phe Glu Gln Lys Thr Val Lys Gly Trp
    1955            1960            1965

Trp Pro Cys Val Ala Glu Glu Gly Glu Lys Lys Ile Leu Ala Gly
    1970            1975            1980

Lys Leu Glu Met Thr Leu Glu Ile Val Ala Glu Ser Glu His Glu
    1985            1990            1995

Glu Arg Pro Ala Gly Gln Gly Arg Asp Glu Pro Asn Met Asn Pro
    2000            2005            2010

Lys Leu Glu Asp Pro Arg Arg Pro Asp Thr Ser Phe Leu Trp Phe
    2015            2020            2025
```

```
Thr Ser  Pro Tyr Lys Thr Met  Lys Phe Ile Leu Trp  Arg Arg Phe
    2030             2035             2040

Arg Trp  Ala Ile Ile Leu Phe  Ile Ile Leu Phe Ile  Leu Leu Leu
    2045             2050             2055

Phe Leu  Ala Ile Phe Ile Tyr  Ala Phe Pro Asn Tyr  Ala Ala Met
    2060             2065             2070

Lys Leu  Val Lys Pro Phe Ser
    2075             2080
```

<210> 18
<211> 1117
<212> PRT
<213> Homo Sapiens

<220>
<221> MISC_FEATURE
<222> (1)..(1117)
<223> OGTA098 = Swiss Prot Accession No. Q14126, Desmoglein-2

<400> 18

Met Ala Arg Thr Arg Asp Arg Val Arg Leu Leu Leu Leu Leu Ile Cys
1           5               10                  15

Phe Asn Val Gly Ser Gly Leu His Leu Gln Val Leu Ser Thr Arg Asn
            20              25              30

Glu Asn Lys Leu Leu Pro Lys His Pro His Leu Val Arg Gln Lys Arg
        35              40              45

Ala Trp Ile Thr Ala Pro Val Ala Leu Arg Glu Gly Glu Asp Leu Ser
    50              55              60

Lys Lys Asn Pro Ile Ala Lys Ile His Ser Asp Leu Ala Glu Glu Arg
65              70              75              80

Gly Leu Lys Ile Thr Tyr Lys Tyr Thr Gly Lys Gly Ile Thr Glu Pro
            85              90              95

Pro Phe Gly Ile Phe Val Phe Asn Lys Asp Thr Gly Glu Leu Asn Val
            100             105             110

Thr Ser Ile Leu Asp Arg Glu Glu Thr Pro Phe Phe Leu Leu Thr Gly
            115             120             125

Tyr Ala Leu Asp Ala Arg Gly Asn Asn Val Glu Lys Pro Leu Glu Leu
    130             135             140

```
Arg Ile Lys Val Leu Asp Ile Asn Asp Asn Glu Pro Val Phe Thr Gln
145                 150             155             160

Asp Val Phe Val Gly Ser Val Glu Glu Leu Ser Ala Ala His Thr Leu
                165             170             175

Val Met Lys Ile Asn Ala Thr Asp Ala Asp Glu Pro Asn Thr Leu Asn
            180             185             190

Ser Lys Ile Ser Tyr Arg Ile Val Ser Leu Glu Pro Ala Tyr Pro Pro
        195             200             205

Val Phe Tyr Leu Asn Lys Asp Thr Gly Glu Ile Tyr Thr Thr Ser Val
    210             215             220

Thr Leu Asp Arg Glu Glu His Ser Ser Tyr Thr Leu Thr Val Glu Ala
225             230             235             240

Arg Asp Gly Asn Gly Glu Val Thr Asp Lys Pro Val Lys Gln Ala Gln
            245             250             255

Val Gln Ile Arg Ile Leu Asp Val Asn Asp Asn Ile Pro Val Val Glu
        260             265             270

Asn Lys Val Leu Glu Gly Met Val Glu Glu Asn Gln Val Asn Val Glu
    275             280             285

Val Thr Arg Ile Lys Val Phe Asp Ala Asp Glu Ile Gly Ser Asp Asn
    290             295             300

Trp Leu Ala Asn Phe Thr Phe Ala Ser Gly Asn Glu Gly Gly Tyr Phe
305             310             315             320

His Ile Glu Thr Asp Ala Gln Thr Asn Glu Gly Ile Val Thr Leu Ile
            325             330             335

Lys Glu Val Asp Tyr Glu Glu Met Lys Asn Leu Asp Phe Ser Val Ile
        340             345             350

Val Ala Asn Lys Ala Ala Phe His Lys Ser Ile Arg Ser Lys Tyr Lys
    355             360             365

Pro Thr Pro Ile Pro Ile Lys Val Lys Val Lys Asn Val Lys Glu Gly
    370             375             380

Ile His Phe Lys Ser Ser Val Ile Ser Ile Tyr Val Ser Glu Ser Met
385             390             395             400
```

205

Asp Arg Ser Ser Lys Gly Gln Ile Ile Gly Asn Phe Gln Ala Phe Asp
                405                 410                 415

Glu Asp Thr Gly Leu Pro Ala His Ala Arg Tyr Val Lys Leu Glu Asp
            420                 425                 430

Arg Asp Asn Trp Ile Ser Val Asp Ser Val Thr Ser Glu Ile Lys Leu
            435                 440                 445

Ala Lys Leu Pro Asp Phe Glu Ser Arg Tyr Val Gln Asn Gly Thr Tyr
    450                 455                 460

Thr Val Lys Ile Val Ala Ile Ser Glu Asp Tyr Pro Arg Lys Thr Ile
465                 470                 475                 480

Thr Gly Thr Val Leu Ile Asn Val Glu Asp Ile Asn Asp Asn Cys Pro
            485                 490                 495

Thr Leu Ile Glu Pro Val Gln Thr Ile Cys His Asp Ala Glu Tyr Val
            500                 505                 510

Asn Val Thr Ala Glu Asp Leu Asp Gly His Pro Asn Ser Gly Pro Phe
            515                 520                 525

Ser Phe Ser Val Ile Asp Lys Pro Pro Gly Met Ala Glu Lys Trp Lys
    530                 535                 540

Ile Ala Arg Gln Glu Ser Thr Ser Val Leu Leu Gln Gln Ser Glu Lys
545                 550                 555                 560

Lys Leu Gly Arg Ser Glu Ile Gln Phe Leu Ile Ser Asp Asn Gln Gly
            565                 570                 575

Phe Ser Cys Pro Glu Lys Gln Val Leu Thr Leu Thr Val Cys Glu Val
            580                 585                 590

Leu His Gly Ser Gly Cys Arg Glu Ala Gln His Asp Ser Tyr Val Gly
            595                 600                 605

Leu Gly Pro Ala Ala Ile Ala Leu Met Ile Leu Ala Phe Leu Leu Leu
    610                 615                 620

Leu Leu Val Pro Leu Leu Leu Leu Met Cys His Cys Gly Lys Gly Ala
625                 630                 635                 640

Lys Ala Phe Thr Pro Ile Pro Gly Thr Ile Glu Met Leu His Pro Trp
            645                 650                 655

Asn Asn Glu Gly Ala Pro Pro Glu Asp Lys Val Val Pro Ser Phe Leu

206

```
                    660                      665                      670


        Pro Val Asp Gln Gly Gly Ser Leu Val Gly Arg Asn Gly Val Gly Gly
                675             680                 685

        Met Ala Lys Glu Ala Thr Met Lys Gly Ser Ser Ser Ala Ser Ile Val
        690             695                 700

        Lys Gly Gln His Glu Met Ser Glu Met Asp Gly Arg Trp Glu Glu His
        705             710                 715                 720

        Arg Ser Leu Leu Ser Gly Arg Ala Thr Gln Phe Thr Gly Ala Thr Gly
                        725             730                 735

        Ala Ile Met Thr Thr Glu Thr Thr Lys Thr Ala Arg Ala Thr Gly Ala
                    740             745                 750

        Ser Arg Asp Met Ala Gly Ala Gln Ala Ala Ala Val Ala Leu Asn Glu
                755             760                 765

        Glu Phe Leu Arg Asn Tyr Phe Thr Asp Lys Ala Ala Ser Tyr Thr Glu
                770             775                 780

        Glu Asp Glu Asn His Thr Ala Lys Asp Cys Leu Leu Val Tyr Ser Gln
        785                 790                 795                 800

        Glu Glu Thr Glu Ser Leu Asn Ala Ser Ile Gly Cys Cys Ser Phe Ile
                        805                 810                 815

        Glu Gly Glu Leu Asp Asp Arg Phe Leu Asp Asp Leu Gly Leu Lys Phe
                820                 825                 830

        Lys Thr Leu Ala Glu Val Cys Leu Gly Gln Lys Ile Asp Ile Asn Lys
                835                 840                 845

        Glu Ile Glu Gln Arg Gln Lys Pro Ala Thr Glu Thr Ser Met Asn Thr
                850                 855                 860

        Ala Ser His Ser Leu Cys Glu Gln Thr Met Val Asn Ser Glu Asn Thr
        865                 870                 875                 880

        Tyr Ser Ser Gly Ser Ser Phe Pro Val Pro Lys Ser Leu Gln Glu Ala
                        885                 890                 895

        Asn Ala Glu Lys Val Thr Gln Glu Ile Val Thr Glu Arg Ser Val Ser
                900                 905                 910

        Ser Arg Gln Ala Gln Lys Val Ala Thr Pro Leu Pro Asp Pro Met Ala
                915                 920                 925
```

```
Ser Arg Asn Val Ile Ala Thr Glu Thr Ser Tyr Val Thr Gly Ser Thr
    930                 935             940

Met Pro Pro Thr Thr Val Ile Leu Gly Pro Ser Gln Pro Gln Ser Leu
945                 950             955                 960

Ile Val Thr Glu Arg Val Tyr Ala Pro Ala Ser Thr Leu Val Asp Gln
                965             970                 975

Pro Tyr Ala Asn Glu Gly Thr Val Val Val Thr Glu Arg Val Ile Gln
                980             985             990

Pro His Gly Gly Gly Ser Asn Pro  Leu Glu Gly Thr Gln  His Leu Gln
        995                 1000                1005

Asp Val  Pro Tyr Val Met Val  Arg Glu Arg Glu Ser  Phe Leu Ala
    1010             1015                1020

Pro Ser  Ser Gly Val Gln Pro  Thr Leu Ala Met Pro  Asn Ile Ala
    1025             1030                1035

Val Gly  Gln Asn Val Thr Val  Thr Glu Arg Val Leu  Ala Pro Ala
    1040             1045                1050

Ser Thr  Leu Gln Ser Ser Tyr  Gln Ile Pro Thr Glu  Asn Ser Met
    1055             1060                1065

Thr Ala  Arg Asn Thr Thr Val  Ser Gly Ala Gly Val  Pro Gly Pro
    1070             1075                1080

Leu Pro  Asp Phe Gly Leu Glu  Glu Ser Gly His Ser  Asn Ser Thr
    1085             1090                1095

Ile Thr  Thr Ser Ser Thr Arg  Val Thr Lys His Ser  Thr Val Gln
    1100             1105                1110

His Ser  Tyr Ser
    1115
```

<210> 19
<211> 1912
<212> PRT
<213> Homo Sapiens

<220>
<221> MISC_FEATURE
<222> (1)..(1912)
<223> OGTA101 = Swiss Prot Accession No. P23468, Receptor-type tyrosine-protein phosphatase delta

<400> 19

```
Met Val His Val Ala Arg Leu Leu Leu Leu Leu Leu Thr Phe Phe Leu
1               5               10              15

Arg Thr Asp Ala Glu Thr Pro Pro Arg Phe Thr Arg Thr Pro Val Asp
            20              25              30

Gln Thr Gly Val Ser Gly Gly Val Ala Ser Phe Ile Cys Gln Ala Thr
        35              40              45

Gly Asp Pro Arg Pro Lys Ile Val Trp Asn Lys Lys Gly Lys Lys Val
        50              55              60

Ser Asn Gln Arg Phe Glu Val Ile Glu Phe Asp Asp Gly Ser Gly Ser
65              70              75              80

Val Leu Arg Ile Gln Pro Leu Arg Thr Pro Arg Asp Glu Ala Ile Tyr
            85              90              95

Glu Cys Val Ala Ser Asn Asn Val Gly Glu Ile Ser Val Ser Thr Arg
        100             105             110

Leu Thr Val Leu Arg Glu Asp Gln Ile Pro Arg Gly Phe Pro Thr Ile
        115             120             125

Asp Met Gly Pro Gln Leu Lys Val Val Glu Arg Thr Arg Thr Ala Thr
    130             135             140

Met Leu Cys Ala Ala Ser Gly Asn Pro Asp Pro Glu Ile Thr Trp Phe
145             150             155             160

Lys Asp Phe Leu Pro Val Asp Thr Ser Asn Asn Asn Gly Arg Ile Lys
            165             170             175

Gln Leu Arg Ser Glu Ser Ile Gly Gly Thr Pro Ile Arg Gly Ala Leu
        180             185             190

Gln Ile Glu Gln Ser Glu Glu Ser Asp Gln Gly Lys Tyr Glu Cys Val
        195             200             205

Ala Thr Asn Ser Ala Gly Thr Arg Tyr Ser Ala Pro Ala Asn Leu Tyr
        210             215             220

Val Arg Glu Leu Arg Glu Val Arg Arg Val Pro Pro Arg Phe Ser Ile
225             230             235             240

Pro Pro Thr Asn His Glu Ile Met Pro Gly Gly Ser Val Asn Ile Thr
            245             250             255
```

```
Cys Val Ala Val Gly Ser Pro Met Pro Tyr Val Lys Trp Met Leu Gly
            260             265             270

Ala Glu Asp Leu Thr Pro Glu Asp Asp Met Pro Ile Gly Arg Asn Val
            275             280             285

Leu Glu Leu Asn Asp Val Arg Gln Ser Ala Asn Tyr Thr Cys Val Ala
            290             295             300

Met Ser Thr Leu Gly Val Ile Glu Ala Ile Ala Gln Ile Thr Val Lys
305             310             315             320

Ala Leu Pro Lys Pro Pro Gly Thr Pro Val Val Thr Glu Ser Thr Ala
            325             330             335

Thr Ser Ile Thr Leu Thr Trp Asp Ser Gly Asn Pro Glu Pro Val Ser
            340             345             350

Tyr Tyr Ile Ile Gln His Lys Pro Lys Asn Ser Glu Glu Leu Tyr Lys
            355             360             365

Glu Ile Asp Gly Val Ala Thr Thr Arg Tyr Ser Val Ala Gly Leu Ser
            370             375             380

Pro Tyr Ser Asp Tyr Glu Phe Arg Val Val Ala Val Asn Asn Ile Gly
385             390             395             400

Arg Gly Pro Pro Ser Glu Pro Val Leu Thr Gln Thr Ser Glu Gln Ala
                405             410             415

Pro Ser Ser Ala Pro Arg Asp Val Gln Ala Arg Met Leu Ser Ser Thr
            420             425             430

Thr Ile Leu Val Gln Trp Lys Glu Pro Glu Glu Pro Asn Gly Gln Ile
            435             440             445

Gln Gly Tyr Arg Val Tyr Tyr Thr Met Asp Pro Thr Gln His Val Asn
            450             455             460

Asn Trp Met Lys His Asn Val Ala Asp Ser Gln Ile Thr Thr Ile Gly
465             470             475             480

Asn Leu Val Pro Gln Lys Thr Tyr Ser Val Lys Val Leu Ala Phe Thr
            485             490             495

Ser Ile Gly Asp Gly Pro Leu Ser Ser Asp Ile Gln Val Ile Thr Gln
            500             505             510
```

Thr Gly Val Pro Gly Gln Pro Leu Asn Phe Lys Ala Glu Pro Glu Ser
        515                 520                 525

Glu Thr Ser Ile Leu Leu Ser Trp Thr Pro Pro Arg Ser Asp Thr Ile
        530                 535                 540

Ala Asn Tyr Glu Leu Val Tyr Lys Asp Gly Glu His Gly Glu Glu Gln
545                 550                 555                 560

Arg Ile Thr Ile Glu Pro Gly Thr Ser Tyr Arg Leu Gln Gly Leu Lys
                565                 570                 575

Pro Asn Ser Leu Tyr Tyr Phe Arg Leu Ala Ala Arg Ser Pro Gln Gly
                580                 585                 590

Leu Gly Ala Ser Thr Ala Glu Ile Ser Ala Arg Thr Met Gln Ser Lys
                595                 600                 605

Pro Ser Ala Pro Pro Gln Asp Ile Ser Cys Thr Ser Pro Ser Ser Thr
        610                 615                 620

Ser Ile Leu Val Ser Trp Gln Pro Pro Val Glu Lys Gln Asn Gly
625                 630                 635                 640

Ile Ile Thr Glu Tyr Ser Ile Lys Tyr Thr Ala Val Asp Gly Glu Asp
                645                 650                 655

Asp Lys Pro His Glu Ile Leu Gly Ile Pro Ser Asp Thr Thr Lys Tyr
                660                 665                 670

Leu Leu Glu Gln Leu Glu Lys Trp Thr Glu Tyr Arg Ile Thr Val Thr
                675                 680                 685

Ala His Thr Asp Val Gly Pro Gly Pro Glu Ser Leu Ser Val Leu Ile
        690                 695                 700

Arg Thr Asn Glu Asp Val Pro Ser Gly Pro Pro Arg Lys Val Glu Val
705                 710                 715                 720

Glu Ala Val Asn Ser Thr Ser Val Lys Val Ser Trp Arg Ser Pro Val
                725                 730                 735

Pro Asn Lys Gln His Gly Gln Ile Arg Gly Tyr Gln Val His Tyr Val
                740                 745                 750

Arg Met Glu Asn Gly Glu Pro Lys Gly Gln Pro Met Leu Lys Asp Val
                755                 760                 765

```
Met Leu Ala Asp Ala Gln Trp Glu Phe Asp Asp Thr Thr Glu His Asp
    770                 775             780

Met Ile Ile Ser Gly Leu Gln Pro Glu Thr Ser Tyr Ser Leu Thr Val
785                 790             795                 800

Thr Ala Tyr Thr Thr Lys Gly Asp Gly Ala Arg Ser Lys Pro Lys Leu
                805             810                 815

Val Ser Thr Thr Gly Ala Val Pro Gly Lys Pro Arg Leu Val Ile Asn
                820             825                 830

His Thr Gln Met Asn Thr Ala Leu Ile Gln Trp His Pro Pro Val Asp
            835             840             845

Thr Phe Gly Pro Leu Gln Gly Tyr Arg Leu Lys Phe Gly Arg Lys Asp
    850             855             860

Met Glu Pro Leu Thr Thr Leu Glu Phe Ser Glu Lys Glu Asp His Phe
865             870             875                 880

Thr Ala Thr Asp Ile His Lys Gly Ala Ser Tyr Val Phe Arg Leu Ser
            885             890             895

Ala Arg Asn Lys Val Gly Phe Gly Glu Glu Met Val Lys Glu Ile Ser
            900             905             910

Ile Pro Glu Glu Val Pro Thr Gly Phe Pro Gln Asn Leu His Ser Glu
    915             920             925

Gly Thr Thr Ser Thr Ser Val Gln Leu Ser Trp Gln Pro Pro Val Leu
    930             935             940

Ala Glu Arg Asn Gly Ile Ile Thr Lys Tyr Thr Leu Leu Tyr Arg Asp
945             950             955                 960

Ile Asn Ile Pro Leu Leu Pro Met Glu Gln Leu Ile Val Pro Ala Asp
            965             970             975

Thr Thr Met Thr Leu Thr Gly Leu Lys Pro Asp Thr Thr Tyr Asp Val
            980             985             990

Lys Val Arg Ala His Thr Ser Lys  Gly Pro Gly Pro Tyr  Ser Pro Ser
    995             1000            1005

Val Gln  Phe Arg Thr Leu Pro  Val Asp Gln Val Phe  Ala Lys Asn
    1010            1015            1020

Phe His  Val Lys Ala Val Met  Lys Thr Ser Val Leu  Leu Ser Trp
```

```
          1025                      1030                        1035


          Glu Ile Pro Glu Asn Tyr Asn  Ser Ala Met Pro Phe  Lys Ile Leu
              1040              1045              1050


          Tyr Asp Asp Gly Lys Met Val  Glu Glu Val Asp Gly  Arg Ala Thr
              1055              1060              1065


          Gln Lys Leu Ile Val Asn Leu  Lys Pro Glu Lys Ser  Tyr Ser Phe
              1070              1075              1080


          Val Leu Thr Asn Arg Gly Asn  Ser Ala Gly Gly Leu  Gln His Arg
              1085              1090              1095


          Val Thr Ala Lys Thr Ala Pro  Asp Val Leu Arg Thr  Lys Pro Ala
              1100              1105              1110


          Phe Ile Gly Lys Thr Asn Leu  Asp Gly Met Ile Thr  Val Gln Leu
              1115              1120              1125


          Pro Glu Val Pro Ala Asn Glu  Asn Ile Lys Gly Tyr  Tyr Ile Ile
              1130              1135              1140


          Ile Val Pro Leu Lys Lys Ser  Arg Gly Lys Phe Ile  Lys Pro Trp
              1145              1150              1155


          Glu Ser Pro Asp Glu Met Glu  Leu Asp Glu Leu Leu  Lys Glu Ile
              1160              1165              1170


          Ser Arg Lys Arg Arg Ser Ile  Arg Tyr Gly Arg Glu  Val Glu Leu
              1175              1180              1185


          Lys Pro Tyr Ile Ala Ala His  Phe Asp Val Leu Pro  Thr Glu Phe
              1190              1195              1200


          Thr Leu Gly Asp Asp Lys His  Tyr Gly Gly Phe Thr  Asn Lys Gln
              1205              1210              1215


          Leu Gln Ser Gly Gln Glu Tyr  Val Phe Phe Val Leu  Ala Val Met
              1220              1225              1230


          Glu His Ala Glu Ser Lys Met  Tyr Ala Thr Ser Pro  Tyr Ser Asp
              1235              1240              1245


          Pro Val Val Ser Met Asp Leu  Asp Pro Gln Pro Ile  Thr Asp Glu
              1250              1255              1260


          Glu Glu Gly Leu Ile Trp Val  Val Gly Pro Val Leu  Ala Val Val
              1265              1270              1275
```

```
Phe Ile  Ile Cys Ile Val Ile  Ala Ile Leu Leu Tyr  Lys Arg Lys
    1280             1285              1290

Arg Ala  Glu Ser Asp Ser Arg  Lys Ser Ser Ile Pro  Asn Asn Lys
    1295             1300              1305

Glu Ile  Pro Ser His His Pro  Thr Asp Pro Val Glu  Leu Arg Arg
    1310             1315              1320

Leu Asn  Phe Gln Thr Pro Gly  Met Ala Ser His Pro  Pro Ile Pro
    1325             1330              1335

Ile Leu  Glu Leu Ala Asp His  Ile Glu Arg Leu Lys  Ala Asn Asp
    1340             1345              1350

Asn Leu  Lys Phe Ser Gln Glu  Tyr Glu Ser Ile Asp  Pro Gly Gln
    1355             1360              1365

Gln Phe  Thr Trp Glu His Ser  Asn Leu Glu Val Asn  Lys Pro Lys
    1370             1375              1380

Asn Arg  Tyr Ala Asn Val Ile  Ala Tyr Asp His Ser  Arg Val Leu
    1385             1390              1395

Leu Ser  Ala Ile Glu Gly Ile  Pro Gly Ser Asp Tyr  Val Asn Ala
    1400             1405              1410

Asn Tyr  Ile Asp Gly Tyr Arg  Lys Gln Asn Ala Tyr  Ile Ala Thr
    1415             1420              1425

Gln Gly  Ser Leu Pro Glu Thr  Phe Gly Asp Phe Trp  Arg Met Ile
    1430             1435              1440

Trp Glu  Gln Arg Ser Ala Thr  Val Val Met Met Thr  Lys Leu Glu
    1445             1450              1455

Glu Arg  Ser Arg Val Lys Cys  Asp Gln Tyr Trp Pro  Ser Arg Gly
    1460             1465              1470

Thr Glu  Thr His Gly Leu Val  Gln Val Thr Leu Leu  Asp Thr Val
    1475             1480              1485

Glu Leu  Ala Thr Tyr Cys Val  Arg Thr Phe Ala Leu  Tyr Lys Asn
    1490             1495              1500

Gly Ser  Ser Glu Lys Arg Glu  Val Arg Gln Phe Gln  Phe Thr Ala
    1505             1510              1515
```

215

```
Trp Pro Asp His Gly Val Pro Glu His Pro Thr Pro Phe Leu Ala
    1520             1525             1530

Phe Leu Arg Arg Val Lys Thr Cys Asn Pro Pro Asp Ala Gly Pro
    1535             1540             1545

Met Val Val His Cys Ser Ala Gly Val Gly Arg Thr Gly Cys Phe
    1550             1555             1560

Ile Val Ile Asp Ala Met Leu Glu Arg Ile Lys His Glu Lys Thr
    1565             1570             1575

Val Asp Ile Tyr Gly His Val Thr Leu Met Arg Ala Gln Arg Asn
    1580             1585             1590

Tyr Met Val Gln Thr Glu Asp Gln Tyr Ile Phe Ile His Asp Ala
    1595             1600             1605

Leu Leu Glu Ala Val Thr Cys Gly Asn Thr Glu Val Pro Ala Arg
    1610             1615             1620

Asn Leu Tyr Ala Tyr Ile Gln Lys Leu Thr Gln Ile Glu Thr Gly
    1625             1630             1635

Glu Asn Val Thr Gly Met Glu Leu Glu Phe Lys Arg Leu Ala Ser
    1640             1645             1650

Ser Lys Ala His Thr Ser Arg Phe Ile Ser Ala Asn Leu Pro Cys
    1655             1660             1665

Asn Lys Phe Lys Asn Arg Leu Val Asn Ile Met Pro Tyr Glu Ser
    1670             1675             1680

Thr Arg Val Cys Leu Gln Pro Ile Arg Gly Val Glu Gly Ser Asp
    1685             1690             1695

Tyr Ile Asn Ala Ser Phe Ile Asp Gly Tyr Arg Gln Gln Lys Ala
    1700             1705             1710

Tyr Ile Ala Thr Gln Gly Pro Leu Ala Glu Thr Thr Glu Asp Phe
    1715             1720             1725

Trp Arg Met Leu Trp Glu His Asn Ser Thr Ile Val Val Met Leu
    1730             1735             1740

Thr Lys Leu Arg Glu Met Gly Arg Glu Lys Cys His Gln Tyr Trp
    1745             1750             1755
```

```
        Pro Ala  Glu Arg Ser Ala Arg  Tyr Gln Tyr Phe Val  Val Asp Pro
            1760             1765             1770

        Met Ala  Glu Tyr Asn Met Pro  Gln Tyr Ile Leu Arg  Glu Phe Lys
            1775             1780             1785

        Val Thr  Asp Ala Arg Asp Gly  Gln Ser Arg Thr Val  Arg Gln Phe
            1790             1795             1800

        Gln Phe  Thr Asp Trp Pro Glu  Gln Gly Val Pro Lys  Ser Gly Glu
            1805             1810             1815

        Gly Phe  Ile Asp Phe Ile Gly  Gln Val His Lys Thr  Lys Glu Gln
            1820             1825             1830

        Phe Gly  Gln Asp Gly Pro Ile  Ser Val His Cys Ser  Ala Gly Val
            1835             1840             1845

        Gly Arg  Thr Gly Val Phe Ile  Thr Leu Ser Ile Val  Leu Glu Arg
            1850             1855             1860

        Met Arg  Tyr Glu Gly Val Val  Asp Ile Phe Gln Thr  Val Lys Met
            1865             1870             1875

        Leu Arg  Thr Gln Arg Pro Ala  Met Val Gln Thr Glu  Asp Gln Tyr
            1880             1885             1890

        Gln Phe  Ser Tyr Arg Ala Ala  Leu Glu Tyr Leu Gly  Ser Phe Asp
            1895             1900             1905

        His Tyr  Ala Thr
            1910
```

<210> 20
<211> 819
<212> PRT
<213> Homo Sapiens

<220>
<221> MISC_FEATURE
<222> (1)..(819)
<223> OGTA104 = Swiss Prot Accession No. Q13443, ADAM 9

<400> 20

Met Gly Ser Gly Ala Arg Phe Pro Ser Gly Thr Leu Arg Val Arg Trp
1                   5                   10                  15

Leu Leu Leu Leu Gly Leu Val Gly Pro Val Leu Gly Ala Ala Arg Pro
                    20                  25                  30

```
Gly Phe Gln Gln Thr Ser His Leu Ser Ser Tyr Glu Ile Ile Thr Pro
        35              40              45

Trp Arg Leu Thr Arg Glu Arg Arg Glu Ala Pro Arg Pro Tyr Ser Lys
        50              55              60

Gln Val Ser Tyr Val Ile Gln Ala Glu Gly Lys Glu His Ile Ile His
65              70              75              80

Leu Glu Arg Asn Lys Asp Leu Leu Pro Glu Asp Phe Val Val Tyr Thr
            85              90              95

Tyr Asn Lys Glu Gly Thr Leu Ile Thr Asp His Pro Asn Ile Gln Asn
            100             105             110

His Cys His Tyr Arg Gly Tyr Val Glu Gly Val His Asn Ser Ser Ile
        115             120             125

Ala Leu Ser Asp Cys Phe Gly Leu Arg Gly Leu Leu His Leu Glu Asn
    130             135             140

Ala Ser Tyr Gly Ile Glu Pro Leu Gln Asn Ser Ser His Phe Glu His
145             150             155             160

Ile Ile Tyr Arg Met Asp Asp Val Tyr Lys Glu Pro Leu Lys Cys Gly
            165             170             175

Val Ser Asn Lys Asp Ile Glu Lys Glu Thr Ala Lys Asp Glu Glu Glu
            180             185             190

Glu Pro Pro Ser Met Thr Gln Leu Leu Arg Arg Arg Arg Ala Val Leu
        195             200             205

Pro Gln Thr Arg Tyr Val Glu Leu Phe Ile Val Val Asp Lys Glu Arg
        210             215             220

Tyr Asp Met Met Gly Arg Asn Gln Thr Ala Val Arg Glu Glu Met Ile
225             230             235             240

Leu Leu Ala Asn Tyr Leu Asp Ser Met Tyr Ile Met Leu Asn Ile Arg
            245             250             255

Ile Val Leu Val Gly Leu Glu Ile Trp Thr Asn Gly Asn Leu Ile Asn
            260             265             270

Ile Val Gly Gly Ala Gly Asp Val Leu Gly Asn Phe Val Gln Trp Arg
            275             280             285

Glu Lys Phe Leu Ile Thr Arg Arg Arg His Asp Ser Ala Gln Leu Val
```

```
              290                      295                         300


         Leu Lys Lys Gly Phe Gly Gly Thr Ala Gly Met Ala Phe Val Gly Thr
         305             310             315                 320

         Val Cys Ser Arg Ser His Ala Gly Gly Ile Asn Val Phe Gly Gln Ile
                     325             330                 335

         Thr Val Glu Thr Phe Ala Ser Ile Val Ala His Glu Leu Gly His Asn
                     340             345                 350

         Leu Gly Met Asn His Asp Asp Gly Arg Asp Cys Ser Cys Gly Ala Lys
                 355             360             365

         Ser Cys Ile Met Asn Ser Gly Ala Ser Gly Ser Arg Asn Phe Ser Ser
             370             375             380

         Cys Ser Ala Glu Asp Phe Glu Lys Leu Thr Leu Asn Lys Gly Gly Asn
         385             390             395                 400

         Cys Leu Leu Asn Ile Pro Lys Pro Asp Glu Ala Tyr Ser Ala Pro Ser
                     405             410                 415

         Cys Gly Asn Lys Leu Val Asp Ala Gly Glu Glu Cys Asp Cys Gly Thr
                     420             425                 430

         Pro Lys Glu Cys Glu Leu Asp Pro Cys Cys Glu Gly Ser Thr Cys Lys
                 435             440             445

         Leu Lys Ser Phe Ala Glu Cys Ala Tyr Gly Asp Cys Cys Lys Asp Cys
                 450             455             460

         Arg Phe Leu Pro Gly Gly Thr Leu Cys Arg Gly Lys Thr Ser Glu Cys
         465             470             475                 480

         Asp Val Pro Glu Tyr Cys Asn Gly Ser Ser Gln Phe Cys Gln Pro Asp
                     485             490                 495

         Val Phe Ile Gln Asn Gly Tyr Pro Cys Gln Asn Asn Lys Ala Tyr Cys
                     500             505             510

         Tyr Asn Gly Met Cys Gln Tyr Tyr Asp Ala Gln Cys Gln Val Ile Phe
                 515             520             525

         Gly Ser Lys Ala Lys Ala Ala Pro Lys Asp Cys Phe Ile Glu Val Asn
             530             535             540

         Ser Lys Gly Asp Arg Phe Gly Asn Cys Gly Phe Ser Gly Asn Glu Tyr
         545             550             555             560
```

Lys Lys Cys Ala Thr Gly Asn Ala Leu Cys Gly Lys Leu Gln Cys Glu
                565                 570                 575

Asn Val Gln Glu Ile Pro Val Phe Gly Ile Val Pro Ala Ile Ile Gln
                580                 585                 590

Thr Pro Ser Arg Gly Thr Lys Cys Trp Gly Val Asp Phe Gln Leu Gly
                595                 600                 605

Ser Asp Val Pro Asp Pro Gly Met Val Asn Glu Gly Thr Lys Cys Gly
                610                 615                 620

Ala Gly Lys Ile Cys Arg Asn Phe Gln Cys Val Asp Ala Ser Val Leu
625                 630                 635                 640

Asn Tyr Asp Cys Asp Val Gln Lys Lys Cys His Gly His Gly Val Cys
                645                 650                 655

Asn Ser Asn Lys Asn Cys His Cys Glu Asn Gly Trp Ala Pro Pro Asn
                660                 665                 670

Cys Glu Thr Lys Gly Tyr Gly Gly Ser Val Asp Ser Gly Pro Thr Tyr
                675                 680                 685

Asn Glu Met Asn Thr Ala Leu Arg Asp Gly Leu Leu Val Phe Phe Phe
                690                 695                 700

Leu Ile Val Pro Leu Ile Val Cys Ala Ile Phe Ile Phe Ile Lys Arg
705                 710                 715                 720

Asp Gln Leu Trp Arg Ser Tyr Phe Arg Lys Lys Arg Ser Gln Thr Tyr
                725                 730                 735

Glu Ser Asp Gly Lys Asn Gln Ala Asn Pro Ser Arg Gln Pro Gly Ser
                740                 745                 750

Val Pro Arg His Val Ser Pro Val Thr Pro Pro Arg Glu Val Pro Ile
                755                 760                 765

Tyr Ala Asn Arg Phe Ala Val Pro Thr Tyr Ala Ala Lys Gln Pro Gln
                770                 775                 780

Gln Phe Pro Ser Arg Pro Pro Pro Gln Pro Lys Val Ser Ser Gln
785                 790                 795                 800

Gly Asn Leu Ile Pro Ala Arg Pro Ala Pro Ala Pro Pro Leu Tyr Ser
                805                 810                 815

221

Ser Leu Thr

<210> 21
<211> 2003
<212> PRT
<213> Homo Sapiens

<220>
<221> MISC_FEATURE
<222> (1)..(2003)
<223> OGTA106 = Swiss Prot Accession No. Q99466, Neurogenic locus notch homologue protein 4

<400> 21

```
Met Gln Pro Pro Ser Leu Leu Leu Leu Leu Leu Leu Leu Leu Leu
1               5                   10                  15

Cys Val Ser Val Val Arg Pro Arg Gly Leu Leu Cys Gly Ser Phe Pro
            20                  25                  30

Glu Pro Cys Ala Asn Gly Gly Thr Cys Leu Ser Leu Ser Leu Gly Gln
            35                  40                  45

Gly Thr Cys Gln Cys Ala Pro Gly Phe Leu Gly Glu Thr Cys Gln Phe
        50                  55                  60

Pro Asp Pro Cys Gln Asn Ala Gln Leu Cys Gln Asn Gly Gly Ser Cys
65                  70                  75                  80

Gln Ala Leu Leu Pro Ala Pro Leu Gly Leu Pro Ser Ser Pro Ser Pro
                85                  90                  95

Leu Thr Pro Ser Phe Leu Cys Thr Cys Leu Pro Gly Phe Thr Gly Glu
            100                 105                 110

Arg Cys Gln Ala Lys Leu Glu Asp Pro Cys Pro Pro Ser Phe Cys Ser
        115                 120                 125

Lys Arg Gly Arg Cys His Ile Gln Ala Ser Gly Arg Pro Gln Cys Ser
        130                 135                 140

Cys Met Pro Gly Trp Thr Gly Glu Gln Cys Gln Leu Arg Asp Phe Cys
145                 150                 155                 160

Ser Ala Asn Pro Cys Val Asn Gly Gly Val Cys Leu Ala Thr Tyr Pro
                165                 170                 175

Gln Ile Gln Cys His Cys Pro Pro Gly Phe Glu Gly His Ala Cys Glu
                180                 185                 190
```

```
Arg Asp Val Asn Glu Cys Phe Gln Asp Pro Gly Pro Cys Pro Lys Gly
    195                 200             205

Thr Ser Cys His Asn Thr Leu Gly Ser Phe Gln Cys Leu Cys Pro Val
    210             215             220

Gly Gln Glu Gly Pro Arg Cys Glu Leu Arg Ala Gly Pro Cys Pro Pro
225             230             235                 240

Arg Gly Cys Ser Asn Gly Gly Thr Cys Gln Leu Met Pro Glu Lys Asp
            245             250                 255

Ser Thr Phe His Leu Cys Leu Cys Pro Pro Gly Phe Ile Gly Pro Asp
            260             265                 270

Cys Glu Val Asn Pro Asp Asn Cys Val Ser His Gln Cys Gln Asn Gly
    275             280                 285

Gly Thr Cys Gln Asp Gly Leu Asp Thr Tyr Thr Cys Leu Cys Pro Glu
    290             295             300

Thr Trp Thr Gly Trp Asp Cys Ser Glu Asp Val Asp Glu Cys Glu Thr
305             310             315                 320

Gln Gly Pro Pro His Cys Arg Asn Gly Gly Thr Cys Gln Asn Ser Ala
            325             330                 335

Gly Ser Phe His Cys Val Cys Val Ser Gly Trp Gly Gly Thr Ser Cys
            340             345                 350

Glu Glu Asn Leu Asp Asp Cys Ile Ala Ala Thr Cys Ala Pro Gly Ser
    355             360                 365

Thr Cys Ile Asp Arg Val Gly Ser Phe Ser Cys Leu Cys Pro Pro Gly
    370             375                 380

Arg Thr Gly Leu Leu Cys His Leu Glu Asp Met Cys Leu Ser Gln Pro
385             390                 395                 400

Cys His Gly Asp Ala Gln Cys Ser Thr Asn Pro Leu Thr Gly Ser Thr
            405             410                 415

Leu Cys Leu Cys Gln Pro Gly Tyr Ser Gly Pro Thr Cys His Gln Asp
            420             425                 430

Leu Asp Glu Cys Leu Met Ala Gln Gln Gly Pro Ser Pro Cys Glu His
    435             440                 445
```

```
Gly Gly Ser Cys Leu Asn Thr Pro Gly Ser Phe Asn Cys Leu Cys Pro
    450                 455             460

Pro Gly Tyr Thr Gly Ser Arg Cys Glu Ala Asp His Asn Glu Cys Leu
465                 470             475                 480

Ser Gln Pro Cys His Pro Gly Ser Thr Cys Leu Asp Leu Leu Ala Thr
                485             490                 495

Phe His Cys Leu Cys Pro Pro Gly Leu Glu Gly Gln Leu Cys Glu Val
            500             505             510

Glu Thr Asn Glu Cys Ala Ser Ala Pro Cys Leu Asn His Ala Asp Cys
        515             520             525

His Asp Leu Leu Asn Gly Phe Gln Cys Ile Cys Leu Pro Gly Phe Ser
    530             535             540

Gly Thr Arg Cys Glu Glu Asp Ile Asp Glu Cys Arg Ser Ser Pro Cys
545             550             555             560

Ala Asn Gly Gly Gln Cys Gln Asp Gln Pro Gly Ala Phe His Cys Lys
                565             570             575

Cys Leu Pro Gly Phe Glu Gly Pro Arg Cys Gln Thr Glu Val Asp Glu
            580             585             590

Cys Leu Ser Asp Pro Cys Pro Val Gly Ala Ser Cys Leu Asp Leu Pro
        595             600             605

Gly Ala Phe Phe Cys Leu Cys Pro Ser Gly Phe Thr Gly Gln Leu Cys
    610             615             620

Glu Val Pro Leu Cys Ala Pro Asn Leu Cys Gln Pro Lys Gln Ile Cys
625             630             635             640

Lys Asp Gln Lys Asp Lys Ala Asn Cys Leu Cys Pro Asp Gly Ser Pro
            645             650             655

Gly Cys Ala Pro Pro Glu Asp Asn Cys Thr Cys His His Gly His Cys
            660             665             670

Gln Arg Ser Ser Cys Val Cys Asp Val Gly Trp Thr Gly Pro Glu Cys
    675             680             685

Glu Ala Glu Leu Gly Gly Cys Ile Ser Ala Pro Cys Ala His Gly Gly
    690             695             700
```

224

```
Thr Cys Tyr Pro Gln Pro Ser Gly Tyr Asn Cys Thr Cys Pro Thr Gly
705             710             715             720

Tyr Thr Gly Pro Thr Cys Ser Glu Glu Met Thr Ala Cys His Ser Gly
            725             730             735

Pro Cys Leu Asn Gly Gly Ser Cys Asn Pro Ser Pro Gly Gly Tyr Tyr
            740             745             750

Cys Thr Cys Pro Pro Ser His Thr Gly Pro Gln Cys Gln Thr Ser Thr
        755             760             765

Asp Tyr Cys Val Ser Ala Pro Cys Phe Asn Gly Gly Thr Cys Val Asn
    770             775             780

Arg Pro Gly Thr Phe Ser Cys Leu Cys Ala Met Gly Phe Gln Gly Pro
785             790             795             800

Arg Cys Glu Gly Lys Leu Arg Pro Ser Cys Ala Asp Ser Pro Cys Arg
            805             810             815

Asn Arg Ala Thr Cys Gln Asp Ser Pro Gln Gly Pro Arg Cys Leu Cys
            820             825             830

Pro Thr Gly Tyr Thr Gly Gly Ser Cys Gln Thr Leu Met Asp Leu Cys
        835             840             845

Ala Gln Lys Pro Cys Pro Arg Asn Ser His Cys Leu Gln Thr Gly Pro
    850             855             860

Ser Phe His Cys Leu Cys Leu Gln Gly Trp Thr Gly Pro Leu Cys Asn
865             870             875             880

Leu Pro Leu Ser Ser Cys Gln Lys Ala Ala Leu Ser Gln Gly Ile Asp
            885             890             895

Val Ser Ser Leu Cys His Asn Gly Gly Leu Cys Val Asp Ser Gly Pro
            900             905             910

Ser Tyr Phe Cys His Cys Pro Pro Gly Phe Gln Gly Ser Leu Cys Gln
    915             920             925

Asp His Val Asn Pro Cys Glu Ser Arg Pro Cys Gln Asn Gly Ala Thr
    930             935             940

Cys Met Ala Gln Pro Ser Gly Tyr Leu Cys Gln Cys Ala Pro Gly Tyr
945             950             955             960

Asp Gly Gln Asn Cys Ser Lys Glu Leu Asp Ala Cys Gln Ser Gln Pro
```

225

                    965                      970                      975

Cys His Asn His Gly Thr Cys Thr Pro Lys Pro Gly Gly Phe His Cys
              980                  985                  990

Ala Cys Pro Pro Gly Phe Val Gly  Leu Arg Cys Glu Gly  Asp Val Asp
         995                 1000                1005

Glu Cys  Leu Asp Gln Pro Cys  His Pro Thr Gly Thr  Ala Ala Cys
    1010             1015                 1020

His Ser  Leu Ala Asn Ala Phe  Tyr Cys Gln Cys Leu  Pro Gly His
    1025                 1030                 1035

Thr Gly  Gln Trp Cys Glu Val  Glu Ile Asp Pro Cys  His Ser Gln
    1040                 1045                 1050

Pro Cys  Phe His Gly Gly Thr  Cys Glu Ala Thr Ala  Gly Ser Pro
    1055                 1060                 1065

Leu Gly  Phe Ile Cys His Cys  Pro Lys Gly Phe Glu  Gly Pro Thr
    1070                 1075                 1080

Cys Ser  His Arg Ala Pro Ser  Cys Gly Phe His His  Cys His His
    1085                 1090                 1095

Gly Gly  Leu Cys Leu Pro Ser  Pro Lys Pro Gly Phe  Pro Pro Arg
    1100                 1105                 1110

Cys Ala  Cys Leu Ser Gly Tyr  Gly Gly Pro Asp Cys  Leu Thr Pro
    1115                 1120                 1125

Pro Ala  Pro Lys Gly Cys Gly  Pro Pro Ser Pro Cys  Leu Tyr Asn
    1130                 1135                 1140

Gly Ser  Cys Ser Glu Thr Thr  Gly Leu Gly Gly Pro  Gly Phe Arg
    1145                 1150                 1155

Cys Ser  Cys Pro His Ser Ser  Pro Gly Pro Arg Cys  Gln Lys Pro
    1160                 1165                 1170

Gly Ala  Lys Gly Cys Glu Gly  Arg Ser Gly Asp Gly  Ala Cys Asp
    1175                 1180                 1185

Ala Gly  Cys Ser Gly Pro Gly  Gly Asn Trp Asp Gly  Gly Asp Cys
    1190                 1195                 1200

Ser Leu  Gly Val Pro Asp Pro  Trp Lys Gly Cys Pro  Ser His Ser
    1205                 1210                 1215

```
Arg Cys Trp Leu Leu Phe Arg Asp Gly Gln Cys His Pro Gln Cys
1220                1225                1230

Asp Ser Glu Glu Cys Leu Phe Asp Gly Tyr Asp Cys Glu Thr Pro
1235                1240                1245

Pro Ala Cys Thr Pro Ala Tyr Asp Gln Tyr Cys His Asp His Phe
1250                1255                1260

His Asn Gly His Cys Glu Lys Gly Cys Asn Thr Ala Glu Cys Gly
1265                1270                1275

Trp Asp Gly Gly Asp Cys Arg Pro Glu Asp Gly Asp Pro Glu Trp
1280                1285                1290

Gly Pro Ser Leu Ala Leu Leu Val Val Leu Ser Pro Pro Ala Leu
1295                1300                1305

Asp Gln Gln Leu Phe Ala Leu Ala Arg Val Leu Ser Leu Thr Leu
1310                1315                1320

Arg Val Gly Leu Trp Val Arg Lys Asp Arg Asp Gly Arg Asp Met
1325                1330                1335

Val Tyr Pro Tyr Pro Gly Ala Arg Ala Glu Glu Lys Leu Gly Gly
1340                1345                1350

Thr Arg Asp Pro Thr Tyr Gln Glu Arg Ala Ala Pro Gln Thr Gln
1355                1360                1365

Pro Leu Gly Lys Glu Thr Asp Ser Leu Ser Ala Gly Phe Val Val
1370                1375                1380

Val Met Gly Val Asp Leu Ser Arg Cys Gly Pro Asp His Pro Ala
1385                1390                1395

Ser Arg Cys Pro Trp Asp Pro Gly Leu Leu Leu Arg Phe Leu Ala
1400                1405                1410

Ala Met Ala Ala Val Gly Ala Leu Glu Pro Leu Leu Pro Gly Pro
1415                1420                1425

Leu Leu Ala Val His Pro His Ala Gly Thr Ala Pro Pro Ala Asn
1430                1435                1440

Gln Leu Pro Trp Pro Val Leu Cys Ser Pro Val Ala Gly Val Ile
1445                1450                1455
```

227

```
Leu Leu Ala Leu Gly Ala Leu Leu Val Leu Gln Leu Ile Arg Arg
    1460            1465            1470

Arg Arg Arg Glu His Gly Ala Leu Trp Leu Pro Pro Gly Phe Thr
    1475            1480            1485

Arg Arg Pro Arg Thr Gln Ser Ala Pro His Arg Arg Arg Pro Pro
    1490            1495            1500

Leu Gly Glu Asp Ser Ile Gly Leu Lys Ala Leu Lys Pro Lys Ala
    1505            1510            1515

Glu Val Asp Glu Asp Gly Val Val Met Cys Ser Gly Pro Glu Glu
    1520            1525            1530

Gly Glu Glu Val Gly Gln Ala Glu Glu Thr Gly Pro Pro Ser Thr
    1535            1540            1545

Cys Gln Leu Trp Ser Leu Ser Gly Gly Cys Gly Ala Leu Pro Gln
    1550            1555            1560

Ala Ala Met Leu Thr Pro Pro Gln Glu Ser Glu Met Glu Ala Pro
    1565            1570            1575

Asp Leu Asp Thr Arg Gly Pro Asp Gly Val Thr Pro Leu Met Ser
    1580            1585            1590

Ala Val Cys Cys Gly Glu Val Gln Ser Gly Thr Phe Gln Gly Ala
    1595            1600            1605

Trp Leu Gly Cys Pro Glu Pro Trp Glu Pro Leu Leu Asp Gly Gly
    1610            1615            1620

Ala Cys Pro Gln Ala His Thr Val Gly Thr Gly Glu Thr Pro Leu
    1625            1630            1635

His Leu Ala Ala Arg Phe Ser Arg Pro Thr Ala Ala Arg Arg Leu
    1640            1645            1650

Leu Glu Ala Gly Ala Asn Pro Asn Gln Pro Asp Arg Ala Gly Arg
    1655            1660            1665

Thr Pro Leu His Ala Ala Val Ala Ala Asp Ala Arg Glu Val Cys
    1670            1675            1680

Gln Leu Leu Leu Arg Ser Arg Gln Thr Ala Val Asp Ala Arg Thr
    1685            1690            1695
```

228

Glu Asp Gly Thr Thr Pro Leu Met Leu Ala Ala Arg Leu Ala Val
    1700            1705            1710

Glu Asp Leu Val Glu Glu Leu Ile Ala Ala Gln Ala Asp Val Gly
    1715            1720            1725

Ala Arg Asp Lys Trp Gly Lys Thr Ala Leu His Trp Ala Ala Ala
    1730            1735            1740

Val Asn Asn Ala Arg Ala Ala Arg Ser Leu Leu Gln Ala Gly Ala
    1745            1750            1755

Asp Lys Asp Ala Gln Asp Asn Arg Glu Gln Thr Pro Leu Phe Leu
    1760            1765            1770

Ala Ala Arg Glu Gly Ala Val Glu Val Ala Gln Leu Leu Leu Gly
    1775            1780            1785

Leu Gly Ala Ala Arg Glu Leu Arg Asp Gln Ala Gly Leu Ala Pro
    1790            1795            1800

Ala Asp Val Ala His Gln Arg Asn His Trp Asp Leu Leu Thr Leu
    1805            1810            1815

Leu Glu Gly Ala Gly Pro Pro Glu Ala Arg His Lys Ala Thr Pro
    1820            1825            1830

Gly Arg Glu Ala Gly Pro Phe Pro Arg Ala Arg Thr Val Ser Val
    1835            1840            1845

Ser Val Pro Pro His Gly Gly Gly Ala Leu Pro Arg Cys Arg Thr
    1850            1855            1860

Leu Ser Ala Gly Ala Gly Pro Arg Gly Gly Gly Ala Cys Leu Gln
    1865            1870            1875

Ala Arg Thr Trp Ser Val Asp Leu Ala Ala Arg Gly Gly Gly Ala
    1880            1885            1890

Tyr Ser His Cys Arg Ser Leu Ser Gly Val Gly Ala Gly Gly Gly
    1895            1900            1905

Pro Thr Pro Arg Gly Arg Arg Phe Ser Ala Gly Met Arg Gly Pro
    1910            1915            1920

Arg Pro Asn Pro Ala Ile Met Arg Gly Arg Tyr Gly Val Ala Ala
    1925            1930            1935

Gly Arg Gly Gly Arg Val Ser Thr Asp Asp Trp Pro Cys Asp Trp

```
                    1940                    1945                    1950


          Val Ala  Leu Gly Ala Cys Gly  Ser Ala Ser Asn Ile  Pro Ile Pro
              1955                1960                1965


          Pro Pro  Cys Leu Thr Pro Ser  Pro Glu Arg Gly Ser  Pro Gln Leu
              1970                1975                1980


          Asp Cys  Gly Pro Pro Ala Leu  Gln Glu Met Pro Ile  Asn Gln Gly
              1985                1990                1995


          Gly Glu  Gly Lys Lys
              2000
```

<210> 22
<211> 390
<212> PRT
<213> Homo Sapiens

<220>
<221> MISC_FEATURE
<222> (1)..(390)
<223> OGTA112 = Swiss Prot Accession No. P48594, Serpin B4

<400> 22

Met Asn Ser Leu Ser Glu Ala Asn Thr Lys Phe Met Phe Asp Leu Phe
1                   5                   10                  15

Gln Gln Phe Arg Lys Ser Lys Glu Asn Asn Ile Phe Tyr Ser Pro Ile
            20                  25                  30

Ser Ile Thr Ser Ala Leu Gly Met Val Leu Leu Gly Ala Lys Asp Asn
        35                  40                  45

Thr Ala Gln Gln Ile Ser Lys Val Leu His Phe Asp Gln Val Thr Glu
    50                  55                  60

Asn Thr Thr Glu Lys Ala Ala Thr Tyr His Val Asp Arg Ser Gly Asn
65                  70                  75                  80

Val His His Gln Phe Gln Lys Leu Leu Thr Glu Phe Asn Lys Ser Thr
            85                  90                  95

Asp Ala Tyr Glu Leu Lys Ile Ala Asn Lys Leu Phe Gly Glu Lys Thr
            100                 105                 110

Tyr Gln Phe Leu Gln Glu Tyr Leu Asp Ala Ile Lys Lys Phe Tyr Gln
            115                 120                 125

Thr Ser Val Glu Ser Thr Asp Phe Ala Asn Ala Pro Glu Glu Ser Arg

                130                          135                              140


Lys Lys Ile Asn Ser Trp Val Glu Ser Gln Thr Asn Glu Lys Ile Lys
145                 150                 155                 160

Asn Leu Phe Pro Asp Gly Thr Ile Gly Asn Asp Thr Thr Leu Val Leu
                165                 170                 175

Val Asn Ala Ile Tyr Phe Lys Gly Gln Trp Glu Asn Lys Phe Lys Lys
                180                 185                 190

Glu Asn Thr Lys Glu Glu Lys Phe Trp Pro Asn Lys Asn Thr Tyr Lys
        195                 200                 205

Ser Val Gln Met Met Arg Gln Tyr Asn Ser Phe Asn Phe Ala Leu Leu
        210                 215                 220

Glu Asp Val Gln Ala Lys Val Leu Glu Ile Pro Tyr Lys Gly Lys Asp
225                 230                 235                 240

Leu Ser Met Ile Val Leu Leu Pro Asn Glu Ile Asp Gly Leu Gln Lys
                245                 250                 255

Leu Glu Glu Lys Leu Thr Ala Glu Lys Leu Met Glu Trp Thr Ser Leu
                260                 265                 270

Gln Asn Met Arg Glu Thr Cys Val Asp Leu His Leu Pro Arg Phe Lys
        275                 280                 285

Met Glu Glu Ser Tyr Asp Leu Lys Asp Thr Leu Arg Thr Met Gly Met
        290                 295                 300

Val Asn Ile Phe Asn Gly Asp Ala Asp Leu Ser Gly Met Thr Trp Ser
305                 310                 315                 320

His Gly Leu Ser Val Ser Lys Val Leu His Lys Ala Phe Val Glu Val
                325                 330                 335

Thr Glu Glu Gly Val Glu Ala Ala Ala Ala Thr Ala Val Val Val Val
                340                 345                 350

Glu Leu Ser Ser Pro Ser Thr Asn Glu Glu Phe Cys Cys Asn His Pro
        355                 360                 365

Phe Leu Phe Phe Ile Arg Gln Asn Lys Thr Asn Ser Ile Leu Phe Tyr
        370                 375                 380

Gly Arg Phe Ser Ser Pro
385                 390

EP 2 447 719 B1

<210> 23
<211> 400
<212> PRT
<213> Homo Sapiens

<220>
<221> MISC_FEATURE
<222> (1)..(400)
<223> OGTA113 = Swiss Prot Accession No. Q9BTV4, Transmembrane protein 43

<400> 23

```
Met Ala Ala Asn Tyr Ser Ser Thr Ser Thr Arg Arg Glu His Val Lys
1               5                   10                  15

Val Lys Thr Ser Ser Gln Pro Gly Phe Leu Glu Arg Leu Ser Glu Thr
            20                  25                  30

Ser Gly Gly Met Phe Val Gly Leu Met Ala Phe Leu Leu Ser Phe Tyr
            35                  40                  45

Leu Ile Phe Thr Asn Glu Gly Arg Ala Leu Lys Thr Ala Thr Ser Leu
        50                  55                  60

Ala Glu Gly Leu Ser Leu Val Val Ser Pro Asp Ser Ile His Ser Val
65                  70                  75                  80

Ala Pro Glu Asn Glu Gly Arg Leu Val His Ile Ile Gly Ala Leu Arg
                85                  90                  95

Thr Ser Lys Leu Leu Ser Asp Pro Asn Tyr Gly Val His Leu Pro Ala
            100                 105                 110

Val Lys Leu Arg Arg His Val Glu Met Tyr Gln Trp Val Glu Thr Glu
            115                 120                 125

Glu Ser Arg Glu Tyr Thr Glu Asp Gly Gln Val Lys Lys Glu Thr Arg
        130                 135                 140

Tyr Ser Tyr Asn Thr Glu Trp Arg Ser Glu Ile Ile Asn Ser Lys Asn
145                 150                 155                 160

Phe Asp Arg Glu Ile Gly His Lys Asn Pro Ser Ala Met Ala Val Glu
                165                 170                 175

Ser Phe Met Ala Thr Ala Pro Phe Val Gln Ile Gly Arg Phe Phe Leu
            180                 185                 190

Ser Ser Gly Leu Ile Asp Lys Val Asp Asn Phe Lys Ser Leu Ser Leu
```

233

```
            195                      200                      205


      Ser Lys Leu Glu Asp Pro His Val Asp Ile Ile Arg Arg Gly Asp Phe
          210                 215                 220


      Phe Tyr His Ser Glu Asn Pro Lys Tyr Pro Glu Val Gly Asp Leu Arg
      225                 230                 235                 240


      Val Ser Phe Ser Tyr Ala Gly Leu Ser Gly Asp Asp Pro Asp Leu Gly
                      245                 250                 255


      Pro Ala His Val Val Thr Val Ile Ala Arg Gln Arg Gly Asp Gln Leu
                      260                 265                 270


      Val Pro Phe Ser Thr Lys Ser Gly Asp Thr Leu Leu Leu Leu His His
                      275                 280                 285


      Gly Asp Phe Ser Ala Glu Glu Val Phe His Arg Glu Leu Arg Ser Asn
          290                 295                 300


      Ser Met Lys Thr Trp Gly Leu Arg Ala Ala Gly Trp Met Ala Met Phe
      305                 310                 315                 320


      Met Gly Leu Asn Leu Met Thr Arg Ile Leu Tyr Thr Leu Val Asp Trp
                      325                 330                 335


      Phe Pro Val Phe Arg Asp Leu Val Asn Ile Gly Leu Lys Ala Phe Ala
                      340                 345                 350


      Phe Cys Val Ala Thr Ser Leu Thr Leu Leu Thr Val Ala Ala Gly Trp
                      355                 360                 365


      Leu Phe Tyr Arg Pro Leu Trp Ala Leu Leu Ile Ala Gly Leu Ala Leu
          370                 375                 380


      Val Pro Ile Leu Val Ala Arg Thr Arg Val Pro Ala Lys Lys Leu Glu
      385                 390                 395                 400
```

<210> 24
<211> 893
<212> PRT
<213> Homo Sapiens

<220>
<221> MISC_FEATURE
<222> (1)..(893)
<223> OGTA119 = Swiss Prot Accession No. Q6UKI4, KIAA1228 protein

<400> 24

Met Thr Pro Pro Ser Arg Ala Glu Ala Gly Val Arg Arg Ser Arg Val

EP 2 447 719 B1

|       | 1   |     |     | 5   |     |     |     | 10  |     |     |     |     | 15  |     |     |
|-------|-----|-----|-----|-----|-----|-----|-----|-----|-----|-----|-----|-----|-----|-----|-----|

Pro Ser Glu Gly Arg Trp Arg Gly Ala Glu Pro Pro Gly Ile Ser Ala
20 25 30

Ser Thr Gln Pro Ala Ser Ala Gly Arg Ala Ala Arg His Cys Gly Ala
35 40 45

Met Ser Gly Ala Arg Gly Glu Gly Pro Glu Ala Gly Ala Gly Gly Ala
50 55 60

Gly Gly Arg Ala Ala Pro Glu Asn Pro Gly Gly Val Leu Ser Val Glu
65 70 75 80

Leu Pro Gly Leu Leu Ala Gln Leu Ala Arg Ser Phe Ala Leu Leu Leu
85 90 95

Pro Val Tyr Ala Leu Gly Tyr Leu Gly Leu Ser Phe Ser Trp Val Leu
100 105 110

Leu Ala Leu Ala Leu Leu Ala Trp Cys Arg Arg Ser Arg Gly Leu Lys
115 120 125

Ala Leu Arg Leu Cys Arg Ala Leu Ala Leu Leu Glu Asp Glu Glu Arg
130 135 140

Val Val Arg Leu Gly Val Arg Ala Cys Asp Leu Pro Ala Trp Val His
145 150 155 160

Phe Pro Asp Thr Glu Arg Ala Glu Trp Leu Asn Lys Thr Val Lys His
165 170 175

Met Trp Pro Phe Ile Cys Gln Phe Ile Glu Lys Leu Phe Arg Glu Thr
180 185 190

Ile Glu Pro Ala Val Arg Gly Ala Asn Thr His Leu Ser Thr Phe Ser
195 200 205

Phe Thr Lys Val Asp Val Gly Gln Gln Pro Leu Arg Ile Asn Gly Val
210 215 220

Lys Val Tyr Thr Glu Asn Val Asp Lys Arg Gln Ile Ile Leu Asp Leu
225 230 235 240

Gln Ile Ser Phe Val Gly Asn Cys Glu Ile Asp Leu Glu Ile Lys Arg
245 250 255

Tyr Phe Cys Arg Ala Gly Val Lys Ser Ile Gln Ile His Gly Thr Met
260 265 270

236

Arg Val Ile Leu Glu Pro Leu Ile Gly Asp Met Pro Leu Val Gly Ala
        275                 280                 285

Leu Ser Ile Phe Phe Leu Arg Lys Pro Leu Leu Glu Ile Asn Trp Thr
        290                 295                 300

Gly Leu Thr Asn Leu Leu Asp Val Pro Gly Leu Asn Gly Leu Ser Asp
305                 310                 315                 320

Thr Ile Ile Leu Asp Ile Ile Ser Asn Tyr Leu Val Leu Pro Asn Arg
                325                 330                 335

Ile Thr Val Pro Leu Val Ser Glu Val Gln Ile Ala Gln Leu Arg Phe
                340                 345                 350

Pro Val Pro Lys Gly Val Leu Arg Ile His Phe Ile Glu Ala Gln Asp
                355                 360                 365

Leu Gln Gly Lys Asp Thr Tyr Leu Lys Gly Leu Val Lys Gly Lys Ser
        370                 375                 380

Asp Pro Tyr Gly Ile Ile Arg Val Gly Asn Gln Ile Phe Gln Ser Arg
385                 390                 395                 400

Val Ile Lys Glu Asn Leu Ser Pro Lys Trp Asn Glu Val Tyr Glu Ala
                405                 410                 415

Leu Val Tyr Glu His Pro Gly Gln Glu Leu Glu Ile Glu Leu Phe Asp
        420                 425                 430

Glu Asp Pro Asp Lys Asp Asp Phe Leu Gly Ser Leu Met Ile Asp Leu
        435                 440                 445

Ile Glu Val Glu Lys Glu Arg Leu Leu Asp Glu Trp Phe Thr Leu Asp
        450                 455                 460

Glu Val Pro Lys Gly Lys Leu His Leu Arg Leu Glu Trp Leu Thr Leu
465                 470                 475                 480

Met Pro Asn Ala Ser Asn Leu Asp Lys Val Leu Thr Asp Ile Lys Ala
                485                 490                 495

Asp Lys Asp Gln Ala Asn Asp Gly Leu Ser Ser Ala Leu Leu Ile Leu
        500                 505                 510

Tyr Leu Asp Ser Ala Arg Asn Leu Pro Ser Gly Lys Lys Ile Ser Ser
        515                 520                 525

237

```
Asn Pro Asn Pro Val Val Gln Met Ser Val Gly His Lys Ala Gln Glu
    530             535             540

Ser Lys Ile Arg Tyr Lys Thr Asn Glu Pro Val Trp Glu Glu Asn Phe
545             550             555             560

Thr Phe Phe Ile His Asn Pro Lys Arg Gln Asp Leu Glu Val Glu Val
            565             570             575

Arg Asp Glu Gln His Gln Cys Ser Leu Gly Asn Leu Lys Val Pro Leu
        580             585             590

Ser Gln Leu Leu Thr Ser Glu Asp Met Thr Val Ser Gln Arg Phe Gln
    595             600             605

Leu Ser Asn Ser Gly Pro Asn Ser Thr Ile Lys Met Lys Ile Ala Leu
    610             615             620

Arg Val Leu His Leu Glu Lys Arg Glu Arg Pro Pro Asp His Gln His
625             630             635             640

Ser Ala Gln Val Lys Arg Pro Ser Val Ser Lys Glu Gly Arg Lys Thr
            645             650             655

Ser Ile Lys Ser His Met Ser Gly Ser Pro Gly Pro Gly Gly Ser Asn
            660             665             670

Thr Ala Pro Ser Thr Pro Val Ile Gly Gly Ser Asp Lys Pro Gly Met
        675             680             685

Glu Glu Lys Ala Gln Pro Pro Glu Ala Gly Pro Gln Gly Leu His Asp
    690             695             700

Leu Gly Arg Ser Ser Ser Ser Leu Leu Ala Ser Pro Gly His Ile Ser
705             710             715             720

Val Lys Glu Pro Thr Pro Ser Ile Ala Ser Asp Ile Ser Leu Pro Ile
            725             730             735

Ala Thr Gln Glu Leu Arg Gln Arg Leu Arg Gln Leu Glu Asn Gly Thr
        740             745             750

Thr Leu Gly Gln Ser Pro Leu Gly Gln Ile Gln Leu Thr Ile Arg His
        755             760             765

Ser Ser Gln Arg Asn Lys Leu Ile Val Val Val His Ala Cys Arg Asn
    770             775             780
```

```
Leu Ile Ala Phe Ser Glu Asp Gly Ser Asp Pro Tyr Val Arg Met Tyr
785             790             795                 800

Leu Leu Pro Asp Lys Arg Arg Ser Gly Arg Arg Lys Thr His Val Ser
            805             810                 815

Lys Lys Thr Leu Asn Pro Val Phe Asp Gln Ser Phe Asp Phe Ser Val
            820             825             830

Ser Leu Pro Glu Val Gln Arg Arg Thr Leu Asp Val Ala Val Lys Asn
        835             840             845

Ser Gly Gly Phe Leu Ser Lys Asp Lys Gly Leu Leu Gly Lys Val Leu
    850             855             860

Val Ala Leu Ala Ser Glu Glu Leu Ala Lys Gly Trp Thr Gln Trp Tyr
865             870             875                 880

Asp Leu Thr Glu Asp Gly Thr Arg Pro Gln Ala Met Thr
            885             890
```

<210> 25
<211> 1085
<212> PRT
<213> Homo Sapiens

<220>
<221> MISC_FEATURE
<222> (1)..(1085)
<223> OGTA124 = Swiss Prot Accession No. Q9UP95, Solute carrier family 12 member 4

<400> 25

239

Met Pro His Phe Thr Val Val Pro Val Asp Gly Pro Arg Arg Gly Asp
1                   5                   10                  15

Tyr Asp Asn Leu Glu Gly Leu Ser Trp Val Asp Tyr Gly Glu Arg Ala
                20                  25                  30

Glu Leu Asp Asp Ser Asp Gly His Gly Asn His Arg Glu Ser Ser Pro
                35                  40                  45

Phe Leu Ser Pro Leu Glu Ala Ser Arg Gly Ile Asp Tyr Tyr Asp Arg
        50                  55                  60

Asn Leu Ala Leu Phe Glu Glu Glu Leu Asp Ile Arg Pro Lys Val Ser
65                  70                  75                  80

Ser Leu Leu Gly Lys Leu Val Ser Tyr Thr Asn Leu Thr Gln Gly Ala
                85                  90                  95

```
Lys Glu His Glu Glu Ala Glu Ser Gly Glu Gly Thr Arg Arg Arg Ala
            100                 105                 110

Ala Glu Ala Pro Ser Met Gly Thr Leu Met Gly Val Tyr Leu Pro Cys
            115                 120                 125

Leu Gln Asn Ile Phe Gly Val Ile Leu Phe Leu Arg Leu Thr Trp Met
            130                 135                 140

Val Gly Thr Ala Gly Val Leu Gln Ala Leu Leu Ile Val Leu Ile Cys
145                 150                 155                 160

Cys Cys Cys Thr Leu Leu Thr Ala Ile Ser Met Ser Ala Ile Ala Thr
                165                 170                 175

Asn Gly Val Val Pro Ala Gly Gly Ser Tyr Phe Met Ile Ser Arg Ser
                180                 185                 190

Leu Gly Pro Glu Phe Gly Gly Ala Val Gly Leu Cys Phe Tyr Leu Gly
            195                 200                 205

Thr Thr Phe Ala Ala Ala Met Tyr Ile Leu Gly Ala Ile Glu Ile Leu
    210                 215                 220

Leu Thr Tyr Ile Ala Pro Pro Ala Ala Ile Phe Tyr Pro Ser Gly Ala
225                 230                 235                 240

His Asp Thr Ser Asn Ala Thr Leu Asn Asn Met Arg Val Tyr Gly Thr
                245                 250                 255

Ile Phe Leu Thr Phe Met Thr Leu Val Val Phe Val Gly Val Lys Tyr
                260                 265                 270

Val Asn Lys Phe Ala Ser Leu Phe Leu Ala Cys Val Ile Ile Ser Ile
            275                 280                 285

Leu Ser Ile Tyr Ala Gly Gly Ile Lys Ser Ile Phe Asp Pro Pro Val
            290                 295                 300

Phe Pro Val Cys Met Leu Gly Asn Arg Thr Leu Ser Arg Asp Gln Phe
305                 310                 315                 320

Asp Ile Cys Ala Lys Thr Ala Val Val Asp Asn Glu Thr Val Ala Thr
                325                 330                 335

Gln Leu Trp Ser Phe Phe Cys His Ser Pro Asn Leu Thr Thr Asp Ser
            340                 345                 350
```

Cys Asp Pro Tyr Phe Met Leu Asn Asn Val Thr Glu Ile Pro Gly Ile
     355              360            365

Pro Gly Ala Ala Ala Gly Val Leu Gln Glu Asn Leu Trp Ser Ala Tyr
     370              375            380

Leu Glu Lys Gly Asp Ile Val Glu Lys His Gly Leu Pro Ser Ala Asp
385              390           395            400

Ala Pro Ser Leu Lys Glu Ser Leu Pro Leu Tyr Val Val Ala Asp Ile
          405          410          415

Ala Thr Ser Phe Thr Val Leu Val Gly Ile Phe Phe Pro Ser Val Thr
          420          425          430

Gly Ile Met Ala Gly Ser Asn Arg Ser Gly Asp Leu Arg Asp Ala Gln
        435          440          445

Lys Ser Ile Pro Val Gly Thr Ile Leu Ala Ile Ile Thr Thr Ser Leu
     450          455          460

Val Tyr Phe Ser Ser Val Val Leu Phe Gly Ala Cys Ile Glu Gly Val
465          470          475            480

Val Leu Arg Asp Lys Tyr Gly Asp Gly Val Ser Arg Asn Leu Val Val
          485          490          495

Gly Thr Leu Ala Trp Pro Ser Pro Trp Val Ile Val Ile Gly Ser Phe
        500          505          510

Phe Ser Thr Cys Gly Ala Gly Leu Gln Ser Leu Thr Gly Ala Pro Arg
        515          520          525

Leu Leu Gln Ala Ile Ala Lys Asp Asn Ile Ile Pro Phe Leu Arg Val
     530          535          540

Phe Gly His Gly Lys Val Asn Gly Glu Pro Thr Trp Ala Leu Leu Leu
545          550          555          560

Thr Ala Leu Ile Ala Glu Leu Gly Ile Leu Ile Ala Ser Leu Asp Met
          565          570          575

Val Ala Pro Ile Leu Ser Met Phe Phe Leu Met Cys Tyr Leu Phe Val
        580          585          590

Asn Leu Ala Cys Ala Val Gln Thr Leu Leu Arg Thr Pro Asn Trp Arg
     595          600          605

Pro Arg Phe Lys Tyr Tyr His Trp Ala Leu Ser Phe Leu Gly Met Ser

242

                          610                         615                              620

Leu Cys Leu Ala Leu Met Phe Val Ser Ser Trp Tyr Tyr Ala Leu Val
625                 630                 635                 640

Ala Met Leu Ile Ala Gly Met Ile Tyr Lys Tyr Ile Glu Tyr Gln Gly
                645                 650                 655

Ala Glu Lys Glu Trp Gly Asp Gly Ile Arg Gly Leu Ser Leu Ser Ala
            660                 665                 670

Ala Arg Tyr Ala Leu Leu Arg Leu Glu Glu Gly Pro Pro His Thr Lys
        675                 680                 685

Asn Trp Arg Pro Gln Leu Leu Val Leu Leu Lys Leu Asp Glu Asp Leu
    690                 695                 700

His Val Lys Tyr Pro Arg Leu Leu Thr Phe Ala Ser Gln Leu Lys Ala
705                 710                 715                 720

Gly Lys Gly Leu Thr Ile Val Gly Ser Val Ile Gln Gly Ser Phe Leu
                725                 730                 735

Glu Ser Tyr Gly Glu Ala Gln Ala Ala Glu Gln Thr Ile Lys Asn Met
            740                 745                 750

Met Glu Ile Glu Lys Val Lys Gly Phe Cys Gln Val Val Val Ala Ser
        755                 760                 765

Lys Val Arg Glu Gly Leu Ala His Leu Ile Gln Ser Cys Gly Leu Gly
    770                 775                 780

Gly Met Arg His Asn Ser Val Val Leu Gly Trp Pro Tyr Gly Trp Arg
785                 790                 795                 800

Gln Ser Glu Asp Pro Arg Ala Trp Lys Thr Phe Ile Asp Thr Val Arg
            805                 810                 815

Cys Thr Thr Ala Ala His Leu Ala Leu Leu Val Pro Lys Asn Ile Ala
            820                 825                 830

Phe Tyr Pro Ser Asn His Glu Arg Tyr Leu Glu Gly His Ile Asp Val
        835                 840                 845

Trp Trp Ile Val His Asp Gly Gly Met Leu Met Leu Leu Pro Phe Leu
    850                 855                 860

Leu Arg Gln His Lys Val Trp Arg Lys Cys Arg Met Arg Ile Phe Thr
865                 870                 875                 880

```
Val Ala Gln Met Asp Asp Asn Ser Ile Gln Met Lys Lys Asp Leu Ala
              885                 890                 895

Val Phe Leu Tyr His Leu Arg Leu Glu Ala Glu Val Glu Val Val Glu
              900                 905                 910

Met His Asn Ser Asp Ile Ser Ala Tyr Thr Tyr Glu Arg Thr Leu Met
              915                 920                 925

Met Glu Gln Arg Ser Gln Met Leu Arg Gln Met Arg Leu Thr Lys Thr
    930                 935                 940

Glu Arg Glu Arg Glu Ala Gln Leu Val Lys Asp Arg His Ser Ala Leu
    945                 950                 955                 960

Arg Leu Glu Ser Leu Tyr Ser Asp Glu Glu Asp Glu Ser Ala Val Gly
              965                 970                 975

Ala Asp Lys Ile Gln Met Thr Trp Thr Arg Asp Lys Tyr Met Thr Glu
              980                 985                 990

Thr Trp Asp Pro Ser His Ala Pro Asp Asn Phe Arg Glu Leu Val His
              995                 1000                1005

Ile Lys Pro Asp Gln Ser Asn Val Arg Arg Met His Thr Ala Val
    1010                1015                1020

Lys Leu Asn Glu Val Ile Val Thr Arg Ser His Asp Ala Arg Leu
    1025                1030                1035

Val Leu Leu Asn Met Pro Gly Pro Pro Arg Asn Ser Glu Gly Asp
    1040                1045                1050

Glu Asn Tyr Met Glu Phe Leu Glu Val Leu Thr Glu Gly Leu Glu
    1055                1060                1065

Arg Val Leu Leu Val Arg Gly Gly Gly Arg Glu Val Ile Thr Ile
    1070                1075                1080

Tyr Ser
    1085
```

<210> 26
<211> 836
<212> PRT
<213> Homo Sapiens

<220>

<221> MISC_FEATURE
<222> (1)..(836)
<223> OGTA126 = Swiss Prot Accession No. Q9H5V8, CUB domain-containing protein 1

<400> 26

```
Met Ala Gly Leu Asn Cys Gly Val Ser Ile Ala Leu Leu Gly Val Leu
1               5               10              15

Leu Leu Gly Ala Ala Arg Leu Pro Arg Gly Ala Glu Ala Phe Glu Ile
        20              25              30

Ala Leu Pro Arg Glu Ser Asn Ile Thr Val Leu Ile Lys Leu Gly Thr
        35              40              45

Pro Thr Leu Leu Ala Lys Pro Cys Tyr Ile Val Ile Ser Lys Arg His
    50              55              60

Ile Thr Met Leu Ser Ile Lys Ser Gly Glu Arg Ile Val Phe Thr Phe
65              70              75              80

Ser Cys Gln Ser Pro Glu Asn His Phe Val Ile Glu Ile Gln Lys Asn
            85              90              95

Ile Asp Cys Met Ser Gly Pro Cys Pro Phe Gly Glu Val Gln Leu Gln
            100             105             110

Pro Ser Thr Ser Leu Leu Pro Thr Leu Asn Arg Thr Phe Ile Trp Asp
        115             120             125

Val Lys Ala His Lys Ser Ile Gly Leu Glu Leu Gln Phe Ser Ile Pro
    130             135             140

Arg Leu Arg Gln Ile Gly Pro Gly Glu Ser Cys Pro Asp Gly Val Thr
145             150             155             160

His Ser Ile Ser Gly Arg Ile Asp Ala Thr Val Val Arg Ile Gly Thr
            165             170             175

Phe Cys Ser Asn Gly Thr Val Ser Arg Ile Lys Met Gln Glu Gly Val
            180             185             190

Lys Met Ala Leu His Leu Pro Trp Phe His Pro Arg Asn Val Ser Gly
            195             200             205

Phe Ser Ile Ala Asn Arg Ser Ser Ile Lys Arg Leu Cys Ile Ile Glu
    210             215             220

Ser Val Phe Glu Gly Glu Gly Ser Ala Thr Leu Met Ser Ala Asn Tyr
225             230             235             240
```

246

Pro Glu Gly Phe Pro Glu Asp Glu Leu Met Thr Trp Gln Phe Val Val
                    245                 250             255

Pro Ala His Leu Arg Ala Ser Val Ser Phe Leu Asn Phe Asn Leu Ser
                    260                 265             270

Asn Cys Glu Arg Lys Glu Glu Arg Val Glu Tyr Tyr Ile Pro Gly Ser
            275                 280             285

Thr Thr Asn Pro Glu Val Phe Lys Leu Glu Asp Lys Gln Pro Gly Asn
        290                 295             300

Met Ala Gly Asn Phe Asn Leu Ser Leu Gln Gly Cys Asp Gln Asp Ala
305                 310                 315                 320

Gln Ser Pro Gly Ile Leu Arg Leu Gln Phe Gln Val Leu Val Gln His
                325                 330             335

Pro Gln Asn Glu Ser Asn Lys Ile Tyr Val Val Asp Leu Ser Asn Glu
            340                 345             350

Arg Ala Met Ser Leu Thr Ile Glu Pro Arg Pro Val Lys Gln Ser Arg
            355                 360             365

Lys Phe Val Pro Gly Cys Phe Val Cys Leu Glu Ser Arg Thr Cys Ser
        370                 375             380

Ser Asn Leu Thr Leu Thr Ser Gly Ser Lys His Lys Ile Ser Phe Leu
385                 390                 395                 400

Cys Asp Asp Leu Thr Arg Leu Trp Met Asn Val Glu Lys Thr Ile Ser
                405                 410             415

Cys Thr Asp His Arg Tyr Cys Gln Arg Lys Ser Tyr Ser Leu Gln Val
            420                 425             430

Pro Ser Asp Ile Leu His Leu Pro Val Glu Leu His Asp Phe Ser Trp
            435                 440             445

Lys Leu Leu Val Pro Lys Asp Arg Leu Ser Leu Val Leu Val Pro Ala
        450                 455                 460

Gln Lys Leu Gln Gln His Thr His Glu Lys Pro Cys Asn Thr Ser Phe
465                 470                 475                 480

Ser Tyr Leu Val Ala Ser Ala Ile Pro Ser Gln Asp Leu Tyr Phe Gly
                485                 490             495

```
Ser Phe Cys Pro Gly Gly Ser Ile Lys Gln Ile Gln Val Lys Gln Asn
            500             505             510

Ile Ser Val Thr Leu Arg Thr Phe Ala Pro Ser Phe Arg Gln Glu Ala
            515             520             525

Ser Arg Gln Gly Leu Thr Val Ser Phe Ile Pro Tyr Phe Lys Glu Glu
            530             535             540

Gly Val Phe Thr Val Thr Pro Asp Thr Lys Ser Lys Val Tyr Leu Arg
545             550             555             560

Thr Pro Asn Trp Asp Arg Gly Leu Pro Ser Leu Thr Ser Val Ser Trp
            565             570             575

Asn Ile Ser Val Pro Arg Asp Gln Val Ala Cys Leu Thr Phe Phe Lys
            580             585             590

Glu Arg Ser Gly Val Val Cys Gln Thr Gly Arg Ala Phe Met Ile Ile
            595             600             605

Gln Glu Gln Arg Thr Arg Ala Glu Glu Ile Phe Ser Leu Asp Glu Asp
            610             615             620

Val Leu Pro Lys Pro Ser Phe His His His Ser Phe Trp Val Asn Ile
625             630             635             640

Ser Asn Cys Ser Pro Thr Ser Gly Lys Gln Leu Asp Leu Leu Phe Ser
            645             650             655

Val Thr Leu Thr Pro Arg Thr Val Asp Leu Thr Val Ile Leu Ile Ala
            660             665             670

Ala Val Gly Gly Gly Val Leu Leu Leu Ser Ala Leu Gly Leu Ile Ile
            675             680             685

Cys Cys Val Lys Lys Lys Lys Lys Thr Asn Lys Gly Pro Ala Val
            690             695             700

Gly Ile Tyr Asn Gly Asn Ile Asn Thr Glu Met Pro Arg Gln Pro Lys
705             710             715             720

Lys Phe Gln Lys Gly Arg Lys Asp Asn Asp Ser His Val Tyr Ala Val
            725             730             735

Ile Glu Asp Thr Met Val Tyr Gly His Leu Leu Gln Asp Ser Ser Gly
            740             745             750
```

```
Ser Phe Leu Gln Pro Glu Val Asp Thr Tyr Arg Pro Phe Gln Gly Thr
        755             760             765

Met Gly Val Cys Pro Pro Ser Pro Pro Thr Ile Cys Ser Arg Ala Pro
        770             775             780

Thr Ala Lys Leu Ala Thr Glu Glu Pro Pro Pro Arg Ser Pro Pro Glu
785             790             795             800

Ser Glu Ser Glu Pro Tyr Thr Phe Ser His Pro Asn Asn Gly Asp Val
            805             810             815

Ser Ser Lys Asp Thr Asp Ile Pro Leu Leu Ser Thr Gln Glu Pro Met
        820             825             830

Glu Pro Ala Glu
        835
```

<210> 27
<211> 1038
<212> PRT
<213> Homo Sapiens

<220>
<221> MISC_FEATURE
<222> (1)..(1038)
<223> OGTA156 = Swiss Prot Accession No. P13612, Integrin alpha-4

<400> 27

```
Met Phe Pro Thr Glu Ser Ala Trp Leu Gly Lys Arg Gly Ala Asn Pro
1               5               10              15

Gly Pro Glu Ala Ala Val Arg Glu Thr Val Met Leu Leu Leu Cys Leu
        20              25              30

Gly Val Pro Thr Gly Arg Pro Tyr Asn Val Asp Thr Glu Ser Ala Leu
        35              40              45

Leu Tyr Gln Gly Pro His Asn Thr Leu Phe Gly Tyr Ser Val Val Leu
    50              55              60

His Ser His Gly Ala Asn Arg Trp Leu Leu Val Gly Ala Pro Thr Ala
65              70              75              80

Asn Trp Leu Ala Asn Ala Ser Val Ile Asn Pro Gly Ala Ile Tyr Arg
                85              90              95

Cys Arg Ile Gly Lys Asn Pro Gly Gln Thr Cys Glu Gln Leu Gln Leu
            100             105             110
```

```
Gly Ser Pro Asn Gly Glu Pro Cys Gly Lys Thr Cys Leu Glu Glu Arg
        115             120             125

Asp Asn Gln Trp Leu Gly Val Thr Leu Ser Arg Gln Pro Gly Glu Asn
        130             135             140

Gly Ser Ile Val Thr Cys Gly His Arg Trp Lys Asn Ile Phe Tyr Ile
145             150             155             160

Lys Asn Glu Asn Lys Leu Pro Thr Gly Gly Cys Tyr Gly Val Pro Pro
            165             170             175

Asp Leu Arg Thr Glu Leu Ser Lys Arg Ile Ala Pro Cys Tyr Gln Asp
            180             185             190

Tyr Val Lys Lys Phe Gly Glu Asn Phe Ala Ser Cys Gln Ala Gly Ile
            195             200             205

Ser Ser Phe Tyr Thr Lys Asp Leu Ile Val Met Gly Ala Pro Gly Ser
        210             215             220

Ser Tyr Trp Thr Gly Ser Leu Phe Val Tyr Asn Ile Thr Thr Asn Lys
225             230             235             240

Tyr Lys Ala Phe Leu Asp Lys Gln Asn Gln Val Lys Phe Gly Ser Tyr
            245             250             255

Leu Gly Tyr Ser Val Gly Ala Gly His Phe Arg Ser Gln His Thr Thr
            260             265             270

Glu Val Val Gly Gly Ala Pro Gln His Glu Gln Ile Gly Lys Ala Tyr
        275             280             285

Ile Phe Ser Ile Asp Glu Lys Glu Leu Asn Ile Leu His Glu Met Lys
        290             295             300

Gly Lys Lys Leu Gly Ser Tyr Phe Gly Ala Ser Val Cys Ala Val Asp
305             310             315             320

Leu Asn Ala Asp Gly Phe Ser Asp Leu Leu Val Gly Ala Pro Met Gln
            325             330             335

Ser Thr Ile Arg Glu Glu Gly Arg Val Phe Val Tyr Ile Asn Ser Gly
            340             345             350

Ser Gly Ala Val Met Asn Ala Met Glu Thr Asn Leu Val Gly Ser Asp
            355             360             365

Lys Tyr Ala Ala Arg Phe Gly Glu Ser Ile Val Asn Leu Gly Asp Ile
```

370     375      380

Asp Asn Asp Gly Phe Glu Asp Val Ala Ile Gly Ala Pro Gln Glu Asp
385     390     395     400

Asp Leu Gln Gly Ala Ile Tyr Ile Tyr Asn Gly Arg Ala Asp Gly Ile
     405     410     415

Ser Ser Thr Phe Ser Gln Arg Ile Glu Gly Leu Gln Ile Ser Lys Ser
     420     425     430

Leu Ser Met Phe Gly Gln Ser Ile Ser Gly Gln Ile Asp Ala Asp Asn
     435     440     445

Asn Gly Tyr Val Asp Val Ala Val Gly Ala Phe Arg Ser Asp Ser Ala
     450     455     460

Val Leu Leu Arg Thr Arg Pro Val Val Ile Val Asp Ala Ser Leu Ser
465     470     475     480

His Pro Glu Ser Val Asn Arg Thr Lys Phe Asp Cys Val Glu Asn Gly
     485     490     495

Trp Pro Ser Val Cys Ile Asp Leu Thr Leu Cys Phe Ser Tyr Lys Gly
     500     505     510

Lys Glu Val Pro Gly Tyr Ile Val Leu Phe Tyr Asn Met Ser Leu Asp
     515     520     525

Val Asn Arg Lys Ala Glu Ser Pro Pro Arg Phe Tyr Phe Ser Ser Asn
     530     535     540

Gly Thr Ser Asp Val Ile Thr Gly Ser Ile Gln Val Ser Ser Arg Glu
545     550     555     560

Ala Asn Cys Arg Thr His Gln Ala Phe Met Arg Lys Asp Val Arg Asp
     565     570     575

Ile Leu Thr Pro Ile Gln Ile Glu Ala Ala Tyr His Leu Gly Pro His
     580     585     590

Val Ile Ser Lys Arg Ser Thr Glu Glu Phe Pro Pro Leu Gln Pro Ile
     595     600     605

Leu Gln Gln Lys Lys Glu Lys Asp Ile Met Lys Lys Thr Ile Asn Phe
     610     615     620

Ala Arg Phe Cys Ala His Glu Asn Cys Ser Ala Asp Leu Gln Val Ser
625     630     635     640

```
Ala Lys Ile Gly Phe Leu Lys Pro His Glu Asn Lys Thr Tyr Leu Ala
            645                 650                 655

Val Gly Ser Met Lys Thr Leu Met Leu Asn Val Ser Leu Phe Asn Ala
            660                 665                 670

Gly Asp Asp Ala Tyr Glu Thr Thr Leu His Val Lys Leu Pro Val Gly
            675                 680                 685

Leu Tyr Phe Ile Lys Ile Leu Glu Leu Glu Glu Lys Gln Ile Asn Cys
        690                 695                 700

Glu Val Thr Asp Asn Ser Gly Val Val Gln Leu Asp Cys Ser Ile Gly
705                 710                 715                 720

Tyr Ile Tyr Val Asp His Leu Ser Arg Ile Asp Ile Ser Phe Leu Leu
                725                 730                 735

Asp Val Ser Ser Leu Ser Arg Ala Glu Glu Asp Leu Ser Ile Thr Val
            740                 745                 750

His Ala Thr Cys Glu Asn Glu Glu Glu Met Asp Asn Leu Lys His Ser
            755                 760                 765

Arg Val Thr Val Ala Ile Pro Leu Lys Tyr Glu Val Lys Leu Thr Val
            770                 775                 780

His Gly Phe Val Asn Pro Thr Ser Phe Val Tyr Gly Ser Asn Asp Glu
785                 790                 795                 800

Asn Glu Pro Glu Thr Cys Met Val Glu Lys Met Asn Leu Thr Phe His
                805                 810                 815

Val Ile Asn Thr Gly Asn Ser Met Ala Pro Asn Val Ser Val Glu Ile
            820                 825                 830

Met Val Pro Asn Ser Phe Ser Pro Gln Thr Asp Lys Leu Phe Asn Ile
            835                 840                 845

Leu Asp Val Gln Thr Thr Thr Gly Glu Cys His Phe Glu Asn Tyr Gln
        850                 855                 860

Arg Val Cys Ala Leu Glu Gln Gln Lys Ser Ala Met Gln Thr Leu Lys
865                 870                 875                 880

Gly Ile Val Arg Phe Leu Ser Lys Thr Asp Lys Arg Leu Leu Tyr Cys
                885                 890                 895
```

253

```
Ile Lys Ala Asp Pro His Cys Leu Asn Phe Leu Cys Asn Phe Gly Lys
            900             905             910

Met Glu Ser Gly Lys Glu Ala Ser Val His Ile Gln Leu Glu Gly Arg
            915             920             925

Pro Ser Ile Leu Glu Met Asp Glu Thr Ser Ala Leu Lys Phe Glu Ile
            930             935             940

Arg Ala Thr Gly Phe Pro Glu Pro Asn Pro Arg Val Ile Glu Leu Asn
945             950             955             960

Lys Asp Glu Asn Val Ala His Val Leu Leu Glu Gly Leu His His Gln
            965             970             975

Arg Pro Lys Arg Tyr Phe Thr Ile Val Ile Ile Ser Ser Ser Leu Leu
            980             985             990

Leu Gly Leu Ile Val Leu Leu Leu  Ile Ser Tyr Val Met  Trp Lys Ala
            995             1000            1005

Gly Phe  Phe Lys Arg Gln Tyr  Lys Ser Ile Leu Gln  Glu Glu Asn
    1010            1015            1020

Arg Arg  Asp Ser Trp Ser Tyr  Ile Asn Ser Lys Ser  Asn Asp Asp
    1025            1030            1035
```

<210> 28
<211> 314
<212> PRT
<213> Homo Sapiens

<220>
<221> MISC_FEATURE
<222> (1)..(314)
<223> OGTA159 = Swiss Prot Accession No. Q96HY6, Uncharacterized protein C20orf116

<400> 28

```
Met Val Ala Pro Val Trp Tyr Leu Val Ala Ala Ala Leu Leu Val Gly
1               5               10              15

Phe Ile Leu Phe Leu Thr Arg Ser Arg Gly Arg Ala Ala Ser Ala Gly
        20              25              30

Gln Glu Pro Leu His Asn Glu Glu Leu Ala Gly Ala Gly Arg Val Ala
        35              40              45

Gln Pro Gly Pro Leu Glu Pro Glu Glu Pro Arg Ala Gly Gly Arg Pro
        50              55              60
```

```
Arg Arg Arg Arg Asp Leu Gly Ser Arg Leu Gln Ala Gln Arg Arg Ala
65              70              75              80

Gln Arg Val Ala Trp Ala Glu Ala Asp Glu Asn Glu Glu Glu Ala Val
                85              90              95

Ile Leu Ala Gln Glu Glu Glu Gly Val Glu Lys Pro Ala Glu Thr His
            100             105             110

Leu Ser Gly Lys Ile Gly Ala Lys Lys Leu Arg Lys Leu Glu Glu Lys
            115             120             125

Gln Ala Arg Lys Ala Gln Arg Glu Ala Glu Glu Ala Glu Arg Glu Glu
            130             135             140

Arg Lys Arg Leu Glu Ser Gln Arg Glu Ala Glu Trp Lys Lys Glu Glu
145             150             155             160

Glu Arg Leu Arg Leu Glu Glu Glu Gln Lys Glu Glu Glu Glu Arg Lys
                165             170             175

Ala Arg Glu Glu Gln Ala Gln Arg Glu His Glu Glu Tyr Leu Lys Leu
            180             185             190

Lys Glu Ala Phe Val Val Glu Glu Glu Gly Val Gly Glu Thr Met Thr
            195             200             205

Glu Glu Gln Ser Gln Ser Phe Leu Thr Glu Phe Ile Asn Tyr Ile Lys
            210             215             220

Gln Ser Lys Val Val Leu Leu Glu Asp Leu Ala Ser Gln Val Gly Leu
225             230             235             240

Arg Thr Gln Asp Thr Ile Asn Arg Ile Gln Asp Leu Leu Ala Glu Gly
            245             250             255

Thr Ile Thr Gly Val Ile Asp Asp Arg Gly Lys Phe Ile Tyr Ile Thr
            260             265             270

Pro Glu Glu Leu Ala Ala Val Ala Asn Phe Ile Arg Gln Arg Gly Arg
            275             280             285

Val Ser Ile Ala Glu Leu Ala Gln Ala Ser Asn Ser Leu Ile Ala Trp
            290             295             300

Gly Arg Glu Ser Pro Ala Gln Ala Pro Ala
305             310
```

<210> 29
<211> 742
<212> PRT
<213> Homo Sapiens

<220>
<221> MISC_FEATURE
<222> (1)..(742)
<223> OGTA168 = Swiss Prot Accession No. Q8WZA4, Collectin placenta 1

<400> 29

```
Met Lys Asp Asp Phe Ala Glu Glu Glu Glu Val Gln Ser Phe Gly Tyr
1               5                   10              15

Lys Arg Phe Gly Ile Gln Glu Gly Thr Gln Cys Thr Lys Cys Lys Asn
            20                  25              30

Asn Trp Ala Leu Lys Phe Ser Ile Ile Leu Leu Tyr Ile Leu Cys Ala
        35                  40                  45

Leu Leu Thr Ile Thr Val Ala Ile Leu Gly Tyr Lys Val Val Glu Lys
    50                  55                  60

Met Asp Asn Val Thr Gly Gly Met Glu Thr Ser Arg Gln Thr Tyr Asp
65                  70                  75                  80

Asp Lys Leu Thr Ala Val Glu Ser Asp Leu Lys Lys Leu Gly Asp Gln
            85                  90                  95

Thr Gly Lys Lys Ala Ile Ser Thr Asn Ser Glu Leu Ser Thr Phe Arg
            100                 105                 110

Ser Asp Ile Leu Asp Leu Arg Gln Gln Leu Arg Glu Ile Thr Glu Lys
        115                 120                 125

Thr Ser Lys Asn Lys Asp Thr Leu Glu Lys Leu Gln Ala Ser Gly Asp
    130                 135                 140

Ala Leu Val Asp Arg Gln Ser Gln Leu Lys Glu Thr Leu Glu Asn Asn
145                 150                 155                 160

Ser Phe Leu Ile Thr Thr Val Asn Lys Thr Leu Gln Ala Tyr Asn Gly
            165                 170                 175

Tyr Val Thr Asn Leu Gln Gln Asp Thr Ser Val Leu Gln Gly Asn Leu
        180                 185                 190

Gln Asn Gln Met Tyr Ser His Asn Val Val Ile Met Asn Leu Asn Asn
        195                 200                 205
```

```
Leu Asn Leu Thr Gln Val Gln Gln Arg Asn Leu Ile Thr Asn Leu Gln
210             215             220

Arg Ser Val Asp Asp Thr Ser Gln Ala Ile Gln Arg Ile Lys Asn Asp
225             230             235             240

Phe Gln Asn Leu Gln Gln Val Phe Leu Gln Ala Lys Lys Asp Thr Asp
                245             250             255

Trp Leu Lys Glu Lys Val Gln Ser Leu Gln Thr Leu Ala Ala Asn Asn
        260             265             270

Ser Ala Leu Ala Lys Ala Asn Asn Asp Thr Leu Glu Asp Met Asn Ser
        275             280             285

Gln Leu Asn Ser Phe Thr Gly Gln Met Glu Asn Ile Thr Thr Ile Ser
    290             295             300

Gln Ala Asn Glu Gln Asn Leu Lys Asp Leu Gln Asp Leu His Lys Asp
305             310             315             320

Ala Glu Asn Arg Thr Ala Ile Lys Phe Asn Gln Leu Glu Glu Arg Phe
                325             330             335

Gln Leu Phe Glu Thr Asp Ile Val Asn Ile Ile Ser Asn Ile Ser Tyr
        340             345             350

Thr Ala His His Leu Arg Thr Leu Thr Ser Asn Leu Asn Glu Val Arg
        355             360             365

Thr Thr Cys Thr Asp Thr Leu Thr Lys His Thr Asp Asp Leu Thr Ser
    370             375             380

Leu Asn Asn Thr Leu Ala Asn Ile Arg Leu Asp Ser Val Ser Leu Arg
385             390             395             400

Met Gln Gln Asp Leu Met Arg Ser Arg Leu Asp Thr Glu Val Ala Asn
                405             410             415

Leu Ser Val Ile Met Glu Glu Met Lys Leu Val Asp Ser Lys His Gly
        420             425             430

Gln Leu Ile Lys Asn Phe Thr Ile Leu Gln Gly Pro Pro Gly Pro Arg
        435             440             445

Gly Pro Arg Gly Asp Arg Gly Ser Gln Gly Pro Pro Gly Pro Thr Gly
        450             455             460
```

259

```
Asn Lys Gly Gln Lys Gly Glu Lys Gly Glu Pro Gly Pro Pro Gly Pro
465             470             475             480

Ala Gly Glu Arg Gly Pro Ile Gly Pro Ala Gly Pro Pro Gly Glu Arg
            485             490             495

Gly Gly Lys Gly Ser Lys Gly Ser Gln Gly Pro Lys Gly Ser Arg Gly
            500             505             510

Ser Pro Gly Lys Pro Gly Pro Gln Gly Ser Ser Gly Asp Pro Gly Pro
        515             520             525

Pro Gly Pro Pro Gly Lys Glu Gly Leu Pro Gly Pro Gln Gly Pro Pro
    530             535             540

Gly Phe Gln Gly Leu Gln Gly Thr Val Gly Glu Pro Gly Val Pro Gly
545             550             555             560

Pro Arg Gly Leu Pro Gly Leu Pro Gly Val Pro Gly Met Pro Gly Pro
            565             570             575

Lys Gly Pro Pro Gly Pro Pro Gly Pro Ser Gly Ala Val Val Pro Leu
            580             585             590

Ala Leu Gln Asn Glu Pro Thr Pro Ala Pro Glu Asp Asn Gly Cys Pro
        595             600             605

Pro His Trp Lys Asn Phe Thr Asp Lys Cys Tyr Tyr Phe Ser Val Glu
    610             615             620

Lys Glu Ile Phe Glu Asp Ala Lys Leu Phe Cys Glu Asp Lys Ser Ser
625             630             635             640

His Leu Val Phe Ile Asn Thr Arg Glu Glu Gln Gln Trp Ile Lys Lys
            645             650             655

Gln Met Val Gly Arg Glu Ser His Trp Ile Gly Leu Thr Asp Ser Glu
            660             665             670

Arg Glu Asn Glu Trp Lys Trp Leu Asp Gly Thr Ser Pro Asp Tyr Lys
        675             680             685

Asn Trp Lys Ala Gly Gln Pro Asp Asn Trp Gly His Gly His Gly Pro
    690             695             700

Gly Glu Asp Cys Ala Gly Leu Ile Tyr Ala Gly Gln Trp Asn Asp Phe
705             710             715             720

Gln Cys Glu Asp Val Asn Asn Phe Ile Cys Glu Lys Asp Arg Glu Thr
```

                        725                    730                         735

          Val Leu Ser Ser Ala Leu
                          740

<210> 30
<211> 1163
<212> PRT
<213> Homo Sapiens

<220>
<221> MISC_FEATURE
<222> (1)..(1163)
<223> OGTA169 = Swiss Prot Accession No. P20702, Integrin alpha-X

<400> 30

```
Met Thr Arg Thr Arg Ala Ala Leu Leu Leu Phe Thr Ala Leu Ala Thr
1               5               10              15

Ser Leu Gly Phe Asn Leu Asp Thr Glu Glu Leu Thr Ala Phe Arg Val
            20              25              30

Asp Ser Ala Gly Phe Gly Asp Ser Val Val Gln Tyr Ala Asn Ser Trp
            35              40              45

Val Val Val Gly Ala Pro Gln Lys Ile Thr Ala Ala Asn Gln Thr Gly
    50              55              60

Gly Leu Tyr Gln Cys Gly Tyr Ser Thr Gly Ala Cys Glu Pro Ile Gly
65              70              75              80

Leu Gln Val Pro Pro Glu Ala Val Asn Met Ser Leu Gly Leu Ser Leu
            85              90              95

Ala Ser Thr Thr Ser Pro Ser Gln Leu Leu Ala Cys Gly Pro Thr Val
            100             105             110

His His Glu Cys Gly Arg Asn Met Tyr Leu Thr Gly Leu Cys Phe Leu
        115             120             125

Leu Gly Pro Thr Gln Leu Thr Gln Arg Leu Pro Val Ser Arg Gln Glu
    130             135             140

Cys Pro Arg Gln Glu Gln Asp Ile Val Phe Leu Ile Asp Gly Ser Gly
145             150             155             160

Ser Ile Ser Ser Arg Asn Phe Ala Thr Met Met Asn Phe Val Arg Ala
            165             170             175

Val Ile Ser Gln Phe Gln Arg Pro Ser Thr Gln Phe Ser Leu Met Gln
```

262

```
                    180                      185                          190


        Phe Ser Asn Lys Phe Gln Thr His Phe Thr Phe Glu Glu Phe Arg Arg
                195             200             205

        Thr Ser Asn Pro Leu Ser Leu Leu Ala Ser Val His Gln Leu Gln Gly
                210             215             220

        Phe Thr Tyr Thr Ala Thr Ala Ile Gln Asn Val Val His Arg Leu Phe
        225             230             235                         240

        His Ala Ser Tyr Gly Ala Arg Arg Asp Ala Thr Lys Ile Leu Ile Val
                        245             250                     255

        Ile Thr Asp Gly Lys Lys Glu Gly Asp Ser Leu Asp Tyr Lys Asp Val
                260             265             270

        Ile Pro Met Ala Asp Ala Ala Gly Ile Ile Arg Tyr Ala Ile Gly Val
                275             280             285

        Gly Leu Ala Phe Gln Asn Arg Asn Ser Trp Lys Glu Leu Asn Asp Ile
            290             295             300

        Ala Ser Lys Pro Ser Gln Glu His Ile Phe Lys Val Glu Asp Phe Asp
        305             310             315                         320

        Ala Leu Lys Asp Ile Gln Asn Gln Leu Lys Glu Lys Ile Phe Ala Ile
                        325             330                     335

        Glu Gly Thr Glu Thr Thr Ser Ser Ser Ser Phe Glu Leu Glu Met Ala
                340             345             350

        Gln Glu Gly Phe Ser Ala Val Phe Thr Pro Asp Gly Pro Val Leu Gly
                355             360             365

        Ala Val Gly Ser Phe Thr Trp Ser Gly Gly Ala Phe Leu Tyr Pro Pro
                370             375             380

        Asn Met Ser Pro Thr Phe Ile Asn Met Ser Gln Glu Asn Val Asp Met
        385             390             395                         400

        Arg Asp Ser Tyr Leu Gly Tyr Ser Thr Glu Leu Ala Leu Trp Lys Gly
                        405             410                     415

        Val Gln Ser Leu Val Leu Gly Ala Pro Arg Tyr Gln His Thr Gly Lys
                420             425             430

        Ala Val Ile Phe Thr Gln Val Ser Arg Gln Trp Arg Met Lys Ala Glu
                435             440             445
```

Val Thr Gly Thr Gln Ile Gly Ser Tyr Phe Gly Ala Ser Leu Cys Ser
450                     455                 460

Val Asp Val Asp Thr Asp Gly Ser Thr Asp Leu Val Leu Ile Gly Ala
465             470                 475                     480

Pro His Tyr Tyr Glu Gln Thr Arg Gly Gly Gln Val Ser Val Cys Pro
                485                 490                     495

Leu Pro Arg Gly Trp Arg Arg Trp Trp Cys Asp Ala Val Leu Tyr Gly
            500                 505                 510

Glu Gln Gly His Pro Trp Gly Arg Phe Gly Ala Ala Leu Thr Val Leu
        515                 520                 525

Gly Asp Val Asn Gly Asp Lys Leu Thr Asp Val Val Ile Gly Ala Pro
        530                 535                 540

Gly Glu Glu Glu Asn Arg Gly Ala Val Tyr Leu Phe His Gly Val Leu
545                 550                 555                 560

Gly Pro Ser Ile Ser Pro Ser His Ser Gln Arg Ile Ala Gly Ser Gln
            565                 570                 575

Leu Ser Ser Arg Leu Gln Tyr Phe Gly Gln Ala Leu Ser Gly Gly Gln
        580                 585                 590

Asp Leu Thr Gln Asp Gly Leu Val Asp Leu Ala Val Gly Ala Arg Gly
        595                 600                 605

Gln Val Leu Leu Leu Arg Thr Arg Pro Val Leu Trp Val Gly Val Ser
    610                 615                 620

Met Gln Phe Ile Pro Ala Glu Ile Pro Arg Ser Ala Phe Glu Cys Arg
625                 630                 635                 640

Glu Gln Val Val Ser Glu Gln Thr Leu Val Gln Ser Asn Ile Cys Leu
                645                 650                 655

Tyr Ile Asp Lys Arg Ser Lys Asn Leu Leu Gly Ser Arg Asp Leu Gln
            660                 665                 670

Ser Ser Val Thr Leu Asp Leu Ala Leu Asp Pro Gly Arg Leu Ser Pro
        675                 680                 685

Arg Ala Thr Phe Gln Glu Thr Lys Asn Arg Ser Leu Ser Arg Val Arg
    690                 695                 700

264

Val Leu Gly Leu Lys Ala His Cys Glu Asn Phe Asn Leu Leu Leu Pro
705                 710             715                 720

Ser Cys Val Glu Asp Ser Val Thr Pro Ile Thr Leu Arg Leu Asn Phe
                725             730                 735

Thr Leu Val Gly Lys Pro Leu Leu Ala Phe Arg Asn Leu Arg Pro Met
            740             745             750

Leu Ala Ala Asp Ala Gln Arg Tyr Phe Thr Ala Ser Leu Pro Phe Glu
        755             760             765

Lys Asn Cys Gly Ala Asp His Ile Cys Gln Asp Asn Leu Gly Ile Ser
    770             775             780

Phe Ser Phe Pro Gly Leu Lys Ser Leu Leu Val Gly Ser Asn Leu Glu
785             790             795             800

Leu Asn Ala Glu Val Met Val Trp Asn Asp Gly Glu Asp Ser Tyr Gly
            805             810             815

Thr Thr Ile Thr Phe Ser His Pro Ala Gly Leu Ser Tyr Arg Tyr Val
        820             825             830

Ala Glu Gly Gln Lys Gln Gly Gln Leu Arg Ser Leu His Leu Thr Cys
    835             840             845

Asp Ser Ala Pro Val Gly Ser Gln Gly Thr Trp Ser Thr Ser Cys Arg
    850             855             860

Ile Asn His Leu Ile Phe Arg Gly Gly Ala Gln Ile Thr Phe Leu Ala
865             870             875             880

Thr Phe Asp Val Ser Pro Lys Ala Val Leu Gly Asp Arg Leu Leu Leu
            885             890             895

Thr Ala Asn Val Ser Ser Glu Asn Asn Thr Pro Arg Thr Ser Lys Thr
            900             905             910

Thr Phe Gln Leu Glu Leu Pro Val Lys Tyr Ala Val Tyr Thr Val Val
        915             920             925

Ser Ser His Glu Gln Phe Thr Lys Tyr Leu Asn Phe Ser Glu Ser Glu
    930             935             940

Glu Lys Glu Ser His Val Ala Met His Arg Tyr Gln Val Asn Asn Leu
945             950             955             960

265

```
Gly Gln Arg Asp Leu Pro Val Ser Ile Asn Phe Trp Val Pro Val Glu
            965                 970                 975

Leu Asn Gln Glu Ala Val Trp Met Asp Val Glu Val Ser His Pro Gln
            980                 985                 990

Asn Pro Ser Leu Arg Cys Ser Ser Glu Lys Ile Ala Pro Pro Ala Ser
            995                 1000                1005

Asp Phe Leu Ala His Ile Gln Lys Asn Pro Val Leu Asp Cys Ser
    1010                1015                1020

Ile Ala Gly Cys Leu Arg Phe Arg Cys Asp Val Pro Ser Phe Ser
    1025                1030                1035

Val Gln Glu Glu Leu Asp Phe Thr Leu Lys Gly Asn Leu Ser Phe
    1040                1045                1050

Gly Trp Val Arg Gln Ile Leu Gln Lys Lys Val Ser Val Val Ser
    1055                1060                1065

Val Ala Glu Ile Thr Phe Asp Thr Ser Val Tyr Ser Gln Leu Pro
    1070                1075                1080

Gly Gln Glu Ala Phe Met Arg Ala Gln Thr Thr Thr Val Leu Glu
    1085                1090                1095

Lys Tyr Lys Val His Asn Pro Thr Pro Leu Ile Val Gly Ser Ser
    1100                1105                1110

Ile Gly Gly Leu Leu Leu Leu Ala Leu Ile Thr Ala Val Leu Tyr
    1115                1120                1125

Lys Val Gly Phe Phe Lys Arg Gln Tyr Lys Glu Met Met Glu Glu
    1130                1135                1140

Ala Asn Gly Gln Ile Ala Pro Glu Asn Gly Thr Gln Thr Pro Ser
    1145                1150                1155

Pro Pro Ser Glu Lys
    1160
```

<210> 31
<211> 495
<212> PRT
<213> Homo Sapiens

<220>

<221> MISC_FEATURE
<222> (1)..(495)
<223> OGTA174 = Swiss Prot Accession No. P06127, T-cell surface glycoprotein CD5

<400> 31

```
Met Pro Met Gly Ser Leu Gln Pro Leu Ala Thr Leu Tyr Leu Leu Gly
1               5               10              15

Met Leu Val Ala Ser Cys Leu Gly Arg Leu Ser Trp Tyr Asp Pro Asp
        20              25              30

Phe Gln Ala Arg Leu Thr Arg Ser Asn Ser Lys Cys Gln Gly Gln Leu
        35              40              45

Glu Val Tyr Leu Lys Asp Gly Trp His Met Val Cys Ser Gln Ser Trp
    50              55              60

Gly Arg Ser Ser Lys Gln Trp Glu Asp Pro Ser Gln Ala Ser Lys Val
65              70              75              80

Cys Gln Arg Leu Asn Cys Gly Val Pro Leu Ser Leu Gly Pro Phe Leu
            85              90              95

Val Thr Tyr Thr Pro Gln Ser Ser Ile Ile Cys Tyr Gly Gln Leu Gly
            100             105             110

Ser Phe Ser Asn Cys Ser His Ser Arg Asn Asp Met Cys His Ser Leu
        115             120             125

Gly Leu Thr Cys Leu Glu Pro Gln Lys Thr Thr Pro Pro Thr Thr Arg
    130             135             140

Pro Pro Pro Thr Thr Thr Pro Glu Pro Thr Ala Pro Pro Arg Leu Gln
145             150             155             160

Leu Val Ala Gln Ser Gly Gly Gln His Cys Ala Gly Val Val Glu Phe
            165             170             175

Tyr Ser Gly Ser Leu Gly Gly Thr Ile Ser Tyr Glu Ala Gln Asp Lys
        180             185             190

Thr Gln Asp Leu Glu Asn Phe Leu Cys Asn Asn Leu Gln Cys Gly Ser
        195             200             205

Phe Leu Lys His Leu Pro Glu Thr Glu Ala Gly Arg Ala Gln Asp Pro
    210             215             220

Gly Glu Pro Arg Glu His Gln Pro Leu Pro Ile Gln Trp Lys Ile Gln
225             230             235             240
```

```
Asn Ser Ser Cys Thr Ser Leu Glu His Cys Phe Arg Lys Ile Lys Pro
              245          250              255

Gln Lys Ser Gly Arg Val Leu Ala Leu Leu Cys Ser Gly Phe Gln Pro
              260          265              270

Lys Val Gln Ser Arg Leu Val Gly Gly Ser Ser Ile Cys Glu Gly Thr
              275          280              285

Val Glu Val Arg Gln Gly Ala Gln Trp Ala Ala Leu Cys Asp Ser Ser
    290              295              300

Ser Ala Arg Ser Ser Leu Arg Trp Glu Glu Val Cys Arg Glu Gln Gln
305              310              315              320

Cys Gly Ser Val Asn Ser Tyr Arg Val Leu Asp Ala Gly Asp Pro Thr
              325          330              335

Ser Arg Gly Leu Phe Cys Pro His Gln Lys Leu Ser Gln Cys His Glu
              340          345              350

Leu Trp Glu Arg Asn Ser Tyr Cys Lys Lys Val Phe Val Thr Cys Gln
              355          360              365

Asp Pro Asn Pro Ala Gly Leu Ala Ala Gly Thr Val Ala Ser Ile Ile
    370              375              380

Leu Ala Leu Val Leu Leu Val Val Leu Leu Val Val Cys Gly Pro Leu
385              390              395              400

Ala Tyr Lys Lys Leu Val Lys Lys Phe Arg Gln Lys Lys Gln Arg Gln
              405          410              415

Trp Ile Gly Pro Thr Gly Met Asn Gln Asn Met Ser Phe His Arg Asn
              420          425              430

His Thr Ala Thr Val Arg Ser His Ala Glu Asn Pro Thr Ala Ser His
    435              440              445

Val Asp Asn Glu Tyr Ser Gln Pro Pro Arg Asn Ser Arg Leu Ser Ala
    450              455              460

Tyr Pro Ala Leu Glu Gly Val Leu His Arg Ser Ser Met Gln Pro Asp
465              470              475              480

Asn Ser Ser Asp Ser Asp Tyr Asp Leu His Gly Ala Gln Arg Leu
              485          490              495
```

269

<210> 32
<211> 359
<212> PRT
<213> Homo Sapiens

<220>
<221> MISC_FEATURE
<222> (1)..(359)
<223> OGTA176 = Swiss Prot Accession No. P21854, B-cell differentiation antigen CD72 homolog

<400> 32

```
Met Ala Glu Ala Ile Thr Tyr Ala Asp Leu Arg Phe Val Lys Ala Pro
1               5               10                  15


Leu Lys Lys Ser Ile Ser Ser Arg Leu Gly Gln Asp Pro Gly Ala Asp
            20              25                  30


Asp Asp Gly Glu Ile Thr Tyr Glu Asn Val Gln Val Pro Ala Val Leu
            35              40                  45


Gly Val Pro Ser Ser Leu Ala Ser Ser Val Leu Gly Asp Lys Ala Ala
    50              55                  60


Val Lys Ser Glu Gln Pro Thr Ala Ser Trp Arg Ala Val Thr Ser Pro
65              70                  75                  80


Ala Val Gly Arg Ile Leu Pro Cys Arg Thr Thr Cys Leu Arg Tyr Leu
            85                  90                  95


Leu Leu Gly Leu Leu Leu Thr Cys Leu Leu Leu Gly Val Thr Ala Ile
            100                 105                 110


Cys Leu Gly Val Arg Tyr Leu Gln Val Ser Gln Gln Leu Gln Gln Thr
            115                 120                 125


Asn Arg Val Leu Glu Val Thr Asn Ser Ser Leu Arg Gln Gln Leu Arg
    130                 135                 140


Leu Lys Ile Thr Gln Leu Gly Gln Ser Ala Glu Asp Leu Gln Gly Ser
145                 150                 155                 160


Arg Arg Glu Leu Ala Gln Ser Gln Glu Ala Leu Gln Val Glu Gln Arg
            165                 170                 175


Ala His Gln Ala Ala Glu Gly Gln Leu Gln Ala Cys Gln Ala Asp Arg
            180                 185                 190


Gln Lys Thr Lys Glu Thr Leu Gln Ser Glu Glu Gln Gln Arg Arg Ala
            195                 200                 205
```

271

```
Leu Glu Gln Lys Leu Ser Asn Met Glu Asn Arg Leu Lys Pro Phe Phe
    210             215             220

Thr Cys Gly Ser Ala Asp Thr Cys Cys Pro Ser Gly Trp Ile Met His
225             230             235                     240

Gln Lys Ser Cys Phe Tyr Ile Ser Leu Thr Ser Lys Asn Trp Gln Glu
                245             250             255

Ser Gln Lys Gln Cys Glu Thr Leu Ser Ser Lys Leu Ala Thr Phe Ser
            260             265             270

Glu Ile Tyr Pro Gln Ser His Ser Tyr Tyr Phe Leu Asn Ser Leu Leu
            275             280             285

Pro Asn Gly Gly Ser Gly Asn Ser Tyr Trp Thr Gly Leu Ser Ser Asn
    290             295             300

Lys Asp Trp Lys Leu Thr Asp Asp Thr Gln Arg Thr Arg Thr Tyr Ala
305             310             315                     320

Gln Ser Ser Lys Cys Asn Lys Val His Lys Thr Trp Ser Trp Trp Thr
            325             330             335

Leu Glu Ser Glu Ser Cys Arg Ser Ser Leu Pro Tyr Ile Cys Glu Met
            340             345             350

Thr Ala Phe Arg Phe Pro Asp
            355
```

<210> 33
<211> 510
<212> PRT
<213> Homo Sapiens

<220>
<221> MISC_FEATURE
<222> (1)..(510)
<223> OGTA177 = Swiss Prot Accession No. P49961, Ectonucleoside triphosphate diphosphohydrolase 1

<400> 33

```
Met Glu Asp Thr Lys Glu Ser Asn Val Lys Thr Phe Cys Ser Lys Asn
1               5               10                  15

Ile Leu Ala Ile Leu Gly Phe Ser Ser Ile Ile Ala Val Ile Ala Leu
        20                  25                  30

Leu Ala Val Gly Leu Thr Gln Asn Lys Ala Leu Pro Glu Asn Val Lys
        35                  40                  45
```

```
Tyr Gly Ile Val Leu Asp Ala Gly Ser Ser His Thr Ser Leu Tyr Ile
    50                  55                  60

Tyr Lys Trp Pro Ala Glu Lys Glu Asn Asp Thr Gly Val Val His Gln
    65                  70                  75                  80

Val Glu Glu Cys Arg Val Lys Gly Pro Gly Ile Ser Lys Phe Val Gln
                85                  90                  95

Lys Val Asn Glu Ile Gly Ile Tyr Leu Thr Asp Cys Met Glu Arg Ala
                100                 105                 110

Arg Glu Val Ile Pro Arg Ser Gln His Gln Glu Thr Pro Val Tyr Leu
                115                 120                 125

Gly Ala Thr Ala Gly Met Arg Leu Leu Arg Met Glu Ser Glu Glu Leu
                130                 135                 140

Ala Asp Arg Val Leu Asp Val Val Glu Arg Ser Leu Ser Asn Tyr Pro
145                 150                 155                 160

Phe Asp Phe Gln Gly Ala Arg Ile Ile Thr Gly Gln Glu Glu Gly Ala
                165                 170                 175

Tyr Gly Trp Ile Thr Ile Asn Tyr Leu Leu Gly Lys Phe Ser Gln Lys
                180                 185                 190

Thr Arg Trp Phe Ser Ile Val Pro Tyr Glu Thr Asn Asn Gln Glu Thr
    195                 200                 205

Phe Gly Ala Leu Asp Leu Gly Gly Ala Ser Thr Gln Val Thr Phe Val
    210                 215                 220

Pro Gln Asn Gln Thr Ile Glu Ser Pro Asp Asn Ala Leu Gln Phe Arg
225                 230                 235                 240

Leu Tyr Gly Lys Asp Tyr Asn Val Tyr Thr His Ser Phe Leu Cys Tyr
                245                 250                 255

Gly Lys Asp Gln Ala Leu Trp Gln Lys Leu Ala Lys Asp Ile Gln Val
                260                 265                 270

Ala Ser Asn Glu Ile Leu Arg Asp Pro Cys Phe His Pro Gly Tyr Lys
                275                 280                 285

Lys Val Val Asn Val Ser Asp Leu Tyr Lys Thr Pro Cys Thr Lys Arg
                290                 295                 300
```

274

```
Phe Glu Met Thr Leu Pro Phe Gln Gln Phe Glu Ile Gln Gly Ile Gly
305             310             315             320


Asn Tyr Gln Gln Cys His Gln Ser Ile Leu Glu Leu Phe Asn Thr Ser
            325             330             335


Tyr Cys Pro Tyr Ser Gln Cys Ala Phe Asn Gly Ile Phe Leu Pro Pro
            340             345             350


Leu Gln Gly Asp Phe Gly Ala Phe Ser Ala Phe Tyr Phe Val Met Lys
            355             360             365


Phe Leu Asn Leu Thr Ser Glu Lys Val Ser Gln Glu Lys Val Thr Glu
    370             375             380


Met Met Lys Lys Phe Cys Ala Gln Pro Trp Glu Glu Ile Lys Thr Ser
385             390             395             400


Tyr Ala Gly Val Lys Glu Lys Tyr Leu Ser Glu Tyr Cys Phe Ser Gly
                405             410             415


Thr Tyr Ile Leu Ser Leu Leu Leu Gln Gly Tyr His Phe Thr Ala Asp
            420             425             430


Ser Trp Glu His Ile His Phe Ile Gly Lys Ile Gln Gly Ser Asp Ala
            435             440             445


Gly Trp Thr Leu Gly Tyr Met Leu Asn Leu Thr Asn Met Ile Pro Ala
    450             455             460


Glu Gln Pro Leu Ser Thr Pro Leu Ser His Ser Thr Tyr Val Phe Leu
465             470             475             480


Met Val Leu Phe Ser Leu Val Leu Phe Thr Val Ala Ile Ile Gly Leu
            485             490             495


Leu Ile Phe His Lys Pro Ser Tyr Phe Trp Lys Asp Met Val
            500             505             510
```

<210> 34

<211> 976

<212> PRT

<213> Homo Sapiens

<220>

<221> MISC_FEATURE

<222> (1)..(976)

<223> OGTA197 = Swiss Prot Accession No. P29317, Ephrin type-A receptor 2

<400> 34

```
Met Glu Leu Gln Ala Ala Arg Ala Cys Phe Ala Leu Leu Trp Gly Cys
1               5               10                  15

Ala Leu Ala Ala Ala Ala Ala Ala Gln Gly Lys Glu Val Val Leu Leu
            20              25                  30

Asp Phe Ala Ala Ala Gly Gly Glu Leu Gly Trp Leu Thr His Pro Tyr
        35              40                  45

Gly Lys Gly Trp Asp Leu Met Gln Asn Ile Met Asn Asp Met Pro Ile
    50              55                  60

Tyr Met Tyr Ser Val Cys Asn Val Met Ser Gly Asp Gln Asp Asn Trp
65              70                  75                  80

Leu Arg Thr Asn Trp Val Tyr Arg Gly Glu Ala Glu Arg Asn Asn Phe
            85              90                  95

Glu Leu Asn Phe Thr Val Arg Asp Cys Asn Ser Phe Pro Gly Gly Ala
            100             105                 110

Ser Ser Cys Lys Glu Thr Phe Asn Leu Tyr Tyr Ala Glu Ser Asp Leu
        115             120                 125

Asp Tyr Gly Thr Asn Phe Gln Lys Arg Leu Phe Thr Lys Ile Asp Thr
    130             135                 140

Ile Ala Pro Asp Glu Ile Thr Val Ser Ser Asp Phe Glu Ala Arg His
145             150                 155                 160

Val Lys Leu Asn Val Glu Glu Arg Ser Val Gly Pro Leu Thr Arg Lys
            165             170                 175

Gly Phe Tyr Leu Ala Phe Gln Asp Ile Gly Ala Cys Val Ala Leu Leu
            180             185                 190

Ser Val Arg Val Tyr Tyr Lys Lys Cys Pro Glu Leu Leu Gln Gly Leu
        195             200                 205

Ala His Phe Pro Glu Thr Ile Ala Gly Ser Asp Ala Pro Ser Leu Ala
    210             215                 220

Thr Val Ala Gly Thr Cys Val Asp His Ala Val Val Pro Pro Gly Gly
225             230                 235                 240

Glu Glu Pro Arg Met His Cys Ala Val Asp Gly Glu Trp Leu Val Pro
            245             250                 255
```

277

Ile Gly Gln Cys Leu Cys Gln Ala Gly Tyr Glu Lys Val Glu Asp Ala
        260                 265                 270

Cys Gln Ala Cys Ser Pro Gly Phe Phe Lys Phe Glu Ala Ser Glu Ser
        275                 280                 285

Pro Cys Leu Glu Cys Pro Glu His Thr Leu Pro Ser Pro Glu Gly Ala
        290                 295                 300

Thr Ser Cys Glu Cys Glu Glu Gly Phe Phe Arg Ala Pro Gln Asp Pro
305                 310                 315                 320

Ala Ser Met Pro Cys Thr Arg Pro Pro Ser Ala Pro His Tyr Leu Thr
                325                 330                 335

Ala Val Gly Met Gly Ala Lys Val Glu Leu Arg Trp Thr Pro Pro Gln
        340                 345                 350

Asp Ser Gly Gly Arg Glu Asp Ile Val Tyr Ser Val Thr Cys Glu Gln
        355                 360                 365

Cys Trp Pro Glu Ser Gly Glu Cys Gly Pro Cys Glu Ala Ser Val Arg
        370                 375                 380

Tyr Ser Glu Pro Pro His Gly Leu Thr Arg Thr Ser Val Thr Val Ser
385                 390                 395                 400

Asp Leu Glu Pro His Met Asn Tyr Thr Phe Thr Val Glu Ala Arg Asn
                405                 410                 415

Gly Val Ser Gly Leu Val Thr Ser Arg Ser Phe Arg Thr Ala Ser Val
        420                 425                 430

Ser Ile Asn Gln Thr Glu Pro Pro Lys Val Arg Leu Glu Gly Arg Ser
        435                 440                 445

Thr Thr Ser Leu Ser Val Ser Trp Ser Ile Pro Pro Pro Gln Gln Ser
        450                 455                 460

Arg Val Trp Lys Tyr Glu Val Thr Tyr Arg Lys Lys Gly Asp Ser Asn
465                 470                 475                 480

Ser Tyr Asn Val Arg Arg Thr Glu Gly Phe Ser Val Thr Leu Asp Asp
                485                 490                 495

Leu Ala Pro Asp Thr Thr Tyr Leu Val Gln Val Gln Ala Leu Thr Gln
                500                 505                 510

Glu Gly Gln Gly Ala Gly Ser Lys Val His Glu Phe Gln Thr Leu Ser

515                         520                         525

Pro Glu Gly Ser Gly Asn Leu Ala Val Ile Gly Gly Val Ala Val Gly
    530                 535                 540

Val Val Leu Leu Leu Val Leu Ala Gly Val Gly Phe Phe Ile His Arg
545                 550                 555                 560

Arg Arg Lys Asn Gln Arg Ala Arg Gln Ser Pro Glu Asp Val Tyr Phe
            565                 570                 575

Ser Lys Ser Glu Gln Leu Lys Pro Leu Lys Thr Tyr Val Asp Pro His
            580                 585                 590

Thr Tyr Glu Asp Pro Asn Gln Ala Val Leu Lys Phe Thr Thr Glu Ile
        595                 600                 605

His Pro Ser Cys Val Thr Arg Gln Lys Val Ile Gly Ala Gly Glu Phe
    610                 615                 620

Gly Glu Val Tyr Lys Gly Met Leu Lys Thr Ser Ser Gly Lys Lys Glu
625                 630                 635                 640

Val Pro Val Ala Ile Lys Thr Leu Lys Ala Gly Tyr Thr Glu Lys Gln
            645                 650                 655

Arg Val Asp Phe Leu Gly Glu Ala Gly Ile Met Gly Gln Phe Ser His
        660                 665                 670

His Asn Ile Ile Arg Leu Glu Gly Val Ile Ser Lys Tyr Lys Pro Met
        675                 680                 685

Met Ile Ile Thr Glu Tyr Met Glu Asn Gly Ala Leu Asp Lys Phe Leu
    690                 695                 700

Arg Glu Lys Asp Gly Glu Phe Ser Val Leu Gln Leu Val Gly Met Leu
705                 710                 715                 720

Arg Gly Ile Ala Ala Gly Met Lys Tyr Leu Ala Asn Met Asn Tyr Val
            725                 730                 735

His Arg Asp Leu Ala Ala Arg Asn Ile Leu Val Asn Ser Asn Leu Val
        740                 745                 750

Cys Lys Val Ser Asp Phe Gly Leu Ser Arg Val Leu Glu Asp Asp Pro
        755                 760                 765

Glu Ala Thr Tyr Thr Thr Ser Gly Gly Lys Ile Pro Ile Arg Trp Thr
    770                 775                 780

```
Ala Pro Glu Ala Ile Ser Tyr Arg Lys Phe Thr Ser Ala Ser Asp Val
785             790             795             800

Trp Ser Phe Gly Ile Val Met Trp Glu Val Met Thr Tyr Gly Glu Arg
            805             810             815

Pro Tyr Trp Glu Leu Ser Asn His Glu Val Met Lys Ala Ile Asn Asp
            820             825             830

Gly Phe Arg Leu Pro Thr Pro Met Asp Cys Pro Ser Ala Ile Tyr Gln
            835             840             845

Leu Met Met Gln Cys Trp Gln Gln Glu Arg Ala Arg Arg Pro Lys Phe
    850             855             860

Ala Asp Ile Val Ser Ile Leu Asp Lys Leu Ile Arg Ala Pro Asp Ser
865             870             875             880

Leu Lys Thr Leu Ala Asp Phe Asp Pro Arg Val Ser Ile Arg Leu Pro
            885             890             895

Ser Thr Ser Gly Ser Glu Gly Val Pro Phe Arg Thr Val Ser Glu Trp
            900             905             910

Leu Glu Ser Ile Lys Met Gln Gln Tyr Thr Glu His Phe Met Ala Ala
    915             920             925

Gly Tyr Thr Ala Ile Glu Lys Val Val Gln Met Thr Asn Asp Asp Ile
    930             935             940

Lys Arg Ile Gly Val Arg Leu Pro Gly His Gln Lys Arg Ile Ala Tyr
945             950             955             960

Ser Leu Leu Gly Leu Lys Asp Gln Val Asn Thr Val Gly Ile Pro Ile
            965             970             975
```

<210> 35
<211> 829
<212> PRT
<213> Homo Sapiens

<220>
<221> MISC_FEATURE
<222> (1)..(829)
<223> OGTA202 = Swiss Prot Accession No. P22223, Cadherin-3

<400> 35

```
Met Gly Leu Pro Arg Gly Pro Leu Ala Ser Leu Leu Leu Leu Gln Val
1               5               10              15
```

```
Cys Trp Leu Gln Cys Ala Ala Ser Glu Pro Cys Arg Ala Val Phe Arg
            20              25              30

Glu Ala Glu Val Thr Leu Glu Ala Gly Gly Ala Glu Gln Glu Pro Gly
            35              40              45

Gln Ala Leu Gly Lys Val Phe Met Gly Cys Pro Gly Gln Glu Pro Ala
            50              55              60

Leu Phe Ser Thr Asp Asn Asp Asp Phe Thr Val Arg Asn Gly Glu Thr
65              70              75              80

Val Gln Glu Arg Arg Ser Leu Lys Glu Arg Asn Pro Leu Lys Ile Phe
            85              90              95

Pro Ser Lys Arg Ile Leu Arg Arg His Lys Arg Asp Trp Val Val Ala
            100             105             110

Pro Ile Ser Val Pro Glu Asn Gly Lys Gly Pro Phe Pro Gln Arg Leu
            115             120             125

Asn Gln Leu Lys Ser Asn Lys Asp Arg Asp Thr Lys Ile Phe Tyr Ser
            130             135             140

Ile Thr Gly Pro Gly Ala Asp Ser Pro Pro Glu Gly Val Phe Ala Val
145             150             155             160

Glu Lys Glu Thr Gly Trp Leu Leu Leu Asn Lys Pro Leu Asp Arg Glu
                165             170             175

Glu Ile Ala Lys Tyr Glu Leu Phe Gly His Ala Val Ser Glu Asn Gly
            180             185             190

Ala Ser Val Glu Asp Pro Met Asn Ile Ser Ile Ile Val Thr Asp Gln
            195             200             205

Asn Asp His Lys Pro Lys Phe Thr Gln Asp Thr Phe Arg Gly Ser Val
            210             215             220

Leu Glu Gly Val Leu Pro Gly Thr Ser Val Met Gln Val Thr Ala Thr
225             230             235             240

Asp Glu Asp Asp Ala Ile Tyr Thr Tyr Asn Gly Val Val Ala Tyr Ser
                245             250             255

Ile His Ser Gln Glu Pro Lys Asp Pro His Asp Leu Met Phe Thr Ile
            260             265             270
```

His Arg Ser Thr Gly Thr Ile Ser Val Ile Ser Ser Gly Leu Asp Arg
    275             280             285

Glu Lys Val Pro Glu Tyr Thr Leu Thr Ile Gln Ala Thr Asp Met Asp
    290             295             300

Gly Asp Gly Ser Thr Thr Thr Ala Val Ala Val Val Glu Ile Leu Asp
305             310             315             320

Ala Asn Asp Asn Ala Pro Met Phe Asp Pro Gln Lys Tyr Glu Ala His
            325             330             335

Val Pro Glu Asn Ala Val Gly His Glu Val Gln Arg Leu Thr Val Thr
            340             345             350

Asp Leu Asp Ala Pro Asn Ser Pro Ala Trp Arg Ala Thr Tyr Leu Ile
            355             360             365

Met Gly Gly Asp Asp Gly Asp His Phe Thr Ile Thr Thr His Pro Glu
    370             375             380

Ser Asn Gln Gly Ile Leu Thr Thr Arg Lys Gly Leu Asp Phe Glu Ala
385             390             395             400

Lys Asn Gln His Thr Leu Tyr Val Glu Val Thr Asn Glu Ala Pro Phe
            405             410             415

Val Leu Lys Leu Pro Thr Ser Thr Ala Thr Ile Val Val His Val Glu
            420             425             430

Asp Val Asn Glu Ala Pro Val Phe Val Pro Pro Ser Lys Val Val Glu
            435             440             445

Val Gln Glu Gly Ile Pro Thr Gly Glu Pro Val Cys Val Tyr Thr Ala
    450             455             460

Glu Asp Pro Asp Lys Glu Asn Gln Lys Ile Ser Tyr Arg Ile Leu Arg
465             470             475             480

Asp Pro Ala Gly Trp Leu Ala Met Asp Pro Asp Ser Gly Gln Val Thr
            485             490             495

Ala Val Gly Thr Leu Asp Arg Glu Asp Glu Gln Phe Val Arg Asn Asn
            500             505             510

Ile Tyr Glu Val Met Val Leu Ala Met Asp Asn Gly Ser Pro Pro Thr
    515             520             525

282

Thr Gly Thr Gly Thr Leu Leu Leu Thr Leu Ile Asp Val Asn Asp His
530 535 540

Gly Pro Val Pro Glu Pro Arg Gln Ile Thr Ile Cys Asn Gln Ser Pro
545 550 555 560

Val Arg His Val Leu Asn Ile Thr Asp Lys Asp Leu Ser Pro His Thr
565 570 575

Ser Pro Phe Gln Ala Gln Leu Thr Asp Asp Ser Asp Ile Tyr Trp Thr
580 585 590

Ala Glu Val Asn Glu Glu Gly Asp Thr Val Val Leu Ser Leu Lys Lys
595 600 605

Phe Leu Lys Gln Asp Thr Tyr Asp Val His Leu Ser Leu Ser Asp His
610 615 620

Gly Asn Lys Glu Gln Leu Thr Val Ile Arg Ala Thr Val Cys Asp Cys
625 630 635 640

His Gly His Val Glu Thr Cys Pro Gly Pro Trp Lys Gly Gly Phe Ile
645 650 655

Leu Pro Val Leu Gly Ala Val Leu Ala Leu Leu Phe Leu Leu Leu Val
660 665 670

Leu Leu Leu Leu Val Arg Lys Lys Arg Lys Ile Lys Glu Pro Leu Leu
675 680 685

Leu Pro Glu Asp Asp Thr Arg Asp Asn Val Phe Tyr Tyr Gly Glu Glu
690 695 700

Gly Gly Gly Glu Glu Asp Gln Asp Tyr Asp Ile Thr Gln Leu His Arg
705 710 715 720

Gly Leu Glu Ala Arg Pro Glu Val Val Leu Arg Asn Asp Val Ala Pro
725 730 735

Thr Ile Ile Pro Thr Pro Met Tyr Arg Pro Arg Pro Ala Asn Pro Asp
740 745 750

Glu Ile Gly Asn Phe Ile Ile Glu Asn Leu Lys Ala Ala Asn Thr Asp
755 760 765

Pro Thr Ala Pro Pro Tyr Asp Thr Leu Leu Val Phe Asp Tyr Glu Gly
770 775 780

Ser Gly Ser Asp Ala Ala Ser Leu Ser Ser Leu Thr Ser Ser Ala Ser

283

```
        785                  790                  795                  800

       Asp Gln Asp Gln Asp Tyr Asp Tyr Leu Asn Glu Trp Gly Ser Arg Phe
                       805             810                 815


       Lys Lys Leu Ala Asp Met Tyr Gly Gly Gly Glu Asp Asp
                   820             825
```

<210> 36
<211> 790
<212> PRT
<213> Homo Sapiens

<220>
<221> MISC_FEATURE
<222> (1)..(790)
<223> OGTA203 = Swiss Prot Accession No. P55285, Cadherin-6

<400> 36

```
Met Arg Thr Tyr Arg Tyr Phe Leu Leu Leu Phe Trp Val Gly Gln Pro
1               5               10              15

Tyr Pro Thr Leu Ser Thr Pro Leu Ser Lys Arg Thr Ser Gly Phe Pro
            20              25              30

Ala Lys Lys Arg Ala Leu Glu Leu Ser Gly Asn Ser Lys Asn Glu Leu
        35              40              45

Asn Arg Ser Lys Arg Ser Trp Met Trp Asn Gln Phe Phe Leu Leu Glu
    50              55              60

Glu Tyr Thr Gly Ser Asp Tyr Gln Tyr Val Gly Lys Leu His Ser Asp
65              70              75              80

Gln Asp Arg Gly Asp Gly Ser Leu Lys Tyr Ile Leu Ser Gly Asp Gly
            85              90              95

Ala Gly Asp Leu Phe Ile Ile Asn Glu Asn Thr Gly Asp Ile Gln Ala
            100             105             110

Thr Lys Arg Leu Asp Arg Glu Glu Lys Pro Val Tyr Ile Leu Arg Ala
        115             120             125

Gln Ala Ile Asn Arg Arg Thr Gly Arg Pro Val Glu Pro Glu Ser Glu
    130             135             140

Phe Ile Ile Lys Ile His Asp Ile Asn Asp Asn Glu Pro Ile Phe Thr
145             150             155             160

Lys Glu Val Tyr Thr Ala Thr Val Pro Glu Met Ser Asp Val Gly Thr
```

165               170               175

Phe Val Val Gln Val Thr Ala Thr Asp Ala Asp Asp Pro Thr Tyr Gly
           180               185                190

Asn Ser Ala Lys Val Val Tyr Ser Ile Leu Gln Gly Gln Pro Tyr Phe
         195               200                205

Ser Val Glu Ser Glu Thr Gly Ile Ile Lys Thr Ala Leu Leu Asn Met
         210               215                220

Asp Arg Glu Asn Arg Glu Gln Tyr Gln Val Val Ile Gln Ala Lys Asp
225               230               235                240

Met Gly Gly Gln Met Gly Gly Leu Ser Gly Thr Thr Thr Val Asn Ile
           245               250                255

Thr Leu Thr Asp Val Asn Asp Asn Pro Pro Arg Phe Pro Gln Ser Thr
         260               265                270

Tyr Gln Phe Lys Thr Pro Glu Ser Ser Pro Pro Gly Thr Pro Ile Gly
           275               280                285

Arg Ile Lys Ala Ser Asp Ala Asp Val Gly Glu Asn Ala Glu Ile Glu
         290               295                300

Tyr Ser Ile Thr Asp Gly Glu Gly Leu Asp Met Phe Asp Val Ile Thr
305               310               315                320

Asp Gln Glu Thr Gln Glu Gly Ile Ile Thr Val Lys Lys Leu Leu Asp
           325               330                335

Phe Glu Lys Lys Lys Val Tyr Thr Leu Lys Val Glu Ala Ser Asn Pro
         340               345                350

Tyr Val Glu Pro Arg Phe Leu Tyr Leu Gly Pro Phe Lys Asp Ser Ala
           355               360                365

Thr Val Arg Ile Val Val Glu Asp Val Asp Glu Pro Pro Val Phe Ser
         370               375                380

Lys Leu Ala Tyr Ile Leu Gln Ile Arg Glu Asp Ala Gln Ile Asn Thr
385               390               395                400

Thr Ile Gly Ser Val Thr Ala Gln Asp Pro Asp Ala Ala Arg Asn Pro
           405               410                415

Val Lys Tyr Ser Val Asp Arg His Thr Asp Met Asp Arg Ile Phe Asn
           420               425                430

```
Ile Asp Ser Gly Asn Gly Ser Ile Phe Thr Ser Lys Leu Leu Asp Arg
    435                 440             445

Glu Thr Leu Leu Trp His Asn Ile Thr Val Ile Ala Thr Glu Ile Asn
    450                 455             460

Asn Pro Lys Gln Ser Ser Arg Val Pro Leu Tyr Ile Lys Val Leu Asp
465                 470             475                 480

Val Asn Asp Asn Ala Pro Glu Phe Ala Glu Phe Tyr Glu Thr Phe Val
            485             490                 495

Cys Glu Lys Ala Lys Ala Asp Gln Leu Ile Gln Thr Leu His Ala Val
        500             505             510

Asp Lys Asp Asp Pro Tyr Ser Gly His Gln Phe Ser Phe Ser Leu Ala
        515             520             525

Pro Glu Ala Ala Ser Gly Ser Asn Phe Thr Ile Gln Asp Asn Lys Asp
    530             535             540

Asn Thr Ala Gly Ile Leu Thr Arg Lys Asn Gly Tyr Asn Arg His Glu
545             550             555             560

Met Ser Thr Tyr Leu Leu Pro Val Val Ile Ser Asp Asn Asp Tyr Pro
            565             570                 575

Val Gln Ser Ser Thr Gly Thr Val Thr Val Arg Val Cys Ala Cys Asp
        580             585                 590

His His Gly Asn Met Gln Ser Cys His Ala Glu Ala Leu Ile His Pro
        595             600             605

Thr Gly Leu Ser Thr Gly Ala Leu Val Ala Ile Leu Leu Cys Ile Val
    610             615             620

Ile Leu Leu Val Thr Val Val Leu Phe Ala Ala Leu Arg Arg Gln Arg
625             630             635             640

Lys Lys Glu Pro Leu Ile Ile Ser Lys Glu Asp Ile Arg Asp Asn Ile
            645             650                 655

Val Ser Tyr Asn Asp Glu Gly Gly Gly Glu Glu Asp Thr Gln Ala Phe
        660             665             670

Asp Ile Gly Thr Leu Arg Asn Pro Glu Ala Ile Glu Asp Asn Lys Leu
    675             680             685
```

287

```
Arg Arg Asp Ile Val Pro Glu Ala Leu Phe Leu Pro Arg Arg Thr Pro
    690             695             700

Thr Ala Arg Asp Asn Thr Asp Val Arg Asp Phe Ile Asn Gln Arg Leu
705             710             715             720

Lys Glu Asn Asp Thr Asp Pro Thr Ala Pro Pro Tyr Asp Ser Leu Ala
                725             730             735

Thr Tyr Ala Tyr Glu Gly Thr Gly Ser Val Ala Asp Ser Leu Ser Ser
        740             745             750

Leu Glu Ser Val Thr Thr Asp Ala Asp Gln Asp Tyr Asp Tyr Leu Ser
        755             760             765

Asp Trp Gly Pro Arg Phe Lys Lys Leu Ala Asp Met Tyr Gly Gly Val
    770             775             780

Asp Ser Asp Lys Asp Ser
785             790
```

<210> 37
<211> 209
<212> PRT
<213> Homo Sapiens

<220>
<221> MISC_FEATURE
<222> (1) .. (209)
<223> OGTA206 = Swiss Prot Accession No. 014493, Claudin-4

<400> 37

```
Met Ala Ser Met Gly Leu Gln Val Met Gly Ile Ala Leu Ala Val Leu
1               5               10              15

Gly Trp Leu Ala Val Met Leu Cys Cys Ala Leu Pro Met Trp Arg Val
            20              25              30

Thr Ala Phe Ile Gly Ser Asn Ile Val Thr Ser Gln Thr Ile Trp Glu
            35              40              45

Gly Leu Trp Met Asn Cys Val Val Gln Ser Thr Gly Gln Met Gln Cys
    50              55              60

Lys Val Tyr Asp Ser Leu Leu Ala Leu Pro Gln Asp Leu Gln Ala Ala
65              70              75              80

Arg Ala Leu Val Ile Ile Ser Ile Ile Val Ala Ala Leu Gly Val Leu
                85              90              95

Leu Ser Val Val Gly Gly Lys Cys Thr Asn Cys Leu Glu Asp Glu Ser
            100             105             110

Ala Lys Ala Lys Thr Met Ile Val Ala Gly Val Val Phe Leu Leu Ala
        115             120             125

Gly Leu Met Val Ile Val Pro Val Ser Trp Thr Ala His Asn Ile Ile
    130             135             140

Gln Asp Phe Tyr Asn Pro Leu Val Ala Ser Gly Gln Lys Arg Glu Met
145             150             155             160

Gly Ala Ser Leu Tyr Val Gly Trp Ala Ala Ser Gly Leu Leu Leu Leu
            165             170             175

Gly Gly Gly Leu Leu Cys Cys Asn Cys Pro Pro Arg Thr Asp Lys Pro
        180             185             190

Tyr Ser Ala Lys Tyr Ser Ala Ala Arg Ser Ala Ala Ala Ser Asn Tyr
    195             200             205

Val
```

<210> 38
<211> 1821
<212> PRT
<213> Homo Sapiens

<220>
<221> MISC_FEATURE

<222> (1)..(1821)
<223> OGTA213 = Swiss Prot Accession No. Q14767, Latent-transforming growth factor beta-binding protein 2

<400> 38

```
Met Arg Pro Arg Thr Lys Ala Arg Ser Pro Gly Arg Ala Leu Arg Asn
1               5                  10                  15

Pro Trp Arg Gly Phe Leu Pro Leu Thr Leu Ala Leu Phe Val Gly Ala
            20                  25                  30

Gly His Ala Gln Arg Asp Pro Val Gly Arg Tyr Glu Pro Ala Gly Gly
        35                  40                  45

Asp Ala Asn Arg Leu Arg Arg Pro Gly Gly Ser Tyr Pro Ala Ala Ala
    50                  55                  60

Ala Ala Lys Val Tyr Ser Leu Phe Arg Glu Gln Asp Ala Pro Val Ala
65                  70                  75                  80
```

```
Gly Leu Gln Pro Val Glu Arg Ala Gln Pro Gly Trp Gly Ser Pro Arg
                85                  90                  95

Arg Pro Thr Glu Ala Glu Ala Arg Arg Pro Ser Arg Ala Gln Gln Ser
               100                 105                 110

Arg Arg Val Gln Pro Pro Ala Gln Thr Arg Arg Ser Thr Pro Leu Gly
               115                 120                 125

Gln Gln Gln Pro Ala Pro Arg Thr Arg Ala Ala Pro Ala Leu Pro Arg
           130                 135                 140

Leu Gly Thr Pro Gln Arg Ser Gly Ala Ala Pro Pro Thr Pro Pro Arg
145                 150                 155                 160

Gly Arg Leu Thr Gly Arg Asn Val Cys Gly Gly Gln Cys Cys Pro Gly
               165                 170                 175

Trp Thr Thr Ala Asn Ser Thr Asn His Cys Ile Lys Pro Val Cys Glu
               180                 185                 190

Pro Pro Cys Gln Asn Arg Gly Ser Cys Ser Arg Pro Gln Leu Cys Val
               195                 200                 205

Cys Arg Ser Gly Phe Arg Gly Ala Arg Cys Glu Glu Val Ile Pro Asp
           210                 215                 220

Glu Glu Phe Asp Pro Gln Asn Ser Arg Leu Ala Pro Arg Arg Trp Ala
225                 230                 235                 240

Glu Arg Ser Pro Asn Leu Arg Arg Ser Ser Ala Ala Gly Glu Gly Thr
               245                 250                 255

Leu Ala Arg Ala Gln Pro Pro Ala Pro Gln Ser Pro Pro Ala Pro Gln
               260                 265                 270

Ser Pro Pro Ala Gly Thr Leu Ser Gly Leu Ser Gln Thr His Pro Ser
               275                 280                 285

Gln Gln His Val Gly Leu Ser Arg Thr Val Arg Leu His Pro Thr Ala
           290                 295                 300

Thr Ala Ser Ser Gln Leu Ser Ser Asn Ala Leu Pro Pro Gly Pro Gly
305                 310                 315                 320

Leu Glu Gln Arg Asp Gly Thr Gln Gln Ala Val Pro Leu Glu His Pro
               325                 330                 335
```

```
Ser Ser Pro Trp Gly Leu Asn Leu Thr Glu Lys Ile Lys Lys Ile Lys
        340             345                 350

Ile Val Phe Thr Pro Thr Ile Cys Lys Gln Thr Cys Ala Arg Gly His
        355             360                 365

Cys Ala Asn Ser Cys Glu Arg Gly Asp Thr Thr Thr Leu Tyr Ser Gln
    370             375                 380

Gly Gly His Gly His Asp Pro Lys Ser Gly Phe Arg Ile Tyr Phe Cys
385             390                 395                 400

Gln Ile Pro Cys Leu Asn Gly Gly Arg Cys Ile Gly Arg Asp Glu Cys
            405                 410                 415

Trp Cys Pro Ala Asn Ser Thr Gly Lys Phe Cys His Leu Pro Ile Pro
        420             425                 430

Gln Pro Asp Arg Glu Pro Pro Gly Arg Gly Ser Arg Pro Arg Ala Leu
        435             440                 445

Leu Glu Ala Pro Leu Lys Gln Ser Thr Phe Thr Leu Pro Leu Ser Asn
        450             455                 460

Gln Leu Ala Ser Val Asn Pro Ser Leu Val Lys Val His Ile His His
465             470                 475                 480

Pro Pro Glu Ala Ser Val Gln Ile His Gln Val Ala Gln Val Arg Gly
            485                 490                 495

Gly Val Glu Glu Ala Leu Val Glu Asn Ser Val Glu Thr Arg Pro Pro
        500             505                 510

Pro Trp Leu Pro Ala Ser Pro Gly His Ser Leu Trp Asp Ser Asn Asn
        515             520                 525

Ile Pro Ala Arg Ser Gly Glu Pro Pro Arg Pro Leu Pro Pro Ala Ala
    530             535                 540

Pro Arg Pro Arg Gly Leu Leu Gly Arg Cys Tyr Leu Asn Thr Val Asn
545             550                 555                 560

Gly Gln Cys Ala Asn Pro Leu Leu Glu Leu Thr Thr Gln Glu Asp Cys
            565                 570                 575

Cys Gly Ser Val Gly Ala Phe Trp Gly Val Thr Leu Cys Ala Pro Cys
            580                 585                 590
```

Pro Pro Arg Pro Ala Ser Pro Val Ile Glu Asn Gly Gln Leu Glu Cys
        595                 600                 605

Pro Gln Gly Tyr Lys Arg Leu Asn Leu Thr His Cys Gln Asp Ile Asn
    610                 615                 620

Glu Cys Leu Thr Leu Gly Leu Cys Lys Asp Ala Glu Cys Val Asn Thr
625                 630                 635                 640

Arg Gly Ser Tyr Leu Cys Thr Cys Arg Pro Gly Leu Met Leu Asp Pro
            645                 650                 655

Ser Arg Ser Arg Cys Val Ser Asp Lys Ala Ile Ser Met Leu Gln Gly
        660                 665                 670

Leu Cys Tyr Arg Ser Leu Gly Pro Gly Thr Cys Thr Leu Pro Leu Ala
    675                 680                 685

Gln Arg Ile Thr Lys Gln Ile Cys Cys Cys Ser Arg Val Gly Lys Ala
    690                 695                 700

Trp Gly Ser Glu Cys Glu Lys Cys Pro Leu Pro Gly Thr Glu Ala Phe
705                 710                 715                 720

Arg Glu Ile Cys Pro Ala Gly His Gly Tyr Thr Tyr Ala Ser Ser Asp
            725                 730                 735

Ile Arg Leu Ser Met Arg Lys Ala Glu Glu Glu Glu Leu Ala Arg Pro
            740                 745                 750

Pro Arg Glu Gln Gly Gln Arg Ser Ser Gly Ala Leu Pro Gly Pro Ala
    755                 760                 765

Glu Arg Gln Pro Leu Arg Val Val Thr Asp Thr Trp Leu Glu Ala Gly
    770                 775                 780

Thr Ile Pro Asp Lys Gly Asp Ser Gln Ala Gly Gln Val Thr Thr Ser
785                 790                 795                 800

Val Thr His Ala Pro Ala Trp Val Thr Gly Asn Ala Thr Thr Pro Pro
            805                 810                 815

Met Pro Glu Gln Gly Ile Ala Glu Ile Gln Glu Glu Gln Val Thr Pro
        820                 825                 830

Ser Thr Asp Val Leu Val Thr Leu Ser Thr Pro Gly Ile Asp Arg Cys
        835                 840                 845

Ala Ala Gly Ala Thr Asn Val Cys Gly Pro Gly Thr Cys Val Asn Leu

293

850                          855                          860

Pro Asp Gly Tyr Arg Cys Val Cys Ser Pro Gly Tyr Gln Leu His Pro
865                     870             875                     880

Ser Gln Ala Tyr Cys Thr Asp Asp Asn Glu Cys Leu Arg Asp Pro Cys
                885             890                     895

Lys Gly Lys Gly Arg Cys Ile Asn Arg Val Gly Ser Tyr Ser Cys Phe
            900             905                 910

Cys Tyr Pro Gly Tyr Thr Leu Ala Thr Ser Gly Ala Thr Gln Glu Cys
        915             920                 925

Gln Asp Ile Asn Glu Cys Glu Gln Pro Gly Val Cys Ser Gly Gly Gln
    930             935                 940

Cys Thr Asn Thr Glu Gly Ser Tyr His Cys Glu Cys Asp Gln Gly Tyr
945             950                 955                     960

Ile Met Val Arg Lys Gly His Cys Gln Asp Ile Asn Glu Cys Arg His
            965                 970                     975

Pro Gly Thr Cys Pro Asp Gly Arg Cys Val Asn Ser Pro Gly Ser Tyr
        980                 985                 990

Thr Cys Leu Ala Cys Glu Glu Gly  Tyr Arg Gly Gln Ser  Gly Ser Cys
        995             1000                 1005

Val Asp  Val Asn Glu Cys Leu  Thr Pro Gly Val Cys  Ala His Gly
    1010             1015                 1020

Lys Cys  Thr Asn Leu Glu Gly  Ser Phe Arg Cys Ser  Cys Glu Gln
    1025             1030                 1035

Gly Tyr  Glu Val Thr Ser Asp  Glu Lys Gly Cys Gln  Asp Val Asp
    1040             1045                 1050

Glu Cys  Ala Ser Arg Ala Ser  Cys Pro Thr Gly Leu  Cys Leu Asn
    1055             1060                 1065

Thr Glu  Gly Ser Phe Ala Cys  Ser Ala Cys Glu Asn  Gly Tyr Trp
    1070             1075                 1080

Val Asn  Glu Asp Gly Thr Ala  Cys Glu Asp Leu Asp  Glu Cys Ala
    1085             1090                 1095

Phe Pro  Gly Val Cys Pro Ser  Gly Val Cys Thr Asn  Thr Ala Gly
    1100             1105                 1110

294

```
Ser Phe  Ser Cys Lys Asp Cys  Asp Gly Gly Tyr Arg  Pro Ser Pro
    1115             1120               1125

Leu Gly  Asp Ser Cys Glu Asp  Val Asp Glu Cys Glu  Asp Pro Gln
    1130             1135               1140

Ser Ser  Cys Leu Gly Gly Glu  Cys Lys Asn Thr Val  Gly Ser Tyr
    1145             1150               1155

Gln Cys  Leu Cys Pro Gln Gly  Phe Gln Leu Ala Asn  Gly Thr Val
    1160             1165               1170

Cys Glu  Asp Val Asn Glu Cys  Met Gly Glu Glu His  Cys Ala Pro
    1175             1180               1185

His Gly  Glu Cys Leu Asn Ser  His Gly Ser Phe Phe  Cys Leu Cys
    1190             1195               1200

Ala Pro  Gly Phe Val Ser Ala  Glu Gly Gly Thr Ser  Cys Gln Asp
    1205             1210               1215

Val Asp  Glu Cys Ala Thr Thr  Asp Pro Cys Val Gly  Gly His Cys
    1220             1225               1230

Val Asn  Thr Glu Gly Ser Phe  Asn Cys Leu Cys Glu  Thr Gly Phe
    1235             1240               1245

Gln Pro  Ser Pro Glu Ser Gly  Glu Cys Val Asp Ile  Asp Glu Cys
    1250             1255               1260

Glu Asp  Tyr Gly Asp Pro Val  Cys Gly Thr Trp Lys  Cys Glu Asn
    1265             1270               1275

Ser Pro  Gly Ser Tyr Arg Cys  Val Leu Gly Cys Gln  Pro Gly Phe
    1280             1285               1290

His Met  Ala Pro Asn Gly Asp  Cys Ile Asp Ile Asp  Glu Cys Ala
    1295             1300               1305

Asn Asp  Thr Met Cys Gly Ser  His Gly Phe Cys Asp  Asn Thr Asp
    1310             1315               1320

Gly Ser  Phe Arg Cys Leu Cys  Asp Gln Gly Phe Glu  Ile Ser Pro
    1325             1330               1335

Ser Gly  Trp Asp Cys Val Asp  Val Asn Glu Cys Glu  Leu Met Leu
    1340             1345               1350
```

```
Ala Val Cys Gly Ala Ala Leu Cys Glu Asn Val Glu Gly Ser Phe
    1355             1360             1365

Leu Cys Leu Cys Ala Ser Asp Leu Glu Glu Tyr Asp Ala Gln Glu
    1370             1375             1380

Gly His Cys Arg Pro Arg Gly Ala Gly Gly Gln Ser Met Ser Glu
    1385             1390             1395

Ala Pro Thr Gly Asp His Ala Pro Ala Pro Thr Arg Met Asp Cys
    1400             1405             1410

Tyr Ser Gly Gln Lys Gly His Ala Pro Cys Ser Ser Val Leu Gly
    1415             1420             1425

Arg Asn Thr Thr Gln Ala Glu Cys Cys Cys Thr Gln Gly Ala Thr
    1430             1435             1440

Trp Gly Asp Ala Cys Asp Leu Cys Pro Ser Glu Asp Ser Ala Glu
    1445             1450             1455

Phe Ser Glu Ile Cys Pro Ser Gly Lys Gly Tyr Ile Pro Val Glu
    1460             1465             1470

Gly Ala Trp Thr Phe Gly Gln Thr Met Tyr Thr Asp Ala Asp Glu
    1475             1480             1485

Cys Val Ile Phe Gly Pro Gly Leu Cys Pro Asn Gly Arg Cys Leu
    1490             1495             1500

Asn Thr Val Pro Gly Tyr Val Cys Leu Cys Asn Pro Gly Phe His
    1505             1510             1515

Tyr Asp Ala Ser His Lys Lys Cys Glu Asp His Asp Glu Cys Gln
    1520             1525             1530

Asp Leu Ala Cys Glu Asn Gly Glu Cys Val Asn Thr Glu Gly Ser
    1535             1540             1545

Phe His Cys Phe Cys Ser Pro Pro Leu Thr Leu Asp Leu Ser Gln
    1550             1555             1560

Gln Arg Cys Met Asn Ser Thr Ser Ser Thr Glu Asp Leu Pro Asp
    1565             1570             1575

His Asp Ile His Met Asp Ile Cys Trp Lys Lys Val Thr Asn Asp
    1580             1585             1590
```

296

```
Val Cys Ser Glu Pro Leu Arg Gly His Arg Thr Thr Tyr Thr Glu
1595               1600           1605

Cys Cys Cys Gln Asp Gly Lys Ala Trp Ser Gln Gln Cys Ala Leu
1610               1615           1620

Cys Pro Pro Arg Ser Ser Glu Val Tyr Ala Gln Leu Cys Asn Val
1625               1630           1635

Ala Arg Ile Glu Ala Glu Arg Glu Ala Gly Val His Phe Arg Pro
1640               1645           1650

Gly Tyr Glu Tyr Gly Pro Gly Pro Asp Asp Leu His Tyr Ser Ile
1655               1660           1665

Tyr Gly Pro Asp Gly Ala Pro Phe Tyr Asn Tyr Leu Gly Pro Glu
1670               1675           1680

Asp Thr Val Pro Glu Pro Ala Phe Pro Asn Thr Ala Gly His Ser
1685               1690           1695

Ala Asp Arg Thr Pro Ile Leu Glu Ser Pro Leu Gln Pro Ser Glu
1700               1705           1710

Leu Gln Pro His Tyr Val Ala Ser His Pro Glu Pro Pro Ala Gly
1715               1720           1725

Phe Glu Gly Leu Gln Ala Glu Glu Cys Gly Ile Leu Asn Gly Cys
1730               1735           1740

Glu Asn Gly Arg Cys Val Arg Val Arg Glu Gly Tyr Thr Cys Asp
1745               1750           1755

Cys Phe Glu Gly Phe Gln Leu Asp Ala Ala His Met Ala Cys Val
1760               1765           1770

Asp Val Asn Glu Cys Asp Asp Leu Asn Gly Pro Ala Val Leu Cys
1775               1780           1785

Val His Gly Tyr Cys Glu Asn Thr Glu Gly Ser Tyr Arg Cys His
1790               1795           1800

Cys Ser Pro Gly Tyr Val Ala Glu Ala Gly Pro Pro His Cys Thr
1805               1810           1815

Ala Lys Glu
1820
```

<210> 39
<211> 512
<212> PRT
<213> Homo Sapiens

<220>
<221> MISC_FEATURE
<222> (1)..(512)
<223> OGTA214 = Swiss Prot Accession No. Q9H3R2, Mucin-13

<400> 39

```
Met Lys Ala Ile Ile His Leu Thr Leu Leu Ala Leu Leu Ser Val Asn
1               5               10              15

Thr Ala Thr Asn Gln Gly Asn Ser Ala Asp Ala Val Thr Thr Thr Glu
            20              25              30

Thr Ala Thr Ser Gly Pro Thr Val Ala Ala Ala Asp Thr Thr Glu Thr
            35              40              45

Asn Phe Pro Glu Thr Ala Ser Thr Thr Ala Asn Thr Pro Ser Phe Pro
    50              55              60

Thr Ala Thr Ser Pro Ala Pro Pro Ile Ile Ser Thr His Ser Ser Ser
65              70              75              80

Thr Ile Pro Thr Pro Ala Pro Pro Ile Ile Ser Thr His Ser Ser Ser
            85              90              95

Thr Ile Pro Ile Pro Thr Ala Ala Asp Ser Glu Ser Thr Thr Asn Val
            100             105             110

Asn Ser Leu Ala Thr Ser Asp Ile Ile Thr Ala Ser Ser Pro Asn Asp
        115             120             125

Gly Leu Ile Thr Met Val Pro Ser Glu Thr Gln Ser Asn Asn Glu Met
    130             135             140

Ser Pro Thr Thr Glu Asp Asn Gln Ser Ser Gly Pro Pro Thr Gly Thr
145             150             155             160

Ala Leu Leu Glu Thr Ser Thr Leu Asn Ser Thr Gly Pro Ser Asn Pro
            165             170             175

Cys Gln Asp Asp Pro Cys Ala Asp Asn Ser Leu Cys Val Lys Leu His
        180             185             190

Asn Thr Ser Phe Cys Leu Cys Leu Glu Gly Tyr Tyr Tyr Asn Ser Ser
        195             200             205

Thr Cys Lys Lys Gly Lys Val Phe Pro Gly Lys Ile Ser Val Thr Val
```

|  | 210 | | | | | 215 | | | | | 220 | | |
|---|---|---|---|---|---|---|---|---|---|---|---|---|---|

Ser Glu Thr Phe Asp Pro Glu Glu Lys His Ser Met Ala Tyr Gln Asp
225 230 235 240

Leu His Ser Glu Ile Thr Ser Leu Phe Lys Asp Val Phe Gly Thr Ser
245 250 255

Val Tyr Gly Gln Thr Val Ile Leu Thr Val Ser Thr Ser Leu Ser Pro
260 265 270

Arg Ser Glu Met Arg Ala Asp Asp Lys Phe Val Asn Val Thr Ile Val
275 280 285

Thr Ile Leu Ala Glu Thr Thr Ser Asp Asn Glu Lys Thr Val Thr Glu
290 295 300

Lys Ile Asn Lys Ala Ile Arg Ser Ser Ser Ser Asn Phe Leu Asn Tyr
305 310 315 320

Asp Leu Thr Leu Arg Cys Asp Tyr Tyr Gly Cys Asn Gln Thr Ala Asp
325 330 335

Asp Cys Leu Asn Gly Leu Ala Cys Asp Cys Lys Ser Asp Leu Gln Arg
340 345 350

Pro Asn Pro Gln Ser Pro Phe Cys Val Ala Ser Ser Leu Lys Cys Pro
355 360 365

Asp Ala Cys Asn Ala Gln His Lys Gln Cys Leu Ile Lys Lys Ser Gly
370 375 380

Gly Ala Pro Glu Cys Ala Cys Val Pro Gly Tyr Gln Glu Asp Ala Asn
385 390 395 400

Gly Asn Cys Gln Lys Cys Ala Phe Gly Tyr Ser Gly Leu Asp Cys Lys
405 410 415

Asp Lys Phe Gln Leu Ile Leu Thr Ile Val Gly Thr Ile Ala Gly Ile
420 425 430

Val Ile Leu Ser Met Ile Ile Ala Leu Ile Val Thr Ala Arg Ser Asn
435 440 445

Asn Lys Thr Lys His Ile Glu Glu Glu Asn Leu Ile Asp Glu Asp Phe
450 455 460

Gln Asn Leu Lys Leu Arg Ser Thr Gly Phe Thr Asn Leu Gly Ala Glu
465 470 475 480

```
Gly Ser Val Phe Pro Lys Val Arg Ile Thr Ala Ser Arg Asp Ser Gln
                485             490             495

Met Gln Asn Pro Tyr Ser Arg His Ser Ser Met Pro Arg Pro Asp Tyr
                500             505             510
```

<210> 40
<211> 1212
<212> PRT
<213> Homo Sapiens

<220>
<221> MISC_FEATURE
<222> (1)..(1212)
<223> OGTA216 = Swiss Prot Accession No. P55011, Solute carrier family 12 member 2

<400> 40

```
Met Glu Pro Arg Pro Thr Ala Pro Ser Ser Gly Ala Pro Gly Leu Ala
1               5               10              15

Gly Val Gly Glu Thr Pro Ser Ala Ala Ala Leu Ala Ala Ala Arg Val
                20              25              30

Glu Leu Pro Gly Thr Ala Val Pro Ser Val Pro Glu Asp Ala Ala Pro
                35              40              45

Ala Ser Arg Asp Gly Gly Gly Val Arg Asp Glu Gly Pro Ala Ala Ala
        50              55              60

Gly Asp Gly Leu Gly Arg Pro Leu Gly Pro Thr Pro Ser Gln Ser Arg
65              70              75              80

Phe Gln Val Asp Leu Val Ser Glu Asn Ala Gly Arg Ala Ala Ala Ala
                85              90              95

Ala Ala Ala Ala Ala Ala Ala Ala Ala Ala Ala Gly Ala Gly Ala Gly
                100             105             110

Ala Lys Gln Thr Pro Ala Asp Gly Glu Ala Ser Gly Glu Ser Glu Pro
        115             120             125

Ala Lys Gly Ser Glu Glu Ala Lys Gly Arg Phe Arg Val Asn Phe Val
        130             135             140

Asp Pro Ala Ala Ser Ser Ser Ala Glu Asp Ser Leu Ser Asp Ala Ala
145             150             155             160

Gly Val Gly Val Asp Gly Pro Asn Val Ser Phe Gln Asn Gly Gly Asp
```

```
                    165                      170                          175


        Thr Val Leu Ser Glu Gly Ser Ser Leu His Ser Gly Gly Gly Gly Gly
                    180                 185                 190


        Ser Gly His His Gln His Tyr Tyr Tyr Asp Thr His Thr Asn Thr Tyr
                    195                 200                 205


        Tyr Leu Arg Thr Phe Gly His Asn Thr Met Asp Ala Val Pro Arg Ile
                    210                 215                 220


        Asp His Tyr Arg His Thr Ala Ala Gln Leu Gly Glu Lys Leu Leu Arg
        225                 230                 235                 240


        Pro Ser Leu Ala Glu Leu His Asp Glu Leu Glu Lys Glu Pro Phe Glu
                    245                 250                 255


        Asp Gly Phe Ala Asn Gly Glu Glu Ser Thr Pro Thr Arg Asp Ala Val
                    260                 265                 270


        Val Thr Tyr Thr Ala Glu Ser Lys Gly Val Val Lys Phe Gly Trp Ile
                    275                 280                 285


        Lys Gly Val Leu Val Arg Cys Met Leu Asn Ile Trp Gly Val Met Leu
                    290                 295                 300


        Phe Ile Arg Leu Ser Trp Ile Val Gly Gln Ala Gly Ile Gly Leu Ser
        305                 310                 315                 320


        Val Leu Val Ile Met Met Ala Thr Val Val Thr Thr Ile Thr Gly Leu
                    325                 330                 335


        Ser Thr Ser Ala Ile Ala Thr Asn Gly Phe Val Arg Gly Gly Gly Ala
                    340                 345                 350


        Tyr Tyr Leu Ile Ser Arg Ser Leu Gly Pro Glu Phe Gly Gly Ala Ile
                    355                 360                 365


        Gly Leu Ile Phe Ala Phe Ala Asn Ala Val Ala Val Ala Met Tyr Val
                    370                 375                 380


        Val Gly Phe Ala Glu Thr Val Val Glu Leu Leu Lys Glu His Ser Ile
        385                 390                 395                 400


        Leu Met Ile Asp Glu Ile Asn Asp Ile Arg Ile Ile Gly Ala Ile Thr
                    405                 410                 415


        Val Val Ile Leu Leu Gly Ile Ser Val Ala Gly Met Glu Trp Glu Ala
                    420                 425                 430
```

Lys Ala Gln Ile Val Leu Leu Val Ile Leu Leu Leu Ala Ile Gly Asp
435 440 445

Phe Val Ile Gly Thr Phe Ile Pro Leu Glu Ser Lys Lys Pro Lys Gly
450 455 460

Phe Phe Gly Tyr Lys Ser Glu Ile Phe Asn Glu Asn Phe Gly Pro Asp
465 470 475 480

Phe Arg Glu Glu Glu Thr Phe Phe Ser Val Phe Ala Ile Phe Phe Pro
485 490 495

Ala Ala Thr Gly Ile Leu Ala Gly Ala Asn Ile Ser Gly Asp Leu Ala
500 505 510

Asp Pro Gln Ser Ala Ile Pro Lys Gly Thr Leu Leu Ala Ile Leu Ile
515 520 525

Thr Thr Leu Val Tyr Val Gly Ile Ala Val Ser Val Gly Ser Cys Val
530 535 540

Val Arg Asp Ala Thr Gly Asn Val Asn Asp Thr Ile Val Thr Glu Leu
545 550 555 560

Thr Asn Cys Thr Ser Ala Ala Cys Lys Leu Asn Phe Asp Phe Ser Ser
565 570 575

Cys Glu Ser Ser Pro Cys Ser Tyr Gly Leu Met Asn Asn Phe Gln Val
580 585 590

Met Ser Met Val Ser Gly Phe Thr Pro Leu Ile Ser Ala Gly Ile Phe
595 600 605

Ser Ala Thr Leu Ser Ser Ala Leu Ala Ser Leu Val Ser Ala Pro Lys
610 615 620

Ile Phe Gln Ala Leu Cys Lys Asp Asn Ile Tyr Pro Ala Phe Gln Met
625 630 635 640

Phe Ala Lys Gly Tyr Gly Lys Asn Asn Glu Pro Leu Arg Gly Tyr Ile
645 650 655

Leu Thr Phe Leu Ile Ala Leu Gly Phe Ile Leu Ile Ala Glu Leu Asn
660 665 670

Val Ile Ala Pro Ile Ile Ser Asn Phe Phe Leu Ala Ser Tyr Ala Leu
675 680 685

```
Ile Asn Phe Ser Val Phe His Ala Ser Leu Ala Lys Ser Pro Gly Trp
    690             695             700

Arg Pro Ala Phe Lys Tyr Tyr Asn Met Trp Ile Ser Leu Leu Gly Ala
705             710             715                 720

Ile Leu Cys Cys Ile Val Met Phe Val Ile Asn Trp Trp Ala Ala Leu
            725             730                 735

Leu Thr Tyr Val Ile Val Leu Gly Leu Tyr Ile Tyr Val Thr Tyr Lys
            740             745             750

Lys Pro Asp Val Asn Trp Gly Ser Ser Thr Gln Ala Leu Thr Tyr Leu
    755             760             765

Asn Ala Leu Gln His Ser Ile Arg Leu Ser Gly Val Glu Asp His Val
    770             775             780

Lys Asn Phe Arg Pro Gln Cys Leu Val Met Thr Gly Ala Pro Asn Ser
785             790             795                 800

Arg Pro Ala Leu Leu His Leu Val His Asp Phe Thr Lys Asn Val Gly
            805             810             815

Leu Met Ile Cys Gly His Val His Met Gly Pro Arg Arg Gln Ala Met
            820             825             830

Lys Glu Met Ser Ile Asp Gln Ala Lys Tyr Gln Arg Trp Leu Ile Lys
            835             840             845

Asn Lys Met Lys Ala Phe Tyr Ala Pro Val His Ala Asp Asp Leu Arg
    850             855             860

Glu Gly Ala Gln Tyr Leu Met Gln Ala Ala Gly Leu Gly Arg Met Lys
865             870             875                 880

Pro Asn Thr Leu Val Leu Gly Phe Lys Lys Asp Trp Leu Gln Ala Asp
            885             890             895

Met Arg Asp Val Asp Met Tyr Ile Asn Leu Phe His Asp Ala Phe Asp
            900             905             910

Ile Gln Tyr Gly Val Val Val Ile Arg Leu Lys Glu Gly Leu Asp Ile
            915             920             925

Ser His Leu Gln Gly Gln Glu Glu Leu Leu Ser Ser Gln Glu Lys Ser
    930             935             940
```

```
Pro Gly Thr Lys Asp Val Val Val Ser Val Glu Tyr Ser Lys Lys Ser
945             950             955             960

Asp Leu Asp Thr Ser Lys Pro Leu Ser Glu Lys Pro Ile Thr His Lys
                965             970             975

Val Glu Glu Glu Asp Gly Lys Thr Ala Thr Gln Pro Leu Leu Lys Lys
            980             985             990

Glu Ser Lys Gly Pro Ile Val Pro  Leu Asn Val Ala Asp  Gln Lys Leu
            995             1000            1005

Leu Glu  Ala Ser Thr Gln Phe  Gln Lys Lys Gln Gly  Lys Asn Thr
    1010            1015            1020

Ile Asp  Val Trp Trp Leu Phe  Asp Asp Gly Gly Leu  Thr Leu Leu
    1025            1030            1035

Ile Pro  Tyr Leu Leu Thr Thr  Lys Lys Lys Trp Lys  Asp Cys Lys
    1040            1045            1050

Ile Arg  Val Phe Ile Gly Gly  Lys Ile Asn Arg Ile  Asp His Asp
    1055            1060            1065

Arg Arg  Ala Met Ala Thr Leu  Leu Ser Lys Phe Arg  Ile Asp Phe
    1070            1075            1080

Ser Asp  Ile Met Val Leu Gly  Asp Ile Asn Thr Lys  Pro Lys Lys
    1085            1090            1095

Glu Asn  Ile Ile Ala Phe Glu  Glu Ile Ile Glu Pro  Tyr Arg Leu
    1100            1105            1110

His Glu  Asp Asp Lys Glu Gln  Asp Ile Ala Asp Lys  Met Lys Glu
    1115            1120            1125

Asp Glu  Pro Trp Arg Ile Thr  Asp Asn Glu Leu Glu  Leu Tyr Lys
    1130            1135            1140

Thr Lys  Thr Tyr Arg Gln Ile  Arg Leu Asn Glu Leu  Leu Lys Glu
    1145            1150            1155

His Ser  Ser Thr Ala Asn Ile  Ile Val Met Ser Leu  Pro Val Ala
    1160            1165            1170

Arg Lys  Gly Ala Val Ser Ser  Ala Leu Tyr Met Ala  Trp Leu Glu
    1175            1180            1185

Ala Leu  Ser Lys Asp Leu Pro  Pro Ile Leu Leu Val  Arg Gly Asn
```

```
                1190                      1195                      1200


        His Gln  Ser Val Leu Thr Phe  Tyr Ser
                1205                   1210
```

<210> 41
<211> 411
<212> PRT
<213> Homo Sapiens

<220>
<221> MISC_FEATURE
<222> (1)..(411)
<223> OGTA222 = Swiss Prot Accession No. P16444, Dipeptidase 1

<400> 41

Met Trp Ser Gly Trp Trp Leu Trp Pro Leu Val Ala Val Cys Thr Ala
1               5               10              15

Asp Phe Phe Arg Asp Glu Ala Glu Arg Ile Met Arg Asp Ser Pro Val
            20              25              30

Ile Asp Gly His Asn Asp Leu Pro Trp Gln Leu Leu Asp Met Phe Asn
        35              40              45

Asn Arg Leu Gln Asp Glu Arg Ala Asn Leu Thr Thr Leu Ala Gly Thr
    50              55              60

His Thr Asn Ile Pro Lys Leu Arg Ala Gly Phe Val Gly Gly Gln Phe
65              70              75              80

Trp Ser Val Tyr Thr Pro Cys Asp Thr Gln Asn Lys Asp Ala Val Arg
            85              90              95

Arg Thr Leu Glu Gln Met Asp Val Val His Arg Met Cys Arg Met Tyr
        100             105             110

Pro Glu Thr Phe Leu Tyr Val Thr Ser Ser Ala Gly Ile Arg Gln Ala
        115             120             125

Phe Arg Glu Gly Lys Val Ala Ser Leu Ile Gly Val Glu Gly Gly His
    130             135             140

Ser Ile Asp Ser Ser Leu Gly Val Leu Arg Ala Leu Tyr Gln Leu Gly
145             150             155             160

Met Arg Tyr Leu Thr Leu Thr His Ser Cys Asn Thr Pro Trp Ala Asp
            165             170             175

Asn Trp Leu Val Asp Thr Gly Asp Ser Glu Pro Gln Ser Gln Gly Leu

EP 2 447 719 B1

                        180                    185                         190

Ser Pro Phe Gly Gln Arg Val Val Lys Glu Leu Asn Arg Leu Gly Val
        195                 200                 205

Leu Ile Asp Leu Ala His Val Ser Val Ala Thr Met Lys Ala Thr Leu
        210                 215                 220

Gln Leu Ser Arg Ala Pro Val Ile Phe Ser His Ser Ser Ala Tyr Ser
225                 230                 235                 240

Val Cys Ala Ser Arg Arg Asn Val Pro Asp Asp Val Leu Arg Leu Val
                245                 250                 255

Lys Gln Thr Asp Ser Leu Val Met Val Asn Phe Tyr Asn Asn Tyr Ile
        260                 265                 270

Ser Cys Thr Asn Lys Ala Asn Leu Ser Gln Val Ala Asp His Leu Asp
        275                 280                 285

His Ile Lys Glu Val Ala Gly Ala Arg Ala Val Gly Phe Gly Gly Asp
        290                 295                 300

Phe Asp Gly Val Pro Arg Val Pro Glu Gly Leu Glu Asp Val Ser Lys
305                 310                 315                 320

Tyr Pro Asp Leu Ile Ala Glu Leu Leu Arg Arg Asn Trp Thr Glu Ala
                325                 330                 335

Glu Val Lys Gly Ala Leu Ala Asp Asn Leu Leu Arg Val Phe Glu Ala
                340                 345                 350

Val Glu Gln Ala Ser Asn Leu Thr Gln Ala Pro Glu Glu Glu Pro Ile
                355                 360                 365

Pro Leu Asp Gln Leu Gly Gly Ser Cys Arg Thr His Tyr Gly Tyr Ser
        370                 375                 380

Ser Gly Ala Ser Ser Leu His Arg His Trp Gly Leu Leu Leu Ala Ser
385                 390                 395                 400

Leu Ala Pro Leu Val Leu Cys Leu Ser Leu Leu
                405                 410

<210> 42
<211> 739
<212> PRT

308

<213> Homo Sapiens

<220>
<221> MISC_FEATURE
<222> (1)..(739)
<223> OGTA236 = Swiss Prot Accession No. P50443, Sulfate transporter

<400> 42

```
Met Ser Ser Glu Ser Lys Glu Gln His Asn Val Ser Pro Arg Asp Ser
1                 5                 10              15

Ala Glu Gly Asn Asp Ser Tyr Pro Ser Gly Ile His Leu Glu Leu Gln
            20              25              30

Arg Glu Ser Ser Thr Asp Phe Lys Gln Phe Glu Thr Asn Asp Gln Cys
        35              40              45

Arg Pro Tyr His Arg Ile Leu Ile Glu Arg Gln Glu Lys Ser Asp Thr
    50              55              60

Asn Phe Lys Glu Phe Val Ile Lys Lys Leu Gln Lys Asn Cys Gln Cys
65              70              75              80

Ser Pro Ala Lys Ala Lys Asn Met Ile Leu Gly Phe Leu Pro Val Leu
            85              90              95

Gln Trp Leu Pro Lys Tyr Asp Leu Lys Lys Asn Ile Leu Gly Asp Val
        100             105             110

Met Ser Gly Leu Ile Val Gly Ile Leu Leu Val Pro Gln Ser Ile Ala
        115             120             125

Tyr Ser Leu Leu Ala Gly Gln Glu Pro Val Tyr Gly Leu Tyr Thr Ser
    130             135             140

Phe Phe Ala Ser Ile Ile Tyr Phe Leu Leu Gly Thr Ser Arg His Ile
145             150             155             160

Ser Val Gly Ile Phe Gly Val Leu Cys Leu Met Ile Gly Glu Thr Val
            165             170             175

Asp Arg Glu Leu Gln Lys Ala Gly Tyr Asp Asn Ala His Ser Ala Pro
        180             185             190

Ser Leu Gly Met Val Ser Asn Gly Ser Thr Leu Leu Asn His Thr Ser
        195             200             205

Asp Arg Ile Cys Asp Lys Ser Cys Tyr Ala Ile Met Val Gly Ser Thr
    210             215             220

Val Thr Phe Ile Ala Gly Val Tyr Gln Val Ala Met Gly Phe Phe Gln
225             230             235             240
```

310

```
Val Gly Phe Val Ser Val Tyr Leu Ser Asp Ala Leu Leu Ser Gly Phe
            245             250                 255

Val Thr Gly Ala Ser Phe Thr Ile Leu Thr Ser Gln Ala Lys Tyr Leu
            260             265                 270

Leu Gly Leu Asn Leu Pro Arg Thr Asn Gly Val Gly Ser Leu Ile Thr
            275             280                 285

Thr Trp Ile His Val Phe Arg Asn Ile His Lys Thr Asn Leu Cys Asp
    290             295                 300

Leu Ile Thr Ser Leu Leu Cys Leu Leu Val Leu Leu Pro Thr Lys Glu
305             310                 315                 320

Leu Asn Glu His Phe Lys Ser Lys Leu Lys Ala Pro Ile Pro Ile Glu
            325             330                 335

Leu Val Val Val Val Ala Ala Thr Leu Ala Ser His Phe Gly Lys Leu
            340             345                 350

His Glu Asn Tyr Asn Ser Ser Ile Ala Gly His Ile Pro Thr Gly Phe
            355             360                 365

Met Pro Pro Lys Val Pro Glu Trp Asn Leu Ile Pro Ser Val Ala Val
    370             375                 380

Asp Ala Ile Ala Ile Ser Ile Ile Gly Phe Ala Ile Thr Val Ser Leu
385             390                 395                 400

Ser Glu Met Phe Ala Lys Lys His Gly Tyr Thr Val Lys Ala Asn Gln
            405             410                 415

Glu Met Tyr Ala Ile Gly Phe Cys Asn Ile Ile Pro Ser Phe Phe His
            420             425                 430

Cys Phe Thr Thr Ser Ala Ala Leu Ala Lys Thr Leu Val Lys Glu Ser
    435             440                 445

Thr Gly Cys His Thr Gln Leu Ser Gly Val Val Thr Ala Leu Val Leu
    450             455                 460

Leu Leu Val Leu Leu Val Ile Ala Pro Leu Phe Tyr Ser Leu Gln Lys
465             470                 475                 480

Ser Val Leu Gly Val Ile Thr Ile Val Asn Leu Arg Gly Ala Leu Arg
            485             490                 495
```

311

```
Lys Phe Arg Asp Leu Pro Lys Met Trp Ser Ile Ser Arg Met Asp Thr
        500             505             510

Val Ile Trp Phe Val Thr Met Leu Ser Ser Ala Leu Leu Ser Thr Glu
        515             520             525

Ile Gly Leu Leu Val Gly Val Cys Phe Ser Ile Phe Cys Val Ile Leu
        530             535             540

Arg Thr Gln Lys Pro Lys Ser Ser Leu Leu Gly Leu Val Glu Glu Ser
545             550             555             560

Glu Val Phe Glu Ser Val Ser Ala Tyr Lys Asn Leu Gln Thr Lys Pro
                565             570             575

Gly Ile Lys Ile Phe Arg Phe Val Ala Pro Leu Tyr Tyr Ile Asn Lys
            580             585             590

Glu Cys Phe Lys Ser Ala Leu Tyr Lys Gln Thr Val Asn Pro Ile Leu
        595             600             605

Ile Lys Val Ala Trp Lys Lys Ala Ala Lys Arg Lys Ile Lys Glu Lys
        610             615             620

Val Val Thr Leu Gly Gly Ile Gln Asp Glu Met Ser Val Gln Leu Ser
625             630             635             640

His Asp Pro Leu Glu Leu His Thr Ile Val Ile Asp Cys Ser Ala Ile
            645             650             655

Gln Phe Leu Asp Thr Ala Gly Ile His Thr Leu Lys Glu Val Arg Arg
        660             665             670

Asp Tyr Glu Ala Ile Gly Ile Gln Val Leu Leu Ala Gln Cys Asn Pro
        675             680             685

Thr Val Arg Asp Ser Leu Thr Asn Gly Glu Tyr Cys Lys Lys Glu Glu
        690             695             700

Glu Asn Leu Leu Phe Tyr Ser Val Tyr Glu Ala Met Ala Phe Ala Glu
705             710             715             720

Val Ser Lys Asn Gln Lys Gly Val Cys Val Pro Asn Gly Leu Ser Leu
            725             730             735

Ser Ser Asp
```

<210> 43
<211> 802
<212> PRT
<213> Homo Sapiens

<220>
<221> MISC_FEATURE
<222> (1)..(802)
<223> OGTA237 = Swiss Prot Accession No. P22455, Fibroblast growth factor receptor 4

<400> 43

```
Met Arg Leu Leu Leu Ala Leu Leu Gly Val Leu Leu Ser Val Pro Gly
1               5               10              15

Pro Pro Val Leu Ser Leu Glu Ala Ser Glu Glu Val Glu Leu Glu Pro
        20              25              30

Cys Leu Ala Pro Ser Leu Glu Gln Gln Glu Gln Glu Leu Thr Val Ala
        35              40              45

Leu Gly Gln Pro Val Arg Leu Cys Cys Gly Arg Ala Glu Arg Gly Gly
    50              55              60

His Trp Tyr Lys Glu Gly Ser Arg Leu Ala Pro Ala Gly Arg Val Arg
65              70              75              80

Gly Trp Arg Gly Arg Leu Glu Ile Ala Ser Phe Leu Pro Glu Asp Ala
            85              90              95

Gly Arg Tyr Leu Cys Leu Ala Arg Gly Ser Met Ile Val Leu Gln Asn
        100             105             110

Leu Thr Leu Ile Thr Gly Asp Ser Leu Thr Ser Ser Asn Asp Asp Glu
        115             120             125

Asp Pro Lys Ser His Arg Asp Pro Ser Asn Arg His Ser Tyr Pro Gln
    130             135             140

Gln Ala Pro Tyr Trp Thr His Pro Gln Arg Met Glu Lys Lys Leu His
145             150             155             160

Ala Val Pro Ala Gly Asn Thr Val Lys Phe Arg Cys Pro Ala Ala Gly
            165             170             175

Asn Pro Thr Pro Thr Ile Arg Trp Leu Lys Asp Gly Gln Ala Phe His
        180             185             190

Gly Glu Asn Arg Ile Gly Gly Ile Arg Leu Arg His Gln His Trp Ser
        195             200             205
```

```
Leu Val Met Glu Ser Val Val Pro Ser Asp Arg Gly Thr Tyr Thr Cys
210             215             220

Leu Val Glu Asn Ala Val Gly Ser Ile Arg Tyr Asn Tyr Leu Leu Asp
225             230             235             240

Val Leu Glu Arg Ser Pro His Arg Pro Ile Leu Gln Ala Gly Leu Pro
            245             250             255

Ala Asn Thr Thr Ala Val Val Gly Ser Asp Val Glu Leu Leu Cys Lys
            260             265             270

Val Tyr Ser Asp Ala Gln Pro His Ile Gln Trp Leu Lys His Ile Val
        275             280             285

Ile Asn Gly Ser Ser Phe Gly Ala Asp Gly Phe Pro Tyr Val Gln Val
        290             295             300

Leu Lys Thr Ala Asp Ile Asn Ser Ser Glu Val Glu Val Leu Tyr Leu
305             310             315             320

Arg Asn Val Ser Ala Glu Asp Ala Gly Glu Tyr Thr Cys Leu Ala Gly
            325             330             335

Asn Ser Ile Gly Leu Ser Tyr Gln Ser Ala Trp Leu Thr Val Leu Pro
            340             345             350

Glu Glu Asp Pro Thr Trp Thr Ala Ala Ala Pro Glu Ala Arg Tyr Thr
            355             360             365

Asp Ile Ile Leu Tyr Ala Ser Gly Ser Leu Ala Leu Ala Val Leu Leu
370             375             380

Leu Leu Ala Gly Leu Tyr Arg Gly Gln Ala Leu His Gly Arg His Pro
385             390             395             400

Arg Pro Pro Ala Thr Val Gln Lys Leu Ser Arg Phe Pro Leu Ala Arg
            405             410             415

Gln Phe Ser Leu Glu Ser Gly Ser Ser Gly Lys Ser Ser Ser Ser Leu
            420             425             430

Val Arg Gly Val Arg Leu Ser Ser Ser Gly Pro Ala Leu Leu Ala Gly
        435             440             445

Leu Val Ser Leu Asp Leu Pro Leu Asp Pro Leu Trp Glu Phe Pro Arg
        450             455             460
```

Asp Arg Leu Val Leu Gly Lys Pro Leu Gly Glu Gly Cys Phe Gly Gln
465            470        475              480

Val Val Arg Ala Glu Ala Phe Gly Met Asp Pro Ala Arg Pro Asp Gln
            485            490            495

Ala Ser Thr Val Ala Val Lys Met Leu Lys Asp Asn Ala Ser Asp Lys
        500            505            510

Asp Leu Ala Asp Leu Val Ser Glu Met Glu Val Met Lys Leu Ile Gly
        515            520            525

Arg His Lys Asn Ile Ile Asn Leu Leu Gly Val Cys Thr Gln Glu Gly
    530            535            540

Pro Leu Tyr Val Ile Val Glu Cys Ala Ala Lys Gly Asn Leu Arg Glu
545            550            555              560

Phe Leu Arg Ala Arg Arg Pro Pro Gly Pro Asp Leu Ser Pro Asp Gly
            565            570            575

Pro Arg Ser Ser Glu Gly Pro Leu Ser Phe Pro Val Leu Val Ser Cys
        580            585            590

Ala Tyr Gln Val Ala Arg Gly Met Gln Tyr Leu Glu Ser Arg Lys Cys
        595            600            605

Ile His Arg Asp Leu Ala Ala Arg Asn Val Leu Val Thr Glu Asp Asn
    610            615            620

Val Met Lys Ile Ala Asp Phe Gly Leu Ala Arg Gly Val His His Ile
625            630            635              640

Asp Tyr Tyr Lys Lys Thr Ser Asn Gly Arg Leu Pro Val Lys Trp Met
            645            650            655

Ala Pro Glu Ala Leu Phe Asp Arg Val Tyr Thr His Gln Ser Asp Val
            660            665            670

Trp Ser Phe Gly Ile Leu Leu Trp Glu Ile Phe Thr Leu Gly Gly Ser
        675            680            685

Pro Tyr Pro Gly Ile Pro Val Glu Glu Leu Phe Ser Leu Leu Arg Glu
    690            695            700

Gly His Arg Met Asp Arg Pro Pro His Cys Pro Pro Glu Leu Tyr Gly
705            710            715              720

Leu Met Arg Glu Cys Trp His Ala Ala Pro Ser Gln Arg Pro Thr Phe

725 730 735

```
Lys Gln Leu Val Glu Ala Leu Asp Lys Val Leu Leu Ala Val Ser Glu
              740                 745              750

Glu Tyr Leu Asp Leu Arg Leu Thr Phe Gly Pro Tyr Ser Pro Ser Gly
         755              760              765

Gly Asp Ala Ser Ser Thr Cys Ser Ser Ser Asp Ser Val Phe Ser His
         770              775              780

Asp Pro Leu Pro Leu Gly Ser Ser Ser Phe Pro Phe Gly Ser Gly Val
785              790              795              800

Gln Thr
```

<210> 44
<211> 1257
<212> PRT
<213> Homo Sapiens

<220>
<221> MISC_FEATURE
<222> (1)..(1257)
<223> OGTA247 = Swiss Prot Accession No. P32004, Neural cell adhesion molecule L1

<400> 44

```
Met Val Val Ala Leu Arg Tyr Val Trp Pro Leu Leu Leu Cys Ser Pro
1             5                 10              15

Cys Leu Leu Ile Gln Ile Pro Glu Glu Tyr Glu Gly His His Val Met
         20              25              30

Glu Pro Pro Val Ile Thr Glu Gln Ser Pro Arg Arg Leu Val Val Phe
         35              40              45

Pro Thr Asp Asp Ile Ser Leu Lys Cys Glu Ala Ser Gly Lys Pro Glu
    50              55              60

Val Gln Phe Arg Trp Thr Arg Asp Gly Val His Phe Lys Pro Lys Glu
65              70              75              80

Glu Leu Gly Val Thr Val Tyr Gln Ser Pro His Ser Gly Ser Phe Thr
             85              90              95

Ile Thr Gly Asn Asn Ser Asn Phe Ala Gln Arg Phe Gln Gly Ile Tyr
             100             105             110
```

```
Arg Cys Phe Ala Ser Asn Lys Leu Gly Thr Ala Met Ser His Glu Ile
    115                 120                 125

Arg Leu Met Ala Glu Gly Ala Pro Lys Trp Pro Lys Glu Thr Val Lys
    130                 135                 140

Pro Val Glu Val Glu Glu Gly Glu Ser Val Val Leu Pro Cys Asn Pro
145                 150                 155                 160

Pro Pro Ser Ala Glu Pro Leu Arg Ile Tyr Trp Met Asn Ser Lys Ile
                165                 170                 175

Leu His Ile Lys Gln Asp Glu Arg Val Thr Met Gly Gln Asn Gly Asn
            180                 185                 190

Leu Tyr Phe Ala Asn Val Leu Thr Ser Asp Asn His Ser Asp Tyr Ile
        195                 200                 205

Cys His Ala His Phe Pro Gly Thr Arg Thr Ile Ile Gln Lys Glu Pro
    210                 215                 220

Ile Asp Leu Arg Val Lys Ala Thr Asn Ser Met Ile Asp Arg Lys Pro
225                 230                 235                 240

Arg Leu Leu Phe Pro Thr Asn Ser Ser Ser His Leu Val Ala Leu Gln
                245                 250                 255

Gly Gln Pro Leu Val Leu Glu Cys Ile Ala Glu Gly Phe Pro Thr Pro
            260                 265                 270

Thr Ile Lys Trp Leu Arg Pro Ser Gly Pro Met Pro Ala Asp Arg Val
        275                 280                 285

Thr Tyr Gln Asn His Asn Lys Thr Leu Gln Leu Leu Lys Val Gly Glu
    290                 295                 300

Glu Asp Asp Gly Glu Tyr Arg Cys Leu Ala Glu Asn Ser Leu Gly Ser
305                 310                 315                 320

Ala Arg His Ala Tyr Tyr Val Thr Val Glu Ala Ala Pro Tyr Trp Leu
                325                 330                 335

His Lys Pro Gln Ser His Leu Tyr Gly Pro Gly Glu Thr Ala Arg Leu
            340                 345                 350

Asp Cys Gln Val Gln Gly Arg Pro Gln Pro Glu Val Thr Trp Arg Ile
        355                 360                 365

Asn Gly Ile Pro Val Glu Glu Leu Ala Lys Asp Gln Lys Tyr Arg Ile
```

318

|     |     |     |     | 370 |     |     |     |     |     | 375 |     |     |     |     |     | 380 |
| --- | --- | --- | --- | --- | --- | --- | --- | --- | --- | --- | --- | --- | --- | --- | --- | --- |

Gln Arg Gly Ala Leu Ile Leu Ser Asn Val Gln Pro Ser Asp Thr Met
385           390               395               400

Val Thr Gln Cys Glu Ala Arg Asn Arg His Gly Leu Leu Leu Ala Asn
          405               410               415

Ala Tyr Ile Tyr Val Val Gln Leu Pro Ala Lys Ile Leu Thr Ala Asp
          420               425               430

Asn Gln Thr Tyr Met Ala Val Gln Gly Ser Thr Ala Tyr Leu Leu Cys
          435               440               445

Lys Ala Phe Gly Ala Pro Val Pro Ser Val Gln Trp Leu Asp Glu Asp
    450               455               460

Gly Thr Thr Val Leu Gln Asp Glu Arg Phe Phe Pro Tyr Ala Asn Gly
465               470               475               480

Thr Leu Gly Ile Arg Asp Leu Gln Ala Asn Asp Thr Gly Arg Tyr Phe
              485               490               495

Cys Leu Ala Ala Asn Asp Gln Asn Asn Val Thr Ile Met Ala Asn Leu
          500               505               510

Lys Val Lys Asp Ala Thr Gln Ile Thr Gln Gly Pro Arg Ser Thr Ile
          515               520               525

Glu Lys Lys Gly Ser Arg Val Thr Phe Thr Cys Gln Ala Ser Phe Asp
    530               535               540

Pro Ser Leu Gln Pro Ser Ile Thr Trp Arg Gly Asp Gly Arg Asp Leu
545               550               555               560

Gln Glu Leu Gly Asp Ser Asp Lys Tyr Phe Ile Glu Asp Gly Arg Leu
              565               570               575

Val Ile His Ser Leu Asp Tyr Ser Asp Gln Gly Asn Tyr Ser Cys Val
              580               585               590

Ala Ser Thr Glu Leu Asp Val Val Glu Ser Arg Ala Gln Leu Leu Val
          595               600               605

Val Gly Ser Pro Gly Pro Val Pro Arg Leu Val Leu Ser Asp Leu His
    610               615               620

Leu Leu Thr Gln Ser Gln Val Arg Val Ser Trp Ser Pro Ala Glu Asp
625               630               635               640

His Asn Ala Pro Ile Glu Lys Tyr Asp Ile Glu Phe Glu Asp Lys Glu
645 650 655

Met Ala Pro Glu Lys Trp Tyr Ser Leu Gly Lys Val Pro Gly Asn Gln
660 665 670

Thr Ser Thr Thr Leu Lys Leu Ser Pro Tyr Val His Tyr Thr Phe Arg
675 680 685

Val Thr Ala Ile Asn Lys Tyr Gly Pro Gly Glu Pro Ser Pro Val Ser
690 695 700

Glu Thr Val Val Thr Pro Glu Ala Ala Pro Glu Lys Asn Pro Val Asp
705 710 715 720

Val Lys Gly Glu Gly Asn Glu Thr Thr Asn Met Val Ile Thr Trp Lys
725 730 735

Pro Leu Arg Trp Met Asp Trp Asn Ala Pro Gln Val Gln Tyr Arg Val
740 745 750

Gln Trp Arg Pro Gln Gly Thr Arg Gly Pro Trp Gln Glu Gln Ile Val
755 760 765

Ser Asp Pro Phe Leu Val Val Ser Asn Thr Ser Thr Phe Val Pro Tyr
770 775 780

Glu Ile Lys Val Gln Ala Val Asn Ser Gln Gly Lys Gly Pro Glu Pro
785 790 795 800

Gln Val Thr Ile Gly Tyr Ser Gly Glu Asp Tyr Pro Gln Ala Ile Pro
805 810 815

Glu Leu Glu Gly Ile Glu Ile Leu Asn Ser Ser Ala Val Leu Val Lys
820 825 830

Trp Arg Pro Val Asp Leu Ala Gln Val Lys Gly His Leu Arg Gly Tyr
835 840 845

Asn Val Thr Tyr Trp Arg Glu Gly Ser Gln Arg Lys His Ser Lys Arg
850 855 860

His Ile His Lys Asp His Val Val Val Pro Ala Asn Thr Thr Ser Val
865 870 875 880

Ile Leu Ser Gly Leu Arg Pro Tyr Ser Ser Tyr His Leu Glu Val Gln
885 890 895

320

```
Ala Phe Asn Gly Arg Gly Ser Gly Pro Ala Ser Glu Phe Thr Phe Ser
        900                 905                 910

Thr Pro Glu Gly Val Pro Gly His Pro Glu Ala Leu His Leu Glu Cys
        915                 920                 925

Gln Ser Asn Thr Ser Leu Leu Leu Arg Trp Gln Pro Pro Leu Ser His
        930                 935                 940

Asn Gly Val Leu Thr Gly Tyr Val Leu Ser Tyr His Pro Leu Asp Glu
945                 950                 955                 960

Gly Gly Lys Gly Gln Leu Ser Phe Asn Leu Arg Asp Pro Glu Leu Arg
            965                 970                 975

Thr His Asn Leu Thr Asp Leu Ser Pro His Leu Arg Tyr Arg Phe Gln
        980                 985                 990

Leu Gln Ala Thr Thr Lys Glu Gly Pro Gly Glu Ala Ile  Val Arg Glu
        995                 1000                1005

Gly Gly  Thr Met Ala Leu Ser  Gly Ile Ser Asp Phe  Gly Asn Ile
    1010                1015                1020

Ser Ala  Thr Ala Gly Glu Asn  Tyr Ser Val Val Ser  Trp Val Pro
    1025                1030                1035

Lys Glu  Gly Gln Cys Asn Phe  Arg Phe His Ile Leu  Phe Lys Ala
    1040                1045                1050

Leu Gly  Glu Glu Lys Gly Gly  Ala Ser Leu Ser Pro  Gln Tyr Val
    1055                1060                1065

Ser Tyr  Asn Gln Ser Ser Tyr  Thr Gln Trp Asp Leu  Gln Pro Asp
    1070                1075                1080

Thr Asp  Tyr Glu Ile His Leu  Phe Lys Glu Arg Met  Phe Arg His
    1085                1090                1095

Gln Met  Ala Val Lys Thr Asn  Gly Thr Gly Arg Val  Arg Leu Pro
    1100                1105                1110

Pro Ala  Gly Phe Ala Thr Glu  Gly Trp Phe Ile Gly  Phe Val Ser
    1115                1120                1125

Ala Ile  Ile Leu Leu Leu Leu  Val Leu Leu Ile Leu  Cys Phe Ile
    1130                1135                1140
```

```
Lys Arg Ser Lys Gly Gly Lys Tyr Ser Val Lys Asp Lys Glu Asp
    1145                1150            1155

Thr Gln Val Asp Ser Glu Ala Arg Pro Met Lys Asp Glu Thr Phe
    1160                1165            1170

Gly Glu Tyr Arg Ser Leu Glu Ser Asp Asn Glu Glu Lys Ala Phe
    1175                1180            1185

Gly Ser Ser Gln Pro Ser Leu Asn Gly Asp Ile Lys Pro Leu Gly
    1190                1195            1200

Ser Asp Asp Ser Leu Ala Asp Tyr Gly Gly Ser Val Asp Val Gln
    1205                1210            1215

Phe Asn Glu Asp Gly Ser Phe Ile Gly Gln Tyr Ser Gly Lys Lys
    1220                1225            1230

Glu Lys Glu Ala Ala Gly Gly Asn Asp Ser Ser Gly Ala Thr Ser
    1235                1240            1245

Pro Ile Asn Pro Ala Val Ala Leu Glu
    1250                1255
```

<210> 45
<211> 864
<212> PRT
<213> Homo Sapiens

<220>
<221> MISC_FEATURE
<222> (1)..(864)
<223> OGTA248 = Swiss Prot Accession No. P29376, Leukocyte tyrosine kinase receptor

<400> 45

```
Met Gly Cys Trp Gly Gln Leu Leu Val Trp Phe Gly Ala Ala Gly Ala
1                   5                   10                  15

Ile Leu Cys Ser Ser Pro Gly Ser Gln Glu Thr Phe Leu Arg Ser Ser
            20                  25                  30

Pro Leu Pro Leu Ala Ser Pro Ser Pro Gln Asp Pro Lys Val Ser Ala
            35                  40                  45

Pro Pro Ser Ile Leu Glu Pro Ala Ser Pro Leu Asn Ser Pro Gly Thr
        50                  55                  60

Glu Gly Ser Trp Leu Phe Ser Thr Cys Gly Ala Ser Gly Arg His Gly
65                  70                  75                  80
```

```
Pro Thr Gln Thr Gln Cys Asp Gly Ala Tyr Ala Gly Thr Ser Val Val
            85                  90                  95

Val Thr Val Gly Ala Ala Gly Gln Leu Arg Gly Val Gln Leu Trp Arg
            100                 105                 110

Val Pro Gly Pro Gly Gln Tyr Leu Ile Ser Ala Tyr Gly Ala Ala Gly
            115                 120                 125

Gly Lys Gly Ala Lys Asn His Leu Ser Arg Ala His Gly Val Phe Val
            130                 135                 140

Ser Ala Ile Phe Ser Leu Gly Leu Gly Glu Ser Leu Tyr Ile Leu Val
145                 150                 155                 160

Gly Gln Gln Gly Glu Asp Ala Cys Pro Gly Gly Ser Pro Glu Ser Gln
            165                 170                 175

Leu Val Cys Leu Gly Glu Ser Arg Ala Val Glu Glu His Ala Ala Met
            180                 185                 190

Asp Gly Ser Glu Gly Val Pro Gly Ser Arg Arg Trp Ala Gly Gly Gly
            195                 200                 205

Gly Gly Gly Gly Gly Ala Thr Tyr Val Phe Arg Val Arg Ala Gly Glu
            210                 215                 220

Leu Glu Pro Leu Leu Val Ala Ala Gly Gly Gly Gly Arg Ala Tyr Leu
225                 230                 235                 240

Arg Pro Arg Asp Arg Gly Arg Thr Gln Ala Ser Pro Glu Lys Leu Glu
            245                 250                 255

Asn Arg Ser Glu Ala Pro Gly Ser Gly Gly Arg Gly Gly Ala Ala Gly
            260                 265                 270

Gly Gly Gly Gly Trp Thr Ser Arg Ala Pro Ser Pro Gln Ala Gly Arg
            275                 280                 285

Ser Leu Gln Glu Gly Ala Glu Gly Gly Gln Gly Cys Ser Glu Ala Trp
            290                 295                 300

Ala Thr Leu Gly Trp Ala Ala Ala Gly Gly Phe Gly Gly Gly Gly Gly
305                 310                 315                 320

Ala Cys Thr Ala Gly Gly Gly Gly Gly Gly Tyr Arg Gly Gly Asp Ala
            325                 330                 335
```

324

```
Ser Glu Thr Asp Asn Leu Trp Ala Asp Gly Glu Asp Gly Val Ser Phe
            340             345             350

Ile His Pro Ser Ser Glu Leu Phe Leu Gln Pro Leu Ala Val Thr Glu
        355             360             365

Asn His Gly Glu Val Glu Ile Arg Arg His Leu Asn Cys Ser His Cys
    370             375             380

Pro Leu Arg Asp Cys Gln Trp Gln Ala Glu Leu Gln Leu Ala Glu Cys
385             390             395             400

Leu Cys Pro Glu Gly Met Glu Leu Ala Val Asp Asn Val Thr Cys Met
            405             410             415

Asp Leu His Lys Pro Pro Gly Pro Leu Val Leu Met Val Ala Val Val
            420             425             430

Ala Thr Ser Thr Leu Ser Leu Leu Met Val Cys Gly Val Leu Ile Leu
        435             440             445

Val Lys Gln Lys Lys Trp Gln Gly Leu Gln Glu Met Arg Leu Pro Ser
    450             455             460

Pro Glu Leu Glu Leu Ser Lys Leu Arg Thr Ser Ala Ile Arg Thr Ala
465             470             475             480

Pro Asn Pro Tyr Tyr Cys Gln Val Gly Leu Gly Pro Ala Gln Ser Trp
            485             490             495

Pro Leu Pro Pro Gly Val Thr Glu Val Ser Pro Ala Asn Val Thr Leu
        500             505             510

Leu Arg Ala Leu Gly His Gly Ala Phe Gly Glu Val Tyr Glu Gly Leu
        515             520             525

Val Ile Gly Leu Pro Gly Asp Ser Ser Pro Leu Gln Val Ala Ile Lys
    530             535             540

Thr Leu Pro Glu Leu Cys Ser Pro Gln Asp Glu Leu Asp Phe Leu Met
545             550             555             560

Glu Ala Leu Ile Ile Ser Lys Phe Arg His Gln Asn Ile Val Arg Cys
            565             570             575

Val Gly Leu Ser Leu Arg Ala Thr Pro Arg Leu Ile Leu Leu Glu Leu
            580             585             590

Met Ser Gly Gly Asp Met Lys Ser Phe Leu Arg His Ser Arg Pro His
```

595 600 605

Leu Gly Gln Pro Ser Pro Leu Val Met Arg Asp Leu Leu Gln Leu Ala
610 615 620

Gln Asp Ile Ala Gln Gly Cys His Tyr Leu Glu Glu Asn His Phe Ile
625 630 635 640

His Arg Asp Ile Ala Ala Arg Asn Cys Leu Leu Ser Cys Ala Gly Pro
645 650 655

Ser Arg Val Ala Lys Ile Gly Asp Phe Gly Met Ala Arg Asp Ile Tyr
660 665 670

Arg Ala Ser Tyr Tyr Arg Arg Gly Asp Arg Ala Leu Leu Pro Val Lys
675 680 685

Trp Met Pro Pro Glu Ala Phe Leu Glu Gly Ile Phe Thr Ser Lys Thr
690 695 700

Asp Ser Trp Ser Phe Gly Val Leu Leu Trp Glu Ile Phe Ser Leu Gly
705 710 715 720

Tyr Met Pro Tyr Pro Gly Arg Thr Asn Gln Glu Val Leu Asp Phe Val
725 730 735

Val Gly Gly Gly Arg Met Asp Pro Pro Arg Gly Cys Pro Gly Pro Val
740 745 750

Tyr Arg Ile Met Thr Gln Cys Trp Gln His Glu Pro Glu Leu Arg Pro
755 760 765

Ser Phe Ala Ser Ile Leu Glu Arg Leu Gln Tyr Cys Thr Gln Asp Pro
770 775 780

Asp Val Leu Asn Ser Leu Leu Pro Met Glu Leu Gly Pro Thr Pro Glu
785 790 795 800

Glu Glu Gly Thr Ser Gly Leu Gly Asn Arg Ser Leu Glu Cys Leu Arg
805 810 815

Pro Pro Gln Pro Gln Glu Leu Ser Pro Glu Lys Leu Lys Ser Trp Gly
820 825 830

Gly Ser Pro Leu Gly Pro Trp Leu Ser Ser Gly Leu Lys Pro Leu Lys
835 840 845

Ser Arg Gly Leu Gln Pro Gln Asn Leu Trp Asn Pro Thr Tyr Arg Ser
850 855 860

326

<210> 46
<211> 176
<212> PRT
<213> Homo Sapiens

<220>
<221> MISC_FEATURE
<222> (1)..(176)
<223> OGTA249 = Swiss Prot Accession No. Q969L2, Protein MAL2

<400> 46

```
Met Ser Ala Gly Gly Ala Ser Val Pro Pro Pro Asn Pro Ala Val
1               5               10              15

Ser Phe Pro Pro Pro Arg Val Thr Leu Pro Ala Gly Pro Asp Ile Leu
        20              25              30

Arg Thr Tyr Ser Gly Ala Phe Val Cys Leu Glu Ile Leu Phe Gly Gly
        35              40              45

Leu Val Trp Ile Leu Val Ala Ser Ser Asn Val Pro Leu Pro Leu Leu
    50              55              60

Gln Gly Trp Val Met Phe Val Ser Val Thr Ala Phe Phe Phe Ser Leu
65              70              75              80

Leu Phe Leu Gly Met Phe Leu Ser Gly Met Val Ala Gln Ile Asp Ala
            85              90              95

Asn Trp Asn Phe Leu Asp Phe Ala Tyr His Phe Thr Val Phe Val Phe
            100             105             110

Tyr Phe Gly Ala Phe Leu Leu Glu Ala Ala Ala Thr Ser Leu His Asp
        115             120             125

Leu His Cys Asn Thr Thr Ile Thr Gly Gln Pro Leu Leu Ser Asp Asn
    130             135             140

Gln Tyr Asn Ile Asn Val Ala Ala Ser Ile Phe Ala Phe Met Thr Thr
145             150             155             160

Ala Cys Tyr Gly Cys Ser Leu Gly Leu Ala Leu Arg Arg Trp Arg Pro
                165             170             175
```

<210> 47
<211> 725
<212> PRT
<213> Homo Sapiens

<220>
<221> MISC_FEATURE
<222> (1)..(725)
<223> OGTA257 = Swiss Prot Accession No. Q9H1D0, Transient receptor potential cation channel subfamily V member 6

<400> 47

```
Met Gly Leu Ser Leu Pro Lys Glu Lys Gly Leu Ile Leu Cys Leu Trp
1               5               10              15

Ser Lys Phe Cys Arg Trp Phe Gln Arg Arg Glu Ser Trp Ala Gln Ser
        20              25              30

Arg Asp Glu Gln Asn Leu Leu Gln Gln Lys Arg Ile Trp Glu Ser Pro
        35              40              45

Leu Leu Leu Ala Ala Lys Asp Asn Asp Val Gln Ala Leu Asn Lys Leu
    50              55              60

Leu Lys Tyr Glu Asp Cys Lys Val His Gln Arg Gly Ala Met Gly Glu
65              70              75              80

Thr Ala Leu His Ile Ala Ala Leu Tyr Asp Asn Leu Glu Ala Ala Met
            85              90              95

Val Leu Met Glu Ala Ala Pro Glu Leu Val Phe Glu Pro Met Thr Ser
            100             105             110

Glu Leu Tyr Glu Gly Gln Thr Ala Leu His Ile Ala Val Val Asn Gln
        115             120             125

Asn Met Asn Leu Val Arg Ala Leu Leu Ala Arg Arg Ala Ser Val Ser
    130             135             140

Ala Arg Ala Thr Gly Thr Ala Phe Arg Arg Ser Pro Cys Asn Leu Ile
145             150             155             160

Tyr Phe Gly Glu His Pro Leu Ser Phe Ala Ala Cys Val Asn Ser Glu
            165             170             175

Glu Ile Val Arg Leu Leu Ile Glu His Gly Ala Asp Ile Arg Ala Gln
        180             185             190

Asp Ser Leu Gly Asn Thr Val Leu His Ile Leu Ile Leu Gln Pro Asn
        195             200             205

Lys Thr Phe Ala Cys Gln Met Tyr Asn Leu Leu Leu Ser Tyr Asp Arg
    210             215             220

His Gly Asp His Leu Gln Pro Leu Asp Leu Val Pro Asn His Gln Gly
```

225 230 235 240

Leu Thr Pro Phe Lys Leu Ala Gly Val Glu Gly Asn Thr Val Met Phe
245 250 255

Gln His Leu Met Gln Lys Arg Lys His Thr Gln Trp Thr Tyr Gly Pro
260 265 270

Leu Thr Ser Thr Leu Tyr Asp Leu Thr Glu Ile Asp Ser Ser Gly Asp
275 280 285

Glu Gln Ser Leu Leu Glu Leu Ile Ile Thr Thr Lys Lys Arg Glu Ala
290 295 300

Arg Gln Ile Leu Asp Gln Thr Pro Val Lys Glu Leu Val Ser Leu Lys
305 310 315 320

Trp Lys Arg Tyr Gly Arg Pro Tyr Phe Cys Met Leu Gly Ala Ile Tyr
325 330 335

Leu Leu Tyr Ile Ile Cys Phe Thr Met Cys Cys Ile Tyr Arg Pro Leu
340 345 350

Lys Pro Arg Thr Asn Asn Arg Thr Ser Pro Arg Asp Asn Thr Leu Leu
355 360 365

Gln Gln Lys Leu Leu Gln Glu Ala Tyr Met Thr Pro Lys Asp Asp Ile
370 375 380

Arg Leu Val Gly Glu Leu Val Thr Val Ile Gly Ala Ile Ile Ile Leu
385 390 395 400

Leu Val Glu Val Pro Asp Ile Phe Arg Met Gly Val Thr Arg Phe Phe
405 410 415

Gly Gln Thr Ile Leu Gly Gly Pro Phe His Val Leu Ile Ile Thr Tyr
420 425 430

Ala Phe Met Val Leu Val Thr Met Val Met Arg Leu Ile Ser Ala Ser
435 440 445

Gly Glu Val Val Pro Met Ser Phe Ala Leu Val Leu Gly Trp Cys Asn
450 455 460

Val Met Tyr Phe Ala Arg Gly Phe Gln Met Leu Gly Pro Phe Thr Ile
465 470 475 480

Met Ile Gln Lys Met Ile Phe Gly Asp Leu Met Arg Phe Cys Trp Leu
485 490 495

```
Met Ala Val Val Ile Leu Gly Phe Ala Ser Ala Phe Tyr Ile Ile Phe
            500                 505             510

Gln Thr Glu Asp Pro Glu Glu Leu Gly His Phe Tyr Asp Tyr Pro Met
            515                 520             525

Ala Leu Phe Ser Thr Phe Glu Leu Phe Leu Thr Ile Ile Asp Gly Pro
            530                 535             540

Ala Asn Tyr Asn Val Asp Leu Pro Phe Met Tyr Ser Ile Thr Tyr Ala
545                 550                 555                 560

Ala Phe Ala Ile Ile Ala Thr Leu Leu Met Leu Asn Leu Leu Ile Ala
            565                 570             575

Met Met Gly Asp Thr His Trp Arg Val Ala His Glu Arg Asp Glu Leu
            580                 585             590

Trp Arg Ala Gln Ile Val Ala Thr Thr Val Met Leu Glu Arg Lys Leu
            595                 600             605

Pro Arg Cys Leu Trp Pro Arg Ser Gly Ile Cys Gly Arg Glu Tyr Gly
            610                 615             620

Leu Gly Asp Arg Trp Phe Leu Arg Val Glu Asp Arg Gln Asp Leu Asn
625                 630                 635                 640

Arg Gln Arg Ile Gln Arg Tyr Ala Gln Ala Phe His Thr Arg Gly Ser
            645                 650             655

Glu Asp Leu Asp Lys Asp Ser Val Glu Lys Leu Glu Leu Gly Cys Pro
            660                 665             670

Phe Ser Pro His Leu Ser Leu Pro Met Pro Ser Val Ser Arg Ser Thr
            675                 680             685

Ser Arg Ser Ser Ala Asn Trp Glu Arg Leu Arg Gln Gly Thr Leu Arg
            690                 695             700

Arg Asp Leu Arg Gly Ile Ile Asn Arg Gly Leu Glu Asp Gly Glu Ser
705                 710                 715                 720

Trp Glu Tyr Gln Ile
                    725
```

<210> 48
<211> 1898

331

<212> PRT
<213> Homo Sapiens

<220>
<221> MISC_FEATURE
<222> (1)..(1898)
<223> OGTA271 = Swiss Prot Accession No. Q5T021, Protein tyrosine phosphatase, receptor type, F

<400> 48

Met Ala Pro Glu Pro Ala Pro Gly Arg Thr Met Val Pro Leu Val Pro
1                   5                   10                  15

Ala Leu Val Met Leu Gly Leu Val Ala Gly Ala His Gly Asp Ser Lys
            20                  25                  30

Pro Val Phe Ile Lys Val Pro Glu Asp Gln Thr Gly Leu Ser Gly Gly
            35                  40                  45

Val Ala Ser Phe Val Cys Gln Ala Thr Gly Glu Pro Lys Pro Arg Ile
    50                  55                  60

Thr Trp Met Lys Lys Gly Lys Lys Val Ser Ser Gln Arg Phe Glu Val
65                  70                  75                  80

Ile Glu Phe Asp Asp Gly Ala Gly Ser Val Leu Arg Ile Gln Pro Leu
            85                  90                  95

Arg Val Gln Arg Asp Glu Ala Ile Tyr Glu Cys Thr Ala Thr Asn Ser
            100                 105                 110

Leu Gly Glu Ile Asn Thr Ser Ala Lys Leu Ser Val Leu Glu Glu Glu
            115                 120                 125

Gln Leu Pro Pro Gly Phe Pro Ser Ile Asp Met Gly Pro Gln Leu Lys
    130                 135                 140

Val Val Glu Lys Ala Arg Thr Ala Thr Met Leu Cys Ala Ala Gly Gly
145                 150                 155                 160

Asn Pro Asp Pro Glu Ile Ser Trp Phe Lys Asp Phe Leu Pro Val Asp
            165                 170                 175

Pro Ala Thr Ser Asn Gly Arg Ile Lys Gln Leu Arg Ser Gly Ala Leu
            180                 185                 190

Gln Ile Glu Ser Ser Glu Glu Ser Asp Gln Gly Lys Tyr Glu Cys Val
    195                 200                 205

Ala Thr Asn Ser Ala Gly Thr Arg Tyr Ser Ala Pro Ala Asn Leu Tyr
    210                 215                 220

Val Arg Val Arg Arg Val Ala Pro Arg Phe Ser Ile Pro Pro Ser Ser
225             230             235             240

Gln Glu Val Met Pro Gly Gly Ser Val Asn Leu Thr Cys Val Ala Val
            245             250             255

Gly Ala Pro Met Pro Tyr Val Lys Trp Met Met Gly Ala Glu Glu Leu
            260             265             270

Thr Lys Glu Asp Glu Met Pro Val Gly Arg Asn Val Leu Glu Leu Ser
            275             280             285

Asn Val Val Arg Ser Ala Asn Tyr Thr Cys Val Ala Ile Ser Ser Leu
            290             295             300

Gly Met Ile Glu Ala Thr Ala Gln Val Thr Val Lys Ala Leu Pro Lys
305             310             315             320

Pro Pro Ile Asp Leu Val Val Thr Glu Thr Thr Ala Thr Ser Val Thr
            325             330             335

Leu Thr Trp Asp Ser Gly Asn Ser Glu Pro Val Thr Tyr Tyr Gly Ile
            340             345             350

Gln Tyr Arg Ala Ala Gly Thr Glu Gly Pro Phe Gln Glu Val Asp Gly
            355             360             365

Val Ala Thr Thr Arg Tyr Ser Ile Gly Gly Leu Ser Pro Phe Ser Glu
            370             375             380

Tyr Ala Phe Arg Val Leu Ala Val Asn Ser Ile Gly Arg Gly Pro Pro
385             390             395             400

Ser Glu Ala Val Arg Ala Arg Thr Gly Glu Gln Ala Pro Ser Ser Pro
            405             410             415

Pro Arg Arg Val Gln Ala Arg Met Leu Ser Ala Ser Thr Met Leu Val
            420             425             430

Gln Trp Glu Pro Pro Glu Glu Pro Asn Gly Leu Val Arg Gly Tyr Arg
            435             440             445

Val Tyr Tyr Thr Pro Asp Ser Arg Arg Pro Pro Asn Ala Trp His Lys
            450             455             460

His Asn Thr Asp Ala Gly Leu Leu Thr Thr Val Gly Ser Leu Leu Pro
465             470             475             480

334

Gly Ile Thr Tyr Ser Leu Arg Val Leu Ala Phe Thr Ala Val Gly Asp
                485              490              495

Gly Pro Pro Ser Pro Thr Ile Gln Val Lys Thr Gln Gln Gly Val Pro
                500              505              510

Ala Gln Pro Ala Asp Phe Gln Ala Glu Val Glu Ser Asp Thr Arg Ile
        515              520              525

Gln Leu Ser Trp Leu Leu Pro Pro Gln Glu Arg Ile Ile Met Tyr Glu
    530              535              540

Leu Val Tyr Trp Ala Ala Glu Asp Glu Asp Gln Gln His Lys Val Thr
545              550              555              560

Phe Asp Pro Thr Ser Ser Tyr Thr Leu Glu Asp Leu Lys Pro Asp Thr
                565              570              575

Leu Tyr Arg Phe Gln Leu Ala Ala Arg Ser Asp Met Gly Val Gly Val
        580              585              590

Phe Thr Pro Thr Ile Glu Ala Arg Thr Ala Gln Ser Thr Pro Ser Ala
        595              600              605

Pro Pro Gln Lys Val Met Cys Val Ser Met Gly Ser Thr Thr Val Arg
    610              615              620

Val Ser Trp Val Pro Pro Pro Ala Asp Ser Arg Asn Gly Val Ile Thr
625              630              635              640

Gln Tyr Ser Val Ala Tyr Glu Ala Val Asp Gly Glu Asp Arg Gly Arg
                645              650              655

His Val Val Asp Gly Ile Ser Arg Glu His Ser Ser Trp Asp Leu Val
                660              665              670

Gly Leu Glu Lys Trp Thr Glu Tyr Arg Val Trp Val Arg Ala His Thr
    675              680              685

Asp Val Gly Pro Gly Pro Glu Ser Ser Pro Val Leu Val Arg Thr Asp
    690              695              700

Glu Asp Val Pro Ser Gly Pro Pro Arg Lys Val Glu Val Glu Pro Leu
705              710              715              720

Asn Ser Thr Ala Val His Val Tyr Trp Lys Leu Pro Val Pro Ser Lys
                725              730              735

```
Gln His Gly Gln Ile Arg Gly Tyr Gln Val Thr Tyr Val Arg Leu Glu
            740                 745             750

Asn Gly Glu Pro Arg Gly Leu Pro Ile Ile Gln Asp Val Met Leu Ala
            755             760             765

Glu Ala Gln Glu Thr Thr Ile Ser Gly Leu Thr Pro Glu Thr Thr Tyr
    770             775             780

Ser Val Thr Val Ala Ala Tyr Thr Thr Lys Gly Asp Gly Ala Arg Ser
785             790             795             800

Lys Pro Lys Ile Val Thr Thr Thr Gly Ala Val Pro Gly Arg Pro Thr
            805             810             815

Met Met Ile Ser Thr Thr Ala Met Asn Thr Ala Leu Leu Gln Trp His
            820             825             830

Pro Pro Lys Glu Leu Pro Gly Glu Leu Leu Gly Tyr Arg Leu Gln Tyr
            835             840             845

Cys Arg Ala Asp Glu Ala Arg Pro Asn Thr Ile Asp Phe Gly Lys Asp
    850             855             860

Asp Gln His Phe Thr Val Thr Gly Leu His Lys Gly Thr Thr Tyr Ile
865             870             875             880

Phe Arg Leu Ala Ala Lys Asn Arg Ala Gly Leu Gly Glu Glu Phe Glu
            885             890             895

Lys Glu Ile Arg Thr Pro Glu Asp Leu Pro Ser Gly Phe Pro Gln Asn
            900             905             910

Leu His Val Thr Gly Leu Thr Thr Ser Thr Thr Glu Leu Ala Trp Asp
            915             920             925

Pro Pro Val Leu Ala Glu Arg Asn Gly Arg Ile Ile Ser Tyr Thr Val
    930             935             940

Val Phe Arg Asp Ile Asn Ser Gln Gln Glu Leu Gln Asn Ile Thr Thr
945             950             955             960

Asp Thr Arg Phe Thr Leu Thr Gly Leu Lys Pro Asp Thr Thr Tyr Asp
            965             970             975

Ile Lys Val Arg Ala Trp Thr Ser Lys Gly Ser Gly Pro Leu Ser Pro
            980             985             990

Ser Ile Gln Ser Arg Thr Met Pro  Val Glu Gln Val Phe  Ala Lys Asn
```

336

995             1000            1005

```
Phe Arg  Val Ala Ala Ala Met  Lys Thr Ser Val Leu  Leu Ser Trp
    1010             1015             1020

Glu Val  Pro Asp Ser Tyr Lys  Ser Ala Val Pro Phe  Lys Ile Leu
    1025             1030             1035

Tyr Asn  Gly Gln Ser Val Glu  Val Asp Gly His Ser  Met Arg Lys
    1040             1045             1050

Leu Ile  Ala Asp Leu Gln Pro  Asn Thr Glu Tyr Ser  Phe Val Leu
    1055             1060             1065

Met Asn  Arg Gly Ser Ser Ala  Gly Gly Leu Gln His  Leu Val Ser
    1070             1075             1080

Ile Arg  Thr Ala Pro Asp Leu  Leu Pro His Lys Pro  Leu Pro Ala
    1085             1090             1095

Ser Ala  Tyr Ile Glu Asp Gly  Arg Phe Asp Leu Ser  Met Pro His
    1100             1105             1110

Val Gln  Asp Pro Ser Leu Val  Arg Trp Phe Tyr Ile  Val Val Val
    1115             1120             1125

Pro Ile  Asp Arg Val Gly Gly  Ser Met Leu Thr Pro  Arg Trp Ser
    1130             1135             1140

Thr Pro  Glu Glu Leu Glu Leu  Asp Glu Leu Leu Glu  Ala Ile Glu
    1145             1150             1155

Gln Gly  Gly Glu Glu Gln Arg  Arg Arg Arg Gln  Ala Glu Arg
    1160             1165             1170

Leu Lys  Pro Tyr Val Ala Ala  Gln Leu Asp Val Leu  Pro Glu Thr
    1175             1180             1185

Phe Thr  Leu Gly Asp Lys Lys  Asn Tyr Arg Gly Phe  Tyr Asn Arg
    1190             1195             1200

Pro Leu  Ser Pro Asp Leu Ser  Tyr Gln Cys Phe Val  Leu Ala Ser
    1205             1210             1215

Leu Lys  Glu Pro Met Asp Gln  Lys Arg Tyr Ala Ser  Ser Pro Tyr
    1220             1225             1230

Ser Asp  Glu Ile Val Val Gln  Val Thr Pro Ala Gln  Gln Gln Glu
    1235             1240             1245
```

```
Glu Pro Glu Met Leu Trp Val  Thr Gly Pro Val Leu  Ala Val Ile
    1250             1255              1260


Leu Ile Ile Leu Ile Val Ile  Ala Ile Leu Leu Phe  Lys Arg Lys
    1265             1270              1275


Arg Thr His Ser Pro Ser Ser  Lys Asp Glu Gln Ser  Ile Gly Leu
    1280             1285              1290


Lys Asp Ser Leu Leu Ala His  Ser Ser Asp Pro Val  Glu Met Arg
    1295             1300              1305


Arg Leu Asn Tyr Gln Thr Pro  Gly Met Arg Asp His  Pro Pro Ile
    1310             1315              1320


Pro Ile Thr Asp Leu Ala Asp  Asn Ile Glu Arg Leu  Lys Ala Asn
    1325             1330              1335


Asp Gly Leu Lys Phe Ser Gln  Glu Tyr Glu Ser Ile  Asp Pro Gly
    1340             1345              1350


Gln Gln Phe Thr Trp Glu Asn  Ser Asn Leu Glu Val  Asn Lys Pro
    1355             1360              1365


Lys Asn Arg Tyr Ala Asn Val  Ile Ala Tyr Asp His  Ser Arg Val
    1370             1375              1380


Ile Leu Thr Ser Ile Asp Gly  Val Pro Gly Ser Asp  Tyr Ile Asn
    1385             1390              1395


Ala Asn Tyr Ile Asp Gly Tyr  Arg Lys Gln Asn Ala  Tyr Ile Ala
    1400             1405              1410


Thr Gln Gly Pro Leu Pro Glu  Thr Met Gly Asp Phe  Trp Arg Met
    1415             1420              1425


Val Trp Glu Gln Arg Thr Ala  Thr Val Val Met Met  Thr Arg Leu
    1430             1435              1440


Glu Glu Lys Ser Arg Val Lys  Cys Asp Gln Tyr Trp  Pro Ala Arg
    1445             1450              1455


Gly Thr Glu Thr Cys Gly Leu  Ile Gln Val Thr Leu  Leu Asp Thr
    1460             1465              1470


Val Glu Leu Ala Thr Tyr Thr  Val Arg Thr Phe Ala  Leu His Lys
    1475             1480              1485
```

```
Ser Gly Ser Ser Glu Lys Arg  Glu Leu Arg Gln Phe  Gln Phe Met
    1490             1495              1500

Ala Trp Pro Asp His Gly Val  Pro Glu Tyr Pro Thr  Pro Ile Leu
    1505             1510              1515

Ala Phe Leu Arg Arg Val Lys  Ala Cys Asn Pro Leu  Asp Ala Gly
    1520             1525              1530

Pro Met Val Val His Cys Ser  Ala Gly Val Gly Arg  Thr Gly Cys
    1535             1540              1545

Phe Ile Val Ile Asp Ala Met  Leu Glu Arg Met Lys  His Glu Lys
    1550             1555              1560

Thr Val Asp Ile Tyr Gly His  Val Thr Cys Met Arg  Ser Gln Arg
    1565             1570              1575

Asn Tyr Met Val Gln Thr Glu  Asp Gln Tyr Val Phe  Ile His Glu
    1580             1585              1590

Ala Leu Leu Glu Ala Ala Thr  Cys Gly His Thr Glu  Val Pro Ala
    1595             1600              1605

Arg Asn Leu Tyr Ala His Ile  Gln Lys Leu Gly Gln  Val Pro Pro
    1610             1615              1620

Gly Glu Ser Val Thr Ala Met  Glu Leu Glu Phe Lys  Leu Leu Ala
    1625             1630              1635

Ser Ser Lys Ala His Thr Ser  Arg Phe Ile Ser Ala  Asn Leu Pro
    1640             1645              1650

Cys Asn Lys Phe Lys Asn Arg  Leu Val Asn Ile Met  Pro Tyr Glu
    1655             1660              1665

Leu Thr Arg Val Cys Leu Gln  Pro Ile Arg Gly Val  Glu Gly Ser
    1670             1675              1680

Asp Tyr Ile Asn Ala Ser Phe  Leu Asp Gly Tyr Arg  Gln Gln Lys
    1685             1690              1695

Ala Tyr Ile Ala Thr Gln Gly  Pro Leu Ala Glu Ser  Thr Glu Asp
    1700             1705              1710

Phe Trp Arg Met Leu Trp Glu  His Asn Ser Thr Ile  Ile Val Met
    1715             1720              1725
```

339

```
Leu Thr Lys Leu Arg Glu Met  Gly Arg Glu Lys Cys  His Gln Tyr
    1730                1735             1740

Trp Pro Ala Glu Arg Ser Ala  Arg Tyr Gln Tyr Phe  Val Val Asp
    1745                1750             1755

Pro Met Ala Glu Tyr Asn Met  Pro Gln Tyr Ile Leu  Arg Glu Phe
    1760                1765             1770

Lys Val Thr Asp Ala Arg Asp  Gly Gln Ser Arg Thr  Ile Arg Gln
    1775                1780             1785

Phe Gln Phe Thr Asp Trp Pro  Glu Gln Gly Val Pro  Lys Thr Gly
    1790                1795             1800

Glu Gly Phe Ile Asp Phe Ile  Gly Gln Val His Lys  Thr Lys Glu
    1805                1810             1815

Gln Phe Gly Gln Asp Gly Pro  Ile Thr Val His Cys  Ser Ala Gly
    1820                1825             1830

Val Gly Arg Thr Gly Val Phe  Ile Thr Leu Ser Ile  Val Leu Glu
    1835                1840             1845

Arg Met Arg Tyr Glu Gly Val  Val Asp Met Phe Gln  Thr Val Lys
    1850                1855             1860

Thr Leu Arg Thr Gln Arg Pro  Ala Met Val Gln Thr  Glu Asp Gln
    1865                1870             1875

Tyr Gln Leu Cys Tyr Arg Ala  Ala Leu Glu Tyr Leu  Gly Ser Phe
    1880                1885             1890

Asp His Tyr Ala Thr
    1895
```

<210> 49
<211> 22
<212> PRT
<213> Homo Sapiens

<400> 49

```
Ala Ala Ala Ala Ala Ala Ala Ala Ala Ala Ala Ala Ala Ala Ala Gly
1               5               10                      15

Ala Gly Ala Gly Ala Lys
        20
```

<210> 50
<211> 18
<212> PRT
<213> Homo Sapiens

<400> 50

```
Ala Ala Gly Thr Glu Gly Pro Phe Gln Glu Val Asp Gly Val Ala Thr
1               5                   10                  15

Thr Arg
```

<210> 51
<211> 14
<212> PRT
<213> Homo Sapiens

<400> 51

```
Ala Ala Leu Glu Tyr Leu Gly Ser Phe Asp His Tyr Ala Thr
1               5                   10
```

<210> 52
<211> 15
<212> PRT
<213> Homo Sapiens

<400> 52

```
Ala Ala Asn Asp Pro Phe Thr Ile Val His Gly Asn Thr Gly Lys
1               5                   10                  15
```

<210> 53
<211> 19
<212> PRT
<213> Homo Sapiens

<400> 53

```
Ala Ala Ser Ala Gly Gln Glu Pro Leu His Asn Glu Glu Leu Ala Gly
1               5                   10                  15

Ala Gly Arg
```

<210> 54
<211> 8
<212> PRT
<213> Homo Sapiens

<400> 54

```
Ala Ala Thr Tyr His Val Asp Arg
1               5
```

<210> 55
<211> 20

EP 2 447 719 B1

<212> PRT
<213> Homo Sapiens

<400> 55

```
Ala Cys Phe Ala Leu Leu Trp Gly Cys Ala Leu Ala Ala Ala Ala Ala
1               5               10              15

Ala Gln Gly Lys
            20
```

<210> 56
<211> 20
<212> PRT
<213> Homo Sapiens

<400> 56

```
Ala Cys Asn Pro Leu Asp Ala Gly Pro Met Val Val His Cys Ser Ala
1               5               10              15

Gly Val Gly Arg
            20
```

<210> 57
<211> 8
<212> PRT
<213> Homo Sapiens

<400> 57

```
Ala Asp Ala Ala Pro Asp Glu Lys
1               5
```

<210> 58
<211> 13
<212> PRT
<213> Homo Sapiens

<400> 58

```
Ala Asp Glu Ala Arg Pro Asn Thr Ile Asp Phe Gly Lys
1               5               10
```

<210> 59
<211> 7
<212> PRT
<213> Homo Sapiens

<400> 59

```
Ala Asp Phe Leu Ile Phe Arg
1               5
```

<210> 60
<211> 15

<212> PRT
<213> Homo Sapiens

<400> 60

```
Ala Asp Phe Val Leu Ala Ala Asn Ser Tyr Asp Leu Ala Ile Lys
1               5                   10                  15
```

<210> 61
<211> 11
<212> PRT
<213> Homo Sapiens

<400> 61

```
Ala Asp Gly Ile Ser Ser Thr Phe Ser Gln Arg
1               5                   10
```

<210> 62
<211> 13
<212> PRT
<213> Homo Sapiens

<400> 62

```
Ala Glu Leu Asp Asp Ser Asp Gly His Gly Asn His Arg
1               5                   10
```

<210> 63
<211> 17
<212> PRT
<213> Homo Sapiens

<400> 63

```
Ala Glu Pro Glu Ser Glu Thr Ser Ile Leu Leu Ser Trp Thr Pro Pro
1               5                   10                  15

Arg
```

<210> 64
<211> 6
<212> PRT
<213> Homo Sapiens

<400> 64

```
Ala Glu Trp Leu Asn Lys
1               5
```

<210> 65
<211> 24
<212> PRT
<213> Homo Sapiens

<400> 65

```
Ala Phe Gly Ala Pro Val Pro Ser Val Gln Trp Leu Asp Glu Asp Gly
1               5                   10                  15


Thr Thr Val Leu Gln Asp Glu Arg



        20
```

<210> 66
<211> 9
<212> PRT
<213> Homo Sapiens

<400> 66

```
                    Ala Phe Met Ile Ile Gln Glu Gln Arg
                    1               5
```

<210> 67
<211> 21
<212> PRT
<213> Homo Sapiens

<400> 67

```
Ala Phe Ser Ser Asp Leu Ile Ser Ile His Ser Leu Ala Asp Val Glu
1               5                   10                  15


Val Val Val Thr Lys
        20
```

<210> 68
<211> 10
<212> PRT
<213> Homo Sapiens

<400> 68

```
                Ala Phe Val Asn Cys Asp Glu Asn Ser Arg
                1               5                   10
```

<210> 69
<211> 12
<212> PRT
<213> Homo Sapiens

<400> 69

```
            Ala Phe Tyr Ala Pro Val His Ala Asp Asp Leu Arg
            1               5                   10
```

<210> 70
<211> 15
<212> PRT

<213> Homo Sapiens

<400> 70

```
        Ala Gly Glu Ile Thr Ser Asp Gly Leu Ser Phe Leu Phe Leu Lys
        1               5                   10                  15
```

<210> 71
<211> 9
<212> PRT
<213> Homo Sapiens

<400> 71

```
                    Ala Gly Leu Gly Glu Glu Phe Glu Lys
                    1               5
```

<210> 72
<211> 17
<212> PRT
<213> Homo Sapiens

<400> 72

```
        Ala His Thr Asp Val Gly Pro Gly Pro Glu Ser Ser Pro Val Leu Val
        1               5                   10                  15


        Arg
```

<210> 73
<211> 7
<212> PRT
<213> Homo Sapiens

<400> 73

```
                        Ala Ile Asn Asp Gly Phe Arg
                        1               5
```

<210> 74
<211> 12
<212> PRT
<213> Homo Sapiens

<400> 74

```
                    Ala Ile Ser Thr Asn Ser Glu Leu Ser Thr Phe Arg
                    1               5                   10
```

<210> 75
<211> 10
<212> PRT
<213> Homo Sapiens

<400> 75

```
            Ala Leu Ala Leu Leu Glu Asp Glu Glu Arg
            1               5               10
```

<210> 76
<211> 14
<212> PRT
<213> Homo Sapiens

<400> 76

```
        Ala Leu Gly Arg Pro Gly Pro Pro Phe Asn Ile Thr Pro Arg
        1               5               10
```

<210> 77
<211> 8
<212> PRT
<213> Homo Sapiens

<400> 77

```
            Ala Leu Tyr Gln Leu Gly Met Arg
            1               5
```

<210> 78
<211> 8
<212> PRT
<213> Homo Sapiens

<400> 78

```
            Ala Met Ala Thr Leu Leu Ser Lys
            1               5
```

<210> 79
<211> 26
<212> PRT
<213> Homo Sapiens

<400> 79

```
        Ala Asn Leu Thr Asn Phe Pro Glu Asn Gly Thr Phe Val Val Asn Ile
        1               5               10              15

        Ala Gln Leu Ser Gln Asp Asp Ser Gly Arg
                20              25
```

<210> 80
<211> 28
<212> PRT
<213> Homo Sapiens

<400> 80

```
Ala Pro Gln Asp Pro Ala Ser Met Pro Cys Thr Arg Pro Pro Ser Ala
1               5               10                  15
```

```
Pro His Tyr Leu Thr Ala Val Gly Met Gly Ala Lys
                20                  25
```

<210> 81
<211> 8
<212> PRT
<213> Homo Sapiens

<400> 81

```
Ala Gln Asp Pro Gly Glu Pro Arg
1               5
```

<210> 82
<211> 14
<212> PRT
<213> Homo Sapiens

<400> 82

```
Ala Gln Leu Leu Val Val Gly Ser Pro Gly Pro Val Pro Arg
1               5               10
```

<210> 83
<211> 16
<212> PRT
<213> Homo Sapiens

<400> 83

```
Ala Gln Pro Pro Glu Ala Gly Pro Gln Gly Leu His Asp Leu Gly Arg
1               5               10                  15
```

<210> 84
<211> 13
<212> PRT
<213> Homo Sapiens

<400> 84

```
Ala Gln Gln Glu Phe Ala Thr Gly Val Met Ser Asn Lys
1               5               10
```

<210> 85
<211> 6
<212> PRT
<213> Homo Sapiens

<400> 85

```
Ala Gln Tyr Glu Gly Arg
1               5
```

<210> 86

347

<211> 16
<212> PRT
<213> Homo Sapiens

<400> 86

```
Ala Ser Val Asp Ser Gly Ser Ser Glu Glu Gln Gly Gly Ser Ser Arg
1               5               10                  15
```

<210> 87
<211> 15
<212> PRT
<213> Homo Sapiens

<400> 87

```
Ala Ser Val Ser Phe Leu Asn Phe Asn Leu Ser Asn Cys Glu Arg
1               5               10                  15
```

<210> 88
<211> 10
<212> PRT
<213> Homo Sapiens

<400> 88

```
Ala Thr Asp Phe Asp Val Leu Ser Tyr Lys
1               5                   10
```

<210> 89
<211> 11
<212> PRT
<213> Homo Sapiens

<400> 89

```
Ala Thr Asp Phe Asp Val Leu Ser Tyr Lys Lys
1               5                   10
```

<210> 90
<211> 10
<212> PRT
<213> Homo Sapiens

<400> 90

```
Ala Thr Gly Phe Pro Glu Pro Asn Pro Arg
1               5                   10
```

<210> 91
<211> 7
<212> PRT
<213> Homo Sapiens

<400> 91

```
Ala Thr Leu Gln Leu Ser Arg
1               5
```

<210> 92
<211> 8
<212> PRT
<213> Homo Sapiens

<400> 92

```
Ala Thr Asn Ser Met Ile Asp Arg
1               5
```

<210> 93
<211> 18
<212> PRT
<213> Homo Sapiens

<400> 93

```
Ala Thr Gln Phe Thr Gly Ala Thr Gly Ala Ile Met Thr Thr Glu Thr
1               5               10                  15


Thr Lys
```

<210> 94
<211> 17
<212> PRT
<213> Homo Sapiens

<400> 94

```
Ala Val Asp Glu Gln Gly Trp Glu Tyr Ser Ile Thr Ile Pro Pro Glu
1               5               10                  15


Arg
```

<210> 95
<211> 13
<212> PRT
<213> Homo Sapiens

<400> 95

```
Ala Val Gly Phe Gly Gly Asp Phe Asp Gly Val Pro Arg
1               5                   10
```

<210> 96
<211> 9
<212> PRT
<213> Homo Sapiens

<400> 96

```
        Ala Val Ile Phe Thr Gln Val Ser Arg
        1               5
```

<210> 97
<211> 21
<212> PRT
<213> Homo Sapiens

<400> 97

```
    Ala Val Ile Ser Gln Phe Gln Arg Pro Ser Thr Gln Phe Ser Leu Met
    1               5               10              15

    Gln Phe Ser Asn Lys
                    20
```

<210> 98
<211> 18
<212> PRT
<213> Homo Sapiens

<400> 98

```
    Ala Tyr Ile Ala Thr Gln Gly Pro Leu Ala Glu Ser Thr Glu Asp Phe
    1               5               10              15

    Trp Arg
```

<210> 99
<211> 9
<212> PRT
<213> Homo Sapiens

<400> 99

```
        Ala Tyr Ile Phe Ser Ile Asp Glu Lys
        1               5
```

<210> 100
<211> 17
<212> PRT
<213> Homo Sapiens

<400> 100

```
    Cys Asp Val Pro Ser Phe Ser Val Gln Glu Glu Leu Asp Phe Thr Leu
    1               5               10              15

    Lys
```

<210> 101
<211> 12
<212> PRT
<213> Homo Sapiens

<400> 101

```
            Cys Glu Ala Ser Gly Lys Pro Glu Val Gln Phe Arg
            1               5                   10
```

<210> 102
<211> 11
<212> PRT
<213> Homo Sapiens

<400> 102

```
            Cys Glu His His Ser Leu Tyr Gly Ala Ala Arg
            1               5                   10
```

<210> 103
<211> 25
<212> PRT
<213> Homo Sapiens

<400> 103

```
        Cys His Ile Gln Ala Ser Gly Arg Pro Gln Cys Ser Cys Met Pro Gly
        1               5                   10                  15

            Trp Thr Gly Glu Gln Cys Gln Leu Arg
                        20                  25
```

<210> 104
<211> 7
<212> PRT
<213> Homo Sapiens

<400> 104

```
            Cys Ile Ile Trp Asn Thr Arg
            1               5
```

<210> 105
<211> 11
<212> PRT
<213> Homo Sapiens

<400> 105

```
            Cys Leu Ala Glu Asn Ser Leu Gly Ser Ala Arg
            1               5                   10
```

<210> 106
<211> 13
<212> PRT
<213> Homo Sapiens

<400> 106

```
            Cys Met Leu Asn Ile Trp Gly Val Met Leu Phe Ile Arg
            1               5                   10
```

<210> 107
<211> 10
<212> PRT
<213> Homo Sapiens

<400> 107

```
Cys Gln Gly Gln Leu Glu Val Tyr Leu Lys
1               5               10
```

<210> 108
<211> 13
<212> PRT
<213> Homo Sapiens

<400> 108

```
Cys Ser Met Leu Ile Ala Ser Asn Glu Thr Trp Lys Lys
1               5               10
```

<210> 109
<211> 10
<212> PRT
<213> Homo Sapiens

<400> 109

```
Asp Ala Thr Gln Ile Thr Gln Gly Pro Arg
1               5               10
```

<210> 110
<211> 11
<212> PRT
<213> Homo Sapiens

<400> 110

```
Asp Ala Val Val Thr Tyr Thr Ala Glu Ser Lys
1               5               10
```

<210> 111
<211> 12
<212> PRT
<213> Homo Sapiens

<400> 111

```
Asp Asp Gln His Phe Thr Val Thr Gly Leu His Lys
1               5               10
```

<210> 112
<211> 21
<212> PRT
<213> Homo Sapiens

<400> 112

```
Asp Glu Ala Ile Tyr Glu Cys Thr Ala Thr Asn Ser Leu Gly Glu Ile
1               5               10              15

Asn Thr Ser Ala Lys
                20
```

<210> 113
<211> 14
<212> PRT
<213> Homo Sapiens

<400> 113

```
Asp Glu Glu Glu Glu Pro Pro Ser Met Thr Gln Leu Leu Arg
1               5               10
```

<210> 114
<211> 23
<212> PRT
<213> Homo Sapiens

<400> 114

```
Asp Glu Gly Pro Ala Ala Ala Gly Asp Gly Leu Gly Arg Pro Leu Gly
1               5               10              15

                    Pro Thr Pro Ser Gln Ser Arg
                            20
```

<210> 115
<211> 12
<212> PRT
<213> Homo Sapiens

<400> 115

```
Asp Glu Gln His Gln Cys Ser Leu Gly Asn Leu Lys
1               5               10
```

<210> 116
<211> 20
<212> PRT
<213> Homo Sapiens

<400> 116

```
Asp Phe Leu Leu Gln Ser Ser Thr Val Ala Ala Glu Ala Gln Asp Gly
1               5               10              15

Pro Gln Glu Ala
        20
```

<210> 117
<211> 13
<212> PRT
<213> Homo Sapiens

<400> 117

```
Asp Phe Leu Pro Val Asp Pro Ala Thr Ser Asn Gly Arg
1               5                   10
```

<210> 118
<211> 13
<212> PRT
<213> Homo Sapiens

<400> 118

```
Asp Phe Leu Pro Val Asp Thr Ser Asn Asn Asn Gly Arg
1               5                   10
```

<210> 119
<211> 12
<212> PRT
<213> Homo Sapiens

<400> 119

```
Asp Phe Tyr Asn Pro Val Val Pro Glu Ala Gln Lys
1               5                   10
```

<210> 120
<211> 13
<212> PRT
<213> Homo Sapiens

<400> 120

```
Asp Phe Tyr Asn Pro Val Val Pro Glu Ala Gln Lys Arg
1               5                   10
```

<210> 121
<211> 14
<212> PRT
<213> Homo Sapiens

<400> 121

```
Asp Gly Glu Phe Ser Val Leu Gln Leu Val Gly Met Leu Arg
1               5                   10
```

<210> 122
<211> 16
<212> PRT
<213> Homo Sapiens

<400> 122

```
Asp Gly His Gly Thr Ala Ile Ser Asn Ala Ser Asp Val Trp Lys Lys
1               5               10                  15
```

<210> 123
<211> 12
<212> PRT

<213> Homo Sapiens

<400> 123

```
            Asp Gly Asn Gly Glu Val Thr Asp Lys Pro Val Lys
            1               5                   10
```

<210> 124
<211> 11
<212> PRT
<213> Homo Sapiens

<400> 124

```
            Asp Gly Pro Glu Phe Thr Phe Thr Thr Pro Lys
            1               5                   10
```

<210> 125
<211> 12
<212> PRT
<213> Homo Sapiens

<400> 125

```
            Asp Gly Ser Phe Ser Val Val Ile Thr Gly Leu Arg
            1               5                   10
```

<210> 126
<211> 8
<212> PRT
<213> Homo Sapiens

<400> 126

```
            Asp Gly Val His Phe Lys Pro Lys
            1               5
```

<210> 127
<211> 16
<212> PRT
<213> Homo Sapiens

<400> 127

```
        Asp His Pro Pro Ile Pro Ile Thr Asp Leu Ala Asp Asn Ile Glu Arg
        1               5                   10                  15
```

<210> 128
<211> 33
<212> PRT
<213> Homo Sapiens

<400> 128

```
Asp His Val Val Val Pro Ala Asn Thr Thr Ser Val Ile Leu Ser Gly
1               5                   10                  15
```

```
Leu Arg Pro Tyr Ser Ser Tyr His Leu Glu Val Gln Ala Phe Asn Gly
            20              25                  30
```

```
Arg
```

<210> 129
<211> 16
<212> PRT
<213> Homo Sapiens

<400> 129

```
Asp Ile Asn Ser Gln Gln Glu Leu Gln Asn Ile Thr Thr Asp Thr Arg
1               5                   10                  15
```

<210> 130
<211> 7
<212> PRT
<213> Homo Sapiens

<400> 130

```
Asp Ile Gln Asn Gln Leu Lys
1               5
```

<210> 131
<211> 11
<212> PRT
<213> Homo Sapiens

<400> 131

```
Asp Ile Gln Val Ala Ser Asn Glu Ile Leu Arg
1               5                   10
```

<210> 132
<211> 7
<212> PRT
<213> Homo Sapiens

<400> 132

```
Asp Leu Ala Ala Met Asp Lys
1               5
```

<210> 133
<211> 10
<212> PRT
<213> Homo Sapiens

<400> 133

```
                    Asp Leu Ala Val Phe Leu Tyr His Leu Arg
                    1               5                   10
```

<210> 134
<211> 14
<212> PRT
<213> Homo Sapiens


<400> 134

```
              Asp Leu Leu Pro Glu Asp Phe Val Val Tyr Thr Tyr Asn Lys
              1               5                   10
```

<210> 135
<211> 10
<212> PRT
<213> Homo Sapiens


<400> 135

```
                    Asp Leu Pro Leu Leu Ile Glu Asn Met Lys
                    1               5                   10
```

<210> 136
<211> 9
<212> PRT
<213> Homo Sapiens


<400> 136

```
                      Asp Leu Pro Pro Ile Leu Leu Val Arg
                      1               5
```

<210> 137
<211> 10
<212> PRT
<213> Homo Sapiens


<400> 137

```
                    Asp Leu Gln Glu Leu Gly Asp Ser Asp Lys
                    1               5                   10
```

<210> 138
<211> 16
<212> PRT
<213> Homo Sapiens


<400> 138

```
              Asp Leu Gln Ser Ser Val Thr Leu Asp Leu Ala Leu Asp Pro Gly Arg
              1               5                   10                  15
```

<210> 139
<211> 9
<212> PRT
<213> Homo Sapiens

<400> 139

Asp Leu Ser Gln Met Glu Ala Leu Lys
1               5

<210> 140
<211> 10
<212> PRT
<213> Homo Sapiens

<400> 140

Asp Leu Ser Gln Met Glu Ala Leu Lys Arg
1               5                   10

<210> 141
<211> 10
<212> PRT
<213> Homo Sapiens

<400> 141

Asp Leu Val Glu Pro Trp Val Val Val Arg
1               5                   10

<210> 142
<211> 18
<212> PRT
<213> Homo Sapiens

<400> 142

Asp Met Ala Gly Ala Gln Ala Ala Ala Val Ala Leu Asn Glu Glu Phe
1               5                   10                  15

Leu Arg

<210> 143
<211> 13
<212> PRT
<213> Homo Sapiens

<400> 143

Asp Met Glu Pro Leu Thr Thr Leu Glu Phe Ser Glu Lys
1               5                   10

<210> 144
<211> 25
<212> PRT
<213> Homo Sapiens

<400> 144

```
                    Asp Asn Ile Val Ser Tyr Asn Asp Glu Gly Gly Gly Glu Glu Asp Thr
                    1               5               10                      15


                    Gln Ala Phe Asp Ile Gly Thr Leu Arg
                                20                      25
```

<210> 145
<211> 12
<212> PRT
<213> Homo Sapiens

<400> 145

```
                            Asp Asn Ile Tyr Pro Ala Phe Gln Met Phe Ala Lys
                            1               5                       10
```

<210> 146
<211> 12
<212> PRT
<213> Homo Sapiens

<400> 146

```
                            Asp Asn Met Leu Trp Val Glu Trp Thr Thr Pro Arg
                            1               5                       10
```

<210> 147
<211> 11
<212> PRT
<213> Homo Sapiens

<400> 147

```
                            Asp Asn Gln Trp Leu Gly Val Thr Leu Ser Arg
                            1               5                   10
```

<210> 148
<211> 14
<212> PRT
<213> Homo Sapiens

<400> 148

```
                        Asp Asn Trp Ile Ser Val Asp Ser Val Thr Ser Glu Ile Lys
                        1               5                   10
```

<210> 149
<211> 9
<212> PRT
<213> Homo Sapiens

<400> 149

```
                            Asp Pro Ser Val Thr Gln Val Thr Arg
                            1               5
```

<210> 150

<211> 7
<212> PRT
<213> Homo Sapiens

<400> 150

```
                        Asp Gln Ala Asp Gly Ser Arg
                        1               5
```

<210> 151
<211> 8
<212> PRT
<213> Homo Sapiens

<400> 151

```
                        Asp Gln Phe Asp Ile Cys Ala Lys
                        1               5
```

<210> 152
<211> 5
<212> PRT
<213> Homo Sapiens

<400> 152

```
                        Asp Gln Leu Trp Arg
                        1               5
```

<210> 153
<211> 10
<212> PRT
<213> Homo Sapiens

<400> 153

```
                    Asp Gln Val Ala Cys Leu Thr Phe Phe Lys
                    1               5                   10
```

<210> 154
<211> 10
<212> PRT
<213> Homo Sapiens

<400> 154

```
                    Asp Gln Val Asn Thr Val Gly Ile Pro Ile
                    1               5                   10
```

<210> 155
<211> 18
<212> PRT
<213> Homo Sapiens

<400> 155

```
Asp Ser Phe Ser Asp Pro Tyr Ala Ile Val Ser Phe Leu His Gln Ser
1               5               10              15

Gln Lys
```

<210> 156
<211> 14
<212> PRT
<213> Homo Sapiens

<400> 156

```
Asp Ser Leu Leu Ala His Ser Ser Asp Pro Val Glu Met Arg
1               5               10
```

<210> 157
<211> 15
<212> PRT
<213> Homo Sapiens

<400> 157

```
Asp Ser Leu Leu Ala His Ser Ser Asp Pro Val Glu Met Arg Arg
1               5               10              15
```

<210> 158
<211> 14
<212> PRT
<213> Homo Sapiens

<400> 158

```
Asp Ser Tyr Leu Gly Tyr Ser Thr Glu Leu Ala Leu Trp Lys
1               5               10
```

<210> 159
<211> 14
<212> PRT
<213> Homo Sapiens

<400> 159

```
Asp Thr Gly Glu Ile Tyr Thr Thr Ser Val Thr Leu Asp Arg
1               5               10
```

<210> 160
<211> 13
<212> PRT
<213> Homo Sapiens

<400> 160

```
Asp Thr Gly Glu Leu Asn Val Thr Ser Ile Leu Asp Arg
1               5               10
```

<210> 161
<211> 13

<212> PRT
<213> Homo Sapiens

<400> 161

Asp Val Ile Pro Met Ala Asp Ala Ala Gly Ile Ile Arg
1               5                   10

<210> 162
<211> 8
<212> PRT
<213> Homo Sapiens

<400> 162

Asp Val Ser Ala Ile Met Asn Lys
1               5

<210> 163
<211> 10
<212> PRT
<213> Homo Sapiens

<400> 163

Asp Val Val Val Ser Val Glu Tyr Ser Lys
1               5                   10

<210> 164
<211> 8
<212> PRT
<213> Homo Sapiens

<400> 164

Asp Trp Leu Gln Ala Asp Met Arg
1               5

<210> 165
<211> 10
<212> PRT
<213> Homo Sapiens

<400> 165

Asp Tyr Val Leu His Met Pro Thr Leu Arg
1               5                   10

<210> 166
<211> 13
<212> PRT
<213> Homo Sapiens

<400> 166

Glu Ala Asp Leu Thr Glu Glu Thr Glu Glu Asn Leu Arg
1               5                   10

<210> 167
<211> 24
<212> PRT
<213> Homo Sapiens

<400> 167

```
Glu Ala Ser Val His Ile Gln Leu Glu Gly Arg Pro Ser Ile Leu Glu
1               5               10              15

Met Asp Glu Thr Ser Ala Leu Lys
            20
```

<210> 168
<211> 21
<212> PRT
<213> Homo Sapiens

<400> 168

```
Glu Asp Ala Gln Ile Asn Thr Thr Ile Gly Ser Val Thr Ala Gln Asp
1               5               10              15

Pro Asp Ala Ala Arg
            20
```

<210> 169
<211> 15
<212> PRT
<213> Homo Sapiens

<400> 169

```
Glu Asp Gly Tyr Ser Asp Ala Ser Gly Phe Gly Tyr Cys Phe Arg
1               5               10              15
```

<210> 170
<211> 13
<212> PRT
<213> Homo Sapiens

<400> 170

```
Glu Asp Thr Gln Val Asp Ser Glu Ala Arg Pro Met Lys
        1               5                       10
```

<210> 171
<211> 14
<212> PRT
<213> Homo Sapiens

<400> 171

```
Glu Glu Phe Val Ala Thr Thr Glu Ser Thr Thr Glu Thr Lys
1               5                   10
```

<210> 172
<211> 12
<212> PRT
<213> Homo Sapiens


<400> 172

```
Glu Glu Gly Val Phe Thr Val Thr Pro Asp Thr Lys
1               5                   10
```

<210> 173
<211> 18
<212> PRT
<213> Homo Sapiens


<400> 173

```
Glu Glu His Glu Val Ala Val Leu Gly Ala Pro Pro Ser Thr Ile Leu
1               5                   10                  15
```

```
Pro Arg
```

<210> 174
<211> 13
<212> PRT
<213> Homo Sapiens


<400> 174

```
Glu Glu His Ser Ser Tyr Thr Leu Thr Val Glu Ala Arg
1               5                   10
```

<210> 175
<211> 7
<212> PRT
<213> Homo Sapiens


<400> 175

```
Glu Glu Ile Gly Asn Leu Lys
1               5
```

<210> 176
<211> 10
<212> PRT
<213> Homo Sapiens


<400> 176

```
Glu Glu Ile Asn Lys Pro Pro Ile Ala Lys
1               5                   10
```

<210> 177
<211> 8

<212> PRT
<213> Homo Sapiens

<400> 177

```
Glu Glu Ile Asn Gln Gly Gly Arg
1               5
```

<210> 178
<211> 28
<212> PRT
<213> Homo Sapiens

<400> 178

```
Glu Glu Leu Gly Val Thr Val Tyr Gln Ser Pro His Ser Gly Ser Phe
1               5                   10                  15

Thr Ile Thr Gly Asn Asn Ser Asn Phe Ala Gln Arg
            20              25
```

<210> 179
<211> 20
<212> PRT
<213> Homo Sapiens

<400> 179

```
Glu Glu Met Ile Leu Leu Ala Asn Tyr Leu Asp Ser Met Tyr Ile Met
1               5                   10                  15

Leu Asn Ile Arg
            20
```

<210> 180
<211> 6
<212> PRT
<213> Homo Sapiens

<400> 180

```
Glu Glu Gln Ala Gln Arg
1               5
```

<210> 181
<211> 5
<212> PRT
<213> Homo Sapiens

<400> 181

```
Glu Glu Arg Lys Arg
1               5
```

<210> 182
<211> 16

<212> PRT
<213> Homo Sapiens

<400> 182

```
Glu Glu Thr Pro Phe Phe Leu Leu Thr Gly Tyr Ala Leu Asp Ala Arg
1               5                   10                  15
```

<210> 183
<211> 13
<212> PRT
<213> Homo Sapiens

<400> 183

```
Glu Phe Ile Tyr Leu Arg Pro Phe Ala Cys Asp Thr Lys
1                   5                   10
```

<210> 184
<211> 19
<212> PRT
<213> Homo Sapiens

<400> 184

```
Glu Phe Leu Ser Glu Ala Ser Ile Met Gly Gln Phe Glu His Pro Asn
1               5                   10                  15

Ile Ile Arg
```

<210> 185
<211> 9
<212> PRT
<213> Homo Sapiens

<400> 185

```
Glu Phe Asn Gln Phe Ala Glu Gly Lys
1               5
```

<210> 186
<211> 14
<212> PRT
<213> Homo Sapiens

<400> 186

```
Glu Gly Ala Gln Tyr Leu Met Gln Ala Ala Gly Leu Gly Arg
1               5                   10
```

<210> 187
<211> 8
<212> PRT
<213> Homo Sapiens

<400> 187

```
                    Glu Gly Glu Asp Leu Ser Lys Lys
                    1                   5
```

<210> 188
<211> 16
<212> PRT
<213> Homo Sapiens


<400> 188

```
        Glu Gly Leu Ala His Leu Ile Gln Ser Cys Gly Leu Gly Gly Met Arg
        1               5                   10                  15
```

<210> 189
<211> 9
<212> PRT
<213> Homo Sapiens


<400> 189

```
                    Glu Gly Pro Gly Glu Ala Ile Val Arg
                    1               5
```

<210> 190
<211> 11
<212> PRT
<213> Homo Sapiens


<400> 190

```
            Glu His Ala Leu Ala Gln Ala Glu Leu Leu Lys
            1               5                   10
```

<210> 191
<211> 12
<212> PRT
<213> Homo Sapiens


<400> 191

```
            Glu His Glu Glu Ala Glu Ser Gly Glu Gly Thr Arg
            1               5                   10
```

<210> 192
<211> 14
<212> PRT
<213> Homo Sapiens


<400> 192

```
        Glu His Glu Glu Ala Glu Ser Gly Glu Gly Thr Arg Arg Arg
        1               5                   10
```

<210> 193
<211> 7
<212> PRT
<213> Homo Sapiens

<400> 193

```
                    Glu His Glu Glu Tyr Leu Lys
                    1               5
```

<210> 194
<211> 10
<212> PRT
<213> Homo Sapiens

<400> 194

```
               Glu His Gln Pro Leu Pro Ile Gln Trp Lys
               1             5               10
```

<210> 195
<211> 17
<212> PRT
<213> Homo Sapiens

<400> 195

```
     Glu His Ser Ser Thr Ala Asn Ile Ile Val Met Ser Leu Pro Val Ala
     1           5               10              15

     Arg
```

<210> 196
<211> 12
<212> PRT
<213> Homo Sapiens

<400> 196

```
               Glu His Ser Ser Trp Asp Leu Val Gly Leu Glu Lys
               1           5               10
```

<210> 197
<211> 11
<212> PRT
<213> Homo Sapiens

<400> 197

```
               Glu Ile Ile Asp Glu Asn Phe Ala Glu Leu Lys
               1             5               10
```

<210> 198
<211> 14
<212> PRT
<213> Homo Sapiens

<400> 198

```
          Glu Ile Pro Ser His His Pro Thr Asp Pro Val Glu Leu Arg
          1           5               10
```

<210> 199
<211> 15
<212> PRT
<213> Homo Sapiens

<400> 199

```
Glu Ile Pro Ser His His Pro Thr Asp Pro Val Glu Leu Arg Arg
1               5                   10                  15
```

<210> 200
<211> 8
<212> PRT
<213> Homo Sapiens

<400> 200

```
Glu Ile Thr Tyr Gln Gly Thr Arg
1               5
```

<210> 201
<211> 18
<212> PRT
<213> Homo Sapiens

<400> 201

```
Glu Lys Pro Ala Ile His His Ile Pro Gly Phe Glu Val Gln Glu Thr
1               5                   10                  15

Ser Arg
```

<210> 202
<211> 14
<212> PRT
<213> Homo Sapiens

<400> 202

```
Glu Leu Ala Gln Ser Gln Glu Ala Leu Gln Val Glu Gln Arg
1               5                   10
```

<210> 203
<211> 8
<212> PRT
<213> Homo Sapiens

<400> 203

```
Glu Leu Leu Gly Asn Asn Ile Pro
1               5
```

<210> 204
<211> 16
<212> PRT
<213> Homo Sapiens

<400> 204

```
Glu Leu Asn Asp Ile Ala Ser Lys Pro Ser Gln Glu His Ile Phe Lys
1               5               10              15
```

<210> 205
<211> 9
<212> PRT
<213> Homo Sapiens

<400> 205

```
Glu Leu Asn Ile Leu His Glu Met Lys
1               5
```

<210> 206
<211> 10
<212> PRT
<213> Homo Sapiens

<400> 206

```
Glu Leu Pro Gly Glu Leu Leu Gly Tyr Arg
1               5               10
```

<210> 207
<211> 15
<212> PRT
<213> Homo Sapiens

<400> 207

```
Glu Leu Thr Leu Pro Val Asp Ser Thr Thr Leu Asp Gly Ser Lys
1               5               10              15
```

<210> 208
<211> 15
<212> PRT
<213> Homo Sapiens

<400> 208

```
Glu Leu Thr Tyr Ser Asn Phe His Pro Leu Leu Val Ser Gly Arg
1               5               10              15
```

<210> 209
<211> 13
<212> PRT
<213> Homo Sapiens

<400> 209

```
Glu Leu Val His Ile Lys Pro Asp Gln Ser Asn Val Arg
1               5               10
```

<210> 210
<211> 25
<212> PRT

<210> Homo Sapiens

<400> 210

```
Glu Met Met Glu Glu Ala Asn Gly Gln Ile Ala Pro Glu Asn Gly Thr
1               5                   10                  15

Gln Thr Pro Ser Pro Pro Ser Glu Lys
                20              25
```

<210> 211
<211> 10
<212> PRT
<213> Homo Sapiens

<400> 211

```
Glu Met Gln Asn Leu Ser Gln His Gly Arg
1               5                   10
```

<210> 212
<211> 11
<212> PRT
<213> Homo Sapiens

<400> 212

```
Glu Met Gln Asn Leu Ser Gln His Gly Arg Lys
1               5                   10
```

<210> 213
<211> 8
<212> PRT
<213> Homo Sapiens

<400> 213

```
Glu Met Ser Ile Asp Gln Ala Lys
1               5
```

<210> 214
<211> 13
<212> PRT
<213> Homo Sapiens

<400> 214

```
Glu Asn Gly Gly Ala Ser His Pro Leu Leu Asp Gln Arg
1               5                   10
```

<210> 215
<211> 10
<212> PRT
<213> Homo Sapiens

<400> 215

```
        Glu Asn Pro Asp Asn Leu Ser Asp Phe Arg
        1               5                   10
```

<210> 216
<211> 17
<212> PRT
<213> Homo Sapiens

<400> 216

```
        Glu Pro Phe Glu Asp Gly Phe Ala Asn Gly Glu Glu Ser Thr Pro Thr
        1               5                   10                  15

        Arg
```

<210> 217
<211> 6
<212> PRT
<213> Homo Sapiens

<400> 217

```
                    Glu Pro Ile Asp Leu Arg
                    1               5
```

<210> 218
<211> 7
<212> PRT
<213> Homo Sapiens

<400> 218

```
                    Glu Pro Met Asp Gln Lys Arg
                    1               5
```

<210> 219
<211> 19
<212> PRT
<213> Homo Sapiens

<400> 219

```
        Glu Gln Phe Gly Gln Asp Gly Pro Ile Ser Val His Cys Ser Ala Gly
        1               5                   10                  15

        Val Gly Arg
```

<210> 220
<211> 19
<212> PRT
<213> Homo Sapiens

<400> 220

```
Glu Gln Phe Gly Gln Asp Gly Pro Ile Thr Val His Cys Ser Ala Gly
1               5                   10                  15
```

```
Val Gly Arg
```

<210> 221
<211> 10
<212> PRT
<213> Homo Sapiens

<400> 221

```
Glu Gln Thr Pro Leu Phe Leu Ala Ala Arg
1               5                   10
```

<210> 222
<211> 13
<212> PRT
<213> Homo Sapiens

<400> 222

```
Glu Arg Pro Pro Asp His Gln His Ser Ala Gln Val Lys
1               5                   10
```

<210> 223
<211> 30
<212> PRT
<213> Homo Sapiens

<400> 223

```
Glu Ser Phe Leu Ala Pro Ser Ser Gly Val Gln Pro Thr Leu Ala Met
1               5                   10                  15
```

```
Pro Asn Ile Ala Val Gly Gln Asn Val Thr Val Thr Glu Arg
        20                  25                  30
```

<210> 224
<211> 8
<212> PRT
<213> Homo Sapiens

<400> 224

```
Glu Ser His Val Ala Met His Arg
1               5
```

<210> 225
<211> 12
<212> PRT
<213> Homo Sapiens

<400> 225

```
            Glu Ser His Trp Ile Gly Leu Thr Asp Ser Glu Arg
            1               5                   10
```

<210> 226
<211> 8
<212> PRT
<213> Homo Sapiens


<400> 226

```
                    Glu Ser Pro Ala Gln Ala Pro Ala
                    1               5
```

<210> 227
<211> 13
<212> PRT
<213> Homo Sapiens


<400> 227

```
            Glu Ser Ser Pro Phe Leu Ser Pro Leu Glu Ala Ser Arg
            1               5                   10
```

<210> 228
<211> 10
<212> PRT
<213> Homo Sapiens


<400> 228

```
                Glu Thr Cys Val Asp Leu His Leu Pro Arg
                1               5                   10
```

<210> 229
<211> 19
<212> PRT
<213> Homo Sapiens


<400> 229

```
        Glu Thr Asp Ser Leu Ser Ala Gly Phe Val Val Val Met Gly Val Asp
        1               5               10                  15


        Leu Ser Arg
```

<210> 230
<211> 8
<212> PRT
<213> Homo Sapiens


<400> 230

```
                    Glu Thr Ile Glu Pro Ala Val Arg
                    1               5
```

<210> 231
<211> 10

EP 2 447 719 B1

<212> PRT
<213> Homo Sapiens

<400> 231

```
Glu Thr Leu Gln Ser Glu Glu Gln Gln Arg
1               5                   10
```

<210> 232
<211> 11
<212> PRT
<213> Homo Sapiens

<400> 232

```
Glu Thr Leu Gln Ser Glu Glu Gln Gln Arg Arg
1               5                   10
```

<210> 233
<211> 7
<212> PRT
<213> Homo Sapiens

<400> 233

```
Glu Val Ile Thr Ile Tyr Ser
1               5
```

<210> 234
<211> 20
<212> PRT
<213> Homo Sapiens

<400> 234

```
Glu Val Leu Ala Thr Pro Ser Leu Ser Ala Ser Phe Asn Ala Pro Leu
1               5                   10                  15

Leu Asp Thr Lys
                20
```

<210> 235
<211> 18
<212> PRT
<213> Homo Sapiens

<400> 235

```
Glu Val Pro Gly Tyr Ile Val Leu Phe Tyr Asn Met Ser Leu Asp Val
1               5                   10                  15

Asn Arg
```

<210> 236
<211> 8
<212> PRT

**375**

<213> Homo Sapiens

<400> 236

```
Glu Val Pro Ile Tyr Ala Asn Arg
1               5
```

<210> 237
<211> 10
<212> PRT
<213> Homo Sapiens

<400> 237

```
Glu Val Pro Pro Ala Val Ser Asp Ile Arg
1               5               10
```

<210> 238
<211> 7
<212> PRT
<213> Homo Sapiens

<400> 238

```
Glu Val Pro Val Ala Ile Lys
1               5
```

<210> 239
<211> 23
<212> PRT
<213> Homo Sapiens

<400> 239

```
Glu Val Val Leu Leu Asp Phe Ala Ala Ala Gly Gly Glu Leu Gly Trp
1               5               10              15

Leu Thr His Pro Tyr Gly Lys
                20
```

<210> 240
<211> 7
<212> PRT
<213> Homo Sapiens

<400> 240

```
Glu Trp Gly Asp Gly Ile Arg
1               5
```

<210> 241
<211> 11
<212> PRT
<213> Homo Sapiens

<400> 241

```
                    Glu Tyr Ser Ile Glu Glu Ile Glu Ala Gly Arg
                    1               5                   10
```

<210> 242
<211> 10
<212> PRT
<213> Homo Sapiens

<400> 242

```
                    Phe Ala Asp Ile Val Ser Ile Leu Asp Lys
                    1               5                   10
```

<210> 243
<211> 21
<212> PRT
<213> Homo Sapiens

<400> 243

```
          Phe Ala Pro Gly Val Thr Ile Glu Glu Asp Asn Cys Cys Gly Cys Asn
          1               5                   10                  15

          Ala Ile Ala Ile Arg
                    20
```

<210> 244
<211> 9
<212> PRT
<213> Homo Sapiens

<400> 244

```
                    Phe Ala Val Pro Thr Tyr Ala Ala Lys
                    1               5
```

<210> 245
<211> 19
<212> PRT
<213> Homo Sapiens

<400> 245

```
          Phe Cys Gln Ala Leu Gly Ala His Leu Ser Ser Phe Ser His Val Asp
          1               5                   10                  15

          Glu Ile Lys
```

<210> 246
<211> 15
<212> PRT
<213> Homo Sapiens

<400> 246

```
Phe Asp Leu Ser Met Pro His Val Gln Asp Pro Ser Leu Val Arg
1               5                   10                  15
```

<210> 247
<211> 11
<212> PRT
<213> Homo Sapiens

<400> 247

```
Phe Glu Gln Glu Tyr Leu Asn Asp Leu Met Lys
1               5                   10
```

<210> 248
<211> 12
<212> PRT
<213> Homo Sapiens

<400> 248

```
Phe Glu Gln Glu Tyr Leu Asn Asp Leu Met Lys Lys
1               5                   10
```

<210> 249
<211> 15
<212> PRT
<213> Homo Sapiens

<400> 249

```
Phe Glu Val Ile Glu Phe Asp Asp Gly Ala Gly Ser Val Leu Arg
1               5                   10                  15
```

<210> 250
<211> 8
<212> PRT
<213> Homo Sapiens

<400> 250

```
Phe Phe Ala Ser Ile Gly Glu Arg
1               5
```

<210> 251
<211> 12
<212> PRT
<213> Homo Sapiens

<400> 251

```
Phe Phe Pro Tyr Ala Asn Gly Thr Leu Gly Ile Arg
1               5                   10
```

<210> 252
<211> 15
<212> PRT
<213> Homo Sapiens

<400> 252

```
Phe Gly Ala Ala Leu Thr Val Leu Gly Asp Val Asn Gly Asp Lys
1               5                   10                  15
```

<210> 253
<211> 6
<212> PRT
<213> Homo Sapiens

<400> 253

```
Phe His Ile Leu Phe Lys
1                   5
```

<210> 254
<211> 6
<212> PRT
<213> Homo Sapiens

<400> 254

```
Phe His Leu Glu Tyr Arg
1                   5
```

<210> 255
<211> 18
<212> PRT
<213> Homo Sapiens

<400> 255

```
Phe Ile Lys Pro Trp Glu Ser Pro Asp Glu Met Glu Leu Asp Glu Leu
1               5                   10                  15

Leu Lys
```

<210> 256
<211> 6
<212> PRT
<213> Homo Sapiens

<400> 256

```
Phe Leu Cys Glu Val Lys
1                   5
```

<210> 257
<211> 8
<212> PRT
<213> Homo Sapiens

<400> 257

```
Phe Leu Asp Asp Leu Gly Leu Lys
1                   5
```

<210> 258
<211> 18
<212> PRT
<213> Homo Sapiens

<400> 258

```
Phe Leu Glu Glu Asn Ser Ser Asp Pro Thr Tyr Thr Ser Ser Leu Gly
1               5                   10                  15

Gly Lys
```

<210> 259
<211> 8
<212> PRT
<213> Homo Sapiens

<400> 259

```
Phe Leu Asn Leu Thr Ser Glu Lys
1               5
```

<210> 260
<211> 9
<212> PRT
<213> Homo Sapiens

<400> 260

```
Phe Leu Pro Gly Gly Thr Leu Cys Arg
1               5
```

<210> 261
<211> 10
<212> PRT
<213> Homo Sapiens

<400> 261

```
Phe Met Phe Asp Leu Phe Gln Gln Phe Arg
1               5                   10
```

<210> 262
<211> 11
<212> PRT
<213> Homo Sapiens

<400> 262

```
Phe Met Phe Asp Leu Phe Gln Gln Phe Arg Lys
1               5                   10
```

<210> 263
<211> 10
<212> PRT

<213> Homo Sapiens

<400> 263

Phe Pro Gln Val Val Ser Ala Leu Asp Lys
1               5               10

<210> 264
<211> 7
<212> PRT
<213> Homo Sapiens

<400> 264

Phe Pro Ser Gly Thr Leu Arg
1               5

<210> 265
<211> 15
<212> PRT
<213> Homo Sapiens

<400> 265

Phe Pro Val Thr Phe Gly Glu Glu Cys Leu Tyr Met Ser Ala Lys
1               5               10              15

<210> 266
<211> 6
<212> PRT
<213> Homo Sapiens

<400> 266

Phe Gln Gly Ile Tyr Arg
1               5

<210> 267
<211> 6
<212> PRT
<213> Homo Sapiens

<400> 267

Phe Gln Leu Ala Ala Arg
1               5

<210> 268
<211> 13
<212> PRT
<213> Homo Sapiens

<400> 268

Phe Gln Leu Ser Asn Ser Gly Pro Asn Ser Thr Ile Lys
1               5               10

<210> 269

<211> 11
<212> PRT
<213> Homo Sapiens

<400> 269

```
Phe Gln Thr His Phe Thr Phe Glu Glu Phe Arg
1               5               10
```

<210> 270
<211> 12
<212> PRT
<213> Homo Sapiens

<400> 270

```
Phe Gln Thr His Phe Thr Phe Glu Glu Phe Arg Arg
1               5               10
```

<210> 271
<211> 12
<212> PRT
<213> Homo Sapiens

<400> 271

```
Phe Gln Val Asp Leu Val Ser Glu Asn Ala Gly Arg
1               5               10
```

<210> 272
<211> 7
<212> PRT
<213> Homo Sapiens

<400> 272

```
Phe Ser Asp Val Thr Gly Lys
1               5
```

<210> 273
<211> 16
<212> PRT
<213> Homo Sapiens

<400> 273

```
Phe Ser Phe Asp Asp Phe Leu Gly Ser Leu Gln Leu Asp Leu Asn Arg
1               5               10                  15
```

<210> 274
<211> 6
<212> PRT
<213> Homo Sapiens

<400> 274

```
Phe Ser Ser Tyr Glu Lys
1               5
```

<210> 275
<211> 15
<212> PRT
<213> Homo Sapiens

<400> 275

```
Phe Thr Leu Thr Gly Leu Lys Pro Asp Thr Thr Tyr Asp Ile Lys
1               5                   10                  15
```

<210> 276
<211> 11
<212> PRT
<213> Homo Sapiens

<400> 276

```
Phe Thr Met Leu Glu Cys Leu Ser Leu Pro Arg
1               5                   10
```

<210> 277
<211> 7
<212> PRT
<213> Homo Sapiens

<400> 277

```
Phe Thr Gln Asp Thr Phe Arg
1               5
```

<210> 278
<211> 12
<212> PRT
<213> Homo Sapiens

<400> 278

```
Phe Thr Thr Glu Ile His Pro Ser Cys Val Thr Arg
1               5                   10
```

<210> 279
<211> 9
<212> PRT
<213> Homo Sapiens

<400> 279

```
Phe Val Trp Asn Gly His Leu Leu Arg
1               5
```

<210> 280
<211> 19
<212> PRT
<213> Homo Sapiens

<400> 280

Phe Tyr Gln Thr Ser Val Glu Ser Thr Asp Phe Ala Asn Ala Pro Glu
1 5 10 15

Glu Ser Arg

<210> 281
<211> 21
<212> PRT
<213> Homo Sapiens

<400> 281

Phe Tyr Gln Thr Ser Val Glu Ser Thr Asp Phe Ala Asn Ala Pro Glu
1 5 10 15

Glu Ser Arg Lys Lys
20

<210> 282
<211> 11
<212> PRT
<213> Homo Sapiens

<400> 282

Gly Ala Glu Ala Phe Glu Ile Ala Leu Pro Arg
1 5 10

<210> 283
<211> 9
<212> PRT
<213> Homo Sapiens

<400> 283

Gly Ala Leu Ala Asp Asn Leu Leu Arg
1 5

<210> 284
<211> 21
<212> PRT
<213> Homo Sapiens

<400> 284

Gly Ala Leu Ile Leu Ser Asn Val Gln Pro Ser Asp Thr Met Val Thr
1 5 10 15

Gln Cys Glu Ala Arg
20

<210> 285
<211> 13
<212> PRT
<213> Homo Sapiens

<400> 285

```
Gly Ala Asn Thr His Leu Ser Thr Phe Ser Phe Thr Lys
1               5                   10
```

<210> 286
<211> 12
<212> PRT
<213> Homo Sapiens

<400> 286

```
Gly Ala Pro Cys Thr Thr Pro Pro Ser Ala Pro Arg
1               5                   10
```

<210> 287
<211> 21
<212> PRT
<213> Homo Sapiens

<400> 287

```
Gly Ala Val Tyr Leu Phe His Gly Val Leu Gly Pro Ser Ile Ser Pro
1               5                   10                  15

Ser His Ser Gln Arg
                20
```

<210> 288
<211> 10
<212> PRT
<213> Homo Sapiens

<400> 288

```
Gly Asp Ser Asn Ser Tyr Asn Val Arg Arg
1               5                   10
```

<210> 289
<211> 17
<212> PRT
<213> Homo Sapiens

<400> 289

```
Gly Glu Gly Asn Glu Thr Thr Asn Met Val Ile Thr Trp Lys Pro Leu
1               5                   10                  15

Arg
```

<210> 290
<211> 14
<212> PRT
<213> Homo Sapiens

<400> 290

```
Gly Glu Gly Pro Glu Ala Gly Ala Gly Gly Ala Gly Gly Arg
1               5                   10
```

<210> 291
<211> 7
<212> PRT
<213> Homo Sapiens

<400> 291

```
Gly Glu Gly Val Ala Tyr Arg
1               5
```

<210> 292
<211> 12
<212> PRT
<213> Homo Sapiens

<400> 292

```
Gly Glu Pro Gly Pro Pro Gly Pro Ala Gly Glu Arg
1               5                   10
```

<210> 293
<211> 15
<212> PRT
<213> Homo Sapiens

<400> 293

```
Gly Glu Thr Tyr Thr Tyr Asp Trp Gln Leu Ile Thr His Pro Arg
1               5                   10                  15
```

<210> 294
<211> 6
<212> PRT
<213> Homo Sapiens

<400> 294

```
Gly Phe Phe Gly Tyr Lys
1               5
```

<210> 295
<211> 12
<212> PRT
<213> Homo Sapiens

<400> 295

```
Gly Phe Pro Thr Ile Asp Met Gly Pro Gln Leu Lys
1               5                   10
```

<210> 296
<211> 16
<212> PRT
<213> Homo Sapiens

<400> 296

```
Gly Gly Ala Gln Ile Thr Phe Leu Ala Thr Phe Asp Val Ser Pro Lys
1               5               10                  15
```

<210> 297
<211> 16
<212> PRT
<213> Homo Sapiens

<400> 297

```
Gly Gly Cys Ile Thr Leu Ile Ser Ser Glu Gly Tyr Val Ser Ser Lys
1               5               10                  15
```

<210> 298
<211> 10
<212> PRT
<213> Homo Sapiens

<400> 298

```
Gly Gly Gly Ala Tyr Tyr Leu Ile Ser Arg
1               5                   10
```

<210> 299
<211> 11
<212> PRT
<213> Homo Sapiens

<400> 299

```
Gly Gly Gln Val Ser Val Cys Pro Leu Pro Arg
1               5                   10
```

<210> 300
<211> 7
<212> PRT
<213> Homo Sapiens

<400> 300

```
Gly Ile Asp Tyr Tyr Asp Arg
1               5
```

<210> 301
<211> 14
<212> PRT
<213> Homo Sapiens

<400> 301

```
Gly Ile Thr Glu Pro Pro Phe Gly Ile Phe Val Phe Asn Lys
1               5                   10
```

<210> 302
<211> 16
<212> PRT

<213> Homo Sapiens

<400> 302

Gly Leu Pro Ser Leu Thr Ser Val Ser Trp Asn Ile Ser Val Pro Arg
1               5                   10                  15

<210> 303
<211> 9
<212> PRT
<213> Homo Sapiens

<400> 303

Gly Asn Leu Ser Phe Gly Trp Val Arg
1                   5

<210> 304
<211> 11
<212> PRT
<213> Homo Sapiens

<400> 304

Gly Asn Asn Val Glu Lys Pro Leu Glu Leu Arg
1                   5                   10

<210> 305
<211> 10
<212> PRT
<213> Homo Sapiens

<400> 305

Gly Asn Ser Ala Gly Gly Leu Gln His Arg
1                   5                   10

<210> 306
<211> 12
<212> PRT
<213> Homo Sapiens

<400> 306

Gly Pro Gly Pro Tyr Ser Pro Ser Val Gln Phe Arg
1                   5                   10

<210> 307
<211> 12
<212> PRT
<213> Homo Sapiens

<400> 307

Gly Pro Ile Val Pro Leu Asn Val Ala Asp Gln Lys
1                   5                   10

<210> 308

<211> 8
<212> PRT
<213> Homo Sapiens

<400> 308

```
                        Gly Pro Pro Ser Glu Ala Val Arg
                        1                   5
```

<210> 309
<211> 21
<212> PRT
<213> Homo Sapiens

<400> 309

```
        Gly Pro Pro Ser Glu Pro Val Leu Thr Gln Thr Ser Glu Gln Ala Pro

            1               5                   10                  15


        Ser Ser Ala Pro Arg
                    20
```

<210> 310
<211> 10
<212> PRT
<213> Homo Sapiens

<400> 310

```
                    Gly Pro Arg Pro Asn Pro Ala Ile Met Arg
                    1               5                   10
```

<210> 311
<211> 21
<212> PRT
<213> Homo Sapiens

<400> 311

```
        Gly Gln Ile Ile Gly Asn Phe Gln Ala Phe Asp Glu Asp Thr Gly Leu
        1               5                   10                  15


        Pro Ala His Ala Arg
                    20
```

<210> 312
<211> 8
<212> PRT
<213> Homo Sapiens

<400> 312

```
                        Gly Gln Leu Ser Phe Asn Leu Arg
                        1               5
```

<210> 313
<211> 17
<212> PRT
<213> Homo Sapiens

<400> 313

```
Gly Gln Val Val Thr Val His Leu Gln Thr Ser Leu Glu Asn Gly Thr
1               5                   10                  15

Arg
```

<210> 314
<211> 23
<212> PRT
<213> Homo Sapiens

<400> 314

```
Gly Ser Glu Lys Pro Leu Glu Gln Thr Phe Ala Thr Met Val Ser Ser
1               5                   10                  15

Leu Gly Ser Gly Met Met Arg
                20
```

<210> 315
<211> 12
<212> PRT
<213> Homo Sapiens

<400> 315

```
Gly Ser Gly Pro Leu Ser Pro Ser Ile Gln Ser Arg
1               5                   10
```

<210> 316
<211> 14
<212> PRT
<213> Homo Sapiens

<400> 316

```
Gly Ser Ile Pro Val Phe Trp Ser Gln Arg Pro Asn Leu Lys
1               5                   10
```

<210> 317
<211> 23
<212> PRT
<213> Homo Sapiens

<400> 317

```
        Gly Ser Pro Gly Lys Pro Gly Pro Gln Gly Ser Ser Gly Asp Pro Gly
        1               5                   10                  15


        Pro Pro Gly Pro Pro Gly Lys
                    20
```

<210> 318
<211> 25
<212> PRT
<213> Homo Sapiens

<400> 318

```
        Gly Ser Pro Gln Leu Asp Cys Gly Pro Pro Ala Leu Gln Glu Met Pro
        1               5                   10                  15


        Ile Asn Gln Gly Gly Glu Gly Lys Lys
                    20                  25
```

<210> 319
<211> 12
<212> PRT
<213> Homo Sapiens

<400> 319

```
                Gly Ser Gln Gly Pro Pro Gly Pro Thr Gly Asn Lys
                1               5                   10
```

<210> 320
<211> 17
<212> PRT
<213> Homo Sapiens

<400> 320

```
        Gly Ser Gln Pro Ser Gly Glu Leu Leu Ala Ser Phe Glu Leu Ile Gln
        1               5                   10                  15


        Arg
```

<210> 321
<211> 14
<212> PRT
<213> Homo Sapiens

<400> 321

```
        Gly Ser Ser Ala Gly Gly Leu Gln His Leu Val Ser Ile Arg
        1               5                   10
```

<210> 322
<211> 9
<212> PRT
<213> Homo Sapiens

<400> 322

```
                          Gly Ser Ser Ser Ala Ser Ile Val Lys
                          1               5
```

<210> 323
<211> 18
<212> PRT
<213> Homo Sapiens

<400> 323

```
          Gly Ser Val Thr Phe His Cys Ala Leu Gly Pro Glu Val Ala Asn Val
          1               5                   10                  15

          Ala Lys
```

<210> 324
<211> 7
<212> PRT
<213> Homo Sapiens

<400> 324

```
                            Gly Thr Thr Tyr Ile Phe Arg
                            1               5
```

<210> 325
<211> 17
<212> PRT
<213> Homo Sapiens

<400> 325

```
          Gly Val Glu Gly Ser Asp Tyr Ile Asn Ala Ser Phe Ile Asp Gly Tyr
          1               5                   10                  15

          Arg
```

<210> 326
<211> 17
<212> PRT
<213> Homo Sapiens

<400> 326

```
          Gly Val Glu Gly Ser Asp Tyr Ile Asn Ala Ser Phe Leu Asp Gly Tyr
          1               5                   10                  15

          Arg
```

<210> 327
<211> 11
<212> PRT
<213> Homo Sapiens

<400> 327

```
Gly Val Gln Ser Leu Val Leu Gly Ala Pro Arg
1               5               10
```

<210> 328
<211> 8
<212> PRT
<213> Homo Sapiens

<400> 328

```
Gly Trp His Phe Tyr Asp Asp Arg
1               5
```

<210> 329
<211> 10
<212> PRT
<213> Homo Sapiens

<400> 329

```
Gly Trp Met Ile Gly Phe Glu Glu His Lys

1               5               10
```

<210> 330
<211> 8
<212> PRT
<213> Homo Sapiens

<400> 330

```
Gly Tyr Asn Val Thr Tyr Trp Arg
1               5
```

<210> 331
<211> 8
<212> PRT
<213> Homo Sapiens

<400> 331

```
Gly Tyr Gln Val Thr Tyr Val Arg
1               5
```

<210> 332
<211> 11
<212> PRT
<213> Homo Sapiens

<400> 332

```
His Phe Asp Ser Gln Val Ile Ile Tyr Gly Lys
1               5               10
```

<210> 333
<211> 8
<212> PRT
<213> Homo Sapiens

<400> 333

```
His Phe Val Ser Leu Cys Gln Lys
1               5
```

<210> 334
<211> 12
<212> PRT
<213> Homo Sapiens

<400> 334

```
His Gly Leu Pro Ser Ala Asp Ala Pro Ser Leu Lys
1               5                   10
```

<210> 335
<211> 11
<212> PRT
<213> Homo Sapiens

<400> 335

```
His Gly Gln Tyr Leu Ile Gly His Gly Thr Lys
1               5                   10
```

<210> 336
<211> 11
<212> PRT
<213> Homo Sapiens

<400> 336

```
His Ile Trp Pro Asn Val Pro Asp Pro Ser Lys
1               5                   10
```

<210> 337
<211> 9
<212> PRT
<213> Homo Sapiens

<400> 337

```
His Leu Pro Glu Thr Glu Ala Gly Arg
1               5
```

<210> 338
<211> 12
<212> PRT
<213> Homo Sapiens

<400> 338

```
His Met Ala Thr Thr Gln Asp Glu Val His Thr Lys
1               5               10
```

<210> 339
<211> 12
<212> PRT
<213> Homo Sapiens

<400> 339

```
His Met Trp Pro Phe Ile Cys Gln Phe Ile Glu Lys
1               5               10
```

<210> 340
<211> 13
<212> PRT
<213> Homo Sapiens

<400> 340

```
His Asn Ser Val Val Leu Gly Trp Pro Tyr Gly Trp Arg
1               5               10
```

<210> 341
<211> 23
<212> PRT
<213> Homo Sapiens

<400> 341

```
His Asn Thr Asp Ala Gly Leu Leu Thr Thr Val Gly Ser Leu Leu Pro
1               5               10                  15

Gly Ile Thr Tyr Ser Leu Arg
            20
```

<210> 342
<211> 6
<212> PRT
<213> Homo Sapiens

<400> 342

```
His Gln Met Ala Val Lys
1               5
```

<210> 343
<211> 6
<212> PRT
<213> Homo Sapiens

<400> 343

```
His Gln Val Val His Lys
1               5
```

<210> 344

<210> 14
<212> PRT
<213> Homo Sapiens

<400> 344

```
His Ser His Thr Asn Leu Ser Ile Ser Thr Gly Val Thr Lys
1               5                   10
```

<210> 345
<211> 14
<212> PRT
<213> Homo Sapiens

<400> 345

```
His Val Glu Met Tyr Gln Trp Val Glu Thr Glu Glu Ser Arg
1               5                   10
```

<210> 346
<211> 9
<212> PRT
<213> Homo Sapiens

<400> 346

```
His Val Ser Pro Val Thr Pro Pro Arg
1               5
```

<210> 347
<211> 8
<212> PRT
<213> Homo Sapiens

<400> 347

```
His Val Val Asp Gly Ile Ser Arg
1               5
```

<210> 348
<211> 7
<212> PRT
<213> Homo Sapiens

<400> 348

```
His Trp Val Pro Ala Glu Lys
1               5
```

<210> 349
<211> 8
<212> PRT
<213> Homo Sapiens

<400> 349

```
Ile Ala Asp Phe Gly Leu Ala Arg
1               5
```

<210> 350
<211> 9
<212> PRT
<213> Homo Sapiens

<400> 350

```
Ile Ala Gly Ser Gln Leu Ser Ser Arg
1               5
```

<210> 351
<211> 10
<212> PRT
<213> Homo Sapiens

<400> 351

```
Ile Ala Pro Cys Tyr Gln Asp Tyr Val Lys
1               5                   10
```

<210> 352
<211> 14
<212> PRT
<213> Homo Sapiens

<400> 352

```
Ile Ala Pro Pro Ala Ser Asp Phe Leu Ala His Ile Gln Lys
1           5               10
```

<210> 353
<211> 7
<212> PRT
<213> Homo Sapiens

<400> 353

```
Ile Asp Ala Thr Val Val Arg
1               5
```

<210> 354
<211> 17
<212> PRT
<213> Homo Sapiens

<400> 354

```
Ile Asp Phe Ser Asp Ile Met Val Leu Gly Asp Ile Asn Thr Lys Pro
1           5               10                  15

Lys
```

<210> 355
<211> 18
<212> PRT
<213> Homo Sapiens

<400> 355

```
Ile Asp Phe Ser Asp Ile Met Val Leu Gly Asp Ile Asn Thr Lys Pro
1               5                   10                  15

Lys Lys
```

<210> 356
<211> 6
<212> PRT
<213> Homo Sapiens

<400> 356

```
Ile Asp His Asp Arg Arg
1               5
```

<210> 357
<211> 5
<212> PRT
<213> Homo Sapiens

<400> 357

```
Ile Asp His Tyr Arg
1               5
```

<210> 358
<211> 13
<212> PRT
<213> Homo Sapiens

<400> 358

```
Ile Asp Ser Leu Glu Asp Gln Ile Glu Glu Phe Ser Lys
1               5                   10
```

<210> 359
<211> 18
<212> PRT
<213> Homo Sapiens

<400> 359

```
Ile Asp Thr Ile Ala Pro Asp Glu Ile Thr Val Ser Ser Asp Phe Glu
1               5                   10                  15

Ala Arg
```

<210> 360
<211> 15
<212> PRT
<213> Homo Sapiens

<400> 360

```
Ile Glu Glu Val Ile Gly Ala Gly Glu Phe Gly Glu Val Cys Arg
1               5                   10                  15
```

<210> 361
<211> 8
<212> PRT
<213> Homo Sapiens

<400> 361

```
                        Ile Glu Gly Leu Gln Ile Ser Lys
                        1               5
```

<210> 362
<211> 7
<212> PRT
<213> Homo Sapiens

<400> 362

```
                        Ile Glu Met Val Asp Tyr Lys
                        1               5
```

<210> 363
<211> 13
<212> PRT
<213> Homo Sapiens

<400> 363

```
    Ile Glu Thr Gln Asn Gln Leu Leu Gly Ile Ala Asp Arg

            1               5               10
```

<210> 364
<211> 15
<212> PRT
<213> Homo Sapiens

<400> 364

```
Ile Phe Thr Val Ala Gln Met Asp Asp Asn Ser Ile Gln Met Lys
1               5                   10                  15
```

<210> 365
<211> 16
<212> PRT
<213> Homo Sapiens

<400> 365

```
Ile Phe Thr Val Ala Gln Met Asp Asp Asn Ser Ile Gln Met Lys Lys
1               5                   10                  15
```

<210> 366
<211> 11

<212> PRT
<213> Homo Sapiens

<400> 366

```
                    Ile Gly Asp Asp Asn Glu Asn Leu Thr Phe Lys
                    1               5                   10
```

<210> 367
<211> 11
<212> PRT
<213> Homo Sapiens

<400> 367

```
                    Ile Gly Glu Thr Val Val Asp Leu Glu Asn Arg
                    1               5                   10
```

<210> 368
<211> 12
<212> PRT
<213> Homo Sapiens

<400> 368

```
                  Ile His Phe Ile Glu Ala Gln Asp Leu Gln Gly Lys
                  1               5                   10
```

<210> 369
<211> 9
<212> PRT
<213> Homo Sapiens

<400> 369

```
                    Ile His Ser Asp Leu Ala Glu Glu Arg
                    1               5
```

<210> 370
<211> 6
<212> PRT
<213> Homo Sapiens

<400> 370

```
                      Ile Ile Asp Trp Asp Arg
                      1               5
```

<210> 371
<211> 9
<212> PRT
<213> Homo Sapiens

<400> 371

```
                    Ile Ile Glu Gly Glu Pro Asn Leu Lys
                    1               5
```

<210> 372
<211> 19
<212> PRT
<213> Homo Sapiens

<400> 372

```
Ile Ile Met Tyr Glu Leu Val Tyr Trp Ala Ala Glu Asp Glu Asp Gln
1               5                   10                  15

Gln His Lys
```

<210> 373
<211> 9
<212> PRT
<213> Homo Sapiens

<400> 373

```
Ile Leu Ala Ser Val Gln His Met Lys
1               5
```

<210> 374
<211> 17
<212> PRT
<213> Homo Sapiens

<400> 374

```
Ile Leu Asp Ala Thr Asp Gln Glu Ser Leu Glu Leu Lys Pro Thr Ser
1               5                   10                  15

Arg
```

<210> 375
<211> 17
<212> PRT
<213> Homo Sapiens

<400> 375

```
Ile Leu Asp Glu Ser Glu Asp Thr Asp Leu Pro Tyr Pro Pro Pro Gln
1               5                   10                  15

Arg
```

<210> 376
<211> 7
<212> PRT
<213> Homo Sapiens

<400> 376

```
Ile Leu Glu Leu Glu Glu Lys
1               5
```

<210> 377
<211> 8
<212> PRT
<213> Homo Sapiens

<400> 377

```
                          Ile Leu Leu Asn Pro Gln Asp Lys
                          1               5
```

<210> 378
<211> 16
<212> PRT
<213> Homo Sapiens

<400> 378

```
        Ile Leu Tyr Asn Gly Gln Ser Val Glu Val Asp Gly His Ser Met Arg
        1               5                   10                  15
```

<210> 379
<211> 17
<212> PRT
<213> Homo Sapiens

<400> 379

```
        Ile Leu Tyr Asn Gly Gln Ser Val Glu Val Asp Gly His Ser Met Arg
        1               5                   10                  15

        Lys
```

<210> 380
<211> 11
<212> PRT
<213> Homo Sapiens

<400> 380

```
                    Ile Met Phe Val Asp Pro Ser Leu Thr Val Arg
                    1               5                   10
```

<210> 381
<211> 15
<212> PRT
<213> Homo Sapiens

<400> 381

```
        Ile Asn Ala Thr Asp Ala Asp Glu Pro Asn Thr Leu Asn Ser Lys
        1               5                   10                  15
```

<210> 382
<211> 11
<212> PRT
<213> Homo Sapiens

<400> 382

```
Ile Asn Gly Ile Pro Val Glu Glu Leu Ala Lys
1               5                   10
```

<210> 383
<211> 7
<212> PRT
<213> Homo Sapiens

<400> 383

```
Ile Asn His Leu Ile Phe Arg
1               5
```

<210> 384
<211> 12
<212> PRT
<213> Homo Sapiens

<400> 384

```
Ile Asn Ser Trp Val Glu Ser Gln Thr Asn Glu Lys
1               5                   10
```

<210> 385
<211> 11
<212> PRT
<213> Homo Sapiens

<400> 385

```
Ile Pro Glu Asn Phe Phe Glu Glu Glu Ser Arg
1               5                   10
```

<210> 386
<211> 12
<212> PRT
<213> Homo Sapiens

<400> 386

```
Ile Pro Asn Pro His Leu Gly Pro Val Glu Glu Arg
1               5                   10
```

<210> 387
<211> 17
<212> PRT
<213> Homo Sapiens

<400> 387

```
Ile Gln Asp Leu Leu Ala Glu Gly Thr Ile Thr Gly Val Ile Asp Asp
1               5                   10                  15

Arg
```

<210> 388
<211> 12
<212> PRT
<213> Homo Sapiens

<400> 388

```
Ile Gln Leu Ser Trp Leu Leu Pro Pro Gln Glu Arg
1               5                   10
```

<210> 389
<211> 7
<212> PRT
<213> Homo Sapiens

<400> 389

```
Ile Gln Met Thr Trp Thr Arg
1               5
```

<210> 390
<211> 15
<212> PRT
<213> Homo Sapiens

<400> 390

```
Ile Gln Asn Ser Ser Cys Thr Ser Leu Glu His Cys Phe Arg Lys
1               5                   10                  15
```

<210> 391
<211> 10
<212> PRT
<213> Homo Sapiens

<400> 391

```
Ile Gln Pro Tyr Thr Glu Gln Ser Thr Lys
1               5                   10
```

<210> 392
<211> 14
<212> PRT
<213> Homo Sapiens

<400> 392

```
Ile Ser Glu Trp Pro Ile Asp Asp His Phe Thr Tyr Ser Arg
1               5                   10
```

<210> 393
<211> 16
<212> PRT
<213> Homo Sapiens

<400> 393

```
         Ile Ser Ser Asn Pro Asn Pro Val Val Gln Met Ser Val Gly His Lys
         1               5                   10                  15
```

<210> 394
<211> 14
<212> PRT
<213> Homo Sapiens


<400> 394

```
            Ile Ser Val Thr Val Ser Glu Thr Phe Asp Pro Glu Glu Lys
            1               5                   10
```

<210> 395
<211> 10
<212> PRT
<213> Homo Sapiens


<400> 395

```
            Ile Thr Asp Asn Glu Leu Glu Leu Tyr Lys
            1               5                   10
```

<210> 396
<211> 15
<212> PRT
<213> Homo Sapiens


<400> 396

```
            Ile Thr Gln Leu Gly Gln Ser Ala Glu Asp Leu Gln Gly Ser Arg
            1               5                   10                  15
```

<210> 397
<211> 16
<212> PRT
<213> Homo Sapiens


<400> 397

```
         Ile Thr Gln Leu Gly Gln Ser Ala Glu Asp Leu Gln Gly Ser Arg Arg
         1               5                   10                  15
```

<210> 398
<211> 15
<212> PRT
<213> Homo Sapiens


<400> 398

```
            Ile Thr Val Pro Leu Val Ser Glu Val Gln Ile Ala Gln Leu Arg
            1               5                   10                  15
```

<210> 399
<211> 6
<212> PRT
<213> Homo Sapiens

<400> 399

                Ile Thr Trp Met Lys Lys
                1            5

<210> 400
<211> 10
<212> PRT
<213> Homo Sapiens

<400> 400

          Ile Val Ala Ile Ser Glu Asp Tyr Pro Arg
          1          5          10

<210> 401
<211> 9
<212> PRT
<213> Homo Sapiens

<400> 401

           Ile Val Phe Thr Pro Thr Ile Cys Lys
           1          5

<210> 402
<211> 14
<212> PRT
<213> Homo Sapiens

<400> 402

       Ile Val Val Glu Asp Val Asp Glu Pro Pro Val Phe Ser Lys
       1          5             10

<210> 403
<211> 15
<212> PRT
<213> Homo Sapiens

<400> 403

      Ile Tyr Pro Leu Pro Glu Asp Pro Ala Ile Pro Met Pro Pro Arg
      1         5           10          15

<210> 404
<211> 10
<212> PRT
<213> Homo Sapiens

<400> 404

         Ile Tyr Val Val Asp Leu Ser Asn Glu Arg
         1          5          10

<210> 405
<211> 7
<212> PRT

<213> Homo Sapiens

<400> 405

Lys Ala Ala Glu Leu Asp Arg
1               5

<210> 406
<211> 8
<212> PRT
<213> Homo Sapiens

<400> 406

Lys Ala Ala Glu Leu Asp Arg Arg
1               5

<210> 407
<211> 7
<212> PRT
<213> Homo Sapiens

<400> 407

Lys Ala Glu Ser Pro Pro Arg
1               5

<210> 408
<211> 9
<212> PRT
<213> Homo Sapiens

<400> 408

Lys Asp Trp Leu Gln Ala Asp Met Arg
1               5

<210> 409
<211> 9
<212> PRT
<213> Homo Sapiens

<400> 409

Lys Glu Gly Asp Ser Leu Asp Tyr Lys
1               5

<210> 410
<211> 15
<212> PRT
<213> Homo Sapiens

<400> 410

Lys Glu Asn Ile Ile Ala Phe Glu Glu Ile Ile Glu Pro Tyr Arg
1               5                   10                  15

<210> 411

<211> 8
<212> PRT
<213> Homo Sapiens

<400> 411

```
Lys Glu Asn Ile Thr Tyr Met Lys
1               5
```

<210> 412
<211> 9
<212> PRT
<213> Homo Sapiens

<400> 412

```
Lys Glu Asn Ile Thr Tyr Met Lys Arg
1               5
```

<210> 413
<211> 8
<212> PRT
<213> Homo Sapiens

<400> 413

```
Lys Glu Ser Cys Val Ala Ile Lys
1               5
```

<210> 414
<211> 8
<212> PRT
<213> Homo Sapiens

<400> 414

```
Lys Glu Val Pro Val Ala Ile Lys
1               5
```

<210> 415
<211> 13
<212> PRT
<213> Homo Sapiens

<400> 415

```
Lys Phe Val Pro Gly Cys Phe Val Cys Leu Glu Ser Arg
1               5                   10
```

<210> 416
<211> 21
<212> PRT
<213> Homo Sapiens

<400> 416

```
Lys Phe Tyr Gln Thr Ser Val Glu Ser Thr Asp Phe Ala Asn Ala Pro
1               5               10              15

Glu Glu Ser Arg Lys
            20
```

<210> 417
<211> 10
<212> PRT
<213> Homo Sapiens

<400> 417

```
Lys Gly Asp Ser Asn Ser Tyr Asn Val Arg
1               5               10
```

<210> 418
<211> 11
<212> PRT
<213> Homo Sapiens

<400> 418

```
Lys Gly Asp Ser Asn Ser Tyr Asn Val Arg Arg
1               5               10
```

<210> 419
<211> 18
<212> PRT
<213> Homo Sapiens

<400> 419

```
Lys Gly Phe Gly Gly Thr Ala Gly Met Ala Phe Val Gly Thr Val Cys
1               5               10              15

Ser Arg
```

<210> 420
<211> 13
<212> PRT
<213> Homo Sapiens

<400> 420

```
Lys Ile Asn Ser Trp Val Glu Ser Gln Thr Asn Glu Lys
1               5               10
```

<210> 421
<211> 9
<212> PRT
<213> Homo Sapiens

<400> 421

Lys Lys Glu Asn Ile Thr Tyr Met Lys
1               5

<210> 422
<211> 11
<212> PRT
<213> Homo Sapiens

<400> 422

Lys Lys Gly Asp Ser Asn Ser Tyr Asn Val Arg
1               5               10

<210> 423
<211> 8
<212> PRT
<213> Homo Sapiens

<400> 423

Lys Leu Gly Asp Gln Thr Gly Lys
1               5

<210> 424
<211> 19
<212> PRT
<213> Homo Sapiens

<400> 424

Lys Leu Ile Ala Asp Leu Gln Pro Asn Thr Glu Tyr Ser Phe Val Leu
1               5               10              15

Met Asn Arg

<210> 425
<211> 8
<212> PRT
<213> Homo Sapiens

<400> 425

Lys Leu Pro Gln Gly Glu Ser Arg
1               5

<210> 426
<211> 14
<212> PRT
<213> Homo Sapiens

<400> 426

Lys Leu Pro Ser Arg Pro Pro Pro His Tyr Pro Gly Ile Lys
1               5               10

<210> 427
<211> 8

<212> PRT
<213> Homo Sapiens

<400> 427

```
                              Lys Met Leu Gly Asn Pro Ser Arg
                              1               5
```

<210> 428
<211> 6
<212> PRT
<213> Homo Sapiens

<400> 428

```
                              Lys Asn Gly Tyr Asn Arg
                              1               5
```

<210> 429
<211> 15
<212> PRT
<213> Homo Sapiens

<400> 429

```
        Lys Asn Leu Val Asp Pro Phe Val Glu Val Ser Phe Ala Gly Lys
        1               5                   10                  15
```

<210> 430
<211> 18
<212> PRT
<213> Homo Sapiens

<400> 430

```
        Lys Asn Pro Asn Phe Asp Ile Cys Thr Leu Phe Met Glu Val Met Leu
        1               5                   10                  15


        Pro Arg
```

<210> 431
<211> 8
<212> PRT
<213> Homo Sapiens

<400> 431

```
                              Lys Pro Phe Pro Glu Asp Leu Lys
                              1               5
```

<210> 432
<211> 19
<212> PRT
<213> Homo Sapiens

<400> 432

```
Lys Pro Leu Met Val Ile His His Leu Glu Asp Cys Gln Tyr Ser Gln
1               5                   10                  15

Ala Leu Lys
```

<210> 433
<211> 21
<212> PRT
<213> Homo Sapiens

<400> 433

```
Lys Gln Asn Ala Tyr Ile Ala Thr Gln Gly Pro Leu Pro Glu Thr Met
1               5                   10                  15

Gly Asp Phe Trp Arg
                20
```

<210> 434
<211> 8
<212> PRT
<213> Homo Sapiens

<400> 434

```
                    Lys Arg Ala Glu Ser Asp Ser Arg
                    1               5
```

<210> 435
<211> 18
<212> PRT
<213> Homo Sapiens

<400> 435

```
Lys Ser Asp Leu Asp Thr Ser Lys Pro Leu Ser Glu Lys Pro Ile Thr
1               5                   10                  15

His Lys
```

<210> 436
<211> 7
<212> PRT
<213> Homo Sapiens

<400> 436

```
                    Lys Thr Asp Ala Phe Arg Arg
                    1               5
```

<210> 437
<211> 6
<212> PRT
<213> Homo Sapiens

<400> 437

```
Lys Thr Glu Glu Lys Lys
1               5
```

<210> 438
<211> 15
<212> PRT
<213> Homo Sapiens

<400> 438

```
Lys Thr Met Leu His Leu Thr Asp Ile Gln Leu Gln Asp Asn Lys
1           5               10              15
```

<210> 439
<211> 10
<212> PRT
<213> Homo Sapiens

<400> 439

```
Lys Val Glu Cys Glu His Gly Phe Gly Arg
1           5               10
```

<210> 440
<211> 16
<212> PRT
<213> Homo Sapiens

<400> 440

```
Lys Val Phe Ala Gln Asn Glu Glu Ile Gln Glu Met Ala Gln Asn Lys
1           5               10              15
```

<210> 441
<211> 7
<212> PRT
<213> Homo Sapiens

<400> 441

```
Lys Val His Gly Leu Tyr Arg
1               5
```

<210> 442
<211> 8
<212> PRT
<213> Homo Sapiens

<400> 442

```
Lys Tyr Phe Trp Thr Gly Leu Arg
1               5
```

<210> 443
<211> 5
<212> PRT

<213> Homo Sapiens

<400> 443

```
                              Lys Tyr Trp Cys Arg
                              1               5
```

<210> 444
<211> 9
<212> PRT
<213> Homo Sapiens

<400> 444

```
                        Leu Ala Thr Glu Glu Pro Pro Pro Arg
                        1               5
```

<210> 445
<211> 16
<212> PRT
<213> Homo Sapiens

<400> 445

```
        Leu Asp Cys Gln Val Gln Gly Arg Pro Gln Pro Glu Val Thr Trp Arg
        1               5               10              15
```

<210> 446
<211> 8
<212> PRT
<213> Homo Sapiens

<400> 446

```
                        Leu Asp Glu Asp Leu His Val Lys
                        1               5
```

<210> 447
<211> 5
<212> PRT
<213> Homo Sapiens

<400> 447

```
                              Leu Asp His Tyr Arg
                              1               5
```

<210> 448
<211> 16
<212> PRT
<213> Homo Sapiens

<400> 448

```
        Leu Asp Thr Glu Val Ala Asn Leu Ser Val Ile Met Glu Glu Met Lys
        1               5               10              15
```

<210> 449

<211> 10
<212> PRT
<213> Homo Sapiens

<400> 449

```
                      Leu Glu Asp Pro His Val Asp Ile Ile Arg
                      1               5                   10
```

<210> 450
<211> 6
<212> PRT
<213> Homo Sapiens

<400> 450

```
                          Leu Glu Glu Glu Gln Lys
                          1               5
```

<210> 451
<211> 9
<212> PRT
<213> Homo Sapiens

<400> 451

```
                      Leu Glu Glu Gly Pro Pro His Thr Lys
                      1               5
```

<210> 452
<211> 11
<212> PRT
<213> Homo Sapiens

<400> 452

```
                  Leu Glu Glu Gly Gln Ala Thr Ser Trp Ser Arg
                  1               5                   10
```

<210> 453
<211> 9
<212> PRT
<213> Homo Sapiens

<400> 453

```
                      Leu Glu Glu Gln Asp Glu Phe Glu Lys
                      1               5
```

<210> 454
<211> 7
<212> PRT
<213> Homo Sapiens

<400> 454

```
                          Leu Glu Gly Val Ile Ser Lys
                          1               5
```

<210> 455
<211> 7
<212> PRT
<213> Homo Sapiens

<400> 455

```
Leu Glu Asn Gly Glu Pro Arg
1               5
```

<210> 456
<211> 7
<212> PRT
<213> Homo Sapiens

<400> 456

```
Leu Glu Thr Ala Asp Leu Lys
1               5
```

<210> 457
<211> 15
<212> PRT
<213> Homo Sapiens

<400> 457

```
Leu Glu Trp Leu Thr Leu Met Pro Asn Ala Ser Asn Leu Asp Lys
1           5               10                  15
```

<210> 458
<211> 9
<212> PRT
<213> Homo Sapiens

<400> 458

```
Leu Phe His Ala Ser Tyr Gly Ala Arg
1               5
```

<210> 459
<211> 10
<212> PRT
<213> Homo Sapiens

<400> 459

```
Leu Phe His Ala Ser Tyr Gly Ala Arg Arg
1               5                   10
```

<210> 460
<211> 8
<212> PRT
<213> Homo Sapiens

<400> 460

```
                        Leu Phe His Leu His Ser Gln Lys
                        1               5
```

<210> 461
<211> 8
<212> PRT
<213> Homo Sapiens

<400> 461

```
                        Leu Gly Asp Gln Thr Gly Lys Lys
                        1               5
```

<210> 462
<211> 18
<212> PRT
<213> Homo Sapiens

<400> 462

```
            Leu Gly Gln Val Pro Pro Gly Glu Ser Val Thr Ala Met Glu Leu Glu
            1           5               10              15

            Phe Lys
```

<210> 463
<211> 10
<212> PRT
<213> Homo Sapiens

<400> 463

```
            Leu Gly Thr Ala Met Ser His Glu Ile Arg
            1           5               10
```

<210> 464
<211> 9
<212> PRT
<213> Homo Sapiens

<400> 464

```
            Leu Gly Val Leu His Val Gly Gln Lys
            1           5
```

<210> 465
<211> 6
<212> PRT
<213> Homo Sapiens

<400> 465

```
            Leu His Glu Asp Asp Lys
            1           5
```

<210> 466
<211> 7

<212> PRT
<213> Homo Sapiens

<400> 466

```
                              Leu His Ile Thr Pro Glu Lys
                              1               5
```

<210> 467
<211> 18
<212> PRT
<213> Homo Sapiens

<400> 467

```
        Leu Ile Ala Asp Leu Gln Pro Asn Thr Glu Tyr Ser Phe Val Leu Met
        1               5                   10                  15

        Asn Arg
```

<210> 468
<211> 10
<212> PRT
<213> Homo Sapiens

<400> 468

```
                    Leu Leu Gln Glu Ala Tyr Met Thr Pro Lys
                    1               5                   10
```

<210> 469
<211> 12
<212> PRT
<213> Homo Sapiens

<400> 469

```
                Leu Leu Ser Asp Lys Pro Gln Asp Phe Gln Ile Arg
                1               5                   10
```

<210> 470
<211> 11
<212> PRT
<213> Homo Sapiens

<400> 470

```
                Leu Met Glu Trp Thr Ser Leu Gln Asn Met Arg
                1               5                   10
```

<210> 471
<211> 6
<212> PRT
<213> Homo Sapiens

<400> 471

```
                        Leu Asn Glu Leu Leu Lys
                        1               5
```

<210> 472
<211> 8
<212> PRT
<213> Homo Sapiens


<400> 472

```
                      Leu Asn Glu Val Ile Val Thr Arg
                      1               5
```

<210> 473
<211> 19
<212> PRT
<213> Homo Sapiens


<400> 473

```
        Leu Asn Gly Ser Ser Leu His Leu Glu Trp Ser Ala Pro Leu Glu Ser
        1               5                 10                  15
```

```
        Gly Gly Arg
```

<210> 474
<211> 6
<212> PRT
<213> Homo Sapiens


<400> 474

```
                        Leu Asn Val Glu Glu Arg
                        1               5
```

<210> 475
<211> 9
<212> PRT
<213> Homo Sapiens


<400> 475

```
                  Leu Asn Tyr Gln Thr Pro Gly Met Arg
                  1               5
```

<210> 476
<211> 7
<212> PRT
<213> Homo Sapiens


<400> 476

```
                        Leu Pro Asp Phe Glu Ser Arg
                        1               5
```

<210> 477
<211> 20

<212> PRT
<213> Homo Sapiens

<400> 477

```
        Leu Pro Gly Gly Gln Trp Ile Tyr Met Ser Asp Asn Tyr Thr Asp Val
        1               5               10                  15


        Asn Gly Glu Lys
                    20
```

<210> 478
<211> 7
<212> PRT
<213> Homo Sapiens

<400> 478

```
                        Leu Pro Gly His Gln Lys Arg
                        1               5
```

<210> 479
<211> 21
<212> PRT
<213> Homo Sapiens

<400> 479

```
        Leu Pro Pro Pro Pro Asp Cys Pro Thr Ser Leu His Gln Leu Met Leu
        1               5               10                  15


        Asp Cys Trp Gln Lys
                    20
```

<210> 480
<211> 7
<212> PRT
<213> Homo Sapiens

<400> 480

```
                        Leu Pro Gln Gly Glu Ser Arg
                        1               5
```

<210> 481
<211> 13
<212> PRT
<213> Homo Sapiens

<400> 481

```
            Leu Pro Ser Thr Ser Gly Ser Glu Gly Val Pro Phe Arg
            1               5               10
```

<210> 482
<211> 14

<212> PRT
<213> Homo Sapiens

<400> 482

```
Leu Pro Thr Gly Gly Cys Tyr Gly Val Pro Pro Asp Leu Arg
1               5                   10
```

<210> 483
<211> 23
<212> PRT
<213> Homo Sapiens

<400> 483

```
Leu Pro Thr Pro Met Asp Cys Pro Ser Ala Ile Tyr Gln Leu Met Met
1               5                   10                  15

Gln Cys Trp Gln Gln Glu Arg
                20
```

<210> 484
<211> 9
<212> PRT
<213> Homo Sapiens

<400> 484

```
Leu Pro Val Gly Leu Tyr Phe Ile Lys
1               5
```

<210> 485
<211> 11
<212> PRT
<213> Homo Sapiens

<400> 485

```
Leu Gln Ala Ser Gly Asp Ala Leu Val Asp Arg
1               5                   10
```

<210> 486
<211> 22
<212> PRT
<213> Homo Sapiens

<400> 486

```
Leu Ser Leu Leu Glu Glu Pro Gly Asn Gly Thr Phe Thr Val Ile Leu
1               5                   10                  15

Asn Gln Leu Thr Ser Arg
                20
```

<210> 487
<211> 10

<212> PRT
<213> Homo Sapiens

<400> 487

```
Leu Ser Leu Val Leu Val Pro Ala Gln Lys
1               5               10
```

<210> 488
<211> 16
<212> PRT
<213> Homo Sapiens

<400> 488

```
Leu Ser Asn Thr Ser Pro Glu Phe Gln Glu Met Ser Leu Leu Glu Arg
1               5               10                  15
```

<210> 489
<211> 10
<212> PRT
<213> Homo Sapiens

<400> 489

```
Leu Ser Pro Tyr Val His Tyr Thr Phe Arg
1               5               10
```

<210> 490
<211> 10
<212> PRT
<213> Homo Sapiens

<400> 490

```
Leu Ser Gln Cys His Glu Leu Trp Glu Arg
1               5               10
```

<210> 491
<211> 13
<212> PRT
<213> Homo Sapiens

<400> 491

```
Leu Ser Val Phe Ala Glu Thr Tyr Glu Asn Glu Thr Lys
1               5               10
```

<210> 492
<211> 23
<212> PRT
<213> Homo Sapiens

<400> 492

```
Leu Ser Val Leu Glu Glu Glu Gln Leu Pro Pro Gly Phe Pro Ser Ile
```

1 5 10 15

Asp Met Gly Pro Gln Leu Lys
20

<210> 493
<211> 11
<212> PRT
<213> Homo Sapiens

<400> 493

Leu Ser Trp Tyr Asp Pro Asp Phe Gln Ala Arg
1 5 10

<210> 494
<211> 13
<212> PRT
<213> Homo Sapiens

<400> 494

Leu Thr Ala Asp Leu Ser Leu Asp Thr Leu Asp Ala Arg
1 5 10

<210> 495
<211> 9
<212> PRT
<213> Homo Sapiens

<400> 495

Leu Thr Ala Val Glu Ser Asp Leu Lys
1 5

<210> 496
<211> 15
<212> PRT
<213> Homo Sapiens

<400> 496

Leu Thr Asp Val Val Ile Gly Ala Pro Gly Glu Glu Glu Asn Arg
1 5 10 15

<210> 497
<211> 9
<212> PRT
<213> Homo Sapiens

<400> 497

Leu Thr Pro Gly Leu Tyr Glu Phe Lys
1 5

<210> 498
<211> 18

<212> PRT
<213> Homo Sapiens

<400> 498

```
Leu Thr Gln Ile Glu Thr Gly Glu Asn Val Thr Gly Met Glu Leu Glu
1               5                   10                  15

Phe Lys
```

<210> 499
<211> 6
<212> PRT
<213> Homo Sapiens

<400> 499

```
Leu Thr Tyr Ser Ser Arg
1               5
```

<210> 500
<211> 9
<212> PRT
<213> Homo Sapiens

<400> 500

```
Leu Val His Ile Ile Gly Ala Leu Arg
1               5
```

<210> 501
<211> 11
<212> PRT
<213> Homo Sapiens

<400> 501

```
Leu Val Leu Leu Asn Met Pro Gly Pro Pro Arg
1               5                   10
```

<210> 502
<211> 15
<212> PRT
<213> Homo Sapiens

<400> 502

```
Leu Val Leu Ser Asp Leu His Leu Leu Thr Gln Ser Gln Val Arg
1               5                   10                  15
```

<210> 503
<211> 11
<212> PRT
<213> Homo Sapiens

<400> 503

```
Leu Val Asn Ile Met Pro Tyr Glu Ser Thr Arg
1               5                   10
```

<210> 504
<211> 12
<212> PRT
<213> Homo Sapiens


<400> 504

```
Leu Val Ser Tyr Thr Asn Leu Thr Gln Gly Ala Lys
1               5                   10
```

<210> 505
<211> 7
<212> PRT
<213> Homo Sapiens


<400> 505

```
Leu Trp Met Asn Val Glu Lys
1               5
```

<210> 506
<211> 7
<212> PRT
<213> Homo Sapiens


<400> 506

```
Leu Tyr Thr Tyr Glu Pro Arg
1               5
```

<210> 507
<211> 6
<212> PRT
<213> Homo Sapiens


<400> 507

```
Met Ala Phe Asp Phe Arg
1               5
```

<210> 508
<211> 11
<212> PRT
<213> Homo Sapiens


<400> 508

```
Met Ala Leu His Leu Pro Trp Phe His Pro Arg
1               5                   10
```

<210> 509
<211> 9
<212> PRT
<213> Homo Sapiens

<400> 509

```
                        Met Ala Pro Glu Pro Ala Pro Gly Arg

                                  1                   5
```

<210> 510
<211> 7
<212> PRT
<213> Homo Sapiens

<400> 510

```
                        Met Asp Ile Leu Glu Glu Arg
                        1                   5
```

<210> 511
<211> 8
<212> PRT
<213> Homo Sapiens

<400> 511

```
                        Met Asp Val Gly Thr Ile Tyr Arg
                        1                   5
```

<210> 512
<211> 7
<212> PRT
<213> Homo Sapiens

<400> 512

```
                        Met Glu Leu Gln Ala Ala Arg
                        1                   5
```

<210> 513
<211> 6
<212> PRT
<213> Homo Sapiens

<400> 513

```
                        Met Glu Pro Leu Glu Lys
                        1                   5
```

<210> 514
<211> 9
<212> PRT
<213> Homo Sapiens

<400> 514

```
                        Met Glu Ser Glu Glu Leu Ala Asp Arg
                        1                   5
```

<210> 515
<211> 12
<212> PRT
<213> Homo Sapiens

<400> 515

```
Met Phe Glu Leu Thr Cys Thr Leu Pro Leu Glu Lys
1               5                   10
```

<210> 516
<211> 17
<212> PRT
<213> Homo Sapiens

<400> 516

```
Met Gly Gln Pro Pro Ala Glu Glu Ala Glu Gln Pro Gly Ala Leu Ala
1               5                   10                  15

Arg
```

<210> 517
<211> 6
<212> PRT
<213> Homo Sapiens

<400> 517

```
Met His Thr Ala Val Lys
1               5
```

<210> 518
<211> 22
<212> PRT
<213> Homo Sapiens

<400> 518

```
Met Leu Ser Ala Ser Thr Met Leu Val Gln Trp Glu Pro Pro Glu Glu
1               5                   10                  15

Pro Asn Gly Leu Val Arg
                20
```

<210> 519
<211> 15
<212> PRT
<213> Homo Sapiens

<400> 519

```
Met Leu Trp Glu His Asn Ser Thr Ile Ile Val Met Leu Thr Lys
1               5                   10                  15
```

<210> 520

<211> 15
<212> PRT
<213> Homo Sapiens

<400> 520

```
Met Leu Trp Glu His Asn Ser Thr Ile Val Val Met Leu Thr Lys
1               5                   10                  15
```

<210> 521
<211> 13
<212> PRT
<213> Homo Sapiens

<400> 521

```
Met Pro His Phe Thr Val Val Pro Val Asp Gly Pro Arg
1                   5                   10
```

<210> 522
<211> 26
<212> PRT
<213> Homo Sapiens

<400> 522

```
Met Pro Asn Val Pro Asn Thr Gln Pro Ala Ile Met Lys Pro Thr Glu
1               5                   10                  15

Glu His Pro Ala Tyr Thr Gln Ile Ala Lys
            20                  25
```

<210> 523
<211> 18
<212> PRT
<213> Homo Sapiens

<400> 523

```
Met Gln Gln Tyr Thr Glu His Phe Met Ala Ala Gly Tyr Thr Ala Ile
1               5                   10                  15

Glu Lys
```

<210> 524
<211> 22
<212> PRT
<213> Homo Sapiens

<400> 524

```
Met Ser Ala Gly Gly Ala Ser Val Pro Pro Pro Pro Asn Pro Ala Val
1               5                   10                  15

Ser Phe Pro Pro Pro Arg
                20
```

<210> 525
<211> 11
<212> PRT
<213> Homo Sapiens

<400> 525

```
                    Met Ser Asp Val Ser Thr Ser Val Gln Ser Lys
                    1               5                   10
```

<210> 526
<211> 16
<212> PRT
<213> Homo Sapiens

<400> 526

```
        Met Thr Thr Ser Gly Ala Leu Phe Pro Ser Leu Val Pro Gly Ser Arg
        1               5                   10                  15
```

<210> 527
<211> 8
<212> PRT
<213> Homo Sapiens

<400> 527

```
                        Met Val Glu Glu Val Asp Gly Arg
                        1               5
```

<210> 528
<211> 15
<212> PRT
<213> Homo Sapiens

<400> 528

```
        Met Val Phe Phe Val Gln Asn Glu Pro Pro His Gln Ile Phe Lys
        1               5                   10                  15
```

<210> 529
<211> 6
<212> PRT
<213> Homo Sapiens

<400> 529

```
                        Met Val Trp Glu Gln Arg
                        1               5
```

<210> 530

<211> 16
<212> PRT
<213> Homo Sapiens

<400> 530

```
Met Tyr Pro Glu Thr Phe Leu Tyr Val Thr Ser Ser Ala Gly Ile Arg
1               5                   10                  15
```

<210> 531
<211> 6
<212> PRT
<213> Homo Sapiens

<400> 531

```
Met Tyr Tyr Thr His Arg
1                   5
```

<210> 532
<211> 7
<212> PRT
<213> Homo Sapiens

<400> 532

```
Met Tyr Tyr Thr His Arg Arg
1                   5
```

<210> 533
<211> 8
<212> PRT
<213> Homo Sapiens

<400> 533

```
Met Tyr Tyr Thr His Arg Arg Arg
1                   5
```

<210> 534
<211> 14
<212> PRT
<213> Homo Sapiens

<400> 534

```
Asn Asp Phe Gln Asn Leu Gln Gln Val Phe Leu Gln Ala Lys
1               5                   10
```

<210> 535
<211> 10
<212> PRT
<213> Homo Sapiens

<400> 535

```
Asn Phe Ala Thr Met Met Asn Phe Val Arg
1               5                   10
```

<210> 536
<211> 8
<212> PRT
<213> Homo Sapiens

<400> 536

```
                        Asn Gly Val Gly Gly Met Ala Lys
                        1               5
```

<210> 537
<211> 19
<212> PRT
<213> Homo Sapiens

<400> 537

```
        Asn Gly Val Ile Thr Gln Tyr Ser Val Ala Tyr Glu Ala Val Asp Gly
        1               5                   10                  15


        Glu Asp Arg
```

<210> 538
<211> 10
<212> PRT
<213> Homo Sapiens

<400> 538

```
                    Asn Gly Val Ser Gly Leu Val Thr Ser Arg
                    1               5                   10
```

<210> 539
<211> 11
<212> PRT
<213> Homo Sapiens

<400> 539

```
                    Asn Ile Ala Phe Tyr Pro Ser Asn His Glu Arg
                    1               5                   10
```

<210> 540
<211> 6
<212> PRT
<213> Homo Sapiens

<400> 540

```
                        Asn Ile Phe Tyr Ile Lys
                        1               5
```

<210> 541
<211> 14
<212> PRT
<213> Homo Sapiens

<400> 541

```
Asn Leu Ala Leu Phe Glu Glu Glu Leu Asp Ile Arg Pro Lys
1               5                   10
```

<210> 542
<211> 15
<212> PRT
<213> Homo Sapiens

<400> 542

```
Asn Leu Ile Ala Phe Ser Glu Asp Gly Ser Asp Pro Tyr Val Arg
1               5                   10                  15
```

<210> 543
<211> 8
<212> PRT
<213> Homo Sapiens

<400> 543

```
Asn Leu Ile Thr Asn Leu Gln Arg
1                   5
```

<210> 544
<211> 13
<212> PRT
<213> Homo Sapiens

<400> 544

```
Asn Leu Gln Ser Gln Ser Ser Val Asn Val Ile Val Lys
1               5                   10
```

<210> 545
<211> 12
<212> PRT
<213> Homo Sapiens

<400> 545

```
Asn Leu Arg Pro Met Leu Ala Ala Asp Ala Gln Arg
1               5                   10
```

<210> 546
<211> 11
<212> PRT
<213> Homo Sapiens

<400> 546

```
Asn Leu Ser Pro Asp Gly Gln Tyr Val Pro Arg
1               5                   10
```

<210> 547
<211> 14
<212> PRT

<213> Homo Sapiens

<400> 547

Asn Leu Val Asp Pro Phe Val Glu Val Ser Phe Ala Gly Lys
1                   5                   10

<210> 548
<211> 19
<212> PRT
<213> Homo Sapiens

<400> 548

Asn Met Tyr Leu Thr Gly Leu Cys Phe Leu Leu Gly Pro Thr Gln Leu
1                   5                   10                  15

Thr Gln Arg

<210> 549
<211> 6
<212> PRT
<213> Homo Sapiens

<400> 549

Asn Asn Glu Pro Leu Arg
1                   5

<210> 550
<211> 8
<212> PRT
<213> Homo Sapiens

<400> 550

Asn Asn Phe Ser Asp Gly Phe Arg
1                   5

<210> 551
<211> 17
<212> PRT
<213> Homo Sapiens

<400> 551

Asn Pro Asn Phe Asp Ile Cys Thr Leu Phe Met Glu Val Met Leu Pro
1                   5                   10                  15

Arg

<210> 552
<211> 13
<212> PRT
<213> Homo Sapiens

<400> 552

```
            Asn Pro Val Leu Asp Cys Ser Ile Ala Gly Cys Leu Arg
            1               5                   10
```

<210> 553
<211> 21
<212> PRT
<213> Homo Sapiens

<400> 553

```
        Asn Ser Glu Gly Asp Glu Asn Tyr Met Glu Phe Leu Glu Val Leu Thr
        1               5                   10                  15

        Glu Gly Leu Glu Arg

                        20
```

<210> 554
<211> 6
<212> PRT
<213> Homo Sapiens

<400> 554

```
                            Asn Ser Tyr Cys Lys Lys
                            1               5
```

<210> 555
<211> 15
<212> PRT
<213> Homo Sapiens

<400> 555

```
        Asn Val Gly Leu Met Ile Cys Gly His Val His Met Gly Pro Arg
        1               5                   10                  15
```

<210> 556
<211> 16
<212> PRT
<213> Homo Sapiens

<400> 556

```
        Asn Val Gly Leu Met Ile Cys Gly His Val His Met Gly Pro Arg Arg
        1               5                   10                  15
```

<210> 557
<211> 9
<212> PRT
<213> Homo Sapiens

<400> 557

```
                    Asn Val His Leu Glu Phe Thr Glu Arg
                    1                   5
```

<210> 558
<211> 10
<212> PRT
<213> Homo Sapiens


<400> 558

```
                    Asn Val Leu Glu Leu Ser Asn Val Val Arg
                    1               5               10
```

<210> 559
<211> 8
<212> PRT
<213> Homo Sapiens


<400> 559

```
                    Asn Val Pro Asp Asp Val Leu Arg
                    1               5
```

<210> 560
<211> 16
<212> PRT
<213> Homo Sapiens


<400> 560

```
        Asn Val Pro Pro Gly Leu Asp Glu Tyr Asn Pro Phe Ser Asp Ser Arg
        1               5               10                  15
```

<210> 561
<211> 10
<212> PRT
<213> Homo Sapiens


<400> 561

```
                    Asn Val Ser Gly Phe Ser Ile Ala Asn Arg
                    1               5               10
```

<210> 562
<211> 22
<212> PRT
<213> Homo Sapiens


<400> 562

```
        Asn Val Ser Val Gln Pro Glu Ile Ser Glu Gly Leu Ala Thr Thr Pro
        1               5               10                  15

        Ser Thr Gln Gln Val Lys
                        20
```

<210> 563

<211> 7
<212> PRT
<213> Homo Sapiens

<400> 563

```
                              Asn Trp Glu Glu Ala Glu Arg
                              1                   5
```

<210> 564
<211> 7
<212> PRT
<213> Homo Sapiens

<400> 564

```
                              Asn Tyr Thr Ile Phe Tyr Arg
                              1                   5
```

<210> 565
<211> 6
<212> PRT
<213> Homo Sapiens

<400> 565

```
                              Gln Cys Leu Ile Lys Lys
                              1                   5
```

<210> 566
<211> 6
<212> PRT
<213> Homo Sapiens

<400> 566

```
                              Gln Glu Glu Leu Glu Arg
                              1                   5
```

<210> 567
<211> 18
<212> PRT
<213> Homo Sapiens

<400> 567

```
        Gln Glu Gln Asp Ile Val Phe Leu Ile Asp Gly Ser Gly Ser Ile Ser
        1               5                   10                  15

        Ser Arg
```

<210> 568
<211> 13
<212> PRT
<213> Homo Sapiens

<400> 568

Gln Glu Ser Thr Ser Val Leu Leu Gln Gln Ser Glu Lys
1               5                   10

<210> 569
<211> 14
<212> PRT
<213> Homo Sapiens

<400> 569

Gln Glu Ser Thr Ser Val Leu Leu Gln Gln Ser Glu Lys Lys
1               5                   10

<210> 570
<211> 16
<212> PRT
<213> Homo Sapiens

<400> 570

Gln Phe His Gln Leu Ala Ala Gln Gly Pro Gln Glu Cys Leu Val Arg

    1           5               10              15

<210> 571
<211> 24
<212> PRT
<213> Homo Sapiens

<400> 571

Gln Phe Gln Phe Met Ala Trp Pro Asp His Gly Val Pro Glu Tyr Pro
1           5               10              15

Thr Pro Ile Leu Ala Phe Leu Arg
            20

<210> 572
<211> 14
<212> PRT
<213> Homo Sapiens

<400> 572

Gln Phe Gln Phe Thr Asp Trp Pro Glu Gln Gly Val Pro Lys
1           5               10

<210> 573
<211> 15
<212> PRT
<213> Homo Sapiens

<400> 573

```
Gln Gly Ala Gln Trp Ala Ala Leu Cys Asp Ser Ser Ser Ala Arg
1               5                   10                  15
```

<210> 574
<211> 18
<212> PRT
<213> Homo Sapiens

<400> 574

```
Gln Gly His Phe Tyr Gly Glu Thr Ala Ala Val Tyr Val Ala Val Glu
1               5                   10                  15
```

```
Glu Arg
```

<210> 575
<211> 19
<212> PRT
<213> Homo Sapiens

<400> 575

```
Gln Gly His Phe Tyr Gly Glu Thr Ala Ala Val Tyr Val Ala Val Glu
1               5                   10                  15
```

```
Glu Arg Lys
```

<210> 576
<211> 12
<212> PRT
<213> Homo Sapiens

<400> 576

```
Gln Gly Leu Thr Val Ser Phe Ile Pro Tyr Phe Lys
1               5                   10
```

<210> 577
<211> 9
<212> PRT
<213> Homo Sapiens

<400> 577

```
Gln Gly Pro Val Gly Ser Gly Arg Arg
1               5
```

<210> 578
<211> 6
<212> PRT
<213> Homo Sapiens

<400> 578

```
Gln His Gly Gln Ile Arg
1               5
```

<210> 579
<211> 9
<212> PRT
<213> Homo Sapiens

<400> 579

```
                        Gln Ile Phe Gly Phe Glu Ser Asn Lys
                        1                   5
```

<210> 580
<211> 19
<212> PRT
<213> Homo Sapiens

<400> 580

```
        Gln Ile Gly Pro Gly Glu Ser Cys Pro Asp Gly Val Thr His Ser Ile
        1               5                   10                  15


        Ser Gly Arg
```

<210> 581
<211> 20
<212> PRT
<213> Homo Sapiens

<400> 581

```
        Gln Asn Ala Tyr Ile Ala Thr Gln Gly Pro Leu Pro Glu Thr Met Gly
        1               5                   10                  15


        Asp Phe Trp Arg
                    20
```

<210> 582
<211> 17
<212> PRT
<213> Homo Sapiens

<400> 582

```
        Gln Asn Cys Ser Gln His Glu Ser Ser Pro Asp Ile Ser His Phe Glu
        1               5                   10                  15


        Arg
```

<210> 583
<211> 27
<212> PRT
<213> Homo Sapiens

<400> 583

```
Gln Pro Gly Asn Met Ala Gly Asn Phe Asn Leu Ser Leu Gln Gly Cys
1               5               10              15
```

```
Asp Gln Asp Ala Gln Ser Pro Gly Ile Leu Arg
            20              25
```

<210> 584
<211> 7
<212> PRT
<213> Homo Sapiens

<400> 584

```
Gln Pro Gly Ser Val Pro Arg
1               5
```

<210> 585
<211> 15
<212> PRT
<213> Homo Sapiens

<400> 585

```
Gln Pro His Tyr Ser Ala Phe Gly Ser Val Gly Glu Trp Leu Arg
1               5               10              15
```

<210> 586
<211> 15
<212> PRT
<213> Homo Sapiens

<400> 586

```
Gln Pro Gln Gln Phe Pro Ser Arg Pro Pro Pro Pro Gln Pro Lys
1               5               10              15
```

<210> 587
<211> 10
<212> PRT
<213> Homo Sapiens

<400> 587

```
Gln Ser Pro Glu Asp Val Tyr Phe Ser Lys
1               5               10
```

<210> 588
<211> 16
<212> PRT
<213> Homo Sapiens

<400> 588

```
Gln Ser Ser Gly Glu Asn Cys Asp Val Val Val Asn Thr Leu Gly Lys
1               5               10              15
```

<210> 589

<211> 17
<212> PRT
<213> Homo Sapiens

<400> 589

```
Gln Ser Ser Gly Glu Asn Cys Asp Val Val Val Asn Thr Leu Gly Lys
1               5                   10                  15

Arg
```

<210> 590
<211> 10
<212> PRT
<213> Homo Sapiens

<400> 590

```
Gln Val Ile Ile Asn Leu Ile Asn Gln Lys
1               5                   10
```

<210> 591
<211> 12
<212> PRT
<213> Homo Sapiens

<400> 591

```
Gln Val Ser Ser Val Asn Glu Glu Asp Phe Val Arg
1               5                   10
```

<210> 592
<211> 10
<212> PRT
<213> Homo Sapiens

<400> 592

```
Gln Trp Ser Asn Val Phe Asn Ile Leu Arg
1               5                   10
```

<210> 593
<211> 9
<212> PRT
<213> Homo Sapiens

<400> 593

```
Gln Tyr Ala Gly Thr Gly Ala Leu Lys
1               5
```

<210> 594
<211> 16
<212> PRT
<213> Homo Sapiens

<400> 594

```
Gln Tyr Asn Ser Phe Asn Phe Ala Leu Leu Glu Asp Val Gln Ala Lys
1               5                   10                  15
```

<210> 595
<211> 8
<212> PRT
<213> Homo Sapiens

<400> 595

```
                        Arg Ala Glu Ser Asp Ser Arg Lys
                        1               5
```

<210> 596
<211> 9
<212> PRT
<213> Homo Sapiens

<400> 596

```
                        Arg Ala Met Ala Thr Leu Leu Ser Lys
                        1               5
```

<210> 597
<211> 8
<212> PRT
<213> Homo Sapiens

<400> 597

```
                        Arg Ala Pro Ala Phe Glu Gly Arg
                        1               5
```

<210> 598
<211> 6
<212> PRT
<213> Homo Sapiens

<400> 598

```
                        Arg Asp Leu Gly Ser Arg
                        1               5
```

<210> 599
<211> 10
<212> PRT
<213> Homo Sapiens

<400> 599

```
                        Arg Asp Leu Ser Gln Met Glu Ala Leu Lys
                        1               5                   10
```

<210> 600
<211> 6
<212> PRT
<213> Homo Sapiens

<400> 600

Arg Asp Gln Leu Trp Arg
1               5

<210> 601
<211> 9
<212> PRT
<213> Homo Sapiens

<400> 601

Arg Glu Ile Thr Tyr Gln Gly Thr Arg
1               5

<210> 602
<211> 9
<212> PRT
<213> Homo Sapiens

<400> 602

Arg Glu Gln Leu Thr Glu Thr Asp Lys
1               5

<210> 603
<211> 12
<212> PRT
<213> Homo Sapiens

<400> 603

Arg Gly Asp Phe Phe Tyr His Ser Glu Asn Pro Lys
1               5                         10

<210> 604
<211> 8
<212> PRT
<213> Homo Sapiens

<400> 604

Arg His Gly Glu Thr Cys Tyr Lys
1               5

<210> 605
<211> 5
<212> PRT
<213> Homo Sapiens

<400> 605

Arg Lys Asn Gln Arg
1               5

<210> 606
<211> 10
<212> PRT

<213> Homo Sapiens

<400> 606

```
                    Arg Leu Asn Tyr Gln Thr Pro Gly Met Arg
                    1               5                   10
```

<210> 607
<211> 7
<212> PRT
<213> Homo Sapiens

<400> 607

```
                        Arg Met Glu Pro Leu Glu Lys
                        1               5
```

<210> 608
<211> 14
<212> PRT
<213> Homo Sapiens

<400> 608

```
                Arg Pro Asp Thr Ser Phe Leu Trp Phe Thr Ser Pro Tyr Lys
                1               5                   10
```

<210> 609
<211> 8
<212> PRT
<213> Homo Sapiens

<400> 609

```
                        Arg Pro Pro Asn Ala Trp His Lys
                        1               5
```

<210> 610
<211> 8
<212> PRT
<213> Homo Sapiens

<400> 610

```
                        Arg Pro Pro Gln Thr Leu Ser Arg
                        1               5
```

<210> 611
<211> 9
<212> PRT
<213> Homo Sapiens

<400> 611

```
                    Arg Gln Asp Leu Glu Val Glu Val Arg
                    1               5
```

<210> 612

<211> 7
<212> PRT
<213> Homo Sapiens

<400> 612

```
                          Arg Gln Glu Glu Leu Glu Arg
                          1               5
```

<210> 613
<211> 8
<212> PRT
<213> Homo Sapiens

<400> 613

```
                       Arg Gln Glu Glu Leu Glu Arg Lys
                       1               5
```

<210> 614
<211> 6
<212> PRT
<213> Homo Sapiens

<400> 614

```
                        Arg Arg Gln Ala Glu Arg
                        1               5
```

<210> 615
<211> 16
<212> PRT
<213> Homo Sapiens

<400> 615

```
      Arg Thr His Leu Gly Leu Ile Met Asp Gly Trp Asn Ser Met Ile Arg
      1           5               10                  15
```

<210> 616
<211> 8
<212> PRT
<213> Homo Sapiens

<400> 616

```
                       Arg Thr His Ser Pro Ser Ser Lys
                       1               5
```

<210> 617
<211> 11
<212> PRT
<213> Homo Sapiens

<400> 617

```
             Arg Thr Leu Glu Gln Met Asp Val Val His Arg
             1               5                   10
```

<210> 618
<211> 6
<212> PRT
<213> Homo Sapiens

<400> 618

```
Arg Thr Pro Thr Ala Arg
1               5
```

<210> 619
<211> 24
<212> PRT
<213> Homo Sapiens

<400> 619

```
Ser Ala Asn Tyr Thr Cys Val Ala Ile Ser Ser Leu Gly Met Ile Glu
1               5                   10                  15

Ala Thr Ala Gln Val Thr Val Lys
                20
```

<210> 620
<211> 14
<212> PRT
<213> Homo Sapiens

<400> 620

```
Ser Cys Ser Leu Val Leu Glu His Gln Pro Asp Asn Ile Lys
1               5                   10
```

<210> 621
<211> 15
<212> PRT
<213> Homo Sapiens

<400> 621

```
Ser Asp Met Gly Val Gly Val Phe Thr Pro Thr Ile Glu Ala Arg
1               5                   10                  15
```

<210> 622
<211> 8
<212> PRT
<213> Homo Sapiens

<400> 622

```
Ser Asp Pro Tyr Gly Ile Ile Arg
1               5
```

<210> 623
<211> 12
<212> PRT
<213> Homo Sapiens

<400> 623

```
          Ser Asp Gln Ala Leu His Ser Phe Ser Glu Ala Lys
          1               5                   10
```

<210> 624
<211> 8
<212> PRT
<213> Homo Sapiens

<400> 624

```
             Ser Asp Ser Ala Val Leu Leu Arg
             1               5
```

<210> 625
<211> 12
<212> PRT
<213> Homo Sapiens

<400> 625

```
          Ser Asp Thr Ile Ala Asn Tyr Glu Leu Val Tyr Lys
          1               5                   10
```

<210> 626
<211> 13
<212> PRT
<213> Homo Sapiens

<400> 626

```
          Ser Glu Ile Phe Asn Glu Asn Phe Gly Pro Asp Phe Arg
          1               5                   10
```

<210> 627
<211> 7
<212> PRT
<213> Homo Sapiens

<400> 627

```
             Ser Glu Ile Ile Asn Ser Lys
             1               5
```

<210> 628
<211> 18
<212> PRT
<213> Homo Sapiens

<400> 628

```
       Ser Glu Ile Gln Phe Leu Ile Ser Asp Asn Gln Gly Phe Ser Cys Pro
       1               5                   10                  15

       Glu Lys
```

**447**

<210> 629
<211> 8
<212> PRT
<213> Homo Sapiens

<400> 629

```
                            Ser Glu Gln Leu Lys Pro Leu Lys
                            1               5
```

<210> 630
<211> 9
<212> PRT
<213> Homo Sapiens

<400> 630

```
                        Ser Glu Gln Pro Thr Ala Ser Trp Arg
                        1               5
```

<210> 631
<211> 16
<212> PRT
<213> Homo Sapiens

<400> 631

```
        Ser Gly Ala Leu Gln Ile Glu Ser Ser Glu Glu Ser Asp Gln Gly Lys
        1               5                   10                  15
```

<210> 632
<211> 14
<212> PRT
<213> Homo Sapiens

<400> 632

```
            Ser Gly Glu Gly Phe Ile Asp Phe Ile Gly Gln Val His Lys
            1               5                   10
```

<210> 633
<211> 9
<212> PRT
<213> Homo Sapiens

<400> 633

```
                        Ser Gly Val Val Cys Gln Thr Gly Arg
                        1               5
```

<210> 634
<211> 20
<212> PRT
<213> Homo Sapiens

<400> 634

```
Ser His Ala Glu Asn Pro Thr Ala Ser His Val Asp Asn Glu Tyr Ser
1               5               10              15


Gln Pro Pro Arg
              20
```

<210> 635
<211> 12
<212> PRT
<213> Homo Sapiens

<400> 635

```
Ser His Leu Gln Asn Tyr Thr Val Asn Ala Thr Lys
1               5               10
```

<210> 636
<211> 16
<212> PRT
<213> Homo Sapiens

<400> 636

```
Ser Ile Phe Asp Pro Pro Val Phe Pro Val Cys Met Leu Gly Asn Arg
1               5               10              15
```

<210> 637
<211> 8
<212> PRT
<213> Homo Sapiens

<400> 637

```
Ser Ile Leu Gln Glu Glu Asn Arg
1               5
```

<210> 638
<211> 9
<212> PRT
<213> Homo Sapiens

<400> 638

```
Ser Ile Gln Ile His Gly Thr Met Arg
1               5
```

<210> 639
<211> 11
<212> PRT
<213> Homo Sapiens

<400> 639

```
Ser Leu Glu Met Ser His Asp Glu His Lys Lys
1               5               10
```

<210> 640

<211> 11
<212> PRT
<213> Homo Sapiens

<400> 640

```
Ser Leu Gly Gly Glu Gly Asn Phe Asn Trp Arg
1               5               10
```

<210> 641
<211> 22
<212> PRT
<213> Homo Sapiens

<400> 641

```
Ser Leu His Leu Thr Cys Asp Ser Ala Pro Val Gly Ser Gln Gly Thr
1               5               10              15

Trp Ser Thr Ser Cys Arg
            20
```

<210> 642
<211> 14
<212> PRT
<213> Homo Sapiens

<400> 642

```
Ser Leu Ser Gly Val Gly Ala Gly Gly Gly Pro Thr Pro Arg
1               5               10
```

<210> 643
<211> 13
<212> PRT
<213> Homo Sapiens

<400> 643

```
Ser Leu Ser Asn Tyr Pro Phe Asp Phe Gln Gly Ala Arg
1               5               10
```

<210> 644
<211> 13
<212> PRT
<213> Homo Sapiens

<400> 644

```
Ser Pro Asp Leu Gln Gly Ser Trp Gln Trp Ser Asp Arg
1               5               10
```

<210> 645
<211> 9
<212> PRT
<213> Homo Sapiens

<400> 645

```
Ser Pro Gly Trp Arg Pro Ala Phe Lys
1               5
```

<210> 646
<211> 18
<212> PRT
<213> Homo Sapiens

<400> 646

```
Ser Gln Ala Lys Pro Gly Thr Pro Gly Gly Thr Gly Gly Pro Ala Pro
1               5                   10                  15

Gln Tyr
```

<210> 647
<211> 17
<212> PRT
<213> Homo Sapiens

<400> 647

```
Ser Gln His Gln Glu Thr Pro Val Tyr Leu Gly Ala Thr Ala Gly Met
1               5                   10                  15

Arg
```

<210> 648
<211> 19
<212> PRT
<213> Homo Sapiens

<400> 648

```
Ser Gln His Thr Thr Glu Val Val Gly Gly Ala Pro Gln His Glu Gln
1               5                   10                  15

Ile Gly Lys
```

<210> 649
<211> 19
<212> PRT
<213> Homo Sapiens

<400> 649

```
Ser Ser His Ser Gly Val Leu Glu Ile Glu Asn Ser Val Asp Asp Leu
1               5                   10                  15

Ser Ser Arg
```

<210> 650

<211> 13
<212> PRT
<213> Homo Sapiens

<400> 650

```
Ser Ser Leu Pro Tyr Ile Cys Glu Met Thr Ala Phe Arg
1               5                   10
```

<210> 651
<211> 14
<212> PRT
<213> Homo Sapiens

<400> 651

```
Ser Ser Ser Ser Asn Phe Leu Asn Tyr Asp Leu Thr Leu Arg
1               5                   10
```

<210> 652
<211> 8
<212> PRT
<213> Homo Sapiens

<400> 652

```
Ser Ser Val Ile Asn Ser Ile Arg
1               5
```

<210> 653
<211> 14
<212> PRT
<213> Homo Sapiens

<400> 653

```
Ser Ser Val Ile Ser Ile Tyr Val Ser Glu Ser Met Asp Arg
1               5                   10
```

<210> 654
<211> 15
<212> PRT
<213> Homo Sapiens

<400> 654

```
Ser Thr Glu Glu Phe Pro Pro Leu Gln Pro Ile Leu Gln Gln Lys
1               5                   10                  15
```

<210> 655
<211> 8
<212> PRT
<213> Homo Sapiens

<400> 655

```
Ser Thr Glu Thr Ala Leu Tyr Arg
1               5
```

<210> 656
<211> 9
<212> PRT
<213> Homo Sapiens

<400> 656

```
                    Ser Thr Glu Thr Ala Leu Tyr Arg Lys
                    1                   5
```

<210> 657
<211> 14
<212> PRT
<213> Homo Sapiens

<400> 657

```
            Ser Thr Gly Pro Gly Ala Ser Leu Gly Thr Gly Tyr Asp Arg
            1                   5                   10
```

<210> 658
<211> 15
<212> PRT
<213> Homo Sapiens

<400> 658

```
        Ser Thr Gly Pro Gly Ala Ser Leu Gly Thr Gly Tyr Asp Arg Lys
        1                   5                   10                  15
```

<210> 659
<211> 6
<212> PRT
<213> Homo Sapiens

<400> 659

```
                    Ser Thr Ile Glu Lys Lys
                    1                   5
```

<210> 660
<211> 18
<212> PRT
<213> Homo Sapiens

<400> 660

```
        Ser Thr Thr Ser Leu Ser Val Ser Trp Ser Ile Pro Pro Pro Gln Gln
        1                   5                   10                  15

            Ser Arg
```

<210> 661
<211> 11
<212> PRT
<213> Homo Sapiens

<400> 661

```
Ser Val Asp Asp Thr Ser Gln Ala Ile Gln Arg
1               5                   10
```

<210> 662
<211> 7
<212> PRT
<213> Homo Sapiens

<400> 662

```
Ser Val Gly Pro Leu Thr Arg
1               5
```

<210> 663
<211> 8
<212> PRT
<213> Homo Sapiens

<400> 663

```
Ser Val Gly Pro Leu Thr Arg Lys
1               5
```

<210> 664
<211> 12
<212> PRT
<213> Homo Sapiens

<400> 664

```
Ser Val Leu Gly Val Ile Thr Ile Val Asn Leu Arg
1               5                   10
```

<210> 665
<211> 6
<212> PRT
<213> Homo Sapiens

<400> 665

```
Ser Val Gln Met Met Arg
1               5
```

<210> 666
<211> 5
<212> PRT
<213> Homo Sapiens

<400> 666

```
Ser Tyr Phe Arg Lys
1               5
```

<210> 667
<211> 27
<212> PRT

<213> Homo Sapiens

<400> 667

```
        Ser Tyr Gln Leu Ala Asn Ile Ser Ser Pro Ser Leu Val Val Glu Cys
        1               5                   10                  15

        Gly Gly Gln Thr Val Gln Ser Cys Val Ile Arg
                    20                  25
```

<210> 668
<211> 9
<212> PRT
<213> Homo Sapiens

<400> 668

```
                    Ser Tyr Ser Phe Val Leu Thr Asn Arg
                    1               5
```

<210> 669
<211> 8
<212> PRT
<213> Homo Sapiens

<400> 669

```
                    Thr Ala Leu Leu Asn Met Asp Arg
                    1               5
```

<210> 670
<211> 22
<212> PRT
<213> Homo Sapiens

<400> 670

```
        Thr Ala Asn Pro Gln Trp Asn Gln Asn Ile Thr Leu Pro Ala Met Phe
        1               5                   10                  15

        Pro Ser Met Cys Glu Lys
                    20
```

<210> 671
<211> 21
<212> PRT
<213> Homo Sapiens

<400> 671

```
        Thr Ala Pro Asp Leu Leu Pro His Lys Pro Leu Pro Ala Ser Ala Tyr
        1               5                   10                  15

        Ile Glu Asp Gly Arg
                    20
```

<210> 672
<211> 12
<212> PRT
<213> Homo Sapiens

<400> 672

```
Thr Ala Gln Ser Thr Pro Ser Ala Pro Pro Gln Lys
1               5                   10
```

<210> 673
<211> 13
<212> PRT
<213> Homo Sapiens

<400> 673

```
Thr Ala Ser Val Ser Ile Asn Gln Thr Glu Pro Pro Lys
1               5                   10
```

<210> 674
<211> 20
<212> PRT
<213> Homo Sapiens

<400> 674

```
Thr Ala Thr Met Leu Cys Ala Ala Gly Gly Asn Pro Asp Pro Glu Ile
1               5                   10                  15

Ser Trp Phe Lys
            20
```

<210> 675
<211> 20
<212> PRT
<213> Homo Sapiens

<400> 675

```
Thr Ala Thr Met Leu Cys Ala Ala Ser Gly Asn Pro Asp Pro Glu Ile
1               5                   10                  15

Thr Trp Phe Lys
            20
```

<210> 676
<211> 9
<212> PRT
<213> Homo Sapiens

<400> 676

```
Thr Ala Thr Val Val Met Met Thr Arg
1               5
```

<210> 677
<211> 16
<212> PRT
<213> Homo Sapiens

<400> 677

```
Thr Ala Val Asp Gly Pro Asp Leu Glu Met Leu Thr Gly Gln Glu Arg
1               5                   10                  15
```

<210> 678
<211> 6
<212> PRT
<213> Homo Sapiens

<400> 678

```
Thr Cys Leu Glu Glu Arg
1               5
```

<210> 679
<211> 13
<212> PRT
<213> Homo Sapiens

<400> 679

```
Thr Cys Ser Ser Asn Leu Thr Leu Thr Ser Gly Ser Lys
1               5                   10
```

<210> 680
<211> 7
<212> PRT
<213> Homo Sapiens

<400> 680

```
Thr Asp Ala Phe Arg Arg Arg
1               5
```

<210> 681
<211> 9
<212> PRT
<213> Homo Sapiens

<400> 681

```
Thr Asp Ala Leu Leu Gly Glu Phe Arg
1               5
```

<210> 682
<211> 11
<212> PRT
<213> Homo Sapiens

<400> 682

```
                    Thr Asp Glu Asp Val Pro Ser Gly Pro Pro Arg
                    1               5                   10
```

<210> 683
<211> 12
<212> PRT
<213> Homo Sapiens


<400> 683

```
                    Thr Asp Glu Asp Val Pro Ser Gly Pro Pro Arg Lys
                    1               5                   10
```

<210> 684
<211> 12
<212> PRT
<213> Homo Sapiens


<400> 684

```
                    Thr Asp Ile Ser Met Ser Asp Phe Glu Asn Ser Arg
                    1               5                   10
```

<210> 685
<211> 9
<212> PRT
<213> Homo Sapiens


<400> 685

```
                    Thr Asp Gln Asp Glu Glu His Cys Arg
                    1               5
```

<210> 686
<211> 6
<212> PRT
<213> Homo Sapiens


<400> 686

```
                    Thr Asp Val His Tyr Arg
                    1               5
```

<210> 687
<211> 13
<212> PRT
<213> Homo Sapiens


<400> 687

```
                    Thr Glu Phe Gln Gln Ile Ile Asn Leu Ala Leu Gln Lys
                    1               5                   10
```

<210> 688
<211> 12
<212> PRT
<213> Homo Sapiens

<400> 688

```
                    Thr Phe Gly His Asn Thr Met Asp Ala Val Pro Arg
                    1               5               10
```

<210> 689
<211> 7
<212> PRT
<213> Homo Sapiens

<400> 689

```
                         Thr Phe Ile Asp Thr Val Arg
                         1               5
```

<210> 690
<211> 9
<212> PRT
<213> Homo Sapiens

<400> 690

```
                    Thr Phe Ser Asp Leu Gln Ser Leu Arg
                    1               5
```

<210> 691
<211> 10
<212> PRT
<213> Homo Sapiens

<400> 691

```
                    Thr Phe Ser Asp Leu Gln Ser Leu Arg Lys
                    1               5               10
```

<210> 692
<211> 13
<212> PRT
<213> Homo Sapiens

<400> 692

```
              Thr Gly Cys Phe Ile Val Ile Asp Ala Met Leu Glu Arg

                    1               5               10
```

<210> 693
<211> 14
<212> PRT
<213> Homo Sapiens

<400> 693

```
              Thr Gly Glu Gly Phe Ile Asp Phe Ile Gly Gln Val His Lys
              1               5               10
```

<210> 694
<211> 11
<212> PRT
<213> Homo Sapiens

<400> 694

```
Thr Gly Glu Gln Ala Pro Ser Ser Pro Pro Arg
1               5                   10
```

<210> 695
<211> 12
<212> PRT
<213> Homo Sapiens

<400> 695

```
Thr Gly Glu Gln Ala Pro Ser Ser Pro Pro Arg Arg
1               5                   10
```

<210> 696
<211> 15
<212> PRT
<213> Homo Sapiens

<400> 696

```
Thr His Leu Gly Leu Ile Met Asp Gly Trp Asn Ser Met Ile Arg
1             5                 10                      15
```

<210> 697
<211> 7
<212> PRT
<213> Homo Sapiens

<400> 697

```
Thr His Gln Ala Phe Met Arg
1               5
```

<210> 698
<211> 8
<212> PRT
<213> Homo Sapiens

<400> 698

```
Thr His Gln Ala Phe Met Arg Lys
1               5
```

<210> 699
<211> 12
<212> PRT
<213> Homo Sapiens

<400> 699

```
Thr His Ser Ser Asn Ser Met Leu Val Phe Leu Lys
1               5               10
```

<210> 700
<211> 8
<212> PRT
<213> Homo Sapiens

<400> 700

```
Thr Ile His Ala Glu Leu Ser Lys
1               5
```

<210> 701
<211> 6
<212> PRT
<213> Homo Sapiens

<400> 701

```
Thr Ile Asn Phe Ala Arg
1               5
```

<210> 702
<211> 8
<212> PRT
<213> Homo Sapiens

<400> 702

```
Thr Leu Ala Asp Phe Asp Pro Arg
1               5
```

<210> 703
<211> 10
<212> PRT
<213> Homo Sapiens

<400> 703

```
Thr Leu Glu Gln Met Asp Val Val His Arg
1               5               10
```

<210> 704
<211> 10
<212> PRT
<213> Homo Sapiens

<400> 704

```
Thr Leu His Thr Glu Glu Leu Thr Ser Lys
1               5               10
```

<210> 705
<211> 9
<212> PRT
<213> Homo Sapiens

<400> 705

```
Thr Leu Ser Ala Gly Ala Gly Pro Arg
1               5
```

<210> 706
<211> 27
<212> PRT
<213> Homo Sapiens

<400> 706

```
Thr Met Gly Met Val Asn Ile Phe Asn Gly Asp Ala Asp Leu Ser Gly
1               5               10              15
```

```
Met Thr Trp Ser His Gly Leu Ser Val Ser Lys
        20              25
```

<210> 707
<211> 14
<212> PRT
<213> Homo Sapiens

<400> 707

```
Thr Met Leu His Leu Thr Asp Ile Gln Leu Gln Asp Asn Lys
1               5               10
```

<210> 708
<211> 10
<212> PRT
<213> Homo Sapiens

<400> 708

```
Thr Met Pro Val Glu Gln Val Phe Ala Lys
1               5               10
```

<210> 709
<211> 12
<212> PRT
<213> Homo Sapiens

<400> 709

```
Thr Asn Glu Asp Val Pro Ser Gly Pro Pro Arg Lys
1               5               10
```

<210> 710
<211> 18
<212> PRT
<213> Homo Sapiens

<400> 710

```
Thr Asn Glu Pro Val Trp Glu Glu Asn Phe Thr Phe Phe Ile His Asn
1               5                   10                  15
```

```
Pro Lys
```

<210> 711
<211> 8
<212> PRT
<213> Homo Sapiens

<400> 711

```
Thr Asn His Phe Thr Ile Pro Lys
1                   5
```

<210> 712
<211> 14
<212> PRT
<213> Homo Sapiens

<400> 712

```
Thr Asn Gln Glu Val Leu Asp Phe Val Val Gly Gly Gly Arg
1               5                   10
```

<210> 713
<211> 9
<212> PRT
<213> Homo Sapiens

<400> 713

```
Thr Asn Ser Ile Leu Phe Tyr Gly Arg
1                   5
```

<210> 714
<211> 10
<212> PRT
<213> Homo Sapiens

<400> 714

```
Thr Asn Val Ile Gln Ser Leu Leu Ala Arg
1               5                   10
```

<210> 715
<211> 6
<212> PRT
<213> Homo Sapiens

<400> 715

```
Thr Asn Trp Val Tyr Arg
1               5
```

<210> 716
<211> 13

<212> PRT
<213> Homo Sapiens

<400> 716

```
                Thr Pro Glu Ser Ser Pro Pro Gly Thr Pro Ile Gly Arg
                1               5                   10
```

<210> 717
<211> 12
<212> PRT
<213> Homo Sapiens

<400> 717

```
                Thr Pro Leu His Ala Ala Val Ala Ala Asp Ala Arg
                1               5                   10
```

<210> 718
<211> 9
<212> PRT
<213> Homo Sapiens

<400> 718

```
                Thr Pro Leu Ser Tyr Thr His Trp Arg
                1               5
```

<210> 719
<211> 6
<212> PRT
<213> Homo Sapiens

<400> 719

```
                Thr Pro Asn Trp Asp Arg
                1               5
```

<210> 720
<211> 7
<212> PRT
<213> Homo Sapiens

<400> 720

```
                Thr Pro Asn Trp Arg Pro Arg
                1               5
```

<210> 721
<211> 8
<212> PRT
<213> Homo Sapiens

<400> 721

```
                Thr Pro Pro Pro Gly Gly Val Lys
                1               5
```

<210> 722
<211> 19
<212> PRT
<213> Homo Sapiens

<400> 722

```
Thr Pro Val Ser Thr Ile Ile Met Pro Asn Glu Phe Gln Gln Asp Tyr
1               5               10                  15

Asp Ile Arg
```

<210> 723
<211> 19
<212> PRT
<213> Homo Sapiens

<400> 723

```
Thr Gln Asp Leu Glu Asn Phe Leu Cys Asn Asn Leu Gln Cys Gly Ser
1               5               10                  15

Phe Leu Lys
```

<210> 724
<211> 15
<212> PRT
<213> Homo Sapiens

<400> 724

```
Thr Gln Glu Glu Thr Glu Asp Pro Ser Val Ile Gly Glu Phe Lys
1               5               10                  15
```

<210> 725
<211> 21
<212> PRT
<213> Homo Sapiens

<400> 725

```
Thr Gln Gln Gly Val Pro Ala Gln Pro Ala Asp Phe Gln Ala Glu Val
1               5               10                  15

Glu Ser Asp Thr Arg
                20
```

<210> 726
<211> 18
<212> PRT
<213> Homo Sapiens

<400> 726

```
Thr Gln Arg Pro Ala Met Val Gln Thr Glu Asp Gln Tyr Gln Leu Cys
1               5                   10                  15

Tyr Arg
```

<210> 727
<211> 8
<212> PRT
<213> Homo Sapiens

<400> 727

```
Thr Ser Gly Phe Pro Ala Lys Lys
1               5
```

<210> 728
<211> 16
<212> PRT
<213> Homo Sapiens

<400> 728

```
Thr Ser Leu Cys Val Leu Gly Pro Gly Asp Glu Ala Pro Leu Glu Arg
1               5                   10                  15
```

<210> 729
<211> 17
<212> PRT
<213> Homo Sapiens

<400> 729

```
Thr Ser Leu Cys Val Leu Gly Pro Gly Asp Glu Ala Pro Leu Glu Arg
1               5                   10                  15

Lys
```

<210> 730
<211> 10
<212> PRT
<213> Homo Sapiens

<400> 730

```
Thr Ser Ser Gln Pro Gly Phe Leu Glu Arg
1               5                   10
```

<210> 731
<211> 20
<212> PRT
<213> Homo Sapiens

<400> 731

```
Thr Ser Val Leu Leu Ser Trp Glu Ile Pro Glu Asn Tyr Asn Ser Ala
1               5               10                  15

Met Pro Phe Lys
            20
```

<210> 732
<211> 14
<212> PRT
<213> Homo Sapiens

<400> 732

```
Thr Ser Val Leu Leu Ser Trp Glu Val Pro Asp Ser Tyr Lys
1               5               10
```

<210> 733
<211> 21
<212> PRT
<213> Homo Sapiens

<400> 733

```
Thr Ser Val Thr Val Ser Asp Leu Glu Pro His Met Asn Tyr Thr Phe
1               5               10                  15

Thr Val Glu Ala Arg
            20
```

<210> 734
<211> 21
<212> PRT
<213> Homo Sapiens

<400> 734

```
Thr Thr Pro Pro Thr Thr Arg Pro Pro Pro Thr Thr Thr Pro Glu Pro
1               5               10                  15

Thr Ala Pro Pro Arg
            20
```

<210> 735
<211> 12
<212> PRT
<213> Homo Sapiens

<400> 735

```
Thr Val Asp Ile Tyr Gly His Val Thr Cys Met Arg
1               5               10
```

<210> 736
<211> 12
<212> PRT

<213> Homo Sapiens

<400> 736

```
            Thr Val Asp Ile Tyr Gly His Val Thr Leu Met Arg
            1               5                   10
```

<210> 737
<211> 23
<212> PRT
<213> Homo Sapiens

<400> 737

```
        Thr Val Gln Thr Ala Ala Ala Asn Ala Ala Ser Thr Ala Ala Ser Ser
        1               5                   10                  15

        Ala Ala Gln Asn Ala Phe Lys
                        20
```

<210> 738
<211> 10
<212> PRT
<213> Homo Sapiens

<400> 738

```
            Thr Val Ser Glu Trp Leu Glu Ser Ile Lys
            1               5                   10
```

<210> 739
<211> 9
<212> PRT
<213> Homo Sapiens

<400> 739

```
            Thr Tyr Glu Val Cys Asp Val Gln Arg
            1               5
```

<210> 740
<211> 9
<212> PRT
<213> Homo Sapiens

<400> 740

```
            Thr Tyr Leu Ala Val Gly Ser Met Lys
            1               5
```

<210> 741
<211> 13
<212> PRT
<213> Homo Sapiens

<400> 741

Thr Tyr Gln Phe Leu Gln Glu Tyr Leu Asp Ala Ile Lys
1                5                  10

<210> 742
<211> 17
<212> PRT
<213> Homo Sapiens

<400> 742

Thr Tyr Val Asp Pro His Thr Tyr Glu Asp Pro Asn Gln Ala Val Leu
1            5             10           15

Lys

<210> 743
<211> 8
<212> PRT
<213> Homo Sapiens

<400> 743

Val Ala Lys Pro Glu Glu Met Lys
1            5

<210> 744
<211> 10
<212> PRT
<213> Homo Sapiens

<400> 744

Val Ala Asn His Met Asp Gly Phe Gln Arg
1            5             10

<210> 745
<211> 13
<212> PRT
<213> Homo Sapiens

<400> 745

Val Ala Gln Pro Gly Pro Leu Glu Pro Glu Glu Pro Arg
1            5             10

<210> 746
<211> 12
<212> PRT
<213> Homo Sapiens

<400> 746

Val Ala Thr Pro Leu Pro Asp Pro Met Ala Ser Arg
1            5             10

<210> 747
<211> 20

<212> PRT
<213> Homo Sapiens

<400> 747

Val Asp Phe Leu Gly Glu Ala Gly Ile Met Gly Gln Phe Ser His His
1               5                   10                  15

Asn Ile Ile Arg
                20

<210> 748
<211> 15
<212> PRT
<213> Homo Sapiens

<400> 748

Val Asp Met Thr Phe Glu Glu Glu Ala Gln Leu Leu Gln Leu Lys
1               5                   10                  15

<210> 749
<211> 11
<212> PRT
<213> Homo Sapiens

<400> 749

Val Asp Thr Val Ala Ala Glu His Leu Thr Arg
1               5                   10

<210> 750
<211> 9
<212> PRT
<213> Homo Sapiens

<400> 750

Val Asp Val Gly Gln Gln Pro Leu Arg
1               5

<210> 751
<211> 11
<212> PRT
<213> Homo Sapiens

<400> 751

Val Glu Ala Ser Asn Pro Tyr Val Glu Pro Arg
1               5                   10

<210> 752
<211> 8
<212> PRT
<213> Homo Sapiens

<400> 752

```
Val Glu Asp Phe Asp Ala Leu Lys
1               5
```

<210> 753
<211> 11
<212> PRT
<213> Homo Sapiens


<400> 753

```
Val Glu Asp Leu Pro Ala Asp Asp Ile Leu Arg
1               5               10
```

<210> 754
<211> 20
<212> PRT
<213> Homo Sapiens


<400> 754

```
Val Glu Leu Pro Gly Thr Ala Val Pro Ser Val Pro Glu Asp Ala Ala
1           5               10              15
```

```
Pro Ala Ser Arg
            20
```

<210> 755
<211> 12
<212> PRT
<213> Homo Sapiens


<400> 755

```
Val Glu Val Glu Ala Val Asn Ser Thr Ser Val Lys
1               5               10
```

<210> 756
<211> 16
<212> PRT
<213> Homo Sapiens


<400> 756

```
Val Glu Val Glu Pro Leu Asn Ser Thr Ala Val His Val Tyr Trp Lys
1           5               10              15
```

<210> 757
<211> 16
<212> PRT
<213> Homo Sapiens


<400> 757

```
Val Glu Tyr Tyr Ile Pro Gly Ser Thr Thr Asn Pro Glu Val Phe Lys
1           5               10              15
```

<210> 758

<211> 15
<212> PRT
<213> Homo Sapiens

<400> 758

```
Val Phe Ala Gln Asn Glu Glu Ile Gln Glu Met Ala Gln Asn Lys
1               5                   10                  15
```

<210> 759
<211> 6
<212> PRT
<213> Homo Sapiens

<400> 759

```
Val Phe His Ala Glu Arg
1               5
```

<210> 760
<211> 7
<212> PRT
<213> Homo Sapiens

<400> 760

```
Val Phe His Arg Pro Trp Arg
1               5
```

<210> 761
<211> 9
<212> PRT
<213> Homo Sapiens

<400> 761

```
Val Phe Leu Ser Ile Glu Glu Phe Arg
1               5
```

<210> 762
<211> 10
<212> PRT
<213> Homo Sapiens

<400> 762

```
Val Gly Glu Glu Asp Asp Gly Glu Tyr Arg
1               5                   10
```

<210> 763
<211> 9
<212> PRT
<213> Homo Sapiens

<400> 763

```
Val Gly Phe Gly Glu Glu Met Val Lys
1               5
```

<210> 764
<211> 9
<212> PRT
<213> Homo Sapiens

<400> 764

```
                        Val Gly Gly Ser Met Leu Thr Pro Arg
                        1               5
```

<210> 765
<211> 9
<212> PRT
<213> Homo Sapiens

<400> 765

```
                        Val Gly Asn Gln Ile Phe Gln Ser Arg
                        1               5
```

<210> 766
<211> 6
<212> PRT
<213> Homo Sapiens

<400> 766

```
                    Val His Gly Leu Tyr Arg
                    1               5
```

<210> 767
<211> 17
<212> PRT
<213> Homo Sapiens

<400> 767

```
        Val Ile Glu Glu Ala Val Tyr Phe His Gln His Ser Ile Leu Ala Cys
        1           5                   10                  15

        Lys
```

<210> 768
<211> 6
<212> PRT
<213> Homo Sapiens

<400> 768

```
                    Val Ile Glu Leu Asn Lys
                    1               5
```

<210> 769
<211> 12
<212> PRT
<213> Homo Sapiens

<400> 769

```
          Val Ile Gly Ala Gly Glu Phe Gly Glu Val Tyr Lys
          1               5                   10
```

<210> 770
<211> 19
<212> PRT
<213> Homo Sapiens

<400> 770

```
      Val Leu Ala Phe Thr Ala Val Gly Asp Gly Pro Pro Ser Pro Thr Ile
      1               5                   10                  15

      Gln Val Lys
```

<210> 771
<211> 12
<212> PRT
<213> Homo Sapiens

<400> 771

```
          Val Leu Ala Leu Leu Cys Ser Gly Phe Gln Pro Lys
          1               5                   10
```

<210> 772
<211> 23
<212> PRT
<213> Homo Sapiens

<400> 772

```
      Val Leu Ala Pro Ala Ser Thr Leu Gln Ser Ser Tyr Gln Ile Pro Thr
      1               5                   10                  15

      Glu Asn Ser Met Thr Ala Arg
                      20
```

<210> 773
<211> 16
<212> PRT
<213> Homo Sapiens

<400> 773

```
      Val Leu Ala Gln Gln Gly Glu Tyr Ser Glu Ala Ile Pro Ile Leu Arg
      1               5                   10                  15
```

<210> 774
<211> 9
<212> PRT
<213> Homo Sapiens

<400> 774

```
                    Val Leu Ala Val Asn Ser Ile Gly Arg
                    1               5
```

<210> 775
<211> 10
<212> PRT
<213> Homo Sapiens


<400> 775

```
                        Val Leu Asp Ala Gly Asp Pro Thr Ser Arg
                        1               5                   10
```

<210> 776
<211> 7
<212> PRT
<213> Homo Sapiens


<400> 776

```
                            Val Leu Asp Ser Gly Phe Arg
                            1               5
```

<210> 777
<211> 16
<212> PRT
<213> Homo Sapiens


<400> 777

```
            Val Leu Glu Asp Asp Pro Glu Ala Thr Tyr Thr Thr Ser Gly Gly Lys
            1               5                   10                      15
```

<210> 778
<211> 17
<212> PRT
<213> Homo Sapiens


<400> 778

```
            Val Leu Glu Gly Met Val Glu Glu Asn Gln Val Asn Val Glu Val Thr
            1               5                   10                      15

                Arg
```

<210> 779
<211> 7
<212> PRT
<213> Homo Sapiens


<400> 779

```
                            Val Leu Glu Ile Pro Tyr Lys
                            1               5
```

<210> 780
<211> 7

<212> PRT
<213> Homo Sapiens

<400> 780

Val Leu Glu Asn Tyr Asn Lys

1                   5

<210> 781
<211> 14
<212> PRT
<213> Homo Sapiens

<400> 781

Val Leu His Phe Asp Gln Val Thr Glu Asn Thr Thr Glu Lys
1               5                   10

<210> 782
<211> 24
<212> PRT
<213> Homo Sapiens

<400> 782

Val Leu Met Asp Glu Gly Ala Val Leu Thr Leu Val Ala Asp Leu Ser
1               5                   10                  15

Ser Ala Thr Leu Asp Ile Ser Lys
                20

<210> 783
<211> 14
<212> PRT
<213> Homo Sapiens

<400> 783

Val Leu Met Glu Ile Gln Asp Leu Met Phe Glu Glu Met Arg
1               5                   10

<210> 784
<211> 12
<212> PRT
<213> Homo Sapiens

<400> 784

Val Leu Val Ala Leu Ala Ser Glu Glu Leu Ala Lys
1               5                   10

<210> 785
<211> 12
<212> PRT

<213> Homo Sapiens

<400> 785

```
                        Val Met Cys Val Ser Met Gly Ser Thr Thr Val Arg
                        1               5                   10
```

<210> 786
<211> 20
<212> PRT
<213> Homo Sapiens

<400> 786

```
        Val Asn Asp Ser Asn Glu Leu Gly Gly Leu Thr Thr Ser Gly Ser Ala
        1               5                   10                  15

        Glu Val His Lys
                    20
```

<210> 787
<211> 10
<212> PRT
<213> Homo Sapiens

<400> 787

```
                    Val Pro Ala His Gln Val Leu Phe Ser Arg
                    1               5                   10
```

<210> 788
<211> 8
<212> PRT
<213> Homo Sapiens

<400> 788

```
                        Val Pro Cys His Phe Pro Cys Lys
                        1               5
```

<210> 789
<211> 26
<212> PRT
<213> Homo Sapiens

<400> 789

```
        Val Pro Glu Asp Gln Thr Gly Leu Ser Gly Gly Val Ala Ser Phe Val
        1               5                   10                  15

        Cys Gln Ala Thr Gly Glu Pro Lys Pro Arg
                    20                  25
```

<210> 790
<211> 8
<212> PRT

<213> Homo Sapiens

<400> 790

```
                        Val Pro Glu Gly Phe Thr Cys Arg
                        1               5
```

<210> 791
<211> 10
<212> PRT
<213> Homo Sapiens

<400> 791

```
                    Val Pro Glu Gly Leu Glu Asp Val Ser Lys
                    1               5                   10
```

<210> 792
<211> 17
<212> PRT
<213> Homo Sapiens

<400> 792

```
        Val Pro Leu Ser Gln Leu Leu Thr Ser Glu Asp Met Thr Val Ser Gln
        1               5                   10                  15


        Arg
```

<210> 793
<211> 13
<212> PRT
<213> Homo Sapiens

<400> 793

```
            Val Gln Cys Ser Glu Gln Trp Ile Pro Phe Gln Asn Lys
            1               5                   10
```

<210> 794
<211> 7
<212> PRT
<213> Homo Sapiens

<400> 794

```
                    Val Gln Val Ile Glu Gly Arg
                    1               5
```

<210> 795
<211> 9
<212> PRT
<213> Homo Sapiens

<400> 795

```
                    Val Gln Trp Arg Pro Gln Gly Thr Arg
                    1                   5
```

<210> 796
<211> 8
<212> PRT
<213> Homo Sapiens


<400> 796

```
                         Val Ser Asp Phe Gly Leu Ser Arg
                         1                   5
```

<210> 797
<211> 10
<212> PRT
<213> Homo Sapiens


<400> 797

```
                    Val Ser Thr Glu Val Thr Leu Ala Val Lys
                    1                   5                   10
```

<210> 798
<211> 15
<212> PRT
<213> Homo Sapiens


<400> 798

```
          Val Ser Trp Ser Pro Ala Glu Asp His Asn Ala Pro Ile Glu Lys
          1               5                   10                  15
```

<210> 799
<211> 11
<212> PRT
<213> Homo Sapiens


<400> 799

```
                    Val Ser Trp Val Pro Pro Pro Ala Asp Ser Arg
                    1                   5                   10
```

<210> 800
<211> 8
<212> PRT
<213> Homo Sapiens


<400> 800

```
                         Val Thr Ala Ala Gly Gln Thr Lys
                         1                   5
```

<210> 801
<211> 6
<212> PRT
<213> Homo Sapiens

<400> 801

```
                        Val Thr Glu Met Met Lys
                        1               5
```

<210> 802
<211> 7
<212> PRT
<213> Homo Sapiens

<400> 802

```
                        Val Thr Glu Met Met Lys Lys
                        1               5
```

<210> 803
<211> 21
<212> PRT
<213> Homo Sapiens

<400> 803

```
        Val Thr Phe Asp Pro Thr Ser Ser Tyr Thr Leu Glu Asp Leu Lys Pro
        1               5               10              15

        Asp Thr Leu Tyr Arg
                    20
```

<210> 804
<211> 20
<212> PRT
<213> Homo Sapiens

<400> 804

```
        Val Thr Phe Thr Cys Gln Ala Ser Phe Asp Pro Ser Leu Gln Pro Ser
        1               5               10              15

        Ile Thr Trp Arg
                    20
```

<210> 805
<211> 11
<212> PRT
<213> Homo Sapiens

<400> 805

```
                    Val Thr Leu Pro Ala Gly Pro Asp Leu Leu Arg
                    1               5               10
```

<210> 806
<211> 9
<212> PRT
<213> Homo Sapiens

<400> 806

```
Val Thr Gln Glu Ile Val Thr Glu Arg
1               5
```

<210> 807
<211> 8
<212> PRT
<213> Homo Sapiens

<400> 807

```
Val Thr Val Ala Ile Pro Leu Lys
1               5
```

<210> 808
<211> 8
<212> PRT
<213> Homo Sapiens

<400> 808

```
Val Thr Tyr Gln Asn His Asn Lys
1               5
```

<210> 809
<211> 8
<212> PRT
<213> Homo Sapiens

<400> 809

```
Val Val Glu Met Gln Gly Val Arg
1               5
```

<210> 810
<211> 10
<212> PRT
<213> Homo Sapiens

<400> 810

```
Val Val Phe Gln Ile Trp Asp Asn Asp Lys
1               5                   10
```

<210> 811
<211> 14
<212> PRT
<213> Homo Sapiens

<400> 811

```
Val Val Leu Leu Glu Asp Leu Ala Ser Gln Val Gly Leu Arg
1               5                   10
```

<210> 812
<211> 17
<212> PRT

<213> Homo Sapiens

<400> 812

```
Val Val Pro Ser Phe Leu Pro Val Asp Gln Gly Gly Ser Leu Val Gly
1               5                   10                  15

Arg
```

<210> 813
<211> 10
<212> PRT
<213> Homo Sapiens

<400> 813

```
Val Val Gln Met Thr Asn Asp Asp Ile Lys
1               5                   10
```

<210> 814
<211> 11
<212> PRT
<213> Homo Sapiens

<400> 814

```
Val Val Gln Met Thr Asn Asp Asp Ile Lys Arg
1               5                   10
```

<210> 815
<211> 7
<212> PRT
<213> Homo Sapiens

<400> 815

```
Val Val Tyr Ser Ala Pro Arg
1               5
```

<210> 816
<211> 24
<212> PRT
<213> Homo Sapiens

<400> 816

```
Val Tyr Ala Pro Ala Ser Thr Leu Val Asp Gln Pro Tyr Ala Asn Glu
1               5                   10                  15

Gly Thr Val Val Val Thr Glu Arg
                20
```

<210> 817
<211> 16
<212> PRT
<213> Homo Sapiens

<400> 817

```
Val Tyr Asp Ser Leu Leu Ala Leu Pro Gln Asp Leu Gln Ala Ala Arg
1               5                   10                  15
```

<210> 818
<211> 16
<212> PRT
<213> Homo Sapiens

<400> 818

```
Val Tyr Ile Asp Pro Phe Thr Tyr Glu Asp Pro Asn Glu Ala Val Arg
1               5                   10                  15
```

<210> 819
<211> 8
<212> PRT
<213> Homo Sapiens

<400> 819

```
Val Tyr Thr Glu Asn Val Asp Lys
1                   5
```

<210> 820
<211> 9
<212> PRT
<213> Homo Sapiens

<400> 820

```
Val Tyr Thr Glu Asn Val Asp Lys Arg
1                   5
```

<210> 821
<211> 8
<212> PRT
<213> Homo Sapiens

<400> 821

```
Val Tyr Thr Val Asp Leu Gly Arg
1                   5
```

<210> 822
<211> 5
<212> PRT
<213> Homo Sapiens

<400> 822

```
Val Tyr Tyr Lys Lys
1                   5
```

<210> 823
<211> 8
<212> PRT

<213> Homo Sapiens

<400> 823

Val Tyr Tyr Thr Pro Asp Ser Arg
1               5

<210> 824
<211> 12
<212> PRT
<213> Homo Sapiens

<400> 824

Trp Glu Asp Glu Glu Trp Ser Thr Asp Leu Asn Arg
1               5                   10

<210> 825
<211> 5
<212> PRT
<213> Homo Sapiens

<400> 825

Trp Glu Glu His Arg
1               5

<210> 826
<211> 6
<212> PRT
<213> Homo Sapiens

<400> 826

Trp Glu Glu Val Cys Arg
1               5

<210> 827
<211> 15
<212> PRT
<213> Homo Sapiens

<400> 827

Trp Leu Leu Leu Ser Asp Pro Asp Asp Phe Ser Ala Gly Ala Arg
1               5                   10                  15

<210> 828
<211> 12
<212> PRT
<213> Homo Sapiens

<400> 828

Trp Leu Arg Pro Ser Gly Pro Met Pro Ala Asp Arg
1               5                   10

<210> 829

<211> 12
<212> PRT
<213> Homo Sapiens

<400> 829

```
Trp Met Asp Trp Asn Ala Pro Gln Val Gln Tyr Arg
1               5                   10
```

<210> 830
<211> 10
<212> PRT
<213> Homo Sapiens

<400> 830

```
Trp Met Met Gly Ala Glu Glu Leu Thr Lys
1               5                   10
```

<210> 831
<211> 10
<212> PRT
<213> Homo Sapiens

<400> 831

```
Trp Arg Pro Val Asp Leu Ala Gln Val Lys
1               5                   10
```

<210> 832
<211> 6
<212> PRT
<213> Homo Sapiens

<400> 832

```
Trp Ser Asn Val Phe Lys
1               5
```

<210> 833
<211> 10
<212> PRT
<213> Homo Sapiens

<400> 833

```
Trp Thr Ala Pro Glu Ala Ile Ala Phe Arg
1               5                   10
```

<210> 834
<211> 10
<212> PRT
<213> Homo Sapiens

<400> 834

```
Trp Thr Ala Pro Glu Ala Ile Ser Tyr Arg
1               5                   10
```

<210> 835
<211> 11
<212> PRT
<213> Homo Sapiens

<400> 835

```
Trp Thr Ala Pro Glu Ala Ile Ser Tyr Arg Lys
1               5                   10
```

<210> 836
<211> 5
<212> PRT
<213> Homo Sapiens

<400> 836

```
Trp Thr Glu Tyr Arg
1               5
```

<210> 837
<211> 10
<212> PRT
<213> Homo Sapiens

<400> 837

```
Trp Thr Pro Pro Gln Asp Ser Gly Gly Arg
1               5                   10
```

<210> 838
<211> 6
<212> PRT
<213> Homo Sapiens

<400> 838

```
Trp Val Ser Gln His Arg
1               5
```

<210> 839
<211> 12
<212> PRT
<213> Homo Sapiens

<400> 839

```
Tyr Ala Ile Gly Val Gly Leu Ala Phe Gln Asn Arg
1               5                   10
```

<210> 840
<211> 11
<212> PRT
<213> Homo Sapiens

<400> 840

```
Tyr Ala Asn Val Ile Ala Tyr Asp His Ser Arg
1               5                   10
```

<210> 841
<211> 15
<212> PRT
<213> Homo Sapiens

<400> 841

```
Tyr Ala Val Tyr Thr Val Val Ser Ser His Glu Gln Phe Thr Lys
1               5                   10                  15
```

<210> 842
<211> 8
<212> PRT
<213> Homo Sapiens

<400> 842

```
Tyr Asp Ile Glu Phe Glu Asp Lys
1               5
```

<210> 843
<211> 12
<212> PRT
<213> Homo Sapiens

<400> 843

```
Tyr Glu Gly Val Val Asp Ile Phe Gln Thr Val Lys
1               5                   10
```

<210> 844
<211> 12
<212> PRT
<213> Homo Sapiens

<400> 844

```
Tyr Glu Gly Val Val Asp Met Phe Gln Thr Val Lys
1               5                   10
```

<210> 845
<211> 6
<212> PRT
<213> Homo Sapiens

<400> 845

```
Tyr Glu Val Thr Tyr Arg
1               5
```

<210> 846
<211> 7
<212> PRT
<213> Homo Sapiens

<400> 846

```
                          Tyr Glu Val Thr Tyr Arg Lys
                          1               5
```

<210> 847
<211> 8
<212> PRT
<213> Homo Sapiens

<400> 847

```
                      Tyr Glu Val Thr Tyr Arg Lys Lys
                      1               5
```

<210> 848
<211> 9
<212> PRT
<213> Homo Sapiens

<400> 848

```
                  Tyr Phe Asp Trp Ile Leu Ile Ser Arg

                          1               5
```

<210> 849
<211> 10
<212> PRT
<213> Homo Sapiens

<400> 849

```
              Tyr Phe Asp Trp Ile Leu Ile Ser Arg Arg
              1           5                   10
```

<210> 850
<211> 7
<212> PRT
<213> Homo Sapiens

<400> 850

```
                          Tyr Phe Ile Glu Asp Gly Arg
                          1               5
```

<210> 851
<211> 7
<212> PRT
<213> Homo Sapiens

<400> 851

```
                          Tyr Phe Trp Thr Gly Leu Arg
                          1               5
```

<210> 852
<211> 6
<212> PRT
<213> Homo Sapiens

<400> 852

```
                              Tyr Gly Ile Gln Glu Lys
                              1               5
```

<210> 853
<211> 18
<212> PRT
<213> Homo Sapiens

<400> 853

```
        Tyr Gly Ile Val Leu Asp Ala Gly Ser Ser His Thr Ser Leu Tyr Ile
        1               5                   10                  15

        Tyr Lys
```

<210> 854
<211> 8
<212> PRT
<213> Homo Sapiens

<400> 854

```
                          Tyr Ile Ala Phe Asp Phe His Lys
                          1               5
```

<210> 855
<211> 9
<212> PRT
<213> Homo Sapiens

<400> 855

```
                      Tyr Ile Glu Tyr Gln Gly Ala Glu Lys
                      1               5
```

<210> 856
<211> 9
<212> PRT
<213> Homo Sapiens

<400> 856

```
                      Tyr Lys Pro Leu Pro Gln Ile Ser Lys
                      1               5
```

<210> 857
<211> 18
<212> PRT
<213> Homo Sapiens

<400> 857

```
Tyr Lys Pro Met Met Ile Ile Thr Glu Tyr Met Glu Asn Gly Ala Leu
1               5                   10                  15

Asp Lys
```

<210> 858
<211> 9
<212> PRT
<213> Homo Sapiens

<400> 858

```
Tyr Lys Pro Thr Pro Ile Pro Ile Lys
1                   5
```

<210> 859
<211> 10
<212> PRT
<213> Homo Sapiens

<400> 859

```
Tyr Leu Ala Glu Met Ser Tyr Val His Arg
1                   5                   10
```

<210> 860
<211> 10
<212> PRT
<213> Homo Sapiens

<400> 860

```
Tyr Leu Ala Asn Met Asn Tyr Val His Arg
1                   5                   10
```

<210> 861
<211> 32
<212> PRT
<213> Homo Sapiens

<400> 861

```
Tyr Leu Cys Gly Ala His Ser Asp Gly Gln Leu Gln Glu Gly Ser Pro
1               5                   10                  15

Ile Gln Ala Trp Gln Leu Phe Val Asn Glu Glu Ser Thr Ile Pro Arg
            20                  25                  30
```

<210> 862
<211> 13
<212> PRT
<213> Homo Sapiens

<400> 862

```
Tyr Leu Gln Val Ser Gln Gln Leu Gln Gln Thr Asn Arg
1               5                   10
```

<210> 863
<211> 16
<212> PRT
<213> Homo Sapiens


<400> 863

```
Tyr Met Thr Glu Thr Trp Asp Pro Ser His Ala Pro Asp Asn Phe Arg
1               5                   10                  15
```

<210> 864
<211> 10
<212> PRT
<213> Homo Sapiens


<400> 864

```
Tyr Pro Asp Leu Ile Ala Glu Leu Leu Arg
1               5                   10
```

<210> 865
<211> 8
<212> PRT
<213> Homo Sapiens


<400> 865

```
Tyr Pro Glu Val Gly Asp Leu Arg
1               5
```

<210> 866
<211> 5
<212> PRT
<213> Homo Sapiens


<400> 866

```
Tyr Pro Trp His Arg
1               5
```

<210> 867
<211> 6
<212> PRT
<213> Homo Sapiens


<400> 867

```
Tyr Gln His Thr Gly Lys
1               5
```

<210> 868
<211> 9
<212> PRT
<213> Homo Sapiens

<400> 868

```
                              Tyr Gln Val Asn Asn Leu Gly Gln Arg
                              1               5
```

<210> 869
<211> 20
<212> PRT
<213> Homo Sapiens

<400> 869

```
                    Tyr Gln Tyr Phe Val Val Asp Pro Met Ala Glu Tyr Asn Met Pro Gln
                    1               5                   10                  15

                    Tyr Ile Leu Arg
                                20
```

<210> 870
<211> 10
<212> PRT
<213> Homo Sapiens

<400> 870

```
                              Tyr Ser Ala Pro Ala Asn Leu Tyr Val Arg
                              1               5                   10
```

<210> 871
<211> 10
<212> PRT
<213> Homo Sapiens

<400> 871

```
                              Tyr Ser Glu Pro Pro His Gly Leu Thr Arg
                              1               5                   10
```

<210> 872
<211> 15
<212> PRT
<213> Homo Sapiens

<400> 872

```
                    Tyr Ser Ile Gly Gly Leu Ser Pro Phe Ser Glu Tyr Ala Phe Arg
                    1               5                   10                  15
```

<210> 873
<211> 15
<212> PRT
<213> Homo Sapiens

<400> 873

```
                    Tyr Ser Val Ala Gly Leu Ser Pro Tyr Ser Asp Tyr Glu Phe Arg
                    1               5                   10                  15
```

<210> 874
<211> 8
<212> PRT
<213> Homo Sapiens

<400> 874

```
                        Tyr Ser Tyr Asn Thr Glu Trp Arg
                        1                   5
```

<210> 875
<211> 6
<212> PRT
<213> Homo Sapiens

<400> 875

```
                        Tyr Val Cys Lys Arg Lys
                        1                   5
```

<210> 876
<211> 10
<212> PRT
<213> Homo Sapiens

<400> 876

```
                     Tyr Val Gln Asn Gly Thr Tyr Thr Val Lys
                     1               5                   10
```

<210> 877
<211> 19
<212> PRT
<213> Homo Sapiens

<400> 877

```
            Ala Ala Gly Ser Arg Asp Val Ser Leu Ala Lys Ala Asp Ala Ala Pro
            1               5                   10                  15

            Asp Glu Lys
```

<210> 878
<211> 20
<212> PRT
<213> Homo Sapiens

<400> 878

```
            Ala Glu Ala Phe Gly Met Asp Pro Ala Arg Pro Asp Gln Ala Ser Thr
            1               5                   10                  15

            Val Ala Val Lys
                        20
```

<210> 879

<210> 6
<212> PRT
<213> Homo Sapiens

<400> 879

```
                          Ala Glu Asp Ile Ile Lys
                          1                   5
```

<210> 880
<211> 7
<212> PRT
<213> Homo Sapiens

<400> 880

```
                        Ala Glu Leu Arg Gly Leu Lys
                        1                   5
```

<210> 881
<211> 20
<212> PRT
<213> Homo Sapiens

<400> 881

```
        Ala Gly Phe Val Gly Gly Gln Phe Trp Ser Val Tyr Thr Pro Cys Asp
        1               5                   10                  15

                            Thr Gln Asn Lys
                                        20
```

<210> 882
<211> 14
<212> PRT
<213> Homo Sapiens

<400> 882

```
          Ala Asn Leu Ser Gln Val Ala Asp His Leu Asp His Ile Lys
          1               5                   10
```

<210> 883
<211> 7
<212> PRT
<213> Homo Sapiens

<400> 883

```
                          Ala Arg Glu Val Ile Pro Arg
                          1                   5
```

<210> 884
<211> 8
<212> PRT
<213> Homo Sapiens

<400> 884

```
                    Ala Thr Gly Thr Ala Phe Arg Arg
                    1                   5
```

<210> 885
<211> 6
<212> PRT
<213> Homo Sapiens


<400> 885

```
                            Ala Val Leu Gly Asp Arg
                            1                   5
```

<210> 886
<211> 9
<212> PRT
<213> Homo Sapiens


<400> 886

```
                    Ala Val Thr Ser Pro Ala Val Gly Arg
                    1                   5
```

<210> 887
<211> 10
<212> PRT
<213> Homo Sapiens


<400> 887

```
                    Cys Gly Val Ser Asn Lys Asp Ile Glu Lys
                    1                   5                   10
```

<210> 888
<211> 17
<212> PRT
<213> Homo Sapiens


<400> 888

```
        Cys Leu Asn Pro Val Pro Ala Val Pro Ser Pro Ser Pro Ser Val Arg
        1                   5                   10                  15

        Lys
```

<210> 889
<211> 11
<212> PRT
<213> Homo Sapiens


<400> 889

```
                    Asp Cys Lys Ile Arg Val Phe Ile Gly Gly Lys
                    1                   5                   10
```

<210> 890
<211> 9

<212> PRT
<213> Homo Sapiens

<400> 890

```
                        Asp Ile Ala Pro Val Leu Asp Leu Lys
                        1               5
```

<210> 891
<211> 11
<212> PRT
<213> Homo Sapiens

<400> 891

```
                  Asp Ile Met Lys Lys Thr Ile Asn Phe Ala Arg
                  1               5                   10
```

<210> 892
<211> 9
<212> PRT
<213> Homo Sapiens

<400> 892

```
                        Asp Pro Cys Phe His Pro Gly Tyr Lys
                        1               5
```

<210> 893
<211> 5
<212> PRT
<213> Homo Sapiens

<400> 893

```
                              Asp Pro Glu Leu Arg
                              1               5
```

<210> 894
<211> 22
<212> PRT
<213> Homo Sapiens

<400> 894

```
        Asp Ser Pro Val Ile Asp Gly His Asn Asp Leu Pro Trp Gln Leu Leu
        1               5                   10                  15

        Asp Met Phe Asn Asn Arg
                    20
```

<210> 895
<211> 10
<212> PRT
<213> Homo Sapiens

<400> 895

```
                    Asp Ser Gln Met Gln Asn Pro Tyr Ser Arg
                    1               5                       10
```

<210> 896
<211> 5
<212> PRT
<213> Homo Sapiens

<400> 896

```
                                Asp Thr Tyr Leu Lys
                                1               5
```

<210> 897
<211> 6
<212> PRT
<213> Homo Sapiens

<400> 897

```
                                Asp Val Leu Pro Gln Lys
                                1               5
```

<210> 898
<211> 5
<212> PRT
<213> Homo Sapiens

<400> 898

```
                                Glu Gly Ile Ala Lys
                                1               5
```

<210> 899
<211> 8
<212> PRT
<213> Homo Sapiens

<400> 899

```
                            Glu Ile Asp Val Ser Tyr Val Lys
                            1               5
```

<210> 900
<211> 9
<212> PRT
<213> Homo Sapiens

<400> 900

```
                        Glu Leu Asn Glu His Phe Lys Ser Lys
                        1               5
```

<210> 901
<211> 6
<212> PRT
<213> Homo Sapiens

<400> 901

```
                    Glu Leu Arg Glu Val Arg
                    1               5
```

<210> 902
<211> 6
<212> PRT
<213> Homo Sapiens

<400> 902

```
                    Glu Leu Val Ser Leu Lys
                    1               5
```

<210> 903
<211> 7
<212> PRT
<213> Homo Sapiens

<400> 903

```
                    Glu Gln Asp Ile Ala Asp Lys
                    1               5
```

<210> 904
<211> 7
<212> PRT
<213> Homo Sapiens

<400> 904

```
                    Glu Gln Leu Thr Val Ile Arg
                    1               5
```

<210> 905
<211> 8
<212> PRT
<213> Homo Sapiens

<400> 905

```
                    Glu Arg Glu Ala Gln Leu Val Lys
                    1               5
```

<210> 906
<211> 5
<212> PRT
<213> Homo Sapiens

<400> 906

```
                    Glu Arg Met Phe Arg
                    1               5
```

<210> 907
<211> 6
<212> PRT

<213> Homo Sapiens

<400> 907

```
                              Glu Ser Lys Ser Ile Lys
                              1                   5
```

<210> 908
<211> 8
<212> PRT
<213> Homo Sapiens

<400> 908

```
                         Glu Val Cys Gln Leu Leu Leu Arg
                         1               5
```

<210> 909
<211> 5
<212> PRT
<213> Homo Sapiens

<400> 909

```
                              Glu Val Ile Pro Arg
                              1               5
```

<210> 910
<211> 7
<212> PRT
<213> Homo Sapiens

<400> 910

```
                         Phe Leu Ile Thr Arg Arg Arg
                         1               5
```

<210> 911
<211> 13
<212> PRT
<213> Homo Sapiens

<400> 911

```
                    Phe Leu Asn Leu Thr Ser Glu Lys Val Ser Gln Glu Lys
                    1               5                   10
```

<210> 912
<211> 9
<212> PRT
<213> Homo Sapiens

<400> 912

```
                         Phe Ser Asp Val Thr Gly Lys Ile Lys
                         1               5
```

<210> 913

<211> 9
<212> PRT
<213> Homo Sapiens

<400> 913

```
                    Gly His Glu Val Ala Ala Met Leu Lys
                    1               5
```

<210> 914
<211> 14
<212> PRT
<213> Homo Sapiens

<400> 914

```
            Gly Asn Ser Ala Gly Gly Leu Gln His Arg Val Thr Ala Lys
            1               5                   10
```

<210> 915
<211> 6
<212> PRT
<213> Homo Sapiens

<400> 915

```
                    Gly Pro Gly Ile Ser Lys
                    1               5
```

<210> 916
<211> 18
<212> PRT
<213> Homo Sapiens

<400> 916

```
        Gly Thr Leu Ser Pro Lys Asp Ala Leu Thr Asp Leu Thr Gly Asp Ala
        1               5                   10                  15

        Glu Arg
```

<210> 917
<211> 16
<212> PRT
<213> Homo Sapiens

<400> 917

```
        His Ile Glu Glu Glu Asn Leu Ile Asp Glu Asp Phe Gln Asn Leu Lys
        1               5                   10                  15
```

<210> 918
<211> 11
<212> PRT
<213> Homo Sapiens

<400> 918

```
                    His Ser Arg Val Thr Val Ala Ile Pro Leu Lys
                    1               5                   10
```

<210> 919
<211> 5
<212> PRT
<213> Homo Sapiens

<400> 919

```
                          Ile Asp Glu Lys Arg
                          1               5
```

<210> 920
<211> 7
<212> PRT
<213> Homo Sapiens

<400> 920

```
                    Ile Phe Gln Ala Leu Cys Lys
                    1               5
```

<210> 921
<211> 4
<212> PRT
<213> Homo Sapiens

<400> 921

```
                       Ile Ile Pro Lys
                       1
```

<210> 922
<211> 5
<212> PRT
<213> Homo Sapiens

<400> 922

```
                          Ile Lys Gln Leu Arg
                          1               5
```

<210> 923
<211> 15
<212> PRT
<213> Homo Sapiens

<400> 923

```
            Ile Leu Tyr Asp Asp Gly Lys Met Val Glu Glu Val Asp Gly Arg
            1               5                   10                  15
```

<210> 924
<211> 15
<212> PRT
<213> Homo Sapiens

<400> 924

```
            Ile Arg Thr Ser Thr Pro Thr Gly His Gly Ala Ser Pro Ala Lys
            1               5                   10                  15
```

<210> 925
<211> 7
<212> PRT
<213> Homo Sapiens

<400> 925

```
                        Ile Ser Tyr Arg Ile Leu Arg
                        1               5
```

<210> 926
<211> 5
<212> PRT
<213> Homo Sapiens

<400> 926

```
                            Ile Tyr Ile Val Arg
                            1               5
```

<210> 927
<211> 11
<212> PRT
<213> Homo Sapiens

<400> 927

```
                    Lys Cys Ala Gln Leu Thr Val Asn Leu Thr Arg
                    1               5                   10
```

<210> 928
<211> 8
<212> PRT
<213> Homo Sapiens

<400> 928

```
                        Lys Arg Ser Ala Pro Thr Ser Arg
                        1               5
```

<210> 929
<211> 4
<212> PRT
<213> Homo Sapiens

<400> 929

```
                            Leu Ala Leu Lys
                            1
```

<210> 930
<211> 10
<212> PRT

<213> Homo Sapiens

<400> 930

Leu Ala Ser Ser Lys Ala His Thr Ser Arg
1               5                   10

<210> 931
<211> 11
<212> PRT
<213> Homo Sapiens

<400> 931

Leu Glu Asp Pro His Val Asp Ile Ile Arg Arg
1               5                   10

<210> 932
<211> 5
<212> PRT
<213> Homo Sapiens

<400> 932

Leu Phe Gly Glu Lys
1               5

<210> 933
<211> 9
<212> PRT
<213> Homo Sapiens

<400> 933

Leu Lys Asn Lys Glu Glu Val Leu Lys

1               5

<210> 934
<211> 6
<212> PRT
<213> Homo Sapiens

<400> 934

Leu Leu Asp Phe Glu Lys
1               5

<210> 935
<211> 17
<212> PRT
<213> Homo Sapiens

<400> 935

```
Leu Leu His Gly Gln Asn Gly Ser Val Pro Asn Gly Gln Thr Pro Leu
1               5                   10                  15
```

Lys

<210> 936
<211> 10
<212> PRT
<213> Homo Sapiens

<400> 936

```
Leu Leu Ile Glu His Gly Ala Asp Ile Arg
1               5                   10
```

<210> 937
<211> 7
<212> PRT
<213> Homo Sapiens

<400> 937

```
Leu Leu Gln Ala Ile Ala Lys
1               5
```

<210> 938
<211> 5
<212> PRT
<213> Homo Sapiens

<400> 938

```
Leu Leu Ser Asp Lys
1               5
```

<210> 939
<211> 15
<212> PRT
<213> Homo Sapiens

<400> 939

```
Leu Leu Ser Asp Pro Asn Tyr Gly Val His Leu Pro Ala Val Lys
1               5                   10                  15
```

<210> 940
<211> 7
<212> PRT
<213> Homo Sapiens

<400> 940

```
Leu Leu Val Pro Lys Asp Arg
1               5
```

<210> 941
<211> 4

<212> PRT
<213> Homo Sapiens

<400> 941

```
                              Leu Asn Pro Lys
                              1
```

<210> 942
<211> 13
<212> PRT
<213> Homo Sapiens

<400> 942

```
               Leu Asn Val Glu Glu Arg Ser Val Gly Pro Leu Thr Arg
               1               5                   10
```

<210> 943
<211> 24
<212> PRT
<213> Homo Sapiens

<400> 943

```
          Leu Gln Cys Glu Asn Val Gln Glu Ile Pro Val Phe Gly Ile Val Pro
          1               5                   10                  15

          Ala Ile Ile Gln Thr Pro Ser Arg
                          20
```

<210> 944
<211> 7
<212> PRT
<213> Homo Sapiens

<400> 944

```
                         Leu Arg Gln Gly Thr Leu Arg
                         1               5
```

<210> 945
<211> 7
<212> PRT
<213> Homo Sapiens

<400> 945

```
                         Leu Arg Thr Ser Ala Ile Arg
                         1               5
```

<210> 946
<211> 6
<212> PRT
<213> Homo Sapiens

<400> 946

EP 2 447 719 B1

Leu Ser Ala Arg Asn Lys
1                   5

<210> 947
<211> 20
<212> PRT
<213> Homo Sapiens

<400> 947

Leu Ser Asp Ala Gly Gln Tyr Leu Cys Gln Ala Gly Asp Asp Ser Asn
1               5                   10                  15

Ser Asn Lys Lys
            20

<210> 948
<211> 9
<212> PRT
<213> Homo Sapiens

<400> 948

Leu Ser Gly Val Glu Asp His Val Lys
1                   5

<210> 949
<211> 5
<212> PRT
<213> Homo Sapiens

<400> 949

Leu Thr Val Leu Arg
1                   5

<210> 950
<211> 9
<212> PRT
<213> Homo Sapiens

<400> 950

Leu Thr Trp Thr Asn Pro Ser Ile Lys
1                   5

<210> 951
<211> 5
<212> PRT
<213> Homo Sapiens

<400> 951

Leu Val Leu Gly Lys
1                   5

<210> 952

506

<211> 14
<212> PRT
<213> Homo Sapiens

<400> 952

```
Met Arg Tyr Glu Gly Val Val Asp Ile Phe Gln Thr Val Lys
1               5                   10
```

<210> 953
<211> 13
<212> PRT
<213> Homo Sapiens

<400> 953

```
Met Thr Pro Pro Ser Arg Ala Glu Ala Gly Val Arg Arg
1               5                   10
```

<210> 954
<211> 16
<212> PRT
<213> Homo Sapiens

<400> 954

```
Met Val Gly Asp Val Thr Gly Ala Gln Ala Tyr Ala Ser Thr Ala Lys
1               5                   10                  15
```

<210> 955
<211> 19
<212> PRT
<213> Homo Sapiens

<400> 955

```
Asn Ala Asp Leu Gln Val Leu Lys Pro Glu Pro Glu Leu Val Tyr Glu
1               5                   10                  15

Asp Leu Arg
```

<210> 956
<211> 18
<212> PRT
<213> Homo Sapiens

<400> 956

```
Asn Asn Leu Val Glu Ile Ile Leu Asp Ile Asn Val Ser Gln Leu Thr
1               5                   10                  15

Glu Arg
```

<210> 957
<211> 5
<212> PRT

<213> Homo Sapiens

<400> 957

```
                              Asn Pro Ile Ala Lys
                              1                   5
```

<210> 958
<211> 6
<212> PRT
<213> Homo Sapiens

<400> 958

```
                              Asn Pro Val Asp Val Lys
                              1                   5
```

<210> 959
<211> 6
<212> PRT
<213> Homo Sapiens

<400> 959

```
                              Asn Gln Thr Ala Val Arg
                              1                   5
```

<210> 960
<211> 14
<212> PRT
<213> Homo Sapiens

<400> 960

```
          Asn Arg Ala Gly Leu Gly Glu Glu Phe Glu Lys Glu Ile Arg
          1               5                   10
```

<210> 961
<211> 8
<212> PRT
<213> Homo Sapiens

<400> 961

```
                         Asn Arg Ser Leu Ser Arg Val Arg
                         1               5
```

<210> 962
<211> 9
<212> PRT
<213> Homo Sapiens

<400> 962

```
                         Gln Ile Leu Asp Gln Thr Pro Val Lys
                         1               5
```

<210> 963

<211> 8
<212> PRT
<213> Homo Sapiens

<400> 963

```
Gln Leu Val Glu Ala Leu Asp Lys
1               5
```

<210> 964
<211> 18
<212> PRT
<213> Homo Sapiens

<400> 964

```
Gln Pro His Tyr Ser Ala Phe Gly Ser Val Gly Glu Trp Leu Arg Ala
1           5               10              15

Ile Lys
```

<210> 965
<211> 11
<212> PRT
<213> Homo Sapiens

<400> 965

```
Arg Asp Leu Gly Ser Arg Leu Gln Ala Gln Arg
1               5               10
```

<210> 966
<211> 11
<212> PRT
<213> Homo Sapiens

<400> 966

```
Arg Arg Asp Leu Ser Gln Met Glu Ala Leu Lys
1               5               10
```

<210> 967
<211> 12
<212> PRT
<213> Homo Sapiens

<400> 967

```
Arg Ser Ser Ala Ala Gly Glu Gly Thr Leu Ala Arg
1               5               10
```

<210> 968
<211> 8
<212> PRT
<213> Homo Sapiens

<400> 968

```
                    Ser Ala Ala Ala Ser Asn Tyr Val
                    1               5
```

<210> 969
<211> 19
<212> PRT
<213> Homo Sapiens


<400> 969

```
          Ser Asp Leu Gln Arg Pro Asn Pro Gln Ser Pro Phe Cys Val Ala Ser
          1               5                   10                  15
```

```
          Ser Leu Lys
```

<210> 970
<211> 9
<212> PRT
<213> Homo Sapiens

<400> 970

```
                    Ser Leu Gln Glu Ala Asn Ala Glu Lys
                    1               5
```

<210> 971
<211> 20
<212> PRT
<213> Homo Sapiens

<400> 971

```
          Ser Gln His Gln Glu Thr Pro Val Tyr Leu Gly Ala Thr Ala Gly Met
          1               5                   10                  15
```

```
          Arg Leu Leu Arg
                      20
```

<210> 972
<211> 9
<212> PRT
<213> Homo Sapiens

<400> 972

```
                    Ser Gln Thr Tyr Glu Ser Asp Gly Lys
                    1               5
```

<210> 973
<211> 8
<212> PRT
<213> Homo Sapiens

<400> 973

```
                    Ser Ser Val Lys Glu Leu Leu Lys
                    1               5
```

<210> 974
<211> 16
<212> PRT
<213> Homo Sapiens

<400> 974

```
          Ser Thr Gly Phe Thr Asn Leu Gly Ala Glu Gly Ser Val Phe Pro Lys
          1               5                   10                  15
```

<210> 975
<211> 16
<212> PRT
<213> Homo Sapiens

<400> 975

```
          Ser Thr Gly Thr Ile Ser Val Ile Ser Ser Gly Leu Asp Arg Glu Lys
          1               5                   10                  15
```

<210> 976
<211> 11
<212> PRT
<213> Homo Sapiens

<400> 976

```
                Thr Ala Ile Glu Ile Leu Ala Trp Gly Leu Arg
                1               5                   10
```

<210> 977
<211> 9
<212> PRT
<213> Homo Sapiens

<400> 977

```
                Thr Ala Arg Ala Thr Gly Ala Ser Arg
                1               5
```

<210> 978
<211> 8
<212> PRT
<213> Homo Sapiens

<400> 978

```
                Thr Ala Thr Gln Pro Leu Leu Lys
                1               5
```

<210> 979
<211> 13
<212> PRT
<213> Homo Sapiens

<400> 979

```
                Thr Ala Thr Val Val Met Met Thr Arg Leu Glu Glu Lys
                1               5                   10
```

<210> 980
<211> 11
<212> PRT
<213> Homo Sapiens

<400> 980

```
            Thr Glu Leu Leu Ala Ala Asp Ser Leu Ser Lys
            1               5                   10
```

<210> 981
<211> 14
<212> PRT
<213> Homo Sapiens

<400> 981

```
        Thr Gly Arg Pro Val Glu Pro Glu Ser Glu Phe Ile Ile Lys
        1               5                   10
```

<210> 982
<211> 10
<212> PRT
<213> Homo Sapiens

<400> 982

```
            Thr Leu Ala Glu Val Cys Leu Gly Gln Lys
            1               5                   10
```

<210> 983
<211> 9
<212> PRT
<213> Homo Sapiens

<400> 983

```
            Thr Leu Arg Leu Gly Pro Leu Ser Lys
            1               5
```

<210> 984
<211> 8
<212> PRT
<213> Homo Sapiens

<400> 984

```
            Thr Asn Gly Thr Gly Arg Val Arg
            1               5
```

<210> 985
<211> 13
<212> PRT

<213> Homo Sapiens

<400> 985

```
Thr Ser Lys Thr Thr Phe Gln Leu Glu Leu Pro Val Lys
1               5                   10
```

<210> 986
<211> 9
<212> PRT
<213> Homo Sapiens

<400> 986

```
Thr Ser Asn Gly Arg Leu Pro Val Lys
1               5
```

<210> 987
<211> 9
<212> PRT
<213> Homo Sapiens

<400> 987

```
Thr Val Thr Ile Asn Cys Pro Phe Lys
1               5
```

<210> 988
<211> 21
<212> PRT
<213> Homo Sapiens

<400> 988

```
Val Ala Ser Leu Ile Gly Val Glu Gly Gly His Ser Ile Asp Ser Ser
1               5                   10                  15

Leu Gly Val Leu Arg
                20
```

<210> 989
<211> 19
<212> PRT
<213> Homo Sapiens

<400> 989

```
Val Glu Ala Ser Asn Pro Tyr Val Glu Pro Arg Phe Leu Tyr Leu Gly
1               5                   10                  15

Pro Phe Lys
```

<210> 990
<211> 5
<212> PRT
<213> Homo Sapiens

<400> 990

```
                        Val Phe Pro Gly Lys
                        1               5
```

<210> 991
<211> 10
<212> PRT
<213> Homo Sapiens

<400> 991

```
            Val Lys Cys Asp Gln Tyr Trp Pro Ala Arg
            1               5                   10
```

<210> 992
<211> 10
<212> PRT
<213> Homo Sapiens

<400> 992

```
            Val Lys Thr Thr Ser Leu Thr Glu Lys Lys
            1               5                   10
```

<210> 993
<211> 8
<212> PRT
<213> Homo Sapiens

<400> 993

```
                Val Leu Ala Ala Asn Asn Val Arg
                1               5
```

<210> 994
<211> 7
<212> PRT
<213> Homo Sapiens

<400> 994

```
                Val Leu Asp Val Val Glu Arg
                1               5
```

<210> 995
<211> 5
<212> PRT
<213> Homo Sapiens

<400> 995

```
                    Val Leu Leu Glu Lys
                    1               5
```

<210> 996
<211> 6
<212> PRT

<213> Homo Sapiens

<400> 996

```
                            Val Leu Thr Asp Ile Lys
                            1                   5
```

<210> 997
<211> 11
<212> PRT
<213> Homo Sapiens

<400> 997

```
                     Val Pro Ala His Gln Val Leu Phe Ser Arg Arg
                     1                   5                   10
```

<210> 998
<211> 9
<212> PRT
<213> Homo Sapiens

<400> 998

```
                        Val Gln Ala Val Asn Ser Gln Gly Lys
                        1                   5
```

<210> 999
<211> 11
<212> PRT
<213> Homo Sapiens

<400> 999

```
                     Val Thr Ala Ala Gly Gln Thr Lys Arg Thr Arg
                     1                   5                   10
```

<210> 1000
<211> 6
<212> PRT
<213> Homo Sapiens

<400> 1000

```
                            Val Thr Ala Ile Asn Lys
                            1                   5
```

<210> 1001
<211> 11
<212> PRT
<213> Homo Sapiens

<400> 1001

```
                     Val Thr Leu Pro Ala Gly Pro Asp Ile Leu Arg
                     1                   5                   10
```

<210> 1002

**EP 2 447 719 B1**

<211> 6
<212> PRT
<213> Homo Sapiens

<400> 1002

```
                              Val Val Ile Ser Ile Arg
                              1               5
```

<210> 1003
<211> 9
<212> PRT
<213> Homo Sapiens

<400> 1003

```
                         Val Val Asn Val Ser Asp Leu Tyr Lys
                         1               5
```

<210> 1004
<211> 10
<212> PRT
<213> Homo Sapiens

<400> 1004

```
                         Tyr Phe Thr Ala Ser Leu Pro Phe Glu Lys
                         1               5                   10
```

<210> 1005
<211> 22
<212> PRT
<213> Homo Sapiens

<400> 1005

```
             Tyr Gly Pro Gly Glu Pro Ser Pro Val Ser Glu Thr Val Val Thr Pro
             1               5                   10                  15

             Glu Ala Ala Pro Glu Lys
                             20
```

<210> 1006
<211> 7
<212> PRT
<213> Homo Sapiens

<400> 1006

```
                              Tyr Leu Asn Asn Leu Tyr Lys
                              1               5
```

<210> 1007
<211> 14
<212> PRT
<213> Homo Sapiens

<400> 1007

```
        Ile Gly Thr Phe Cys Ser Asn Gly Thr Val Ser Arg Ile Lys
        1               5                   10
```

<210> 1008
<211> 16
<212> PRT
<213> Homo Sapiens

<400> 1008

```
        Gln Thr Pro Ala Asp Gly Glu Ala Ser Gly Glu Ser Glu Pro Ala Lys
        1               5                   10                      15
```

**Description**

INTRODUCTION

The present invention relates to the identification of a membrane protein associated with lung cancer which hasutility as a marker for lung cancer and which also forms a biological target against which antibodies such as therapeutic antibodies (or other affinity reagents) or other pharmaceutical agents can be made.

BACKGROUND OF THE INVENTION

**Lung Cancer**

Lung cancer is the most common form of cancer worldwide (accounting for about 12% of cancer cases) and the main cause of death from cancer (accounting for about 18% of deaths). Global incidence of lung cancer is over 1,300,000 per year, with the number of deaths over 1,100,000. In the USA, there are about 170,000 new cases per year (about 13% of all cancers), with about 160,000 deaths (about 28% of cancer deaths). Lung cancer is much more prevalent among men than women. Nearly 70% of people diagnosed with lung cancer are older than 65; fewer than 3% of all cases are found in people under the age of 45. Around 15% of all lung cancers are small cell type (SCLC), which tend to spread widely through the body, while the remaining 85% are non-small cell (NSCLC). It has been estimated that approximately US$9.6 billion is spent in the USA each year on treating lung cancer.

**Lung Cancer Diagnosis**

Lung cancer is a life-threatening disease because it often metastasises even before it can be detected on a chest x-ray. Usually symptoms of lung cancer do not appear until the disease is in an advanced stage. So far, there is no screening test that has been shown to improve a person's chance for a cure. Imaging tests such as a chest x-ray, CT scan, MRI scan or PET scan may be used to detect lung cancer. Tests to confirm the diagnosis are then performed and include sputum cytology, needle biopsy, bronchoscopy, endobronchial ultrasound and complete blood count (CBC).

**Lung Cancer Staging**

Nearly 60% of people diagnosed with lung cancer die within one year of diagnosis; 75% die within 2 years. The 5-year survival rate for people diagnosed with NSCLC is about 15%; for SCLC the 5-year survival rate is about 6%. NSCLC is staged using the American Joint Committee on Cancer (AJCC) TNM system - Stage 0 - Stage IV. The 5-year survival rates by stage are as follows: stage I: 47%; stage II; 26%; stage III: 8% and stage IV: 2%. SCLC has a 2-stage system - limited stage and extensive stage. About two thirds of SCLC patients have extensive disease at diagnosis. If SCLC is found very early and is localised to the lung alone, the 5-year survival rate is around 21%, but only 6% of patients fall into this category. Where the cancer has spread, the 5-year survival is around 11%. For patients with extensive disease, the 5-year survival is just 2%.

**Lung Cancer Treatment**

Surgery is the only reliable method to cure NSCLC. Types of surgery include lobectomy, pneumonectomy, segmentectomy and video-assisted thoracic surgery (for small tumours). External beam radiation therapy is sometimes used as the primary treatment, especially if the patient's health is too poor to undergo surgery. Radiation therapy can also be used after surgery. Chemotherapy may be given as the primary treatment or as an adjuvant to surgery. Targeted therapy using epidermal growth factor receptor (EGFR) antagonists such as gefitinib or erlotinib can also be given after other treatments have failed. Antiangiogenic drugs, such as bevacizumab, have been found to prolong survival of patients with advanced lung cancer. Photodynamic therapy is also being researched as a treatment for lung cancer.
The main treatment for SCLC is chemotherapy, either alone or in combination with external beam radiation therapy and very rarely, surgery.
Chemotherapeutic agents used for NSCLC and SCLC include cisplatin, carboplatin, mitomycin C, ifosfamide, vinblastine, gemcitabine, etoposide, vinorelbine, paclitaxel, docetaxel and irinotecan.

**Therapeutic Challenges**

The major challenges in treatment of lung cancer is to improve early detection rates, to find new non-invasive markers that can be used to follow disease progression and identify relapse, and to find improved and less toxic therapies, especially for more advanced disease where 5 year survival is still poor. There is a great need to identify targets which are more specific to the cancer cells, e.g. ones which are expressed on the surface of the tumour cells so that they can be attacked by promising new approaches like immunotherapeutics and targeted toxins.

## SUMMARY OF THE INVENTION

The present invention provides an isolated monoclonal antibody capable of immuno-specific binding to CUB domain-containing protein 1 (CDCP1), or a fragment or derivative thereof which comprises the antigen binding domain, for use in treating lung cancer wherein the antibody or fragment or derivative thereof is conjugated to a cytotoxic moiety or a drug moiety The present disclosure also provides methods and compositions for:

- therapy of, or
- drug development for, or
- screening, diagnosis and prognosis for, or
- monitoring the effectiveness of treatment for

B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma (including patient stratification).

We have used mass spectrometry to identify peptides generated by gel electrophoresis or tagging with iTRAQ reagents and tryptic digest of membrane proteins extracted from lymphoid, breast, cervical, colorectal, gastric, brain, liver, lung, skin, neuronal, osteoblast, ovarian, pancreatic, prostate, kidney and eye cancer tissue samples. Peptide sequences were compared to existing protein and cDNA databases and the corresponding gene sequences identified. The proteins of the invention have not been previously reported to originate from lymphocytic, breast, cervical, colorectal, gastric, brain, liver, lung, skin, neuronal, osteoblast, ovarian, pancreatic, prostate, kidney or eye cancer cell membranes and represent proteins of new therapeutic and/or diagnostic value.

A first example of the disclosure provides methods of treating B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma, comprising administering to a patient a therapeutically effective amount of a compound that modulates (e.g., upregulates or downregulates) or complements the expression or the biological activity (or both) of one or more of the proteins of the invention in patients having B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma, in order to ((a) prevent the onset or development of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma; (b) prevent the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma; or (c) ameliorate the symptoms of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma.

According to a second example of the disclosure we provide a method of detecting, diagnosing and/or screening for or monitoring the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or of monitoring the effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in a subject which comprises detecting the presence or level of the proteins of the disclosure, or one or more fragments thereof, or the presence or level of nucleic acid encoding the proteins of the disclosure or the presence or level of the activity of the proteins of the discloure or which comprises detecting a change in the level thereof in said subject.

According to a third example of the disclosure we provide a method of detecting, diagnosing and/or screening for B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in a candidate subject which comprises detecting the presence of the proteins of the disclosure, or one or more fragments thereof, or the presence of nucleic acid encoding the proteins of the discloure or the presence of the activity of the proteins of the disclosure in said candidate subject, in which either (a) the presence of an elevated level of the proteins of the disclosure or said one or more fragments thereof or an elevated level of nucleic acid encoding the proteins of the disclosure or the presence of an elevated level of the activity of the proteins of the disclosure in the candidate subject as compared with the level in a healthy subject or (b) the presence of a detectable level of the proteins of the disclosure or said one or more fragments thereof or a detectable level of nucleic acid encoding the proteins of the disclosure or the presence of a detectable level of the activity of the proteins of the disclosure in the candidate subject as compared with a corresponding undetectable level in a healthy subject indicates the presence of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in said subject.

According to a fourth example of the discloure we provide a method of monitoring the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in a subject or of monitoring the effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy which comprises detecting the presence of the proteins of the invention, or one or more fragments thereof, or the presence of nucleic acid encoding the proteins of the disclosure or the presence of the activity of the proteins of the invention in said candidate subject at a first time point and at a later time point, the presence of an elevated or lowered level of the proteins of the disclosure or said one or more fragments thereof or an elevated or lowered level of nucleic acid encoding the proteins of the discloure or the presence of an elevated or lowered level of the activity of the proteins of the disclosure in the subject at the later time point as compared with the level in the subject at said first time point, indicating the progression or regression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or indicating the effect or non-effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in said subject.

The presence of the proteins of the discloure, or one or more fragments thereof, or the presence of nucleic acid encoding the proteins of the discloure or the presence of the activity of the proteins of the disclosure may, for example, be detected by analysis of a biological sample obtained from said subject.

The method of discloure may typically include the step of obtaining a biological sample for analysis from said subject. One or more examples the methods of the discloure do not include the step of obtaining a biological sample from a subject.

One example of the discloure provides monoclonal and polyclonal antibodies or other affinity reagents such as an Affibodies capable of immunospecific binding to the proteins of the discloure and compositions comprising same, for example for use in treatment or prophylaxis, such for the treatment or prophylaxis of cancer, particularly a cancer described herein.

The biological sample used can be from any source such as a serum sample or a tissue sample, e.g. lymphoid, breast, cervical, colorectal, gastric, brain, liver, lung, skin, neuronal, osteoblast, ovarian, pancreatic, prostate, kidney or eye tissue. For instance, when looking for evidence of metastatic breast cancer, cervical cancer, colorectal cancer, gastric cancer, hepatocellular carcinoma, lung cancer, melanoma, neuroblastoma, osteosarcoma, ovarian cancer, pancreatic cancer, prostate cancer, renal cell cancer and retinoblastoma, one would look at major sites of breast cancer, cervical cancer, colorectal cancer, gastric cancer, hepatocellular carcinoma, lung cancer, melanoma, neuroblastoma, osteosarcoma, ovarian cancer, pancreatic cancer, prostate cancer, renal cell cancer and retinoblastoma metastasis, e.g. the liver, the lungs and bones for breast cancer; the bladder and the rectum for cervical cancer; the liver, the peritoneal cavity, the pelvis, the retroperitoneum and the lungs for colorectal cancer; the liver, the lungs, the brain and bones for gastric cancer; the lungs and bones for hepatocellular carcinoma; the brain, the liver, the bones and adrenal glands for lung cancer; the lungs, the brain and bones for melanoma; the liver and bones for neuroblastoma; the lungs and other bones for osteosarcoma; the abdomen for ovarian cancer; the liver for pancreatic cancer; the bladder, the rectum and bones for prostate cancer; the lungs, the liver and bones for renal cell cancer and the brain and bones for retinoblastoma.

Alternatively the presence of the proteins of the discloure, or one or more fragments thereof, or the presence of nucleic acid encoding the proteins of the discloure or the presence of the activity of the proteins of the discloure may be detected by analysis in situ.

In certain examples, methods of diagnosis described herein may be at least partly, or wholly, performed *in vitro*.

Suitably the presence of the proteins of the discloure, or one or more fragments thereof, or the presence of nucleic acid encoding the proteins of the discloure or the presence of the activity of the proteins of the discloure is detected quantitatively.

For example, quantitatively detecting may comprise:

(a) contacting a biological sample with an affinity reagent that is specific for the proteins of the discloure, said affinity reagent optionally being conjugated to a detectable label; and
(b) detecting whether binding has occurred between the affinity reagent and at least one species in the sample, said detection being performed either directly or indirectly.

Alternatively the presence of the proteins of the discloure, or one or more fragments thereof, or the presence of nucleic acid encoding the proteins of the discloure or the presence of the activity of the proteins of the discloure may be detected quantitatively by means involving use of an imaging technology.

In another example, the method of the discloure involves use of immunohistochemistry on tissue sections in order to determine the presence of the proteins of the discloure, or one or more fragments thereof, or the presence of nucleic acid encoding the proteins of the discloure or the presence of the activity of the proteins of the discloure, and thereby to localise B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma cells.

In one example the presence of the proteins of the discloure or one or more epitope-containing fragments thereof is detected, for example using an affinity reagent capable of specific binding to the proteins of the discloure or one or more fragments thereof, such as an antibody.

In another example the activity of the proteins of the discloure is detected.

According to another example of the discloure there is provided a method of detecting, diagnosing and/or screening for or monitoring the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or of monitoring the effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in a subject which comprises detecting the presence or level of antibodies capable of immunospecific binding to the proteins of the discloure, or one or more epitope-containing fragments thereof or which comprises detecting a change in the level thereof in said subject.

According to another example of the discloure there is also provided a method of detecting, diagnosing and/or screening for B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in a subject which comprises detecting the presence of antibodies capable of immunospecific binding to the proteins of the discloure, or one or more epitope-containing fragments thereof in said subject, in which (a) the presence of an elevated level of antibodies capable of immunospecific binding to the proteins of the discloure or said one or more epitope-containing fragments thereof in said subject as compared with the level in a healthy subject or (b) the presence of a detectable level of antibodies capable of immunospecific binding to the proteins of the discloure or said one or more epitope-containing fragments thereof in said subject as compared with a corresponding undetectable level in a healthy subject indicates the presence of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma in said subject.

One particular method of detecting, diagnosing and/or screening for B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma comprises:

(a) bringing into contact with a biological sample to be tested the proteins of the discloure, or one or more epitope-containing fragments thereof; and
(b) detecting the presence of antibodies in the subject capable of immunospecific binding to the proteins of the discloure, or one or more epitope-containing fragments thereof

According to another example of the discloure there is provided a method of monitoring the progression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or of monitoring the effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in a subject which comprises detecting the presence of antibodies capable of immunospecific binding to the proteins of the discloure, or one or more epitope-containing fragments thereof in said subject at a first time point and at a later time point, the presence of an elevated or lowered level of antibodies capable of immunospecific binding to the proteins of the discloure, or one or more epitope-containing fragments thereof in said subject at the later time point as compared with the level in said subject at said first time point, indicating the progression or regression of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma or the effect or non-effect of an anti-B-cell non-Hodgkin's lymphoma, anti-breast cancer, anti-cervical cancer, anti-colorectal cancer, anti-gastric cancer, anti-glioblastoma, anti-hepatocellular carcinoma, anti-lung cancer, anti-lymphoid leukaemia (particularly acute T-cell leukaemia and chronic lymphocytic leukaemia), anti-melanoma, anti-neuroblastoma, anti-osteosarcoma, anti-ovarian cancer, anti-pancreatic cancer, anti-prostate cancer, anti-renal cell cancer or anti-retinoblastoma drug or therapy in said subject.

The presence of antibodies capable of immunospecific binding to the proteins of the discloure, or one or more epitope-containing fragments thereof is typically detected by analysis of a biological sample obtained from said subject (exemplary biological samples are mentioned above, e.g. the sample is a sample of lymphoid, breast, cervical, colorectal, gastric, brain, liver, lung, skin, neuronal, osteoblast, ovarian, pancreatic, prostate, kidney or eye tissue, or else a sample of blood or saliva).

The method typically includes the step of obtaining said biological sample for analysis from said subject.

The antibodies that may be detected include IgA, IgM and IgG antibodies.

In any of the above methods, the level that may be detected in the candidate subject who has B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma is 2 or more fold higher than the level in the healthy subject.

Also described in the discloure is an agent capable of specific binding to the proteins of the discloure, or a fragment thereof, or a hybridising agent capable of hybridizing to nucleic acid encoding the proteins of the discloure or an agent capable of detecting the activity of the proteins of the discloure for use in screening for, detecting and/or diagnosing disease, such as cancer, and especially B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma.

Also described are the proteins of the discloure, or fragments thereof for use in screening for, detecting and/or diagnosing disease such as cancer, and especially B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma.

Also described are affinity reagents capable of specific binding to the proteins of the discloure or fragments thereof, for example an affinity reagent which contains or is conjugated to a detectable label or contains or is conjugated to a therapeutic moiety such as a cytotoxic moiety. The affinity reagent may, for example, be an antibody.

Also described are hybridizing agents capable of hybridizing to nucleic acid encoding the proteins of the discloure, for example, a hybridizing agent which contains or is conjugated to a detectable label. One example of a hybridizing agent is an inhibitory RNA (RNAi). Other examples include anti-sense oligonucleotides and ribozymes.

The discloure also provides kits containing the proteins of the discloure and/or one or more fragments thereof or containing one or more aforementioned affinity reagents and/or hybridizing agents or containing one or more agents capable of detecting the activity of the proteins of the discloure together with instructions for their use in an aforementioned method. The kit may further contain reagents capable of detecting and reporting the binding of said affinity reagents and/or hybridizing agents to their binding partners.

Also described are pharmaceutical compositions comprising a therapeutically effective amount of affinity reagents capable of specific binding to the proteins of the discloure or a fragment thereof.

Another example of the discloure is a pharmaceutically acceptable diluent or carrier and a pharmaceutical composition comprising one or more affinity reagents or hybridizing reagents as aforesaid and a pharmaceutically acceptable diluent or carrier.

In one example the cancer to be detected, prevented or treated is B-cell non-Hodgkin's lymphoma.

In one example the cancer to be detected, prevented or treated is breast cancer.

In one example the cancer to be detected, prevented or treated is cervical cancer.

In one example the cancer to be detected, prevented or treated is colorectal cancer.

In one example the cancer to be detected, prevented or treated is gastric cancer.

In one example the cancer to be detected, prevented or treated is glioblastoma.

In one example the cancer to be detected, prevented or treated is hepatocellular carcinoma.

In one embodiment the cancer to be detected, prevented or treated is lung cancer.

In one example the cancer to be detected, prevented or treated is lymphoid leukaemia (particularly acute T-cell leukaemia and chronic lymphocytic leukaemia).

In one example the cancer to be detected, prevented or treated is melanoma.

In one example the cancer to be detected, prevented or treated is neuroblastoma.

In one example the cancer to be detected, prevented or treated is osteosarcoma.

In another example the cancer to be detected, prevented or treated is ovarian cancer.

In another example the cancer to be detected, prevented or treated is pancreatic cancer.

In another example the cancer to be detected, prevented or treated is prostate cancer.

In another example the cancer to be detected, prevented or treated is renal cell cancer.

In another example the cancer to be detected, prevented or treated is retinoblastoma.

Other aspects of the present invention are set out below and in the claims herein.

## BRIEF DESCRIPTION OF THE FIGURES

Figure 1 shows the amino acid sequences of the proteins of the disclosure. The tryptics detected experimentally by mass spectrometry are highlighted - mass match peptides are shown in bold, tandem peptides are underlined.

Figures 2 and 4 show so-called Box Plot data for OGTA066, as described in Examples 3 and 4.

Figure 3 shows ROC curve data for OGTA066, as described in Example 4.

## DETAILED DESCRIPTION OF THE INVENTION

The invention encompasses the administration of therapeutic compositions to a mammalian subject to treat or prevent lung cancer. Also described is the administration of therapeutic compositions to a mammalian subject to treat or prevent B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma.

The disclosure also encompasses clinical screening, diagnosis and prognosis in a mammalian subject for identifying patients most likely to respond to a particular therapeutic treatment, for monitoring the results of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma therapy, for drug screening and drug development. The mammalian subject may be a non-human mammal, for example a human, such as a human adult, i.e. a human subject at least 21 (more preferably at least 35, at least 50, at least 60, at least 70, or at least 80) years old. For clarity of disclosure, and not by way of limitation, the disclosure will be described with respect to the analysis of lymphoid, breast, cervical, colorectal, gastric, brain, liver, lung, skin, neuronal, osteoblast, ovarian, pancreatic, prostate, kidney and eye tissue. However, as one skilled in the art will appreciate, the assays and techniques described below can be applied to other types of patient samples, including body fluids (*e.g.* blood, urine or saliva), a tissue sample from a patient at risk of having B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma (e.g. a biopsy such as a bone marrow, breast, cervical, liver, stomach, brain, lung, skin, bone, ovarian, pancreatic, prostate or kidney biopsy) or homogenate thereof.

The methods and compositions of the present disclosure are specially suited for screening, diagnosis and prognosis of a living subject, but may also be used for postmortem diagnosis in a subject, for example, to identify family members at risk of developing the same disease.

A relevant cancer as used herein refers to B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and/or retinoblastoma.

Proteins of the invention

In one example, one-dimensional electrophoresis or isobaric tags for relative and absolute quantification (iTRAQ) or other appropriate methods are used to analyse relevant cancer tissue samples from a subject, such as a living subject, in order to measure the expression of the proteins of the disclosure for screening or diagnosis of a relevant cancer, to determine the prognosis of a patient with same, to monitor the effectiveness of therapy for same, or for drug development in relation to same.

As used herein, the terms:

- OGTA(s), or
- OGTA according to the invention, or
- OGTA employed in the invention or
- "Proteins of the invention",

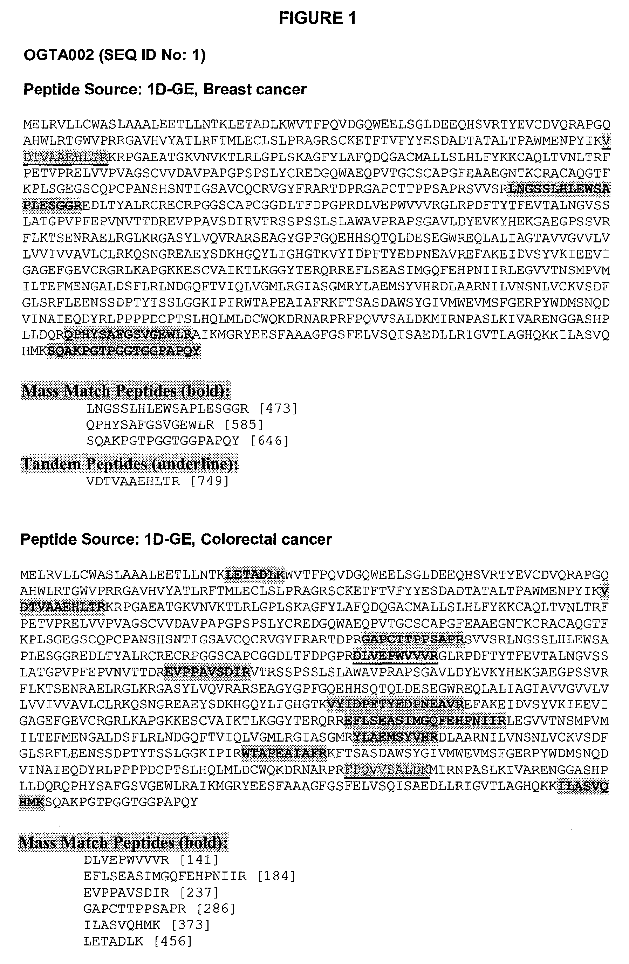

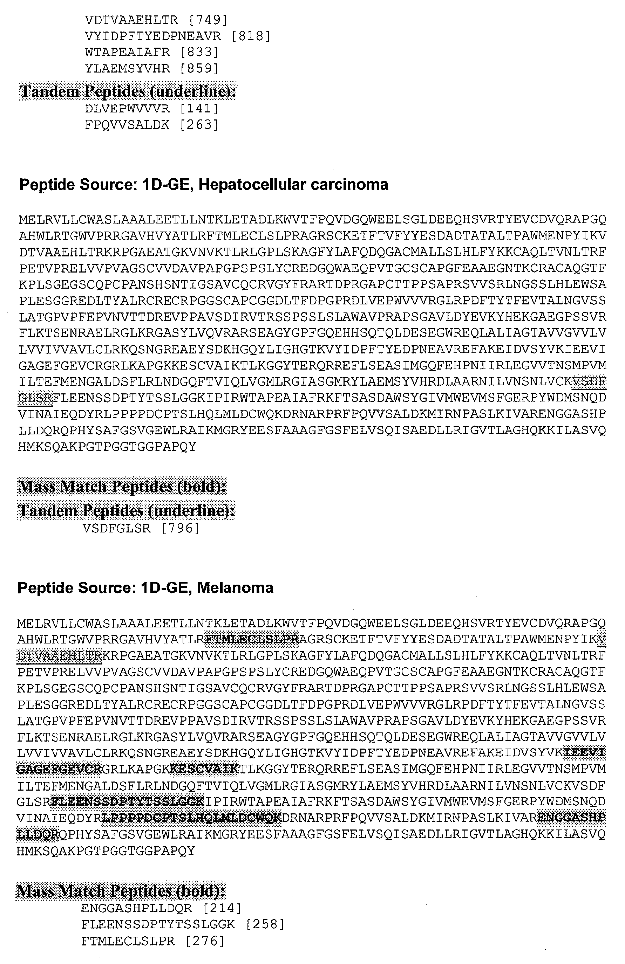

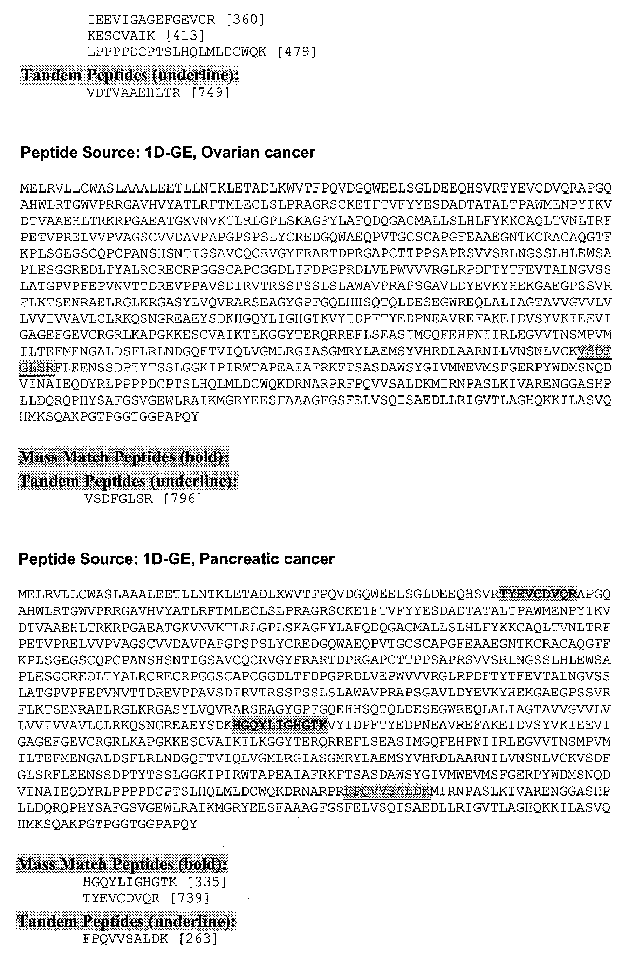

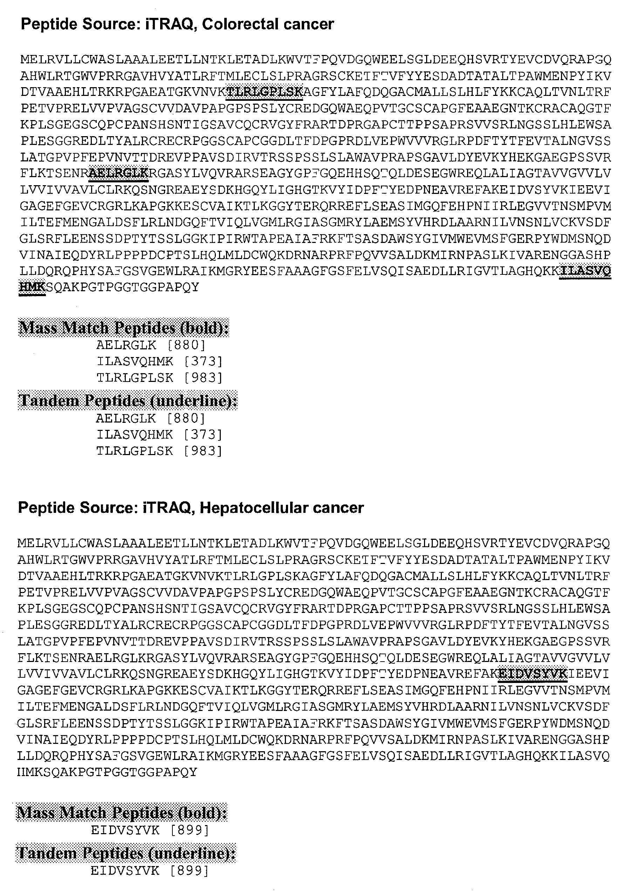

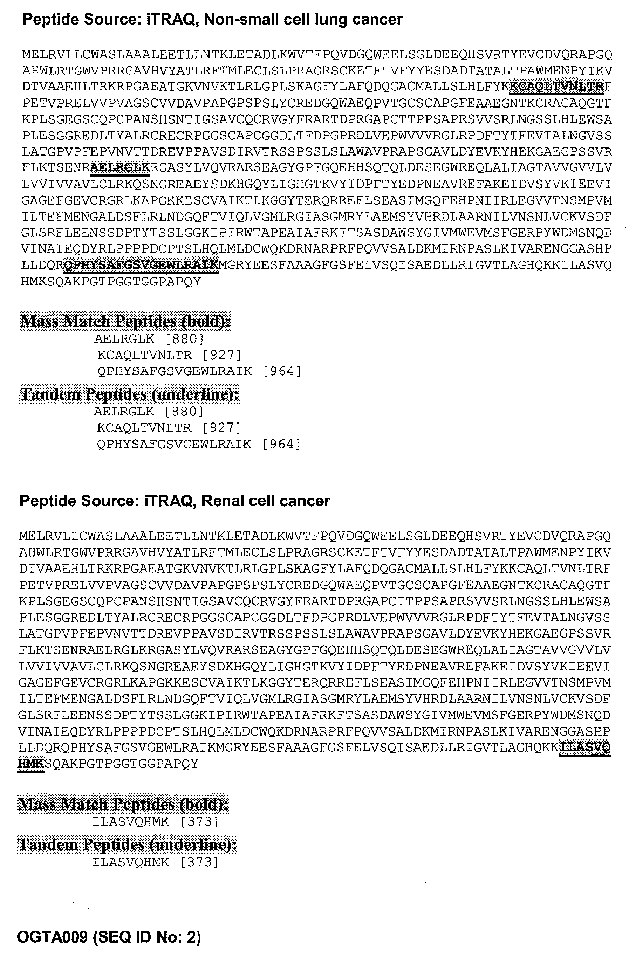

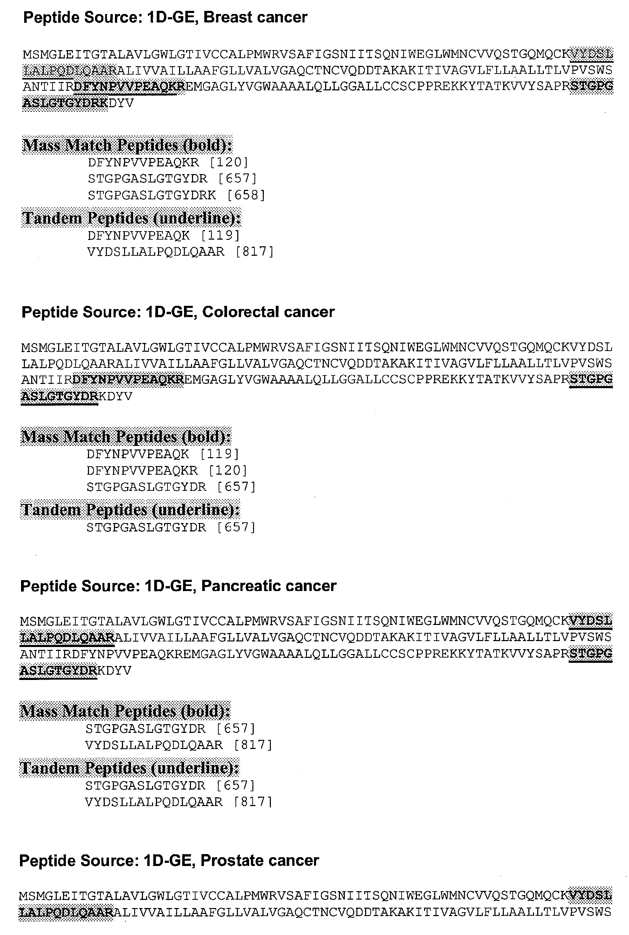

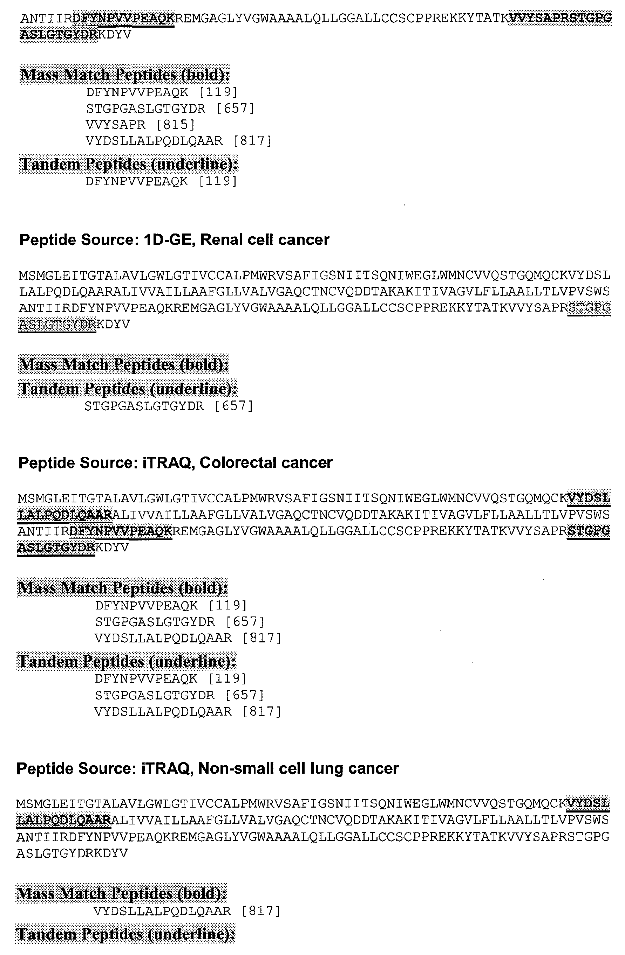

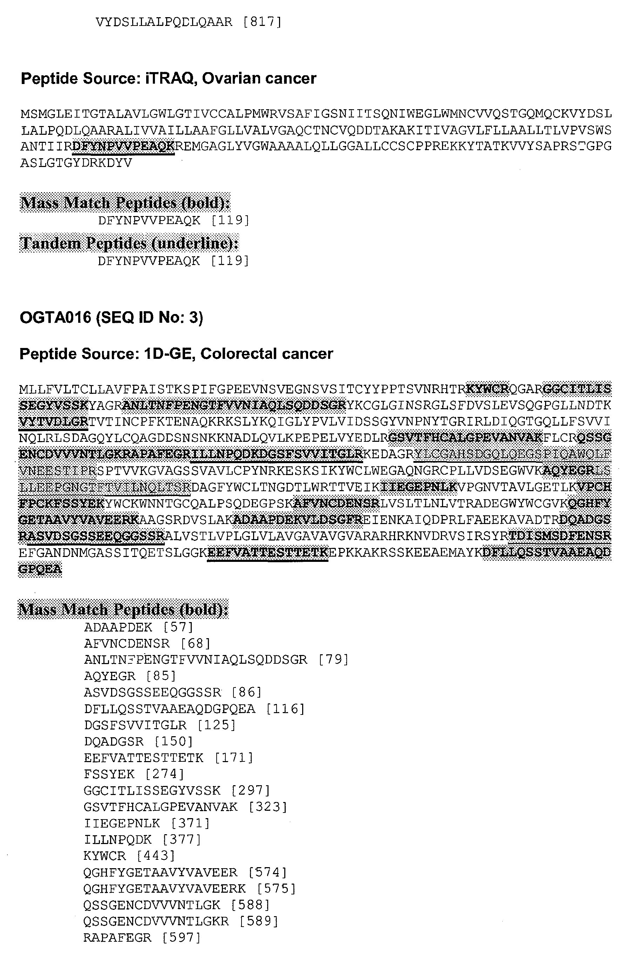

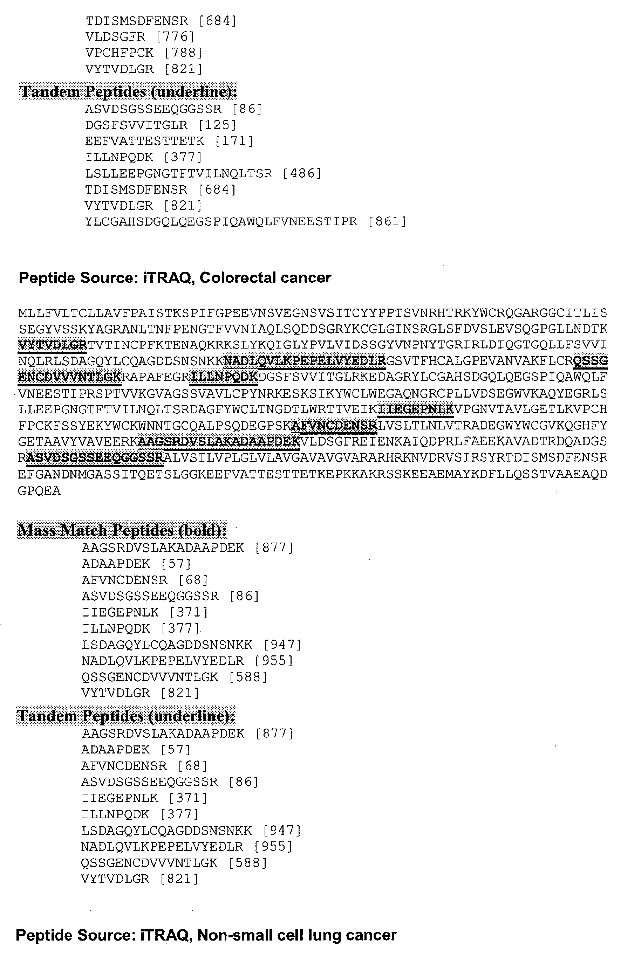

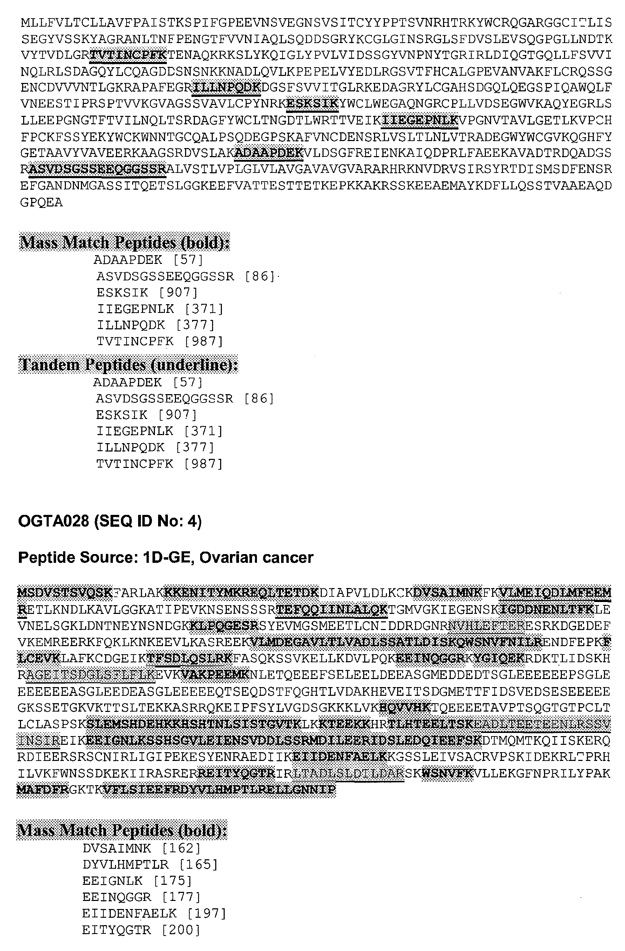

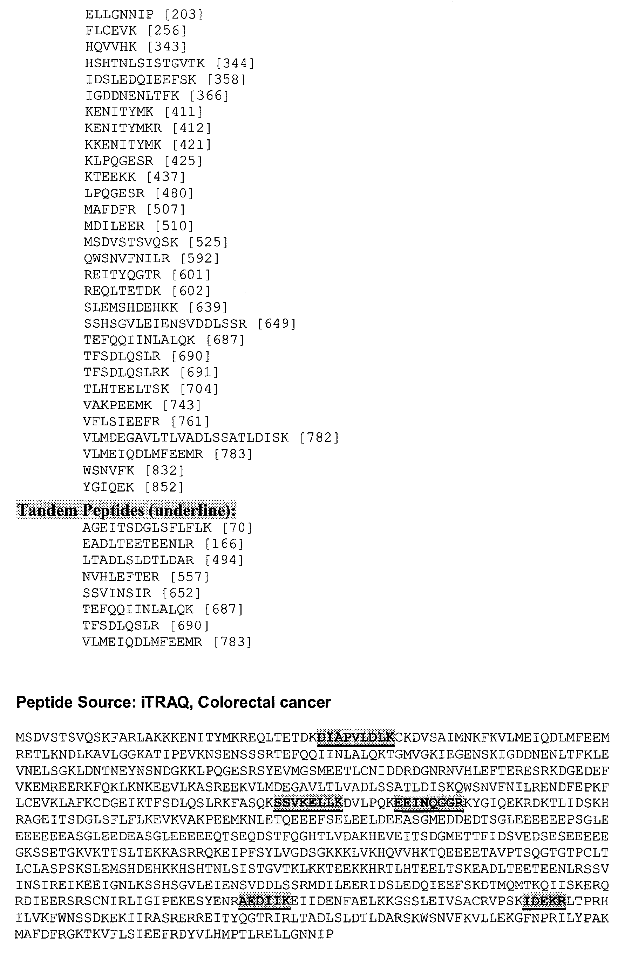

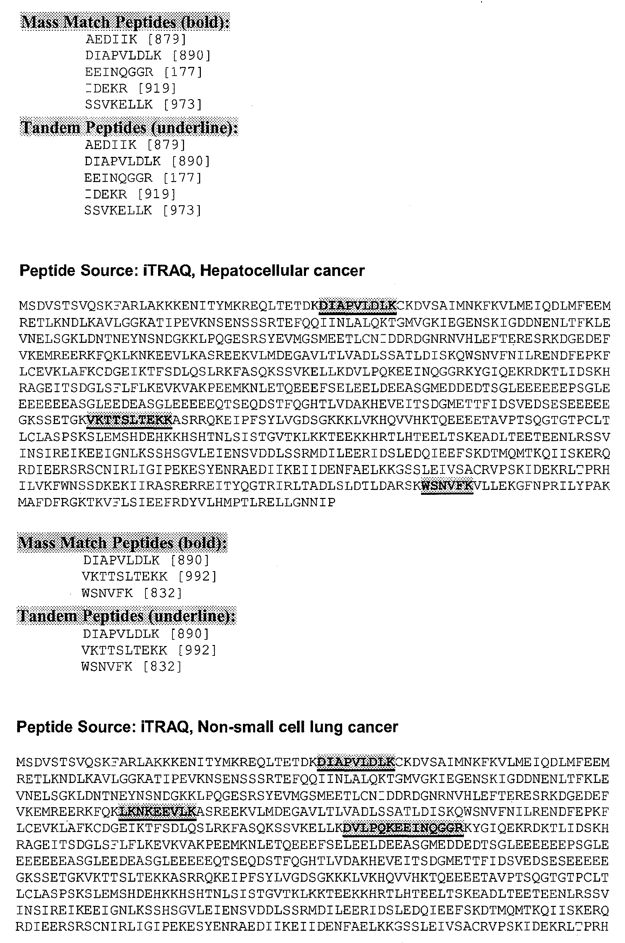

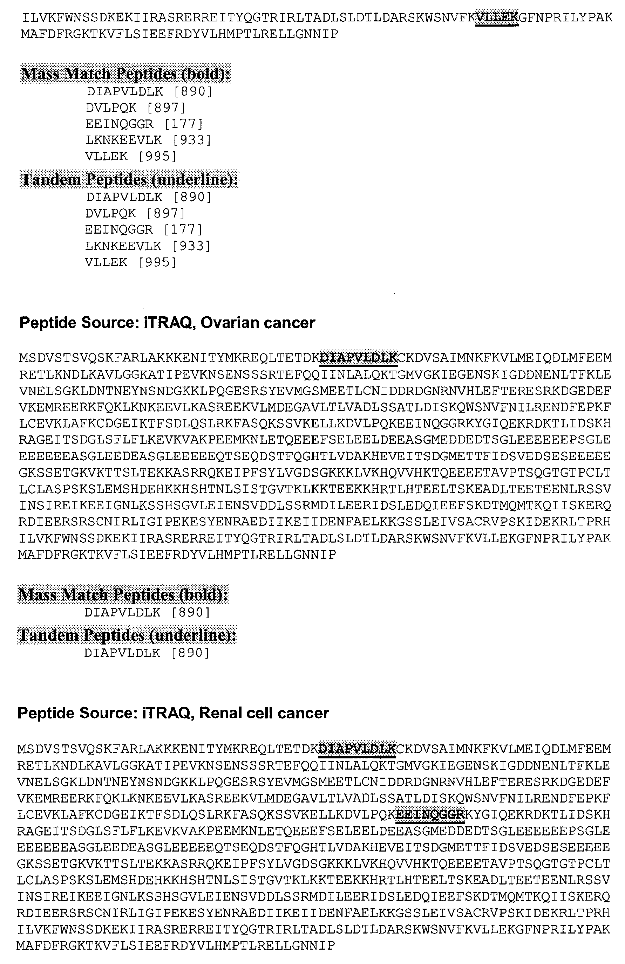

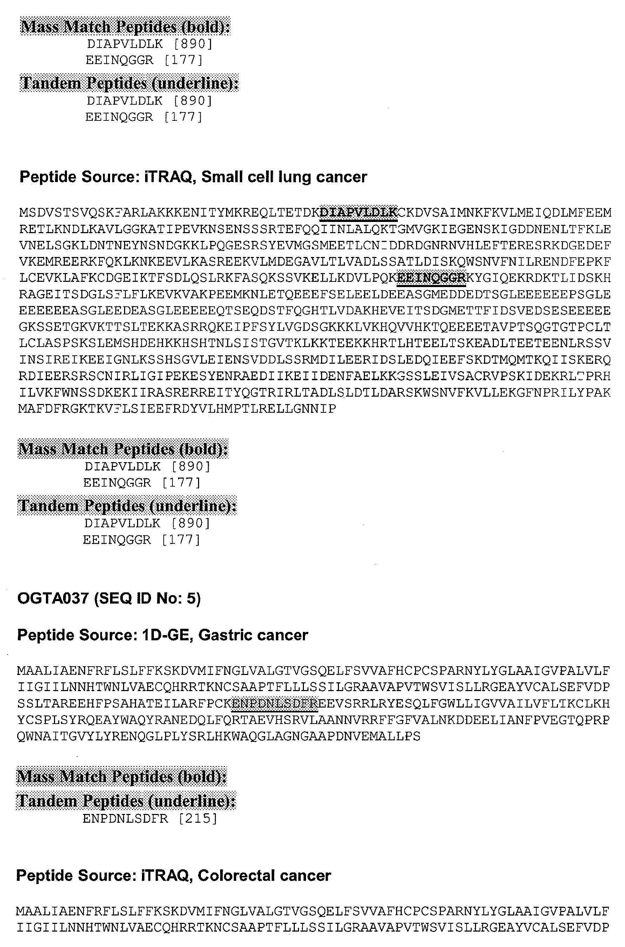

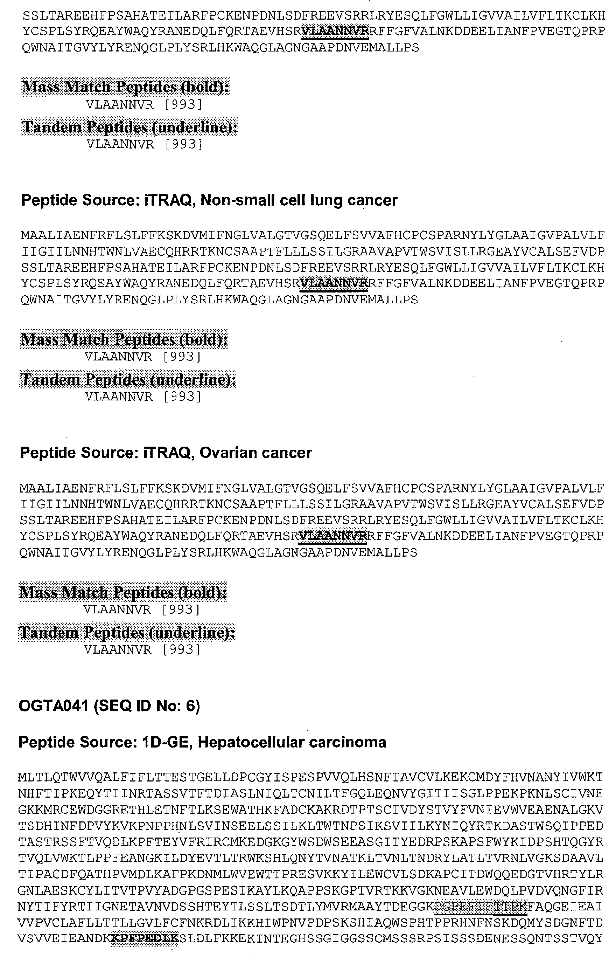

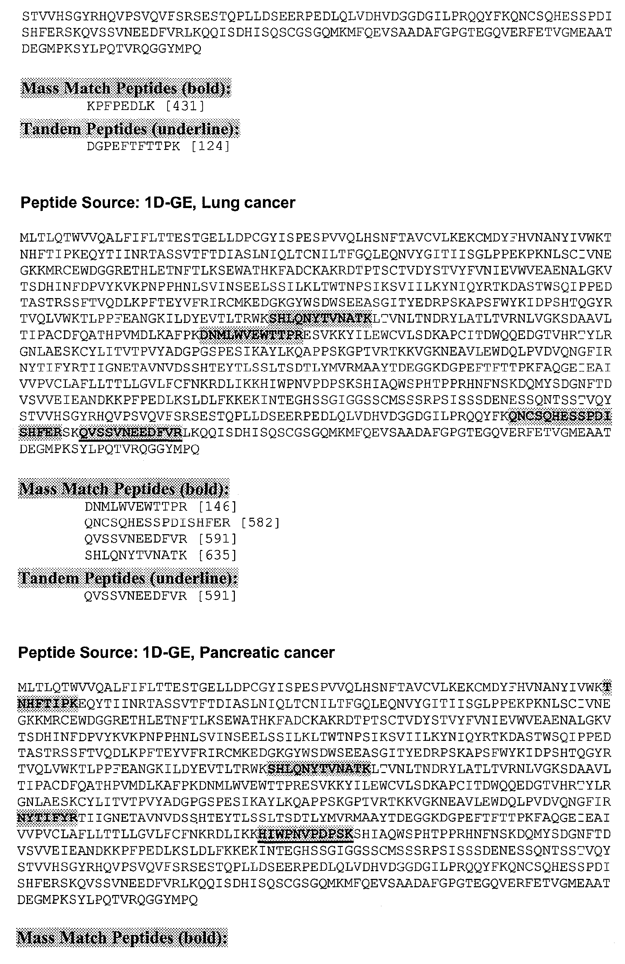

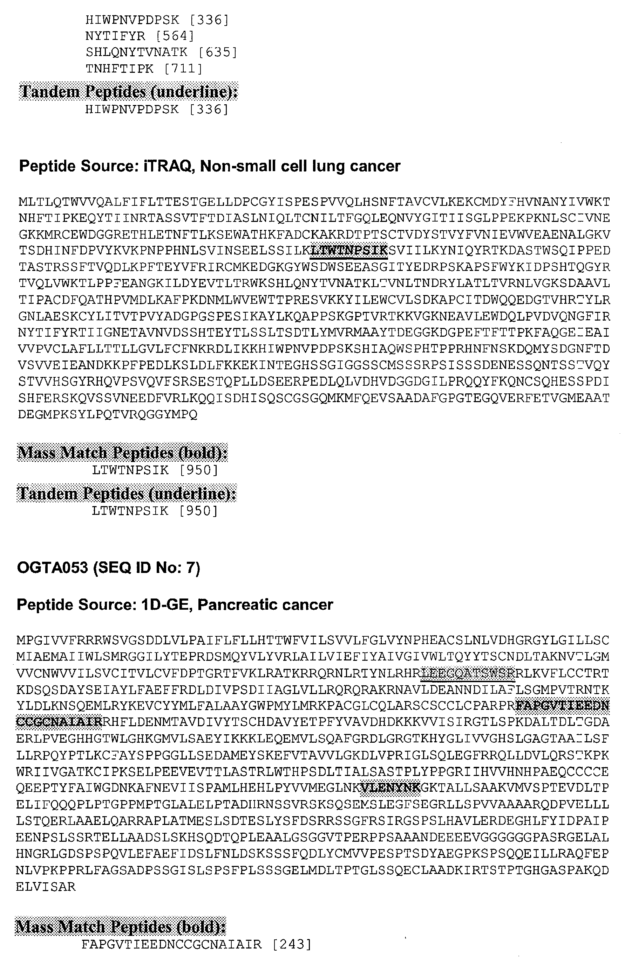

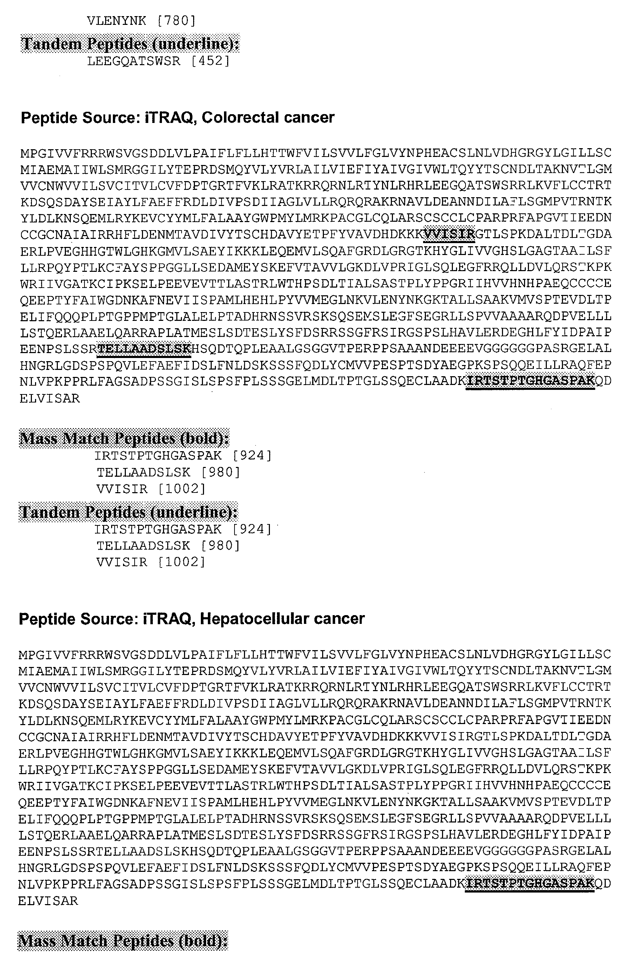

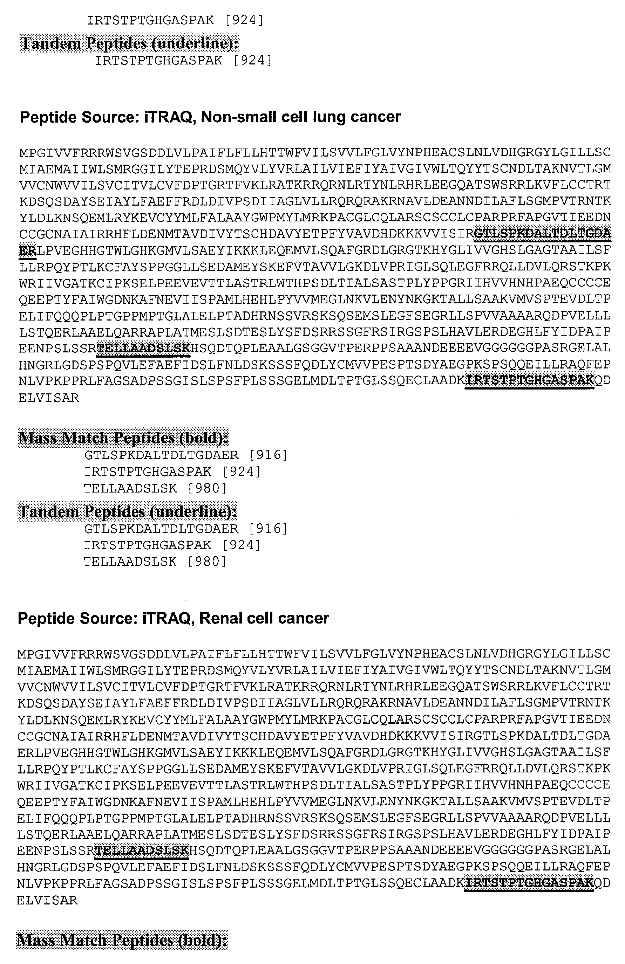

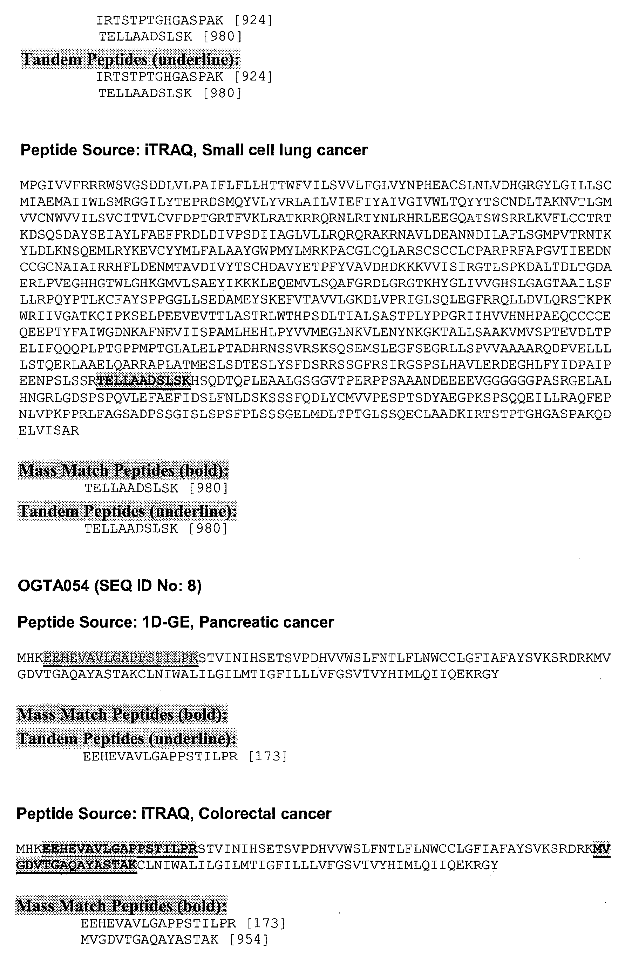

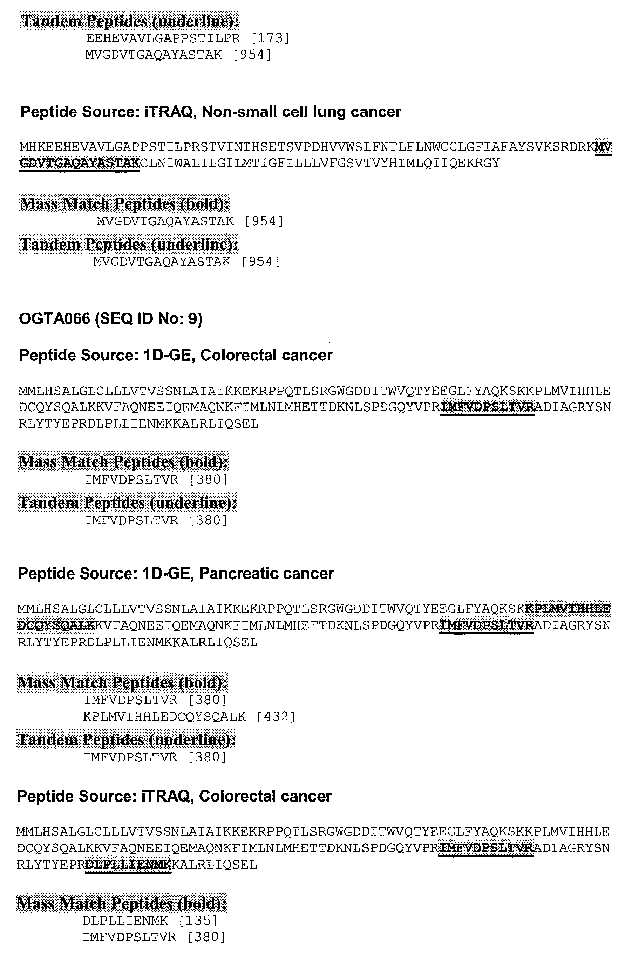

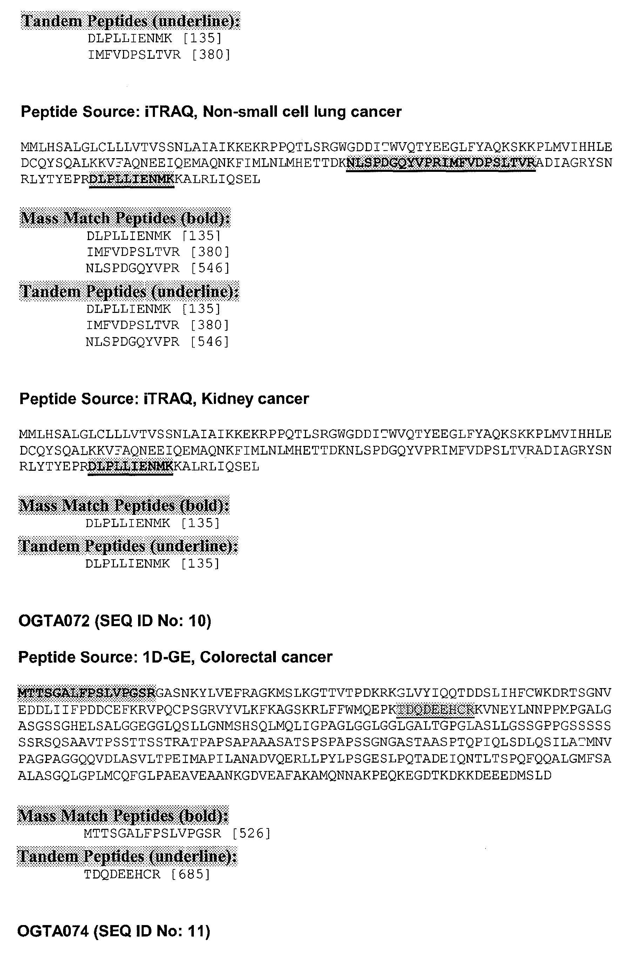

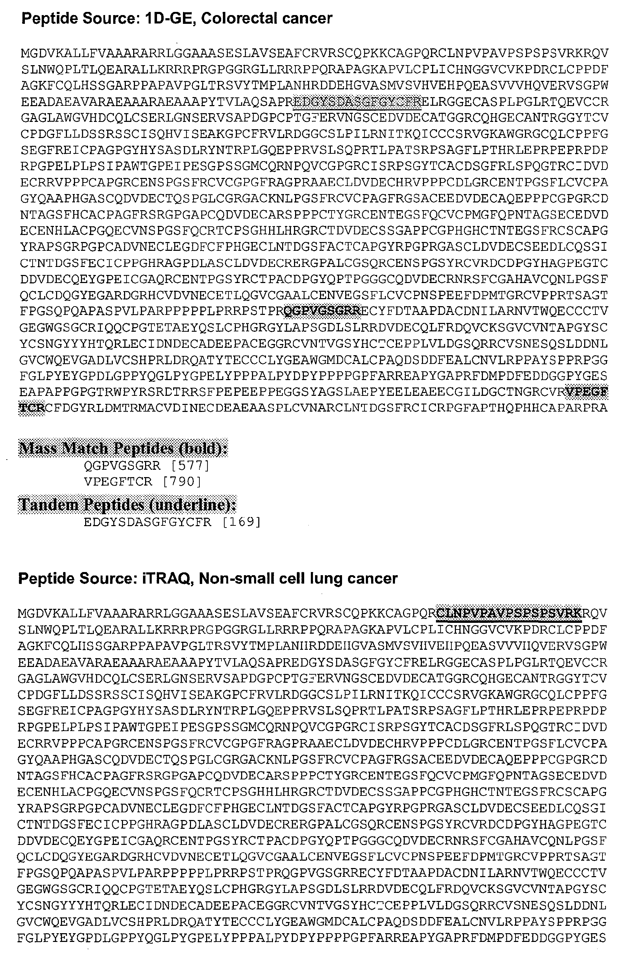

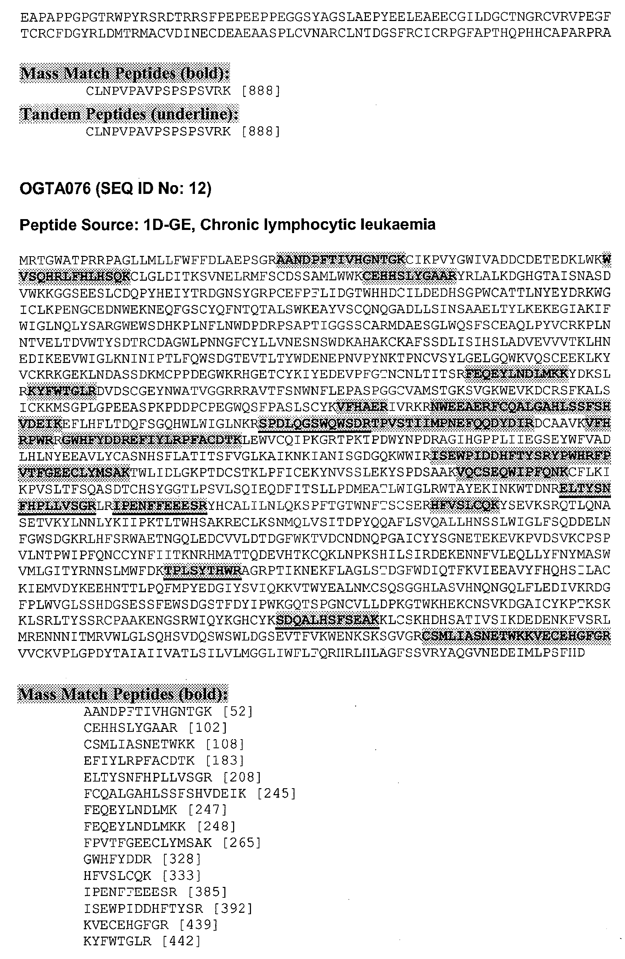

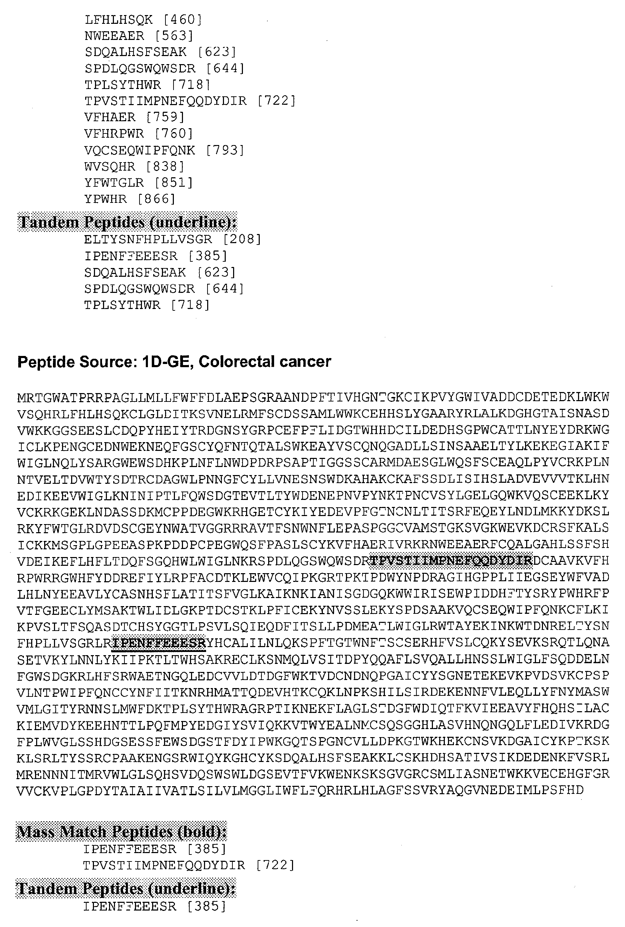

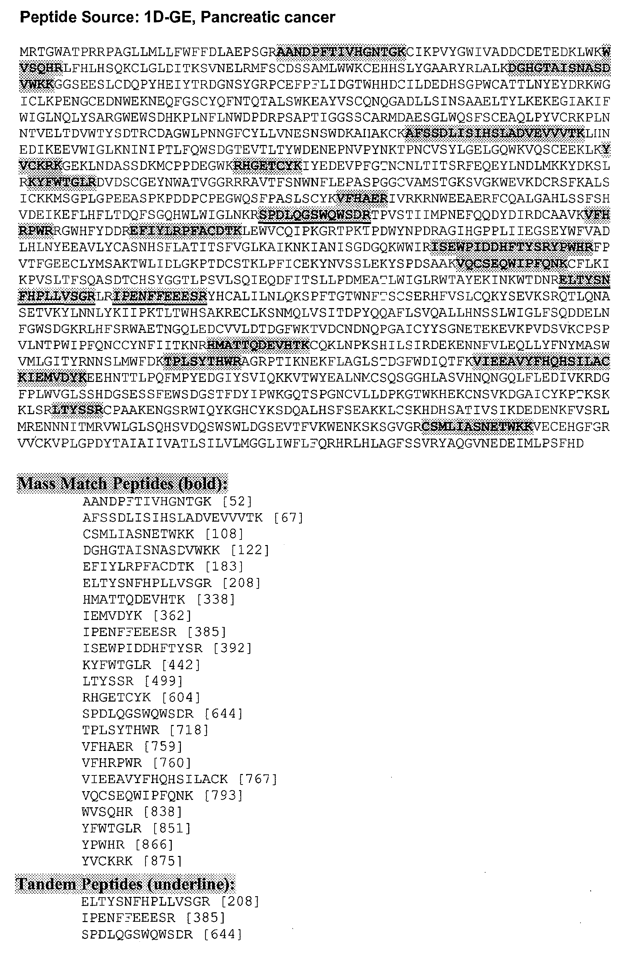

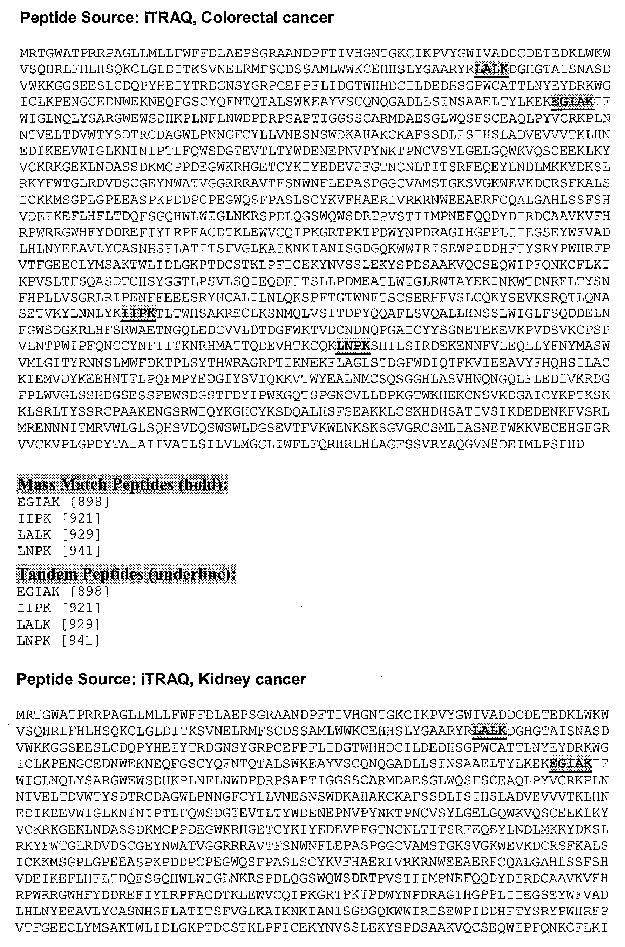

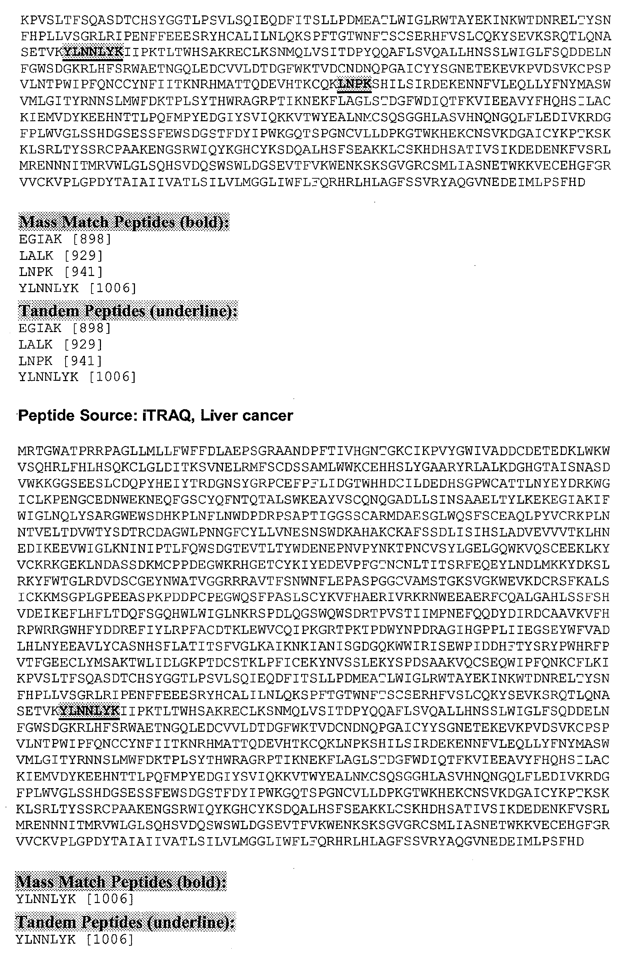

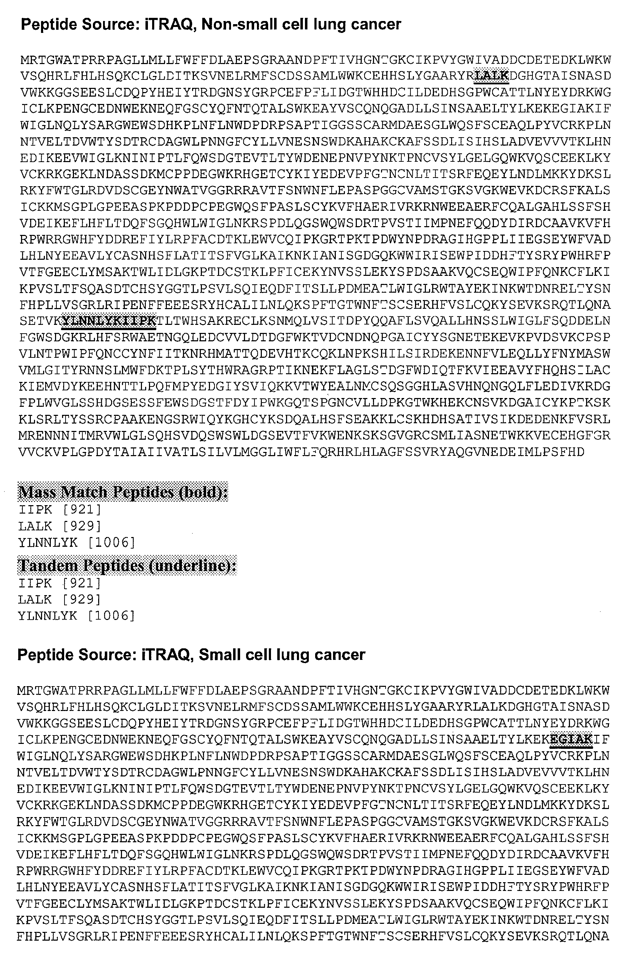

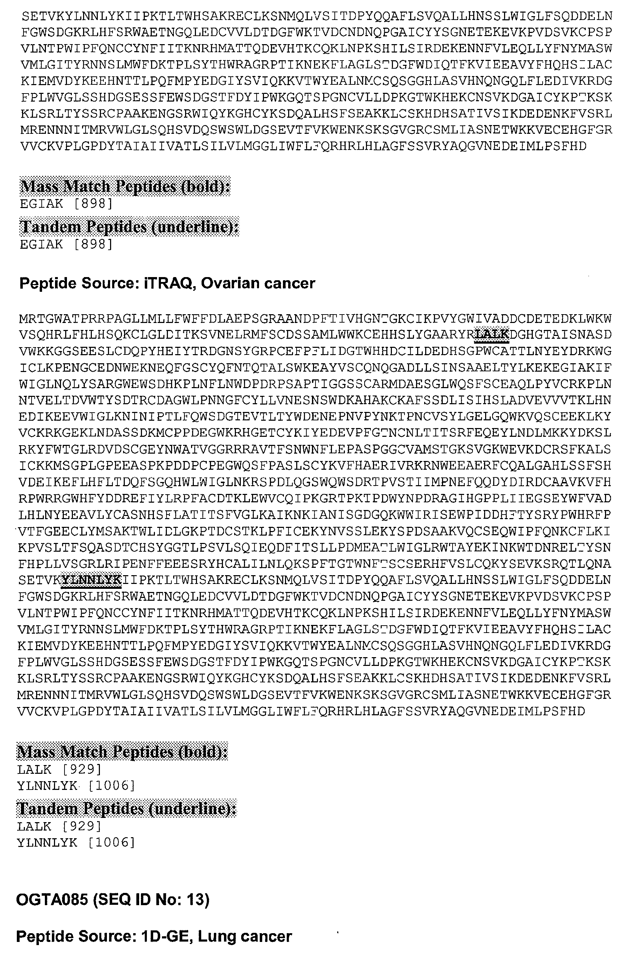

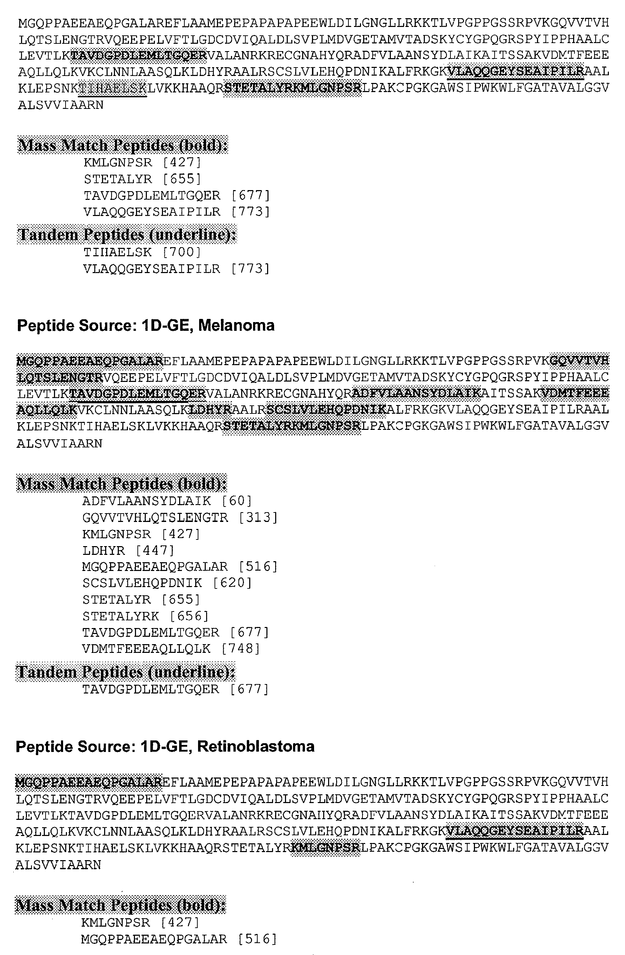

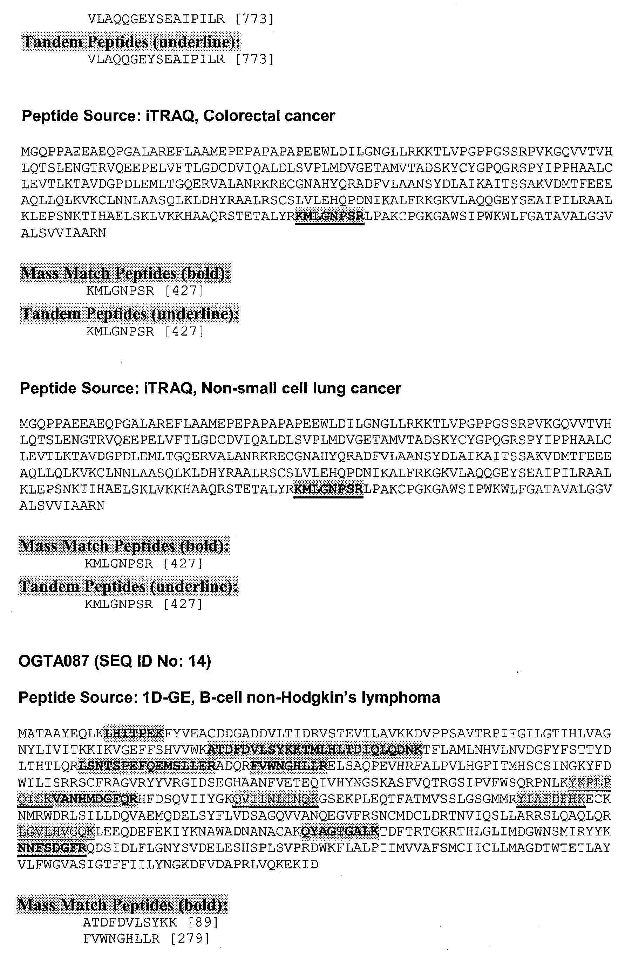

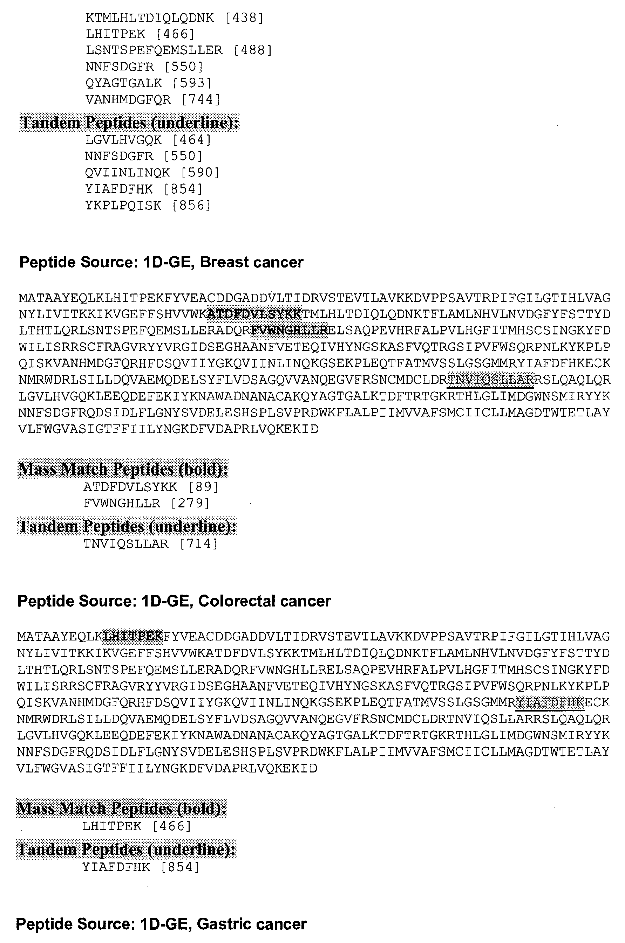

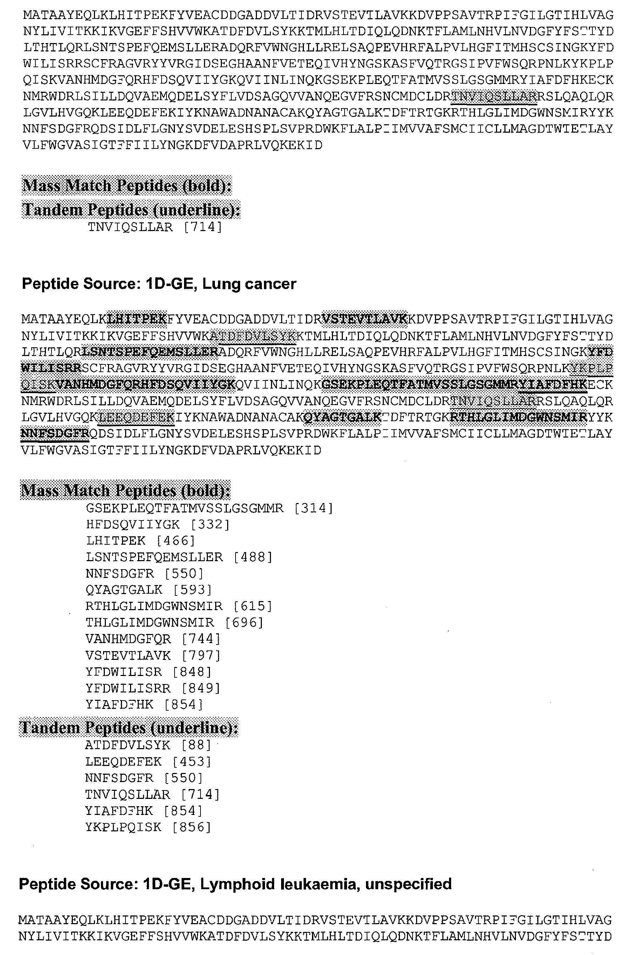

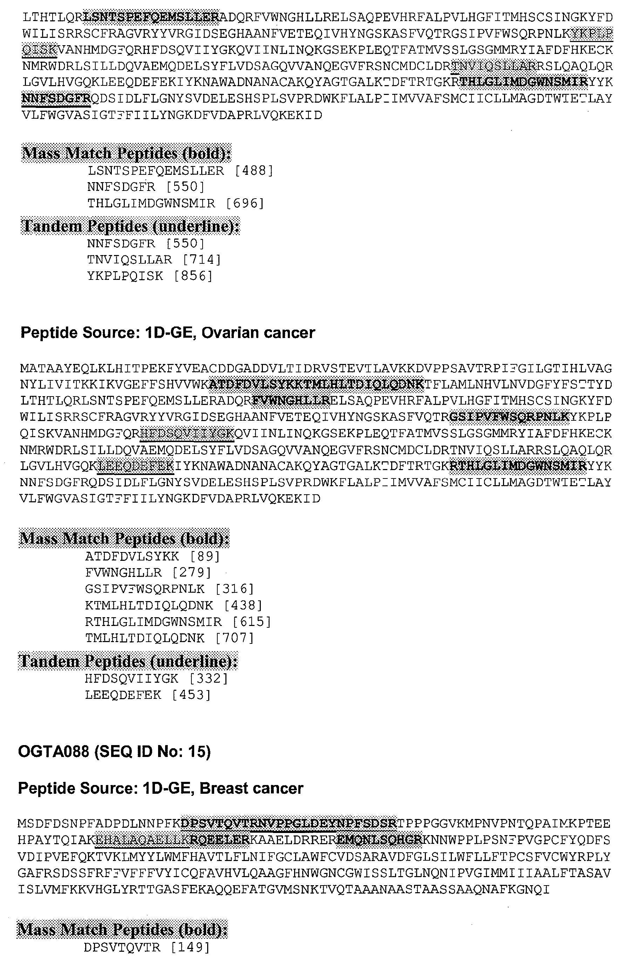

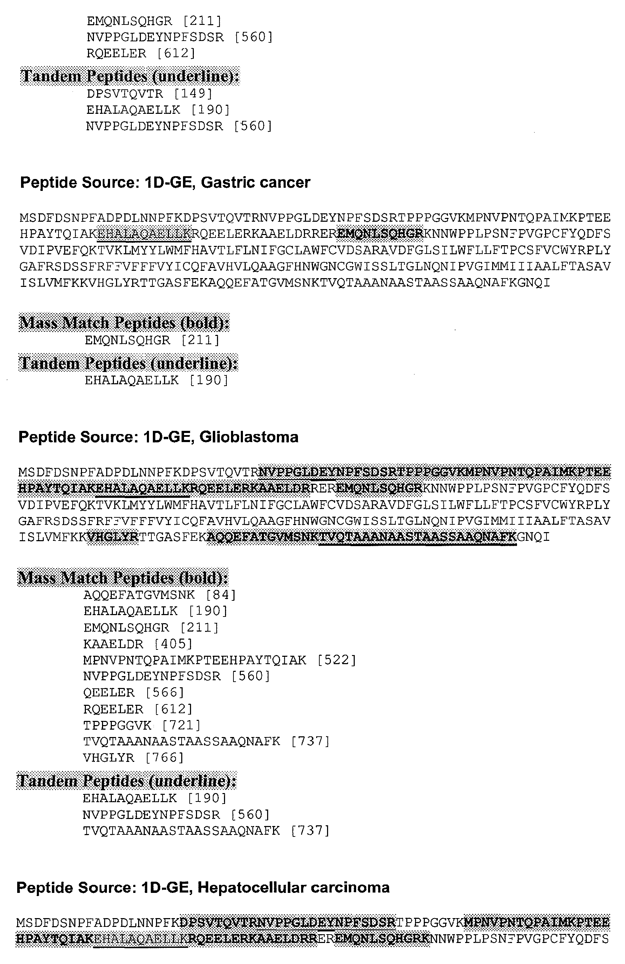

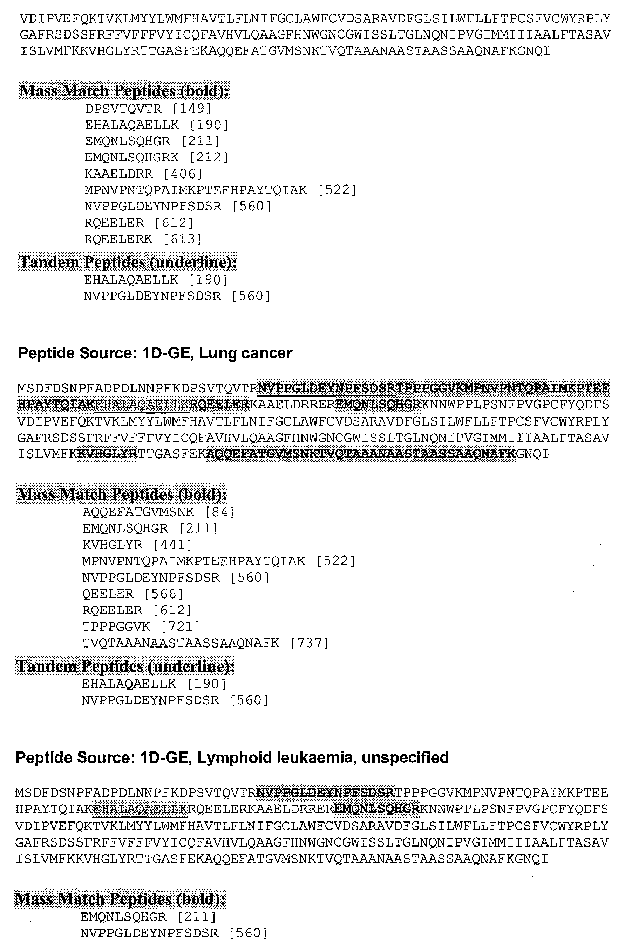

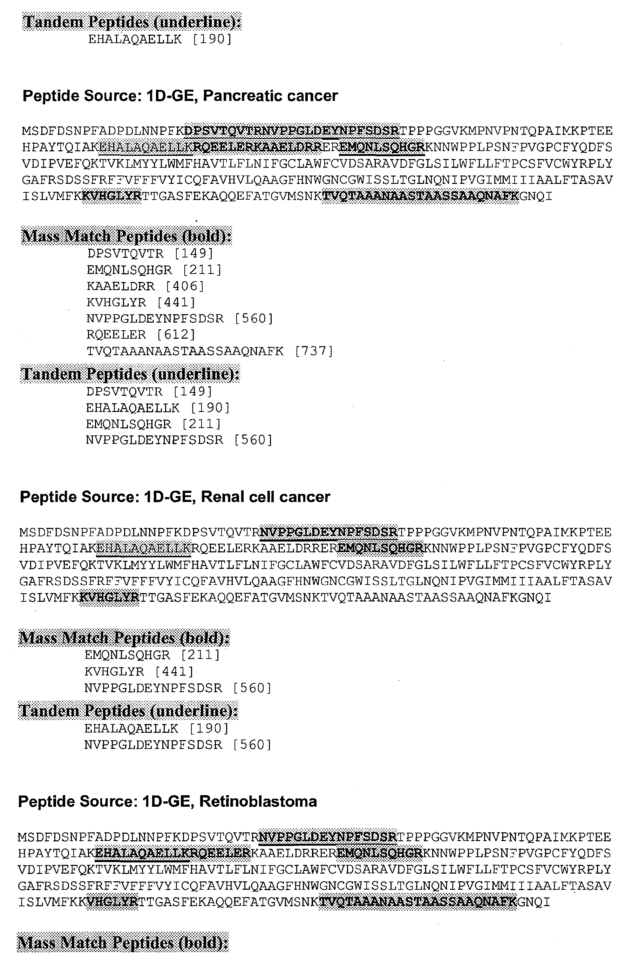

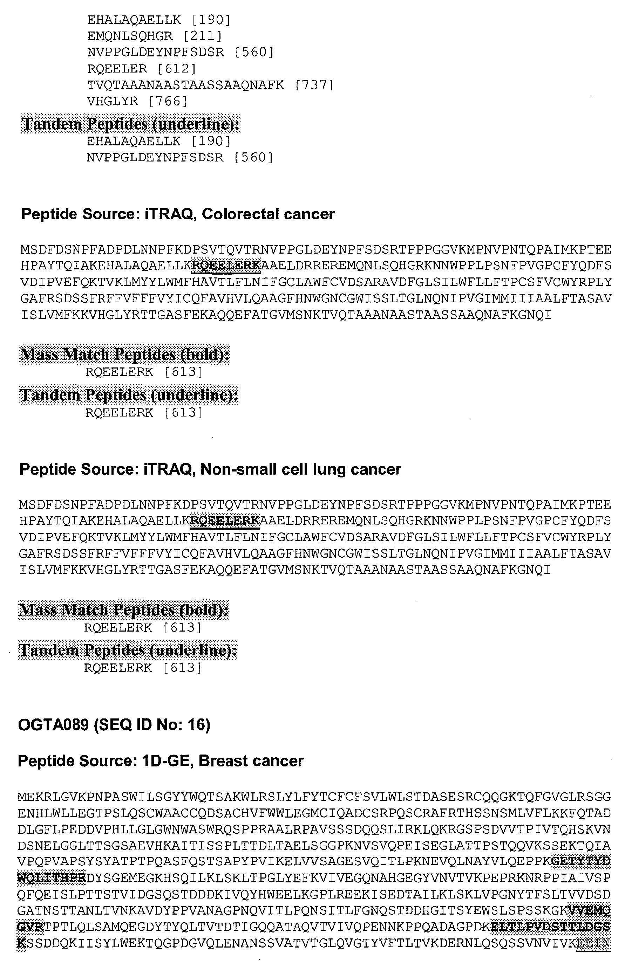

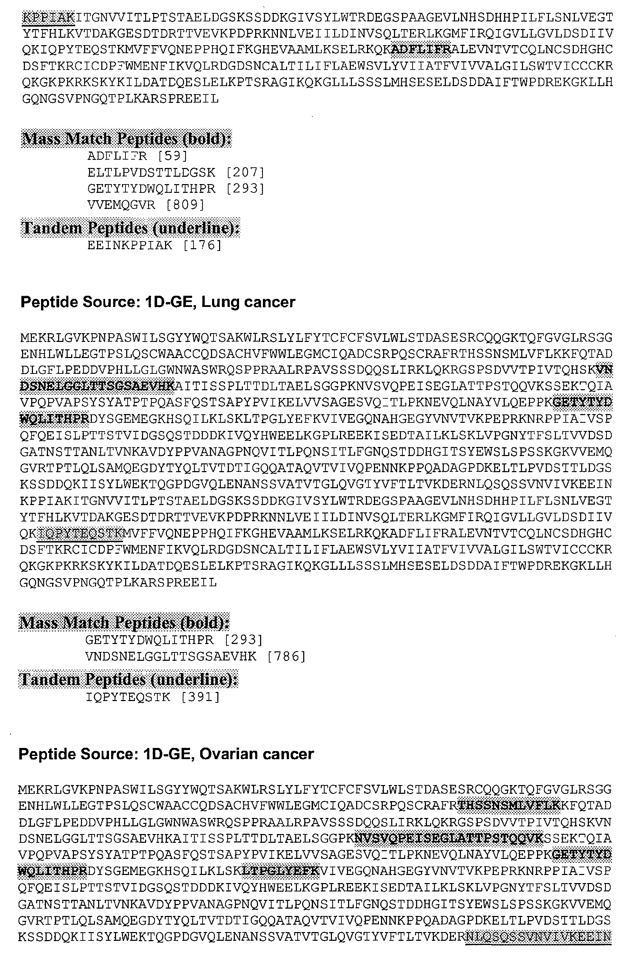

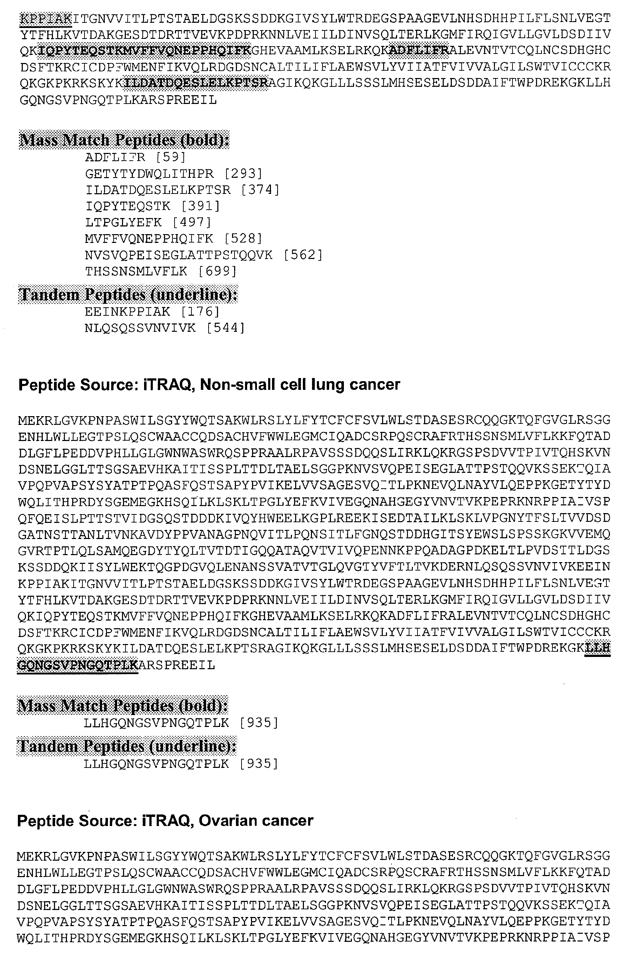

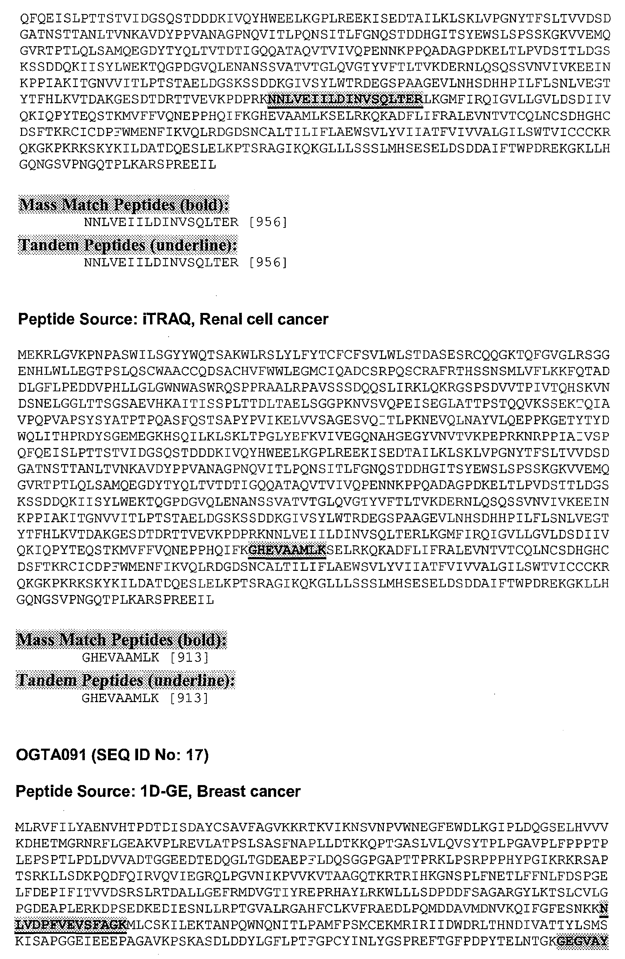

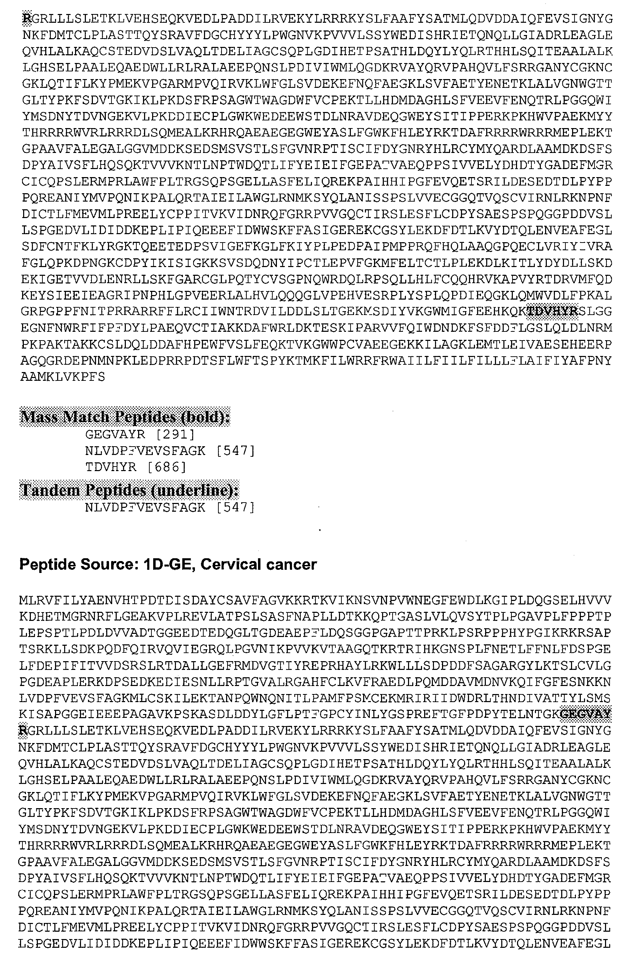

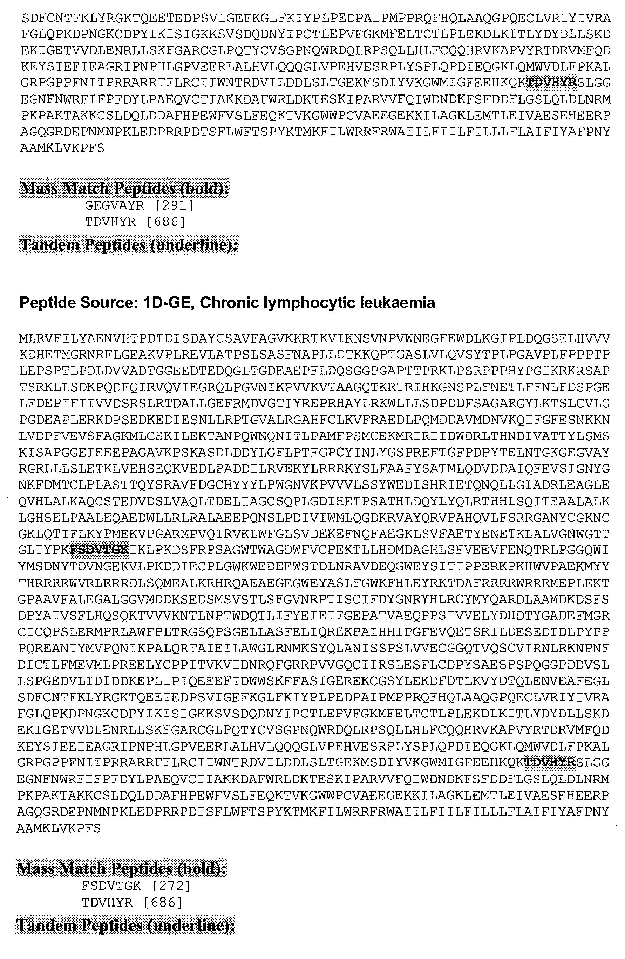

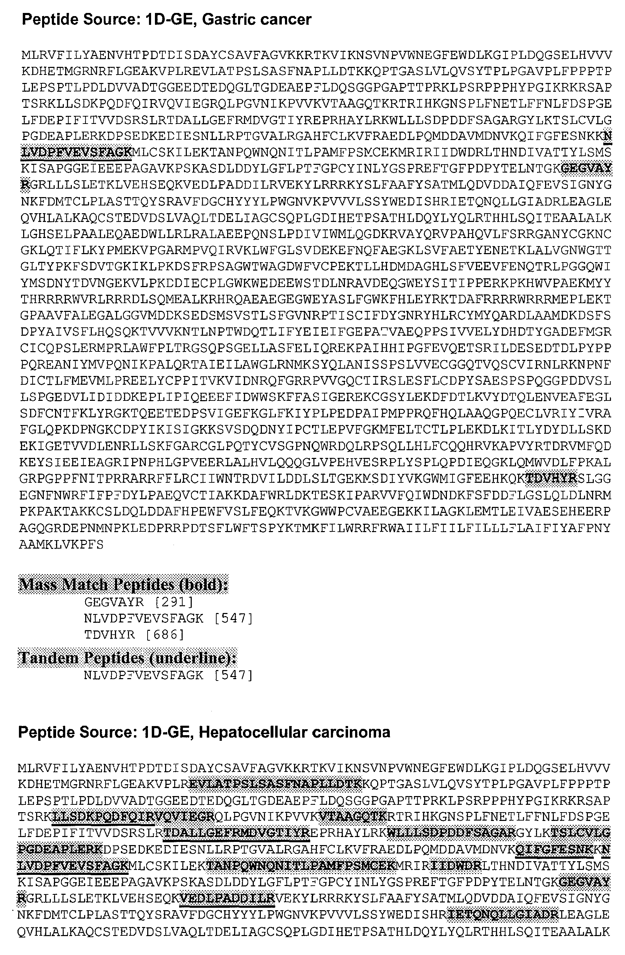

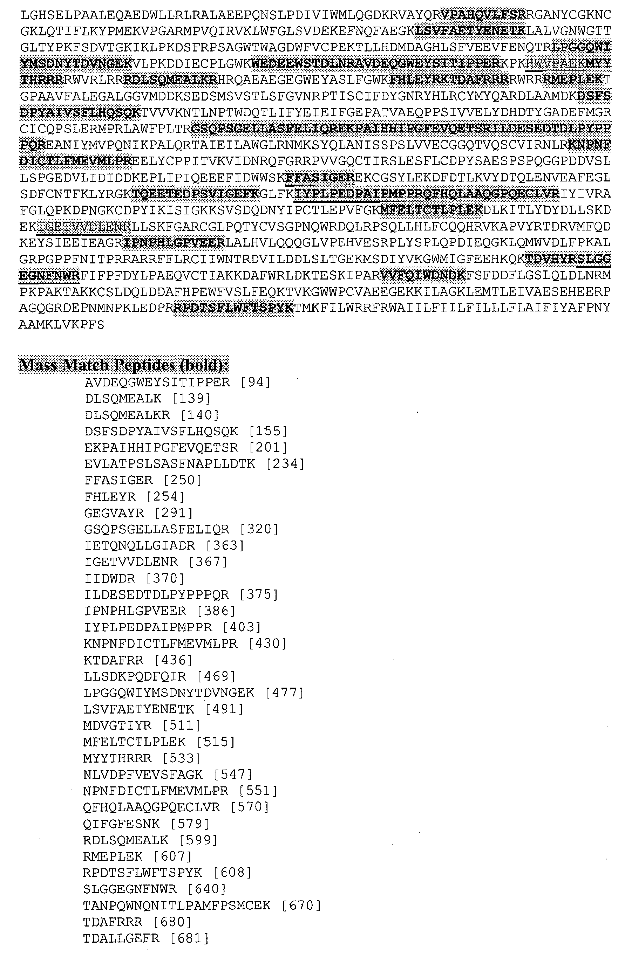

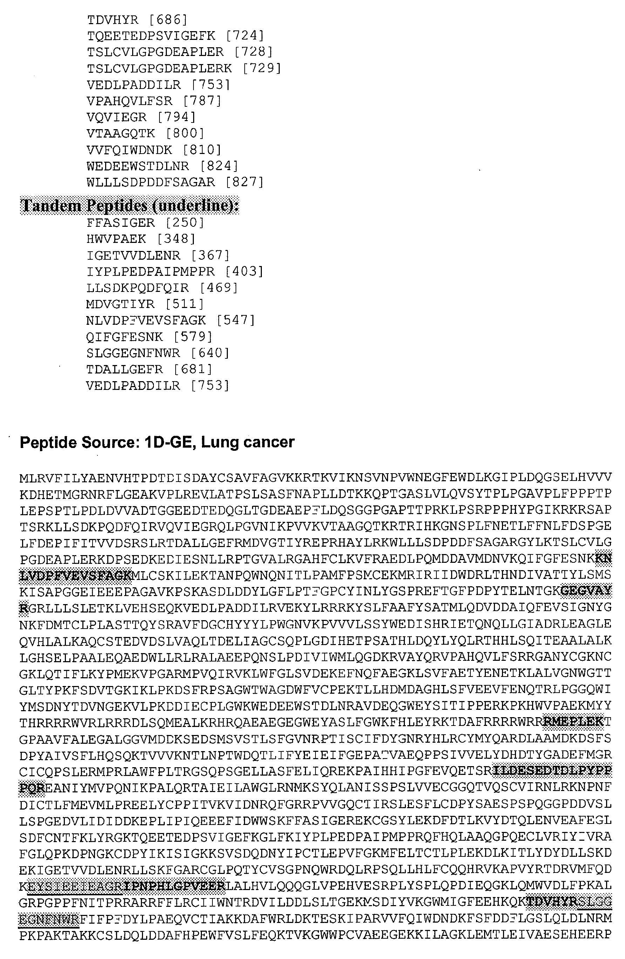

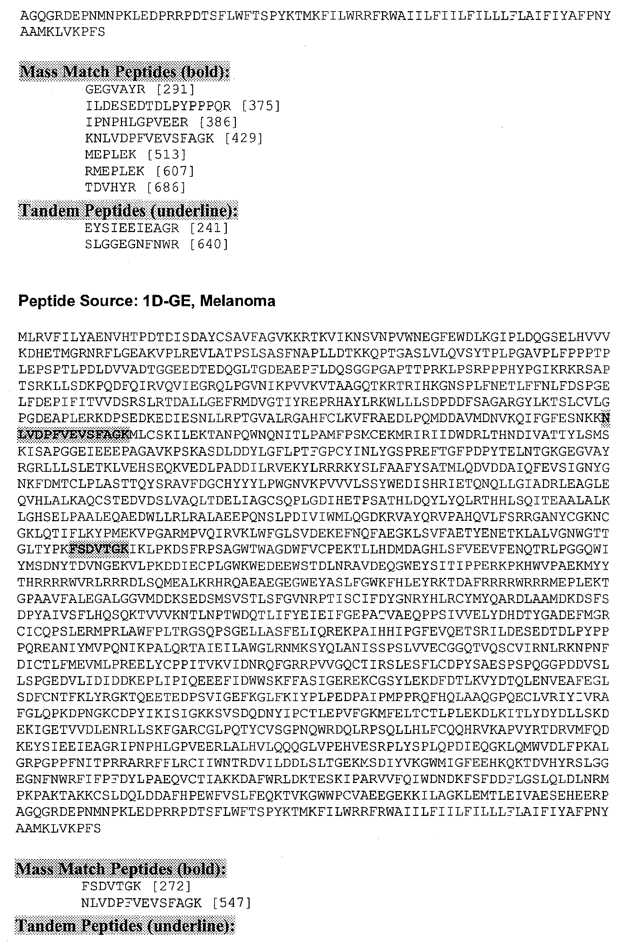

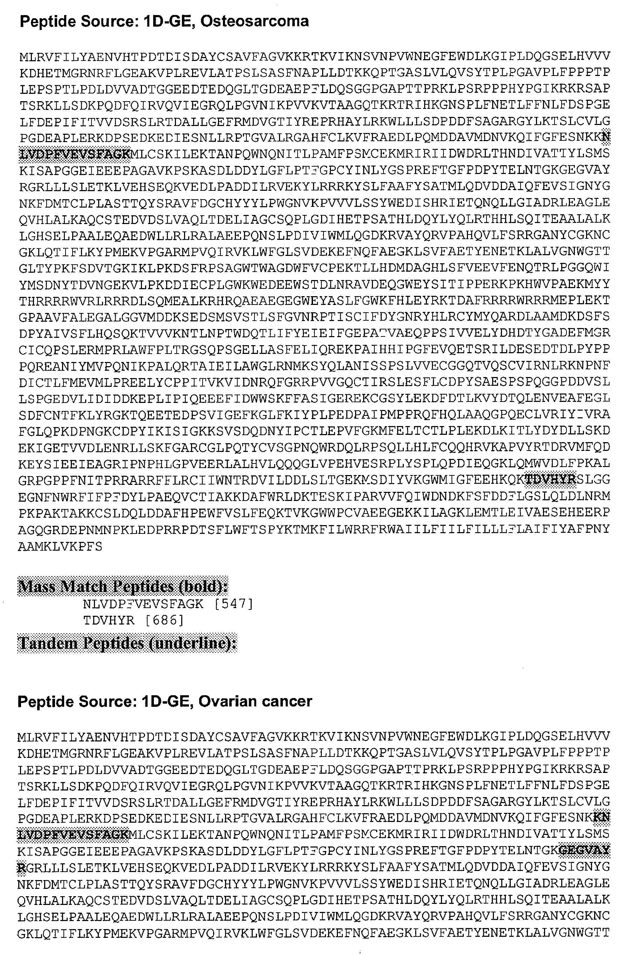

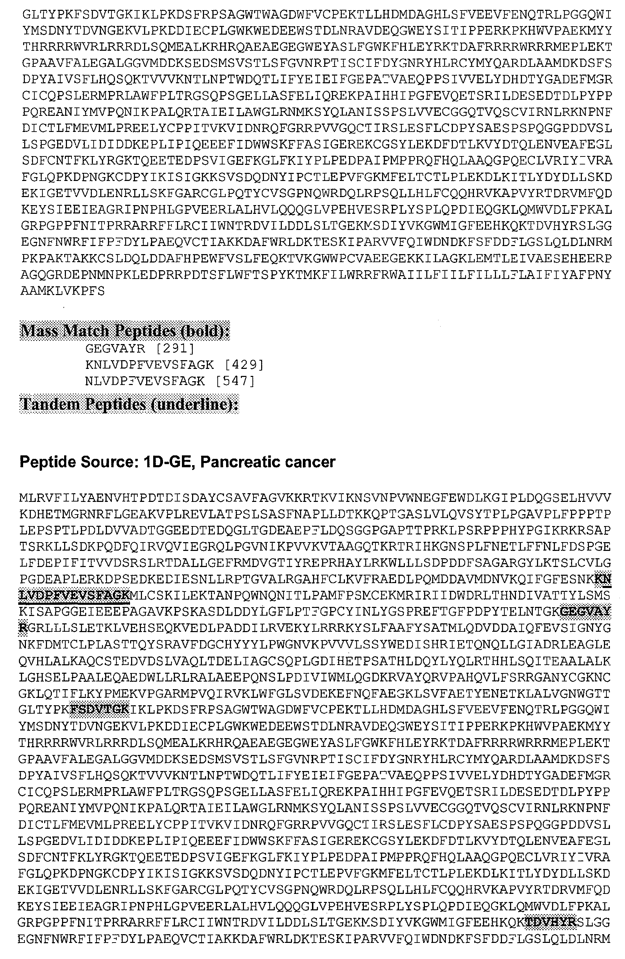

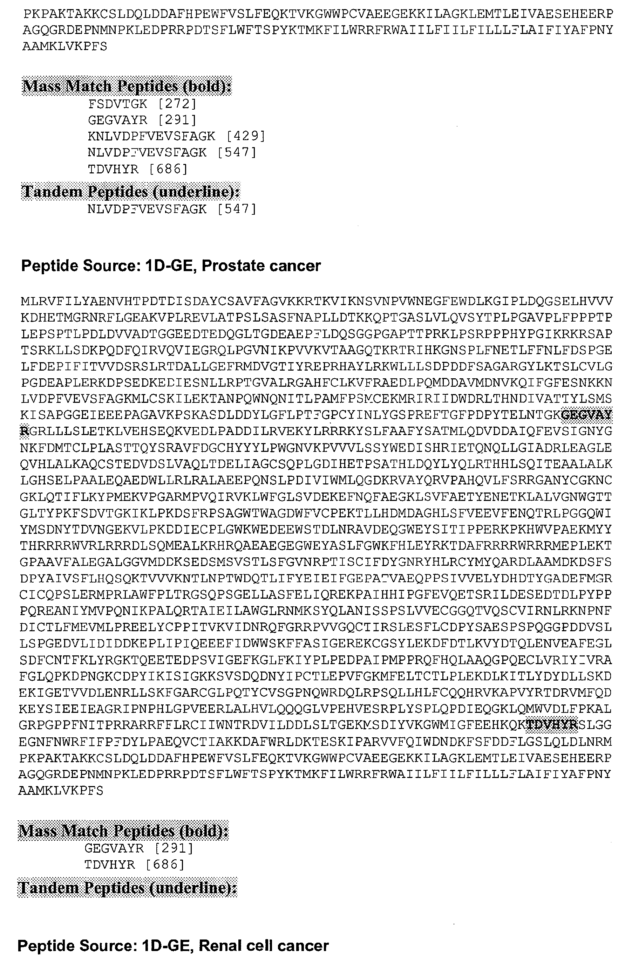

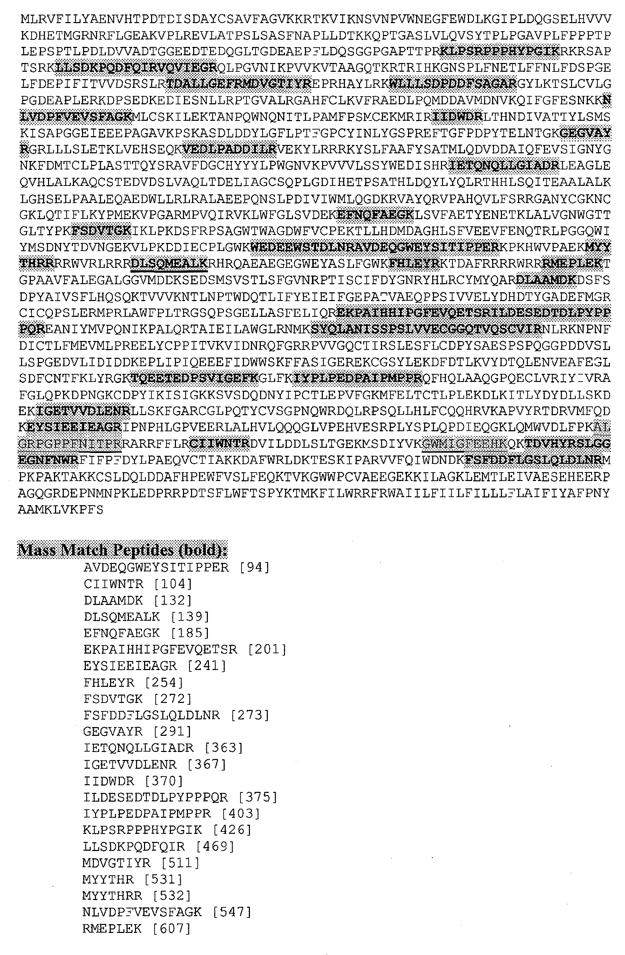

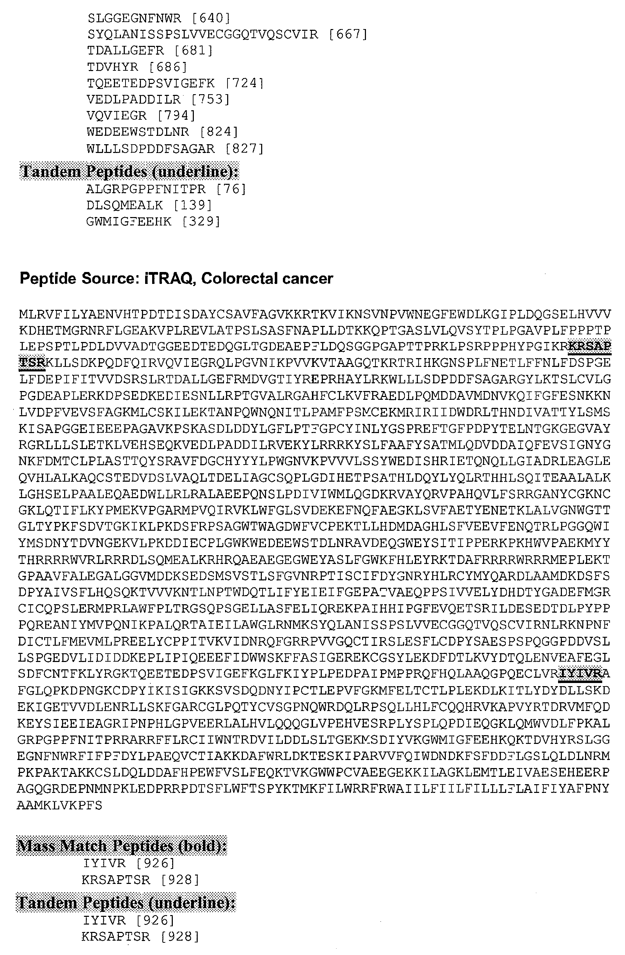

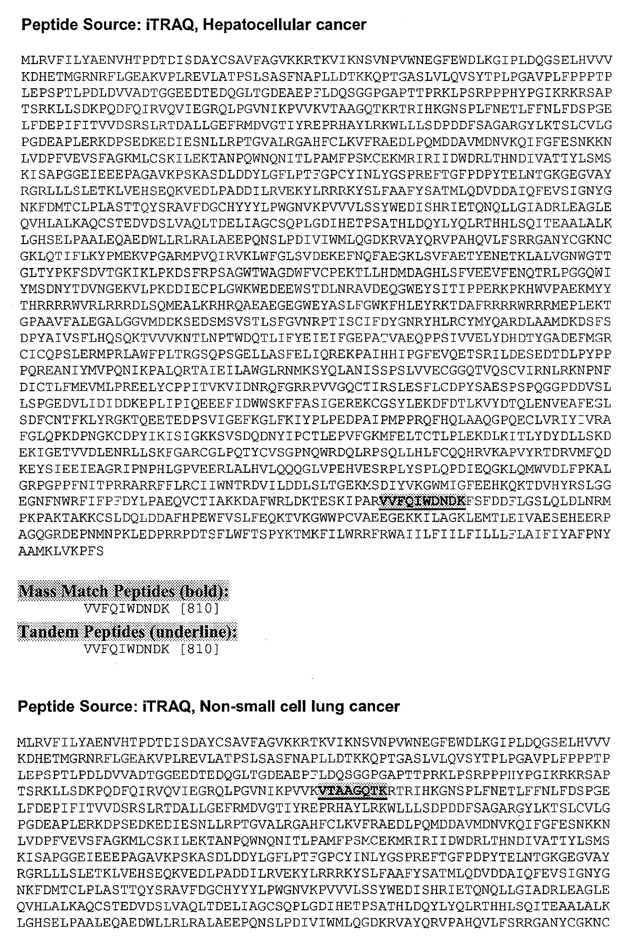

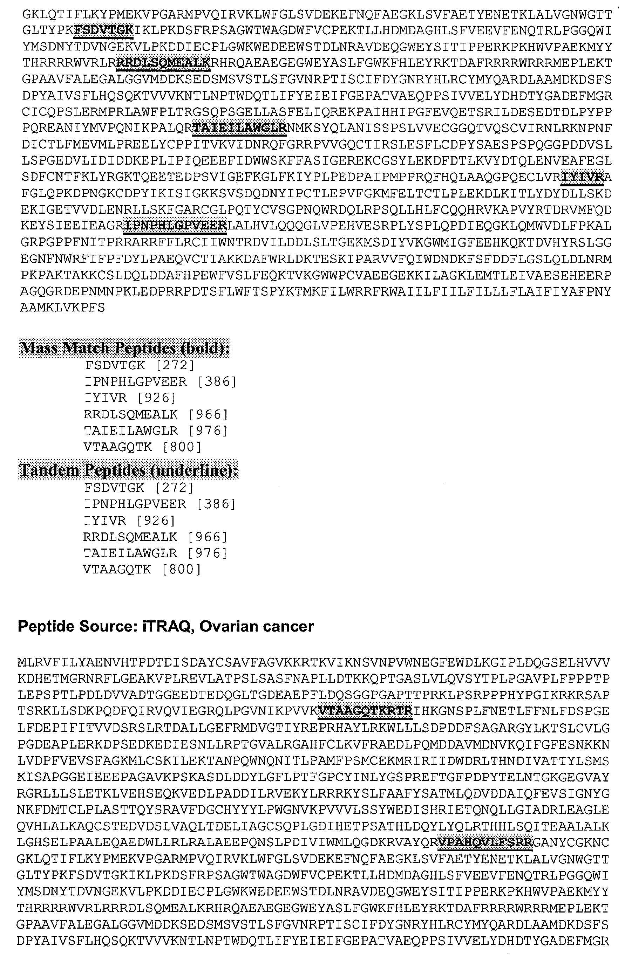

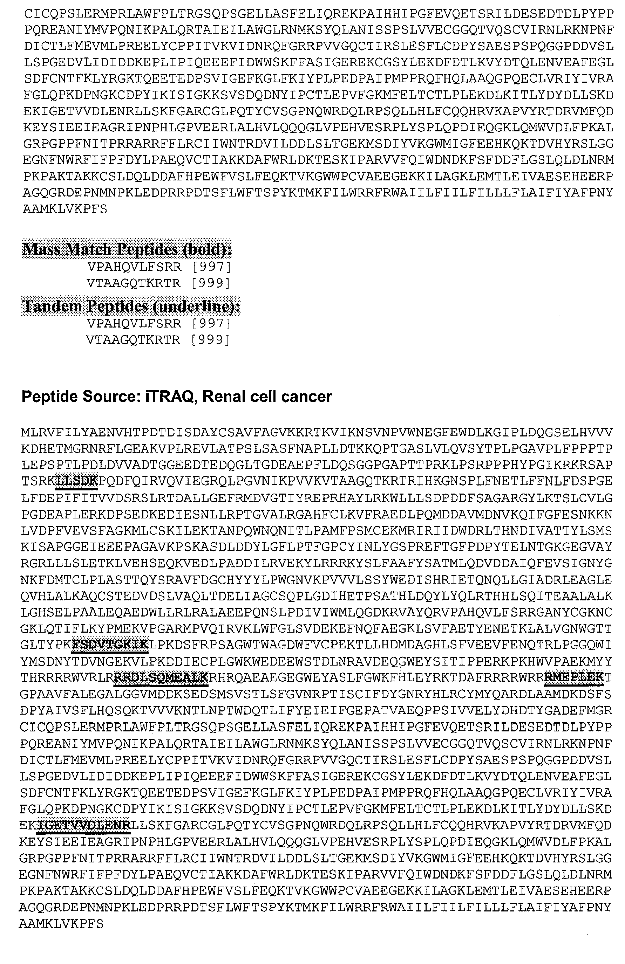

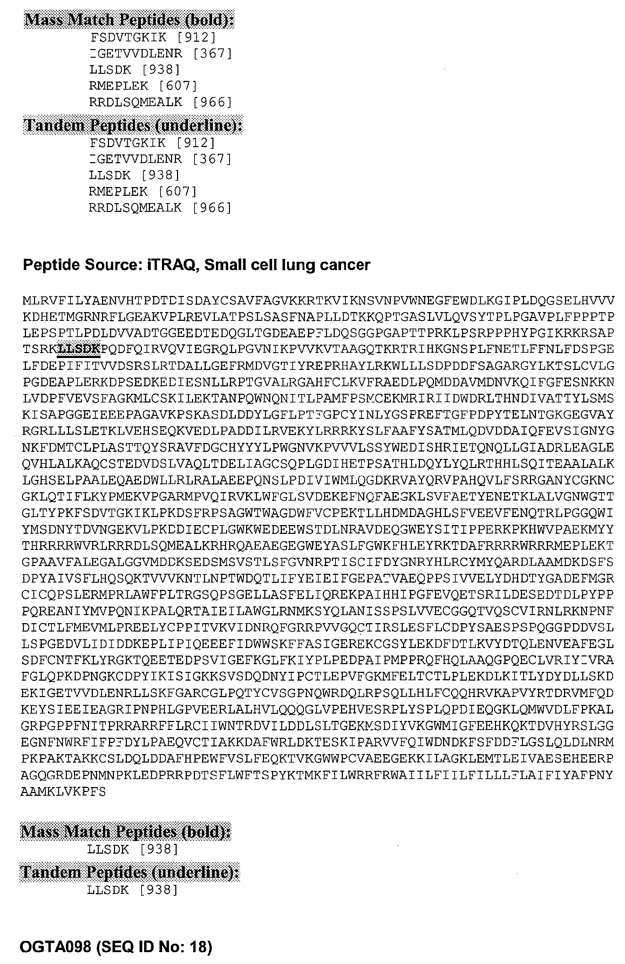

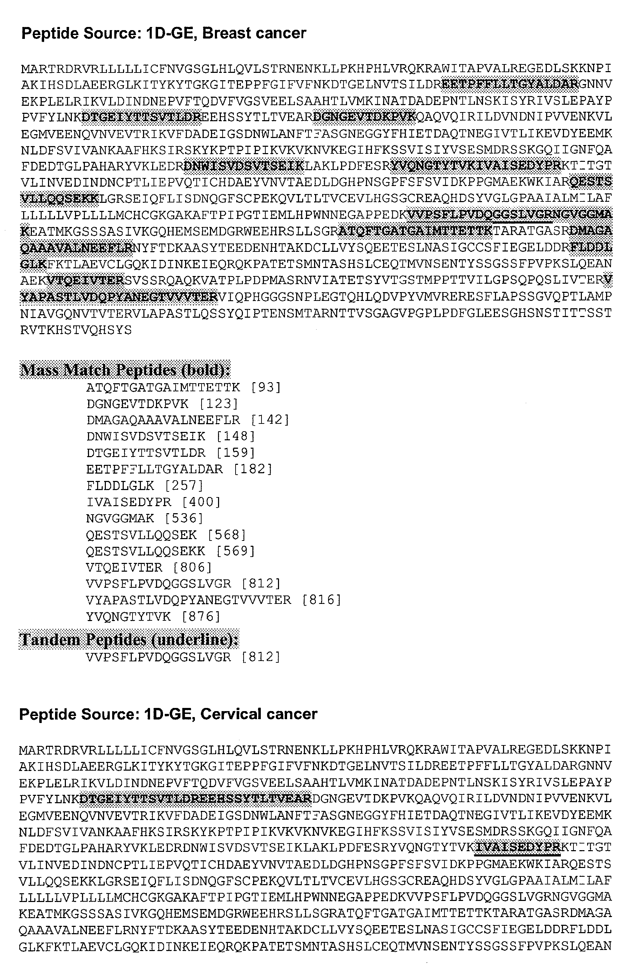

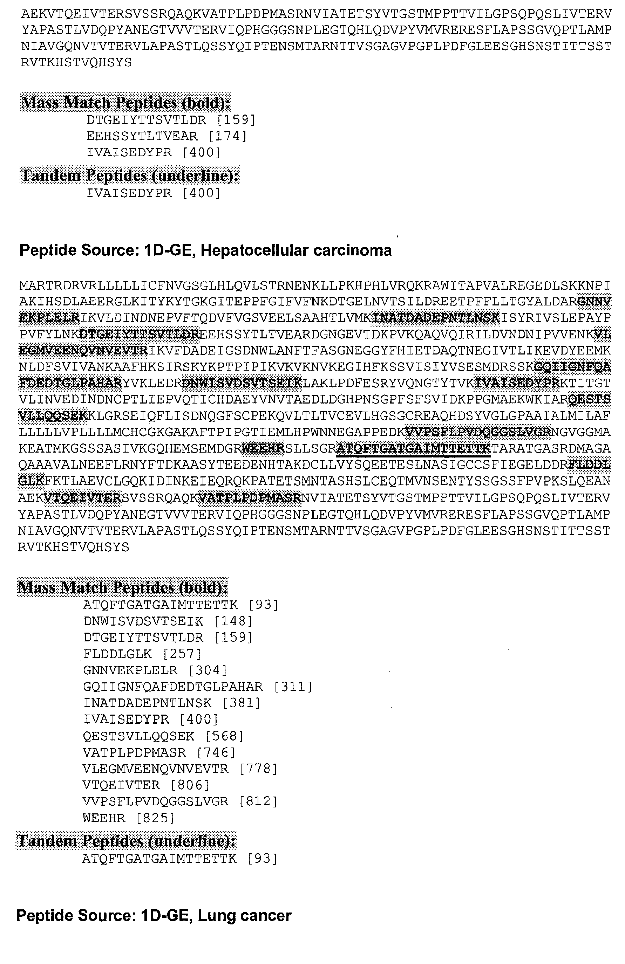

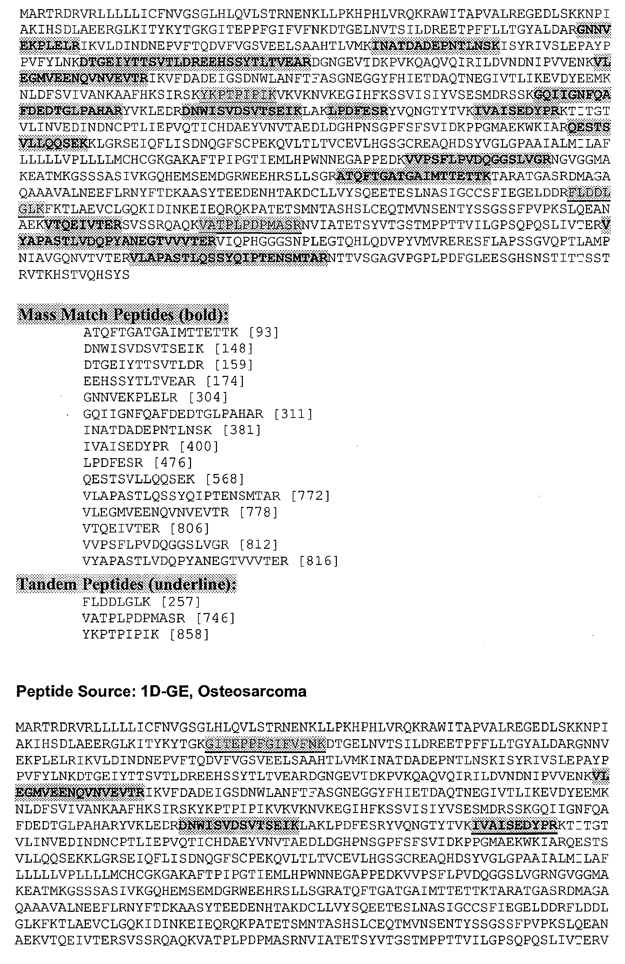

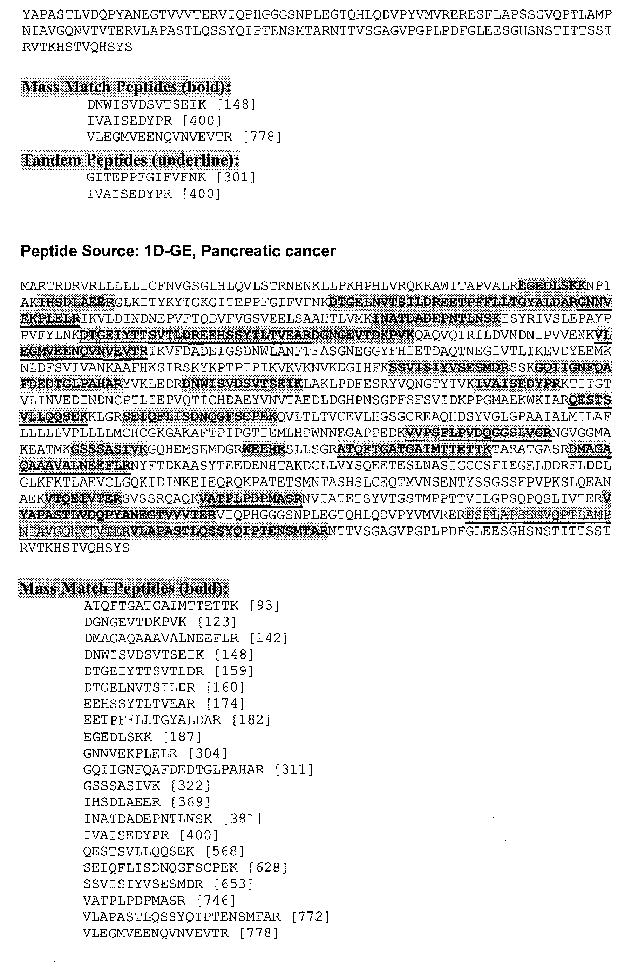

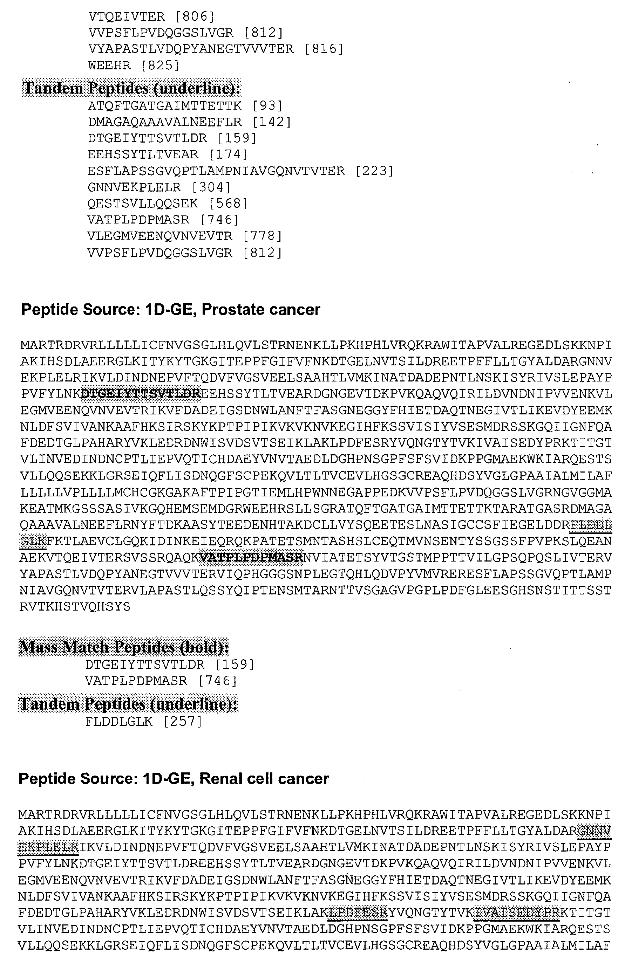

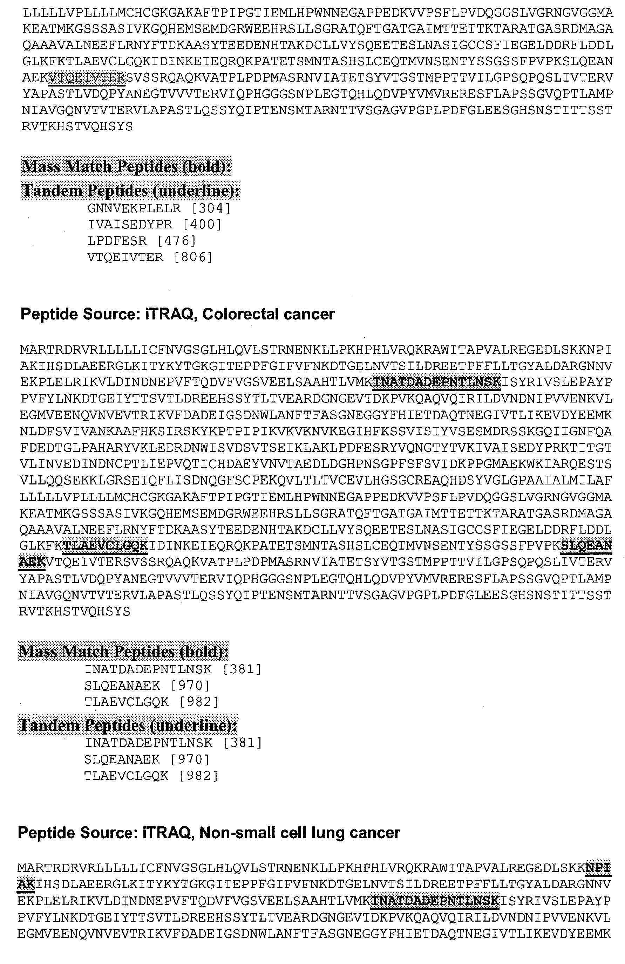

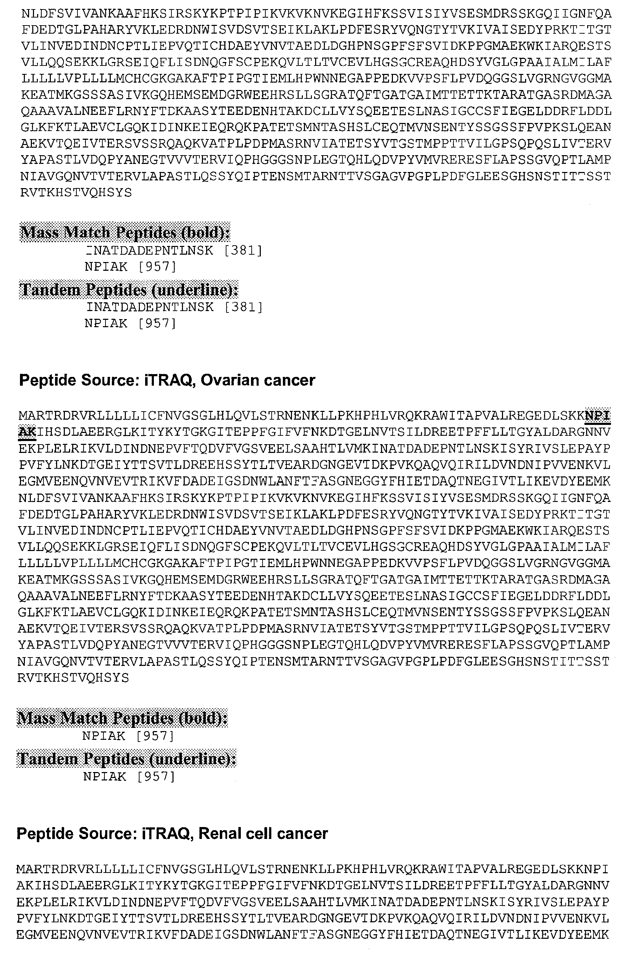

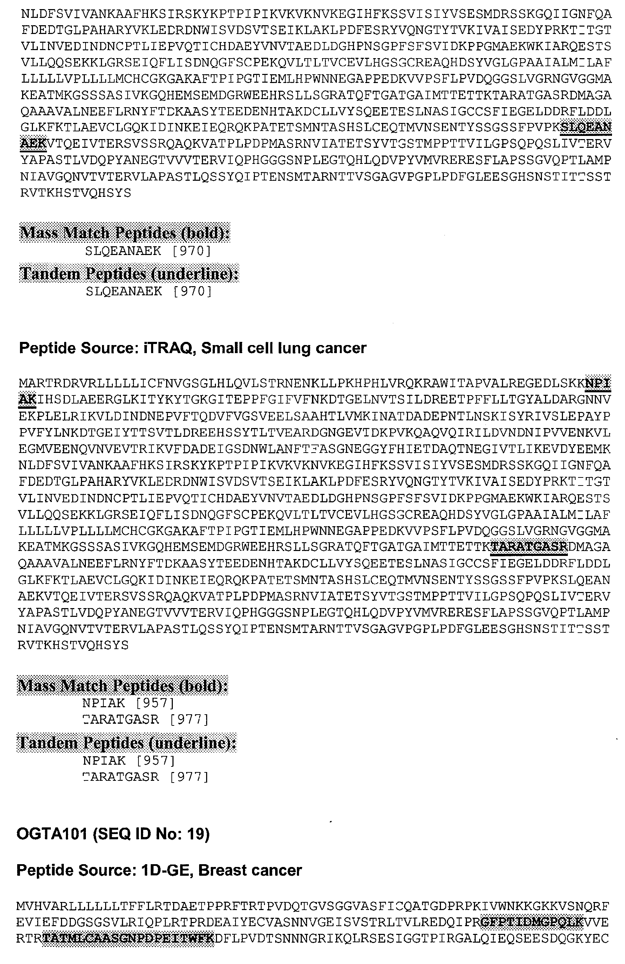

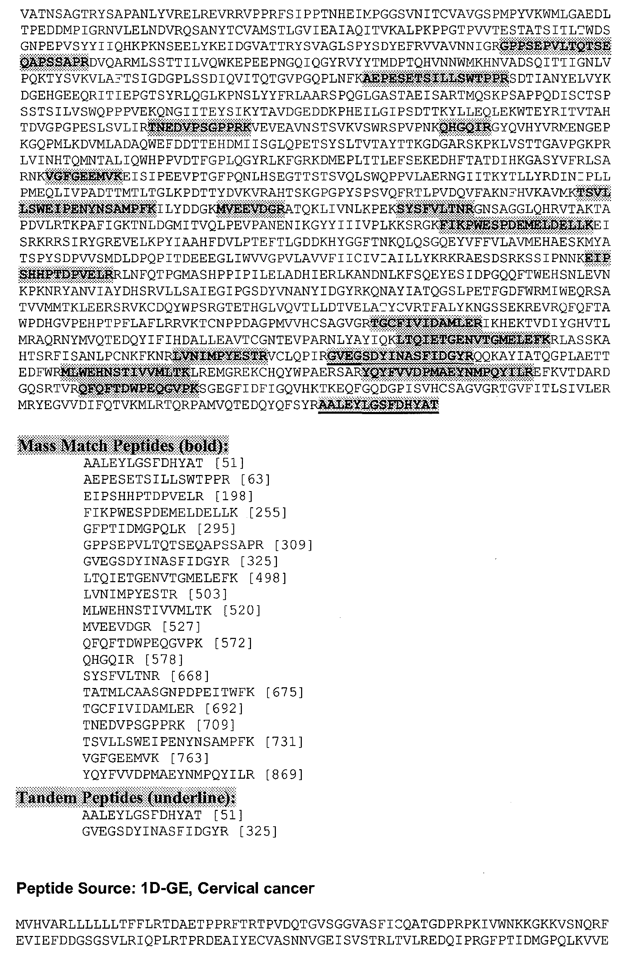

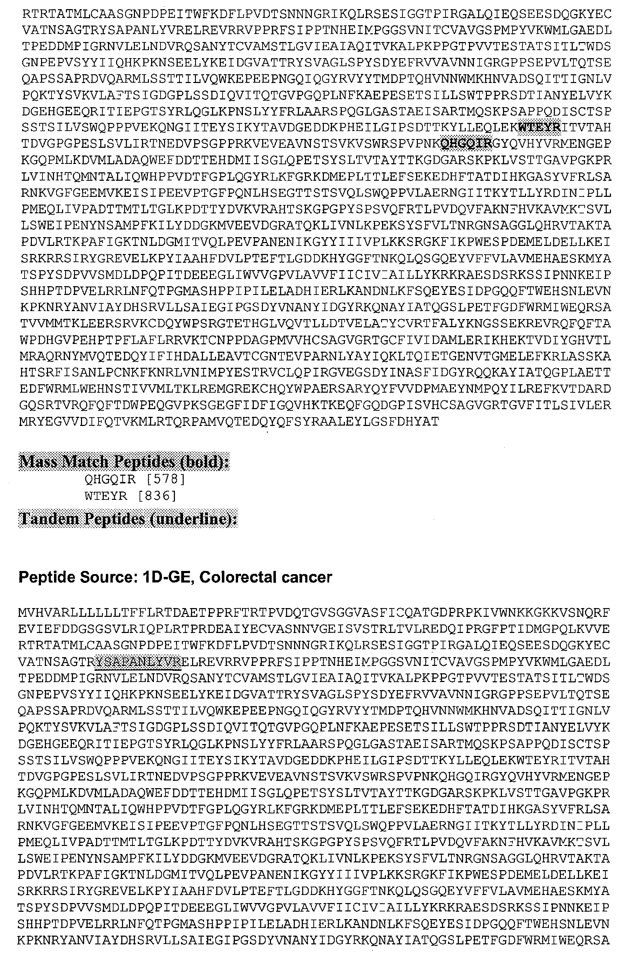

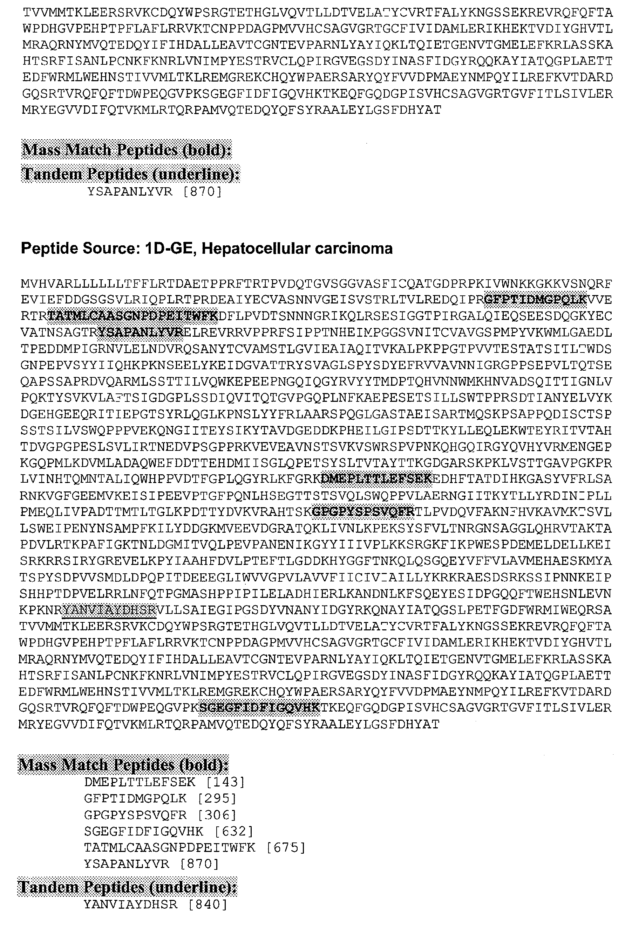

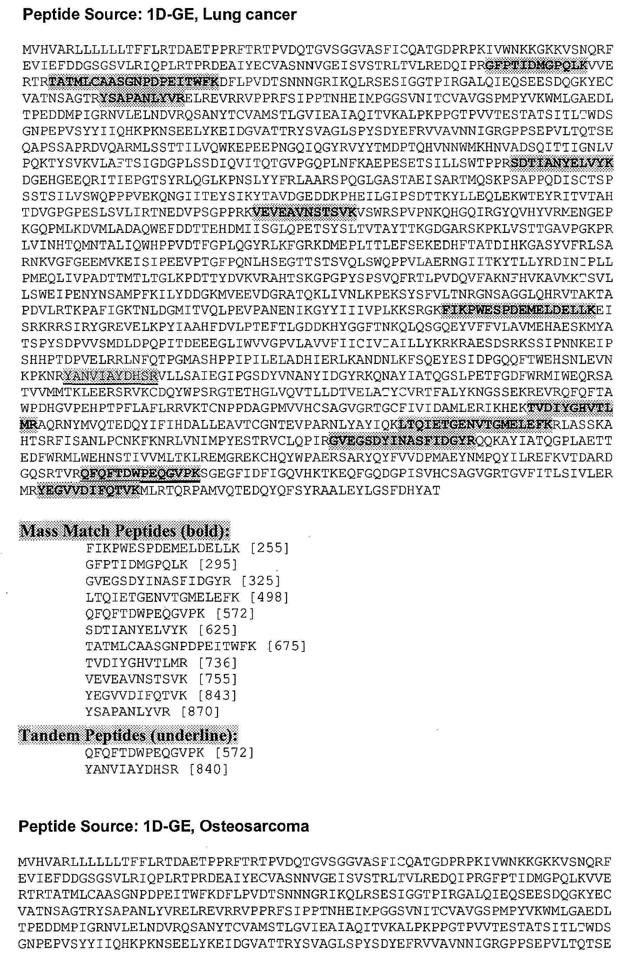

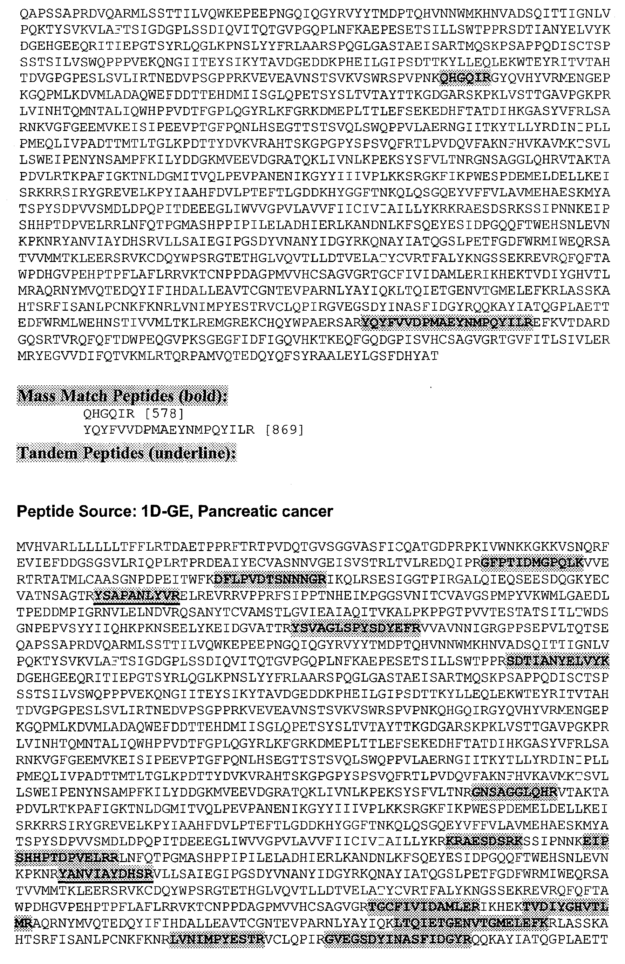

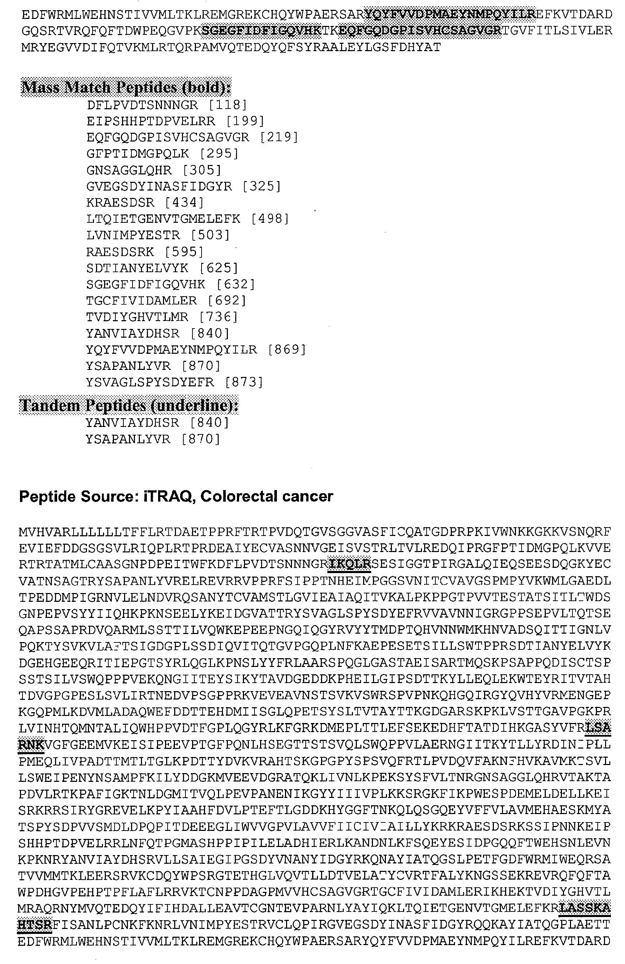

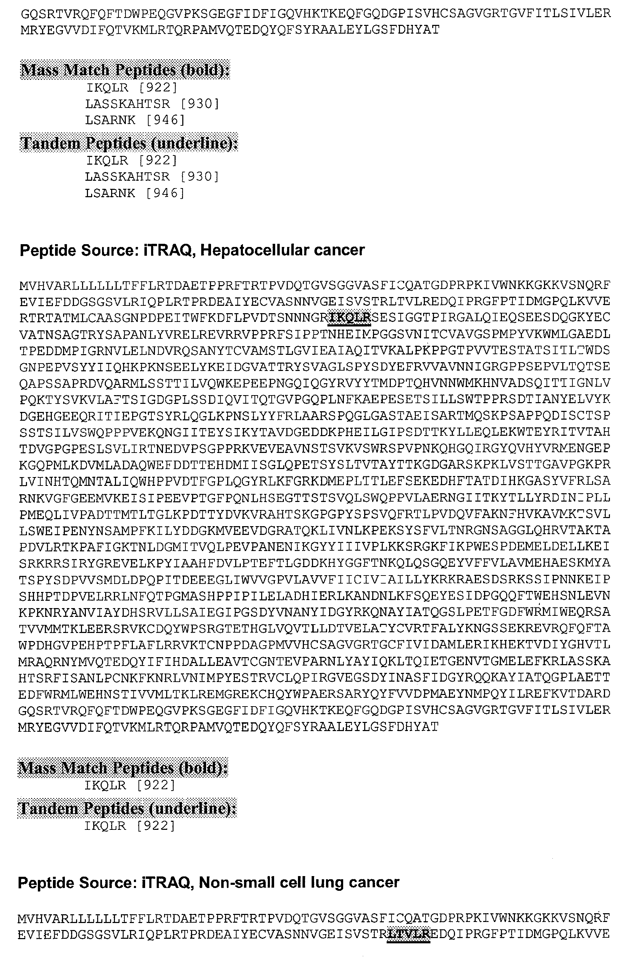

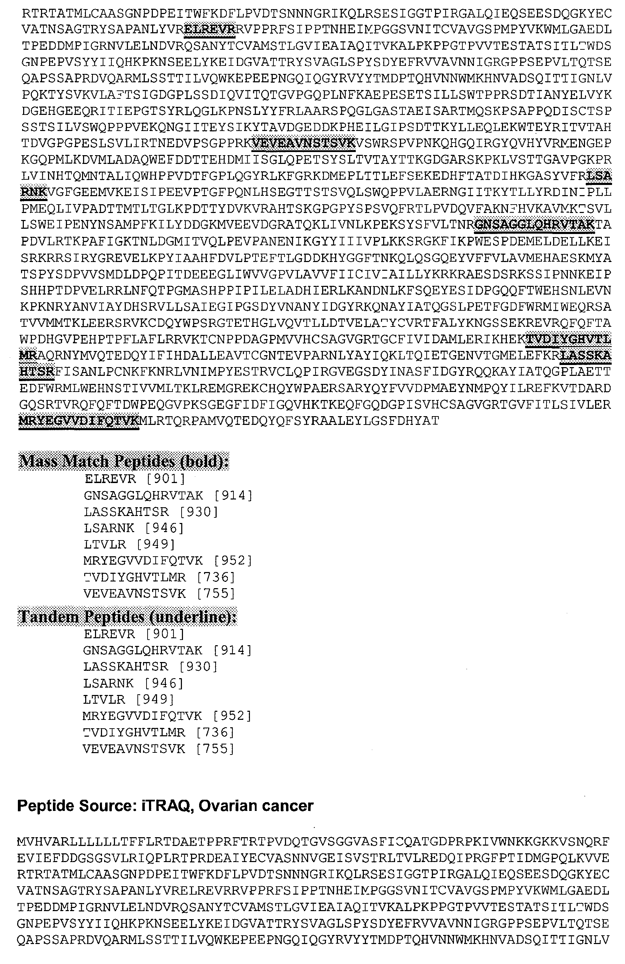

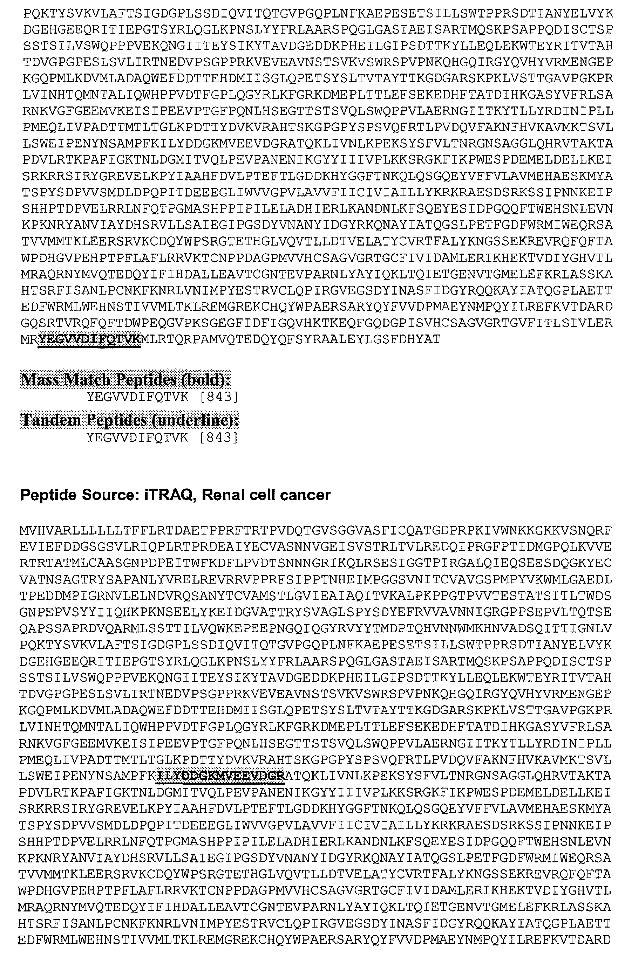

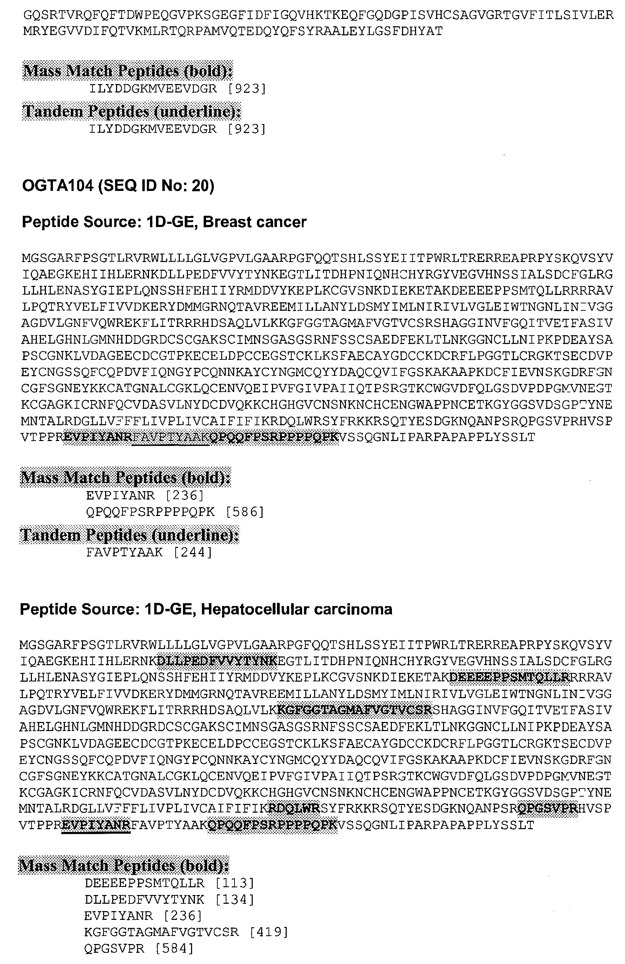

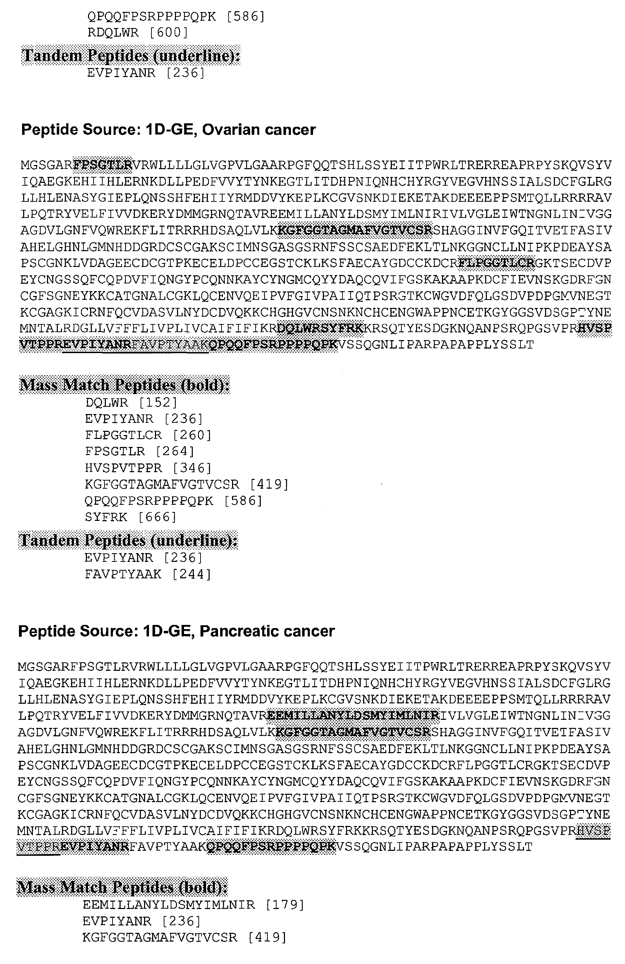

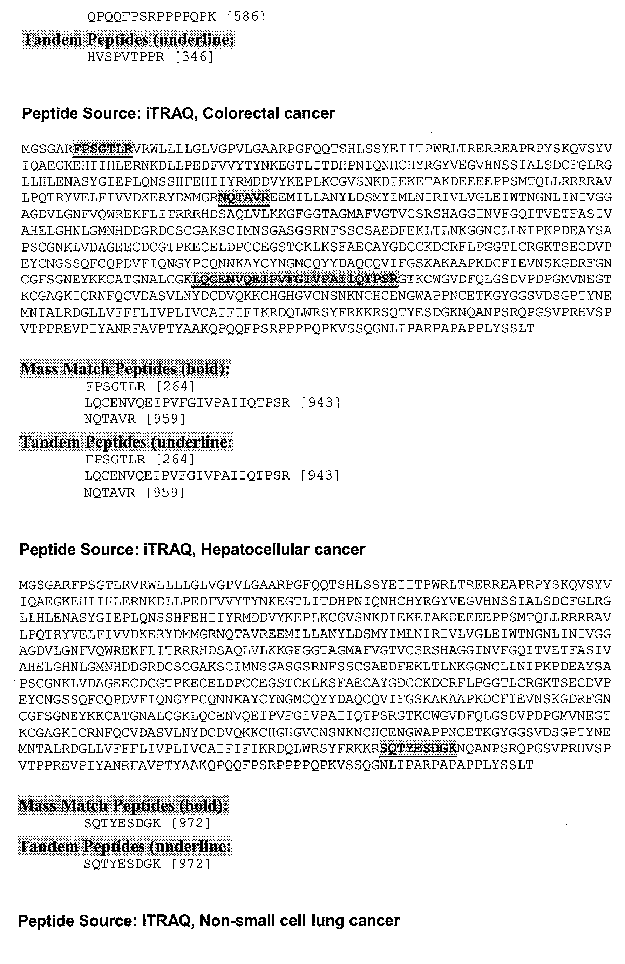

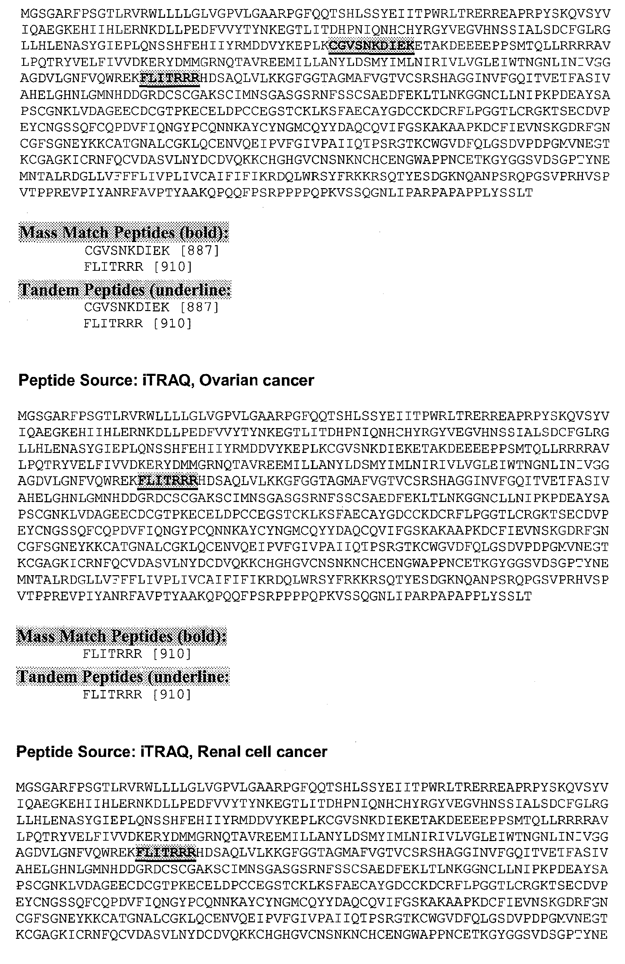

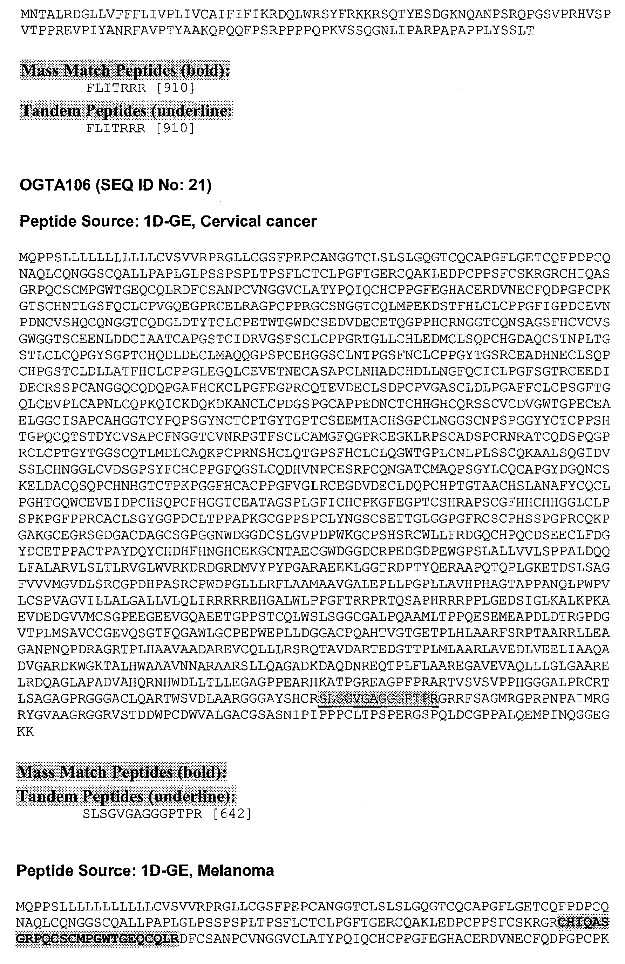

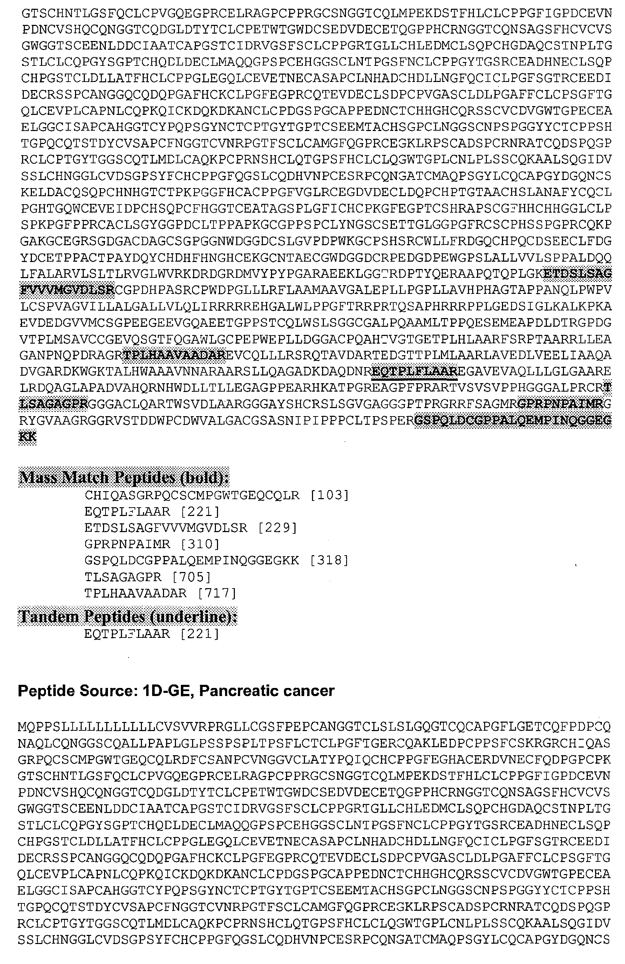

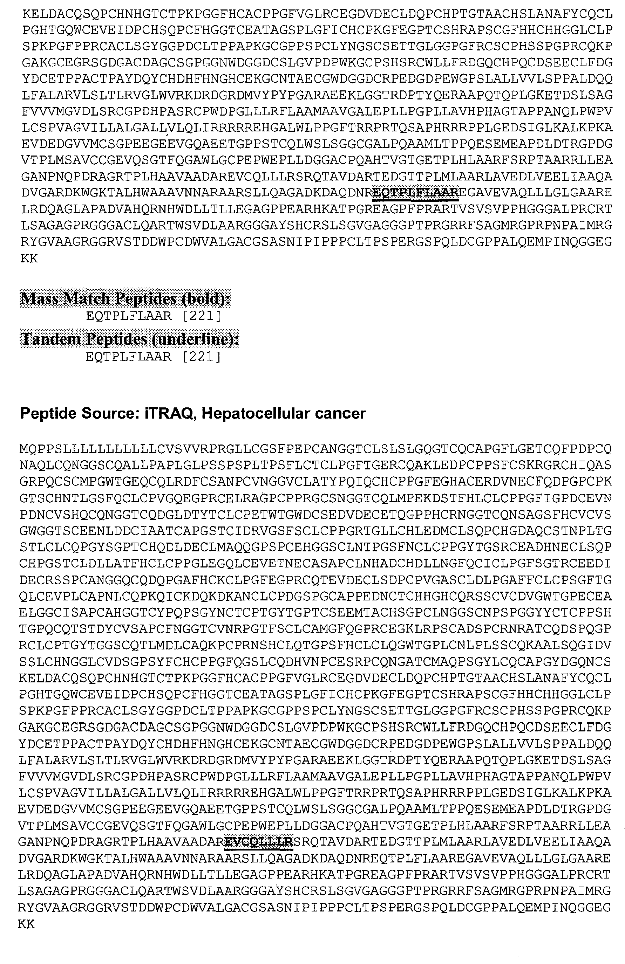

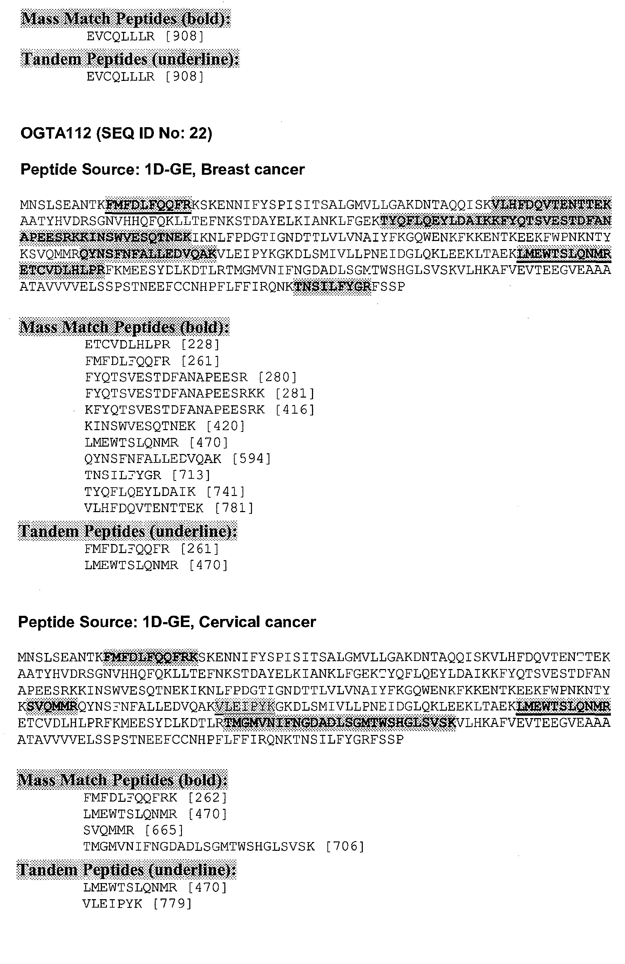

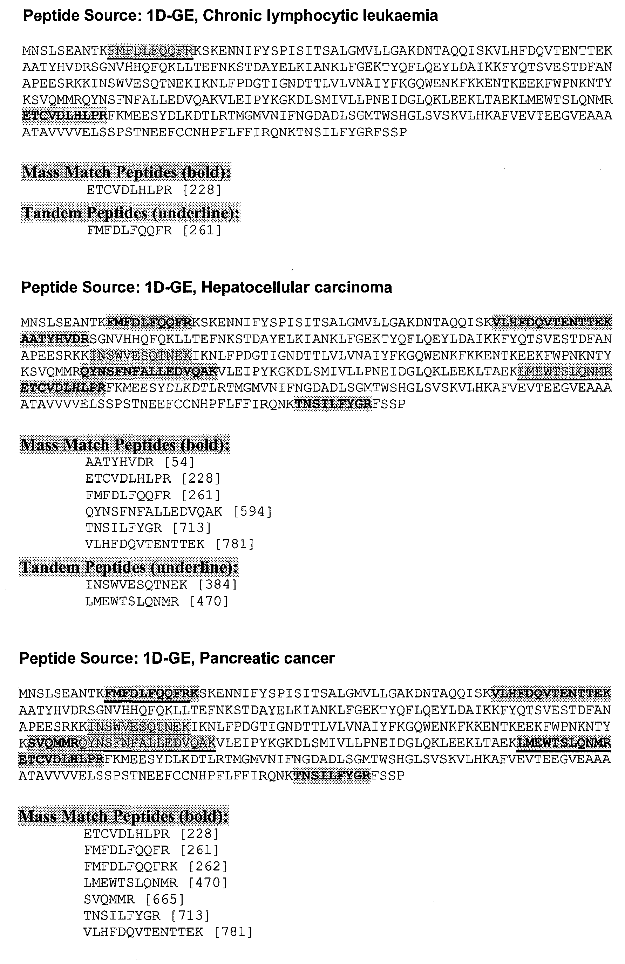

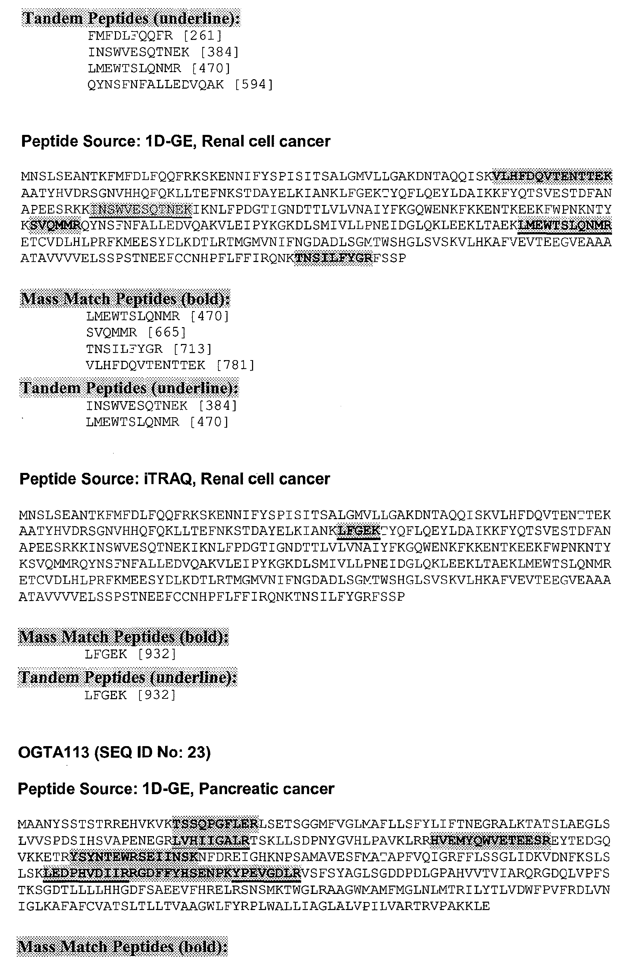

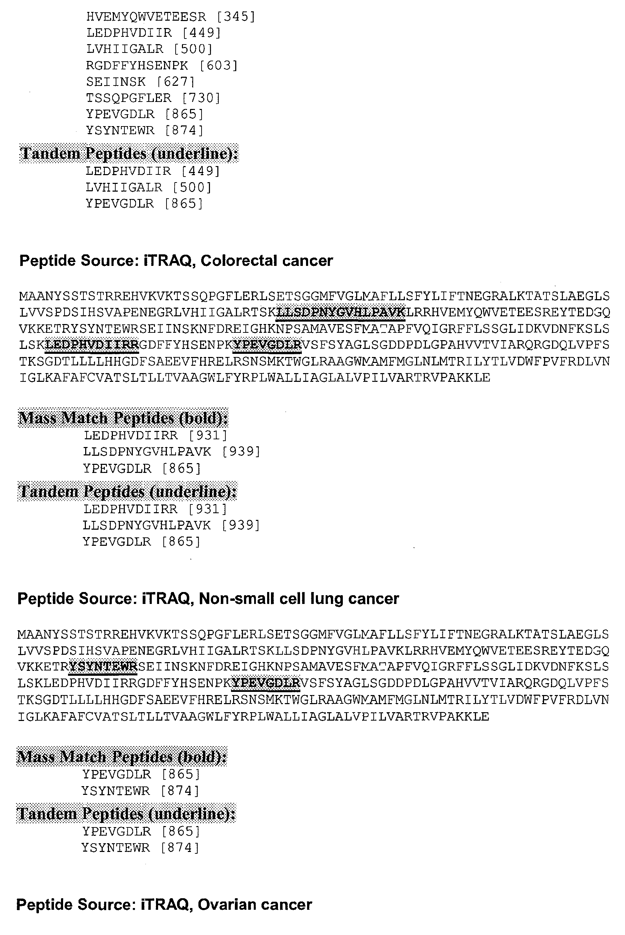

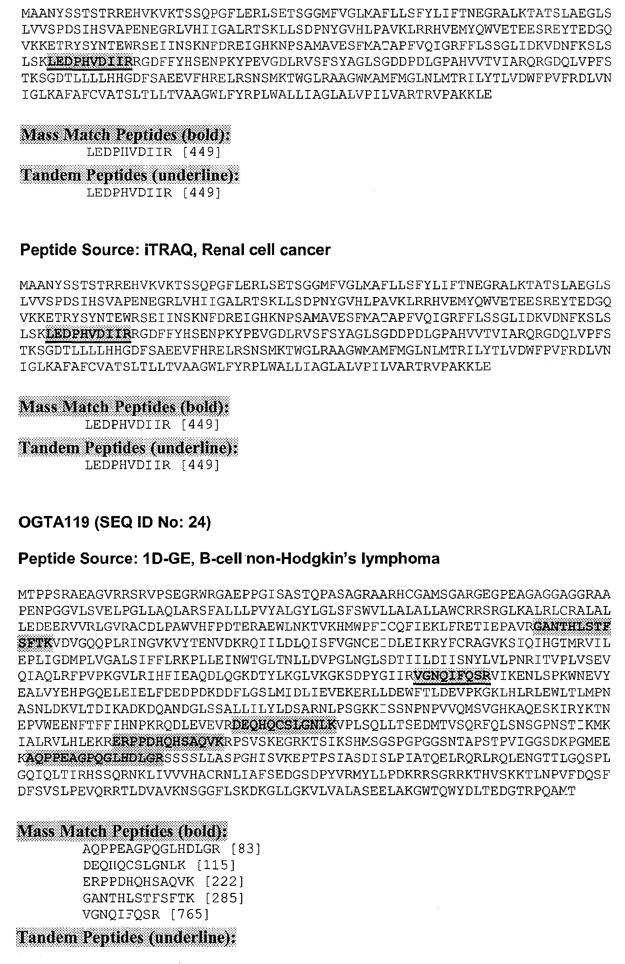

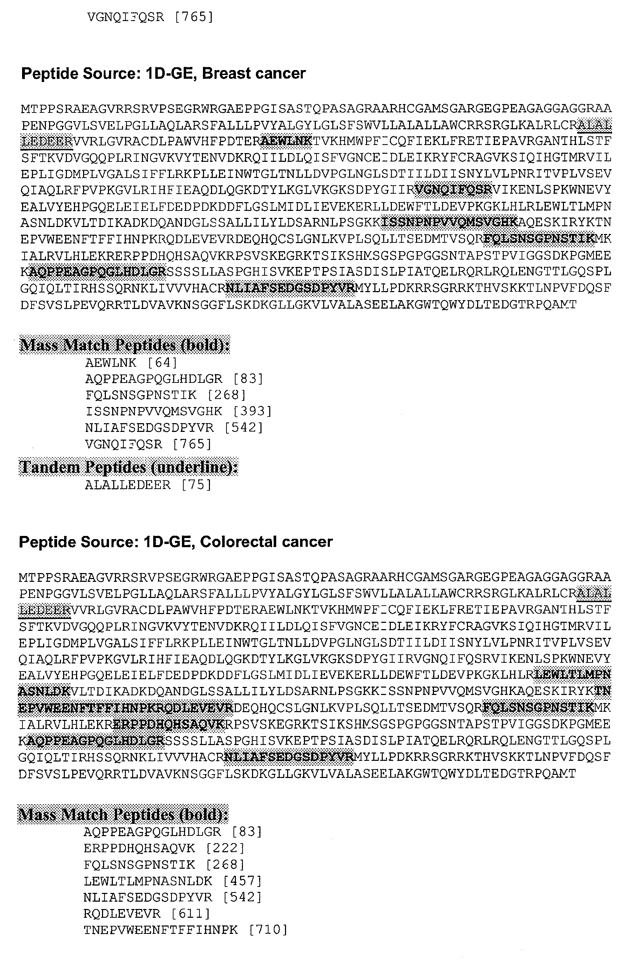

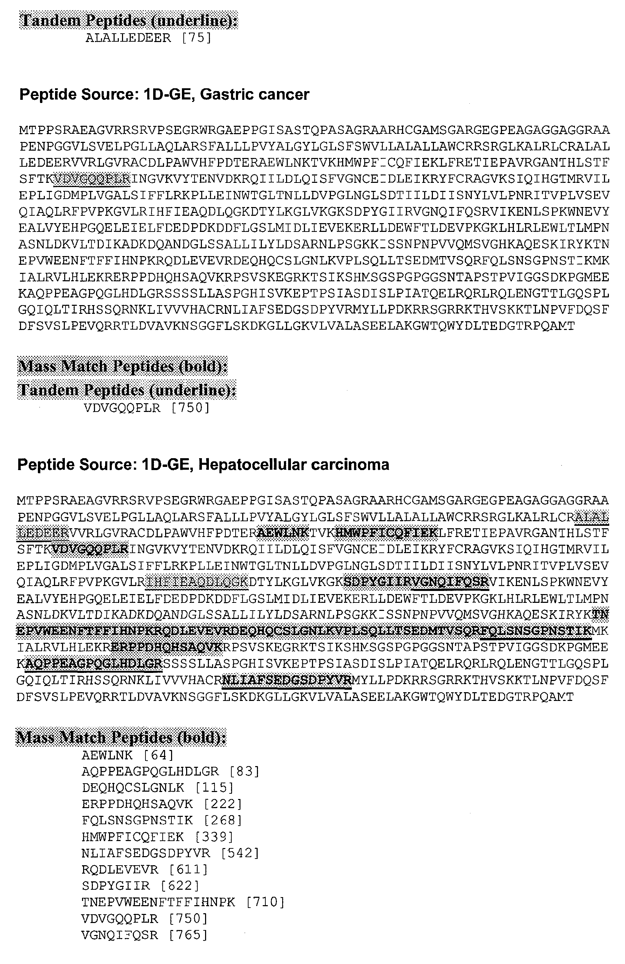

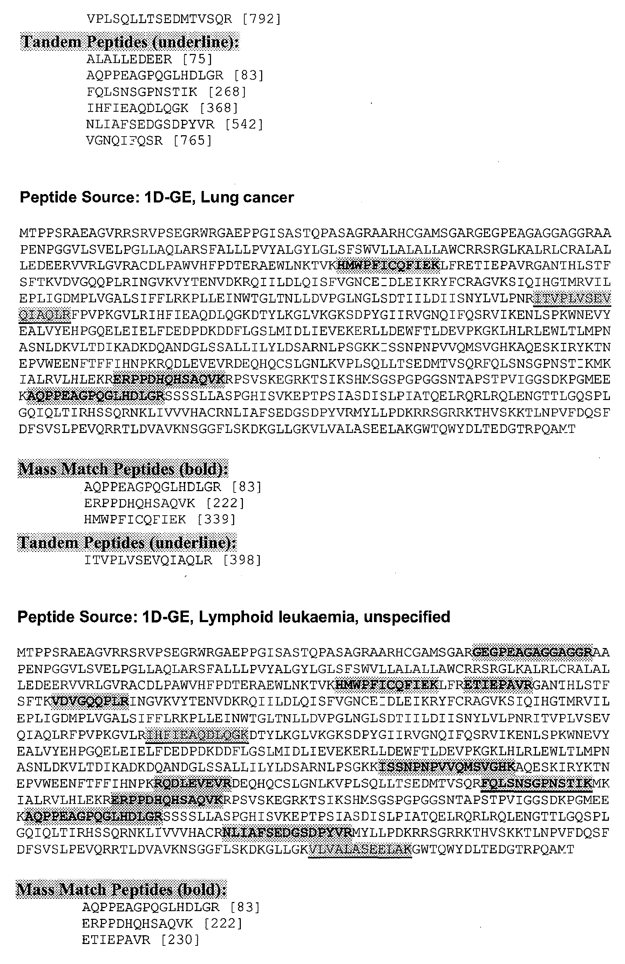

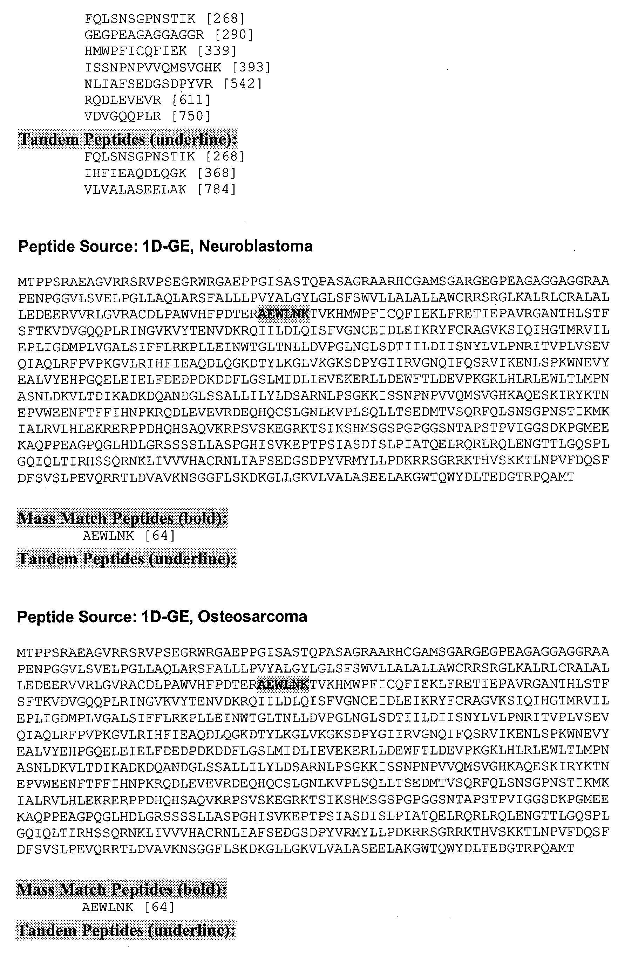

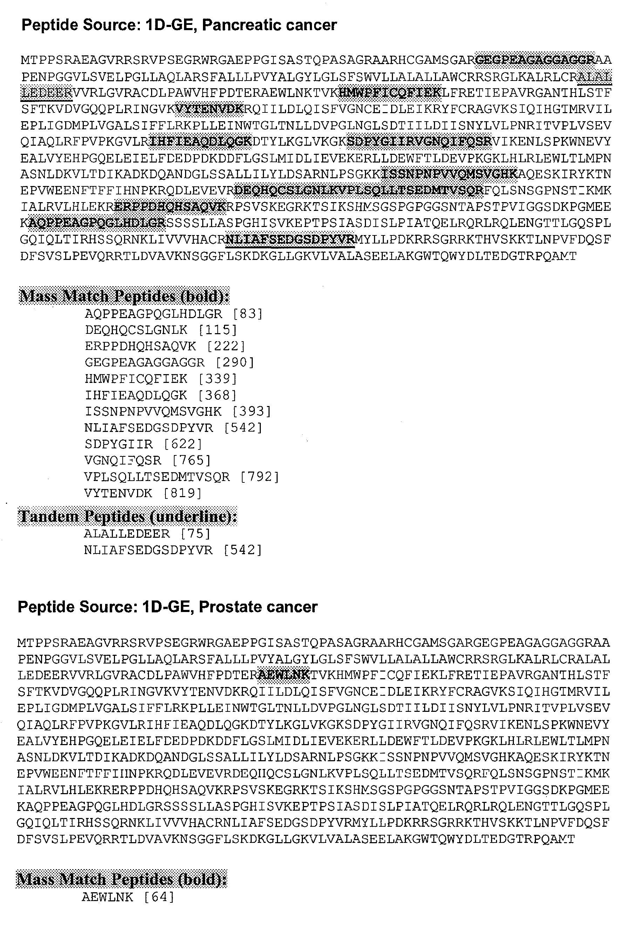

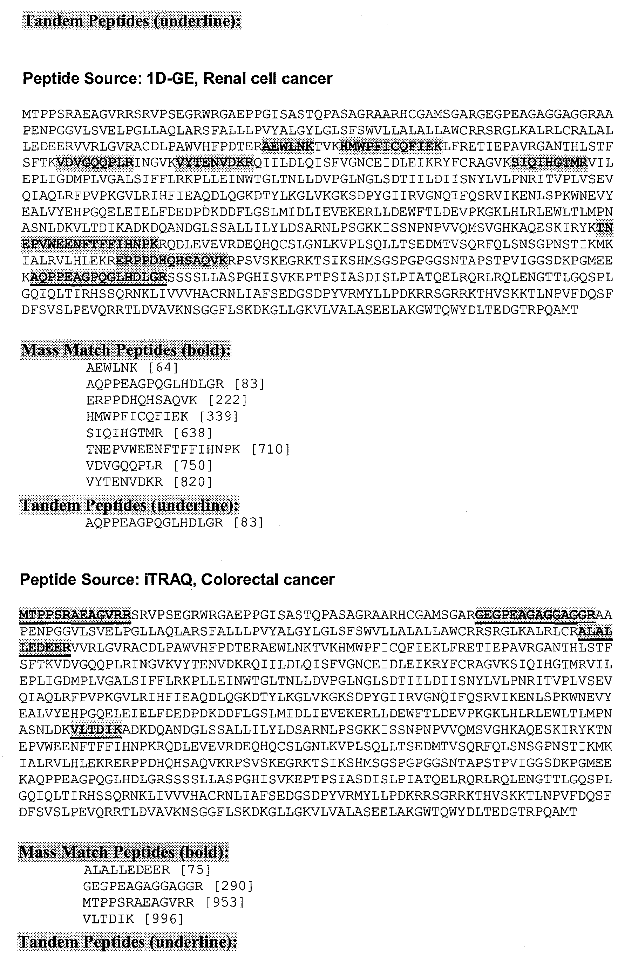

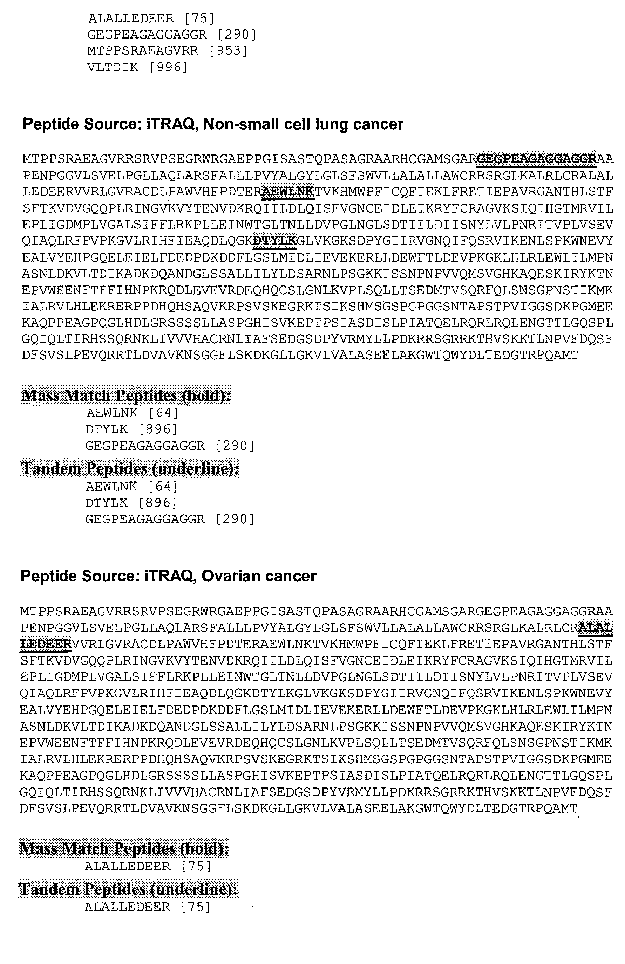

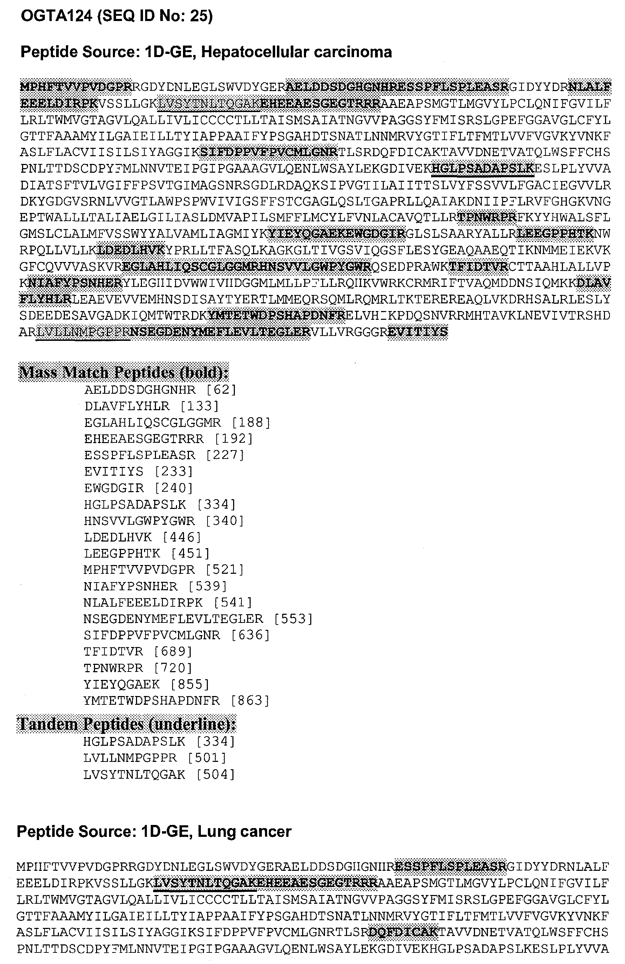

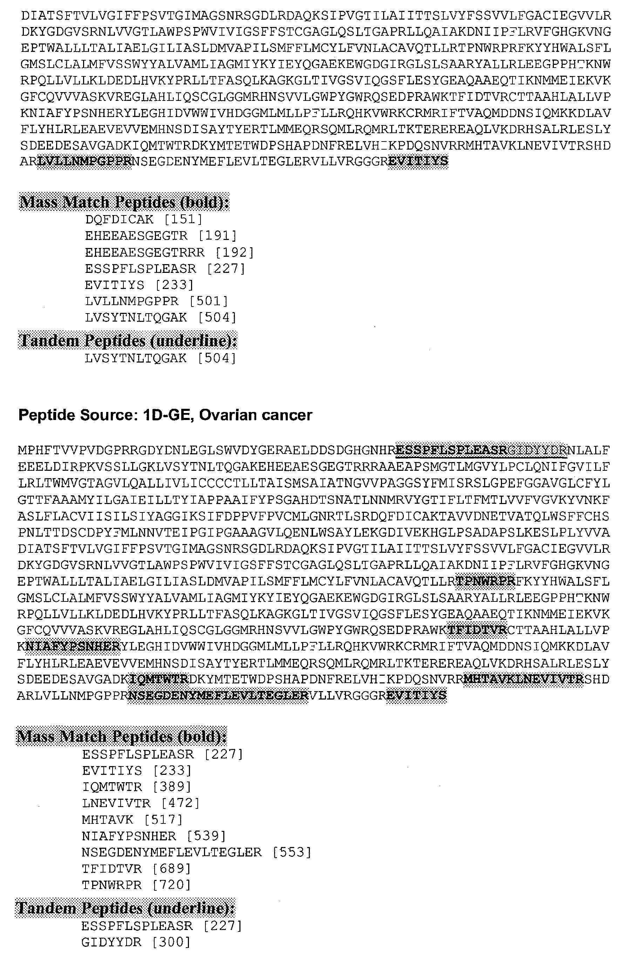

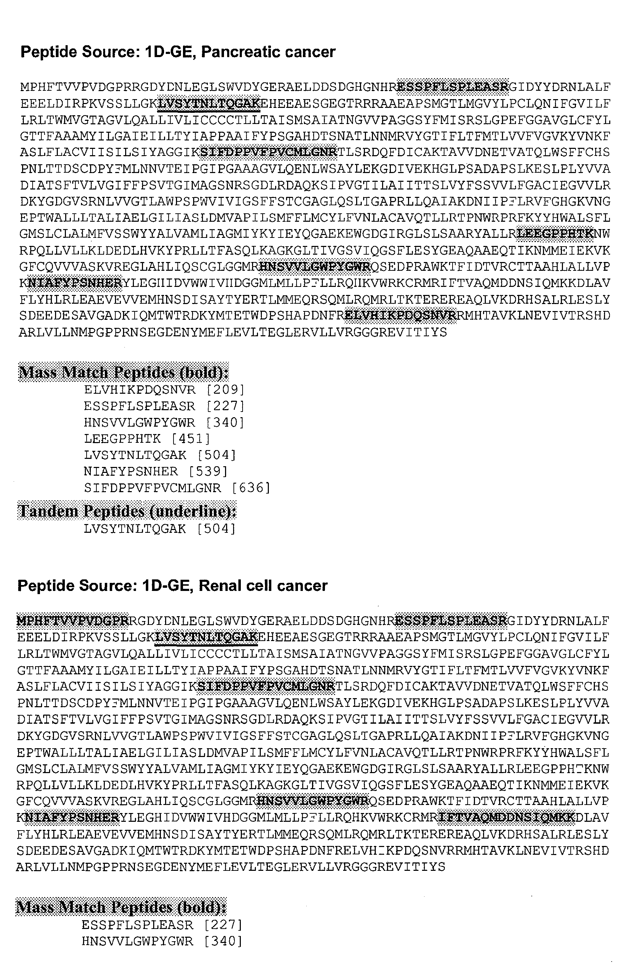

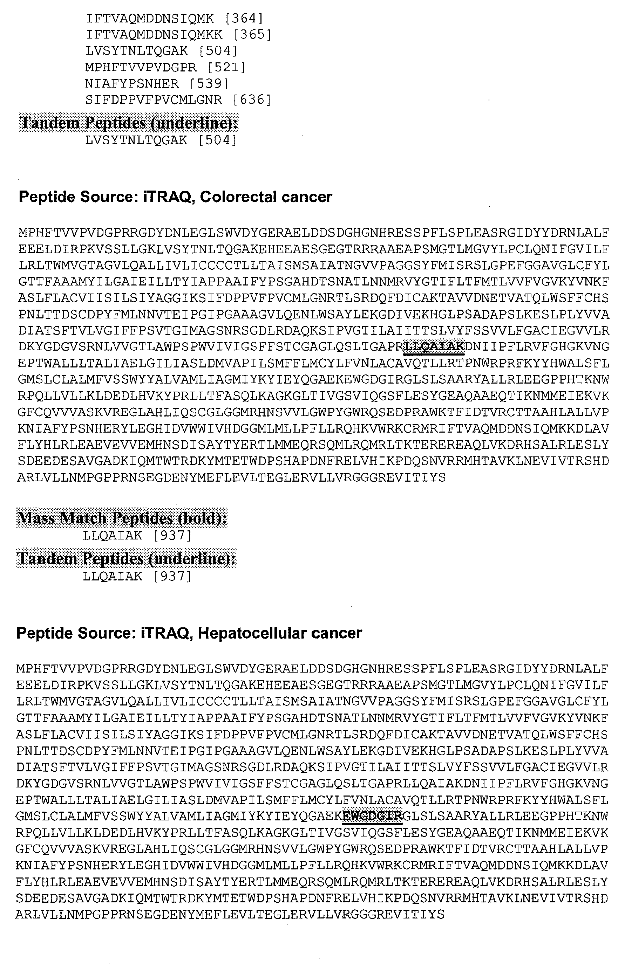

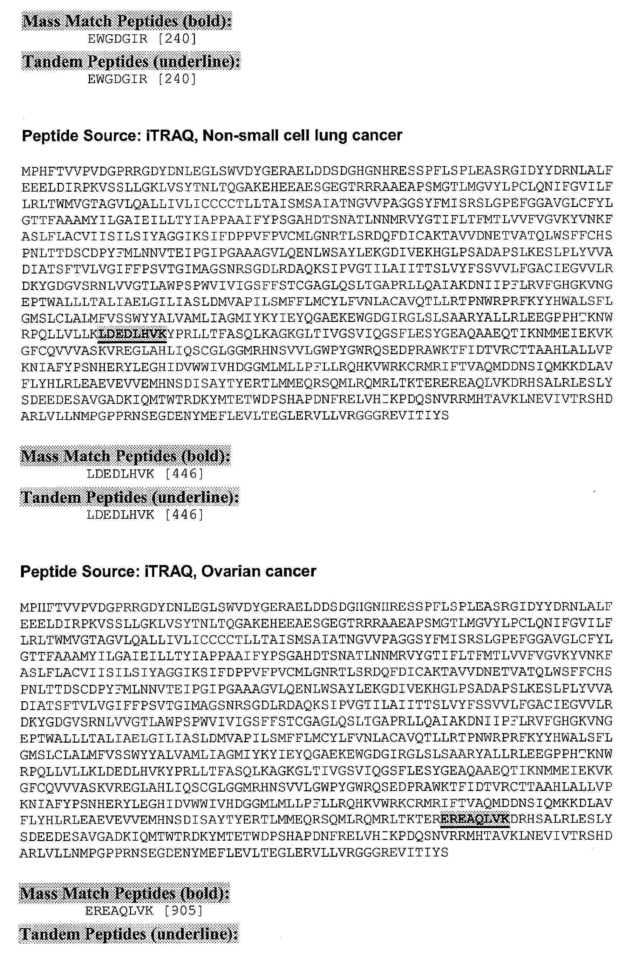

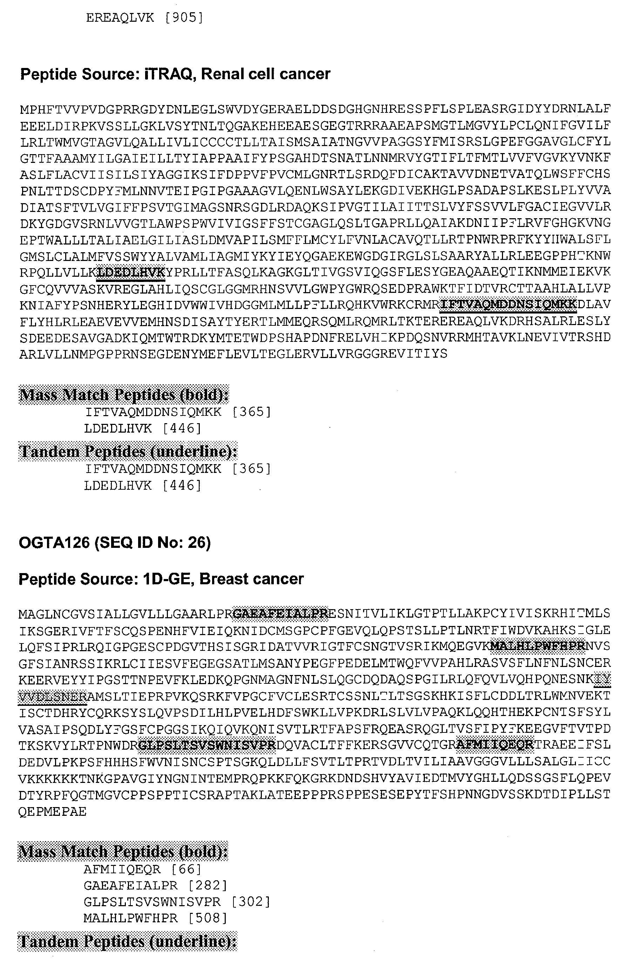

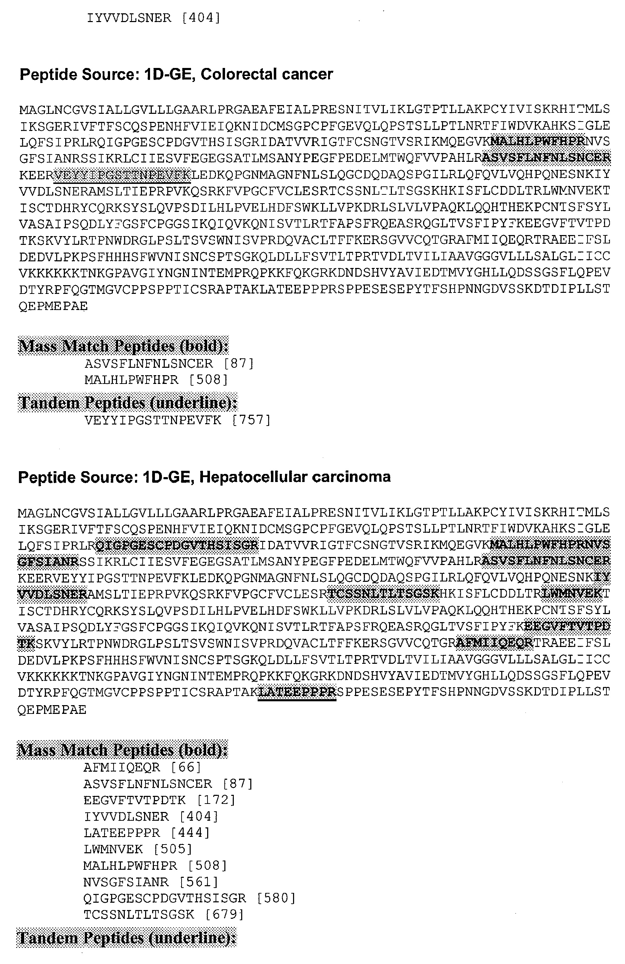

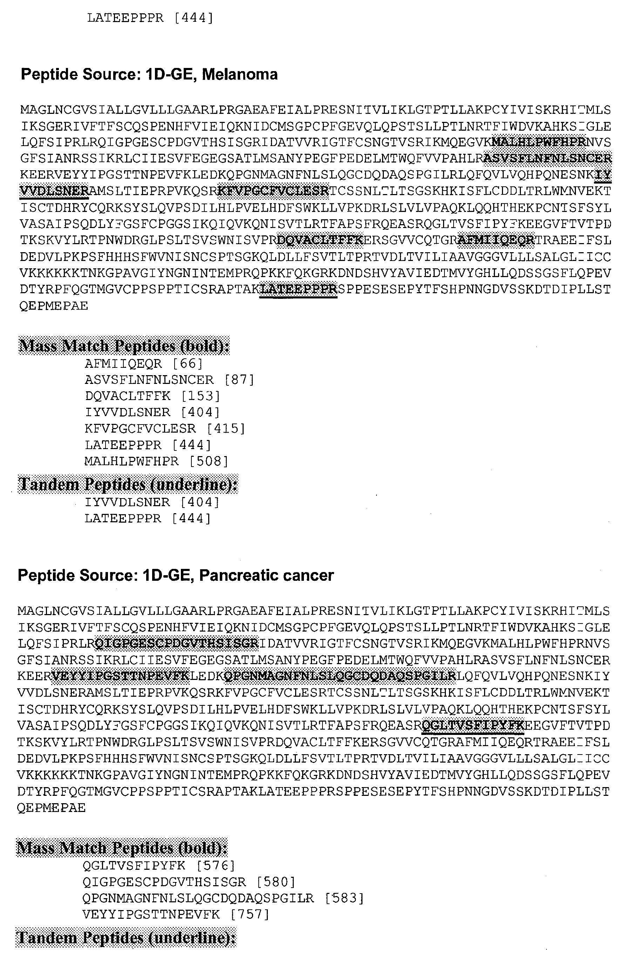

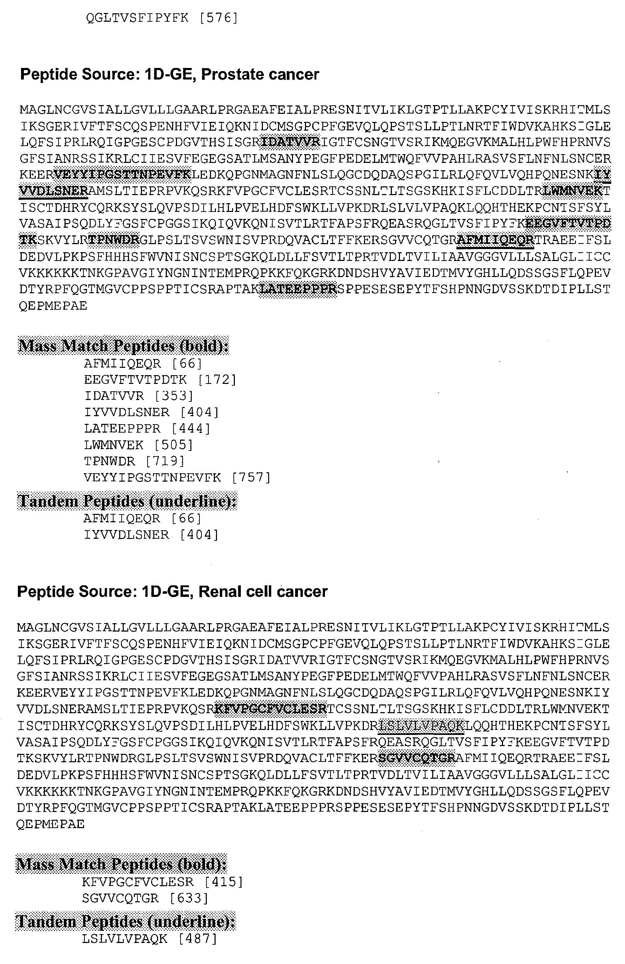

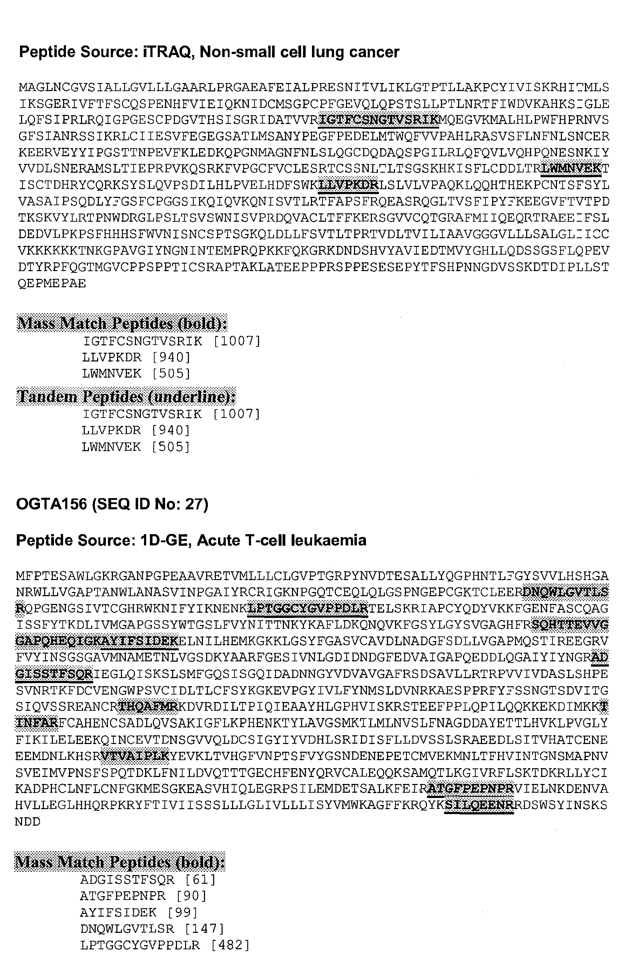

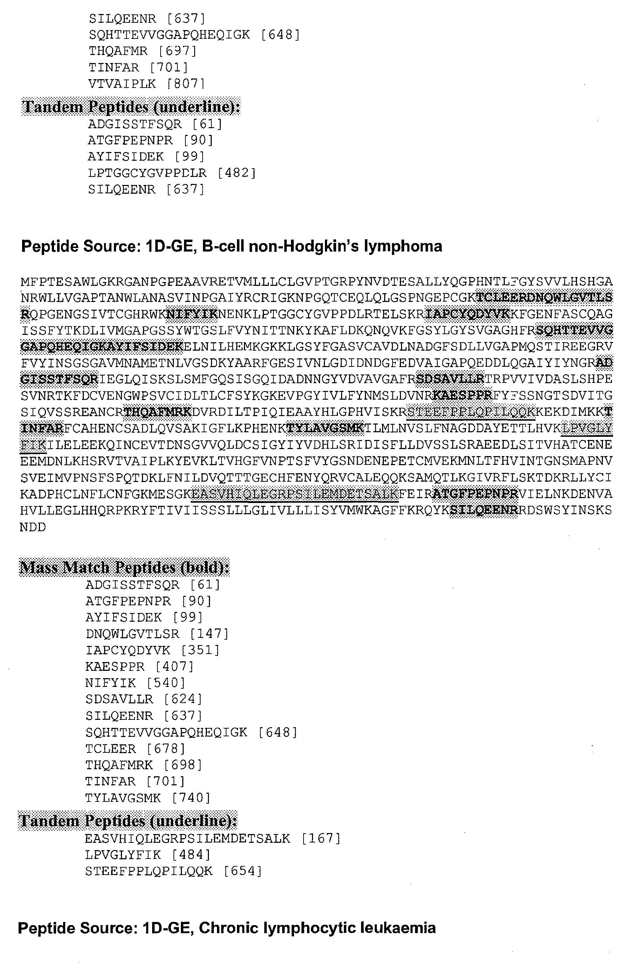

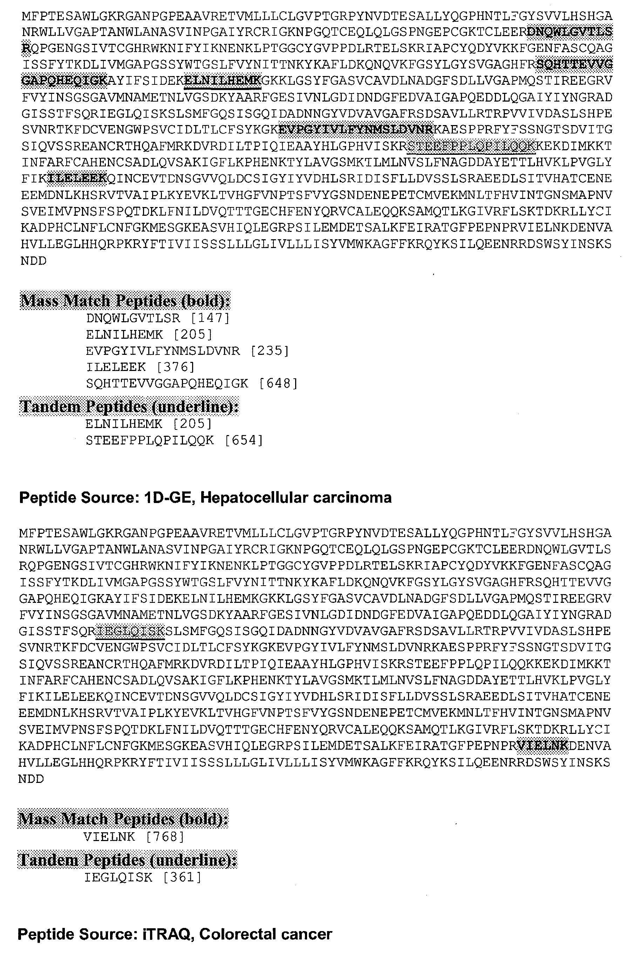

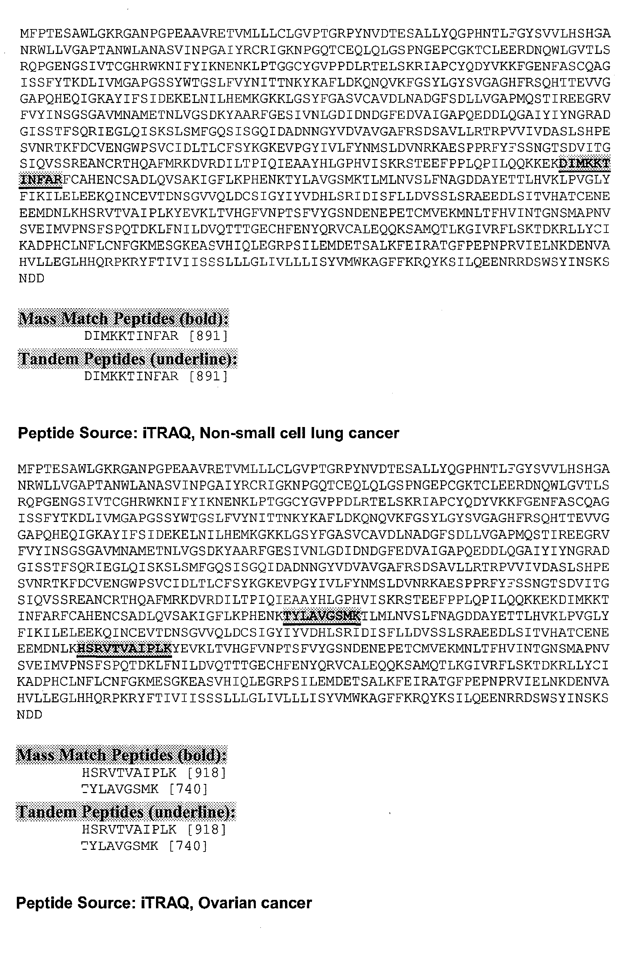

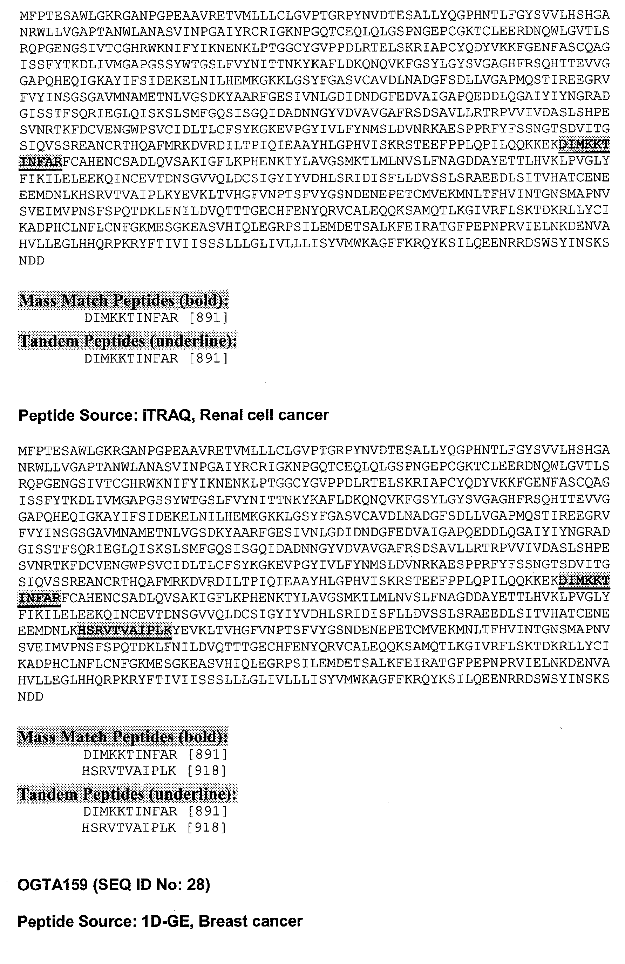

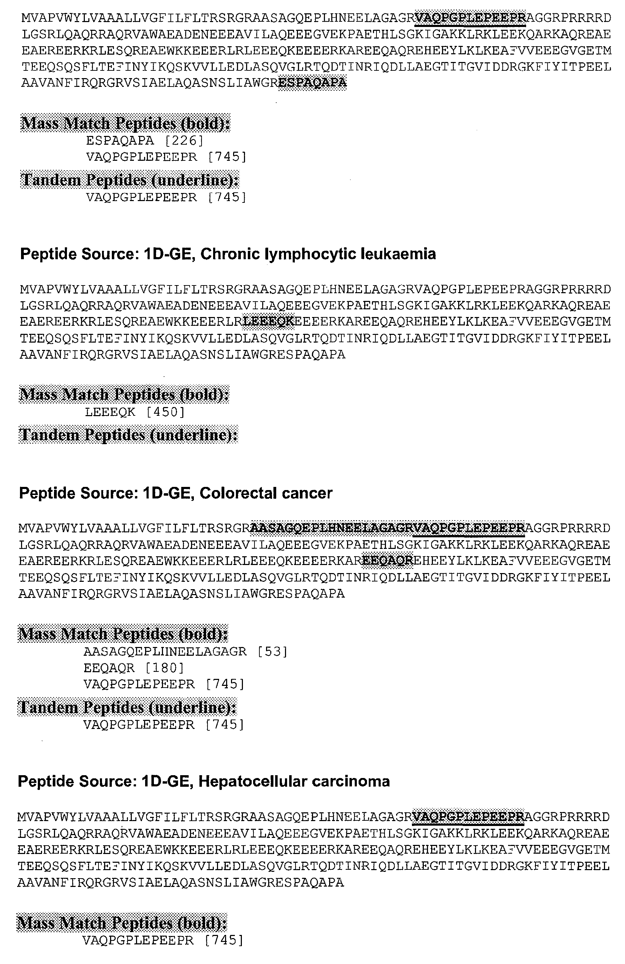

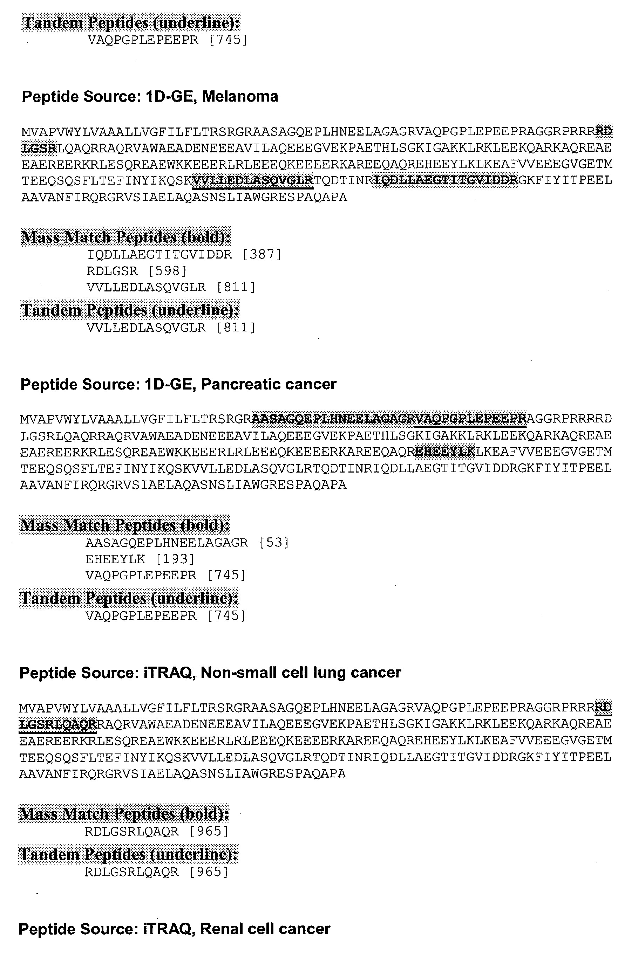

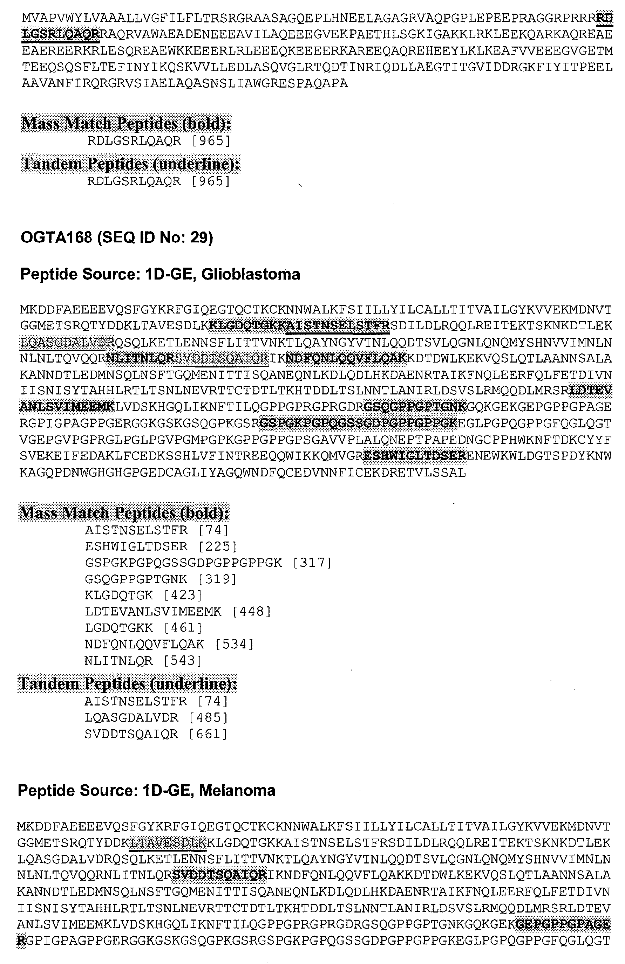

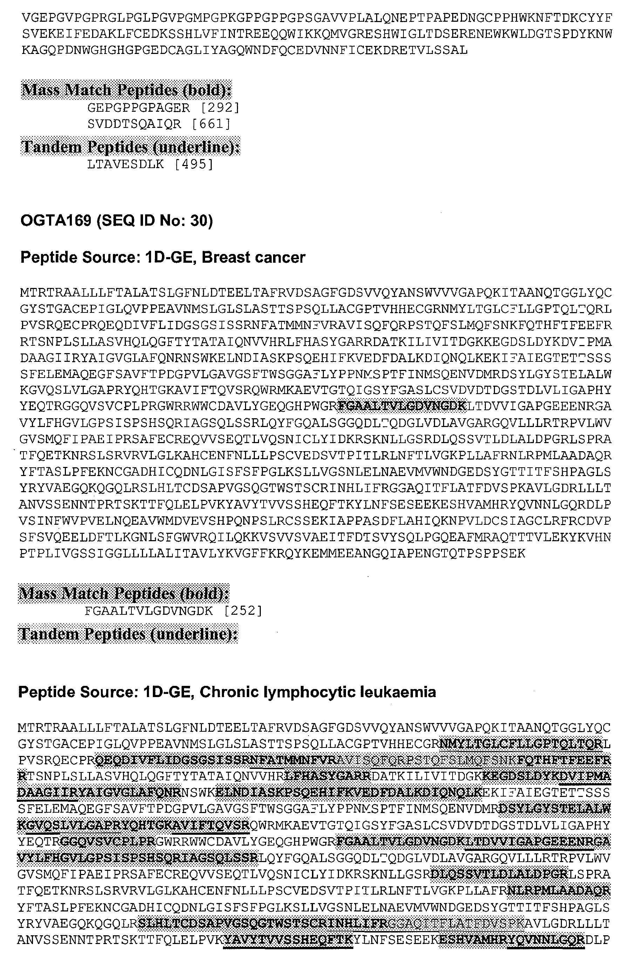

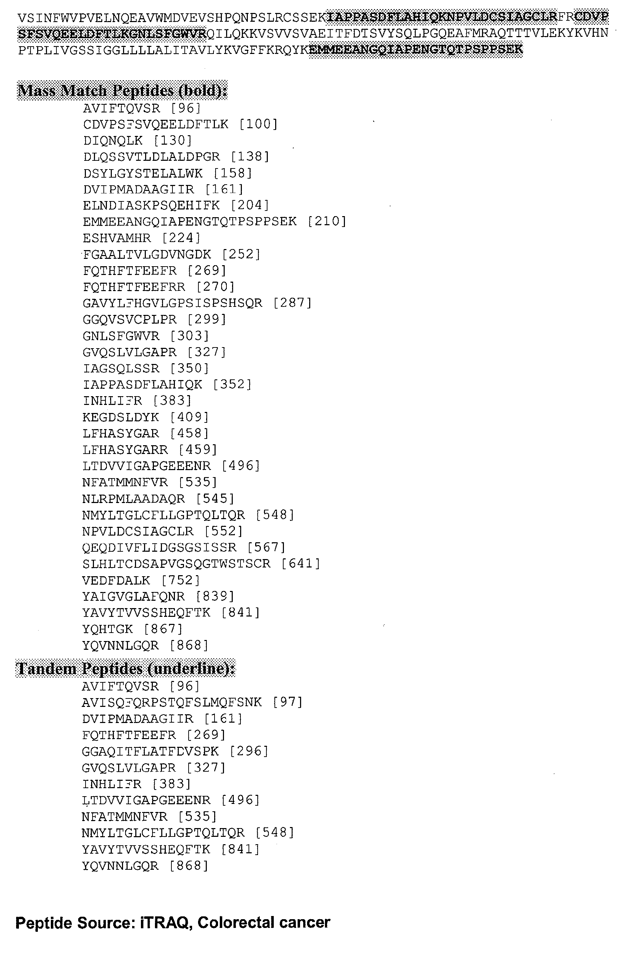

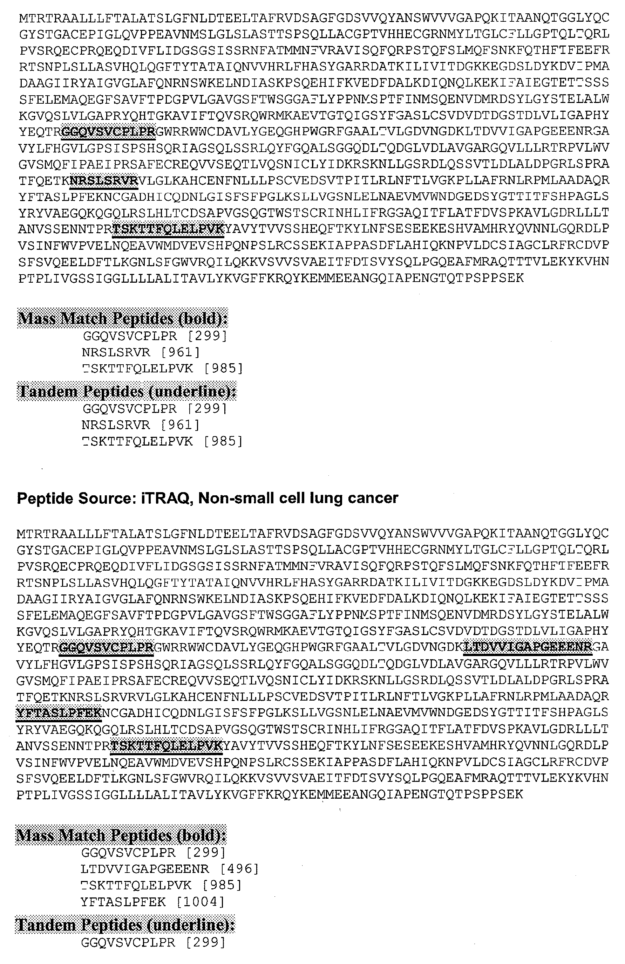

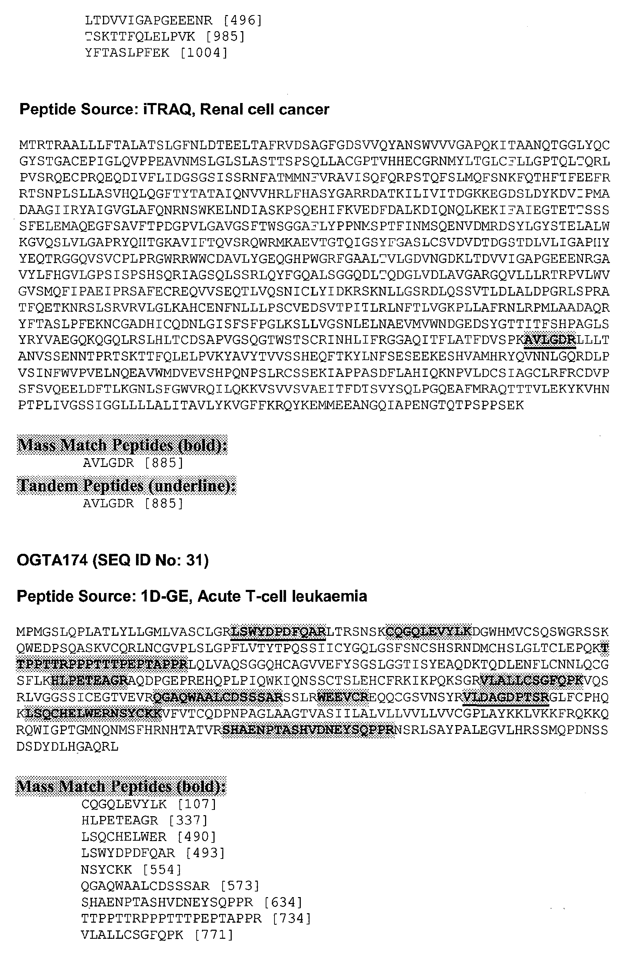

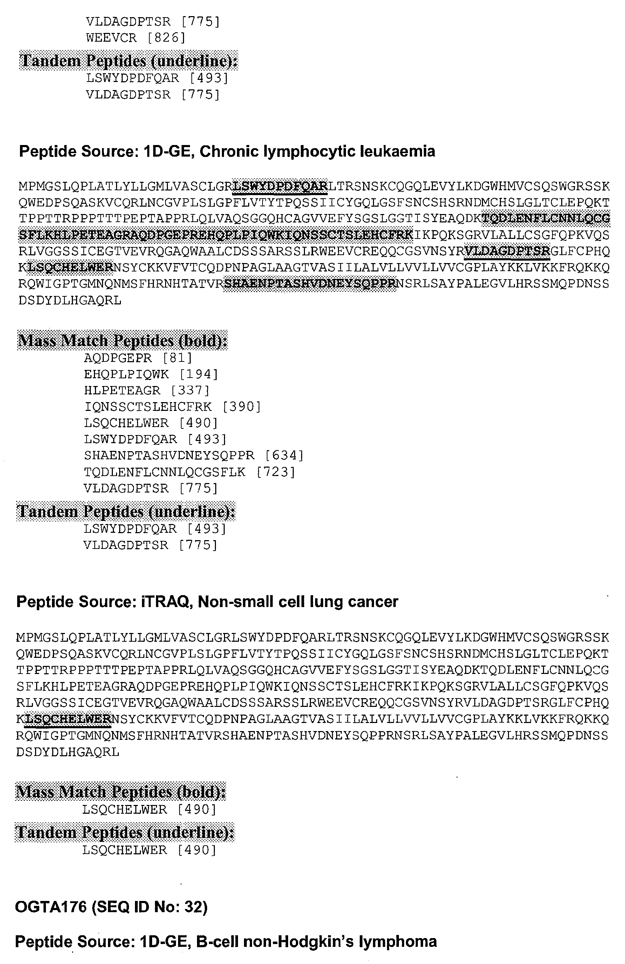

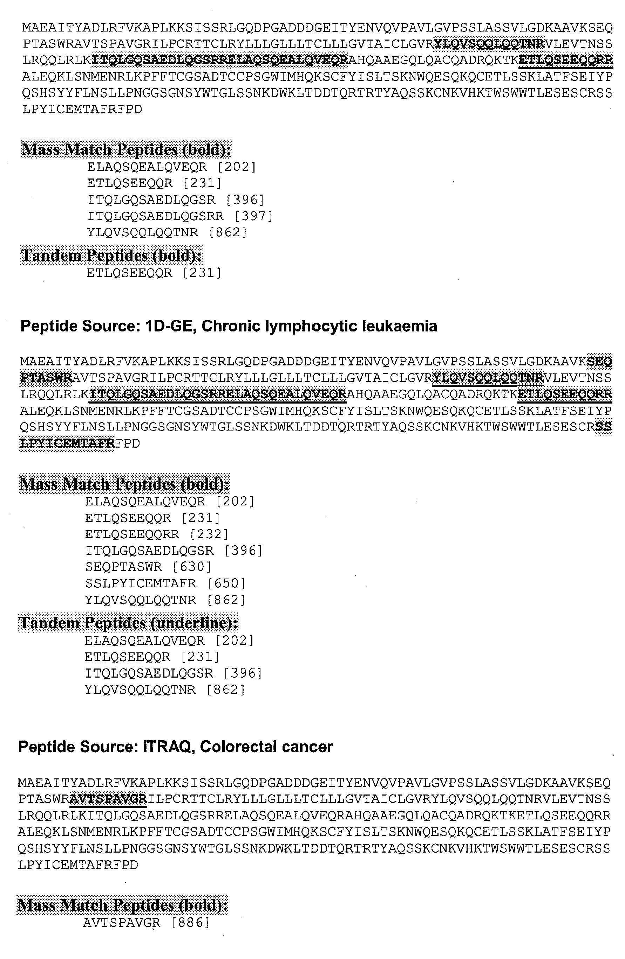

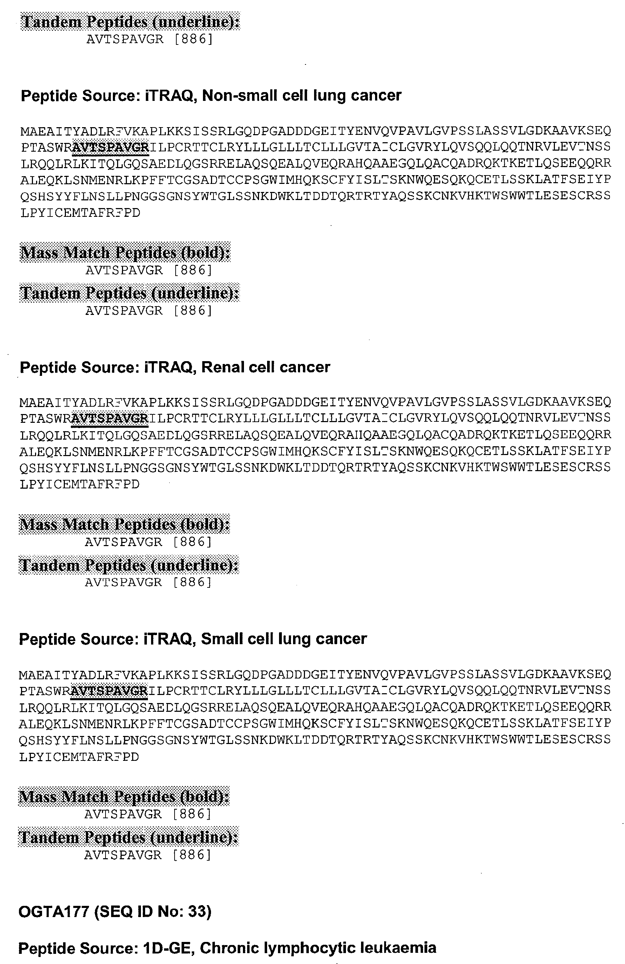

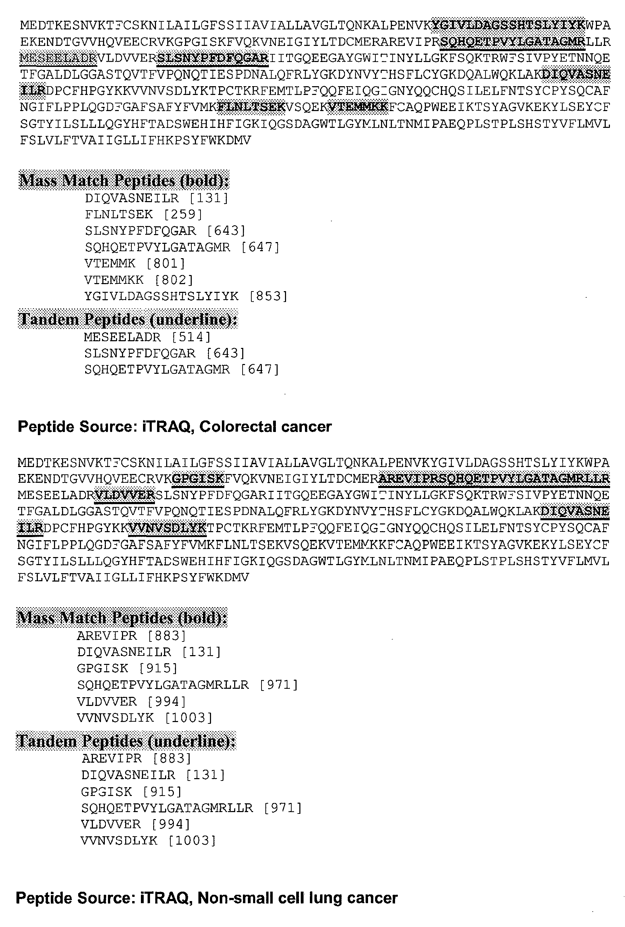

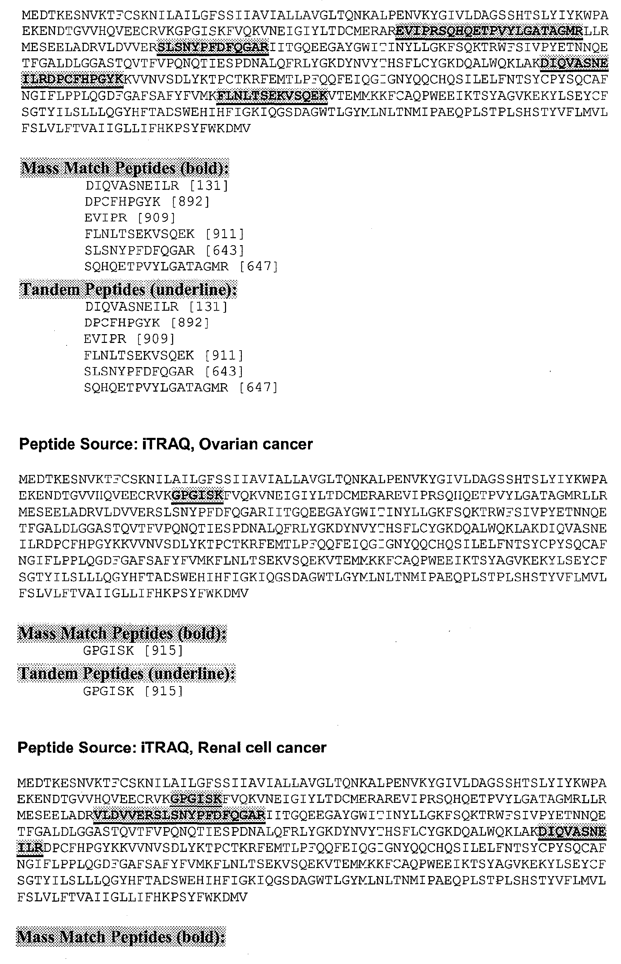

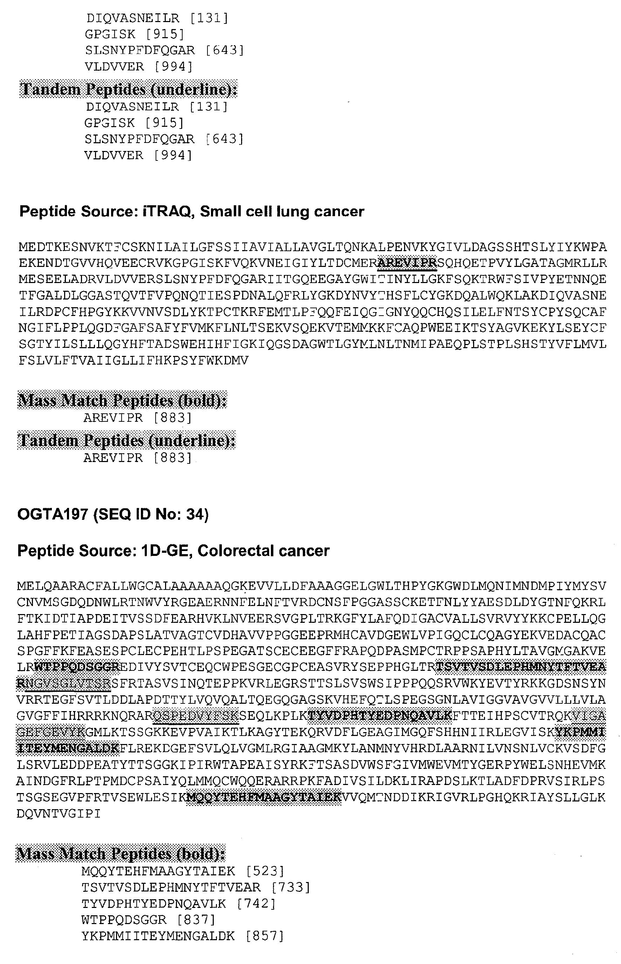

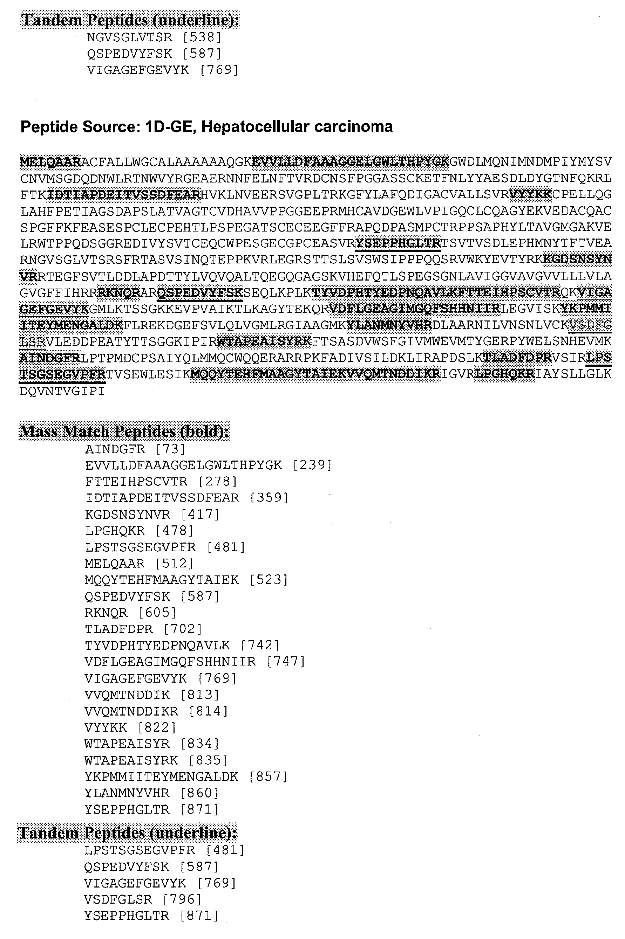

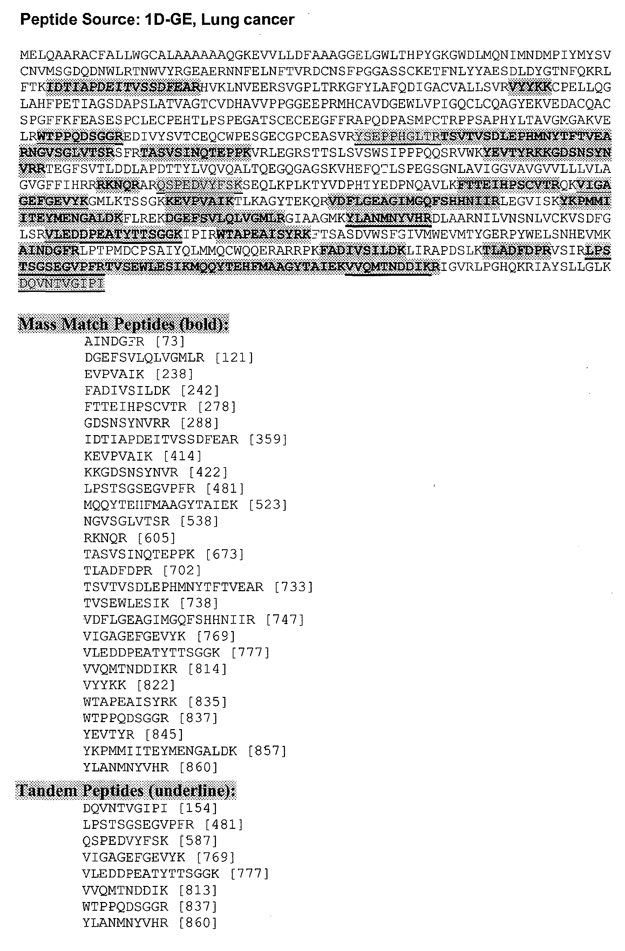

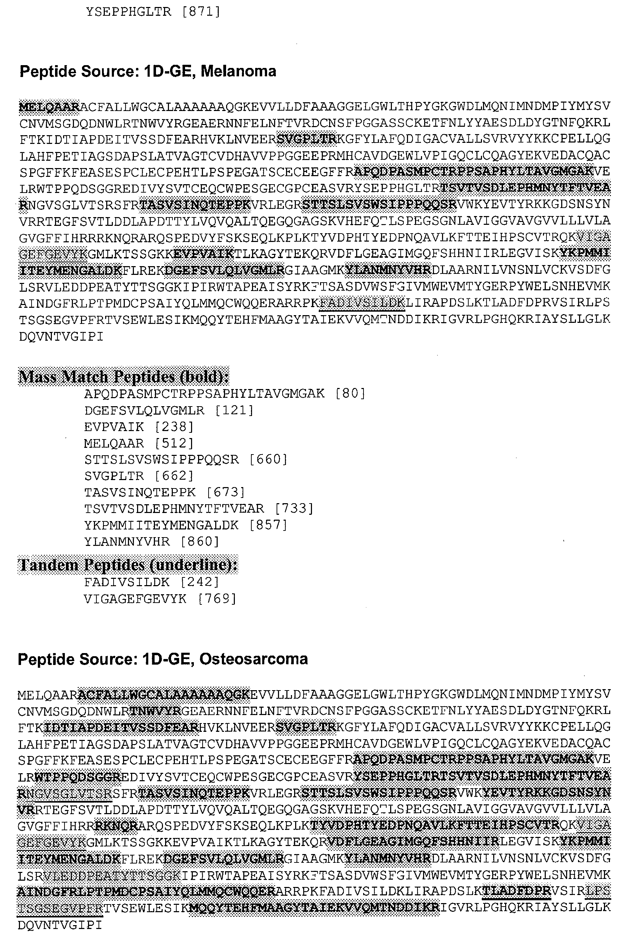

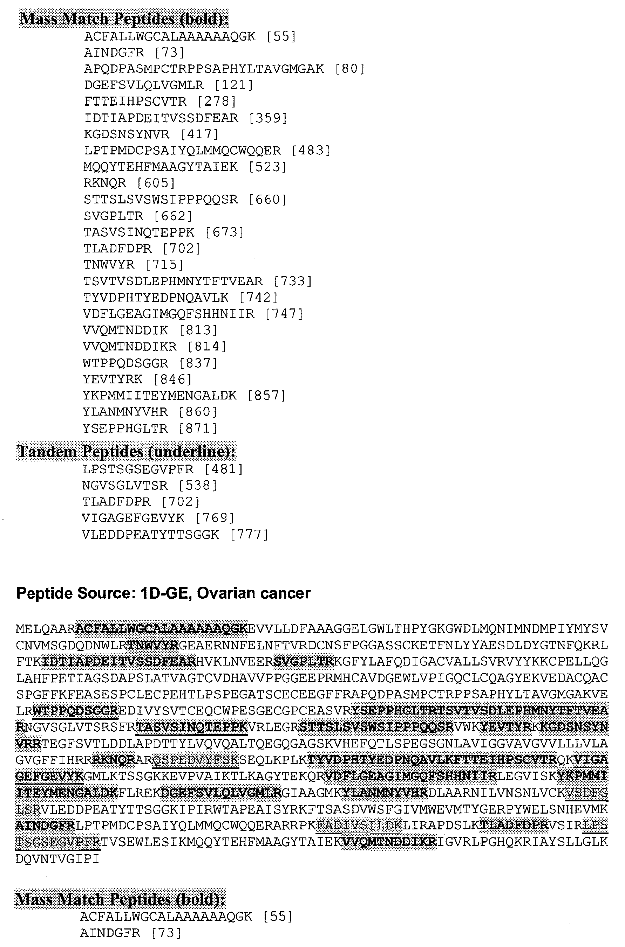

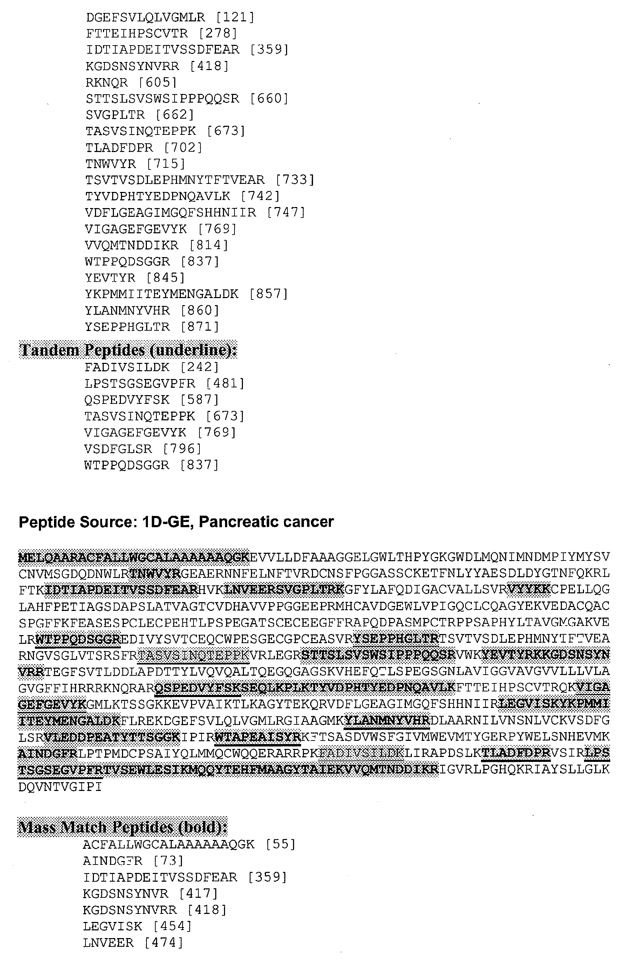

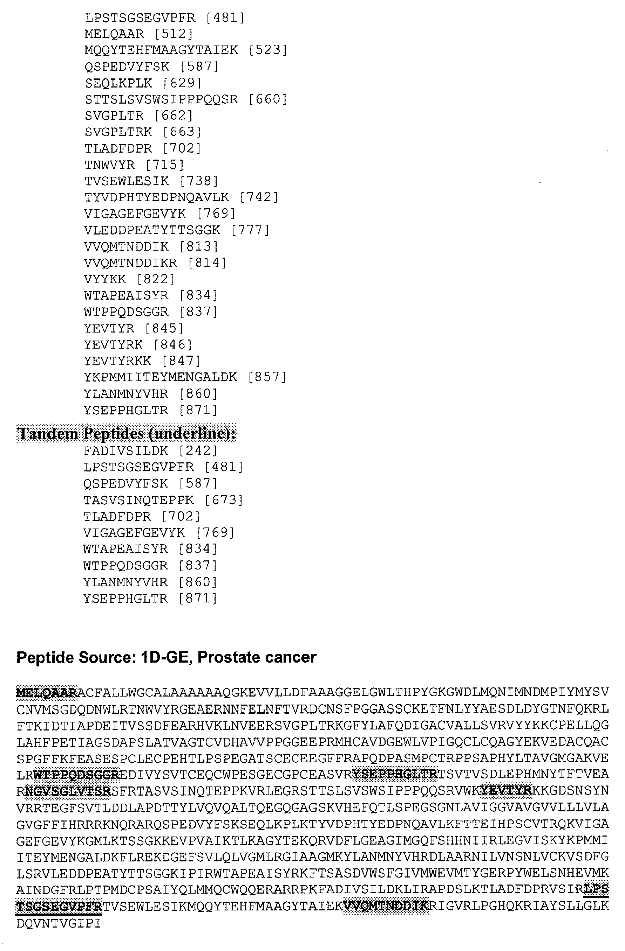

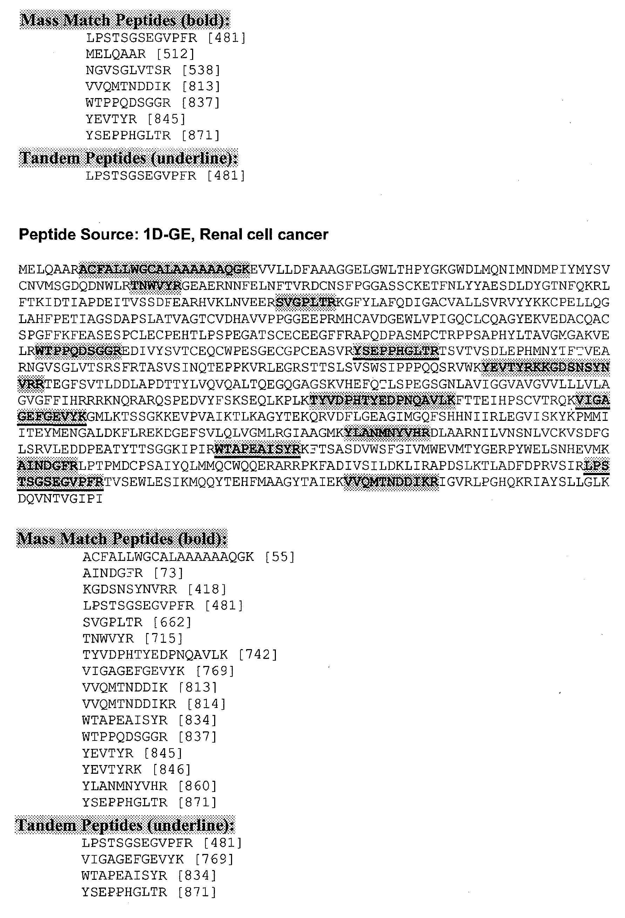

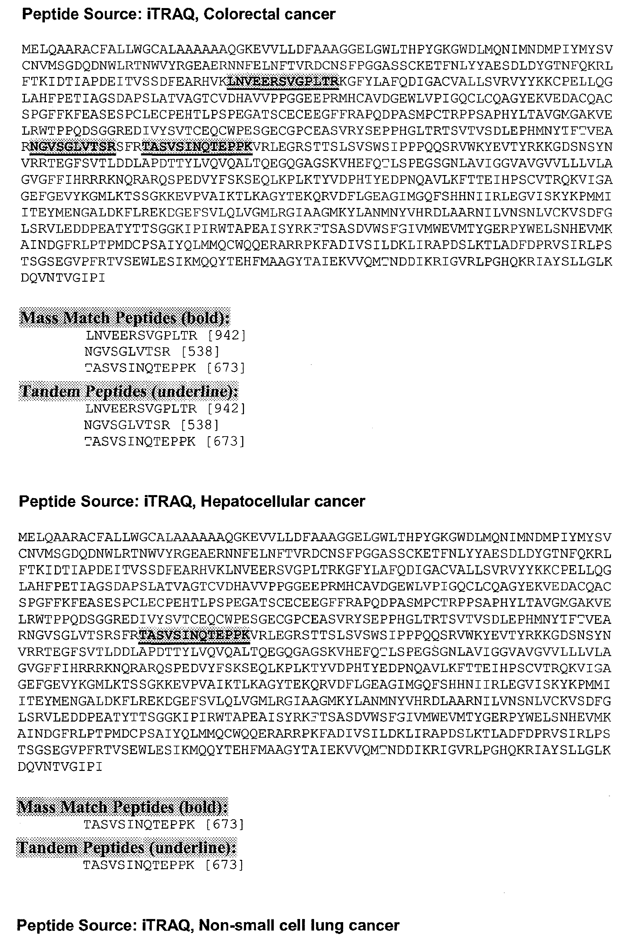

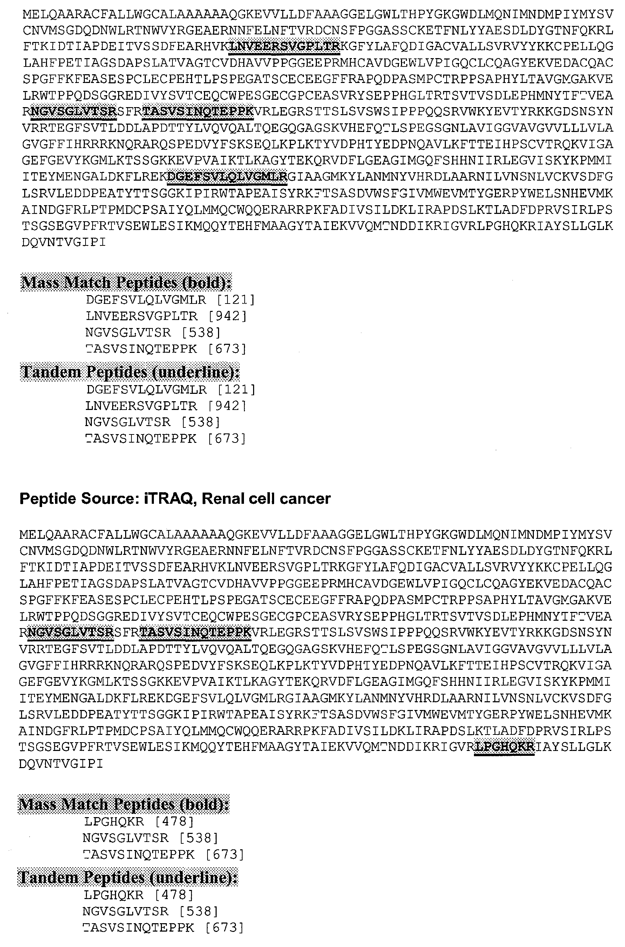

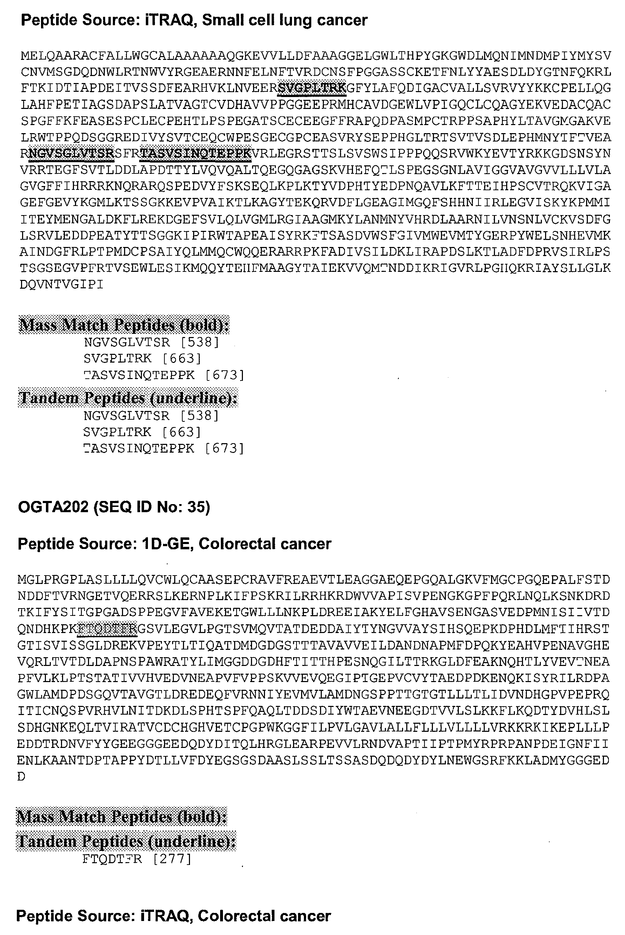

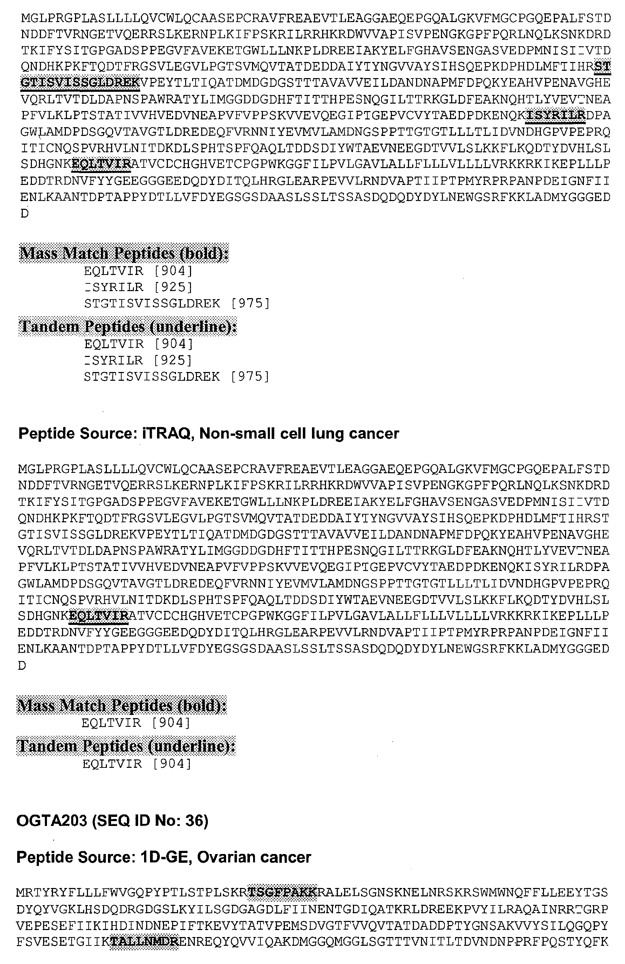

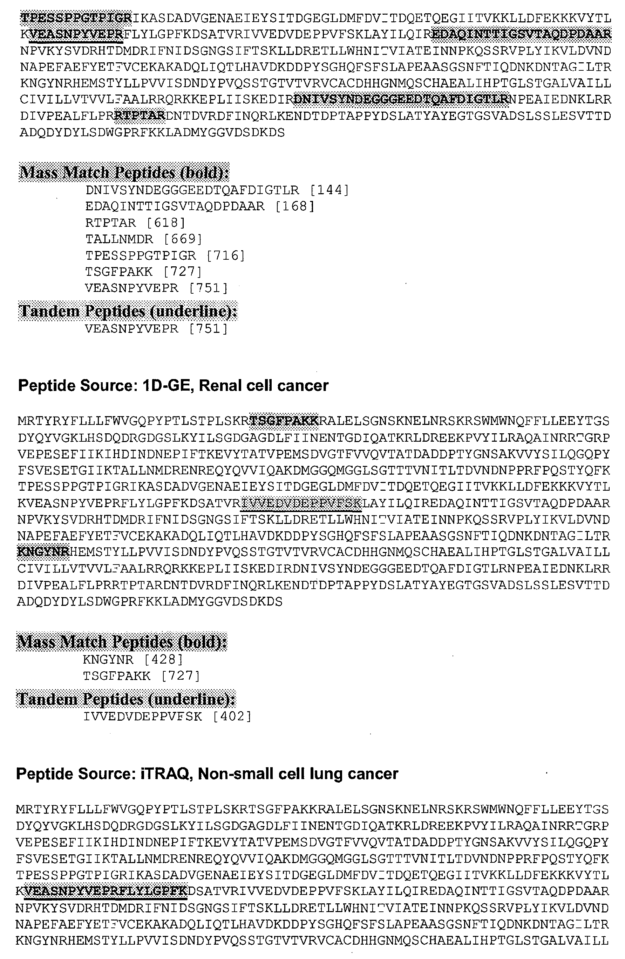

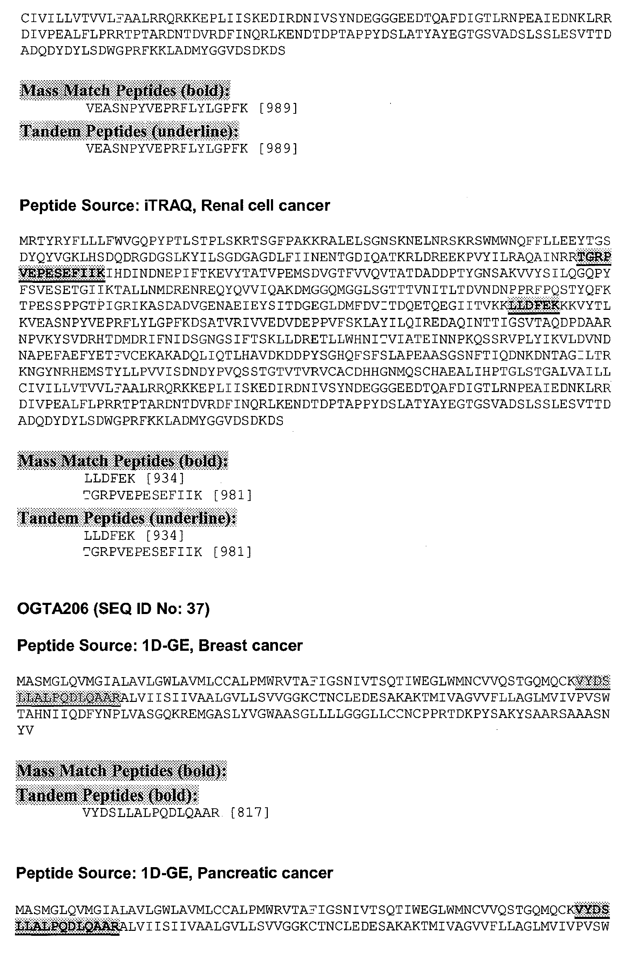

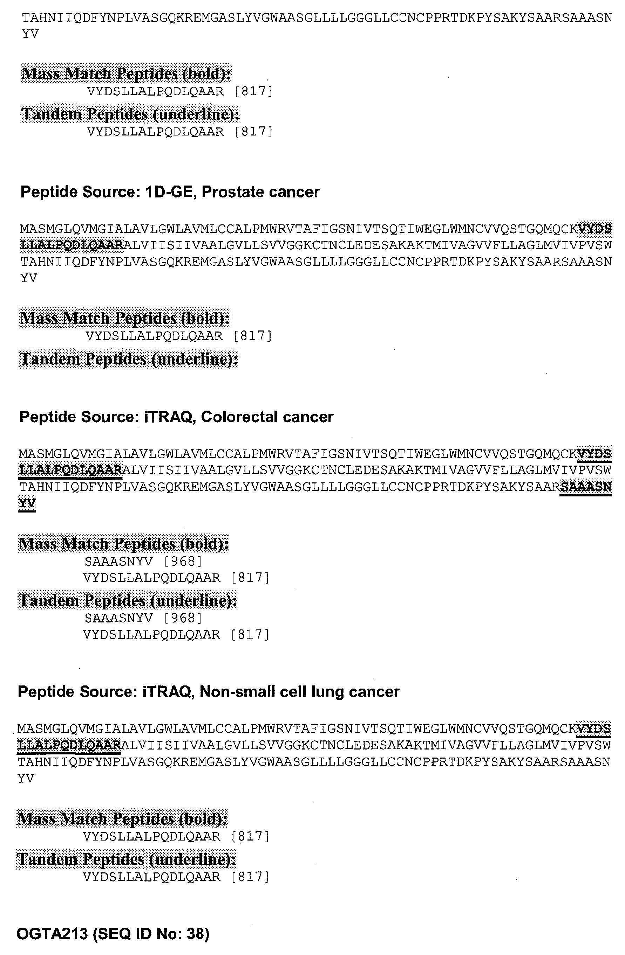

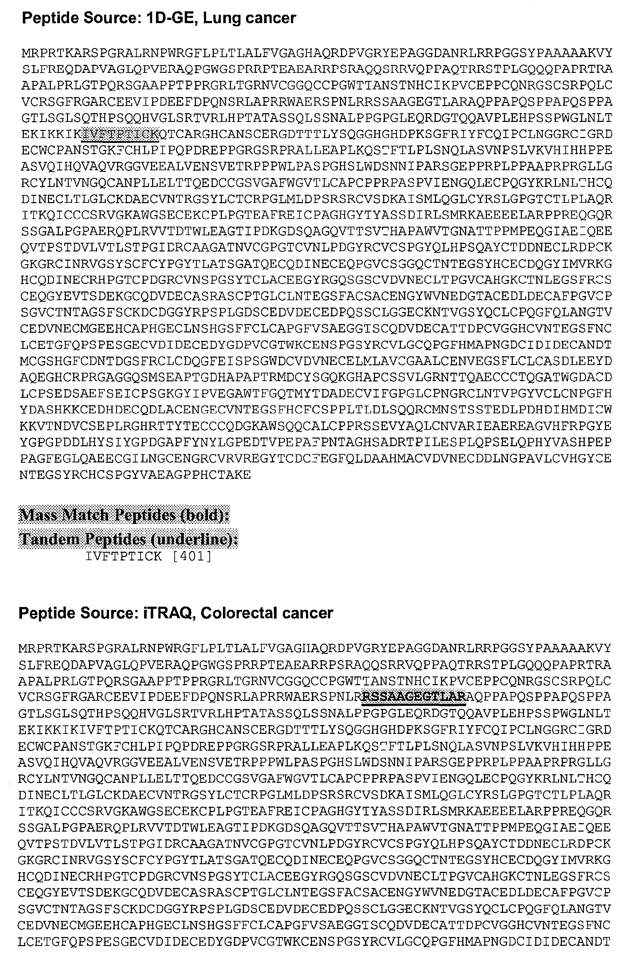

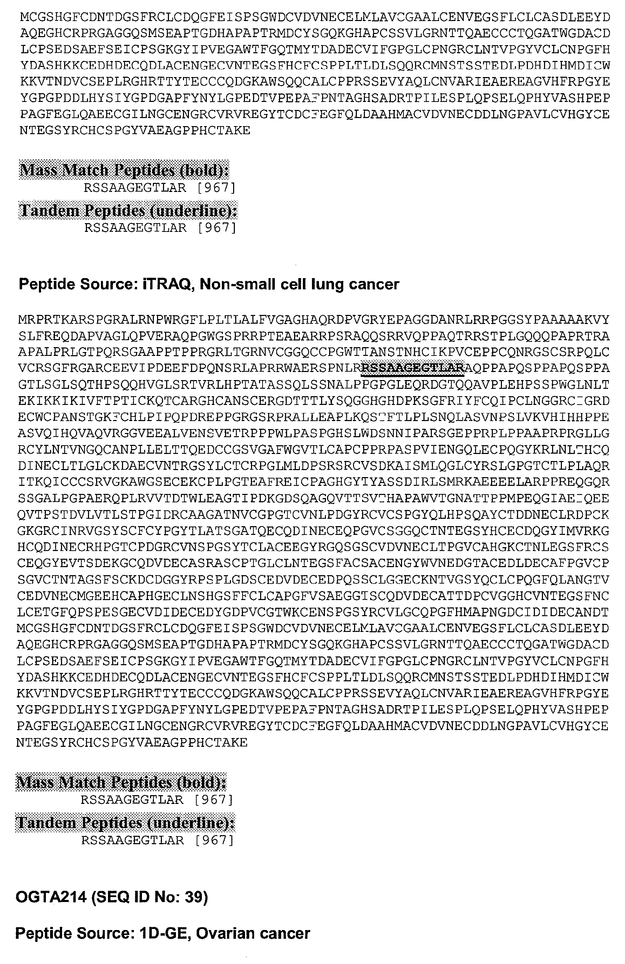

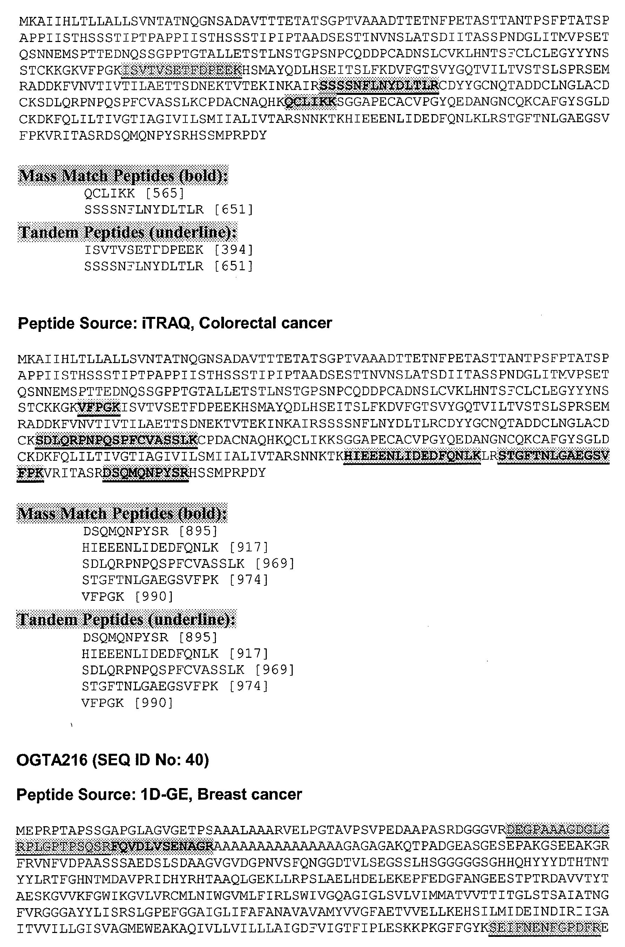

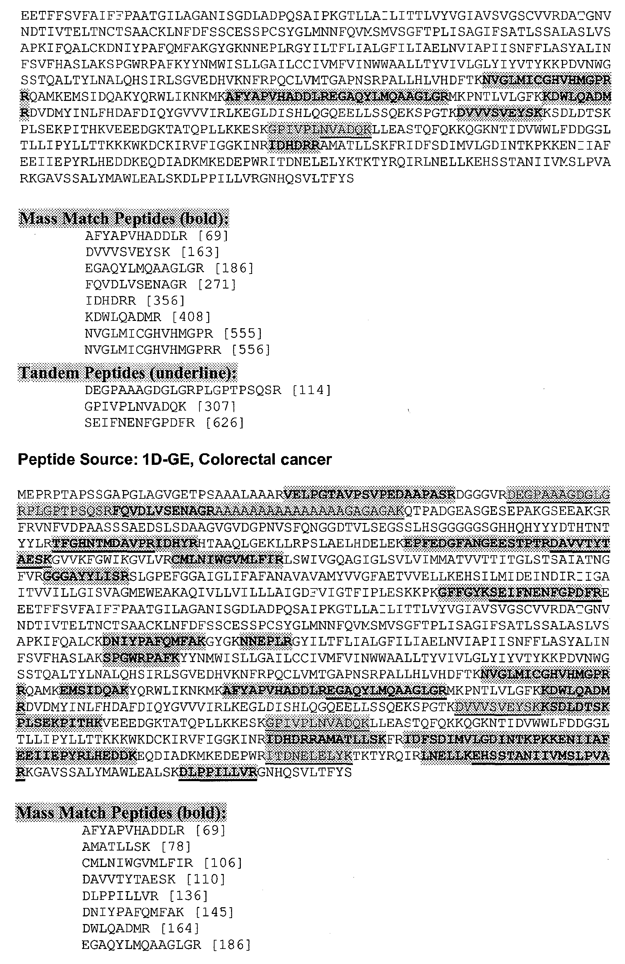

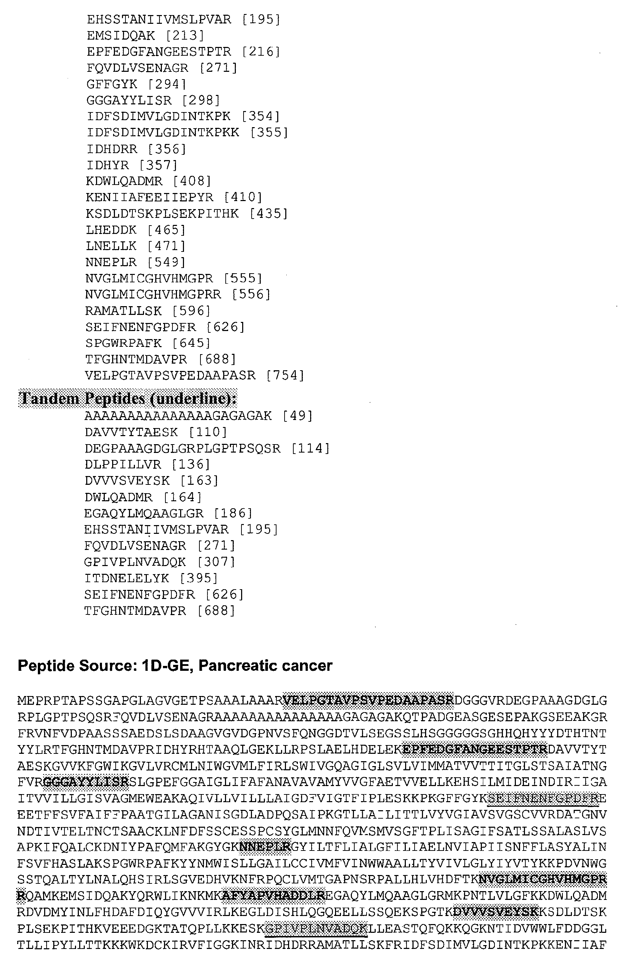

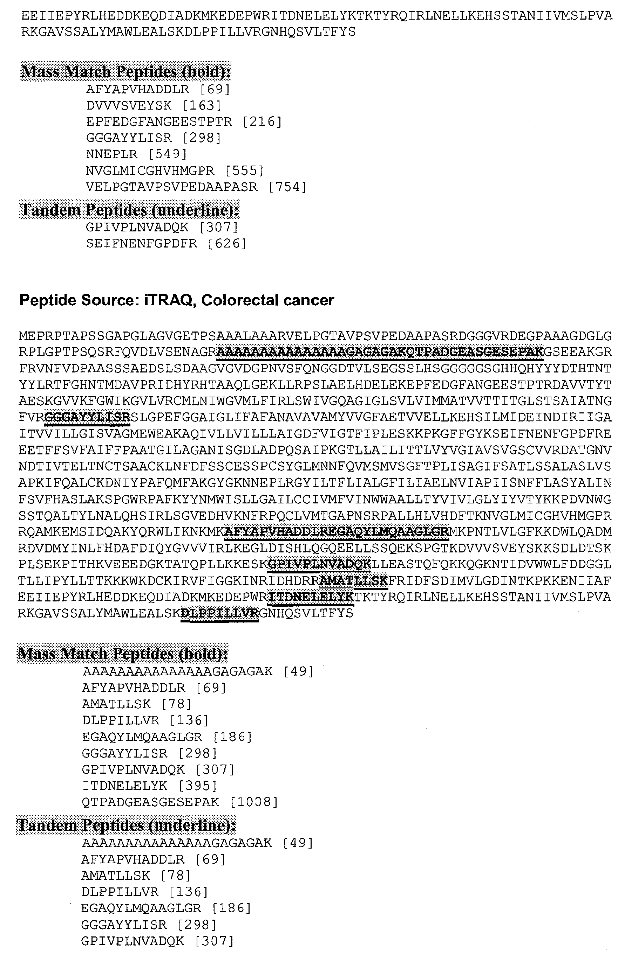

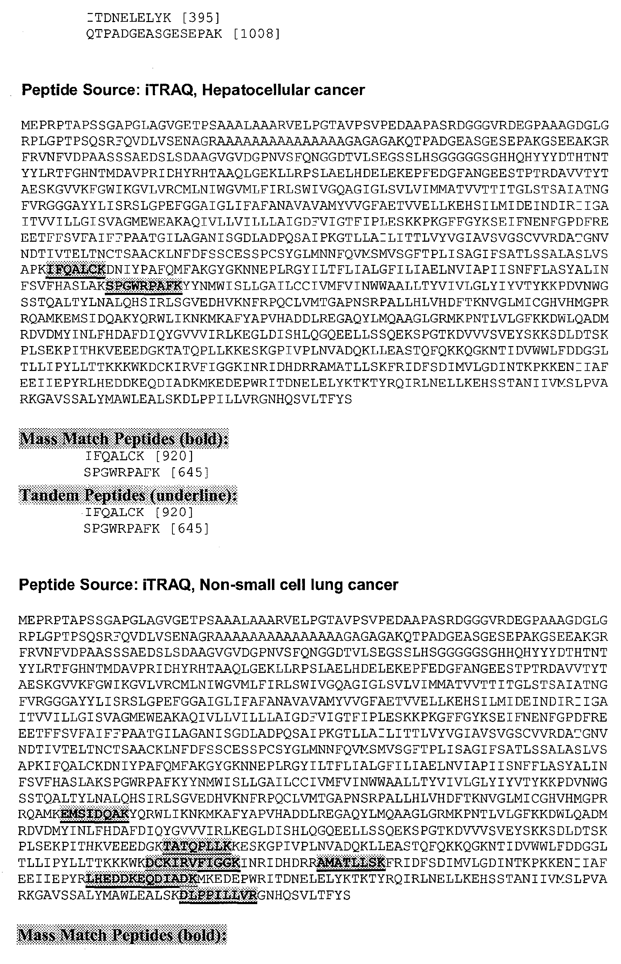

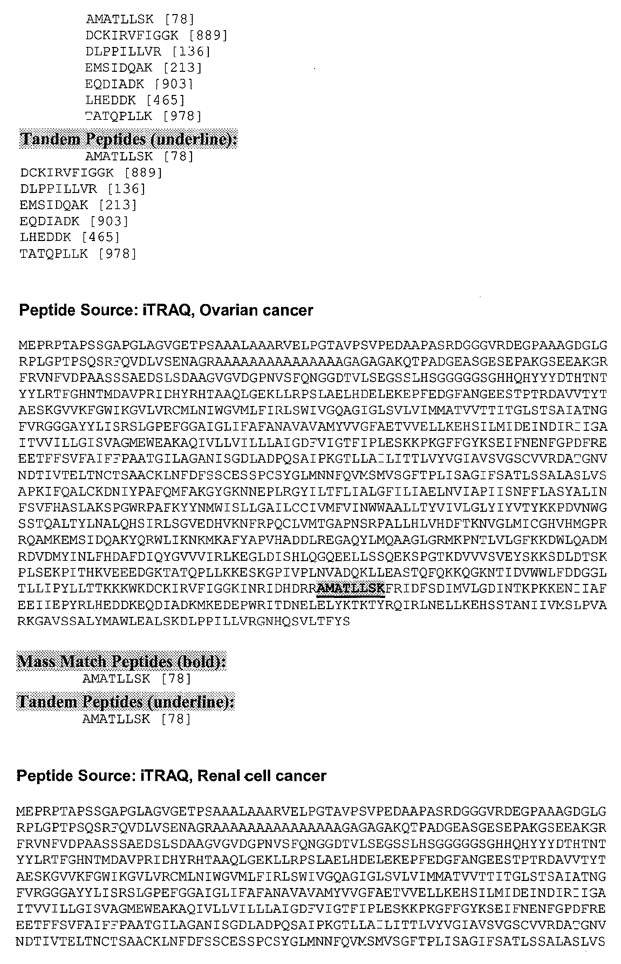

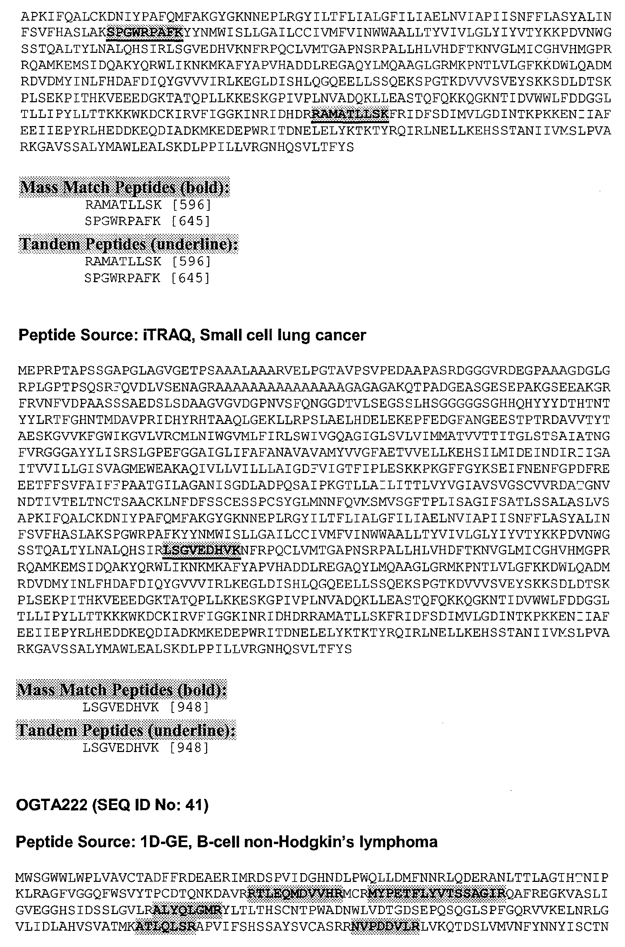

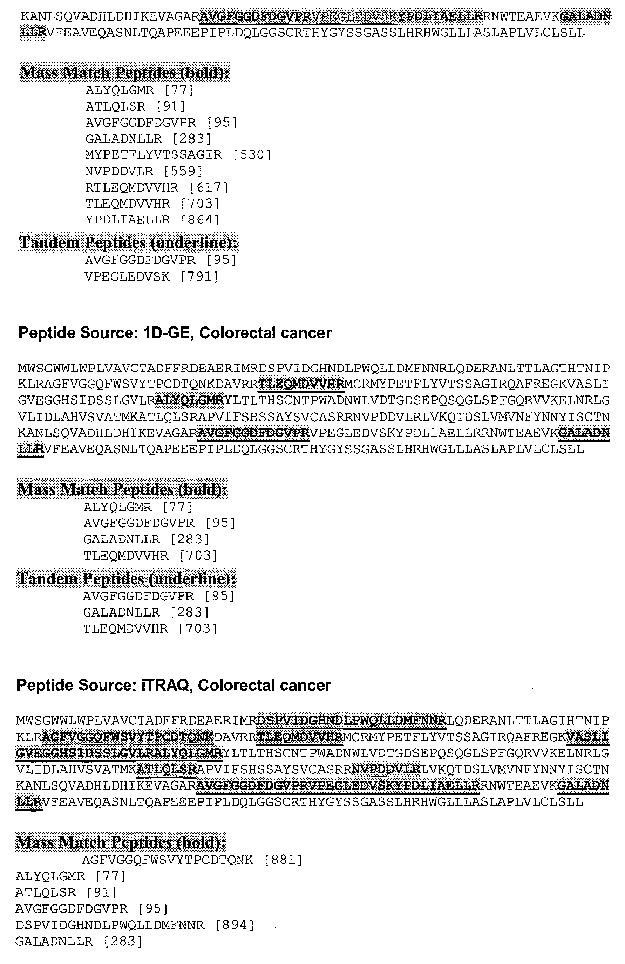

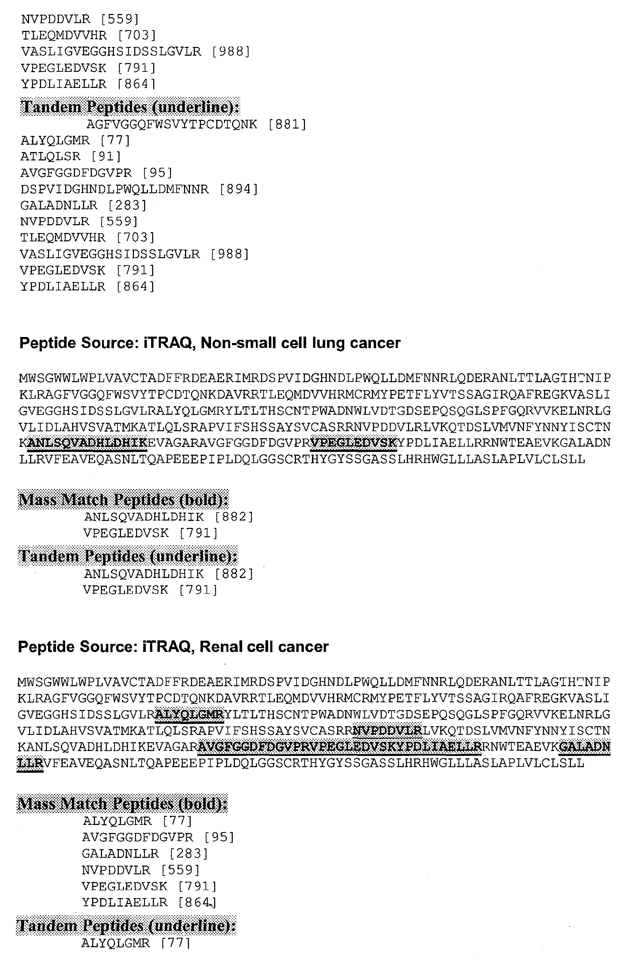

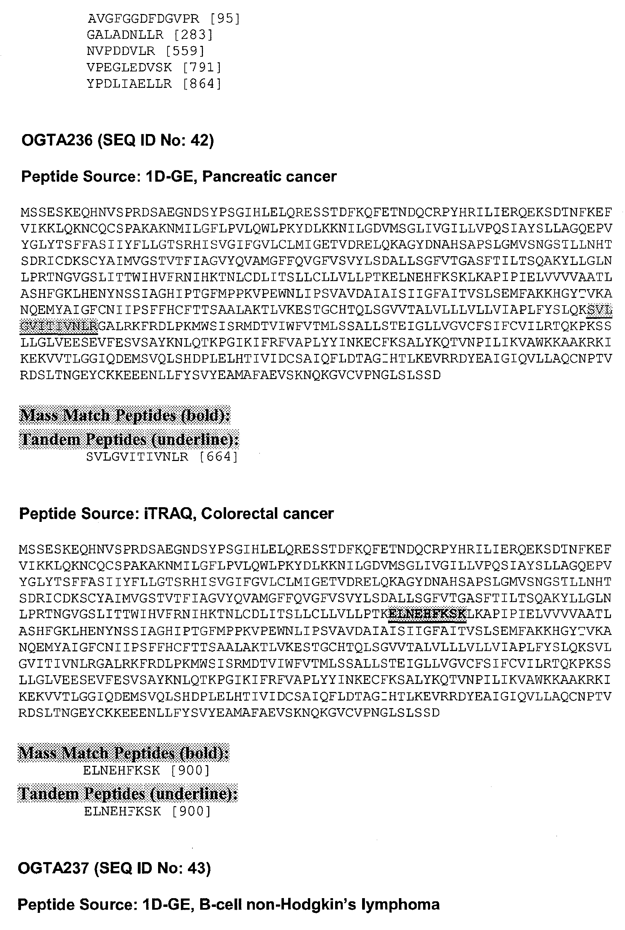

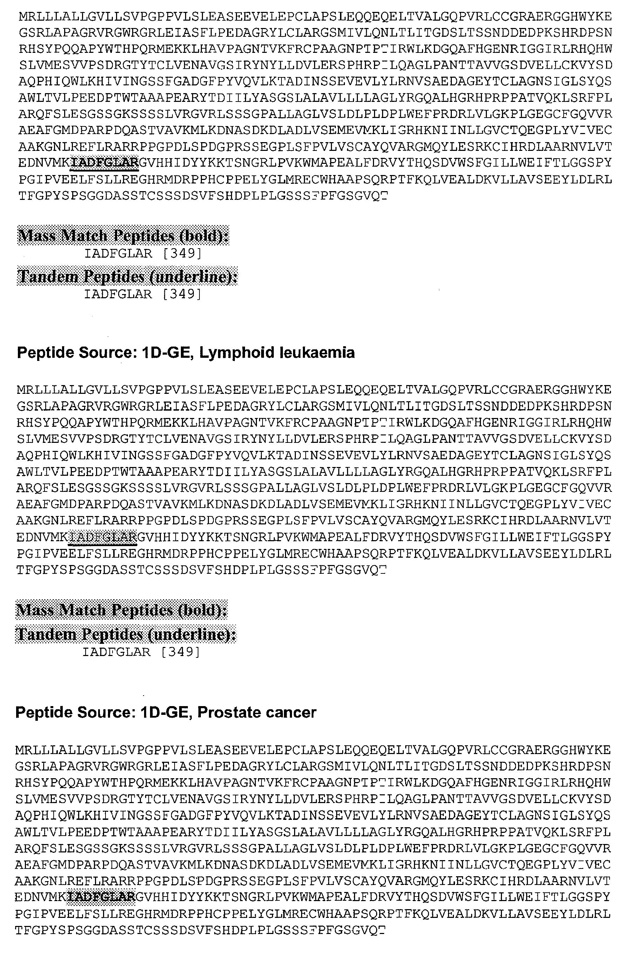

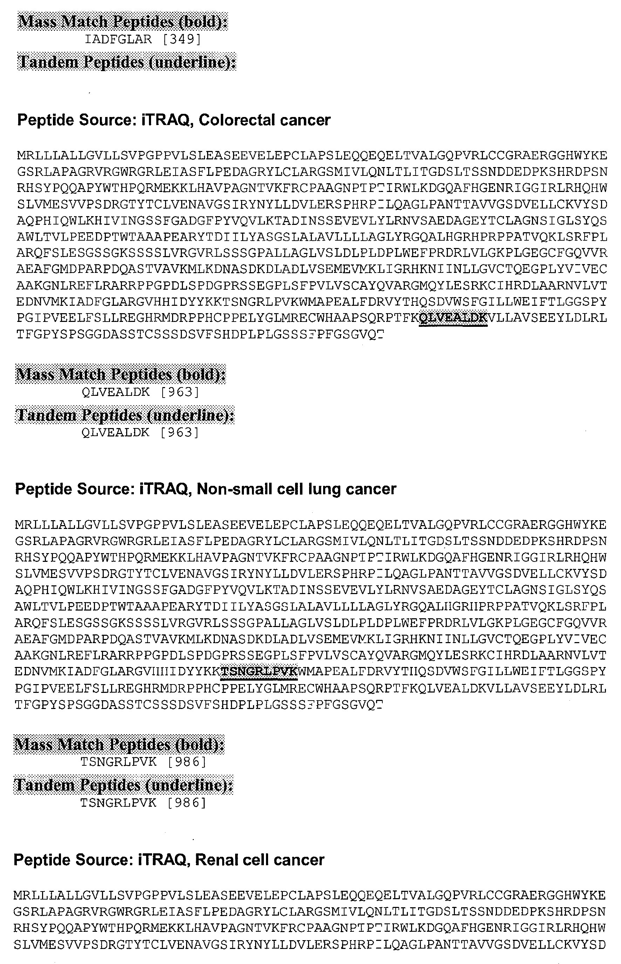

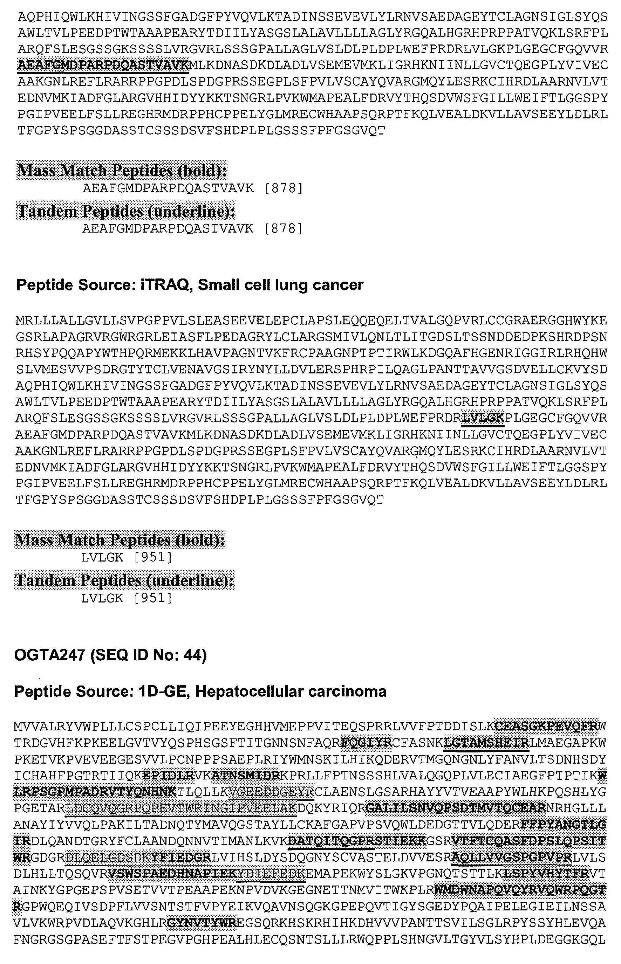

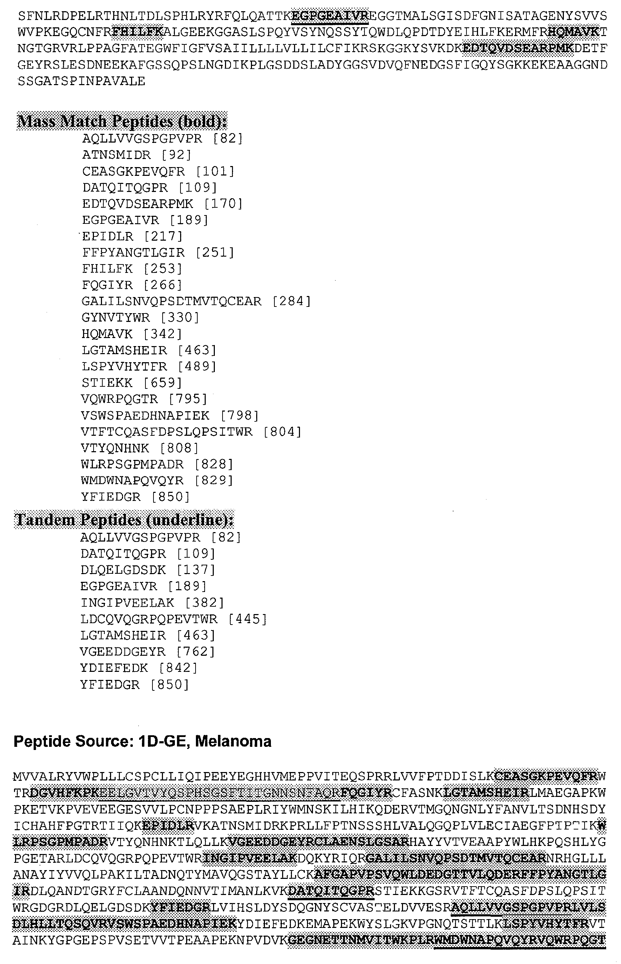

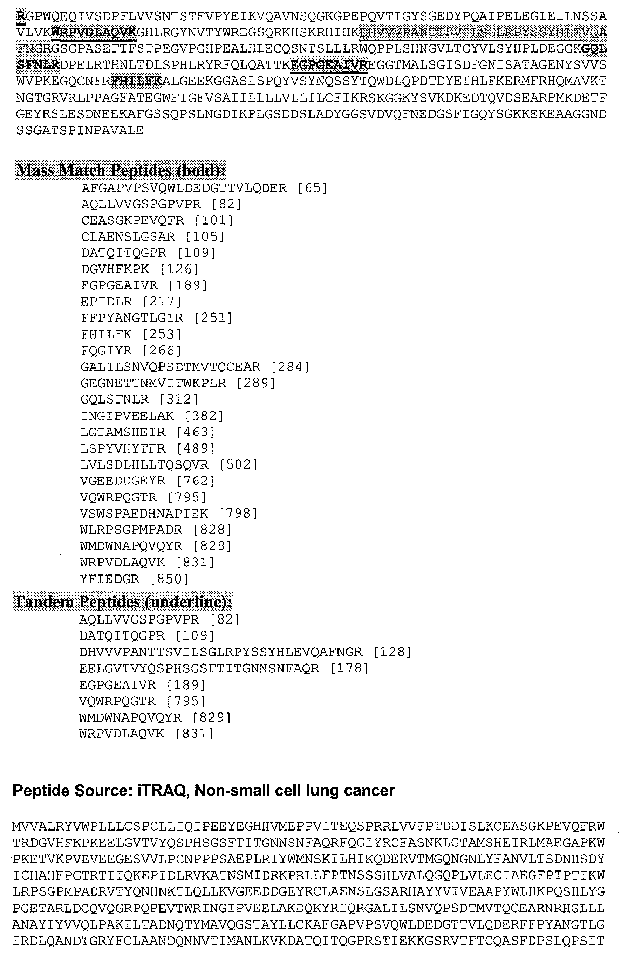

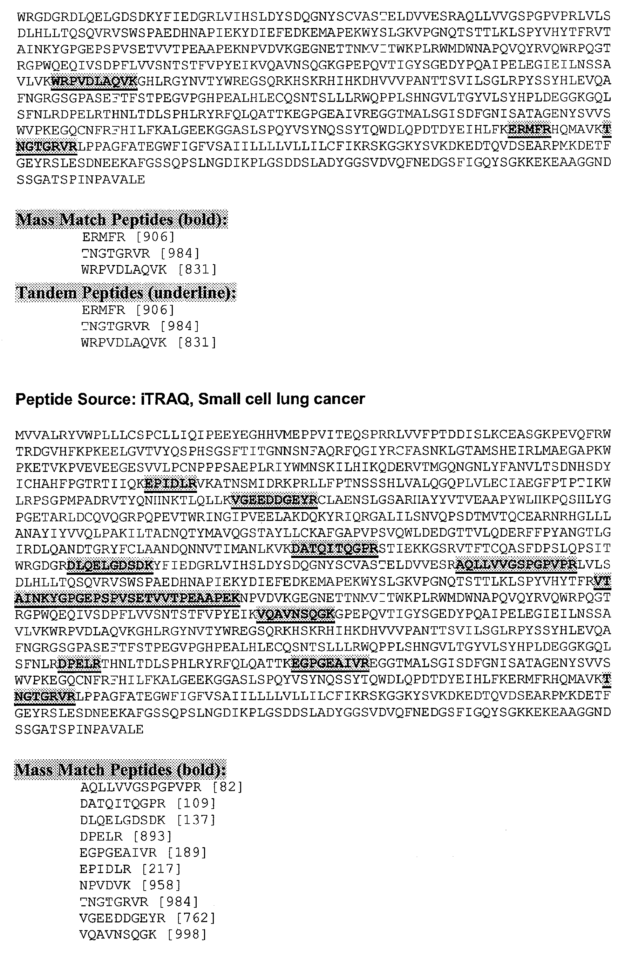

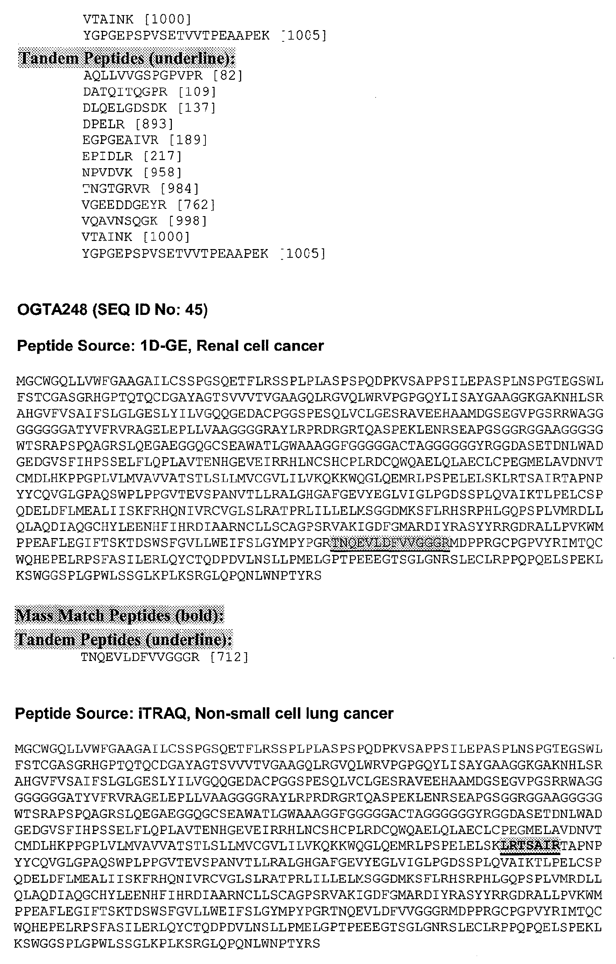

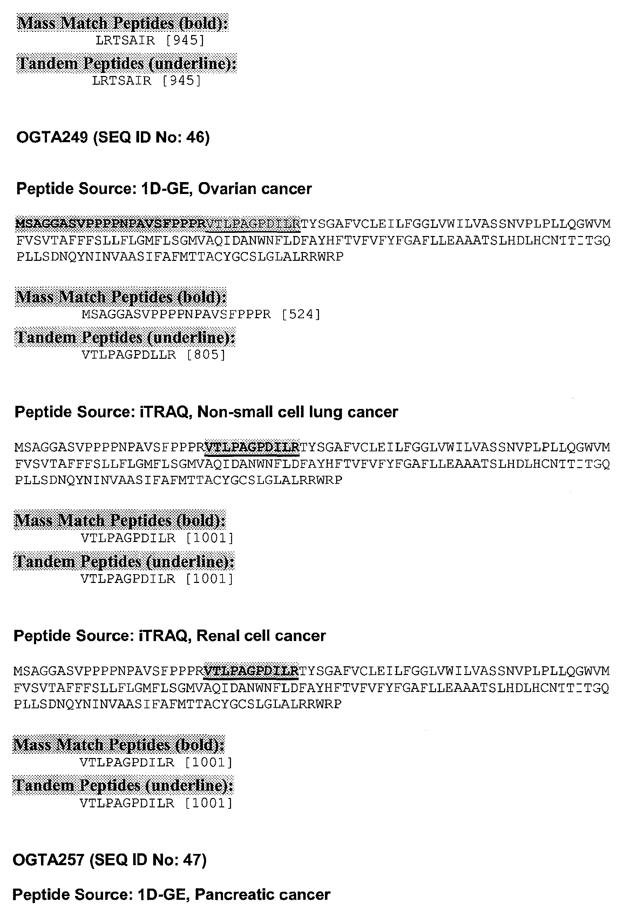

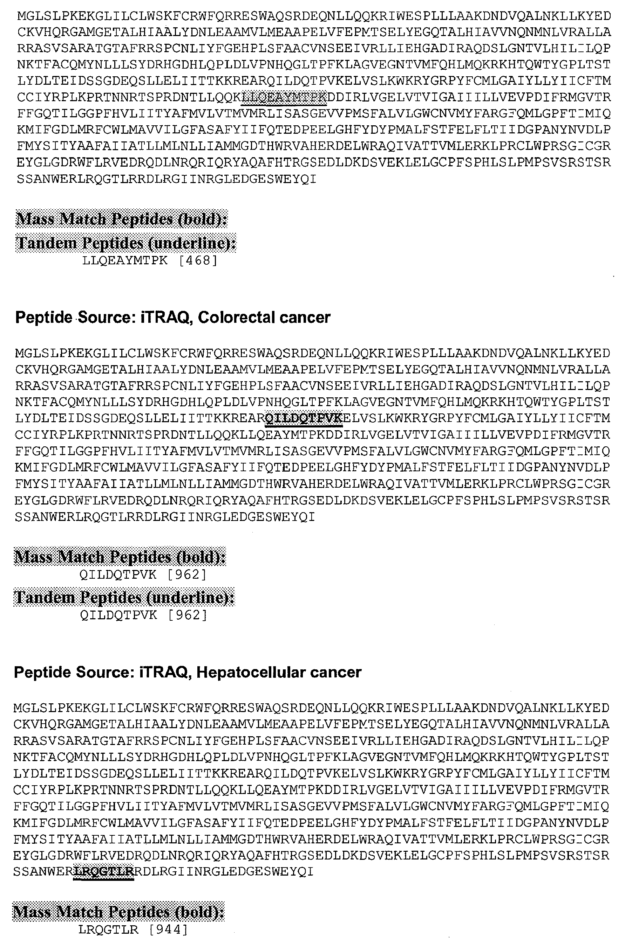

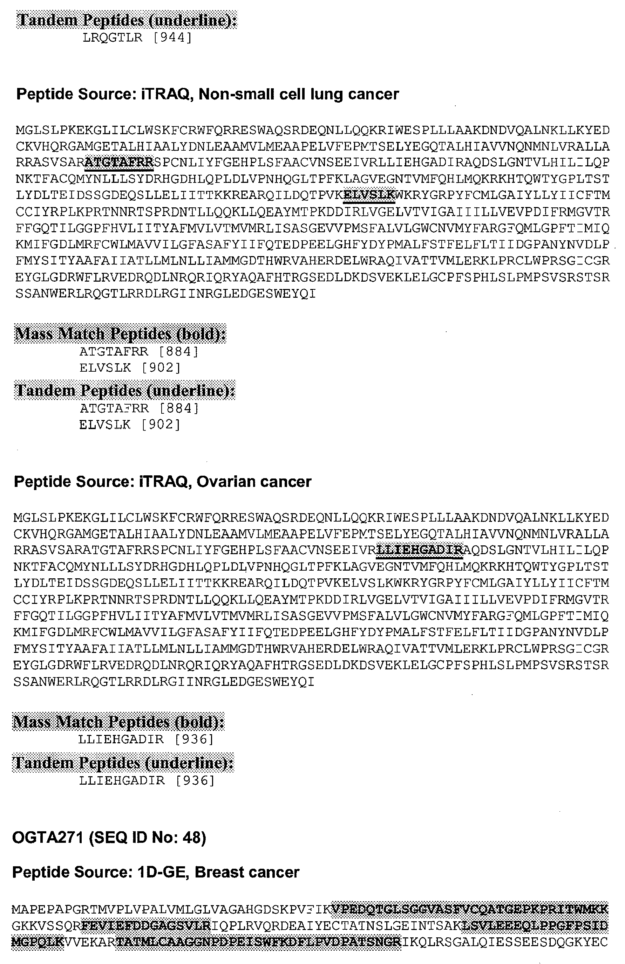

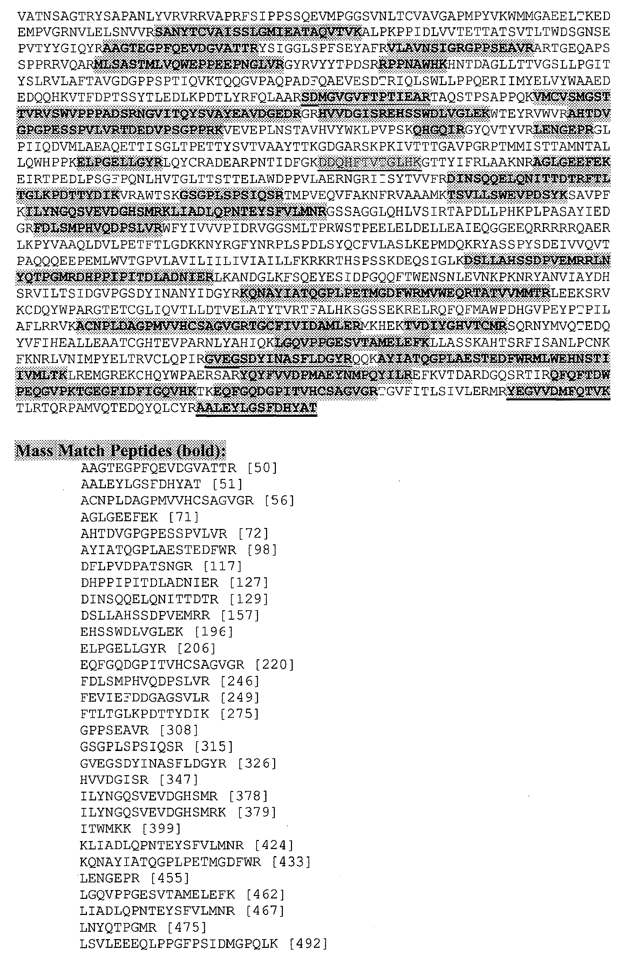

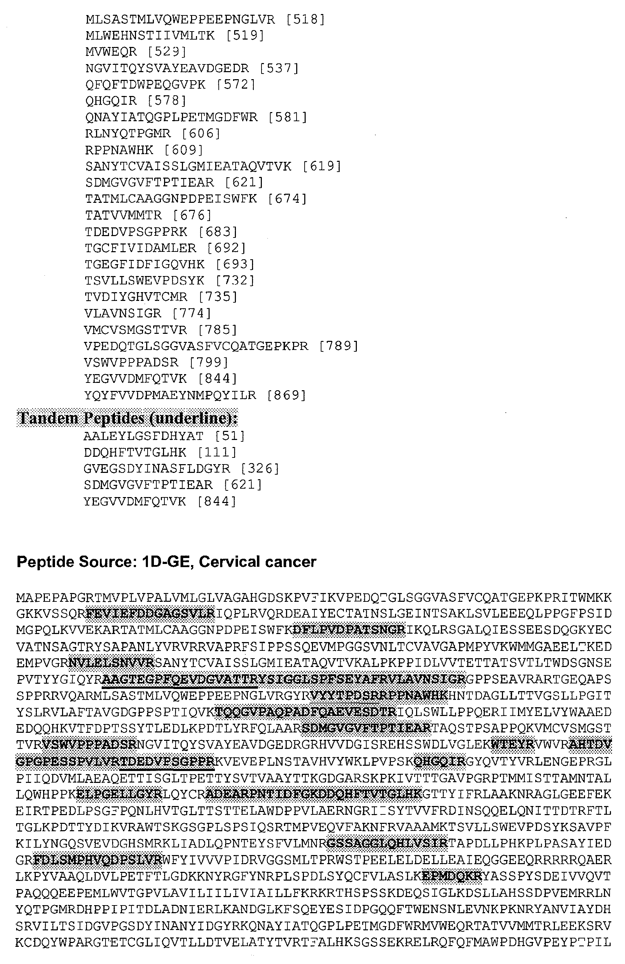

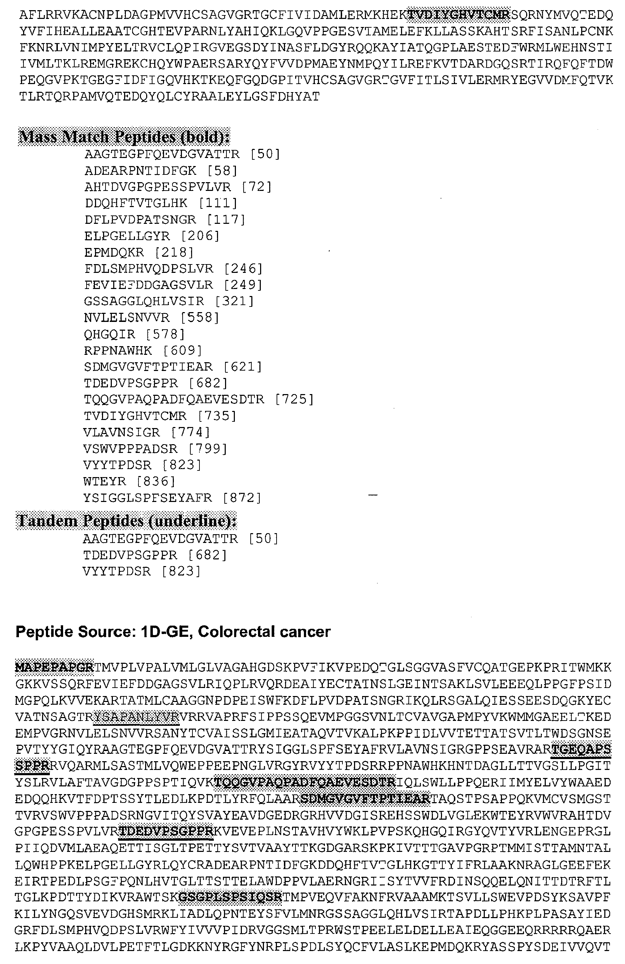

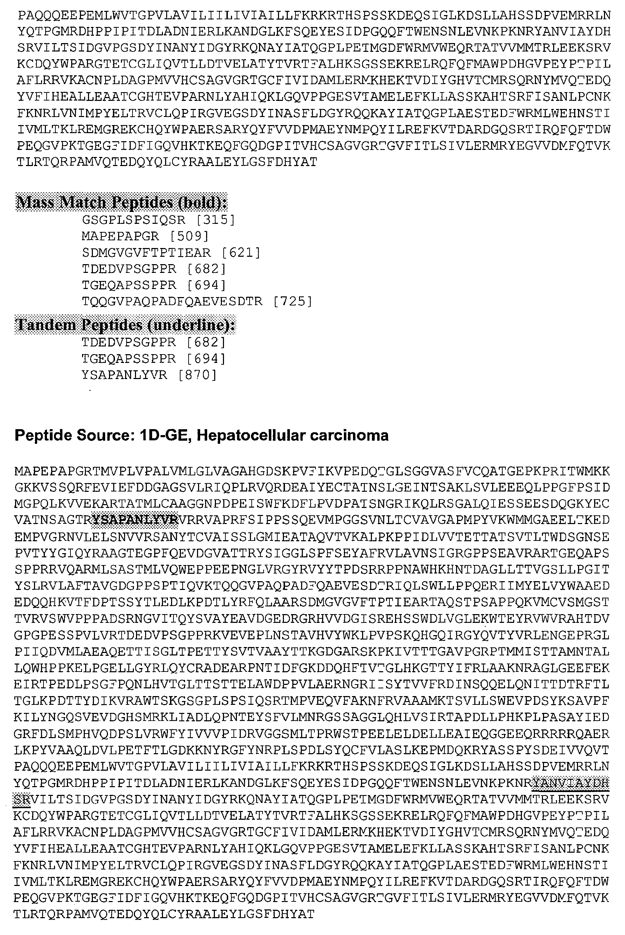

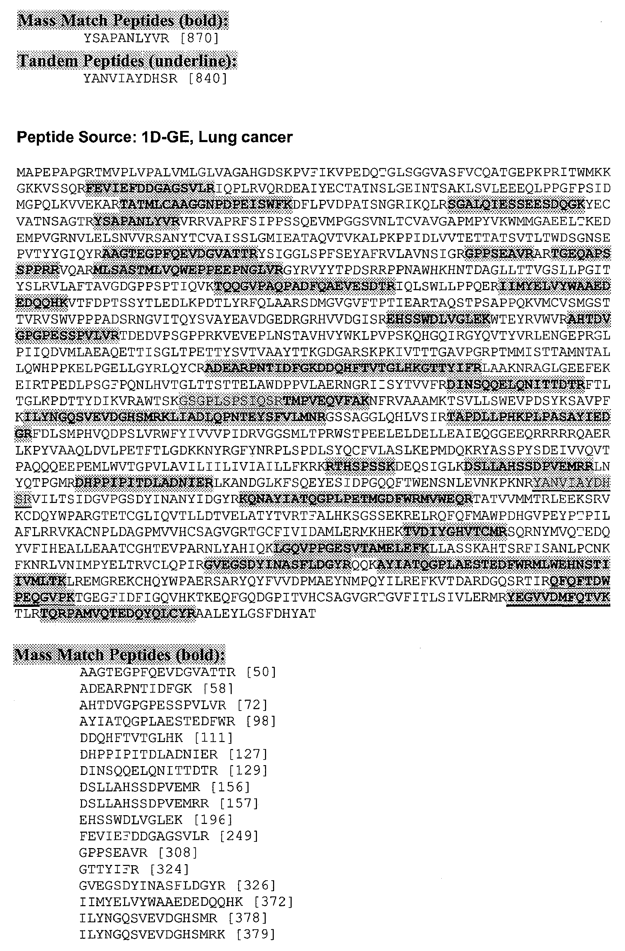

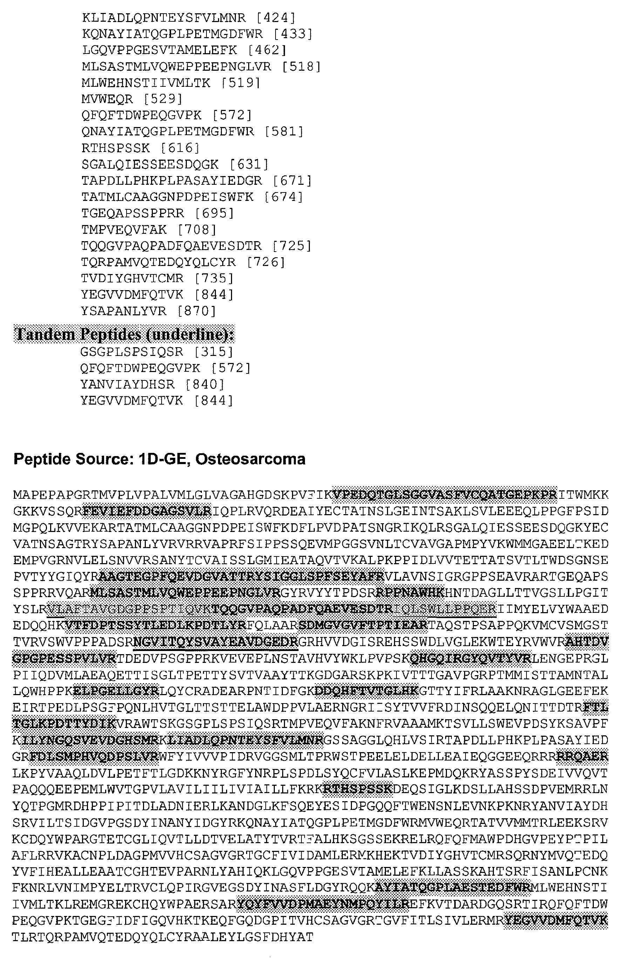

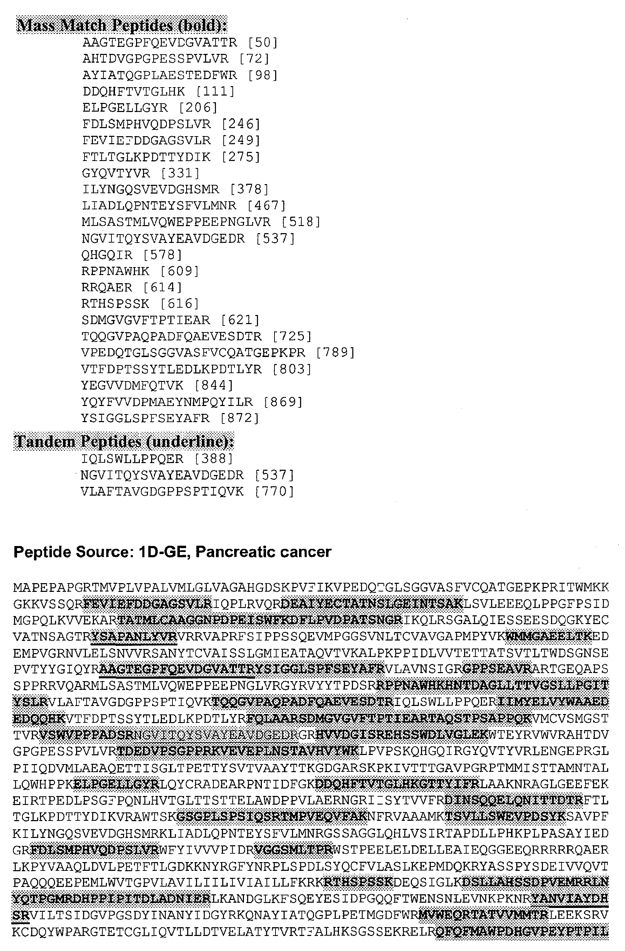

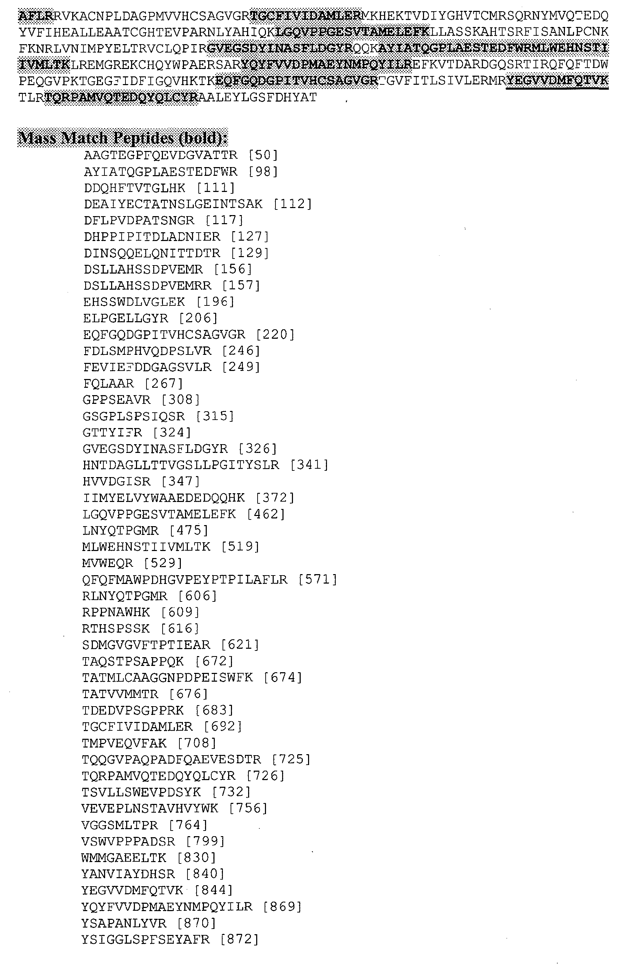

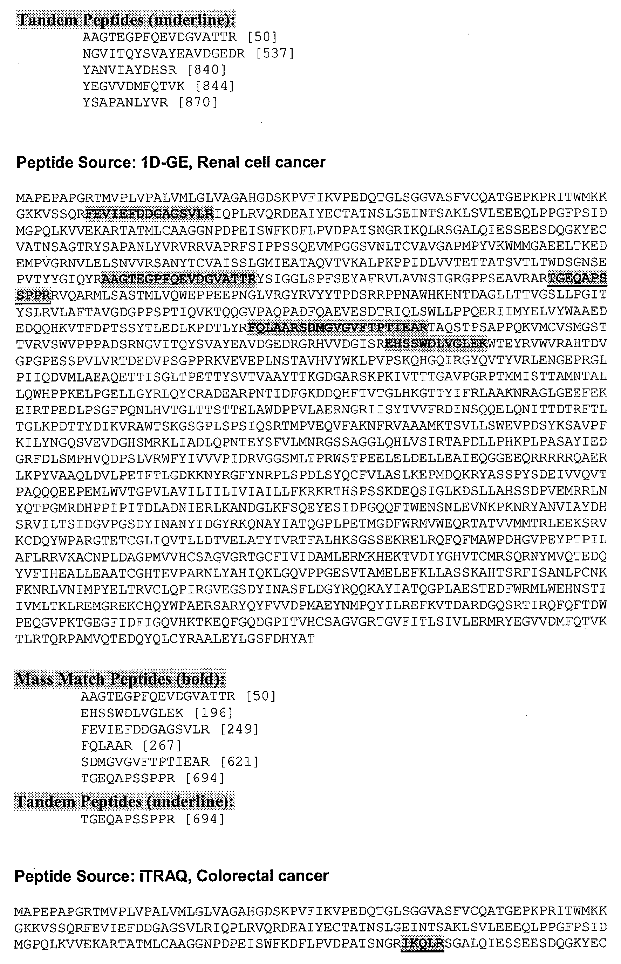

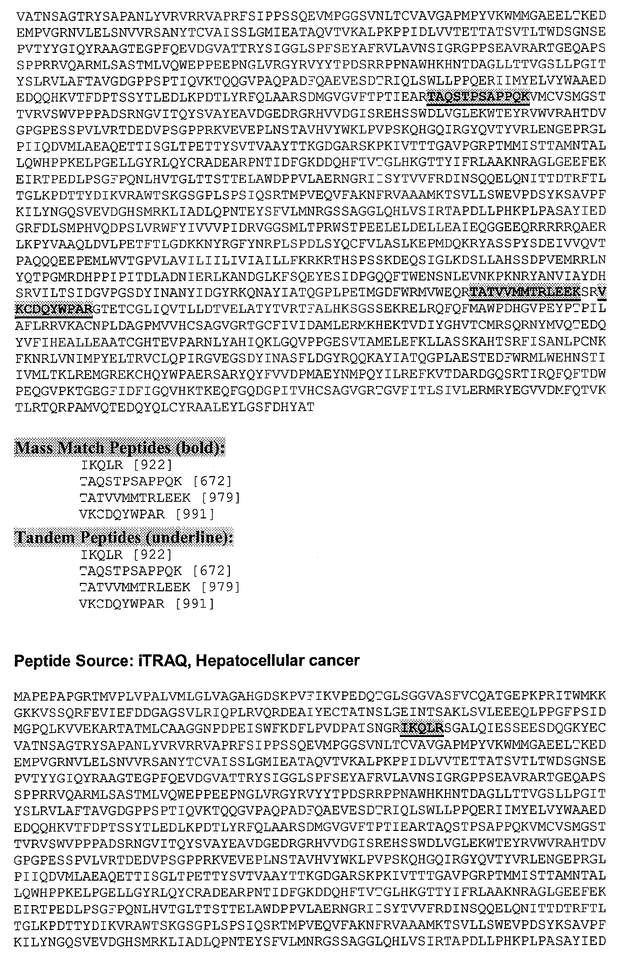

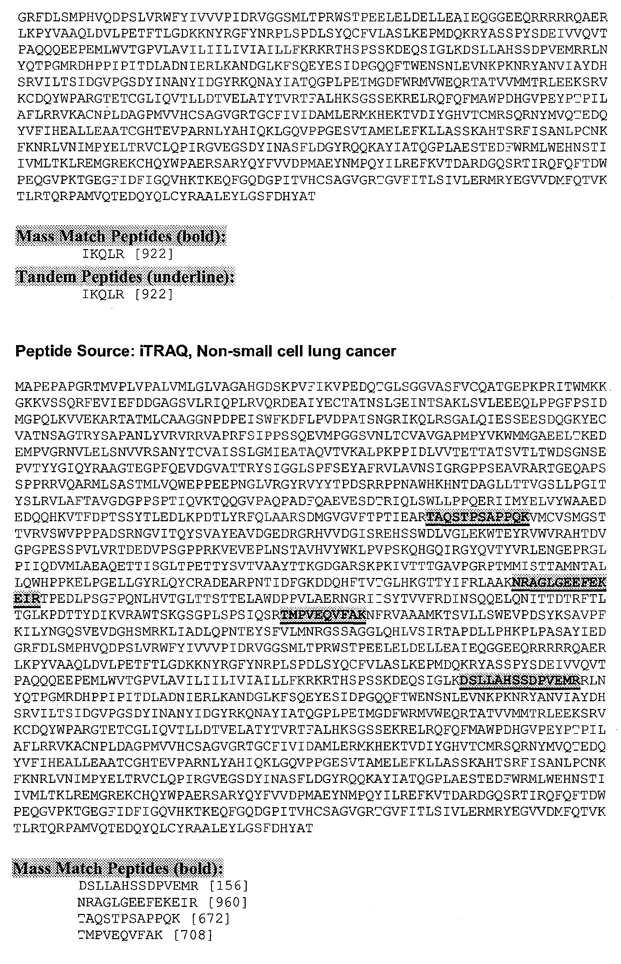

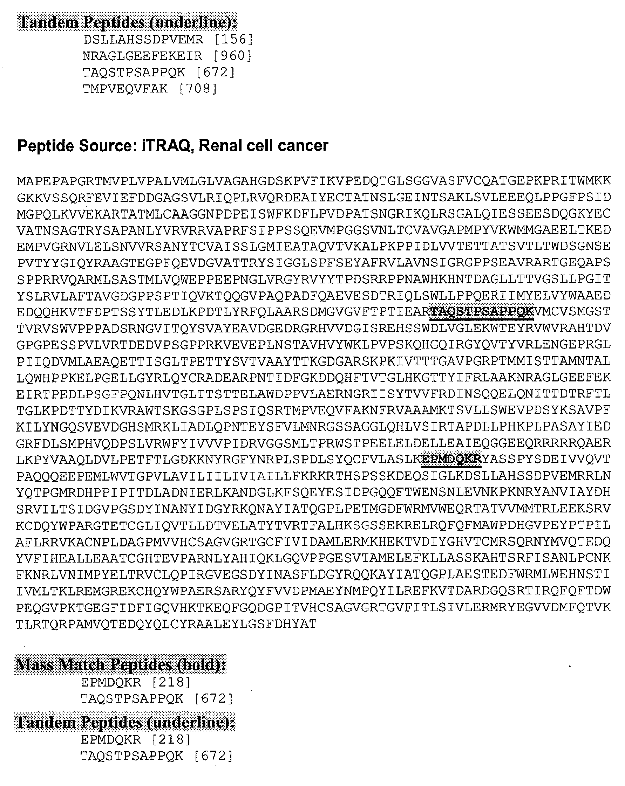

relates to OGTA 126. Other proteins disclosed herein relate to "OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and/or OGTA271"as illustrated in Figure 1 detected experimentally by 1D gel electrophoresis and iTRAQ analysis of lymphoid, breast, cervical, colorectal, gastric, brain, liver, lung, skin, neuronal, osteoblast, ovarian, pancreatic, prostate, kidney and/or eye tissue samples. These terms are used interchangeably in this specification.

OGTA002 has been identified in membrane protein extracts of breast, colorectal, liver, skin, pancreatic, lung and kidney tissue samples from breast cancer, colorectal cancer, heaptocellular carcinoma, melanoma, ovarian cancer, pancreatic cancer, lung cancer and kidney cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P54760, Ephrin type-B receptor 4, was identified.

Ephrin type-B receptor 4 is known to be abundantly expressed in placenta and in a range of primary tissues and malignant cell lines. It is expressed in fetal, but not adult, brain, and in primitive and myeloid, but not lymphoid, hematopoietic cells. It is a receptor for members of the ephrin-B family. It binds to ephrin-B2. It may have a role in events mediating differentiation and development.

OGTA009 has been identified in membrane protein extracts of breast, pancreatic, prostate, kidney, colorectal, lung and ovarian tissue samples from breast cancer, pancreatic cancer, prostate cancer, renal cell cancer, colorectal cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/),

and the following entry: 015551, Claudin-3, was identified.

Claudin-3 plays a major role in tight junction-specific obliteration of the intercellular space, through calcium-independent cell-adhesion activity.

OGTA016 has been identified in membrane protein extracts of colorectal and lung tissue samples from colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P01833, Polymeric-immunoglobulin receptor, was identified.

Polymeric-immunoglobulin receptor binds polymeric IgA and IgM at the basolateral surface of epithelial cells. The complex is then transported across the cell to be secreted at the apical surface. During this process a cleavage occurs that separates the extracellular (known as the secretory component) from the transmembrane segment.

OGTA028 has been identified in membrane protein extracts of ovarian, colorectal, kidney, liver and lung samples from ovarian cancer, colorectal cancer, kidney cancer, liver cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q5T7N2, FLJ10884, was identified.

OGTA037 has been identified in membrane protein extracts of gastric, colorectal, lung and ovarian tissue samples from gastric cancer, colorectal cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9HA72, Protein FAM26B, was identified.

OGTA041 has been identified in membrane protein extracts of liver, lung and pancreatic tissue samples from hepatocellular carcinoma, lung cancer and pancreatic cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P40189, Interleukin-6 receptor subunit beta, was identified.

Interleukin-6 receptor subunit beta is expressed in all the tissues and cell lines examined. Expression is not restricted to IL6 responsive cells. It is a signal-transducing molecule. The receptor systems for IL6, LIF, OSM, CNTF, IL11, CTF1 and BSF3 can utilize gp130 for initiating signal transmission. It binds to IL6/IL6R (alpha chain) complex, resulting in the formation of high-affinity IL6 binding sites, and transduces the signal. It does not bind IL6. It may have a role in embryonic development. The type I OSM receptor is capable of transducing OSM-specific signaling events.

OGTA053 has been identified in membrane protein extracts of pancreatic, colorectal, kidney, liver and lung tissue samples from pancreatic cancer, colorectal cancer, kidney cancer, liver cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/). and the following entry: Q9Y4D2, Sn1-specific diacylglycerol lipase alpha, was identified. Sn1-specific diacylglycerol lipase alpha is known to be highly expressed in the brain and pancreas. It catalyses the hydrolysis of diacylglycerol (DAG) to 2-arachidonoyl-glycerol (2-AG), the most abundant endocannabinoid in tissues. It is required for axonal growth during development and for retrograde synaptic signaling at mature synapses.

OGTA054 has been identified in membrane protein extracts of pancreatic, colorectal and lung tissue samples from pancreatic cancer, colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P13164, Interferon-induced transmembrane protein 1, was identified.

Interferon-induced transmembrane protein 1 is implicated in the control of cell growth. It is a component of a multimeric complex involved in the transduction of antiproliferative and homotypic adhesion signals.

OGTA066 has been identified in membrane protein extracts of colorectal, pancreatic, kidney and lung tissue samples from colorectal cancer, pancreatic cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q8TD06, Anterior gradient protein 3 homolog, was identified.

OGTA072 has been identified in membrane protein extracts of colorectal tissue samples from colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q16186, Adhesion-regulating molecule 1, was identified.

Adhesion-regulating molecule 1 promotes cell adhesion.

OGTA074 has been identified in membrane protein extracts of colorectal and lung tissue samples from colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 000508, Latent TGF-beta binding protein-4, was identified.

OGTA076 has been identified in membrane protein extracts of lymphoid, colorectal, pancreatic, kidney, liver, lung and ovarian tissue samples from chronic lymphocytic leukaemia, colorectal cancer, pancreatic cancer, kidney cancer, liver cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 060449, Lymphocyte antigen 75, was identified.

Lymphocyte antigen 75 is known to be predominantly expressed in the spleen, thymus, colon and peripheral blood lymphocytes. It is detected in myeloid and B lymphoid cell lines. It is also expressed in malignant Hodgkin's lymphoma cells called Hodgkin's and Reed-Sternberg (HRS) cells. It acts as an endocytic receptor to direct captured antigens from the extracellular space to a specialized antigen-processing compartment. It causes reduced proliferation of B-lymphocytes.

OGTA085 has been identified in membrane protein extracts of lung, skin, eye and colorectal tissue samples from lung cancer, melanoma, retinoblastoma and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q14318, 38 kDa FK506-binding protein homologue, was identified. 38 kDa FK506-binding protein homologue is known to be widely expressed, with the highest levels seen in the brain. It has no PPIase/rotamase activity.

OGTA087 has been identified in membrane protein extracts of lymphoid, breast, colorectal, lung, stomach and ovarian tissue samples from B-cell non-Hodgkin's lymphoma, breast cancer, colorectal cancer, gastric cancer, lung cancer, lymphoid leukaemia and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 094935, KIAA0851 protein, was identified.

OGTA088 has been identified in membrane protein extracts of breast, gastric, brain, liver, lung, lymphoid, pancreatic, kidney, eye and colorectal tissue samples from breast cancer, gastric cancer, glioblastoma, hepatocellular carcinoma, lung cancer, lymphoid leukaemia, pancreatic cancer, renal cell cancer, retinoblastoma and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 015126, Secretory carrier-associated membrane protein 1, was identified.

Secretory carrier-associated membrane protein 1 is known to be widely expressed, with the highest levels seen in the brain. It functions in post-Golgi recycling pathways. It acts as a recycling carrier to the cell surface.

OGTA089 has been identified in membrane protein extracts of breast, lung, ovarian and kidney tissue samples from breast cancer, lung cancer, ovarian cancer and kidney cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q5T225, Polycystic kidney disease 1-like, was identified.

OGTA091 has been identified in membrane protein extracts of breast, cervical, lymphoid, stomach, liver, lung, skin, bone, ovarian, pancreatic, prostate, kidney, eye and colorectal tissue samples from breast cancer, cervical cancer, chronic lymphocytic leukaemia, gastric cancer, hepatocellular carcinoma, lung cancer, melanoma, neuroblastoma, osteosarcoma, ovarian cancer, pancreatic cancer, prostate cancer, renal cell cancer, retinoblastoma and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 075923, Dysferlin, was identified.

Dysferlin is known to be highly expressed in skeletal muscle. It is also found in heart, placenta and at lower levels in liver, lung, kidney and pancreas.

OGTA098 has been identified in membrane protein extracts of breast, cervical, liver, lung, bone, pancreatic, prostate, kidney, colorectal and ovarian tissue samples from breast cancer, cervical cancer, hepatocellular carcinoma, lung cancer, osteosarcoma, pancreatic cancer, prostate cancer, renal cell cancer, colorectal cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q14126, Desmoglein-2, was identified.

Desmoglein-2 is found in all of the tissues tested and carcinomas. It is a component of intercellular desmosome junctions. It is involved in the interaction of plaque proteins and intermediate filaments mediating cell-cell adhesion.

OGTA101 has been identified in membrane protein extracts of breast, cervical, colorectal, liver, lung, bone, pancreatic, kidney and ovarian tissue samples from breast cancer, cervical cancer, colorectal cancer, gastric cancer, hepatocellular carcinoma, lung cancer, osteosarcoma, pancreatic cancer, kidney cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P23468, Receptor-type tyrosine-protein phosphatase delta, was identified.

OGTA104 has been identified in membrane protein extracts of breast, liver, ovarian, pancreatic, colorectal, kidney and lung tissue samples from breast cancer, hepatocellular carcinoma, ovarian cancer, pancreatic cancer, colorectal cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q13443, ADAM 9, was identified.

ADAM 9 is known to be widely expressed. It is expressed in chondrocytes, liver and heart. It is a probable zinc protease. It may mediate cell-cell or cell-matrix interactions. It displays alpha-secretase activity for APP.

OGTA106 has been identified in membrane protein extracts of cervical, skin, pancreatic and liver tissue samples from cervical cancer, melanoma, pancreatic cancer and liver cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q99466, Neurogenic locus notch homologue protein 4, was identified.

Neurogenic locus notch homologue protein 4 is known to be highly expressed in the heart, moderately expressed in the lung and placenta and at low levels in the liver, skeletal muscle, kidney, pancreas, spleen, lymph node, thymus, bone marrow and fetal liver. No expression was seen in adult brain or peripheral blood leukocytes. It functions as a receptor for membrane-bound ligands Jagged1, Jagged2 and Delta1 to regulate cell-fate determination. Upon ligand activation through the released notch intracellular domain (NICD) it forms a transcriptional activator complex with RBP-J kappa and activates genes of the enhancer of split locus. It affects the implementation of differentiation, proliferation and apoptotic programs. It may regulate branching morphogenesis in the developing vascular system.

OGTA112 has been identified in membrane protein extracts of breast, cervical, lymphoid, liver, pancreatic and kidney tissue samples from breast cancer, cervical cancer, chronic lymphocytic leukaemia, hepatocellular carcinoma, pancreatic cancer and renal cell cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P48594, Serpin B4, was identified.

Serpin B4 is known to be predominantly expressed in squamous cells. It may act as a protease inhibitor to modulate the host immune response against tumour cells.

OGTA113 has been identified in membrane protein extracts of pancreatic, colorectal, kidney, lung and ovarian tissue samples from pancreatic cancer, colorectal cancer, kidney cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/). and the following entry: Q9BTV4, Transmembrane protein 43, was identified.

OGTA119 has been identified in membrane protein extracts of lymphoid, breast, colorectal, stomach, liver, lung, brain, bone, pancreatic, prostate, kidney and ovarian tissue samples from B-cell non-Hodgkin's lymphoma, breast cancer, colorectal cancer, gastric cancer, hepatocellular carcinoma, lung cancer, lymphoid leukaemia, neuroblastoma, osteosarcoma, pancreatic cancer, prostate cancer, renal cell cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q6UKI4, KIAA1228 protein, was identified.

OGTA124 has been identified in membrane protein extracts of liver, lung, ovarian, pancreatic, kidney and colorectal tissue samples from hepatocellular carcinoma, lung cancer, ovarian cancer, pancreatic cancer, renal cell cancer and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9UP95, Solute carrier family 12 member 4, was identified.

Solute carrier family 12 member 4 is known to be ubiquitous. Levels are much higher in erythrocytes from patients with Hb SC and Hb SS compared to normal AA erythrocytes. This may contribute to red blood cell dehydration and to the manifestation of sickle cell disease by increasing the intracellular concentration of HbS. It mediates electroneutral potassium-chloride cotransport when activated by cell swelling. It may contribute to cell volume homeostasis in single cells. It may be involved in the regulation of basolateral Cl(-) exit in NaCl absorbing epithelia.

OGTA126 has been identified in membrane protein extracts of breast, colorectal, liver, skin, pancreatic, prostate, kidney and lung tissue samples from breast cancer, colorectal cancer, hepatocellular carcinoma, melanoma, pancreatic cancer, prostate cancer, renal cell cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9H5V8, CUB domain-containing protein 1, was identified.

CUB domain-containing protein 1 is known to be highly expressed in mitotic cells with low expression during interphase. It is detected at highest levels in skeletal muscle and colon with lower levels in kidney, small intestine, placenta and lung. It is expressed in a number of human tumour cell lines as well as in colorectal cancer, breast carcinoma and lung cancer. It is also expressed in cells with phenotypes reminiscent of mesenchymal stem cells and neural stem cells. It may be involved in cell adhesion and cell matrix association. It may play a role in the regulation of anchorage versus migration or proliferation versus differentiation via its phosphorylation. It may be a novel marker for leukaemia diagnosis and for immature hematopoietic stem cell subsets. It belongs to the tetraspanin web involved in tumour progression and metastasis.

OGTA156 has been identified in membrane protein extracts of lymphoid, liver, colorectal, kidney, lung and ovarian tissue samples from acute T-cell leukaemia, B-cell non-Hodgkin's lymphoma, chronic lymphocytic leukaemia, hepatocellular carcinoma, colorectal cancer, kidney cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P13612, Integrin alpha-4, was identified. Integrins alpha-4/beta-1 (VLA-4) and alpha-4/beta-7 are receptors for fibronectin. They recognize one or more domains within the alternatively spliced CS-1 and CS-5 regions of fibronectin. They are also receptors for VCAM1. Integrin alpha-4/beta-1 recognizes the sequence Q-I-D-S in VCAM1. Integrin alpha-4/beta-7 is also a receptor for MADCAM1. It recognizes the sequence L-D-T in MADCAM1. On activated endothelial cells integrin VLA-4 triggers homotypic aggregation for most VLA-4-positive leukocyte cell lines. It may also participate in cytolytic T-cell interactions with target cells.

OGTA159 has been identified in membrane protein extracts of breast, lymphoid, colorectal, liver, skin, pancreatic, eye, kidney and lung tissue samples from breast cancer, chronic lymphocytic leukaemia, colorectal cancer, hepatocellular carcinoma, melanoma, pancreatic cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q96HY6, Uncharacterized protein C20orf116, was identified.

OGTA168 has been identified in membrane protein extracts of brain and skin tissue samples from glioblastoma and melanoma patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q8WZA4, Collectin placenta 1, was identified.

OGTA169 has been identified in membrane protein extracts of breast, lymphoid, colorectal, kidney and lung tissue samples from breast cancer, chronic lymphocytic leukaemia, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/). and the following entry: P20702, Integrin alpha-X, was identified.

Integrin alpha-X is known to be predominantly expressed in monocytes and granulocytes. Integrin alpha-X/beta-2 is a receptor for fibrinogen. It recognizes the sequence G-P-R in fibrinogen. It mediates cell-cell interaction during inflammatory responses. It is especially important in monocyte adhesion and chemotaxis.

OGTA174 has been identified in membrane protein extracts of lymphoid and lung tissue samples from acute T-cell leukaemia, chronic lymphocytic leukaemia and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P06127, T-cell surface glycoprotein CD5, was identified.

T-cell surface glycoprotein CD5 may act as a receptor in regulating T-cell proliferation. CD5 interacts with CD72/LYB-2.

OGTA176 has been identified in membrane protein extracts of lymphoid, colorectal, kidney and lung tissue samples from B-cell non-Hodgkin's lymphoma, chronic lymphocytic leukaemia, colorectal cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P21854, B-cell differentiation antigen CD72 homolog, was identified.

B-cell differentiation antigen CD72 is known to be predominantly expressed in pre-B-cells and B-cells but not terminally differentiated plasma cells. It plays a role in B-cell proliferation and differentiation. It associates with CD5.

OGTA177 has been identified in membrane protein extracts of lymphoid, colorectal, kidney, lung and ovarian tissue samples from chronic lymphocytic leukaemia, colorectal cancer, kidney cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P49961, Ectonucleoside triphosphate diphosphohydrolase 1, was identified.

Ectonucleoside triphosphate diphosphohydrolase 1 is known to be expressed primarily on activated lymphoid cells. It is also expressed in endothelial tissues. It is present in both placenta and umbilical vein. In the nervous system, it could hydrolyze ATP and other nucleotides to regulate purinergic neurotransmission. It could also be implicated in the prevention of platelet aggregation. It hydrolyses ATP and ADP equally well.

OGTA197 has been identified in membrane protein extracts of colorectal, liver, lung, skin, bone, ovarian, pancreatic, prostate and kidney tissue samples from colorectal cancer, hepatocellular carcinoma, lung cancer, melanoma, neuroblastoma, osteosarcoma, ovarian cancer, pancreatic cancer, prostate cancer, renal cell cancer and retinoblastoma patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P29317, Ephrin type-A receptor 2, was identified.

Ephrin type-A receptor 2 is known to be expressed most highly in tissues that contain a high proportion of epithelial cells e.g. skin, intestine, lung, and ovary. It is a receptor for members of the ephrin-A family. It binds to ephrin-A1, -A3, -A4 and -A5.

OGTA202 has been identified in membrane protein extracts of colorectal and lung tissue samples from colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P22223, Cadherin-3, was identified. Cadherin-3 is known to be expressed in some normal epithelial tissues and in some carcinoma cell lines. Cadherins are calcium dependent cell adhesion proteins. They preferentially interact with themselves in a homophilic manner in connecting cells; cadherins may thus contribute to the sorting of heterogeneous cell types.

OGTA203 has been identified in membrane protein extracts of ovarian, kidney and lung tissue samples from ovarian cancer, renal cell cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P55285, Cadherin-6, was identified.

Cadherin-6 is known to be highly expressed in brain, cerebellum, and kidney. Lung, pancreas, and gastric mucosa show a weak expression. It is also expressed in certain liver and kidney carcinomas. Cadherins are calcium dependent cell adhesion proteins. They preferentially interact with themselves in a homophilic manner in connecting cells; cadherins may thus contribute to the sorting of heterogeneous cell types.

OGTA206 has been identified in membrane protein extracts of breast, pancreatic, prostate, colorectal and lung tissue samples from breast cancer, pancreatic cancer, prostate cancer, colorectal cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: 014493, Claudin-4, was identified.

Claudin-4 plays a major role in tight junction-specific obliteration of the intercellular space.

OGTA213 has been identified in membrane protein extracts of lung and colorectal tissue samples from lung cancer and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q14767, Latent-transforming growth factor beta-binding protein 2, was identified.

Latent-transforming growth factor beta-binding protein 2 is known to be expressed in lung and weakly expressed in heart, placenta, liver and skeletal muscle. It may play an integral structural role in elastic-fiber architectural organization and/or assembly.

OGTA214 has been identified in membrane protein extracts of ovarian and colorectal tissue samples from ovarian cancer and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9H3R2, Mucin-13, was identified. Mucin-13 is known to be highly expressed in epithelial tissues, particularly those of the gastrointestinal and respiratory tracts, such as large intestine and trachea, followed by kidney, small intestine, appendix and stomach. It is an epithelial and hemopoietic transmembrane mucin that may play a role in cell signaling.

OGTA216 has been identified in membrane protein extracts of breast, pancreatic, colorectal, kidney, liver, lung and ovarian tissue samples from breast cancer, pancreatic cancer, colorectal cancer, kidney cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P55011, Solute carrier family 12 member 2, was identified.

Solute carrier family 12 member 2 is known to be expressed in many tissues. It is an electrically silent transporter system. It mediates sodium and chloride reabsorption. It plays a vital role in the regulation of ionic balance and cell volume.

OGTA222 has been identified in membrane protein extracts of lymphoid and colorectal tissue samples from B-cell non-Hodgkin's lymphoma and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P16444, Dipeptidase 1, was identified.

Dipeptidase 1 hydrolyses a wide range of dipeptides. It is implicated in the renal metabolism of glutathione and its conjugates. It converts leukotriene D4 to leukotriene E4; it may play an important role in the regulation of leukotriene activity.

OGTA236 has been identified in membrane protein extracts of pancreatic and colorectal tissue samples from pancreatic cancer and colorectal cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P50443, Sulfate transporter, was identified. Sulfate transporter is known to be ubiquitously expressed. It is a sulfate transporter. It may play a role in endochondral bone formation.

OGTA237 has been identified in membrane protein extracts of lymphoid, prostate, colorectal, kidney and lung tissue samples from B-cell non-Hodgkin's lymphoma, lymphoid leukaemia, prostate cancer, colorectal cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P22455, Fibroblast growth factor receptor 4, was identified.

Fibroblast growth factor receptor 4 is a receptor for acidic fibroblast growth factor. It does not bind to basic fibroblast growth factor. It binds FGF19.

OGTA247 has been identified in membrane protein extracts of liver, skin and lung tissue samples from hepatocellular carcinoma, melanoma and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P32004, Neural cell adhesion molecule L1, was identified.

Neural cell adhesion molecule L1 is a cell adhesion molecule with an important role in the development of the nervous system. It is involved in neuron-neuron adhesion, neurite fasciculation, outgrowth of neurites, etc. It binds to axonin on neurons.

OGTA248 has been identified in membrane protein extracts of kidney and lung tissue samples from renal cell cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: P29376, Leukocyte tyrosine kinase receptor, was identified.

Leukocyte tyrosine kinase receptor is known to be expressed in non-hematopoietic cell lines and T- and B-cell lines. The exact function of this protein is not known. It is probably a receptor with a tyrosine-protein kinase activity.

OGTA249 has been identified in membrane protein extracts of ovarian, kidney and lung tissue samples from ovarian cancer, kidney cancer and lung cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q969L2, Protein MAL2, was identified.

Protein MAL2 is known to be predominantly expressed in kidney, lung, and liver. It is also found in thyroid gland, stomach and at lower levels in testis and small intestine. It is a member of the machinery of polarized transport. It is required for the indirect transcytotic route at the step of the egress of the transcytosing cargo from perinuclear endosomes in order for it to travel to the apical surface via a raft-dependent pathway.

OGTA257 has been identified in membrane protein extracts of pancreatic, colorectal, liver, lung and ovarian tissue samples from pancreatic cancer, colorectal cancer, liver cancer, lung cancer and ovarian cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q9H1D0, Transient receptor potential cation channel subfamily V member 6, was identified.

Transient receptor potential cation channel subfamily V member 6 is known to be expressed at high levels in the gastrointestinal tract, including esophagus, stomach, duodenum, jejunum, ileum and colon, and in pancreas, placenta, prostate and salivary gland. It is expressed at moderate levels in liver, kidney and testis. It is expressed in locally advanced prostate cancer, metastatic and androgen-insensitive prostatic lesions but not detected in healthy prostate tissue and benign prostatic hyperplasia. It is a calcium selective cation channel probably involved in Ca(2+) uptake in various tissues, including Ca(2+) reabsorption in intestine. The channel is activated by low internal calcium level, probably including intracellular calcium store depletion, and the current exhibits an inward rectification. Inactivation includes both a rapid Ca(2+)-dependent and a slower Ca(2+)-calmodulin-dependent mechanism, the latter may be regulated by phosphorylation. In vitro, is slowly inhibited by Mg(2+) in a voltage-independent manner. Heteromeric assembly with TRPV5 seems to modify channel properties. TRPV5-TRPV6 heteromultimeric concatemers exhibit voltage-dependent gating.

OGTA271 has been identified in membrane protein extracts of breast, cervical, colorectal, liver, lung, osteoblast, pancreatic and kidney tissue samples from breast cancer, cervical cancer, colorectal cancer, hepatocellular carcinoma, lung cancer, osteosarcoma, pancreatic cancer and renal cell cancer patients, through the methods and apparatus of the Preferred Technologies described in Examples 1 and 2 (1D gel electrophoresis or iTRAQ together with tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at http://www.expasy.com/), and the following entry: Q5T021, Protein tyrosine phosphatase, receptor type, F, was identified.

Proteins of the disclosure are useful as are fragments e.g. antigenic or immunogenic fragments thereof and derivatives thereof. Epitope containing fragments including antigenic or immunogenic fragments will typically be of length 12 amino acids or more e.g. 20 amino acids or more e.g. 50 or 100 amino acids or more. Fragments may be 95% or more of the length of the full protein e.g. 90% or more e.g. 75% or 50% or 25% or 10% or more of the length of the full protein.

Eptiope containing fragments including antigenic or immunogenic fragments will be capable of eliciting a relevant immune response in a patient. DNA encoding proteins of the disclosure is also useful as are fragments thereof e.g. DNA encoding fragments of proteins of the disclosure such as immunogenic fragments thereof. Fragments of nucleic acid (e.g. DNA) encoding Proteins of the disclosure may be 95% or more of the length of the full coding region e.g. 90% or more e.g. 75% or 50% or 25% or 10% or more of the length of the full coding region. Fragments of nucleic acid (e.g. DNA) may be 36 nucleotides or more e.g. 60 nucleotides or more e.g. 150 or 300 nucleotides or more in length.

Derivatives of proteins of the disclosure include variants on the sequence in which one or more (e.g. 1-20 such as 15, or up to 20% such as up to 10% or 5% or 1% by number based on the total length of the protein) deletions, insertions or substitutions have been made. Substitutions may typically be conservative substitutions. Derivatives of proteins of the disclosure include variants on the sequence which have a sequence identity over the entire length thereof of typically at least 60%, 70%, 80%, 90 or 95%. Derivatives will typically have essentially the same biological function as the protein from which they are derived. Derivatives will typically be comparably antigenic or immunogenic to the protein from which they are derived. Derivatives will typically have either the ligand-binding activity, or the active receptor-complex forming ability, or preferably both, of the protein from which they are derived.

Tables 1-48 below illustrate the different occurrences of OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 as detected by ID gel electrophoresis and mass spectrometry of membrane protein extracts of lymphoid, breast, cervical, colorectal, gastric, brain, liver, lung, skin, neuronal, osteoblast, ovarian, pancreatic, prostate, kidney and eye tissue samples from relevant cancer patients. The first column provides the molecular weight, the second column gives information on the sub fractionation protocol used, if any (see Example 1 below), and the last column provides a list of the sequences observed by mass spectrometry and the corresponding SEQ ID Nos.

[0149] Tables 49-93 illustrate the different occurrences of OGTA002, OGTA009, OGTA016, OGTA028, OGTA037, OGTA041, OGTA053, OGTA054, OGTA066, OGTA074, OGTA076, OGTA085, OGTA088, OGTA089, OGTA091, OGTA098, OGTA101, OGTA104, OGTA106, OGTA112, OGTA113, OGTA119, OGTA124, OGTA126, OGTA156, OGTA159, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 as detected by iTRAQ and mass spectrometry of membrane protein extracts of colorectal, kidney, liver, lung and ovarian tissue samples from colorectal cancer, kidney cancer, liver cancer, lung cancer and ovarian cancer patients respectively. The first column provides the samples batch number, the second column gives the iTRAQ experiment number and the last column provides a list of the sequences observed by mass spectrometry and the corresponding SEQ ID Nos.

**OGTA002**

Table 1a - Breast cancer

| MW (Da) | Subfractionation | Tryptics identified [SEQ ID No] |
|---|---|---|
| 88475 | | LNGSSLHLEWSAPLESGGR [473], QPHYSAFGSVGEWLR [585], SQAKPGTPGGTGGPAPQY [646], VDTVAAEHLTR [749] |

Table 1b - Colorectal cancer

| MW (Da) | Subfractionation | Tryptics identified [SEQ ID No] |
|---|---|---|
| | Heparin Binding | DLVEPWVVVR [141], EFLSEASIMGQFEHPNIIR [184], EVPPAVSDIR [237], LETADLK [456], VDTVAAEHLTR [749] |
| 46639 | Nucleotide Binding | FPQVVSALDK [263], ILASVQHMK [373], VYIDPFTYEDPNEAVR [818], WTAPEAIAFR [833], YLAEMSYVHR [859] |
| 119837 | Nucleotide Binding | FPQVVSALDK [263], GAPCTTPPSAPR [286] |

Table 1c - Hepatocellular carcinoma

| MW (Da) | Subfractionation | Tryptics identified [SEQ ID No] |
|---|---|---|
| 88432 | | VSDFGLSR [796] |

Table 1d - Melanoma

| MW (Da) | Subfractionation | Tryptics identified [SEQ ID No] |
|---|---|---|
| 81352 | | ENGGASHPLLDQR [214], FLEENSSDPTYTSSLGGK [258], FTMLECLSLPR [276], IEEVIGAGEFGEVCR [360], KESCVAIK [413], LPPPPDCPTSLHQLMLDCWQK [479], VDTVAAEHLTR [749] |

Table 1e - Ovarian cancer

| MW (Da) | Subfractionation | Tryptics identified [SEQ ID No] |
|---|---|---|
| 69811 | | VSDFGLSR [796] |

Table 1f - Pancreatic cancer

| MW (Da) | Subfractionation | Tryptics identified [SEQ ID No] |
|---|---|---|
| 116302 | | FPQVVSALDK [263], HGQYLIGHGTK [335], TYEVCDVQR [739] |

**OGTA009**

Table 2a - Breast cancer

| MW (Da) | Subfractionation | Tryptics identified [SEQ ID No] |
|---------|------------------|-------------------------------|
| 23489 | Vesicles | DFYNPVVPEAQK [119], DFYNPVVPEAQKR [120], STGPGASLGTGYDR [657] |
| 23739 | Vesicles | DFYNPVVPEAQK [119] |
| 26156 | Vesicles | DFYNPVVPEAQK [119], STGPGASLGTGYDRK [658] |
| 27751 | Vesicles | VYDSLLALPQDLQAAR [817] |

Table 2b - Colorectal cancer

| MW (Da) | Subfractionation | Tryptics identified [SEQ ID No] |
|---------|------------------|-------------------------------|
| 20920 | | DFYNPVVPEAQKR [120], STGPGASLGTGYDR [657] |
| 21129 | | DFYNPVVPEAQK [119], STGPGASLGTGYDR [657] |
| 21235 | | STGPGASLGTGYDR [657] |
| 21342 | | STGPGASLGTGYDR [657] |
| 21450 | | STGPGASLGTGYDR [657] |
| 21560 | | STGPGASLGTGYDR [657] |
| 21671 | | STGPGASLGTGYDR [657] |

Table 2c - Pancreatic cancer

| MW (Da) | Subfractionation | Tryptics identified [SEQ ID No] |
|---------|------------------|-------------------------------|
| 26738 | | VYDSLLALPQDLQAAR [817] |
| 27019 | | VYDSLLALPQDLQAAR [817] |
| 27306 | | STGPGASLGTGYDR [657] |

Table 2d - Prostate cancer

| MW (Da) | Subfractionation | Tryptics identified [SEQ ID No] |
|---------|------------------|-------------------------------|
| | Digitonin | DFYNPVVPEAQK [120], STGPGASLGTGYDR [657], VVYSAPR [815], VYDSLLALPQDLQAAR [817] |

Table 2e - Renal cell cancer

| MW (Da) | Subfractionation | Tryptics identified [SEQ ID No] |
|---------|------------------|-------------------------------|
| 19493 | | STGPGASLGTGYDR [657] |

**OGTA016**

Table 3 - Colorectal cancer

| MW (Da) | Subfractionation | Tryptics identified [SEQ ID No] |
|---------|------------------|-------------------------------|
| | Heparin Binding | ADAAPDEK [57], DGSFSVVITGLR [125] |

(continued)

| MW (Da) | Subfractionation | Tryptics identified [SEQ ID No] |
|---|---|---|
| | Heparin Binding | AFVNCDENSR [68], ASVDSGSSEEQGGSSR [86], DGSFSVVITGLR [125], ILLNPQDK [377], QGHFYGETAAVYVAVEER [574] |
| | Heparin Binding | ANLTNFPENGTFVVNIAQLSQDDSGR [79], AQYEGR [85], DGSFSVVITGLR [125], EEFVATTESTTETK [171], QGHFYGETAAVYVAVEER [574], VYTVDLGR [821] |
| | Heparin Binding | DGSFSVVITGLR [125], GSVTFHCALGPEVANVAK [323], ILLNPQDK [377], QGHFYGETAAVYVAVEER [574], VYTVDLGR [821] |
| | Heparin Binding | DGSFSVVITGLR [125], QGHFYGETAAVYVAVEER [574] |
| | Heparin Binding | DGSFSVVITGLR [125], DQADGSR [150], IIEGEPNLK [371], ILLNPQDK [377], QGHFYGETAAVYVAVEER [574], TDISMSDFENSR [684], VPCHFPCK [788], VYTVDLGR [821] |
| 70703 | Nucleotide Binding | AQYEGR [85], EEFVATTESTTETK [171] |
| 84772 | Nucleotide Binding | DGSFSVVITGLR [125], FSSYEK [274], KYWCR [443], QGHFYGETAAVYVAVEER [574] |
| 88695 | Nucleotide Binding | DGSFSVVITGLR [125], FSSYEK [274], QGHFYGETAAVYVAVEER [574], YLCGAHSDGQLQEGSPIQAWQLFVNEESTIPR [861] |
| 91099 | | DFLLQSSTVAAEAQDGPQEA [116], DGSFSVVITGLR [125], EEFVATTESTTETK [171], QGHFYGETAAVYVAVEER [574], TDISMSDFENSR [684], VYTVDLGR [821] |
| 91554 | Nucleotide Binding | ILLNPQDK [377], QGHFYGETAAVYVAVEER [574], RAPAFEGR [597] |
| 93409 | | EEFVATTESTTETK [171], QGHFYGETAAVYVAVEER [574], QSSGENCDVVVNTLGK [588], QSSGENCDVVVNTLGKR [589], VLDSGFR [776], TDISMSDFENSR [684] |
| 95846 | | EEFVATTESTTETK [171], QGHFYGETAAVYVAVEER [574], QGHFYGETAAVYVAVEERK [575], QSSGENCDVVVNTLGK [588] |
| 107634 | Nucleotide Binding | DGSFSVVITGLR [125], EEFVATTESTTETK [171], ILLNPQDK [377], QGHFYGETAAVYVAVEER [574], VYTVDLGR [821] |
| 109842 | Nucleotide Binding | ASVDSGSSEEQGGSSR [86], DGSFSVVITGLR [125], EEFVATTESTTETK [171], FSSYEK [274], ILLNPQDK [377], LSLLEEPGNGTFTVILNQLTSR [486], QGHFYGETAAVYVAVEER [574], TDISMSDFENSR [684], VYTVDLGR [821] |
| 117144 | Nucleotide Binding | ASVDSGSSEEQGGSSR [86], DGSFSVVITGLR [125], GGCITLISSEGYVSSK [297], QGHFYGETAAVYVAVEER [574], TDISMSDFENSR [684] |
| 125678 | Nucleotide Binding | EEFVATTESTTETK [171] |

**OGTA028**

Table 4 - Ovarian cancer

| MW (Da) | Subfractionation | Tryptics identified [SEQ ID No] |
|---|---|---|
| 22364 | | HQVVHK [343], KENITYMKR [412], MSDVSTSVQSK [525], TEFQQIINLALQK [687], TFSDLQSLRK [691], VAKPEEMK [743], VLMEIQDLMFEEMR [783] |
| 22505 | | EADLTEETEENLR [166], IDSLEDQIEEFSK [358], KENITYMKR [412], VAKPEEMK [743], VFLSIEEFR [761] |
| 24585 | | EITYQGTR [200], KKENITYMK [421], KLPQGESR [425], MDILEER [510], TFSDLQSLR [690], VFLSIEEFR [761], VLMEIQDLMFEEMR [783] |
| 26031 | | KENITYMKR [412], KKENITYMK [421], KLPQGESR [425], QWSNVFNILR [592], REQLTETDK [602], VLMEIQDLMFEEMR [783] |
| 26256 | | EEINQGGR [177], FLCEVK [256], KKENITYMK [421], LPQGESR [480], QWSNVFNILR [592], VLMEIQDLMFEEMR [783] |
| 26487 | | DYVLHMPTLR [165], EEINQGGR [177], IGDDNENLTFK [366], KENITYMKR [412], KKENITYMK [421], LPQGESR [480], NVHLEFTER [557], QWSNVFNILR [592], TEFQQIINLALQK [687], VAKPEEMK [743], VLMEIQDLMFEEMR [783], YGIQEK [852] |
| 26724 | | EEINQGGR [177], IGDDNENLTFK [366], KENITYMKR [412], KKENITYMK [421], LPQGESR [480], QWSNVFNILR [592], TEFQQIINLALQK [687], TLHTEELTSK [704], VLMEIQDLMFEEMR [783], YGIQEK [852] |
| 26966 | | EEIGNLK [175], EIIDENFAELK [197], ELLGNNIP [203], KENITYMK [411], KKENITYMK [421], LPQGESR [480], QWSNVFNILR [592], TLHTEELTSK [704], VLMDEGAVLTLVADLSSATLDISK [782], VLMEIQDLMFEEMR [783] |
| 27470 | | AGEITSDGLSFLFLK [70], DYVLHMPTLR [165], ELLGNNIP [203], KKENITYMK [421], KLPQGESR [425], YGIQEK [852] |
| 28000 | | AGEITSDGLSFLFLK [70], KKENITYMK [421] |
| 47275 | | DVSAIMNK [162], DYVLHMPTLR [165], EIIDENFAELK [197], EITYQGTR [200], HSHTNLSISTGVTK [344], KKENITYMK [421], KLPQGESR [425], KTEEKK [437], LPQGESR [480], LTADLSLDTLDAR [494], REITYQGTR [601], SSHSGVLEIENSVDDLSSR [649], SSVINSIR [652], TFSDLQSLR [690], VFLSIEEFR [761], VLMEIQDLMFEEMR [783] |

(continued)

| MW (Da) | Subfractionation | Tryptics identified [SEQ ID No] |
|---|---|---|
| 48293 | | DYVLHMPTLR [165], EIIDENFAELK [197], EITYQGTR [200], HSHTNLSISTGVTK [344], KLPQGESR [425], KTEEKK [437], LTADLSLDTLDAR [494], MAFDFR [507], SLEMSHDEHKK [639], VFLSIEEFR [761], WSNVFK [832] |

**OGTA037**

Table 5 - Gastric cancer

| MW (Da) | Subfractionation | Tryptics identified [SEQ ID No] |
|---|---|---|
| 24774 | | ENPDNLSDFR [215] |

**OGTA041**

Table 6a - Hepatocellular carcinoma

| MW (Da) | Subfractionation | Tryptics identified [SEQ ID No] |
|---|---|---|
| 90607 | | DGPEFTFTTPK [124], KPFPEDLK [431] |

Table 6b - Lung cancer

| MW (Da) | Subfractionation | Tryptics identified [SEQ ID No] |
|---|---|---|
| 122412 | | DNMLWVEWTTPR [146], QNCSQHESSPDISHFER [582], QVSSVNEEDFVR [591], SHLQNYTVNATK [635] |

Table 6c - Pancreatic cancer

| MW (Da) | Subfractionation | Tryptics identified [SEQ ID No] |
|---|---|---|
| 104240 | | HIWPNVPDPSK [336], NYTIFYR [564], SHLQNYTVNATK [635], TNHFTIPK [711] |

**OGTA053**

Table 7 - Pancreatic cancer

| MW (Da) | Subfractionation | Tryptics identified [SEQ ID No] |
|---|---|---|
| 67953 | | FAPGVTIEEDNCCGCNAIAIR [243], LEEGQATSWSR [452], VLENYNK [780] |

**OGTA054**

Table 8 - Pancreatic cancer

| MW (Da) | Subfractionation | Tryptics identified [SEQ ID No] |
|---------|------------------|--------------------------------|
| 9213 | | EEHEVAVLGAPPSTILPR [173] |

**OGTA066**

Table 9a - Colorectal cancer

| MW (Da) | Subfractionation | Tryptics identified [SEQ ID No] |
|---------|------------------|--------------------------------|
| 19046 | | IMFVDPSLTVR [380] |
| 19131 | | IMFVDPSLTVR [380] |
| 19217 | | IMFVDPSLTVR [380] |
| 19304 | | IMFVDPSLTVR [380] |

Table 9c - Pancreatic cancer

| MW (Da) | Subfractionation | Tryptics identified [SEQ ID No] |
|---------|------------------|--------------------------------|
| 24387 | | IMFVDPSLTVR [380] |
| 24611 | | IMFVDPSLTVR [380], KPLMVTHHL FDCQYSQALK [432] |

In one or more examples of the disclosure OGTA does not relate to, for example use/methods of diagnosis for colorectal cancer.

**OGTA072**

Table 10 - Colorectal cancer

| MW (Da) | Subfractionation | Tryptics identified [SEQ ID No] |
|---------|------------------|--------------------------------|
| | Heparin Binding | MTTSGALFPSLVPGSR [526], TDQDEEHCR [685] |

**OGTA074**

Table 11 - Colorectal cancer

| MW (Da) | Subfractionation | Tryptics identified [SEQ ID No] |
|---------|------------------|--------------------------------|
| 36031 | Nucleotide Binding | EDGYSDASGFGYCFR [169] |
| | Heparin Binding | EDGYSDASGFGYCFR [169], QGPVGSGRR [577], VPEGFTCR [790] |

**OGTA076**

Table 12a - Chronic lymphocytic leukaemia

| MW (Da) | Subfractionation | Tryptics identified [SEQ ID No] |
|---|---|---|
| 198008 | Chloroform Extracted | AANDPFTIVHGNTGK [52], CEHHSLYGAAR [102], CSMLIASNETWKK [108], EFIYLRPFACDTK [183], ELTYSNFHPLLVSGR [208], FCQALGAHLSSFSHVDEIK [245], FEQEYLNDLMK [247], GWHFYDDR [328], IPENFFEEESR [385], ISEWPIDDHFTYSR [392], KYFWTGLR [442], LFHLHSQK [460], SPDLQGSWQWSDR [644], TPLSYTHWR [718], TPVSTIIMPNEFQQDYDIR [722], VFHRPWR [760], VQCSEQWIPFQNK [793], WVSQHR [838], YFWTGLR [851], YPWHR [866] |
| 223568 | | AANDPFTIVHGNTGK [52], CSMLIASNETWKK [108], ELTYSNFHPLLVSGR [208], FEQEYLNDLMKK [248], FPVTFGEECLYMSAK [265], GWHFYDDR [328], HFVSLCQK [333], IPENFFEEESR [385], ISEWPIDDHFTYSR [392], KVECEHGFGR [439], NWEEAER [563], SDQALHSFSEAK [623], SPDLQGSWQWSDR [644], TPLSYTHWR [718], VFHAER [759], VQCSEQWIPFQNK [793], WVSQHR [838], YPWHR [866] |
| 238644 | | KVECEHGFGR [439], SDQALHSFSEAK [623], TPLSYTHWR [718], VFHAER [759] |

Table 12b - Colorectal cancer

| MW (Da) | Subfractionation | Tryptics identified [SEQ ID No] |
|---|---|---|
| 167535 | | IPENFFEEESR [385], TPVSTIIMPNEFQQDYDIR [722] |

Table 12c - Pancreatic cancer

| MW (Da) | Subfractionation | Tryptics identified [SEQ ID No] |
|---|---|---|
| 161314 | | AANDPFTIVHGNTGK [52], EFIYLRPFACDTK [183], ELTYSNFHPLLVSGR [208], IEMVDYK [362], IPENFFEEESR [385], ISEWPIDDHFTYSR [392], LTYSSR [499], RHGETCYK [604], SPDLQGSWQWSDR [644], TPLSYTHWR [718], VFHAER [759], VFHRPWR [760], VQCSEQWIPFQNK [793], WVSQHR [838], YFWTGLR [851], YPWHR [866], YVCKRK [875] |
| 261976 | | AFSSDLISIHSLADVEVVVTK [67], CSMLIASNETWKK [108], DGHGTAISNASDVWKK [122], EFIYLRPFACDTK [183], HMATTQDEVHTK [338], IPENFFEEESR [385], ISEWPIDDHFTYSR [392], KYFWTGLR [442], SPDLQGSWQWSDR [644], TPLSYTHWR [718], VFHRPWR [760], VIEEAVYFHQHSILACK [767], VQCSEQWIPFQNK [793], YFWTGLR [851], YPWHR [866], YVCKRK [875] |

**OGTA085**

Table 13a - Lung cancer

| MW (Da) | Subfractionation | Tryptics identified [SEQ ID No] |
|---------|------------------|---------------------------------|
| 31005 | | KMLGNPSR [427], STETALYR [655], TIHAELSK [700], VLAQQGEYSEAIPILR [773] |
| 64045 | | TAVDGPDLEMLTGQER [677], VLAQQGEYSEAIPILR [773] |
| 69488 | | TAVDGPDLEMLTGQER [677], VLAQQGEYSEAIPILR [773] |

Table 13b - Melanoma

| MW (Da) | Subfractionation | Tryptics identified [SEQ ID No] |
|---------|------------------|---------------------------------|
| 22017 | | KMLGNPSR [427], MGQPPAEEAEQPGALAR [516], STETALYR [655], TAVDGPDLEMLTGQER [677], VDMTFEEEAQLLQLK [748] |
| 22380 | | STETALYRK [656], TAVDGPDLEMLTGQER [677], VDMTFEEEAQLLQLK [748] |
| 22505 | | SCSLVLEHQPDNIK [620], STETALYRK [656], TAVDGPDLEMLTGQER [677] |
| 22632 | | SCSLVLEHQPDNIK [620], TAVDGPDLEMLTGQER [677], VDMTFEEEAQLLQLK [748] |
| 27711 | | ADFVLAANSYDLAIK [60], GQVVTVHLQTSLENGTR [313], KMLGNPSR [427], LDHYR [447], TAVDGPDLEMLTGQER [677] |

Table 13c - Retinoblastoma

| MW (Da) | Subfractionation | Tryptics identified [SEQ ID No] |
|---------|------------------|---------------------------------|
| 50346 | | KMLGNPSR [427], MGQPPAEEAEQPGALAR [516], VLAQQGEYSEAIPILR [773] |

**OGTA087**

Table 14a - B-cell non-Hodgkin's lymphoma

| MW (Da) | Subfractionation | Tryptics identified [SEQ ID No] |
|---------|------------------|---------------------------------|
| 54163 | | FVWNGHLLR [279], LGVLHVGQK [464], LHITPEK [466], LSNTSPEFQEMSLLER [488], NNFSDGFR [550], YIAFDFHK [854], YKPLPQISK [856] |
| 55443 | | ATDFDVLSYKK [89], FVWNGHLLR [279], KTMLHLTDIQLQDNK [438], LHITPEK [466], NNFSDGFR [550], QVIINLINQK [590], QYAGTGALK [593], VANHMDGFQR [744], YKPLPQISK [856] |
| 75222 | | LHITPEK [466], NNFSDGFR [550], QVIINLINQK [590], QYAGTGALK [593], VANHMDGFQR [744], YKPLPQISK [856] |

Table 14b - Breast cancer

| MW (Da) | Subfractionation | Tryptics identified [SEQ ID No] |
|---|---|---|
| 52857 | | ATDFDVLSYKK [89], FVWNGHLLR [279], TNVIQSLLAR [714] |

Table 14c - Colorectal cancer

| MW (Da) | Subfractionation | Tryptics identified [SEQ ID No] |
|---|---|---|
| | Heparin Binding | LHITPEK [466], YIAFDFHK [854] |

Table 14d - Gastric cancer

| MW (Da) | Subfractionation | Tryptics identified [SEQ ID No] |
|---|---|---|
| 50390 | | TNVIQSLLAR [714] |
| 50979 | | TNVIQSLLAR [714] |

Table 14e - Lung cancer

| MW (Da) | Subfractionation | Tryptics identified [SEQ ID No] |
|---|---|---|
| 34397 | | LSNTSPEFQEMSLLER [488], NNFSDGFR [550], YFDWILISR [848], YFDWILISRR [849], YIAFDFHK [854], YKPLPQISK [856] |
| 63738 | | HFDSQVIIYGK [332], LHITPEK [466], NNFSDGFR [550], RTHLGLIMDGWNSMIR [615], TNVIQSLLAR [714] |
| 66502 | | GSEKPLEQTFATMVSSLGSGMMR [314], HFDSQVIIYGK [332], LHITPEK [466], NNFSDGFR [550], QYAGTGALK [593], VANHMDGFQR [744], YIAFDFHK [854] |
| 69051 | | ATDFDVLSYK [88], GSEKPLEQTFATMVSSLGSGMMR [314], LEEQDEFEK [453], THLGLIMDGWNSMIR [696], VSTEVTLAVK [797] |

Table 14f - Lymphoid leukaemia, unspecified

| MW (Da) | Subfractionation | Tryptics identified [SEQ ID No] |
|---|---|---|
| 56356 | | TNVIQSLLAR [714], YKPLPQISK [856] |
| 58422 | | LSNTSPEFQEMSLLER [488], NNFSDGFR [550], THLGLIMDGWNSMIR [696] |

Table 14g - Ovarian cancer

| MW (Da) | Subfractionation | Tryptics identified [SEQ ID No] |
|---|---|---|
| 42632 | | ATDFDVLSYKK [89], FVWNGHLLR [279], GSIPVFWSQRPNLK [316], HFDSQVIIYGK [332], KTMLHLTDIQLQDNK [438], LEEQDEFEK [453], RTHLGLIMDGWNSMIR [615], TMLHLTDIQLQDNK [707] |

**OGTA088**

Table 15a - Breast cancer

| MW (Da) | Subfractionation | Tryptics identified [SEQ ID No] |
|---|---|---|
| 29702 | | EHALAQAELLK [190], EMQNLSQHGR [211], NVPPGLDEYNPFSDSR [560], RQEELER [612] |
| 29901 | | DPSVTQVTR [149], EMQNLSQHGR [211], NVPPGLDEYNPFSDSR [560], RQEELER [612] |
| 30103 | | EHALAQAELLK [190], EMQNLSQHGR [211], NVPPGLDEYNPFSDSR [560], RQEELER [612] |
| 30309 | | EMQNLSQHGR [211], NVPPGLDEYNPFSDSR [560], RQEELER [612] |

Table 15b - Gastric cancer

| MW (Da) | Subfractionation | Tryptics identified [SEQ ID No] |
|---|---|---|
| 29509 | | EHALAQAELLK [190], EMQNLSQHGR [211] |

Table 15c - Glioblastoma

| MW (Da) | Subfractionation | Tryptics identified [SEQ ID No] |
|---|---|---|
| 29544 | | EHALAQAELLK [190], EMQNLSQHGR [211], KAAELDR [405], MPNVPNTQPAIMKPTEEHPAYTQIAK [522], NVPPGLDEYNPFSDSR [560], QEELER [566], RQEELER [612], TPPPGGVK [721], TVQTAAANAASTAASSAAQNAFK [737], VHGLYR [766] |
| 29779 | | EHALAQAELLK [190], EMQNLSQHGR [211], NVPPGLDEYNPFSDSR [560], RQEELER [612] |
| 30027 | | AQQEFATGVMSNK [84], EHALAQAELLK [190], EMQNLSQHGR [211], NVPPGLDEYNPFSDSR [560], RQEELER [612], TVQTAAANAASTAASSAAQNAFK [737], VHGLYR [766] |

Table 15d - Hepatocellular carcinoma

| MW (Da) | Sub fractionation | Tryptics identified [SEQ ID No] |
|---|---|---|
| 26264 | | DPSVTQVTR [149], EHALAQAELLK [190], EMQNLSQHGR [211], KAAELDRR [406], NVPPGLDEYNPFSDSR [560], RQEELER [612] |
| 26522 | | EMQNLSQHGR [211], NVPPGLDEYNPFSDSR [560] |
| 27053 | | EMQNLSQHGR [211], EMQNLSQHGRK [212], NVPPGLDEYNPFSDSR [560], RQEELERK [613] |
| 28538 | | EHALAQAELLK [190], EMQNLSQHGR [211], MPNVPNTQPAIMKPTEEHPAYTQIAK [522], NVPPGLDEYNPFSDSR [560] |

Table 15e - Lung cancer

| MW (Da) | Subfractionation | Tryptics identified [SEQ ID No] |
|---|---|---|
| 31584 | | KVHGLYR [441], NVPPGLDEYNPFSDSR [560] |
| 31835 | | EMQNLSQHGR [211], NVPPGLDEYNPFSDSR [560] |
| 33009 | | EMQNLSQHGR [211], NVPPGLDEYNPFSDSR [560], QEELER [566] |
| 33422 | | EHALAQAELLK [190], EMQNLSQHGR [211], MPNVPNTQPAIMKPTEEHPAYTQIAK [522], NVPPGLDEYNPFSDSR [560], RQEELER [612] |
| 33847 | | EMQNLSQHGR [211], NVPPGLDEYNPFSDSR [560] |
| 34135 | | AQQEFATGVMSNK [84], EMQNLSQHGR [211], NVPPGLDEYNPFSDSR [560] |
| 35643 | | EMQNLSQHGR [211], NVPPGLDEYNPFSDSR [560], TPPPGGVK [721], TVQTAAANAASTAASSAAQNAFK [737] |

Table 15f- Lymphoid leukaemia, unspecified

| MW (Da) | Subfractionation | Tryptics identified [SEQ ID No] |
|---|---|---|
| 28206 | | EHALAQAELLK [190], EMQNLSQHGR [211], NVPPGLDEYNPFSDSR [560] |

Table 15g - Pancreatic cancer

| MW (Da) | Subfractionation | Tryptics identified [SEQ ID No] |
|---|---|---|
| 26742 | | EHALAQAELLK [190], NVPPGLDEYNPFSDSR [560] |
| 31738 | | EHALAQAELLK [190], EMQNLSQHGR [211], NVPPGLDEYNPFSDSR [560] |
| 32245 | | EHALAQAELLK [190], EMQNLSQHGR [211], NVPPGLDEYNPFSDSR [560] |

(continued)

| MW (Da) | Subfractionation | Tryptics identified [SEQ ID No] |
|---|---|---|
| 34059 | | DPSVTQVTR [149], EMQNLSQHGR [211], KVHGLYR [441], NVPPGLDEYNPFSDSR [560], TVQTAAANAASTAASSAAQNAFK [737] |
| 34270 | | DPSVTQVTR [149], EMQNLSQHGR [211], NVPPGLDEYNPFSDSR [560] |
| 36671 | | EHALAQAELLK [190], EMQNLSQHGR [211], KAAELDRR [406], NVPPGLDEYNPFSDSR [560], RQEELER [612] |
| 36974 | | DPSVTQVTR [149], EHALAQAELLK [190], EMQNLSQHGR [211], NVPPGLDEYNPFSDSR [560], RQEELER [612] |

Table 15h - Renal cell cancer

| MW (Da) | Subfractionation | Tryptics identified [SEQ ID No] |
|---|---|---|
| 30318 | | EHALAQAELLK [190], EMQNLSQHGR [211], NVPPGLDEYNPFSDSR [560] |
| 33762 | | EHALAQAELLK [190], EMQNLSQHGR [211], KVHGLYR [441], NVPPGLDEYNPFSDSR [560] |

Table 15i - Retinoblastoma

| MW (Da) | Subfractionation | Tryptics identified [SEQ ID No] |
|---|---|---|
| 30816 | | EHALAQAELLK [190], EMQNLSQHGR [211], NVPPGLDEYNPFSDSR [560], RQEELER [612], TVQTAAANAASTAASSAAQNAFK [737], VHGLYR [766] |

**OGTA089**

Table 16a - Breast cancer

| MW (Da) | Subfractionation | Tryptics identified [SEQ ID No] |
|---|---|---|
| 110512 | | ADFLIFR [59], EEINKPPIAK [176], ELTLPVDSTTLDGSK [207], GETYTYDWQLITHPR [293], VVEMQGVR [809] |

Table 16b - Lung cancer

| MW (Da) | Subfractionation | Tryptics identified [SEQ ID No] |
|---|---|---|
| 72110 | | GETYTYDWQLITHPR [293], IQPYTEQSTK [391], VNDSNELGGLTTSGSAEVHK [786] |

Table 16c - Ovarian cancer

| MW (Da) | Subfractionation | Tryptics identified [SEQ ID No] |
|---------|------------------|--------------------------------|
| 77125 | | GETYTYDWQLITHPR [293], NLQSQSSVNVIVK [544] |
| 89348 | | ADFLIFR [59], EEINKPPIAK [176], GETYTYDWQLITHPR [293], ILDATDQESLELKPTSR [374], IQPYTEQSTK [391], LTPGLYEFK [497], MVFFVQNEPPHQIFK [528], NVSVQPEISEGLATTPSTQQVK [562], THSSNSMLVFLK [699] |

**OGTA091**

Table 17a - Breast cancer

| MW (Da) | Subfractionation | Tryptics identified [SEQ ID No] |
|---------|------------------|--------------------------------|
| 24993 | Vesicles | NLVDPFVEVSFAGK [547] |
| 114969 | | TDVHYR [686] |
| 120658 | | TDVHYR [686] |
| 124535 | PNGase F Deglycosylation | GEGVAYR [291] |
| 142847 | | GEGVAYR [291] |
| 164389 | | GEGVAYR [291], NLVDPFVEVSFAGK [547] |
| 165165 | | GEGVAYR [291], NLVDPFVEVSFAGK [547] |
| 171988 | | GEGVAYR [291], NLVDPFVEVSFAGK [547], TDVHYR [686] |
| 176984 | | GEGVAYR [291], TDVHYR [686] |
| 179325 | | GEGVAYR [291], NLVDPFVEVSFAGK [547] |
| 210126 | PNGase F Deglycosylation | TDVHYR [686] |
| 230530 | PNGase F Deglycosylation | TDVHYR [686] |

Table 17b - Cervical cancer

| MW (Da) | Subfractionation | Tryptics identified [SEQ ID No] |
|---------|------------------|--------------------------------|
| | | GEGVAYR [291], TDVHYR [686] |
| | | GEGVAYR [291], TDVHYR [686] |
| | | TDVHYR [686] |

Table 17c - Chronic lymphocytic leukaemia

| MW (Da) | Subfractionation | Tryptics identified [SEQ ID No] |
|---------|------------------|--------------------------------|
| 223568 | | FSDVTGK [272], TDVHYR [686] |

Table 17d - Gastric cancer

| MW (Da) | Subfractionation | Tryptics identified [SEQ ID No] |
|---------|------------------|--------------------------------|
| 95956 | | TDVHYR [686] |
| 125740 | | TDVHYR [686] |

(continued)

| MW (Da) | Subfractionation | Tryptics identified [SEQ ID No] |
|---|---|---|
| 129831 | | NLVDPFVEVSFAGK [547] |
| 138914 | | GEGVAYR [291], NLVDPFVEVSFAGK [547], TDVHYR [686] |
| 143975 | | GEGVAYR [291], TDVHYR [686] |

Table 17e - Hepatocellular carcinoma

| MW (Da) | Subfractionation | Tryptics identified [SEQ ID No] |
|---|---|---|
| 98239 | | GEGVAYR [291] |
| 102119 | | GEGVAYR [291], NLVDPFVEVSFAGK [547] |
| 106345 | | GEGVAYR [291], TDVHYR [686] |
| 107073 | | GEGVAYR [291] |
| 110964 | | GEGVAYR [291], TDVHYR [686] |
| 114377 | | GEGVAYR [291] |
| 116037 | | GEGVAYR [291], TDVHYR [686] |
| 118513 | | GEGVAYR [291], NLVDPFVEVSFAGK [547], TDVHYR [686] |
| 121636 | | FFASIGER [250], FHLEYR [254], GEGVAYR [291], IGETVVDLENR [367], IIDWDR [370], IPNPHLGPVEER [386], KTDAFRR [436], LLSDKPQDFQIR [469], QIFGFESNK [579], SLGGEGNFNWR [640], TDALLGEFR [681], TDVHYR [686], VEDLPADDILR [753], VQVIEGR [794], VTAAGQTK [800] |
| 123033 | | NLVDPFVEVSFAGK [547], TDVHYR [686] |
| 127849 | | GEGVAYR [291] |
| 128000 | | TDVHYR [686] |
| 133487 | | AVDEQGWEYSITIPPER [94], DLSQMEALK [139], DSFSDPYAIVSFLHQSQK [155], EKPAIHHIPGFEVQETSR [201], EVLATPSLSASFNAPLLDTK [234], FHLEYR [254], GEGVAYR [291], GSQPSGELLASFELIQR [320], HWVPAEK [348], IETQNQLLGIADR [363], IGETVVDLENR [367], ILDESEDTDLPYPPPQR [375], IPNPHLGPVEER [386], IYPLPEDPAIPMPPR [403], LPGGQWIYMSDNYTDVNGEK [477], LSVFAETYENETK [491], MDVGTIYR [511], MFELTCTLPLEK [515], MYYTHRRR [533], RMEPLEK [607], RPDTSFLWFTSPYK [608], SLGGEGNFNWR [640], TDALLGEFR [681], TDVHYR [686], TSLCVLGPGDEAPLERK [729], VEDLPADDILR [753], VPAHQVLFSR [787], VQVIEGR [794], VVFQIWDNDK [810], WEDEEWSTDLNR [824], WLLLSDPDDFSAGAR [827] |

(continued)

| MW (Da) | Subfractionation | Tryptics identified [SEQ ID No] |
|---|---|---|
| 139587 | | AVDEQGWEYSITIPPER [94], DLSQMEALK [139], DLSQMEALKR [140], FFASIGER [250], ILDESEDTDLPYPPPQR [375], KNPNFDICTLFMEVMLPR [430], LPGGQWIYMSDNYTDVNGEK [477], MFELTCTLPLEK [515], NPNFDICTLFMEVMLPR [551], QFHQLAAQGPQECLVR [570], RDLSQMEALK [599], RMEPLEK [607], TANPQWNQNITLPAMFPSMCEK [670], TDAFRRR [680], TDALLGEFR [681], TQEETEDPSVIGEFK [724], TSLCVLGPGDEAPLER [728], VEDLPADDILR [753], VVFQIWDNDK [810] |
| 192580 | | TDVHYR [686] |
| 200565 | | GEGVAYR [291], TDVHYR [686] |
| 209214 | | TDVHYR [686] |

Table 17f - Lung cancer

| MW (Da) | Subfractionation | Tryptics identified [SEQ ID No] |
|---|---|---|
| 195217 | | GEGVAYR [291], TDVHYR [686] |
| 202986 | | GEGVAYR [291], KNLVDPFVEVSFAGK [429], TDVHYR [686] |
| 211353 | | GEGVAYR [291], KNLVDPFVEVSFAGK [429], TDVHYR [686] |
| 220388 | | GEGVAYR [291], KNLVDPFVEVSFAGK [429], TDVHYR [686] |
| 230173 | | EYSIEEIEAGR [241], ILDESEDTDLPYPPPQR [375], IPNPHLGPVEER [386], MEPLEK [513], RMEPLEK [607], SLGGEGNFNWR [640], TDVHYR [686] |

Table 17g - Melanoma

| MW (Da) | Subfractionation | Tryptics identified [SEQ ID No] |
|---|---|---|
| 20629 | | FSDVTGK [272], NLVDPFVEVSFAGK [547] |

Table 17h - Osteosarcoma

| MW (Da) | Subfractionation | Tryptics identified [SEQ ID No] |
|---|---|---|
| 151810 | | NLVDPFVEVSFAGK [547] |
| 159457 | | TDVHYR [686] |
| 181827 | | NLVDPFVEVSFAGK [547] |

Table 17i - Ovarian cancer

| MW (Da) | Subfractionation | Tryptics identified [SEQ ID No] |
|---|---|---|
| 70973 | | KNLVDPFVEVSFAGK [429] |
| 74967 | | KNLVDPFVEVSFAGK [429] |
| 110436 | | NLVDPFVEVSFAGK [547] |

(continued)

| MW (Da) | Subfractionation | Tryptics identified [SEQ ID No] |
|---|---|---|
| 115528 | | NLVDPFVEVSFAGK [547] |
| 127159 | | GEGVAYR [291] |

Table 17i - Pancreatic cancer

| MW (Da) | Subfractionation | Tryptics identified [SEQ ID No] |
|---|---|---|
| 55323 | | TDVHYR [686] |
| 70820 | | GEGVAYR [291] |
| 88202 | | FSDVTGK [272], KNLVDPFVEVSFAGK [429] |
| 100099 | | GEGVAYR [291] |
| 132648 | | TDVHYR [686] |
| 133778 | | TDVHYR [686] |
| 142218 | | GEGVAYR [291] |
| 142690 | | NLVDPFVEVSFAGK [547] |
| 154726 | | GEGVAYR [291], NLVDPFVEVSFAGK [547] |
| 158113 | | GEGVAYR [291] |
| 168430 | | GEGVAYR [291], TDVHYR [686] |
| 174222 | | NLVDPFVEVSFAGK [547] |
| 184505 | | FSDVTGK [272], TDVHYR [686] |
| 189061 | | KNLVDPFVEVSFAGK [429] |
| 192682 | | TDVHYR [686] |
| 208929 | | GEGVAYR [291], NLVDPFVEVSFAGK [547], TDVHYR [686] |
| 216690 | | GEGVAYR [291], NLVDPFVEVSFAGK [547], TDVHYR [686] |
| 217481 | | TDVHYR [686] |
| 222673 | | TDVHYR [686] |
| 229012 | | GEGVAYR [291], TDVHYR [686] |
| 230839 | | GEGVAYR [291], TDVHYR [686] |
| 261976 | | TDVHYR [686] |

Table 17k - Prostate cancer

| MW (Da) | Subfractionation | Tryptics identified [SEQ ID No] |
|---|---|---|
| | | GEGVAYR [291], TDVHYR [686] |
| | | TDVHYR [686] |

Table 171- Renal cell cancer

| MW (Da) | Subfractionation | Tryptics identified [SEQ ID No] |
|---|---|---|
| 62841 | | TDVHYR [686] |
| 83490 | | GEGVAYR [291] |

(continued)

| MW (Da) | Subfractionation | Tryptics identified [SEQ ID No] |
|---|---|---|
| 122161 | | FSDVTGK [272], GEGVAYR [291] |
| 126235 | | GEGVAYR [291] |
| 135291 | | GEGVAYR [291] |
| 143822 | | GEGVAYR [291], TDVHYR [686] |
| 148249 | | TDVHYR [686] |
| 151721 | | GEGVAYR [291], TDVHYR [686] |
| 158158 | | ALGRPGPPFNITPR [76], AVDEQGWEYSITIPPER [94], CIIWNTR [104], DLAAMDK [132], DLSQMEALK [139], EFNQFAEGK [185], EKPAIHHIPGFEVQETSR [201], EYSIEEIEAGR [241], FHLEYR [254], FSFDDFLGSLQLDLNR [273], GEGVAYR [291], GWMIGFEEHK [329], IETQNQLLGIADR [363], IGETVVDLENR [367], IIDWDR [370], ILDESEDTDLPYPPPQR [375], IYPLPEDPAIPMPPR [403], KLPSRPPPHYPGIK [426], LLSDKPQDFQIR [469], MDVGTIYR [511], MYYTHR [531], MYYTHRR [532], RMEPLEK [607], SLGGEGNFNWR [640], SYQLANISSPSLVVECGGQTVQSCVIR [667], TDALLGEFR [681], TQEETEDPSVIGEFK [724], VEDLPADDILR [753], VQVIEGR [794], WEDEEWSTDLNR [824], WLLLSDPDDFSAGAR [827] |
| 200334 | | NLVDPFVEVSFAGK [547] |
| 221217 | | GEGVAYR [291], NLVDPFVEVSFAGK [547], TDVHYR [686] |
| 233732 | | TDVHYR [686] |

**OGTA098**

Table 18a - Breast cancer

| MW (Da) | Subfractionation | Tryptics identified [SEQ ID No] |
|---|---|---|
| 49867 | | VVPSFLPVDQGGSLVGR [812] |
| 50576 | | DGNGEVTDKPVK [123], DMAGAQAAAVALNEEFLR [142], DNWISVDSVTSEIK [148], DTGEIYTTSVTLDR [159], EETPFFLLTGYALDAR [182], NGVGGMAK [536], QESTSVLLQQSEKK [569], VTQEIVTER [806], VVPSFLPVDQGGSLVGR [812], VYAPASTLVDQPYANEGTVVVTER [816], YVQNGTYTVK [876] |
| 115607 | | ATQFTGATGAIMTTETTK [93], DGNGEVTDKPVK [123], FLDDLGLK [257], IVAISEDYPR [400], QESTSVLLQQSEK [568], VVPSFLPVDQGGSLVGR [812] |

33

Table 18b - Cervical cancer

| MW (Da) | Subfractionation | Tryptics identified [SEQ ID No] |
|---|---|---|
| 82244 | MonoQ Aqueous | DTGEIYTTSVTLDR [159], EEHSSYTLTVEAR [174], IVAISEDYPR [400] |

Table 18c - Hepatocellular carcinoma

| MW (Da) | Subfractionation | Tryptics identified [SEQ ID No] |
|---|---|---|
| 83238 | | ATQFTGATGAIMTTETTK [93], GNNVEKPLELR [304], INATDADEPNTLNSK [381], IVAISEDYPR [400], QESTSVLLQQSEK [568] |
| 112208 | | ATQFTGATGAIMTTETTK [93], DNWISVDSVTSEIK [148], DTGEIYTTSVTLDR [159], FLDDLGLK [257], GNNVEKPLELR [304], GQIIGNFQAFDEDTGLPAHAR [311], IVAISEDYPR [400], VATPLPDPMASR [746], VLEGMVEENQVNVEVTR [778], VTQEIVTER [806], VVPSFLPVDQGGSLVGR [812], WEEHR [825] |
| 134896 | | ATQFTGATGAIMTTETTK [93] |

Table 18d - Lung cancer

| MW (Da) | Subfractionation | Tryptics identified [SEQ ID No] |
|---|---|---|
| | | ATQFTGATGAIMTTETTK [93], DTGEIYTTSVTLDR [159], EEHSSYTLTVEAR [174], FLDDLGLK [257], GNNVEKPLELR [304], GQIIGNFQAFDEDTGLPAHAR [311], INATDADEPNTLNSK [381], IVAISEDYPR [400], LPDFESR [476], QESTSVLLQQSEK [568], VATPLPDPMASR [746], VLEGMVEENQVNVEVTR [778], VTQEIVTER [806], VVPSFLPVDQGGSLVGR [812] |
| | | DNWISVDSVTSEIK [148], DTGEIYTTSVTLDR [159], EEHSSYTLTVEAR [174], GNNVEKPLELR [304], GQIIGNFQAFDEDTGLPAHAR [311], INATDADEPNTLNSK [381], IVAISEDYPR [400], LPDFESR [476], VLAPASTLQSSYQIPTENSMTAR [772], VLEGMVEENQVNVEVTR [778], VVPSFLPVDQGGSLVGR [812], VYAPASTLVDQPYANEGTVVVTER [816], YKPTPIPIK [858] |

Table 18e - Osteosarcoma

| MW (Da) | Subfractionation | Tryptics identified [SEQ ID No] |
|---|---|---|
| 24158 | | GITEPPFGIFVFNK [301] |
| 41065 | | DNWISVDSVTSEIK [148], IVAISEDYPR [400], VLEGMVEENQVNVEVTR [778] |

Table 18f - Pancreatic cancer

| MW (Da) | Subfractionation | Tryptics identified [SEQ ID No] |
|---|---|---|
| 47024 | | ATQFTGATGAIMTTETTK [93], DGNGEVTDKPVK [123], EGEDLSKK [187], ESFLAPSSGVQPTLAMPNIAVGQNVTVTER [223], GSSSASIVK [322], VATPLPDPMASR [746], VTQEIVTER [806], VVPSFLPVDQGGSLVGR [812], VYAPASTLVDQPYANEGTVVVTER [816] |
| 47767 | | ATQFTGATGAIMTTETTK [93], DTGEIYTTSVTLDR [159], ESFLAPSSGVQPTLAMPNIAVGQNVTVTER [223], GSSSASIVK [322], VATPLPDPMASR [746], VLAPASTLQSSYQIPTENSMTAR [772], VTQEIVTER [806], VVPSFLPVDQGGSLVGR [812], VYAPASTLVDQPYANEGTVVVTER [816], WEEHR [825] |
| 48531 | | ATQFTGATGAIMTTETTK [93], DGNGEVTDKPVK [123], VTQEIVTER [806], VVPSFLPVDQGGSLVGR [812], VYAPASTLVDQPYANEGTVVVTER [816], WEEHR [825] |
| 57915 | | ATQFTGATGAIMTTETTK [93], DGNGEVTDKPVK [123], DMAGAQAAAVALNEEFLR [142], DTGELNVTSILDR [160], EEHSSYTLTVEAR [174], EETPFFLLTGYALDAR [182], ESFLAPSSGVQPTLAMPNIAVGQNVTVTER [223], GNNVEKPLELR [304], INATDADEPNTLNSK [381], SSVISIYVSESMDR [653], VTQEIVTER [806], VVPSFLPVDQGGSLVGR [812], VYAPASTLVDQPYANEGTVVVTER [816], WEEHR [825] |
| 58846 | | ATQFTGATGAIMTTETTK [93], DGNGEVTDKPVK [123], EGEDLSKK [187], ESFLAPSSGVQPTLAMPNIAVGQNVTVTER [223], IHSDLAEER [369], INATDADEPNTLNSK [381], VTQEIVTER [806], VVPSFLPVDQGGSLVGR [812], VYAPASTLVDQPYANEGTVVVTER [816], WEEHR [825] |
| 119656 | | DGNGEVTDKPVK [123], DMAGAQAAAVALNEEFLR [142], GNNVEKPLELR [304], INATDADEPNTLNSK [381], IVAISEDYPR [400], SEIQFLISDNQGFSCPEK [628], SSVISIYVSESMDR [653] |
| 134980 | | ATQFTGATGAIMTTETTK [93], DNWISVDSVTSEIK [148], DTGEIYTTSVTLDR [159], EEHSSYTLTVEAR [174], GNNVEKPLELR [304], GQIIGNFQAFDEDTGLPAHAR [311], VTQEIVTER [806], VYAPASTLVDQPYANEGTVVVTER [816] |
| 142690 | | ATQFTGATGAIMTTETTK [93], DTGEIYTTSVTLDR [159], EEHSSYTLTVEAR [174], EETPFFLLTGYALDAR [182], GNNVEKPLELR [304], GQIIGNFQAFDEDTGLPAHAR [311], QESTSVLLQQSEK [568], VLEGMVEENQVNVEVTR [778] |

Table 18g - Prostate cancer

| MW (Da) | Subfractionation | Tryptics identified [SEQ ID No] |
|---|---|---|
| | | DTGEIYTTSVTLDR [159], FLDDLGLK [257], VATPLPDPMASR [746] |

Table 18h - Renal cell cancer

| MW (Da) | Subfractionation | Tryptics identified [SEQ ID No] |
|---|---|---|
| 111421 | | GNNVEKPLELR [304], IVAISEDYPR [400], LPDFESR [476], VTQEIVTER [806] |

**OGTA101**

Table 19a - Breast cancer

| MW (Da) | Subfractionation | Tryptics identified [SEQ ID No] |
|---|---|---|
| 24841 | Vesicles | QFQFTDWPEQGVPK [572], YQYFVVDPMAEYNMPQYILR [869] |
| 24993 | Vesicles | AALEYLGSFDHYAT [51], QFQFTDWPEQGVPK [572], YQYFVVDPMAEYNMPQYILR [869] |
| 55008 | | GVEGSDYINASFIDGYR [325], YQYFVVDPMAEYNMPQYILR [869] |
| 55921 | | AALEYLGSFDHYAT [51], QFQFTDWPEQGVPK [572] |
| 56324 | | QHGQIR [578] |
| 56872 | | AEPESETSILLSWTPPR [63], EIPSHHPTDPVELR [198], FIKPWESPDEMELDELLK [255], GFPTIDMGPQLK [295], GPPSEPVLTQTSEQAPSSAPR [309], GVEGSDYINASFIDGYR [325], LTQIETGENVTGMELEFK [498], LVNIMPYESTR [503], MLWEHNSTIVVMLTK [520], MVEEVDGR [527], SYSFVLTNR [668], TATMLCAASGNPDPEITWFK [675], TNEDVPSGPPRK [709], TSVLLSWEIPENYNSAMPFK [731], VGFGEEMVK [763] |
| 105325 | | TGCFIVIDAMLER [692] |

Table 19b - Cervical cancer

| MW (Da) | Subfractionation | Tryptics identified [SEQ ID No] |
|---|---|---|
| 118858 | Integral | QHGQIR [578], WTEYR [836] |
| 124604 | Integral | WTEYR [836] |

Table 19c - Colorectal cancer

| MW (Da) | Subfractionation | Tryptics identified [SEQ ID No] |
|---|---|---|
| | Heparin Binding | YSAPANLYVR [870] |

Table 19d - Hepatocellular carcinoma

| MW (Da) | Subfractionation | Tryptics identified [SEQ ID No] |
|---|---|---|
| 72397 | | DMEPLTTLEFSEK [143], GFPTIDMGPQLK [295], GPGPYSPSVQFR [306], SGEGFIDFIGQVHK [632], TATMLCAASGNPDPEITWFK [675], YSAPANLYVR [870], YANVIAYDHSR [840] |

Table 19e - Lung cancer

| MW (Da) | Subfractionation | Tryptics identified [SEQ ID No] |
|---|---|---|
| | | YSAPANLYVR [870] |
| 77419 | | FIKPWESPDEMELDELLK [255], GFPTIDMGPQLK [295], GVEGSDYINASFIDGYR [325], LTQIETGENVTGMELEFK [498], QFQFTDWPEQGVPK [572], SDTIANYELVYK [625], TATMLCAASGNPDPEITWFK [675], TVDIYGHVTLMR [736], VEVEAVNSTSVK [755], YEGVVDIFQTVK [843] |
| 78771 | | YANVIAYDHSR [840] |

Table 19f - Osteosarcoma

| MW (Da) | Subfractionation | Tryptics identified [SEQ ID No] |
|---|---|---|
| 63624 | | QHGQIR [578], YQYFVVDPMAEYNMPQYILR [869] |

Table 19g - Pancreatic cancer

| MW (Da) | Subfractionation | Tryptics identified [SEQ ID No] |
|---|---|---|
| 74307 | | DFLPVDTSNNNGR [118], EIPSHHPTDPVELRR [199], EQFGQDGPISVHCSAGVGR [219], GFPTIDMGPQLK [295], GNSAGGLQHR [305], GVEGSDYINASFIDGYR [325], KRAESDSR [434], LTQIETGENVTGMELEFK [498], LVNIMPYESTR [503], RAESDSRK [595], SDTIANYELVYK [625], SGEGFIDFIGQVHK [632], TGCFIVIDAMLER [692], TVDIYGHVTLMR [736], YANVIAYDHSR [840], YSVAGLSPYSDYEFR [873] |
| 115291 | | TGCFIVIDAMLER [692], YQYFVVDPMAEYNMPQYILR [869], YSAPANLYVR [870] |
| 124451 | | TGCFIVIDAMLER [692], YSAPANLYVR [870] |
| 126215 | | YSAPANLYVR [870] |
| 129890 | | YSAPANLYVR [870] |

**OGTA104**

Table 20a - Breast cancer

| MW (Da) | Subfractionation | Tryptics identified [SEQ ID No] |
|---|---|---|
| 78181 | | EVPIYANR [236], FAVPTYAAK [244], QPQQFPSRPPPPQPK [586] |

Table 20b - Hepatocellular carcinoma

| MW (Da) | Subfractionation | Tryptics identified [SEQ ID No] |
|---|---|---|
| 63302 | | DEEEEPPSMTQLLR [113], DLLPEDFVVYTYNK [134], EVPIYANR [236], KGFGGTAGMAFVGTVCSR [419], QPGSVPR [584], QPQQFPSRPPPPQPK [586], RDQLWR [600] |

Table 20c - Ovarian cancer

| MW (Da) | Subfractionation | Tryptics identified [SEQ ID No] |
|---|---|---|
| 49732 | | EVPIYANR [236], KGFGGTAGMAFVGTVCSR [419], QPQQFPSRPPPPQPK [586] |
| 50782 | | DQLWR [152], FAVPTYAAK [244], HVSPVTPPR [346], KGFGGTAGMAFVGTVCSR [419], QPQQFPSRPPPPQPK [586], SYFRK [666] |
| 54191 | | FAVPTYAAK [244], FLPGGTLCR [260], FPSGTLR [264], QPQQFPSRPPPPQPK [586], SYFRK [666] |

Table 20d - Pancreatic cancer

| MW (Da) | Subfractionation | Tryptics identified [SEQ ID No] |
|---|---|---|
| 73243 | | EEMILLANYLDSMYIMLNIR [179], EVPIYANR [236], HVSPVTPPR [346], KGFGGTAGMAFVGTVCSR [419], QPQQFPSRPPPPQPK [586] |

**OGTA106**

Table 21a - Cervical cancer

| MW (Da) | Subfractionation | Tryptics identified [SEQ ID No] |
|---|---|---|
| 61458 | MonoQ Aqueous | SLSGVGAGGGPTPR [642] |

Table 21b - Melanoma

| MW (Da) | Subfractionation | Tryptics identified [SEQ ID No] |
|---|---|---|
| 75458 | | CHIQASGRPQCSCMPGWTGEQCQLR [103], EQTPLFLAAR [221], ETDSLSAGFVVVMGVDLSR [229], GPRPNPAIMR [310], GSPQLDCGPPALQEMPINQGGEGKK [318], TLSAGAGPR [705], TPLHAAVAADAR [717] |

Table 21c - Pancreatic cancer

| MW (Da) | Subfractionation | Tryptics identified [SEQ ID No] |
|---|---|---|
| 88318 | | EQTPLFLAAR [221] |

**OGTA112**

Table 22a - Breast cancer

| MW (Da) | Subfractionation | Tryptics identified [SEQ ID No] |
|---|---|---|
| | Triton X114 | ETCVDLHLPR [228], FMFDLFQQFR [261], FYQTSVESTDFANAPEESR [280], FYQTSVESTDFANAPEESRKK [281], KFYQTSVESTDFANAPEESRK [416], KINSWVESQTNEK [420], LMEWTSLQNMR [470], QYNSFNFALLEDVQAK [594], TNSILFYGR [713], TYQFLQEYLDAIK [741], VLHFDQVTENTTEK [781] |

Table 22b - Cervical cancer

| MW (Da) | Subfractionation | Tryptics identified [SEQ ID No] |
|---|---|---|
| 133844 | MonoQ Aqueous | FMFDLFQQFRK [262], LMEWTSLQNMR [470], SVQMMR [665], TMGMVNIFNGDADLSGMTWSHGLSVSK [706], VLEIPYK [779] |

Table 22c - Chronic lymphocytic leukaemia

| MW (Da) | Subfractionation | Tryptics identified [SEQ ID No] |
|---|---|---|
| 34501 | | ETCVDLHLPR [228], FMFDLFQQFR [261] |

Table 22d - Hepatocellular carcinoma

| MW (Da) | Subfractionation | Tryptics identified [SEQ ID No] |
|---|---|---|
| 26010 | | AATYHVDR [54], ETCVDLHLPR [228], FMFDLFQQFR [261], INSWVESQTNEK [384], LMEWTSLQNMR [470], QYNSFNFALLEDVQAK [594], TNSILFYGR [713], VLHFDQVTENTTEK [781] |

Table 22e - Pancreatic cancer

| MW (Da) | Subfractionation | Tryptics identified [SEQ ID No] |
|---|---|---|
| 37971 | | ETCVDLHLPR [228], FMFDLFQQFR [261], FMFDLFQQFRK [262], INSWVESQTNEK [384], LMEWTSLQNMR [470], QYNSFNFALLEDVQAK [594], SVQMMR [665], TNSILFYGR [713], VLHFDQVTENTTEK [781] |

(continued)

| MW (Da) | Subfractionation | Tryptics identified [SEQ ID No] |
|---|---|---|
| 77017 | | ETCVDLHLPR [228], FMFDLFQQFR [261], INSWVESQTNEK [384], LMEWTSLQNMR [470], QYNSFNFALLEDVQAK [594], SVQMMR [665] |

Table 22f - Renal cell cancer

| MW (Da) | Subfractionation | Tryptics identified [SEQ ID No] |
|---|---|---|
| 34256 | | INSWVESQTNEK [384], LMEWTSLQNMR [470], SVQMMR [665], TNSILFYGR [713], VLHFDQVTENTTEK [781] |

**OGTA113**

Table 23 - Pancreatic cancer

| MW (Da) | Subfractionation | Tryptics identified [SEQ ID No] |
|---|---|---|
| 32378 | | LEDPHVDIIR [449], LVHIIGALR [500], RGDFFYHSENPK [603], SEIINSK [627], TSSQPGFLER [730], YSYNTEWR [874] |
| 32754 | | LEDPHVDIIR [449], LVHIIGALR [500], RGDFFYHSENPK [603], TSSQPGFLER [730], YPEVGDLR [865], YSYNTEWR [874] |
| 42556 | | HVEMYQWVETEESR [345], LEDPHVDIIR [449], RGDFFYHSENPK [603], TSSQPGFLER [730], YPEVGDLR [865], YSYNTEWR [874] |
| 43294 | | HVEMYQWVETEESR [345], LEDPHVDIIR [449], LVHIIGALR [500], RGDFFYHSENPK [603], TSSQPGFLER [730], YSYNTEWR [874] |

**OGTA119**

Table 24a - B-cell non-Hodgkin's lymphoma

| MW (Da) | Subfractionation | Tryptics identified [SEQ ID No] |
|---|---|---|
| 115552 | | AQPPEAGPQGLHDLGR [83], DEQHQCSLGNLK [115], ERPPDHQHSAQVK [222], GANTHLSTFSFTK [285], VGNQIFQSR [765] |

Table 24b - Breast cancer

| MW (Da) | Subfractionation | Tryptics identified [SEQ ID No] |
|---|---|---|
| | | AEWLNK [64] |

(continued)

| MW (Da) | Subfractionation | Tryptics identified [SEQ ID No] |
|---|---|---|
| 70772 | | ALALLEDEER [75], AQPPEAGPQGLHDLGR [83], FQLSNSGPNSTIK [268], ISSNPNPVVQMSVGHK [393], NLIAFSEDGSDPYVR [542], VGNQIFQSR [765] |
| 98013 | | AEWLNK [64] |
| 121313 | | AEWLNK [64] |

Table 24c - Colorectal cancer

| MW (Da) | Subfractionation | Tryptics identified [SEQ ID No] |
|---|---|---|
| 96268 | Nucleotide Binding | ALALLEDEER [75], AQPPEAGPQGLHDLGR [83], ERPPDHQHSAQVK [222], FQLSNSGPNSTIK [268], LEWLTLMPNASNLDK [457], NLIAFSEDGSDPYVR [542], RQDLEVEVR [611], TNEPVWEENFTFFIHNPK [710] |

Table 24d - Gastric cancer

| MW (Da) | Subfractionation | Tryptics identified [SEQ ID No] |
|---|---|---|
| 56270 | | VDVGQQPLR [750] |

Table 24e - Hepatocellular carcinoma

| MW (Da) | Subfractionation | Tryptics identified [SEQ ID No] |
|---|---|---|
| 59358 | | ALALLEDEER [75], ERPPDHQHSAQVK [222] |
| 60649 | | ALALLEDEER [75], AQPPEAGPQGLHDLGR [83], RQDLEVEVR [611], VDVGQQPLR [750], VGNQIFQSR [765] |
| 73339 | | AEWLNK [64], AQPPEAGPQGLHDLGR [83], ERPPDHQHSAQVK [222], NLIAFSEDGSDPYVR [542], SDPYGIIR [622], VGNQIFQSR [765], VPLSQLLTSEDMTVSQR [792] |
| 84442 | | AEWLNK [64] |
| 87814 | | AQPPEAGPQGLHDLGR [83], DEQHQCSLGNLK [115], ERPPDHQHSAQVK [222], FQLSNSGPNSTIK [268], HMWPFICQFIEK [339], IHFIEAQDLQGK [368], NLIAFSEDGSDPYVR [542], TNEPVWEENFTFFIHNPK [710], VDVGQQPLR [750] |
| 98646 | | AEWLNK [64] |

Table 24f - Lung cancer

| MW (Da) | Subfractionation | Tryptics identified [SEQ ID No] |
|---|---|---|
| 92181 | | AQPPEAGPQGLHDLGR [83], ERPPDHQHSAQVK [222], HMWPFICQFIEK [339], ITVPLVSEVQIAQLR [398] |

Table 24g - Lymphoid leukaemia, unspecified

| MW (Da) | Subfractionation | Tryptics identified [SEQ ID No] |
|---|---|---|
| 85026 | | ETIEPAVR [230], FQLSNSGPNSTIK [268], GEGPEAGAGGAGGR [290], RQDLEVEVR [611], VDVGQQPLR [750] |
| 86516 | | AQPPEAGPQGLHDLGR [83], ERPPDHQHSAQVK [222], GEGPEAGAGGAGGR [290], HMWPFICQFIEK [339], IHFIEAQDLQGK [368], ISSNPNPVVQMSVGHK [393], NLIAFSEDGSDPYVR [542], RQDLEVEVR [611], VDVGQQPLR [750], VLVALASEELAK [784] |

Table 24h - Neuroblastoma

| MW (Da) | Subfractionation | Tryptics identified [SEQ ID No] |
|---|---|---|
| | Sodium Carbonate Scrub | AEWLNK [64] |

Table 24i - Osteosarcoma

| MW (Da) | Subfractionation | Tryptics identified [SEQ ID No] |
|---|---|---|
| 73285 | | AEWLNK [64] |

Table 24j - Pancreatic cancer

| MW (Da) | Subfractionation | Tryptics identified [SEQ ID No] |
|---|---|---|
| 80573 | | AQPPEAGPQGLHDLGR [83], ERPPDHQHSAQVK [222], GEGPEAGAGGAGGR [290], HMWPFICQFIEK [339], IHFIEAQDLQGK [368], NLIAFSEDGSDPYVR [542], SDPYGIIR [622], VGNQIFQSR [765], VYTENVDK [819] |
| 93931 | | ALALLEDEER [75], DEQHQCSLGNLK [115], ERPPDHQHSAQVK [222], GEGPEAGAGGAGGR [290], ISSNPNPVVQMSVGHK [393], VPLSQLLTSEDMTVSQR [792] |

Table 24k - Prostate cancer

| MW (Da) | Subfractionation | Tryptics identified [SEQ ID No] |
|---|---|---|
| | Digitonin Insoluble, Triton X114, Aqueous | AEWLNK [64] |

Table 24l- Renal cell cancer

| MW (Da) | Subfractionation | Tryptics identified [SEQ ID No] |
|---|---|---|
| 76269 | | AEWLNK [64], AQPPEAGPQGLHDLGR [83], ERPPDHQHSAQVK [222], HMWPFICQFIEK [339], SIQIHGTMR [638], TNEPVWEENFTFFIHNPK [710], VDVGQQPLR [750], VYTENVDKR [820] |

**OGTA124**

Table 25a - Hepatocellular carcinoma

| MW (Da) | Subfractionation | Tryptics identified [SEQ ID No] |
|---|---|---|
| 98003 | | DLAVFLYHLR [133], EGLAHLIQSCGLGGMR [188], ESSPFLSPLEASR [227], EWGDGIR [240], HGLPSADAPSLK [334], HNSVVLGWPYGWR [340], LDEDLHVK [446], LEEGPPHTK [451], LVLLNMPGPPR [501], MPHFTVVPVDGPR [521], NIAFYPSNHER [539], NLALFEEELDIRPK [541], SIFDPPVFPVCMLGNR [636] |
| 116154 | | ESSPFLSPLEASR [227], EWGDGIR [240], HGLPSADAPSLK [334], LEEGPPHTK [451], NIAFYPSNHER [539], TPNWRPR [720], YIEYQGAEK [855] |
| 126143 | | EHEEAESGEGTRRR [192], ESSPFLSPLEASR [227], EWGDGIR [240], HNSVVLGWPYGWR [340], LVSYTNLTQGAK [504], MPHFTVVPVDGPR [521], NSEGDENYMEFLEVLTEGLER [553], TFIDTVR [689], YMTETWDPSHAPDNFR [863] |
| 137186 | | AELDDSDGHGNHR [62], EVITIYS [233], LVSYTNLTQGAK [504], MPHFTVVPVDGPR [521], NIAFYPSNHER [539] |

Table 25b - Lung cancer

| MW (Da) | Subfractionation | Tryptics identified [SEQ ID No] |
|---|---|---|
| 153348 | | DQFDICAK [151], EHEEAESGEGTR [191], EHEEAESGEGTRRR [192], ESSPFLSPLEASR [227], EVITIYS [233], LVLLNMPGPPR [501], LVSYTNLTQGAK [504] |

Table 25c - Ovarian cancer

| MW (Da) | Subfractionation | Tryptics identified [SEQ ID No] |
|---|---|---|
| 69811 | | ESSPFLSPLEASR [227], EVITIYS [233], IQMTWTR [389], LNEVIVTR [472], MHTAVK [517], NIAFYPSNHER [539], NSEGDENYMEFLEVLTEGLER [553], TPNWRPR [720] |
| 74557 | | ESSPFLSPLEASR [227], GIDYYDR [300], NIAFYPSNHER [539], TFIDTVR [689], TPNWRPR [720] |

Table 25d - Pancreatic cancer

| MW (Da) | Subfractionation | Tryptics identified [SEQ ID No] |
|---|---|---|
| 137609 | | ELVHIKPDQSNVR [209], ESSPFLSPLEASR [227], HNSVVLGWPYGWR [340], LEEGPPHTK [451], LVSYTNLTQGAK [504], NIAFYPSNHER [539], SIFDPPVFPVCMLGNR [636] |

Table 25e - Renal cell cancer

| MW (Da) | Subfractionation | Tryptics identified [SEQ ID No] |
|---|---|---|
| 135833 | | HNSVVLGWPYGWR [340], IFTVAQMDDNSIQMK [364], LVSYTNLTQGAK [504], MPHFTVVPVDGPR [521], NIAFYPSNHER [539] |
| 154919 | | ESSPFLSPLEASR [227], HNSVVLGWPYGWR [340], IFTVAQMDDNSIQMKK [365], LVSYTNLTQGAK [504], SIFDPPVFPVCMLGNR [636] |

**OGTA126**

Table 26a - Breast cancer

| MW (Da) | Subfractionation | Tryptics identified [SEQ ID No] |
|---|---|---|
| 105325 | | AFMIIQEQR [66], GAEAFEIALPR [282], GLPSLTSVSWNISVPR [302], IYVVDLSNER [404], MALHLPWFHPR [508] |

Table 26b - Colorectal cancer

| MW (Da) | Subfractionation | Tryptics identified [SEQ ID No] |
|---|---|---|
| 104028 | | ASVSFLNFNLSNCER [87], MALHLPWFHPR [508], VEYYIPGSTTNPEVFK [757] |

Table 26c - Hepatocellular carcinoma

| MW (Da) | Subfractionation | Tryptics identified [SEQ ID No] |
|---|---|---|
| 82606 | | AFMIIQEQR [66], ASVSFLNFNLSNCER [87], EEGVFTVTPDTK [172], LATEEPPPR [444], LWMNVEK [505], QIGPGESCPDGVTHSISGR [580], TCSSNLTLTSGSK [679] |
| 86381 | | AFMIIQEQR [66], LATEEPPPR [444] |
| 88432 | | AFMIIQEQR [66], ASVSFLNFNLSNCER [87], IYVVDLSNER [404], LATEEPPPR [444], LWMNVEK [505], MALHLPWFHPR [508], NVSGFSIANR [561], QIGPGESCPDGVTHSISGR [580] |
| 90607 | | AFMIIQEQR [66], LATEEPPPR [444], NVSGFSIANR [561] |

Table 26d - Melanoma

| MW (Da) | Subfractionation | Tryptics identified [SEQ ID No] |
|---|---|---|
| 81352 | | DQVACLTFFK [153], IYVVDLSNER [404], KFVPGCFVCLESR [415] |
| 87990 | | AFMIIQEQR [66], DQVACLTFFK [153], IYVVDLSNER [404], MALHLPWFHPR [508] |

44

(continued)

| MW (Da) | Subfractionation | Tryptics identified [SEQ ID No] |
|---|---|---|
| 102001 | | AFMIIQEQR [66], ASVSFLNFNLSNCER [87], IYVVDLSNER [404], LATEEPPPR [444] |

Table 26e - Pancreatic cancer

| MW (Da) | Subfractionation | Tryptics identified [SEQ ID No] |
|---|---|---|
| 57018 | | QGLTVSFIPYFK [576], QIGPGESCPDGVTHSISGR [580], QPGNMAGNFNLSLQGCDQDAQSPGILR [583], VEYYIPGSTTNPEVFK [757] |

Table 26f - Prostate cancer

| MW (Da) | Subfractionation | Tryptics identified [SEQ ID No] |
|---|---|---|
| | | AFMIIQEQR [66], EEGVFTVTPDTK [172], IDATVVR [353], IYVVDLSNER [404], LWMNVEK [505], TPNWDR [719] |
| | | AFMIIQEQR [66], IYVVDLSNER [404], LATEEPPPR [444], TPNWDR [719] |
| | | AFMIIQEQR [66], EEGVFTVTPDTK [172], IYVVDLSNER [404], LWMNVEK [505], TPNWDR [719], VEYYIPGSTTNPEVFK [757] |

Table 26g - Renal cell cancer

| MW (Da) | Subfractionation | Tryptics identified [SEQ ID No] |
|---|---|---|
| 125611 | | KFVPGCFVCLESR [415], LSLVLVPAQK [487], SGVVCQTGR [633] |

**OGTA156**

Table 27a - Acute T-cell leukaemia

| MW (Da) | Subfractionation | Tryptics identified [SEQ ID No] |
|---|---|---|
| | | ADGISSTFSQR [61], ATGFPEPNPR [90], AYIFSIDEK [99], SILQEENR [637], THQAFMR [697], TINFAR [701] |
| | | ADGISSTFSQR [61], ATGFPEPNPR [90], AYIFSIDEK [99], DNQWLGVTLSR [147], LPTGGCYGVPPDLR [482], SILQEENR [637], SQHTTEVVGGAPQHEQIGK [648], TINFAR [701] |
| | | ATGFPEPNPR [90], AYIFSIDEK [99], SILQEENR [637], THQAFMR [697], TINFAR [701], VTVAIPLK [807] |
| | | ATGFPEPNPR [90], AYIFSIDEK [99], SILQEENR [637], SQHTTEVVGGAPQHEQIGK [648], THQAFMR [697] |

(continued)

| MW (Da) | Subfractionation | Tryptics identified [SEQ ID No] |
|---|---|---|
|  |  | ATGFPEPNPR [90], AYIFSIDEK [99], SILQEENR [637] |

Table 27b - B-cell non-Hodekin's lymphoma

| MW (Da) | Subfractionation | Tryptics identified [SEQ ID No] |
|---|---|---|
| 112390 |  | ADGISSTFSQR [61], ATGFPEPNPR [90], AYIFSIDEK [99], DNQWLGVTLSR [147], EASVHIQLEGRPSILEMDETSALK [167], LPVGLYFIK [484], NIFYIK [540], SDSAVLLR [624], SILQEENR [637], SQHTTEVVGGAPQHEQIGK [648], TINFAR [701], TYLAVGSMK [740] |
| 114990 |  | ADGISSTFSQR [61], ATGFPEPNPR [90], AYIFSIDEK [99], DNQWLGVTLSR [147], IAPCYQDYVK [351], KAESPPR [407], NIFYIK [540], SDSAVLLR [624], SILQEENR [637], SQHTTEVVGGAPQHEQIGK [648], STEEFPPLQPILQQK [654], TCLEER [678], THQAFMRK [698], TINFAR [701], TYLAVGSMK [740] |

Table 27c - Chronic lymphocytic leukaemia

| MW (Da) | Subfractionation | Tryptics identified [SEQ ID No] |
|---|---|---|
| 95173 |  | DNQWLGVTLSR [147], ELNILHEMK [205], SQHTTEVVGGAPQHEQIGK [648] |
| 168205 |  | EVPGYIVLFYNMSLDVNR [235], ILELEEK [376], SQHTTEVVGGAPQHEQIGK [648], STEEFPPLQPILQQK [654] |

Table 27d - Hepatocellular carcinoma

| MW (Da) | Subfractionation | Tryptics identified [SEQ ID No] |
|---|---|---|
| 66260 |  | IEGLQISK [361], VIELNK [768] |

**OGTA159**

Table 28a - Breast cancer

| MW (Da) | Subfractionation | Tryptics identified [SEQ ID No] |
|---|---|---|
| Unknown |  | ESPAQAPA [226], VAQPGPLEPEEPR [745] |

Table 28b - Chronic lymphocytic leukaemia

| MW (Da) | Subfractionation | Tryptics identified [SEQ ID No] |
|---|---|---|
| 24836 |  | LEEEQK [450] |

Table 28c - Colorectal cancer

| MW (Da) | Subfractionation | Tryptics identified [SEQ ID No] |
|---|---|---|
| 42137 | | AASAGQEPLHNEELAGAGR [53], EEQAQR [180], VAQPGPLEPEEPR [745] |
| 42590 | | AASAGQEPLHNEELAGAGR [53], VAQPGPLEPEEPR [745] |

Table 28d - Hepatocellular carcinoma

| MW (Da) | Subfractionation | Tryptics identified [SEQ ID No] |
|---|---|---|
| 44183 | | VAQPGPLEPEEPR [745] |

Table 28e - Melanoma

| MW (Da) | Subfractionation | Tryptics identified [SEQ ID No] |
|---|---|---|
| 21554 | | IQDLLAEGTITGVIDDR [387], VVLLEDLASQVGLR [811] |
| 21899 | | RDLGSR [598], VVLLEDLASQVGLR [811] |

Table 28f - Pancreatic cancer

| MW (Da) | Subfractionation | Tryptics identified [SEQ ID No] |
|---|---|---|
| 33531 | | AASAGQEPLHNEELAGAGR [53], EHEEYLK [193], VAQPGPLEPEEPR [745] |
| 33932 | | AASAGQEPLHNEELAGAGR [53], VAQPGPLEPEEPR [745] |

**OGTA168**

Table 29a - Glioblastoma

| MW (Da) | Subfractionation | Tryptics identified [SEQ ID No] |
|---|---|---|
| 82431 | MonoQ/Digitonin | AISTNSELSTFR [74], ESHWIGLTDSER [225], GSPGKPGPQGSSGDPGPPGPPGK [317], LQASGDALVDR [485], NDFQNLQQVFLQAK [534], NLITNLQR [543] |
| 99352 | MonoQ/Digitonin | GSPGKPGPQGSSGDPGPPGPPGK [317], GSQGPPGPTGNK [319], KLGDQTGK [423], LDTEVANLSVIMEEMK [448], LGDQTGKK [461], LQASGDALVDR [485], NDFQNLQQVFLQAK [534], SVDDTSQAIQR [661] |

Table 29b - Melanoma

| MW (Da) | Subfractionation | Tryptics identified [SEQ ID No] |
|---|---|---|
| 33451 | | GEPGPPGPAGER [292], LTAVESDLK [495], SVDDTSQAIQR [661] |

**OGTA169**

Table 30a - Breast cancer

| MW (Da) | Subfractionation | Tryptics identified [SEQ ID No] |
|---|---|---|
| 100968 | PNGase F Deglycosylation | FGAALTVLGDVNGDK [252] |

Table 30b - Chronic lymphocytic leukaemia

| MW (Da) | Subfractionation | Tryptics identified [SEQ ID No] |
|---|---|---|
|  | Triton X114, Detergent Soluble | AVIFTQVSR [96], DLQSSVTLDLALDPGR [138], ELNDIASKPSQEHIFK [204], ESHVAMHR [224], FQTHFTFEEFR [269], FQTHFTFEEFRR [270], GAVYLFHGVLGPSISPSHSQR [287], GVQSLVLGAPR [327], IAGSQLSSR [350], IAPPASDFLAHIQK [352], INHLIFR [383], LFHASYGAR [458], LTDVVIGAPGEEENR [496], NFATMMNFVR [535], NLRPMLAADAQR [545], QEQDIVFLIDGSGSISSR [567], SLHLTCDSAPVGSQGTWSTSCR [641], VEDFDALK [752], YAVYTVVSSHEQFTK [841] |
| 108778 |  | AVIFTQVSR [96], DVIPMADAAGIIR [161], ESHVAMHR [224], FQTHFTFEEFR [269], GVQSLVLGAPR [327], INHLIFR [383], LFHASYGAR [458], LTDVVIGAPGEEENR [496], NLRPMLAADAQR [545], QEQDIVFLIDGSGSISSR [567], YAVYTVVSSHEQFTK [841] |
| 111501 |  | AVIFTQVSR [96], DVIPMADAAGIIR [161], ELNDIASKPSQEHIFK [204], ESHVAMHR [224], FGAALTVLGDVNGDK [252], FQTHFTFEEFR [269], FQTHFTFEEFRR [270], GAVYLFHGVLGPSISPSHSQR [287], GVQSLVLGAPR [327], IAGSQLSSR [350], INHLIFR [383], LFHASYGAR [458], LTDVVIGAPGEEENR [496], NFATMMNFVR [535], NLRPMLAADAQR [545], NMYLTGLCFLLGPTQLTQR [548], QEQDIVFLIDGSGSISSR [567], YAIGVGLAFQNR [839], YAVYTVVSSHEQFTK [841], YQVNNLGQR [868] |
| 114387 |  | AVIFTQVSR [96], DVIPMADAAGIIR [161], EMMEEANGQIAPENGTQTPSPPSEK [210], ESHVAMHR [224], FQTHFTFEEFR [269], FQTHFTFEEFRR [270], GGAQITFLATFDVSPK [296], IAGSQLSSR [350], INHLIFR [383], KEGDSLDYK [409], LFHASYGAR [458], LTDVVIGAPGEEENR [496], NFATMMNFVR [535], NLRPMLAADAQR [545], VEDFDALK [752], YAIGVGLAFQNR [839], YAVYTVVSSHEQFTK [841], YQVNNLGQR [868] |
| 117454 |  | DVIPMADAAGIIR [161], FQTHFTFEEFR [269], GGQVSVCPLPR [299], IAGSQLSSR [350], INHLIFR [383], KEGDSLDYK [409], LFHASYGAR [458], LTDVVIGAPGEEENR [496], NMYLTGLCFLLGPTQLTQR [548], QEQDIVFLIDGSGSISSR [567], YQVNNLGQR [868] |

(continued)

| MW (Da) | Subfractionation | Tryptics identified [SEQ ID No] |
|---|---|---|
| 136634 | | AVIFTQVSR [96], CDVPSFSVQEELDFTLK [100], FQTHFTFEEFR [269], FQTHFTFEEFRR [270], GVQSLVLGAPR [327], INHLIFR [383], KEGDSLDYK [409], LTDVVIGAPGEEENR [496], NFATMMNFVR [535], NLRPMLAADAQR [545], NPVLDCSIAGCLR [552], SLHLTCDSAPVGSQGTWSTSCR [641], YQHTGK [867] |
| 139470 | | AVIFTQVSR [96], DSYLGYSTELALWK [158], FQTHFTFEEFR [269], GVQSLVLGAPR [327], INHLIFR [383], KEGDSLDYK [409], LTDVVIGAPGEEENR [496], NFATMMNFVR [535], NLRPMLAADAQR [545], QEQDIVFLIDGSGSISSR [567] |
| 142364 | | GGQVSVCPLPR [299] |
| 152450 | | AVIFTQVSR [96], AVISQFQRPSTQFSLMQFSNK [97], DIQNQLK [130], DVIPMADAAGIIR [161], ELNDIASKPSQEHIFK [204], ESHVAMHR [224], FQTHFTFEEFR [269], GNLSFGWVR [303], GVQSLVLGAPR [327], IAPPASDFLAHIQK [352], INHLIFR [383], KEGDSLDYK [409], LFHASYGAR [458], LFHASYGARR [459], LTDVVIGAPGEEENR [496], NLRPMLAADAQR [545], QEQDIVFLIDGSGSISSR [567], YAVYTVVSSHEQFTK [841], YQVNNLGQR [868] |
| 159984 | | AVIFTQVSR [96], DIQNQLK [130], DVIPMADAAGIIR [161], ESHVAMHR [224], FQTHFTFEEFR [269], GAVYLFHGVLGPSISPSHSQR [287], GVQSLVLGAPR [327], INHLIFR [383], KEGDSLDYK [409], LFHASYGAR [458], LTDVVIGAPGEEENR [496], NLRPMLAADAQR [545], NMYLTGLCFLLGPTQLTQR [548], QEQDIVFLIDGSGSISSR [567], YAVYTVVSSHEQFTK [841], YQVNNLGQR [868] |

**OGTA174**

Table 31a - Acute T-cell leukaemia

| MW (Da) | Subfractionation | Tryptics identified [SEQ ID No] |
|---|---|---|
| | | CQGQLEVYLK [107], HLPETEAGR [337], LSQCHELWER [490], LSWYDPDFQAR [493], NSYCKK [554], QGAQWAALCDSSSAR [573], SHAENPTASHVDNEYSQPPR [634], TTPPTTRPPPTTTPEPTAPPR [734], VLALLCSGFQPK [771], WEEVCR [826] |
| | | HLPETEAGR [337], NSYCKK [554], QGAQWAALCDSSSAR [573], VLDAGDPTSR [775] |
| | | VLDAGDPTSR [775] |

Table 31b - Chronic lymphocytic leukaemia

| MW (Da) | Subfractionation | Tryptics identified [SEQ ID No] |
|---|---|---|
| 52248 | | HLPETEAGR [337], LSWYDPDFQAR [493], TQDLENFLCNNLQCGSFLK [723] |
| 53229 | | LSWYDPDFQAR [493] |
| 70807 | | AQDPGEPR [81], HLPETEAGR [337], IQNSSCTSLEHCFRK [390], LSWYDPDFQAR [493], SHAENPTASHVDNEYSQPPR [634] |
| 72277 | | EHQPLPIQWK [194], HLPETEAGR [337], LSQCHELWER [490], LSWYDPDFQAR [493], SHAENPTASHVDNEYSQPPR [634], VLDAGDPTSR [775] |
| 73777 | | HLPETEAGR [337], LSWYDPDFQAR [493], SHAENPTASHVDNEYSQPPR [634], VLDAGDPTSR [775] |

**OGTA176**

Table 32a - B-cell non-Hodekin's lymphoma

| MW (Da) | Subfractionation | Tryptics identified [SEQ ID No] |
|---|---|---|
| 33825 | | ELAQSQEALQVEQR [202], ETLQSEEQQR [231], ITQLGQSAEDLQGSR [396], ITQLGQSAEDLQGSRR [397], YLQVSQQLQQTNR [862] |

Table 32b - Chronic lymphocytic leukaemia

| MW (Da) | Subfractionation | Tryptics identified [SEQ ID No] |
|---|---|---|
| 38233 | | ELAQSQEALQVEQR [202], ETLQSEEQQR [231], ETLQSEEQQRR [232], ITQLGQSAEDLQGSR [396], SEQPTASWR [630], SSLPYICEMTAFR [650], YLQVSQQLQQTNR [862] |
| 39836 | | ELAQSQEALQVEQR [202], ITQLGQSAEDLQGSR [396] |
| 40663 | | ELAQSQEALQVEQR [202], ITQLGQSAEDLQGSR [396] |

**OGTA177**

Table 33 - Chronic lymphocytic leukaemia

| MW (Da) | Subfractionation | Tryptics identified [SEQ ID No] |
|---|---|---|
| 81514 | | FLNLTSEK [259], MESEELADR [514], SLSNYPFDFQGAR [643] |
| 81757 | | SLSNYPFDFQGAR [643] |

(continued)

| MW (Da) | Subfractionation | Tryptics identified [SEQ ID No] |
|---------|------------------|-------------------------------|
| 83454 | | DIQVASNEILR [131], FLNLTSEK [259], SLSNYPFDFQGAR [643], SQHQETPVYLGATAGMR [647], YGIVLDAGSSHTSLYIYK [853], VTEMMK [801] |
| 83957 | | DIQVASNEILR [131], SLSNYPFDFQGAR [643], SQHQETPVYLGATAGMR [647], VTEMMKK [802] |
| 85186 | | SLSNYPFDFQGAR [643] |
| 86534 | | SQHQETPVYLGATAGMR [647] |
| 86954 | | DIQVASNEILR [131], SLSNYPFDFQGAR [643] |

**OGTA197**

Table 34a - Colorectal cancer

| MW (Da) | Subfractionation | Tryptics identified [SEQ ID No] |
|---------|------------------|-------------------------------|
| 93409 | | QSPEDVYFSK [587], TYVDPHTYEDPNQAVLK [742], WTPPQDSGGR [837], YKPMMIITEYMENGALDK [857] |
| 95846 | | MQQYTEHFMAAGYTAIEK [523], NGVSGLVTSR [538], VIGAGEFGEVYK [769], WTPPQDSGGR [837], YKPMMIITEYMENGALDK [857] |
| 101142 | | QSPEDVYFSK [587], TSVTVSDLEPHMNYTFTVEAR [733], YKPMMIITEYMENGALDK [857] |

Table 34b - Hepatocellular carcinoma

| MW (Da) | Subfractionation | Tryptics identified [SEQ ID No] |
|---------|------------------|-------------------------------|
| 75736 | | AINDGFR [73], LPSTSGSEGVPFR [481], TLADFDPR [702], YLANMNYVHR [860], YSEPPHGLTR [871] |
| 82606 | | AINDGFR [73], EVVLLDFAAAGGELGWLTHPYGK [239], IDTIAPDEITVSSDFEAR [359], LPSTSGSEGVPFR [481], RKNQR [605], TYVDPHTYEDPNQAVLK [742], VDFLGEAGIMGQFSHHNIIR [747], VVQMTNDDIK [813], YLANMNYVHR [860], YSEPPHGLTR [871] |
| 84442 | | AINDGFR [73], FTTEIHPSCVTR [278], IDTIAPDEITVSSDFEAR [359], LPSTSGSEGVPFR [481], MQQYTEHFMAAGYTAIEK [523], TYVDPHTYEDPNQAVLK [742], VVQMTNDDIKR [814], YKPMMIITEYMENGALDK [857], YLANMNYVHR [860], YSEPPHGLTR [871] |
| 86381 | | AINDGFR [73], QSPEDVYFSK [587] |

(continued)

| MW (Da) | Subfractionation | Tryptics identified [SEQ ID No] |
|---|---|---|
| 88432 | | AINDGFR [73], LPGHQKR [478], QSPEDVYFSK [587], VIGAGEFGEVYK [769], VSDFGLSR [796], VVQMTNDDIK [813], VVQMTNDDIKR [814], WTAPEAISYR [834], WTAPEAISYRK [835], YKPMMIITEYMENGALDK [857], YSEPPHGLTR [871] |
| 92956 | | KGDSNSYNVR [417], QSPEDVYFSK [587], RKNQR [605], VIGAGEFGEVYK [769], VVQMTNDDIK [813], WTAPEAISYR [834], YLANMNYVHR [860], YSEPPHGLTR [871] |
| 116695 | | LPSTSGSEGVPFR [481], MELQAAR [512], MQQYTEHFMAAGYTAIEK [523], TLADFDPR [702], VYYKK [822], YKPMMIITEYMENGALDK [857] |

Table 34c - Lung cancer

| MW (Da) | Subfractionation | Tryptics identified [SEQ ID No] |
|---|---|---|
| | | DQVNTVGIPI [154], EVPVAIK [238], FTTEIHPSCVTR [278], IDTIAPDEITVSSDFEAR [359], KKGDSNSYNVR [422], VIGAGEFGEVYK [769], VLEDDPEATYTTSGGK [777], VVQMTNDDIK [813], VVQMTNDDIKR [814], YKPMMIITEYMENGALDK [857] |
| | | KEVPVAIK [414], LPSTSGSEGVPFR [481], TSVTVSDLEPHMNYTFTVEAR [733], VLEDDPEATYTTSGGK [777], WTPPQDSGGR [837], YEVTYR [845], YLANMNYVHR [860], YSEPPHGLTR [871] |
| 113648 | | DGEFSVLQLVGMLR [121], LPSTSGSEGVPFR [481], MQQYTEHFMAAGYTAIEK [523], QSPEDVYFSK [587], RKNQR [605], VDFLGEAGIMGQFSHHNIIR [747], VIGAGEFGEVYK [769], VVQMTNDDIK [813], WTAPEAISYRK [835], YKPMMIITEYMENGALDK [857], YLANMNYVHR [860] |
| 116436 | | AINDGFR [73], FADIVSILDK [242], FTTEIHPSCVTR [278], GDSNSYNVRR [288], LPSTSGSEGVPFR [481], MQQYTEHFMAAGYTAIEK [523], NGVSGLVTSR [538], TASVSINQTEPPK [673], TLADFDPR [702], TVSEWLESIK [738], VIGAGEFGEVYK [769], WTPPQDSGGR [837], YEVTYR [845], YKPMMIITEYMENGALDK [857], YLANMNYVHR [860] |
| 122412 | | LPSTSGSEGVPFR [481], VYYKK [822], YLANMNYVHR [860], QSPEDVYFSK [587] |

Table 34d - Melanoma

| MW (Da) | Subfractionation | Tryptics identified [SEQ ID No] |
|---|---|---|
| 78319 | | APQDPASMPCTRPPSAPHYLTAVGMGAK [80], DGEFSVLQLVGMLR [121], EVPVAIK [238], FADIVSILDK [242], MELQAAR [512], STTSLSVSWSIPPPQQSR [660], SVGPLTR [662], TASVSINQTEPPK [673], TSVTVSDLEPHMNYTFTVEAR [733], VIGAGEFGEVYK [769], YKPMMIITEYMENGALDK [857], YLANMNYVHR [860] |

Table 34e - Osteosarcoma

| MW (Da) | Subfractionation | Tryptics identified [SEQ ID No] |
|---|---|---|
| 30076 | | LPSTSGSEGVPFR [481], VVQMTNDDIK [813], VVQMTNDDIKR [814], YEVTYRK [846], YKPMMIITEYMENGALDK [857], YLANMNYVHR [860] |
| 55008 | | AINDGFR [73], APQDPASMPCTRPPSAPHYLTAVGMGAK [80], DGEFSVLQLVGMLR [121], IDTIAPDEITVSSDFEAR [359], RKNQR [605], STTSLSVSWSIPPPQQSR [660], SVGPLTR [662], TASVSINQTEPPK [673], TNWVYR [715], TSVTVSDLEPHMNYTFTVEAR [733], TYVDPHTYEDPNQAVLK [742], VLEDDPEATYTTSGGK [777], WTPPQDSGGR [837], YKPMMIITEYMENGALDK [857] |
| 67385 | | AINDGFR [73], IDTIAPDEITVSSDFEAR [359], KGDSNSYNVR [417], LPTPMDCPSAIYQLMMQCWQQER [483], MQQYTEHFMAAGYTAIEK [523], NGVSGLVTSR [538], STTSLSVSWSIPPPQQSR [660], SVGPLTR [662], TASVSINQTEPPK [673], TLADFDPR [702], TNWVYR [715], TSVTVSDLEPHMNYTFTVEAR [733], TYVDPHTYEDPNQAVLK [742], VDFLGEAGIMGQFSHHNIIR [747], WTPPQDSGGR [837], YSEPPHGLTR [871] |
| 93496 | | ACFALLWGCALAAAAAAQGK [55], AINDGFR [73], FTTEIHPSCVTR [278], IDTIAPDEITVSSDFEAR [359], RKNQR [605], STTSLSVSWSIPPPQQSR [660], SVGPLTR [662], TASVSINQTEPPK [673], TNWVYR [715], TYVDPHTYEDPNQAVLK [742], VIGAGEFGEVYK [769], VVQMTNDDIKR [814], YEVTYRK [846], YLANMNYVHR [860], YSEPPHGLTR [871] |

Table 34f - Ovarian cancer

| MW (Da) | Subfractionation | Tryptics identified [SEQ ID No] |
|---|---|---|
| 63557 | | AINDGFR [73], FTTEIHPSCVTR [278], LPSTSGSEGVPFR [481], STTSLSVSWSIPPPQQSR [660], TASVSINQTEPPK [673], TNWVYR [715], VVQMTNDDIKR [814], WTPPQDSGGR [837], YEVTYR [845], YLANMNYVHR [860], YSEPPHGLTR [871] |

(continued)

| MW (Da) | Subfractionation | Tryptics identified [SEQ ID No] |
|---|---|---|
| 67618 | | ACFALLWGCALAAAAAAQGK [55], QSPEDVYFSK [587], RKNQR [605], SVGPLTR [662], TASVSINQTEPPK [673], TLADFDPR [702], TSVTVSDLEPHMNYTFTVEAR [733], VDFLGEAGIMGQFSHHNIIR [747], WTPPQDSGGR [837], YKPMMIITEYMENGALDK [857] |
| 69811 | | FADIVSILDK [242], KGDSNSYNVRR [418], RKNQR [605], TASVSINQTEPPK [673], TYVDPHTYEDPNQAVLK [742], VSDFGLSR [796] |
| 79417 | | ACFALLWGCALAAAAAAQGK [55], DGEFSVLQLVGMLR [121], IDTIAPDEITVSSDFEAR [359], SVGPLTR [662], TSVTVSDLEPHMNYTFTVEAR [733], TYVDPHTYEDPNQAVLK [742], VIGAGEFGEVYK [769], YKPMMIITEYMENGALDK [857], YLANMNYVHR [860], YSEPPHGLTR [871] |

Table 34g - Pancreatic cancer

| MW (Da) | Subfractionation | Tryptics identified [SEQ ID No] |
|---|---|---|
| 53057 | | AINDGFR [73], IDTIAPDEITVSSDFEAR [359], LEGVISK [454], LNVEER [474], LPSTSGSEGVPFR [481], SVGPLTR [662], VIGAGEFGEVYK [769], VVQMTNDDIK [813], YEVTYRKK [847] |
| 53737 | | IDTIAPDEITVSSDFEAR [359], LPSTSGSEGVPFR [481], SEQLKPLK [629], SVGPLTRK [663], YLANMNYVHR [860] |
| 93964 | | LPSTSGSEGVPFR [481], TVSEWLESIK [738], WTPPQDSGGR [837], YSEPPHGLTR [871] |
| 102286 | | VIGAGEFGEVYK [769], VVQMTNDDIKR [814], WTPPQDSGGR [837], YSEPPHGLTR [871] |
| 107693 | | KGDSNSYNVR [417], QSPEDVYFSK [587], TNWVYR [715], TYVDPHTYEDPNQAVLK [742], VIGAGEFGEVYK [769], VVQMTNDDIKR [814], WTPPQDSGGR [837], YLANMNYVHR [860], YSEPPHGLTR [871] |
| 110489 | | TASVSINQTEPPK [673], TLADFDPR [702], TYVDPHTYEDPNQAVLK [742], VIGAGEFGEVYK [769], WTPPQDSGGR [837] |
| 113324 | | FADIVSILDK [242], IDTIAPDEITVSSDFEAR [359], KGDSNSYNVR [417], SVGPLTR [662], TLADFDPR [702], TYVDPHTYEDPNQAVLK [742], VYYKK [822], WTPPQDSGGR [837], YEVTYR [845], YEVTYRK [846], YLANMNYVHR [860], YSEPPHGLTR [871] |

(continued)

| MW (Da) | Subfractionation | Tryptics identified [SEQ ID No] |
|---|---|---|
| 116302 | | IDTIAPDEITVSSDFEAR [359], TYVDPHTYEDPNQAVLK [742], VVQMTNDDIK [813], WTPPQDSGGR [837], YEVTYR [845], YLANMNYVHR [860], YSEPPHGLTR [871] |
| 116866 | | ACFALLWGCALAAAAAAQGK [55], AINDGFR [73], IDTIAPDEITVSSDFEAR [359], LPSTSGSEGVPFR [481], STTSLSVSWSIPPPQQSR [660], TYVDPHTYEDPNQAVLK [742], VIGAGEFGEVYK [769], VLEDDPEATYTTSGGK [777], WTAPEAISYR [834], WTPPQDSGGR [837], YKPMMIITEYMENGALDK [857], YLANMNYVHR [860], YSEPPHGLTR [871] |
| 122219 | | ACFALLWGCALAAAAAAQGK [55], AINDGFR [73], IDTIAPDEITVSSDFEAR [359], KGDSNSYNVR [417], LPSTSGSEGVPFR [481], MQQYTEHFMAAGYTAIEK [523], QSPEDVYFSK [587], STTSLSVSWSIPPPQQSR [660], TNWVYR [715], VIGAGEFGEVYK [769], WTAPEAISYR [834], WTPPQDSGGR [837], YKPMMIITEYMENGALDK [857], YSEPPHGLTR [871] |
| 180074 | | KGDSNSYNVR [417], KGDSNSYNVRR [418], LPSTSGSEGVPFR [481], MELQAAR [512], QSPEDVYFSK [587], TNWVYR [715], WTAPEAISYR [834], WTPPQDSGGR [837], YEVTYR [845], YSEPPHGLTR [871] |
| 184373 | | AINDGFR [73], LPSTSGSEGVPFR [481], QSPEDVYFSK [587], TNWVYR [715], WTAPEAISYR [834], WTPPQDSGGR [837], YEVTYR [845], YLANMNYVHR [860], YSEPPHGLTR [871] |
| 188775 | | LPSTSGSEGVPFR [481], TNWVYR [715], VIGAGEFGEVYK [769], VVQMTNDDIK [813], WTAPEAISYR [834], WTPPQDSGGR [837], YLANMNYVHR [860], YSEPPHGLTR [871] |

Table 34h - Prostate cancer

| MW (Da) | Subfractionation | Tryptics identified [SEQ ID No] |
|---|---|---|
| | | LPSTSGSEGVPFR [481], MELQAAR [512], YEVTYR [845] |
| | | LPSTSGSEGVPFR [481], NGVSGLVTSR [538], VVQMTNDDIK [813], WTPPQDSGGR [837], YSEPPHGLTR [871] |

Table 34i - Renal cell cancer

| MW (Da) | Subfractionation | Tryptics identified [SEQ ID No] |
|---|---|---|
| 88378 | | AINDGFR [73], KGDSNSYNVRR [418], LPSTSGSEGVPFR [481], TNWVYR [715], WTAPEAISYR [834], WTPPQDSGGR [837], YSEPPHGLTR [871] |

(continued)

| MW (Da) | Subfractionation | Tryptics identified [SEQ ID No] |
|---|---|---|
| 90442 | | AINDGFR [73], LPSTSGSEGVPFR [481], SVGPLTR [662], TNWVYR [715], TYVDPHTYEDPNQAVLK [742], VIGAGEFGEVYK [769], VVQMTNDDIKR [814], WTAPEAISYR [834], WTPPQDSGGR [837], YEVTYR [845], YLANMNYVHR [860], YSEPPHGLTR [871] |
| 117578 | | AINDGFR [73], TNWVYR [715], VIGAGEFGEVYK [769], VVQMTNDDIK [813], VVQMTNDDIKR [814], WTAPEAISYR [834], YLANMNYVHR [860], YSEPPHGLTR [871] |
| 122597 | | ACFALLWGCALAAAAAAQGK [55], TYVDPHTYEDPNQAVLK [742], VIGAGEFGEVYK [769], VVQMTNDDIKR [814], WTAPEAISYR [834], YEVTYRK [846], YLANMNYVHR [860], YSEPPHGLTR [871] |

**OGTA202**

Table 35 - Colorectal cancer

| MW (Da) | Subfractionation | Tryptics identified [SEQ ID No] |
|---|---|---|
| 51531 | Nucleotide Binding | FTQDTFR [277] |

**OGTA203**

Table 36a - Ovarian cancer

| MW (Da) | Subfractionation | Tryptics identified [SEQ ID No] |
|---|---|---|
| 69811 | | DNIVSYNDEGGGEEDTQAFDIGTLR [144], EDAQINTTIGSVTAQDPDAAR [168], RTPTAR [618], TALLNMDR [669], TPESSPPGTPIGR [716], TSGFPAKK [727], VEASNPYVEPR [751] |

Table 36b - Renal cell cancer

| MW (Da) | Subfractionation | Tryptics identified [SEQ ID No] |
|---|---|---|
| 82732 | | IVVEDVDEPPVFSK [402], KNGYNR [428], TSGFPAKK [727] |

**OGTA206**

Table 37a - Breast cancer

| MW (Da) | Subfractionation | Tryptics identified [SEQ ID No] |
|---|---|---|
| 27751 | Vesicles | VYDSLLALPQDLQAAR [817] |

Table 37b - Pancreatic cancer

| MW (Da) | Subfractionation | Tryptics identified [SEQ ID No] |
|---|---|---|
| 26738 | | VYDSLLALPQDLQAAR [817] |
| 27019 | | VYDSLLALPQDLQAAR [817] |

Table 37c - Prostate cancer

| MW (Da) | Subfractionation | Tryptics identified [SEQ ID No] |
|---|---|---|
| | Digitonin | VYDSLLALPQDLQAAR [817] |

**OGTA213**

Table 38 - Lung cancer

| MW (Da) | Subfractionation | Tryptics identified [SEQ ID No] |
|---|---|---|
| 27644 | | IVFTPTICK [401] |

**OGTA214**

Table 39 - Ovarian cancer

| MW (Da) | Subfractionation | Tryptics identified [SEQ ID No] |
|---|---|---|
| 65600 | | QCLIKK [565], SSSSNFLNYDLTLR [651] |
| 79331 | | ISVTVSETFDPEEK [394], SSSSNFLNYDLTLR [651] |
| 89348 | | ISVTVSETFDPEEK [394], SSSSNFLNYDLTLR [651] |

**OGTA216**

Table 40a - Breast cancer

| MW (Da) | Subfractionation | Tryptics identified [SEQ ID No] |
|---|---|---|
| 196008 | | AFYAPVHADDLR [69], EGAQYLMQAAGLGR [186], FQVDLVSENAGR [271], GPIVPLNVADQK [307], IDHDRR [356], KDWLQADMR [408], NVGLMICGHVHMGPR [555], SEIFNENFGPDFR [626] |
| 248063 | | DEGPAAAGDGLGRPLGPTPSQSR [114], DVVVSVEYSK [163], EGAQYLMQAAGLGR [186], FQVDLVSENAGR [271], NVGLMICGHVHMGPRR [556], SEIFNENFGPDFR [626] |

Table 40b - Colorectal cancer

| MW (Da) | Subfractionation | Tryptics identified [SEQ ID No] |
|---|---|---|
| | Heparin Binding | DAVVTYTAESK [110], DVVVSVEYSK [163], SEIFNENFGPDFR [626] |
| | Heparin Binding | KENIIAFEEIIEPYR [410], SEIFNENFGPDFR [626] |
| | Heparin Binding | KENIIAFEEIIEPYR [410], LNELLK [471], SEIFNENFGPDFR [626] |
| | Heparin Binding | AFYAPVHADDLR [69], EHSSTANIIVMSLPVAR [195], FQVDLVSENAGR [271], SEIFNENFGPDFR [626], SPGWRPAFK [645] |
| | Heparin Binding | DEGPAAAGDGLGRPLGPTPSQSR [114], GFFGYK [294], KENIIAFEEIIEPYR [410], SEIFNENFGPDFR [626] |
| | Heparin Binding | FQVDLVSENAGR [271], GGGAYYLISR [298], KENIIAFEEIIEPYR [410], SEIFNENFGPDFR [626] |
| | Heparin Binding | AAAAAAAAAAAAAAAGAGAGAK [49], DLPPILLVR [136], DNIYPAFQMFAK [145], EGAQYLMQAAGLGR [186], EHSSTANIIVMSLPVAR [195], EPFEDGFANGEESTPTR [216], FQVDLVSENAGR [271], IDFSDIMVLGDINTKPK [354], IDHYR [357], KENIIAFEEIIEPYR [410], SEIFNENFGPDFR [626] |
| | Heparin Binding | CMLNIWGVMLFIR [106], IDHDRR [356], ITDNELELYK [395], SEIFNENFGPDFR [626] |
| | Heparin Binding | DLPPILLVR [136], NVGLMICGHVHMGPR [555], SEIFNENFGPDFR [626] |
| | Heparin Binding | AMATLLSK [78], EGAQYLMQAAGLGR [186], GGGAYYLISR [298], GPIVPLNVADQK [307], NVGLMICGHVHMGPRR [556], SPGWRPAFK [645] |
| 121506 | | DEGPAAAGDGLGRPLGPTPSQSR [114], DLPPILLVR [136], DNIYPAFQMFAK [145], DWLQADMR [164], EGAQYLMQAAGLGR [186], EPFEDGFANGEESTPTR [216], IDFSDIMVLGDINTKPKK [355], IDHYR [357], KENIIAFEEIIEPYR [410], KSDLDTSKPLSEKPITHK [435], LNELLK [471], NVGLMICGHVHMGPRR [556], SEIFNENFGPDFR [626], TFGHNTMDAVPR [688], VELPGTAVPSVPEDAAPASR [754] |
| 125772 | | DLPPILLVR [136], DWLQADMR [164], EPFEDGFANGEESTPTR [216], KDWLQADMR [408], SEIFNENFGPDFR [626], SPGWRPAFK [645], TFGHNTMDAVPR [688], VELPGTAVPSVPEDAAPASR [754] |
| 140688 | | DLPPILLVR [136], DWLQADMR [164], EGAQYLMQAAGLGR [186], EPFEDGFANGEESTPTR [216], KDWLQADMR [408], KSDLDTSKPLSEKPITHK [435], NVGLMICGHVHMGPR [555], TFGHNTMDAVPR [688], VELPGTAVPSVPEDAAPASR [754] |

(continued)

| MW (Da) | Subfractionation | Tryptics identified [SEQ ID No] |
|---|---|---|
| 146522 | | DLPPILLVR [136], DWLQADMR [164], EGAQYLMQAAGLGR [186], EPFEDGFANGEESTPTR [216], IDHDRR [356], KDWLQADMR [408], KENIIAFEEIIEPYR [410], KSDLDTSKPLSEKPITHK [435], SEIFNENFGPDFR [626], TFGHNTMDAVPR [688], VELPGTAVPSVPEDAAPASR [754] |
| 167535 | | DWLQADMR [164], EPFEDGFANGEESTPTR [216], GPIVPLNVADQK [307], IDHDRR [356], SEIFNENFGPDFR [626], TFGHNTMDAVPR [688], VELPGTAVPSVPEDAAPASR [754] |
| 175447 | Nucleotide Binding | NNEPLR [549], SEIFNENFGPDFR [626], TFGHNTMDAVPR [688], VELPGTAVPSVPEDAAPASR [754] |
| 190098 | Nucleotide Binding | DAVVTYTAESK [110], EGAQYLMQAAGLGR [186], EMSIDQAK [213], LHEDDK [465], LNELLK [471], RAMATLLSK [596], SEIFNENFGPDFR [626], SPGWRPAFK [645], TFGHNTMDAVPR [688] |
| 218400 | Nucleotide Binding | DEGPAAAGDGLGRPLGPTPSQSR [114], EGAQYLMQAAGLGR [186], KENIIAFEEIIEPYR [410], KSDLDTSKPLSEKPITHK [435], SEIFNENFGPDFR [626] |
| 221484 | | DEGPAAAGDGLGRPLGPTPSQSR [114], EGAQYLMQAAGLGR [186], EPFEDGFANGEESTPTR [216], IDHDRR [356], KDWLQADMR [408], NVGLMICGHVHMGPRR [556], SEIFNENFGPDFR [626], TFGHNTMDAVPR [688] |
| 255000 | | EGAQYLMQAAGLGR [186], EPFEDGFANGEESTPTR [216], SEIFNENFGPDFR [626], TFGHNTMDAVPR [688], VELPGTAVPSVPEDAAPASR [754] |
| 301246 | | DEGPAAAGDGLGRPLGPTPSQSR [114], EGAQYLMQAAGLGR [186], EPFEDGFANGEESTPTR [216], IDHDRR [356], IDHYR [357], SEIFNENFGPDFR [626], TFGHNTMDAVPR [688] |

Table 40c - Pancreatic cancer

| MW (Da) | Subfractionation | Tryptics identified [SEQ ID No] |
|---|---|---|
| 148609 | | AFYAPVHADDLR [69], DVVVSVEYSK [163], GGGAYYLISR [298], NNEPLR [549], SEIFNENFGPDFR [626], VELPGTAVPSVPEDAAPASR [754] |
| 154726 | | DVVVSVEYSK [163], EPFEDGFANGEESTPTR [216], GPIVPLNVADQK [307], NVGLMICGHVHMGPR [555], SEIFNENFGPDFR [626] |

**OGTA222**

Table 41a - B-cell non-Hodgkin's lymphoma

| MW (Da) | Subfractionation | Tryptics identified [SEQ ID No] |
|---|---|---|
| 45662 | | AVGFGGDFDGVPR [95], TLEQMDVVHR [703] |
| 48028 | | ALYQLGMR [77], AVGFGGDFDGVPR [95], GALADNLLR [283], MYPETFLYVTSSAGIR [530], NVPDDVLR [559], RTLEQMDVVHR [617], TLEQMDVVHR [703], YPDLIAELLR [864], VPEGLEDVSK [791] |
| 48530 | | ALYQLGMR [77], AVGFGGDFDGVPR [95], GALADNLLR [283], MYPETFLYVTSSAGIR [530], TLEQMDVVHR [703], VPEGLEDVSK [791] |
| 49042 | | ALYQLGMR [77], AVGFGGDFDGVPR [95], GALADNLLR [283], MYPETFLYVTSSAGIR [530], TLEQMDVVHR [703], VPEGLEDVSK [791] |
| 66777 | | ALYQLGMR [77], AVGFGGDFDGVPR [95], GALADNLLR [283], TLEQMDVVHR [703], ATLQLSR [91] |
| 68315 | | ALYQLGMR [77], AVGFGGDFDGVPR [95], GALADNLLR [283], TLEQMDVVHR [703] |
| 69923 | | ALYQLGMR [77], ATLQLSR [91], AVGFGGDFDGVPR [95], GALADNLLR [283], TLEQMDVVHR [703] |
| 71607 | | ALYQLGMR [77], AVGFGGDFDGVPR [95], GALADNLLR [283], TLEQMDVVHR [703], VPEGLEDVSK [791] |

Table 41b - Colorectal cancer

| MW (Da) | Subfractionation | Tryptics identified [SEQ ID No] |
|---|---|---|
| 52940 | | ALYQLGMR [77], AVGFGGDFDGVPR [95], GALADNLLR [283], TLEQMDVVHR [703] |
| 55204 | | AVGFGGDFDGVPR [95] |
| 58558 | | AVGFGGDFDGVPR [95], TLEQMDVVHR [703] |

**OGTA236**

Table 42 - Pancreatic cancer

| MW (Da) | Subfractionation | Tryptics identified [SEQ ID No] |
|---|---|---|
| 73411 | | SVLGVITIVNLR [664] |

**OGTA237**

Table 43a - B-cell non-Hodgkin's lymphoma

| MW (Da) | Subfractionation | Tryptics identified [SEQ ID No] |
|---------|------------------|--------------------------------|
| 47536 | | IADFGLAR [349] |
| 48028 | | IADFGLAR [349] |
| 48530 | | IADFGLAR [349] |
| 49565 | | IADFGLAR [349] |
| 50098 | | IADFGLAR [349] |
| 51766 | | IADFGLAR [349] |
| 52346 | | IADFGLAR [349] |

Table 43b - Lymphoid leukaemia, unspecified

| MW (Da) | Subfractionation | Tryptics identified [SEQ ID No] |
|---------|------------------|--------------------------------|
| 52041 | | IADFGLAR [349] |
| 52618 | | IADFGLAR [349] |
| 55054 | | IADFGLAR [349] |

Table 43c - Prostate cancer

| MW (Da) | Subfractionation | Tryptics identified [SEQ ID No] |
|---------|------------------|--------------------------------|
| | | IADFGLAR [349] |
| | | IADFGLAR [349] |

**OGTA247**

Table 44a - Hepatocellular carcinoma

| MW (Da) | Subfractionation | Tryptics identified [SEQ ID No] |
|---------|------------------|--------------------------------|
| 102119 | | DATQITQGPR [109], EDTQVDSEARPMK [170], GYNVTYWR [330], STIEKK [659], YDIEFEDK [842], YFIEDGR [850] |
| 114377 | | DATQITQGPR [109], FFPYANGTLGIR [251], FHILFK [253], GYNVTYWR [330], LGTAMSHEIR [463], LSPYVHYTFR [489], STIEKK [659], VSWSPAEDHNAPIEK [798], VTFTCQASFDPSLQPSITWR [804], VTYQNHNK [808], WLRPSGPMPADR [828], WMDWNAPQVQYR [829], YDIEFEDK [842] |
| 116037 | | DATQITQGPR [109], EPIDLR [217], FQGIYR [266], LDCQVQGRPQPEVTWR [445], LGTAMSHEIR [463], YFIEDGR [850] |
| 118513 | | AQLLVVGSPGPVPR [82], ATNSMIDR [92], DATQITQGPR [109], EGPGEAIVR [189], FFPYANGTLGIR [251], LGTAMSHEIR [463], LSPYVHYTFR [489], VTYQNHNK [808], WLRPSGPMPADR [828], YFIEDGR [850] |

(continued)

| MW (Da) | Subfractionation | Tryptics identified [SEQ ID No] |
|---|---|---|
| 121636 | | DATQITQGPR [109], EPIDLR [217], FHILFK [253], FQGIYR [266], YDIEFEDK [842], YFIEDGR [850] |
| 123033 | | AQLLVVGSPGPVPR [82], CEASGKPEVQFR [101], DATQITQGPR [109], EGPGEAIVR [189], FFPYANGTLGIR [251], LSPYVHYTFR [489], VGEEDDGEYR [762], VQWRPQGTR [795], YFIEDGR [850] |
| 128000 | | AQLLVVGSPGPVPR [82], DATQITQGPR [109], LGTAMSHEIR [463], LSPYVHYTFR [489], YFIEDGR [850] |
| 200565 | | CEASGKPEVQFR [101], EPIDLR [217], INGIPVEELAK [382], VSWSPAEDHNAPIEK [798], WLRPSGPMPADR [828] |
| 209214 | | ATNSMIDR [92], DATQITQGPR [109], EGPGEAIVR [189], EPIDLR [217], GALILSNVQPSDTMVTQCEAR [284], HQMAVK [342] |
| 218613 | | AQLLVVGSPGPVPR [82], CEASGKPEVQFR [101], DLQELGDSDK [137], FFPYANGTLGIR [251], LGTAMSHEIR [463], WLRPSGPMPADR [828] |

Table 44b - Melanoma

| MW (Da) | Subfractionation | Tryptics identified [SEQ ID No] |
|---|---|---|
| 146479 | | AQLLVVGSPGPVPR [82], CEASGKPEVQFR [101], CLAENSLGSAR [105], DATQITQGPR [109], DGVHFKPK [126], EGPGEAIVR [189], EPIDLR [217], FQGIYR [266], GQLSFNLR [312], INGIPVEELAK [382], LGTAMSHEIR [463], LSPYVHYTFR [489], LVLSDLHLLTQSQVR [502], VGEEDDGEYR [762], VSWSPAEDHNAPIEK [798], WLRPSGPMPADR [828], WMDWNAPQVQYR [829], WRPVDLAQVK [831], YFIEDGR [850] |
| 152349 | | AQLLVVGSPGPVPR [82], CLAENSLGSAR [105], DATQITQGPR [109], DGVHFKPK [126], EGPGEAIVR [189], EPIDLR [217], FQGIYR [266], LGTAMSHEIR [463], LSPYVHYTFR [489], LVLSDLHLLTQSQVR [502], WLRPSGPMPADR [828], WMDWNAPQVQYR [829], WRPVDLAQVK [831], YFIEDGR [850] |
| 163936 | | AFGAPVPSVQWLDEDGTTVLQDER [65], AQLLVVGSPGPVPR [82], CEASGKPEVQFR [101], FQGIYR [266], GQLSFNLR [312], LGTAMSHEIR [463], LVLSDLHLLTQSQVR [502], VSWSPAEDHNAPIEK [798], WLRPSGPMPADR [828], WMDWNAPQVQYR [829], WRPVDLAQVK [831], YFIEDGR [850] |

(continued)

| MW (Da) | Subfractionation | Tryptics identified [SEQ ID No] |
|---|---|---|
| 172991 | | AFGAPVPSVQWLDEDGTTVLQDER [65], AQLLVVGSPGPVPR [82], CEASGKPEVQFR [101], CLAENSLGSAR [105], DHVVVPANTTSVILSGLRPYSSYHLEVQAFNGR [128], EELGVTVYQSPHSGSFTITGNNSNFAQR [178], EGPGEAIVR [189], EPIDLR [217], FHILFK [253], FQGIYR [266], GQLSFNLR [312], LVLSDLHLLTQSQVR [502], VGEEDDGEYR [762], VQWRPQGTR [795], VSWSPAEDHNAPIEK [798], WLRPSGPMPADR [828], WMDWNAPQVQYR [829], WRPVDLAQVK [831], YFIEDGR [850] |
| 193221 | | AFGAPVPSVQWLDEDGTTVLQDER [65], AQLLVVGSPGPVPR [82], FFPYANGTLGIR [251], FHILFK [253], FQGIYR [266], GALILSNVQPSDTMVTQCEAR [284], GEGNETTNMVITWKPLR [289], GQLSFNLR [312], INGIPVEELAK [382], LGTAMSHEIR [463], LVLSDLHLLTQSQVR [502], WLRPSGPMPADR [828], WMDWNAPQVQYR [829], WRPVDLAQVK [831] |

**OGTA248**

Table 45 - Renal cell cancer

| MW (Da) | Subfractionation | Tryptics identified [SEQ ID No] |
|---|---|---|
| 34832 | | TNQEVLDFVVGGGR [712] |

**OGTA249**

Table 46 - Ovarian cancer

| MW (Da) | Subfractionation | Tryptics identified [SEQ ID No] |
|---|---|---|
| | | VTLPAGPDILR [805] |
| | | MSAGGASVPPPPNPAVSFPPPR [524] |

**OGTA257**

Table 47 - Pancreatic cancer

| MW (Da) | Subfractionation | Tryptics identified [SEQ ID No] |
|---|---|---|
| 176135 | | LLQEAYMTPK [468] |

**OGTA271**

Table 48a - Breast cancer

| MW (Da) | Subfractionation | Tryptics identified [SEQ ID No] |
|---|---|---|
| 24841 | Vesicles | AHTDVGPGPESSPVLVR [72], AYIATQGPLAESTEDFWR [98], DHPPIPITDLADNIER [127], DSLLAHSSDPVEMRR [157], GVEGSDYINASFLDGYR [326], LGQVPPGESVTAMELEFK [462], LIADLQPNTEYSFVLMNR [467], MLWEHNSTIIVMLTK [519], QFQFTDWPEQGVPK [572], QNAYIATQGPLPETMGDFWR [581], SDMGVGVFTPTIEAR [621], YEGVVDMFQTVK [844], YQYFVVDPMAEYNMPQYILR [869] |
| 24993 | Vesicles | AALEYLGSFDHYAT [51], AYIATQGPLAESTEDFWR [98], DHPPIPITDLADNIER [127], DINSQQELQNITTDTR [129], DSLLAHSSDPVEMRR [157], FDLSMPHVQDPSLVR [246], GVEGSDYINASFLDGYR [326], HVVDGISR [347], ITWMKK [399], KQNAYIATQGPLPETMGDFWR [433], LGQVPPGESVTAMELEFK [462], LIADLQPNTEYSFVLMNR [467], LSVLEEEQLPPGFPSIDMGPQLK [492], MLSASTMLVQWEPPEEPNGLVR [518], MLWEHNSTIIVMLTK [519], NGVITQYSVAYEAVDGEDR [537], QFQFTDWPEQGVPK [572], TGEGFIDFIGQVHK [693], VPEDQTGLSGGVASFVCQATGEPKPR [789], YQYFVVDPMAEYNMPQYILR [869] |
| 55008 | | MVWEQR [529], YQYFVVDPMAEYNMPQYILR [869] |
| 55921 | | AALEYLGSFDHYAT [51], AYIATQGPLAESTEDFWR [98], DFLPVDPATSNGR [117], DHPPIPITDLADNIER [127], EHSSWDLVGLEK [196], GVEGSDYINASFLDGYR [326], HVVDGISR [347], ILYNGQSVEVDGHSMRK [379], KLIADLQPNTEYSFVLMNR [424], LENGEPR [455], LGQVPPGESVTAMELEFK [462], LNYQTPGMR [475], MLWEHNSTIIVMLTK [519], MVWEQR [529], NGVITQYSVAYEAVDGEDR [537], QFQFTDWPEQGVPK [572], RLNYQTPGMR [606], TGEGFIDFIGQVHK [693], YEGVVDMFQTVK [844] |
| 56324 | | DFLPVDPATSNGR [117], DHPPIPITDLADNIER [127], ILYNGQSVEVDGHSMR [378], LNYQTPGMR [475], MLWEHNSTIIVMLTK [519], QHGQIR [578], RLNYQTPGMR [606], TDEDVPSGPPRK [683], TVDIYGHVTCMR [735], VMCVSMGSTTVR [785], YEGVVDMFQTVK [844] |
| 56872 | | |

(continued)

| MW (Da) | Subfractionation | Tryptics identified [SEQ ID No] |
|---|---|---|
| 105325 | | AAGTEGPFQEVDGVATTR [50], ACNPLDAGPMVVHCSAGVGR [56], AYIATQGPLAESTEDFWR [98], DFLPVDPATSNGR [117], DINSQQELQNITTDTR [129], FDLSMPHVQDPSLVR [246], FEVIEFDDGAGSVLR [249], FTLTGLKPDTTYDIK [275], GPPSEAVR [308], GSGPLSPSIQSR [315], ILYNGQSVEVDGHSMR [378], MVWEQR [529], RPPNAWHK [609], SANYTCVAISSLGMIEATAQVTVK [619], SDMGVGVFTPTIEAR [621], TATMLCAAGGNPDPEISWFK [674], TATVVMMTR [676], TDEDVPSGPPRK [683], TGCFIVIDAMLER [692], TGEGFIDFIGQVHK [693], TSVLLSWEVPDSYK [732], VSWVPPPADSR [799] |
| 109891 | | AAGTEGPFQEVDGVATTR [50], ACNPLDAGPMVVHCSAGVGR [56], AYIATQGPLAESTEDFWR [98], DDQHFTVTGLHK [111], ELPGELLGYR [206], EQFGQDGPITVHCSAGVGR [220], FEVIEFDDGAGSVLR [249], ILYNGQSVEVDGHSMRK [379], KQNAYIATQGPLPETMGDFWR [433], SDMGVGVFTPTIEAR [621], YEGVVDMFQTVK [844] |

Table 48b - Cervical cancer

| MW (Da) | Subfractionation | Tryptics identified [SEQ ID No] |
|---|---|---|
| 118858 | Integral | AAGTEGPFQEVDGVATTR [50], AHTDVGPGPESSPVLVR [72], DDQHFTVTGLHK [111], DFLPVDPATSNGR [117], ELPGELLGYR [206], EPMDQKR [218], FEVIEFDDGAGSVLR [249], GSSAGGLQHLVSIR [321], QHGQIR [578], RPPNAWHK [609], TDEDVPSGPPR [682], TQQGVPAQPADFQAEVESDTR [725], TVDIYGHVTCMR [735], VSWVPPPADSR [799], WTEYR [836], YSIGGLSPFSEYAFR [872] |
| 124604 | Integral | AAGTEGPFQEVDGVATTR [50], ADEARPNTIDFGK [58], AHTDVGPGPESSPVLVR [72], DDQHFTVTGLHK [111], ELPGELLGYR [206], EPMDQKR [218], FDLSMPHVQDPSLVR [246], FEVIEFDDGAGSVLR [249], GSSAGGLQHLVSIR [321], NVLELSNVVR [558], RPPNAWHK [609], SDMGVGVFTPTIEAR [621], TQQGVPAQPADFQAEVESDTR [725], VLAVNSIGR [774], VSWVPPPADSR [799], VYYTPDSR [823], WTEYR [836], YSIGGLSPFSEYAFR [872] |

Table 48c - Colorectal cancer

| MW (Da) | Subfractionation | Tryptics identified [SEQ ID No] |
|---|---|---|
| | Heparin Binding | GSGPLSPSIQSR [315], MAPEPAPGR [509], SDMGVGVFTPTIEAR [621], TDEDVPSGPPR [682], TGEQAPSSPPR [694], TQQGVPAQPADFQAEVESDTR [725], YSAPANLYVR [870] |

Table 48d - Hepatocellular carcinoma

| MW (Da) | Subfractionation | Tryptics identified [SEQ ID No] |
|---------|------------------|-------------------------------|
| 72397   |                  | YANVIAYDHSR [840], YSAPANLYVR [870] |

Table 48e - Lung cancer

| MW (Da) | Subfractionation | Tryptics identified [SEQ ID No] |
|---------|------------------|-------------------------------|
|         |                  | AAGTEGPFQEVDGVATTR [50], ADEARPNTIDFGK [58], AHTDVGPGPESSPVLVR [72], DSLLAHSSDPVEMR [156], EHSSWDLVGLEK [196], FEVIEFDDGAGSVLR [249], GSGPLSPSIQSR [315], GTTYIFR [324], ILYNGQSVEVDGHSMR [378], ILYNGQSVEVDGHSMRK [379], KLIADLQPNTEYSFVLMNR [424], KQNAYIATQGPLPETMGDFWR [433], SGALQIESSEESDQGK [631], TAPDLLPHKPLPASAYIEDGR [671], TATMLCAAGGNPDPEISWFK [674], TMPVEQVFAK [708], TQQGVPAQPADFQAEVESDTR [725], TQRPAMVQTEDQYQLCYR [726], TVDIYGHVTCMR [735], YEGVVDMFQTVK [844], YSAPANLYVR [870] |
| 77419   |                  | AYIATQGPLAESTEDFWR [98], DDQHFTVTGLHK [111], GPPSEAVR [308], GVEGSDYINASFLDGYR [326], IIMYELVYWAAEDEDQQHK [372], KQNAYIATQGPLPETMGDFWR [433], LGQVPPGESVTAMELEFK [462], QFQFTDWPEQGVPK [572], RTHSPSSK [616], YEGVVDMFQTVK [844] |
| 78771   |                  | DHPPIPITDLADNIER [127], DINSQQELQNITTDTR [129], DSLLAHSSDPVEMRR [157], EHSSWDLVGLEK [196], GVEGSDYINASFLDGYR [326], ILYNGQSVEVDGHSMRK [379], LGQVPPGESVTAMELEFK [462], MLSASTMLVQWEPPEEPNGLVR [518], MLWEHNSTIIVMLTK [519], MVWEQR [529], QNAYIATQGPLPETMGDFWR [581], TAPDLLPHKPLPASAYIEDGR [671], TGEQAPSSPPRR [695], YANVIAYDHSR [840], YEGVVDMFQTVK [844] |

Table 48f - Osteosarcoma

| MW (Da) | Subfractionation | Tryptics identified [SEQ ID No] |
|---|---|---|
| 63624 | | AAGTEGPFQEVDGVATTR [50], AHTDVGPGPESSPVLVR [72], AYIATQGPLAESTEDFWR [98], DDQHFTVTGLHK [111], ELPGELLGYR [206], FDLSMPHVQDPSLVR [246], FEVIEFDDGAGSVLR [249], FTLTGLKPDTTYDIK [275], GYQVTYVR [331], ILYNGQSVEVDGHSMR [378], IQLSWLLPPQER [388], LIADLQPNTEYSFVLMNR [467], MLSASTMLVQWEPPEEPNGLVR [518], NGVITQYSVAYEAVDGEDR [537], QHGQIR [578], RPPNAWHK [609], RRQAER [614], RTHSPSSK [616], SDMGVGVFTPTIEAR [621], TQQGVPAQPADFQAEVESDTR [725], VLAFTAVGDGPPSPTIQVK [770], VPEDQTGLSGGVASFVCQATGEPKPR [789], VTFDPTSSYTLEDLKPDTLYR [803], YEGVVDMFQTVK [844], YQYFVVDPMAEYNMPQYILR [869], YSIGGLSPFSEYAFR [872] |

Table 48g - Pancreatic cancer

| MW (Da) | Subfractionation | Tryptics identified [SEQ ID No] |
|---|---|---|
| 73243 | | DDQHFTVTGLHK [111], DSLLAHSSDPVEMRR [157], GVEGSDYINASFLDGYR [326], LGQVPPGESVTAMELEFK [462], RLNYQTPGMR [606], TATMLCAAGGNPDPEISWFK [674], TMPVEQVFAK [708], VGGSMLTPR [764], YEGVVDMFQTVK [844] |
| 74307 | | AYIATQGPLAESTEDFWR [98], DEAIYECTATNSLGEINTSAK [112], DSLLAHSSDPVEMRR [157], FDLSMPHVQDPSLVR [246], GPPSEAVR [308], GVEGSDYINASFLDGYR [326], IIMYELVYWAAEDEDQQHK [372], LNYQTPGMR [475], MVWEQR [529], SDMGVGVFTPTIEAR [621], TAQSTPSAPPQK [672], TDEDVPSGPPRK [683], TGCFIVIDAMLER [692], YANVIAYDHSR [840], YEGVVDMFQTVK [844], YSIGGLSPFSEYAFR [872] |
| 115291 | | AAGTEGPFQEVDGVATTR [50], DINSQQELQNITTDTR [129], DSLLAHSSDPVEMRR [157], EHSSWDLVGLEK [196], EQFGQDGPITVHCSAGVGR [220], FQLAAR [267], LNYQTPGMR [475], RPPNAWHK [609], RTHSPSSK [616], SDMGVGVFTPTIEAR [621], TDEDVPSGPPRK [683], TGCFIVIDAMLER [692], TQQGVPAQPADFQAEVESDTR [725], YEGVVDMFQTVK [844], YQYFVVDPMAEYNMPQYILR [869], YSAPANLYVR [870] |

(continued)

| MW (Da) | Subfractionation | Tryptics identified [SEQ ID No] |
|---|---|---|
| 124451 | | DDQHFTVTGLHK [111], DEAIYECTATNSLGEINTSAK [112], DFLPVDPATSNGR [117], DHPPIPITDLADNIER [127], FDLSMPHVQDPSLVR [246], HNTDAGLLTTVGSLLPGITYSLR [341], LNYQTPGMR [475], MLWEHNSTIIVMLTK [519], QFQFMAWPDHGVPEYPTPILAFLR [571], RLNYQTPGMR [606], RTHSPSSK [616], TATVVMMTR [676], TGCFIVIDAMLER [692], TQRPAMVQTEDQYQLCYR [726], TSVLLSWEVPDSYK [732], VEVEPLNSTAVHVYWK [756], YEGVVDMFQTVK [844], YSAPANLYVR [870] |
| 126215 | | DDQHFTVTGLHK [111], DSLLAHSSDPVEMR [156], EHSSWDLVGLEK [196], ELPGELLGYR [206], GTTYIFR [324], LNYQTPGMR [475], MVWEQR [529], TATVVMMTR [676], VGGSMLTPR [764], WMMGAEELTK [830], YSAPANLYVR [870] |
| 129890 | | AAGTEGPFQEVDGVATTR [50], DSLLAHSSDPVEMR [156], ELPGELLGYR [206], FEVIEFDDGAGSVLR [249], GPPSEAVR [308], GSGPLSPSIQSR [315], HVVDGISR [347], LNYQTPGMR [475], NGVITQYSVAYEAVDGEDR [537], RLNYQTPGMR [606], RTHSPSSK [616], VSWVPPPADSR [799], YEGVVDMFQTVK [844], YSAPANLYVR [870], YSIGGLSPFSEYAFR [872] |

Table 48h - Renal cell cancer

| MW (Da) | Subfractionation | Tryptics identified [SEQ ID No] |
|---|---|---|
| 135833 | | AAGTEGPFQEVDGVATTR [50], EHSSWDLVGLEK [196], FEVIEFDDGAGSVLR [249], FQLAAR [267], SDMGVGVFTPTIEAR [621], TGEQAPSSPPR [694] |

**OGTA002**

Table 49a - Colorectal cancer, iTRAQ

| Samples batch no. | Experiment no. | Tryptics identified [SEQ ID No] |
|---|---|---|
| Samples 1 | Experiment 1 | AELRGLK [880], TLRLGPLSK [983] |
| Samples 2 | Experiment 1 | ILASVQHMK [373] |
| Samples 2 | Experiment 2 | ILASVQHMK [373] |

Table 49b - Kidney cancer, iTRAQ

| Sample no. | Experiment no. | Tryptics identified [SEQ ID No] |
|---|---|---|
| Samples 1 | Experiment 1 | ILASVQHMK [373] |

Table 49c - Liver cancer, iTRAQ

| Sample no. | Experiment no. | Tryptics identified [SEQ ID No] |
|---|---|---|
| Samples 1 | Experiment 1 | EIDVSYVK [899] |

Table 49d - Non-small cell lung cancer, iTRAQ

| Sample no. | Experiment no. | Tryptics identified [SEQ ID No] |
|---|---|---|
| Samples 1 | Experiment 1 | AELRGLK [880], KCAQLTVNLTR [927] |
| Samples 2 | Experiment 1 | QPHYSAFGSVGEWLRAIK [964] |

**OGTA009**

Table 50a - Colorectal cancer, iTRAQ

| Samples batch no. | Experiment no. | Tryptics identified [SEQ ID No] |
|---|---|---|
| Samples 1 | Experiment 1 | STGPGASLGTGYDR [657] |
| Samples 1 | Experiment 2 | DFYNPVVPEAQK [119] |
| Samples 2 | Experiment 1 | VYDSLLALPQDLQAAR [817] |
| Samples 2 | Experiment 2 | VYDSLLALPQDLQAAR [817] |
| Samples 2 | Experiment 3 | VYDSLLALPQDLQAAR [817] |
| Samples 2 | Experiment 4 | VYDSLLALPQDLQAAR [817] |

Table 50b - Non-small cell lung cancer, iTRAQ

| Sample no. | Experiment no. | Tryptics identified [SEQ ID No] |
|---|---|---|
| Samples 1 | Experiment 1 | VYDSLLALPQDLQAAR [817] |
| Samples 2 | Experiment 1 | VYDSLLALPQDLQAAR [817] |
| Samples 2 | Experiment 2 | VYDSLLALPQDLQAAR [817] |

Table 50c - Ovarian cancer, iTRAQ

| Sample no. | Experiment no. | Tryptics identified [SEQ ID No] |
|---|---|---|
| Samples 1 | Experiment 1 | DFYNPVVPEAQK [119] |
| Samples 1 | Experiment 2 | DFYNPVVPEAQK [119] |

**OGTA016**

Table 51a - Colorectal cancer, iTRAQ

| Samples batch no. | Experiment no. | Tryptics identified [SEQ ID No] |
|---|---|---|
| Samples 1 | Experiment 1 | ADAAPDEK [57], AFVNCDENSR [68], ASVDSGSSEEQGGSSR [86], LSDAGQYLCQAGDDSNSNKK [947], NADLQVLKPEPELVYEDLR [955], QSSGENCDVVVNTLGK [588], VYTVDLGR [821] |
| Samples 1 | Experiment 2 | ADAAPDEK [57] |
| Samples 2 | Experiment 1 | ADAAPDEK [57], ASVDSGSSEEQGGSSR [86] |
| Samples 2 | Experiment 2 | AAGSRDVSLAKADAAPDEK [877] |
| Samples 2 | Experiment 3 | ADAAPDEK [57], IIEGEPNLK [371], ILLNPQDK [377], VYTVDLGR [821] |
| Samples 2 | Experiment 4 | ASVDSGSSEEQGGSSR [86] |
| Samples 2 | Experiment 5 | ASVDSGSSEEQGGSSR [86] |
| Samples 2 | Experiment 6 | ASVDSGSSEEQGGSSR [86], IIEGEPNLK [371], ILLNPQDK [377], QSSGENCDVVVNTLGK [588] |

Table 51b - Non-small cell lung cancer, iTRAQ

| Sample no. | Experiment no. | Tryptics identified [SEQ ID No] |
|---|---|---|
| Samples 1 | Experiment 1 | ADAAPDEK [57], ASVDSGSSEEQGGSSR [86], ILLNPQDK [377] |
| Samples 2 | Experiment 1 | IIEGEPNLK [371] |
| Samples 2 | Experiment 2 | ESKSIK [907], TVTINCPFK [987] |

**OGTA028**

Table 52a - Colorectal cancer, iTRAQ

| Samples batch no. | Experiment no. | Tryptics identified [SEQ ID No] |
|---|---|---|
| Samples 1 | Experiment 1 | AEDIIK [879], DIAPVLDLK [890] |
| Samples 2 | Experiment 1 | DIAPVLDLK [890] |
| Samples 2 | Experiment 2 | DIAPVLDLK [890], EEINQGGR [177], IDEKR [919] |
| Samples 2 | Experiment 3 | DIAPVLDLK [890], SSVKELLK [973] |
| Samples 2 | Experiment 4 | DIAPVLDLK [890] |
| Samples 2 | Experiment 5 | DIAPVLDLK [890] |
| Samples 2 | Experiment 6 | DIAPVLDLK [890] |

Table 52b - Kidney cancer, iTRAQ

| Sample no. | Experiment no. | Tryptics identified [SEQ ID No] |
|---|---|---|
| Samples 1 | Experiment 1 | DIAPVLDLK [890], EEINQGGR [177] |
| Samples 1 | Experiment 2 | DIAPVLDLK [890], EEINQGGR [177] |

(continued)

| Sample no. | Experiment no. | Tryptics identified [SEQ ID No] |
|---|---|---|
| Samples 1 | Experiment 3 | DIAPVLDLK [890] |

Table 52c - Liver cancer, iTRAQ

| Sample no. | Experiment no. | Tryptics identified [SEQ ID No] |
|---|---|---|
| Samples 1 | Experiment 1 | VKTTSLTEKK [992] |
| Samples 1 | Experiment 2 | DIAPVLDLK [890], WSNVFK [832] |

Table 52d - Non-small cell lung cancer, iTRAQ

| Sample no. | Experiment no. | Tryptics identified [SEQ ID No] |
|---|---|---|
| Samples 1 | Experiment 1 | DIAPVLDLK [890], LKNKEEVLK [933], VLLEK [995] |
| Samples 1 | Experiment 2 | DIAPVLDLK [890] |
| Samples 2 | Experiment 1 | DIAPVLDLK [890] |
| Samples 2 | Experiment 2 | DIAPVLDLK [890] |
| Samples 2 | Experiment 3 | DIAPVLDLK [890] |
| Samples 2 | Experiment 4 | DIAPVLDLK [890], DVLPQK [897] |
| Samples 2 | Experiment 5 | DIAPVLDLK [890], EEINQGGR [177] |

Table 52e - Small cell lung cancer, iTRAQ

| Sample no. | Experiment no. | Tryptics identified [SEQ ID No] |
|---|---|---|
| Samples 1 | Experiment 1 | DIAPVLDLK [890] |
| Samples 1 | Experiment 2 | DIAPVLDLK [890], EEINQGGR [177] |

Table 52f - Ovarian cancer, iTRAQ

| Sample no. | Experiment no. | Tryptics identified [SEQ ID No] |
|---|---|---|
| Samples 1 | Experiment 1 | DIAPVLDLK [890] |
| Samples 1 | Experiment 2 | DIAPVLDLK [890] |
| Samples 1 | Experiment 3 | DIAPVLDLK [890] |
| Samples 1 | Experiment 4 | DIAPVLDLK [890] |

**OGTA037**

Table 53a - Colorectal cancer, iTRAQ

| Samples batch no. | Experiment no. | Tryptics identified [SEQ ID No] |
|---|---|---|
| Samples 1 | Experiment 1 | VLAANNVR [993] |

Table 53b - Non-small cell lung cancer, iTRAQ

| Sample no. | Experiment no. | Tryptics identified [SEQ ID No] |
|---|---|---|
| Samples 1 | Experiment 1 | VLAANNVR [993] |

Table 53c - Ovarian cancer, iTRAQ

| Sample no. | Experiment no. | Tryptics identified [SEQ ID No] |
|---|---|---|
| Samples 1 | Experiment 1 | VLAANNVR [993] |

**OGTA041**

Table 54 - Non-small cell lung cancer, iTRAQ

| Sample no. | Experiment no. | Tryptics identified [SEQ ID No] |
|---|---|---|
| Samples 1 | Experiment 1 | LTWTNPSIK [950] |
| Samples 2 | Experiment 1 | LTWTNPSIK [950] |

**OGTA053**

Table 55a - Colorectal cancer, iTRAQ

| Samples batch no. | Experiment no. | Tryptics identified [SEQ ID No] |
|---|---|---|
| Samples 1 | Experiment 1 | VVISIR [1002] |
| Samples 2 | Experiment 1 | TELLAADSLSK [980] |
| Samples 2 | Experiment 2 | VVISIR [1002] |
| Samples 2 | Experiment 3 | IRTSTPTGHGASPAK [924] |
| Samples 2 | Experiment 4 | TELLAADSLSK [980] |
| Samples 2 | Experiment 5 | IRTSTPTGHGASPAK [924], TELLAADSLSK [980] |

Table 55b - Kidney cancer, iTRAQ

| Sample no. | Experiment no. | Tryptics identified [SEQ ID No] |
|---|---|---|
| Samples 1 | Experiment 1 | TELLAADSLSK [980] |
| Samples 1 | Experiment 2 | IRTSTPTGHGASPAK [924], TELLAADSLSK [980] |
| Samples 1 | Experiment 3 | IRTSTPTGHGASPAK [924] |

Table 55c - Liver cancer, iTRAQ

| Sample no. | Experiment no. | Tryptics identified [SEQ ID No] |
|---|---|---|
| Samples 1 | Experiment 1 | IRTSTPTGHGASPAK [924] |

Table 55d - Non-small cell lung cancer, iTRAQ

| Sample no. | Experiment no. | Tryptics identified [SEQ ID No] |
|---|---|---|
| Samples 1 | Experiment 1 | IRTSTPTGHGASPAK [924], TELLAADSLSK [980] |
| Samples 1 | Experiment 2 | GTLSPKDALTDLTGDAER [916], IRTSTPTGHGASPAK [924], TELLAADSLSK [980] |
| Samples 2 | Experiment 1 | IRTSTPTGHGASPAK [924] |
| Samples 2 | Experiment 2 | IRTSTPTGHGASPAK [924], TELLAADSLSK [980] |

Table 55e - Small cell lung cancer, iTRAQ

| Sample no. | Experiment no. | Tryptics identified [SEQ ID No] |
|---|---|---|
| Samples 1 | Experiment 1 | TELLAADSLSK [980] |
| Samples 1 | Experiment 2 | TELLAADSLSK [980] |

**OGTA054**

Table 56a - Colorectal cancer, iTRAQ

| Samples batch no. | Experiment no. | Tryptics identified [SEQ ID No] |
|---|---|---|
| Samples 1 | Experiment 1 | EEHEVAVLGAPPSTILPR [173], MVGDVTGAQAYASTAK [954] |
| Samples 2 | Experiment 1 | MVGDVTGAQAYASTAK [954] |

Table 56b -Non-small cell lung cancer, iTRAQ

| Sample no. | Experiment no. | Tryptics identified [SEQ ID No] |
|---|---|---|
| Samples 1 | Experiment 1 | MVGDVTGAQAYASTAK [954] |
| Samples 1 | Experiment 2 | MVGDVTGAQAYASTAK [954] |

**OGTA066**

Table 57a -Colorectal cancer iTRAQ

| Samples batch no. | Experiment no. | Tryptics identified [SEQ ID No] |
|---|---|---|
| Samples 1 | Experiment 1 | IMFVDPSLTVR [380] [3] |
| Samples 1 | Experiment 2 | IMFVDPSLTVR [380] [3] |
| Samples 2 | Experiment 1 | IMFVDPSLTVR [380] [3] |
| Samples 2 | Experiment 2 | DLPLLIENMK [135] [2], IMFVDPSLTVR [380] [3] |
| Samples 2 | Experiment 3 | IMFVDPSLTVR [380] [3] |
| Samples 2 | Experiment 4 | DLPLLIENMK [135] [2], IMFVDPSLTVR [380] [3] |
| Samples 2 | Experiment 5 | IMFVDPSLTVR [380] [3] |

Table 57b - Kidney cancer iTRAQ

| Sample no. | Experiment no. | Tryptics identified [SEQ ID No] |
|---|---|---|
| Samples 1 | Experiment 1 | DLPLLIENMK [135] [2] |

Table 57c -Non-small cell lung cancer iTRAQ

| Sample no. | Experiment no. | Tryptics identified [SEQ ID No] |
|---|---|---|
| Samples 1 | Experiment 1 | DLPLLIENMK [135] [2], IMFVDPSLTVR [380] [3], NLSPDGQYVPR [546] [5] |
| Samples 2 | Experiment 1 | IMFVDPSLTVR [380] [3] |
| Samples 2 | Experiment 2 | IMFVDPSLTVR [380] [3] |
| Samples 2 | Experiment 3 | IMFVDPSLTVR [380] [3] |
| Samples 2 | Experiment 4 | IMFVDPSLTVR [380] [3] |

**OGTA074**

Table 58 -Non-small cell lung cancer, iTRAQ

| Sample no. | Experiment no. | Tryptics identified [SEQ ID No] |
|---|---|---|
| Samples 1 | Experiment 1 | CLNPVPAVPSPSPSVRK [888] |

**OGTA076**

Table 59a- Colorectal cancer iTRAQ

| Samples batch no. | Experiment no. | Tryptics identified [SEQ ID No] |
|---|---|---|
| Samples 1 | Experiment 1 | EGIAK [898] [14], IIPK [921] [28], LALK [929] [35], LNPK [941] [40] |
| Samples 1 | Experiment 2 | EGIAK [898] [14], LALK [929] [35] |
| Samples 1 | Experiment 3 | EGIAK [898] [14], IIPK [921] [28], LALK [929] [35] |
| Samples 1 | Experiment 4 | LNPK [941] [40] |

Table 59b - Kidney cancer iTRAQ

| Sample no. | Experiment no. | Tryptics identified [SEQ ID No] |
|---|---|---|
| Samples 1 | Experiment 1 | EGIAK [898] [14], LALK [929] [35], LNPK [941] [40], YLNNLYK [1006] [66] |
| Samples 1 | Experiment 2 | LALK [929] [35] |

Table 59c - Liver cancer iTRAQ

| Sample no. | Experiment no. | Tryptics identified [SEQ ID No] |
|---|---|---|
| Samples 1 | Experiment 1 | YLNNLYK [1006] [66] |

Table 59d - Non-small cell lung cancer iTRAQ

| Sample no. | Experiment no. | Tryptics identified [SEQ ID No] |
|---|---|---|
| Samples 1 | Experiment 1 | YLNNLYK [1006] [66] |
| Samples 1 | Experiment 2 | LALK [929] [35] |
| Samples 2 | Experiment 1 | IIPK [921] [28], LALK [929] [35], YLNNLYK [1006] [66] |
| Samples 2 | Experiment 2 | YLNNLYK [1006] [66] |

Table 59e- Small cell lung cancer iTRAQ

| Sample no. | Experiment no. | Tryptics identified [SEQ ID No] |
|---|---|---|
| Samples 1 | Experiment 1 | EGIAK [898] [14] |
| Samples 1 | Experiment 2 | EGIAK [898] [14] |

Table 59f - Ovarian cancer iTRAQ

| Sample no. | Experiment no. | Tryptics identified [SEQ ID No] |
|---|---|---|
| Samples 1 | Experiment 1 | LALK [929] [35] |
| Samples 1 | Experiment 2 | LALK [929] [35], YLNNLYK [1006] [66] |
| Samples 1 | Experiment 3 | LALK [929] [35] |

**OGTA085**

Table 60a - Colorectal cancer, iTRAQ

| Samples batch no. | Experiment no. | Tryptics identified [SEQ ID No] |
|---|---|---|
| Samples 1 | Experiment 1 | KMLGNPSR [427] |

Table 60b - Non-small cell lung cancer, iTRAQ

| Sample no. | Experiment no. | Tryptics identified [SEQ ID No] |
|---|---|---|
| Samples 1 | Experiment 1 | KMLGNPSR [427] |

**OGTA088**

Table 61a - Colorectal cancer, iTRAO

| Samples batch no. | Experiment no. | Tryptics identified [SEQ ID No] |
|---|---|---|
| Samples 1 | Experiment 1 | RQEELERK [613] |

Table 61b - Non-small cell lung cancer, iTRAQ

| Sample no. | Experiment no. | Tryptics identified [SEQ ID No] |
|---|---|---|
| Samples 1 | Experiment 1 | RQEELERK [613] |

**OGTA089**

Table 62a - Kidney cancer, iTRAQ

| Sample no. | Experiment no. | Tryptics identified [SEQ ID No] |
|---|---|---|
| Samples 1 | Experiment 1 | GHEVAAMLK [913] |

Table 62b - Non-small cell lung cancer, iTRAQ

| Sample no. | Experiment no. | Tryptics identified [SEQ ID No] |
|---|---|---|
| Samples 1 | Experiment 1 | LLHGQNGSVPNGQTPLK [935] |

Table 62c - Ovarian cancer, iTRAQ

| Sample no. | Experiment no. | Tryptics identified [SEQ ID No] |
|---|---|---|
| Samples 1 | Experiment 1 | NNLVEIILDINVSQLTER [956] |

**OGTA091**

Table 63a - Colorectal cancer, iTRAQ

| Samples batch no. | Experiment no. | Tryptics identified [SEQ ID No] |
|---|---|---|
| Samples 1 | Experiment 1 | IYIVR [926] |
| Samples 2 | Experiment 1 | KRSAPTSR [928] |

Table 63b - Kidney cancer, iTRAQ

| Sample no. | Experiment no. | Tryptics identified [SEQ ID No] |
|---|---|---|
| Samples 1 | Experiment 1 | LLSDK [938], RMEPLEK [607] |
| Samples 1 | Experiment 2 | FSDVTGKIK [912], IGETVVDLENR [367], RRDLSQMEALK [966] |

Table 63c - Liver cancer, iTRAQ

| Sample no. | Experiment no. | Tryptics identified [SEQ ID No] |
|---|---|---|
| Samples 1 | Experiment 1 | VVFQIWDNDK [810] |

Table 63d - Non-small cell lung cancer, iTRAQ

| Sample no. | Experiment no. | Tryptics identified [SEQ ID No] |
|---|---|---|
| Samples 1 | Experiment 1 | IPNPHLGPVEER [386], IYIVR [926], TAIEILAWGLR [976] |
| Samples 1 | Experiment 2 | FSDVTGK [272], VTAAGQTK [800] |
| Samples 2 | Experiment 1 | RRDLSQMEALK [966] |

Table 63e - Small cell lung cancer, iTRAQ

| Sample no. | Experiment no. | Tryptics identified [SEQ ID No] |
|---|---|---|
| Samples 1 | Experiment 1 | LLSDK [938] |

Table 63f - Ovarian cancer, iTRAQ

| Sample no. | Experiment no. | Tryptics identified [SEQ ID No] |
|---|---|---|
| Samples 1 | Experiment 1 | VTAAGQTKRTR [999] |
| Samples 1 | Experiment 2 | VPAHQVLFSRR [997], VTAAGQTKRTR [999] |

**OGTA098**

Table 64a - Colorectal cancer, iTRAQ

| Samples batch no. | Experiment no. | Tryptics identified [SEQ ID No] |
|---|---|---|
| Samples 1 | Experiment 1 | SLQEANAEK [970], TLAEVCLGQK [982] |
| Samples 2 | Experiment 1 | INATDADEPNTLNSK [381], SLQEANAEK [970] |

Table 64b - Kidney cancer, iTRAQ

| Sample no. | Experiment no. | Tryptics identified [SEQ ID No] |
|---|---|---|
| Samples 1 | Experiment 1 | SLQEANAEK [970] |

Table 64c - Non-small cell lung cancer, iTRAQ

| Sample no. | Experiment no. | Tryptics identified [SEQ ID No] |
|---|---|---|
| Samples 1 | Experiment 1 | INATDADEPNTLNSK [381] |
| Samples 1 | Experiment 2 | NPIAK [957] |
| Samples 2 | Experiment 1 | NPIAK [957] |

Table 64d - Small cell lung cancer, iTRAQ

| Sample no. | Experiment no. | Tryptics identified [SEQ ID No] |
|---|---|---|
| Samples 1 | Experiment 1 | NPIAK [957], TARATGASR [977] |

Table 64e - Ovarian cancer, iTRAQ

| Sample no. | Experiment no. | Tryptics identified [SEQ ID No] |
|---|---|---|
| Samples 1 | Experiment 1 | NPIAK [957] |

**OGTA101**

Table 65a - Colorectal cancer, iTRAQ

| Samples batch no. | Experiment no. | Tryptics identified [SEQ ID No] |
|---|---|---|
| Samples 1 | Experiment 1 | LSARNK [946] |
| Samples 2 | Experiment 1 | IKQLR [922] |
| Samples 2 | Experiment 2 | LASSKAHTSR [930] |

Table 65b - Kidney cancer, iTRAQ

| Sample no. | Experiment no. | Tryptics identified [SEQ ID No] |
|---|---|---|
| Samples 1 | Experiment 1 | ILYDDGKMVEEVDGR [923] |

Table 65c - Liver cancer, iTRAQ

| Sample no. | Experiment no. | Tryptics identified [SEQ ID No] |
|---|---|---|
| Samples 1 | Experiment 1 | IKQLR [922] |

Table 65d - Non-small cell lung cancer, iTRAQ

| Sample no. | Experiment no. | Tryptics identified [SEQ ID No] |
|---|---|---|
| Samples 1 | Experiment 1 | ELREVR [901], LTVLR [949], MRYEGVVDIFQTVK [952], VEVEAVNSTSVK [755] |
| Samples 1 | Experiment 2 | LASSKAHTSR [930], LTVLR [949] |
| Samples 2 | Experiment 1 | GNSAGGLQHRVTAK [914], LSARNK [946] |
| Samples 2 | Experiment 2 | TVDIYGHVTLMR [736] |

Table 65e - Ovarian cancer, iTRAQ

| Sample no. | Experiment no. | Tryptics identified [SEQ ID No] |
|---|---|---|
| Samples 1 | Experiment 1 | YEGVVDIFQTVK [843] |

**OGTA104**

Table 66a - Colorectal cancer, iTRAQ

| Samples batch no. | Experiment no. | Tryptics identified [SEQ ID No] |
|---|---|---|
| Samples 1 | Experiment 1 | FPSGTLR [264], NQTAVR [959] |

(continued)

| Samples batch no. | Experiment no. | Tryptics identified [SEQ ID No] |
|---|---|---|
| Samples 2 | Experiment 1 | LQCENVQEIPVFGIVPAIIQTPSR [943] |
| Samples 2 | Experiment 2 | FPSGTLR [264] |

Table 66b - Kidney cancer, iTRAQ

| Sample no. | Experiment no. | Tryptics identified [SEQ ID No] |
|---|---|---|
| Samples 1 | Experiment 1 | FLITRRR [910] |

Table 66c - Liver cancer, iTRAQ

| Sample no. | Experiment no. | Tryptics identified [SEQ ID No] |
|---|---|---|
| Samples 1 | Experiment 1 | SQTYESDGK [972] |

Table 66d - Non-small cell lung cancer, iTRAQ

| Sample no. | Experiment no. | Tryptics identified [SEQ ID No] |
|---|---|---|
| Samples 1 | Experiment 1 | FLITRRR [910] |
| Samples 1 | Experiment 2 | CGVSNKDIEK [887], FLITRRR [910] |

Table 66e - Ovarian cancer, iTRAQ

| Sample no. | Experiment no. | Tryptics identified [SEQ ID No] |
|---|---|---|
| Samples 1 | Experiment 1 | FLITRRR [910] |

**OGTA106**

Table 67 - Liver cancer, iTRAQ

| Sample no. | Experiment no. | Tryptics identified [SEQ ID No] |
|---|---|---|
| Samples 1 | Experiment 1 | EVCQLLLR [908] |

**OGTA112**

Table 68 - Kidney cancer, iTRAQ

| Sample no. | Experiment no. | Tryptics identified [SEQ ID No] |
|---|---|---|
| Samples 1 | Experiment 1 | LFGEK [932] |

**OGTA113**

Table 69a - Colorectal cancer, iTRAQ

| Samples batch no. | Experiment no. | Tryptics identified [SEQ ID No] |
|---|---|---|
| Samples 1 | Experiment 1 | LLSDPNYGVHLPAVK [939] |
| Samples 2 | Experiment 1 | YPEVGDLR [865] |
| Samples 2 | Experiment 2 | LEDPHVDIIRR [931] |

Table 69b - Kidney cancer, iTRAQ

| Sample no. | Experiment no. | Tryptics identified [SEQ ID No] |
|---|---|---|
| Samples 1 | Experiment 1 | LEDPHVDIIR [449] |

Table 69c - Non-small cell lung cancer, iTRAQ

| Sample no. | Experiment no. | Tryptics identified [SEQ ID No] |
|---|---|---|
| Samples 1 | Experiment 1 | YPEVGDLR [865], YSYNTEWR [874] |

Table 69d - Ovarian cancer, iTRAQ

| Sample no. | Experiment no. | Tryptics identified [SEQ ID No] |
|---|---|---|
| Samples 1 | Experiment 1 | LEDPHVDIIR [449] |

**OGTA119**

Table 70a - Colorectal cancer, iTRAQ

| Samples batch no. | Experiment no. | Tryptics identified [SEQ ID No] |
|---|---|---|
| Samples 1 | Experiment 1 | ALALLEDEER [751], GEGPEAGAGGAGGR [290] |
| Samples 2 | Experiment 1 | VLTDIK [996] |
| Samples 2 | Experiment 2 | MTPPSRAEAGVRR [953] |

Table 70b - Non-small cell lung cancer, iTRAQ

| Sample no. | Experiment no. | Tryptics identified [SEQ ID No] |
|---|---|---|
| Samples 1 | Experiment 1 | AEWLNK [64], DTYLK [896] |
| Samples 1 | Experiment 2 | GEGPEAGAGGAGGR [290] |
| Samples 2 | Experiment 1 | DTYLK [896] |

Table 70c - Ovarian cancer, iTRAQ

| Sample no. | Experiment no. | Tryptics identified [SEQ ID No] |
|---|---|---|
| Samples 1 | Experiment 1 | ALALLEDEER [75] |
| Samples 1 | Experiment 2 | ALALLEDEER [75] |

**OGTA124**

Table 71a - Colorectal cancer, iTRAQ

| Samples batch no. | Experiment no. | Tryptics identified [SEQ ID No] |
|---|---|---|
| Samples 1 | Experiment 1 | LLQAIAK [937] |

Table 71b - Kidney cancer, iTRAQ

| Sample no. | Experiment no. | Tryptics identified [SEQ ID No] |
|---|---|---|
| Samples 1 | Experiment 1 | IFTVAQMDDNSIQMKK [365], LDEDLHVK [446] |

Table 71c - Liver cancer, iTRAQ

| Sample no. | Experiment no. | Tryptics identified [SEQ ID No] |
|---|---|---|
| Samples 1 | Experiment 1 | EWGDGIR [240] |

Table 71d - Non-small cell lung cancer, iTRAQ

| Sample no. | Experiment no. | Tryptics identified [SEQ ID No] |
|---|---|---|
| Samples 1 | Experiment 1 | LDEDLHVK [446] |
| Samples 1 | Experiment 2 | LDEDLHVK [446] |

Table 71e - Ovarian cancer, iTRAQ

| Sample no. | Experiment no. | Tryptics identified [SEQ ID No] |
|---|---|---|
| Samples 1 | Experiment 1 | EREAQLVK [905] |

**OGTA126**

Table 72 - Non-small cell lung cancer, iTRAQ

| Sample no. | Experiment no. | Tryptics identified [SEQ ID No] |
|---|---|---|
| Samples 1 | Experiment 1 | IGTFCSNGTVSRIK [1007], LLVPKDR [940], LWMNVEK [505] |
| Samples 2 | Experiment 1 | LLVPKDR [940] |

**OGTA156**

Table 73a - Colorectal cancer, iTRAQ

| Samples batch no. | Experiment no. | Tryptics identified [SEQ ID No] |
|---|---|---|
| Samples 1 | Experiment 1 | DIMKKTINFAR [891] |

Table 73b - Kidney cancer, iTRAQ

| Sample no. | Experiment no. | Tryptics identified [SEQ ID No] |
|---|---|---|
| Samples 1 | Experiment 1 | DIMKKTINFAR [891], HSRVTVAIPLK [918] |

Table 73c - Non-small cell lung cancer, iTRAQ

| Sample no. | Experiment no. | Tryptics identified [SEQ ID No] |
|---|---|---|
| Samples 1 | Experiment 1 | HSRVTVAIPLK [918] |
| Samples 2 | Experiment 1 | HSRVTVAIPLK [918], TYLAVGSMK [740] |

Table 73d - Ovarian cancer, iTRAQ

| Sample no. | Experiment no. | Tryptics identified [SEQ ID No] |
|---|---|---|
| Samples 1 | Experiment 1 | DIMKKTINFAR [891] |

**OGTA159**

Table 74a - Kidney cancer, iTRAQ

| Sample no. | Experiment no. | Tryptics identified [SEQ ID No] |
|---|---|---|
| Samples 1 | Experiment 1 | RDLGSRLQAQR [965] |
| Samples 1 | Experiment 2 | RDLGSRLQAQR [965] |

Table 74b - Non-small cell lung cancer, iTRAQ

| Sample no. | Experiment no. | Tryptics identified [SEQ ID No] |
|---|---|---|
| Samples 1 | Experiment 1 | RDLGSRLQAQR [965] |
| Samples 1 | Experiment 2 | RDLGSRLQAQR [965] |

**OGTA169**

Table 75a - Colorectal cancer, iTRAQ

| Samples batch no. | Experiment no. | Tryptics identified [SEQ ID No] |
|---|---|---|
| Samples 1 | Experiment 1 | GGQVSVCPLPR [299], NRSLSRVR [961] |
| Samples 2 | Experiment 1 | GGQVSVCPLPR [299] |
| Samples 2 | Experiment 2 | TSKTTFQLELPVK [985] |

Table 75b - Kidney cancer, iTRAQ

| Sample no. | Experiment no. | Tryptics identified [SEQ ID No] |
|---|---|---|
| Samples 1 | Experiment 1 | AVLGDR [885] |

Table 75c - Non-small cell lung cancer, iTRAQ

| Sample no. | Experiment no. | Tryptics identified [SEQ ID No] |
|---|---|---|
| Samples 1 | Experiment 1 | GGQVSVCPLPR [299] |
| Samples 2 | Experiment 1 | TSKTTFQLELPVK [985] |
| Samples 2 | Experiment 2 | GGQVSVCPLPR [299], LTDVVIGAPGEEENR [496], YFTASLPFEK [1004] |

**OGTA174**

Table 76 - Non-small cell lung cancer, iTRAQ

| Sample no. | Experiment no. | Tryptics identified [SEQ ID No] |
|---|---|---|
| Samples 1 | Experiment 1 | LSQCHELWER [490] |

**OGTA176**

Table 77a - Colorectal cancer, iTRAQ

| Samples batch no. | Experiment no. | Tryptics identified [SEQ ID No] |
|---|---|---|
| Samples 1 | Experiment 1 | AVTSPAVGR [886] |
| Samples 1 | Experiment 2 | AVTSPAVGR [886] |

Table 77b - Kidney cancer, iTRAQ

| Sample no. | Experiment no. | Tryptics identified [SEQ ID No] |
|---|---|---|
| Samples 1 | Experiment 1 | AVTSPAVGR [886] |

Table 77c - Non-small cell lung cancer, iTRAQ

| Sample no. | Experiment no. | Tryptics identified [SEQ ID No] |
|---|---|---|
| Samples 1 | Experiment 1 | AVTSPAVGR [886] |

Table 77d - Small cell lung cancer, iTRAQ

| Sample no. | Experiment no. | Tryptics identified [SEQ ID No] |
|---|---|---|
| Samples 1 | Experiment 1 | AVTSPAVGR [886] |
| Samples 1 | Experiment 2 | AVTSPAVGR [886] |

**OGTA177**

Table 78a - Colorectal cancer, iTRAQ

| Samples batch no. | Experiment no. | Tryptics identified [SEQ ID No] |
|---|---|---|
| Samples 1 | Experiment 1 | VLDVVER [994], VVNVSDLYK [1003] |
| Samples 2 | Experiment 1 | AREVIPR [883] |
| Samples 2 | Experiment 2 | DIQVASNEILR [131], SQHQETPVYLGATAGMRLLR [971] |
| Samples 2 | Experiment 3 | GPGISK [915], SQHQETPVYLGATAGMRLLR [971] |

Table 78b - Kidney cancer, iTRAQ

| Sample no. | Experiment no. | Tryptics identified [SEQ ID No] |
|---|---|---|
| Samples 1 | Experiment 1 | DIQVASNEILR [131], GPGISK [915], SLSNYPFDFQGAR [643], VLDVVER [994] |

Table 78c - Non-small cell lung cancer, iTRAQ

| Sample no. | Experiment no. | Tryptics identified [SEQ ID No] |
|---|---|---|
| Samples 1 | Experiment 1 | DIQVASNEILR [131], DPCFHPGYK [892], SLSNYPFDFQGAR [643], SQHQETPVYLGATAGMR [647] |
| Samples 1 | Experiment 2 | EVIPR [909], SLSNYPFDFQGAR [643] |
| Samples 2 | Experiment 1 | FLNLTSEKVSQEK [911] |

Table 78d - Small cell lung cancer, iTRAQ

| Sample no. | Experiment no. | Tryptics identified [SEQ ID No] |
|---|---|---|
| Samples 1 | Experiment 1 | AREVIPR [883] |

Table 78e - Ovarian cancer, iTRAQ

| Sample no. | Experiment no. | Tryptics identified [SEQ ID No] |
|---|---|---|
| Samples 1 | Experiment 1 | GPGISK [915] |

**OGTA197**

Table 79a - Colorectal cancer, iTRAQ

| Samples batch no. | Experiment no. | Tryptics identified [SEQ ID No] |
|---|---|---|
| Samples 1 | Experiment 1 | LNVEERSVGPLTR [942] |
| Samples 2 | Experiment 1 | NGVSGLVTSR [538], TASVSINQTEPPK [673] |
| Samples 2 | Experiment 2 | NGVSGLVTSR [538] |

Table 79b - Kidney cancer, iTRAQ

| Sample no. | Experiment no. | Tryptics identified [SEQ ID No] |
|---|---|---|
| Samples 1 | Experiment 1 | LPGHQKR [478], NGVSGLVTSR [538], TASVSINQTEPPK [673] |

Table 79c - Liver cancer, iTRAQ

| Sample no. | Experiment no. | Tryptics identified [SEQ ID No] |
|---|---|---|
| Samples 1 | Experiment 1 | TASVSINQTEPPK [673] |

Table 79d - Non-small cell lung cancer, iTRAQ

| Sample no. | Experiment no. | Tryptics identified [SEQ ID No] |
|---|---|---|
| Samples 1 | Experiment 1 | TASVSINQTEPPK [673] |
| Samples 1 | Experiment 2 | DGEFSVLQLVGMLR [121], LNVEERSVGPLTR [942] |
| Samples 2 | Experiment 1 | NGVSGLVTSR [538] |

Table 79e - Small cell lung cancer, iTRAQ

| Sample no. | Experiment no. | Tryptics identified [SEQ ID No] |
|---|---|---|
| Samples 1 | Experiment 1 | SVGPLTRK [663], TASVSINQTEPPK [673] |
| Samples 1 | Experiment 2 | NGVSGLVTSR [538], SVGPLTRK [663], TASVSINQTEPPK [673] |

**OGTA202**

Table 80a - Colorectal cancer, iTRAQ

| Samples batch no. | Experiment no. | Tryptics identified [SEQ ID No] |
|---|---|---|
| Samples 1 | Experiment 1 | STGTISVISSGLDREK [975] |
| Samples 2 | Experiment 1 | EQLTVIR [904], ISYRILR [925] |

Table 80b - Non-small cell lung cancer, iTRAQ

| Sample no. | Experiment no. | Tryptics identified [SEQ ID No] |
|---|---|---|
| Samples 1 | Experiment 1 | EQLTVIR [904] |
| Samples 1 | Experiment 2 | EQLTVIR [904] |
| Samples 2 | Experiment 1 | EQLTVIR [904] |

**OGTA203**

Table 81a - Kidney cancer, iTRAQ

| Sample no. | Experiment no. | Tryptics identified [SEQ ID No] |
|---|---|---|
| Samples 1 | Experiment 1 | LLDFEK [934], TGRPVEPESEFIIK [981] |

Table 81b - Non-small cell lung cancer, iTRAQ

| Sample no. | Experiment no. | Tryptics identified [SEQ ID No] |
|---|---|---|
| Samples 1 | Experiment 1 | VEASNPYVEPRFLYLGPFK [989] |

**OGTA206**

Table 82a - Colorectal cancer, iTRAQ

| Samples batch no. | Experiment no. | Tryptics identified [SEQ ID No] |
|---|---|---|
| Samples 1 | Experiment 1 | VYDSLLALPQDLQAAR [817] |
| Samples 2 | Experiment 1 | SAAASNYV [968], VYDSLLALPQDLQAAR [817] |
| Samples 2 | Experiment 2 | VYDSLLALPQDLQAAR [817] |
| Samples 2 | Experiment 3 | VYDSLLALPQDLQAAR [817] |
| Samples 2 | Experiment 4 | VYDSLLALPQDLQAAR [817] |

Table 82b - Non-small cell lung cancer, iTRAQ

| Sample no. | Experiment no. | Tryptics identified [SEQ ID No] |
|---|---|---|
| Samples 1 | Experiment 1 | VYDSLLALPQDLQAAR [817] |
| Samples 2 | Experiment 1 | VYDSLLALPQDLQAAR [817] |
| Samples 2 | Experiment 2 | VYDSLLALPQDLQAAR [817] |

**OGTA213**

Table 83a - Colorectal cancer, iTRAQ

| Samples batch no. | Experiment no. | Tryptics identified [SEQ ID No] |
|---|---|---|
| Samples 1 | Experiment 1 | RSSAAGEGTLAR [967] |
| Samples 2 | Experiment 1 | RSSAAGEGTLAR [967] |
| Samples 2 | Experiment 2 | RSSAAGEGTLAR [967] |

Table 83b - Non-small cell lung cancer, iTRAQ

| Sample no. | Experiment no. | Tryptics identified [SEQ ID No] |
|---|---|---|
| Samples 1 | Experiment 1 | RSSAAGEGTLAR [967] |
| Samples 1 | Experiment 2 | RSSAAGEGTLAR [967] |
| Samples 2 | Experiment 1 | RSSAAGEGTLAR [967] |

**OGTA214**

Table 84 - Colorectal cancer, iTRAQ

| Samples batch no. | Experiment no. | Tryptics identified [SEQ ID No] |
|---|---|---|
| | | |
| Samples 1 | Experiment 1 | DSQMQNPYSR [895], HIEEENLIDEDFQNLK [917], SDLQRPNPQSPFCVASSLK [969], STGFTNLGAEGSVFPK [974] |
| Samples 1 | Experiment 2 | STGFTNLGAEGSVFPK [974] |
| Samples 2 | Experiment 1 | HIEEENLIDEDFQNLK [917] |
| Samples 2 | Experiment 2 | VFPGK [990] |

**OGTA216**

Table 85a - Colorectal cancer, iTRAQ

| Samples batch no. | Experiment no. | Tryptics identified [SEQ ID No] |
|---|---|---|
| Samples 1 | Experiment 1 | AAAAAAAAAAAAAAAGAGAGAK [49], AFYAPVHADDLR [69], GGGAYYLISR [298], GPIVPLNVADQK [307], ITDNELELYK [395] |
| Samples 1 | Experiment 2 | DLPPILLVR [136], EGAQYLMQAAGLGR [186] |
| Samples 2 | Experiment 1 | AMATLLSK [78], EGAQYLMQAAGLGR [186] |
| Samples 2 | Experiment 2 | DLPPILLVR [136] |
| Samples 2 | Experiment 3 | QTPADGEASGESEPAK [1008] |

Table 85b - Kidney cancer, iTRAQ

| Sample no. | Experiment no. | Tryptics identified [SEQ ID No] |
|---|---|---|
| Samples 1 | Experiment 1 | RAMATLLSK [596] |
| Samples 1 | Experiment 2 | RAMATLLSK [596], SPGWRPAFK [645] |

Table 85c - Liver cancer, iTRAQ

| Sample no. | Experiment no. | Tryptics identified [SEQ ID No] |
|---|---|---|
| Samples 1 | Experiment 1 | IFQALCK [920] |
| Samples 1 | Experiment 2 | SPGWRPAFK [645] |

Table 85d - Non-small cell lung cancer, iTRAQ

| Sample no. | Experiment no. | Tryptics identified [SEQ ID No] |
|---|---|---|
| Samples 1 | Experiment 1 | AMATLLSK [78], DCKIRVFIGGK [889], TATQPLLK [978] |

(continued)

| Sample no. | Experiment no. | Tryptics identified [SEQ ID No] |
|---|---|---|
| Samples 1 | Experiment 2 | EMSIDQAK [213], LHEDDK [465] |
| Samples 2 | Experiment 1 | EQDIADK [903] |
| Samples 2 | Experiment 2 | DLPPILLVR [136] |

Table 85e - Small cell lung cancer, iTRAQ

| Sample no. | Experiment no. | Tryptics identified [SEQ ID No] |
|---|---|---|
| Samples 1 | Experiment 1 | LSGVEDHVK [948] |

Table 85f - Ovarian cancer, iTRAQ

| Sample no. | Experiment no. | Tryptics identified [SEQ ID No] |
|---|---|---|
| Samples 1 | Experiment 1 | AMATLLSK [78] |

**OGTA222**

Table 86a - Colorectal cancer, iTRAQ

| Samples batch no. | Experiment no. | Tryptics identified [SEQ ID No] |
|---|---|---|
| Samples 1 | Experiment 1 | AGFVGGQFWSVYTPCDTQNK [881], ATLQLSR [91], AVGFGGDFDGVPR [95], GALADNLLR [283], NVPDDVLR [559], TLEQMDVVHR [703] |
| Samples 1 | Experiment 2 | AGFVGGQFWSVYTPCDTQNK [881], ALYQLGMR [77], AVGFGGDFDGVPR [95], DSPVIDGHNDLPWQLLDMFNNR [894]. GALADNLLR [283], TLEQMDVVHR [703], VASLIGVEGGHSIDSSLGVLR [988], VPEGLEDVSK [791], YPDLIAELLR [864] |
| Samples 2 | Experiment 1 | AVGFGGDFDGVPR [95] |

Table 86b - Kidney cancer, iTRAQ

| Sample no. | Experiment no. | Tryptics identified [SEQ ID No] |
|---|---|---|
| Samples 1 | Experiment 1 | AVGFGGDFDGVPR [95], VPEGLEDVSK [791] |
| Samples 1 | Experiment 2 | ALYQLGMR [77], AVGFGGDFDGVPR [95], GALADNLLR [283], NVPDDVLR [559], VPEGLEDVSK [791], YPDLIAELLR [864] |
| Samples 1 | Experiment 3 | AVGFGGDFDGVPR [95] |

Table 86c - Non-small cell lung cancer, iTRAQ

| Sample no. | Experiment no. | Tryptics identified [SEQ ID No] |
|---|---|---|
| Samples 1 | Experiment 1 | ANLSQVADHLDHIK [882] |
| Samples 1 | Experiment 2 | VPEGLEDVSK [791] |

**OGTA236**

Table 87 - Colorectal cancer, iTRAQ

| Samples batch no. | Experiment no. | Tryptics identified [SEQ ID No] |
|---|---|---|
| Samples 1 | Experiment 1 | ELNEHFKSK [900] |

**OGTA237**

Table 88a - Colorectal cancer, iTRAQ

| Samples batch no. | Experiment no. | Tryptics identified [SEQ ID No] |
|---|---|---|
| Samples 1 | Experiment 1 | QLVEALDK [963] |

Table 88b - Kidney cancer, iTRAQ

| Sample no. | Experiment no. | Tryptics identified [SEQ ID No] |
|---|---|---|
| Samples 1 | Experiment 1 | AEAFGMDPARPDQASTVAVK [878] |

Table 88c - Non-small cell lung cancer, iTRAQ

| Sample no. | Experiment no. | Tryptics identified [SEQ ID No] |
|---|---|---|
| Samples 1 | Experiment 1 | TSNGRLPVK [986] |

Table 88d - Small cell lung cancer, iTRAQ

| Sample no. | Experiment no. | Tryptics identified [SEQ ID No] |
|---|---|---|
| Samples 1 | Experiment 1 | LVLGK [951] |

**OGTA247**

Table 89a - Non-small cell lung cancer, iTRAQ

| Sample no. | Experiment no. | Tryptics identified [SEQ ID No] |
|---|---|---|
| Samples 1 | Experiment 1 | TNGTGRVR [984], WRPVDLAQVK [831] |
| Samples 1 | Experiment 2 | ERMFR [906] |

Table 89b - Small cell lung cancer, iTRAQ

| Sample no. | Experiment no. | Tryptics identified [SEQ ID No] |
|---|---|---|
| Samples 1 | Experiment 1 | DATQITQGPR [109], DLQELGDSDK [137], DPELR [893], EGPGEAIVR [189], NPVDVK [958], TNGTGRVR [984], VGEEDDGEYR [762], VTAINK [1000], YGPGEPSPVSETVVTPEAAPEK [1005] |

(continued)

| Sample no. | Experiment no. | Tryptics identified [SEQ ID No] |
|---|---|---|
| Samples 1 | Experiment 2 | AQLLVVGSPGPVPR [82], DATQITQGPR [109], DPELR [893], EPIDLR [217], NPVDVK [958], TNGTGRVR [984], VGEEDDGEYR [762], VQAVNSQGK [998], VTAINK [1000], YGPGEPSPVSETVVTPEAAPEK [1005] |

**OGTA248**

Table 90 - Non-small cell lung cancer, iTRAQ

| Sample no. | Experiment no. | Tryptics identified [SEQ ID No] |
|---|---|---|
| Samples 1 | Experiment 1 | LRTSAIR [945] |

**OGTA249**

Table 91a - Kidney cancer, iTRAQ

| Sample no. | Experiment no. | Tryptics identified [SEQ ID No] |
|---|---|---|
| Samples 1 | Experiment 1 | VTLPAGPDILR [1001] |

Table 91b - Non-small cell lung cancer, iTRAQ

| Sample no. | Experiment no. | Tryptics identified [SEQ ID No] |
|---|---|---|
| Samples 1 | Experiment 1 | VTLPAGPDILR [1001] |
| Samples 2 | Experiment 1 | VTLPAGPDILR [1001] |
| Samples 2 | Experiment 2 | VTLPAGPDILR [1001] |
| Samples 2 | Experiment 3 | VTLPAGPDILR [1001] |

**OGTA257**

Table 92a - Colorectal cancer, iTRAQ

| Samples batch no. | Experiment no. | Tryptics identified [SEQ ID No] |
|---|---|---|
| Samples 1 | Experiment 1 | QILDQTPVK [962] |

Table 92b - Liver cancer, iTRAQ

| Sample no. | Experiment no. | Tryptics identified [SEQ ID No] |
|---|---|---|
| Samples 1 | Experiment 1 | LRQGTLR [944] |

Table 92c - Non-small cell lung cancer, iTRAQ

| Sample no. | Experiment no. | Tryptics identified [SEQ ID No] |
|---|---|---|
| Samples 1 | Experiment 1 | ATGTAFRR [884], ELVSLK [902] |

Table 92d - Ovarian cancer, iTRAQ

| Sample no. | Experiment no. | Tryptics identified [SEQ ID No] |
|---|---|---|
| Samples 1 | Experiment 1 | LLIEHGADIR [936] |

**OGTA271**

Table 93a - Colorectal cancer, iTRAQ

| Samples batch no. | Experiment no. | Tryptics identified [SEQ ID No] |
|---|---|---|
| Samples 1 | Experiment 1 | IKQLR [922], TAQSTPSAPPQK [672], TATVVMMTRLEEK [979] |
| Samples 1 | Experiment 2 | VKCDQYWPAR [991] |

Table 93b - Kidney cancer, iTRAQ

| Sample no. | Experiment no. | Tryptics identified [SEQ ID No] |
|---|---|---|
| Samples 1 | Experiment 1 | TAQSTPSAPPQK [672] |
| Samples 1 | Experiment 2 | EPMDQKR [218] |

Table 93c - Liver cancer, iTRAQ

| Sample no. | Experiment no. | Tryptics identified [SEQ ID No] |
|---|---|---|
| Samples 1 | Experiment 1 | IKQLR [922] |

Table 93d - Non-small cell lung cancer, iTRAQ

| Sample no. | Experiment no. | Tryptics identified [SEQ ID No] |
|---|---|---|
| Samples 1 | Experiment 1 | NRAGLGEEFEKEIR [960], TAQSTPSAPPQK [672] |
| Samples 2 | Experiment 1 | DSLLAHSSDPVEMR [156], TMPVEQVFAK [708] |

For proteins of the disclosure the detected level obtained upon analyzing tissue from subjects having a relevant cancer relative to the detected level obtained upon analyzing tissue from subjects free from said cancers will depend upon the particular analytical protocol and detection technique that is used. Accordingly, the present disclosure contemplates that each laboratory will establish a reference range in subjects free from said cancers according to the analytical protocol and detection technique in use, as is conventional in the diagnostic art. For example, at least one control positive tissue sample from a subject known to have a relevant cancer or at least one control negative tissue sample from a subject known to be free from said cancer (and more preferably both positive and negative control samples) are included in each batch of test samples analysed.

Proteins of the disclosure can be used for detection, prognosis, diagnosis, or monitoring of a relevant cancer or for drug development. In one example of the disclosure, tissue from a subject *(e.g.,* a subject suspected of having a relevant cancer) is analysed by ID electrophoresis or iTRAQ for detection of a protein according to the disclosure. An increased abundance of of a protein according to the disclosure in the tissue from the subject relative to tissue from a subject or subjects free from said cancer *(e.g.,* a control sample) or a previously determined reference range indicates the presence of a relevant cancer.

In particular, OGTA002 can be used for detection, prognosis, diagnosis, or monitoring of breast cancer, colorectal cancer, hepatocellular carcinoma, lung cancer, melanoma, ovarian cancer, pancreatic cancer and renal cell cancer.

In particular, OGTA009 can be used for detection, prognosis, diagnosis, or monitoring of breast cancer, colorectal cancer, lung cancer, ovarian cancer, pancreatic cancer, prostate cancer and renal cell cancer.

In particular, OGTA016 can be used for detection, prognosis, diagnosis, or monitoring of colorectal cancer and lung cancer.

In particular, OGTA028 can be used for detection, prognosis, diagnosis, or monitoring of colorectal cancer, hepatocellular carcinoma, lung cancer, ovarian cancer and renal cell cancer. In particular, OGTA037 can be used for detection, prognosis, diagnosis, or monitoring of colorectal cancer, lung cancer, gastric cancer and ovarian cancer.

In particular, OGTA041 can be used for detection, prognosis, diagnosis, or monitoring of hepatocellular carcinoma, lung cancer and pancreatic cancer.

In particular, OGTA053 can be used for detection, prognosis, diagnosis, or monitoring of colorectal cancer, hepatocellular carcinoma, lung cancer, pancreatic cancer and renal cell cancer.

In particular, OGTA054 can be used for detection, prognosis, diagnosis, or monitoring of colorectal cancer, lung cancer and pancreatic cancer.

In particular, OGTA066 can be used for detection, prognosis, diagnosis, or monitoring of colorectal cancer, lung cancer, pancreatic cancer and renal cell cancer.

In particular, OGTA072 can be used for detection, prognosis, diagnosis, or monitoring of colorectal cancer.

In particular, OGTA074 can be used for detection, prognosis, diagnosis, or monitoring of colorectal cancer and lung cancer.

In particular, OGTA076 can be used for detection, prognosis, diagnosis, or monitoring of chronic lymphocytic leukaemia, colorectal cancer, hepatocellular carcinoma, lung cancer, ovarian cancer, pancreatic cancer and renal cell cancer.

In particular, OGTA085 can be used for detection, prognosis, diagnosis, or monitoring of colorectal cancer, lung cancer, melanoma and retinoblastoma.

In particular, OGTA087 can be used for detection, prognosis, diagnosis, or monitoring of B-cell non-Hodgkin's lymphoma, breast cancer, colorectal cancer, gastric cancer, lung cancer, lymphoid leukaemia and ovarian cancer.

In particular, OGTA088 can be used for detection, prognosis, diagnosis, or monitoring of breast cancer, colorectal cancer, gastric cancer, glioblastoma, hepatocellular carcinoma, lung cancer, lymphoid leukaemia, pancreatic cancer, renal cell cancer and retinoblastoma.

In particular, OGTA089 can be used for detection, prognosis, diagnosis, or monitoring of breast cancer, lung cancer, ovarian cancer and renal cell cancer.

In particular, OGTA091 can be used for detection, prognosis, diagnosis, or monitoring of breast cancer, cervical cancer, chronic lymphocytic leukaemia, colorectal cancer, gastric cancer, hepatocellular carcinoma, lung cancer, melanoma, osteosarcoma, ovarian cancer, pancreatic cancer, prostate cancer and renal cell cancer.

In particular, OGTA098 can be used for detection, prognosis, diagnosis, or monitoring of breast cancer, cervical cancer, colorectal cancer, hepatocellular carcinoma, lung cancer, osteosarcoma, ovarian cancer, pancreatic cancer, prostate cancer and renal cell cancer.

In particular, OGTA101 can be used for detection, prognosis, diagnosis, or monitoring of breast cancer, cervical cancer, colorectal cancer, hepatocellular carcinoma, lung cancer, osteosarcoma, ovarian cancer, pancreatic cancer and renal cell cancer.

In particular, OGTA104 can be used for detection, prognosis, diagnosis, or monitoring of breast cancer, colorectal cancer, hepatocellular carcinoma, lung cancer, ovarian cancer, pancreatic cancer and renal cell cancer.

In particular, OGTA106 can be used for detection, prognosis, diagnosis, or monitoring of cervical cancer, hepatocellular carcinoma, melanoma and pancreatic cancer.

In particular, OGTA112 can be used for detection, prognosis, diagnosis, or monitoring of breast cancer, cervical cancer, chronic lymphocytic leukaemia, hepatocellular carcinoma, pancreatic cancer and renal cell cancer.

In particular, OGTA113 can be used for detection, prognosis, diagnosis, or monitoring of colorectal cancer, lung cancer, ovarian cancer, pancreatic cancer and renal cell cancer.

In particular, OGTA119 can be used for detection, prognosis, diagnosis, or monitoring of B-cell non-Hodgkin's lymphoma, breast cancer, colorectal cancer, gastric cancer, hepatocellular carcinoma, lung cancer, lymphoid leukaemia, neuroblastoma, osteosarcoma, ovarian cancer, pancreatic cancer, prostate cancer and renal cell cancer.

In particular, OGTA124 can be used for detection, prognosis, diagnosis, or monitoring of colorectal cancer, hepatocellular carcinoma, lung cancer, ovarian cancer, pancreatic cancer and renal cell cancer.

In particular, OGTA126 can be used for detection, prognosis, diagnosis, or monitoring of breast cancer, colorectal cancer, hepatocellular carcinoma, lung cancer, melanoma, pancreatic cancer, prostate cancer and renal cell cancer.

In particular, OGTA156 can be used for detection, prognosis, diagnosis, or monitoring of acute T-cell leukaemia, B-cell non-Hodgkin's lymphoma, chronic lymphocytic leukaemia, colorectal cancer, hepatocellular carcinoma, lung cancer, ovarian cancer and renal cell cancer.

In particular, OGTA159 can be used for detection, prognosis, diagnosis, or monitoring of breast cancer, chronic lymphocytic leukaemia, colorectal cancer, hepatocellular carcinoma, lung cancer, melanoma, pancreatic cancer and renal cell cancer.

In particular, OGTA168 can be used for detection, prognosis, diagnosis, or monitoring of glioblastoma and melanoma.

In particular, OGTA169 can be used for detection, prognosis, diagnosis, or monitoring of breast cancer, chronic lymphocytic leukaemia, colorectal cancer, lung cancer and renal cell cancer.

In particular, OGTA174 can be used for detection, prognosis, diagnosis, or monitoring of acute T-cell leukaemia, chronic lymphocytic leukaemia and lung cancer.

In particular, OGTA176 can be used for detection, prognosis, diagnosis, or monitoring of B-cell non-Hodgkin's lymphoma, chronic lymphocytic leukaemia, colorectal cancer, lung cancer and renal cell cancer.

In particular, OGTA177 can be used for detection, prognosis, diagnosis, or monitoring of chronic lymphocytic leukaemia, colorectal cancer, lung cancer, ovarian cancer and renal cell cancer.

In particular, OGTA197 can be used for detection, prognosis, diagnosis, or monitoring of colorectal cancer, hepatocellular carcinoma, lung cancer, melanoma, osteosarcoma, ovarian cancer, pancreatic cancer, prostate cancer and renal cell cancer.

In particular, OGTA202 can be used for detection, prognosis, diagnosis, or monitoring of colorectal cancer and lung cancer.

In particular, OGTA203 can be used for detection, prognosis, diagnosis, or monitoring of lung cancer, ovarian cancer and renal cell cancer.

In particular, OGTA206 can be used for detection, prognosis, diagnosis, or monitoring of breast cancer, colorectal cancer, lung cancer, pancreatic cancer and prostate cancer.

In particular, OGTA213 can be used for detection, prognosis, diagnosis, or monitoring of colorectal cancer and lung cancer.

In particular, OGTA214 can be used for detection, prognosis, diagnosis, or monitoring of colorectal cancer and ovarian cancer.

In particular, OGTA216 can be used for detection, prognosis, diagnosis, or monitoring of breast cancer, colorectal cancer, hepatocellular carcinoma, lung cancer, ovarian cancer, pancreatic cancer and renal cell cancer.

In particular, OGTA222 can be used for detection, prognosis, diagnosis, or monitoring of B-cell non-Hodgkin's lymphoma, colorectal cancer, lung cancer and renal cell cancer.

In particular, OGTA236 can be used for detection, prognosis, diagnosis, or monitoring of colorectal cancer and pancreatic cancer.

In particular, OGTA237 can be used for detection, prognosis, diagnosis, or monitoring of B-cell non-Hodgkin's lymphoma, colorectal cancer, lung cancer, lymphoid leukaemia, prostate cancer and renal cell cancer.

In particular, OGTA247 can be used for detection, prognosis, diagnosis, or monitoring of hepatocellular carcinoma, lung cancer and melanoma.

In particular, OGTA248 can be used for detection, prognosis, diagnosis, or monitoring of lung cancer and renal cell cancer.

In particular, OGTA249 can be used for detection, prognosis, diagnosis, or monitoring of gastric cancer, lung cancer, ovarian cancer and renal cell cancer.

In particular, OGTA257 can be used for detection, prognosis, diagnosis, or monitoring of colorectal cancer, hepatocellular carcinoma, lung cancer, ovarian cancer and pancreatic cancer.

In particular, OGTA271 can be used for detection, prognosis, diagnosis, or monitoring of breast cancer, cervical cancer, colorectal cancer, hepatocellular carcinoma, lung cancer, osteosarcoma, pancreatic cancer and renal cell cancer.

In one or more examples of the disclosure:

OGTA002 may, in particular, be characterized as an isoform having a MW substantially as recited (e.g. +/- 10%, particularly +/-5% of the value) in column 1 of any of the rows of Tables 1a-1f

OGTA009 may, in particular, be characterized as an isoform having a MW substantially as recited (e.g. +/- 10%, particularly +/-5% of the value) in column 1 of any of the rows of Tables 2a-2e

OGTA016 may, in particular, be characterized as an isoform having a MW substantially as recited (e.g. +/- 10%, such as +/-5% of the value) in column 1 of any of the rows of Table 3

OGTA028 may, in particular, be characterized as an isoform having a MW substantially as recited (e.g. +/- 10%, such as +/-5% of the value) in column 1 of any of the rows of Table 4

OGTA037 may, in particular, be characterized as an isoform having a MW substantially as recited (e.g. +/- 10%, such as +/-5% of the value) in column 1 of any of the rows of Table 5

OGTA041 may, in particular, be characterized as an isoform having a MW substantially as recited (e.g. +/- 10%, particularly +/-5% of the value) in column 1 of any of the rows of Tables 6a-6c

OGTA053 may, in particular, be characterized as an isoform having a MW substantially as recited (e.g. +/- 10%, such as +/-5% of the value) in column 1 of any of the rows of Table 7

OGTA054 may, in particular, be characterized as an isoform having a MW substantially as recited (e.g. +/- 10%, such as +/-5% of the value) in column 1 of any of the rows of Table 8

OGTA066 may, in particular, be characterized as an isoform having a MW substantially as recited (e.g. +/- 10%, particularly +/-5% of the value) in column 1 of any of the rows of Tables 9a-9b

OGTA072 may, in particular, be characterized as an isoform having a MW substantially as recited (e.g. +/- 10%, such as +/-5% of the value) in column 1 of any of the rows of Table 10

OGTA074 may, in particular, be characterized as an isoform having a MW substantially as recited (e.g. +/- 10%, such as +/-5% of the value) in column 1 of any of the rows of Table 11

OGTA076 may, in particular, be characterized as an isoform having a MW substantially as recited (e.g. +/- 10%, particularly +/-5% of the value) in column 1 of any of the rows of Tables 12a-12c

OGTA085 may, in particular, be characterized as an isoform having a MW substantially as recited (e.g. +/- 10%, particularly +/-5% of the value) in column 1 of any of the rows of Tables 13a-13c

OGTA087 may, in particular, be characterized as an isoform having a MW substantially as recited (e.g. +/- 10%, particularly +/-5% of the value) in column 1 of any of the rows of Tables 14a-14g

OGTA088 may, in particular, be characterized as an isoform having a MW substantially as recited (e.g. +/- 10%, particularly +/-5% of the value) in column 1 of any of the rows of Tables 15a-15i

OGTA089 may, in particular, be characterized as an isoform having a MW substantially as recited (e.g. +/- 10%, particularly +/-5% of the value) in column 1 of any of the rows of Tables 16a-16c

OGTA091 may, in particular, be characterized as an isoform having a MW substantially as recited (e.g. +/- 10%, particularly +/-5% of the value) in column 1 of any of the rows of Tables 17a-17l

OGTA098 may, in particular, be characterized as an isoform having a MW substantially as recited (e.g. +/- 10%, particularly +/-5% of the value) in column 1 of any of the rows of Tables 18a-18h

OGTA101 may, in particular, be characterized as an isoform having a MW substantially as recited (e.g. +/- 10%, particularly +/-5% of the value) in column 1 of any of the rows of Tables 19a-19g

OGTA104 may, in particular, be characterized as an isoform having a MW substantially as recited (e.g. +/- 10%, particularly +/-5% of the value) in column 1 of any of the rows of Tables 20a-20d

OGTA106 may, in particular, be characterized as an isoform having a MW substantially as recited (e.g. +/- 10%, particularly +/-5% of the value) in column 1 of any of the rows of Tables 21a-21c

OGTA112 may, in particular, be characterized as an isoform having a MW substantially as recited (e.g. +/- 10%, particularly +/-5% of the value) in column 1 of any of the rows of Tables 22a-22f

OGTA113 may, in particular, be characterized as an isoform having a MW substantially as recited (e.g. +/- 10%, particularly +/-5% of the value) in column 1 of any of the rows of Table 23

OGTA119 may, in particular, be characterized as an isoform having a MW substantially as recited (e.g. +/- 10%, particularly +/-5% of the value) in column 1 of any of the rows of Tables 24a-24l

OGTA124 may, in particular, be characterized as an isoform having a MW substantially as recited (e.g. +/- 10%, particularly +/-5% of the value) in column 1 of any of the rows of Tables 25a-25e

OGTA126 may, in particular, be characterized as an isoform having a MW substantially as recited (e.g. +/- 10%, particularly +/-5% of the value) in column 1 of any of the rows of Tables 26a-26g

OGTA156 may, in particular, be characterized as an isoform having a MW substantially as recited (e.g. +/- 10%, particularly +/-5% of the value) in column 1 of any of the rows of Tables 27a-27d

OGTA159 may, in particular, be characterized as an isoform having a MW substantially as recited (e.g. +/- 10%, particularly +/-5% of the value) in column 1 of any of the rows of Tables 28a-28f

OGTA168 may, in particular, be characterized as an isoform having a MW substantially as recited (e.g. +/- 10%, particularly +/-5% of the value) in column 1 of any of the rows of Tables 29a-29b

OGTA169 may, in particular, be characterized as an isoform having a MW substantially as recited (e.g. +/- 10%, particularly +/-5% of the value) in column 1 of any of the rows of Tables 30a-30b

OGTA174 may, in particular, be characterized as an isoform having a MW substantially as recited (e.g. +/- 10%, particularly +/-5% of the value) in column 1 of any of the rows of Tables 31a-31b

OGTA176 may, in particular, be characterized as an isoform having a MW substantially as recited (e.g. +/- 10%, particularly +/-5% of the value) in column 1 of any of the rows of Tables 32a-32b

OGTA177 may, in particular, be characterized as an isoform having a MW substantially as recited (e.g. +/- 10%, such as +/-5% of the value) in column 1 of any of the rows of Table 33

OGTA197 may, in particular, be characterized as an isoform having a MW substantially as recited (e.g. +/- 10%, particularly +/-5% of the value) in column 1 of any of the rows of Tables 34a-34i

OGTA202 may, in particular, be characterized as an isoform having a MW substantially as recited (e.g. +/- 10%, particularly +/-5% of the value) in column 1 of any of the rows of Table 35

OGTA203 may, in particular, be characterized as an isoform having a MW substantially as recited (e.g. +/- 10%, particularly +/-5% of the value) in column 1 of any of the rows of Tables 36a-36b

OGTA206 may, in particular, be characterized as an isoform having a MW substantially as recited (e.g. +/- 10%, particularly +/-5% of the value) in column 1 of any of the rows of Tables 37a-37c

OGTA213 may, in particular, be characterized as an isoform having a MW substantially as recited (e.g. +/- 10%, such as +/-5% of the value) in column 1 of any of the rows of Table 38 OGTA214 may, in particular, be characterized as an isoform having a MW substantially as recited (e.g. +/- 10%, particularly +/-5% of the value) in column 1 of any of the rows of Table 39

OGTA216 may, in particular, be characterized as an isoform having a MW substantially as recited (e.g. +/- 10%, particularly +/-5% of the value) in column 1 of any of the rows of Tables 40a-40b

OGTA222 may, in particular, be characterized as an isoform having a MW substantially as recited (e.g. +/- 10%, particularly +/-5% of the value) in column 1 of any of the rows of Tables 41a-41b

OGTA236 may, in particular, be characterized as an isoform having a MW substantially as recited (e.g. +/- 10%, such as +/-5% of the value) in column 1 of any of the rows of Table 42

OGTA237 may, in particular, be characterized as an isoform having a MW substantially as recited (e.g. +/- 10%, particularly +/-5% of the value) in column 1 of any of the rows of Tables 43a-43c

OGTA247 may, in particular, be characterized as an isoform having a MW substantially as recited (e.g. +/- 10%, particularly +/-5% of the value) in column 1 of any of the rows of Tables 44a-44b

OGTA248 may, in particular, be characterized as an isoform having a MW substantially as recited (e.g. +/- 10%, such as +/-5% of the value) in column 1 of any of the rows of Table 45

OGTA249 may, in particular, be characterized as an isoform having a MW substantially as recited (e.g. +/- 10%, particularly +/-5% of the value) in column 1 of any of the rows of Table 46.

OGTA257 may, in particular, be characterized as an isoform having a MW substantially as recited (e.g. +/- 10%, such as +/-5% of the value) in column 1 of any of the rows of Table 47

OGTA271 may, in particular, be characterized as an isoform having a MW substantially as recited (e.g. +/- 10%, particularly +/-5% of the value) in column 1 of any of the rows of Tables 48a-48h

In relation to fragments, immunogenic fragments or antigenic fragments of OGTA002:

- for breast cancer applications: for example these comprise one or more of the sequences identified as tryptic sequences in the 3rd column of Table 1a;
- for colorectal cancer applications: for example these comprise one or more of the sequences identified as tryptic sequences in the 3rd column of Table 1b and Table 49a;
- for hepatocellular carcinoma applications: , for example these comprise one or more of the sequences identified as tryptic sequences in the 3rd column of Table 1c and Table 49c;

- for lung cancer applications: for example these comprise one or more of the sequences identified as tryptic sequences in the 3rd column of Table 49d;
- for melanoma applications: for example these comprise one or more of the sequences identified as tryptic sequences in the 3rd column of Table 1d;
- for ovarian cancer applications: for example these comprise one or more of the sequences identified as tryptic sequences in the 3rd column of Table 1e;
- for pancreatic cancer applications: for example these comprise one or more of the sequences identified as tryptic sequences in the 3rd column of Table If;
- for renal cell cancer applications: for example these comprise one or more of the sequences identified as tryptic sequences in the 3rd column of Table 49b.

In relation to fragments, immunogenic fragments or antigenic fragments of OGTA009:

- for breast cancer applications: for example these comprise one or more of the sequences identified as tryptic sequences in the 3rd column of Table 2a;
- for colorectal cancer applications: for example these comprise one or more of the sequences identified as tryptic sequences in the 3rd column of Table 2b and Table 50a;
- for lung cancer applications: for example these comprise one or more of the sequences identified as tryptic sequences in the 3rd column of Table 50b;

- for ovarian cancer applications: for example these comprise one or more of the sequences identified as tryptic sequences in the 3$^{rd}$ column of Table 50c;
- for pancreatic cancer applications: for example these comprise one or more of the sequences identified as tryptic sequences in the 3$^{rd}$ column of Table 2c;
- for prostate cancer applications: for example these comprise one or more of the sequences identified as tryptic sequences in the 3$^{rd}$ column of Table 2d;
- for renal cell cancer applications: for example these comprise one or more of the sequences identified as tryptic sequences in the 3$^{rd}$ column of Table 2e;

In relation to fragments, immunogenic fragments or antigenic fragments of OGTA016:

- for colorectal cancer applications: for example these comprise one or more of the sequences identified as tryptic sequences in the 3$^{rd}$ column of Table 3 and Table 51a;
- for lung cancer applications: for example these comprise one or more of the sequences identified as tryptic sequences in the 3$^{rd}$ column of Table 51b.

In relation to fragments, immunogenic fragments or antigenic fragments of OGTA028:

- for colorectal cancer applications: for example these comprise one or more of the sequences identified as tryptic sequences in the 3$^{rd}$ column of Table 52a;
- for hepatocellular carcinoma applications: for example these comprise one or more of the sequences identified as tryptic sequences in the 3$^{rd}$ column of Table 52c;
- for lung cancer applications: for example these comprise one or more of the sequences identified as tryptic sequences in the 3$^{rd}$ column of Table 52d and Table 52e;
- for ovarian cancer applications: for example these comprise one or more of the sequences identified as tryptic sequences in the 3$^{rd}$ column of Table 4 and Table 52f;
- for renal cell cancer applications: for example these comprise one or more of the sequences identified as tryptic sequences in the 3$^{rd}$ column of Table 52b.

In relation to fragments, immunogenic fragments or antigenic fragments of OGTA037:

- for colorectal cancer applications: for example these comprise one or more of the sequences identified as tryptic sequences in the 3$^{rd}$ column of Table 53a;
- for lung cancer applications: for example these comprise one or more of the sequences identified as tryptic sequences in the 3$^{rd}$ column of Table 53b;
- for gastric cancer applications: for example these comprise one or more of the sequences identified as tryptic sequences in the 3$^{rd}$ column of Table 5;
- for ovarian cancer applications: for example these comprise one or more of the sequences identified as tryptic sequences in the 3$^{rd}$ column of Table 53c.

In relation to fragments, immunogenic fragments or antigenic fragments of OGTA041:

- for hepatocellular carcinoma applications: for example these comprise one or more of the sequences identified as tryptic sequences in the 3$^{rd}$ column of Table 6a;
- for lung cancer applications: for example these comprise one or more of the sequences identified as tryptic sequences in the 3$^{rd}$ column of Table 6b and Table 54;
- for pancreatic cancer applications: for example these comprise one or more of the sequences identified as tryptic sequences in the 3$^{rd}$ column of Table 6c.

In relation to fragments, immunogenic fragments or antigenic fragments of OGTA053:

- for colorectal cancer applications: for example these comprise one or more of the sequences identified as tryptic sequences in the 3$^{rd}$ column of Table 55a;
- for hepatocellular carcinoma applications: for example these comprise one or more of the sequences identified as tryptic sequences in the 3$^{rd}$ column of Table 55c;
- for lung cancer applications: for example these comprise one or more of the sequences identified as tryptic sequences in the 3$^{rd}$ column of Table 55d and Table 55e;
- for pancreatic cancer applications: for example these comprise one or more of the sequences identified as tryptic sequences in the 3<sup>rd</sup> column of Table 7;

- for renal cell cancer applications: for example these comprise one or more of the sequences identified as tryptic sequences in the 3<sup>rd</sup> column of Table 55b.

In relation to fragments, immunogenic fragments or antigenic fragments of OGTA054:

- for colorectal cancer applications: for example these comprise one or more of the sequences identified as tryptic sequences in the 3<sup>rd</sup> column of Table 56a;
- for lung cancer applications: for example these comprise one or more of the sequences identified as tryptic sequences in the 3<sup>rd</sup> column of Table 56b;
- for pancreatic cancer applications: for example these comprise one or more of the sequences identified as tryptic sequences in the 3<sup>rd</sup> column of Table 8.

In relation to fragments, immunogenic fragments or antigenic fragments of OGTA066:

- for colorectal cancer applications: for example these comprise one or more of the sequences identified as tryptic sequences in the 3<sup>rd</sup> column of Table 9a and Table 57a;
- for lung cancer applications: for example these comprise one or more of the sequences identified as tryptic sequences in the 3<sup>rd</sup> column of Table 57c;
- for pancreatic cancer applications: for example these comprise one or more of the sequences identified as tryptic sequences in the 3<sup>rd</sup> column of Table 9b;
- for renal cell cancer applications: for example these comprise one or more of the sequences identified as tryptic sequences in the 3<sup>rd</sup> column of Table 57b.

In relation to fragments, immunogenic fragments or antigenic fragments of OGTA072:

- for colorectal cancer applications: for example these comprise one or more of the sequences identified as tryptic sequences in the 3<sup>rd</sup> column of Table 10.

In relation to fragments, immunogenic fragments or antigenic fragments of OGTA074:

- for colorectal cancer applications: for example these comprise one or more of the sequences identified as tryptic sequences in the 3<sup>rd</sup> column of Table 11;
- for lung cancer applications: for example these comprise one or more of the sequences identified as tryptic sequences in the 3<sup>rd</sup> column of Table 58.

In relation to fragments, immunogenic fragments or antigenic fragments of OGTA076:

- for chronic lymphocytic leukaemia applications: for example these comprise one or more of the sequences identified as tryptic sequences in the 3<sup>rd</sup> column of Table 12a;
- for colorectal cancer applications: for example these comprise one or more of the sequences identified as tryptic sequences in the 3<sup>rd</sup> column of Table 12b and Table 59a;
- for hepatocellular carcinoma applications: for example these comprise one or more of the sequences identified as tryptic sequences in the 3<sup>rd</sup> column of Table 59c;

- for lung cancer applications: for example these comprise one or more of the sequences identified as tryptic sequences in the 3<sup>rd</sup> column of Table 59d and Table 59e;
- for ovarian cancer applications: for example these comprise one or more of the sequences identified as tryptic sequences in the 3<sup>rd</sup> column of Table 59f;
- for pancreatic cancer applications: for example these comprise one or more of the sequences identified as tryptic sequences in the 3<sup>rd</sup> column of Table 12c;
- for renal cell cancer applications: for example these comprise one or more of the sequences identified as tryptic sequences in the 3<sup>rd</sup> column of Table 59b.

In relation to fragments, immunogenic fragments or antigenic fragments of OGTA085:

- for colorectal cancer applications: for example these comprise one or more of the sequences identified as tryptic sequences in the 3<sup>rd</sup> column of Table 60a;

- for lung cancer applications: for example these comprise one or more of the sequences identified as tryptic sequences in the 3rd column of Table 13a and Table 60b;
- for melanoma applications: for example these comprise one or more of the sequences identified as tryptic sequences in the 3rd column of Table 13b;
- for retinoblastoma applications: for example these comprise one or more of the sequences identified as tryptic sequences in the 3rd column of Table 13c.

In relation to fragments, immunogenic fragments or antigenic fragments of OGTA087:

- for B-cell non-Hodgkin's lymphoma applications: for example these comprise one or more of the sequences identified as tryptic sequences in the 3rd column of Table 14a;
- for breast cancer applications: for example these comprise one or more of the sequences identified as tryptic sequences in the 3rd column of Table 14b;
- for colorectal cancer applications: for example these comprise one or more of the sequences identified as tryptic sequences in the 3rd column of Table 14c;
- for gastric cancer applications: for example these comprise one or more of the sequences identified as tryptic sequences in the 3rd column of Table 14d;
- for lung cancer applications: for example these comprise one or more of the sequences identified as tryptic sequences in the 3rd column of Table 14e;
- for lymphoid leukaemia applications: for example these comprise one or more of the sequences identified as tryptic sequences in the 3rd column of Table 14f;
- for ovarian cancer applications: for example these comprise one or more of the sequences identified as tryptic sequences in the 3rd column of Table 14g.

In relation to fragments, immunogenic fragments or antigenic fragments of OGTA088:

- for breast cancer applications: for example these comprise one or more of the sequences identified as tryptic sequences in the 3rd column of Table 15a;
- for colorectal cancer applications: for example these comprise one or more of the sequences identified as tryptic sequences in the 3rd column of Table 61a;
- for gastric cancer applications: for example these comprise one or more of the sequences identified as tryptic sequences in the 3rd column of Table 15b;
- for glioblastoma applications: for example these comprise one or more of the sequences identified as tryptic sequences in the 3rd column of Table 15c;
- for hepatocellular carcinoma applications: for example these comprise one or more of the sequences identified as tryptic sequences in the 3rd column of Table 15d;
- for lung cancer applications: for example these comprise one or more of the sequences identified as tryptic sequences in the 3rd column of Table 15e and Table 61b;
- for lymphoid leukaemia applications: for example these comprise one or more of the sequences identified as tryptic sequences in the 3rd column of Table 15f;
- for pancreatic cancer applications: for example these comprise one or more of the sequences identified as tryptic sequences in the 3rd column of Table 15g;
- for renal cell cancer applications: for example these comprise one or more of the sequences identified as tryptic sequences in the 3rd column of Table 15h;
- for retinoblastoma applications: for example these comprise one or more of the sequences identified as tryptic sequences in the 3rd column of Table 15i.

In relation to fragments, immunogenic fragments or antigenic fragments of OGTA089:

- for breast cancer applications: for example these comprise one or more of the sequences identified as tryptic sequences in the 3rd column of Table 16a;
- for lung cancer applications: for example these comprise one or more of the sequences identified as tryptic sequences in the 3rd column of Table 16b and Table 62b;
- for ovarian cancer applications: for example these comprise one or more of the sequences identified as tryptic sequences in the 3rd column of Table 16c and Table 62c;
- for renal cell cancer applications: for example these comprise one or more of the sequences identified as tryptic sequences in the 3rd column of Table 62a.

In relation to fragments, immunogenic fragments or antigenic fragments of OGTA091:

- for breast cancer applications: for example these comprise one or more of the sequences identified as tryptic sequences in the 3rd column of Table 17a;
- for cervical cancer applications: for example these comprise one or more of the sequences identified as tryptic sequences in the 3rd column of Table 17b;
- for chronic lymphocytic leukaemia applications: for example these comprise one or more of the sequences identified as tryptic sequences in the 3rd column of Table 17c;
- for colorectal cancer applications: for example these comprise one or more of the sequences identified as tryptic sequences in the 3rd column of Table 63a;
- for gastric cancer applications: for example these comprise one or more of the sequences identified as tryptic sequences in the 3rd column of Table 17d;
- for hepatocellular carcinoma applications: for example these comprise one or more of the sequences identified as tryptic sequences in the 3rd column of Table 17e and Table 63c;
- for lung cancer applications: for example these comprise one or more of the sequences identified as tryptic sequences in the 3rd column of Table 17f, Table 63d and Table 63e;
- for melanoma applications: for example these comprise one or more of the sequences identified as tryptic sequences in the 3rd column of Table 17g;
- for osteosarcoma applications: for example these comprise one or more of the sequences identified as tryptic sequences in the 3rd column of Table 17h;
- for ovarian cancer applications: for example these comprise one or more of the sequences identified as tryptic sequences in the 3rd column of Table 17i and Table 63f;
- for pancreatic cancer applications: for example these comprise one or more of the sequences identified as tryptic sequences in the 3rd column of Table 17j;
- for prostate cancer applications: for example these comprise one or more of the sequences identified as tryptic sequences in the 3rd column of Table 17k;
- for renal cell cancer applications: for example these comprise one or more of the sequences identified as tryptic sequences in the 3rd column of Table 171 and Table 63b.

In relation to fragments, immunogenic fragments or antigenic fragments of OGTA098:

- for breast cancer applications: for example these comprise one or more of the sequences identified as tryptic sequences in the 3rd column of Table 18a;
- for cervical cancer applications: for example these comprise one or more of the sequences identified as tryptic sequences in the 3rd column of Table 18b;
- for colorectal cancer applications: for example these comprise one or more of the sequences identified as tryptic sequences in the 3rd column of Table 64a;
- for hepatocellular carcinoma applications: for example these comprise one or more of the sequences identified as tryptic sequences in the 3rd column of Table 18c;
- for lung cancer applications: for example these comprise one or more of the sequences identified as tryptic sequences in the 3rd column of Table 18d, Table 64c and Table 64d;
- for osteosarcoma applications: for example these comprise one or more of the sequences identified as tryptic sequences in the 3rd column of Table 18e;
- for ovarian cancer applications: for example these comprise one or more of the sequences identified as tryptic sequences in the 3rd column of Table 64e;
- for pancreatic cancer applications: for example these comprise one or more of the sequences identified as tryptic sequences in the 3rd column of Table 18f;
- for prostate cancer applications: for example these comprise one or more of the sequences identified as tryptic sequences in the 3rd column of Table 18g;
- for renal cell cancer applications: for example these comprise one or more of the sequences identified as tryptic sequences in the 3rd column of Table 18h and Table 64b.

In relation to fragments, immunogenic fragments or antigenic fragments of OGTA101:

- for breast cancer applications: for example these comprise one or more of the sequences identified as tryptic sequences in the 3rd column of Table 19a;
- for cervical cancer applications: for example these comprise one or more of the sequences identified as tryptic sequences in the 3rd column of Table 19b;

- for colorectal cancer applications: for example these comprise one or more of the sequences identified as tryptic sequences in the 3rd column of Table 19c and Table 65a;
- for hepatocellular carcinoma applications: for example these comprise one or more of the sequences identified as tryptic sequences in the 3rd column of Table 19d and Table 65c;
- for lung cancer applications: for example these comprise one or more of the sequences identified as tryptic sequences in the 3rd column of Table 19e and Table 65d;
- for osteosarcoma applications: for example these comprise one or more of the sequences identified as tryptic sequences in the 3rd column of Table 19f;
- for ovarian cancer applications: for example these comprise one or more of the sequences identified as tryptic sequences in the 3rd column of Table 65e;
- for pancreatic cancer applications: for example these comprise one or more of the sequences identified as tryptic sequences in the 3rd column of Table 19g;
- for renal cell cancer applications: for example these comprise one or more of the sequences identified as tryptic sequences in the 3rd column of Table 65b.

In relation to fragments, immunogenic fragments or antigenic fragments of OGTA104:

- for breast cancer applications: for example these comprise one or more of the sequences identified as tryptic sequences in the 3rd column of Table 20a;
- for colorectal cancer applications: for example these comprise one or more of the sequences identified as tryptic sequences in the 3rd column of Table 66a;
- for hepatocellular carcinoma applications: for example these comprise one or more of the sequences identified as tryptic sequences in the 3rd column of Table 20b and Table 66c;
- for lung cancer applications: for example these comprise one or more of the sequences identified as tryptic sequences in the 3rd column of Table 66d;
- for ovarian cancer applications: for example these comprise one or more of the sequences identified as tryptic sequences in the 3rd column of Table 20c and Table 66e;
- for pancreatic cancer applications: for example these comprise one or more of the sequences identified as tryptic sequences in the 3rd column of Table 20d;
- for renal cell cancer applications: for example these comprise one or more of the sequences identified as tryptic sequences in the 3rd column of Table 66b.

In relation to fragments, immunogenic fragments or antigenic fragments ofOGTA106:

- for cervical cancer applications: for example these comprise one or more of the sequences identified as tryptic sequences in the 3rd column of Table 21 a;
- for hepatocellular carcinoma applications: for example these comprise one or more of the sequences identified as tryptic sequences in the 3rd column of Table 67;
- for melanoma applications: for example these comprise one or more of the sequences identified as tryptic sequences in the 3rd column of Table 21b;
- for pancreatic cancer applications: for example these comprise one or more of the sequences identified as tryptic sequences in the 3rd column of Table 21c.

In relation to fragments, immunogenic fragments or antigenic fragments of OGTA112:

- for breast cancer applications: for example these comprise one or more of the sequences identified as tryptic sequences in the 3rd column of Table 22a;
- for cervical cancer applications: for example these comprise one or more of the sequences identified as tryptic sequences in the 3rd column of Table 22b;
- for chronic lymphocytic leukaemia applications: for example these comprise one or more of the sequences identified as tryptic sequences in the 3rd column of Table 22c;
- for hepatocellular carcinoma applications: for example these comprise one or more of the sequences identified as tryptic sequences in the 3rd column of Table 22d;
- for pancreatic cancer applications: for example these comprise one or more of the sequences identified as tryptic sequences in the 3rd column of Table 22e;
- for renal cell cancer applications: for example these comprise one or more of the sequences identified as tryptic sequences in the 3rd column of Table 22f and Table 68.

In relation to fragments, immunogenic fragments or antigenic fragments of OGTA113:

- for colorectal cancer applications: for example these comprise one or more of the sequences identified as tryptic sequences in the 3rd column of Table 69a;
- for lung cancer applications: for example these comprise one or more of the sequences identified as tryptic sequences in the 3rd column of Table 69c;
- for ovarian cancer applications: for example these comprise one or more of the sequences identified as tryptic sequences in the 3rd column of Table 69d;
- for pancreatic cancer applications: for example these comprise one or more of the sequences identified as tryptic sequences in the 3rd column of Table 23;
- for renal cell cancer applications: for example these comprise one or more of the sequences identified as tryptic sequences in the 3rd column of Table 69b.

In relation to fragments, immunogenic fragments or antigenic fragments of OGTA119:

- for B-cell non-Hodgkin's lymphoma applications: for example these comprise one or more of the sequences identified as tryptic sequences in the 3rd column of Table 24a;
- for breast cancer applications: for example these comprise one or more of the sequences identified as tryptic sequences in the 3rd column of Table 24b;
- for colorectal cancer applications: for example these comprise one or more of the sequences identified as tryptic sequences in the 3rd column of Table 24c and Table 70a;
- for gastric cancer applications: for example these comprise one or more of the sequences identified as tryptic sequences in the 3rd column of Table 24d;
- for hepatocellular carcinoma applications: for example these comprise one or more of the sequences identified as tryptic sequences in the 3rd column of Table 24e;
- for lung cancer applications: for example these comprise one or more of the sequences identified as tryptic sequences in the 3rd column of Table 24f and Table 70b;
- for lymphoid leukaemia applications: for example these comprise one or more of the sequences identified as tryptic sequences in the 3rd column of Table 24g;
- for neuroblastoma applications: for example these comprise one or more of the sequences identified as tryptic sequences in the 3rd column of Table 24h;
- for osteosarcoma applications: for example these comprise one or more of the sequences identified as tryptic sequences in the 3rd column of Table 24i;
- for ovarian cancer applications: for example these comprise one or more of the sequences identified as tryptic sequences in the 3rd column of Table 70c;
- for pancreatic cancer applications: for example these comprise one or more of the sequences identified as tryptic sequences in the 3rd column of Table 24j;
- for prostate cancer applications: for example these comprise one or more of the sequences identified as tryptic sequences in the 3rd column of Table 24k;
- for renal cell cancer applications: for example these comprise one or more of the sequences identified as tryptic sequences in the 3rd column of Table 24l.

In relation to fragments, immunogenic fragments or antigenic fragments of OGTA124:

- for colorectal cancer applications: for example these comprise one or more of the sequences identified as tryptic sequences in the 3rd column of Table 71a;
- for hepatocellular carcinoma applications: for example these comprise one or more of the sequences identified as tryptic sequences in the 3rd column of Table 25a and Table 71c;
- for lung cancer applications: for example these comprise one or more of the sequences identified as tryptic sequences in the 3rd column of Table 25b and Table 71d;
- for ovarian cancer applications: for example these comprise one or more of the sequences identified as tryptic sequences in the 3rd column of Table 25c and Table 71e;
- for pancreatic cancer applications: for example these comprise one or more of the sequences identified as tryptic sequences in the 3rd column of Table 25d;
- for renal cell cancer applications: for example these comprise one or more of the sequences identified as tryptic sequences in the 3rd column of Table 25e and Table 71b.

In relation to fragments, immunogenic fragments or antigenic fragments of OGTA126:

- for breast cancer applications: for example these comprise one or more of the sequences identified as tryptic sequences in the 3rd column of Table 26a;
- for colorectal cancer applications: for example these comprise one or more of the sequences identified as tryptic sequences in the 3rd column of Table 26b;
- for hepatocellular carcinoma applications: for example these comprise one or more of the sequences identified as tryptic sequences in the 3rd column of Table 26c;
- for lung cancer applications: for example these comprise one or more of the sequences identified as tryptic sequences in the 3rd column of Table 72;
- for melanoma applications: for example these comprise one or more of the sequences identified as tryptic sequences in the 3rd column of Table 26d;
- for pancreatic cancer applications: for example these comprise one or more of the sequences identified as tryptic sequences in the 3rd column of Table 26e;
- for prostate cancer applications: for example these comprise one or more of the sequences identified as tryptic sequences in the 3rd column of Table 26f;
- for renal cell cancer applications: for example these comprise one or more of the sequences identified as tryptic sequences in the 3rd column of Table 26g.

In relation to fragments, immunogenic fragments or antigenic fragments of OGTA156:

- for acute T-cell leukaemia applications: for example these comprise one or more of the sequences identified as tryptic sequences in the 3rd column of Table 27a;
- for B-cell non-Hodgkin's lymphoma applications: for example these comprise one or more of the sequences identified as tryptic sequences in the 3rd column of Table 27b;
- for chronic lymphocytic leukaemia applications: for example these comprise one or more of the sequences identified as tryptic sequences in the 3rd column of Table 27c;
- for colorectal cancer applications: for example these comprise one or more of the sequences identified as tryptic sequences in the 3rd column of Table 73a;
- for hepatocellular carcinoma applications: for example these comprise one or more of the sequences identified as tryptic sequences in the 3rd column of Table 27d;
- for lung cancer applications: for example these comprise one or more of the sequences identified as tryptic sequences in the 3rd column of Table 73c;
- for ovarian cancer applications: for example these comprise one or more of the sequences identified as tryptic sequences in the 3rd column of Table 73d;
- for renal cell cancer applications: for example these comprise one or more of the sequences identified as tryptic sequences in the 3rd column of Table 73b.

In relation to fragments, immunogenic fragments or antigenic fragments of OGTA159:

- for breast cancer applications: for example these comprise one or more of the sequences identified as tryptic sequences in the 3rd column of Table 28a;
- for chronic lymphocytic leukaemia applications: for example these comprise one or more of the sequences identified as tryptic sequences in the 3rd column of Table 28b;
- for colorectal cancer applications: for example these comprise one or more of the sequences identified as tryptic sequences in the 3rd column of Table 28c;
- for hepatocellular carcinoma applications: for example these comprise one or more of the sequences identified as tryptic sequences in the 3rd column of Table 28d;
- for lung cancer applications: for example these comprise one or more of the sequences identified as tryptic sequences in the 3rd column of Table 74b;
- for melanoma applications: for example these comprise one or more of the sequences identified as tryptic sequences in the 3rd column of Table 28e;
- for pancreatic cancer applications: for example these comprise one or more of the sequences identified as tryptic sequences in the 3rd column of Table 28f;
- for renal cell cancer applications: for example these comprise one or more of the sequences identified as tryptic sequences in the 3rd column of Table 74a.

In relation to fragments, immunogenic fragments or antigenic fragments of OGTA168:

- for glioblastoma applications: for example these comprise one or more of the sequences identified as tryptic sequences in the 3$^{rd}$ column of Table 29a;
- for melanoma applications: for example these comprise one or more of the sequences identified as tryptic sequences in the 3$^{rd}$ column of Table 29b.

In relation to fragments, immunogenic fragments or antigenic fragments of OGTA169:

- for breast cancer applications: for example these comprise one or more of the sequences identified as tryptic sequences in the 3$^{rd}$ column of Table 30a;
- for chronic lymphocytic leukaemia applications: for example these comprise one or more of the sequences identified as tryptic sequences in the 3$^{rd}$ column of Table 30b;
- for colorectal cancer applications: for example these comprise one or more of the sequences identified as tryptic sequences in the 3$^{rd}$ column of Table 75a;
- for lung cancer applications: for example these comprise one or more of the sequences identified as tryptic sequences in the 3$^{rd}$ column of Table 75c;
- for renal cell cancer applications: for example these comprise one or more of the sequences identified as tryptic sequences in the 3$^{rd}$ column of Table 75b.

In relation to fragments, immunogenic fragments or antigenic fragments of OGTA174:

- for acute T-cell leukaemia applications: for example these comprise one or more of the sequences identified as tryptic sequences in the 3$^{rd}$ column of Table 31a;
- for chronic lymphocytic leukaemia applications: for example these comprise one or more of the sequences identified as tryptic sequences in the 3$^{rd}$ column of Table 31b;
- for lung cancer applications: for example these comprise one or more of the sequences identified as tryptic sequences in the 3$^{rd}$ column of Table 76.

In relation to fragments, immunogenic fragments or antigenic fragments of OGTA176:

- for B-cell non-Hodgkin's lymphoma applications: for example these comprise one or more of the sequences identified as tryptic sequences in the 3$^{rd}$ column of Table 32a;
- for chronic lymphocytic leukaemia applications: for example these comprise one or more of the sequences identified as tryptic sequences in the 3$^{rd}$ column of Table 32b;
- for colorectal cancer applications: for example these comprise one or more of the sequences identified as tryptic sequences in the 3$^{rd}$ column of Table 77a;
- for lung cancer applications: for example these comprise one or more of the sequences identified as tryptic sequences in the 3$^{rd}$ column of Table 77c and Table 77d;
- for renal cell cancer applications: for example these comprise one or more of the sequences identified as tryptic sequences in the 3$^{rd}$ column of Table 77b.

In relation to fragments, immunogenic fragments or antigenic fragments of OGTA177:

- for chronic lymphocytic leukaemia applications: for example these comprise one or more of the sequences identified as tryptic sequences in the 3$^{rd}$ column of Table 33;
- for colorectal cancer applications: for example these comprise one or more of the sequences identified as tryptic sequences in the 3$^{rd}$ column of Table 78a;
- for lung cancer applications: for example these comprise one or more of the sequences identified as tryptic sequences in the 3$^{rd}$ column of Table 78c and Table 78d;
- for ovarian cancer applications: for example these comprise one or more of the sequences identified as tryptic sequences in the 3$^{rd}$ column of Table 78e;
- for renal cell cancer applications: for example these comprise one or more of the sequences identified as tryptic sequences in the 3$^{rd}$ column of Table 78b;

In relation to fragments, immunogenic fragments or antigenic fragments of OGTA197:

- for colorectal cancer applications: for example these comprise one or more of the sequences identified as tryptic sequences in the 3$^{rd}$ column of Table 34a and Table 79a;
- for hepatocellular carcinoma applications: for example these comprise one or more of the sequences identified as tryptic sequences in the 3$^{rd}$ column of Table 34b and Table 79c;

- for lung cancer applications: for example these comprise one or more of the sequences identified as tryptic sequences in the 3rd column of Table 34c, Table 79d and Table 79e;
- for melanoma applications: for example these comprise one or more of the sequences identified as tryptic sequences in the 3rd column of Table 34d;
- for osteosarcoma applications: for example these comprise one or more of the sequences identified as tryptic sequences in the 3rd column of Table 34e;
- for ovarian cancer applications: for example these comprise one or more of the sequences identified as tryptic sequences in the 3rd column of Table 34f;
- for pancreatic cancer applications: for example these comprise one or more of the sequences identified as tryptic sequences in the 3rd column of Table 34g;
- for prostate cancer applications: for example these comprise one or more of the sequences identified as tryptic sequences in the 3rd column of Table 34h;
- for renal cell cancer applications: for example these comprise one or more of the sequences identified as tryptic sequences in the 3rd column of Table 34i and Table 79b.

In relation to fragments, immunogenic fragments or antigenic fragments of OGTA0202:

- for colorectal cancer applications: for example these comprise one or more of the sequences identified as tryptic sequences in the 3rd column of Table 35 and Table 80a;
- for lung cancer applications: for example these comprise one or more of the sequences identified as tryptic sequences in the 3rd column of Table 80b.

In relation to fragments, immunogenic fragments or antigenic fragments of OGTA203:

- for lung cancer applications: for example these comprise one or more of the sequences identified as tryptic sequences in the 3rd column of Table 81b;
- for ovarian cancer applications: for example these comprise one or more of the sequences identified as tryptic sequences in the 3rd column of Table 36a;
- for renal cell cancer applications: for example these comprise one or more of the sequences identified as tryptic sequences in the 3rd column of Table 36b and Table 81a.

In relation to fragments, immunogenic fragments or antigenic fragments of OGTA206:

- for breast cancer applications: for example these comprise one or more of the sequences identified as tryptic sequences in the 3rd column of Table 37a;
- for colorectal cancer applications: for example these comprise one or more of the sequences identified as tryptic sequences in the 3rd column of Table 82a;
- for lung cancer applications: for example these comprise one or more of the sequences identified as tryptic sequences in the 3rd column of Table 82b;
- for pancreatic cancer applications: for example these comprise one or more of the sequences identified as tryptic sequences in the 3rd column of Table 37b;
- for prostate cancer applications: for example these comprise one or more of the sequences identified as tryptic sequences in the 3rd column of Table 37c.

In relation to fragments, immunogenic fragments or antigenic fragments of OGTA213:

- for colorectal cancer applications: for example these comprise one or more of the sequences identified as tryptic sequences in the 3rd column of Table 83a;
- for lung cancer applications: for example these comprise one or more of the sequences identified as tryptic sequences in the 3rd column of Table 38 and Table 83b.

In relation to fragments, immunogenic fragments or antigenic fragments of OGTA214:

- for colorectal cancer applications: for example these comprise one or more of the sequences identified as tryptic sequences in the 3rd column of Table 84;
- for ovarian cancer applications: for example these comprise one or more of the sequences identified as tryptic sequences in the 3rd column of Table 39.

In relation to fragments, immunogenic fragments or antigenic fragments of OGTA216:

- for breast cancer applications: for example these comprise one or more of the sequences identified as tryptic sequences in the 3rd column of Table 40a;
- for colorectal cancer applications: for example these comprise one or more of the sequences identified as tryptic sequences in the 3rd column of Table 40b and Table 85a;
- for hepatocellular carcinoma applications: for example these comprise one or more of the sequences identified as tryptic sequences in the 3rd column of Table 85c;
- for lung cancer applications: for example these comprise one or more of the sequences identified as tryptic sequences in the 3rd column of Table 85d and Table 85e;
- for ovarian cancer applications: for example these comprise one or more of the sequences identified as tryptic sequences in the 3rd column of Table 85f;
- for pancreatic cancer applications: for example these comprise one or more of the sequences identified as tryptic sequences in the 3rd column of Table 40c;
- for renal cell cancer applications: for example these comprise one or more of the sequences identified as tryptic sequences in the 3rd column of Table 85b.

In relation to fragments, immunogenic fragments or antigenic fragments of OGTA222:

- for B-cell non-Hodgkin's lymphoma applications: for example these comprise one or more of the sequences identified as tryptic sequences in the 3rd column of Table 41 a;
- for colorectal cancer applications: for example these comprise one or more of the sequences identified as tryptic sequences in the 3rd column of Table 41b and Table 86a;
- for lung cancer applications: for example these comprise one or more of the sequences identified as tryptic sequences in the 3rd column of Table 86c;
- for renal cell applications: for example these comprise one or more of the sequences identified as tryptic sequences in the 3rd column of Table 86b.

In relation to fragments, immunogenic fragments or antigenic fragments of OGTA236:

- for colorectal cancer applications: for example these comprise one or more of the sequences identified as tryptic sequences in the 3rd column of Table 87;
- for pancreatic cancer applications: for example these comprise one or more of the sequences identified as tryptic sequences in the 3rd column of Table 42.

In relation to fragments, immunogenic fragments or antigenic fragments of OGTA237:

- for B-cell non-Hodgkin's lymphoma applications: for example these comprise one or more of the sequences identified as tryptic sequences in the 3rd column of Table 43a;
- for colorectal cancer applications: for example these comprise one or more of the sequences identified as tryptic sequences in the 3rd column of Table 88a;
- for lung cancer applications: for example these comprise one or more of the sequences identified as tryptic sequences in the 3rd column of Table 88c and table 88d;
- for lymphoid leukaemia applications: for example these comprise one or more of the sequences identified as tryptic sequences in the 3rd column of Table 43b;
- for prostate cancer applications: for example these comprise one or more of the sequences identified as tryptic sequences in the 3rd column of Table 43c;
- for renal cell cancer applications: for example these comprise one or more of the sequences identified as tryptic sequences in the 3rd column of Table 88b.

In relation to fragments, immunogenic fragments or antigenic fragments of OGTA247:

- for hepatocellular carcinoma applications: for example these comprise one or more of the sequences identified as tryptic sequences in the 3rd column of Table 44a;
- for lung cancer applications: for example these comprise one or more of the sequences identified as tryptic sequences in the 3rd column of Table 89a and Table 89b;
- for melanoma applications: for example these comprise one or more of the sequences identified as tryptic sequences in the 3rd column of Table 44b.

In relation to fragments, immunogenic fragments or antigenic fragments of OGTA248:

- for lung cancer applications: for example these comprise one or more of the sequences identified as tryptic sequences in the 3rd column of Table 90.
- for renal cell cancer applications: for example these comprise one or more of the sequences identified as tryptic sequences in the 3rd column of Table 45.

In relation to fragments, immunogenic fragments or antigenic fragments of OGTA249:

- for lung cancer applications: for example these comprise one or more of the sequences identified as tryptic sequences in the 3rd column of Table 91b;
- for ovarian cancer applications: for example these comprise one or more of the sequences identified as tryptic sequences in the 3rd column of Table 46;
- for renal cell cancer applications: for example these comprise one or more of the sequences identified as tryptic sequences in the 3rd column of Table 91a.

In relation to fragments, immunogenic fragments or antigenic fragments of OGTA257:

- for colorectal cancer applications: for example these comprise one or more of the sequences identified as tryptic sequences in the 3rd column of Table 92a;
- for hepatocellular carcinoma applications: for example these comprise one or more of the sequences identified as tryptic sequences in the 3rd column of Table 92b;
- for lung cancer applications: for example these comprise one or more of the sequences identified as tryptic sequences in the 3rd column of Table 92c;
- for ovarian cancer applications: for example these comprise one or more of the sequences identified as tryptic sequences in the 3rd column of Table 92d;
- for pancreatic cancer applications: for example these comprise one or more of the sequences identified as tryptic sequences in the 3rd column of Table 47.

In relation to fragments, immunogenic fragments or antigenic fragments of OGTA271:

- for breast cancer applications: for example these comprise one or more of the sequences identified as tryptic sequences in the 3rd column of Table 48a;
- for cervical cancer applications: for example these comprise one or more of the sequences identified as tryptic sequences in the 3rd column of Table 48b;
- for colorectal cancer applications: for example these comprise one or more of the sequences identified as tryptic sequences in the 3rd column of Table 48c and Table 93a;
- for hepatocellular carcinoma applications: for example these comprise one or more of the sequences identified as tryptic sequences in the 3rd column of Table 48d and table 93c;
- for lung cancer applications: for example these comprise one or more of the sequences identified as tryptic sequences in the 3rd column of Table 48e and Table 93d;
- for osteosarcoma applications: for example these comprise one or more of the sequences identified as tryptic sequences in the 3rd column of Table 48f;
- for pancreatic cancer applications: for example these comprise one or more of the sequences identified as tryptic sequences in the 3rd column of Table 48g;
- for renal cell cancer applications: for example these comprise one or more of the sequences identified as tryptic sequences in the 3rd column of Table 48f and table 93b.

The present disclosure additionally provides: (a) preparations comprising an isolated OGTA according to the disclosure; (b) preparations comprising one or more fragments of an OGTA according to the disclosure; and (c) antibodies or other affinity reagents that bind to an OGTA according to the disclosure, to fragments fragments thereof, or both. As used herein, OGTA(s) are "isolated" when they are present in preparations that are substantially free of contaminating proteins, *i.e.,* preparations in which less than 10% by weight (for example less than 5%, such as less than 1%) of the total protein present is contaminating protein(s). A contaminating protein is a protein having a significantly different amino acid sequence from that of an isolated OGTA according to the disclosure, as determined by mass spectral analysis. As used herein, a "significantly different" sequence is one that permits the contaminating protein to be resolved from an OGTA of the disclosure by mass spectral analysis, performed according to the Reference Protocols.

An OGTA according to the disclosure can be assayed by any method known to those skilled in the art, including but not limited to, the Preferred Technologies described herein in Examples 1 and 2, kinase assays, enzyme assays, binding assays and other functional assays, immunoassays, and western blotting. In one example, the OGTAs according to the disclosure are separated on 1-D gels by virtue of their MW and visualized by staining the gel. In one example, OGTA(s) are stained with a fluorescent dye and imaged with a fluorescence scanner. Sypro Red (Molecular Probes, Inc., Eugene, Oregon) is a suitable dye for this purpose. A preferred fluorescent dye is disclosed in U.S. Application No. 09/412,168, filed on October 5, 1999. In another example, OGTA(s) are analysed using isobaric tags for relative and absolute quantification (iTRAQ).

Alternatively, OGTAs according to the disclosure can be detected in an immunoassay. In one example, an immunoassay is performed by contacting a sample from a subject to be tested with an anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 antibody (or other affinity reagent) under conditions such that immunospecific binding can occur if the relevant OGTA is present, and detecting or measuring the amount of any immunospecific binding by the affinity reagent. Anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 and anti-OGTA271 affinity reagents can be produced by the methods and techniques taught herein.

Proteins according to the disclosure may be detected by virtue of the detection of a fragment thereof e.g. an immunogenic or antigenic fragment thereof. Fragments may have a length of at least 10, more typically at least 20 amino acids e.g. at least 50 or 100 amino acids e.g. at least 200 or 500 amino acids e.g. at least 1000 amino acids e.g. at least 1500 amino acids e.g. at least 2000 amino acids.

In one example one or more for example 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 proteins according to the disclosure are dectected using an appropriate array analysis.

In one example, binding of antibody (or other affinity reagent) in tissue sections can be used to detect aberrant OGTA(s) localization or an aberrant level of OGTA(s). In a specific example, an antibody (or other affinity reagent) to an OGTA according to the disclosure can be used to assay a patient tissue (e.g., a lymphoid, breast, cervical, colorectal, gastric, brain, liver, lung, skin, neuronal, osteoblast, ovarian, pancreatic, prostate, kidney or eye tissue) for the level of OGTA(s) where an aberrant level of OGTA(s) is indicative of a relevant cancer. As used herein, an "aberrant level" means a level that is increased compared with the level in a subject free from said cancer or a reference level. This example may be suitable for detecting elevated levels of OGTA066 in cancer tissue, such as colorectal cancer, kidney cancer, lung cancer and/or pancreatic cancer tissue.

Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, a protein according to the disclosure can be detected in a fluid sample (e.g., blood, urine, or saliva) by means of a two-step sandwich assay. In the first step, a capture reagent (e.g., an anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 antibody or other affinity reagent) is used to capture the relevant OGTA(s). The capture reagent can optionally be immobilized on a solid phase. In the second step, a directly or indirectly labeled detection reagent is used to detect the captured OGTA(s). In one example, the detection reagent is a lectin. Any lectin can be used for this purpose that preferentially binds to an OGTA according to the disclosure rather than to other isoforms that have the same core protein as an OGTA according to the disclosure or to other proteins that share the antigenic determinant recognized by the antibody. In one example, the chosen lectin binds an OGTA according to the disclosure with at least 2-fold greater affinity, more for example at least 5-fold greater affinity, still more preferably at least 10-fold greater affinity, than to said other isoforms that have the same core protein as an OGTA according to the disclosure or to said other proteins that share the antigenic determinant recognized by the affinity reagent.

Whilst not wishing to be bound by theory it is thought that in patients with a relevant cancer such as colorectal cancer, kidney cancer, lung cancer and/or pancreatic cancer that the levels of OGTA066 are reduced in fluid samples such as serum samples in comparison to a subject free of said cancer. This is thought to be because the protein seems to be recruited to the cancerous tissue and thus the levels found in the cancerous tissue are higher in patients with the relevant cancer than subjects free from same.

Based on the present description, a lectin that is suitable for detecting an OGTA according to the disclosure can readily be identified by methods well known in the art, for instance upon testing one or more lectins enumerated in Table I on pages 158-159 of Sumar et al., Lectins as Indicators of Disease-Associated Glycoforms, *In*: Gabius H-J & Gabius S (eds.), 1993, Lectins and Glycobiology, at pp. 158-174. In an alternative example, the detection reagent is an antibody (or other affinity reagent), e.g. an antibody that immunospecifically detects other post-translational modifications, such as an antibody that immunospecifically binds to phosphorylated amino acids. Examples of such antibodies include those that bind to phosphotyrosine (BD Transduction Laboratories, catalog nos.: P11230-050/P11230-150; P11120; P38820; P39020), those that bind to phosphoserine (Zymed Laboratories Inc., South San Francisco, CA, catalog no. 61-8100) and those that bind to phosphothreonine (Zymed Laboratories Inc., South San Francisco, CA, catalogue nos. 71-8200, 13-9200).

If desired, a gene encoding a protein according to the disclosure a related gene, or related nucleic acid sequences or subsequences, including complementary sequences, can also be used in hybridization assays. A nucleotide encoding a protein according to the disclosure, or subsequences thereof comprising at least 8 nucleotides, for example at least 12 nucleotides, and most preferably at least 15 nucleotides can be used as a hybridization probe. Hybridization assays can be used for detection, prognosis, diagnosis, or monitoring of conditions, disorders, or disease states, associated with aberrant expression of the gene encoding a protein according to the disclosure, or for differential diagnosis of subjects with signs or symptoms suggestive of a relevant cancer. In particular, such a hybridization assay can be carried out by a method comprising contacting a subject's sample containing nucleic acid with a nucleic acid probe capable of hybridizing to a DNA or RNA that encodes a protein according to the disclosure under conditions such that hybridization can occur, and detecting or measuring any resulting hybridization.

Hence nucleic acid encoding OGTA(s) (e.g. DNA or more suitably RNA) may be detected, for example, using a hybridizing agent capable of hybridizing to nucleic acid encoding OGTA.

One such exemplary method comprises:

(a) contacting one or more oligonucleotide probes comprising 10 or more consecutive nucleotides complementary to a nucleotide sequence encoding OGTA, with an RNA obtained from a biological sample from the subject or with cDNA copied from the RNA, wherein said contacting occurs under conditions that permit hybridization of the probe to the nucleotide sequence if present;
(b) detecting hybridization, if any, between the probe and the nucleotide sequence; and
(c) comparing the hybridization, if any, detected in step (b) with the hybridization detected in a control sample, or with a previously determined reference range.

Thus in one example the analysis for differential expression of the gene or proteins in performed using microarrays comprising probes sets, allowing simulateous analysis in relation to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 proteins according to the disclosure. This technology is well known in the relevant field.

The disclosure also provides diagnostic kits, comprising an anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 antibody (or other affinity reagent).

In addition, such a kit may optionally comprise one or more of the following: (1) instructions for using the anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 affinity reagent for diagnosis, prognosis, therapeutic monitoring or any combination of these applications; (2) a labeled binding partner to the affinity reagent; (3) a solid phase (such as a reagent strip) upon which the anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 affinity reagent is immobilized; and (4) a label or insert indicating regulatory approval for diagnostic, prognostic or therapeutic use or any combination thereof.

If no labeled binding partner to the affinity reagent is provided, the anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 affinity reagent itself can be labeled with a detectable marker, *e.g.* a chemiluminescent, enzymatic, fluorescent, or radioactive moiety.

The disclosure also provides a kit comprising a nucleic acid probe capable of hybridizing to RNA encoding a protein according to the disclosure. In a specific example, a kit comprises in one or more containers a pair of primers (e.g. each in the size range of 6-30 nucleotides, more preferably 10-30 nucleotides and still more preferably 10-20 nucleotides) that under appropriate reaction conditions can prime amplification of at least a portion of a nucleic acid encoding a protein according to the disclosure, such as by polymerase chain reaction (see, *e.g.,* Innis et al., 1990, PCR Protocols, Academic Press, Inc., San Diego, CA), ligase chain reaction (see EP 320,308) use of Qβ replicase, cyclic probe reaction, or other methods known in the art.

A kit can optionally further comprise a predetermined amount of an OGTA according to the disclosure or a nucleic acid encoding same, *e.g.* for use as a standard or control.

Use in Clinical Studies

The diagnostic methods and compositions of the present disclosure can assist in monitoring a clinical study, *e.g.* to evaluate drugs for therapy of a relevant cancer. In one example, candidate molecules are tested for their ability to restore levels of a protein(s) according to the disclosure in a subject having a relevant cancer to levels found in subjects free from said cancer or, in a treated subject, to preserve said levels at or near non-B-cell non-Hodgkin's lymphoma, non-breast cancer, non-cervical cancer, non-colorectal cancer, non-gastric cancer, non-glioblastoma, non-hepatocellular carcinoma, non-lung cancer, non-lymphoid leukaemia (particularly acute T-cell leukaemia and chronic lymphocytic leukaemia), non-melanoma, non-neuroblastoma, non-osteosarcoma, non-ovarian cancer, non-pancreatic cancer, non-prostate cancer, non-renal cell cancer or non-retinoblastoma values.

In another example, the methods and compositions of the present disclosure are used to screen candidates for a clinical study to identify individuals having a relevant cancer; such individuals can then be excluded from the study or can be placed in a separate cohort for treatment or analysis.

Production of Proteins of the Invention and Corresponding Nucleic Acid

A DNA of the present disclosure can be obtained by isolation as a cDNA fragment from cDNA libraries using as starter materials commercial mRNAs and determining and identifying the nucleotide sequences thereof. That is, specifically, clones are randomly isolated from cDNA libraries, which are prepared according to Ohara et al's method (DNA Research Vol.4, 53-59 (1997)). Next, through hybridization, duplicated clones (which appear repeatedly) are removed and then in vitro transcription and translation are carried out. Nucleotide sequences of both termini of clones, for which products of 50 kDa or more are confirmed, are determined. Furthermore, databases of known genes are searched for homology using the thus obtained terminal nucleotide sequences as queries. The entire nucleotide sequence of a clone revealed to be novel as a result is determined. In addition to the above screening method, the 5' and 3' terminal sequences of cDNA are related to a human genome sequence. Then an unknown long-chain gene is confirmed in a region between the sequences, and the full-length of the cDNA is analyzed. In this way, an unknown gene that is unable to be obtained by a conventional cloning method that depends on known genes can be systematically cloned.

Moreover, all of the regions of a human-derived gene containing a DNA of the present disclosure can also be prepared using a PCR method such as RACE while paying sufficient attention to prevent artificial errors from taking place in short fragments or obtained sequences. As described above, clones having DNA of the present disclosure can be obtained.

In another means for cloning DNA of the present disclosure, a synthetic DNA primer having an appropriate nucleotide sequence of a portion of a polypeptide of the present disclosure is produced, followed by amplification by the PCR method using an appropriate library.

Alternatively, selection can be carried out by hybridization of the DNA of the present disclosure with a DNA that has been incorporated into an appropriate vector and labeled with a DNA fragment or a synthetic DNA encoding some or all of the regions of the polypeptide of the present disclosure. Hybridization can be carried out by, for example, the method described in Current Protocols in Molecular Biology (edited by Frederick M. Ausubel et al., 1987). DNA of the present disclosure may be any DNA, as long as they contain nucleotide sequences encoding the polypeptides of the present disclosure as described above. Such a DNA may be a cDNA identified and isolated from cDNA libraries or the like that are derived from lymphoid, breast, cervical, colorectal, gastric, brain, liver, lung, skin, neuronal, osteoblast, ovarian, pancreatic, prostate, kidney or eye tissue. Such a DNA may also be a synthetic DNA or the like. Vectors for use in library construction may be any of bacteriophages, plasmids, cosmids, phargemids, or the like. Furthermore, by the use of a total RNA fraction or a mRNA fraction prepared from the above cells and/or tissues, amplification can be carried out by a direct reverse transcription coupled polymerase chain reaction (hereinafter abbreviated as "RT-PCR method").

DNA encoding the above polypeptides consisting of amino acid sequences that are substantially identical to the amino acid sequences of OGTA(s) according to the disclosure or DNA encoding the above polypeptides consisting of amino acid sequences derived from the amino acid sequences of OGTA(s) by deletion, substitution, or addition of one or more amino acids composing a portion of the amino acid sequence can be easily produced by an appropriate combination of, for example, a site-directed mutagenesis method, a gene homologous recombination method, a primer elongation method, and the PCR method known by persons skilled in the art. In addition, at this time, a possible method for causing a polypeptide to have substantially equivalent biological activity is substitution of homologous amino acids (e.g. polar and nonpolar amino acids, hydrophobic and hydrophilic amino acids, positively-charged and negatively charged amino acids, and aromatic amino acids) among amino acids composing the polypeptide. Furthermore, to maintain substantially equivalent biological activity, amino acids within functional domains contained in each polypeptide of the present disclosure are for example conserved.

Furthermore, examples of DNA of the present disclosure include DNA comprising nucleotide sequences that encode the amino acid sequences of an OGTA according to the disclosure and DNA hybridizing under stringent conditions to the DNA and encoding polypeptides (proteins) having biological activity (functions) equivalent to the functions of the polypeptides consisting of the amino acid sequence of an OGTA according to the disclosure. Under such conditions, an example of such DNA capable of hybridizing to DNA comprising the nucleotide sequences that encode the amino acid sequence of a protein according to the disclosure is DNA comprising nucleotide sequences that have a degree of overall mean homology with the entire nucleotide sequence of the DNA, such as approximately 80% or more, for example approximately 90% or more, and more preferably approximately 95% or more. Hybridization can be carried out according to a method known in the art such as a method described in Current Protocols in Molecular Biology (edited by Frederick M. Ausubel et al., 1987) or a method according thereto. Here, "stringent conditions" are, for example, conditions of approximately "1*SSC, 0.1% SDS, and 37°C, more stringent conditions of approximately "0.5*SSC, 0.1% SDS, and 42°C, or even more stringent conditions of approximately "0.2*SSC, 0.1% SDS, and 65°C. With more stringent hybridization conditions, the isolation of a DNA having high homology with a probe sequence can be expected. The above combinations of SSC, SDS, and temperature conditions are given for illustrative purposes. Stringency similar to the above can be achieved by persons skilled in the art using an appropriate combination of the above factors or other factors (for example, probe concentration, probe length, and reaction time for hybridization) for determination of hybridization stringency.

A cloned DNA of the present disclosure can be directly used or used, if desired, after digestion with a restriction enzyme or addition of a linker, depending on purposes. The DNA may have ATG as a translation initiation codon at the 5' terminal side and have TAA, TGA, or TAG as a translation termination codon at the 3' terminal side. These translation initiation and translation termination codons can also be added using an appropriate synthetic DNA adapter.

In methods of the disclosure the OGTA employed is, for example provided in isolated form, such as a form where the polypeptide has been purified to at least to some extent. The polypeptide may be provided in substantially pure form, that is to say free, to a substantial extent, from other proteins. The polypeptide can also be produced using recombinant methods, synthetically produced or produced by a combination of these methods. OGTA(s) according to the disclosure can be easily prepared by any method known by persons skilled in the art, which involves producing an expression vector containing a DNA of the present disclosure or a gene containing a DNA of the present disclosure, culturing a transformant transformed using the expression vector, generating and accumulating a polypeptide of the present disclosure or a recombinant protein containing the polypeptide, and then collecting the resultant.

Recombinant OGTA polypeptide may be prepared by processes well known in the art from genetically engineered host cells comprising expression systems. Accordingly, the present disclosure also relates to expression systems which comprise an OGTA polypeptide or nucleic acid, to host cells which are genetically engineered with such expression systems and to the production of OGTA polypeptide by recombinant techniques. For recombinant polypeptide production, host cells can be genetically engineered to incorporate expression systems or portions thereof for nucleic acids. Such incorporation can be performed using methods well known in the art, such as, calcium phosphate transfection, DEAD-dextran mediated transfection, transvection, microinjection, cationic lipid-mediated transfection, electroporation, transduction, scrape loading, ballistic introduction or infection (see e.g. Davis et al., Basic Methods in Molecular Biology, 1986 and Sambrook et al. , Molecular Cloning: A Laboratory Manual, 2nd Ed., Cold Spring Harbour laboratory Press, Cold Spring Harbour, NY, 1989).

As host cells, for example, bacteria of the genus Escherichia, Streptococci, Staphylococci, Streptomyces, bacteria of the genus Bacillus, yeast, Aspergillus cells, insect cells, insects, and animal cells are used. Specific examples of bacteria of the genus Escherichia, which are used herein, include Escherichia coli K12 and DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), and HB101 (Journal of Molecular Biology, Vol. 41, 459 (1969)). As bacteria of the genus Bacillus, for example, Bacillus subtilis MI114 (Gene, Vol. 24, 255 (1983)) and 207-21 (Journal of Biochemistry, Vol. 95, 87 (1984)) are used. As yeast, for example, Saccaromyces cerevisiae AH22, AH22R-, NA87-11A, DKD-5D, and 20B-12, Schizosaccaromyces pombe NCYC1913 and NCYC2036, and Pichia pastoris are used. As insect cells, for example, Drosophila S2 and Spodoptera Sf9 cells are used. As animal cells, for example, COS-7 and Vero monkey cells, CHO Chinese hamster cells (hereinafter abbreviated as CHO cells), dhfr-gene-deficient CHO cells, mouse L cells, mouse AtT-20 cells, mouse myeloma cells, rat GH3 cells, human FL cells, COS, HeLa, C127,3T3, HEK 293, BHK and Bowes melanoma cells are used.

Cell-free translation systems can also be employed to produce recombinant polypeptides (e.g. rabbit reticulocyte lysate, wheat germ lysate, SP6/T7 in vitro T&T and RTS 100 E. Coli HY transcription and translation kits from Roche Diagnostics Ltd., Lewes, UK and the TNT Quick coupled Transcription/Translation System from Promega UK, Southampton, UK). The expression vector can be produced according to a method known in the art. For example, the vector can be produced by (1) excising a DNA fragment containing a DNA of the present disclosure or a gene containing a DNA of the present disclosure and (2) ligating the DNA fragment downstream of the promoter in an appropriate expression vector. A wide variety of expression systems can be used, such as and without limitation, chromosomal, episomal and virus-derived systems, e.g. plasmids derived from Escherichia coli (e.g. pBR322, pBR325, pUC18, and pUC118), plasmids derived from Bacillus subtilis (e.g. pUB110, pTP5, and pC194), from bacteriophage, from transposons, from yeast episomes (e.g. pSH19 and pSH15), from insertion elements, from yeast chromosomal elements, from viruses such as baculoviruses, papova viruses such as SV40, vaccinia viruses, adenoviruses, fowl pox viruses, pseudorabies viruses and retroviruses, and vectors derived from combinations thereof, such as those derived from plasmid and bacteriophage (such as [lambda] phage) genetic elements, such as cosmids and phagemids. The expression systems may contain control regions that regulate as well as engender expression. Promoters to be used in the present disclosure may be any promoters as long as they are appropriate for hosts to be used for gene expression. For example, when a host is Escherichia coli, a trp promoter, a lac promoter, a recA promoter, a pL promoter, an lpp promoter, and the like are preferred. When a host is Bacillus subtilis, an SPO1 promoter, an SPO2 promoter, a penP promoter, and the like are preferred. When a host is yeast, a PHO5 promoter, a PGK promoter, a GAP promoter, an ADH promoter, and the like are preferred. When an animal cell is used as a host, examples of promoters for use in this case include an SRa promoter, an SV40 promoter, an LTR promoter, a CMV promoter, and an HSV-TK promoter. Generally, any system or vector that is able to maintain, propagate or express a nucleic acid to produce a polypeptide in a host may be used.

The appropriate nucleic acid sequence may be inserted into an expression system by any variety of well known and routine techniques, such as those set forth in Sambrook et al., supra. Appropriate secretion signals may be incorporated into the OGTA polypeptide to allow secretion of the translated protein into the lumen of the endoplasmic reticulum, the periplasmic space or the extracellular environment. These signals may be endogenous to the OGTA polypeptide or they may be heterologous signals. Transformation of the host cells can be carried out according to methods known in the art. For example, the following documents can be referred to: Proc. Natl. Acad. Sci. U.S.A., Vol. 69, 2110 (1972); Gene, Vol. 17, 107 (1982); Molecular & General Genetics, Vol. 168, 111 (1979); Methods in Enzymology, Vol. 194, 182-187 (1991); Proc. Natl. Acad. Sci. U.S.A.), Vol. 75, 1929 (1978); Cell Technology, separate volume 8, New Cell Technology, Experimental Protocol. 263-267 (1995) (issued by Shujunsha); and Virology, Vol. 52, 456 (1973). The thus obtained transformant transformed with an expression vector containing a DNA of the present disclosure or a gene containing a DNA of the present disclosure can be cultured according to a method known in the art. For example, when hosts are bacteria of the genus Escherichia, the bacteria are generally cultured at approximately 15°C to 43°C for approximately 3 to 24 hours. If necessary, aeration or agitation can also be added. When hosts are bacteria of the genus Bacillus, the bacteria are generally cultured at approximately 30°C to 40°C for approximately 6 to 24 hours. If necessary, aeration or agitation can also be added. When transformants whose hosts are yeast are cultured, culture is generally carried out at approximately 20°C to 35°C for approximately 24 to 72 hours using media with pH adjusted to be approximately 5 to 8. If necessary, aeration or agitation can also be added. When transformants whose hosts are animal cells are cultured, the cells are generally cultured at approximately 30°C to 40°C for approximately 15 to 60 hours using media with the pH adjusted to be approximately 6 to 8. If necessary, aeration or agitation can also be added. If an OGTA(s) polypeptide is to be expressed for use in cell-based screening assays, it is preferred that the polypeptide be produced at the cell surface. In this event, the cells may be harvested prior to use in the screening assay. If the OGTA polypeptide is secreted into the medium, the medium can be recovered in order to isolate said polypeptide. If produced intracellularly, the cells must first be lysed before the OGTA polypeptide is recovered. OGTA polypeptide can be recovered and purified from recombinant cell cultures or from other biological sources by well known methods including, ammonium sulphate or ethanol precipitation, acid extraction, anion or cation exchange chromatography, phosphocellulose chromatography, affinity chromatography, hydrophobic interaction chromatography, hydroxylapatite chromatography, molecular sieving chromatography, centrifugation methods, electrophoresis methods and lectin chromatography. In one example, a combination of these methods is used. In another example, high performance liquid chromatography is used. In a further example, an antibody which specifically binds to an OGTA polypeptide according to the disclosure can be used to deplete a sample comprising an a relevant OGTA polypeptide of said polypeptide or to purify said polypeptide.

To separate and purify a polypeptide or a protein of the present disclosure from the culture products, for example, after culture, microbial bodies or cells are collected by a known method, they are suspended in an appropriate buffer, the microbial bodies or the cells are disrupted by, for example, ultrasonic waves, lysozymes, and/or freeze-thawing, the resultant is then subjected to centrifugation or filtration, and then a crude extract of the protein can be obtained. The buffer may also contain a protein denaturation agent such as urea or guanidine hydrochloride or a surfactant such as Triton X-100(TM). When the protein is secreted in a culture solution, microbial bodies or cells and a supernatant are separated by a known method after the completion of culture and then the supernatant is collected. The protein contained in the thus obtained culture supernatant or the extract can be purified by an appropriate combination of known separation and purification methods. The thus obtained polypeptide (protein) of the present disclosure can be converted into a salt by a known method or a method according thereto. Conversely, when the polypeptide (protein) of the present disclosure is obtained in the form of a salt, it can be converted into a free protein or peptide or another salt by a known method or a method according thereto. Moreover, an appropriate protein modification enzyme such as trypsin or chymotrypsin is caused to act on a protein produced by a recombinant before or after purification, so that modification can be arbitrarily added or a polypeptide can be partially removed. The presence of a polypeptide (protein) of the present disclosure or a salt thereof can be measured by various binding assays, enzyme immunoassays using specific antibodies, and the like.

Techniques well known in the art, may be used for refolding to regenerate native or active conformations of the OGTA polypeptide when the polypeptide has been denatured during isolation and or purification. In the context of the present disclosure, OGTA polypeptide can be obtained from a biological sample from any source, such as and without limitation, a blood sample or tissue sample, e.g. lymphoid, breast, cervical, colorectal, gastric, brain, liver, lung, skin, neuronal, osteoblast, ovarian, pancreatic, prostate, kidney or eye tissue sample.

OGTA polypeptide may be in the form of a "mature protein" or may be part of a larger protein such as a fusion protein. It is often advantageous to include an additional amino acid sequence which contains secretory or leader sequences, a pre-, pro- or prepro- protein sequence, or a sequence which aids in purification such as an affinity tag, for example, but without limitation, multiple histidine residues, a FLAG tag, HA tag or myc tag.

An additional sequence that may provide stability during recombinant production may also be used. Such sequences may be optionally removed as required by incorporating a cleavable sequence as an additional sequence or part thereof. Thus, an OGTA polypeptide may be fused to other moieties including other polypeptides or proteins (for example, glutathione S-transferase and protein A). Such a fusion protein can be cleaved using an appropriate protease, and then separated into each protein. Such additional sequences and affinity tags are well known in the art. In addition to the above, features known in the art, such as an enhancer, a splicing signal, a polyA addition signal, a selection marker, and an SV40 replication origin can be added to an expression vector, if desired.

In one example one or more proteins according to the disclosure or fragments thereof, for example one or more sequences described herein may be fused with a heterologous fusion partner such as the surface protein, known as protein D from Haemophilus Influenza B, a non-structural protein from influenzae virus such as NS1, the S antigen from Hepatitis B or a protein known as LYTA such as the C terminal thereof.

Production of Affinity Reagents to the Proteins of the Invention

According to those in the art, there are three main types of immunoaffinity reagent - monoclonal antibodies, phage display antibodies and smaller antibody-derived molecules such as Affibodies, Domain Antibodies (dAbs), Nanobodies or UniBodies. In general in applications according to the present invention where the use of antibodies is stated, other affinity reagents (e.g. Affibodies, domain antibodies, Nanobodies or UniBodies) may be employed. Such substances may be said to be capable of immunospecific binding to the proteins of the invention. Where appropriate the term "affinity agent" shall be construed to embrace immunoaffinity reagents and other substances capable of specific binding to the proteins of the invention including but not limited to ligands, lectins, streptavidins, antibody mimetics and synthetic binding agents.

Production of Antibodies to the Proteins of the Invention

According to the disclosure OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271, an analog of any of same, a related protein or a fragment or derivative of any of the foregoing may be used as an immunogen to generate antibodies which immunospecifically bind such an immunogen. Such immunogens can be isolated by any convenient means, including the methods described above. The term "antibody" as used herein refers to a peptide or polypeptide derived from, modeled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a $F(ab')_2$ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody." Antibodies of the invention include, monoclonal antibodies, these may be bispecific, humanized or chimeric antibodies, single chain antibodies, Fab fragments and $F(ab')_2$ fragments, fragments produced by a Fab expression library, anti-idiotypic (anti-Id) antibodies, and epitope-binding fragments of any of the above. The immunoglobulin molecules of the invention can be of any class *(e.g.,* IgG, IgE, IgM, IgD and IgA) or subclass of immunoglobulin molecule.

The term "specifically binds" (or "immunospecifically binds") is not intended to indicate that an antibody binds exclusively to its intended target. Rather, an antibody "specifically binds" if its affinity for its intended target is about 5-fold greater when compared to its affinity for a non-target molecule. For example the affinity of the antibody will be at least about 5 fold, for example 10 fold, such as 25-fold, particularly 50-fold, and especially 100-fold or more, greater for a target molecule than its affinity for a non-target molecule. In some examples, specific binding between an antibody or other binding agent and an antigen means a binding affinity of at least $10^6$ M$^{-1}$. Antibodies may bind with affinities of at least about $10^7$ M$^{-1}$, such as between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{11}$ M$^{-1}$.

Affinity is calculated as $K_d = k_{off}/k_{on}$ ($k_{off}$ is the dissociation rate constant, $k_{on}$ is the association rate constant and $K_d$ is the equilibrium constant. Affinity can be determined at equilibrium by measuring the fraction bound (r) of labeled ligand at various concentrations (c). The data are graphed using the Scatchard equation:

$$\text{The data are graphed using the Scatchard equation: } r/c = K(n-r)$$

where

    r = moles of bound ligand/mole of receptor at equilibrium;
    c = free ligand concentration at equilibrium;
    K = equilibrium association constant; and
    n = number of ligand binding sites per receptor molecule

By graphical analysis, r/c is plotted on the Y-axis versus r on the X-axis thus producing a Scatchard plot. The affinity is the negative slope of the line. $k_{off}$ can be determined by competing bound labeled ligand with unlabeled excess ligand (see, e.g., U.S. Pat No. 6,316,409). The affinity of a targeting agent for its target molecule is for example at least about 1 x 10$^{-6}$ moles/liter, is more preferably at least about 1 x 10$^{-7}$ moles/liter, is even more preferably at least about

$1 \times 10^{-8}$ moles/liter, is yet even more preferably at least about $1 \times 10^{-9}$ moles/liter, and is most preferably at least about $1 \times 10^{-10}$ moles/liter. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

In one example, antibodies that recognize gene products of genes encoding a protein of the disclosure are publicly available. In another example, methods known to those skilled in the art are used to produce antibodies that recognize OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271, an analog thereof, a related polypeptide, or a fragment or derivative of any of the foregoing. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures (Antibody Engineering: A Practical Approach (Borrebaeck, C., ed.), 1995, Oxford University Press, Oxford; J. Immunol. 149, 3914-3920 (1992)).

In one example of the disclosure, antibodies to a specific domain of an OGTA according to the disclosure are produced. In a specific example, hydrophilic fragments of protein according to the disclosure are used as immunogens for antibody production.

In the production of antibodies, screening for the desired antibody can be accomplished by techniques known in the art, *e.g.* ELISA (enzyme-linked immunosorbent assay). For example, to select antibodies which recognize a specific domain of a protein according to the disclosure, one may assay generated hybridomas for a product which binds to an OGTA fragment containing such domain. For selection of an antibody that specifically binds a first OGTA homolog but which does not specifically bind to (or binds less avidly to) a second OGTA homolog, one can select on the basis of positive binding to the first OGTA homolog and a lack of binding to (or reduced binding to) the second OGTA homolog. Similarly, for selection of an antibody that specifically binds an OGTA according to the disclosure but which does not specifically bind to (or binds less avidly to) a different isoform of the same protein (such as a different glycoform having the same core peptide as an OGTA according to the disclosure, one can select on the basis of positive binding to an OGTA according to the disclosure and a lack of binding to (or reduced binding to) the different isoform (*e.g.*, a different glycoform). Thus, the present disclosure provides an antibody (for example a monoclonal antibody) that binds with greater affinity (for example at least 2-fold, such as at least 5-fold, particularly at least 10-fold greater affinity) to OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271than to a different isoform or isoforms (e.g., glycoforms) of same.

Polyclonal antibodies which may be used in the methods of the disclosure are heterogeneous populations of antibody molecules derived from the sera of immunized animals. Unfractionated immune serum can also be used. Various procedures known in the art may be used for the production of polyclonal antibodies to OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271, a fragment thereof, a related polypeptide, or a fragment of an OGTA related polypeptide. For example, one way is to purify polypeptides of interest or to synthesize the polypeptides of interest using, e.g., solid phase peptide synthesis methods well known in the art. See, e.g., Guide to Protein Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996. The selected polypeptides may then be used to immunize by injection various host animals, including but not limited to rabbits, mice, rats, etc., to generate polyclonal or monoclonal antibodies. The Preferred Technology described herein in Example 1 provides isolated OGTA suitable for such immunization. If an OGTA is purified by gel electrophoresis, it can be used for immunization with or without prior extraction from the polyacrylamide gel. Various adjuvants (i.e. immunostimulants) may be used to enhance the immunological response, depending on the host species, including, but not limited to, complete or incomplete Freund's adjuvant, a mineral gel such as aluminum hydroxide, surface active substance such as lysolecithin, pluronic polyol, a polyanion, a peptide, an oil emulsion, keyhole limpet hemocyanin, dinitrophenol, and an adjuvant such as BCG (bacille Calmette-Guerin) or corynebacterium parvum. Additional adjuvants are also well known in the art.

For preparation of monoclonal antibodies (mAbs) directed toward proteins of the disclosure, a fragment thereof, a related polypeptide, or a fragment of a related polypeptide, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the disclosure may be cultivated *in vitro* or *in vivo.* In an additional example of the disclosure, monoclonal antibodies can be produced in germ-free animals utilizing known technology (PCT/US90/02545,).

The monoclonal antibodies include but are not limited to human monoclonal antibodies and chimeric monoclonal antibodies (e.g., human-mouse chimeras). A chimeric antibody is a molecule in which different portions are derived from different animal species, such as those having a human immunoglobulin constant region and a variable region derived from a murine mAb. (See, *e.g.,* Cabilly et al., U.S. Patent No. 4,816,567; and Boss et al., U.S. Patent No. 4,816397.) Humanized antibodies are antibody molecules from non-human species having one or more complementarity determining regions (CDRs) from the non-human species and a framework region from a human immunoglobulin molecule. (See, *e.g.,* Queen, U.S. Patent No. 5,585,089.)

Chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, Bio/Techniques 4:214; U.S. Patent 5,225,539; Jones et al., 1986, Nature 321:552-525; Verhoeyan et al. (1988) Science 239:1534; and Beidler et al., 1988, J. Immunol. 141:4053-4060.

Completely human antibodies are for example desirable for therapeutic treatment of human subjects.

Antibodies can be produced using transgenic mice which are incapable of expressing endogenous immunoglobulin heavy and light chain genes, but which can express human heavy and light chain genes. The transgenic mice are immunized in the normal fashion with a selected antigen, *e.g.,* all or a portion of a protein according to the disclosure. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce useful (including therapeutically useful) IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.,* U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e.g.*, a mouse antibody, is used to guide the selection of a completely human antibody recognizing the same epitope. (Jespers et al. (1994) Bio/technology 12:899-903).

The antibodies of the present disclosure can also be generated by the use of phage display technology to produce and screen libraries of polypeptides for binding to a selected target. See, e.g., Cwirla et al., Pro. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. See, e.g., U.S. Patent No. 6,057,098. In particular, such phage can be utilized to display antigen binding domains expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Phage display methods that can be used to make the antibodies of the present disclosure include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT Application No. PCT/GB91/01134; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and $F(ab')_2$ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988).

Examples of techniques which can be used to produce single-chain Fvs and antibodies include those described in U.S. Patents 4,946,778 and 5,258,498; Huston et al., Methods in Enzymology, 203:46-88 (1991); Shu et al., PNAS 90:7995-7999 (1993); and Skerra et al., Science 240:1038-1040 (1988).

The disclosure further provides for the use of bispecific antibodies, which can be made by methods known in the art. Traditional production of full length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in examples when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance. In a preferred example of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3, 1994. For further details for generating bispecific antibodies see, for example, Suresh et al., Methods in Enzymology, 1986, 121:210.

The disclosure provides functionally active fragments, derivatives or analogs of anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 or anti-OGTA271 immunoglobulin molecules.

Functionally active means that the fragment, derivative or analog is able to elicit anti-anti-idiotype antibodies (*i.e.*, tertiary antibodies) that recognize the same antigen that is recognized by the antibody from which the fragment, derivative or analog is derived. Specifically, in a preferred example the antigenicity of the idiotype of the immunoglobulin molecule may be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art.

The present disclosure provides antibody fragments such as, but not limited to, F(ab')$_2$ fragments and Fab fragments. Antibody fragments which recognize specific epitopes may be generated by known techniques. F(ab')$_2$ fragments consist of the variable region, the light chain constant region and the CH1 domain of the heavy chain and are generated by pepsin digestion of the antibody molecule. Fab fragments are generated by reducing the disulfide bridges of the F(ab')$_2$ fragments. The disclosure also provides heavy chain and light chain dimers of the antibodies of the disclosure, or any minimal fragment thereof such as Fvs or single chain antibodies (SCAs) (e.g., as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody of the disclosure. Single chain antibodies are formed by linking the heavy and light chain fragments of the Fv region via an amino acid bridge, resulting in a single chain polypeptide. Techniques for the assembly of functional Fv fragments in *E. coli* may be used (Skerra et al., 1988, Science 242:1038-1041).

In other examples, the disclosure provides fusion proteins of the immunoglobulins of the disclosure (or functionally active fragments thereof), for example in which the immunoglobulin is fused via a covalent bond (e.g., a peptide bond), at either the N-terminus or the C-terminus to an amino acid sequence of another protein (or portion thereof, for example at least 10, 20 or 50 amino acid portion of the protein) that is not the immunoglobulin. For example the immunoglobulin, or fragment thereof, is covalently linked to the other protein at the N-terminus of the constant domain. As stated above, such fusion proteins may facilitate purification, increase half-life *in vivo,* and enhance the delivery of an antigen across an epithelial barrier to the immune system.

The immunoglobulins of the disclosure include analogs and derivatives that are modified, *i.e.,* by the covalent attachment of any type of molecule as long as such covalent attachment does not impair immunospecific binding. For example, but not by way of limitation, the derivatives and analogs of the immunoglobulins include those that have been further modified, *e.g.,* by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analog or derivative may contain one or more non-classical amino acids.

The foregoing antibodies can be used in methods known in the art relating to the localization and activity of an OGTA, *e.g.,* for imaging this protein, measuring levels thereof in appropriate physiological samples, in diagnostic methods, etc.

## Production of Affibodies to the Proteins of the Invention

Affibody molecules represent a new class of affinity proteins based on a 58-amino acid residue protein domain, derived from one of the IgG-binding domains of staphylococcal protein A. This three helix bundle domain has been used as a scaffold for the construction of combinatorial phagemid libraries, from which Affibody variants that target the desired molecules can be selected using phage display technology (Nord K, Gunneriusson E, Ringdahl J, Stahl S, Uhlen M, Nygren PA, Binding proteins selected from combinatorial libraries of an α-helical bacterial receptor domain, Nat Biotechnol 1997;15:772-7. Ronmark J, Gronlund H, Uhlen M, Nygren PA, Human immunoglobulin A (IgA)-specific ligands from combinatorial engineering of protein A, Eur J Biochem 2002;269:2647-55.). The simple, robust structure of Affibody molecules in combination with their low molecular weight (6 kDa), make them suitable for a wide variety of applications, for instance, as detection reagents (Ronmark J, Hansson M, Nguyen T, et al, Construction and characterization of affibody-Fc chimeras produced in Escherichia coli, J Immunol Methods 2002;261:199-211) and to inhibit receptor interactions (Sandstorm K, Xu Z, Forsberg G, Nygren PA, Inhibition of the CD28-CD80 co-stimulation signal by a CD28-binding Affibody ligand developed by combinatorial protein engineering, Protein Eng 2003;16:691-7). Further details of Affibodies and methods of production thereof may be obtained by reference to US Patent No 5831012.

Labelled Affibodies or the like may also be useful in imaging applications for determining abundance of Isoforms.

## Production of Domain Antibodies to the Proteins of the Invention

References to antibodies herein embrace references to Domain Antibodies. Domain Antibodies (dAbs) are the smallest functional binding units of antibodies, corresponding to the variable regions of either the heavy (V$_H$) or light (V$_L$) chains of human antibodies. Domain Antibodies have a molecular weight of approximately 13 kDa. Domantis has developed a series of large and highly functional libraries of fully human V$_H$ and V$_L$ dAbs (more than ten billion different sequences in each library), and uses these libraries to select dAbs that are specific to therapeutic targets. In contrast to many conventional antibodies, Domain Antibodies are well expressed in bacterial, yeast, and mammalian cell systems. Further details of domain antibodies and methods of production thereof may be obtained by reference to US Patent 6,291,158; 6,582,915; 6,593,081; 6,172,197; 6,696,245; US Serial No. 2004/0110941; European patent application No. 1433846 and European Patents 0368684 & 0616640; WO05/035572, WO04/101790, WO04/081026, WO04/058821, WO04/003019 and WO03/002609.

Production of Nanobodies to the Proteins of the Invention

Nanobodies are antibody-derived therapeutic proteins that contain the unique structural and functional properties of naturally-occurring heavy-chain antibodies. These heavy-chain antibodies contain a single variable domain (VHH) and two constant domains ($C_H2$ and $C_H3$). Importantly, the cloned and isolated VHH domain is a perfectly stable polypeptide harbouring the full antigen-binding capacity of the original heavy-chain antibody. Nanobodies have a high homology with the VH domains of human antibodies and can be further humanised without any loss of activity. Importantly, Nanobodies have a low immunogenic potential, which has been confirmed in primate studies with Nanobody lead compounds.

Nanobodies combine the advantages of conventional antibodies with important features of small molecule drugs. Like conventional antibodies, Nanobodies show high target specificity, high affinity for their target and low inherent toxicity. However, like small molecule drugs they can inhibit enzymes and readily access receptor clefts. Furthermore, Nanobodies are extremely stable, can be administered by means other than injection (see e.g. WO 04/041867) and are easy to manufacture. Other advantages of Nanobodies include recognising uncommon or hidden epitopes as a result of their small size, binding into cavities or active sites of protein targets with high affinity and selectivity due to their unique 3-dimensional, drug format flexibility, tailoring of half-life and ease and speed of drug discovery.

Nanobodies are encoded by single genes and are efficiently produced in almost all prokaryotic and eukaryotic hosts e.g. *E. coli* (see e.g. US 6,765,087), moulds (for example *Aspergillus* or *Trichoderma*) and yeast (for example *Saccharomyces*, *Kluyveromyces*, *Hansenula* or *Pichia*) (see e.g. US 6,838,254). The production process is scalable and multi-kilogram quantities of Nanobodies have been produced. Because Nanobodies exhibit a superior stability compared with conventional antibodies, they can be formulated as a long shelf-life, ready-to-use solution.

The Nanoclone method (see e.g. WO 06/079372) is a proprietary method for generating Nanobodies against a desired target, based on automated high-throughout selection of B-cells.

Production of UniBodies to the Proteins of the Disclosure

UniBody is a new proprietary antibody technology that creates a stable, smaller antibody format with an anticipated longer therapeutic window than current small antibody formats. IgG4 antibodies are considered inert and thus do not interact with the immune system. Genmab modified fully human IgG4 antibodies by eliminating the hinge region of the antibody. Unlike the full size IgG4 antibody, the half molecule fragment is very stable and is termed a UniBody. Halving the IgG4 molecule left only one area on the UniBody that can bind to disease targets and the UniBody therefore binds univalently to only one site on target cells. This univalent binding does not stimulate cancer cells to grow like bivalent antibodies might and opens the door for treatment of some types of cancer which ordinary antibodies cannot treat.

The UniBody is about half the size of a regular IgG4 antibody. This small size can be a great benefit when treating some forms of cancer, allowing for better distribution of the molecule over larger solid tumors and potentially increasing efficacy.

Fabs typically do not have a very long half-life. UniBodies, however, were cleared at a similar rate to whole IgG4 antibodies and were able to bind as well as whole antibodies and antibody fragments in pre-clinical studies. Other antibodies primarily work by killing the targeted cells whereas UniBodies only inhibit or silence the cells.

Further details of UniBodies may be obtained by reference to patent WO2007/059782,.

Expression of Affinity Reagents

Expression of Antibodies

The antibodies of the disclosure can be produced by any method known in the art for the synthesis of antibodies, in particular, by chemical synthesis or by recombinant expression, and are for example produced by recombinant expression techniques.

Recombinant expression of antibodies, or fragments, derivatives or analogs thereof, requires construction of a nucleic acid that encodes the antibody. If the nucleotide sequence of the antibody is known, a nucleic acid encoding the antibody may be assembled from chemically synthesized oligonucleotides (e.g., as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (*e.g.,* an antibody cDNA library, or cDNA library generated from any tissue or cells expressing the antibody) by PCR amplification using synthetic primers hybridizable to the 3' and 5' ends of the sequence or by cloning using an oligonucleotide probe specific for the particular gene sequence.

If an antibody molecule that specifically recognizes a particular antigen is not available (or a source for a cDNA library for cloning a nucleic acid encoding such an antibody), antibodies specific for a particular antigen may be generated by any method known in the art, for example, by immunizing an animal, such as a rabbit, to generate polyclonal antibodies or, more preferably, by generating monoclonal antibodies. Alternatively, a clone encoding at least the Fab portion of the antibody may be obtained by screening Fab expression libraries (e.g., as described in Huse et al., 1989, Science 246:1275-1281) for clones of Fab fragments that bind the specific antigen or by screening antibody libraries (See, e.g., Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, *e.g.,* PCT Publication WO 86/05807; PCT Publication WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, in vitro site directed mutagenesis (Hutchinson et al., 1978, J. Biol. Chem. 253:6551), PCT based methods, etc.

In addition, techniques developed for the production of "chimeric antibodies" (Morrison et al., 1984, Proc. Natl. Acad. Sci. 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity can be used. As described *supra*, a chimeric antibody is a molecule in which different portions are derived from different animal species, such as those having a variable region derived from a murine mAb and a human antibody constant region, *e.g.,* humanized antibodies.

Once a nucleic acid encoding an antibody molecule of the disclosure has been obtained, the vector for the production of the antibody molecule may be produced by recombinant DNA technology using techniques well known in the art. Thus, methods for preparing the proteins of the disclosure by expressing nucleic acid containing the antibody molecule sequences are described herein. Methods which are well known to those skilled in the art can be used to construct expression vectors containing antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2nd Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the disclosure.

The host cells used to express a recombinant antibody of the disclosure may be either bacterial cells such as *Escherichia coli,* or, for example, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus are an effective expression system for antibodies (Foecking et al., 1986, Gene 45:101; Cockett et al., 1990, Bio/Technology 8:2).

A variety of host-expression vector systems may be utilized to express an antibody molecule of the disclosure. Such host-expression systems represent vehicles by which the coding sequences of interest may be produced and subsequently purified, but also represent cells which may, when transformed or transfected with the appropriate nucleotide coding sequences, express the antibody molecule of the disclosure *in situ.* These include but are not limited to microorganisms such as bacteria (*e.g., E. coli, B. subtilis*) transformed with recombinant bacteriophage DNA, plasmid DNA or cosmid DNA expression vectors containing antibody coding sequences; yeast (*e.g., Saccharomyces, Pichia*) transformed with recombinant yeast expression vectors containing antibody coding sequences; insect cell systems infected with recombinant virus expression vectors (*e.g.,* baculovirus) containing the antibody coding sequences; plant cell systems infected with recombinant virus expression vectors (e.g., cauliflower mosaic virus, CaMV; tobacco mosaic virus, TMV) or transformed with recombinant plasmid expression vectors (*e.g.,* Ti plasmid) containing antibody coding sequences; or mammalian cell systems (*e.g.,* COS, CHO, BHK, 293, 3T3 cells) harboring recombinant expression constructs containing promoters derived from the genome of mammalian cells *(e.g.,* metallothionein promoter) or from mammalian viruses *(e.g.,* the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for example for the generation of pharmaceutical compositions comprising an antibody molecule, vectors which direct the expression of high levels of fusion protein products that are readily purified may be desirable. Such vectors include, but are not limited, to the *E. coli* expression vector pUR278 (Ruther et al., 1983, EMBO J. 2:1791), in which the antibody coding sequence may be ligated individually into the vector in frame with the *lac* Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems (*e.g.,* an adenovirus expression system) may be utilized.

As discussed above, a host cell strain may be chosen which modulates the expression of the inserted sequences, or modifies and processes the gene product in the specific fashion desired. Such modifications (*e.g.,* glycosylation) and processing (*e.g.,* cleavage) of protein products may be important for the function of the protein.

For long-term, high-yield production of recombinant antibodies, stable expression is preferred. For example, cell lines that stably express an antibody of interest can be produced by transfecting the cells with an expression vector comprising the nucleotide sequence of the antibody and the nucleotide sequence of a selectable (*e.g.,* neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the disclosure, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197). The coding sequences for the heavy and light chains may comprise cDNA or genomic DNA.

Once an antibody molecule of the disclosure has been recombinantly expressed, it may be purified by any method known in the art for purification of an antibody molecule, for example, by chromatography (*e.g.,* ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto $Ni^{2+}$ nitriloacetic acid-agarose columns and histidine-tagged proteins are selectively eluted with imidazole-containing buffers.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) is present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (*e.g.,* in sandwich assays) may interfere with one another sterically, etc., assay performance of an antibody may be a more important measure than absolute affinity and specificity of an antibody.

Those skilled in the art will recognise that many approaches can be taken in producing antibodies or binding fragments and screening and selecting for affinity and specificity for the various polypeptides, but these approaches do not change the scope of the disclosure.

For some applications (including therpeutica applications), antibodies (particularly monoclonal antibodies) may suitably be human or humanized animal (e.g. mouse) antibodies. Animal antibodies may be raised in animals using the human protein (e.g. OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271) as immunogen. Humanisation typically involves grafting CDRs identified thereby into human framework regions. Normally some subsequent retromutation to optimize the conformation of chains is required. Such processes are known to persons skilled in the art.

## Expression of Affibodies

The construction of affibodies has been described elsewhere (Ronnmark J, Gronlund H, Uhle' n, M., Nygren P.A°, Human immunoglobulin A (IgA)-specific ligands from combinatorial engineering of protein A, 2002, Eur. J. Biochem. 269, 2647-2655.), including the construction of affibody phage display libraries (Nord, K., Nilsson, J., Nilsson, B., Uhle' n, M. & Nygren, P.A, A combinatorial library of an a-helical bacterial receptor domain, 1995, Protein Eng. 8, 601-608. Nord, K., Gunneriusson, E., Ringdahl, J., Sta° hl, S., Uhle' n, M. & Nygren, P.A°, Binding proteins selected from combinatorial libraries of an a-helical bacterial receptor domain, 1997, Nat. Biotechnol.15, 772-777.)

The biosensor analyses to investigate the optimal affibody variants using biosensor binding studies has also been described elsewhere (Ronnmark J, Gronlund H, Uhle' n, M., Nygren P.A°, Human immunoglobulin A (IgA)-specific ligands from combinatorial engineering of protein A, 2002, Eur. J. Biochem. 269, 2647-2655.).

## Affinity Reagent Modifications

In a preferred example, anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214, anti-OGTA216, anti-OGTA222, anti-OGTA236, anti-OGTA237, anti-OGTA247, anti-OGTA248, anti-OGTA249, anti-OGTA257 and/or anti-OGTA271 affinity reagents such as antibodies or fragments thereof are conjugated to a diagnostic or therapeutic moiety.

Anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214. anti-OGTA216, anti-OGTA222, anti-OGTA236, anti-OGTA237, anti-OGTA247, anti-OGTA248, anti-OGTA249, anti-OGTA257 or anti-OGTA271 antibodies or fragments thereof can be conjugated to a therapeutic agent or drug moiety to modify a given biological response. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumour necrosis factor, □-interferon, □-interferon, nerve growth factor, platelet derived growth factor, tissue plasminogen activator, a thrombotic agent or an anti-angiogenic agent, e.g., angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor

The antibodies can be used for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present disclosure. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include $^{125}$I, $^{131}$I, $^{111}$In and $^{99}$Tc $^{68}$Ga may also be employed.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described by Segal in U.S. Patent No. 4,676,980.

An antibody with or without a therapeutic moiety conjugated to it can be used as a therapeutic that is administered alone or in combination with cytotoxic factor(s) and/or cytokine(s).

The disclosure also provides for fully human, or humanised antibodies that induce antibody-directed cell-mediated cytotoxicity (ADCC). A fully human antibody is one in which the protein sequences are encoded by naturally occurring human immunoglobulin sequences, either from isolated antibody-producing human B-lymphocytes, or from transgenic murine B-lymphocytes of mice in which the murine immunoglobulin coding chromosomal regions have been replaced by orthologous human sequences. Transgenic antibodies of the latter type include, but are not restricted to, HuMab (Medarex, Inc, CA) and Xenomouse (Abgenix Inc., CA). A humanised antibody is one in which the constant region of a non-human antibody molecule of appropriate antigen specificity, is replaced by the constant region of a human antibody, preferably of the IgG subtype, with appropriate effector functions (Morrison et al., 1984, Proc. Natl. Acad. Sci. 81:851-855; Neuberger et al., 1984, Nature 312:604-608; Takeda et al., 1985, Nature 314:452-454). Appropriate effector functions include ADCC, which is a natural process by which fully-human antibodies or humanized antibodies, when bound to targets on the surface of cancer cells, switch on the cell killing properties of lymphocytes that are part of the normal immune system. These active lymphocytes, called Natural Killer (NK) cells, use a cytotoxic process to destroy living cells to which the antibodies are bound. ADCC activity may be detected and quantified by measuring release of Europium (Eu3+) from Eu3+ labelled, living cells in the presence of an antigen-specific antibody and peripheral blood mononuclear cells extracted from an immunocompetent, living human subject. The ADCC process is described in detail in Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W.-K. et al., Journal of Clinical Oncology, 2003, 21:p 3940-3947. Suitable methods for the detection and quantification of ADCC can be found in Blomberg et al., Journal of Immunological Methods. 1986, 86:p225-9; Blomberg et al., Journal of Immunological Methods. 1986, 21;92:p117-23 and Patel & Boyd, Journal of Immunological Methods. 1995, 184:p29-38.

ADCC typically involves activation of NK cells and is dependent on the recognition of antibody-coated cells by Fc receptors on the surface of the NK cell. The Fc receptors recognize the Fc (crystalline) portion of antibodies such as IgG, bound specifically to the surface of a target cell. The Fc receptor that triggers activation of the NK cell is called CD16 or FcγRIIIa. Once the FcγRIIIa receptor is bound to the IgG Fc, the NK cell releases cytokines such as IFN-γ, and cytotoxic granules containing perforin and granzymes that enter the target cell and promote cell death by triggering apoptosis.

The induction of antibody-dependent cellular cytotoxicity (ADCC) by an antibody can be enhanced by modifications that alter interactions between the antibody constant region (Fc) and various receptors that are present on the surface of cells of the immune system. Such modifications include the reduction or absence of alpha1,6-linked fucose moieties in the complex oligosaccharide chains that are normally added to the Fc of antibodies during natural or recombinant synthesis in mammalian cells. In a preferred example, non-fucosylated anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214. anti-OGTA216, anti-OGTA222, anti-OGTA236, anti-OGTA237, anti-OGTA247, anti-OGTA248, anti-OGTA249, anti-OGTA257 or anti-OGTA271 affinity reagents such as antibodies or fragments thereof are produced for the purpose of enhancing their ability to induce the ADCC response.

Techniques for reducing or ablating alpha1,6-linked fucose moieties in the oligosaccharide chains of the Fc are well established. In one example, the recombinant antibody is synthesized in a cell line that is impaired in its ability to add fucose in an alpha 1,6 linkage to the innermost N-acetylglucosamine of the N-linked biantennary complex-type Fc oligosaccharides. Such cell lines include, but are not limited to, the rat hybridoma YB2/0, which expresses a reduced level of the alpha 1,6-fucosyltransferase gene, FUT8. Preferably, the antibody is synthesized in a cell line that is incapable of adding alpha 1,6-linked fucosyl moieties to complex oligosaccharide chains, due to the deletion of both copies of the FUT8 gene. Such cell lines include, but are not limited to, FUT8-/- CHO/DG44 cell lines. Techniques for synthesizing partially fucosylated, or non-fucosylated antibodies and affinity reagents are described in Shinkawa et al., J. Biol. Chem. 278:3466-34735 (2003); Yamane-Ohnuki et al., Biotechnology and Bioengineering 87: 614-22 (2004) and in WO00/61739 A1, WO02/31140 A1 and WO03/085107 A1. In a second example, the fucosylation of a recombinant antibody is reduced or abolished by synthesis in a cell line that has been genetically engineered to overexpress a glycoprotein-modifying glycosyl transferase at a level that maximizes the production of complex N-linked oligosaccharides carrying bisecting N-acetylglucosamine. For example, the antibody is synthesized in a Chinese Hamster Ovary cell line expressing the enzyme N-acetyl glucosamine transferase III (GnT III). Cell lines stably transfected with suitable glycoprotein-modifying glycosyl transferases, and methods of synthesizing antibodies using these cells are described in WO9954342.

A non-fucosylated antibody or affinity reagent can be used as a therapeutic that is administered alone or in combination with cytotoxic factor(s) and/or cytokine(s).

In a further modification, the amino acid sequences of the antibody Fc are altered in a way that enhances ADCC activation, without affecting ligand affinity. Examples of such modifications are described in Lazar et al., Proceedings of the National Academy of Sciences 2006, 103:p4005-4010; WO03074679 and WO2007039818. In these examples, substitution of amino acids in the antibody Fc, such as aspartate for serine at position 239, and isoleucine for glutamate at position 332, altered the binding affinity of an antibody for Fc receptors, leading to an increase in ADCC activation.

An antibody reagent with enhanced ADCC activation due to amino acid substitutions can be used as a therapeutic that is administered alone or in combination with cytotoxic factor(s) and/or cytokine(s).

Therapeutic Use of the Proteins of the Invention

The disclosure provides for treatment or prevention of various diseases and disorders by administration of a therapeutic compound. Such compounds include but are not limited to: OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271, analogs thereof, related polypeptides and derivatives (including fragments) thereof; antibodies (or other affinity reagents) to the foregoing; nucleic acids encoding an OGTA, analogs of the latter or a related polypeptides and fragments thereof; antisense nucleic acids to a gene encoding an OGTA, analogs of the latter or a related polypeptides and fragments thereof; and modulator (e.g., agonists and antagonists) of a gene encoding an OGTA or a related polypeptide. An important feature of the present disclosure is the identification of genes encoding OGTA(s) involved in a relevant cancer. B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma can be treated (e.g. to ameliorate symptoms or to retard onset or progression) or prevented by administration of a therapeutic compound that reduces function or expression of an OGTA in the serum or tissue of subjects having said cancer.

In one embodiment, one or more antibodies (or other affinity reagents) each specifically binding to OGTA126 are administered alone or in combination with one or more additional therapeutic compounds or treatments.

In one embodiment a biological product such as an antibody (or other affinity reagent) is allogeneic to the subject to which it is administered. In another example, a human OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 or a related polypeptide, a nucleotide sequence encoding any of the aforementioned moieties, or an antibody (or other affinity reagent) to a human OGTA or a related polypeptide, is administered to a human subject for therapy (e.g. to ameliorate symptoms or to retard onset or progression) or prophylaxis.

Without being limited by theory, it is conceived that the therapeutic activity of antibodies (or other affinity reagents) which specifically bind to the proteins of the disclosure may be achieved through the phenomenon of Antibody -Dependent Cell-mediated Cytotoxicity (ADCC) (see e.g. Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W.-K. et al., Journal of Clinical Oncology, 2003, 21:p 3940-3947).

Treatment and Prevention and/or diagnosis of B-cell Non-Hodgkin's Lymphoma, Breast Cancer, Cervical Cancer, Colorectal Cancer, Gastric Cancer, Glioblastoma, Hepatocellular Carcinoma, Lung Cancer, Lymphoid Leukaemia (Particularly Acute T-cell Leukaemia and Chronic Lymphocytic Leukaemia), Melanoma, Neuroblastoma, Osteosarcoma, Ovarian Cancer, <u>Pancreatic Cancer, Prostate Cancer, Renal Cell Cancer and Retinoblastoma</u>

Relevant cancers are treated or prevented by administration to a subject, suspected of having or known to have or to be at risk of developing said cancer, of a compound that modulates (*i.e.,* increases or decreases) the level or activity (*i.e.,* function or expression) of an OGTA according to the disclosure that is differentially present in the serum or tissue of subjects having a relevant cancer listed herein compared with serum or tissue of subjects free from said cancer.

In one example, the cancer/cancers is/are treated or prevented by administering to a subject suspected having or known to have or to be at risk of developing a relevant cancer a compound that upregulates (*i.e.,* increases) the level or activity (*i.e.,* function or expression) of an OGTA according to the disclosure that are decreased in the serum or tissue of subjects having a relevant cancer. Examples of such a compound include, but are not limited to, OGTA antisense oligonucleotides, ribozymes, antibodies (or other affinity reagents) directed against the proteins of the disclosure, and compounds that inhibit the enzymatic activity of the proteins of the disclosure. Other useful compounds e.g. OGTA antagonists and small molecule OGTA antagonists, can be identified using *in vitro* assays.

A relevant cancer/cancers is/are also treated or prevented by administration to a subject suspected of having or known to have said cancer or to be at risk of developing said cancer of a compound that downregulates the level or activity (i.e. function) of an OGTA that are increased in the serum or tissue of subjects having said cancer. Examples of such a compound include but are not limited to: OGTAs according to the disclosure, OGTA fragments and OGTA-related polypeptides; nucleic acids encoding an OGTA, an OGTA fragment and an OGTA-related polypeptide (e.g. for use in gene therapy); and, for those OGTA or OGTA-related polypeptides with enzymatic activity, compounds or molecules known to modulate that enzymatic activity. Other compounds that can be used, e.g. OGTA agonists, can be identified using in *in vitro* assays.

In one example, therapy or prophylaxis is tailored to the needs of an individual subject. Thus, in specific examples, compounds that promote the level or function of an OGTA according to the disclosure are therapeutically or prophylactically administered to a subject suspected of having or known to have a relevant cancer, in whom the levels or functions of an OGTA according to the disclosure are absent or are decreased relative to a control or normal reference range. In further examples, compounds that promote the level or function of an OGTA according to the disclosure are therapeutically or prophylactically administered to a subject suspected of having or known to have a relevant cancer in whom the levels or functions of an OGTA according to the disclosure are increased relative to a control or to a reference range. In further examples, compounds are employed that decrease the level or function of an OGTA according to the disclosure, for example in subjects in whom said levels or functions are increased relative to a control or to a reference range.

In further examples, compounds that decrease the level or function of OGTA according to the disclosure are therapeutically or prophylactically administered to a subject suspected of having or known to have a relevant cancer in whom said levels or functions are increased relative to a control or to a reference range.

In further examples, compounds that decrease the level or function of an OGTA according to the disclosure are therapeutically or prophylactically administered to a subject suspected of having or known to have a relevant cancer in whom said levels or functions are decreased relative to a control or to a reference range.

The change in OGTA function or level due to the administration of such compounds can be readily detected, *e.g.,* by obtaining a sample *(e.g.,* blood or urine) and assaying *in vitro* the levels or activities of an OGTA according to the disclosure, or the levels of mRNAs encoding same, or any combination of the foregoing. Such assays can be performed before and after the administration of the compound as described herein.

The compounds of the disclosure include but are not limited to any compound, e.g., a small organic molecule, protein, peptide, antibody (or other affinity reagent), nucleic acid, etc. that restores the relevant OGTAs profile towards normal. The compounds of the disclosure may be given in combination with any other chemotherapy drugs.

In accordance with the present disclosure, test samples of lymphoid, breast, cervical, colorectal, gastric, brain, liver, lung, skin, neuronal, osteoblast, ovarian, pancreatic, prostate, kidney or eye tissue, serum, plasma or urine obtained from a subject suspected of having or known to have a relevant cancer can be used for diagnosis or monitoring. In one example, a change in the abundance of an OGTA according to the disclosure in a test sample relative to a control sample (from a subject or subjects free from said cancer or a previously determined reference range indicates the presence of the relevant cancer.

In another example, the relative abundance of an OGTA according to the disclosure in a test sample compared to a control sample or a previously determined reference range indicates a subtype of the relevant cancer (*e.g.* pre-T-cell or mature T-cell acute lymphocytic leukaemia, diffuse large B-cell lymphoma, inflammatory breast cancer, squamous cell cervical carcinoma, T-cell chronic lymphocytic leukaemia, familial or sporadic colorectal cancer, gastrointestinal stromal tumours, fibrolamellar hepatocellular carcinoma, squamous cell lung carcinoma, ganglioneuroblastoma or familial neuroblastoma, parosteal or periosteal osteosarcoma, malignant papillary serous adenocarcinoma, endocrine tumours of the pancreas or bilateral, multifocal retinoblastoma or unilateral, unifocal retinoblastoma). In yet another example, the relative abundance of OGTA(s) in a test sample relative to a control sample or a previously determined reference range indicates the degree or severity of the relevant cancer (e.g., the likelihood for metastasis). In any of the aforesaid methods, detection of OGTA(s) may optionally be combined with detection of one or more of additional biomarkers for a relevant cancer. Any suitable method in the art can be employed to measure the level of OGTA(s), including but not limited to the Preferred Technologies described in Examples 1 and 2 described herein, kinase assays, immunoassays to detect and/or visualize OGTA(s) (*e.g.,* Western blot, immunoprecipitation followed by sodium dodecyl sulfate polyacrylamide gel electrophoresis, immunocytochemistry, etc.). In a further example, a change in the abundance of mRNA encoding an OGTA according to the disclosure in a test sample relative to a control sample or a previously determined reference range indicates the presence of a relevant cancer. Any suitable hybridization assay can be used to detect expression of an OGTA according to the disclosure by detecting and/or visualizing mRNA encoding an OGTA according to the disclosure (*e.g.,* Northern assays, dot blots, *in situ* hybridization, etc.).

In another example of the disclosure, labeled antibodies (or other affinity reagents), derivatives and analogs thereof, which specifically bind to an OTGA according to the disclosure can be used for diagnostic purposes to detect, diagnose, or monitor a relevant cancer. For example a relevant cancer is detected in an animal, such as in a mammal and particularly in a human.

Identification of Compounds that Inhibit the Proteins of the Disclosure to Treat B-cell Non-Hodgkin's Lymphoma, Breast Cancer, Cervical Cancer, Colorectal Cancer, Gastric Cancer, Glioblastoma, Hepatocellular Carcinoma, Lung Cancer, Lymphoid Leukaemia (Particularly Acute T-cell Leukaemia and Chronic Lymphocytic Leukaemia), Melanoma, Neuroblastoma, Osteosarcoma, Ovarian Cancer, Pancreatic Cancer, Prostate Cancer, Renal Cell Cancer and Retinoblastoma

In one example of the disclosure, B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma is treated or prevented by administration of a compound that antagonizes (inhibits) the level(s) and/or function(s) of OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 which are elevated in the serum or tissue of subjects having B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma as compared with serum or tissue of subjects free from B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma.

Compounds useful for this purpose include but are not limited to anti-OGTA002, anti-OGTA009, anti-OGTA016, anti-OGTA028, anti-OGTA037, anti-OGTA041, anti-OGTA053, anti-OGTA054, anti-OGTA066, anti-OGTA072, anti-OGTA074, anti-OGTA076, anti-OGTA085, anti-OGTA087, anti-OGTA088, anti-OGTA089, anti-OGTA091, anti-OGTA098, anti-OGTA101, anti-OGTA104, anti-OGTA106, anti-OGTA112, anti-OGTA113, anti-OGTA119, anti-OGTA124, anti-OGTA126, anti-OGTA156, anti-OGTA159, anti-OGTA168, anti-OGTA169, anti-OGTA174, anti-OGTA176, anti-OGTA177, anti-OGTA197, anti-OGTA202, anti-OGTA203, anti-OGTA206, anti-OGTA213, anti-OGTA214. anti-OGTA216, anti-OGTA222, anti-OGTA236, anti-OGTA237, anti-OGTA247, anti-OGTA248, anti-OGTA249, anti-OGTA257 or anti-OGTA271 antibodies (or other affinity reagents, and fragments and derivatives containing the binding region thereof), OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 antisense or ribozyme nucleic acids, and nucleic acids encoding dysfunctional OGTAs that are used to "knockout" endogenous OGTA function by homologous recombination (see, *e.g.,* Capecchi, 1989, Science 244:1288-1292). Other compounds that inhibit OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 function can be identified by use of known in *vitro* assays, *e.g.,* assays for the ability of a test compound to inhibit binding of an OGTA according to the disclosure to another protein or a binding partner, or to inhibit a known OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 function. Such inhibition may, for example be assayed *in vitro* or in cell culture, but genetic assays may also be employed. The Preferred Technologies described in Examples 1 and 2 can also be used to detect levels of an OGTA according to the disclosure before and after the administration of the compound. Preferably, suitable *in vitro* or *in vivo* assays are utilized to determine the effect of a specific compound and whether its administration is indicated for treatment of the affected tissue.

In a specific example, a compound that inhibits OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 function is administered therapeutically or prophylactically to a subject in whom an increased serum or tissue level or functional activity of OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 (e.g., greater than the normal level or desired level) is detected as compared with serum or tissue of subjects free from B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma or a predetermined reference range.

Methods standard in the art can be employed to measure the increase in the level or function of an OGTA according to the disclosure, as outlined above.

In one example OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 or OGTA271 inhibitor compositions include small molecules, i.e. molecules of 1000 daltons or less. Such small molecules can be identified by the screening methods described herein.

Therapeutic and Prophylactic Compositions and their Use

The disclosure provides methods of treatment (and prophylaxis) comprising administering to a subject an effective amount of a compound of the disclosure. In a preferred example, the compound is substantially purified (e.g., substantially free from substances that limit its effect or produce undesired side-effects). The subject is preferably an animal, including but not limited to animals such as cows, pigs, horses, chickens, cats, dogs, etc., and is preferably a mammal, and most preferably human. In a specific example, a non-human mammal is the subject.

Formulations and methods of administration that can be employed when the compound comprises a nucleic acid are described above; additional appropriate formulations and routes of administration are described below.

Various delivery systems are known and can be used to administer a compound of the invention, e.g., encapsulation in liposomes, microparticles, microcapsules, recombinant cells capable of expressing the compound, receptor-mediated endocytosis (see, *e.g.,* Wu and Wu, 1987, J. Biol. Chem. 262:4429-4432), construction of a nucleic acid as part of a retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (e.g., oral mucosa, rectal and intestinal mucosa, etc.) and maybe administered together with other biologically active agents. Administration can be systemic or local. In addition, it may be desirable to introduce the pharmaceutical compositions of the disclosure into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.,* by use of an inhaler or nebulizer, and formulation with an aerosolizing agent.

In a specific example, it may be desirable to administer the pharmaceutical compositions of the disclosure locally to the area in need of treatment; this may be achieved, for example, and not by way of limitation, by local infusion during surgery, topical application, e.g., by injection, by means of a catheter, or by means of an implant, said implant being of a porous, non-porous, or gelatinous material, including membranes, such as sialastic membranes, or fibers. In one example, administration can be by direct injection into lymphoid, breast, cervical, colorectal, gastric, brain, liver, lung, skin, neuronal, osteoblast, ovarian, pancreatic, prostate, kidney and eye tissue or at the site (or former site) of a malignant tumour or neoplastic or pre-neoplastic tissue.

In another example, the compound can be delivered in a vesicle, in particular a liposome (*see* Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; *see* generally *ibid*).

In yet another example, the compound can be delivered in a controlled release system. In one example, a pump may be used (see Langer, *supra*; Sefton, 1987, CRC Crit. Ref. Biomed. Eng. 14:201; Buchwald et al., 1980, Surgery 88:507; Saudek et al., 1989, N. Engl. J. Med. 321:574). In another example, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105). In yet another example, a controlled release system can be placed in proximity of the therapeutic target, *e.g.* the breast, cervix, colon, stomach, brain, liver, lung, skin, bone, ovary, pancreas, prostate, kidney or eye, thus requiring only a fraction of the systemic dose (see, *e.g.,* Goodson, in Medical Applications of Controlled Release, supra, vol. 2, pp. 115-138 (1984)).

Other controlled release systems are discussed in the review by Langer (1990, Science 249:1527-1533).

In a specific example where the compound of the disclosure is a nucleic acid encoding a protein, the nucleic acid can be administered *in vivo* to promote expression of its encoded protein, by constructing it as part of an appropriate nucleic acid expression vector and administering it so that it becomes intracellular, *e.g.,* by use of a retroviral vector (see U.S. Patent No. 4,980,286), or by direct injection, or by use of microparticle bombardment (*e.g.,* a gene gun; Biolistic, Dupont), or coating with lipids or cell-surface receptors or transfecting agents, or by administering it in linkage to a homeobox-like peptide which is known to enter the nucleus (see *e.g.,* Joliot et al., 1991, Proc. Natl. Acad. Sci. USA 88:1864-1868), etc. Alternatively, a nucleic acid can be introduced intracellularly and incorporated within host cell DNA for expression, by homologous recombination.

The present disclosure also provides pharmaceutical compositions. Such compositions comprise a therapeutically effective amount of a compound, and a pharmaceutically acceptable carrier. In a specific example, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water may be a suitable carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the subject. The formulation should suit the mode of administration.

In one embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the disclosure can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc. The amount of the compound of the disclosure which will be effective in the treatment of B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer or retinoblastoma can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight or more such as 10 or 100mg/kg. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The disclosure also provides a pharmaceutical pack or kit comprising one or more containers filled with one or more of the ingredients of the pharmaceutical compositions of the disclosure. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

Diagnosis and Treatment of B-cell Non-Hodgkin's Lymphoma, Breast Cancer, Cervical Cancer, Colorectal Cancer, Gastric Cancer, Glioblastoma, Hepatocellular Carcinoma, Lung Cancer, Lymphoid Leukaemia (Particularly Acute T-cell Leukaemia and Chronic Lymphocytic Leukaemia), Melanoma, Neuroblastoma, Osteosarcoma, Ovarian Cancer, Pancreatic Cancer, Prostate Cancer, Renal Cell Cancer and Retinoblastoma using Immunohistochemistry

Immunohistochemistry is an excellent detection technique and may therefore be very useful in the diagnosis and treatment of a relevant cancer. Immunohistochemistry may be used to detect, diagnose, or monitor a relevant cancer through the localization of OGTA antigens in tissue sections by the use of labeled antibodies (or other affinity reagents), derivatives and analogs thereof, which specifically bind to the proteins of the disclosure, as specific reagents through antigen-antibody interactions that are visualized by a marker such as fluorescent dye, enzyme, radioactive element or colloidal gold.

The advancement of monoclonal antibody technology has been of great significance in assuring the place of immunohistochemistry in the modern accurate microscopic diagnosis of human neoplasms. The identification of disseminated neoplastically transformed cells by immunohistochemistry allows for a clearer picture of cancer invasion and metastasis, as well as the evolution of the tumour cell associated immunophenotype towards increased malignancy. Future antineoplastic therapeutical approaches may include a variety of individualized immunotherapies, specific for the particular immunophenotypical pattern associated with each individual patient's neoplastic disease. For further discussion see e.g. Bodey B, The significance of immunohistochemistry in the diagnosis and therapy of neoplasms, Expert Opin Biol Ther. 2002 Apr;2(4):371-93.

**EXAMPLE 1:**

IDENTIFICATION OF MEMBRANE PROTEINS EXPRESSED IN B-CELL NON-HODGKIN'S LYMPHOMA, BREAST CANCER, CERVICAL CANCER, COLORECTAL CANCER, GASTRIC CANCER, GLIOBLASTOMA, HEPATOCELLULAR CARCINOMA, LUNG CANCER, LYMPHOID LEUKAEMIA (PARTICULARLY ACUTE T-CELL LEUKAEMIA AND CHRONIC LYMPHOCYTIC LEUKAEMIA), MELANOMA, NEUROBLASTOMA, OSTEOSARCOMA, OVARIAN CANCER, PANCREATIC CANCER, PROSTATE CANCER, RENAL CELL CANCER AND RETINOBLASTOMA BLOOD AND TISSUE SAMPLES

Using the following Reference Protocol, membrane proteins extracted from B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma tissue samples were separated by 1D gel and analysed.

**1.1 MATERIALS AND METHODS**

*1.1.1 - Plasma Membrane Fractionation*

The cells recovered from a B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma were lysed and submitted to centrifugation at 1000G. The supernatant was taken, and it was subsequently centrifuged at 3000G. Once again, the supernatant was taken, and it was then centrifuged at 100 000G.

The resulting pellet was recovered and put on 15-60% sucrose gradient.

A Western blot was used to identify sub cellular markers, and the Plasma Membrane fractions were pooled.

The pooled solution was either run directly on 1D gels (see section 1.1.4 below), or further fractionated into heparin binding and nucleotide binding fractions as described below.

*1.1.2 - Plasma Membrane Heparin-binding Fraction*

The pooled solution from 1.1.1 above was applied to a Heparin column, eluted from column and run on 1D gels (see section 1.1.4 below).

*1.1.3 - Plasma Nucleotide-binding Fraction*

The pooled solution from 1.1.1 above was applied to a Cibacrom Blue 3GA column, eluted from column and run on 1D gels (see section 1.1.4 below).

*1.1.4 - ID gel technology*

Protein or membrane pellets were solubilised in 1D sample buffer (1-2 $\mu$g/$\mu$l). The sample buffer and protein mixture was then heated to 95 °C for 3 min.

A 9-16% acrylamide gradient gel was cast with a stacking gel and a stacking comb according to the procedure described in Ausubel F.M. et al., eds., 1989, Current Protocols in Molecular Biology, Vol. II, Green Publishing Associates, Inc., and John Wiley & Sons, Inc., New York, section 10.2.

30-50 micrograms of the protein mixtures obtained from detergent and the molecular weight standards (66, 45 ,31, 21,14 kDa) were added to the stacking gel wells using a 10 microlitre pipette tip and the samples run at 40mA for 5 hours.

The plates were then prised open, the gel placed in a tray of fixer (10% acetic acid, 40% ethanol, 50% water) and shaken overnight. Following this, the gel was primed by 30 minutes shaking in a primer solution (7.5% acetic acid (75ml), 0.05% SDS (5ml of 10%)). The gel was then incubated with a fluorescent dye (7.5% acetic acid, 0.06% OGS in-house dye (600$\mu$l)) with shaking for 3 hrs. Sypro Red (Molecular Probes, Inc., Eugene, Oregon) is a suitable dye for this purpose. A preferred fluorescent dye is disclosed in U.S. Application No. 09/412,168, filed on October 5, 1999.

A computer-readable output was produced by imaging the fluorescently stained gels with an Apollo 3 scanner (Oxford Glycosciences, Oxford, UK). This scanner is developed from the scanner described in WO 96/36882 and in the Ph.D. thesis of David A. Basiji, entitled "Development of a High-throughput Fluorescence Scanner Employing Internal Reflection Optics and Phase-sensitive Detection (Total Internal Reflection, Electrophoresis)", University of Washington (1997), Volume 58/12-B of Dissertation Abstracts International, page 6686. The latest embodiment of this instrument includes the following improvements: The gel is transported through the scanner on a precision lead-screw drive system. This is preferable to laying the glass plate on the belt-driven system that is defined in the Basiji thesis as it provides a reproducible means of accurately transporting the gel past the imaging optics.

The gel is secured into the scanner against three alignment stops that rigidly hold the glass plate in a known position. By doing this in conjunction with the above precision transport system and the fact that the gel is bound to the glass plate, the absolute position of the gel can be predicted and recorded. This ensures that accurate co-ordinates of each feature on the gel can be communicated to the cutting robot for excision. This cutting robot has an identical mounting arrangement for the glass plate to preserve the positional accuracy.

The carrier that holds the gel in place has integral fluorescent markers (Designated M1, M2, M3) that are used to correct the image geometry and are a quality control feature to confirm that the scanning has been performed correctly.

The optical components of the system have been inverted. The laser, mirror, waveguide and other optical components are now above the glass plate being scanned. The embodiment of the Basiji thesis has these underneath. The glass plate is therefore mounted onto the scanner gel side down, so that the optical path remains through the glass plate. By doing this, any particles of gel that may break away from the glass plate will fall onto the base of the instrument rather than into the optics.

In scanning the gels, they were removed from the stain, rinsed with water and allowed to air dry briefly and imaged on the Apollo 3. After imaging, the gels were sealed in polyethylene bags containing a small volume of staining solution, and then stored at 4°C.

Apparent molecular weights were calculated by interpolation from a set of known molecular weight markers run alongside the samples.

*1.1.5 - Recovery and analysis of selected proteins*

Proteins were robotically excised from the gels by the process described in U.S. Patent No. 6,064,754, Sections 5.4 and 5.6, 5.7, 5.8, as is applicable to 1D-electrophoresis, with modification to the robotic cutter as follows: the cutter begins at the top of the lane, and cuts a gel disc 1.7mm in diameter from the left edge of the lane. The cutter then moves 2mm to the right, and 0.7mm down and cuts a further disc. This is then repeated. The cutter then moves back to a position directly underneath the first gel cut, but offset by 2.2mm downwards, and the pattern of three diagonal cuts are repeated. This is continued for the whole length of the gel.

NOTE: If the lane is observed to broaden significantly then a correction can be made also sideways i.e. instead of returning to a position directly underneath a previous gel cut, the cut can be offset slightly to the left (on the left of the lane) and/or the right (on the right of the lane). The proteins contained within the gel fragments were processed to generate tryptic peptides; partial amino acid sequences of these peptides were determined by mass spectroscopy as described in WO98/53323 and Application No. 09/094,996, filed June 15, 1998.

Proteins were processed to generate tryptic digest peptides. Tryptic peptides were analyzed by mass spectrometry using a PerSeptive Biosystems Voyager- DETM STR Matrix-Assisted Laser Desorption Ionization Time-of-Flight (MALDI-TOF) mass spectrometer, and selected tryptic peptides were analyzed by tandem mass spectrometry (MS/MS) using a Micromass Quadrupole Time-of-Flight (Q-TOF) mass spectrometer (Micromass, Altrincham, U.K.) equipped with a nanoflowTM electrospray Z-spray source. For partial amino acid sequencing and identification of OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271, uninterpreted tandem mass spectra of tryptic peptides were searched using the SEQUEST search program (Eng et al., 1994, J. Am. Soc. Mass Spectrom. 5:976-989), version v.C.1. Criteria for database identification included: the cleavage specificity of trypsin; the detection of a suite of a, b and y ions in peptides returned from the database, and a mass increment for all Cys residues to account for carbamidomethylation. The database searched was a database constructed of protein entries in the non-redundant database held by the National Centre for Biotechnology Information (NCBI) which is accessible at http://www.ncbi.nlm.nih.gov/. Following identification of proteins through spectral-spectral correlation using the SEQUEST program, masses detected in MALDI-TOF mass spectra were assigned to tryptic digest peptides within the proteins identified. In cases where no amino acid sequences could be identified through searching with uninterpreted MS/MS spectra of tryptic digest peptides using the SEQUEST program, tandem mass spectra of the peptides were interpreted manually, using methods known in the art. (In the case of interpretation of low-energy fragmentation mass spectra of peptide ions see Gaskell et al., 1992, Rapid Commun. Mass Spectrom. 6:658-662).

*1.1.6 - Discrimination of B-cell non-Hodgkin's lymphoma, breast cancel, cervical cancer, colorectal cancer, gastric cancer, glioblastoma, Hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma associated proteins*

The process to identify OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 uses the peptide sequences obtained experimentally by mass spectrometry described above of naturally occurring human proteins to identify and organise coding exons in the published human genome sequence.

Recent dramatic advances in defining the chemical sequence of the human genome have led to the near completion of this immense task (Venter, J.C. et al. (2001). The sequence of the human genome. Science 16: 1304-51; International Human Genome Sequencing Consortium. (2001). Initial sequencing and analysis of the human genome Nature 409: 860-921). There is little doubt that this sequence information will have a substantial impact on our understanding of many biological processes, including molecular evolution, comparative genomics, pathogenic mechanisms and molecular medicine. For the full medical value inherent in the sequence of the human genome to be realised, the genome needs to be 'organised' and annotated. By this, is meant at least the following three things: (i) The assembly of the sequences of the individual portions of the genome into a coherent, continuous sequence for each chromosome. (ii) The unambiguous identification of those regions of each chromosome that contain genes. (iii) Determination of the fine structure of the genes and the properties of its mRNA and protein products. While the definition of a 'gene' is an increasingly complex issue (H Pearson: What is a gene? Nature (2006) 24: 399 - 401), what is of immediate interest for drug discovery and development is a catalogue of those genes that encode functional, expressed proteins. A subset of these genes will be involved in the molecular basis of most if not all pathologies. Therefore an important and immediate goal for the pharmaceutical industry is to identify all such genes in the human genome and describe their fine structure.

Processing and integration of peptide masses, peptide signatures, ESTs and Public Domain Genomic Sequence Data to form OGAP® database

Discrete genetic units (exons, transcripts and genes) were identified using the following sequential steps:

1. A 'virtual transcriptome' is generated, containing the tryptic peptides which map to the human genome by combining the gene identifications available from Ensembl and various gene prediction programs. This also incorporates SNP data (from dbSNP) and all alternate splicing of gene identifications. Known contaminants were also added to the virtual transcriptome.

2. All tandem spectra in the OGeS Mass Spectrometry Database are interpreted in order to produce a peptide that can be mapped to one in the virtual transcriptome. A set of automated spectral interpretation algorithms were used to produce the peptide identifications.

3. The set of all mass-matched peptides in the OGeS Mass Spectrometry Database is generated by searching all peptides from transcripts hit by the tandem peptides using a tolerance based on the mass accuracy of the mass spectrometer, typically 20ppm.

4. All tandem and mass-matched peptides are combined in the form of "protein clusters". This is done using a recursive process which groups sequences into clusters based on common peptide hits. Biological sequences are considered to belong to the same cluster if they share one or more tandem or mass-matched peptide.

5. After initial filtering to screen out incorrectly identified peptides, the resulting clusters are then mapped on the human genome.

6. The protein clusters are then aggregated into regions that define preliminary gene boundaries using their proximity and the co-observation of peptides within protein clusters. Proximity is defined as the peptide being within 80,000 nucleotides on the same strand of the same chromosome. Various elimination rules, based on cluster observation scoring and multiple mapping to the genome are used to refine the output. The resulting 'confirmed genes' are those which best account for the peptides and masses observed by mass spectrometry in each cluster. Nominal co-ordinates for the gene are also an output of this stage.

7. The best set of transcripts for each confirmed gene are created from the protein clusters, peptides, ESTs, candidate exons and molecular weight of the original protein spot.

8. Each identified transcript was linked to the sample providing the observed peptides.

9. Use of an application for viewing and mining the data. The result of steps 1-8 was a database containing genes, each of which consisted of a number of exons and one or more transcripts. An application was written to display and search this integrated genome / proteome data. Any features (OMIM disease locus, InterPro etc.) that had been mapped to the same Golden Path co-ordinate system by Ensembl could be cross-referenced to these genes by coincidence of location and fine structure.

The process was used to generate approximately 1 million peptide sequences to identify protein-coding genes and their exons resulted in the identification of protein sequences for 18083 genes across 67 different tissues and 57 diseases including 2,025 genes in acute T-cell leukaemia, 501 genes in B-cell non-Hodgkin's lymphoma, 4,713 genes in breast cancer, 1,371 genes in cervical cancer, 2,424 genes in chronic lymphocytic leukaemia, 949 genes in colorectal cancer, 524 genes in gastric cancer, 1,544 genes in glioblastoma, 1,782 genes in hepatocellular carcinoma, 978 genes in lung cancer, 373 genes in lymphoid leukaemia (unspecified), 1,764 genes in melanoma, 1,391 genes in neuroblastoma, 1,324 genes in osteosarcoma, 1,033 genes in ovarian cancer, 2,961 genes in pancreatic cancer, 3,307 genes in prostate cancer, 1005 genes in renal cell cancer and 1,783 genes in retinoblastoma, illustrated here by OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 isolated and identified from B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma samples. Following comparison of the experimentally determined sequences with sequences in the OGAP® database, OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271 showed a high degree of specificity to B-cell non-Hodgkin's lymphoma, breast cancer, cervical cancer, colorectal cancer, gastric cancer, glioblastoma, hepatocellular carcinoma, lung cancer, lymphoid leukaemia (particularly acute T-cell leukaemia and chronic lymphocytic leukaemia), melanoma, neuroblastoma, osteosarcoma, ovarian cancer, pancreatic cancer, prostate cancer, renal cell cancer and retinoblastoma indicative of the prognostic and diagnostic nature.

## 1.2 RESULTS

These experiments identified OGTA002, OGTA009, OGTA016, OGTA028, OGTA037, OGTA041, OGTA053, OGTA054, OGTA066, OGTA072, OGTA074, OGTA076, OGTA085, OGTA087, OGTA088, OGTA089, OGTA091, OGTA098, OGTA101, OGTA104, OGTA106, OGTA112, OGTA113, OGTA119, OGTA124, OGTA126, OGTA156, OGTA159, OGTA168, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271, as further described herein.

The full-length OGTA002 was detected in the plasma membrane of breast cancer, colorectal cancer, hepatocellular carcinoma, melanoma, ovarian cancer and pancreatic cancer samples. The full-length OGTA009 was detected in the plasma membrane of breast cancer, colorectal cancer, pancreatic cancer, prostate cancer and renal cell cancer samples. The full-length OGTA016 was detected in the plasma membrane of colorectal cancer samples. The full-length OGTA028 was detected in the plasma membrane of ovarian cancer samples. The full-length OGTA037 was detected in the plasma membrane of gastric cancer samples. The full-length OGTA041 was detected in the plasma membrane of hepatocellular carcinoma, lung cancer and pancreatic cancer samples. The full-length OGTA053 was detected in the plasma membrane of pancreatic cancer samples. The full-length OGTA054 was detected in the plasma membrane of pancreatic cancer samples. The full-length OGTA066 was detected in the plasma membrane of colorectal cancer and pancreatic cancer samples. The full-length OGTA072 was detected in the plasma membrane of colorectal cancer samples. The full-length OGTA074 was detected in the plasma membrane of colorectal cancer samples. The full-length OGTA076 was detected in the plasma membrane of chronic lymphocytic leukaemia, colorectal cancer and pancreatic cancer samples. The full-length OGTA085 was detected in the plasma membrane of lung cancer, melanoma and retinoblastoma samples. The full-length OGTA087 was detected in the plasma membrane of B-cell non-Hodgkin's lymphoma, breast cancer, colorectal cancer, gastric cancer, lung cancer, lymphoid leukaemia and ovarian cancer samples. The full-length OGTA088 was detected in the plasma membrane of breast cancer, gastric cancer, glioblastoma, hepatocellular carcinoma, lung cancer, lymphoid leukaemia, pancreatic cancer, renal cell cancer and retinoblastoma samples. The full-length OGTA089 was detected in the plasma membrane of breast cancer, lung cancer and ovarian cancer samples. The full-length OGTA091 was detected in the plasma membrane of breast cancer, cervical cancer, chronic lymphocytic leukaemia, gastric cancer, hepatocellular carcinoma, lung cancer, melanoma, osteosarcoma, ovarian cancer, pancreatic cancer, prostate cancer and renal cell cancer samples. The full-length OGTA098 was detected in the plasma membrane of breast cancer, cervical cancer, hepatocellular carcinoma, lung cancer, osteosarcoma, pancreatic cancer, prostate cancer and renal cell cancer samples. The full-length OGTA101 was detected in the plasma membrane of breast cancer, cervical cancer, colorectal cancer, hepatocellular carcinoma, lung cancer, osteosarcoma and pancreatic cancer samples. The full-length OGTA104 was detected in the plasma membrane of breast cancer, hepatocellular carcinoma, ovarian cancer and pancreatic cancer samples. The full-length OGTA106 was detected in the plasma membrane of cervical cancer, melanoma and pancreatic cancer samples. The full-length OGTA112 was detected in the plasma membrane of breast cancer, cervical cancer, chronic lymphocytic leukaemia, hepatocellular carcinoma, pancreatic cancer and renal cell cancer samples. The full-length OGTA113 was detected in the plasma membrane of pancreatic cancer samples. The full-length OGTA119 was detected in the plasma membrane of B-cell non-Hodgkin's lymphoma, breast cancer, colorectal cancer, gastric cancer, hepatocellular carcinoma, lung cancer, lymphoid leukaemia, neuroblastoma, osteosarcoma, pancreatic cancer, prostate cancer and renal cell cancer samples. The full-length OGTA124 was detected in the plasma membrane of hepatocellular carcinoma, lung cancer, ovarian cancer, pancreatic cancer and renal cell cancer samples. The full-length OGTA126 was detected in the plasma membrane of breast cancer, colorectal cancer, hepatocellular carcinoma, melanoma, pancreatic cancer, prostate cancer and renal cell cancer samples. The full-length OGTA156 was detected in the plasma membrane of acute T-cell leukaemia, B-cell non-Hodgkin's lymphoma, chronic lymphocytic leukaemia and hepatocellular carcinoma samples. The full-length OGTA159 was detected in the plasma membrane of breast cancer, chronic lymphocytic leukaemia, colorectal cancer, hepatocellular carcinoma, melanoma and pancreatic cancer samples. The full-length OGTA168 was detected in the plasma membrane of glioblastoma and melanoma samples. The full-length OGTA169 was detected in the plasma membrane of breast cancer and chronic lymphocytic leukaemia samples. The full-length OGTA174 was detected in the plasma membrane of acute T-cell leukaemia and chronic lymphocytic leukaemia samples. The full-length OGTA176 was detected in the plasma membrane of B-cell non-Hodgkin's lymphoma and chronic lymphocytic leukaemia samples. The full-length OGTA177 was detected in the plasma membrane of chronic lymphocytic leukaemia samples. The full-length OGTA197 was detected in the plasma membrane of colorectal cancer, hepatocellular carcinoma, lung cancer, melanoma, osteosarcoma, ovarian cancer, pancreatic cancer, prostate cancer and renal cell cancer samples. The full-length OGTA202 was detected in the plasma membrane of colorectal cancer samples. The full-length OGTA203 was detected in the plasma membrane of ovarian cancer and renal cell cancer samples. The full-length OGTA206 was detected in the plasma membrane of breast cancer, pancreatic cancer and prostate cancer samples. The full-length OGTA213 was detected in the plasma membrane of lung cancer samples. The full-length OGTA214 was detected in the plasma membrane of ovarian cancer samples. The full-length OGTA216 was detected in the plasma membrane of breast cancer, colorectal cancer and pancreatic cancer samples. The full-length OGTA222 was detected in the plasma membrane of B-cell non-Hodgkin's lymphoma and colorectal cancer samples. The full-length OGTA236 was detected in the plasma membrane of pancreatic cancer samples. The full-length OGTA237 was detected in the plasma membrane of B-cell non-Hodgkin's lymphoma, lymphoid leukaemia and prostate cancer samples. The full-length OGTA247 was detected in the plasma membrane of hepatocellular carcinoma and melanoma samples. The full-length OGTA248 was detected in the plasma membrane of renal cell cancer samples. The full-length OGTA249 was detected in the plasma membrane of ovarian cancer samples. The full-length OGTA257 was detected in the plasma membrane of pancreatic cancer samples. The full-length OGTA271 was detected in the plasma membrane of breast cancer, cervical cancer, colorectal cancer, hepatocellular carcinoma, lung cancer, osteosarcoma, pancreatic cancer and renal cell cancer samples.

EXAMPLE 2: IDENTIFICATION OF MEMBRANE PROTEINS EXPRESSED IN COLORECTAL CANCER, KIDNEY CANCER, LIVER CANCER, LUNG CANCER OR OVARIAN CANCER BLOOD AND TISSUE SAMPLES USING ISOTOPE TAGGING FOR ABSOLUTE AND RELATIVE QUANTITATION (iTRAQ)

Using the following Reference Protocol, membrane proteins extracted from colorectal cancer, kidney cancer, liver cancer, lung cancer and ovarian cancer tissue and normal adjacent colorectal and lung tissue samples were digested, labelled with Isotope Tagging for Absolute & Relative Quantitation reagents (iTRAQ; Applied Biosystems, Foster City, CA, USA) and resulting peptides sequenced by tandem mass spectrometry.

*2.1 MATERIALS AND METHODS*

*2.1.1 - Plasma Membrane Fractionation*

The cells recovered from a colorectal cancer, kidney cancer, liver cancer, lung cancer or ovarian cancer or normal adjacent colorectal, kidney, liver, lung or ovarian tissue were lysed and submitted to centrifugation at 1000G. The supernatant was taken, and it was subsequently centrifuged at 3000G. Once again, the supernatant was taken, and it was then centrifuged at 100 000G.

The resulting pellet was recovered and put on 15-60% sucrose gradient.

A Western blot was used to identify sub cellular markers, and the Plasma Membrane fractions were pooled.

The pooled solution was then analysed directly by iTRAQ (see section 2.1.2 below).

*2.1.2 - iTRAQ methodology*

Membrane protein pellets from colorectal cancer, kidney cancer, liver cancer, lung cancer or ovarian cancer and normal adjacent colorectal, kidney, liver, lung or ovarian tissue were solubilised in sample buffer (2-4 $\mu$g/$\mu$l in 0.5% SDS) by the addition of buffer and then heating to 95°C for 3 min.

To a volume of each protein solution equating to 50$\mu$g, 150$\mu$l of 0.5M triethylammonium bicarbonate (TEAB) solution was added. To each sample, 3$\mu$l of 50mM tris-(2-carboxyethyl)phosphine was added and the mixture was incubated at 60°C for 1 hour. 1 $\mu$l of cysteine blocking reagent, 200mM methyl methanethiosulphonate (MMTS) in isopropanol, was then added. After incubation at room temperature for 10 minutes, 15 $\mu$l of 1 $\mu$g/$\mu$l trypsin was added to each sample followed by incubation at 37°C overnight.

The digested samples were dried under a vacuum and re-constituted with 30$\mu$l or 0.5M TEAB solution. 70$\mu$l ethanol was added to each of the four iTRAQ reagents (114/115/116/117) and one reagent added to each of the four samples analysed (two colorectal cancer, kidney cancer, liver cancer, lung cancer or ovarian cancer samples and two corresponding normal adjacent tissue samples) and left at room temperature for 1 hour. The specific reagent added to each sample was recorded. The four labeled samples were combined & vortexed.

The combined sample was reduced to dryness under a vacuum and de-salted by loading onto a C18 spin column, washing with aqueous solvent and then eluting with 70% acetonitrile. The sample fraction was again reduced to dryness and then re-dissolved in 40$\mu$l of solvent A (97.9 water, 2% acetonitrile, 0.1% formic acid) prior to ion exchange fractionation.

*2.1.3 - Fractionation and analysis of labeled peptides*

The sample was fractionated by strong cation exchange chromatography using an Agilent 1200 chromatograph (Agilent, Santa Clara, CA, USA). Samples were eluted off an Agilent Zorbax Bio-SCXII column (3.5$\mu$m; 50 x 0.8mm) using a 20$\mu$l/min gradient of 0-100mM sodium acetate over 20 minutes and then to 1M over 10 minutes. 1 minute fractions were collected over the 30 minute run.

Each fraction was analysed by liquid chromatography/mass spectrometry using an Agilent 1200 chromatograph fitted with a Zorbax 300SB-C18 (150mm x 75$\mu$m) and an Agilent 6510 quadrupole - time-of-flight instrument (Agilent, Santa Clara, CA, USA). Peptides were eluted with a 300nl/min gradient increasing from 15% to 45% acetonitrile in 60 minutes. Data was acquired in auto MS/MS mode such that up to 3 precursor ions above the intensity threshold were selected and product ion spectra accumulated to facilitate the sequencing of the labeled peptides. Raw was processed to create peak lists using Spectrum Mill software (Agilent, Santa Clara, CA, USA).

*2.1.4 - Amino acid sequence analysis of labeled peptides*

For partial amino acid sequencing and identification of OGTA002, OGTA009, OGTA016, OGTA028, OGTA037, OGTA041, OGTA053, OGTA054, OGTA066, OGTA074, OGTA076, OGTA085, OGTA088, OGTA089, OGTA091, OGTA098, OGTA101, OGTA104, OGTA106, OGTA112, OGTA113, OGTA119, OGTA124, OGTA126, OGTA156, OGTA159, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271, uninterpreted tandem mass spectra of tryptic peptides were searched using the SEQUEST search program (Eng et al., 1994, J. Am. Soc. Mass Spectrom. 5:976-989). Criteria for database identification included: the cleavage specificity of trypsin; the detection of a suite of a, b and y ions in peptides returned from the database, and a mass increment for all cysteine residues to account for modification with methyl methanethiosulphonate and the addition of iTRAQ labels to free amines (N-terminus & lysine). The data was searched through IPI Human v3.23 (www.ebi.ac.uk/IPI/IPIhuman.html).

*2.1.5 - Discrimination of colorectal cancer, kidney cancer, liver cancer, lung cancer and ovarian cancer associated proteins*

The process described in Example 1 section 1.1.6 was employed to discriminate the colorectal cancer, kidney cancer, liver cancer, lung cancer and ovarian cancer associated proteins in the experimental samples.

## 2.2 RESULTS

These experiments identified OGTA002, OGTA009, OGTA016, OGTA028, OGTA037, OGTA041, OGTA053, OGTA054, OGTA066, OGTA074, OGTA076, OGTA085, OGTA088, OGTA089, OGTA091, OGTA098, OGTA101, OGTA104, OGTA106, OGTA112, OGTA113, OGTA119, OGTA124, OGTA126, OGTA156, OGTA159, OGTA169, OGTA174, OGTA176, OGTA177, OGTA197, OGTA202, OGTA203, OGTA206, OGTA213, OGTA214, OGTA216, OGTA222, OGTA236, OGTA237, OGTA247, OGTA248, OGTA249, OGTA257 and OGTA271, as further described herein.

The full-length OGTA002 was detected in the plasma membrane of colorectal cancer, hepatocellular carcinoma, lung cancer and renal cell cancer samples. The full-length OGTA009 was detected in the plasma membrane of colorectal cancer, lung cancer and ovarian cancer samples. The full-length OGTA016 was detected in the plasma membrane of colorectal cancer and lung cancer samples. The full-length OGTA028 was detected in the plasma membrane of colorectal cancer, hepatocellular carcinoma, lung cancer, ovarian cancer and renal cell cancer samples. The full-length OGTA037 was detected in the plasma membrane of colorectal cancer, lung cancer and ovarian cancer samples. The full-length OGTA041 was detected in the plasma membrane of lung cancer samples. The full-length OGTA053 was detected in the plasma membrane of colorectal cancer, hepatocellular carcinoma, lung cancer and renal cell cancer samples. The full-length OGTA054 was detected in the plasma membrane of colorectal cancer and lung cancer samples. The full-length OGTA066 was detected in the plasma membrane of colorectal cancer, lung cancer and renal cell cancer samples. The full-length OGTA074 was detected in the plasma membrane of lung cancer samples. The full-length OGTA076 was detected in the plasma membrane of colorectal cancer, hepatocellular carcinoma, lung cancer, ovarian cancer and renal cell cancer samples. The full-length OGTA085 was detected in the plasma membrane of colorectal cancer and lung cancer samples. The full-length OGTA088 was detected in the plasma membrane of colorectal cancer and lung cancer samples. The full-length OGTA089 was detected in the plasma membrane of lung cancer, ovarian cancer and renal cell cancer samples. The full-length OGTA091 was detected in the plasma membrane of breast colorectal cancer, hepatocellular carcinoma, lung cancer, ovarian cancer and renal cell cancer samples. The full-length OGTA098 was detected in the plasma membrane of colorectal cancer, lung cancer, ovarian cancer and renal cell cancer samples. The full-length OGTA101 was detected in the plasma membrane of colorectal cancer, hepatocellular carcinoma, lung cancer, ovarian cancer and renal cell cancer samples. The full-length OGTA104 was detected in the plasma membrane of colorectal cancer, hepatocellular carcinoma, lung cancer, ovarian cancer and renal cell cancer samples. The full-length OGTA106 was detected in the plasma membrane of hepatocellular carcinoma samples. The full-length OGTA112 was detected in the plasma membrane of renal cell cancer samples. The full-length OGTA113 was detected in the plasma membrane of colorectal cancer, lung cancer, ovarian cancer and renal cell cancer samples. The full-length OGTA119 was detected in the plasma membrane of colorectal cancer, lung cancer, and ovarian cancer samples. The full-length OGTA124 was detected in the plasma membrane of colorectal cancer, hepatocellular carcinoma, lung cancer, ovarian cancer and renal cell cancer samples. The full-length OGTA126 was detected in the plasma membrane of lung cancer samples. The full-length OGTA156 was detected in the plasma membrane of colorectal cancer, lung cancer, ovarian cancer and renal cell cancer samples. The full-length OGTA159 was detected in the plasma membrane of lung cancer and renal cell cancer samples. The full-length OGTA169 was detected in the plasma membrane of colorectal cancer, lung cancer and renal cell cancer samples. The full-length OGTA174 was detected in the plasma membrane of lung cancer samples. The full-length OGTA176 was detected in the plasma membrane of colorectal cancer, lung cancer and renal cell cancer samples. The full-length OGTA177 was detected in the plasma membrane of colorectal cancer, lung cancer, ovarian cancer and renal cell cancer samples. The full-length OGTA197 was detected in the plasma membrane of colorectal cancer, hepatocellular carcinoma, lung cancer and renal cell cancer samples. The full-length OGTA202 was detected in the plasma membrane of colorectal cancer and lung cancer samples. The full-length OGTA203 was detected in the plasma membrane of lung cancer and renal cell cancer samples. The full-length OGTA206 was detected in the plasma membrane of colorectal cancer and lung cancer samples. The full-length OGTA213 was detected in the plasma membrane of colorectal cancer and lung cancer samples. The full-length OGTA214 was detected in the plasma membrane of colorectal cancer samples. The full-length OGTA216 was detected in the plasma membrane of colorectal cancer, hepatocellular carcinoma, lung cancer, ovarian cancer and renal cell cancer samples. The full-length OGTA222 was detected in the plasma membrane of colorectal cancer, lung cancer and renal cell cancer samples. The full-length OGTA236 was detected in the plasma membrane of colorectal cancer samples. The full-length OGTA237 was detected in the plasma membrane of colorectal cancer, lung cancer and renal cell cancer samples. The full-length OGTA247 was detected in the plasma membrane of lung cancer samples. The full-length OGTA248 was detected in the plasma membrane of lung cancer samples. The full-length OGTA249 was detected in the plasma membrane of lung cancer and renal cell cancer samples. The full-length OGTA257 was detected in the plasma membrane of colorectal cancer, hepatocellular carcinoma, lung cancer and ovarian cancer samples. The full-length OGTA271 was detected in the plasma membrane of colorectal cancer, hepatocellular carcinoma, lung cancer and renal cell cancer samples.

**EXAMPLE 3:**

EVALUATION OF COLORECTAL CANCER MARKER PROTEINS IN SANDWICH ELISA

Using the following Reference Protocol, colorectal cancer marker proteins, including OGTA066 were evaluated in a sandwich ELISA.

3.1 MATERIALS AND METHODS

Antibodies for the sandwich ELISAs were developed at Biosite. Biotinylated antibody (primary antibody) was diluted into assay buffer (10 mM Tris, 150 mM NaCl, 1% BSA) to 2 ug/ml and added to 384 well neutravidin coated plate (Pierce Chemical Company, Rockford IL) and allowed to incubate at room temperature for 1 hour. Wells were then washed with wash buffer (20mM Borate, 150 mM NaCl, 0.2% Tween 20). Samples and standards were added and allowed to incubate at room temperature for 1 hour. Wells again were washed. An antibody conjugated to fluorscein (secondary antibody) was diluted into assay buffer to 2 ug/ml and was then added to the plate and allowed to incubate at room temperature for 1 hour. Wells again were washed. Anti-fluorscein antibody conjugated to alkaline phosphatase, diluted 1/2338 into assay buffer, was added and allowed to incubate at room temperature for 1 hour. Final wash was then performed. Finally substrate (Promega Attophos Product#S1011, Promega Corporation, Madison, WI) was added and the plate was read immediately. All additions were 10 ul/well. The plate was washed 3 times between each addition and final wash was 9 times prior to the addition of substrate. Standards were prepared by spiking specific antigen into a normal serum patient pool. Reading was performed using a Tecan Spectrafluor plus (Tecan Inc, Mannedorf, Switzerland) in kinetic mode for 6 read cycles with excitation filter of 430nm and an emission filter 570nm emission. Slope of RFU/seconds was determined.

Final Box and ROC results were analyzed using Analyse-it General + Clinical Laboratory 1.73 (Analyse-it Software Ltd., Leeds England).

3.2 RESULTS

These experiments identified proteins of particular interest including, but not limited to, OGTA066 (SEQ ID No: 9).

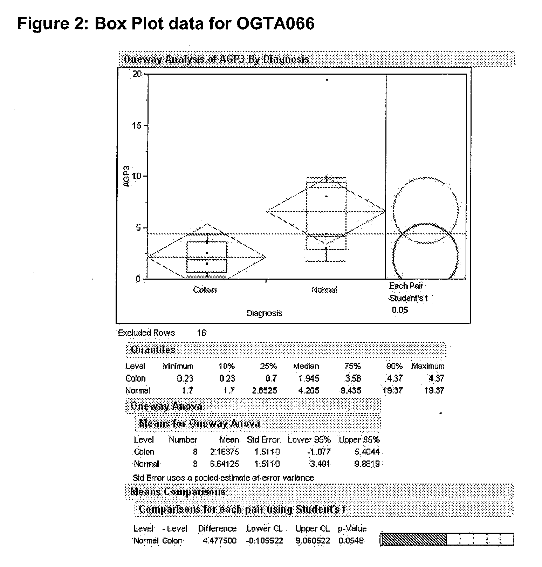

Figure 2 shows Box plot data for OGTA066. The vertical axis on this graph is concentration of OGTA066 in ng/ml. These data show decreased concentration of OGTA066 in colorectal cancer samples compared to normal samples, with an almost significant p value, thereby indicating that OGTA066 discriminates well between colorectal cancer and normal, making it a good potential marker for colorectal cancer.

**EXAMPLE 4:**

EVALUATION OF COLORECTAL CANCER MARKER PROTEINS IN MULTIPLEX ASSAY USING LUMINEX TECHNOLOGY

Using the following Reference Protocol, OGTA066 was evaluated in a multiplex assay using the Luminex technology.

4.1 MATERIALS AND METHODS

Each primary antibody was conjugated to a unique Luminex magnetic microsphere (Mug beads, Luminex Corporation, Austin, TX). Mag bead cocktail (50ul) was added to a 96 black well round bottom Costar plate (Corning Incorporated, Corning NY). Using a 96 well magnetic ring stand, the Mag beads were pulled down for 1 minute and washed with wash/assay buffer (PBS with 1% BSA and 0.02% Tween 20). 50ul of sample or standard was added along with an additional 50ul of wash/assay buffer and allowed to incubate on a shaker for 1 hour at room temperature. Plate was placed on magnetic ring stand and allowed to sit for 1 minute. Mag beads were then washed again. Biotin labeled antibody was then added at 50ul per well with an additional 50ul of wash/assay buffer and allowed to incubate on a shaker for 1 hour at room temperature. The plate again was placed on a magnetic stand and the Mag beads were washed. Streptavidin-RPE (Prozyme, San Leandro, CA, Phycolin, Code#PJ31S) was diluted to 1ug/ml in wash/assay buffer and 50ul was added to each well along with an additional 50ul of wash/assay buffer and allowed to incubate on a shaker for 1 hour at room temperature. Final wash was performed and the beads were re-suspended with 100 ul of wash/assay buffer and each well was then read in a Luminex 200 reader using Xponent software 3.0. All reagent dilutions were made in wash/assay buffer. Biotin-antibody varied for each assay to optimal concentration. Initial Mag bead amounts added were approximately 50,000 for each assay. Magnetic beads were allowed 1 minute pull down time prior to each wash. Each wash step was 3 times washed with 100ul of wash/assay buffer. Assay standard curves were made in a normal donor patient serum pool. Luminex reader and Mag beads were used and prepared according to manufacturer guidelines. Standard curves were calculated using a 5 parameter log-logistic fit and each sample concentration was determined from this curve fit.

Final Box and ROC results were analyzed using Analyse-it General + Clinical Laboratory 1.73 (Analyse-it Software Ltd., Leeds England).

### 4.2 RESULTS

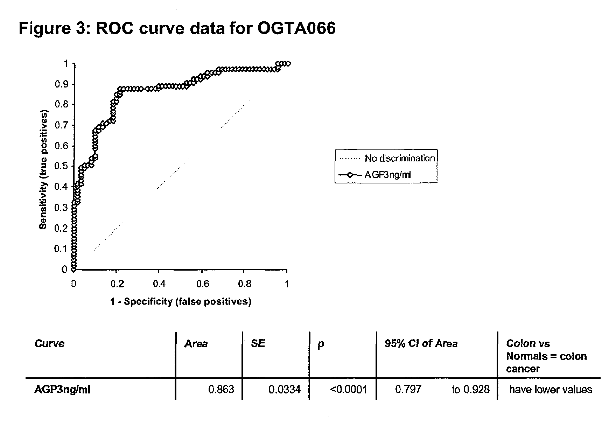

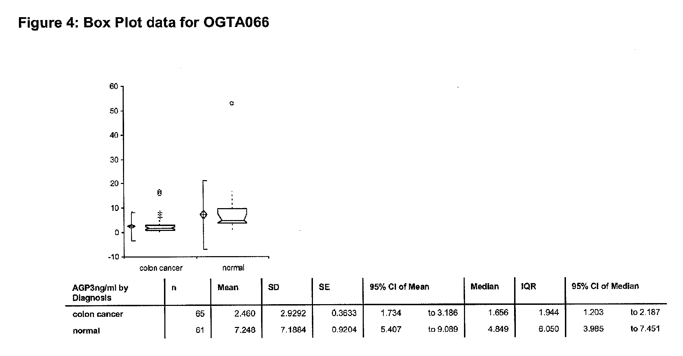

Experiments using 61 normal samples and 65 colorectal cancer samples resulted in further evidence for some of the proteins of interest identified in Example 3 above, including, but not limited to, OGTA066. Figure 4 shows Box plot data for OGTA066. Figure 3 shows ROC curve data for OGTA066.

The ROC curves plot sensitivity (true positives) against 1-specificity (false positives). The area under the ROC curve is a measure of the probability that the measured marker level will allow correct identification of a disease or condition. An area of greater than 0.5 indicates that the marker can discriminate between disease and normal. This is the case in the data shown in Figure 3, with OGTA066 having a high area under the curve and a very low p value therefore indicating that OGTA066 is a good potential marker to discriminate between colorectal cancer and normal.

The vertical axes on the box plot in Figure 4 is concentration of the OGTA066 in ng/ml. Figure 4 shows lower concentration in serum samples of OGTA066 in colorectal cancer samples than in normal samples. OGTA066 shows good discrimination between colorectal cancer and normal, indicating it may be useful as a marker for colorectal cancer.

## Claims

1. An isolated monoclonal antibody capable of immuno-specific binding to CUB domain-containing protein 1, or a fragment or derivative thereof which comprises the antigen binding domain, for use in treating lung cancer wherein the antibody or fragment or derivative thereof is conjugated to a cytotoxic moiety or a drug moiety.

2. The isolated monoclonal antibody for use according to claim 1 wherein the lung cancer is non-small cell lung cancer.

3. The isolated monoclonal antibody for use as claimed in claim 1 or claim 2, wherein:

   (A) said antibody is selected from the group consisting of a chimeric antibody, a human antibody, a humanised antibody, a single chain antibody, a bispecific antibody and a non-fucosylated antibody;
   (B) the fragment is a domain antibody.

## Patentansprüche

1. Isolierter monoklonaler Antikörper, der in der Lage ist, immunspezifisch an CUB-Domäne enthaltendes Protein 1 oder ein Fragment oder ein Derivat davon zu binden, umfassend die Antigenbindungsdomäne, zur Verwendung bei der Behandlung von Lungenkrebs, wobei der Antikörper oder das Fragment oder Derivat davon an eine zytotoxische Gruppe oder eine Arzneimittelgruppe konjugiert ist.

2. Isolierter monoklonaler Antikörper zur Verwendung nach Anspruch 1, wobei es sich bei dem Lungenkrebs um nicht-kleinzelligen Lungenkrebs handelt.

3. Isolierter monoklonaler Antikörper zur Verwendung nach Anspruch 1 oder Anspruch 2, wobei:

   (A) der Antikörper aus der Gruppe, bestehend aus einem chimären Antikörper, einem humanen Antikörper, einem humanisierten Antikörper, einem einkettigen Antikörper, einem bispezifischen Antikörper und einem nicht-fucosyliertem Antikörper, ausgewählt ist;
   (B) das Fragment ein Domänen-Antikörper ist.

## Revendications

1. Anticorps monoclonal isolé capable d'effectuer une liaison immuno-spécifique avec la protéine 1 contenant le domaine CUB, fragment ou dérivé dudit anticorps, qui comprend le domaine de liaison de l'antigène, pour utilisation dans le traitement de cancer du poumon, où l'anticorps, fragment ou dérivé de celui-ci est conjugué à un groupement cytotoxique ou un groupement médicamenteux.

2. Anticorps monoclonal isolé pour utilisation selon la revendication 1, où le cancer du poumon est un cancer du poumon non à petites cellules.

**3.** Anticorps monoclonal isolé pour utilisation selon la revendication 1 ou la revendication 2, où :

(A) ledit anticorps est choisi dans le groupe comprenant un anticorps chimérique, un anticorps humain, un anticorps humanisé, un anticorps monocaténaire, un anticorps bispécifique et un anticorps non fucosylé ;
(B) le fragment est un anticorps de domaine.

## FIGURE 1

### OGTA002 (SEQ ID No: 1)

**Peptide Source: 1D-GE, Breast cancer**

```
MELRVLLCWASLAAALEETLLNTKLETADLKWVTFPQVDGQWEELSGLDEEQHSVRTYEVCDVQRAPGQ
AHWLRTGWVPRRGAVHVYATLRFTMLECLSLPRAGRSCKETFTVFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGPFGQEHHSQTQLDESEGWREQLALIAGTAVVGVVLV
LVVIVVAVLCLRKQSNGREAEYSDKHGQYLIGHGTKVYIDPFTYEDPNEAVREFAKEIDVSYVKIEEVT
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIAFRKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSAFGSVGEWLRAIKMGRYEESFAAAGFGSFELVSQISAEDLLRIGVTLAGHQKKILASVQ
HMKSQAKPGTPGGTGGPAPQY
```

**Mass Match Peptides (bold):**

```
        LNGSSLHLEWSAPLESGGR [473]
        QPHYSAFGSVGEWLR [585]
        SQAKPGTPGGTGGPAPQY [646]
```

**Tandem Peptides (underline):**

```
        VDTVAAEHLTR [749]
```

**Peptide Source: 1D-GE, Colorectal cancer**

```
MELRVLLCWASLAAALEETLLNTKLETADLKWVTFPQVDGQWEELSGLDEEQHSVRTYEVCDVQRAPGQ
AHWLRTGWVPRRGAVHVYATLRFTMLECLSLPRAGRSCKETFTVFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGPFGQEHHSQTQLDESEGWREQLALIAGTAVVGVVLV
LVVIVVAVLCLRKQSNGREAEYSDKHGQYLIGHGTKVYIDPFTYEDPNEAVREFAKEIDVSYVKIEEVT
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIAFRKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSAFGSVGEWLRAIKMGRYEESFAAAGFGSFELVSQISAEDLLRIGVTLAGHQKKILASVQ
HMKSQAKPGTPGGTGGPAPQY
```

**Mass Match Peptides (bold):**

```
        DLVEPWVVVR [141]
        EFLSEASIMGQFEHPNIIR [184]
        EVPPAVSDIR [237]
        GAPCTTPPSAPR [286]
        ILASVQHMK [373]
        LETADLK [456]
```

519

```
VDTVAAEHLTR [749]
VYIDPFTYEDPNEAVR [818]
WTAPEAIAFR [833]
YLAEMSYVHR [859]
```

**Tandem Peptides (underline):**
```
DLVEPWVVVR [141]
FPQVVSALDK [263]
```

## Peptide Source: 1D-GE, Hepatocellular carcinoma

```
MELRVLLCWASLAAALEETLLNTKLETADLKWVTFPQVDGQWEELSGLDEEQHSVRTYEVCDVQRAPGQ
AHWLRTGWVPRRGAVHVYATLRFTMLECLSLPRAGRSCKETFFVFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGPFGQEHHSQTQLDESEGWREQLALIAGTAVVGVVLV
LVVIVVAVLCLRKQSNGREAEYSDKHGQYLIGHGTKVYIDPFTYEDPNEAVREFAKEIDVSYVKIEEVI
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIAFRKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSAFGSVGEWLRAIKMGRYEESFAAAGFGSFELVSQISAEDLLRIGVTLAGHQKKILASVQ
HMKSQAKPGTPGGTGGPAPQY
```

**Mass Match Peptides (bold):**

**Tandem Peptides (underline):**
```
VSDFGLSR [796]
```

## Peptide Source: 1D-GE, Melanoma

```
MELRVLLCWASLAAALEETLLNTKLETADLKWVTFPQVDGQWEELSGLDEEQHSVRTYEVCDVQRAPGQ
AHWLRTGWVPRRGAVHVYATLRFTMLECLSLPRAGRSCKETFFVFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGPFGQEHHSQTQLDESEGWREQLALIAGTAVVGVVLV
LVVIVVAVLCLRKQSNGREAEYSDKHGQYLIGHGTKVYIDPFTYEDPNEAVREFAKEIDVSYVKIEEVI
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIAFRKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSAFGSVGEWLRAIKMGRYEESFAAAGFGSFELVSQISAEDLLRIGVTLAGHQKKILASVQ
HMKSQAKPGTPGGTGGPAPQY
```

**Mass Match Peptides (bold):**
```
ENGGASHPLLDQR [214]
FLEENSSDPTYTSSLGGK [258]
FTMLECLSLPR [276]
```

520

```
IEEVIGAGEFGEVCR [360]
KESCVAIK [413]
LPPPPDCPTSLHQLMLDCWQK [479]
```

**Tandem Peptides (underline):**
```
VDTVAAEHLTR [749]
```


## Peptide Source: 1D-GE, Ovarian cancer

```
MELRVLLCWASLAAALEETLLNTKLETADLKWVTFPQVDGQWEELSGLDEEQHSVRTYEVCDVQRAPGQ
AHWLRTGWVPRRGAVHVYATLRFTMLECLSLPRAGRSCKETFTVFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGPFGQEHHSQTQLDESEGWREQLALIAGTAVVGVVLV
LVVIVVAVLCLRKQSNGREAEYSDKHGQYLIGHGTKVYIDPFTYEDPNEAVREFAKEIDVSYVKIEEVI
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIAFRKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSAFGSVGEWLRAIKMGRYEESFAAAGFGSFELVSQISAEDLLRIGVTLAGHQKKILASVQ
HMKSQAKPGTPGGTGGPAPQY
```

**Mass Match Peptides (bold):**
**Tandem Peptides (underline):**
```
VSDFGLSR [796]
```


## Peptide Source: 1D-GE, Pancreatic cancer

```
MELRVLLCWASLAAALEETLLNTKLETADLKWVTFPQVDGQWEELSGLDEEQHSVRTYEVCDVQRAPGQ
AHWLRTGWVPRRGAVHVYATLRFTMLECLSLPRAGRSCKETFTVFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGPFGQEHHSQTQLDESEGWREQLALIAGTAVVGVVLV
LVVIVVAVLCLRKQSNGREAEYSDKHGQYLIGHGTKVYIDPFTYEDPNEAVREFAKEIDVSYVKIEEVI
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIAFRKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSAFGSVGEWLRAIKMGRYEESFAAAGFGSFELVSQISAEDLLRIGVTLAGHQKKILASVQ
HMKSQAKPGTPGGTGGPAPQY
```

**Mass Match Peptides (bold):**
```
HGQYLIGHGTK [335]
TYEVCDVQR [739]
```
**Tandem Peptides (underline):**
```
FPQVVSALDK [263]
```

## Peptide Source: iTRAQ, Colorectal cancer

MELRVLLCWASLAAALEETLLNTKLETADLKWVTFPQVDGQWEELSGLDEEQHSVRTYEVCDVQRAPGQ
AHWLRTGWVPRRGAVHVYATLRFTMLECLSLPRAGRSCKETFTVFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGPFGQEHHSQTQLDESEGWREQLALIAGTAVVGVVLV
LVVIVVAVLCLRKQSNGREAEYSDKHGQYLIGHGTKVYIDPFTYEDPNEAVREFAKEIDVSYVKIEEVI
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIAFRKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSAFGSVGEWLRAIKMGRYEESFAAAGFGSFELVSQISAEDLLRIGVTLAGHQKKILASVQ
HMKSQAKPGTPGGTGGPAPQY

**Mass Match Peptides (bold):**
        AELRGLK [880]
        ILASVQHMK [373]
        TLRLGPLSK [983]
**Tandem Peptides (underline):**
        AELRGLK [880]
        ILASVQHMK [373]
        TLRLGPLSK [983]


## Peptide Source: iTRAQ, Hepatocellular cancer

MELRVLLCWASLAAALEETLLNTKLETADLKWVTFPQVDGQWEELSGLDEEQHSVRTYEVCDVQRAPGQ
AHWLRTGWVPRRGAVHVYATLRFTMLECLSLPRAGRSCKETFTVFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGPFGQEHHSQTQLDESEGWREQLALIAGTAVVGVVLV
LVVIVVAVLCLRKQSNGREAEYSDKHGQYLIGHGTKVYIDPFTYEDPNEAVREFAKEIDVSYVKIEEVI
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIAFRKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSAFGSVGEWLRAIKMGRYEESFAAAGFGSFELVSQISAEDLLRIGVTLAGHQKKILASVQ
IIMKSQAKPGTPGGTGGPAPQY

**Mass Match Peptides (bold):**
        EIDVSYVK [899]
**Tandem Peptides (underline):**
        EIDVSYVK [899]

## Peptide Source: iTRAQ, Non-small cell lung cancer

MELRVLLCWASLAAALEETLLNTKLETADLKWVT⁐PQVDGQWEELSGLDEEQHSVRTYEVCDVQRAPGQ
AHWLRTGWVPRRGAVHVYATLRFTMLECLSLPRAGRSCKETF⁐VFYYESDADTATALTPAWMENPYIKV
DTVAAEHLTRKRPGAEATGKVNVKTLRLGPLSKAGFYLAFQDQGACMALLSLHLFYK**KCAQLTVNLTR**F
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENR**AELRGLK**RGASYLVQVRARSEAGYGP⁐GQEHHSQ⁐QLDESEGWREQLALIAGTAVVGVVLV
LVVIVVAVLCLRKQSNGREAEYSDKHGQYLIGHGTKVYIDPF⁐YEDPNEAVREFAKEIDVSYVKIEEVI
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIA⁐RKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQR**QPHYSAFGSVGEWLRAIK**MGRYEESFAAAGFGSFELVSQISAEDLLRIGVTLAGHQKKILASVQ
HMKSQAKPGTPGGTGGPAPQY

### Mass Match Peptides (bold):
        AELRGLK [880]
        KCAQLTVNLTR [927]
        QPHYSAFGSVGEWLRAIK [964]

### Tandem Peptides (underline):
        AELRGLK [880]
        KCAQLTVNLTR [927]
        QPHYSAFGSVGEWLRAIK [964]

## Peptide Source: iTRAQ, Renal cell cancer

MELRVLLCWASLAAALEETLLNTKLETADLKWVT⁐PQVDGQWEELSGLDEEQHSVRTYEVCDVQRAPGQ
AHWLRTGWVPRRGAVHVYATLRFTMLECLSLPRAGRSCKETF⁐VFYYESDADTATALTPAWMENPYIKV
DTVAAEHLTRKRPGAEATGKVNVKTLRLGPLSKAGFYLAFQDQGACMALLSLHLFYKKCAQLTVNLTRF
PETVPRELVVPVAGSCVVDAVPAPGPSPSLYCREDGQWAEQPVTGCSCAPGFEAAEGNTKCRACAQGTF
KPLSGEGSCQPCPANSHSNTIGSAVCQCRVGYFRARTDPRGAPCTTPPSAPRSVVSRLNGSSLHLEWSA
PLESGGREDLTYALRCRECRPGGSCAPCGGDLTFDPGPRDLVEPWVVVRGLRPDFTYTFEVTALNGVSS
LATGPVPFEPVNVTTDREVPPAVSDIRVTRSSPSSLSLAWAVPRAPSGAVLDYEVKYHEKGAEGPSSVR
FLKTSENRAELRGLKRGASYLVQVRARSEAGYGP⁐GQEIIIISQ⁐QLDESEGWREQLALIAGTAVVGVVLV
LVVIVVAVLCLRKQSNGREAEYSDKHGQYLIGHGTKVYIDPF⁐YEDPNEAVREFAKEIDVSYVKIEEVI
GAGEFGEVCRGRLKAPGKKESCVAIKTLKGGYTERQRREFLSEASIMGQFEHPNIIRLEGVVTNSMPVM
ILTEFMENGALDSFLRLNDGQFTVIQLVGMLRGIASGMRYLAEMSYVHRDLAARNILVNSNLVCKVSDF
GLSRFLEENSSDPTYTSSLGGKIPIRWTAPEAIA⁐RKFTSASDAWSYGIVMWEVMSFGERPYWDMSNQD
VINAIEQDYRLPPPPDCPTSLHQLMLDCWQKDRNARPRFPQVVSALDKMIRNPASLKIVARENGGASHP
LLDQRQPHYSA⁐GSVGEWLRAIKMGRYEESFAAAGFGSFELVSQISAEDLLRIGVTLAGHQKK**ILASVQ**
**HMK**SQAKPGTPGGTGGPAPQY

### Mass Match Peptides (bold):
        ILASVQHMK [373]

### Tandem Peptides (underline):
        ILASVQHMK [373]

## OGTA009 (SEQ ID No: 2)

## Peptide Source: 1D-GE, Breast cancer

MSMGLEITGTALAVLGWLGTIVCCALPMWRVSAFIGSNIITSQNIWEGLWMNCVVQSTGQMQCK**VYDSL
LALPQDLQAAR**ALIVVAILLAAFGLLVALVGAQCTNCVQDDTAKAKITIVAGVLFLLAALLTLVPVSWS
ANTIIRD**FYNPVVPEAQKR**EMGAGLYVGWAAAALQLLGGALLCCSCPPREKKYTATKVVYSAPR**STGPG
ASLGTGYDR**KDYV

### Mass Match Peptides (bold):
DFYNPVVPEAQKR [120]
STGPGASLGTGYDR [657]
STGPGASLGTGYDRK [658]

### Tandem Peptides (underline):
DFYNPVVPEAQK [119]
VYDSLLALPQDLQAAR [817]


## Peptide Source: 1D-GE, Colorectal cancer

MSMGLEITGTALAVLGWLGTIVCCALPMWRVSAFIGSNIITSQNIWEGLWMNCVVQSTGQMQCKVYDSL
LALPQDLQAARALIVVAILLAAFGLLVALVGAQCTNCVQDDTAKAKITIVAGVLFLLAALLTLVPVSWS
ANTIIRD**FYNPVVPEAQKR**EMGAGLYVGWAAAALQLLGGALLCCSCPPREKKYTATKVVYSAPR**STGPG
ASLGTGYDR**KDYV

### Mass Match Peptides (bold):
DFYNPVVPEAQK [119]
DFYNPVVPEAQKR [120]
STGPGASLGTGYDR [657]

### Tandem Peptides (underline):
STGPGASLGTGYDR [657]


## Peptide Source: 1D-GE, Pancreatic cancer

MSMGLEITGTALAVLGWLGTIVCCALPMWRVSAFIGSNIITSQNIWEGLWMNCVVQSTGQMQCK**VYDSL
LALPQDLQAAR**ALIVVAILLAAFGLLVALVGAQCTNCVQDDTAKAKITIVAGVLFLLAALLTLVPVSWS
ANTIIRDFYNPVVPEAQKREMGAGLYVGWAAAALQLLGGALLCCSCPPREKKYTATKVVYSAPR**STGPG
ASLGTGYDR**KDYV

### Mass Match Peptides (bold):
STGPGASLGTGYDR [657]
VYDSLLALPQDLQAAR [817]

### Tandem Peptides (underline):
STGPGASLGTGYDR [657]
VYDSLLALPQDLQAAR [817]


## Peptide Source: 1D-GE, Prostate cancer

MSMGLEITGTALAVLGWLGTIVCCALPMWRVSAFIGSNIITSQNIWEGLWMNCVVQSTGQMQCK**VYDSL
LALPQDLQAAR**ALIVVAILLAAFGLLVALVGAQCTNCVQDDTAKAKITIVAGVLFLLAALLTLVPVSWS

ANTIIRDFYNPVVPEAQKREMGAGLYVGWAAAALQLLGGALLCCSCPPREKKYTATKVVYSAPRSTGPG
ASLGTGYDRKDYV


**Mass Match Peptides (bold):**

    DFYNPVVPEAQK [119]
    STGPGASLGTGYDR [657]
    VVYSAPR [815]
    VYDSLLALPQDLQAAR [817]

**Tandem Peptides (underline):**

    DFYNPVVPEAQK [119]


## Peptide Source: 1D-GE, Renal cell cancer

MSMGLEITGTALAVLGWLGTIVCCALPMWRVSAFIGSNIITSQNIWEGLWMNCVVQSTGQMQCKVYDSL
LALPQDLQAARALIVVAILLAAFGLLVALVGAQCTNCVQDDTAKAKITIVAGVLFLLAALLTLVPVSWS
ANTIIRDFYNPVVPEAQKREMGAGLYVGWAAAALQLLGGALLCCSCPPREKKYTATKVVYSAPRSTGPG
ASLGTGYDRKDYV


**Mass Match Peptides (bold):**

**Tandem Peptides (underline):**

    STGPGASLGTGYDR [657]


## Peptide Source: iTRAQ, Colorectal cancer

MSMGLEITGTALAVLGWLGTIVCCALPMWRVSAFIGSNIITSQNIWEGLWMNCVVQSTGQMQCKVYDSL
LALPQDLQAARALIVVAILLAAFGLLVALVGAQCTNCVQDDTAKAKITIVAGVLFLLAALLTLVPVSWS
ANTIIRDFYNPVVPEAQKREMGAGLYVGWAAAALQLLGGALLCCSCPPREKKYTATKVVYSAPRSTGPG
ASLGTGYDRKDYV


**Mass Match Peptides (bold):**

    DFYNPVVPEAQK [119]
    STGPGASLGTGYDR [657]
    VYDSLLALPQDLQAAR [817]

**Tandem Peptides (underline):**

    DFYNPVVPEAQK [119]
    STGPGASLGTGYDR [657]
    VYDSLLALPQDLQAAR [817]


## Peptide Source: iTRAQ, Non-small cell lung cancer

MSMGLEITGTALAVLGWLGTIVCCALPMWRVSAFIGSNIITSQNIWEGLWMNCVVQSTGQMQCKVYDSL
LALPQDLQAARALIVVAILLAAFGLLVALVGAQCTNCVQDDTAKAKITIVAGVLFLLAALLTLVPVSWS
ANTIIRDFYNPVVPEAQKREMGAGLYVGWAAAALQLLGGALLCCSCPPREKKYTATKVVYSAPRSTGPG
ASLGTGYDRKDYV


**Mass Match Peptides (bold):**

    VYDSLLALPQDLQAAR [817]

**Tandem Peptides (underline):**

VYDSLLALPQDLQAAR [817]


## Peptide Source: iTRAQ, Ovarian cancer

MSMGLEITGTALAVLGWLGTIVCCALPMWRVSAFIGSNIITSQNIWEGLWMNCVVQSTGQMQCKVYDSL
LALPQDLQAARALIVVAILLAAFGLLVALVGAQCTNCVQDDTAKAKITIVAGVLFLLAALLTLVPVSWS
ANTIIRDFYNPVVPEAQKREMGAGLYVGWAAAALQLLGGALLCCSCPPREKKYTATKVVYSAPRSTGPG
ASLGTGYDRKDYV


**Mass Match Peptides (bold):**
DFYNPVVPEAQK [119]
**Tandem Peptides (underline):**
DFYNPVVPEAQK [119]


## OGTA016 (SEQ ID No: 3)

## Peptide Source: 1D-GE, Colorectal cancer

MLLFVLTCLLAVFPAISTKSPIFGPEEVNSVEGNSVSITCYYPPTSVNRHTRKYWCRQGARGGCITLIS
SEGYVSSKYAGRANLTNFPENGTFVVNIAQLSQDDSGRYKCGLGINSRGLSFDVSLEVSQGPGLLNDTK
VYTVDLGRTVTINCPFKTENAQKRKSLYKQIGLYPVLVIDSSGYVNPNYTGRIRLDIQGTGQLLFSVVI
NQLRLSDAGQYLCQAGDDSNSNKKNADLQVLKPEPELVYEDLRGSVTFHCALGPEVANVAKFLCRQSSG
ENCDVVVNTLGKRAPAFEGRILLNPQDKDGSFSVVITGLRKEDAGRYLCGAHSDGQLQEGSPIQAWQLF
VNEESTIPRSPTVVKGVAGSSVAVLCPYNRKESKSIKYWCLWEGAQNGRCPLLVDSEGWVKAQYEGRLS
LLEEPGNGTFTVILNQLTSRDAGFYWCLTNGDTLWRTTVEIKIIEGEPNLKVPGNVTAVLGETLKVPCH
FPCKFSSYEKYWCKWNNTGCQALPSQDEGPSKAFVNCDENSRLVSLTLNLVTRADEGWYWCGVKQGHFY
GETAAVYVAVEERKAAGSRDVSLAKADAAPDEKVLDSGFREIENKAIQDPRLFAEEKAVADTRDQADGS
RASVDSGSSEEQGGSSRALVSTLVPLGLVLAVGAVAVGVARARHRKNVDRVSIRSYRTDISMSDFENSR
EFGANDNMGASSITQETSLGGKEEFVATTESTTETKEPKKAKRSSKEEAEMAYKDFLLQSSTVAAEAQD
GPQEA


**Mass Match Peptides (bold):**
ADAAPDEK [57]
AFVNCDENSR [68]
ANLTNFPENGTFVVNIAQLSQDDSGR [79]
AQYEGR [85]
ASVDSGSSEEQGGSSR [86]
DFLLQSSTVAAEAQDGPQEA [116]
DGSFSVVITGLR [125]
DQADGSR [150]
EEFVATTESTTETK [171]
FSSYEK [274]
GGCITLISSEGYVSSK [297]
GSVTFHCALGPEVANVAK [323]
IIEGEPNLK [371]
ILLNPQDK [377]
KYWCR [443]
QGHFYGETAAVYVAVEER [574]
QGHFYGETAAVYVAVEERK [575]
QSSGENCDVVVNTLGK [588]
QSSGENCDVVVNTLGKR [589]
RAPAFEGR [597]

```
TDISMSDFENSR [684]
VLDSGFR [776]
VPCHFPCK [788]
VYTVDLGR [821]
```

## Tandem Peptides (underline):

```
ASVDSGSSEEQGGSSR [86]
DGSFSVVITGLR [125]
EEFVATTESTTETK [171]
ILLNPQDK [377]
LSLLEEPGNGTFTVILNQLTSR [486]
TDISMSDFENSR [684]
VYTVDLGR [821]
YLCGAHSDGQLQEGSPIQAWQLFVNEESTIPR [861]
```

## Peptide Source: iTRAQ, Colorectal cancer

MLLFVLTCLLAVFPAISTKSPIFGPEEVNSVEGNSVSITCYYPPTSVNRHTRKYWCRQGARGGCITLIS
SEGYVSSKYAGRANLTNFPENGTFVVNIAQLSQDDSGRYKCGLGINSRGLSFDVSLEVSQGPGLLNDTK
**VYTVDLGR**TVTINCPFKTENAQKRKSLYKQIGLYPVLVIDSSGYVNPNYTGRIRLDIQGTGQLLFSVVI
NQLRLSDAGQYLCQAGDDSNSNKK**NADLQVLKPEPELVYEDLR**GSVTFHCALGPEVANVAKFLCR**QSSG
ENCDVVVNTLGK**RAPAFEGR**ILLNPQDK**DGSFSVVITGLRKEDAGRYLCGAHSDGQLQEGSPIQAWQLF
VNEESTIPRSPTVVKGVAGSSVAVLCPYNRKESKSIKYWCLWEGAQNGRCPLLVDSEGWVKAQYEGRLS
LLEEPGNGTFTVILNQLTSRDAGFYWCLTNGDTLWRTTVEIK**IIEGEPNLK**VPGNVTAVLGETLKVPCH
FPCKFSSYEKYWCKWNNTGCQALPSQDEGPSK**AFVNCDENSR**LVSLTLNLVTRADEGWYWCGVKQGHFY
GETAAVYVAVEERK**AAGSRDVSLAKADAAPDEK**VLDSGFREIENKAIQDPRLFAEEKAVADTRDQADGS
R**ASVDSGSSEEQGGSSR**ALVSTLVPLGLVLAVGAVAVGVARARHRKNVDRVSIRSYRTDISMSDFENSR
EFGANDNMGASSITQETSLGGKEEFVATTESTTETKEPKKAKRSSKEEAEMAYKDFLLQSSTVAAEAQD
GPQEA

## Mass Match Peptides (bold):

```
AAGSRDVSLAKADAAPDEK [877]
ADAAPDEK [57]
AFVNCDENSR [68]
ASVDSGSSEEQGGSSR [86]
IIEGEPNLK [371]
ILLNPQDK [377]
LSDAGQYLCQAGDDSNSNKK [947]
NADLQVLKPEPELVYEDLR [955]
QSSGENCDVVVNTLGK [588]
VYTVDLGR [821]
```

## Tandem Peptides (underline):

```
AAGSRDVSLAKADAAPDEK [877]
ADAAPDEK [57]
AFVNCDENSR [68]
ASVDSGSSEEQGGSSR [86]
IIEGEPNLK [371]
ILLNPQDK [377]
LSDAGQYLCQAGDDSNSNKK [947]
NADLQVLKPEPELVYEDLR [955]
QSSGENCDVVVNTLGK [588]
VYTVDLGR [821]
```

## Peptide Source: iTRAQ, Non-small cell lung cancer

MLLFVLTCLLAVFPAISTKSPIFGPEEVNSVEGNSVSITCYYPPTSVNRHTRKYWCRQGARGGCITLIS
SEGYVSSKYAGRANLTNFPENGTFVVNIAQLSQDDSGRYKCGLGINSRGLSFDVSLEVSQGPGLLNDTK
VYTVDLGR**TVTINCPFK**TENAQKRKSLYKQIGLYPVLVIDSSGYVNPNYTGRIRLDIQGTGQLLFSVVI
NQLRLSDAGQYLCQAGDDSNSNKKNADLQVLKPEPELVYEDLRGSVTFHCALGPEVANVAKFLCRQSSG
ENCDVVVNTLGKRAPAFEGR_ILLNPQDK_DGSFSVVITGLRKEDAGRYLCGAHSDGQLQEGSPIQAWQLF
VNEESTIPRSPTVVKGVAGSSVAVLCPYNRK**ESKSIK**YWCLWEGAQNGRCPLLVDSEGWVKAQYEGRLS
LLEEPGNGTFTVILNQLTSRDAGFYWCLTNGDTLWRTTVEIK_IIEGEPNLK_VPGNVTAVLGETLKVPCH
FPCKFSSYEKYWCKWNNTGCQALPSQDEGPSKAFVNCDENSRLVSLTLNLVTRADEGWYWCGVKQGHFY
GETAAVYVAVEERKAAGSRDVSLAK**ADAAPDEK**VLDSGFREIENKAIQDPRLFAEEKAVADTRDQADGS
R**ASVDSGSSEEQGGSSR**ALVSTLVPLGLVLAVGAVAVGVARARHRKNVDRVSIRSYRTDISMSDFENSR
EFGANDNMGASSITQETSLGGKEEFVATTESTTETKEPKKAKRSSKEEAEMAYKDFLLQSSTVAAEAQD
GPQEA

### Mass Match Peptides (bold):
ADAAPDEK [57]
ASVDSGSSEEQGGSSR [86]·
ESKSIK [907]
IIEGEPNLK [371]
ILLNPQDK [377]
TVTINCPFK [987]

### Tandem Peptides (underline):
ADAAPDEK [57]
ASVDSGSSEEQGGSSR [86]
ESKSIK [907]
IIEGEPNLK [371]
ILLNPQDK [377]
TVTINCPFK [987]

## OGTA028 (SEQ ID No: 4)

### Peptide Source: 1D-GE, Ovarian cancer

MSDVSTSVQSKFARLAKKKENITYMKREQLTETDKDIAPVLDLKCKDVSAIMNKFKVLMEIQDLMFEEM
RETLKNDLKAVLGGKATIPEVKNSENSSSRTEFQQIINLALQKTGMVGKIEGENSKIGDDNENLTFKLE
VNELSGKLDNTNEYNSNDGKKLPQGESRSYEVMGSMEETLCNIDDRDGNRNVHLEFTERESRKDGEDEF
VKEMREERKFQKLKNKEEVLKASREEKVLMDEGAVLTLVADLSSATLDISKQWSNVFNILRENDFEPKF
LCEVKLAFKCDGEIKTFSDLQSLRKFASQKSSVKELLKDVLPQKEEINQGGRKYGIQEKRDKTLIDSKH
RAGEITSDGLSTLFLKEVKVAKPEEMKNLETQEEEFSELEELDEEASGMEDDEDTSGLEEEEEEPSGLE
EEEEEEASGLEEDEASGLEEEEEQTSEQDSTFQGHTLVDAKHEVEITSDGMETTFIDSVEDSESEEEEE
GKSSETGKVKTTSLTEKKASRRQKEIPFSYLVGDSGKKKLVKHQVVHKTQEEEETAVPTSQGTGTPCLT
LCLASPSKSLEMSHDEHKKHSHTNLSTSTGVTKLKKTEEKKHRTLHTEELTSKEADLTEETEENLRSSV
INSIREIKEEIGNLKSSHSGVLEIENSVDDLSSRMDILEERIDSLEDQIEEFSKDTMQMTKQIISKERQ
RDIEERSRSCNIRLIGIPEKESYENRAEDIIKEIIDENFAELKKGSSLEIVSACRVPSKIDEKRLTPRH
ILVKFWNSSDKEKIIRASRERREITYQGTRIRLTADLSLDTLDARSKWSNVFKVLLEKGFNPRILYPAK
MAFDFRGKTKVFLSIEEFRDYVLHMPTLRELLGNNIP

### Mass Match Peptides (bold):
DVSAIMNK [162]
DYVLHMPTLR [165]
EEIGNLK [175]
EEINQGGR [177]
EIIDENFAELK [197]
EITYQGTR [200]

```
ELLGNNIP [203]
FLCEVK [256]
HQVVHK [343]
HSHTNLSISTGVTK [344]
IDSLEDQIEEFSK [358]
IGDDNENLTFK [366]
KENITYMK [411]
KENITYMKR [412]
KKENITYMK [421]
KLPQGESR [425]
KTEEKK [437]
LPQGESR [480]
MAFDFR [507]
MDILEER [510]
MSDVSTSVQSK [525]
QWSNVFNILR [592]
REITYQGTR [601]
REQLTETDK [602]
SLEMSHDEHKK [639]
SSHSGVLEIENSVDDLSSR [649]
TEFQQIINLALQK [687]
TFSDLQSLR [690]
TFSDLQSLRK [691]
TLHTEELTSK [704]
VAKPEEMK [743]
VFLSIEEFR [761]
VLMDEGAVLTLVADLSSATLDISK [782]
VLMEIQDLMFEEMR [783]
WSNVFK [832]
YGIQEK [852]
```

**Tandem Peptides (underline):**

```
AGEITSDGLSFLFLK [70]
EADLTEETEENLR [166]
LTADLSLDTLDAR [494]
NVHLEFTER [557]
SSVINSIR [652]
TEFQQIINLALQK [687]
TFSDLQSLR [690]
VLMEIQDLMFEEMR [783]
```

## Peptide Source: iTRAQ, Colorectal cancer

```
MSDVSTSVQSKFARLAKKKENITYMKREQLTETDKDIAPVLDLKCKDVSAIMNKFKVLMEIQDLMFEEM
RETLKNDLKAVLGGKATIPEVKNSENSSSRTEFQQIINLALQKTGMVGKIEGENSKIGDDNENLTFKLE
VNELSGKLDNTNEYNSNDGKKLPQGESRSYEVMGSMEETLCNIDDRDGNRNVHLEFTERESRKDGEDEF
VKEMREERKFQKLKNKEEVLKASREEKVLMDEGAVLTLVADLSSATLDISKQWSNVFNILRENDFEPKF
LCEVKLAFKCDGEIKTFSDLQSLRKFASQKSSVKELLKDVLPQKEEINQGGRKYGIQEKRDKTLIDSKH
RAGEITSDGLSFLFLKEVKVAKPEEMKNLETQEEEFSELEELDEEASGMEDDEDTSGLEEEEEEPSGLE
EEEEEEASGLEEDEASGLEEEEEQTSEQDSTFQGHTLVDAKHEVEITSDGMETTFIDSVEDSESEEEEE
GKSSETGKVKTTSLTEKKASRRQKEIPFSYLVGDSGKKKLVKHQVVHKTQEEEETAVPTSQGTGTPCLT
LCLASPSKSLEMSHDEHKKHSHTNLSISTGVTKLKKTEEKKHRTLHTEELTSKEADLTEETEENLRSSV
INSIREIKEEIGNLKSSHSGVLEIENSVDDLSSRMDILEERIDSLEDQIEEFSKDTMQMTKQIISKERQ
RDIEERSRSCNIRLIGIPEKESYENRAEDIIKEIIDENFAELKKGSSLEIVSACRVPSKIDEKRLTPRH
ILVKFWNSSDKEKIIRASRERREITYQGTRIRLTADLSLDTLDARSKWSNVFKVLLEKGFNPRILYPAK
MAFDFRGKTKVFLSIEEFRDYVLHMPTLRELLGNNIP
```

**Mass Match Peptides (bold):**

```
AEDIIK [879]
DIAPVLDLK [890]
EEINQGGR [177]
IDEKR [919]
SSVKELLK [973]
```

**Tandem Peptides (underline):**

```
AEDIIK [879]
DIAPVLDLK [890]
EEINQGGR [177]
IDEKR [919]
SSVKELLK [973]
```

## Peptide Source: iTRAQ, Hepatocellular cancer

```
MSDVSTSVQSKFARLAKKKENITYMKREQLTETDKDIAPVLDLKCKDVSAIMNKFKVLMEIQDLMFEEM
RETLKNDLKAVLGGKATIPEVKNSENSSSRTEFQQIINLALQKTGMVGKIEGENSKIGDDNENLTFKLE
VNELSGKLDNTNEYNSNDGKKLPQGESRSYEVMGSMEETLCNIDDRDGNRNVHLEFTERESRKDGEDEF
VKEMREERKFQKLKNKEEVLKASREEKVLMDEGAVLTLVADLSSATLDISKQWSNVFNILRENDFEPKF
LCEVKLAFKCDGEIKTFSDLQSLRKFASQKSSVKELLKDVLPQKEEINQGGRKYGIQEKRDKTLIDSKH
RAGEITSDGLSFLFLKEVKVAKPEEMKNLETQEEEFSELEELDEEASGMEDDEDTSGLEEEEEEPSGLE
EEEEEEASGLEEDEASGLEEEEEQTSEQDSTFQGHTLVDAKHEVEITSDGMETTFIDSVEDSESEEEEE
GKSSETGKVKTTSLTEKKASRRQKEIPFSYLVGDSGKKKLVKHQVVHKTQEEEETAVPTSQGTGTPCLT
LCLASPSKSLEMSHDEHKKHSHTNLSISTGVTKLKKTEEKKHRTLHTEELTSKEADLTEETEENLRSSV
INSIREIKEEIGNLKSSHSGVLEIENSVDDLSSRMDILEERIDSLEDQIEEFSKDTMQMTKQIISKERQ
RDIEERSRSCNIRLIGIPEKESYENRAEDIIKEIIDENFAELKKGSSLEIVSACRVPSKIDEKRLIPRH
ILVKFWNSSDKEKIIRASRERREITYQGTRIRLTADLSLDTILDARSKWSNVFKVLLEKGFNPRILYPAK
MAFDFRGKTKVFLSIEEFRDYVLHMPTLRELLGNNIP
```

**Mass Match Peptides (bold):**

```
DIAPVLDLK [890]
VKTTSLTEKK [992]
WSNVFK [832]
```

**Tandem Peptides (underline):**

```
DIAPVLDLK [890]
VKTTSLTEKK [992]
WSNVFK [832]
```

## Peptide Source: iTRAQ, Non-small cell lung cancer

```
MSDVSTSVQSKFARLAKKKENITYMKREQLTETDKDIAPVLDLKCKDVSAIMNKFKVLMEIQDLMFEEM
RETLKNDLKAVLGGKATIPEVKNSENSSSRTEFQQIINLALQKTGMVGKIEGENSKIGDDNENLTFKLE
VNELSGKLDNTNEYNSNDGKKLPQGESRSYEVMGSMEETLCNIDDRDGNRNVHLEFTERESRKDGEDEF
VKEMREERKFQKLKNKEEVLKASREEKVLMDEGAVLTLVADLSSATLDISKQWSNVFNILRENDFEPKF
LCEVKLAFKCDGEIKTFSDLQSLRKFASQKSSVKELLKDVLPQKEEINQGGRKYGIQEKRDKTLIDSKH
RAGEITSDGLSFLFLKEVKVAKPEEMKNLETQEEEFSELEELDEEASGMEDDEDTSGLEEEEEEPSGLE
EEEEEEASGLEEDEASGLEEEEEQTSEQDSTFQGHTLVDAKHEVEITSDGMETTFIDSVEDSESEEEEE
GKSSETGKVKTTSLTEKKASRRQKEIPFSYLVGDSGKKKLVKHQVVHKTQEEEETAVPTSQGTGTPCLT
LCLASPSKSLEMSHDEHKKHSHTNLSISTGVTKLKKTEEKKHRTLHTEELTSKEADLTEETEENLRSSV
INSIREIKEEIGNLKSSHSGVLEIENSVDDLSSRMDILEERIDSLEDQIEEFSKDTMQMTKQIISKERQ
RDIEERSRSCNIRLIGIPEKESYENRAEDIIKEIIDENFAELKKGSSLEIVSACRVPSKIDEKRLIPRH
```

```
ILVKFWNSSDKEKIIRASRERREITYQGTRIRLTADLSLDTILDARSKWSNVFKVLLEKGFNPRILYPAK
MAFDFRGKTKVFLSIEEFRDYVLHMPTLRELLGNNIP
```

**Mass Match Peptides (bold):**

```
        DIAPVLDLK [890]
        DVLPQK [897]
        EEINQGGR [177]
        LKNKEEVLK [933]
        VLLEK [995]
```

**Tandem Peptides (underline):**

```
        DIAPVLDLK [890]
        DVLPQK [897]
        EEINQGGR [177]
        LKNKEEVLK [933]
        VLLEK [995]
```

## Peptide Source: iTRAQ, Ovarian cancer

```
MSDVSTSVQSKFARLAKKKENITYMKREQLTETDKDIAPVLDLKCKDVSAIMNKFKVLMEIQDLMFEEM
RETLKNDLKAVLGGKATIPEVKNSENSSSRTEFQQIINLALQKTGMVGKIEGENSKIGDDNENLTFKLE
VNELSGKLDNTNEYNSNDGKKLPQGESRSYEVMGSMEETLCNIDDRDGNRNVHLEFTERESRKDGEDEF
VKEMREERKFQKLKNKEEVLKASREEKVLMDEGAVLTLVADLSSATLDISKQWSNVFNILRENDFEPKF
LCEVKLAFKCDGEIKTFSDLQSLRKFASQKSSVKELLKDVLPQKEEINQGGRKYGIQEKRDKTLIDSKH
RAGEITSDGLSFLFLKEVKVAKPEEMKNLETQEEEFSELEELDEEASGMEDDEDTSGLEEEEEEPSGLE
EEEEEEASGLEEDEASGLEEEEEQTSEQDSTFQGHTLVDAKHEVEITSDGMETTFIDSVEDSESEEEEE
GKSSETGKVKTTSLTEKKASRRQKEIPFSYLVGDSGKKKLVKHQVVHKTQEEEETAVPTSQGTGTPCLT
LCLASPSKSLEMSHDEHKKHSHTNLSISTGVTKLKKTEEKKHRTLHTEELTSKEADLTEETEENLRSSV
INSIREIKEEIGNLKSSHSGVLEIENSVDDLSSRMDILEERIDSLEDQIEEFSKDTMQMTKQIISKERQ
RDIEERSRSCNIRLIGIPEKESYENRAEDIIKEIIDENFAELKKGSSLEIVSACRVPSKIDEKRLTPRH
ILVKFWNSSDKEKIIRASRERREITYQGTRIRLTADLSLDTILDARSKWSNVFKVLLEKGFNPRILYPAK
MAFDFRGKTKVFLSIEEFRDYVLHMPTLRELLGNNIP
```

**Mass Match Peptides (bold):**

```
        DIAPVLDLK [890]
```

**Tandem Peptides (underline):**

```
        DIAPVLDLK [890]
```

## Peptide Source: iTRAQ, Renal cell cancer

```
MSDVSTSVQSKFARLAKKKENITYMKREQLTETDKDIAPVLDLKCKDVSAIMNKFKVLMEIQDLMFEEM
RETLKNDLKAVLGGKATIPEVKNSENSSSRTEFQQIINLALQKTGMVGKIEGENSKIGDDNENLTFKLE
VNELSGKLDNTNEYNSNDGKKLPQGESRSYEVMGSMEETLCNIDDRDGNRNVHLEFTERESRKDGEDEF
VKEMREERKFQKLKNKEEVLKASREEKVLMDEGAVLTLVADLSSATLDISKQWSNVFNILRENDFEPKF
LCEVKLAFKCDGEIKTFSDLQSLRKFASQKSSVKELLKDVLPQKEEINQGGRKYGIQEKRDKTLIDSKH
RAGEITSDGLSFLFLKEVKVAKPEEMKNLETQEEEFSELEELDEEASGMEDDEDTSGLEEEEEEPSGLE
EEEEEEASGLEEDEASGLEEEEEQTSEQDSTFQGHTLVDAKHEVEITSDGMETTFIDSVEDSESEEEEE
GKSSETGKVKTTSLTEKKASRRQKEIPFSYLVGDSGKKKLVKHQVVHKTQEEEETAVPTSQGTGTPCLT
LCLASPSKSLEMSHDEHKKHSHTNLSISTGVTKLKKTEEKKHRTLHTEELTSKEADLTEETEENLRSSV
INSIREIKEEIGNLKSSHSGVLEIENSVDDLSSRMDILEERIDSLEDQIEEFSKDTMQMTKQIISKERQ
RDIEERSRSCNIRLIGIPEKESYENRAEDIIKEIIDENFAELKKGSSLEIVSACRVPSKIDEKRLTPRH
ILVKFWNSSDKEKIIRASRERREITYQGTRIRLTADLSLDTILDARSKWSNVFKVLLEKGFNPRILYPAK
MAFDFRGKTKVFLSIEEFRDYVLHMPTLRELLGNNIP
```

**Mass Match Peptides (bold):**
    DIAPVLDLK [890]
    EEINQGGR [177]
**Tandem Peptides (underline):**
    DIAPVLDLK [890]
    EEINQGGR [177]

## Peptide Source: iTRAQ, Small cell lung cancer

MSDVSTSVQSKFARLAKKKENITYMKREQLTETDK<u>DIAPVLDLK</u>CKDVSAIMNKFKVLMEIQDLMFEEM
RETLKNDLKAVLGGKATIPEVKNSENSSSRTEFQQIINLALQKTGMVGKIEGENSKIGDDNENLTFKLE
VNELSGKLDNTNEYNSNDGKKLPQGESRSYEVMGSMEETLCNIDDRDGNRNVHLEFTERESRKDGEDEF
VKEMREERKFQKLKNKEEVLKASREEKVLMDEGAVLTLVADLSSATLDISKQWSNVFNILRENDFEPKF
LCEVKLAFKCDGEIKTFSDLQSLRKFASQKSSVKELLKDVLPQK<u>EEINQGGR</u>KYGIQEKRDKTLIDSKH
RAGEITSDGLSFLFLKEVKVAKPEEMKNLETQEEEFSELEELDEEASGMEDDEDTSGLEEEEEEPSGLE
EEEEEEASGLEEDEASGLEEEEEQTSEQDSTFQGHTLVDAKHEVEITSDGMETTFIDSVEDSESEEEEE
GKSSETGKVKTTSLTEKKASRRQKEIPFSYLVGDSGKKKLVKHQVVHKTQEEEETAVPTSQGTGTPCLT
LCLASPSKSLEMSHDEHKKHSHTNLSISTGVTKLKKTEEKKHRTLHTEELTSKEADLTEETEENLRSSV
INSIREIKEEIGNLKSSHSGVLEIENSVDDLSSRMDILEERIDSLEDQIEEFSKDTMQMTKQIISKERQ
RDIEERSRSCNIRLIGIPEKESYENRAEDIIKEIIDENFAELKKGSSLEIVSACRVPSKIDEKRLTPRH
ILVKFWNSSDKEKIIRASRERREITYQGTRIRLTADLSLDTLDARSKWSNVFKVLLEKGFNPRILYPAK
MAFDFRGKTKVFLSIEEFRDYVLHMPTLRELLGNNIP

**Mass Match Peptides (bold):**
    DIAPVLDLK [890]
    EEINQGGR [177]
**Tandem Peptides (underline):**
    DIAPVLDLK [890]
    EEINQGGR [177]

## OGTA037 (SEQ ID No: 5)

## Peptide Source: 1D-GE, Gastric cancer

MAALIAENFRFLSLFFKSKDVMIFNGLVALGTVGSQELFSVVAFHCPCSPARNYLYGLAAIGVPALVLF
IIGIILNNHTWNLVAECQHRRTKNCSAAPTFLLLSSILGRAAVAPVTWSVISLLRGEAYVCALSEFVDP
SSLTAREEHFPSAHATEILARFPCK<u>ENPDNLSDFR</u>EEVSRRLRYESQLFGWLLIGVVAILVFLTKCLKH
YCSPLSYRQEAYWAQYRANEDQLFQRTAEVHSRVLAANNVRRFFGFVALNKDDEELIANFPVEGTQPRP
QWNAITGVYLYRENQGLPLYSRLHKWAQGLAGNGAAPDNVEMALLPS

**Mass Match Peptides (bold):**
**Tandem Peptides (underline):**
    ENPDNLSDFR [215]

## Peptide Source: iTRAQ, Colorectal cancer

MAALIAENFRFLSLFFKSKDVMIFNGLVALGTVGSQELFSVVAFHCPCSPARNYLYGLAAIGVPALVLF
IIGIILNNHTWNLVAECQHRRTKNCSAAPTFLLLSSILGRAAVAPVTWSVISLLRGEAYVCALSEFVDP

SSLTAREEHFPSAHATEILARFPCKENPDNLSDFREEVSRRLRYESQLFGWLLIGVVAILVFLTKCLKH
YCSPLSYRQEAYWAQYRANEDQLFQRTAEVHSRVLAANNVRRFFGFVALNKDDEELIANFPVEGTQPRP
QWNAITGVYLYRENQGLPLYSRLHKWAQGLAGNGAAPDNVEMALLPS

**Mass Match Peptides (bold):**
VLAANNVR [993]

**Tandem Peptides (underline):**
VLAANNVR [993]

## Peptide Source: iTRAQ, Non-small cell lung cancer

MAALIAENFRFLSLFFKSKDVMIFNGLVALGTVGSQELFSVVAFHCPCSPARNYLYGLAAIGVPALVLF
IIGIILNNHTWNLVAECQHRRTKNCSAAPTFLLLSSILGRAAVAPVTWSVISLLRGEAYVCALSEFVDP
SSLTAREEHFPSAHATEILARFPCKENPDNLSDFREEVSRRLRYESQLFGWLLIGVVAILVFLTKCLKH
YCSPLSYRQEAYWAQYRANEDQLFQRTAEVHSRVLAANNVRRFFGFVALNKDDEELIANFPVEGTQPRP
QWNAITGVYLYRENQGLPLYSRLHKWAQGLAGNGAAPDNVEMALLPS

**Mass Match Peptides (bold):**
VLAANNVR [993]

**Tandem Peptides (underline):**
VLAANNVR [993]

## Peptide Source: iTRAQ, Ovarian cancer

MAALIAENFRFLSLFFKSKDVMIFNGLVALGTVGSQELFSVVAFHCPCSPARNYLYGLAAIGVPALVLF
IIGIILNNHTWNLVAECQHRRTKNCSAAPTFLLLSSILGRAAVAPVTWSVISLLRGEAYVCALSEFVDP
SSLTAREEHFPSAHATEILARFPCKENPDNLSDFREEVSRRLRYESQLFGWLLIGVVAILVFLTKCLKH
YCSPLSYRQEAYWAQYRANEDQLFQRTAEVHSRVLAANNVRRFFGFVALNKDDEELIANFPVEGTQPRP
QWNAITGVYLYRENQGLPLYSRLHKWAQGLAGNGAAPDNVEMALLPS

**Mass Match Peptides (bold):**
VLAANNVR [993]

**Tandem Peptides (underline):**
VLAANNVR [993]

## OGTA041 (SEQ ID No: 6)

## Peptide Source: 1D-GE, Hepatocellular carcinoma

MLTLQTWVVQALFIFLTTESTGELLDPCGYISPESPVVQLHSNFTAVCVLKEKCMDYFHVNANYIVWKT
NHFTIPKEQYTIINRTASSVTFTDIASLNIQLTCNILTFGQLEQNVYGITIISGLPPEKPKNLSCIVNE
GKKMRCEWDGGRETHLETNFTLKSEWATHKFADCKAKRDTPTSCTVDYSTVYFVNIEVWVEAENALGKV
TSDHINFDPVYKVKPNPPHNLSVINSEELSSILKLTWTNPSIKSVIILKYNIQYRTKDASTWSQIPPED
TASTRSSFTVQDLKPFTEYVFRIRCMKEDGKGYWSDWSEEASGITYEDRPSKAPSFWYKIDPSHTQGYR
TVQLVWKTLPPFEANGKILDYEVTLTRWKSHLQNYTVNATKLIVNLTNDRYLATLTVRNLVGKSDAAVL
TIPACDFQATHPVMDLKAFPKDNMLWVEWTTPRESVKKYILEWCVLSDKAPCITDWQQEDGTVHRIYLR
GNLAESKCYLITVTPVYADGPGSPESIKAYLKQAPPSKGPTVRTKKVGKNEAVLEWDQLPVDVQNGFIR
NYTIFYRTIIGNETAVNVDSSHTEYTLSSLTSDTLYMVRMAAYTDEGGKDGPEEIETTPKFAQGEIEAI
VVPVCLAFLLTTLLGVLFCFNKRDLIKKHIWPNVPDPSKSHIAQWSPHTPPRHNFNSKDQMYSDGNFTD
VSVVEIEANDKKPFPEDLKSLDLFKKEKINTEGHSSGIGGSSCMSSSRPSISSSDENESSQNTSSIVQY

STVVHSGYRHQVPSVQVFSRSESTQPLLDSEERPEDLQLVDHVDGGDGILPRQQYFKQNCSQHESSPDI
SHFERSKQVSSVNEEDFVRLKQQISDHISQSCGSGQMKMFQEVSAADAFGPGTEGQVERFETVGMEAAT
DEGMPKSYLPQTVRQGGYMPQ

**Mass Match Peptides (bold):**
KPFPEDLK [431]

**Tandem Peptides (underline):**
DGPEFTFTTPK [124]


## Peptide Source: 1D-GE, Lung cancer

MLTLQTWVVQALFIFLTTESTGELLDPCGYISPESPVVQLHSNFTAVCVLKEKCMDYFHVNANYIVWKT
NHFTIPKEQYTIINRTASSVTFTDIASLNIQLTCNILTFGQLEQNVYGITIISGLPPEKPKNLSCIVNE
GKKMRCEWDGGRETHLETNFTLKSEWATHKFADCKAKRDTPTSCTVDYSTVYFVNIEVWVEAENALGKV
TSDHINFDPVYKVKPNPPHNLSVINSEELSSILKLTWTNPSIKSVIILKYNIQYRTKDASTWSQIPPED
TASTRSSFTVQDLKPFTEYVFRIRCMKEDGKGYWSDWSEEASGITYEDRPSKAPSFWYKIDPSHTQGYR
TVQLVWKTLPPFEANGKILDYEVTLTRWK**SHLQNYTVNATK**LIVNLTNDRYLATLTVRNLVGKSDAAVL
TIPACDFQATHPVMDLKAFPK**DNMLWVEWTTPR**ESVKKYILEWCVLSDKAPCITDWQQEDGTVHRIYLR
GNLAESKCYLITVTPVYADGPGSPESIKAYLKQAPPSKGPTVRTKKVGKNEAVLEWDQLPVDVQNGFIR
NYTIFYRTIIGNETAVNVDSSHTEYTLSSLTSDTLYMVRMAAYTDEGGKDGPEFTFTTPKFAQGEIEAI
VVPVCLAFLLTTLLGVLFCFNKRDLIKKHIWPNVPDPSKSHIAQWSPHTPPRHNFNSKDQMYSDGNFTD
VSVVEIEANDKKPFPEDLKSLDLFKKEKINTEGHSSGIGGSSCMSSSRPSISSSDENESSQNTSSIVQY
STVVHSGYRHQVPSVQVFSRSESTQPLLDSEERPEDLQLVDHVDGGDGILPRQQYFK**QNCSQHESSPDI
SHFER**SKQVSSVNEEDFVRLKQQISDHISQSCGSGQMKMFQEVSAADAFGPGTEGQVERFETVGMEAAT
DEGMPKSYLPQTVRQGGYMPQ

**Mass Match Peptides (bold):**
DNMLWVEWTTPR [146]
QNCSQHESSPDISHFER [582]
QVSSVNEEDFVR [591]
SHLQNYTVNATK [635]

**Tandem Peptides (underline):**
QVSSVNEEDFVR [591]


## Peptide Source: 1D-GE, Pancreatic cancer

MLTLQTWVVQALFIFLTTESTGELLDPCGYISPESPVVQLHSNFTAVCVLKEKCMDYFHVNANYIVWKT
NHFTIPKEQYTIINRTASSVTFTDIASLNIQLTCNILTFGQLEQNVYGITIISGLPPEKPKNLSCIVNE
GKKMRCEWDGGRETHLETNFTLKSEWATHKFADCKAKRDTPTSCTVDYSTVYFVNIEVWVEAENALGKV
TSDHINFDPVYKVKPNPPHNLSVINSEELSSILKLTWTNPSIKSVIILKYNIQYRTKDASTWSQIPPED
TASTRSSFTVQDLKPFTEYVFRIRCMKEDGKGYWSDWSEEASGITYEDRPSKAPSFWYKIDPSHTQGYR
TVQLVWKTLPPFEANGKILDYEVTLTRWK**SHLQNYTVNATK**LIVNLTNDRYLATLTVRNLVGKSDAAVL
TIPACDFQATHPVMDLKAFPKDNMLWVEWTTPRESVKKYILEWCVLSDKAPCITDWQQEDGTVHRIYLR
GNLAESKCYLITVTPVYADGPGSPESIKAYLKQAPPSKGPTVRTKKVGKNEAVLEWDQLPVDVQNGFIR
**NYTIFYR**TIIGNETAVNVDSSHTEYTLSSLTSDTLYMVRMAAYTDEGGKDGPEFTFTTPKFAQGEIEAI
VVPVCLAFLLTTLLGVLFCFNKRDLIKK**HIWPNVPDPSK**SHIAQWSPHTPPRHNFNSKDQMYSDGNFTD
VSVVEIEANDKKPFPEDLKSLDLFKKEKINTEGHSSGIGGSSCMSSSRPSISSSDENESSQNTSSIVQY
STVVHSGYRHQVPSVQVFSRSESTQPLLDSEERPEDLQLVDHVDGGDGILPRQQYFKQNCSQHESSPDI
SHFERSKQVSSVNEEDFVRLKQQISDHISQSCGSGQMKMFQEVSAADAFGPGTEGQVERFETVGMEAAT
DEGMPKSYLPQTVRQGGYMPQ

**Mass Match Peptides (bold):**

```
HIWPNVPDPSK [336]
NYTIFYR [564]
SHLQNYTVNATK [635]
TNHFTIPK [711]
```

**Tandem Peptides (underline):**

```
HIWPNVPDPSK [336]
```

## Peptide Source: iTRAQ, Non-small cell lung cancer

```
MLTLQTWVVQALFIFLTTESTGELLDPCGYISPESPVVQLHSNFTAVCVLKEKCMDYFHVNANYIVWKT
NHFTIPKEQYTIINRTASSVTFTDIASLNIQLTCNILTFGQLEQNVYGITIISGLPPEKPKNLSCIVNE
GKKMRCEWDGGRETHLETNFTLKSEWATHKFADCKAKRDTPTSCTVDYSTVYFVNIEVWVEAENALGKV
TSDHINFDPVYKVKPNPPHNLSVINSEELSSILKLTWTNPSIKSVIILKYNIQYRTKDASTWSQIPPED
TASTRSSFTVQDLKPFTEYVFRIRCMKEDGKGYWSDWSEEASGITYEDRPSKAPSFWYKIDPSHTQGYR
TVQLVWKTLPPFEANGKILDYEVTLTRWKSHLQNYTVNATKLCVNLTNDRYLATLTVRNLVGKSDAAVL
TIPACDFQATHPVMDLKAFPKDNMLWVEWTTPRESVKKYILEWCVLSDKAPCITDWQQEDGTVHRCYLR
GNLAESKCYLITVTPVYADGPGSPESIKAYLKQAPPSKGPTVRTKKVGKNEAVLEWDQLPVDVQNGFIR
NYTIFYRTIIGNETAVNVDSSHTEYTLSSLTSDTLYMVRMAAYTDEGGKDGPEFTFTTPKFAQGEIEAI
VVPVCLAFLLTTLLGVLFCFNKRDLIKKHIWPNVPDPSKSHIAQWSPHTPPRHNFNSKDQMYSDGNFTD
VSVVEIEANDKKPFPEDLKSLDLFKKEKINTEGHSSGIGGSSCMSSSRPSISSSDENESSQNTSSCVQY
STVVHSGYRHQVPSVQVFSRSESTQPLLDSEERPEDLQLVDHVDGGDGILPRQQYFKQNCSQHESSPDI
SHFERSKQVSSVNEEDFVRLKQQISDHISQSCGSGQMKMFQEVSAADAFGPGTEGQVERFETVGMEAAT
DEGMPKSYLPQTVRQGGYMPQ
```

**Mass Match Peptides (bold):**

```
LTWTNPSIK [950]
```

**Tandem Peptides (underline):**

```
LTWTNPSIK [950]
```

## OGTA053 (SEQ ID No: 7)

## Peptide Source: 1D-GE, Pancreatic cancer

```
MPGIVVFRRRWSVGSDDLVLPAIFLFLLHTTWFVILSVVLFGLVYNPHEACSLNLVDHGRGYLGILLSC
MIAEMAIIWLSMRGGILYTEPRDSMQYVLYVRLAILVIEFIYAIVGIVWLTQYYTSCNDLTAKNVCLGM
VVCNWVVILSVCITVLCVFDPTGRTFVKLRATKRRQRNLRTYNLRHRLEEGQATSWSRRLKVFLCCTRT
KDSQSDAYSEIAYLFAEFFRDLDIVPSDIIAGLVLLRQRQRAKRNAVLDEANNDILAFLSGMPVTRNTK
YLDLKNSQEMLRYKEVCYYMLFALAAYGWPMYLMRKPACGLCQLARSCSCCLCPARPRFAPGVTIEEDN
CCGCNAIAIRRHFLDENMTAVDIVYTSCHDAVYETPFYVAVDHDKKKVVISIRGTLSPKDALTDLCGDA
ERLPVEGHHGTWLGHKGMVLSAEYIKKKLEQEMVLSQAFGRDLGRGTKHYGLIVVGHSLGAGTAAILSF
LLRPQYPTLKCFAYSPPGGLLSEDAMEYSKEFVTAVVLGKDLVPRIGLSQLEGFRRQLLDVLQRSCKPK
WRIIVGATKCIPKSELPEEVEVTTLASTRLWTHPSDLTIALSASTPLYPPGRIIHVVHNHPAEQCCCCE
QEEPTYFAIWGDNKAFNEVIISPAMLHEHLPYVVMEGLNKVLENYNKGKTALLSAAKVMVSPTEVDLTP
ELIFQQQPLPTGPPMPTGLALELPTADIIRNSSVRSKSQSEMSLEGFSEGRLLSPVVAAAARQDPVELLL
LSTQERLAAELQARRAPLATMESLSDTESLYSFDSRRSSGFRSIRGSPSLHAVLERDEGHLFYIDPAIP
EENPSLSSRTELLAADSLSKHSQDTQPLEAALGSGGVTPERPPSAAANDEEEEVGGGGGGPASRGELAL
HNGRLGDSPSPQVLEFAEFIDSLFNLDSKSSSFQDLYCMVVPESPTSDYAEGPKSPSQQEILLRAQFEP
NLVPKPPRLFAGSADPSSGISLSPSFPLSSSGELMDLTPTGLSSQECLAADKIRTSTPTGHGASPAKQD
ELVISAR
```

**Mass Match Peptides (bold):**

```
FAPGVTIEEDNCCGCNAIAIR [243]
```

VLENYNK [780]

**Tandem Peptides (underline):**
LEEGQATSWSR [452]


## Peptide Source: iTRAQ, Colorectal cancer

MPGIVVFRRRWSVGSDDLVLPAIFLFLLHTTWFVILSVVLFGLVYNPHEACSLNLVDHGRGYLGILLSC
MIAEMAIIWLSMRGGILYTEPRDSMQYVLYVRLAILVIEFIYAIVGIVWLTQYYTSCNDLTAKNVTLGM
VVCNWVVILSVCITVLCVFDPTGRTFVKLRATKRRQRNLRTYNLRHRLEEGQATSWSRRLKVFLCCTRT
KDSQSDAYSEIAYLFAEFFRDLDIVPSDIIAGLVLLRQRQRAKRNAVLDEANNDILATLSGMPVTRNTK
YLDLKNSQEMLRYKEVCYYMLFALAAYGWPMYLMRKPACGLCQLARSCSCCLCPARPRFAPGVTIEEDN
CCGCNAIAIRRHFLDENMTAVDIVYTSCHDAVYETPFYVAVDHDKKK**VVISIR**GTLSPKDALTDLTGDA
ERLPVEGHHGTWLGHKGMVLSAEYIKKKLEQEMVLSQAFGRDLGRGTKHYGLIVVGHSLGAGTAAILSF
LLRPQYPTLKCTAYSPPGGLLSEDAMEYSKEFVTAVVLGKDLVPRIGLSQLEGFRRQLLDVLQRSTKPK
WRIIVGATKCIPKSELPEEVEVTTLASTRLWTHPSDLTIALSASTPLYPPGRIIHVVHNHPAEQCCCCE
QEEPTYFAIWGDNKAFNEVIISPAMLHEHLPYVVMEGLNKVLENYNKGKTALLSAAKVMVSPTEVDLTP
ELIFQQQPLPTGPPMPTGLALELPTADHRNSSVRSKSQSEMSLEGFSEGRLLSPVVAAAARQDPVELLL
LSTQERLAAELQARRAPLATMESLSDTESLYSFDSRRSSGFRSIRGSPSLHAVLERDEGHLFYIDPAIP
EENPSLSSR**TELLAADSLSK**HSQDTQPLEAALGSGGVTPERPPSAAANDEEEEVGGGGGGPASRGELAL
HNGRLGDSPSPQVLEFAEFIDSLFNLDSKSSSFQDLYCMVVPESPTSDYAEGPKSPSPQQEILLRAQFEP
NLVPKPPRLFAGSADPSSGISLSPSFPLSSSGELMDLTPTGLSSQECLAADK**IRTSTPTGHGASPAK**QD
ELVISAR

**Mass Match Peptides (bold):**
IRTSTPTGHGASPAK [924]
TELLAADSLSK [980]
VVISIR [1002]

**Tandem Peptides (underline):**
IRTSTPTGHGASPAK [924]
TELLAADSLSK [980]
VVISIR [1002]


## Peptide Source: iTRAQ, Hepatocellular cancer

MPGIVVFRRRWSVGSDDLVLPAIFLFLLHTTWFVILSVVLFGLVYNPHEACSLNLVDHGRGYLGILLSC
MIAEMAIIWLSMRGGILYTEPRDSMQYVLYVRLAILVIEFIYAIVGIVWLTQYYTSCNDLTAKNVTLGM
VVCNWVVILSVCITVLCVFDPTGRTFVKLRATKRRQRNLRTYNLRHRLEEGQATSWSRRLKVFLCCTRT
KDSQSDAYSEIAYLFAEFFRDLDIVPSDIIAGLVLLRQRQRAKRNAVLDEANNDILATLSGMPVTRNTK
YLDLKNSQEMLRYKEVCYYMLFALAAYGWPMYLMRKPACGLCQLARSCSCCLCPARPRFAPGVTIEEDN
CCGCNAIAIRRHFLDENMTAVDIVYTSCHDAVYETPFYVAVDHDKKKVVISIRGTLSPKDALTDLTGDA
ERLPVEGHHGTWLGHKGMVLSAEYIKKKLEQEMVLSQAFGRDLGRGTKHYGLIVVGHSLGAGTAAILSF
LLRPQYPTLKCTAYSPPGGLLSEDAMEYSKEFVTAVVLGKDLVPRIGLSQLEGFRRQLLDVLQRSTKPK
WRIIVGATKCIPKSELPEEVEVTTLASTRLWTHPSDLTIALSASTPLYPPGRIIHVVHNHPAEQCCCCE
QEEPTYFAIWGDNKAFNEVIISPAMLHEHLPYVVMEGLNKVLENYNKGKTALLSAAKVMVSPTEVDLTP
ELIFQQQPLPTGPPMPTGLALELPTADHRNSSVRSKSQSEMSLEGFSEGRLLSPVVAAAARQDPVELLL
LSTQERLAAELQARRAPLATMESLSDTESLYSFDSRRSSGFRSIRGSPSLHAVLERDEGHLFYIDPAIP
EENPSLSSRTELLAADSLSKHSQDTQPLEAALGSGGVTPERPPSAAANDEEEEVGGGGGGPASRGELAL
HNGRLGDSPSPQVLEFAEFIDSLFNLDSKSSSFQDLYCMVVPESPTSDYAEGPKSPSPQQEILLRAQFEP
NLVPKPPRLFAGSADPSSGISLSPSFPLSSSGELMDLTPTGLSSQECLAADK**IRTSTPTGHGASPAK**QD
ELVISAR

**Mass Match Peptides (bold):**

IRTSTPTGHGASPAK [924]
**Tandem Peptides (underline):**
IRTSTPTGHGASPAK [924]


## Peptide Source: iTRAQ, Non-small cell lung cancer

MPGIVVFRRRWSVGSDDLVLPAIFLFLLHTTWFVILSVVLFGLVYNPHEACSLNLVDHGRGYLGILLSC
MIAEMAIIWLSMRGGILYTEPRDSMQYVLYVRLAILVIEFIYAIVGIVWLTQYYTSCNDLTAKNV␣LGM
VVCNWVVILSVCITVLCVFDPTGRTFVKLRATKRRQRNLRTYNLRHRLEEGQATSWSRRLKVFLCCTRT
KDSQSDAYSEIAYLFAEFFRDLDIVPSDIIAGLVLLRQRQRAKRNAVLDEANNDILA␣LSGMPVTRNTK
YLDLKNSQEMLRYKEVCYYMLFALAAYGWPMYLMRKPACGLCQLARSCSCCLCPARPRFAPGVTIEEDN
CCGCNAIAIRRHFLDENMTAVDIVYTSCHDAVYETPFYVAVDHDKKKVVISIR**GTLSPKDALTDLTGDA**
**ER**LPVEGHHGTWLGHKGMVLSAEYIKKKLEQEMVLSQAFGRDLGRGTKHYGLIVVGHSLGAGTAA␣LSF
LLRPQYPTLKC␣AYSPPGGLLSEDAMEYSKEFVTAVVLGKDLVPRIGLSQLEGFRRQLLDVLQRS␣KPK
WRIIVGATKCIPKSELPEEVEVTTLASTRLWTHPSDLTIALSASTPLYPPGRIIHVVHNHPAEQCCCCE
QEEPTYFAIWGDNKAFNEVIISPAMLHEHLPYVVMEGLNKVLENYNKGKTALLSAAKVMVSPTEVDLTP
ELIFQQQPLPTGPPMPTGLALELPTADHRNSSVRSKSQSEM␣SLEGFSEGRLLSPVVAAAARQDPVELLL
LSTQERLAAELQARRAPLATMESLSDTESLYSFDSRRSSGFRSIRGSPSLHAVLERDEGHLFYIDPAIP
EENPSLSSR**TELLAADSLSK**HSQDTQPLEAALGSGGVTPERPPSAAANDEEEEVGGGGGGPASRGELAL
HNGRLGDSPSPQVLEFAEFIDSLFNLDSKSSSFQDLYCMVVPESPTSDYAEGPKSPSQQEILLRAQFEP
NLVPKPPRLFAGSADPSSGISLSPSFPLSSSGELMDLTPTGLSSQECLAADK**IRTSTPTGHGASPAK**QD
ELVISAR

**Mass Match Peptides (bold):**
GTLSPKDALTDLTGDAER [916]
␣RTSTPTGHGASPAK [924]
␣ELLAADSLSK [980]
**Tandem Peptides (underline):**
GTLSPKDALTDLTGDAER [916]
␣RTSTPTGHGASPAK [924]
␣ELLAADSLSK [980]


## Peptide Source: iTRAQ, Renal cell cancer

MPGIVVFRRRWSVGSDDLVLPAIFLFLLHTTWFVILSVVLFGLVYNPHEACSLNLVDHGRGYLGILLSC
MIAEMAIIWLSMRGGILYTEPRDSMQYVLYVRLAILVIEFIYAIVGIVWLTQYYTSCNDLTAKNV␣LGM
VVCNWVVILSVCITVLCVFDPTGRTFVKLRATKRRQRNLRTYNLRHRLEEGQATSWSRRLKVFLCCTRT
KDSQSDAYSEIAYLFAEFFRDLDIVPSDIIAGLVLLRQRQRAKRNAVLDEANNDILA␣LSGMPVTRNTK
YLDLKNSQEMLRYKEVCYYMLFALAAYGWPMYLMRKPACGLCQLARSCSCCLCPARPRFAPGVTIEEDN
CCGCNAIAIRRHFLDENMTAVDIVYTSCHDAVYETPFYVAVDHDKKKVVISIRGTLSPKDALTDL␣GDA
ERLPVEGHHGTWLGHKGMVLSAEYIKKKLEQEMVLSQAFGRDLGRGTKHYGLIVVGHSLGAGTAA␣LSF
LLRPQYPTLKC␣AYSPPGGLLSEDAMEYSKEFVTAVVLGKDLVPRIGLSQLEGFRRQLLDVLQRS␣KPK
WRIIVGATKCIPKSELPEEVEVTTLASTRLWTHPSDLTIALSASTPLYPPGRIIHVVHNHPAEQCCCCE
QEEPTYFAIWGDNKAFNEVIISPAMLHEHLPYVVMEGLNKVLENYNKGKTALLSAAKVMVSPTEVDLTP
ELIFQQQPLPTGPPMPTGLALELPTADHRNSSVRSKSQSEM␣SLEGFSEGRLLSPVVAAAARQDPVELLL
LSTQERLAAELQARRAPLATMESLSDTESLYSFDSRRSSGFRSIRGSPSLHAVLERDEGHLFYIDPAIP
EENPSLSSR**TELLAADSLSK**HSQDTQPLEAALGSGGVTPERPPSAAANDEEEEVGGGGGGPASRGELAL
HNGRLGDSPSPQVLEFAEFIDSLFNLDSKSSSFQDLYCMVVPESPTSDYAEGPKSPSQQEILLRAQFEP
NLVPKPPRLFAGSADPSSGISLSPSFPLSSSGELMDLTPTGLSSQECLAADK**IRTSTPTGHGASPAK**QD
ELVISAR

**Mass Match Peptides (bold):**

```
IRTSTPTGHGASPAK [924]
TELLAADSLSK [980]
```

**Tandem Peptides (underline):**
```
IRTSTPTGHGASPAK [924]
TELLAADSLSK [980]
```

## Peptide Source: iTRAQ, Small cell lung cancer

```
MPGIVVFRRRWSVGSDDLVLPAIFLFLLHTTWFVILSVVLFGLVYNPHEACSLNLVDHGRGYLGILLSC
MIAEMAIIWLSMRGGILYTEPRDSMQYVLYVRLAILVIEFIYAIVGIVWLTQYYTSCNDLTAKNVTLGM
VVCNWVVILSVCITVLCVFDPTGRTFVKLRATKRRQRNLRTYNLRHRLEEGQATSWSRRLKVFLCCTRT
KDSQSDAYSEIAYLFAEFFRDLDIVPSDIIAGLVLLRQRQRAKRNAVLDEANNDILAFLSGMPVTRNTK
YLDLKNSQEMLRYKEVCYYMLFALAAYGWPMYLMRKPACGLCQLARSCSCCLCPARPRFAPGVTIEEDN
CCGCNAIAIRRHFLDENMTAVDIVYTSCHDAVYETPFYVAVDHDKKKVVISIRGTLSPKDALTDLTGDA
ERLPVEGHHGTWLGHKGMVLSAEYIKKKLEQEMVLSQAFGRDLGRGTKHYGLIVVGHSLGAGTAAILSF
LLRPQYPTLKCFAYSPPGGLLSEDAMEYSKEFVTAVVLGKDLVPRIGLSQLEGFRRQLLDVLQRSTKPK
WRIIVGATKCIPKSELPEEVEVTTLASTRLWTHPSDLTIALSASTPLYPPGRIIHVVHNHPAEQCCCCE
QEEPTYFAIWGDNKAFNEVIISPAMLHEHLPYVVMEGLNKVLENYNKGKTALLSAAKVMVSPTEVDLTP
ELIFQQQPLPTGPPMPTGLALELPTADHRNSSVRSKSQSEMSLEGFSEGRLLSPVVAAAARQDPVELLL
LSTQERLAAELQARRAPLATMESLSDTESLYSFDSRRSSGFRSIRGSPSLHAVLERDEGHLFYIDPAIP
EENPSLSSR**TELLAADSLSK**HSQDTQPLEAALGSGGVTPERPPSAAANDEEEEVGGGGGGPASRGELAL
HNGRLGDSPSPQVLEFAEFIDSLFNLDSKSSSFQDLYCMVVPESPTSDYAEGPKSPSQQEILLRAQFEP
NLVPKPPRLFAGSADPSSGISLSPSFPLSSSGELMDLTPTGLSSQECLAADKIRTSTPTGHGASPAKQD
ELVISAR
```

**Mass Match Peptides (bold):**
```
TELLAADSLSK [980]
```
**Tandem Peptides (underline):**
```
TELLAADSLSK [980]
```

## OGTA054 (SEQ ID No: 8)

## Peptide Source: 1D-GE, Pancreatic cancer

```
MHKEEHEVAVLGAPPSTILPRSTVINIHSETSVPDHVVWSLFNTLFLNWCCLGFIAFAYSVKSRDRKMV
GDVTGAQAYASTAKCLNIWALILGILMTIGFILLLVFGSVTVYHIMLQIIQEKRGY
```

**Mass Match Peptides (bold):**
**Tandem Peptides (underline):**
```
EEHEVAVLGAPPSTILPR [173]
```

## Peptide Source: iTRAQ, Colorectal cancer

```
MHKEEHEVAVLGAPPSTILPRSTVINIHSETSVPDHVVWSLFNTLFLNWCCLGFIAFAYSVKSRDRKMV
GDVTGAQAYASTAKCLNIWALILGILMTIGFILLLVFGSVTVYHIMLQIIQEKRGY
```

**Mass Match Peptides (bold):**
```
EEHEVAVLGAPPSTILPR [173]
MVGDVTGAQAYASTAK [954]
```

**Tandem Peptides (underline):**
    EEHEVAVLGAPPSTILPR [173]
    MVGDVTGAQAYASTAK [954]


## Peptide Source: iTRAQ, Non-small cell lung cancer

MHKEEHEVAVLGAPPSTILPRSTVINIHSETSVPDHVVWSLFNTLFLNWCCLGFIAFAYSVKSRDRKMV
GDVTGAQAYASTAKCLNIWALILGILMTIGFILLLVFGSVTVYHIMLQIIQEKRGY


**Mass Match Peptides (bold):**
    MVGDVTGAQAYASTAK [954]
**Tandem Peptides (underline):**
    MVGDVTGAQAYASTAK [954]


## OGTA066 (SEQ ID No: 9)

## Peptide Source: 1D-GE, Colorectal cancer

MMLHSALGLCLLLVTVSSNLAIAIKKEKRPPQTLSRGWGDDI□WVQTYEEGLFYAQKSKKPLMVIHHLE
DCQYSQALKKV□AQNEEIQEMAQNKFIMLNLMHETTDKNLSPDGQYVPRIMFVDPSLTVRADIAGRYSN
RLYTYEPRDLPLLIENMKKALRLIQSEL


**Mass Match Peptides (bold):**
    IMFVDPSLTVR [380]
**Tandem Peptides (underline):**
    IMFVDPSLTVR [380]


## Peptide Source: 1D-GE, Pancreatic cancer

MMLHSALGLCLLLVTVSSNLAIAIKKEKRPPQTLSRGWGDDI□WVQTYEEGLFYAQKSKKPLMVIHHLE
DCQYSQALKKV□AQNEEIQEMAQNKFIMLNLMHETTDKNLSPDGQYVPRIMFVDPSLTVRADIAGRYSN
RLYTYEPRDLPLLIENMKKALRLIQSEL


**Mass Match Peptides (bold):**
    IMFVDPSLTVR [380]
    KPLMVIHHLEDCQYSQALK [432]
**Tandem Peptides (underline):**
    IMFVDPSLTVR [380]

## Peptide Source: iTRAQ, Colorectal cancer

MMLHSALGLCLLLVTVSSNLAIAIKKEKRPPQTLSRGWGDDI□WVQTYEEGLFYAQKSKKPLMVIHHLE
DCQYSQALKKV□AQNEEIQEMAQNKFIMLNLMHETTDKNLSPDGQYVPRIMFVDPSLTVRADIAGRYSN
RLYTYEPRDLPLLIENMKKALRLIQSEL


**Mass Match Peptides (bold):**
    DLPLLIENMK [135]
    IMFVDPSLTVR [380]

**Tandem Peptides (underline):**
      DLPLLIENMK [135]
      IMFVDPSLTVR [380]


## Peptide Source: iTRAQ, Non-small cell lung cancer

MMLHSALGLCLLLVTVSSNLAIAIKKEKRPPQTLSRGWGDDI⊐WVQTYEEGLFYAQKSKKPLMVIHHLE
DCQYSQALKKV⊐AQNEEIQEMAQNKFIMLNLMHETTDK<u>**NLSPDGQYVPRIMFVDPSLTVR**</u>ADIAGRYSN
RLYTYEPR<u>**DLPLLIENMK**</u>KALRLIQSEL


**Mass Match Peptides (bold):**
      DLPLLIENMK [135]
      IMFVDPSLTVR [380]
      NLSPDGQYVPR [546]

**Tandem Peptides (underline):**
      DLPLLIENMK [135]
      IMFVDPSLTVR [380]
      NLSPDGQYVPR [546]


## Peptide Source: iTRAQ, Kidney cancer

MMLHSALGLCLLLVTVSSNLAIAIKKEKRPPQTLSRGWGDDI⊐WVQTYEEGLFYAQKSKKPLMVIHHLE
DCQYSQALKKV⊐AQNEEIQEMAQNKFIMLNLMHETTDKNLSPDGQYVPRIMFVDPSLTVRADIAGRYSN
RLYTYEPR<u>**DLPLLIENMK**</u>KALRLIQSEL


**Mass Match Peptides (bold):**
      DLPLLIENMK [135]

**Tandem Peptides (underline):**
      DLPLLIENMK [135]


## OGTA072 (SEQ ID No: 10)


## Peptide Source: 1D-GE, Colorectal cancer

<u>**MTTSGALFPSLVPGSR**</u>GASNKYLVEFRAGKMSLKGTTVTPDKRKGLVYIQQTDDSLIHFCWKDRTSGNV
EDDLIIFPDDCEFKRVPQCPSGRVYVLKFKAGSKRLFFWMQEPK<u>TDQDEEHCR</u>KVNEYLNNPPMPGALG
ASGSSGHELSALGGEGGGLQSLLGNMSHSQLMQLIGPAGLGGLGGLGALTGPGLASLLGSSGPPGSSSSS
SSRSQSAAVTPSSTTSSTRATPAPSAPAAASATSPSPAPSSGNGASTAASPTQPIQLSDLQSILA⊐MNV
PAGPAGGQQVDLASVLTPEIMAPILANADVQERLLPYLPSGESLPQTADEIQNTLTSPQFQQALGMFSA
ALASGQLGPLMCQFGLPAEAVEAANKGDVEAFAKAMQNNAKPEQKEGDTKDKKDEEEDMSLD


**Mass Match Peptides (bold):**
      MTTSGALFPSLVPGSR [526]

**Tandem Peptides (underline):**
      TDQDEEHCR [685]


## OGTA074 (SEQ ID No: 11)

## Peptide Source: 1D-GE, Colorectal cancer

```
MGDVKALLFVAAARARRLGGAAASESLAVSEAFCRVRSCQPKKCAGPQRCLNPVPAVPSPSPSVRKRQV
SLNWQPLTLQEARALLKRRRPRGPGGRGLLRRRPPQRAPAGKAPVLCPLICHNGGVCVKPDRCLCPPDF
AGKFCQLHSSGARPPAPAVPGLTRSVYTMPLANHRDDEHGVASMVSVHVEHPQEASVVVHQVERVSGPW
EEADAEAVARAEAAARAEAAAPYTVLAQSAPREDGYSDASGFGYCFRELRGGECASPLPGLRTQEVCCR
GAGLAWGVHDCQLCSERLGNSERVSAPDGPCPTGFERVNGSCEDVDECATGGRCQHGECANTRGGYTCV
CPDGFLLDSSRSSCISQHVISEAKGPCFRVLRDGGCSLPILRNITKQICCCSRVGKAWGRGCQLCPPFG
SEGFREICPAGPGYHYSASDLRYNTRPLGQEPPRVSLSQPRTLPATSRPSAGFLPTHRLEPRPEPRPDP
RPGPELPLPSIPAWTGPEIPESGPSSGMCQRNPQVCGPGRCISRPSGYTCACDSGFRLSPQGTRCIDVD
ECRRVPPPCAPGRCENSPGSFRCVCGPGFRAGPRAAECLDVDECHRVPPPCDLGRCENTPGSFLCVCPA
GYQAAPHGASCQDVDECTQSPGLCGRGACKNLPGSFRCVCPAGFRGSACEEDVDECAQEPPPCGPGRCD
NTAGSFHCACPAGFRSRGPGAPCQDVDECARSPPPCTYGRCENTEGSFQCVCPMGFQPNTAGSECEDVD
ECENHLACPGQECVNSPGSFQCRTCPSGHHLHRGRCTDVDECSSGAPPCGPHGHCTNTEGSFRCSCAPG
YRAPSGRPGPCADVNECLEGDFCFPHGECLNTDGSFACTCAPGYRPGPRGASCLDVDECSEEDLCQSGI
CTNTDGSFECICPPGHRAGPDLASCLDVDECRERGPALCGSQRCENSPGSYRCVRDCDPGYHAGPEGTC
DDVDECQEYGPEICGAQRCENTPGSYRCTPACDPGYQPTPGGGCQDVDECRNRSFCGAHAVCQNLPGSF
QCLCDQGYEGARDGRHCVDVNECETLQGVCGAALCENVEGSFLCVCPNSPEEFDPMTGRCVPPRTSAGT
FPGSQPQAPASPVLPARPPPPPLPRRPSTPRQGPVGSGRRECYFDTAAPDACDNILARNVTWQECCCTV
GEGWGSGCRIQQCPGTETAEYQSLCPHGRGYLAPSGDLSLRRDVDECQLFRDQVCKSGVCVNTAPGYSC
YCSNGYYYHTQRLECIDNDECADEEPACEGGRCVNTVGSYHCTCEPPLVLDGSQRRCVSNESQSLDDNL
GVCWQEVGADLVCSHPRLDRQATYTECCCLYGEAWGMDCALCPAQDSDDFEALCNVLRPPAYSPPRPGG
FGLPYEYGPDLGPPYQGLPYGPELYPPPALPYDPYPPPPGPFARREAPYGAPRFDMPDFEDDGGPYGES
EAPAPPGPGTRWPYRSRDTRRSFPEPEEPPEGGSYAGSLAEPYEELEAEECGILDGCTNGRCVRVPEGF
TCRCFDGYRLDMTRMACVDINECDEAEAASPLCVNARCLNIDGSFRCICRPGFAPTHQPHHCAPARPRA
```

### Mass Match Peptides (bold):
QGPVGSGRR [577]
VPEGFTCR [790]

### Tandem Peptides (underline):
EDGYSDASGFGYCFR [169]


## Peptide Source: iTRAQ, Non-small cell lung cancer

```
MGDVKALLFVAAARARRLGGAAASESLAVSEAFCRVRSCQPKKCAGPQRCLNPVPAVPSPSPSVRKRQV
SLNWQPLTLQEARALLKRRRPRGPGGRGLLRRRPPQRAPAGKAPVLCPLICHNGGVCVKPDRCLCPPDF
AGKFCQLHSSGARPPAPAVPGLTRSVYTMPLANHRDDEHGVASMVSVHVEHPQEASVVVHQVERVSGPW
EEADAEAVARAEAAARAEAAAPYTVLAQSAPREDGYSDASGFGYCFRELRGGECASPLPGLRTQEVCCR
GAGLAWGVHDCQLCSERLGNSERVSAPDGPCPTGFERVNGSCEDVDECATGGRCQHGECANTRGGYTCV
CPDGFLLDSSRSSCISQHVISEAKGPCFRVLRDGGCSLPILRNITKQICCCSRVGKAWGRGCQLCPPFG
SEGFREICPAGPGYHYSASDLRYNTRPLGQEPPRVSLSQPRTLPATSRPSAGFLPTHRLEPRPEPRPDP
RPGPELPLPSIPAWTGPEIPESGPSSGMCQRNPQVCGPGRCISRPSGYTCACDSGFRLSPQGTRCIDVD
ECRRVPPPCAPGRCENSPGSFRCVCGPGFRAGPRAAECLDVDECHRVPPPCDLGRCENTPGSFLCVCPA
GYQAAPHGASCQDVDECTQSPGLCGRGACKNLPGSFRCVCPAGFRGSACEEDVDECAQEPPPCGPGRCD
NTAGSFHCACPAGFRSRGPGAPCQDVDECARSPPPCTYGRCENTEGSFQCVCPMGFQPNTAGSECEDVD
ECENHLACPGQECVNSPGSFQCRTCPSGHHLHRGRCTDVDECSSGAPPCGPHGHCTNTEGSFRCSCAPG
YRAPSGRPGPCADVNECLEGDFCFPHGECLNTDGSFACTCAPGYRPGPRGASCLDVDECSEEDLCQSGI
CTNTDGSFECICPPGHRAGPDLASCLDVDECRERGPALCGSQRCENSPGSYRCVRDCDPGYHAGPEGTC
DDVDECQEYGPEICGAQRCENTPGSYRCTPACDPGYQPTPGGGCQDVDECRNRSFCGAHAVCQNLPGSF
QCLCDQGYEGARDGRHCVDVNECETLQGVCGAALCENVEGSFLCVCPNSPEEFDPMTGRCVPPRTSAGT
FPGSQPQAPASPVLPARPPPPPLPRRPSTPRQGPVGSGRRECYFDTAAPDACDNILARNVTWQECCCTV
GEGWGSGCRIQQCPGTETAEYQSLCPHGRGYLAPSGDLSLRRDVDECQLFRDQVCKSGVCVNTAPGYSC
YCSNGYYYHTQRLECIDNDECADEEPACEGGRCVNTVGSYHCTCEPPLVLDGSQRRCVSNESQSLDDNL
GVCWQEVGADLVCSHPRLDRQATYTECCCLYGEAWGMDCALCPAQDSDDFEALCNVLRPPAYSPPRPGG
FGLPYEYGPDLGPPYQGLPYGPELYPPPALPYDPYPPPPGPFARREAPYGAPRFDMPDFEDDGGPYGES
```

EAPAPPGPGTRWPYRSRDTRRSFPEPEEPPEGGSYAGSLAEPYEELEAEECGILDGCTNGRCVRVPEGF
TCRCFDGYRLDMTRMACVDINECDEAEAASPLCVNARCLNTDGSFRCICRPGFAPTHQPHHCAPARPRA

## Mass Match Peptides (bold):

CLNPVPAVPSPSPSVRK [888]

## Tandem Peptides (underline):

CLNPVPAVPSPSPSVRK [888]

## OGTA076 (SEQ ID No: 12)

## Peptide Source: 1D-GE, Chronic lymphocytic leukaemia

MRTGWATPRRPAGLLMLLFWFFDLAEPSGRAANDPFTIVHGNTGKCIKPVYGWIVADDCDETEDKLWKW
VSQHRLFHLHSQKCLGLDITKSVNELRMFSCDSSAMLWWKCEHHSLYGAARYRLALKDGHGTAISNASD
VWKKGGSEESLCDQPYHEIYTRDGNSYGRPCEFPFLIDGTWHHDCILDEDHSGPWCATTLNYEYDRKWG
ICLKPENGCEDNWEKNEQFGSCYQFNTQTALSWKEAYVSCQNQGADLLSINSAAELTYLKEKEGIAKIF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAHAKCKAFSSDLISIHSLADVEVVVTKLHN
EDIKEEVWIGLKNINIPTLFQWSDGTEVTLTYWDENEPNVPYNKTPNCVSYLGELGQWKVQSCEEKLKY
VCKRKGEKLNDASSDKMCPPDEGWKRHGETCYKIYEDEVPFGCNCNLTITSRFEQEYLNDLMKKYDKSL
RKYFWTGLRDVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYKVFHAERIVRKRNWEEAERFCQALGAHLSSFSH
VDEIKEFLHFLTDQFSGQHWLWIGLNKRSPDLQGSWQWSDRTPVSTIIMPNEFQQDYDIRDCAAVKVFH
RPWRRGWHFYDDREFIYLRPFACDTKLEWVCQIPKGRTPKTPDWYNPDRAGIHGPPLIIEGSEYWFVAD
LHLNYEEAVLYCASNHSFLATITSFVGLKAIKNKIANISGDGQKWWIRISEWPIDDHFTYSRYPWHRFP
VTFGEECLYMSAKTWLIDLGKPTDCSTKLPFICEKYNVSSLEKYSPDSAAKVQCSEQWIPFQNKCFLKI
KPVSLTFSQASDTCHSYGGTLPSVLSQIEQDFITSLLPDMEATLWIGLRWTAYEKINKWTDNRELTYSN
FHPLLVSGRLRIPENFFEEESRYHCALILNLQKSPFTGTWNFCSCSERHFVSLCQKYSEVKSRQTLQNA
SETVKYLNNLYKIIPKTLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNRHMATTQDEVHTKCQKLNPKSHILSIRDEKENNFVLEQLLYFNYMASW
VMLGITYRNNSLMWFDKTPLSYTHWRAGRPTIKNEKFLAGLSTDGFWDIQTFKVIEEAVYFHQHSILAC
KIEMVDYKEEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSRLTYSSRCPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGRCSMLIASNETWKKVECEHGFGR
VVCKVPLGPDYTAIAIIVATLSILVLMGGLIWFLTQRIIRLIILAGFSSVRYAQGVNEDEIMLPSFIID

## Mass Match Peptides (bold):

AANDPFTIVHGNTGK [52]
CEHHSLYGAAR [102]
CSMLIASNETWKK [108]
EFIYLRPFACDTK [183]
ELTYSNFHPLLVSGR [208]
FCQALGAHLSSFSHVDEIK [245]
FEQEYLNDLMK [247]
FEQEYLNDLMKK [248]
FPVTFGEECLYMSAK [265]
GWHFYDDR [328]
HFVSLCQK [333]
IPENFFEEESR [385]
ISEWPIDDHFTYSR [392]
KVECEHGFGR [439]
KYFWTGLR [442]

```
LFHLHSQK [460]
NWEEAER [563]
SDQALHSFSEAK [623]
SPDLQGSWQWSDR [644]
TPLSYTHWR [718]
TPVSTIIMPNEFQQDYDIR [722]
VFHAER [759]
VFHRPWR [760]
VQCSEQWIPFQNK [793]
WVSQHR [838]
YFWTGLR [851]
YPWHR [866]
```

**Tandem Peptides (underline):**
```
ELTYSNFHPLLVSGR [208]
IPENFFEEESR [385]
SDQALHSFSEAK [623]
SPDLQGSWQWSDR [644]
TPLSYTHWR [718]
```

## Peptide Source: 1D-GE, Colorectal cancer

```
MRTGWATPRRPAGLLMLLFWFFDLAEPSGRAANDPFTIVHGNTGKCIKPVYGWIVADDCDETEDKLWKW
VSQHRLFHLHSQKCLGLDITKSVNELRMFSCDSSAMLWWKCEHHSLYGAARYRLALKDGHGTAISNASD
VWKKGGSEESLCDQPYHEIYTRDGNSYGRPCEFPFLIDGTWHHDCILDEDHSGPWCATTLNYEYDRKWG
ICLKPENGCEDNWEKNEQFGSCYQFNTQTALSWKEAYVSCQNQGADLLSINSAAELTYLKEKEGIAKIF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAHAKCKAFSSDLISIHSLADVEVVVTKLHN
EDIKEEVWIGLKNINIPTLFQWSDGTEVTLTYWDENEPNVPYNKTPNCVSYLGELGQWKVQSCEEKLKY
VCKRKGEKLNDASSDKMCPPDEGWKRHGETCYKIYEDEVPFGTNCNLTITSRFEQEYLNDLMKKYDKSL
RKYFWTGLRDVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYKVFHAERIVRKRNWEEAERFCQALGAHLSSFSH
VDEIKEFLHFLTDQFSGQHWLWIGLNKRSPDLQGSWQWSDR**TPVSTIIMPNEFQQDYDIR**DCAAVKVFH
RPWRRGWHFYDDREFIYLRPFACDTKLEWVCQIPKGRTPKTPDWYNPDRAGIHGPPLIIEGSEYWFVAD
LHLNYEEAVLYCASNHSFLATITSFVGLKAIKNKIANISGDGQKWWIRISEWPIDDHFTYSRYPWHRFP
VTFGEECLYMSAKTWLIDLGKPTDCSTKLPFICEKYNVSSLEKYSPDSAAKVQCSEQWIPFQNKCFLKI
KPVSLTFSQASDTCHSYGGTLPSVLSQIEQDFITSLLPDMEATLWIGLRWTAYEKINKWTDNRELTYSN
FHPLLVSGRLR**IPENFFEEESR**YHCALILNLQKSPFTGTWNFTSCSERHFVSLCQKYSEVKSRQTLQNA
SETVKYLNNLYKIIPKTLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNRHMATTQDEVHTKCQKLNPKSHILSIRDEKENNFVLEQLLYFNYMASW
VMLGITYRNNSLMWFDKTPLSYTHWRAGRPTIKNEKFLAGLSTDGFWDIQTFKVIEEAVYFHQHSILAC
KIEMVDYKEEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSRLTYSSRCPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGRCSMLIASNETWKKVECEHGFGR
VVCKVPLGPDYTAIAIIVATLSILVLMGGLIWFLFQRHRLHLAGFSSVRYAQGVNEDEIMLPSFHD
```

**Mass Match Peptides (bold):**
```
IPENFFEEESR [385]
TPVSTIIMPNEFQQDYDIR [722]
```
**Tandem Peptides (underline):**
```
IPENFFEEESR [385]
```

543

## Peptide Source: 1D-GE, Pancreatic cancer

MRTGWATPRRPAGLLMLLFWFFDLAEPSGR**AANDPFTIVHGNTGK**CIKPVYGWIVADDCDETEDKLWK**W
VSQHR**LFHLHSQKCLGLDITKSVNELRMFSCDSSAMLWWKCEHHSLYGAARYRLALK**DGHGTAISNASD
VWKK**GGSEESLCDQPYHEIYTRDGNSYGRPCEFPFLIDGTWHHDCILDEDHSGPWCATTLNYEYDRKWG
ICLKPENGCEDNWEKNEQFGSCYQFNTQTALSWKEAYVSCQNQGADLLSINSAAELTYLKEKEGIAKIF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAIIAKCK**AFSSDLISIHSLADVEVVVTK**LIIN
EDIKEEVWIGLKNINIPTLFQWSDGTEVTLTYWDENEPNVPYNKTPNCVSYLGELGQWKVQSCEEKLK**Y
VCKRK**GEKLNDASSDKMCPPDEGWK**RHGETCYK**IYEDEVPFGTNCNLTITSRFEQEYLNDLMKKYDKSL
RK**YFWTGLR**DVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYK**VFHAER**IVRKRNWEEAERFCQALGAHLSSFSH
VDEIKEFLHFLTDQFSGQHWLWIGLNKR**SPDLQGSWQWSDR**TPVSTIIMPNEFQQDYDIRDCAAVK**VFH
RPWR**RGWHFYDDR**EFIYLRPFACDTK**LEWVCQIPKGRTPKTPDWYNPDRAGIHGPPLIIEGSEYWFVAD
LHLNYEEAVLYCASNHSFLATITSFVGLKAIKNKIANISGDGQKWWIR**ISEWPIDDHFTYSRYPWHR**FP
VTFGEECLYMSAKTWLIDLGKPTDCSTKLPFICEKYNVSSLEKYSPDSAAK**VQCSEQWIPFQNK**CFLKI
KPVSLTFSQASDTCHSYGGTLPSVLSQIEQDFITSLLPDMEATLWIGLRWTAYEKINKWTDNR**ELTYSN
FHPLLVSGR**LR**IPENFFEEESR**YHCALILNLQKSPFTGTWNFTSCSERHFVSLCQKYSEVKSRQTLQNA
SETVKYLNNLYKIIPKTLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNR**HMATTQDEVHTK**CQKLNPKSHILSIRDEKENNFVLEQLLYFNYMASW
VMLGITYRNNSLMWFDK**TPLSYTHWR**AGRPTIKNEKFLAGLSTDGFWDIQTFK**VIEEAVYFHQHSILAC
KIEMVDYK**EEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSR**LTYSSR**CPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGR**CSMLIASNETWKK**VECEHGFGR
VVCKVPLGPDYTAIAIIVATLSILVLMGGLIWFLFQRHRLHLAGFSSVRYAQGVNEDEIMLPSFHD

## Mass Match Peptides (bold):

                AANDPFTIVHGNTGK [52]
                AFSSDLISIHSLADVEVVVTK [67]
                CSMLIASNETWKK [108]
                DGHGTAISNASDVWKK [122]
                EFIYLRPFACDTK [183]
                ELTYSNFHPLLVSGR [208]
                HMATTQDEVHTK [338]
                IEMVDYK [362]
                IPENFFEEESR [385]
                ISEWPIDDHFTYSR [392]
                KYFWTGLR [442]
                LTYSSR [499]
                RHGETCYK [604]
                SPDLQGSWQWSDR [644]
                TPLSYTHWR [718]
                VFHAER [759]
                VFHRPWR [760]
                VIEEAVYFHQHSILACK [767]
                VQCSEQWIPFQNK [793]
                WVSQHR [838]
                YFWTGLR [851]
                YPWHR [866]
                YVCKRK [875]

## Tandem Peptides (underline):

                ELTYSNFHPLLVSGR [208]
                IPENFFEEESR [385]
                SPDLQGSWQWSDR [644]

## Peptide Source: iTRAQ, Colorectal cancer

MRTGWATPRRPAGLLMLLFWFFDLAEPSGRAANDPFTIVHGNTGKCIKPVYGWIVADDCDETEDKLWKW
VSQHRLFHLHSQKCLGLDITKSVNELRMFSCDSSAMLWWKCEHHSLYGAARYR**LALK**DGHGTAISNASD
VWKKGGSEESLCDQPYHEIYTRDGNSYGRPCEFPFLIDGTWHHDCILDEDHSGPWCATTLNYEYDRKWG
ICLKPENGCEDNWEKNEQFGSCYQFNTQTALSWKEAYVSCQNQGADLLSINSAAELTYLKEK**EGIAK**IF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAHAKCKAFSSDLISIHSLADVEVVVTKLHN
EDIKEEVWIGLKNINIPTLFQWSDGTEVTLTYWDENEPNVPYNKTPNCVSYLGELGQWKVQSCEEKLKY
VCKRKGEKLNDASSDKMCPPDEGWKRHGETCYKIYEDEVPFGTNCNLTITSRFEQEYLNDLMKKYDKSL
RKYFWTGLRDVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYKVFHAERIVRKRNWEEAERFCQALGAHLSSFSH
VDEIKEFLHFLTDQFSGQHWLWIGLNKRSPDLQGSWQWSDRTPVSTIIMPNEFQQDYDIRDCAAVKVFH
RPWRRGWHFYDDREFIYLRPFACDTKLEWVCQIPKGRTPKTPDWYNPDRAGIHGPPLIIEGSEYWFVAD
LHLNYEEAVLYCASNHSFLATITSFVGLKAIKNKIANISGDGQKWWIRISEWPIDDHFTYSRYPWHRFP
VTFGEECLYMSAKTWLIDLGKPTDCSTKLPFICEKYNVSSLEKYSPDSAAKVQCSEQWIPFQNKCFLKI
KPVSLTFSQASDTCHSYGGTLPSVLSQIEQDFITSLLPDMEATLWIGLRWTAYEKINKWTDNRELTYSN
FHPLLVSGRLRIPENFFEEESRYHCALILNLQKSPFTGTWNFTSCSERHFVSLCQKYSEVKSRQTLQNA
SETVKYLNNLYK**IIPK**TLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNRHMATTQDEVHTKCQK**LNPK**SHILSIRDEKENNFVLEQLLYFNYMASW
VMLGITYRNNSLMWFDKTPLSYTHWRAGRPTIKNEKFLAGLSTDGFWDIQTFKVIEEAVYFHQHSILAC
KIEMVDYKEEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSRLTYSSRCPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGRCSMLIASNETWKKVECEHGFGR
VVCKVPLGPDYTAIAIIVATLSILVLMGGLIWFLFQRHRLHLAGFSSVRYAQGVNEDEIMLPSFHD

### Mass Match Peptides (bold):
EGIAK [898]
IIPK [921]
LALK [929]
LNPK [941]

### Tandem Peptides (underline):
EGIAK [898]
IIPK [921]
LALK [929]
LNPK [941]

## Peptide Source: iTRAQ, Kidney cancer

MRTGWATPRRPAGLLMLLFWFFDLAEPSGRAANDPFTIVHGNTGKCIKPVYGWIVADDCDETEDKLWKW
VSQHRLFHLHSQKCLGLDITKSVNELRMFSCDSSAMLWWKCEHHSLYGAARYR<u>LALK</u>DGHGTAISNASD
VWKKGGSEESLCDQPYHEIYTRDGNSYGRPCEFPFLIDGTWHHDCILDEDHSGPWCATTLNYEYDRKWG
ICLKPENGCEDNWEKNEQFGSCYQFNTQTALSWKEAYVSCQNQGADLLSINSAAELTYLKEK<u>EGIAK</u>IF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAHAKCKAFSSDLISIHSLADVEVVVTKLHN
EDIKEEVWIGLKNINIPTLFQWSDGTEVTLTYWDENEPNVPYNKTPNCVSYLGELGQWKVQSCEEKLKY
VCKRKGEKLNDASSDKMCPPDEGWKRHGETCYKIYEDEVPFGTNCNLTITSRFEQEYLNDLMKKYDKSL
RKYFWTGLRDVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYKVFHAERIVRKRNWEEAERFCQALGAHLSSFSH
VDEIKEFLHFLTDQFSGQHWLWIGLNKRSPDLQGSWQWSDRTPVSTIIMPNEFQQDYDIRDCAAVKVFH
RPWRRGWHFYDDREFIYLRPFACDTKLEWVCQIPKGRTPKTPDWYNPDRAGIHGPPLIIEGSEYWFVAD
LHLNYEEAVLYCASNHSFLATITSFVGLKAIKNKIANISGDGQKWWIRISEWPIDDHFTYSRYPWHRFP
VTFGEECLYMSAKTWLIDLGKPTDCSTKLPFICEKYNVSSLEKYSPDSAAKVQCSEQWIPFQNKCFLKI

```
KPVSLTFSQASDTCHSYGGTLPSVLSQIEQDFITSLLPDMEATLWIGLRWTAYEKINKWTDNRELTYSN
FHPLLVSGRLRIPENFFEEESRYHCALILNLQKSPFTGTWNFTSCSERHFVSLCQKYSEVKSRQTLQNA
SETVKYLNNLYKIIPKTLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNRHMATTQDEVHTKCQKLNPKSHILSIRDEKENNFVLEQLLYFNYMASW
VMLGITYRNNSLMWFDKTPLSYTHWRAGRPTIKNEKFLAGLSTDGFWDIQTFKVIEEAVYFHQHSILAC
KIEMVDYKEEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSRLTYSSRCPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGRCSMLIASNETWKKVECEHGFGR
VVCKVPLGPDYTAIAIIVATLSILVLMGGLIWFLTQRHRLHLAGFSSVRYAQGVNEDEIMLPSFHD
```

## Mass Match Peptides (bold):
```
EGIAK [898]
LALK [929]
LNPK [941]
YLNNLYK [1006]
```

## Tandem Peptides (underline):
```
EGIAK [898]
LALK [929]
LNPK [941]
YLNNLYK [1006]
```

## Peptide Source: iTRAQ, Liver cancer

```
MRTGWATPRRPAGLLMLLLFWFFDLAEPSGRAANDPFTIVHGNTGKCIKPVYGWIVADDCDETEDKLWKW
VSQHRLFHLHSQKCLGLDITKSVNELRMFSCDSSAMLWWKCEHHSLYGAARYRLALKDGHGTAISNASD
VWKKGGSEESLCDQPYHEIYTRDGNSYGRPCEFPTLIDGTWHHDCILDEDHSGPWCATTLNYEYDRKWG
ICLKPENGCEDNWEKNEQFGSCYQFNTQTALSWKEAYVSCQNQGADLLSINSAAELTYLKEKEGIAKIF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAHAKCKAFSSDLISIHSLADVEVVVTKLHN
EDIKEEVWIGLKNINIPTLFQWSDGTEVTLTYWDENEPNVPYNKTPNCVSYLGELGQWKVQSCEEKLKY
VCKRKGEKLNDASSDKMCPPDEGWKRHGETCYKIYEDEVPFGTNCNLTITSRFEQEYLNDLMKKYDKSL
RKYFWTGLRDVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYKVFHAERIVRKRNWEEAERFCQALGAHLSSFSH
VDEIKEFLHFLTDQFSGQHWLWIGLNKRSPDLQGSWQWSDRTPVSTIIMPNEFQQDYDIRDCAAVKVFH
RPWRRGWHFYDDREFIYLRPFACDTKLEWVCQIPKGRTPKTPDWYNPDRAGIHGPPLIIEGSEYWFVAD
LHLNYEEAVLYCASNHSFLATITSFVGLKAIKNKIANISGDGQKWWIRISEWPIDDHTTYSRYPWHRFP
VTFGEECLYMSAKTWLIDLGKPTDCSTKLPFICEKYNVSSLEKYSPDSAAKVQCSEQWIPFQNKCFLKI
KPVSLTFSQASDTCHSYGGTLPSVLSQIEQDFITSLLPDMEATLWIGLRWTAYEKINKWTDNRELTYSN
FHPLLVSGRLRIPENFFEEESRYHCALILNLQKSPFTGTWNFTSCSERHFVSLCQKYSEVKSRQTLQNA
SETVKYLNNLYKIIPKTLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNRHMATTQDEVHTKCQKLNPKSHILSIRDEKENNFVLEQLLYFNYMASW
VMLGITYRNNSLMWFDKTPLSYTHWRAGRPTIKNEKFLAGLSTDGFWDIQTFKVIEEAVYFHQHSILAC
KIEMVDYKEEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSRLTYSSRCPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGRCSMLIASNETWKKVECEHGFGR
VVCKVPLGPDYTAIAIIVATLSILVLMGGLIWFLTQRHRLHLAGFSSVRYAQGVNEDEIMLPSFHD
```

## Mass Match Peptides (bold):
```
YLNNLYK [1006]
```

## Tandem Peptides (underline):
```
YLNNLYK [1006]
```

## Peptide Source: iTRAQ, Non-small cell lung cancer

MRTGWATPRRPAGLLMLLFWFFDLAEPSGRAANDPFTIVHGNTGKCIKPVYGWIVADDCDETEDKLWKW
VSQHRLFHLHSQKCLGLDITKSVNELRMFSCDSSAMLWWKCEHHSLYGAARYRLALKDGHGTAISNASD
VWKKGGSEESLCDQPYHEIYTRDGNSYGRPCEFPFLIDGTWHHDCILDEDHSGPWCATTLNYEYDRKWG
ICLKPENGCEDNWEKNEQFGSCYQFNTQTALSWKEAYVSCQNQGADLLSINSAAELTYLKEKEGIAKIF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAHAKCKAFSSDLISIHSLADVEVVVTKLHN
EDIKEEVWIGLKNINIPTLFQWSDGTEVTLTYWDENEPNVPYNKTPNCVSYLGELGQWKVQSCEEKLKY
VCKRKGEKLNDASSDKMCPPDEGWKRHGETCYKIYEDEVPFGTNCNLTITSRFEQEYLNDLMKKYDKSL
RKYFWTGLRDVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYKVFHAERIVRKRNWEEAERFCQALGAHLSSFSH
VDEIKEFLHFLTDQFSGQHWLWIGLNKRSPDLQGSWQWSDRTPVSTIIMPNEFQQDYDIRDCAAVKVFH
RPWRRGWHFYDDREFIYLRPFACDTKLEWVCQIPKGRTPKTPDWYNPDRAGIHGPPLIIEGSEYWFVAD
LHLNYEEAVLYCASNHSFLATITSFVGLKAIKNKIANISGDGQKWWIRISEWPIDDHFTYSRYPWHRFP
VTFGEECLYMSAKTWLIDLGKPTDCSTKLPFICEKYNVSSLEKYSPDSAAKVQCSEQWIPFQNKCFLKI
KPVSLTFSQASDTCHSYGGTLPSVLSQIEQDFITSLLPDMEATLWIGLRWTAYEKINKWTDNRELTYSN
FHPLLVSGRLRIPENFFEEESRYHCALILNLQKSPFTGTWNFTSCSERHFVSLCQKYSEVKSRQTLQNA
SETVKYLNNLYKIIPKTLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNRHMATTQDEVHTKCQKLNPKSHILSIRDEKENNFVLEQLLYFNYMASW
VMLGITYRNNSLMWFDKTPLSYTHWRAGRPTIKNEKFLAGLSTDGFWDIQTFKVIEEAVYFHQHSILAC
KIEMVDYKEEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSRLTYSSRCPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGRCSMLIASNETWKKVECEHGFGR
VVCKVPLGPDYTAIAIIVATLSILVLMGGLIWFLFQRHRLHLAGFSSVRYAQGVNEDEIMLPSFHD

### Mass Match Peptides (bold):
IIPK [921]
LALK [929]
YLNNLYK [1006]

### Tandem Peptides (underline):
IIPK [921]
LALK [929]
YLNNLYK [1006]

## Peptide Source: iTRAQ, Small cell lung cancer

MRTGWATPRRPAGLLMLLFWFFDLAEPSGRAANDPFTIVHGNTGKCIKPVYGWIVADDCDETEDKLWKW
VSQHRLFHLHSQKCLGLDITKSVNELRMFSCDSSAMLWWKCEHHSLYGAARYRLALKDGHGTAISNASD
VWKKGGSEESLCDQPYHEIYTRDGNSYGRPCEFPFLIDGTWHHDCILDEDHSGPWCATTLNYEYDRKWG
ICLKPENGCEDNWEKNEQFGSCYQFNTQTALSWKEAYVSCQNQGADLLSINSAAELTYLKEKEGIAKIF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAHAKCKAFSSDLISIHSLADVEVVVTKLHN
EDIKEEVWIGLKNINIPTLFQWSDGTEVTLTYWDENEPNVPYNKTPNCVSYLGELGQWKVQSCEEKLKY
VCKRKGEKLNDASSDKMCPPDEGWKRHGETCYKIYEDEVPFGTNCNLTITSRFEQEYLNDLMKKYDKSL
RKYFWTGLRDVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYKVFHAERIVRKRNWEEAERFCQALGAHLSSFSH
VDEIKEFLHFLTDQFSGQHWLWIGLNKRSPDLQGSWQWSDRTPVSTIIMPNEFQQDYDIRDCAAVKVFH
RPWRRGWHFYDDREFIYLRPFACDTKLEWVCQIPKGRTPKTPDWYNPDRAGIHGPPLIIEGSEYWFVAD
LHLNYEEAVLYCASNHSFLATITSFVGLKAIKNKIANISGDGQKWWIRISEWPIDDHFTYSRYPWHRFP
VTFGEECLYMSAKTWLIDLGKPTDCSTKLPFICEKYNVSSLEKYSPDSAAKVQCSEQWIPFQNKCFLKI
KPVSLTFSQASDTCHSYGGTLPSVLSQIEQDFITSLLPDMEATLWIGLRWTAYEKINKWTDNRELTYSN
FHPLLVSGRLRIPENFFEEESRYHCALILNLQKSPFTGTWNFTSCSERHFVSLCQKYSEVKSRQTLQNA

SETVKYLNNLYKIIPKTLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNRHMATTQDEVHTKCQKLNPKSHILSIRDEKENNFVLEQLLYFNYMASW
VMLGITYRNNSLMWFDKTPLSYTHWRAGRPTIKNEKFLAGLSTDGFWDIQTFKVIEEAVYFHQHSILAC
KIEMVDYKEEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSRLTYSSRCPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGRCSMLIASNETWKKVECEHGFGR
VVCKVPLGPDYTAIAIIVATLSILVLMGGLIWFLTQRHRLHLAGFSSVRYAQGVNEDEIMLPSFHD

**Mass Match Peptides (bold):**
EGIAK [898]

**Tandem Peptides (underline):**
EGIAK [898]


## Peptide Source: iTRAQ, Ovarian cancer

MRTGWATPRRPAGLLMLLFWFFDLAEPSGRAANDPFTIVHGNTGKCIKPVYGWIVADDCDETEDKLWKW
VSQHRLFHLHSQKCLGLDITKSVNELRMFSCDSSAMLWWKCEHHSLYGAARYRLALKDGHGTAISNASD
VWKKGGSEESLCDQPYHEIYTRDGNSYGRPCEFPTLIDGTWHHDCILDEDHSGPWCATTLNYEYDRKWG
ICLKPENGCEDNWEKNEQFGSCYQFNTQTALSWKEAYVSCQNQGADLLSINSAAELTYLKEKEGIAKIF
WIGLNQLYSARGWEWSDHKPLNFLNWDPDRPSAPTIGGSSCARMDAESGLWQSFSCEAQLPYVCRKPLN
NTVELTDVWTYSDTRCDAGWLPNNGFCYLLVNESNSWDKAHAKCKAFSSDLISIHSLADVEVVVTKLHN
EDIKEEVWIGLKNINIPTLFQWSDGTEVTLTYWDENEPNVPYNKTPNCVSYLGELGQWKVQSCEEKLKY
VCKRKGEKLNDASSDKMCPPDEGWKRHGETCYKIYEDEVPFGTNCNLTITSRFEQEYLNDLMKKYDKSL
RKYFWTGLRDVDSCGEYNWATVGGRRRAVTFSNWNFLEPASPGGCVAMSTGKSVGKWEVKDCRSFKALS
ICKKMSGPLGPEEASPKPDDPCPEGWQSFPASLSCYKVFHAERIVRKRNWEEAERFCQALGAHLSSFSH
VDEIKEFLHFLTDQFSGQHWLWIGLNKRSPDLQGSWQWSDRTPVSTIIMPNEFQQDYDIRDCAAVKVFH
RPWRRGWHFYDDREFIYLRPFACDTKLEWVCQIPKGRTPKTPDWYNPDRAGIHGPPLIIEGSEYWFVAD
LHLNYEEAVLYCASNHSFLATITSFVGLKAIKNKIANISGDGQKWWIRISEWPIDDHTTYSRYPWHRFP
·VTFGEECLYMSAKTWLIDLGKPTDCSTKLPFICEKYNVSSLEKYSPDSAAKVQCSEQWIPFQNKCFLKI
KPVSLTFSQASDTCHSYGGTLPSVLSQIEQDFITSLLPDMEATLWIGLRWTAYEKINKWTDNRELTYSN
FHPLLVSGRLRIPENFFEEESRYHCALILNLQKSPFTGTWNFTSCSERHFVSLCQKYSEVKSRQTLQNA
SETVKYLNNLYKIIPKTLTWHSAKRECLKSNMQLVSITDPYQQAFLSVQALLHNSSLWIGLFSQDDELN
FGWSDGKRLHFSRWAETNGQLEDCVVLDTDGFWKTVDCNDNQPGAICYYSGNETEKEVKPVDSVKCPSP
VLNTPWIPFQNCCYNFIITKNRHMATTQDEVHTKCQKLNPKSHILSIRDEKENNFVLEQLLYFNYMASW
VMLGITYRNNSLMWFDKTPLSYTHWRAGRPTIKNEKFLAGLSTDGFWDIQTFKVIEEAVYFHQHSILAC
KIEMVDYKEEHNTTLPQFMPYEDGIYSVIQKKVTWYEALNMCSQSGGHLASVHNQNGQLFLEDIVKRDG
FPLWVGLSSHDGSESSFEWSDGSTFDYIPWKGQTSPGNCVLLDPKGTWKHEKCNSVKDGAICYKPTKSK
KLSRLTYSSRCPAAKENGSRWIQYKGHCYKSDQALHSFSEAKKLCSKHDHSATIVSIKDEDENKFVSRL
MRENNNITMRVWLGLSQHSVDQSWSWLDGSEVTFVKWENKSKSGVGRCSMLIASNETWKKVECEHGFGR
VVCKVPLGPDYTAIAIIVATLSILVLMGGLIWFLTQRHRLHLAGFSSVRYAQGVNEDEIMLPSFHD

**Mass Match Peptides (bold):**
LALK [929]
YLNNLYK [1006]

**Tandem Peptides (underline):**
LALK [929]
YLNNLYK [1006]


## OGTA085 (SEQ ID No: 13)

## Peptide Source: 1D-GE, Lung cancer

MGQPPAEEAEQPGALAREFLAAMEPEPAPAPAPEEWLDILGNGLLRKKTLVPGPPGSSRPVKGQVVTVH
LQTSLENGTRVQEEPELVFTLGDCDVIQALDLSVPLMDVGETAMVTADSKYCYGPQGRSPYIPPHAALC
LEVTLK**TAVDGPDLEMLTGQER**VALANRKRECGNAHYQRADFVLAANSYDLAIKAITSSAKVDMTFEEE
AQLLQLKVKCLNNLAASQLKLDHYRAALRSCSLVLEHQPDNIKALFRKGK<u>**VLAQQGEYSEAIPILR**</u>AAL
KLEPSNK<u>TIIIAELSK</u>LVKKHAAQR<u>**STETALYRKMLGNPSR**</u>LPAKCPGKGAWSIPWKWLFGATAVALGGV
ALSVVIAARN

### Mass Match Peptides (bold):
    KMLGNPSR [427]
    STETALYR [655]
    TAVDGPDLEMLTGQER [677]
    VLAQQGEYSEAIPILR [773]

### Tandem Peptides (underline):
    TIIIAELSK [700]
    VLAQQGEYSEAIPILR [773]


## Peptide Source: 1D-GE, Melanoma

<u>MGQPPAEEAEQPGALAR</u>EFLAAMEPEPAPAPAPEEWLDILGNGLLRKKTLVPGPPGSSRPVK<u>GQVVTVH</u>
<u>LQTSLENGTR</u>VQEEPELVFTLGDCDVIQALDLSVPLMDVGETAMVTADSKYCYGPQGRSPYIPPHAALC
LEVTLK**TAVDGPDLEMLTGQER**VALANRKRECGNAHYQR<u>**ADFVLAANSYDLAIKA**</u>ITSSAK<u>VDMTFEEE</u>
<u>AQLLQLK</u>VKCLNNLAASQLK<u>LDHYR</u>AALR<u>SCSLVLEHQPDNIK</u>ALFRKGKVLAQQGEYSEAIPILRAAL
KLEPSNKTIHAELSKLVKKHAAQR<u>**STETALYRKMLGNPSR**</u>LPAKCPGKGAWSIPWKWLFGATAVALGGV
ALSVVIAARN

### Mass Match Peptides (bold):
    ADFVLAANSYDLAIK [60]
    GQVVTVHLQTSLENGTR [313]
    KMLGNPSR [427]
    LDHYR [447]
    MGQPPAEEAEQPGALAR [516]
    SCSLVLEHQPDNIK [620]
    STETALYR [655]
    STETALYRK [656]
    TAVDGPDLEMLTGQER [677]
    VDMTFEEEAQLLQLK [748]

### Tandem Peptides (underline):
    TAVDGPDLEMLTGQER [677]


## Peptide Source: 1D-GE, Retinoblastoma

<u>MGQPPAEEAEQPGALAR</u>EFLAAMEPEPAPAPAPEEWLDILGNGLLRKKTLVPGPPGSSRPVKGQVVTVH
LQTSLENGTRVQEEPELVFTLGDCDVIQALDLSVPLMDVGETAMVTADSKYCYGPQGRSPYIPPHAALC
LEVTLKTAVDGPDLEMLTGQERVALANRKRECGNAIIYQRADFVLAANSYDLAIKAITSSAKVDMTFEEE
AQLLQLKVKCLNNLAASQLKLDHYRAALRSCSLVLEHQPDNIKALFRKGK<u>VLAQQGEYSEAIPILR</u>AAL
KLEPSNKTIHAELSKLVKKHAAQRSTETALYR**KMLGNPSR**LPAKCPGKGAWSIPWKWLFGATAVALGGV
ALSVVIAARN

### Mass Match Peptides (bold):
    KMLGNPSR [427]
    MGQPPAEEAEQPGALAR [516]

VLAQQGEYSEAIPILR [773]

**Tandem Peptides (underline):**

VLAQQGEYSEAIPILR [773]

## Peptide Source: iTRAQ, Colorectal cancer

MGQPPAEEAEQPGALAREFLAAMEPEPAPAPAPEEWLDILGNGLLRKKTLVPGPPGSSRPVKGQVVTVH
LQTSLENGTRVQEEPELVFTLGDCDVIQALDLSVPLMDVGETAMVTADSKYCYGPQGRSPYIPPHAALC
LEVTLKTAVDGPDLEMLTGQERVALANRKRECGNAHYQRADFVLAANSYDLAIKAITSSAKVDMTFEEE
AQLLQLKVKCLNNLAASQLKLDHYRAALRSCSLVLEHQPDNIKALFRKGKVLAQQGEYSEAIPILRAAL
KLEPSNKTIHAELSKLVKKHAAQRSTETALYR**KMLGNPSR**LPAKCPGKGAWSIPWKWLFGATAVALGGV
ALSVVIAARN

**Mass Match Peptides (bold):**

KMLGNPSR [427]

**Tandem Peptides (underline):**

KMLGNPSR [427]

## Peptide Source: iTRAQ, Non-small cell lung cancer

MGQPPAEEAEQPGALAREFLAAMEPEPAPAPAPEEWLDILGNGLLRKKTLVPGPPGSSRPVKGQVVTVH
LQTSLENGTRVQEEPELVFTLGDCDVIQALDLSVPLMDVGETAMVTADSKYCYGPQGRSPYIPPHAALC
LEVTLKTAVDGPDLEMLTGQERVALANRKRECGNAIIYQRADFVLAANSYDLAIKAITSSAKVDMTFEEE
AQLLQLKVKCLNNLAASQLKLDHYRAALRSCSLVLEHQPDNIKALFRKGKVLAQQGEYSEAIPILRAAL
KLEPSNKTIHAELSKLVKKHAAQRSTETALYR**KMLGNPSR**LPAKCPGKGAWSIPWKWLFGATAVALGGV
ALSVVIAARN

**Mass Match Peptides (bold):**

KMLGNPSR [427]

**Tandem Peptides (underline):**

KMLGNPSR [427]

## OGTA087 (SEQ ID No: 14)

## Peptide Source: 1D-GE, B-cell non-Hodgkin's lymphoma

MATAAYEQLK**LHITPEK**FYVEACDDGADDVLTIDRVSTEVTLAVKKDVPPSAVTRPIFGILGTIHLVAG
NYLIVITKKIKVGEFFSHVVWK**ATDFDVLSYKKTMLHLTDIQLQDNK**TFLAMLNHVLNVDGFYFSTTYD
LTHTLQR**LSNTSPEFQEMSLLER**ADQR**FVWNGHLLR**ELSAQPEVHRFALPVLHGFITMHSCSINGKYFD
WILISRRSCFRAGVRYYVRGIDSEGHAANFVETEQIVHYNGSKASFVQTRGSIPVFWSQRPNLK**YKPLP
QISK**VANHMDGFQR HFDSQVIIYGK**QVIINLINQK**GSEKPLEQTFATMVSSLGSGMMR**YIAFDFHKEC**K
NMRWDRLSILLDQVAEMQDELSYFLVDSAGQVVANQEGVFRSNCMDCLDRTNVIQSLLARRSLQAQLQR
**LGVLHVGQK**LEEQDEFEKIYKNAWADNANACAK**QYAGTGALK**CDFTRTGKRTHLGLIMDGWNSMIRYYK
**NNFSDGFR**QDSIDLFLGNYSVDELESHSPLSVPRDWKFLALPIIMVVAFSMCIICLLMAGDTWIETLAY
VLFWGVASIGTFFIILYNGKDFVDAPRLVQKEKID

**Mass Match Peptides (bold):**

ATDFDVLSYKK [89]
FVWNGHLLR [279]

```
KTMLHLTDIQLQDNK [438]
LHITPEK [466]
LSNTSPEFQEMSLLER [488]
NNFSDGFR [550]
QYAGTGALK [593]
VANHMDGFQR [744]
```

**Tandem Peptides (underline):**

```
LGVLHVGQK [464]
NNFSDGFR [550]
QVIINLINQK [590]
YIAFDFHK [854]
YKPLPQISK [856]
```

## Peptide Source: 1D-GE, Breast cancer

```
MATAAYEQLKLHITPEKFYVEACDDGADDVLTIDRVSTEVILAVKKDVPPSAVTRPIFGILGTIHLVAG
NYLIVITKKIKVGEFFSHVVWKATDFDVLSYKKTMLHLTDIQLQDNKTFLAMLNHVLNVDGFYFSTTYD
LTHTLQRLSNTSPEFQEMSLLERADQRFVWNGHLLRELSAQPEVHRFALPVLHGFITMHSCSINGKYFD
WILISRRSCFRAGVRYYVRGIDSEGHAANFVETEQIVHYNGSKASFVQTRGSIPVFWSQRPNLKYKPLP
QISKVANHMDGFQRHFDSQVIIYGKQVIINLINQKGSEKPLEQTFATMVSSLGSGMMRYIAFDFHKECK
NMRWDRLSILLDQVAEMQDELSYFLVDSAGQVVANQEGVFRSNCMDCLDRTNVIQSLLARRSLQAQLQR
LGVLHVGQKLEEQDEFEKIYKNAWADNANACAKQYAGTGALKTDFTRTGKRTHLGLIMDGWNSMIRYYK
NNFSDGFRQDSIDLFLGNYSVDELESHSPLSVPRDWKFLALPIIMVVAFSMCIICLLMAGDTWTETLAY
VLFWGVASIGTFFIILYNGKDFVDAPRLVQKEKID
```

**Mass Match Peptides (bold):**

```
ATDFDVLSYKK [89]
FVWNGHLLR [279]
```

**Tandem Peptides (underline):**

```
TNVIQSLLAR [714]
```

## Peptide Source: 1D-GE, Colorectal cancer

```
MATAAYEQLKLHITPEKFYVEACDDGADDVLTIDRVSTEVILAVKKDVPPSAVTRPIFGILGTIHLVAG
NYLIVITKKIKVGEFFSHVVWKATDFDVLSYKKTMLHLTDIQLQDNKTFLAMLNHVLNVDGFYFSTTYD
LTHTLQRLSNTSPEFQEMSLLERADQRFVWNGHLLRELSAQPEVHRFALPVLHGFITMHSCSINGKYFD
WILISRRSCFRAGVRYYVRGIDSEGHAANFVETEQIVHYNGSKASFVQTRGSIPVFWSQRPNLKYKPLP
QISKVANHMDGFQRHFDSQVIIYGKQVIINLINQKGSEKPLEQTFATMVSSLGSGMMRYIAFDFHKECK
NMRWDRLSILLDQVAEMQDELSYFLVDSAGQVVANQEGVFRSNCMDCLDRTNVIQSLLARRSLQAQLQR
LGVLHVGQKLEEQDEFEKIYKNAWADNANACAKQYAGTGALKTDFTRTGKRTHLGLIMDGWNSMIRYYK
NNFSDGFRQDSIDLFLGNYSVDELESHSPLSVPRDWKFLALPIIMVVAFSMCIICLLMAGDTWTETLAY
VLFWGVASIGTFFIILYNGKDFVDAPRLVQKEKID
```

**Mass Match Peptides (bold):**

```
LHITPEK [466]
```

**Tandem Peptides (underline):**

```
YIAFDFHK [854]
```

## Peptide Source: 1D-GE, Gastric cancer

MATAAYEQLKLHITPEKFYVEACDDGADDVLTIDRVSTEVTLAVKKDVPPSAVTRPIFGILGTIHLVAG
NYLIVITKKIKVGEFFSHVVWKATDFDVLSYKKTMLHLTDIQLQDNKTFLAMLNHVLNVDGFYFSTTYD
LTHTLQRLSNTSPEFQEMSLLERADQRFVWNGHLLRELSAQPEVHRFALPVLHGFITMHSCSINGKYFD
WILISRRSCFRAGVRYYVRGIDSEGHAANFVETEQIVHYNGSKASFVQTRGSIPVFWSQRPNLKYKPLP
QISKVANHMDGFQRHFDSQVIIYGKQVIINLINQKGSEKPLEQTFATMVSSLGSGMMRYIAFDFHKECK
NMRWDRLSILLDQVAEMQDELSYFLVDSAGQVVANQEGVFRSNCMDCLDR<u>TNVIQSLLAR</u>RSLQAQLQR
LGVLHVGQKLEEQDEFEKIYKNAWADNANACAKQYAGTGALKTDFTRTGKRTHLGLIMDGWNSMIRYYK
NNFSDGFRQDSIDLFLGNYSVDELESHSPLSVPRDWKFLALPTIMVVAFSMCIICLLMAGDTWTETLAY
VLFWGVASIGTFFIILYNGKDFVDAPRLVQKEKID

**Mass Match Peptides (bold):**
**Tandem Peptides (underline):**
TNVIQSLLAR [714]

## Peptide Source: 1D-GE, Lung cancer

MATAAYEQLK**LHITPEK**FYVEACDDGADDVLTIDR**VSTEVTLAVK**KDVPPSAVTRPIFGILGTIHLVAG
NYLIVITKKIKVGEFFSHVVWK<u>ATDFDVLSYK</u>KTMLHLTDIQLQDNKTFLAMLNHVLNVDGFYFSTTYD
LTHTLQR**LSNTSPEFQEMSLLER**ADQRFVWNGHLLRELSAQPEVHRFALPVLHGFITMHSCSINGK**YFD**
**WILISRR**SCFRAGVRYYVRGIDSEGHAANFVETEQIVHYNGSKASFVQTRGSIPVFWSQRPNLK**YKPLP**
QISK**VANHMDGFQRHFDSQVIIYGK**QVIINLINQK**GSEKPLEQTFATMVSSLGSGMMRYIAFDFHKE**CK
NMRWDRLSILLDQVAEMQDELSYFLVDSAGQVVANQEGVFRSNCMDCLDR<u>TNVIQSLLAR</u>RSLQAQLQR
LGVLHVGQK<u>LEEQDEFEK</u>IYKNAWADNANACAK**QYAGTGALK**TDFTRTGK**RTHLGLIMDGWNSMIR**YYK
**NNFSDGFR**QDSIDLFLGNYSVDELESHSPLSVPRDWKFLALPTIMVVAFSMCIICLLMAGDTWTETLAY
VLFWGVASIGTFFIILYNGKDFVDAPRLVQKEKID

**Mass Match Peptides (bold):**
GSEKPLEQTFATMVSSLGSGMMR [314]
HFDSQVIIYGK [332]
LHITPEK [466]
LSNTSPEFQEMSLLER [488]
NNFSDGFR [550]
QYAGTGALK [593]
RTHLGLIMDGWNSMIR [615]
THLGLIMDGWNSMIR [696]
VANHMDGFQR [744]
VSTEVTLAVK [797]
YFDWILISR [848]
YFDWILISRR [849]
YIAFDFHK [854]

**Tandem Peptides (underline):**
ATDFDVLSYK [88]
LEEQDEFEK [453]
NNFSDGFR [550]
TNVIQSLLAR [714]
YIAFDFHK [854]
YKPLPQISK [856]

## Peptide Source: 1D-GE, Lymphoid leukaemia, unspecified

MATAAYEQLKLHITPEKFYVEACDDGADDVLTIDRVSTEVTLAVKKDVPPSAVTRPIFGILGTIHLVAG
NYLIVITKKIKVGEFFSHVVWKATDFDVLSYKKTMLHLTDIQLQDNKTFLAMLNHVLNVDGFYFSTTYD

LTHTLQRLSNTSPEFQEMSLLERADQRFVWNGHLLRELSAQPEVHRFALPVLHGFITMHSCSINGKYFD
WILISRRSCFRAGVRYYVRGIDSEGHAANFVETEQIVHYNGSKASFVQTRGSIPVFWSQRPNLKYKPLP
QISKVANHMDGFQRHFDSQVIIYGKQVIINLINQKGSEKPLEQTFATMVSSLGSGMMRYIAFDFHKECK
NMRWDRLSILLDQVAEMQDELSYFLVDSAGQVVANQEGVFRSNCMDCLDRTNVIQSLLARRSLQAQLQR
LGVLHVGQKLEEQDEFEKIYKNAWADNANACAKQYAGTGALKTDFTRTGKRTHLGLIMDGWNSMIRYYK
NNFSDGFRQDSIDLFLGNYSVDELESHSPLSVPRDWKFLALPIIMVVAFSMCIICLLMAGDTWIETLAY
VLFWGVASIGTFFIILYNGKDFVDAPRLVQKEKID

## Mass Match Peptides (bold):

LSNTSPEFQEMSLLER [488]
NNFSDGFR [550]
THLGLIMDGWNSMIR [696]

## Tandem Peptides (underline):

NNFSDGFR [550]
TNVIQSLLAR [714]
YKPLPQISK [856]

## Peptide Source: 1D-GE, Ovarian cancer

MATAAYEQLKLHITPEKFYVEACDDGADDVLTIDRVSTEVTLAVKKDVPPSAVTRPIFGILGTIHLVAG
NYLIVITKKIKVGEFFSHVVWKATDFDVLSYKKTMLHLTDIQLQDNKTFLAMLNHVLNVDGFYFSTTYD
LTHTLQRLSNTSPEFQEMSLLERADQRFVWNGHLLRELSAQPEVHRFALPVLHGFITMHSCSINGKYFD
WILISRRSCFRAGVRYYVRGIDSEGHAANFVETEQIVHYNGSKASFVQTRGSIPVFWSQRPNLKYKPLP
QISKVANHMDGFQRHFDSQVIIYGKQVIINLINQKGSEKPLEQTFATMVSSLGSGMMRYIAFDFHKECK
NMRWDRLSILLDQVAEMQDELSYFLVDSAGQVVANQEGVFRSNCMDCLDRTNVIQSLLARRSLQAQLQR
LGVLHVGQKLEEQDEFEKIYKNAWADNANACAKQYAGTGALKTDFTRTGKRTHLGLIMDGWNSMIRYYK
NNFSDGFRQDSIDLFLGNYSVDELESHSPLSVPRDWKFLALPIIMVVAFSMCIICLLMAGDTWIETLAY
VLFWGVASIGTFFIILYNGKDFVDAPRLVQKEKID

## Mass Match Peptides (bold):

ATDFDVLSYKK [89]
FVWNGHLLR [279]
GSIPVFWSQRPNLK [316]
KTMLHLTDIQLQDNK [438]
RTHLGLIMDGWNSMIR [615]
TMLHLTDIQLQDNK [707]

## Tandem Peptides (underline):

HFDSQVIIYGK [332]
LEEQDEFEK [453]

## OGTA088 (SEQ ID No: 15)

## Peptide Source: 1D-GE, Breast cancer

MSDFDSNPFADPDLNNPFKDPSVTQVTRNVPPGLDEYNPFSDSRTPPPGGVKMPNVPNTQPAIMKPTEE
HPAYTQIAKEHAIAQAELLKRQEELERKAAELDRREREMQNLSQHGRKNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIAALFTASAV
ISLVMFKKVHGLYRTTGASFEKAQQEFATGVMSNKTVQTAAANAASTAASSAAQNAFKGNQI

## Mass Match Peptides (bold):

DPSVTQVTR [149]

EMQNLSQHGR [211]
NVPPGLDEYNPFSDSR [560]
RQEELER [612]

**Tandem Peptides (underline):**
DPSVTQVTR [149]
EHALAQAELLK [190]
NVPPGLDEYNPFSDSR [560]


## Peptide Source: 1D-GE, Gastric cancer

```
MSDFDSNPFADPDLNNPFKDPSVTQVTRNVPPGLDEYNPFSDSRTPPPGGVKMPNVPNTQPAIMKPTEE
HPAYTQIAKEHALAQAELLKRQEELERKAAELDRREREMQNLSQHGRKNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFKKVHGLYRTTGASFEKAQQEFATGVMSNKTVQTAAANAASTAASSAAQNAFKGNQI
```

**Mass Match Peptides (bold):**
EMQNLSQHGR [211]

**Tandem Peptides (underline):**
EHALAQAELLK [190]


## Peptide Source: 1D-GE, Glioblastoma

```
MSDFDSNPFADPDLNNPFKDPSVTQVTRNVPPGLDEYNPFSDSRTPPPGGVKMPNVPNTQPAIMKPTEE
HPAYTQIAKEHALAQAELLKRQEELERKAAELDRREREMQNLSQHGRKNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFKKVHGLYRTTGASFEKAQQEFATGVMSNKTVQTAAANAASTAASSAAQNAFKGNQI
```

**Mass Match Peptides (bold):**
AQQEFATGVMSNK [84]
EHALAQAELLK [190]
EMQNLSQHGR [211]
KAAELDR [405]
MPNVPNTQPAIMKPTEEHPAYTQIAK [522]
NVPPGLDEYNPFSDSR [560]
QEELER [566]
RQEELER [612]
TPPPGGVK [721]
TVQTAAANAASTAASSAAQNAFK [737]
VHGLYR [766]

**Tandem Peptides (underline):**
EHALAQAELLK [190]
NVPPGLDEYNPFSDSR [560]
TVQTAAANAASTAASSAAQNAFK [737]


## Peptide Source: 1D-GE, Hepatocellular carcinoma

```
MSDFDSNPFADPDLNNPFKDPSVTQVTRNVPPGLDEYNPFSDSRTPPPGGVKMPNVPNTQPAIMKPTEE
HPAYTQIAKEHALAQAELLKRQEELERKAAELDRREREMQNLSQHGRKNNWPPLPSNFPVGPCFYQDFS
```

VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFKKVHGLYRTTGASFEKAQQEFATGVMSNKTVQTAAANAASTAASSAAQNAFKGNQI

**Mass Match Peptides (bold):**
  DPSVTQVTR [149]
  EHALAQAELLK [190]
  EMQNLSQHGR [211]
  EMQNLSQHGRK [212]
  KAAELDRR [406]
  MPNVPNTQPAIMKPTEEHPAYTQIAK [522]
  NVPPGLDEYNPFSDSR [560]
  RQEELER [612]
  RQEELERK [613]

**Tandem Peptides (underline):**
  EHALAQAELLK [190]
  NVPPGLDEYNPFSDSR [560]

## Peptide Source: 1D-GE, Lung cancer

MSDFDSNPFADPDLNNPFKDPSVTQVTR**NVPPGLDEYNPFSDSRTPPPGGVKMPNVPNTQPAIMKPTEE**
**HPAYTQIAK**EHALAQAELLK**RQEELER**KAAELDRRER**EMQNLSQHGR**KNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFK**KVHGLYR**TTGASFEK**AQQEFATGVMSNKTVQTAAANAASTAASSAAQNAFK**GNQI

**Mass Match Peptides (bold):**
  AQQEFATGVMSNK [84]
  EMQNLSQHGR [211]
  KVHGLYR [441]
  MPNVPNTQPAIMKPTEEHPAYTQIAK [522]
  NVPPGLDEYNPFSDSR [560]
  QEELER [566]
  RQEELER [612]
  TPPPGGVK [721]
  TVQTAAANAASTAASSAAQNAFK [737]

**Tandem Peptides (underline):**
  EHALAQAELLK [190]
  NVPPGLDEYNPFSDSR [560]

## Peptide Source: 1D-GE, Lymphoid leukaemia, unspecified

MSDFDSNPFADPDLNNPFKDPSVTQVTR**NVPPGLDEYNPFSDSR**TPPPGGVKMPNVPNTQPAIMKPTEE
HPAYTQIAK_EHALAQAELLK_RQEELERKAAELDRRER**EMQNLSQHGR**KNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFKKVHGLYRTTGASFEKAQQEFATGVMSNKTVQTAAANAASTAASSAAQNAFKGNQI

**Mass Match Peptides (bold):**
  EMQNLSQHGR [211]
  NVPPGLDEYNPFSDSR [560]

**Tandem Peptides (underline):**

     EHALAQAELLK [190]

## Peptide Source: 1D-GE, Pancreatic cancer

MSDFDSNPFADPDLNNPFK**DPSVTQVTR**NVPPGLDEYNPFSDSRTPPPGGVKMPNVPNTQPAIMKPTEE
HPAYTQIAKEHALAQAELLKRQEELERKAAELDRREREMQNLSQHGRKNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFK**KVHGLYR**TTGASFEKAQQEFATGVMSNK**TVQTAAANAASTAASSAAQNAFK**GNQI

**Mass Match Peptides (bold):**

     DPSVTQVTR [149]
     EMQNLSQHGR [211]
     KAAELDRR [406]
     KVHGLYR [441]
     NVPPGLDEYNPFSDSR [560]
     RQEELER [612]
     TVQTAAANAASTAASSAAQNAFK [737]

**Tandem Peptides (underline):**

     DPSVTQVTR [149]
     EHALAQAELLK [190]
     EMQNLSQHGR [211]
     NVPPGLDEYNPFSDSR [560]

## Peptide Source: 1D-GE, Renal cell cancer

MSDFDSNPFADPDLNNPFKDPSVTQVTR**NVPPGLDEYNPFSDSR**TPPPGGVKMPNVPNTQPAIMKPTEE
HPAYTQIAKEHALAQAELLKRQEELERKAAELDRRER**EMQNLSQHGR**KNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFK**KVHGLYR**TTGASFEKAQQEFATGVMSNKTVQTAAANAASTAASSAAQNAFKGNQI

**Mass Match Peptides (bold):**

     EMQNLSQHGR [211]
     KVHGLYR [441]
     NVPPGLDEYNPFSDSR [560]

**Tandem Peptides (underline):**

     EHALAQAELLK [190]
     NVPPGLDEYNPFSDSR [560]

## Peptide Source: 1D-GE, Retinoblastoma

MSDFDSNPFADPDLNNPFKDPSVTQVTRNVPPGLDEYNPFSDSRTPPPGGVKMPNVPNTQPAIMKPTEE
HPAYTQIAKEHALAQAELLKRQEELERKAAELDRREREMQNLSQHGRKNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFKKVHGLYRTTGASFEKAQQEFATGVMSNKTVQTAAANAASTAASSAAQNAFKGNQI

**Mass Match Peptides (bold):**

EHALAQAELLK [190]
EMQNLSQHGR [211]
NVPPGLDEYNPFSDSR [560]
RQEELER [612]
TVQTAAANAASTAASSAAQNAFK [737]
VHGLYR [766]

**Tandem Peptides (underline):**
EHALAQAELLK [190]
NVPPGLDEYNPFSDSR [560]

## Peptide Source: iTRAQ, Colorectal cancer

MSDFDSNPFADPDLNNPFKDPSVTQVTRNVPPGLDEYNPFSDSRTPPPGGVKMPNVPNTQPAIMKPTEE
HPAYTQIAKEHALAQAELLKRQEELERKAAELDRREREMQNLSQHGRKNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFKKVHGLYRTTGASFEKAQQEFATGVMSNKTVQTAAANAASTAASSAAQNAFKGNQI

**Mass Match Peptides (bold):**
RQEELERK [613]
**Tandem Peptides (underline):**
RQEELERK [613]

## Peptide Source: iTRAQ, Non-small cell lung cancer

MSDFDSNPFADPDLNNPFKDPSVTQVTRNVPPGLDEYNPFSDSRTPPPGGVKMPNVPNTQPAIMKPTEE
HPAYTQIAKEHALAQAELLKRQEELERKAAELDRREREMQNLSQHGRKNNWPPLPSNFPVGPCFYQDFS
VDIPVEFQKTVKLMYYLWMFHAVTLFLNIFGCLAWFCVDSARAVDFGLSILWFLLFTPCSFVCWYRPLY
GAFRSDSSFRFFVFFFVYICQFAVHVLQAAGFHNWGNCGWISSLTGLNQNIPVGIMMIIIAALFTASAV
ISLVMFKKVHGLYRTTGASFEKAQQEFATGVMSNKTVQTAAANAASTAASSAAQNAFKGNQI

**Mass Match Peptides (bold):**
RQEELERK [613]
**Tandem Peptides (underline):**
RQEELERK [613]

## OGTA089 (SEQ ID No: 16)

## Peptide Source: 1D-GE, Breast cancer

MEKRLGVKPNPASWILSGYYWQTSAKWLRSLYLFYTCFCFSVLWLSTDASESRCQQGKTQFGVGLRSGG
ENHLWLLEGTPSLQSCWAACCQDSACHVFWWLEGMCIQADCSRPQSCRAFRTHSSNSMLVFLKKFQTAD
DLGFLPEDDVPHLLGLGWNWASWRQSPPRAALRPAVSSSDQQSLIRKLQKRGSPSDVVTPIVTQHSKVN
DSNELGGLTTSGSAEVHKAITISSPLTTDLTAELSGGPKNVSVQPEISEGLATTPSTQQVKSSEKTQIA
VPQPVAPSYSYATPTPQASFQSTSAPYPVIKELVVSAGESVQITLPKNEVQLNAYVLQEPPKGETYTYD
WQLITHPRDYSGEMEGKHSQILKLSKLTPGLYEFKVIVEGQNAHGEGYVNVTVKPEPRKNRPPIAIVSP
QFQEISLPTTSTVIDGSQSTDDDKIVQYHWEELKGPLREEKISEDTAILKLSKLVPGNYTFSLTVVDSD
GATNSTTANLTVNKAVDYPPVANAGPNQVITLPQNSITLFGNQSTDDHGITSYEWSLSPSSKGKVVEMQ
GVRTPTLQLSAMQEGDYTYQLTVTDTIGQQATAQVTVIVQPENNKPPQADAGPDKELTLPVDSTTLDGS
KSSDDQKIISYLWEKTQGPDGVQLENANSSVATVTGLQVGTYVFTLTVKDERNLQSQSSVNVIVKEEIN

KPPIAKITGNVVITLPTSTAELDGSKSSDDKGIVSYLWTRDEGSPAAGEVLNHSDHHPILFLSNLVEGT
YTFHLKVTDAKGESDTDRTTVEVKPDPRKNNLVEIILDINVSQLTERLKGMFIRQIGVLLGVLDSDIIV
QKIQPYTEQSTKMVFFVQNEPPHQIFKGHEVAAMLKSELRKQKADFLIFRALEVNTVTCQLNCSDHGHC
DSFTKRCICDPFWMENFIKVQLRDGDSNCALTILIFLAEWSVLYVIIATFVIVVALGILSWTVICCCKR
QKGKPKRKSKYKILDATDQESLELKPTSRAGIKQKGLLLSSSLMHSESELDSDDAIFTWPDREKGKLLH
GQNGSVPNGQTPLKARSPREEIL

**Mass Match Peptides (bold):**
ADFLIFR [59]
ELTLPVDSTTLDGSK [207]
GETYTYDWQLITHPR [293]
VVEMQGVR [809]

**Tandem Peptides (underline):**
EEINKPPIAK [176]


## Peptide Source: 1D-GE, Lung cancer

MEKRLGVKPNPASWILSGYYWQTSAKWLRSLYLFYTCFCFSVLWLSTDASESRCQQGKTQFGVGLRSGG
ENHLWLLEGTPSLQSCWAACCQDSACHVFWWLEGMCIQADCSRPQSCRAFRTHSSNSMLVFLKKFQTAD
DLGFLPEDDVPHLLGLGWNWASWRQSPPRAALRPAVSSSDQQSLIRKLQKRGSPSDVVTPIVTQHSKVN
DSNELGGLTTSGSAEVHKAITISSPLTTDLTAELSGGPKNVSVQPEISEGLATTPSTQQVKSSEKTQIA
VPQPVAPSYSYATPTPQASFQSTSAPYPVIKELVVSAGESVQTLPKNEVQLNAYVLQEPPKGETYTYD
WQLITHPRDYSGEMEGKHSQILKLSKLTPGLYEFKVIVEGQNAHGEGYVNVTVKPEPRKNRPPIAVSP
QFQEISLPTTSTVIDGSQSTDDDKIVQYHWEELKGPLREEKISEDTAILKLSKLVPGNYTFSLTVVDSD
GATNSTTANLTVNKAVDYPPVANAGPNQVITLPQNSITLFGNQSTDDHGITSYEWSLSPSSKGKVVEMQ
GVRTPTLQLSAMQEGDYTYQLTVTDTIGQQATAQVTVIVQPENNKPPQADAGPDKELTLPVDSTTLDGS
KSSDDQKIISYLWEKTQGPDGVQLENANSSVATVTGLQVGTYVFTLTVKDERNLQSQSSVNVIVKEEIN
KPPIAKITGNVVITLPTSTAELDGSKSSDDKGIVSYLWTRDEGSPAAGEVLNHSDHHPILFLSNLVEGT
YTFHLKVTDAKGESDTDRTTVEVKPDPRKNNLVEIILDINVSQLTERLKGMFIRQIGVLLGVLDSDIIV
QKIQPYTEQSTKMVFFVQNEPPHQIFKGHEVAAMLKSELRKQKADFLIFRALEVNTVTCQLNCSDHGHC
DSFTKRCICDPFWMENFIKVQLRDGDSNCALTILIFLAEWSVLYVIIATFVIVVALGILSWTVICCCKR
QKGKPKRKSKYKILDATDQESLELKPTSRAGIKQKGLLLSSSLMHSESELDSDDAIFTWPDREKGKLLH
GQNGSVPNGQTPLKARSPREEIL

**Mass Match Peptides (bold):**
GETYTYDWQLITHPR [293]
VNDSNELGGLTTSGSAEVHK [786]

**Tandem Peptides (underline):**
IQPYTEQSTK [391]


## Peptide Source: 1D-GE, Ovarian cancer

MEKRLGVKPNPASWILSGYYWQTSAKWLRSLYLFYTCFCFSVLWLSTDASESRCQQGKTQFGVGLRSGG
ENHLWLLEGTPSLQSCWAACCQDSACHVFWWLEGMCIQADCSRPQSCRAFRTHSSNSMLVFLKKFQTAD
DLGFLPEDDVPHLLGLGWNWASWRQSPPRAALRPAVSSSDQQSLIRKLQKRGSPSDVVTPIVTQHSKVN
DSNELGGLTTSGSAEVHKAITISSPLTTDLTAELSGGPKNVSVQPEISEGLATTPSTQQVKSSEKTQIA
VPQPVAPSYSYATPTPQASFQSTSAPYPVIKELVVSAGESVQTLPKNEVQLNAYVLQEPPKGETYTYD
WQLITHPRDYSGEMEGKHSQILKLSKLTPGLYEFKVIVEGQNAHGEGYVNVTVKPEPRKNRPPIAVSP
QFQEISLPTTSTVIDGSQSTDDDKIVQYHWEELKGPLREEKISEDTAILKLSKLVPGNYTFSLTVVDSD
GATNSTTANLTVNKAVDYPPVANAGPNQVITLPQNSITLFGNQSTDDHGITSYEWSLSPSSKGKVVEMQ
GVRTPTLQLSAMQEGDYTYQLTVTDTIGQQATAQVTVIVQPENNKPPQADAGPDKELTLPVDSTTLDGS
KSSDDQKIISYLWEKTQGPDGVQLENANSSVATVTGLQVGTYVFTLTVKDERNLQSQSSVNVIVKEEIN

KPPIAKITGNVVITLPTSTAELDGSKSSDDKGIVSYLWTRDEGSPAAGEVLNHSDHHPILFLSNLVEGT
YTFHLKVTDAKGESDTDRTTVEVKPDPRKNNLVEIILDINVSQLTERLKGMFIRQIGVLLGVLDSDIIV
QKIQPYTEQSTKMVFFVQNEPPHQIFKGHEVAAMLKSELRKQKADFLIFRALEVNTVTCQLNCSDHGHC
DSFTKRCICDPFWMENFIKVQLRDGDSNCALTILIFLAEWSVLYVIIATFVIVVALGILSWTVICCCKR
QKGKPKRKSKYKILDATDQESLELKPTSRAGIKQKGLLLSSSLMHSESELDSDDAIFTWPDREKGKLLH
GQNGSVPNGQTPLKARSPREEIL

## Mass Match Peptides (bold):

    ADFLIFR [59]
    GETYTYDWQLITHPR [293]
    ILDATDQESLELKPTSR [374]
    IQPYTEQSTK [391]
    LTPGLYEFK [497]
    MVFFVQNEPPHQIFK [528]
    NVSVQPEISEGLATTPSTQQVK [562]
    THSSNSMLVFLK [699]

## Tandem Peptides (underline):

    EEINKPPIAK [176]
    NLQSQSSVNVIVK [544]

## Peptide Source: iTRAQ, Non-small cell lung cancer

MEKRLGVKPNPASWILSGYYWQTSAKWLRSLYLFYTCFCFSVLWLSTDASESRCQQGKTQFGVGLRSGG
ENHLWLLEGTPSLQSCWAACCQDSACHVFWWLEGMCIQADCSRPQSCRAFRTHSSNSMLVFLKKFQTAD
DLGFLPEDDVPHLLGLGWNWASWRQSPPRAALRPAVSSSDQQSLIRKLQKRGSPSDVVTPIVTQHSKVN
DSNELGGLTTSGSAEVHKAITISSPLTTDLTAELSGGPKNVSVQPEISEGLATTPSTQQVKSSEKTQIA
VPQPVAPSYSYATPTPQASFQSTSAPYPVIKELVVSAGESVQTLPKNEVQLNAYVLQEPPKGETYTYD
WQLITHPRDYSGEMEGKHSQILKLSKLTPGLYEFKVIVEGQNAHGEGYVNVTVKPEPRKNRPPIAVSP
QFQEISLPTTSTVIDGSQSTDDDKIVQYHWEELKGPLREEKISEDTAILKLSKLVPGNYTFSLTVVDSD
GATNSTTANLTVNKAVDYPPVANAGPNQVITLPQNSITLFGNQSTDDHGITSYEWSLSPSSKGKVVEMQ
GVRTPTLQLSAMQEGDYTYQLTVTDTIGQQATAQVTVIVQPENNKPPQADAGPDKELTLPVDSTTLDGS
KSSDDQKIISYLWEKTQGPDGVQLENANSSVATVTGLQVGTYVFTLTVKDERNLQSQSSVNVIVKEEIN
KPPIAKITGNVVITLPTSTAELDGSKSSDDKGIVSYLWTRDEGSPAAGEVLNHSDHHPILFLSNLVEGT
YTFHLKVTDAKGESDTDRTTVEVKPDPRKNNLVEIILDINVSQLTERLKGMFIRQIGVLLGVLDSDIIV
QKIQPYTEQSTKMVFFVQNEPPHQIFKGHEVAAMLKSELRKQKADFLIFRALEVNTVTCQLNCSDHGHC
DSFTKRCICDPFWMENFIKVQLRDGDSNCALTILIFLAEWSVLYVIIATFVIVVALGILSWTVICCCKR
QKGKPKRKSKYKILDATDQESLELKPTSRAGIKQKGLLLSSSLMHSESELDSDDAIFTWPDREKGKLLH
GQNGSVPNGQTPLKARSPREEIL

## Mass Match Peptides (bold):
    LLHGQNGSVPNGQTPLK [935]
## Tandem Peptides (underline):
    LLHGQNGSVPNGQTPLK [935]

## Peptide Source: iTRAQ, Ovarian cancer

MEKRLGVKPNPASWILSGYYWQTSAKWLRSLYLFYTCFCFSVLWLSTDASESRCQQGKTQFGVGLRSGG
ENHLWLLEGTPSLQSCWAACCQDSACHVFWWLEGMCIQADCSRPQSCRAFRTHSSNSMLVFLKKFQTAD
DLGFLPEDDVPHLLGLGWNWASWRQSPPRAALRPAVSSSDQQSLIRKLQKRGSPSDVVTPIVTQHSKVN
DSNELGGLTTSGSAEVHKAITISSPLTTDLTAELSGGPKNVSVQPEISEGLATTPSTQQVKSSEKTQIA
VPQPVAPSYSYATPTPQASFQSTSAPYPVIKELVVSAGESVQTLPKNEVQLNAYVLQEPPKGETYTYD
WQLITHPRDYSGEMEGKHSQILKLSKLTPGLYEFKVIVEGQNAHGEGYVNVTVKPEPRKNRPPIAVSP

QFQEISLPTTSTVIDGSQSTDDDKIVQYHWEELKGPLREEKISEDTAILKLSKLVPGNYTFSLTVVDSD
GATNSTTANLTVNKAVDYPPVANAGPNQVITLPQNSITLFGNQSTDDHGITSYEWSLSPSSKGKVVEMQ
GVRTPTLQLSAMQEGDYTYQLTVTDTIGQQATAQVTVIVQPENNKPPQADAGPDKELTLPVDSTTLDGS
KSSDDQKIISYLWEKTQGPDGVQLENANSSVATVTGLQVGTYVFTLTVKDERNLQSQSSVNVIVKEEIN
KPPIAKITGNVVITLPTSTAELDGSKSSDDKGIVSYLWTRDEGSPAAGEVLNHSDHHPILFLSNLVEGT
YTFHLKVTDAKGESDTDRTTVEVKPDPRK**NNLVEIILDINVSQLTER**LKGMFIRQIGVLLGVLDSDIIV
QKIQPYTEQSTKMVFFVQNEPPHQIFKGHEVAAMLKSELRKQKADFLIFRALEVNTVTCQLNCSDHGHC
DSFTKRCICDPFWMENFIKVQLRDGDSNCALTILIFLAEWSVLYVIIATFVIVVALGILSWTVICCCKR
QKGKPKRKSKYKILDATDQESLELKPTSRAGIKQKGLLLSSSLMHSESELDSDDAIFTWPDREKGKLLH
GQNGSVPNGQTPLKARSPREEIL

**Mass Match Peptides (bold):**
NNLVEIILDINVSQLTER [956]
**Tandem Peptides (underline):**
NNLVEIILDINVSQLTER [956]


## Peptide Source: iTRAQ, Renal cell cancer

MEKRLGVKPNPASWILSGYYWQTSAKWLRSLYLFYTCFCFSVLWLSTDASESRCQQGKTQFGVGLRSGG
ENHLWLLEGTPSLQSCWAACCQDSACHVFWWLEGMCIQADCSRPQSCRAFRTHSSNSMLVFLKKFQTAD
DLGFLPEDDVPHLLGLGWNWASWRQSPPRAALRPAVSSSDQQSLIRKLQKRGSPSDVVTPIVTQHSKVN
DSNELGGLTTSGSAEVHKAITISSPLTTDLTAELSGGPKNVSVQPEISEGLATTPSTQQVKSSEKTQIA
VPQPVAPSYSYATPTPQASFQSTSAPYPVIKELVVSAGESVQITLPKNEVQLNAYVLQEPPKGETYTYD
WQLITHPRDYSGEMEGKHSQILKLSKLTPGLYEFKVIVEGQNAHGEGYVNVTVKPEPRKNRPPIAIVSP
QFQEISLPTTSTVIDGSQSTDDDKIVQYHWEELKGPLREEKISEDTAILKLSKLVPGNYTFSLTVVDSD
GATNSTTANLTVNKAVDYPPVANAGPNQVITLPQNSITLFGNQSTDDHGITSYEWSLSPSSKGKVVEMQ
GVRTPTLQLSAMQEGDYTYQLTVTDTIGQQATAQVTVIVQPENNKPPQADAGPDKELTLPVDSTTLDGS
KSSDDQKIISYLWEKTQGPDGVQLENANSSVATVTGLQVGTYVFTLTVKDERNLQSQSSVNVIVKEEIN
KPPIAKITGNVVITLPTSTAELDGSKSSDDKGIVSYLWTRDEGSPAAGEVLNHSDHHPILFLSNLVEGT
YTFHLKVTDAKGESDTDRTTVEVKPDPRKNNLVEIILDINVSQLTERLKGMFIRQIGVLLGVLDSDIIV
QKIQPYTEQSTKMVFFVQNEPPHQIFK**GHEVAAMLK**SELRKQKADFLIFRALEVNTVTCQLNCSDHGHC
DSFTKRCICDPFWMENFIKVQLRDGDSNCALTILIFLAEWSVLYVIIATFVIVVALGILSWTVICCCKR
QKGKPKRKSKYKILDATDQESLELKPTSRAGIKQKGLLLSSSLMHSESELDSDDAIFTWPDREKGKLLH
GQNGSVPNGQTPLKARSPREEIL

**Mass Match Peptides (bold):**
GHEVAAMLK [913]
**Tandem Peptides (underline):**
GHEVAAMLK [913]


## OGTA091 (SEQ ID No: 17)

## Peptide Source: 1D-GE, Breast cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKK**N**
**LVDPFVEVSFAGK**MLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGK**GEGVAY**

RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQK**TDVHYR**SLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

## Mass Match Peptides (bold):
    GEGVAYR [291]
    NLVDPFVEVSFAGK [547]
    TDVHYR [686]

## Tandem Peptides (underline):
    NLVDPFVEVSFAGK [547]

## Peptide Source: 1D-GE, Cervical cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGK**GEGVAY**
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL

SDFCNTFKLYRGKTQEETEDPSVIGEFKGLFKIYPLPEDPAIPMPPRQFHQLAAQGPQECLVRIYZVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQK**TDVHYR**SLGG
EGNFNWRFIFPZDYLPAEQVCTIAKKDAFWRLDKTESKIPARVVFQIWDNDKFSFDDZLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLZLAIFIYAFPNY
AAMKLVKPFS

**Mass Match Peptides (bold):**
    GEGVAYR [291]
    TDVHYR [686]
**Tandem Peptides (underline):**

## Peptide Source: 1D-GE, Chronic lymphocytic leukaemia

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPZLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTZGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPK**FSDVTGK**IKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDZKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPAZVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYZVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQK**TDVHYR**SLGG
EGNFNWRFIFPZDYLPAEQVCTIAKKDAFWRLDKTESKIPARVVFQIWDNDKFSFDDZLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLZLAIFIYAFPNY
AAMKLVKPFS

**Mass Match Peptides (bold):**
    FSDVTGK [272]
    TDVHYR [686]
**Tandem Peptides (underline):**

## Peptide Source: 1D-GE, Gastric cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKK**N**
**LVDPFVEVSFAGK**MLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGK**GEGVAY**
**R**GRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQK**TDVHYR**SLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

### Mass Match Peptides (bold):

GEGVAYR [291]
NLVDPFVEVSFAGK [547]
TDVHYR [686]

### Tandem Peptides (underline):

NLVDPFVEVSFAGK [547]

## Peptide Source: 1D-GE, Hepatocellular carcinoma

MLRVFILYAENVHTPDTDISDAYCSAVFAGVKKRTKVIKNSVNPVWNEGFEWDLKGIPLDQGSELHVVV
KDHETMGRNRFLGEAKVPLR**EVLATPSLSASFNAPLLDTK**KQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRK**LLSDKPQDFQIRVQVIEGR**QLPGVNIKPVVK**VTAAGQTK**RTRIHKGNSPLFNETLFFNLFDSPGE
LFDEPIFITVVDSRSLR**TDALLGEFRMDVGTIYR**EPRHAYLRK**WLLLSDPDDFSAGAR**GYLK**TSLCVLG**
**PGDEAPLERK**DPSEDKEDIESNLLRPTGVALRGAHFCLKVFRAEDLPQMDDAVMDNVK**QIFGFESNKK**N
**LVDPFVEVSFAGK**MLCSKILEK**TANPQWNQNITLPAMFPSMCEK**MRIR**IIDWDR**LTHNDIVATTYLSMS
KISAPGGEIEEEPAGAVKPSKASDLDDYLGFLPTFGPCYINLYGSPREFTGFPDPYTELNTGK**GEGVAY**
**R**GRLLLSLETKLVEHSEQK**VEDLPADDILR**VEKYLRRRKYSLFAAFYSATMLQDVDDAIQFEVSIGNYG
NKFDMTCLPLASTTQYSRAVFDGCHYYYLPWGNVKPVVVLSSYWEDISHR**IETQNQLLGIADR**LEAGLE
QVHLALKAQCSTEDVDSLVAQLTDELIAGCSQPLGDIHETPSATHLDQYLYQLRTHHLSQITEAALALK

```
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQKTDVHYRSLGG
EGNFNWRFIFPTDYLPAEQVCTIAKKDAFWRLDKTESKIPARVVFQIWDNDKFSFDDTLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLTLAIFIYAFPNY
AAMKLVKPFS
```

**Mass Match Peptides (bold):**

```
AVDEQGWEYSITIPPER [94]
DLSQMEALK [139]
DLSQMEALKR [140]
DSFSDPYAIVSFLHQSQK [155]
EKPAIHHIPGFEVQETSR [201]
EVLATPSLSASFNAPLLDTK [234]
FFASIGER [250]
FHLEYR [254]
GEGVAYR [291]
GSQPSGELLASFELIQR [320]
IETQNQLLGIADR [363]
IGETVVDLENR [367]
IIDWDR [370]
ILDESEDTDLPYPPPQR [375]
IPNPHLGPVEER [386]
IYPLPEDPAIPMPPR [403]
KNPNFDICTLFMEVMLPR [430]
KTDAFRR [436]
LLSDKPQDFQIR [469]
LPGGQWIYMSDNYTDVNGEK [477]
LSVFAETYENETK [491]
MDVGTIYR [511]
MFELTCTLPLEK [515]
MYYTHRRR [533]
NLVDPTVEVSFAGK [547]
NPNFDICTLFMEVMLPR [551]
QFHQLAAQGPQECLVR [570]
QIFGFESNK [579]
RDLSQMEALK [599]
RMEPLEK [607]
RPDTSFLWFTSPYK [608]
SLGGEGNFNWR [640]
TANPQWNQNITLPAMFPSMCEK [670]
TDAFRRR [680]
TDALLGEFR [681]
```

    TDVHYR [686]
    TQEETEDPSVIGEFK [724]
    TSLCVLGPGDEAPLER [728]
    TSLCVLGPGDEAPLERK [729]
    VEDLPADDILR [753]
    VPAHQVLFSR [787]
    VQVIEGR [794]
    VTAAGQTK [800]
    VVFQIWDNDK [810]
    WEDEEWSTDLNR [824]
    WLLLSDPDDFSAGAR [827]

## Tandem Peptides (underline):

    FFASIGER [250]
    HWVPAEK [348]
    IGETVVDLENR [367]
    IYPLPEDPAIPMPPR [403]
    LLSDKPQDFQIR [469]
    MDVGTIYR [511]
    NLVDPFVEVSFAGK [547]
    QIFGFESNK [579]
    SLGGEGNFNWR [640]
    TDALLGEFR [681]
    VEDLPADDILR [753]

## Peptide Source: 1D-GE, Lung cancer

MLRVFILYAENVHTPDTEISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNK**KN
LVDPFVEVSFAGK**MLCSKILEKTANPQWNQNITLPAMFPSMCEKMRIRIIDWDRLTHNDIVATIYLSMS
KISAPGGEIEEEPAGAVKPSKASDLDDYLGFLPTFGPCYINLYGSPREFTGFPDPYTELNTGK**GEGVAY
R**GRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRR**RMEPLEK**T
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSR**ILDESEDTDLPYPF
PQR**EANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
K**EYSIEEIEAGRIPNPHLGPVEER**LALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQK**TDVHYR**SLGG
**EGNFNWR**FIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP

AGQGRDEPNMNPKLEDPRRPDTSFLWFTSPYKTMKFILWRRFRWAIILFIILFILLLFLAIFIYAFPNY
AAMKLVKPFS

**Mass Match Peptides (bold):**

         GEGVAYR [291]
         ILDESEDTDLPYPPPQR [375]
         IPNPHLGPVEER [386]
         KNLVDPFVEVSFAGK [429]
         MEPLEK [513]
         RMEPLEK [607]
         TDVHYR [686]
**Tandem Peptides (underline):**
         EYSIEEIEAGR [241]
         SLGGEGNFNWR [640]


## Peptide Source: 1D-GE, Melanoma

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
**LVDPFVEVSFAGK**MLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLLSLETKLVEHSEQKVEDLPADDILRVEKYLRRRKYSLFAAFYSATMLQDVDDAIQFEVSIGNYG
NKFDMTCLPLASTTQYSRAVFDGCHYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPK**FSDVTGK**IKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

**Mass Match Peptides (bold):**

         FSDVTGK [272]
         NLVDPFVEVSFAGK [547]
**Tandem Peptides (underline):**

## Peptide Source: 1D-GE, Osteosarcoma

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKK**N
LVDPFVEVSFAGK**MLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQK**TDVHYR**SLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

**Mass Match Peptides (bold):**
    NLVDPFVEVSFAGK [547]
    TDVHYR [686]
**Tandem Peptides (underline):**

## Peptide Source: 1D-GE, Ovarian cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKK**N
LVDPFVEVSFAGK**MLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGK**GEGVAY
R**GRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT

GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

**Mass Match Peptides (bold):**
       GEGVAYR [291]
       KNLVDPFVEVSFAGK [429]
       NLVDPFVEVSFAGK [547]

**Tandem Peptides (underline):**

## Peptide Source: 1D-GE, Pancreatic cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNK**KN
LVDPFVEVSFAGK**MLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGK**GEGVAY
R**GRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPK**FSDVTGK**IKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQK**TDVHYR**SLGG
EGNFNWRFIFPFDYLPAEQVCTIAKKDAFWRLDKTESKIPARVVFQIWDNDKFSFDDFLGSLQLDLNRM

PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

## Mass Match Peptides (bold):

FSDVTGK [272]
GEGVAYR [291]
KNLVDPFVEVSFAGK [429]
NLVDPFVEVSFAGK [547]
TDVHYR [686]

## Tandem Peptides (underline):

NLVDPFVEVSFAGK [547]


## Peptide Source: 1D-GE, Prostate cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGK**GEGVAY**
**R**GRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPAFVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQK**TDVHYR**SLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

## Mass Match Peptides (bold):

GEGVAYR [291]
TDVHYR [686]

## Tandem Peptides (underline):


## Peptide Source: 1D-GE, Renal cell cancer

```
MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPAIVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS
```

## Mass Match Peptides (bold):

```
AVDEQGWEYSITIPPER [94]
CIIWNTR [104]
DLAAMDK [132]
DLSQMEALK [139]
EFNQFAEGK [185]
EKPAIHHIPGFEVQETSR [201]
EYSIEEIEAGR [241]
FHLEYR [254]
FSDVTGK [272]
FSFDDFLGSLQLDLNR [273]
GEGVAYR [291]
IETQNQLLGIADR [363]
IGETVVDLENR [367]
IIDWDR [370]
ILDESEDTDLPYPPPQR [375]
IYPLPEDPAIPMPPR [403]
KLPSRPPPHYPGIK [426]
LLSDKPQDFQIR [469]
MDVGTIYR [511]
MYYTHR [531]
MYYTHRR [532]
NLVDPFVEVSFAGK [547]
RMEPLEK [607]
```

```
SLGGEGNFNWR [640]
SYQLANISSPSLVVECGGQTVQSCVIR [667]
TDALLGEFR [681]
TDVHYR [686]
TQEETEDPSVIGEFK [724]
VEDLPADDILR [753]
VQVIEGR [794]
WEDEEWSTDLNR [824]
WLLLSDPDDFSAGAR [827]
```

**Tandem Peptides (underline):**
```
ALGRPGPPFNITPR [76]
DLSQMEALK [139]
GWMIGFEEHK [329]
```


## Peptide Source: iTRAQ, Colorectal cancer

```
MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS
```


**Mass Match Peptides (bold):**
```
IYIVR [926]
KRSAPTSR [928]
```

**Tandem Peptides (underline):**
```
IYIVR [926]
KRSAPTSR [928]
```

## Peptide Source: iTRAQ, Hepatocellular cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPAR**VVFQIWDNDK**FSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

### Mass Match Peptides (bold):
VVFQIWDNDK [810]

### Tandem Peptides (underline):
VVFQIWDNDK [810]

## Peptide Source: iTRAQ, Non-small cell lung cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPIIYPGIKRKRSAP
TSRKLLSDKPQDFQIRVQVIEGRQLPGVNIKPVVK**VTAAGQTK**RTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC

GKLQTIFLKYPMEKVPGARMPVQIRVKLWFGLSVDEKEFNQFAEGKLSVFAETYENETKLALVGNWGTT
GLTYPK**FSDVTGK**IKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLR**RRDLSQMEALK**RHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQR**TAIEILAWGLR**NMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVR**IYIVR**A
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGR**IPNPHLGPVEER**LALHVLQQQGLVPEHVESRPLYSPLQPDIEQGKLQMWVDLFPKAL
GRPGPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

### Mass Match Peptides (bold):

   FSDVTGK [272]
   IPNPHLGPVEER [386]
   IYIVR [926]
   RRDLSQMEALK [966]
   TAIEILAWGLR [976]
   VTAAGQTK [800]

### Tandem Peptides (underline):

   FSDVTGK [272]
   IPNPHLGPVEER [386]
   IYIVR [926]
   RRDLSQMEALK [966]
   TAIEILAWGLR [976]
   VTAAGQTK [800]

## Peptide Source: iTRAQ, Ovarian cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVK**VTAAGQTKRTR**IHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQR**VPAHQVLFSRR**GANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR

CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

**Mass Match Peptides (bold):**
    VPAHQVLFSRR [997]
    VTAAGQTKRTR [999]

**Tandem Peptides (underline):**
    VPAHQVLFSRR [997]
    VTAAGQTKRTR [999]


## Peptide Source: iTRAQ, Renal cell cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

**Mass Match Peptides (bold):**
FSDVTGKIK [912]
IGETVVDLENR [367]
LLSDK [938]
RMEPLEK [607]
RRDLSQMEALK [966]

**Tandem Peptides (underline):**
FSDVTGKIK [912]
IGETVVDLENR [367]
LLSDK [938]
RMEPLEK [607]
RRDLSQMEALK [966]

## Peptide Source: iTRAQ, Small cell lung cancer

MLRVFILYAENVHTPDTDISDAYCSAVFAGVKKRTKVIKNSVNPVWNEGFEWDLKGIPLDQGSELHVVV
KDHETMGRNRFLGEAKVPLREVLATPSLSASFNAPLLDTKKQPTGASLVLQVSYTPLPGAVPLFPPPTP
LEPSPTLPDLDVVADTGGEEDTEDQGLTGDEAEPFLDQSGGPGAPTTPRKLPSRPPPHYPGIKRKRSAP
TSRKLLSDKPQDFQIRVQVIEGRQLPGVNIKPVVKVTAAGQTKRTRIHKGNSPLFNETLFFNLFDSPGE
LFDEPIFITVVDSRSLRTDALLGEFRMDVGTIYREPRHAYLRKWLLLSDPDDFSAGARGYLKTSLCVLG
PGDEAPLERKDPSEDKEDIESNLLRPTGVALRGAHFCLKVFRAEDLPQMDDAVMDNVKQIFGFESNKKN
LVDPFVEVSFAGKMLCSKILEKTANPQWNQNITLPAMFPSMCEKMRIRIIDWDRLTHNDIVATTYLSMS
KISAPGGEIEEEPAGAVKPSKASDLDDYLGFLPTFGPCYINLYGSPREFTGFPDPYTELNTGKGEGVAY
RGRLLLSLETKLVEHSEQKVEDLPADDILRVEKYLRRRKYSLFAAFYSATMLQDVDDAIQFEVSIGNYG
NKFDMTCLPLASTTQYSRAVFDGCHYYYLPWGNVKPVVVLSSYWEDISHRIETQNQLLGIADRLEAGLE
QVHLALKAQCSTEDVDSLVAQLTDELIAGCSQPLGDIHETPSATHLDQYLYQLRTHHLSQITEAALALK
LGHSELPAALEQAEDWLLRLRALAEEPQNSLPDIVIWMLQGDKRVAYQRVPAHQVLFSRRGANYCGKNC
GKLQTIFLKYPMEKVPGARMPVQIRVKLWFGLSVDEKEFNQFAEGKLSVFAETYENETKLALVGNWGTT
GLTYPKFSDVTGKIKLPKDSFRPSAGWTWAGDWFVCPEKTLLHDMDAGHLSFVEEVFENQTRLPGGQWI
YMSDNYTDVNGEKVLPKDDIECPLGWKWEDEEWSTDLNRAVDEQGWEYSITIPPERKPKHWVPAEKMYY
THRRRRWVRLRRRDLSQMEALKRHRQAEAEGEGWEYASLFGWKFHLEYRKTDAFRRRRWRRRMEPLEKT
GPAAVFALEGALGGVMDDKSEDSMSVSTLSFGVNRPTISCIFDYGNRYHLRCYMYQARDLAAMDKDSFS
DPYAIVSFLHQSQKTVVVKNTLNPTWDQTLIFYEIEIFGEPATVAEQPPSIVVELYDHDTYGADEFMGR
CICQPSLERMPRLAWFPLTRGSQPSGELLASFELIQREKPAIHHIPGFEVQETSRILDESEDTDLPYPP
PQREANIYMVPQNIKPALQRTAIEILAWGLRNMKSYQLANISSPSLVVECGGQTVQSCVIRNLRKNPNF
DICTLFMEVMLPREELYCPPITVKVIDNRQFGRRPVVGQCTIRSLESFLCDPYSAESPSPQGGPDDVSL
LSPGEDVLIDIDDKEPLIPIQEEEFIDWWSKFFASIGEREKCGSYLEKDFDTLKVYDTQLENVEAFEGL
SDFCNTFKLYRGKTQEETEDPSVIGEFKGLFKIYPLPEDPAIPMPPRQFHQLAAQGPQECLVRIYIVRA
FGLQPKDPNGKCDPYIKISIGKKSVSDQDNYIPCTLEPVFGKMFELTCTLPLEKDLKITLYDYDLLSKD
EKIGETVVDLENRLLSKFGARCGLPQTYCVSGPNQWRDQLRPSQLLHLFCQQHRVKAPVYRTDRVMFQD
KEYSIEEIEAGRIPNPHLGPVEERLALHVLQQQGLVPEHVESRPLYSPLQPDIEQGKLQMWVDLFPKAL
GRPGPPPFNITPRRARRFFLRCIIWNTRDVILDDLSLTGEKMSDIYVKGWMIGFEEHKQKTDVHYRSLGG
EGNFNWRFIFPFDYLPAEQVCTIAKKDAFWRLDKTESKIPARVVFQIWDNDKFSFDDFLGSLQLDLNRM
PKPAKTAKKCSLDQLDDAFHPEWFVSLFEQKTVKGWWPCVAEEGEKKILAGKLEMTLEIVAESEHEERP
AGQGRDEPNMNPKLEDPRRPDTSFLWFTSPYKTMKFILWRRFRWAIILFIILFILLLFLAIFIYAFPNY
AAMKLVKPFS

**Mass Match Peptides (bold):**
LLSDK [938]

**Tandem Peptides (underline):**
LLSDK [938]

## OGTA098 (SEQ ID No: 18)

## Peptide Source: 1D-GE, Breast cancer

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDR**EETPFFLLTGYALDAR**GNNV
EKPLELRIKVLDINDNEPVFTQDVFVGSVEELSAAHTLVMKINATDADEPNTLNSKISYRIVSLEPAYP
PVFYLNK**DTGEIYTTSVTLDR**EEHSSYTLTVEAR**DGNGEVTDKPVK**QAQVQIRILDVNDNIPVVENKVL
EGMVEENQVNVEVTRIKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDR**DNWISVDSVTSEIK**LAKLPDFESR**YVQNGTYTVKIVAISEDYPR**KTITGT
VLINVEDINDNCPTLIEPVQTICHDAEYVNVTAEDLDGHPNSGPFSFSVIDKPPGMAEKWKIAR**QESTS**
**VLLQQSEKK**LGRSEIQFLISDNQGFSCPEKQVLTLTVCEVLHGSGCREAQHDSYVGLGPAAIALMILAF
LLLLLVPLLLLMCHCGKGAKAFTPIPGTIEMLHPWNNEGAPPEDK**VVPSFLPVDQGGSLVGRNGVGGMA**
**K**EATMKGSSSASIVKGQHEMSEMDGRWEEHRSLLSGR**ATQFTGATGAIMTTETTK**TARATGASR**DMAGA**
**QAAAVALNEEFLR**NYFTDKAASYTEEDENHTAKDCLLVYSQEETESLNASIGCCSFIEGELDDR**FLDDL**
**GLK**FKTLAEVCLGQKIDINKEIEQRQKPATETSMNTASHSLCEQTMVNSENTYSSGSSFPVPKSLQEAN
AEK**VTQEIVTER**SVSSRQAQKVATPLPDPMASRNVIATETSYVTGSTMPPTTVILGPSQPQSLIVIERV
**YAPASTLVDQPYANEGTVVVTER**VIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTITISST
RVTKHSTVQHSYS

### Mass Match Peptides (bold):
ATQFTGATGAIMTTETTK [93]
DGNGEVTDKPVK [123]
DMAGAQAAAVALNEEFLR [142]
DNWISVDSVTSEIK [148]
DTGEIYTTSVTLDR [159]
EETPFFLLTGYALDAR [182]
FLDDLGLK [257]
IVAISEDYPR [400]
NGVGGMAK [536]
QESTSVLLQQSEK [568]
QESTSVLLQQSEKK [569]
VTQEIVTER [806]
VVPSFLPVDQGGSLVGR [812]
VYAPASTLVDQPYANEGTVVVTER [816]
YVQNGTYTVK [876]

### Tandem Peptides (underline):
VVPSFLPVDQGGSLVGR [812]

## Peptide Source: 1D-GE, Cervical cancer

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMKINATDADEPNTLNSKISYRIVSLEPAYP
PVFYLNK**DTGEIYTTSVTLDREEHSSYTLTVEAR**DGNGEVTDKPVKQAQVQIRILDVNDNIPVVENKVL
EGMVEENQVNVEVTRIKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVK**IVAISEDYPR**KTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALMILAF
LLLLLVPLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPKSLQEAN

```
AEKVTQEIVTERSVSSRQAQKVATPLPDPMASRNVIATETSYVTGSTMPPTTVILGPSQPQSLIVTERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS
```

**Mass Match Peptides (bold):**
```
        DTGEIYTTSVTLDR [159]
        EEHSSYTLTVEAR [174]
        IVAISEDYPR [400]
```
**Tandem Peptides (underline):**
```
        IVAISEDYPR [400]
```

## Peptide Source: 1D-GE, Hepatocellular carcinoma

```
MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMKINATDADEPNTLNSKISYRIVSLEPAYP
PVFYLNKDTGEIYTTSVTLDREEHSSYTLTVEARDGNGEVIDKPVKQAQVQIRILDVNDNIPVVENKVL
EGMVEENQVNVEVTRIKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALMILAF
LLLLLVPLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPKSLQEAN
AEKVTQEIVTERSVSSRQAQKVATPLPDPMASRNVIATETSYVTGSTMPPTTVILGPSQPQSLIVTERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS
```

**Mass Match Peptides (bold):**
```
        ATQFTGATGAIMTTETTK [93]
        DNWISVDSVTSEIK [148]
        DTGEIYTTSVTLDR [159]
        FLDDLGLK [257]
        GNNVEKPLELR [304]
        GQIIGNFQAFDEDTGLPAHAR [311]
        INATDADEPNTLNSK [381]
        IVAISEDYPR [400]
        QESTSVLLQQSEK [568]
        VATPLPDPMASR [746]
        VLEGMVEENQVNVEVTR [778]
        VTQEIVTER [806]
        VVPSFLPVDQGGSLVGR [812]
        WEEHR [825]
```
**Tandem Peptides (underline):**
```
        ATQFTGATGAIMTTETTK [93]
```

## Peptide Source: 1D-GE, Lung cancer

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDAR**GNNV
EKPLELR**IKVLDINDNEPVFTQDVFVGSVEELSAAHTLVMK**INATDADEPNTLNSK**ISYRIVSLEPAYP
PVFYLNK**DTGEIYTTSVTLDREEHSSYTLTVEAR**DGNGEVIDKPVKQAQVQIRILDVNDNIPVVENK**VL
EGMVEENQVNVEVTR**IKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSK**GQIIGNFQA
FDEDTGLPAHAR**YVKLEDR**DNWISVDSVTSEIK**LAK**LPDFESR**YVQNGTYTVK**IVAISEDYPR**KTITGT
VLINVEDINDNCPTLIEPVQTICHDAEYVNVTAEDLDGHPNSGPFSFSVIDKPPGMAEKWKIAR**QESTS
VLLQQSEK**KLGRSEIQFLISDNQGFSCPEKQVLTLTVCEVLHGSGCREAQHDSYVGLGPAAIALMILAF
LLLLLVPLLLLMCHCGKGAKAFTPIPGTIEMLHPWNNEGAPPEDK**VVPSFLPVDQGGSLVGR**NGVGGMA
KEATMKGSSSASIVKGQHEMSEMDGRWEEHRSLLSGR**ATQFTGATGAIMTTETTK**TARATGASRDMAGA
QAAAVALNEEFLRNYFTDKAASYTEEDENHTAKDCLLVYSQEETESLNASIGCCSFIEGELDDR**FLDDL
GLK**FKTLAEVCLGQKIDINKEIEQRQKPATETSMNTASHSLCEQTMVNSENTYSSGSSFPVPKSLQEAN
AEK**VTQEIVTER**SVSSRQAQK**VATPLPDPMASR**NVIATETSYVTGSTMPPTTVILGPSQPQSLIVTER**V
YAPASTLVDQPYANEGTVVVTER**VIQPHGGGSNPLEGTQHLQDVPYVMVRERESFLAPSSGVQPTLAMP
NIAVGQNVTVTER**VLAPASTLQSSYQIPTENSMTAR**NTTVSGAGVPGPLPDFGLEESGHSNSTITISST
RVTKHSTVQHSYS

## Mass Match Peptides (bold):

ATQFTGATGAIMTTETTK [93]
DNWISVDSVTSEIK [148]
DTGEIYTTSVTLDR [159]
EEHSSYTLTVEAR [174]
GNNVEKPLELR [304]
GQIIGNFQAFDEDTGLPAHAR [311]
INATDADEPNTLNSK [381]
IVAISEDYPR [400]
LPDFESR [476]
QESTSVLLQQSEK [568]
VLAPASTLQSSYQIPTENSMTAR [772]
VLEGMVEENQVNVEVTR [778]
VTQEIVTER [806]
VVPSFLPVDQGGSLVGR [812]
VYAPASTLVDQPYANEGTVVVTER [816]

## Tandem Peptides (underline):

FLDDLGLK [257]
VATPLPDPMASR [746]
YKPTPIPIK [858]

## Peptide Source: 1D-GE, Osteosarcoma

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMKINATDADEPNTLNSKISYRIVSLEPAYP
PVFYLNKDTGEIYTTSVTLDREEHSSYTLTVEARDGNGEVIDKPVKQAQVQIRILDVNDNIPVVENKVL
EGMVEENQVNVEVTRIKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALMILAF
LLLLLVPLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPKSLQEAN
AEKVTQEIVTERSVSSRQAQKVATPLPDPMASRNVIATETSYVTGSTMPPTTVILGPSQPQSLIVTERV

YAPASTLVDQPYANEGTVVVTERVIQPHGGGSNPLEGTQHLQDVPYVMRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS

## Mass Match Peptides (bold):

DNWISVDSVTSEIK [148]
IVAISEDYPR [400]
VLEGMVEENQVNVEVTR [778]

## Tandem Peptides (underline):

GITEPPFGIFVFNK [301]
IVAISEDYPR [400]

## Peptide Source: 1D-GE, Pancreatic cancer

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMKINATDADEPNTLNSKISYRIVSLEPAYP
PVFYLNKDTGEIYTTSVTLDREEHSSYTLTVEARDGNGEVTDKPVKQAQVQIRILDVNDNIPVVENKVL
EGMVEENQVNVEVTRIKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALMILAF
LLLLLVPLLLLMCHCGKGAKAFTPIPGTIEMLHPWNNEGAPPEDKVVPSFLPVDQGGSIVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPKSLQEAN
AEKVTQEIVTERSVSSRQAQKVATPLPDPMASRNVIATETSYVTGSTMPPTTVILGPSQPQSLIVTERV
YAPASTLVDQPYANEGTVVVTERVIQPHGGGSNPLEGTQHLQDVPYVMRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS

## Mass Match Peptides (bold):

ATQFTGATGAIMTTETTK [93]
DGNGEVTDKPVK [123]
DMAGAQAAAVALNEEFLR [142]
DNWISVDSVTSEIK [148]
DTGEIYTTSVTLDR [159]
DTGELNVTSILDR [160]
EEHSSYTLTVEAR [174]
EETPFFLLTGYALDAR [182]
EGEDLSKK [187]
GNNVEKPLELR [304]
GQIIGNFQAFDEDTGLPAHAR [311]
GSSSASIVK [322]
IHSDLAEER [369]
INATDADEPNTLNSK [381]
IVAISEDYPR [400]
QESTSVLLQQSEK [568]
SEIQFLISDNQGFSCPEK [628]
SSVISIYVSESMDR [653]
VATPLPDPMASR [746]
VLAPASTLQSSYQIPTENSMTAR [772]
VLEGMVEENQVNVEVTR [778]

VTQEIVTER [806]
VVPSFLPVDQGGSLVGR [812]
VYAPASTLVDQPYANEGTVVVTER [816]
WEEHR [825]

**Tandem Peptides (underline):**

ATQFTGATGAIMTTETTK [93]
DMAGAQAAAVALNEEFLR [142]
DTGEIYTTSVTLDR [159]
EEHSSYTLTVEAR [174]
ESFLAPSSGVQPTLAMPNIAVGQNVTVTER [223]
GNNVEKPLELR [304]
QESTSVLLQQSEK [568]
VATPLPDPMASR [746]
VLEGMVEENQVNVEVTR [778]
VVPSFLPVDQGGSLVGR [812]

## Peptide Source: 1D-GE, Prostate cancer

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMKINATDADEPNTLNSKISYRIVSLEPAYP
PVFYLNK**DTGEIYTTSVTLDR**EEHSSYTLTVEARDGNGEVTDKPVKQAQVQIRILDVNDNIPVVENKVL
EGMVEENQVNVEVTRIKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALMILAF
LLLLLVPLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDR**FLDDL**
**GLK**FKTLAEVCLGQKIDINKEIEQRQKPATETSMNTASHSLCEQTMVNSENTYSSGSSFPVPKSLQEAN
AEKVTQEIVTERSVSSRQAQK**VATPLPDPMASR**NVIATETSYVTGSTMPPTTVILGPSQPQSLIVTERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS

**Mass Match Peptides (bold):**

DTGEIYTTSVTLDR [159]
VATPLPDPMASR [746]

**Tandem Peptides (underline):**

FLDDLGLK [257]

## Peptide Source: 1D-GE, Renal cell cancer

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDAR**GNNV**
**EKPLELR**IKVLDINDNEPVFTQDVFVGSVEELSAAHTLVMKINATDADEPNTLNSKISYRIVSLEPAYP
PVFYLNKDTGEIYTTSVTLDREEHSSYTLTVEARDGNGEVTDKPVKQAQVQIRILDVNDNIPVVENKVL
EGMVEENQVNVEVTRIKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAK**LPDFESR**YVQNGTYTVK**IVAISEDYP**RKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALMILAF

LLLLLVPLLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPKSLQEAN
AEK**VTQEIVTER**SVSSRQAQKVATPLPDPMASRNVIATETSYVTGSTMPPTTVILGPSQPQSLIV⎺ERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTIT⎺SST
RVTKHSTVQHSYS

**Mass Match Peptides (bold):**
**Tandem Peptides (underline):**

GNNVEKPLELR [304]
IVAISEDYPR [400]
LPDFESR [476]
VTQEIVTER [806]

## Peptide Source: iTRAQ, Colorectal cancer

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMK**INATDADEPNTLNSK**ISYRIVSLEPAYP
PVFYLNKDTGEIYTTSVTLDREEHSSYTLTVEARDGNGEVTDKPVKQAQVQIRILDVNDNIPVVENKVL
EGMVEENQVNVEVTRIKVFDADEIGSDNWLANFT⎺ASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKT⎺TGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALM⎺LAF
LLLLLVPLLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFK**TLAEVCLGQK**IDINKEIEQRQKPATETSMNTASHSLCEQTMVNSENTYSSGSSFPVPK**SLQEAN
AEK**VTQEIVTERSVSSRQAQKVATPLPDPMASRNVIATETSYVTGSTMPPTTVILGPSQPQSLIV⎺ERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTIT⎺SST
RVTKHSTVQHSYS

**Mass Match Peptides (bold):**

⎺INATDADEPNTLNSK [381]
SLQEANAEK [970]
⎺LAEVCLGQK [982]

**Tandem Peptides (underline):**

INATDADEPNTLNSK [381]
SLQEANAEK [970]
⎺LAEVCLGQK [982]

## Peptide Source: iTRAQ, Non-small cell lung cancer

MARTRDRVRLLLLLLICFNVGSGLHLQVLSTRNENKLLPKHPHLVRQKRAWITAPVALREGEDLSKK**NPI
AK**IHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMK**INATDADEPNTLNSK**ISYRIVSLEPAYP
PVFYLNKDTGEIYTTSVTLDREEHSSYTLTVEARDGNGEVTDKPVKQAQVQIRILDVNDNIPVVENKVL
EGMVEENQVNVEVTRIKVFDADEIGSDNWLANFT⎺ASGNEGGYFHIETDAQTNEGIVTLIKEVDYEEMK

NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALMILAF
LLLLLVPLLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPKSLQEAN
AEKVTQEIVTERSVSSRQAQKVATPLPDPMASRNVIATETSYVTGSTMPPTTVILGPSQPQSLIVTERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS

**Mass Match Peptides (bold):**
        INATDADEPNTLNSK [381]
        NPIAK [957]
**Tandem Peptides (underline):**
        INATDADEPNTLNSK [381]
        NPIAK [957]


## Peptide Source: iTRAQ, Ovarian cancer

MARTRDRVRLLLLLLICFNVGSGLHLQVLSTRNENKLLPKHPHLVRQKRAWITAPVALREGEDLSKK**NPI**
**AK**IHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMKINATDADEPNTLNSKISYRIVSLEPAYP
PVFYLNKDTGEIYTTSVTLDREEHSSYTLTVEARDGNGEVIDKPVKQAQVQIRILDVNDNIPVVENKVL
EGMVEENQVNVEVTRIKVFDADEIGSDNWLANFTFASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLGPAAIALMILAF
LLLLLVPLLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPKSLQEAN
AEKVTQEIVTERSVSSRQAQKVATPLPDPMASRNVIATETSYVTGSTMPPTTVILGPSQPQSLIVTERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS

**Mass Match Peptides (bold):**
        NPIAK [957]
**Tandem Peptides (underline):**
        NPIAK [957]


## Peptide Source: iTRAQ, Renal cell cancer

MARTRDRVRLLLLLLICFNVGSGLHLQVLSTRNENKLLPKHPHLVRQKRAWITAPVALREGEDLSKKNPI
AKIHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMKINATDADEPNTLNSKISYRIVSLEPAYP
PVFYLNKDTGEIYTTSVTLDREEHSSYTLTVEARDGNGEVIDKPVKQAQVQIRILDVNDNIPVVENKVL
EGMVEENQVNVEVTRIKVFDADEIGSDNWLANFTFASGNEGGYFHIETDAQTNEGIVTLIKEVDYEEMK

NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLPAAIALMILAF
LLLLLVPLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTKTARATGASRDMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPK**SLQEAN**
**AEK**VTQEIVTERSVSSRQAQKVATPLPDPMASRNVIATETSYVTGSTMPPTTVILGPSQPQSLIVTERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS

**Mass Match Peptides (bold):**
    SLQEANAEK [970]

**Tandem Peptides (underline):**
    SLQEANAEK [970]


**Peptide Source: iTRAQ, Small cell lung cancer**

MARTRDRVRLLLLLLICFNVGSGLHLQVLSTRNENKLLPKHPHLVRQKRAWITAPVALREGEDLSKK**NPI**
**AK**IHSDLAEERGLKITYKYTGKGITEPPFGIFVFNKDTGELNVTSILDREETPFFLLTGYALDARGNNV
EKPLELRIKVLDINDNEPVFTQDVFVGSVEELSAAHTLVMKINATDADEPNTLNSKISYRIVSLEPAYP
PVFYLNKDTGEIYTTSVTLDREEHSSYTLTVEARDGNGEVTDKPVKQAQVQIRILDVNDNIPVVENKVL
EGMVEENQVNVEVTRIKVFDADEIGSDNWLANFTTASGNEGGYFHIETDAQTNEGIVTLIKEVDYEEMK
NLDFSVIVANKAAFHKSIRSKYKPTPIPIKVKVKNVKEGIHFKSSVISIYVSESMDRSSKGQIIGNFQA
FDEDTGLPAHARYVKLEDRDNWISVDSVTSEIKLAKLPDFESRYVQNGTYTVKIVAISEDYPRKTITGT
VLINVEDINDNCPTLIEPVQTICHDAEYVNVTAEDLDGHPNSGPFSFSVIDKPPGMAEKWKIARQESTS
VLLQQSEKKLGRSEIQFLISDNQGFSCPEKQVLTLTVCEVLHGSGCREAQHDSYVGLPAAIALMILAF
LLLLLVPLLLLMCHCGKGAKAFTPIPGTIEMLHPWNNEGAPPEDKVVPSFLPVDQGGSLVGRNGVGGMA
KEATMKGSSSASIVKGQHEMSEMDGRWEEHRSLLSGRATQFTGATGAIMTTETTK**TARATGASR**DMAGA
QAAAVALNEEFLRNYFTDKAASYTEEDENHTAKDCLLVYSQEETESLNASIGCCSFIEGELDDRFLDDL
GLKFKTLAEVCLGQKIDINKEIEQRQKPATETSMNTASHSLCEQTMVNSENTYSSGSSFPVPKSLQEAN
AEKVTQEIVTERSVSSRQAQKVATPLPDPMASRNVIATETSYVTGSTMPPTTVILGPSQPQSLIVTERV
YAPASTLVDQPYANEGTVVVTERVIQPHGGGSNPLEGTQHLQDVPYVMVRERESFLAPSSGVQPTLAMP
NIAVGQNVTVTERVLAPASTLQSSYQIPTENSMTARNTTVSGAGVPGPLPDFGLEESGHSNSTITTSST
RVTKHSTVQHSYS

**Mass Match Peptides (bold):**
    NPIAK [957]
    TARATGASR [977]

**Tandem Peptides (underline):**
    NPIAK [957]
    TARATGASR [977]


**OGTA101 (SEQ ID No: 19)**

**Peptide Source: 1D-GE, Breast cancer**

MVHVARLLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPR**GFPTIDMGPQLK**VVE
RTR**TATMLCAASGNPDPEITWFK**DFLPVDTSNNNGRIKQLRSESIGGTPIRGALQIEQSEESDQGKYEC

EP 2 447 719 B1

VATNSAGTRYSAPANLYVRELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSITLTWDS
GNPEPVSYYIIQHKPKNSEELYKEIDGVATTRYSVAGLSPYSDYEFRVVAVNNIGR**GPPSEPVLTQTSE**
**QAPSSAPR**DVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFK**AEPESETSILLSWTPPR**SDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIR**TNEDVPSGPPRK**VEVEAVNSTSVKVSWRSPVPNK**QHGQIR**GYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFRLSA
RNK**VGFGEEMVK**EISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMK**TSVL**
**LSWEIPENYNSAMPFK**ILYDDGK**MVEEVDGR**ATQKLIVNLKPEKS**YSFVLTNR**GNSAGGLQHRVTAKTA
PDVLRTKPAFIGKTNLDGMITVQLPEVPANENIKGYYIIIVPLKKSRGK**FIKPWESPDEMELDELLK**EI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKRKRAESDSRKSSIPNNK**EIP**
**SHHPTDPVELR**RLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGR**TGCFIVIDAMLER**IKHEKTVDIYGHVTL
MRAQRNYMVQTEDQYIFIHDALLEAVTCGNTEVPARNLYAYIQK**LTQIETGENVTGMELEFK**RLASSKA
HTSRFISANLPCNKFKNR**LVNIMPYESTR**VCLQPIR**GVEGSDYINASFIDGYR**QQKAYIATQGPLAETT
EDFWR**MLWEHNSTIVVMLTK**LREMGREKCHQYWPAERSAR**YQYFVVDPMAEYNMPQYILR**EFKVTDARD
GQSRTVR**QFQFTDWPEQGVPK**SGEGFIDFIGQVHKTKEQFGQDGPISVHCSAGVGRTGVFITLSIVLER
MRYEGVVDIFQTVKMLRTQRPAMVQTEDQYQFSYR**AALEYLGSFDHYAT**

**Mass Match Peptides (bold):**

 AALEYLGSFDHYAT [51]
 AEPESETSILLSWTPPR [63]
 EIPSHHPTDPVELR [198]
 FIKPWESPDEMELDELLK [255]
 GFPTIDMGPQLK [295]
 GPPSEPVLTQTSEQAPSSAPR [309]
 GVEGSDYINASFIDGYR [325]
 LTQIETGENVTGMELEFK [498]
 LVNIMPYESTR [503]
 MLWEHNSTIVVMLTK [520]
 MVEEVDGR [527]
 QFQFTDWPEQGVPK [572]
 QHGQIR [578]
 SYSFVLTNR [668]
 TATMLCAASGNPDPEITWFK [675]
 TGCFIVIDAMLER [692]
 TNEDVPSGPPRK [709]
 TSVLLSWEIPENYNSAMPFK [731]
 VGFGEEMVK [763]
 YQYFVVDPMAEYNMPQYILR [869]

**Tandem Peptides (underline):**

 AALEYLGSFDHYAT [51]
 GVEGSDYINASFIDGYR [325]


## Peptide Source: 1D-GE, Cervical cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPRGFPTIDMGPQLKVVE

584

RTRTATMLCAASGNPDPEITWFKDFLPVDTSNNNGRIKQLRSESIGGTPIRGALQIEQSEESDQGKYEC
VATNSAGTRYSAPANLYVRELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSIITLIWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEK**WTEYR**ITVTAH
TDVGPGPESLSVLIRTNEDVPSGPPRKVEVEAVNSTSVKVSWRSPVPNK<u>**QHGQIR**</u>GYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKISVL
LSWEIPENYNSAMPFKILYDDGKMVEEVDGRATQKLIVNLKPEKSYSFVLTNRGNSAGGLQHRVTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEKTVDIYGHVTL
MRAQRNYMVQTEDQYIFIHDALLEAVTCGNTEVPARNLYAYIQKLTQIETGENVTGMELEFKRLASSKA
HTSRFISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSARYQYFVVDPMAEYNMPQYILREFKVTDARD
GQSRTVRQFQFTDWPEQGVPKSGEGFIDFIGQVHKTKEQFGQDGPISVHCSAGVGRTGVFITLSIVLER
MRYEGVVDIFQTVKMLRTQRPAMVQTEDQYQFSYRAALEYLGSFDHYAT

## Mass Match Peptides (bold):
QHGQIR [578]
WTEYR [836]

## Tandem Peptides (underline):


## Peptide Source: 1D-GE, Colorectal cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPRGFPTIDMGPQLKVVE
RTRTATMLCAASGNPDPEITWFKDFLPVDTSNNNGRIKQLRSESIGGTPIRGALQIEQSEESDQGKYEC
VATNSAGTR<u>YSAPANLYVR</u>ELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSIITLIWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRKVEVEAVNSTSVKVSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKISVL
LSWEIPENYNSAMPFKILYDDGKMVEEVDGRATQKLIVNLKPEKSYSFVLTNRGNSAGGLQHRVTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA

TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEKTVDIYGHVTL
MRAQRNYMVQTEDQYIFIHDALLEAVTCGNTEVPARNLYAYIQKLTQIETGENVTGMELEFKRLASSKA
HTSRFISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSARYQYFVVDPMAEYNMPQYILREFKVTDARD
GQSRTVRQFQFTDWPEQGVPKSGEGFIDFIGQVHKTKEQFGQDGPISVHCSAGVGRTGVFITLSIVLER
MRYEGVVDIFQTVKMLRTQRPAMVQTEDQYQFSYRAALEYLGSFDHYAT

## Mass Match Peptides (bold):
## Tandem Peptides (underline):
YSAPANLYVR [870]


## Peptide Source: 1D-GE, Hepatocellular carcinoma

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPR**GFPTIDMGPQLK**VVE
RTR**TATMLCAASGNPDPEITWFK**DFLPVDTSNNNGRIKQLRSESIGGTPIRGALQIEQSEESDQGKYEC
VATNSAGTR<u>YSAPANLYVR</u>ELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSITLTWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLATSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRKVEVEAVNSTSVKVSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRK**DMEPLTTLEFSEK**EDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSK**GPGPYSPSVQFR**TLPVDQVFAKNTHVKAVMKTSVL
LSWEIPENYNSAMPFKILYDDGKMVEEVDGRATQKLIVNLKPEKSYSFVLTNRGNSAGGLQHRVTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVTAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNR<u>YANVIAYDHSR</u>VLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEKTVDIYGHVTL
MRAQRNYMVQTEDQYIFIHDALLEAVTCGNTEVPARNLYAYIQKLTQIETGENVTGMELEFKRLASSKA
HTSRFISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSARYQYFVVDPMAEYNMPQYILREFKVTDARD
GQSRTVRQFQFTDWPEQGVPK**SGEGFIDFIGQVHK**TKEQFGQDGPISVHCSAGVGRTGVFITLSIVLER
MRYEGVVDIFQTVKMLRTQRPAMVQTEDQYQFSYRAALEYLGSFDHYAT

## Mass Match Peptides (bold):
DMEPLTTLEFSEK [143]
GFPTIDMGPQLK [295]
GPGPYSPSVQFR [306]
SGEGFIDFIGQVHK [632]
TATMLCAASGNPDPEITWFK [675]
YSAPANLYVR [870]
## Tandem Peptides (underline):
YANVIAYDHSR [840]

## Peptide Source: 1D-GE, Lung cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPR**GFPTIDMGPQLK**VVE
RTR<u>**TATMLCAASGNPDPEITWFK**</u>DFLPVDTSNNNGRIKQLRSESIGGTPIRGALQIEQSEESDQGKYEC
VATNSAGTR<u>**YSAPANLYVR**</u>ELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSITLTWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFKAEPESETSILLSWTPPR<u>**SDTIANYELVYK**</u>
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRK**VEVEAVNSTSVK**VSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLITLEFSEKEDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKTSVL
LSWEIPENYNSAMPFKILYDDGKMVEEVDGRATQKLIVNLKPEKSYSFVLTNRGNSAGGLQHRVTAKTA
PDVLRTKPAFIGKTNLDGMITVQLPEVPANENIKGYYIIIVPLKKSRGK**FIKPWESPDEMELDELLK**EI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVTAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNR<u>YANVIAYDHSR</u>VLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEK**TVDIYGHVTL**
**MR**AQRNYMVQTEDQYIFIHDALLEAVTCGNTEVPARNLYAYIQK<u>**LTQIETGENVTGMELEFK**</u>RLASSKA
HTSRFISANLPCNKFKNRLVNIMPYESTRVCLQPIR**GVEGSDYINASFIDGYR**QQKAYIATQGPLAETT
EDFWRMLWEHNSTIVVMLTKLREMGREKCHQYWPAERSARYQYFVVDPMAEYNMPQYILREFKVTDARD
GQSRTVR<u>**QFQFTDWPEQGVPK**</u>SGEGFIDFIGQVHKTKEQFGQDGPISVHCSAGVGRTGVFITLSIVLER
MR<u>**YEGVVDIFQTVK**</u>MLRTQRPAMVQTEDQYQFSYRAALEYLGSFDHYAT

### Mass Match Peptides (bold):
FIKPWESPDEMELDELLK [255]
GFPTIDMGPQLK [295]
GVEGSDYINASFIDGYR [325]
LTQIETGENVTGMELEFK [498]
QFQFTDWPEQGVPK [572]
SDTIANYELVYK [625]
TATMLCAASGNPDPEITWFK [675]
TVDIYGHVTLMR [736]
VEVEAVNSTSVK [755]
YEGVVDIFQTVK [843]
YSAPANLYVR [870]

### Tandem Peptides (underline):
QFQFTDWPEQGVPK [572]
YANVIAYDHSR [840]

## Peptide Source: 1D-GE, Osteosarcoma

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPRGFPTIDMGPQLKVVE
RTRTATMLCAASGNPDPEITWFKDFLPVDTSNNNGRIKQLRSESIGGTPIRGALQIEQSEESDQGKYEC
VATNSAGTRYSAPANLYVRELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSIILTWDS
GNPEPVSYYIIQHKPKNSEELYKEIDGVATTRYSVAGLSPYSDYEFRVVAVNNIGRGPPSEPVLTQTSE

587

QAPSSAPRDVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRKVEVEAVNSTSVKVSWRSPVPNK**QHGQIR**GYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKTSVL
LSWEIPENYNSAMPFKILYDDGKMVEEVDGRATQKLIVNLKPEKSYSFVLTNRGNSAGGLQHRVTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEKTVDIYGHVTL
MRAQRNYMVQTEDQYIFIHDALLEAVTCGNTEVPARNLYAYIQKLTQIETGENVTGMELEFKRLASSKA
HTSRFISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSAR**YQYFVVDPMAEYNMPQYILR**EFKVTDARD
GQSRTVRQFQFTDWPEQGVPKSGEGFIDFIGQVHKTKEQFGQDGPISVHCSAGVGRTGVFITLSIVLER
MRYEGVVDIFQTVKMLRTQRPAMVQTEDQYQFSYRAALEYLGSFDHYAT

**Mass Match Peptides (bold):**

QHGQIR [578]

YQYFVVDPMAEYNMPQYILR [869]

**Tandem Peptides (underline):**

## Peptide Source: 1D-GE, Pancreatic cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPR**GFPTIDMGPQLK**VVE
RTRTATMLCAASGNPDPEITWFK**DFLPVDTSNNNGR**IKQLRSESIGGTPIRGALQIEQSEESDQGKYEC
VATNSAGTR**YSAPANLYVR**ELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSITLTWDS
GNPEPVSYYIIQHKPKNSEELYKEIDGVATTR**YSVAGLSPYSDYEFR**VVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFKAEPESETSILLSWTPPR**SDTIANYELVYK**
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRKVEVEAVNSTSVKVSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKTSVL
LSWEIPENYNSAMPFKILYDDGKMVEEVDGRATQKLIVNLKPEKSYSFVLTNR**GNSAGGLQHR**VTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKR**KRAESDSRK**SSIPNNK**EIP**
**SHHPTDPVELRR**LNFQTPGMASHPPIPILELADHIERLKANDNLKFSQEYESIDPGQQFTWEHSNLEVN
KPKNR**YANVIAYDHSR**VLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGR**TGCFIVIDAMLER**IKHEK**TVDIYGHVTL**
**MR**AQRNYMVQTEDQYIFIHDALLEAVTCGNTEVPARNLYAYIQK**LTQIETGENVTGMELEFK**RLASSKA
HTSRFISANLPCNKFKNR**LVNIMPYESTR**VCLQPIR**GVEGSDYINASFIDGYR**QQKAYIATQGPLAETT

EDFWRMLWEHNSTIVVMLTKLREMGREKCHQYWPAERSAR**YQYFVVDPMAEYNMPQYILR**EFKVTDARD
GQSRTVRQFQFTDWPEQGVPK**SGEGFIDFIGQVHK**TK**EQFGQDGPISVHCSAGVGR**TGVFITLSIVLER
MRYEGVVDIFQTVKMLRTQRPAMVQTEDQYQFSYRAALEYLGSFDHYAT

## Mass Match Peptides (bold):

> DFLPVDTSNNNGR [118]
> EIPSHHPTDPVELRR [199]
> EQFGQDGPISVHCSAGVGR [219]
> GFPTIDMGPQLK [295]
> GNSAGGLQHR [305]
> GVEGSDYINASFIDGYR [325]
> KRAESDSR [434]
> LTQIETGENVTGMELEFK [498]
> LVNIMPYESTR [503]
> RAESDSRK [595]
> SDTIANYELVYK [625]
> SGEGFIDFIGQVHK [632]
> TGCFIVIDAMLER [692]
> TVDIYGHVTLMR [736]
> YANVIAYDHSR [840]
> YQYFVVDPMAEYNMPQYILR [869]
> YSAPANLYVR [870]
> YSVAGLSPYSDYEFR [873]

## Tandem Peptides (underline):

> YANVIAYDHSR [840]
> YSAPANLYVR [870]

## Peptide Source: iTRAQ, Colorectal cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPRGFPTIDMGPQLKVVE
RTRTATMLCAASGNPDPEITWFKDFLPVDTSNNNGR**IKQLR**SESIGGTPIRGALQIEQSEESDQGKYEC
VATNSAGTRYSAPANLYVRELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSIILTWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRKVEVEAVNSTSVKVSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFR**LSA
RNK**VGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKTSVL
LSWEIPENYNSAMPFKILYDDGKMVEEVDGRATQKLIVNLKPEKSYSFVLTNRGNSAGGLQHRVTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEKTVDIYGHVTL
MRAQRNYMVQTEDQYIFIHDALLEAVTCGNTEVPARNLYAYIQKLTQIETGENVTGMELEFKR**LASSKA
HTSR**FISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSARYQYFVVDPMAEYNMPQYILREFKVTDARD

GQSRTVRQFQFTDWPEQGVPKSGEGFIDFIGQVHKTKEQFGQDGPISVHCSAGVGRTGVFITLSIVLER
MRYEGVVDIFQTVKMLRTQRPAMVQTEDQYQFSYRAALEYLGSFDHYAT


**Mass Match Peptides (bold):**
> IKQLR [922]
> LASSKAHTSR [930]
> LSARNK [946]

**Tandem Peptides (underline):**
> IKQLR [922]
> LASSKAHTSR [930]
> LSARNK [946]


## Peptide Source: iTRAQ, Hepatocellular cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPRGFPTIDMGPQLKVVE
RTRTATMLCAASGNPDPEITWFKDFLPVDTSNNNGRIKQLRSESIGGTPIRGALQIEQSEESDQGKYEC
VATNSAGTRYSAPANLYVRELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSIILTWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRKVEVEAVNSTSVKVSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKTSVL
LSWEIPENYNSAMPFKILYDDGKMVEEVDGRATQKLIVNLKPEKSYSFVLTNRGNSAGGLQHRVTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEKTVDIYGHVTL
MRAQRNYMVQTEDQYIFIHDALLEAVTCGNTEVPARNLYAYIQKLTQIETGENVTGMELEFKRLASSKA
HTSRFISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSARYQYFVVDPMAEYNMPQYILREFKVTDARD
GQSRTVRQFQFTDWPEQGVPKSGEGFIDFIGQVHKTKEQFGQDGPISVHCSAGVGRTGVFITLSIVLER
MRYEGVVDIFQTVKMLRTQRPAMVQTEDQYQFSYRAALEYLGSFDHYAT


**Mass Match Peptides (bold):**
> IKQLR [922]

**Tandem Peptides (underline):**
> IKQLR [922]


## Peptide Source: iTRAQ, Non-small cell lung cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPRGFPTIDMGPQLKVVE

RTRTATMLCAASGNPDPEITWFKDFLPVDTSNNNGRIKQLRSESIGGTPIRGALQIEQSEESDQGKYEC
VATNSAGTRYSAPANLYVR<u>ELREVR</u>RVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSITLⁱWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLAᶠTSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRK<u>VEVEAVNSTSVK</u>VSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFR<u>LSA
RNK</u>VGFGEEMVKEISIPEEVPTGFPQNLHSEGTTSTSVQLSWQPPVLAERNGIITKYTLLYRDINⁱPLL
PMEQLIVPADTTMTLTGLKPDTTYDVKVRAHTSKGPGPYSPSVQFRTLPVDQVFAKNᶠHVKAVMKⁱSVL
LSWEIPENYNSAMPFKILYDDGKMVEEVDGRATQKLIVNLKPEKSYSFVLTNR<u>GNSAGGLQHRVTAK</u>TA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVⁱAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA
TVVMMTKLEERSRVKCDQYWPSRGTETHGLVQVTLLDTVELAⁱYCVRTFALYKNGSSEKREVRQFQFTA
WPDHGVPEHPTPFLAFLRRVKTCNPPDAGPMVVHCSAGVGRTGCFIVIDAMLERIKHEK<u>TVDIYGHVTL
MR</u>AQRNYMVQTEDQYIFIHDALLEAVTCGNTEVPARNLYAYIQKLTQIETGENVTGMELEFKR<u>LASSKA
HTSR</u>FISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSARYQYFVVDPMAEYNMPQYILREFKVTDARD
GQSRTVRQFQFTDWPEQGVPKSGEGFIDFIGQVHKTKEQFGQDGPISVHCSAGVGRTGVFITLSIVLER
<u>MRYEGVVDIFQTVK</u>MLRTQRPAMVQTEDQYQFSYRAALEYLGSFDHYAT

## Mass Match Peptides (bold):

        ELREVR [901]
        GNSAGGLQHRVTAK [914]
        LASSKAHTSR [930]
        LSARNK [946]
        LTVLR [949]
        MRYEGVVDIFQTVK [952]
        ⁱVDIYGHVTLMR [736]
        VEVEAVNSTSVK [755]

## Tandem Peptides (underline):

        ELREVR [901]
        GNSAGGLQHRVTAK [914]
        LASSKAHTSR [930]
        LSARNK [946]
        LTVLR [949]
        MRYEGVVDIFQTVK [952]
        ⁱVDIYGHVTLMR [736]
        VEVEAVNSTSVK [755]

## Peptide Source: iTRAQ, Ovarian cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPRGFPTIDMGPQLKVVE
RTRTATMLCAASGNPDPEITWFKDFLPVDTSNNNGRIKQLRSESIGGTPIRGALQIEQSEESDQGKYEC
VATNSAGTRYSAPANLYVRELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSITLⁱWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV

PQKTYSVKVLAFTSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRKVEVEAVNSTSVKVSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKISVL
LSWEIPENYNSAMPFKILYDDGKMVEEVDGRATQKLIVNLKPEKSYSFVLTNRGNSAGGLQHRVTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEKTVDIYGHVTL
MRAQRNYMVQTEDQYIFIHDALLEAVTCGNTEVPARNLYAYIQKLTQIETGENVTGMELEFKRLASSKA
HTSRFISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSARYQYFVVDPMAEYNMPQYILREFKVTDARD
GQSRTVRQFQFTDWPEQGVPKSGEGFIDFIGQVHKTKEQFGQDGPISVHCSAGVGRTGVFITLSIVLER
MR**YEGVVDIFQTVK**MLRTQRPAMVQTEDQYQFSYRAALEYLGSFDHYAT

### Mass Match Peptides (bold):
YEGVVDIFQTVK [843]

### Tandem Peptides (underline):
YEGVVDIFQTVK [843]


## Peptide Source: iTRAQ, Renal cell cancer

MVHVARLLLLLLTFFLRTDAETPPRFTRTPVDQTGVSGGVASFICQATGDPRPKIVWNKKGKKVSNQRF
EVIEFDDGSGSVLRIQPLRTPRDEAIYECVASNNVGEISVSTRLTVLREDQIPRGFPTIDMGPQLKVVE
RTRTATMLCAASGNPDPEITWFKDFLPVDTSNNNGRIKQLRSESIGGTPIRGALQIEQSEESDQGKYEC
VATNSAGTRYSAPANLYVRELREVRRVPPRFSIPPTNHEIMPGGSVNITCVAVGSPMPYVKWMLGAEDL
TPEDDMPIGRNVLELNDVRQSANYTCVAMSTLGVIEAIAQITVKALPKPPGTPVVTESTATSITLIWDS
GNPEPVSYYIIQHKPKNSEELYKEIDGVATTRYSVAGLSPYSDYEFRVVAVNNIGRGPPSEPVLTQTSE
QAPSSAPRDVQARMLSSTTILVQWKEPEEPNGQIQGYRVYYTMDPTQHVNNWMKHNVADSQITTIGNLV
PQKTYSVKVLAFTSIGDGPLSSDIQVITQTGVPGQPLNFKAEPESETSILLSWTPPRSDTIANYELVYK
DGEHGEEQRITIEPGTSYRLQGLKPNSLYYFRLAARSPQGLGASTAEISARTMQSKPSAPPQDISCTSP
SSTSILVSWQPPPVEKQNGIITEYSIKYTAVDGEDDKPHEILGIPSDTTKYLLEQLEKWTEYRITVTAH
TDVGPGPESLSVLIRTNEDVPSGPPRKVEVEAVNSTSVKVSWRSPVPNKQHGQIRGYQVHYVRMENGEP
KGQPMLKDVMLADAQWEFDDTTEHDMIISGLQPETSYSLTVTAYTTKGDGARSKPKLVSTTGAVPGKPR
LVINHTQMNTALIQWHPPVDTFGPLQGYRLKFGRKDMEPLTTLEFSEKEDHFTATDIHKGASYVFRLSA
RNKVGFGEEMVKEISIPEEVPTGFPQNLHSEGTTSTSVQLSWQPPVLAERNGIITKYTLLYRDINIPLL
PMEQLIVPADTTMTLTGLKPDTTYDVKVRAHTSKGPGPYSPSVQFRTLPVDQVFAKNFHVKAVMKISVL
LSWEIPENYNSAMPFK**ILYDDGKMVEEVDGR**ATQKLIVNLKPEKSYSFVLTNRGNSAGGLQHRVTAKTA
PDVLRTKPAFIGKTNLDGMITVQLPEVPANENIKGYYIIIVPLKKSRGKFIKPWESPDEMELDELLKEI
SRKRRSIRYGREVELKPYIAAHFDVLPTEFTLGDDKHYGGFTNKQLQSGQEYVFFVLAVMEHAESKMYA
TSPYSDPVVSMDLDPQPITDEEEGLIWVVGPVLAVVFIICIVIAILLYKRKRAESDSRKSSIPNNKEIP
SHHPTDPVELRRLNFQTPGMASHPPIPILELADHIERLKANDNLKFSQEYESIDPGQQFTWEHSNLEVN
KPKNRYANVIAYDHSRVLLSAIEGIPGSDYVNANYIDGYRKQNAYIATQGSLPETFGDFWRMIWEQRSA
TVVMMTKLEERSRVKCDQYWPSRGTETHGLVQVTLLDTVELATYCVRTFALYKNGSSEKREVRQFQFTA
WPDHGVPEHPTPFLAFLRRVKTCNPPDAGPMVVHCSAGVGRTGCFIVIDAMLERIKHEKTVDIYGHVTL
MRAQRNYMVQTEDQYIFIHDALLEAVTCGNTEVPARNLYAYIQKLTQIETGENVTGMELEFKRLASSKA
HTSRFISANLPCNKFKNRLVNIMPYESTRVCLQPIRGVEGSDYINASFIDGYRQQKAYIATQGPLAETT
EDFWRMLWEHNSTIVVMLTKLREMGREKCHQYWPAERSARYQYFVVDPMAEYNMPQYILREFKVTDARD

GQSRTVRQFQFTDWPEQGVPKSGEGFIDFIGQVHKTKEQFGQDGPISVHCSAGVGRTGVFITLSIVLER
MRYEGVVDIFQTVKMLRTQRPAMVQTEDQYQFSYRAALEYLGSFDHYAT

**Mass Match Peptides (bold):**
    ILYDDGKMVEEVDGR [923]

**Tandem Peptides (underline):**
    ILYDDGKMVEEVDGR [923]


## OGTA104 (SEQ ID No: 20)

### Peptide Source: 1D-GE, Breast cancer

MGSGARFPSGTLRVRWLLLLGLVGPVLGAARPGFQQTSHLSSYEIITPWRLTRERREAPRPYSKQVSYV
IQAEGKEHIIHLERNKDLLPEDFVVYTYNKEGTLITDHPNIQNHCHYRGYVEGVHNSSIALSDCFGLRG
LLHLENASYGIEPLQNSSHFEHIIYRMDDVYKEPLKCGVSNKDIEKETAKDEEEEPPSMTQLLRRRRAV
LPQTRYVELFIVVDKERYDMMGRNQTAVREEMILLANYLDSMYIMLNIRIVLVGLEIWTNGNLINIVGG
AGDVLGNFVQWREKFLITRRRHDSAQLVLKKGFGGTAGMAFVGTVCSRSHAGGINVFGQITVETFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGKLQCENVQEIPVFGIVPAIIQTPSRGTKCWGVDFQLGSDVPDPGMVNEGT
KCGAGKICRNFQCVDASVLNYDCDVQKKCHGHGVCNSNKNCHCENGWAPPNCETKGYGGSVDSGPIYNE
MNTALRDGLLVFFFLIVPLIVCAIFIFIKRDQLWRSYFRKKRSQTYESDGKNQANPSRQPGSVPRHVSP
VTPPR<u>**EVPIYANR**</u><u>FAVPTYAAK</u>**QPQQFPSRPPPPQPK**VSSQGNLIPARPAPAPPLYSSLT


**Mass Match Peptides (bold):**
    EVPIYANR [236]
    QPQQFPSRPPPPQPK [586]

**Tandem Peptides (underline):**
    FAVPTYAAK [244]


### Peptide Source: 1D-GE, Hepatocellular carcinoma

MGSGARFPSGTLRVRWLLLLGLVGPVLGAARPGFQQTSHLSSYEIITPWRLTRERREAPRPYSKQVSYV
IQAEGKEHIIHLERNK**DLLPEDFVVYTYNK**EGTLITDHPNIQNHCHYRGYVEGVHNSSIALSDCFGLRG
LLHLENASYGIEPLQNSSHFEHIIYRMDDVYKEPLKCGVSNKDIEKETAK**DEEEEPPSMTQLLR**RRRAV
LPQTRYVELFIVVDKERYDMMGRNQTAVREEMILLANYLDSMYIMLNIRIVLVGLEIWTNGNLINIVGG
AGDVLGNFVQWREKFLITRRRHDSAQLVLK**KGFGGTAGMAFVGTVCSR**SHAGGINVFGQITVETFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGKLQCENVQEIPVFGIVPAIIQTPSRGTKCWGVDFQLGSDVPDPGMVNEGT
KCGAGKICRNFQCVDASVLNYDCDVQKKCHGHGVCNSNKNCHCENGWAPPNCETKGYGGSVDSGPIYNE
MNTALRDGLLVFFFLIVPLIVCAIFIFIK<u>RDQLWR</u>SYFRKKRSQTYESDGKNQANPSR<u>QPGSVPR</u>HVSP
VTPPR<u>**EVPIYANR**</u>FAVPTYAAK<u>**QPQQFPSRPPPPQPK**</u>VSSQGNLIPARPAPAPPLYSSLT


**Mass Match Peptides (bold):**
    DEEEEPPSMTQLLR [113]
    DLLPEDFVVYTYNK [134]
    EVPIYANR [236]
    KGFGGTAGMAFVGTVCSR [419]
    QPGSVPR [584]

QPQQFPSRPPPPQPK [586]
RDQLWR [600]

**Tandem Peptides (underline):**
EVPIYANR [236]


## Peptide Source: 1D-GE, Ovarian cancer

MGSGARFPSGTLRVRWLLLLGLVGPVLGAARPGFQQTSHLSSYEIITPWRLTRERREAPRPYSKQVSYV
IQAEGKEHIIHLERNKDLLPEDFVVYTYNKEGTLITDHPNIQNHCHYRGYVEGVHNSSIALSDCFGLRG
LLHLENASYGIEPLQNSSHFEHIIYRMDDVYKEPLKCGVSNKDIEKETAKDEEEEPPSMTQLLRRRRAV
LPQTRYVELFIVVDKERYDMMGRNQTAVREEMILLANYLDSMYIMLNIRIVLVGLEIWTNGNLINIVGG
AGDVLGNFVQWREKFLITRRRHDSAQLVLKKGFGGTAGMAFVGTVCSRSHAGGINVFGQITVETFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGKLQCENVQEIPVFGIVPAIIQTPSRGTKCWGVDFQLGSDVPDPGMVNEGT
KCGAGKICRNFQCVDASVLNYDCDVQKKCHGHGVCNSNKNCHCENGWAPPNCETKGYGGSVDSGPTYNE
MNTALRDGLLVFFFLIVPLIVCAIFIFIKRDQLWRSYFRKKRSQTYESDGKNQANPSRQPGSVPRHVSP
VTPPREVPIYANRFAVPTYAAKQPQQFPSRPPPPQPKVSSQGNLIPARPAPAPPLYSSLT


**Mass Match Peptides (bold):**
DQLWR [152]
EVPIYANR [236]
FLPGGTLCR [260]
FPSGTLR [264]
HVSPVTPPR [346]
KGFGGTAGMAFVGTVCSR [419]
QPQQFPSRPPPPQPK [586]
SYFRK [666]

**Tandem Peptides (underline):**
EVPIYANR [236]
FAVPTYAAK [244]


## Peptide Source: 1D-GE, Pancreatic cancer

MGSGARFPSGTLRVRWLLLLGLVGPVLGAARPGFQQTSHLSSYEIITPWRLTRERREAPRPYSKQVSYV
IQAEGKEHIIHLERNKDLLPEDFVVYTYNKEGTLITDHPNIQNHCHYRGYVEGVHNSSIALSDCFGLRG
LLHLENASYGIEPLQNSSHFEHIIYRMDDVYKEPLKCGVSNKDIEKETAKDEEEEPPSMTQLLRRRRAV
LPQTRYVELFIVVDKERYDMMGRNQTAVREEMILLANYLDSMYIMLNIRIVLVGLEIWTNGNLINIVGG
AGDVLGNFVQWREKFLITRRRHDSAQLVLKKGFGGTAGMAFVGTVCSRSHAGGINVFGQITVETFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGKLQCENVQEIPVFGIVPAIIQTPSRGTKCWGVDFQLGSDVPDPGMVNEGT
KCGAGKICRNFQCVDASVLNYDCDVQKKCHGHGVCNSNKNCHCENGWAPPNCETKGYGGSVDSGPTYNE
MNTALRDGLLVFFFLIVPLIVCAIFIFIKRDQLWRSYFRKKRSQTYESDGKNQANPSRQPGSVPRHVSP
VTPPREVPIYANRFAVPTYAAKQPQQFPSRPPPPQPKVSSQGNLIPARPAPAPPLYSSLT


**Mass Match Peptides (bold):**
EEMILLANYLDSMYIMLNIR [179]
EVPIYANR [236]
KGFGGTAGMAFVGTVCSR [419]

QPQQFPSRPPPPQPK [586]

**Tandem Peptides (underline:**
HVSPVTPPR [346]


## Peptide Source: iTRAQ, Colorectal cancer

MGSGAR<u>FPSGTLR</u>VRWLLLLGLVGPVLGAARPGFQQTSHLSSYEIITPWRLTRERREAPRPYSKQVSYV
IQAEGKEHIIHLERNKDLLPEDFVVYTYNKEGTLITDHPNIQNHCHYRGYVEGVHNSSIALSDCFGLRG
LLHLENASYGIEPLQNSSHFEHIIYRMDDVYKEPLKCGVSNKDIEKETAKDEEEEPPSMTQLLRRRRAV
LPQTRYVELFIVVDKERYDMMGR<u>NQTAVR</u>EEMILLANYLDSMYIMLNIRIVLVGLEIWTNGNLINIVGG
AGDVLGNFVQWREKFLITRRRHDSAQLVLKKGFGGTAGMAFVGTVCSRSHAGGINVFGQITVEIFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGK<u>LQCENVQEIPVFGIVPAIIQTPSR</u>GTKCWGVDFQLGSDVPDPGMVNEGT
KCGAGKICRNFQCVDASVLNYDCDVQKKCHGHGVCNSNKNCHCENGWAPPNCETKGYGGSVDSGPIYNE
MNTALRDGLLVIFFLIVPLIVCAIFIFIKRDQLWRSYFRKKRSQTYESDGKNQANPSRQPGSVPRHVSP
VTPPREVPIYANRFAVPTYAAKQPQQFPSRPPPPQPKVSSQGNLIPARPAPAPPLYSSLT


**Mass Match Peptides (bold):**
FPSGTLR [264]
LQCENVQEIPVFGIVPAIIQTPSR [943]
NQTAVR [959]
**Tandem Peptides (underline:**
FPSGTLR [264]
LQCENVQEIPVFGIVPAIIQTPSR [943]
NQTAVR [959]


## Peptide Source: iTRAQ, Hepatocellular cancer

MGSGARFPSGTLRVRWLLLLGLVGPVLGAARPGFQQTSHLSSYEIITPWRLTRERREAPRPYSKQVSYV
IQAEGKEHIIHLERNKDLLPEDFVVYTYNKEGTLITDHPNIQNHCHYRGYVEGVHNSSIALSDCFGLRG
LLHLENASYGIEPLQNSSHFEHIIYRMDDVYKEPLKCGVSNKDIEKETAKDEEEEPPSMTQLLRRRRAV
LPQTRYVELFIVVDKERYDMMGRNQTAVREEMILLANYLDSMYIMLNIRIVLVGLEIWTNGNLINIVGG
AGDVLGNFVQWREKFLITRRRHDSAQLVLKKGFGGTAGMAFVGTVCSRSHAGGINVFGQITVEIFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGKLQCENVQEIPVFGIVPAIIQTPSRGTKCWGVDFQLGSDVPDPGMVNEGT
KCGAGKICRNFQCVDASVLNYDCDVQKKCHGHGVCNSNKNCHCENGWAPPNCETKGYGGSVDSGPIYNE
MNTALRDGLLVIFFLIVPLIVCAIFIFIKRDQLWRSYFRKKR<u>SQTYESDGK</u>NQANPSRQPGSVPRHVSP
VTPPREVPIYANRFAVPTYAAKQPQQFPSRPPPPQPKVSSQGNLIPARPAPAPPLYSSLT


**Mass Match Peptides (bold):**
SQTYESDGK [972]
**Tandem Peptides (underline:**
SQTYESDGK [972]


## Peptide Source: iTRAQ, Non-small cell lung cancer

MGSGARFPSGTLRVRWLLLLGLVGPVLGAARPGFQQTSHLSSYEIITPWRLTRERREAPRPYSKQVSYV
IQAEGKEHIIHLERNKDLLPEDFVVYTYNKEGTLITDHPNIQNHCHYRGYVEGVHNSSIALSDCFGLRG
LLHLENASYGIEPLQNSSHFEHIIYRMDDVYKEPLKCGVSNKDIEKETAKDEEEEPPSMTQLLRRRRAV
LPQTRYVELFIVVDKERYDMMGRNQTAVREEMILLANYLDSMYIMLNIRIVLVGLEIWTNGNLINIVGG
AGDVLGNFVQWREKFLITRRRHDSAQLVLKKGFGGTAGMAFVGTVCSRSHAGGINVFGQITVETFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGKLQCENVQEIPVFGIVPAIIQTPSRGTKCWGVDFQLGSDVPDPGMVNEGT
KCGAGKICRNFQCVDASVLNYDCDVQKKCHGHGVCNSNKNCHCENGWAPPNCETKGYGGSVDSGPTYNE
MNTALRDGLLVFFFLIVPLIVCAIFIFIKRDQLWRSYFRKKRSQTYESDGKNQANPSRQPGSVPRHVSP
VTPPREVPIYANRFAVPTYAAKQPQQFPSRPPPPQPKVSSQGNLIPARPAPAPPLYSSLT

**Mass Match Peptides (bold):**
    CGVSNKDIEK [887]
    FLITRRR [910]

**Tandem Peptides (underline:**
    CGVSNKDIEK [887]
    FLITRRR [910]


## Peptide Source: iTRAQ, Ovarian cancer

MGSGARFPSGTLRVRWLLLLGLVGPVLGAARPGFQQTSHLSSYEIITPWRLTRERREAPRPYSKQVSYV
IQAEGKEHIIHLERNKDLLPEDFVVYTYNKEGTLITDHPNIQNHCHYRGYVEGVHNSSIALSDCFGLRG
LLHLENASYGIEPLQNSSHFEHIIYRMDDVYKEPLKCGVSNKDIEKETAKDEEEEPPSMTQLLRRRRAV
LPQTRYVELFIVVDKERYDMMGRNQTAVREEMILLANYLDSMYIMLNIRIVLVGLEIWTNGNLINIVGG
AGDVLGNFVQWREKFLITRRRHDSAQLVLKKGFGGTAGMAFVGTVCSRSHAGGINVFGQITVETFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGKLQCENVQEIPVFGIVPAIIQTPSRGTKCWGVDFQLGSDVPDPGMVNEGT
KCGAGKICRNFQCVDASVLNYDCDVQKKCHGHGVCNSNKNCHCENGWAPPNCETKGYGGSVDSGPTYNE
MNTALRDGLLVFFFLIVPLIVCAIFIFIKRDQLWRSYFRKKRSQTYESDGKNQANPSRQPGSVPRHVSP
VTPPREVPIYANRFAVPTYAAKQPQQFPSRPPPPQPKVSSQGNLIPARPAPAPPLYSSLT

**Mass Match Peptides (bold):**
    FLITRRR [910]

**Tandem Peptides (underline:**
    FLITRRR [910]


## Peptide Source: iTRAQ, Renal cell cancer

MGSGARFPSGTLRVRWLLLLGLVGPVLGAARPGFQQTSHLSSYEIITPWRLTRERREAPRPYSKQVSYV
IQAEGKEHIIHLERNKDLLPEDFVVYTYNKEGTLITDHPNIQNHCHYRGYVEGVHNSSIALSDCFGLRG
LLHLENASYGIEPLQNSSHFEHIIYRMDDVYKEPLKCGVSNKDIEKETAKDEEEEPPSMTQLLRRRRAV
LPQTRYVELFIVVDKERYDMMGRNQTAVREEMILLANYLDSMYIMLNIRIVLVGLEIWTNGNLINIVGG
AGDVLGNFVQWREKFLITRRRHDSAQLVLKKGFGGTAGMAFVGTVCSRSHAGGINVFGQITVETFASIV
AHELGHNLGMNHDDGRDCSCGAKSCIMNSGASGSRNFSSCSAEDFEKLTLNKGGNCLLNIPKPDEAYSA
PSCGNKLVDAGEECDCGTPKECELDPCCEGSTCKLKSFAECAYGDCCKDCRFLPGGTLCRGKTSECDVP
EYCNGSSQFCQPDVFIQNGYPCQNNKAYCYNGMCQYYDAQCQVIFGSKAKAAPKDCFIEVNSKGDRFGN
CGFSGNEYKKCATGNALCGKLQCENVQEIPVFGIVPAIIQTPSRGTKCWGVDFQLGSDVPDPGMVNEGT
KCGAGKICRNFQCVDASVLNYDCDVQKKCHGHGVCNSNKNCHCENGWAPPNCETKGYGGSVDSGPTYNE

MNTALRDGLLVFFFLIVPLIVCAIFIFIKRDQLWRSYFRKKRSQTYESDGKNQANPSRQPGSVPRHVSP
VTPPREVPIYANRFAVPTYAAKQPQQFPSRPPPPQPKVSSQGNLIPARPAPAPPLYSSLT

**Mass Match Peptides (bold):**
FLITRRR [910]

**Tandem Peptides (underline):**
FLITRRR [910]

## OGTA106 (SEQ ID No: 21)

## Peptide Source: 1D-GE, Cervical cancer

MQPPSLLLLLLLLLLLLCVSVVRPRGLLCGSFPEPCANGGTCLSLSLGQGTCQCAPGFLGETCQFPDPCQ
NAQLCQNGGSCQALLPAPLGLPSSPSPLTPSFLCTCLPGFTGERCQAKLEDPCPPSFCSKRGRCHIQAS
GRPQCSCMPGWTGEQCQLRDFCSANPCVNGGVCLATYPQIQCHCPPGFEGHACERDVNECFQDPGPCPK
GTSCHNTLGSFQCLCPVGQEGPRCELRAGPCPPRGCSNGGICQLMPEKDSTFHLCLCPPGFIGPDCEVN
PDNCVSHQCQNGGTCQDGLDTYTCLCPETWTGWDCSEDVDECETQGPPHCRNGGTCQNSAGSFHCVCVS
GWGGTSCEENLDDCIAATCAPGSTCIDRVGSFSCLCPPGRTGLLCHLEDMCLSQPCHGDAQCSTNPLTG
STLCLCQPGYSGPTCHQDLDECLMAQQGPSPCEHGGSCLNTPGSFNCLCPPGYTGSRCEADHNECLSQP
CHPGSTCLDLLATFHCLCPPGLEGQLCEVETNECASAPCLNHADCHDLLNGFQCICLPGFSGTRCEEDI
DECRSSPCANGGQCQDQPGAFHCKCLPGFEGPRCQTEVDECLSDPCPVGASCLDLPGAFFCLCPSGFTG
QLCEVPLCAPNLCQPKQICKDQKDKANCLCPDGSPGCAPPEDNCTCHHGHCQRSSCVCDVGWTGPECEA
ELGGCISAPCAHGGTCYPQPSGYNCTCPTGYTGPTCSEEMTACHSGPCLNGGSCNPSPGGYYCTCPPSH
TGPQCQTSTDYCVSAPCFNGGTCVNRPGTFSCLCAMGFQGPRCEGKLRPSCADSPCRNRATCQDSPQGP
RCLCPTGYTGGSCQTLMDLCAQKPCPRNSHCLQTGPSFHCLCLQGWTGPLCNLPLSSCQKAALSQGIDV
SSLCHNGGLCVDSGPSYFCHCPPGFQGSLCQDHVNPCESRPCQNGATCMAQPSGYLCQCAPGYDGQNCS
KELDACQSQPCHNHGTCTPKPGGFHCACPPGFVGLRCEGDVDECLDQPCHPTGTAACHSLANAFYCQCL
PGHTGQWCEVEIDPCHSQPCFHGGTCEATAGSPLGFICHCPKGFEGPTCSHRAPSCGFHHCHHGGLCLP
SPKPGFPPRCACLSGYGGPDCLTPPAPKGCGPPSPCLYNGSCSETTGLGGPGFRCSCPHSSPGPRCQKP
GAKGCEGRSGDGACDAGCSGPGGNWDGGDCSLGVPDPWKGCPSHSRCWLLFRDGQCHPQCDSEECLFDG
YDCETPPACTPAYDQYCHDHFHNGHCEKGCNTAECGWDGGDCRPEDGDPEWGPSLALLVVLSPPALDQQ
LFALARVLSLTLRVGLWVRKDRDGRDMVYPYPGARAEEKLGGTRDPTYQERAAPQTQPLGKETDSLSAG
FVVVMGVDLSRCGPDHPASRCPWDPGLLLRFLAAMAAVGALEPLLPGPLLAVHPHAGTAPPANQLPWPV
LCSPVAGVILLALGALLVLQLIRRRREHGALWLPPGFTRRPRTQSAPHRRRPPLGEDSIGLKALKPKA
EVDEDGVVMCSGPEEGEEVGQAEETGPPSTCQLWSLSGGCGALPQAAMLTPPQESEMEAPDLDTRGPDG
VTPLMSAVCCGEVQSGTFQGAWLGCPEPWEPLLDGGACPQAHTVGTGETPLHLAARFSRPTAARRLLEA
GANPNQPDRAGRTPLIIAAVAADAREVCQLLLRSRQTAVDARTEDGTTPLMLAARLAVEDLVEELIAAQA
DVGARDKWGKTALHWAAAVNNARAARSLLQAGADKDAQDNREQTPLFLAAREGAVEVAQLLLGLGAARE
LRDQAGLAPADVAHQRNHWDLLTLLEGAGPPEARHKATPGREAGPFPRARTVSVSVPPHGGGALPRCRT
LSAGAGPRGGGACLQARTWSVDLAARGGGAYSHCRSLSGVGAGGGPTPRGRRFSAGMRGPRPNPAIMRG
RYGVAAGRGGRVSTDDWPCDWVALGACGSASNIPIPPPCLTPSPERGSPQLDCGPPALQEMPINQGGEG
KK

**Mass Match Peptides (bold):**

**Tandem Peptides (underline):**
SLSGVGAGGGPTPR [642]

## Peptide Source: 1D-GE, Melanoma

MQPPSLLLLLLLLLLLLCVSVVRPRGLLCGSFPEPCANGGTCLSLSLGQGTCQCAPGFLGETCQFPDPCQ
NAQLCQNGGSCQALLPAPLGLPSSPSPLTPSFLCTCLPGFTGERCQAKLEDPCPPSFCSKRGRCHIQAS
GRPQCSCMPGWTGEQCQLRDFCSANPCVNGGVCLATYPQIQCHCPPGFEGHACERDVNECFQDPGPCPK

GTSCHNTLGSFQCLCPVGQEGPRCELRAGPCPPRGCSNGGTCQLMPEKDSTFHLCLCPPGFIGPDCEVN
PDNCVSHQCQNGGTCQDGLDTYTCLCPETWTGWDCSEDVDECETQGPPHCRNGGTCQNSAGSFHCVCVS
GWGGTSCEENLDDCIAATCAPGSTCIDRVGSFSCLCPPGRTGLLCHLEDMCLSQPCHGDAQCSTNPLTG
STLCLCQPGYSGPTCHQDLDECLMAQQGPSPCEHGGSCLNTPGSFNCLCPPGYTGSRCEADHNECLSQP
CHPGSTCLDLLATFHCLCPPGLEGQLCEVETNECASAPCLNHADCHDLLNGFQCICLPGFSGTRCEEDI
DECRSSPCANGGQCQDQPGAFHCKCLPGFEGPRCQTEVDECLSDPCPVGASCLDLPGAFFCLCPSGFTG
QLCEVPLCAPNLCQPKQICKDQKDKANCLCPDGSPGCAPPEDNCTCHHGHCQRSSCVCDVGWTGPECEA
ELGGCISAPCAHGGTCYPQPSGYNCTCPTGYTGPTCSEEMTACHSGPCLNGGSCNPSPGGYYCTCPPSH
TGPQCQTSTDYCVSAPCFNGGTCVNRPGTFSCLCAMGFQGPRCEGKLRPSCADSPCRNRATCQDSPQGP
RCLCPTGYTGGSCQTLMDLCAQKPCPRNSHCLQTGPSFHCLCLQGWTGPLCNLPLSSCQKAALSQGIDV
SSLCHNGGLCVDSGPSYFCHCPPGFQGSLCQDHVNPCESRPCQNGATCMAQPSGYLCQCAPGYDGQNCS
KELDACQSQPCHNHGTCTPKPGGFHCACPPGFVGLRCEGDVDECLDQPCHPTGTAACHSLANAFYCQCL
PGHTGQWCEVEIDPCHSQPCFHGGTCEATAGSPLGFICHCPKGFEGPTCSHRAPSCGFHHCHHGGLCLP
SPKPGFPPRCACLSGYGGPDCLTPPAPKGCGPPSPCLYNGSCSETTGLGGPGFRCSCPHSSPGPRCQKP
GAKGCEGRSGDGACDAGCSGPGGNWDGGDCSLGVPDPWKGCPSHSRCWLLFRDGQCHPQCDSEECLFDG
YDCETPPACTPAYDQYCHDHFHNGHCEKGCNTAECGWDGGDCRPEDGDPEWGPSLALLVVLSPPALDQQ
LFALARVLSLTLRVGLWVRKDRDGRDMVYPYPGARAEEKLGGTRDPTYQERAAPQTQPLGK**ETDSLSAG
FVVVMGVDLSR**CGPDHPASRCPWDPGLLLRFLAAMAAVGALEPLLPGPLLAVHPHAGTAPPANQLPWPV
LCSPVAGVILLALGALLVLQLIRRRRREHGALWLPPGFTRRPRTQSAPHRRRPPLGEDSIGLKALKPKA
EVDEDGVVMCSGPEEGEEVGQAEETGPPSTCQLWSLSGGCGALPQAAMLTPPQESEMEAPDLDTRGPDG
VTPLMSAVCCGEVQSGTFQGAWLGCPEPWEPLLDGGACPQAHTVGTGETPLHLAARFSRPTAARRLLEA
GANPNQPDRAGR**TPLHAAVAADAR**EVCQLLLRSRQTAVDARTEDGTTPLMLAARLAVEDLVEELIAAQA
DVGARDKWGKTALHWAAAVNNARAARSLLQAGADKDAQDNR<u>**EQTPLFLAAR**</u>EGAVEVAQLLLGLGAARE
LRDQAGLAPADVAHQRNHWDLLTLLEGAGPPEARHKATPGREAGPFPRARTVSVSVPPHGGGALPRCR**T
LSAGAGPR**GGGACLQARTWSVDLAARGGGAYSHCRSLSGVGAGGGPTPRGRRFSAGMR**GPRPNPAIMR**G
RYGVAAGRGGRVSTDDWPCDWVALGACGSASNIPIPPPCLTPSPER**GSPQLDCGPPALQEMPINQGGEG
KK**

**Mass Match Peptides (bold):**
    CHIQASGRPQCSCMPGWTGEQCQLR [103]
    EQTPLFLAAR [221]
    ETDSLSAGFVVVMGVDLSR [229]
    GPRPNPAIMR [310]
    GSPQLDCGPPALQEMPINQGGEGKK [318]
    TLSAGAGPR [705]
    TPLHAAVAADAR [717]

**Tandem Peptides (underline):**
    EQTPLFLAAR [221]


**Peptide Source: 1D-GE, Pancreatic cancer**

MQPPSLLLLLLLLLLLLCVSVVRPRGLLCGSFPEPCANGGTCLSLSLGQGTCQCAPGFLGETCQFPDPCQ
NAQLCQNGGSCQALLPAPLGLPSSPSPLTPSFLCTCLPGFTGERCQAKLEDPCPPSFCSKRGRCHIQAS
GRPQCSCMPGWTGEQCQLRDFCSANPCVNGGVCLATYPQIQCHCPPGFEGHACERDVNECFQDPGPCPK
GTSCHNTLGSFQCLCPVGQEGPRCELRAGPCPPRGCSNGGTCQLMPEKDSTFHLCLCPPGFIGPDCEVN
PDNCVSHQCQNGGTCQDGLDTYTCLCPETWTGWDCSEDVDECETQGPPHCRNGGTCQNSAGSFHCVCVS
GWGGTSCEENLDDCIAATCAPGSTCIDRVGSFSCLCPPGRTGLLCHLEDMCLSQPCHGDAQCSTNPLTG
STLCLCQPGYSGPTCHQDLDECLMAQQGPSPCEHGGSCLNTPGSFNCLCPPGYTGSRCEADHNECLSQP
CHPGSTCLDLLATFHCLCPPGLEGQLCEVETNECASAPCLNHADCHDLLNGFQCICLPGFSGTRCEEDI
DECRSSPCANGGQCQDQPGAFHCKCLPGFEGPRCQTEVDECLSDPCPVGASCLDLPGAFFCLCPSGFTG
QLCEVPLCAPNLCQPKQICKDQKDKANCLCPDGSPGCAPPEDNCTCHHGHCQRSSCVCDVGWTGPECEA
ELGGCISAPCAHGGTCYPQPSGYNCTCPTGYTGPTCSEEMTACHSGPCLNGGSCNPSPGGYYCTCPPSH
TGPQCQTSTDYCVSAPCFNGGTCVNRPGTFSCLCAMGFQGPRCEGKLRPSCADSPCRNRATCQDSPQGP
RCLCPTGYTGGSCQTLMDLCAQKPCPRNSHCLQTGPSFHCLCLQGWTGPLCNLPLSSCQKAALSQGIDV
SSLCHNGGLCVDSGPSYFCHCPPGFQGSLCQDHVNPCESRPCQNGATCMAQPSGYLCQCAPGYDGQNCS

KELDACQSQPCHNHGTCTPKPGGFHCACPPGFVGLRCEGDVDECLDQPCHPTGTAACHSLANAFYCQCL
PGHTGQWCEVEIDPCHSQPCFHGGTCEATAGSPLGFICHCPKGFEGPTCSHRAPSCGFHHCHHGGLCLP
SPKPGFPPRCACLSGYGGPDCLTPPAPKGCGPPSPCLYNGSCSETTGLGGPGFRCSCPHSSPGPRCQKP
GAKGCEGRSGDGACDAGCSGPGGNWDGGDCSLGVPDPWKGCPSHSRCWLLFRDGQCHPQCDSEECLFDG
YDCETPPACTPAYDQYCHDHFHNGHCEKGCNTAECGWDGGDCRPEDGDPEWGPSLALLVVLSPPALDQQ
LFALARVLSLTLRVGLWVRKDRDGRDMVYPYPGARAEEKLGGTRDPTYQERAAPQTQPLGKETDSLSAG
FVVVMGVDLSRCGPDHPASRCPWDPGLLLRFLAAMAAVGALEPLLPGPLLAVHPHAGTAPPANQLPWPV
LCSPVAGVILLALGALLVLQLIRRRRREHGALWLPPGFTRRPRTQSAPHRRRPPLGEDSIGLKALKPKA
EVDEDGVVMCSGPEEGEEVGQAEETGPPSTCQLWSLSGGCGALPQAAMLTPPQESEMEAPDLDTRGPDG
VTPLMSAVCCGEVQSGTFQGAWLGCPEPWEPLLDGGACPQAHTVGTGETPLHLAARFSRPTAARRLLEA
GANPNQPDRAGRTPLHAAVAADAREVCQLLLRSRQTAVDARTEDGTTPLMLAARLAVEDLVEELIAAQA
DVGARDKWGKTALHWAAAVNNARAARSLLQAGADKDAQDNR**EQTPLFLAAR**EGAVEVAQLLLGLGAARE
LRDQAGLAPADVAHQRNHWDLLTLLEGAGPPEARHKATPGREAGPFPRARTVSVSVPPHGGGALPRCRT
LSAGAGPRGGGACLQARTWSVDLAARGGGAYSHCRSLSGVGAGGGPTPRGRRFSAGMRGPRPNPAIMRG
RYGVAAGRGGRVSTDDWPCDWVALGACGSASNIPIPPPCLTPSPERGSPQLDCGPPALQEMPINQGGEG
KK

**Mass Match Peptides (bold):**
EQTPLFLAAR [221]

**Tandem Peptides (underline):**
EQTPLFLAAR [221]


## Peptide Source: iTRAQ, Hepatocellular cancer

MQPPSLLLLLLLLLLLCVSVVRPRGLLCGSFPEPCANGGTCLSLSLGQGTCQCAPGFLGETCQFPDPCQ
NAQLCQNGGSCQALLPAPLGLPSSPSPLTPSFLCTCLPGFTGERCQAKLEDPCPPSFCSKRGRCHIQAS
GRPQCSCMPGWTGEQCQLRDFCSANPCVNGGVCLATYPQIQCHCPPGFEGHACERDVNECFQDPGPCPK
GTSCHNTLGSFQCLCPVGQEGPRCELRAGPCPPRGCSNGGTCQLMPEKDSTFHLCLCPPGFIGPDCEVN
PDNCVSHQCQNGGTCQDGLDTYTCLCPETWTGWDCSEDVDECETQGPPHCRNGGTCQNSAGSFHCVCVS
GWGGTSCEENLDDCIAATCAPGSTCIDRVGSFSCLCPPGRTGLLCHLEDMCLSQPCHGDAQCSTNPLTG
STLCLCQPGYSGPTCHQDLDECLMAQQGPSPCEHGGSCLNTPGSFNCLCPPGYTGSRCEADHNECLSQP
CHPGSTCLDLLATFHCLCPPGLEGQLCEVETNECASAPCLNHADCHDLLNGFQCICLPGFSGTRCEEDI
DECRSSPCANGGQCQDQPGAFHCKCLPGFEGPRCQTEVDECLSDPCPVGASCLDLPGAFFCLCPSGFTG
QLCEVPLCAPNLCQPKQICKDQKDKANCLCPDGSPGCAPPEDNCTCHHGHCQRSSCVCDVGWTGPECEA
ELGGCISAPCAHGGTCYPQPSGYNCTCPTGYTGPTCSEEMTACHSGPCLNGGSCNPSPGGYYCTCPPSH
TGPQCQTSTDYCVSAPCFNGGTCVNRPGTFSCLCAMGFQGPRCEGKLRPSCADSPCRNRATCQDSPQGP
RCLCPTGYTGGSCQTLMDLCAQKPCPRNSHCLQTGPSFHCLCLQGWTGPLCNLPLSSCQKAALSQGIDV
SSLCHNGGLCVDSGPSYFCHCPPGFQGSLCQDHVNPCESRPCQNGATCMAQPSGYLCQCAPGYDGQNCS
KELDACQSQPCHNHGTCTPKPGGFHCACPPGFVGLRCEGDVDECLDQPCHPTGTAACHSLANAFYCQCL
PGHTGQWCEVEIDPCHSQPCFHGGTCEATAGSPLGFICHCPKGFEGPTCSHRAPSCGFHHCHHGGLCLP
SPKPGFPPRCACLSGYGGPDCLTPPAPKGCGPPSPCLYNGSCSETTGLGGPGFRCSCPHSSPGPRCQKP
GAKGCEGRSGDGACDAGCSGPGGNWDGGDCSLGVPDPWKGCPSHSRCWLLFRDGQCHPQCDSEECLFDG
YDCETPPACTPAYDQYCHDHFHNGHCEKGCNTAECGWDGGDCRPEDGDPEWGPSLALLVVLSPPALDQQ
LFALARVLSLTLRVGLWVRKDRDGRDMVYPYPGARAEEKLGGTRDPTYQERAAPQTQPLGKETDSLSAG
FVVVMGVDLSRCGPDHPASRCPWDPGLLLRFLAAMAAVGALEPLLPGPLLAVHPHAGTAPPANQLPWPV
LCSPVAGVILLALGALLVLQLIRRRRREHGALWLPPGFTRRPRTQSAPHRRRPPLGEDSIGLKALKPKA
EVDEDGVVMCSGPEEGEEVGQAEETGPPSTCQLWSLSGGCGALPQAAMLTPPQESEMEAPDLDTRGPDG
VTPLMSAVCCGEVQSGTFQGAWLGCPEPWEPLLDGGACPQAHTVGTGETPLHLAARFSRPTAARRLLEA
GANPNQPDRAGRTPLHAAVAADAR**EVCQLLLR**SRQTAVDARTEDGTTPLMLAARLAVEDLVEELIAAQA
DVGARDKWGKTALHWAAAVNNARAARSLLQAGADKDAQDNREQTPLFLAAREGAVEVAQLLLGLGAARE
LRDQAGLAPADVAHQRNHWDLLTLLEGAGPPEARHKATPGREAGPFPRARTVSVSVPPHGGGALPRCRT
LSAGAGPRGGGACLQARTWSVDLAARGGGAYSHCRSLSGVGAGGGPTPRGRRFSAGMRGPRPNPAIMRG
RYGVAAGRGGRVSTDDWPCDWVALGACGSASNIPIPPPCLTPSPERGSPQLDCGPPALQEMPINQGGEG
KK

599

    EVCQLLLR [908]

**Tandem Peptides (underline):**
    EVCQLLLR [908]


## OGTA112 (SEQ ID No: 22)

### Peptide Source: 1D-GE, Breast cancer

MNSLSEANTK**FMFDLFQQFR**KSKENNIFYSPISITSALGMVLLGAKDNTAQQISK**VLHFDQVTENTTEK**
AATYHVDRSGNVHHQFQKLLTEFNKSTDAYELKIANKLFGEK**TYQFLQEYLDAIKKFYQTSVESTDFAN**
**APEESRKKINSWVESQTNEK**IKNLFPDGTIGNDTTLVLVNAIYFKGQWENKFKKENTKEEKFWPNKNTY
KSVQMMR**QYNSFNFALLEDVQAK**VLEIPYKGKDLSMIVLLPNEIDGLQKLEEKLTAEK**LMEWTSLQNMR**
**ETCVDLHLPR**FKMEESYDLKDTLRTMGMVNIFNGDADLSGMTWSHGLSVSKVLHKAFVEVTEEGVEAAA
ATAVVVVELSSPSTNEEFCCNHPFLFFIRQNK**TNSILFYGR**FSSP


**Mass Match Peptides (bold):**
    ETCVDLHLPR [228]
    FMFDLFQQFR [261]
    FYQTSVESTDFANAPEESR [280]
    FYQTSVESTDFANAPEESRKK [281]
    KFYQTSVESTDFANAPEESRK [416]
    KINSWVESQTNEK [420]
    LMEWTSLQNMR [470]
    QYNSFNFALLEDVQAK [594]
    TNSILFYGR [713]
    TYQFLQEYLDAIK [741]
    VLHFDQVTENTTEK [781]

**Tandem Peptides (underline):**
    FMFDLFQQFR [261]
    LMEWTSLQNMR [470]


### Peptide Source: 1D-GE, Cervical cancer

MNSLSEANTK**FMFDLFQQFRK**SKENNIFYSPISITSALGMVLLGAKDNTAQQISKVLHFDQVTENTTEK
AATYHVDRSGNVHHQFQKLLTEFNKSTDAYELKIANKLFGEKTYQFLQEYLDAIKKFYQTSVESTDFAN
APEESRKKINSWVESQTNEKIKNLFPDGTIGNDTTLVLVNAIYFKGQWENKFKKENTKEEKFWPNKNTY
K**SVQMMR**QYNSFNFALLEDVQAK**VLEIPYK**GKDLSMIVLLPNEIDGLQKLEEKLTAEK**LMEWTSLQNMR**
ETCVDLHLPRFKMEESYDLKDTLR**TMGMVNIFNGDADLSGMTWSHGLSVSK**VLHKAFVEVTEEGVEAAA
ATAVVVVELSSPSTNEEFCCNHPFLFFIRQNKTNSILFYGRFSSP


**Mass Match Peptides (bold):**
    FMFDLFQQFRK [262]
    LMEWTSLQNMR [470]
    SVQMMR [665]
    TMGMVNIFNGDADLSGMTWSHGLSVSK [706]

**Tandem Peptides (underline):**
    LMEWTSLQNMR [470]
    VLEIPYK [779]

## Peptide Source: 1D-GE, Chronic lymphocytic leukaemia

MNSLSEANTKFMFDLFQQFRKSKENNIFYSPISITSALGMVLLGAKDNTAQQISKVLHFDQVTENTTEK
AATYHVDRSGNVHHQFQKLLTEFNKSTDAYELKIANKLFGEKTYQFLQEYLDAIKKFYQTSVESTDFAN
APEESRKKINSWVESQTNEKIKNLFPDGTIGNDTTLVLVNAIYFKGQWENKFKKENTKEEKFWPNKNTY
KSVQMMRQYNSFNFALLEDVQAKVLEIPYKGKDLSMIVLLPNEIDGLQKLEEKLTAEKLMEWTSLQNMR
ETCVDLHLPRFKMEESYDLKDTLRTMGMVNIFNGDADLSGMTWSHGLSVSKVLHKAFVEVTEEGVEAAA
ATAVVVVELSSPSTNEEFCCNHPFLFFIRQNKTNSILFYGRFSSP

### Mass Match Peptides (bold):
ETCVDLHLPR [228]

### Tandem Peptides (underline):
FMFDLFQQFR [261]


## Peptide Source: 1D-GE, Hepatocellular carcinoma

MNSLSEANTKFMFDLFQQFRKSKENNIFYSPISITSALGMVLLGAKDNTAQQISKVLHFDQVTENTTEK
AATYHVDRSGNVHHQFQKLLTEFNKSTDAYELKIANKLFGEKTYQFLQEYLDAIKKFYQTSVESTDFAN
APEESRKKINSWVESQTNEKIKNLFPDGTIGNDTTLVLVNAIYFKGQWENKFKKENTKEEKFWPNKNTY
KSVQMMRQYNSFNFALLEDVQAKVLEIPYKGKDLSMIVLLPNEIDGLQKLEEKLTAEKLMEWTSLQNMR
ETCVDLHLPRFKMEESYDLKDTLRTMGMVNIFNGDADLSGMTWSHGLSVSKVLHKAFVEVTEEGVEAAA
ATAVVVVELSSPSTNEEFCCNHPFLFFIRQNKTNSILFYGRFSSP

### Mass Match Peptides (bold):
AATYHVDR [54]
ETCVDLHLPR [228]
FMFDLFQQFR [261]
QYNSFNFALLEDVQAK [594]
TNSILFYGR [713]
VLHFDQVTENTTEK [781]

### Tandem Peptides (underline):
INSWVESQTNEK [384]
LMEWTSLQNMR [470]


## Peptide Source: 1D-GE, Pancreatic cancer

MNSLSEANTKFMFDLFQQFRKSKENNIFYSPISITSALGMVLLGAKDNTAQQISKVLHFDQVTENTTEK
AATYHVDRSGNVHHQFQKLLTEFNKSTDAYELKIANKLFGEKTYQFLQEYLDAIKKFYQTSVESTDFAN
APEESRKKINSWVESQTNEKIKNLFPDGTIGNDTTLVLVNAIYFKGQWENKFKKENTKEEKFWPNKNTY
KSVQMMRQYNSFNFALLEDVQAKVLEIPYKGKDLSMIVLLPNEIDGLQKLEEKLTAEKLMEWTSLQNMR
ETCVDLHLPRFKMEESYDLKDTLRTMGMVNIFNGDADLSGMTWSHGLSVSKVLHKAFVEVTEEGVEAAA
ATAVVVVELSSPSTNEEFCCNHPFLFFIRQNKTNSILFYGRFSSP

### Mass Match Peptides (bold):
ETCVDLHLPR [228]
FMFDLFQQFR [261]
FMFDLFQQFRK [262]
LMEWTSLQNMR [470]
SVQMMR [665]
TNSILFYGR [713]
VLHFDQVTENTTEK [781]

**Tandem Peptides (underline):**

        FMFDLFQQFR [261]
        INSWVESQTNEK [384]
        LMEWTSLQNMR [470]
        QYNSFNFALLEDVQAK [594]


## Peptide Source: 1D-GE, Renal cell cancer

MNSLSEANTKFMFDLFQQFRKSKENNIFYSPISITSALGMVLLGAKDNTAQQISK**VLHFDQVTENTTEK**
AATYHVDRSGNVHHQFQKLLTEFNKSTDAYELKIANKLFGEKTYQFLQEYLDAIKKFYQTSVESTDFAN
APEESRKK**INSWVESQTNEK**IKNLFPDGTIGNDTTLVLVNAIYFKGQWENKFKKENTKEEKFWPNKNTY
KS**VQMMR**QYNSFNFALLEDVQAKVLEIPYKGKDLSMIVLLPNEIDGLQKLEEKLTAEK**LMEWTSLQNMR**
ETCVDLHLPRFKMEESYDLKDTLRTMGMVNIFNGDADLSGMTWSHGLSVSKVLHKAFVEVTEEGVEAAA
ATAVVVVELSSPSTNEEFCCNHPFLFFIRQNK**TNSILFYGR**FSSP


**Mass Match Peptides (bold):**

        LMEWTSLQNMR [470]
        SVQMMR [665]
        TNSILFYGR [713]
        VLHFDQVTENTTEK [781]

**Tandem Peptides (underline):**

        INSWVESQTNEK [384]
        LMEWTSLQNMR [470]


## Peptide Source: iTRAQ, Renal cell cancer

MNSLSEANTKFMFDLFQQFRKSKENNIFYSPISITSALGMVLLGAKDNTAQQISKVLHFDQVTENTTEK
AATYHVDRSGNVHHQFQKLLTEFNKSTDAYELKIANK**LFGEK**TYQFLQEYLDAIKKFYQTSVESTDFAN
APEESRKKINSWVESQTNEKIKNLFPDGTIGNDTTLVLVNAIYFKGQWENKFKKENTKEEKFWPNKNTY
KSVQMMRQYNSFNFALLEDVQAKVLEIPYKGKDLSMIVLLPNEIDGLQKLEEKLTAEKLMEWTSLQNMR
ETCVDLHLPRFKMEESYDLKDTLRTMGMVNIFNGDADLSGMTWSHGLSVSKVLHKAFVEVTEEGVEAAA
ATAVVVVELSSPSTNEEFCCNHPFLFFIRQNKTNSILFYGRFSSP


**Mass Match Peptides (bold):**

        LFGEK [932]

**Tandem Peptides (underline):**

        LFGEK [932]


## OGTA113 (SEQ ID No: 23)

## Peptide Source: 1D-GE, Pancreatic cancer

MAANYSSTSTRREHVKVK**TSSQPGFLER**LSETSGGMFVGLMAFLLSFYLIFTNEGRALKTATSLAEGLS
LVVSPDSIHSVAPENEGR**LVHIIGALR**TSKLLSDPNYGVHLPAVKLRR**HVEMYQWVETEESR**EYTEDGQ
VKKETR**YSYNTEWRSEIINSK**NFDREIGHKNPSAMAVESFMATAPFVQIGRFFLSSGLIDKVDNFKSLS
LSK**LEDPHVDIIRRGDFFYHSENPKYPEVGDLR**VSFSYAGLSGDDPDLGPAHVVTVIARQRGDQLVPFS
TKSGDTLLLLHHGDFSAEEVFHRELRSNSMKTWGLRAAGWMAMFMGLNLMTRILYTLVDWFPVFRDLVN
IGLKAFAFCVATSLTLLTVAAGWLFYRPLWALLIAGLALVPILVARTRVPAKKLE


**Mass Match Peptides (bold):**

```
HVEMYQWVETEESR [345]
LEDPHVDIIR [449]
LVHIIGALR [500]
RGDFFYHSENPK [603]
SEIINSK [627]
TSSQPGFLER [730]
YPEVGDLR [865]
YSYNTEWR [874]
```

**Tandem Peptides (underline):**
```
LEDPHVDIIR [449]
LVHIIGALR [500]
YPEVGDLR [865]
```

## Peptide Source: iTRAQ, Colorectal cancer

```
MAANYSSTSTRREHVKVKTSSQPGFLERLSETSGGMFVGLMAFLLSFYLIFTNEGRALKTATSLAEGLS
LVVSPDSIHSVAPENEGRLVHIIGALRTSKLLSDPNYGVHLPAVKLRRHVEMYQWVETEESREYTEDGQ
VKKETRYSYNTEWRSEIINSKNFDREIGHKNPSAMAVESFMATAPFVQIGRFFLSSGLIDKVDNFKSLS
LSKLEDPHVDIIRRGDFFYHSENPKYPEVGDLRVSFSYAGLSGDDPDLGPAHVVTVIARQRGDQLVPFS
TKSGDTLLLLHHGDFSAEEVFHRELRSNSMKTWGLRAAGWMAMFMGLNLMTRILYTLVDWFPVFRDLVN
IGLKAFAFCVATSLTLLTVAAGWLFYRPLWALLIAGLALVPILVARTRVPAKKLE
```

**Mass Match Peptides (bold):**
```
LEDPHVDIIRR [931]
LLSDPNYGVHLPAVK [939]
YPEVGDLR [865]
```

**Tandem Peptides (underline):**
```
LEDPHVDIIRR [931]
LLSDPNYGVHLPAVK [939]
YPEVGDLR [865]
```

## Peptide Source: iTRAQ, Non-small cell lung cancer

```
MAANYSSTSTRREHVKVKTSSQPGFLERLSETSGGMFVGLMAFLLSFYLIFTNEGRALKTATSLAEGLS
LVVSPDSIHSVAPENEGRLVHIIGALRTSKLLSDPNYGVHLPAVKLRRHVEMYQWVETEESREYTEDGQ
VKKETRYSYNTEWRSEIINSKNFDREIGHKNPSAMAVESFMATAPFVQIGRFFLSSGLIDKVDNFKSLS
LSKLEDPHVDIIRRGDFFYHSENPKYPEVGDLRVSFSYAGLSGDDPDLGPAHVVTVIARQRGDQLVPFS
TKSGDTLLLLHHGDFSAEEVFHRELRSNSMKTWGLRAAGWMAMFMGLNLMTRILYTLVDWFPVFRDLVN
IGLKAFAFCVATSLTLLTVAAGWLFYRPLWALLIAGLALVPILVARTRVPAKKLE
```

**Mass Match Peptides (bold):**
```
YPEVGDLR [865]
YSYNTEWR [874]
```

**Tandem Peptides (underline):**
```
YPEVGDLR [865]
YSYNTEWR [874]
```

## Peptide Source: iTRAQ, Ovarian cancer

MAANYSSTSTRREHVKVKTSSQPGFLERLSETSGGMFVGLMAFLLSFYLIFTNEGRALKTATSLAEGLS
LVVSPDSIHSVAPENEGRLVHIIGALRTSKLLSDPNYGVHLPAVKLRRHVEMYQWVETEESREYTEDGQ
VKKETRYSYNTEWRSEIINSKNFDREIGHKNPSAMAVESFMATAPFVQIGRFFLSSGLIDKVDNFKSLS
LSKLEDPHVDIIRRGDFFYHSENPKYPEVGDLRVSFSYAGLSGDDPDLGPAHVVTVIARQRGDQLVPFS
TKSGDTLLLLHHGDFSAEEVFHRELRSNSMKTWGLRAAGWMAMFMGLNLMTRILYTLVDWFPVFRDLVN
IGLKAFAFCVATSLTLLTVAAGWLFYRPLWALLIAGLALVPILVARTRVPAKKLE

**Mass Match Peptides (bold):**
> LEDPHVDIIR [449]

**Tandem Peptides (underline):**
> LEDPHVDIIR [449]

## Peptide Source: iTRAQ, Renal cell cancer

MAANYSSTSTRREHVKVKTSSQPGFLERLSETSGGMFVGLMAFLLSFYLIFTNEGRALKTATSLAEGLS
LVVSPDSIHSVAPENEGRLVHIIGALRTSKLLSDPNYGVHLPAVKLRRHVEMYQWVETEESREYTEDGQ
VKKETRYSYNTEWRSEIINSKNFDREIGHKNPSAMAVESFMATAPFVQIGRFFLSSGLIDKVDNFKSLS
LSKLEDPHVDIIRRGDFFYHSENPKYPEVGDLRVSFSYAGLSGDDPDLGPAHVVTVIARQRGDQLVPFS
TKSGDTLLLLHHGDFSAEEVFHRELRSNSMKTWGLRAAGWMAMFMGLNLMTRILYTLVDWFPVFRDLVN
IGLKAFAFCVATSLTLLTVAAGWLFYRPLWALLIAGLALVPILVARTRVPAKKLE

**Mass Match Peptides (bold):**
> LEDPHVDIIR [449]

**Tandem Peptides (underline):**
> LEDPHVDIIR [449]

## OGTA119 (SEQ ID No: 24)

## Peptide Source: 1D-GE, B-cell non-Hodgkin's lymphoma

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

**Mass Match Peptides (bold):**
> AQPPEAGPQGLHDLGR [83]
> DEQHQCSLGNLK [115]
> ERPPDHQHSAQVK [222]
> GANTHLSTFSFTK [285]
> VGNQIFQSR [765]

**Tandem Peptides (underline):**

VGNQIFQSR [765]


## Peptide Source: 1D-GE, Breast cancer

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT


### Mass Match Peptides (bold):
AEWLNK [64]
AQPPEAGPQGLHDLGR [83]
FQLSNSGPNSTIK [268]
ISSNPNPVVQMSVGHK [393]
NLIAFSEDGSDPYVR [542]
VGNQIFQSR [765]

### Tandem Peptides (underline):
ALALLEDEER [75]


## Peptide Source: 1D-GE, Colorectal cancer

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT


### Mass Match Peptides (bold):
AQPPEAGPQGLHDLGR [83]
ERPPDHQHSAQVK [222]
FQLSNSGPNSTIK [268]
LEWLTLMPNASNLDK [457]
NLIAFSEDGSDPYVR [542]
RQDLEVEVR [611]
TNEPVWEENFTFFIHNPK [710]

**Tandem Peptides (underline):**
ALALLEDEER [75]

## Peptide Source: 1D-GE, Gastric cancer

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

**Mass Match Peptides (bold):**
**Tandem Peptides (underline):**
VDVGQQPLR [750]

## Peptide Source: 1D-GE, Hepatocellular carcinoma

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

**Mass Match Peptides (bold):**
AEWLNK [64]
AQPPEAGPQGLHDLGR [83]
DEQHQCSLGNLK [115]
ERPPDHQHSAQVK [222]
FQLSNSGPNSTIK [268]
HMWPFICQFIEK [339]
NLIAFSEDGSDPYVR [542]
RQDLEVEVR [611]
SDPYGIIR [622]
TNEPVWEENFTFFIHNPK [710]
VDVGQQPLR [750]
VGNQIFQSR [765]

VPLSQLLTSEDMTVSQR [792]

Tandem Peptides (underline):

ALALLEDEER [75]
AQPPEAGPQGLHDLGR [83]
FQLSNSGPNSTIK [268]
IHFIEAQDLQGK [368]
NLIAFSEDGSDPYVR [542]
VGNQIFQSR [765]

## Peptide Source: 1D-GE, Lung cancer

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

Mass Match Peptides (bold):

AQPPEAGPQGLHDLGR [83]
ERPPDHQHSAQVK [222]
HMWPFICQFIEK [339]
ITVPLVSEVQIAQLR [398]

## Peptide Source: 1D-GE, Lymphoid leukaemia, unspecified

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

Mass Match Peptides (bold):

AQPPEAGPQGLHDLGR [83]
ERPPDHQHSAQVK [222]
ETIEPAVR [230]

FQLSNSGPNSTIK [268]
GEGPEAGAGGAGGR [290]
HMWPFICQFIEK [339]
ISSNPNPVVQMSVGHK [393]
NLIAFSEDGSDPYVR [542]
RQDLEVEVR [611]
VDVGQQPLR [750]

**Tandem Peptides (underline):**
FQLSNSGPNSTIK [268]
IHFIEAQDLQGK [368]
VLVALASEELAK [784]

## Peptide Source: 1D-GE, Neuroblastoma

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTER**AEWLNK**TVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

**Mass Match Peptides (bold):**
AEWLNK [64]
**Tandem Peptides (underline):**

## Peptide Source: 1D-GE, Osteosarcoma

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTER**AEWLNK**TVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

**Mass Match Peptides (bold):**
AEWLNK [64]
**Tandem Peptides (underline):**

**Peptide Source: 1D-GE, Pancreatic cancer**

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIHNPKRQDLEVEVRDEQHQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

### Mass Match Peptides (bold):

    AQPPEAGPQGLHDLGR [83]
    DEQHQCSLGNLK [115]
    ERPPDHQHSAQVK [222]
    GEGPEAGAGGAGGR [290]
    HMWPFICQFIEK [339]
    IHFIEAQDLQGK [368]
    ISSNPNPVVQMSVGHK [393]
    NLIAFSEDGSDPYVR [542]
    SDPYGIIR [622]
    VGNQIFQSR [765]
    VPLSQLLTSEDMTVSQR [792]
    VYTENVDK [819]

### Tandem Peptides (underline):

    ALALLEDEER [75]
    NLIAFSEDGSDPYVR [542]

**Peptide Source: 1D-GE, Prostate cancer**

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTERAEWLNKTVKHMWPFICQFIEKLFRETIEPAVRGANTHLSTF
SFTKVDVGQQPLRINGVKVYTENVDKRQIILDLQISFVGNCEIDLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKKISSNPNPVVQMSVGHKAQESKIRYKTN
EPVWEENFTFFIINPKRQDLEVEVRDEQIIQCSLGNLKVPLSQLLTSEDMTVSQRFQLSNSGPNSTIKMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

### Mass Match Peptides (bold):

    AEWLNK [64]

**Tandem Peptides (underline):**


## Peptide Source: 1D-GE, Renal cell cancer

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCRALAL
LEDEERVVRLGVRACDLPAWVHFPDTER**AEWLNK**TVK**HMWPFICQFIEK**LFRETIEPAVRGANTHLSTF
SFTK**VDVGQQPLR**INGVK**VYTENVDKR**QIILDLQISFVGNCE‒DLEIKRYFCRAGVK**SIQIHGTMR**VIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKK‒SSNPNPVVQMSVGHKAQESKIRYK**TN**
**EPVWEENFTFFIHNPK**RQDLEVEVRDEQHQCSLGNLKVPLSQLLTSEDMTVSQRFQLSNSGPNST‒KMK
IALRVLHLEKR**ERPPDHQHSAQVK**RPSVSKEGRKTSIKSHMSGSPGPGGSNTAPSTPVIGGSDKPGMEE
K**AQPPEAGPQGLHDLGR**SSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT


**Mass Match Peptides (bold):**

    AEWLNK [64]
    AQPPEAGPQGLHDLGR [83]
    ERPPDHQHSAQVK [222]
    HMWPFICQFIEK [339]
    SIQIHGTMR [638]
    TNEPVWEENFTFFIHNPK [710]
    VDVGQQPLR [750]
    VYTENVDKR [820]

**Tandem Peptides (underline):**

    AQPPEAGPQGLHDLGR [83]


## Peptide Source: iTRAQ, Colorectal cancer

<u>MTPPSRAEAGVRR</u>SRVPSEGRWRGAEPPGISASTQPASAGRAARHCGAMSGAR<u>GEGPEAGAGGAGGR</u>AA
PENPGGVLSVELPGLLAQLARSFALLLPVYALGYLGLSFSWVLLALALLAWCRRSRGLKALRLCR<u>ALAL</u>
<u>LEDEER</u>VVRLGVRACDLPAWVHFPDTERAEWLNKTVKHMWPF‒CQFIEKLFRETIEPAVRGANTHLSTF
SFTKVDVGQQPLRINGVKVYTENVDKRQIILDLQISFVGNCE‒DLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDK<u>VLTDIK</u>ADKDQANDGLSSALLILYLDSARNLPSGKK‒SSNPNPVVQMSVGHKAQESKIRYKTN
EPVWEENFTFFIHNPKRQDLEVEVRDEQHQCSLGNLKVPLSQLLTSEDMTVSQRFQLSNSGPNST‒KMK
IALRVLHLEKRERPPDHQHSAQVKRPSVSKEGRKTSIKSHMSGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT


**Mass Match Peptides (bold):**

    ALALLEDEER [75]
    GEGPEAGAGGAGGR [290]
    MTPPSRAEAGVRR [953]
    VLTDIK [996]

**Tandem Peptides (underline):**

ALALLEDEER [75]
GEGPEAGAGGAGGR [290]
MTPPSRAEAGVRR [953]
VLTDIK [996]


## Peptide Source: iTRAQ, Non-small cell lung cancer

MTPPSRAEAGVRRSRVPSEGRWRGAEPPGISASTQPASAGRAARHCGAMSGAR**GEGPEAGAGGAGGR**AA
PENPGGVLSVELPGLLAQLARSFALLLPVYALGYLGLSFSWVLLALALLAWCRRSRGLKALRLCRALAL
LEDEERVVRLGVRACDLPAWVHFPDTER**AEWLNK**TVKHMWPF:CQFIEKLFRETIEPAVRGANTHLSTF
SFTKVDVGQQPLRINGVKVYTENVDKRQIILDLQISFVGNCE:DLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGK<u>DTYLK</u>GLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKK:SSNPNPVVQMSVGHKAQESKIRYKTN
EPVWEENFTFFIHNPKRQDLEVEVRDEQHQCSLGNLKVPLSQLLTSEDMTVSQRFQLSNSGPNST:KMK
IALRVLHLEKRERPPDHQHSAQVKRPSVSKEGRKTSIKSHM:SGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

### Mass Match Peptides (bold):
AEWLNK [64]
DTYLK [896]
GEGPEAGAGGAGGR [290]

### Tandem Peptides (underline):
AEWLNK [64]
DTYLK [896]
GEGPEAGAGGAGGR [290]


## Peptide Source: iTRAQ, Ovarian cancer

MTPPSRAEAGVRRSRVPSEGRWRGAEPPGISASTQPASAGRAARHCGAMSGARGEGPEAGAGGAGGRAA
PENPGGVLSVELPGLLAQLARSFALLLPVYALGYLGLSFSWVLLALALLAWCRRSRGLKALRLCR<u>ALAL</u>
<u>LEDEER</u>VVRLGVRACDLPAWVHFPDTERAEWLNKTVKHMWPF:CQFIEKLFRETIEPAVRGANTHLSTF
SFTKVDVGQQPLRINGVKVYTENVDKRQIILDLQISFVGNCE:DLEIKRYFCRAGVKSIQIHGTMRVIL
EPLIGDMPLVGALSIFFLRKPLLEINWTGLTNLLDVPGLNGLSDTIILDIISNYLVLPNRITVPLVSEV
QIAQLRFPVPKGVLRIHFIEAQDLQGKDTYLKGLVKGKSDPYGIIRVGNQIFQSRVIKENLSPKWNEVY
EALVYEHPGQELEIELFDEDPDKDDFLGSLMIDLIEVEKERLLDEWFTLDEVPKGKLHLRLEWLTLMPN
ASNLDKVLTDIKADKDQANDGLSSALLILYLDSARNLPSGKK:SSNPNPVVQMSVGHKAQESKIRYKTN
EPVWEENFTFFIHNPKRQDLEVEVRDEQHQCSLGNLKVPLSQLLTSEDMTVSQRFQLSNSGPNST:KMK
IALRVLHLEKRERPPDHQHSAQVKRPSVSKEGRKTSIKSHM:SGSPGPGGSNTAPSTPVIGGSDKPGMEE
KAQPPEAGPQGLHDLGRSSSSLLASPGHISVKEPTPSIASDISLPIATQELRQRLRQLENGTTLGQSPL
GQIQLTIRHSSQRNKLIVVVHACRNLIAFSEDGSDPYVRMYLLPDKRRSGRRKTHVSKKTLNPVFDQSF
DFSVSLPEVQRRTLDVAVKNSGGFLSKDKGLLGKVLVALASEELAKGWTQWYDLTEDGTRPQAMT

### Mass Match Peptides (bold):
ALALLEDEER [75]

### Tandem Peptides (underline):
ALALLEDEER [75]

## OGTA124 (SEQ ID No: 25)

**Peptide Source: 1D-GE, Hepatocellular carcinoma**

MPHFTVVPVDGPRRGDYDNLEGLSWVDYGERAELDDSDGHGNHRESSPFLSPLEASRGIDYYDRNLALF
EEELDIRPKVSSLLGKLVSYTNLTQGAKEHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIKSIFDPPVFPVCMLGNRTLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYHWALSFL
GMSLCLALMFVSSWYYALVAMLIAGMIYKYIEYQGAEKEWGDGIRGLSLSAARYALLRLEEGPPHTKNW
RPQLLVLLKLDEDLHVKYPRLLTFASQLKAGKGLTIVGSVIQGSFLESYGEAQAAEQTIKNMMEIEKVK
GFCQVVVASKVREGLAHLIQSCGLGGMRHNSVVLGWPYGWRQSEDPRAWKTFIDTVRCTTAAHLALLVP
KNIAFYPSNHERYLEGIIIDVWWIVIIDGGMLMLLPFLLRQIIKVWRKCRMRIFTVAQMDDNSIQMKKDLAV
FLYHLRLEAEVEVVEMHNSDISAYTYERTLMMEQRSQMLRQMRLTKTEREREAQLVKDRHSALRLESLY
SDEEDESAVGADKIQMTWTRDKYMTETWDPSHAPDNFRELVHIKPDQSNVRRMHTAVKLNEVIVTRSHD
ARLVLLNMPGPPRNSEGDENYMEFLEVLTEGLERVLLVRGGGREVITIYS

**Mass Match Peptides (bold):**
        AELDDSDGHGNHR [62]
        DLAVFLYHLR [133]
        EGLAHLIQSCGLGGMR [188]
        EHEEAESGEGTRRR [192]
        ESSPFLSPLEASR [227]
        EVITIYS [233]
        EWGDGIR [240]
        HGLPSADAPSLK [334]
        HNSVVLGWPYGWR [340]
        LDEDLHVK [446]
        LEEGPPHTK [451]
        MPHFTVVPVDGPR [521]
        NIAFYPSNHER [539]
        NLALFEEELDIRPK [541]
        NSEGDENYMEFLEVLTEGLER [553]
        SIFDPPVFPVCMLGNR [636]
        TFIDTVR [689]
        TPNWRPR [720]
        YIEYQGAEK [855]
        YMTETWDPSHAPDNFR [863]

**Tandem Peptides (underline):**
        HGLPSADAPSLK [334]
        LVLLNMPGPPR [501]
        LVSYTNLTQGAK [504]

**Peptide Source: 1D-GE, Lung cancer**

MPIIFTVVPVDGPRRGDYDNLEGLSWVDYGERAELDDSDGIIGNIIRESSPFLSPLEASRGIDYYDRNLALF
EEELDIRPKVSSLLGKLVSYTNLTQGAKEHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIKSIFDPPVFPVCMLGNRTLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA

612

```
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYHWALSFL
GMSLCLALMFVSSWYYALVAMLIAGMIYKYIEYQGAEKEWGDGIRGLSLSAARYALLRLEEGPPHᴵKNW
RPQLLVLLKLDEDLHVKYPRLLTFASQLKAGKGLTIVGSVIQGSFLESYGEAQAAEQTIKNMMEIEKVK
GFCQVVVASKVREGLAHLIQSCGLGGMRHNSVVLGWPYGWRQSEDPRAWKTFIDTVRCTTAAHLALLVP
KNIAFYPSNHERYLEGHIDVWWIVHDGGMLMLLPFLLRQHKVWRKCRMRIFTVAQMDDNSIQMKKDLAV
FLYHLRLEAEVEVVEMHNSDISAYTYERTLMMEQRSQMLRQMRLTKTEREREAQLVKDRHSALRLESLY
SDEEDESAVGADKIQMTWTRDKYMTETWDPSHAPDNFRELVHᴵKPDQSNVRRMHTAVKLNEVIVTRSHD
ARL**LVLLNMPGPPR**NSEGDENYMEFLEVLTEGLERVLLVRGGGR**EVITIYS**
```

## Mass Match Peptides (bold):

DQFDICAK [151]
EHEEAESGEGTR [191]
EHEEAESGEGTRRR [192]
ESSPFLSPLEASR [227]
EVITIYS [233]
LVLLNMPGPPR [501]
LVSYTNLTQGAK [504]

## Tandem Peptides (underline):

LVSYTNLTQGAK [504]

## Peptide Source: 1D-GE, Ovarian cancer

```
MPHFTVVPVDGPRRGDYDNLEGLSWVDYGERAELDDSDGHGNHR**ESSPFLSPLEASR**GIDYYDRNLALF
EEELDIRPKVSSLLGKLVSYTNLTQGAKEHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIKSIFDPPVFPVCMLGNRTLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLR**TPNWRPR**FKYYHWALSFL
GMSLCLALMFVSSWYYALVAMLIAGMIYKYIEYQGAEKEWGDGIRGLSLSAARYALLRLEEGPPHᴵKNW
RPQLLVLLKLDEDLHVKYPRLLTFASQLKAGKGLTIVGSVIQGSFLESYGEAQAAEQTIKNMMEIEKVK
GFCQVVVASKVREGLAHLIQSCGLGGMRHNSVVLGWPYGWRQSEDPRAWK**TFIDTVR**CTTAAHLALLVP
K**NIAFYPSNHER**YLEGHIDVWWIVHDGGMLMLLPFLLRQHKVWRKCRMRIFTVAQMDDNSIQMKKDLAV
FLYHLRLEAEVEVVEMHNSDISAYTYERTLMMEQRSQMLRQMRLTKTEREREAQLVKDRHSALRLESLY
SDEEDESAVGADK**IQMTWTR**DKYMTETWDPSHAPDNFRELVHᴵKPDQSNVRR**MHTAVKLNEVIVTR**SHD
ARLVLLNMPGPPR**NSEGDENYMEFLEVLTEGLER**VLLVRGGGR**EVITIYS**
```

## Mass Match Peptides (bold):

ESSPFLSPLEASR [227]
EVITIYS [233]
IQMTWTR [389]
LNEVIVTR [472]
MHTAVK [517]
NIAFYPSNHER [539]
NSEGDENYMEFLEVLTEGLER [553]
TFIDTVR [689]
TPNWRPR [720]

## Tandem Peptides (underline):

ESSPFLSPLEASR [227]
GIDYYDR [300]

## Peptide Source: 1D-GE, Pancreatic cancer

MPHFTVVPVDGPRRGDYDNLEGLSWVDYGERAELDDSDGHGNHR**ESSPFLSPLEASR**GIDYYDRNLALF
EEELDIRPKVSSLLGK<u>LVSYTNLTQGAK</u>EHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIK**SIFDPPVFPVCMLGNR**TLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYHWALSFL
GMSLCLALMFVSSWYYALVAMLIAGMIYKYIEYQGAEKEWGDGIRGLSLSAARYALLR**LEEGPPHTK**NW
RPQLLVLLKLDEDLHVKYPRLLTFASQLKAGKGLTIVGSVIQGSFLESYGEAQAAEQTIKNMMEIEKVK
GFCQVVVASKVREGLAHLIQSCGLGGMR**HNSVVLGWPYGWR**QSEDPRAWKTFIDTVRCTTAAHLALLVP
K**NIAFYPSNHER**YLEGIIIDVWWIVIIDGGMLMLLPFLLRQIIKVWRKCRMRIFTVAQMDDDNSIQMKKDLAV
FLYHLRLEAEVEVVEMHNSDISAYTYERTLMMEQRSQMLRQMRLTKTEREREAQLVKDRHSALRLESLY
SDEEDESAVGADKIQMTWTRDKYMTETWDPSHAPDNFR**ELVHIKPDQSNVR**RMHTAVKLNEVIVTRSHD
ARLVLLNMPGPPRNSEGDENYMEFLEVLTEGLERVLLVRGGGREVITIYS

## Mass Match Peptides (bold):

ELVHIKPDQSNVR [209]
ESSPFLSPLEASR [227]
HNSVVLGWPYGWR [340]
LEEGPPHTK [451]
LVSYTNLTQGAK [504]
NIAFYPSNHER [539]
SIFDPPVFPVCMLGNR [636]

## Tandem Peptides (underline):

LVSYTNLTQGAK [504]

## Peptide Source: 1D-GE, Renal cell cancer

<u>MPHFTVVPVDGPR</u>RGDYDNLEGLSWVDYGERAELDDSDGHGNHR**ESSPFLSPLEASR**GIDYYDRNLALF
EEELDIRPKVSSLLGK<u>LVSYTNLTQGAK</u>EHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIK**SIFDPPVFPVCMLGNR**TLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYHWALSFL
GMSLCLALMFVSSWYYALVAMLIAGMIYKYIEYQGAEKEWGDGIRGLSLSAARYALLRLEEGPPHTKNW
RPQLLVLLKLDEDLHVKYPRLLTFASQLKAGKGLTIVGSVIQGSFLESYGEAQAAEQTIKNMMEIEKVK
GFCQVVVASKVREGLAHLIQSCGLGGMR**HNSVVLGWPYGWR**QSEDPRAWKTFIDTVRCTTAAHLALLVP
K<u>NIAFYPSNHER</u>YLEGHIDVWWIVHDGGMLMLLPFLLRQHKVWRKCRMR**IFTVAQMDDDNSIQMKK**DLAV
FLYHLRLEAEVEVVEMHNSDISAYTYERTLMMEQRSQMLRQMRLTKTEREREAQLVKDRHSALRLESLY
SDEEDESAVGADKIQMTWTRDKYMTETWDPSHAPDNFRELVHIKPDQSNVRRMHTAVKLNEVIVTRSHD
ARLVLLNMPGPPRNSEGDENYMEFLEVLTEGLERVLLVRGGGREVITIYS

## Mass Match Peptides (bold):

ESSPFLSPLEASR [227]
HNSVVLGWPYGWR [340]

```
IFTVAQMDDNSIQMK [364]
IFTVAQMDDNSIQMKK [365]
LVSYTNLTQGAK [504]
MPHFTVVPVDGPR [521]
NIAFYPSNHER [539]
SIFDPPVFPVCMLGNR [636]
```

**Tandem Peptides (underline):**

```
LVSYTNLTQGAK [504]
```

## Peptide Source: iTRAQ, Colorectal cancer

```
MPHFTVVPVDGPRRGDYDNLEGLSWVDYGERAELDDSDGHGNHRESSPFLSPLEASRGIDYYDRNLALF
EEELDIRPKVSSLLGKLVSYTNLTQGAKEHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIKSIFDPPVFPVCMLGNRTLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYHWALSFL
GMSLCLALMFVSSWYYALVAMLIAGMIYKYIEYQGAEKEWGDGIRGLSLSAARYALLRLEEGPPHIKNW
RPQLLVLLKLDEDLHVKYPRLLTFASQLKAGKGLTIVGSVIQGSFLESYGEAQAAEQTIKNMMEIEKVK
GFCQVVVASKVREGLAHLIQSCGLGGMRHNSVVLGWPYGWRQSEDPRAWKTFIDTVRCTTAAHLALLVP
KNIAFYPSNHERYLEGHIDVWWIVHDGGMLMLLPFLLRQHKVWRKCRMRIFTVAQMDDNSIQMKKDLAV
FLYHLRLEAEVEVVEMHNSDISAYTYERTLMMEQRSQMLRQMRLTKTEREREAQLVKDRHSALRLESLY
SDEEDESAVGADKIQMTWTRDKYMTETWDPSHAPDNFRELVHIKPDQSNVRRMHTAVKLNEVIVTRSHD
ARLVLLNMPGPPRNSEGDENYMEFLEVLTEGLERVLLVRGGGREVITIYS
```

**Mass Match Peptides (bold):**

```
LLQAIAK [937]
```

**Tandem Peptides (underline):**

```
LLQAIAK [937]
```

## Peptide Source: iTRAQ, Hepatocellular cancer

```
MPHFTVVPVDGPRRGDYDNLEGLSWVDYGERAELDDSDGHGNHRESSPFLSPLEASRGIDYYDRNLALF
EEELDIRPKVSSLLGKLVSYTNLTQGAKEHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIKSIFDPPVFPVCMLGNRTLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYHWALSFL
GMSLCLALMFVSSWYYALVAMLIAGMIYKYIEYQGAEKEWGDGIRGLSLSAARYALLRLEEGPPHIKNW
RPQLLVLLKLDEDLHVKYPRLLTFASQLKAGKGLTIVGSVIQGSFLESYGEAQAAEQTIKNMMEIEKVK
GFCQVVVASKVREGLAHLIQSCGLGGMRHNSVVLGWPYGWRQSEDPRAWKTFIDTVRCTTAAHLALLVP
KNIAFYPSNHERYLEGHIDVWWIVHDGGMLMLLPFLLRQHKVWRKCRMRIFTVAQMDDNSIQMKKDLAV
FLYHLRLEAEVEVVEMHNSDISAYTYERTLMMEQRSQMLRQMRLTKTEREREAQLVKDRHSALRLESLY
SDEEDESAVGADKIQMTWTRDKYMTETWDPSHAPDNFRELVHIKPDQSNVRRMHTAVKLNEVIVTRSHD
ARLVLLNMPGPPRNSEGDENYMEFLEVLTEGLERVLLVRGGGREVITIYS
```

**Mass Match Peptides (bold):**
    EWGDGIR [240]
**Tandem Peptides (underline):**
    EWGDGIR [240]


## Peptide Source: iTRAQ, Non-small cell lung cancer

MPHFTVVPVDGPRRGDYDNLEGLSWVDYGERAELDDSDGHGNHRESSPFLSPLEASRGIDYYDRNLALF
EEELDIRPKVSSLLGKLVSYTNLTQGAKEHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIKSIFDPPVFPVCMLGNRTLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYHWALSFL
GMSLCLALMFVSSWYYALVAMLIAGMIYKYIEYQGAEKEWGDGIRGLSLSAARYALLRLEEGPPHꓕKNW
RPQLLVLLK**LDEDLHVK**YPRLLTFASQLKAGKGLTIVGSVIQGSFLESYGEAQAAEQTIKNMMEIEKVK
GFCQVVVASKVREGLAHLIQSCGLGGMRHNSVVLGWPYGWRQSEDPRAWKTFIDTVRCTTAAHLALLVP
KNIAFYPSNHERYLEGHIDVWWIVHDGGMLMLLPFLLRQHKVWRKCRMRIFTVAQMDDNSIQMKKDLAV
FLYHLRLEAEVEVVEMHNSDISAYTYERTLMMEQRSQMLRQMRLTKTEREREAQLVKDRHSALRLESLY
SDEEDESAVGADKIQMTWTRDKYMTETWDPSHAPDNFRELVHꓕKPDQSNVRRMHTAVKLNEVIVTRSHD
ARLVLLNMPGPPRNSEGDENYMEFLEVLTEGLERVLLVRGGGREVITIYS

**Mass Match Peptides (bold):**
    LDEDLHVK [446]
**Tandem Peptides (underline):**
    LDEDLHVK [446]


## Peptide Source: iTRAQ, Ovarian cancer

MPIIFTVVPVDGPRRGDYDNLEGLSWVDYGERAELDDSDGIIGNIIRESSPFLSPLEASRGIDYYDRNLALF
EEELDIRPKVSSLLGKLVSYTNLTQGAKEHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIKSIFDPPVFPVCMLGNRTLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYHWALSFL
GMSLCLALMFVSSWYYALVAMLIAGMIYKYIEYQGAEKEWGDGIRGLSLSAARYALLRLEEGPPHꓕKNW
RPQLLVLLKLDEDLHVKYPRLLTFASQLKAGKGLTIVGSVIQGSFLESYGEAQAAEQTIKNMMEIEKVK
GFCQVVVASKVREGLAHLIQSCGLGGMRHNSVVLGWPYGWRQSEDPRAWKTFIDTVRCTTAAHLALLVP
KNIAFYPSNHERYLEGHIDVWWIVHDGGMLMLLPFLLRQHKVWRKCRMRIFTVAQMDDNSIQMKKDLAV
FLYHLRLEAEVEVVEMHNSDISAYTYERTLMMEQRSQMLRQMRLTKTER**EREAQLVK**DRHSALRLESLY
SDEEDESAVGADKIQMTWTRDKYMTETWDPSHAPDNFRELVHꓕKPDQSNVRRMHTAVKLNEVIVTRSHD
ARLVLLNMPGPPRNSEGDENYMEFLEVLTEGLERVLLVRGGGREVITIYS

**Mass Match Peptides (bold):**
    EREAQLVK [905]
**Tandem Peptides (underline):**

EREAQLVK [905]

## Peptide Source: iTRAQ, Renal cell cancer

MPHFTVVPVDGPRRGDYDNLEGLSWVDYGERAELDDSDGHGNHRESSPFLSPLEASRGIDYYDRNLALF
EEELDIRPKVSSLLGKLVSYTNLTQGAKEHEEAESGEGTRRRAAEAPSMGTLMGVYLPCLQNIFGVILF
LRLTWMVGTAGVLQALLIVLICCCCTLLTAISMSAIATNGVVPAGGSYFMISRSLGPEFGGAVGLCFYL
GTTFAAAMYILGAIEILLTYIAPPAAIFYPSGAHDTSNATLNNMRVYGTIFLTFMTLVVFVGVKYVNKF
ASLFLACVIISILSIYAGGIKSIFDPPVFPVCMLGNRTLSRDQFDICAKTAVVDNETVATQLWSFFCHS
PNLTTDSCDPYFMLNNVTEIPGIPGAAAGVLQENLWSAYLEKGDIVEKHGLPSADAPSLKESLPLYVVA
DIATSFTVLVGIFFPSVTGIMAGSNRSGDLRDAQKSIPVGTIILAIITTSLVYFSSVVLFGACIEGVVLR
DKYGDGVSRNLVVGTLAWPSPWVIVIGSFFSTCGAGLQSLTGAPRLLQAIAKDNIIPFLRVFGHGKVNG
EPTWALLLTALIAELGILIASLDMVAPILSMFFLMCYLFVNLACAVQTLLRTPNWRPRFKYYIIWALSFL
GMSLCLALMFVSSWYYALVAMLIAGMIYKYIEYQGAEKEWGDGIRGLSLSAARYALLRLEEGPPHFKNW
RPQLLVLLK<u>**LDEDLHVK**</u>YPRLLTFASQLKAGKGLTIVGSVIQGSFLESYGEAQAAEQTIKNMMEIEKVK
GFCQVVVASKVREGLAHLIQSCGLGGMRHNSVVLGWPYGWRQSEDPRAWKTFIDTVRCTTAAHLALLVP
KNIAFYPSNHERYLEGHIDVWWIVHDGGMLMLLPFLLRQHKVWRKCRMR<u>**IFTVAQMDDNSIQMKK**</u>DLAV
FLYHLRLEAEVEVVEMHNSDISAYTYERTLMMEQRSQMLRQMRLTKTEREREAQLVKDRHSALRLESLY
SDEEDESAVGADKIQMTWTRDKYMTETWDPSHAPDNFRELVHIKPDQSNVRRMHTAVKLNEVIVTRSHD
ARLVLLNMPGPPRNSEGDENYMEFLEVLTEGLERVLLVRGGGREVITIYS

### Mass Match Peptides (bold):
IFTVAQMDDNSIQMKK [365]
LDEDLHVK [446]
### Tandem Peptides (underline):
IFTVAQMDDNSIQMKK [365]
LDEDLHVK [446]

## OGTA126 (SEQ ID No: 26)

## Peptide Source: 1D-GE, Breast cancer

MAGLNCGVSIALLGVLLLGAARLPR<u>**GAEAFEIALPR**</u>ESNITVLIKLGTPTLLAKPCYIVISKRHIFMLS
IKSGERIVFTFSCQSPENHFVIEIQKNIDCMSGPCPFGEVQLQPSTSLLPTLNRTFIWDVKAHKSFGLE
LQFSIPRLRQIGPGESCPDGVTHSISGRIDATVVRIGTFCSNGTVSRIKMQEGVK<u>**MALHLPWFHPR**</u>NVS
GFSIANRSSIKRLCIIESVFEGEGSATLMSANYPEGFPEDELMTWQFVVPAHLRASVSFLNFNLSNCER
KEERVEYYIPGSTTNPEVFKLEDKQPGNMAGNFNLSLQGCDQDAQSPGILRLQFQVLVQHPQNESNK<u>IY</u>
<u>VVDLSNER</u>AMSLTIEPRPVKQSRKFVPGCFVCLESRTCSSNLFLTSGSKHHKISFLCDDLTRLWMNVEKT
ISCTDHRYCQRKSYSLQVPSDILHLPVELHDFSWKLLVPKDRLSLVLVPAQKLQQHTHEKPCNTSFSYL
VASAIPSQDLYFGSFCPGGSIKQIQVKQNISVTLRTFAPSFRQEASRQGLTVSFIPYFKEEGVFTVTPD
TKSKVYLRTPNWDR<u>**GLPSLTSVSWNISVPR**</u>DQVACLTFFKERSGVVCQTGR<u>**AFMIIQEQR**</u>TRAEEIFSL
DEDVLPKPSFHHHSFWVNISNCSPTSGKQLDLLFSVTLTPRTVDLTVILIAAVGGGVLLLSALGLIICC
VKKKKKKTNKGPAVGIYNGNINTEMPRQPKKFQKGRKDNDSHVYAVIEDTMVYGHLLQDSSGSFLQPEV
DTYRPFQGTMGVCPPSPPTICSRAPTAKLATEEPPPRSPPESESEPYTFSHPNNGDVSSKDTDIPLLST
QEPMEPAE

### Mass Match Peptides (bold):
AFMIIQEQR [66]
GAEAFEIALPR [282]
GLPSLTSVSWNISVPR [302]
MALHLPWFHPR [508]
### Tandem Peptides (underline):

IYVVDLSNER [404]


## Peptide Source: 1D-GE, Colorectal cancer

MAGLNCGVSIALLGVLLLGAARLPRGAEAFEIALPRESNITVLIKLGTPTLLAKPCYIVISKRHI▯MLS
IKSGERIVFTFSCQSPENHFVIEIQKNIDCMSGPCPFGEVQLQPSTSLLPTLNRTFIWDVKAHKS▯GLE
LQFSIPRLRQIGPGESCPDGVTHSISGRIDATVVRIGTFCSNGTVSRIKMQEGVK**MALHLPWFHPR**NVS
GFSIANRSSIKRLCIIESVFEGEGSATLMSANYPEGFPEDELMTWQFVVPAHLR**ASVSFLNFNLSNCER**
KEER<u>VEYYIPGSTTNPEVFK</u>LEDKQPGNMAGNFNLSLQGCDQDAQSPGILRLQFQVLVQHPQNESNKIY
VVDLSNERAMSLTIEPRPVKQSRKFVPGCFVCLESRTCSSNL▯LTSGSKHKISFLCDDLTRLWMNVEKT
ISCTDHRYCQRKSYSLQVPSDILHLPVELHDFSWKLLVPKDRLSLVLVPAQKLQQHTHEKPCNTSFSYL
VASAIPSQDLY▯GSFCPGGSIKQIQVKQNISVTLRTFAPSFRQEASRQGLTVSFIPY▯KEEGVFTVTPD
TKSKVYLRTPNWDRGLPSLTSVSWNISVPRDQVACLTFFKERSGVVCQTGRAFMIIQEQRTRAEE▯FSL
DEDVLPKPSFHHHSFWVNISNCSPTSGKQLDLLFSVTLTPRTVDLTVILIAAVGGGVLLLSALGL▯ICC
VKKKKKKTNKGPAVGIYNGNINTEMPRQPKKFQKGRKDNDSHVYAVIEDTMVYGHLLQDSSGSFLQPEV
DTYRPFQGTMGVCPPSPPTICSRAPTAKLATEEPPPRSPPESESEPYTFSHPNNGDVSSKDTDIPLLST
QEPMEPAE


### Mass Match Peptides (bold):
ASVSFLNFNLSNCER [87]
MALHLPWFHPR [508]

### Tandem Peptides (underline):
VEYYIPGSTTNPEVFK [757]


## Peptide Source: 1D-GE, Hepatocellular carcinoma

MAGLNCGVSIALLGVLLLGAARLPRGAEAFEIALPRESNITVLIKLGTPTLLAKPCYIVISKRHI▯MLS
IKSGERIVFTFSCQSPENHFVIEIQKNIDCMSGPCPFGEVQLQPSTSLLPTLNRTFIWDVKAHKS▯GLE
LQFSIPRLR**QIGPGESCPDGVTHSISGR**IDATVVRIGTFCSNGTVSRIKMQEGVK**MALHLPWFHPRNVS**
**GFSIANR**SSIKRLCIIESVFEGEGSATLMSANYPEGFPEDELMTWQFVVPAHLR**ASVSFLNFNLSNCER**
KEERVEYYIPGSTTNPEVFKLEDKQPGNMAGNFNLSLQGCDQDAQSPGILRLQFQVLVQHPQNESNK**IY**
**VVDLSNER**AMSLTIEPRPVKQSRKFVPGCFVCLESR**TCSSNLTLTSGSK**HKISFLCDDLTR**LWMNVEK**T
ISCTDHRYCQRKSYSLQVPSDILHLPVELHDFSWKLLVPKDRLSLVLVPAQKLQQHTHEKPCNTSFSYL
VASAIPSQDLY▯GSFCPGGSIKQIQVKQNISVTLRTFAPSFRQEASRQGLTVSFIPY▯K**EEGVFTVTPD**
**TK**SKVYLRTPNWDRGLPSLTSVSWNISVPRDQVACLTFFKERSGVVCQTGR**AFMIIQEQR**TRAEE▯FSL
DEDVLPKPSFHHHSFWVNISNCSPTSGKQLDLLFSVTLTPRTVDLTVILIAAVGGGVLLLSALGL▯ICC
VKKKKKKTNKGPAVGIYNGNINTEMPRQPKKFQKGRKDNDSHVYAVIEDTMVYGHLLQDSSGSFLQPEV
DTYRPFQGTMGVCPPSPPTICSRAPTAK<u>LATEEPPPR</u>SPPESESEPYTFSHPNNGDVSSKDTDIPLLST
QEPMEPAE


### Mass Match Peptides (bold):
AFMIIQEQR [66]
ASVSFLNFNLSNCER [87]
EEGVFTVTPDTK [172]
IYVVDLSNER [404]
LATEEPPPR [444]
LWMNVEK [505]
MALHLPWFHPR [508]
NVSGFSIANR [561]
QIGPGESCPDGVTHSISGR [580]
TCSSNLTLTSGSK [679]

### Tandem Peptides (underline):

LATEEPPPR [444]


## Peptide Source: 1D-GE, Melanoma

MAGLNCGVSIALLGVLLLGAARLPRGAEAFEIALPRESNITVLIKLGTPTLLAKPCYIVISKRHI⁻MLS
IKSGERIVFTFSCQSPENHFVIEIQKNIDCMSGPCPFGEVQLQPSTSLLPTLNRTFIWDVKAHKS⁻GLE
LQFSIPRLRQIGPGESCPDGVTHSISGRIDATVVRIGTFCSNGTVSRIKMQEGVK**MALHLPWFHPRN**VS
GFSIANRSSIKRLCIIESVFEGEGSATLMSANYPEGFPEDELMTWQFVVPAHLR**ASVSFLNFNLSNCER**
KEERVEYYIPGSTTNPEVFKLEDKQPGNMAGNFNLSLQGCDQDAQSPGILRLQFQVLVQHPQNESNK**IY**
**VVDLSNER**AMSLTIEPRPVKQSR**KFVPGCFVCLESR**TCSSNLᵀLTSGSKHKISFLCDDLTRLWMNVEKT
ISCTDHRYCQRKSYSLQVPSDILHLPVELHDFSWKLLVPKDRLSLVLVPAQKLQQHTHEKPCNTSFSYL
VASAIPSQDLYᵀGSFCPGGSIKQIQVKQNISVTLRTFAPSFRQEASRQGLTVSFIPYᵀKEEGVFTVTPD
TKSKVYLRTPNWDRGLPSLTSVSWNISVPR**DQVACLTFFK**ERSGVVCQTGR**AFMIIQEQR**TRAEE⁻FSL
DEDVLPKPSFHHHSFWVNISNCSPTSGKQLDLLFSVTLTPRTVDLTVILIAAVGGGVLLLSALGL⁻ICC
VKKKKKKTNKGPAVGIYNGNINTEMPRQPKKFQKGRKDNDSHVYAVIEDTMVYGHLLQDSSGSFLQPEV
DTYRPFQGTMGVCPPSPPTICSRAPTAK**LATEEPPPR**SPPESESEPYTFSHPNNGDVSSKDTDIPLLST
QEPMEPAE


### Mass Match Peptides (bold):

> AFMIIQEQR [66]
> ASVSFLNFNLSNCER [87]
> DQVACLTFFK [153]
> IYVVDLSNER [404]
> KFVPGCFVCLESR [415]
> LATEEPPPR [444]
> MALHLPWFHPR [508]

### Tandem Peptides (underline):

> IYVVDLSNER [404]
> LATEEPPPR [444]


## Peptide Source: 1D-GE, Pancreatic cancer

MAGLNCGVSIALLGVLLLGAARLPRGAEAFEIALPRESNITVLIKLGTPTLLAKPCYIVISKRHI⁻MLS
IKSGERIVFTFSCQSPENHFVIEIQKNIDCMSGPCPFGEVQLQPSTSLLPTLNRTFIWDVKAHKS⁻GLE
LQFSIPRLR**QIGPGESCPDGVTHSISGR**IDATVVRIGTFCSNGTVSRIKMQEGVKMALHLPWFHPRNVS
GFSIANRSSIKRLCIIESVFEGEGSATLMSANYPEGFPEDELMTWQFVVPAHLRASVSFLNFNLSNCER
KEER**VEYYIPGSTTNPEVFK**LEDK**QPGNMAGNFNLSLQGCDQDAQSPGILR**LQFQVLVQHPQNESNKIY
VVDLSNERAMSLTIEPRPVKQSRKFVPGCFVCLESRTCSSNLᵀLTSGSKHKISFLCDDLTRLWMNVEKT
ISCTDHRYCQRKSYSLQVPSDILHLPVELHDFSWKLLVPKDRLSLVLVPAQKLQQHTHEKPCNTSFSYL
VASAIPSQDLYᵀGSFCPGGSIKQIQVKQNISVTLRTFAPSFRQEASR**QGLTVSFIPYFK**EEGVFTVTPD
TKSKVYLRTPNWDRGLPSLTSVSWNISVPRDQVACLTFFKERSGVVCQTGRAFMIIQEQRTRAEE⁻FSL
DEDVLPKPSFHHHSFWVNISNCSPTSGKQLDLLFSVTLTPRTVDLTVILIAAVGGGVLLLSALGL⁻ICC
VKKKKKKTNKGPAVGIYNGNINTEMPRQPKKFQKGRKDNDSHVYAVIEDTMVYGHLLQDSSGSFLQPEV
DTYRPFQGTMGVCPPSPPTICSRAPTAKLATEEPPPRSPPESESEPYTFSHPNNGDVSSKDTDIPLLST
QEPMEPAE


### Mass Match Peptides (bold):

> QGLTVSFIPYFK [576]
> QIGPGESCPDGVTHSISGR [580]
> QPGNMAGNFNLSLQGCDQDAQSPGILR [583]
> VEYYIPGSTTNPEVFK [757]

### Tandem Peptides (underline):

QGLTVSFIPYFK [576]

## Peptide Source: 1D-GE, Prostate cancer

MAGLNCGVSIALLGVLLLGAARLPRGAEAFEIALPRESNITVLIKLGTPTLLAKPCYIVISKRHI⁻MLS
IKSGERIVFTFSCQSPENHFVIEIQKNIDCMSGPCPFGEVQLQPSTSLLPTLNRTFIWDVKAHKS⁻GLE
LQFSIPRLRQIGPGESCPDGVTHSISGR**IDATVVR**IGTFCSNGTVSRIKMQEGVKMALHLPWFHPRNVS
GFSIANRSSIKRLCIIESVFEGEGSATLMSANYPEGFPEDELMTWQFVVPAHLRASVSFLNFNLSNCER
KEER<u>**VEYYIPGSTTNPEVFK**</u>LEDKQPGNMAGNFNLSLQGCDQDAQSPGILRLQFQVLVQHPQNESNK<u>IY</u>
<u>VVDLSNER</u>AMSLTIEPRPVKQSRKFVPGCFVCLESRTCSSNL⁻LTSGSKHHISFLCDDLTR**LWMNVEK**T
ISCTDHRYCQRKSYSLQVPSDILHLPVELHDFSWKLLVPKDRLSLVLVPAQKLQQHTHEKPCNTSFSYL
VASAIPSQDLY⁻GSFCPGGSIKQIQVKQNISVTLRTFAPSFRQEASRQGLTVSFIPY⁻K**EEGVFTVTPD**
**TK**SKVYLR**TPNWDR**GLPSLTSVSWNISVPRDQVACLTFFKERSGVVCQTGR<u>AFMIIQEQR</u>TRAEE⁻FSL
DEDVLPKPSFHHHSFWVNISNCSPTSGKQLDLLFSVTLTPRTVDLTVILIAAVGGGVLLLSALGL⁻ICC
VKKKKKKTNKGPAVGIYNGNINTEMPRQPKKFQKGRKDNDSHVYAVIEDTMVYGHLLQDSSGSFLQPEV
DTYRPFQGTMGVCPPSPPTICSRAPTAK**LATEEPPPR**SPPESESEPYTFSHPNNGDVSSKDTDIPLLST
QEPMEPAE

### Mass Match Peptides (bold):

AFMIIQEQR [66]
EEGVFTVTPDTK [172]
IDATVVR [353]
IYVVDLSNER [404]
LATEEPPPR [444]
LWMNVEK [505]
TPNWDR [719]
VEYYIPGSTTNPEVFK [757]

### Tandem Peptides (underline):

AFMIIQEQR [66]
IYVVDLSNER [404]

## Peptide Source: 1D-GE, Renal cell cancer

MAGLNCGVSIALLGVLLLGAARLPRGAEAFEIALPRESNITVLIKLGTPTLLAKPCYIVISKRHI⁻MLS
IKSGERIVFTFSCQSPENHFVIEIQKNIDCMSGPCPFGEVQLQPSTSLLPTLNRTFIWDVKAHKS⁻GLE
LQFSIPRLRQIGPGESCPDGVTHSISGRIDATVVRIGTFCSNGTVSRIKMQEGVKMALHLPWFHPRNVS
GFSIANRSSIKRLCIIESVFEGEGSATLMSANYPEGFPEDELMTWQFVVPAHLRASVSFLNFNLSNCER
KEERVEYYIPGSTTNPEVFKLEDKQPGNMAGNFNLSLQGCDQDAQSPGILRLQFQVLVQHPQNESNKIY
VVDLSNERAMSLTIEPRPVKQSR**KFVPGCFVCLESR**TCSSNL⁻LTSGSKHHISFLCDDLTRLWMNVEKT
ISCTDHRYCQRKSYSLQVPSDILHLPVELHDFSWKLLVPKDR<u>LSLVLVPAQK</u>LQQHTHEKPCNTSFSYL
VASAIPSQDLY⁻GSFCPGGSIKQIQVKQNISVTLRTFAPSFRQEASRQGLTVSFIPY⁻KEEGVFTVTPD
TKSKVYLRTPNWDRGLPSLTSVSWNISVPRDQVACLTFFKER**SGVVCQTGR**AFMIIQEQRTRAEE⁻FSL
DEDVLPKPSFHHHSFWVNISNCSPTSGKQLDLLFSVTLTPRTVDLTVILIAAVGGGVLLLSALGL⁻ICC
VKKKKKKTNKGPAVGIYNGNINTEMPRQPKKFQKGRKDNDSHVYAVIEDTMVYGHLLQDSSGSFLQPEV
DTYRPFQGTMGVCPPSPPTICSRAPTAKLATEEPPPRSPPESESEPYTFSHPNNGDVSSKDTDIPLLST
QEPMEPAE

### Mass Match Peptides (bold):

KFVPGCFVCLESR [415]
SGVVCQTGR [633]

### Tandem Peptides (underline):

LSLVLVPAQK [487]

## Peptide Source: iTRAQ, Non-small cell lung cancer

MAGLNCGVSIALLGVLLLGAARLPRGAEAFEIALPRESNITVLIKLGTPTLLAKPCYIVISKRHITMLS
IKSGERIVFTFSCQSPENHFVIEIQKNIDCMSGPCPFGEVQLQPSTSLLPTLNRTFIWDVKAHKSIGLE
LQFSIPRLRQIGPGESCPDGVTHSISGRIDATVVR<u>IGTFCSNGTVSRIK</u>MQEGVKMALHLPWFHPRNVS
GFSIANRSSIKRLCIIESVFEGEGSATLMSANYPEGFPEDELMTWQFVVPAHLRASVSFLNFNLSNCER
KEERVEYYIPGSTTNPEVFKLEDKQPGNMAGNFNLSLQGCDQDAQSPGILRLQFQVLVQHPQNESNKIY
VVDLSNERAMSLTIEPRPVKQSRKFVPGCFVCLESRTCSSNLTLTSGSKHKISFLCDDLTR<u>LWMNVEK</u>T
ISCTDHRYCQRKSYSLQVPSDILHLPVELHDFSWK<u>LLVPKDR</u>LSLVLVPAQKLQQHTHEKPCNTSFSYL
VASAIPSQDLYFGSFCPGGSIKQIQVKQNISVTLRTFAPSFRQEASRQGLTVSFIPYFKEEGVFTVTPD
TKSKVYLRTPNWDRGLPSLTSVSWNISVPRDQVACLTFFKERSGVVCQTGRAFMIIQEQRTRAEEIFSL
DEDVLPKPSFHHHSFWVNISNCSPTSGKQLDLLFSVTLTPRTVDLTVILIAAVGGGVLLLSALGLIICC
VKKKKKKTNKGPAVGIYNGNINTEMPRQPKKFQKGRKDNDSHVYAVIEDTMVYGHLLQDSSGSFLQPEV
DTYRPFQGTMGVCPPSPPTICSRAPTAKLATEEPPPRSPPESESEPYTFSHPNNGDVSSKDTDIPLLST
QEPMEPAE

### Mass Match Peptides (bold):
    IGTFCSNGTVSRIK [1007]
    LLVPKDR [940]
    LWMNVEK [505]

### Tandem Peptides (underline):
    IGTFCSNGTVSRIK [1007]
    LLVPKDR [940]
    LWMNVEK [505]


## OGTA156 (SEQ ID No: 27)

## Peptide Source: 1D-GE, Acute T-cell leukaemia

MFPTESAWLGKRGANPGPEAAVRETVMLLLCLGVPTGRPYNVDTESALLYQGPHNTLFGYSVVLHSHGA
NRWLLVGAPTANWLANASVINPGAIYRCRIGKNPGQTCEQLQLGSPNGEPCGKTCLEER<u>DNQWLGVTLS</u>
<u>R</u>QPGENGSIVTCGHRWKNIFYIKNENK<u>LPTGGCYGVPPDLR</u>TELSKRIAPCYQDYVKKFGENFASCQAG
ISSFYTKDLIVMGAPGSSYWTGSLFVYNITTNKYKAFLDKQNQVKFGSYLGYSVGAGHFR<u>SQHTTEVVG</u>
<u>GAPQHEQIGKAYIFSIDEK</u>ELNILHEMKGKKLGSYFGASVCAVDLNADGFSDLLVGAPMQSTIREEGRV
FVYINSGSGAVMNAMETNLVGSDKYAARFGESIVNLGDIDNDGFEDVAIGAPQEDDLQGAIYIYNGR<u>AD</u>
<u>GISSTFSQR</u>IEGLQISKSLSMFGQSISGQIDADNNGYVDVAVGAFRSDSAVLLRTRPVVIVDASLSHPE
SVNRTKFDCVENGWPSVCIDLTLCFSYKGKEVPGYIVLFYNMSLDVNRKAESPPRFYFSSNGTSDVITG
SIQVSSREANCR<u>THQAFMR</u>KDVRDILTPIQIEAAYHLGPHVISKRSTEEFPPLQPILQQKKEKDIMKK<u>T</u>
<u>INFAR</u>FCAHENCSADLQVSAKIGFLKPHENKTYLAVGSMKTLMLNVSLFNAGDDAYETTLHVKLPVGLY
FIKILELEEKQINCEVTDNSGVVQLDCSIGYIYVDHLSRIDISFLLDVSSLSRAEEDLSITVHATCENE
EEMDNLKHSR<u>VTVAIPLK</u>YEVKLTVHGFVNPTSFVYGSNDENEPETCMVEKMNLTFHVINTGNSMAPNV
SVEIMVPNSFSPQTDKLFNILDVQTTTGECHFENYQRVCALEQQKSAMQTLKGIVRFLSKTDKRLLYCI
KADPHCLNFLCNFGKMESGKEASVHIQLEGRPSILEMDETSALKFEIR<u>ATGFPEPNPR</u>VIELNKDENVA
HVLLEGLHHQRPKRYFTIVIISSSLLLGLIVLLLISYVMWKAGFFKRQYK<u>SILQEENR</u>RDSWSYINSKS
NDD

### Mass Match Peptides (bold):
    ADGISSTFSQR [61]
    ATGFPEPNPR [90]
    AYIFSIDEK [99]
    DNQWLGVTLSR [147]
    LPTGGCYGVPPDLR [482]

SILQEENR [637]
SQHTTEVVGGAPQHEQIGK [648]
THQAFMR [697]
TINFAR [701]
VTVAIPLK [807]

**Tandem Peptides (underline):**
ADGISSTFSQR [61]
ATGFPEPNPR [90]
AYIFSIDEK [99]
LPTGGCYGVPPDLR [482]
SILQEENR [637]


**Peptide Source: 1D-GE, B-cell non-Hodgkin's lymphoma**

MFPTESAWLGKRGANPGPEAAVRETVMLLLCLGVPTGRPYNVDTESALLYQGPHNTLFGYSVVLHSHGA
NRWLLVGAPTANWLANASVINPGAIYRCRIGKNPGQTCEQLQLGSPNGEPCGKTCLEERDNQWLGVTLS
RQPGENGSIVTCGHRWKNIFYIKNENKLPTGGCYGVPPDLRTELSKRIAPCYQDYVKKFGENFASCQAG
ISSFYTKDLIVMGAPGSSYWTGSLFVYNITTNKYKAFLDKQNQVKFGSYLGYSVGAGHFRSQHTTEVVG
GAPQHEQIGKAYIFSIDEKELNILHEMKGKKLGSYFGASVCAVDLNADGFSDLLVGAPMQSTIREEGRV
FVYINSGSGAVMNAMETNLVGSDKYAARFGESIVNLGDIDNDGFEDVAIGAPQEDDLQGAIYIYNGRAD
GISSTFSQRIEGLQISKSLSMFGQSISGQIDADNNGYVDVAVGAFRSDSAVLLRTRPVVIVDASLSHPE
SVNRTKFDCVENGWPSVCIDLTLCFSYKGKEVPGYIVLFYNMSLDVNRKAESPPRFYFSSNGTSDVITG
SIQVSSREANCRTHQAFMRKDVRDILTPIQIEAAYHLGPHVISKRSTEEFPPLQPILQQKKEKDIMKKT
INFARFCAHENCSADLQVSAKIGFLKPHENKTYLAVGSMKTLMLNVSLFNAGDDAYETTLHVKLPVGLY
FIKILELEEKQINCEVTDNSGVVQLDCSIGYIYVDHLSRIDISFLLDVSSLSRAEEDLSITVHATCENE
EEMDNLKHSRVTVAIPLKYEVKLTVHGFVNPTSFVYGSNDENEPETCMVEKMNLTFHVINTGNSMAPNV
SVEIMVPNSFSPQTDKLFNILDVQTTTGECHFENYQRVCALEQQKSAMQTLKGIVRFLSKTDKRLLYCI
KADPHCLNFLCNFGKMESGKEASVHIQLEGRPSILEMDETSALKFEIRATGFPEPNPRVIELNKDENVA
HVLLEGLHHQRPKRYFTIVIISSSLLLGLIVLLLISYVMWKAGFFKRQYKSILQEENRRDSWSYINSKS
NDD

**Mass Match Peptides (bold):**
ADGISSTFSQR [61]
ATGFPEPNPR [90]
AYIFSIDEK [99]
DNQWLGVTLSR [147]
IAPCYQDYVK [351]
KAESPPR [407]
NIFYIK [540]
SDSAVLLR [624]
SILQEENR [637]
SQHTTEVVGGAPQHEQIGK [648]
TCLEER [678]
THQAFMRK [698]
TINFAR [701]
TYLAVGSMK [740]

**Tandem Peptides (underline):**
EASVHIQLEGRPSILEMDETSALK [167]
LPVGLYFIK [484]
STEEFPPLQPILQQK [654]


**Peptide Source: 1D-GE, Chronic lymphocytic leukaemia**

MFPTESAWLGKRGANPGPEAAVRETVMLLLCLGVPTGRPYNVDTESALLYQGPHNTLFGYSVVLHSHGA
NRWLLVGAPTANWLANASVINPGAIYRCRIGKNPGQTCEQLQLGSPNGEPCGKTCLEER**DNQWLGVTLS**
**R**QPGENGSIVTCGHRWKNIFYIKNENKLPTGGCYGVPPDLRTELSKRIAPCYQDYVKKFGENFASCQAG
**I**SSFYTKDLIVMGAPGSSYWTGSLFVYNITTNKYKAFLDKQNQVKFGSYLGYSVGAGHFR**SQHTTEVVG**
**GAPQHEQIGK**AYIFSIDEK**ELNILHEMK**GKKLGSYFGASVCAVDLNADGFSDLLVGAPMQSTIREEGRV
FVYINSGSGAVMNAMETNLVGSDKYAARFGESIVNLGDIDNDGFEDVAIGAPQEDDLQGAIYIYNGRAD
GISSTFSQRIEGLQISKSLSMFGQSISGQIDADNNGYVDVAVGAFRSDSAVLLRTRPVVIVDASLSHPE
SVNRTKFDCVENGWPSVCIDLTLCFSYKGK**EVPGYIVLFYNMSLDVNR**KAESPPRFYFSSNGTSDVITG
SIQVSSREANCRTHQAFMRKDVRDILTPIQIEAAYHLGPHVISKR**STEEFPPLQPILQQK**KEKDIMKKT
INFARFCAHENCSADLQVSAKIGFLKPHENKTYLAVGSMKTLMLNVSLFNAGDDAYETTLHVKLPVGLY
FIK**ILELEEK**QINCEVTDNSGVVQLDCSIGYIYVDHLSRIDISFLLDVSSLSRAEEDLSITVHATCENE
EEMDNLKHSRVTVAIPLKYEVKLTVHGFVNPTSFVYGSNDENEPETCMVEKMNLTFHVINTGNSMAPNV
SVEIMVPNSFSPQTDKLFNILDVQTTTGECHFENYQRVCALEQQKSAMQTLKGIVRFLSKTDKRLLYCI
KADPHCLNFLCNFGKMESGKEASVHIQLEGRPSILEMDETSALKFEIRATGFPEPNPRVIELNKDENVA
HVLLEGLHHQRPKRYFTIVIISSSLLLGLIVLLLISYVMWKAGFFKRQYKSILQEENRRDSWSYINSKS
NDD

**Mass Match Peptides (bold):**

    DNQWLGVTLSR [147]
    ELNILHEMK [205]
    EVPGYIVLFYNMSLDVNR [235]
    ILELEEK [376]
    SQHTTEVVGGAPQHEQIGK [648]

**Tandem Peptides (underline):**

    ELNILHEMK [205]
    STEEFPPLQPILQQK [654]


## Peptide Source: 1D-GE, Hepatocellular carcinoma

MFPTESAWLGKRGANPGPEAAVRETVMLLLCLGVPTGRPYNVDTESALLYQGPHNTLFGYSVVLHSHGA
NRWLLVGAPTANWLANASVINPGAIYRCRIGKNPGQTCEQLQLGSPNGEPCGKTCLEERDNQWLGVTLS
RQPGENGSIVTCGHRWKNIFYIKNENKLPTGGCYGVPPDLRTELSKRIAPCYQDYVKKFGENFASCQAG
ISSFYTKDLIVMGAPGSSYWTGSLFVYNITTNKYKAFLDKQNQVKFGSYLGYSVGAGHFRSQHTTEVVG
GAPQHEQIGKAYIFSIDEKELNILHEMKGKKLGSYFGASVCAVDLNADGFSDLLVGAPMQSTIREEGRV
FVYINSGSGAVMNAMETNLVGSDKYAARFGESIVNLGDIDNDGFEDVAIGAPQEDDLQGAIYIYNGRAD
GISSTFSQR**IEGLQISK**SLSMFGQSISGQIDADNNGYVDVAVGAFRSDSAVLLRTRPVVIVDASLSHPE
SVNRTKFDCVENGWPSVCIDLTLCFSYKGKEVPGYIVLFYNMSLDVNRKAESPPRFYFSSNGTSDVITG
SIQVSSREANCRTHQAFMRKDVRDILTPIQIEAAYHLGPHVISKRSTEEFPPLQPILQQKKEKDIMKKT
INFARFCAHENCSADLQVSAKIGFLKPHENKTYLAVGSMKTLMLNVSLFNAGDDAYETTLHVKLPVGLY
FIKILELEEKQINCEVTDNSGVVQLDCSIGYIYVDHLSRIDISFLLDVSSLSRAEEDLSITVHATCENE
EEMDNLKHSRVTVAIPLKYEVKLTVHGFVNPTSFVYGSNDENEPETCMVEKMNLTFHVINTGNSMAPNV
SVEIMVPNSFSPQTDKLFNILDVQTTTGECHFENYQRVCALEQQKSAMQTLKGIVRFLSKTDKRLLYCI
KADPHCLNFLCNFGKMESGKEASVHIQLEGRPSILEMDETSALKFEIRATGFPEPNPR**VIELNK**DENVA
HVLLEGLHHQRPKRYFTIVIISSSLLLGLIVLLLISYVMWKAGFFKRQYKSILQEENRRDSWSYINSKS
NDD

**Mass Match Peptides (bold):**

    VIELNK [768]

**Tandem Peptides (underline):**

    IEGLQISK [361]


## Peptide Source: iTRAQ, Colorectal cancer

MFPTESAWLGKRGANPGPEAAVRETVMLLLCLGVPTGRPYNVDTESALLYQGPHNTLᖷGYSVVLHSHGA
NRWLLVGAPTANWLANASVINPGAIYRCRIGKNPGQTCEQLQLGSPNGEPCGKTCLEERDNQWLGVTLS
RQPGENGSIVTCGHRWKNIFYIKNENKLPTGGCYGVPPDLRTELSKRIAPCYQDYVKKFGENFASCQAG
ISSFYTKDLIVMGAPGSSYWTGSLFVYNITTNKYKAFLDKQNQVKFGSYLGYSVGAGHFRSQHTTEVVG
GAPQHEQIGKAYIFSIDEKELNILHEMKGKKLGSYFGASVCAVDLNADGFSDLLVGAPMQSTIREEGRV
FVYINSGSGAVMNAMETNLVGSDKYAARFGESIVNLGDIDNDGFEDVAIGAPQEDDLQGAIYIYNGRAD
GISSTFSQRIEGLQISKSLSMFGQSISGQIDADNNGYVDVAVGAFRSDSAVLLRTRPVVIVDASLSHPE
SVNRTKFDCVENGWPSVCIDLTLCFSYKGKEVPGYIVLFYNMSLDVNRKAESPPRFYᖷSSNGTSDVITG
SIQVSSREANCRTHQAFMRKDVRDILTPIQIEAAYHLGPHVISKRSTEEFPPLQPILQQKKEK**DIMKKT**
**INFAR**FCAHENCSADLQVSAKIGFLKPHENKTYLAVGSMKTLMLNVSLFNAGDDAYETTLHVKLPVGLY
FIKILELEEKQINCEVTDNSGVVQLDCSIGYIYVDHLSRIDISFLLDVSSLSRAEEDLSITVHATCENE
EEMDNLKHSRVTVAIPLKYEVKLTVHGFVNPTSFVYGSNDENEPETCMVEKMNLTFHVINTGNSMAPNV
SVEIMVPNSFSPQTDKLFNILDVQTTTGECHFENYQRVCALEQQKSAMQTLKGIVRFLSKTDKRLLYCI
KADPHCLNFLCNFGKMESGKEASVHIQLEGRPSILEMDETSALKFEIRATGFPEPNPRVIELNKDENVA
HVLLEGLHHQRPKRYFTIVIISSSLLLGLIVLLLISYVMWKAGFFKRQYKSILQEENRRDSWSYINSKS
NDD

**Mass Match Peptides (bold):**
   DIMKKTINFAR [891]
**Tandem Peptides (underline):**
   DIMKKTINFAR [891]


## Peptide Source: iTRAQ, Non-small cell lung cancer

MFPTESAWLGKRGANPGPEAAVRETVMLLLCLGVPTGRPYNVDTESALLYQGPHNTLᖷGYSVVLHSHGA
NRWLLVGAPTANWLANASVINPGAIYRCRIGKNPGQTCEQLQLGSPNGEPCGKTCLEERDNQWLGVTLS
RQPGENGSIVTCGHRWKNIFYIKNENKLPTGGCYGVPPDLRTELSKRIAPCYQDYVKKFGENFASCQAG
ISSFYTKDLIVMGAPGSSYWTGSLFVYNITTNKYKAFLDKQNQVKFGSYLGYSVGAGHFRSQHTTEVVG
GAPQHEQIGKAYIFSIDEKELNILHEMKGKKLGSYFGASVCAVDLNADGFSDLLVGAPMQSTIREEGRV
FVYINSGSGAVMNAMETNLVGSDKYAARFGESIVNLGDIDNDGFEDVAIGAPQEDDLQGAIYIYNGRAD
GISSTFSQRIEGLQISKSLSMFGQSISGQIDADNNGYVDVAVGAFRSDSAVLLRTRPVVIVDASLSHPE
SVNRTKFDCVENGWPSVCIDLTLCFSYKGKEVPGYIVLFYNMSLDVNRKAESPPRFYᖷSSNGTSDVITG
SIQVSSREANCRTHQAFMRKDVRDILTPIQIEAAYHLGPHVISKRSTEEFPPLQPILQQKKEKDIMKKT
INFARFCAHENCSADLQVSAKIGFLKPHENK**TYLAVGSMK**TLMLNVSLFNAGDDAYETTLHVKLPVGLY
FIKILELEEKQINCEVTDNSGVVQLDCSIGYIYVDHLSRIDISFLLDVSSLSRAEEDLSITVHATCENE
EEMDNLK**HSRVTVAIPLK**YEVKLTVHGFVNPTSFVYGSNDENEPETCMVEKMNLTFHVINTGNSMAPNV
SVEIMVPNSFSPQTDKLFNILDVQTTTGECHFENYQRVCALEQQKSAMQTLKGIVRFLSKTDKRLLYCI
KADPHCLNFLCNFGKMESGKEASVHIQLEGRPSILEMDETSALKFEIRATGFPEPNPRVIELNKDENVA
HVLLEGLHHQRPKRYFTIVIISSSLLLGLIVLLLISYVMWKAGFFKRQYKSILQEENRRDSWSYINSKS
NDD

**Mass Match Peptides (bold):**
   HSRVTVAIPLK [918]
   ᖷYLAVGSMK [740]
**Tandem Peptides (underline):**
   HSRVTVAIPLK [918]
   ᖷYLAVGSMK [740]


## Peptide Source: iTRAQ, Ovarian cancer

MFPTESAWLGKRGANPGPEAAVRETVMLLLCLGVPTGRPYNVDTESALLYQGPHNTLFGYSVVLHSHGA
NRWLLVGAPTANWLANASVINPGAIYRCRIGKNPGQTCEQLQLGSPNGEPCGKTCLEERDNQWLGVTLS
RQPGENGSIVTCGHRWKNIFYIKNENKLPTGGCYGVPPDLRTELSKRIAPCYQDYVKKFGENFASCQAG
ISSFYTKDLIVMGAPGSSYWTGSLFVYNITTNKYKAFLDKQNQVKFGSYLGYSVGAGHFRSQHTTEVVG
GAPQHEQIGKAYIFSIDEKELNILHEMKGKKLGSYFGASVCAVDLNADGFSDLLVGAPMQSTIREEGRV
FVYINSGSGAVMNAMETNLVGSDKYAARFGESIVNLGDIDNDGFEDVAIGAPQEDDLQGAIYIYNGRAD
GISSTFSQRIEGLQISKSLSMFGQSISGQIDADNNGYVDVAVGAFRSDSAVLLRTRPVVIVDASLSHPE
SVNRTKFDCVENGWPSVCIDLTLCFSYKGKEVPGYIVLFYNMSLDVNRKAESPPRFYFSSNGTSDVITG
SIQVSSREANCRTHQAFMRKDVRDILTPIQIEAAYHLGPHVISKRSTEEFPPLQPILQQKKEK**<u>DIMKKT
INFAR</u>**FCAHENCSADLQVSAKIGFLKPHENKTYLAVGSMKTLMLNVSLFNAGDDAYETTLHVKLPVGLY
FIKILELEEKQINCEVTDNSGVVQLDCSIGYIYVDHLSRIDISFLLDVSSLSRAEEDLSITVHATCENE
EEMDNLKHSRVTVAIPLKYEVKLTVHGFVNPTSFVYGSNDENEPETCMVEKMNLTFHVINTGNSMAPNV
SVEIMVPNSFSPQTDKLFNILDVQTTTGECHFENYQRVCALEQQKSAMQTLKGIVRFLSKTDKRLLYCI
KADPHCLNFLCNFGKMESGKEASVHIQLEGRPSILEMDETSALKFEIRATGFPEPNPRVIELNKDENVA
HVLLEGLHHQRPKRYFTIVIISSSLLLGLIVLLLISYVMWKAGFFKRQYKSILQEENRRDSWSYINSKS
NDD

**Mass Match Peptides (bold):**
DIMKKTINFAR [891]

**Tandem Peptides (underline):**
DIMKKTINFAR [891]


## Peptide Source: iTRAQ, Renal cell cancer

MFPTESAWLGKRGANPGPEAAVRETVMLLLCLGVPTGRPYNVDTESALLYQGPHNTLFGYSVVLHSHGA
NRWLLVGAPTANWLANASVINPGAIYRCRIGKNPGQTCEQLQLGSPNGEPCGKTCLEERDNQWLGVTLS
RQPGENGSIVTCGHRWKNIFYIKNENKLPTGGCYGVPPDLRTELSKRIAPCYQDYVKKFGENFASCQAG
ISSFYTKDLIVMGAPGSSYWTGSLFVYNITTNKYKAFLDKQNQVKFGSYLGYSVGAGHFRSQHTTEVVG
GAPQHEQIGKAYIFSIDEKELNILHEMKGKKLGSYFGASVCAVDLNADGFSDLLVGAPMQSTIREEGRV
FVYINSGSGAVMNAMETNLVGSDKYAARFGESIVNLGDIDNDGFEDVAIGAPQEDDLQGAIYIYNGRAD
GISSTFSQRIEGLQISKSLSMFGQSISGQIDADNNGYVDVAVGAFRSDSAVLLRTRPVVIVDASLSHPE
SVNRTKFDCVENGWPSVCIDLTLCFSYKGKEVPGYIVLFYNMSLDVNRKAESPPRFYFSSNGTSDVITG
SIQVSSREANCRTHQAFMRKDVRDILTPIQIEAAYHLGPHVISKRSTEEFPPLQPILQQKKEK**<u>DIMKKT
INFAR</u>**FCAHENCSADLQVSAKIGFLKPHENKTYLAVGSMKTLMLNVSLFNAGDDAYETTLHVKLPVGLY
FIKILELEEKQINCEVTDNSGVVQLDCSIGYIYVDHLSRIDISFLLDVSSLSRAEEDLSITVHATCENE
EEMDNLK**<u>HSRVTVAIPLK</u>**YEVKLTVHGFVNPTSFVYGSNDENEPETCMVEKMNLTFHVINTGNSMAPNV
SVEIMVPNSFSPQTDKLFNILDVQTTTGECHFENYQRVCALEQQKSAMQTLKGIVRFLSKTDKRLLYCI
KADPHCLNFLCNFGKMESGKEASVHIQLEGRPSILEMDETSALKFEIRATGFPEPNPRVIELNKDENVA
HVLLEGLHHQRPKRYFTIVIISSSLLLGLIVLLLISYVMWKAGFFKRQYKSILQEENRRDSWSYINSKS
NDD

**Mass Match Peptides (bold):**
DIMKKTINFAR [891]
HSRVTVAIPLK [918]

**Tandem Peptides (underline):**
DIMKKTINFAR [891]
HSRVTVAIPLK [918]


## OGTA159 (SEQ ID No: 28)

## Peptide Source: 1D-GE, Breast cancer

MVAPVWYLVAAALLVGFILFLTRSRGRAASAGQEPLHNEELAGAGR**VAQPGPLEPEEPR**AGGRPRRRRD
LGSRLQAQRRAQRVAWAEADENEEEAVILAQEEEGVEKPAETHLSGKIGAKKLRKLEEKQARKAQREAE
EAEREERKRLESQREAEWKKEEEERLRLEEEQKEEEERKAREEQAQREHEEYLKLKEAFVVEEEGVGETM
TEEQSQSFLTEFINYIKQSKVVLLEDLASQVGLRTQDTINRIQDLLAEGTITGVIDDRGKFIYITPEEL
AAVANFIRQRGRVSIAELAQASNSLIAWGR**ESPAQAPA**

### Mass Match Peptides (bold):
    ESPAQAPA [226]
    VAQPGPLEPEEPR [745]
### Tandem Peptides (underline):
    VAQPGPLEPEEPR [745]


## Peptide Source: 1D-GE, Chronic lymphocytic leukaemia

MVAPVWYLVAAALLVGFILFLTRSRGRAASAGQEPLHNEELAGAGRVAQPGPLEPEEPRAGGRPRRRRD
LGSRLQAQRRAQRVAWAEADENEEEAVILAQEEEGVEKPAETHLSGKIGAKKLRKLEEKQARKAQREAE
EAEREERKRLESQREAEWKKEEEERLR**LEEEQK**EEEERKAREEQAQREHEEYLKLKEAFVVEEEGVGETM
TEEQSQSFLTEFINYIKQSKVVLLEDLASQVGLRTQDTINRIQDLLAEGTITGVIDDRGKFIYITPEEL
AAVANFIRQRGRVSIAELAQASNSLIAWGRESPAQAPA

### Mass Match Peptides (bold):
    LEEEQK [450]
### Tandem Peptides (underline):


## Peptide Source: 1D-GE, Colorectal cancer

MVAPVWYLVAAALLVGFILFLTRSRGR**AASAGQEPLHNEELAGAGRVAQPGPLEPEEPR**AGGRPRRRRD
LGSRLQAQRRAQRVAWAEADENEEEAVILAQEEEGVEKPAETHLSGKIGAKKLRKLEEKQARKAQREAE
EAEREERKRLESQREAEWKKEEEERLRLEEEQKEEEERKAR**EEQAQR**EHEEYLKLKEAFVVEEEGVGETM
TEEQSQSFLTEFINYIKQSKVVLLEDLASQVGLRTQDTINRIQDLLAEGTITGVIDDRGKFIYITPEEL
AAVANFIRQRGRVSIAELAQASNSLIAWGRESPAQAPA

### Mass Match Peptides (bold):
    AASAGQEPLIINEELAGAGR [53]
    EEQAQR [180]
    VAQPGPLEPEEPR [745]
### Tandem Peptides (underline):
    VAQPGPLEPEEPR [745]


## Peptide Source: 1D-GE, Hepatocellular carcinoma

MVAPVWYLVAAALLVGFILFLTRSRGRAASAGQEPLHNEELAGAGR**VAQPGPLEPEEPR**AGGRPRRRRD
LGSRLQAQRRAQRVAWAEADENEEEAVILAQEEEGVEKPAETHLSGKIGAKKLRKLEEKQARKAQREAE
EAEREERKRLESQREAEWKKEEEERLRLEEEQKEEEERKAREEQAQREHEEYLKLKEAFVVEEEGVGETM
TEEQSQSFLTEFINYIKQSKVVLLEDLASQVGLRTQDTINRIQDLLAEGTITGVIDDRGKFIYITPEEL
AAVANFIRQRGRVSIAELAQASNSLIAWGRESPAQAPA

### Mass Match Peptides (bold):
    VAQPGPLEPEEPR [745]

Tandem Peptides (underline):

        VAQPGPLEPEEPR [745]

## Peptide Source: 1D-GE, Melanoma

MVAPVWYLVAAALLVGFILFLTRSRGRAASAGQEPLHNEELAGAGRVAQPGPLEPEEPRAGGRPRRRRD
LGSRLQAQRRAQRVAWAEADENEEEAVILAQEEEGVEKPAETHLSGKIGAKKLRKLEEKQARKAQREAE
EAEREERKRLESQREAEWKKEEERLRLEEEQKEEEERKAREEQAQREHEEYLKLKEAFVVEEEGVGETM
TEEQSQSFLTEFINYIKQSKVVLLEDLASQVGLRTQDTINRIQDLLAEGTITGVIDDRGKFIYITPEEL
AAVANFIRQRGRVSIAELAQASNSLIAWGRESPAQAPA

Mass Match Peptides (bold):

        IQDLLAEGTITGVIDDR [387]
        RDLGSR [598]
        VVLLEDLASQVGLR [811]
        VVLLEDLASQVGLR [811]

## Peptide Source: 1D-GE, Pancreatic cancer

MVAPVWYLVAAALLVGFILFLTRSRGRAASAGQEPLHNEELAGAGRVAQPGPLEPEEPRAGGRPRRRRD
LGSRLQAQRRAQRVAWAEADENEEEAVILAQEEEGVEKPAETHLSGKIGAKKLRKLEEKQARKAQREAE
EAEREERKRLESQREAEWKKEEERLRLEEEQKEEEERKAREEQAQREHEEYLKLKEAFVVEEEGVGETM
TEEQSQSFLTEFINYIKQSKVVLLEDLASQVGLRTQDTINRIQDLLAEGTITGVIDDRGKFIYITPEEL
AAVANFIRQRGRVSIAELAQASNSLIAWGRESPAQAPA

Mass Match Peptides (bold):

        AASAGQEPLHNEELAGAGR [53]
        EHEEYLK [193]
        VAQPGPLEPEEPR [745]
        VAQPGPLEPEEPR [745]

## Peptide Source: iTRAQ, Non-small cell lung cancer

MVAPVWYLVAAALLVGFILFLTRSRGRAASAGQEPLHNEELAGAGRVAQPGPLEPEEPRAGGRPRRRRD
LGSRLQAQRRAQRVAWAEADENEEEAVILAQEEEGVEKPAETHLSGKIGAKKLRKLEEKQARKAQREAE
EAEREERKRLESQREAEWKKEEERLRLEEEQKEEEERKAREEQAQREHEEYLKLKEAFVVEEEGVGETM
TEEQSQSFLTEFINYIKQSKVVLLEDLASQVGLRTQDTINRIQDLLAEGTITGVIDDRGKFIYITPEEL
AAVANFIRQRGRVSIAELAQASNSLIAWGRESPAQAPA

Mass Match Peptides (bold):

        RDLGSRLQAQR [965]
        RDLGSRLQAQR [965]

## Peptide Source: iTRAQ, Renal cell cancer

MVAPVWYLVAAALLVGFILFLTRSRGRAASAGQEPLHNEELAGAGRVAQPGPLEPEEPRAGGRPRRRRD
LGSRLQAQRRAQRVAWAEADENEEEAVILAQEEEGVEKPAETHLSGKIGAKKLRKLEEKQARKAQREAE
EAEREERKRLESQREAEWKKEEERLRLEEEQKEEEERKAREEQAQREHEEYLKLKEAFVVEEEGVGETM
TEEQSQSFLTEFINYIKQSKVVLLEDLASQVGLRTQDTINRIQDLLAEGTITGVIDDRGKFIYITPEEL
AAVANFIRQRGRVSIAELAQASNSLIAWGRESPAQAPA

**Mass Match Peptides (bold):**
RDLGSRLQAQR [965]

**Tandem Peptides (underline):**
RDLGSRLQAQR [965]

## OGTA168 (SEQ ID No: 29)

## Peptide Source: 1D-GE, Glioblastoma

MKDDFAEEEEVQSFGYKRFGIQEGTQCTKCKNNWALKFSIILLYILCALLTITVAILGYKVVEKMDNVT
GGMETSRQTYDDKLTAVESDLKKLGDQTGKKAISTNSELSTFRSDILDLRQQLREITEKTSKNKDFLEK
LQASGDALVDRQSQLKETLENNSFLITTVNKTLQAYNGYVTNLQQDTSVLQGNLQNQMYSHNVVIMNLN
NLNLTQVQQRNLITNLQRSVDDTSQAIQRIKNDFQNLQQVFLQAKKDTDWLKEKVQSLQTLAANNSALA
KANNDTLEDMNSQLNSFTGQMENITTISQANEQNLKDLQDLHKDAENRTAIKFNQLEERFQLFETDIVN
IISNISYTAHHLRTLTSNLNEVRTTCTDTLTKHTDDLTSLNNFLANIRLDSVSLRMQQDLMRSRLDTEV
ANLSVIMEEMKLVDSKHGQLIKNFTILQGPPGPRGPRGDRGSQGPPGPTGNKGQKGEKGEPGPPGPAGE
RGPIGPAGPPGERGGKGSKGSQGPKGSRGSPGKPGPQGSSGDPGPPGPPGKEGLPGPQGPPGFQGLQGT
VGEPGVPGPRGLPGLPGVPGMPGPKGPPGPPGPSGAVVPLALQNEPTPAPEDNGCPPHWKNFTDKCYYF
SVEKEIFEDAKLFCEDKSSHLVFINTREEQQWIKKQMVGRESHWIGLTDSERENEWKWLDGTSPDYKNW
KAGQPDNWGHGHGPGEDCAGLIYAGQWNDFQCEDVNNFICEKDRETVLSSAL

**Mass Match Peptides (bold):**
AISTNSELSTFR [74]
ESHWIGLTDSER [225]
GSPGKPGPQGSSGDPGPPGPPGK [317]
GSQGPPGPTGNK [319]
KLGDQTGK [423]
LDTEVANLSVIMEEMK [448]
LGDQTGKK [461]
NDFQNLQQVFLQAK [534]
NLITNLQR [543]

**Tandem Peptides (underline):**
AISTNSELSTFR [74]
LQASGDALVDR [485]
SVDDTSQAIQR [661]

## Peptide Source: 1D-GE, Melanoma

MKDDFAEEEEVQSFGYKRFGIQEGTQCTKCKNNWALKFSIILLYILCALLTITVAILGYKVVEKMDNVT
GGMETSRQTYDDKLTAVESDLKKLGDQTGKKAISTNSELSTFRSDILDLRQQLREITEKTSKNKDFLEK
LQASGDALVDRQSQLKETLENNSFLITTVNKTLQAYNGYVTNLQQDTSVLQGNLQNQMYSHNVVIMNLN
NLNLTQVQQRNLITNLQRSVDDTSQAIQRIKNDFQNLQQVFLQAKKDTDWLKEKVQSLQTLAANNSALA
KANNDTLEDMNSQLNSFTGQMENITTISQANEQNLKDLQDLHKDAENRTAIKFNQLEERFQLFETDIVN
IISNISYTAHHLRTLTSNLNEVRTTCTDTLTKHTDDLTSLNNFLANIRLDSVSLRMQQDLMRSRLDTEV
ANLSVIMEEMKLVDSKHGQLIKNFTILQGPPGPRGPRGDRGSQGPPGPTGNKGQKGEKGEPGPPGPAGE
RGPIGPAGPPGERGGKGSKGSQGPKGSRGSPGKPGPQGSSGDPGPPGPPGKEGLPGPQGPPGFQGLQGT

VGEPGVPGPRGLPGLPGVPGMPGPKGPPGPPGPSGAVVPLALQNEPTPAPEDNGCPPHWKNFTDKCYYF
SVEKEIFEDAKLFCEDKSSHLVFINTREEQQWIKKQMVGRESHWIGLTDSERENEWKWLDGTSPDYKNW
KAGQPDNWGHGHGPGEDCAGLIYAGQWNDFQCEDVNNFICEKDRETVLSSAL

**Mass Match Peptides (bold):**
GEPGPPGPAGER [292]
SVDDTSQAIQR [661]

**Tandem Peptides (underline):**
LTAVESDLK [495]


## OGTA169 (SEQ ID No: 30)


## Peptide Source: 1D-GE, Breast cancer

MTRTRAALLLFTALATSLGFNLDTEELTAFRVDSAGFGDSVVQYANSWVVVGAPQKITAANQTGGLYQC
GYSTGACEPIGLQVPPEAVNMSLGLSLASTTSPSQLLACGPTVHHECGRNMYLTGLCFLLGPTQLTQRL
PVSRQECPRQEQDIVFLIDGSGSISSRNFATMMNFVRAVISQFQRPSTQFSLMQFSNKFQTHFTFEEFR
RTSNPLSLLASVHQLQGFTYTATAIQNVVHRLFHASYGARRDATKILIVITDGKKEGDSLDYKDVIPMA
DAAGIIRYAIGVGLAFQNRNSWKELNDIASKPSQEHIFKVEDFDALKDIQNQLKEKIFAIEGTETTSSS
SFELEMAQEGFSAVFTPDGPVLGAVGSFTWSGGAFLYPPNMSPTFINMSQENVDMRDSYLGYSTELALW
KGVQSLVLGAPRYQHTGKAVIFTQVSRQWRMKAEVTGTQIGSYFGASLCSVDVDTDGSTDLVLIGAPHY
YEQTRGGQVSVCPLPRGWRRWWCDAVLYGEQGHPWGR**FGAALTVLGDVNGDK**LTDVVIGAPGEEENRGA
VYLFHGVLGPSISPSHSQRIAGSQLSSRLQYFGQALSGGQDLTQDGLVDLAVGARGQVLLLRTRPVLWV
GVSMQFIPAEIPRSAFECREQVVSEQTLVQSNICLYIDKRSKNLLGSRDLQSSVTLDLALDPGRLSPRA
`TFQETKNRSLSRVRVLGLKAHCENFNLLLPSCVEDSVTPITLRLNFTLVGKPLLAFRNLRPMLAADAQR
YFTASLPFEKNCGADHICQDNLGISFSFPGLKSLLVGSNLELNAEVMVWNDGEDSYGTTITFSHPAGLS
YRYVAEGQKQGQLRSLHLTCDSAPVGSQGTWSTSCRINHLIFRGGAQITFLATFDVSPKAVLGDRLLLT
ANVSSENNTPRTSKTTFQLELPVKYAVYTVVSSHEQFTKYLNFSESEEKESHVAMHRYQVNNLGQRDLP
VSINFWVPVELNQEAVWMDVEVSHPQNPSLRCSSEKIAPPASDFLAHIQKNPVLDCSIAGCLRFRCDVP
SFSVQEELDFTLKGNLSFGWVRQILQKKVSVVSVAEITFDTSVYSQLPGQEAFMRAQTTTVLEKYKVHN
PTPLIVGSSIGGLLLLALITAVLYKVGFFKRQYKEMMEEANGQIAPENGTQTPSPPSEK

**Mass Match Peptides (bold):**
FGAALTVLGDVNGDK [252]

**Tandem Peptides (underline):**


## Peptide Source: 1D-GE, Chronic lymphocytic leukaemia

MTRTRAALLLFTALATSLGFNLDTEELTAFRVDSAGFGDSVVQYANSWVVVGAPQKITAANQTGGLYQC
GYSTGACEPIGLQVPPEAVNMSLGLSLASTTSPSQLLACGPTVHHECGR<u>NMYLTGLCFLLGPTQLTQRL</u>
PVSRQECPR<u>QEQDIVFLIDGSGSISSRNFATMMNFVR</u>AVISQFQRPSTQFSLMQFSNK**FQTHFTFEEFR**
<u>R</u>TSNPLSLLASVHQLQGFTYTATAIQNVVHR<u>LFHASYGARR</u>DATKILIVITDGK<u>KEGDSLDYKDVIPMA</u>
<u>DAAGIIRYAIGVGLAFQNR</u>NSWK<u>ELNDIASKPSQEHIFKVEDFDALKDIQNQLK</u>EKIFAIEGTETTSSS
SFELEMAQEGFSAVFTPDGPVLGAVGSFTWSGGAFLYPPNMSPTFINMSQENVDMR<u>DSYLGYSTELALW</u>
<u>KGVQSLVLGAPRYQHTGKAVIFTQVSR</u>QWRMKAEVTGTQIGSYFGASLCSVDVDTDGSTDLVLIGAPHY
YEQTR<u>GGQVSVCPLPR</u>GWRRWWCDAVLYGEQGHPWGR<u>FGAALTVLGDVNGDKLTDVVIGAPGEEENRGA</u>
<u>VYLFHGVLGPSISPSHSQRIAGSQLSSR</u>LQYFGQALSGGQDLTQDGLVDLAVGARGQVLLLRTRPVLWV
GVSMQFIPAEIPRSAFECREQVVSEQTLVQSNICLYIDKRSKNLLGSR<u>DLQSSVTLDLALDPGR</u>LSPRA
TFQETKNRSLSRVRVLGLKAHCENFNLLLPSCVEDSVTPITLRLNFTLVGKPLLAFR<u>NLRPMLAADAQR</u>
YFTASLPFEKNCGADHICQDNLGISFSFPGLKSLLVGSNLELNAEVMVWNDGEDSYGTTITFSHPAGLS
YRYVAEGQKQGQLR<u>SLHLTCDSAPVGSQGTWSTSCRINHLIFRGGAQITFLATFDVSPK</u>AVLGDRLLLT
ANVSSENNTPRTSKTTFQLELPVK<u>YAVYTVVSSHEQFTK</u>YLNFSESEEK<u>ESHVAMHRYQVNNLGQR</u>DLP

VSINFWVPVELNQEAVWMDVEVSHPQNPSLRCSSEKIAPPASDFLAHIQKNPVLDCSIAGCLRFRCDVP
SFSVQEELDFTLKGNLSFGWVRQILQKKVSVVSVAEITFDTSVYSQLPGQEAFMRAQTTTVLEKYKVHN
PTPLIVGSSIGGLLLLALITAVLYKVGFFKRQYKEMMEEANGQIAPENGTQTPSPPSEK

## Mass Match Peptides (bold):

AVIFTQVSR [96]
CDVPSFSVQEELDFTLK [100]
DIQNQLK [130]
DLQSSVTLDLALDPGR [138]
DSYLGYSTELALWK [158]
DVIPMADAAGIIR [161]
ELNDIASKPSQEHIFK [204]
EMMEEANGQIAPENGTQTPSPPSEK [210]
ESHVAMHR [224]
FGAALTVLGDVNGDK [252]
FQTHFTFEEFR [269]
FQTHFTFEEFRR [270]
GAVYLFHGVLGPSISPSHSQR [287]
GGQVSVCPLPR [299]
GNLSFGWVR [303]
GVQSLVLGAPR [327]
IAGSQLSSR [350]
IAPPASDFLAHIQK [352]
INHLIFR [383]
KEGDSLDYK [409]
LFHASYGAR [458]
LFHASYGARR [459]
LTDVVIGAPGEEENR [496]
NFATMMNFVR [535]
NLRPMLAADAQR [545]
NMYLTGLCFLLGPTQLTQR [548]
NPVLDCSIAGCLR [552]
QEQDIVFLIDGSGSISSR [567]
SLHLTCDSAPVGSQGTWSTSCR [641]
VEDFDALK [752]
YAIGVGLAFQNR [839]
YAVYTVVSSHEQFTK [841]
YQHTGK [867]
YQVNNLGQR [868]

## Tandem Peptides (underline):

AVIFTQVSR [96]
AVISQFQRPSTQFSLMQFSNK [97]
DVIPMADAAGIIR [161]
FQTHFTFEEFR [269]
GGAQITFLATFDVSPK [296]
GVQSLVLGAPR [327]
INHLIFR [383]
LTDVVIGAPGEEENR [496]
NFATMMNFVR [535]
NMYLTGLCFLLGPTQLTQR [548]
YAVYTVVSSHEQFTK [841]
YQVNNLGQR [868]

**Peptide Source: iTRAQ, Colorectal cancer**

630

MTRTRAALLLFTALATSLGFNLDTEELTAFRVDSAGFGDSVVQYANSWVVVGAPQKITAANQTGGLYQC
GYSTGACEPIGLQVPPEAVNMSLGLSLASTTSPSQLLACGPTVHHECGRNMYLTGLCFLLGPTQLTQRL
PVSRQECPRQEQDIVFLIDGSGSISSRNFATMMNFVRAVISQFQRPSTQFSLMQFSNKFQTHFTFEEFR
RTSNPLSLLASVHQLQGFTYTATAIQNVVHRLFHASYGARRDATKILIVITDGKKEGDSLDYKDVIPMA
DAAGIIRYAIGVGLAFQNRNSWKELNDIASKPSQEHIFKVEDFDALKDIQNQLKEKIFAIEGTETTSSS
SFELEMAQEGFSAVFTPDGPVLGAVGSFTWSGGAFLYPPNMSPTFINMSQENVDMRDSYLGYSTELALW
KGVQSLVLGAPRYQHTGKAVIFTQVSRQWRMKAEVTGTQIGSYFGASLCSVDVDTGSTDLVLIGAPHY
YEQTR**GGQVSVCPLPR**GWRRWWCDAVLYGEQGHPWGRFGAALTVLGDVNGDKLTDVVIGAPGEEENRGA
VYLFHGVLGPSISPSHSQRIAGSQLSSRLQYFGQALSGGQDLTQDGLVDLAVGARGQVLLLRTRPVLWV
GVSMQFIPAEIPRSAFECREQVVSEQTLVQSNICLYIDKRSKNLLGSRDLQSSVTLDLALDPGRLSPRA
TFQETK**NRSLSRVR**VLGLKAHCENFNLLLPSCVEDSVTPITLRLNFTLVGKPLLAFRNLRPMLAADAQR
YFTASLPFEKNCGADHICQDNLGISFSFPGLKSLLVGSNLELNAEVMVWNDGEDSYGTTITFSHPAGLS
YRYVAEGQKQGQLRSLHLTCDSAPVGSQGTWSTSCRINHLIFRGGAQITFLATFDVSPKAVLGDRLLLT
ANVSSENNTPR**TSKTTFQLELPVK**YAVYTVVSSHEQFTKYLNFSESEEKESHVAMHRYQVNNLGQRDLP
VSINFWVPVELNQEAVWMDVEVSHPQNPSLRCSSEKIAPPASDFLAHIQKNPVLDCSIAGCLRFRCDVP
SFSVQEELDFTLKGNLSFGWVRQILQKKVSVVSVAEITFDTSVYSQLPGQEAFMRAQTTTVLEKYKVHN
PTPLIVGSSIGGLLLLALITAVLYKVGFFKRQYKEMMEEANGQIAPENGTQTPSPPSEK

## Mass Match Peptides (bold):

     GGQVSVCPLPR [299]

     NRSLSRVR [961]

     TSKTTFQLELPVK [985]

## Tandem Peptides (underline):

     GGQVSVCPLPR [299]

     NRSLSRVR [961]

     TSKTTFQLELPVK [985]


## Peptide Source: iTRAQ, Non-small cell lung cancer

MTRTRAALLLFTALATSLGFNLDTEELTAFRVDSAGFGDSVVQYANSWVVVGAPQKITAANQTGGLYQC
GYSTGACEPIGLQVPPEAVNMSLGLSLASTTSPSQLLACGPTVHHECGRNMYLTGLCFLLGPTQLTQRL
PVSRQECPRQEQDIVFLIDGSGSISSRNFATMMNFVRAVISQFQRPSTQFSLMQFSNKFQTHFTFEEFR
RTSNPLSLLASVHQLQGFTYTATAIQNVVHRLFHASYGARRDATKILIVITDGKKEGDSLDYKDVIPMA
DAAGIIRYAIGVGLAFQNRNSWKELNDIASKPSQEHIFKVEDFDALKDIQNQLKEKIFAIEGTETTSSS
SFELEMAQEGFSAVFTPDGPVLGAVGSFTWSGGAFLYPPNMSPTFINMSQENVDMRDSYLGYSTELALW
KGVQSLVLGAPRYQHTGKAVIFTQVSRQWRMKAEVTGTQIGSYFGASLCSVDVDTGSTDLVLIGAPHY
YEQTR**GGQVSVCPLPR**GWRRWWCDAVLYGEQGHPWGRFGAALTVLGDVNGDK**LTDVVIGAPGEEENR**GA
VYLFHGVLGPSISPSHSQRIAGSQLSSRLQYFGQALSGGQDLTQDGLVDLAVGARGQVLLLRTRPVLWV
GVSMQFIPAEIPRSAFECREQVVSEQTLVQSNICLYIDKRSKNLLGSRDLQSSVTLDLALDPGRLSPRA
TFQETKNRSLSRVRVLGLKAHCENFNLLLPSCVEDSVTPITLRLNFTLVGKPLLAFRNLRPMLAADAQR
**YFTASLPFEK**NCGADHICQDNLGISFSFPGLKSLLVGSNLELNAEVMVWNDGEDSYGTTITFSHPAGLS
YRYVAEGQKQGQLRSLHLTCDSAPVGSQGTWSTSCRINHLIFRGGAQITFLATFDVSPKAVLGDRLLLT
ANVSSENNTPR**TSKTTFQLELPVK**YAVYTVVSSHEQFTKYLNFSESEEKESHVAMHRYQVNNLGQRDLP
VSINFWVPVELNQEAVWMDVEVSHPQNPSLRCSSEKIAPPASDFLAHIQKNPVLDCSIAGCLRFRCDVP
SFSVQEELDFTLKGNLSFGWVRQILQKKVSVVSVAEITFDTSVYSQLPGQEAFMRAQTTTVLEKYKVHN
PTPLIVGSSIGGLLLLALITAVLYKVGFFKRQYKEMMEEANGQIAPENGTQTPSPPSEK

## Mass Match Peptides (bold):

     GGQVSVCPLPR [299]

     LTDVVIGAPGEEENR [496]

     TSKTTFQLELPVK [985]

     YFTASLPFEK [1004]

## Tandem Peptides (underline):

     GGQVSVCPLPR [299]

LTDVVIGAPGEEENR [496]
ΤSKTTFQLELPVK [985]
YFTASLPFEK [1004]


## Peptide Source: iTRAQ, Renal cell cancer

MTRTRAALLLFTALATSLGFNLDTEELTAFRVDSAGFGDSVVQYANSWVVVGAPQKITAANQTGGLYQC
GYSTGACEPIGLQVPPEAVNMSLGLSLASTTSPSQLLACGPTVHHECGRNMYLTGLCFLLGPTQLΤQRL
PVSRQECPRQEQDIVFLIDGSGSISSRNFATMMNFVRAVISQFQRPSTQFSLMQFSNKFQTHFΤFEEFR
RTSNPLSLLASVHQLQGFTYTATAIQNVVHRLFHASYGARRDATKILIVITDGKKEGDSLDYKDVΤPMA
DAAGIIRYAIGVGLAFQNRNSWKELNDIASKPSQEHIFKVEDFDALKDIQNQLKEKIFAIEGTETΤSSS
SFELEMAQEGFSAVFTPDGPVLGAVGSFTWSGGAFLYPPNMSPTFINMSQENVDMRDSYLGYSTELALW
KGVQSLVLGAPRYQΙTGKAVIFTQVSRQWRMKAEVTGTQIGSYFGASLCSVDVDTDGSTDLVLIGAPΙIY
YEQTRGGQVSVCPLPRGWRRWWCDAVLYGEQGHPWGRFGAALΤVLGDVNGDKLTDVVIGAPGEEENRGA
VYLFHGVLGPSISPSHSQRIAGSQLSSRLQYFGQALSGGQDLΤQDGLVDLAVGARGQVLLLRTRPVLWV
GVSMQFIPAEIPRSAFECREQVVSEQTLVQSNICLYIDKRSKNLLGSRDLQSSVTLDLALDPGRLSPRA
TFQETKNRSLSRVRVLGLKAHCENFNLLLPSCVEDSVTPITLRLNFTLVGKPLLAFRNLRPMLAADAQR
YFTASLPFEKNCGADHICQDNLGISFSFPGLKSLLVGSNLELNAEVMVWNDGEDSYGTTITFSHPAGLS
YRYVAEGQKQGQLRSLHLTCDSAPVGSQGTWSTSCRINHLIFRGGAQITFLATFDVSPK**AVLGDR**LLLT
ANVSSENNTPRTSKTTFQLELPVKYAVYTVVSSHEQFTKYLNFSESEEKESHVAMHRYQVNNLGQRDLP
VSINFWVPVELNQEAVWMDVEVSHPQNPSLRCSSEKIAPPASDFLAHIQKNPVLDCSIAGCLRFRCDVP
SFSVQEELDFTLKGNLSFGWVRQILQKKVSVVSVAEITFDTSVYSQLPGQEAFMRAQTTTVLEKYKVHN
PTPLIVGSSIGGLLLLALITAVLYKVGFFKRQYKEMMEEANGQIAPENGTQTPSPPSEK


**Mass Match Peptides (bold):**
AVLGDR [885]
**Tandem Peptides (underline):**
AVLGDR [885]


## OGTA174 (SEQ ID No: 31)

## Peptide Source: 1D-GE, Acute T-cell leukaemia

MPMGSLQPLATLYLLGMLVASCLGR**LSWYDPDFQAR**LTRSNSK**CQGQLEVYLK**DGWHMVCSQSWGRSSK
QWEDPSQASKVCQRLNCGVPLSLGPFLVTYTPQSSIICYGQLGSFSNCSHSRNDMCHSLGLTCLEPQK**Τ**
**TPPTTRPPPTTTPEPTAPPR**LQLVAQSGGQHCAGVVEFYSGSLGGTISYEAQDKTQDLENFLCNNLQCG
SFLK**HLPETEAGR**AQDPGEPREHQPLPIQWKIQNSSCTSLEHCFRKIKPQKSGR**VLALLCSGFQPK**VQS
RLVGGSSICEGTVEVR**QGAQWAALCDSSSAR**SSLR**WEEVCR**EQQCGSVNSYR**VLDAGDPTSR**GLFCPHQ
K**LSQCHELWERNSYCKK**VFVTCQDPNPAGLAAGTVASIILALVLLVVLLVVCGPLAYKKLVKKFRQKKQ
RQWIGPTGMNQNMSFHRNHTATVR**SHAENPTASHVDNEYSQPPR**NSRLSAYPALEGVLHRSSMQPDNSS
DSDYDLHGAQRL


**Mass Match Peptides (bold):**
CQGQLEVYLK [107]
HLPETEAGR [337]
LSQCHELWER [490]
LSWYDPDFQAR [493]
NSYCKK [554]
QGAQWAALCDSSSAR [573]
SHAENPTASHVDNEYSQPPR [634]
TTPPTTRPPPTTTPEPTAPPR [734]
VLALLCSGFQPK [771]

```
VLDAGDPTSR [775]
WEEVCR [826]
```

**Tandem Peptides (underline):**
```
LSWYDPDFQAR [493]
VLDAGDPTSR [775]
```

## Peptide Source: 1D-GE, Chronic lymphocytic leukaemia

```
MPMGSLQPLATLYLLGMLVASCLGRLSWYDPDFQARLTRSNSKCQGQLEVYLKDGWHMVCSQSWGRSSK
QWEDPSQASKVCQRLNCGVPLSLGPFLVTYTPQSSIICYGQLGSFSNCSHSRNDMCHSLGLTCLEPQKT
TPPTTRPPPTTTPEPTAPPRLQLVAQSGGQHCAGVVEFYSGSLGGTISYEAQDKTQDLENFLCNNLQCG
SFLKHLPETEAGRAQDPGEPREHQPLPIQWKIQNSSCTSLEHCFRKIKPQKSGRVLALLCSGFQPKVQS
RLVGGSSICEGTVEVRQGAQWAALCDSSSARSSLRWEEVCREQQCGSVNSYRVLDAGDPTSRGLFCPHQ
KLSQCHELWERNSYCKKVFVTCQDPNPAGLAAGTVASIILALVLLVVLLVVCGPLAYKKLVKKFRQKKQ
RQWIGPTGMNQNMSFHRNHTATVRSHAENPTASHVDNEYSQPPRNSRLSAYPALEGVLHRSSMQPDNSS
DSDYDLHGAQRL
```

**Mass Match Peptides (bold):**
```
AQDPGEPR [81]
EHQPLPIQWK [194]
HLPETEAGR [337]
IQNSSCTSLEHCFRK [390]
LSQCHELWER [490]
LSWYDPDFQAR [493]
SHAENPTASHVDNEYSQPPR [634]
TQDLENFLCNNLQCGSFLK [723]
VLDAGDPTSR [775]
```
**Tandem Peptides (underline):**
```
LSWYDPDFQAR [493]
VLDAGDPTSR [775]
```

## Peptide Source: iTRAQ, Non-small cell lung cancer

```
MPMGSLQPLATLYLLGMLVASCLGRLSWYDPDFQARLTRSNSKCQGQLEVYLKDGWHMVCSQSWGRSSK
QWEDPSQASKVCQRLNCGVPLSLGPFLVTYTPQSSIICYGQLGSFSNCSHSRNDMCHSLGLTCLEPQKT
TPPTTRPPPTTTPEPTAPPRLQLVAQSGGQHCAGVVEFYSGSLGGTISYEAQDKTQDLENFLCNNLQCG
SFLKHLPETEAGRAQDPGEPREHQPLPIQWKIQNSSCTSLEHCFRKIKPQKSGRVLALLCSGFQPKVQS
RLVGGSSICEGTVEVRQGAQWAALCDSSSARSSLRWEEVCREQQCGSVNSYRVLDAGDPTSRGLFCPHQ
KLSQCHELWERNSYCKKVFVTCQDPNPAGLAAGTVASIILALVLLVVLLVVCGPLAYKKLVKKFRQKKQ
RQWIGPTGMNQNMSFHRNHTATVRSHAENPTASHVDNEYSQPPRNSRLSAYPALEGVLHRSSMQPDNSS
DSDYDLHGAQRL
```

**Mass Match Peptides (bold):**
```
LSQCHELWER [490]
```
**Tandem Peptides (underline):**
```
LSQCHELWER [490]
```

## OGTA176 (SEQ ID No: 32)

## Peptide Source: 1D-GE, B-cell non-Hodgkin's lymphoma

MAEAITYADLRFVKAPLKKSISSRLGQDPGADDDGEITYENVQVPAVLGVPSSLASSVLGDKAAVKSEQ
PTASWRAVTSPAVGRILPCRTTCLRYLLLGLLLTCLLLGVTAICLGVR**YLQVSQQLQQTNR**VLEVTNSS
LRQQLRLK**ITQLGQSAEDLQGSRRELAQSQEALQVEQR**AHQAAEGQLQACQADRQKTK**ETLQSEEQQRR**
ALEQKLSNMENRLKPFFTCGSADTCCPSGWIMHQKSCFYISLTSKNWQESQKQCETLSSKLATFSEIYP
QSHSYYFLNSLLPNGGSGNSYWTGLSSNKDWKLTDDTQRTRTYAQSSKCNKVHKTWSWWTLESESCRSS
LPYICEMTAFRFPD

### Mass Match Peptides (bold):

     ELAQSQEALQVEQR [202]
     ETLQSEEQQR [231]
     ITQLGQSAEDLQGSR [396]
     ITQLGQSAEDLQGSRR [397]
     YLQVSQQLQQTNR [862]

### Tandem Peptides (bold):

     ETLQSEEQQR [231]

## Peptide Source: 1D-GE, Chronic lymphocytic leukaemia

MAEAITYADLRFVKAPLKKSISSRLGQDPGADDDGEITYENVQVPAVLGVPSSLASSVLGDKAAVK**SEQ
PTASWR**AVTSPAVGRILPCRTTCLRYLLLGLLLTCLLLGVTAICLGVR**YLQVSQQLQQTNR**VLEVTNSS
LRQQLRLK**ITQLGQSAEDLQGSRRELAQSQEALQVEQR**AHQAAEGQLQACQADRQKTK**ETLQSEEQQRR**
ALEQKLSNMENRLKPFFTCGSADTCCPSGWIMHQKSCFYISLTSKNWQESQKQCETLSSKLATFSEIYP
QSHSYYFLNSLLPNGGSGNSYWTGLSSNKDWKLTDDTQRTRTYAQSSKCNKVHKTWSWWTLESESCR**SS
LPYICEMTAFR**FPD

### Mass Match Peptides (bold):

     ELAQSQEALQVEQR [202]
     ETLQSEEQQR [231]
     ETLQSEEQQRR [232]
     ITQLGQSAEDLQGSR [396]
     SEQPTASWR [630]
     SSLPYICEMTAFR [650]
     YLQVSQQLQQTNR [862]

### Tandem Peptides (underline):

     ELAQSQEALQVEQR [202]
     ETLQSEEQQR [231]
     ITQLGQSAEDLQGSR [396]
     YLQVSQQLQQTNR [862]

## Peptide Source: iTRAQ, Colorectal cancer

MAEAITYADLRFVKAPLKKSISSRLGQDPGADDDGEITYENVQVPAVLGVPSSLASSVLGDKAAVKSEQ
PTASWR**AVTSPAVGR**ILPCRTTCLRYLLLGLLLTCLLLGVTAICLGVRYLQVSQQLQQTNRVLEVTNSS
LRQQLRLKITQLGQSAEDLQGSRRELAQSQEALQVEQRAHQAAEGQLQACQADRQKTKETLQSEEQQRR
ALEQKLSNMENRLKPFFTCGSADTCCPSGWIMHQKSCFYISLTSKNWQESQKQCETLSSKLATFSEIYP
QSHSYYFLNSLLPNGGSGNSYWTGLSSNKDWKLTDDTQRTRTYAQSSKCNKVHKTWSWWTLESESCRSS
LPYICEMTAFRFPD

### Mass Match Peptides (bold):

     AVTSPAVGR [886]

## Tandem Peptides (underline):
AVTSPAVGR [886]


## Peptide Source: iTRAQ, Non-small cell lung cancer

MAEAITYADLRFVKAPLKKSISSRLGQDPGADDDGEITYENVQVPAVLGVPSSLASSVLGDKAAVKSEQ
PTASWR**AVTSPAVGR**ILPCRTTCLRYLLLGLLLTCLLLGVTAICLGVRYLQVSQQLQQTNRVLEVINSS
LRQQLRLKITQLGQSAEDLQGSRRELAQSQEALQVEQRAHQAAEGQLQACQADRQKTKETLQSEEQQRR
ALEQKLSNMENRLKPFFTCGSADTCCPSGWIMHQKSCFYISLISKNWQESQKQCETLSSKLATFSEIYP
QSHSYYFLNSLLPNGGSGNSYWTGLSSNKDWKLTDDTQRTRTYAQSSKCNKVHKTWSWWTLESESCRSS
LPYICEMTAFRFPD


## Mass Match Peptides (bold):
AVTSPAVGR [886]
## Tandem Peptides (underline):
AVTSPAVGR [886]


## Peptide Source: iTRAQ, Renal cell cancer

MAEAITYADLRFVKAPLKKSISSRLGQDPGADDDGEITYENVQVPAVLGVPSSLASSVLGDKAAVKSEQ
PTASWR**AVTSPAVGR**ILPCRTTCLRYLLLGLLLTCLLLGVTAICLGVRYLQVSQQLQQTNRVLEVINSS
LRQQLRLKITQLGQSAEDLQGSRRELAQSQEALQVEQRAIIQAAEGQLQACQADRQKTKETLQSEEQQRR
ALEQKLSNMENRLKPFFTCGSADTCCPSGWIMHQKSCFYISLISKNWQESQKQCETLSSKLATFSEIYP
QSHSYYFLNSLLPNGGSGNSYWTGLSSNKDWKLTDDTQRTRTYAQSSKCNKVHKTWSWWTLESESCRSS
LPYICEMTAFRFPD


## Mass Match Peptides (bold):
AVTSPAVGR [886]
## Tandem Peptides (underline):
AVTSPAVGR [886]


## Peptide Source: iTRAQ, Small cell lung cancer

MAEAITYADLRFVKAPLKKSISSRLGQDPGADDDGEITYENVQVPAVLGVPSSLASSVLGDKAAVKSEQ
PTASWR**AVTSPAVGR**ILPCRTTCLRYLLLGLLLTCLLLGVTAICLGVRYLQVSQQLQQTNRVLEVINSS
LRQQLRLKITQLGQSAEDLQGSRRELAQSQEALQVEQRAHQAAEGQLQACQADRQKTKETLQSEEQQRR
ALEQKLSNMENRLKPFFTCGSADTCCPSGWIMHQKSCFYISLISKNWQESQKQCETLSSKLATFSEIYP
QSHSYYFLNSLLPNGGSGNSYWTGLSSNKDWKLTDDTQRTRTYAQSSKCNKVHKTWSWWTLESESCRSS
LPYICEMTAFRFPD


## Mass Match Peptides (bold):
AVTSPAVGR [886]
## Tandem Peptides (underline):
AVTSPAVGR [886]


## OGTA177 (SEQ ID No: 33)

## Peptide Source: 1D-GE, Chronic lymphocytic leukaemia

MEDTKESNVKTFCSKNILAILGFSSIIAVIALLAVGLTQNKALPENVK**YGIVLDAGSSHTSLYIYK**WPA
EKENDTGVVHQVEECRVKGPGISKFVQKVNEIGIYLTDCMERAREVIPR<u>SQHQETPVYLGATAGMR</u>LLR
<u>MESEELADR</u>VLDVVER**<u>SLSNYPFDFQGAR</u>**IITGQEEGAYGWITINYLLGKFSQKTRWFSIVPYETNNQE
TFGALDLGGASTQVTFVPQNQTIESPDNALQFRLYGKDYNVYTHSFLCYGKDQALWQKLAK**DIQVASNE**
**ILR**DPCFHPGYKKVVNVSDLYKTPCTKRFEMTLPFQQFEIQGIGNYQQCHQSILELFNTSYCPYSQCAF
NGIFLPPLQGDFGAFSAFYFVMK**FLNLTSEK**VSQEK**VTEMMKK**FCAQPWEEIKTSYAGVKEKYLSEYCF
SGTYILSLLLQGYHFTADSWEHIHFIGKIQGSDAGWTLGYMLNLTNMIPAEQPLSTPLSHSTYVFLMVL
FSLVLFTVAIIGLLIFHKPSYFWKDMV

## Mass Match Peptides (bold):

DIQVASNEILR [131]
FLNLTSEK [259]
SLSNYPFDFQGAR [643]
SQHQETPVYLGATAGMR [647]
VTEMMK [801]
VTEMMKK [802]
YGIVLDAGSSHTSLYIYK [853]

## Tandem Peptides (underline):

MESEELADR [514]
SLSNYPFDFQGAR [643]
SQHQETPVYLGATAGMR [647]

## Peptide Source: iTRAQ, Colorectal cancer

MEDTKESNVKTFCSKNILAILGFSSIIAVIALLAVGLTQNKALPENVKYGIVLDAGSSHTSLYIYKWPA
EKENDTGVVHQVEECRVK<u>GPGISK</u>FVQKVNEIGIYLTDCMER<u>AREVIPRSQHQETPVYLGATAGMRLLR</u>
MESEELADR<u>VLDVVER</u>SLSNYPFDFQGARIITGQEEGAYGWITINYLLGKFSQKTRWFSIVPYETNNQE
TFGALDLGGASTQVTFVPQNQTIESPDNALQFRLYGKDYNVYTHSFLCYGKDQALWQKLAK**DIQVASNE**
**ILR**DPCFHPGYKK**VVNVSDLYK**TPCTKRFEMTLPFQQFEIQGIGNYQQCHQSILELFNTSYCPYSQCAF
NGIFLPPLQGDFGAFSAFYFVMKFLNLTSEKVSQEKVTEMMKKFCAQPWEEIKTSYAGVKEKYLSEYCF
SGTYILSLLLQGYHFTADSWEHIHFIGKIQGSDAGWTLGYMLNLTNMIPAEQPLSTPLSHSTYVFLMVL
FSLVLFTVAIIGLLIFHKPSYFWKDMV

## Mass Match Peptides (bold):

AREVIPR [883]
DIQVASNEILR [131]
GPGISK [915]
SQHQETPVYLGATAGMRLLR [971]
VLDVVER [994]
VVNVSDLYK [1003]

## Tandem Peptides (underline):

AREVIPR [883]
DIQVASNEILR [131]
GPGISK [915]
SQHQETPVYLGATAGMRLLR [971]
VLDVVER [994]
VVNVSDLYK [1003]

## Peptide Source: iTRAQ, Non-small cell lung cancer

MEDTKESNVKTFCSKNILAILGFSSIIAVIALLAVGLTQNKALPENVKYGIVLDAGSSHTSLYIYKWPA
EKENDTGVVHQVEECRVKGPGISKFVQKVNEIGIYLTDCMERAR**EVIPRSQHQETPVYLGATAGMR**LLR
MESEELADRVLDVVERS**LSNYPFDFQGAR**IITGQEEGAYGWITINYLLGKFSQKTRWFSIVPYETNNQE
TFGALDLGGASTQVTFVPQNQTIESPDNALQFRLYGKDYNVYTHSFLCYGKDQALWQKLAK**DIQVASNE**
**ILRDPCFHPGYK**KVVNVSDLYKTPCTKRFEMTLPFQQFEIQGIGNYQQCHQSILELFNTSYCPYSQCAF
NGIFLPPLQGDFGAFSAFYFVMK**FLNLTSEKVSQEK**VTEMMKKFCAQPWEEIKTSYAGVKEKYLSEYCF
SGTYILSLLLQGYHFTADSWEHIHFIGKIQGSDAGWTLGYMLNLTNMIPAEQPLSTPLSHSTYVFLMVL
FSLVLFTVAIIGLLIFHKPSYFWKDMV

**Mass Match Peptides (bold):**
        DIQVASNEILR [131]
        DPCFHPGYK [892]
        EVIPR [909]
        FLNLTSEKVSQEK [911]
        SLSNYPFDFQGAR [643]
        SQHQETPVYLGATAGMR [647]

**Tandem Peptides (underline):**
        DIQVASNEILR [131]
        DPCFHPGYK [892]
        EVIPR [909]
        FLNLTSEKVSQEK [911]
        SLSNYPFDFQGAR [643]
        SQHQETPVYLGATAGMR [647]

## Peptide Source: iTRAQ, Ovarian cancer

MEDTKESNVKTFCSKNILAILGFSSIIAVIALLAVGLTQNKALPENVKYGIVLDAGSSHTSLYIYKWPA
EKENDTGVVHQVEECRVK**GPGISK**FVQKVNEIGIYLTDCMERAREVIPRSQHQETPVYLGATAGMRLLR
MESEELADRVLDVVERSLSNYPFDFQGARIITGQEEGAYGWITINYLLGKFSQKTRWFSIVPYETNNQE
TFGALDLGGASTQVTFVPQNQTIESPDNALQFRLYGKDYNVYTHSFLCYGKDQALWQKLAKDIQVASNE
ILRDPCFHPGYKKVVNVSDLYKTPCTKRFEMTLPFQQFEIQGIGNYQQCHQSILELFNTSYCPYSQCAF
NGIFLPPLQGDFGAFSAFYFVMKFLNLTSEKVSQEKVTEMMKKFCAQPWEEIKTSYAGVKEKYLSEYCF
SGTYILSLLLQGYHFTADSWEHIHFIGKIQGSDAGWTLGYMLNLTNMIPAEQPLSTPLSHSTYVFLMVL
FSLVLFTVAIIGLLIFHKPSYFWKDMV

**Mass Match Peptides (bold):**
        GPGISK [915]

**Tandem Peptides (underline):**
        GPGISK [915]

## Peptide Source: iTRAQ, Renal cell cancer

MEDTKESNVKTFCSKNILAILGFSSIIAVIALLAVGLTQNKALPENVKYGIVLDAGSSHTSLYIYKWPA
EKENDTGVVHQVEECRVK**GPGISK**FVQKVNEIGIYLTDCMERAREVIPRSQHQETPVYLGATAGMRLLR
MESEELADR**VLDVVERSLSNYPFDFQGAR**IITGQEEGAYGWITINYLLGKFSQKTRWFSIVPYETNNQE
TFGALDLGGASTQVTFVPQNQTIESPDNALQFRLYGKDYNVYTHSFLCYGKDQALWQKLAK**DIQVASNE**
**ILR**DPCFHPGYKKVVNVSDLYKTPCTKRFEMTLPFQQFEIQGIGNYQQCHQSILELFNTSYCPYSQCAF
NGIFLPPLQGDFGAFSAFYFVMKFLNLTSEKVSQEKVTEMMKKFCAQPWEEIKTSYAGVKEKYLSEYCF
SGTYILSLLLQGYHFTADSWEHIHFIGKIQGSDAGWTLGYMLNLTNMIPAEQPLSTPLSHSTYVFLMVL
FSLVLFTVAIIGLLIFHKPSYFWKDMV

**Mass Match Peptides (bold):**

```
DIQVASNEILR [131]
GPGISK [915]
SLSNYPFDFQGAR [643]
VLDVVER [994]
```

**Tandem Peptides (underline):**
```
DIQVASNEILR [131]
GPGISK [915]
SLSNYPFDFQGAR [643]
VLDVVER [994]
```

## Peptide Source: iTRAQ, Small cell lung cancer

```
MEDTKESNVKTFCSKNILAILGFSSIIAVIALLAVGLTQNKALPENVKYGIVLDAGSSHTSLYIYKWPA
EKENDTGVVHQVEECRVKGPGISKFVQKVNEIGIYLTDCMERAREVIPRSQHQETPVYLGATAGMRLLR
MESEELADRVLDVVERSLSNYPFDFQGARIITGQEEGAYGWITINYLLGKFSQKTRWFSIVPYETNNQE
TFGALDLGGASTQVTFVPQNQTIESPDNALQFRLYGKDYNVYTHSFLCYGKDQALWQKLAKDIQVASNE
ILRDPCFHPGYKKVVNVSDLYKTPCTKRFEMTLPFQQFEIQGTGNYQQCHQSILELFNTSYCPYSQCAF
NGIFLPPLQGDFGAFSAFYFVMKFLNLTSEKVSQEKVTEMMKKFCAQPWEEIKTSYAGVKEKYLSEYCF
SGTYILSLLLQGYHFTADSWEHIHFIGKIQGSDAGWTLGYMLNLTNMIPAEQPLSTPLSHSTYVFLMVL
FSLVLFTVAIIGLLIFHKPSYFWKDMV
```

**Mass Match Peptides (bold):**
```
AREVIPR [883]
```
**Tandem Peptides (underline):**
```
AREVIPR [883]
```

## OGTA197 (SEQ ID No: 34)

## Peptide Source: 1D-GE, Colorectal cancer

```
MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI
```

**Mass Match Peptides (bold):**
```
MQQYTEHFMAAGYTAIEK [523]
TSVTVSDLEPHMNYTFTVEAR [733]
TYVDPHTYEDPNQAVLK [742]
WTPPQDSGGR [837]
YKPMMIITEYMENGALDK [857]
```

**Tandem Peptides (underline):**
    NGVSGLVTSR [538]
    QSPEDVYFSK [587]
    VIGAGEFGEVYK [769]


## Peptide Source: 1D-GE, Hepatocellular carcinoma

MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYIFCVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQCLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI

**Mass Match Peptides (bold):**
    AINDGFR [73]
    EVVLLDFAAAGGELGWLTHPYGK [239]
    FTTEIHPSCVTR [278]
    IDTIAPDEITVSSDFEAR [359]
    KGDSNSYNVR [417]
    LPGHQKR [478]
    LPSTSGSEGVPFR [481]
    MELQAAR [512]
    MQQYTEHFMAAGYTAIEK [523]
    QSPEDVYFSK [587]
    RKNQR [605]
    TLADFDPR [702]
    TYVDPHTYEDPNQAVLK [742]
    VDFLGEAGIMGQFSHHNIIR [747]
    VIGAGEFGEVYK [769]
    VVQMTNDDIK [813]
    VVQMTNDDIKR [814]
    VYYKK [822]
    WTAPEAISYR [834]
    WTAPEAISYRK [835]
    YKPMMIITEYMENGALDK [857]
    YLANMNYVHR [860]
    YSEPPHGLTR [871]

**Tandem Peptides (underline):**
    LPSTSGSEGVPFR [481]
    QSPEDVYFSK [587]
    VIGAGEFGEVYK [769]
    VSDFGLSR [796]
    YSEPPHGLTR [871]

## Peptide Source: 1D-GE, Lung cancer

MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI

## Mass Match Peptides (bold):

        AINDGFR [73]
        DGEFSVLQLVGMLR [121]
        EVPVAIK [238]
        FADIVSILDK [242]
        FTTEIHPSCVTR [278]
        GDSNSYNVRR [288]
        IDTIAPDEITVSSDFEAR [359]
        KEVPVAIK [414]
        KKGDSNSYNVR [422]
        LPSTSGSEGVPFR [481]
        MQQYTEHFMAAGYTAIEK [523]
        NGVSGLVTSR [538]
        RKNQR [605]
        TASVSINQTEPPK [673]
        TLADFDPR [702]
        TSVTVSDLEPHMNYTFTVEAR [733]
        TVSEWLESIK [738]
        VDFLGEAGIMGQFSHHNIIR [747]
        VIGAGEFGEVYK [769]
        VLEDDPEATYTTSGGK [777]
        VVQMTNDDIKR [814]
        VYYKK [822]
        WTAPEAISYRK [835]
        WTPPQDSGGR [837]
        YEVTYR [845]
        YKPMMIITEYMENGALDK [857]
        YLANMNYVHR [860]

## Tandem Peptides (underline):

        DQVNTVGIPI [154]
        LPSTSGSEGVPFR [481]
        QSPEDVYFSK [587]
        VIGAGEFGEVYK [769]
        VLEDDPEATYTTSGGK [777]
        VVQMTNDDIK [813]
        WTPPQDSGGR [837]
        YLANMNYVHR [860]

YSEPPHGLTR [871]

## Peptide Source: 1D-GE, Melanoma

MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQCLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI

### Mass Match Peptides (bold):
     APQDPASMPCTRPPSAPHYLTAVGMGAK [80]
     DGEFSVLQLVGMLR [121]
     EVPVAIK [238]
     MELQAAR [512]
     STTSLSVSWSIPPPQQSR [660]
     SVGPLTR [662]
     TASVSINQTEPPK [673]
     TSVTVSDLEPHMNYTFTVEAR [733]
     YKPMMIITEYMENGALDK [857]
     YLANMNYVHR [860]

### Tandem Peptides (underline):
     FADIVSILDK [242]
     VIGAGEFGEVYK [769]

## Peptide Source: 1D-GE, Osteosarcoma

MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQCLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI

**Mass Match Peptides (bold):**

ACFALLWGCALAAAAAAQGK [55]
AINDGFR [73]
APQDPASMPCTRPPSAPHYLTAVGMGAK [80]
DGEFSVLQLVGMLR [121]
FTTEIHPSCVTR [278]
IDTIAPDEITVSSDFEAR [359]
KGDSNSYNVR [417]
LPTPMDCPSAIYQLMMQCWQQER [483]
MQQYTEHFMAAGYTAIEK [523]
RKNQR [605]
STTSLSVSWSIPPPQQSR [660]
SVGPLTR [662]
TASVSINQTEPPK [673]
TLADFDPR [702]
TNWVYR [715]
TSVTVSDLEPHMNYTFTVEAR [733]
TYVDPHTYEDPNQAVLK [742]
VDFLGEAGIMGQFSHHNIIR [747]
VVQMTNDDIK [813]
VVQMTNDDIKR [814]
WTPPQDSGGR [837]
YEVTYRK [846]
YKPMMIITEYMENGALDK [857]
YLANMNYVHR [860]
YSEPPHGLTR [871]

**Tandem Peptides (underline):**

LPSTSGSEGVPFR [481]
NGVSGLVTSR [538]
TLADFDPR [702]
VIGAGEFGEVYK [769]
VLEDDPEATYTTSGGK [777]

## Peptide Source: 1D-GE, Ovarian cancer

MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI

**Mass Match Peptides (bold):**

ACFALLWGCALAAAAAAQGK [55]
AINDGFR [73]

```
DGEFSVLQLVGMLR [121]
FTTEIHPSCVTR [278]
IDTIAPDEITVSSDFEAR [359]
KGDSNSYNVRR [418]
RKNQR [605]
STTSLSVSWSIPPPQQSR [660]
SVGPLTR [662]
TASVSINQTEPPK [673]
TLADFDPR [702]
TNWVYR [715]
TSVTVSDLEPHMNYTFTVEAR [733]
TYVDPHTYEDPNQAVLK [742]
VDFLGEAGIMGQFSHHNIIR [747]
VIGAGEFGEVYK [769]
VVQMTNDDIKR [814]
WTPPQDSGGR [837]
YEVTYR [845]
YKPMMIITEYMENGALDK [857]
YLANMNYVHR [860]
YSEPPHGLTR [871]
```

**Tandem Peptides (underline):**
```
FADIVSILDK [242]
LPSTSGSEGVPFR [481]
QSPEDVYFSK [587]
TASVSINQTEPPK [673]
VIGAGEFGEVYK [769]
VSDFGLSR [796]
WTPPQDSGGR [837]
```

## Peptide Source: 1D-GE, Pancreatic cancer

```
MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYIFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI
```

**Mass Match Peptides (bold):**
```
ACFALLWGCALAAAAAAQGK [55]
AINDGFR [73]
IDTIAPDEITVSSDFEAR [359]
KGDSNSYNVR [417]
KGDSNSYNVRR [418]
LEGVISK [454]
LNVEER [474]
```

```
LPSTSGSEGVPFR [481]
MELQAAR [512]
MQQYTEHFMAAGYTAIEK [523]
QSPEDVYFSK [587]
SEQLKPLK [629]
STTSLSVSWSIPPPQQSR [660]
SVGPLTR [662]
SVGPLTRK [663]
TLADFDPR [702]
TNWVYR [715]
TVSEWLESIK [738]
TYVDPHTYEDPNQAVLK [742]
VIGAGEFGEVYK [769]
VLEDDPEATYTTSGGK [777]
VVQMTNDDIK [813]
VVQMTNDDIKR [814]
VYYKK [822]
WTAPEAISYR [834]
WTPPQDSGGR [837]
YEVTYR [845]
YEVTYRK [846]
YEVTYRKK [847]
YKPMMIITEYMENGALDK [857]
YLANMNYVHR [860]
YSEPPHGLTR [871]
```

**Tandem Peptides (underline):**
```
FADIVSILDK [242]
LPSTSGSEGVPFR [481]
QSPEDVYFSK [587]
TASVSINQTEPPK [673]
TLADFDPR [702]
VIGAGEFGEVYK [769]
WTAPEAISYR [834]
WTPPQDSGGR [837]
YLANMNYVHR [860]
YSEPPHGLTR [871]
```

## Peptide Source: 1D-GE, Prostate cancer

```
MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI
```

**Mass Match Peptides (bold):**

LPSTSGSEGVPFR [481]
MELQAAR [512]
NGVSGLVTSR [538]
VVQMTNDDIK [813]
WTPPQDSGGR [837]
YEVTYR [845]
YSEPPHGLTR [871]

**Tandem Peptides (underline):**

LPSTSGSEGVPFR [481]

## Peptide Source: 1D-GE, Renal cell cancer

MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYIFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI

**Mass Match Peptides (bold):**

ACFALLWGCALAAAAAAQGK [55]
AINDGFR [73]
KGDSNSYNVRR [418]
LPSTSGSEGVPFR [481]
SVGPLTR [662]
TNWVYR [715]
TYVDPHTYEDPNQAVLK [742]
VIGAGEFGEVYK [769]
VVQMTNDDIK [813]
VVQMTNDDIKR [814]
WTAPEAISYR [834]
WTPPQDSGGR [837]
YEVTYR [845]
YEVTYRK [846]
YLANMNYVHR [860]
YSEPPHGLTR [871]

**Tandem Peptides (underline):**

LPSTSGSEGVPFR [481]
VIGAGEFGEVYK [769]
WTAPEAISYR [834]
YSEPPHGLTR [871]

## Peptide Source: iTRAQ, Colorectal cancer

```
MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYIFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI
```

**Mass Match Peptides (bold):**
```
LNVEERSVGPLTR [942]
NGVSGLVTSR [538]
TASVSINQTEPPK [673]
```

**Tandem Peptides (underline):**
```
LNVEERSVGPLTR [942]
NGVSGLVTSR [538]
TASVSINQTEPPK [673]
```

## Peptide Source: iTRAQ, Hepatocellular cancer

```
MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYIFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI
```

**Mass Match Peptides (bold):**
```
TASVSINQTEPPK [673]
```

**Tandem Peptides (underline):**
```
TASVSINQTEPPK [673]
```

## Peptide Source: iTRAQ, Non-small cell lung cancer

MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI

**Mass Match Peptides (bold):**

    DGEFSVLQLVGMLR [121]
    LNVEERSVGPLTR [942]
    NGVSGLVTSR [538]
    TASVSINQTEPPK [673]

**Tandem Peptides (underline):**

    DGEFSVLQLVGMLR [121]
    LNVEERSVGPLTR [942]
    NGVSGLVTSR [538]
    TASVSINQTEPPK [673]

## Peptide Source: iTRAQ, Renal cell cancer

MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEHFMAAGYTAIEKVVQMTNDDIKRIGVRLPGHQKRIAYSLLGLK
DQVNTVGIPI

**Mass Match Peptides (bold):**

    LPGHQKR [478]
    NGVSGLVTSR [538]
    TASVSINQTEPPK [673]

**Tandem Peptides (underline):**

    LPGHQKR [478]
    NGVSGLVTSR [538]
    TASVSINQTEPPK [673]

**Peptide Source: iTRAQ, Small cell lung cancer**

```
MELQAARACFALLWGCALAAAAAAQGKEVVLLDFAAAGGELGWLTHPYGKGWDLMQNIMNDMPIYMYSV
CNVMSGDQDNWLRTNWVYRGEAERNNFELNFTVRDCNSFPGGASSCKETFNLYYAESDLDYGTNFQKRL
FTKIDTIAPDEITVSSDFEARHVKLNVEERSVGPLTRKGFYLAFQDIGACVALLSVRVYYKKCPELLQG
LAHFPETIAGSDAPSLATVAGTCVDHAVVPPGGEEPRMHCAVDGEWLVPIGQCLCQAGYEKVEDACQAC
SPGFFKFEASESPCLECPEHTLPSPEGATSCECEEGFFRAPQDPASMPCTRPPSAPHYLTAVGMGAKVE
LRWTPPQDSGGREDIVYSVTCEQCWPESGECGPCEASVRYSEPPHGLTRTSVTVSDLEPHMNYTFTVEA
RNGVSGLVTSRSFRTASVSINQTEPPKVRLEGRSTTSLSVSWSIPPPQQSRVWKYEVTYRKKGDSNSYN
VRRTEGFSVTLDDLAPDTTYLVQVQALTQEGQGAGSKVHEFQTLSPEGSGNLAVIGGVAVGVVLLLVLA
GVGFFIHRRRKNQRARQSPEDVYFSKSEQLKPLKTYVDPHTYEDPNQAVLKFTTEIHPSCVTRQKVIGA
GEFGEVYKGMLKTSSGKKEVPVAIKTLKAGYTEKQRVDFLGEAGIMGQFSHHNIIRLEGVISKYKPMMI
ITEYMENGALDKFLREKDGEFSVLQLVGMLRGIAAGMKYLANMNYVHRDLAARNILVNSNLVCKVSDFG
LSRVLEDDPEATYTTSGGKIPIRWTAPEAISYRKFTSASDVWSFGIVMWEVMTYGERPYWELSNHEVMK
AINDGFRLPTPMDCPSAIYQLMMQCWQQERARRPKFADIVSILDKLIRAPDSLKTLADFDPRVSIRLPS
TSGSEGVPFRTVSEWLESIKMQQYTEIIFMAAGYTAIEKVVQMTNDDIKRIGVRLPGIIQKRIAYSLLGLK
DQVNTVGIPI
```

**Mass Match Peptides (bold):**
  NGVSGLVTSR [538]
  SVGPLTRK [663]
  TASVSINQTEPPK [673]

**Tandem Peptides (underline):**
  NGVSGLVTSR [538]
  SVGPLTRK [663]
  TASVSINQTEPPK [673]

## OGTA202 (SEQ ID No: 35)

**Peptide Source: 1D-GE, Colorectal cancer**

```
MGLPRGPLASLLLLQVCWLQCAASEPCRAVFREAEVTLEAGGAEQEPGQALGKVFMGCPGQEPALFSTD
NDDFTVRNGETVQERRSLKERNPLKIFPSKRILRRHKRDWVVAPISVPENGKGPFPQRLNQLKSNKDRD
TKIFYSITGPGADSPPEGVFAVEKETGWLLLNKPLDREEIAKYELFGHAVSENGASVEDPMNISITVTD
QNDHKPKFTQDTFRGSVLEGVLPGTSVMQVTATDEDDAIYTYNGVVAYSIHSQEPKDPHDLMFTIHRST
GTISVISSGLDREKVPEYTLTIQATDMDGDGSTTTAVAVVEILDANDNAPMFDPQKYEAHVPENAVGHE
VQRLTVTDLDAPNSPAWRATYLIMGGDDGDHFTITTHPESNQGILTTRKGLDFEAKNQHTLYVEVTNEA
PFVLKLPTSTATIVVHVEDVNEAPVFVPPSKVVEVQEGIPTGEPVCVYTAEDPDKENQKISYRILRDPA
GWLAMDPDSGQVTAVGTLDREDEQFVRNNIYEVMVLAMDNGSPPTTGTGTLLLTLIDVNDHGPVPEPRQ
ITICNQSPVRHVLNITDKDLSPHTSPFQAQLTDDSDIYWTAEVNEEGDTVVLSLKKFLKQDTYDVHLSL
SDHGNKEQLTVIRATVCDCHGHVETCPGPWKGGFILPVLGAVLALLFLLLVLLLLVRKKKRKIKEPLLLP
EDDTRDNVFYYGEEGGGEEDQDYDITQLHRGLEARPEVVLRNDVAPTIIPTPMYRPRPANPDEIGNFII
ENLKAANTDPTAPPYDTLLVFDYEGSGSDAASLSSLTSSASDQDQDYDYLNEWGSRFKKLADMYGGGED
D
```

**Mass Match Peptides (bold):**
**Tandem Peptides (underline):**
  FTQDTFR [277]

**Peptide Source: iTRAQ, Colorectal cancer**

MGLPRGPLASLLLLQVCWLQCAASEPCRAVFREAEVTLEAGGAEQEPGQALGKVFMGCPGQEPALFSTD
NDDFTVRNGETVQERRSLKERNPLKIFPSKRILRRHKRDWVVAPISVPENGKGPFPQRLNQLKSNKDRD
TKIFYSITGPGADSPPEGVFAVEKETGWLLLNKPLDREEIAKYELFGHAVSENGASVEDPMNISI￢VTD
QNDHKPKFTQDTFRGSVLEGVLPGTSVMQVTATDEDDAIYTYNGVVAYSIHSQEPKDPHDLMFTIHR**ST**
**GTISVISSGLDREK**VPEYTLTIQATDMDGDGSTTTAVAVVEILDANDNAPMFDPQKYEAHVPENAVGHE
VQRLTVTDLDAPNSPAWRATYLIMGGDDGDHFTITTHPESNQGILTTRKGLDFEAKNQHTLYVEV￢NEA
PFVLKLPTSTATIVVHVEDVNEAPVFVPPSKVVEVQEGIPTGEPVCVYTAEDPDKENQK**ISYRILR**DPA
GWLAMDPDSGQVTAVGTLDREDEQFVRNNIYEVMVLAMDNGSPPTTGTGTLLLTLIDVNDHGPVPEPRQ
ITICNQSPVRHVLNITDKDLSPHTSPFQAQLTDDSDIYWTAEVNEEGDTVVLSLKKFLKQDTYDVHLSL
SDHGNK**EQLTVIR**ATVCDCHGHVETCPGPWKGGFILPVLGAVLALLFLLLVLLLLVRKKRKIKEPLLLP
EDDTRDNVFYYGEEGGGEEDQDYDITQLHRGLEARPEVVLRNDVAPTIIPTPMYRPRPANPDEIGNFII
ENLKAANTDPTAPPYDTLLVFDYEGSGSDAASLSSLTSSASDQDQDYDYLNEWGSRFKKLADMYGGGED
D

**Mass Match Peptides (bold):**
      EQLTVIR [904]
      ￢SYRILR [925]
      STGTISVISSGLDREK [975]

**Tandem Peptides (underline):**
      EQLTVIR [904]
      ￢SYRILR [925]
      STGTISVISSGLDREK [975]

## Peptide Source: iTRAQ, Non-small cell lung cancer

MGLPRGPLASLLLLQVCWLQCAASEPCRAVFREAEVTLEAGGAEQEPGQALGKVFMGCPGQEPALFSTD
NDDFTVRNGETVQERRSLKERNPLKIFPSKRILRRHKRDWVVAPISVPENGKGPFPQRLNQLKSNKDRD
TKIFYSITGPGADSPPEGVFAVEKETGWLLLNKPLDREEIAKYELFGHAVSENGASVEDPMNISI￢VTD
QNDHKPKFTQDTFRGSVLEGVLPGTSVMQVTATDEDDAIYTYNGVVAYSIHSQEPKDPHDLMFTIHRST
GTISVISSGLDREKVPEYTLTIQATDMDGDGSTTTAVAVVEILDANDNAPMFDPQKYEAHVPENAVGHE
VQRLTVTDLDAPNSPAWRATYLIMGGDDGDHFTITTHPESNQGILTTRKGLDFEAKNQHTLYVEV￢NEA
PFVLKLPTSTATIVVHVEDVNEAPVFVPPSKVVEVQEGIPTGEPVCVYTAEDPDKENQKISYRILRDPA
GWLAMDPDSGQVTAVGTLDREDEQFVRNNIYEVMVLAMDNGSPPTTGTGTLLLTLIDVNDHGPVPEPRQ
ITICNQSPVRHVLNITDKDLSPHTSPFQAQLTDDSDIYWTAEVNEEGDTVVLSLKKFLKQDTYDVHLSL
SDHGNK**EQLTVIR**ATVCDCHGHVETCPGPWKGGFILPVLGAVLALLFLLLVLLLLVRKKRKIKEPLLLP
EDDTRDNVFYYGEEGGGEEDQDYDITQLHRGLEARPEVVLRNDVAPTIIPTPMYRPRPANPDEIGNFII
ENLKAANTDPTAPPYDTLLVFDYEGSGSDAASLSSLTSSASDQDQDYDYLNEWGSRFKKLADMYGGGED
D

**Mass Match Peptides (bold):**
      EQLTVIR [904]

**Tandem Peptides (underline):**
      EQLTVIR [904]

## OGTA203 (SEQ ID No: 36)

## Peptide Source: 1D-GE, Ovarian cancer

MRTYRYFLLLFWVGQPYPTLSTPLSKR**TSGFPAKK**RALELSGNSKNELNRSKRSWMWNQFFLLEEYTGS
DYQYVGKLHSDQDRGDGSLKYILSGDGAGDLFIINENTGDIQATKRLDREEKPVYILRAQAINRR￢GRP
VEPESEFIIKIHDINDNEPIFTKEVYTATVPEMSDVGTFVVQVTATDADDPTYGNSAKVVYSILQGQPY
FSVESETGIIK**TALLNMDR**ENREQYQVVIQAKDMGGQMGGLSGTTTVNITLTDVNDNPPRFPQSTYQFK

TPESSPPGTPIGRIKASDADVGENAEIEYSITDGEGLDMFDVITDQETQEGIITVKKLLDFEKKKVYTL
KVEASNPYVEPRFLYLGPFKDSATVRIVVEDVDEPPVFSKLAYILQIREDAQINTTIGSVTAQDPDAAR
NPVKYSVDRHTDMDRIFNIDSGNGSIFTSKLLDRETLLWHNITVIATEINNPKQSSRVPLYIKVLDVND
NAPEFAEFYETFVCEKAKADQLIQTLHAVDKDDPYSGHQFSFSLAPEAASGSNFTIQDNKDNTAGILTR
KNGYNRHEMSTYLLPVVISDNDYPVQSSTGTVTVRVCACDHHGNMQSCHAEALIHPTGLSTGALVAILL
CIVILLVTVVLFAALRRQRKKEPLIISKEDIRDNIVSYNDEGGGEEDTQAFDIGTLRNPEAIEDNKLRR
DIVPEALFLPRRTPTARDNTDVRDFINQRLKENDTDPTAPPYDSLATYAYEGTGSVADSLSSLESVTTD
ADQDYDYLSDWGPRFKKLADMYGGVDSDKDS

### Mass Match Peptides (bold):

DNIVSYNDEGGGEEDTQAFDIGTLR [144]
EDAQINTTIGSVTAQDPDAAR [168]
RTPTAR [618]
TALLNMDR [669]
TPESSPPGTPIGR [716]
TSGFPAKK [727]
VEASNPYVEPR [751]

### Tandem Peptides (underline):

VEASNPYVEPR [751]

## Peptide Source: 1D-GE, Renal cell cancer

MRTYRYFLLLFWVGQPYPTLSTPLSKRTSGFPAKKRALELSGNSKNELNRSKRSWMWNQFFLLEEYTGS
DYQYVGKLHSDQDRGDGSLKYILSGDGAGDLFIINENTGDIQATKRLDREEKPVYILRAQAINRRTGRP
VEPESEFIIKIHDINDNEPIFTKEVYTATVPEMSDVGTFVVQVTATDADDPTYGNSAKVVYSILQGQPY
FSVESETGIIKTALLNMDRENREQYQVVIQAKDMGGQMGGLSGTTTVNITLTDVNDNPPRFPQSTYQFK
TPESSPPGTPIGRIKASDADVGENAEIEYSITDGEGLDMFDVITDQETQEGIITVKKLLDFEKKKVYTL
KVEASNPYVEPRFLYLGPFKDSATVRIVVEDVDEPPVFSKLAYILQIREDAQINTTIGSVTAQDPDAAR
NPVKYSVDRHTDMDRIFNIDSGNGSIFTSKLLDRETLLWHNITVIATEINNPKQSSRVPLYIKVLDVND
NAPEFAEFYETFVCEKAKADQLIQTLHAVDKDDPYSGHQFSFSLAPEAASGSNFTIQDNKDNTAGILTR
KNGYNRHEMSTYLLPVVISDNDYPVQSSTGTVTVRVCACDHHGNMQSCHAEALIHPTGLSTGALVAILL
CIVILLVTVVLFAALRRQRKKEPLIISKEDIRDNIVSYNDEGGGEEDTQAFDIGTLRNPEAIEDNKLRR
DIVPEALFLPRRTPTARDNTDVRDFINQRLKENDTDPTAPPYDSLATYAYEGTGSVADSLSSLESVTTD
ADQDYDYLSDWGPRFKKLADMYGGVDSDKDS

### Mass Match Peptides (bold):

KNGYNR [428]
TSGFPAKK [727]

### Tandem Peptides (underline):

IVVEDVDEPPVFSK [402]

## Peptide Source: iTRAQ, Non-small cell lung cancer

MRTYRYFLLLFWVGQPYPTLSTPLSKRTSGFPAKKRALELSGNSKNELNRSKRSWMWNQFFLLEEYTGS
DYQYVGKLHSDQDRGDGSLKYILSGDGAGDLFIINENTGDIQATKRLDREEKPVYILRAQAINRRTGRP
VEPESEFIIKIHDINDNEPIFTKEVYTATVPEMSDVGTFVVQVTATDADDPTYGNSAKVVYSILQGQPY
FSVESETGIIKTALLNMDRENREQYQVVIQAKDMGGQMGGLSGTTTVNITLTDVNDNPPRFPQSTYQFK
TPESSPPGTPIGRIKASDADVGENAEIEYSITDGEGLDMFDVITDQETQEGIITVKKLLDFEKKKVYTL
KVEASNPYVEPRFLYLGPFKDSATVRIVVEDVDEPPVFSKLAYILQIREDAQINTTIGSVTAQDPDAAR
NPVKYSVDRHTDMDRIFNIDSGNGSIFTSKLLDRETLLWHNITVIATEINNPKQSSRVPLYIKVLDVND
NAPEFAEFYETFVCEKAKADQLIQTLHAVDKDDPYSGHQFSFSLAPEAASGSNFTIQDNKDNTAGILTR
KNGYNRHEMSTYLLPVVISDNDYPVQSSTGTVTVRVCACDHHGNMQSCHAEALIHPTGLSTGALVAILL

```
CIVILLVTVVLFAALRRQRKKEPLIISKEDIRDNIVSYNDEGGGEEDTQAFDIGTLRNPEAIEDNKLRR
DIVPEALFLPRRTPTARDNTDVRDFINQRLKENDTDPTAPPYDSLATYAYEGTGSVADSLSSLESVTTD
ADQDYDYLSDWGPRFKKLADMYGGVDSDKDS
```

**Mass Match Peptides (bold):**
VEASNPYVEPRFLYLGPFK [989]

**Tandem Peptides (underline):**
VEASNPYVEPRFLYLGPFK [989]

## Peptide Source: iTRAQ, Renal cell cancer

```
MRTYRYFLLLFWVGQPYPTLSTPLSKRTSGFPAKKRALELSGNSKNELNRSKRSWMWNQFFLLEEYTGS
DYQYVGKLHSDQDRGDGSLKYILSGDGAGDLFIINENTGDIQATKRLDREEKPVYILRAQAINRRTGRP
VEPESEFIIKIHDINDNEPIFTKEVYTATVPEMSDVGTFVVQVTATDADDPTYGNSAKVVYSILQGQPY
FSVESETGIIKTALLNMDRENREQYQVVIQAKDMGGQMGGLSGTTTVNITLTDVNDNPPRFPQSTYQFK
TPESSPPGTPIGRIKASDADVGENAEIEYSITDGEGLDMFDVITDQETQEGIITVKKLLDFEKKKVYTL
KVEASNPYVEPRFLYLGPFKDSATVRIVVEDVDEPPVFSKLAYILQIREDAQINTTIGSVTAQDPDAAR
NPVKYSVDRHTDMDRIFNIDSGNGSIFTSKLLDRETLLWHNITVIATEINNPKQSSRVPLYIKVLDVND
NAPEFAEFYETFVCEKAKADQLIQTLHAVDKDDPYSGHQFSFSLAPEAASGSNFTIQDNKDNTAGILTR
KNGYNRHEMSTYLLPVVISDNDYPVQSSTGTVTVRVCACDHHGNMQSCHAEALIHPTGLSTGALVAILL
CIVILLVTVVLFAALRRQRKKEPLIISKEDIRDNIVSYNDEGGGEEDTQAFDIGTLRNPEAIEDNKLRR
DIVPEALFLPRRTPTARDNTDVRDFINQRLKENDTDPTAPPYDSLATYAYEGTGSVADSLSSLESVTTD
ADQDYDYLSDWGPRFKKLADMYGGVDSDKDS
```

**Mass Match Peptides (bold):**
LLDFEK [934]
TGRPVEPESEFIIK [981]

**Tandem Peptides (underline):**
LLDFEK [934]
TGRPVEPESEFIIK [981]

## OGTA206 (SEQ ID No: 37)

## Peptide Source: 1D-GE, Breast cancer

```
MASMGLQVMGIALAVLGWLAVMLCCALPMWRVTAFIGSNIVTSQTIWEGLWMNCVVQSTGQMQCKVYDS
LLALPQDLQAARALVIISIIVAALGVLLSVVGGKCTNCLEDESAKAKTMIVAGVVFLLAGLMVIVPVSW
TAHNIIQDFYNPLVASGQKREMGASLYVGWAASGLLLLGGGLLCCNCPPRTDKPYSAKYSAARSAAASN
YV
```

**Mass Match Peptides (bold):**
**Tandem Peptides (bold):**
VYDSLLALPQDLQAAR [817]

## Peptide Source: 1D-GE, Pancreatic cancer

```
MASMGLQVMGIALAVLGWLAVMLCCALPMWRVTAFIGSNIVTSQTIWEGLWMNCVVQSTGQMQCKVYDS
LLALPQDLQAARALVIISIIVAALGVLLSVVGGKCTNCLEDESAKAKTMIVAGVVFLLAGLMVIVPVSW
```

TAHNIIQDFYNPLVASGQKREMGASLYVGWAASGLLLLGGGLLCCNCPPRTDKPYSAKYSAARSAAASN
YV


**Mass Match Peptides (bold):**
      VYDSLLALPQDLQAAR [817]

**Tandem Peptides (underline):**
      VYDSLLALPQDLQAAR [817]


**Peptide Source: 1D-GE, Prostate cancer**

MASMGLQVMGIALAVLGWLAVMLCCALPMWRVTAFIGSNIVTSQTIWEGLWMNCVVQSTGQMQCK**VYDS
LLALPQDLQAAR**ALVIISIIVAALGVLLSVVGGKCTNCLEDESAKAKTMIVAGVVFLLAGLMVIVPVSW
TAHNIIQDFYNPLVASGQKREMGASLYVGWAASGLLLLGGGLLCCNCPPRTDKPYSAKYSAARSAAASN
YV


**Mass Match Peptides (bold):**
      VYDSLLALPQDLQAAR [817]

**Tandem Peptides (underline):**


**Peptide Source: iTRAQ, Colorectal cancer**

MASMGLQVMGIALAVLGWLAVMLCCALPMWRVTAFIGSNIVTSQTIWEGLWMNCVVQSTGQMQCK**VYDS
LLALPQDLQAAR**ALVIISIIVAALGVLLSVVGGKCTNCLEDESAKAKTMIVAGVVFLLAGLMVIVPVSW
TAHNIIQDFYNPLVASGQKREMGASLYVGWAASGLLLLGGGLLCCNCPPRTDKPYSAKYSAAR**SAAASN
YV**


**Mass Match Peptides (bold):**
      SAAASNYV [968]
      VYDSLLALPQDLQAAR [817]

**Tandem Peptides (underline):**
      SAAASNYV [968]
      VYDSLLALPQDLQAAR [817]


**Peptide Source: iTRAQ, Non-small cell lung cancer**

MASMGLQVMGIALAVLGWLAVMLCCALPMWRVTAFIGSNIVTSQTIWEGLWMNCVVQSTGQMQCK**VYDS
LLALPQDLQAAR**ALVIISIIVAALGVLLSVVGGKCTNCLEDESAKAKTMIVAGVVFLLAGLMVIVPVSW
TAHNIIQDFYNPLVASGQKREMGASLYVGWAASGLLLLGGGLLCCNCPPRTDKPYSAKYSAARSAAASN
YV


**Mass Match Peptides (bold):**
      VYDSLLALPQDLQAAR [817]

**Tandem Peptides (underline):**
      VYDSLLALPQDLQAAR [817]


**OGTA213 (SEQ ID No: 38)**

## Peptide Source: 1D-GE, Lung cancer

```
MRPRTKARSPGRALRNPWRGFLPLTLALFVGAGHAQRDPVGRYEPAGGDANRLRRPGGSYPAAAAAKVY
SLFREQDAPVAGLQPVERAQPGWGSPRRPTEAEARRPSRAQQSRRVQPPAQTRRSTPLGQQQPAPRTRA
APALPRLGTPQRSGAAPPTPPRGRLTGRNVCGGQCCPGWTTANSTNHCIKPVCEPPCQNRGSCSRPQLC
VCRSGFRGARCEEVIPDEEFDPQNSRLAPRRWAERSPNLRRSSAAGEGTLARAQPPAPQSPPAPQSPPA
GTLSGLSQTHPSQQHVGLSRTVRLHPTATASSQLSSNALPPGPGLEQRDGTQQAVPLEHPSSPWGLNLT
EKIKKIK**IVFTPTICK**QTCARGHCANSCERGDTTTLYSQGGHGHDPKSGFRIYFCQIPCLNGGRCIGRD
ECWCPANSTGKFCHLPIPQPDREPPGRGSRPRALLEAPLKQSTFTLPLSNQLASVNPSLVKVHIHHPPE
ASVQIHQVAQVRGGVEEALVENSVETRPPPWLPASPGHSLWDSNNIPARSGEPPRPLPPAAPRPRGLLG
RCYLNTVNGQCANPLLELTTQEDCCGSVGAFWGVTLCAPCPPRPASPVIENGQLECPQGYKRLNLTHCQ
DINECLTLGLCKDAECVNTRGSYLCTCRPGLMLDPSRSRCVSDKAISMLQGLCYRSLGPGTCTLPLAQR
ITKQICCCSRVGKAWGSECEKCPLPGTEAFREICPAGHGYTYASSDIRLSMRKAEEEELARPPREQGQR
SSGALPGPAERQPLRVVTDTWLEAGTIPDKGDSQAGQVTTSVTHAPAWVTGNATTPPMPEQGIAEIQEE
QVTPSTDVLVTLSTPGIDRCAAGATNVCGPGTCVNLPDGYRCVCSPGYQLHPSQAYCTDDNECLRDPCK
GKGRCINRVGSYSCFCYPGYTLATSGATQECQDINECEQPGVCSSGQCTNTEGSYHCECDQGYIMVRKG
HCQDINECRHPGTCPDGRCVNSPGSYTCLACEEGYRGQSGSCVDVNECLTPGVCAHGKCTNLEGSFRCS
CEQGYEVTSDEKGCQDVDECASRASCPTGLCLNTEGSFACSACENGYWVNEDGTACEDLDECAFPGVCP
SGVCTNTAGSFSCKDCDGGYRPSPLGDSCEDVDECEDPQSSCLGGECKNTVGSYQCLCPQGFQLANGTV
CEDVNECMGEEHCAPHGECLNSHGSFFCLCAPGFVSAEGGISCQDVDECATTDPCVGGHCVNTEGSFNC
LCETGFQPSPESGECVDIDECEDYGDPVCGTWKCENSPGSYRCVLGCQPGFHMAPNGDCIDIDECANDT
MCGSHGFCDNTDGSFRCLCDQGFEISPSGWDCVDVNECELMLAVCGAALCENVEGSFLCLCASDLEEYD
AQEGHCRPRGAGGQSMSEAPTGDHAPAPTRMDCYSGQKGHAPCSSVLGRNTTQAECCCTQGATWGDACD
LCPSEDSAEFSEICPSGKGYIPVEGAWTFGQTMYTDADECVIFGPGLCPNGRCLNTVPGYVCLCNPGFH
YDASHKKCEDHDECQDLACENGECVNTEGSFHCFCSPPLTLDLSQQRCMNSTSSTEDLPDHDIHMDICW
KKVTNDVCSEPLRGHRTTYTECCCQDGKAWSQQCALCPPRSSEVYAQLCNVARIEAEREAGVHFRPGYE
YGPGPDDLHYSIYGPDGAPFYNYLGPEDTVPEPAFPNTAGHSADRTPILESPLQPSELQPHYVASHPEP
PAGFEGLQAEECGILNGCENGRCVRVREGYTCDCFEGFQLDAAHMACVDVNECDDLNGPAVLCVHGYCE
NTEGSYRCHCSPGYVAEAGPPHCTAKE
```

**Mass Match Peptides (bold):**
**Tandem Peptides (underline):**
```
IVFTPTICK [401]
```

## Peptide Source: iTRAQ, Colorectal cancer

```
MRPRTKARSPGRALRNPWRGFLPLTLALFVGAGHAQRDPVGRYEPAGGDANRLRRPGGSYPAAAAAKVY
SLFREQDAPVAGLQPVERAQPGWGSPRRPTEAEARRPSRAQQSRRVQPPAQTRRSTPLGQQQPAPRTRA
APALPRLGTPQRSGAAPPTPPRGRLTGRNVCGGQCCPGWTTANSTNHCIKPVCEPPCQNRGSCSRPQLC
VCRSGFRGARCEEVIPDEEFDPQNSRLAPRRWAERSPNLR**RSSAAGEGTLAR**AQPPAPQSPPAPQSPPA
GTLSGLSQTHPSQQHVGLSRTVRLHPTATASSQLSSNALPPGPGLEQRDGTQQAVPLEHPSSPWGLNLT
EKIKKIKIVFTPTICKQTCARGHCANSCERGDTTTLYSQGGHGHDPKSGFRIYFCQIPCLNGGRCIGRD
ECWCPANSTGKFCHLPIPQPDREPPGRGSRPRALLEAPLKQSTFTLPLSNQLASVNPSLVKVHIHHPPE
ASVQIHQVAQVRGGVEEALVENSVETRPPPWLPASPGHSLWDSNNIPARSGEPPRPLPPAAPRPRGLLG
RCYLNTVNGQCANPLLELTTQEDCCGSVGAFWGVTLCAPCPPRPASPVIENGQLECPQGYKRLNLTHCQ
DINECLTLGLCKDAECVNTRGSYLCTCRPGLMLDPSRSRCVSDKAISMLQGLCYRSLGPGTCTLPLAQR
ITKQICCCSRVGKAWGSECEKCPLPGTEAFREICPAGHGYTYASSDIRLSMRKAEEEELARPPREQGQR
SSGALPGPAERQPLRVVTDTWLEAGTIPDKGDSQAGQVTTSVTHAPAWVTGNATTPPMPEQGIAEIQEE
QVTPSTDVLVTLSTPGIDRCAAGATNVCGPGTCVNLPDGYRCVCSPGYQLHPSQAYCTDDNECLRDPCK
GKGRCINRVGSYSCFCYPGYTLATSGATQECQDINECEQPGVCSSGQCTNTEGSYHCECDQGYIMVRKG
HCQDINECRHPGTCPDGRCVNSPGSYTCLACEEGYRGQSGSCVDVNECLTPGVCAHGKCTNLEGSFRCS
CEQGYEVTSDEKGCQDVDECASRASCPTGLCLNTEGSFACSACENGYWVNEDGTACEDLDECAFPGVCP
SGVCTNTAGSFSCKDCDGGYRPSPLGDSCEDVDECEDPQSSCLGGECKNTVGSYQCLCPQGFQLANGTV
CEDVNECMGEEHCAPHGECLNSHGSFFCLCAPGFVSAEGGISCQDVDECATTDPCVGGHCVNTEGSFNC
LCETGFQPSPESGECVDIDECEDYGDPVCGTWKCENSPGSYRCVLGCQPGFHMAPNGDCIDIDECANDT
```

MCGSHGFCDNTDGSFRCLCDQGFEISPSGWDCVDVNECELMLAVCGAALCENVEGSFLCLCASDLEEYD
AQEGHCRPRGAGGQSMSEAPTGDHAPAPTRMDCYSGQKGHAPCSSVLGRNTTQAECCCTQGATWGDACD
LCPSEDSAEFSEICPSGKGYIPVEGAWTFGQTMYTDADECVIFGPGLCPNGRCLNTVPGYVCLCNPGFH
YDASHKKCEDHDECQDLACENGECVNTEGSFHCFCSPPLTLDLSQQRCMNSTSSTEDLPDHDIHMDICW
KKVTNDVCSEPLRGHRTTYTECCCQDGKAWSQQCALCPPRSSEVYAQLCNVARIEAEREAGVHFRPGYE
YGPGPDDLHYSIYGPDGAPFYNYLGPEDTVPEPAFPNTAGHSADRTPILESPLQPSELQPHYVASHPEP
PAGFEGLQAEECGILNGCENGRCVRVREGYTCDCFEGFQLDAAHMACVDVNECDDLNGPAVLCVHGYCE
NTEGSYRCHCSPGYVAEAGPPHCTAKE

**Mass Match Peptides (bold):**
　　　　RSSAAGEGTLAR [967]

**Tandem Peptides (underline):**
　　　　RSSAAGEGTLAR [967]

## Peptide Source: iTRAQ, Non-small cell lung cancer

MRPRTKARSPGRALRNPWRGFLPLTLALFVGAGHAQRDPVGRYEPAGGDANRLRRPGGSYPAAAAAKVY
SLFREQDAPVAGLQPVERAQPGWGSPRRPTEAEARRPSRAQQSRRVQPPAQTRRSTPLGQQQPAPRTRA
APALPRLGTPQRSGAAPPTPPRGRLTGRNVCGGQCCPGWTTANSTNHCIKPVCEPPCQNRGSCSRPQLC
VCRSGFRGARCEEVIPDEEFDPQNSRLAPRRWAERSPNLR**RSSAAGEGTLAR**AQPPAPQSPPAPQSPPA
GTLSGLSQTHPSQQHVGLSRTVRLHPTATASSQLSSNALPPGPGLEQRDGTQQAVPLEHPSSPWGLNLT
EKIKKIKIVFTPTICKQTCARGHCANSCERGDTTTLYSQGGHGHDPKSGFRIYFCQIPCLNGGRCIGRD
ECWCPANSTGKFCHLPIPQPDREPPGRGSRPRALLEAPLKQSFFTLPLSNQLASVNPSLVKVHIHHPPE
ASVQIHQVAQVRGGVEEALVENSVETRPPPWLPASPGHSLWDSNNIPARSGEPPRPLPPAAPRPRGLLG
RCYLNTVNGQCANPLLELTTQEDCCGSVGAFWGVTLCAPCPPRPASPVIENGQLECPQGYKRLNLTHCQ
DINECLTLGLCKDAECVNTRGSYLCTCRPGLMLDPSRSRCVSDKAISMLQGLCYRSLGPGTCTLPLAQR
ITKQICCCSRVGKAWGSECEKCPLPGTEAFREICPAGHGYTYASSDIRLSMRKAEEEELARPPREQGQR
SSGALPGPAERQPLRVVTDTWLEAGTIPDKGDSQAGQVTTSVTHAPAWVTGNATTPPMPEQGIAEIQEE
QVTPSTDVLVTLSTPGIDRCAAGATNVCGPGTCVNLPDGYRCVCSPGYQLHPSQAYCTDDNECLRDPCK
GKGRCINRVGSYSCFCYPGYTLATSGATQECQDINECEQPGVCSGGQCTNTEGSYHCECDQGYIMVRKG
HCQDINECRHPGTCPDGRCVNSPGSYTCLACEEGYRGQSGSCVDVNECLTPGVCAHGKCTNLEGSFRCS
CEQGYEVTSDEKGCQDVDECASRASCPTGLCLNTEGSFACSACENGYWVNEDGTACEDLDECAFPGVCP
SGVCTNTAGSFSCKDCDGGYRPSPLGDSCEDVDECEDPQSSCLGGECKNTVGSYQCLCPQGFQLANGTV
CEDVNECMGEEHCAPHGECLNSHGSFFCLCAPGFVSAEGGTSCQDVDECATTDPCVGGHCVNTEGSFNC
LCETGFQPSPESGECVDIDECEDYGDPVCGTWKCENSPGSYRCVLGCQPGFHMAPNGDCIDIDECANDT
MCGSHGFCDNTDGSFRCLCDQGFEISPSGWDCVDVNECELMLAVCGAALCENVEGSFLCLCASDLEEYD
AQEGHCRPRGAGGQSMSEAPTGDHAPAPTRMDCYSGQKGHAPCSSVLGRNTTQAECCCTQGATWGDACD
LCPSEDSAEFSEICPSGKGYIPVEGAWTFGQTMYTDADECVIFGPGLCPNGRCLNTVPGYVCLCNPGFH
YDASHKKCEDHDECQDLACENGECVNTEGSFHCFCSPPLTLDLSQQRCMNSTSSTEDLPDHDIHMDICW
KKVTNDVCSEPLRGHRTTYTECCCQDGKAWSQQCALCPPRSSEVYAQLCNVARIEAEREAGVHFRPGYE
YGPGPDDLHYSIYGPDGAPFYNYLGPEDTVPEPAFPNTAGHSADRTPILESPLQPSELQPHYVASHPEP
PAGFEGLQAEECGILNGCENGRCVRVREGYTCDCFEGFQLDAAHMACVDVNECDDLNGPAVLCVHGYCE
NTEGSYRCHCSPGYVAEAGPPHCTAKE

**Mass Match Peptides (bold):**
　　　　RSSAAGEGTLAR [967]

**Tandem Peptides (underline):**
　　　　RSSAAGEGTLAR [967]

## OGTA214 (SEQ ID No: 39)

## Peptide Source: 1D-GE, Ovarian cancer

MKAIIHLTLLALLSVNTATNQGNSADAVTTTETATSGPTVAAADTTETNFPETASTTANTPSFPTATSP
APPIISTHSSSTIPTPAPPIISTHSSSTIPIPTAADSESTTNVNSLATSDIITASSPNDGLITMVPSET
QSNNEMSPTTEDNQSSGPPTGTALLETSTLNSTGPSNPCQDDPCADNSLCVKLHNTSFCLCLEGYYYNS
STCKKGKVFPGKISVTVSETFDPEEKHSMAYQDLHSEITSLFKDVFGTSVYGQTVILTVSTSLSPRSEM
RADDKFVNVTIVTILAETTSDNEKTVTEKINKAIRSSSSNFLNYDLTLRCDYYGCNQTADDCLNGLACD
CKSDLQRPNPQSPFCVASSLKCPDACNAQHKQCLIKKSGGAPECACVPGYQEDANGNCQKCAFGYSGLD
CKDKFQLILTIVGTIAGIVILSMIIALIVTARSNNKTKHIEEENLIDEDFQNLKLRSTGFTNLGAEGSV
FPKVRITASRDSQMQNPYSRHSSMPRPDY

**Mass Match Peptides (bold):**
>        QCLIKK [565]
>        SSSSNFLNYDLTLR [651]

**Tandem Peptides (underline):**
>        ISVTVSETFDPEEK [394]
>        SSSSNFLNYDLTLR [651]


## Peptide Source: iTRAQ, Colorectal cancer

MKAIIHLTLLALLSVNTATNQGNSADAVTTTETATSGPTVAAADTTETNFPETASTTANTPSFPTATSP
APPIISTHSSSTIPTPAPPIISTHSSSTIPIPTAADSESTTNVNSLATSDIITASSPNDGLITMVPSET
QSNNEMSPTTEDNQSSGPPTGTALLETSTLNSTGPSNPCQDDPCADNSLCVKLHNTSFCLCLEGYYYNS
STCKKGKVFPGKISVTVSETFDPEEKHSMAYQDLHSEITSLFKDVFGTSVYGQTVILTVSTSLSPRSEM
RADDKFVNVTIVTILAETTSDNEKTVTEKINKAIRSSSSNFLNYDLTLRCDYYGCNQTADDCLNGLACD
CKSDLQRPNPQSPFCVASSLKCPDACNAQHKQCLIKKSGGAPECACVPGYQEDANGNCQKCAFGYSGLD
CKDKFQLILTIVGTIAGIVILSMIIALIVTARSNNKTKHIEEENLIDEDFQNLKLRSTGFTNLGAEGSV
FPKVRITASRDSQMQNPYSRHSSMPRPDY

**Mass Match Peptides (bold):**
>        DSQMQNPYSR [895]
>        HIEEENLIDEDFQNLK [917]
>        SDLQRPNPQSPFCVASSLK [969]
>        STGFTNLGAEGSVFPK [974]
>        VFPGK [990]

**Tandem Peptides (underline):**
>        DSQMQNPYSR [895]
>        HIEEENLIDEDFQNLK [917]
>        SDLQRPNPQSPFCVASSLK [969]
>        STGFTNLGAEGSVFPK [974]
>        VFPGK [990]


## OGTA216 (SEQ ID No: 40)

## Peptide Source: 1D-GE, Breast cancer

MEPRPTAPSSGAPGLAGVGETPSAAALAAARVELPGTAVPSVPEDAAPASRDGGGVRDEGPAAAGDGLG
RPLGPTPSQSRFQVDLVSENAGRAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGGSGHHQHYYYDTHTNT
YYLRTFGHNTMDAVPRIDHYRHTAAQLGEKLLRPSLAELHDELEKEPFEDGFANGEESTPTRDAVVTYT
AESKGVVKFGWIKGVLVRCMLNIWGVMLFIRLSWIVGQAGIGLSVLVIMMATVVTTITGLSTSAIATNG
FVRGGGAYYLISRSLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPKGFFGYKSEIFNENFCPDFRE

EP 2 447 719 B1

EETFFSVFAIFFPAATGILAGANISGDLADPQSAIPKGTLLAILITTLVYVGIAVSVGSCVVRDATGNV
NDTIVTELTNCTSAACKLNFDFSSCESSPCSYGLMNNFQVMSMVSGFTPLISAGIFSATLSSALASLVS
APKIFQALCKDNIYPAFQMFAKGYGKNNEPLRGYILTFLIALGFILIAELNVIAPIISNFFLASYALIN
FSVFHASLAKSPGWRPAFKYYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTK**NVGLMICGHVHMGPR**
**R**QAMKEMSIDQAKYQRWLIKNKMK**AFYAPVHADDLREGAQYLMQAAGLGR**MKPNTLVLGFK**KDWLQADM**
**R**DVDMYINLFHDAFDIQYGVVVIRLKEGLDISHLQGQEELLSSQEKSPGTK**DVVVSVEYSK**KSDLDTSK
PLSEKPITHKVEEEDGKTATQPLLKKESKGPIVPLNVADQKLLEASTQFQKKQGKNTIDVWWLFDDGGL
TLLIPYLLTTKKKWKDCKIRVFIGGKINR**IDHDRR**AMATLLSKFRIDFSDIMVLGDINTKPKKENIIAF
EEIIEPYRLHEDDKEQDIADKMKEDEPWRITDNELELYKTKTYRQIRLNELLKEHSSTANIIVMSLPVA
RKGAVSSALYMAWLEALSKDLPPILLVRGNHQSVLTFYS

## Mass Match Peptides (bold):

AFYAPVHADDLR [69]
DVVVSVEYSK [163]
EGAQYLMQAAGLGR [186]
FQVDLVSENAGR [271]
IDHDRR [356]
KDWLQADMR [408]
NVGLMICGHVHMGPR [555]
NVGLMICGHVHMGPRR [556]

## Tandem Peptides (underline):

DEGPAAAGDGLGRPLGPTPSQSR [114]
GPIVPLNVADQK [307]
SEIFNENFGPDFR [626]

## Peptide Source: 1D-GE, Colorectal cancer

MEPRPTAPSSGAPGLAGVGETPSAAALAAAR**VELPGTAVPSVPEDAAPASR**DGGGVRDEGPAAAGDGLG
RPLGPTPSQSR**FQVDLVSENAGR**AAAAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGGSGHHQHYYYDTHTNT
YYLR**TFGHNTMDAVPRIDHYR**HTAAQLGEKLLRPSLAELHDELEK**EPFEDGFANGEESTPTRDAVVTYT**
**AESK**GVVKFGWIKGVLVR**CMLNIWGVMLFIR**LSWIVGQAGIGLSVLVIMMATVVTTITGLSTSAIATNG
FVR**GGGAYYLISR**SLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPK**GFFGYKSEIFNENFGPDFR**E
EETFFSVFAIFFPAATGILAGANISGDLADPQSAIPKGTLLAILITTLVYVGIAVSVGSCVVRDATGNV
NDTIVTELTNCTSAACKLNFDFSSCESSPCSYGLMNNFQVMSMVSGFTPLISAGIFSATLSSALASLVS
APKIFQALCK**DNIYPAFQMFAK**GYGK**NNEPLR**GYILTFLIALGFILIAELNVIAPIISNFFLASYALIN
FSVFHASLAK**SPGWRPAFK**YYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTK**NVGLMICGHVHMGPR**
**R**QAMK**EMSIDQAK**YQRWLIKNKMK**AFYAPVHADDLREGAQYLMQAAGLGR**MKPNTLVLGFK**KDWLQADM**
**R**DVDMYINLFHDAFDIQYGVVVIRLKEGLDISHLQGQEELLSSQEKSPGTK**DVVVSVEYSKKSDLDTSK**
**PLSEKPITHK**VEEEDGKTATQPLLKKESKGPIVPLNVADQKLLEASTQFQKKQGKNTIDVWWLFDDGGL
TLLIPYLLTTKKKWKDCKIRVFIGGKINR**IDHDRRAMATLLSK**FR**IDFSDIMVLGDINTKPKKENIIAF**
**EEIIEPYRLHEDDK**EQDIADKMKEDEPWRITDNELELYKTKTYRQIR**LNELLKEHSSTANIIVMSLPVA**
**R**KGAVSSALYMAWLEALSK**DLPPILLVR**GNHQSVLTFYS

## Mass Match Peptides (bold):

AFYAPVHADDLR [69]
AMATLLSK [78]
CMLNIWGVMLFIR [106]
DAVVTYTAESK [110]
DLPPILLVR [136]
DNIYPAFQMFAK [145]
DWLQADMR [164]
EGAQYLMQAAGLGR [186]

656

```
EHSSTANIIVMSLPVAR [195]
EMSIDQAK [213]
EPFEDGFANGEESTPTR [216]
FQVDLVSENAGR [271]
GFFGYK [294]
GGGAYYLISR [298]
IDFSDIMVLGDINTKPK [354]
IDFSDIMVLGDINTKPKK [355]
IDHDRR [356]
IDHYR [357]
KDWLQADMR [408]
KENIIAFEEIIEPYR [410]
KSDLDTSKPLSEKPITHK [435]
LHEDDK [465]
LNELLK [471]
NNEPLR [549]
NVGLMICGHVHMGPR [555]
NVGLMICGHVHMGPRR [556]
RAMATLLSK [596]
SEIFNENFGPDFR [626]
SPGWRPAFK [645]
TFGHNTMDAVPR [688]
VELPGTAVPSVPEDAAPASR [754]
```

## Tandem Peptides (underline):

```
AAAAAAAAAAAAAAAAGAGAGAK [49]
DAVVTYTAESK [110]
DEGPAAAGDGLGRPLGPTPSQSR [114]
DLPPILLVR [136]
DVVVSVEYSK [163]
DWLQADMR [164]
EGAQYLMQAAGLGR [186]
EHSSTANIIVMSLPVAR [195]
FQVDLVSENAGR [271]
GPIVPLNVADQK [307]
ITDNELELYK [395]
SEIFNENFGPDFR [626]
TFGHNTMDAVPR [688]
```

## Peptide Source: 1D-GE, Pancreatic cancer

```
MEPRPTAPSSGAPGLAGVGETPSAAALAAARVELPGTAVPSVPEDAAPASRDGGGVRDEGPAAAGDGLG
RPLGPTPSQSRFQVDLVSENAGRAAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGGSGHHQHYYYDTHTNT
YYLRTFGHNTMDAVPRIDHYRHTAAQLGEKLLRPSLAELHDELEKEPFEDGFANGEESTPTRDAVVTYT
AESKGVVKFGWIKGVLVRCMLNIWGVMLFIRLSWIVGQAGIGLSVLVIMMATVVTTITGLSTSAIATNG
FVRGGGAYYLISRSLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPKGFFGYKSEIFNENFGPDFRE
EETFFSVFAIFFPAATGILAGANISGDLADPQSAIPKGTLLAILITTLVYVGIAVSVGSCVVRDAIGNV
NDTIVTELTNCTSAACKLNFDFSSCESSPCSYGLMNNFQVMSMVSGFTPLISAGIFSATLSSALASLVS
APKIFQALCKDNIYPAFQMFAKGYGKNNEPLRGYILTFLIALGFILIAELNVIAPIISNFFLASYALIN
FSVFHASLAKSPGWRPAFKYYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTKNVGLMICGHVHMGPR
RQAMKEMSIDQAKYQRWLIKNKMKAFYAPVHADDLREGAQYLMQAAGLGRMKPNTLVLGFKKDWLQADM
RDVDMYINLFHDAFDIQYGVVVIRLKEGLDISHLQGGQEELLSSQEKSPGTKDVVVSVEYSKKSDLDTSK
PLSEKPITHKVEEEDGKTATQPLLKKESKGPIVPLNVADQKLLEASTQFQKKQGKNTIDVWWLFDDGGL
TLLIPYLLTTKKKWKDCKIRVFIGGKINRIDHDRRAMATLLSKFRIDFSDIMVLGDINTKPKKENIIAF
```

EEIIEPYRLHEDDKEQDIADKMKEDEPWRITDNELELYKTKTYRQIRLNELLKEHSSTANIIVMSLPVA
RKGAVSSALYMAWLEALSKDLPPILLVRGNHQSVLTFYS

**Mass Match Peptides (bold):**

AFYAPVHADDLR [69]
DVVVSVEYSK [163]
EPFEDGFANGEESTPTR [216]
GGGAYYLISR [298]
NNEPLR [549]
NVGLMICGHVHMGPR [555]
VELPGTAVPSVPEDAAPASR [754]

**Tandem Peptides (underline):**

GPIVPLNVADQK [307]
SEIFNENFGPDFR [626]


## Peptide Source: iTRAQ, Colorectal cancer

MEPRPTAPSSGAPGLAGVGETPSAAALAAARVELPGTAVPSVPEDAAPASRDGGGVRDEGPAAAGDGLG
RPLGPTPSQSRFQVDLVSENAGRAAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGGSGHHQHYYYDTHTNT
YYLRTFGHNTMDAVPRIDHYRHTAAQLGEKLLRPSLAELHDELEKEPFEDGFANGEESTPTRDAVVTYT
AESKGVVKFGWIKGVLVRCMLNIWGVMLFIRLSWIVGQAGIGLSVLVIMMATVVTTITGLSTSAIATNG
FVRGGGAYYLISRSLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPKGFFGYKSEIFNENFGPDFRE
EETFFSVFAIFFPAATGILAGANISGDLADPQSAIPKGTLLAILITTLVYVGIAVSVGSCVVRDAIGNV
NDTIVTELTNCTSAACKLNFDFSSCESSPCSYGLMNNFQVMSMVSGFTPLISAGIFSATLSSALASLVS
APKIFQALCKDNIYPAFQMFAKGYGKNNEPLRGYILTFLIALGFILIAELNVIAPIISNFFLASYALIN
FSVFHASLAKSPGWRPAFKYYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTKNVGLMICGHVHMGPR
RQAMKEMSIDQAKYQRWLIKNKMKAFYAPVHADDLREGAQYLMQAAGLGRMKPNTLVLGFKKDWLQADM
RDVDMYINLFHDAFDIQYGVVVIRLKEGLDISHLQGQEELLSSQEKSPGTKDVVVSVEYSKKSDLDTSK
PLSEKPITHKVEEEDGKTATQPLLKKESKGPIVPLNVADQKLLEASTQFQKKQGKNTIDVWWLFDDGGL
TLLIPYLLTTKKKWKDCKIRVFIGGKINRIDHDRRAMATLLSKFRIDFSDIMVLGDINTKPKKENIIAF
EEIIEPYRLHEDDKEQDIADKMKEDEPWRITDNELELYKTKTYRQIRLNELLKEHSSTANIIVMSLPVA
RKGAVSSALYMAWLEALSKDLPPILLVRGNHQSVLTFYS

**Mass Match Peptides (bold):**

AAAAAAAAAAAAAAAGAGAGAK [49]
AFYAPVHADDLR [69]
AMATLLSK [78]
DLPPILLVR [136]
EGAQYLMQAAGLGR [186]
GGGAYYLISR [298]
GPIVPLNVADQK [307]
ITDNELELYK [395]
QTPADGEASGESEPAK [1008]

**Tandem Peptides (underline):**

AAAAAAAAAAAAAAAGAGAGAK [49]
AFYAPVHADDLR [69]
AMATLLSK [78]
DLPPILLVR [136]
EGAQYLMQAAGLGR [186]
GGGAYYLISR [298]
GPIVPLNVADQK [307]

ITDNELELYK [395]
QTPADGEASGESEPAK [1008]


## Peptide Source: iTRAQ, Hepatocellular cancer

MEPRPTAPSSGAPGLAGVGETPSAAALAAARVELPGTAVPSVPEDAAPASRDGGGVRDEGPAAAGDGLG
RPLGPTPSQSRFQVDLVSENAGRAAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGSGHHQHYYYDTHTNT
YYLRTFGHNTMDAVPRIDHYRHTAAQLGEKLLRPSLAELHDELEKEPFEDGFANGEESTPTRDAVVTYT
AESKGVVKFGWIKGVLVRCMLNIWGVMLFIRLSWIVGQAGIGLSVLVIMMATVVTTITGLSTSAIATNG
FVRGGGAYYLISRSLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPKGFFGYKSEIFNENFGPDFRE
EETFFSVFAIFFPAATGILAGANISGDLADPQSAIPKGTLLAILITTLVYVGIAVSVGSCVVRDAIGNV
NDTIVTELTNCTSAACKLNFDFSSCESSPCSYGLMNNFQVMSMVSGFTPLISAGIFSATLSSALASLVS
APKI**FQALCK**DNIYPAFQMFAKGYGKNNEPLRGYILTFLIALGFILIAELNVIAPIISNFFLASYALIN
FSVFHASLAK**SPGWRPAFK**YYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTKNVGLMICGHVHMGPR
RQAMKEMSIDQAKYQRWLIKNKMKAFYAPVHADDLREGAQYLMQAAGLGRMKPNTLVLGFKKDWLQADM
RDVDMYINLFHDAFDIQYGVVVIRLKEGLDISHLQGGQEELLSSQEKSPGTKDVVVSVEYSKKSDLDTSK
PLSEKPITHKVEEEDGKTATQPLLKKESKGPIVPLNVADQKLLEASTQFQKKQGKNTIDVWWLFDDGGL
TLLIPYLLTTKKKWKDCKIRVFIGGKINRIDHDRRAMATLLSKFRIDFSDIMVLGDINTKPKKENIIAF
EEIIEPYRLHEDDKEQDIADKMKEDEPWRITDNELELYKTKTYRQIRLNELLKEHSSTANIIVMSLPVA
RKGAVSSALYMAWLEALSKDLPPILLVRGNHQSVLTFYS

**Mass Match Peptides (bold):**
IFQALCK [920]
SPGWRPAFK [645]
**Tandem Peptides (underline):**
IFQALCK [920]
SPGWRPAFK [645]


## Peptide Source: iTRAQ, Non-small cell lung cancer

MEPRPTAPSSGAPGLAGVGETPSAAALAAARVELPGTAVPSVPEDAAPASRDGGGVRDEGPAAAGDGLG
RPLGPTPSQSRFQVDLVSENAGRAAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGSGHHQHYYYDTHTNT
YYLRTFGHNTMDAVPRIDHYRHTAAQLGEKLLRPSLAELHDELEKEPFEDGFANGEESTPTRDAVVTYT
AESKGVVKFGWIKGVLVRCMLNIWGVMLFIRLSWIVGQAGIGLSVLVIMMATVVTTITGLSTSAIATNG
FVRGGGAYYLISRSLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPKGFFGYKSEIFNENFGPDFRE
EETFFSVFAIFFPAATGILAGANISGDLADPQSAIPKGTLLAILITTLVYVGIAVSVGSCVVRDAIGNV
NDTIVTELTNCTSAACKLNFDFSSCESSPCSYGLMNNFQVMSMVSGFTPLISAGIFSATLSSALASLVS
APKIFQALCKDNIYPAFQMFAKGYGKNNEPLRGYILTFLIALGFILIAELNVIAPIISNFFLASYALIN
FSVFHASLAKSPGWRPAFKYYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTKNVGLMICGHVHMGPR
RQAMK**EMSIDQAK**YQRWLIKNKMKAFYAPVHADDLREGAQYLMQAAGLGRMKPNTLVLGFKKDWLQADM
RDVDMYINLFHDAFDIQYGVVVIRLKEGLDISHLQGGQEELLSSQEKSPGTKDVVVSVEYSKKSDLDTSK
PLSEKPITHKVEEEDGK**TATQPLLK**KESKGPIVPLNVADQKLLEASTQFQKKQGKNTIDVWWLFDDGGL
TLLIPYLLTTKKKWK**DCKIRVFIGGK**INRIDHDRR**AMATLLSK**FRIDFSDIMVLGDINTKPKKENIIAF
EEIIEPYR**LHEDDKEQDIADK**MKEDEPWRITDNELELYKTKTYRQIRLNELLKEHSSTANIIVMSLPVA
RKGAVSSALYMAWLEALSK**DLPPILLVR**GNHQSVLTFYS


**Mass Match Peptides (bold):**

```
AMATLLSK [78]
DCKIRVFIGGK [889]
DLPPILLVR [136]
EMSIDQAK [213]
EQDIADK [903]
LHEDDK [465]
TATQPLLK [978]
```

**Tandem Peptides (underline):**

```
           AMATLLSK [78]
DCKIRVFIGGK [889]
DLPPILLVR [136]
EMSIDQAK [213]
EQDIADK [903]
LHEDDK [465]
TATQPLLK [978]
```

## Peptide Source: iTRAQ, Ovarian cancer

```
MEPRPTAPSSGAPGLAGVGETPSAAALAAARVELPGTAVPSVPEDAAPASRDGGGVRDEGPAAAGDGLG
RPLGPTPSQSRFQVDLVSENAGRAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGSGHHQHYYYDTHTNT
YYLRTFGHNTMDAVPRIDHYRHTAAQLGEKLLRPSLAELHDELEKEPFEDGFANGEESTPTRDAVVTYT
AESKGVVKFGWIKGVLVRCMLNIWGVMLFIRLSWIVGQAGIGLSVLVIMMATVVTTITGLSTSAIATNG
FVRGGGAYYLISRSLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPKGFFGYKSEIFNENFGPDFRE
EETFFSVFAIFFPAATGILAGANISGDLADPQSAIPKGTLLAILITTLVYVGIAVSVGSCVVRDATGNV
NDTIVTELTNCTSAACKLNFDFSSCESSPCSYGLMNNFQVMSMVSGFTPLISAGIFSATLSSALASLVS
APKIFQALCKDNIYPAFQMFAKGYGKNNEPLRGYILTFLIALGFILIAELNVIAPIISNFFLASYALIN
FSVFHASLAKSPGWRPAFKYYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTKNVGLMICGHVHMGPR
RQAMKEMSIDQAKYQRWLIKNKMKAFYAPVHADDLREGAQYLMQAAGLGRMKPNTLVLGFKKDWLQADM
RDVDMYINLFHDAFDIQYGVVVIRLKEGLDISHLQGQEELLSSQEKSPGTKDVVVSVEYSKKSDLDTSK
PLSEKPITHKVEEEDGKTATQPLLKKESKGPIVPLNVADQKLLEASTQFQKKQGKNTIDVWWLFDDGGL
TLLIPYLLTTKKKWKDCKIRVFIGGKINRIDHDRRAMATLLSKFRIDFSDIMVLGDINTKPKKENIIAF
EEIIEPYRLHEDDKEQDIADKMKEDEPWRITDNELELYKTKTYRQIRLNELLKEHSSTANIIVMSLPVA
RKGAVSSALYMAWLEALSKDLPPILLVRGNHQSVLTFYS
```

**Mass Match Peptides (bold):**

```
           AMATLLSK [78]
```

**Tandem Peptides (underline):**

```
           AMATLLSK [78]
```

## Peptide Source: iTRAQ, Renal cell cancer

```
MEPRPTAPSSGAPGLAGVGETPSAAALAAARVELPGTAVPSVPEDAAPASRDGGGVRDEGPAAAGDGLG
RPLGPTPSQSRFQVDLVSENAGRAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGSGHHQHYYYDTHTNT
YYLRTFGHNTMDAVPRIDHYRHTAAQLGEKLLRPSLAELHDELEKEPFEDGFANGEESTPTRDAVVTYT
AESKGVVKFGWIKGVLVRCMLNIWGVMLFIRLSWIVGQAGIGLSVLVIMMATVVTTITGLSTSAIATNG
FVRGGGAYYLISRSLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPKGFFGYKSEIFNENFGPDFRE
EETFFSVFAIFFPAATGILAGANISGDLADPQSAIPKGTLLAILITTLVYVGIAVSVGSCVVRDATGNV
NDTIVTELTNCTSAACKLNFDFSSCESSPCSYGLMNNFQVMSMVSGFTPLISAGIFSATLSSALASLVS
```

APKIFQALCKDNIYPAFQMFAKGYGKNNEPLRGYILTFLIALGFILIAELNVIAPIISNFFLASYALIN
FSVFHASLAKSPGWRPAFKYYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTKNVGLMICGHVHMGPR
RQAMKEMSIDQAKYQRWLIKNKMKAFYAPVHADDLREGAQYLMQAAGLGRMKPNTLVLGFKKDWLQADM
RDVDMYINLFHDAFDIQYGVVVIRLKEGLDISHLQGQEELLSSQEKSPGTKDVVVSVEYSKKSDLDTSK
PLSEKPITHKVEEEDGKTATQPLLKKESKGPIVPLNVADQKLLEASTQFQKKQGKNTIDVWWLFDDGGL
TLLIPYLLTTKKKWKDCKIRVFIGGKINRIDHDRRAMATLLSKFRIDFSDIMVLGDINTKPKKENIIAF
EEIIEPYRLHEDDKEQDIADKMKEDEPWRITDNELELYKTKTYRQIRLNELLKEHSSTANIIVMSLPVA
RKGAVSSALYMAWLEALSKDLPPILLVRGNHQSVLTFYS

**Mass Match Peptides (bold):**

RAMATLLSK [596]
SPGWRPAFK [645]

**Tandem Peptides (underline):**

RAMATLLSK [596]
SPGWRPAFK [645]

## Peptide Source: iTRAQ, Small cell lung cancer

MEPRPTAPSSGAPGLAGVGETPSAAALAAARVELPGTAVPSVPEDAAPASRDGGGVRDEGPAAAGDGLG
RPLGPTPSQSRFQVDLVSENAGRAAAAAAAAAAAAAAAAGAGAGAKQTPADGEASGESEPAKGSEEAKGR
FRVNFVDPAASSSAEDSLSDAAGVGVDGPNVSFQNGGDTVLSEGSSLHSGGGGGSGHHQHYYYDTHTNT
YYLRTFGHNTMDAVPRIDHYRHTAAQLGEKLLRPSLAELHDELEKEPFEDGFANGEESTPTRDAVVTYT
AESKGVVKFGWIKGVLVRCMLNIWGVMLFIRLSWIVGQAGIGLSVLVIMMATVVTTITGLSTSAIATNG
FVRGGGAYYLISRSLGPEFGGAIGLIFAFANAVAVAMYVVGFAETVVELLKEHSILMIDEINDIRIIGA
ITVVILLGISVAGMEWEAKAQIVLLVILLLAIGDFVIGTFIPLESKKPKGFFGYKSEIFNENFGPDFRE
EETFFSVFAIFFPAATGILAGANISGDLADPQSAIPKGTLLAILITTLVYVGIAVSVGSCVVRDAIGNV
NDTIVTELTNCTSAACKLNFDFSSCESSPCSYGLMNNFQVMSMVSGFTPLISAGIFSATLSSALASLVS
APKIFQALCKDNIYPAFQMFAKGYGKNNEPLRGYILTFLIALGFILIAELNVIAPIISNFFLASYALIN
FSVFHASLAKSPGWRPAFKYYNMWISLLGAILCCIVMFVINWWAALLTYVIVLGLYIYVTYKKPDVNWG
SSTQALTYLNALQHSIRLSGVEDHVKNFRPQCLVMTGAPNSRPALLHLVHDFTKNVGLMICGHVHMGPR
RQAMKEMSIDQAKYQRWLIKNKMKAFYAPVHADDLREGAQYLMQAAGLGRMKPNTLVLGFKKDWLQADM
RDVDMYINLFHDAFDIQYGVVVIRLKEGLDISHLQGQEELLSSQEKSPGTKDVVVSVEYSKKSDLDTSK
PLSEKPITHKVEEEDGKTATQPLLKKESKGPIVPLNVADQKLLEASTQFQKKQGKNTIDVWWLFDDGGL
TLLIPYLLTTKKKWKDCKIRVFIGGKINRIDHDRRAMATLLSKFRIDFSDIMVLGDINTKPKKENIIAF
EEIIEPYRLHEDDKEQDIADKMKEDEPWRITDNELELYKTKTYRQIRLNELLKEHSSTANIIVMSLPVA
RKGAVSSALYMAWLEALSKDLPPILLVRGNHQSVLTFYS

**Mass Match Peptides (bold):**

LSGVEDHVK [948]

**Tandem Peptides (underline):**

LSGVEDHVK [948]

## OGTA222 (SEQ ID No: 41)

## Peptide Source: 1D-GE, B-cell non-Hodgkin's lymphoma

MWSGWWLWPLVAVCTADFFRDEAERIMRDSPVIDGHNDLPWQLLDMFNNRLQDERANLTTLAGTHINIP
KLRAGFVGGQFWSVYTPCDTQNKDAVRRTLEQMDVVHRMCRMYPETFLYVTSSAGIRQAFREGKVASLI
GVEGGHSIDSSLGVLRALYQLGMRYLTLTHSCNTPWADNWLVDTGDSEPQSQGLSPFGQRVVKELNRLG
VLIDLAHVSVATMKATLQLSRAPVIFSHSSAYSVCASRRNVPDDVLRLVKQTDSLVMVNFYNNYISCTN

KANLSQVADHLDHIKEVAGAR**AVGFGGDFDGVPR**VPEGLEDVSK**YPDLIAELLR**RNWTEAEVK**GALADN**
**LLR**VFEAVEQASNLTQAPEEEPIPLDQLGGSCRTHYGYSSGASSLHRHWGLLLASLAPLVLCLSLL

### Mass Match Peptides (bold):

ALYQLGMR [77]
ATLQLSR [91]
AVGFGGDFDGVPR [95]
GALADNLLR [283]
MYPETFLYVTSSAGIR [530]
NVPDDVLR [559]
RTLEQMDVVHR [617]
TLEQMDVVHR [703]
YPDLIAELLR [864]

### Tandem Peptides (underline):

AVGFGGDFDGVPR [95]
VPEGLEDVSK [791]


## Peptide Source: 1D-GE, Colorectal cancer

MWSGWWLWPLVAVCTADFFRDEAERIMRDSPVIDGHNDLPWQLLDMFNNRLQDERANLTTLAGTIH̃NIP
KLRAGFVGGQFWSVYTPCDTQNKDAVRR**TLEQMDVVHR**MCRMYPETFLYVTSSAGIRQAFREGKVASLI
GVEGGHSIDSSLGVLR**ALYQLGMR**YLTLTHSCNTPWADNWLVDTGDSEPQSQGLSPFGQRVVKELNRLG
VLIDLAHVSVATMKATLQLSRAPVIFSHSSAYSVCASRRNVPDDVLRLVKQTDSLVMVNFYNNYISCTN
KANLSQVADHLDHIKEVAGAR**AVGFGGDFDGVPR**VPEGLEDVSKYPDLIAELLRRNWTEAEVK**GALADN**
**LLR**VFEAVEQASNLTQAPEEEPIPLDQLGGSCRTHYGYSSGASSLHRHWGLLLASLAPLVLCLSLL

### Mass Match Peptides (bold):

ALYQLGMR [77]
AVGFGGDFDGVPR [95]
GALADNLLR [283]
TLEQMDVVHR [703]

### Tandem Peptides (underline):

AVGFGGDFDGVPR [95]
GALADNLLR [283]
TLEQMDVVHR [703]


## Peptide Source: iTRAQ, Colorectal cancer

MWSGWWLWPLVAVCTADFFRDEAERIMR**DSPVIDGHNDLPWQLLDMFNNR**LQDERANLTTLAGTIH̃NIP
KLR**AGFVGGQFWSVYTPCDTQNK**DAVRR**TLEQMDVVHR**MCRMYPETFLYVTSSAGIRQAFREGK**VASLI**
**GVEGGHSIDSSLGVLRALYQLGMR**YLTLTHSCNTPWADNWLVDTGDSEPQSQGLSPFGQRVVKELNRLG
VLIDLAHVSVATMK**ATLQLSR**APVIFSHSSAYSVCASRR**NVPDDVLR**LVKQTDSLVMVNFYNNYISCTN
KANLSQVADHLDHIKEVAGAR**AVGFGGDFDGVPRVPEGLEDVSKYPDLIAELLR**RNWTEAEVK**GALADN**
**LLR**VFEAVEQASNLTQAPEEEPIPLDQLGGSCRTHYGYSSGASSLHRHWGLLLASLAPLVLCLSLL

### Mass Match Peptides (bold):

AGFVGGQFWSVYTPCDTQNK [881]
ALYQLGMR [77]
ATLQLSR [91]
AVGFGGDFDGVPR [95]
DSPVIDGHNDLPWQLLDMFNNR [894]
GALADNLLR [283]

NVPDDVLR [559]
TLEQMDVVHR [703]
VASLIGVEGGHSIDSSLGVLR [988]
VPEGLEDVSK [791]
YPDLIAELLR [864]

**Tandem Peptides (underline):**
AGFVGGQFWSVYTPCDTQNK [881]
ALYQLGMR [77]
ATLQLSR [91]
AVGFGGDFDGVPR [95]
DSPVIDGHNDLPWQLLDMFNNR [894]
GALADNLLR [283]
NVPDDVLR [559]
TLEQMDVVHR [703]
VASLIGVEGGHSIDSSLGVLR [988]
VPEGLEDVSK [791]
YPDLIAELLR [864]

## Peptide Source: iTRAQ, Non-small cell lung cancer

MWSGWWLWPLVAVCTADFFRDEAERIMRDSPVIDGHNDLPWQLLDMFNNRLQDERANLTTLAGTHⁿNIP
KLRAGFVGGQFWSVYTPCDTQNKDAVRRTLEQMDVVHRMCRMYPETFLYVTSSAGIRQAFREGKVASLI
GVEGGHSIDSSLGVLRALYQLGMRYLTLTHSCNTPWADNWLVDTGDSEPQSQGLSPFGQRVVKELNRLG
VLIDLAHVSVATMKATLQLSRAPVIFSHSSAYSVCASRRNVPDDVLRLVKQTDSLVMVNFYNNYISCTN
KANLSQVADHLDHIKEVAGARAVGFGGDFDGVPRVPEGLEDVSKYPDLIAELLRRNWTEAEVKGALADN
LLRVFEAVEQASNLTQAPEEEPIPLDQLGGSCRTHYGYSSGASSLHRHWGLLLASLAPLVLCLSLL

**Mass Match Peptides (bold):**
ANLSQVADHLDHIK [882]
VPEGLEDVSK [791]

**Tandem Peptides (underline):**
ANLSQVADHLDHIK [882]
VPEGLEDVSK [791]

## Peptide Source: iTRAQ, Renal cell cancer

MWSGWWLWPLVAVCTADFFRDEAERIMRDSPVIDGHNDLPWQLLDMFNNRLQDERANLTTLAGTHⁿNIP
KLRAGFVGGQFWSVYTPCDTQNKDAVRRTLEQMDVVHRMCRMYPETFLYVTSSAGIRQAFREGKVASLI
GVEGGHSIDSSLGVLRALYQLGMRYLTLTHSCNTPWADNWLVDTGDSEPQSQGLSPFGQRVVKELNRLG
VLIDLAHVSVATMKATLQLSRAPVIFSHSSAYSVCASRRNVPDDVLRLVKQTDSLVMVNFYNNYISCTN
KANLSQVADHLDHIKEVAGARAVGFGGDFDGVPRVPEGLEDVSKYPDLIAELLRRNWTEAEVKGALADN
LLRVFEAVEQASNLTQAPEEEPIPLDQLGGSCRTHYGYSSGASSLHRHWGLLLASLAPLVLCLSLL

**Mass Match Peptides (bold):**
ALYQLGMR [77]
AVGFGGDFDGVPR [95]
GALADNLLR [283]
NVPDDVLR [559]
VPEGLEDVSK [791]
YPDLIAELLR [864]

**Tandem Peptides (underline):**
ALYQLGMR [77]

```
AVGFGGDFDGVPR [95]
GALADNLLR [283]
NVPDDVLR [559]
VPEGLEDVSK [791]
YPDLIAELLR [864]
```

## OGTA236 (SEQ ID No: 42)

### Peptide Source: 1D-GE, Pancreatic cancer

```
MSSESKEQHNVSPRDSAEGNDSYPSGIHLELQRESSTDFKQFETNDQCRPYHRILIERQEKSDTNFKEF
VIKKLQKNCQCSPAKAKNMILGFLPVLQWLPKYDLKKNILGDVMSGLIVGILLVPQSIAYSLLAGQEPV
YGLYTSFFASIIYFLLGTSRHISVGIFGVLCLMIGETVDRELQKAGYDNAHSAPSLGMVSNGSTLLNHT
SDRICDKSCYAIMVGSTVTFIAGVYQVAMGFFQVGFVSVYLSDALLSGFVTGASFTILTSQAKYLLGLN
LPRTNGVGSLITTWIHVFRNIHKTNLCDLITSLLCLLVLLPTKELNEHFKSKLKAPIPIELVVVVAATL
ASHFGKLHENYNSSIAGHIPTGFMPPKVPEWNLIPSVAVDAIAISIIGFAITVSLSEMFAKKHGYTVKA
NQEMYAIGFCNIIPSFFHCFTTSAALAKTLVKESTGCHTQLSGVVTALVLLLVLLVIAPLFYSLQKSVL
GVITIVNLRGALRKFRDLPKMWSISRMDTVIWFVTMLSSALLSTEIGLLVGVCFSIFCVILRTQKPKSS
LLGLVEESEVFESVSAYKNLQTKPGIKIFRFVAPLYYINKECFKSALYKQTVNPILIKVAWKKAAKRKI
KEKVVTLGGIQDEMSVQLSHDPLELHTIVIDCSAIQFLDTAGIHTLKEVRRDYEAIGIQVLLAQCNPTV
RDSLTNGEYCKKEEENLLFYSVYEAMAFAEVSKNQKGVCVPNGLSLSSD
```

**Mass Match Peptides (bold):**
**Tandem Peptides (underline):**
```
          SVLGVITIVNLR [664]
```

### Peptide Source: iTRAQ, Colorectal cancer

```
MSSESKEQHNVSPRDSAEGNDSYPSGIHLELQRESSTDFKQFETNDQCRPYHRILIERQEKSDTNFKEF
VIKKLQKNCQCSPAKAKNMILGFLPVLQWLPKYDLKKNILGDVMSGLIVGILLVPQSIAYSLLAGQEPV
YGLYTSFFASIIYFLLGTSRHISVGIFGVLCLMIGETVDRELQKAGYDNAHSAPSLGMVSNGSTLLNHT
SDRICDKSCYAIMVGSTVTFIAGVYQVAMGFFQVGFVSVYLSDALLSGFVTGASFTILTSQAKYLLGLN
LPRTNGVGSLITTWIHVFRNIHKTNLCDLITSLLCLLVLLPTKELNEHFKSKLKAPIPIELVVVVAATL
ASHFGKLHENYNSSIAGHIPTGFMPPKVPEWNLIPSVAVDAIAISIIGFAITVSLSEMFAKKHGYTVKA
NQEMYAIGFCNIIPSFFHCFTTSAALAKTLVKESTGCHTQLSGVVTALVLLLVLLVIAPLFYSLQKSVL
GVITIVNLRGALRKFRDLPKMWSISRMDTVIWFVTMLSSALLSTEIGLLVGVCFSIFCVILRTQKPKSS
LLGLVEESEVFESVSAYKNLQTKPGIKIFRFVAPLYYINKECFKSALYKQTVNPILIKVAWKKAAKRKI
KEKVVTLGGIQDEMSVQLSHDPLELHTIVIDCSAIQFLDTAGIHTLKEVRRDYEAIGIQVLLAQCNPTV
RDSLTNGEYCKKEEENLLFYSVYEAMAFAEVSKNQKGVCVPNGLSLSSD
```

**Mass Match Peptides (bold):**
```
          ELNEHFKSK [900]
```
**Tandem Peptides (underline):**
```
          ELNEHFKSK [900]
```

## OGTA237 (SEQ ID No: 43)

### Peptide Source: 1D-GE, B-cell non-Hodgkin's lymphoma

MRLLLALLGVLLSVPGPPVLSLEASEEVELEPCLAPSLEQQEQELTVALGQPVRLCCGRAERGGHWYKE
GSRLAPAGRVRGWRGRLEIASFLPEDAGRYLCLARGSMIVLQNLTLITGDSLTSSNDDEDPKSHRDPSN
RHSYPQQAPYWTHPQRMEKKLHAVPAGNTVKFRCPAAGNPTPTIRWLKDGQAFHGENRIGGIRLRHQHW
SLVMESVVPSDRGTYTCLVENAVGSIRYNYLLDVLERSPHRPILQAGLPANTTAVVGSDVELLCKVYSD
AQPHIQWLKHIVINGSSFGADGFPYVQVLKTADINSSEVEVLYLRNVSAEDAGEYTCLAGNSIGLSYQS
AWLTVLPEEDPTWTAAAPEARYTDIILYASGSLALAVLLLLAGLYRGQALHGRHPRPPATVQKLSRFPL
ARQFSLESGSSGKSSSSLVRGVRLSSSGPALLAGLVSLDLPLDPLWEFPRDRLVLGKPLGEGCFGQVVR
AEAFGMDPARPDQASTVAVKMLKDNASDKDLADLVSEMEVMKLIGRHKNIINLLGVCTQEGPLYVIVEC
AAKGNLREFLRARRPPGPDLSPDGPRSSEGPLSFPVLVSCAYQVARGMQYLESRKCIHRDLAARNVLVT
EDNVMK**IADFGLAR**GVHHIDYYKKTSNGRLPVKWMAPEALFDRVYTHQSDVWSFGILLWEIFTLGGSPY
PGIPVEELFSLLREGHRMDRPPHCPPELYGLMRECWHAAPSQRPTFKQLVEALDKVLLAVSEEYLDLRL
TFGPYSPSGGDASSTCSSSDSVFSHDPLPLGSSSFPFGSGVQT

**Mass Match Peptides (bold):**
    IADFGLAR [349]

**Tandem Peptides (underline):**
    IADFGLAR [349]

## Peptide Source: 1D-GE, Lymphoid leukaemia

MRLLLALLGVLLSVPGPPVLSLEASEEVELEPCLAPSLEQQEQELTVALGQPVRLCCGRAERGGHWYKE
GSRLAPAGRVRGWRGRLEIASFLPEDAGRYLCLARGSMIVLQNLTLITGDSLTSSNDDEDPKSHRDPSN
RHSYPQQAPYWTHPQRMEKKLHAVPAGNTVKFRCPAAGNPTPTIRWLKDGQAFHGENRIGGIRLRHQHW
SLVMESVVPSDRGTYTCLVENAVGSIRYNYLLDVLERSPHRPILQAGLPANTTAVVGSDVELLCKVYSD
AQPHIQWLKHIVINGSSFGADGFPYVQVLKTADINSSEVEVLYLRNVSAEDAGEYTCLAGNSIGLSYQS
AWLTVLPEEDPTWTAAAPEARYTDIILYASGSLALAVLLLLAGLYRGQALHGRHPRPPATVQKLSRFPL
ARQFSLESGSSGKSSSSLVRGVRLSSSGPALLAGLVSLDLPLDPLWEFPRDRLVLGKPLGEGCFGQVVR
AEAFGMDPARPDQASTVAVKMLKDNASDKDLADLVSEMEVMKLIGRHKNIINLLGVCTQEGPLYVIVEC
AAKGNLREFLRARRPPGPDLSPDGPRSSEGPLSFPVLVSCAYQVARGMQYLESRKCIHRDLAARNVLVT
EDNVMK<u>IADFGLAR</u>GVHHIDYYKKTSNGRLPVKWMAPEALFDRVYTHQSDVWSFGILLWEIFTLGGSPY
PGIPVEELFSLLREGHRMDRPPHCPPELYGLMRECWHAAPSQRPTFKQLVEALDKVLLAVSEEYLDLRL
TFGPYSPSGGDASSTCSSSDSVFSHDPLPLGSSSFPFGSGVQT

**Mass Match Peptides (bold):**

**Tandem Peptides (underline):**
    IADFGLAR [349]

## Peptide Source: 1D-GE, Prostate cancer

MRLLLALLGVLLSVPGPPVLSLEASEEVELEPCLAPSLEQQEQELTVALGQPVRLCCGRAERGGHWYKE
GSRLAPAGRVRGWRGRLEIASFLPEDAGRYLCLARGSMIVLQNLTLITGDSLTSSNDDEDPKSHRDPSN
RHSYPQQAPYWTHPQRMEKKLHAVPAGNTVKFRCPAAGNPTPTIRWLKDGQAFHGENRIGGIRLRHQHW
SLVMESVVPSDRGTYTCLVENAVGSIRYNYLLDVLERSPHRPILQAGLPANTTAVVGSDVELLCKVYSD
AQPHIQWLKHIVINGSSFGADGFPYVQVLKTADINSSEVEVLYLRNVSAEDAGEYTCLAGNSIGLSYQS
AWLTVLPEEDPTWTAAAPEARYTDIILYASGSLALAVLLLLAGLYRGQALHGRHPRPPATVQKLSRFPL
ARQFSLESGSSGKSSSSLVRGVRLSSSGPALLAGLVSLDLPLDPLWEFPRDRLVLGKPLGEGCFGQVVR
AEAFGMDPARPDQASTVAVKMLKDNASDKDLADLVSEMEVMKLIGRHKNIINLLGVCTQEGPLYVIVEC
AAKGNLREFLRARRPPGPDLSPDGPRSSEGPLSFPVLVSCAYQVARGMQYLESRKCIHRDLAARNVLVT
EDNVMK**IADFGLAR**GVHHIDYYKKTSNGRLPVKWMAPEALFDRVYTHQSDVWSFGILLWEIFTLGGSPY
PGIPVEELFSLLREGHRMDRPPHCPPELYGLMRECWHAAPSQRPTFKQLVEALDKVLLAVSEEYLDLRL
TFGPYSPSGGDASSTCSSSDSVFSHDPLPLGSSSFPFGSGVQT

Mass Match Peptides (bold):

IADFGLAR [349]

Tandem Peptides (underline):

## Peptide Source: iTRAQ, Colorectal cancer

MRLLLALLGVLLSVPGPPVLSLEASEEVELEPCLAPSLEQQEQELTVALGQPVRLCCGRAERGGHWYKE
GSRLAPAGRVRGWRGRLEIASFLPEDAGRYLCLARGSMIVLQNLTLITGDSLTSSNDDEDPKSHRDPSN
RHSYPQQAPYWTHPQRMEKKLHAVPAGNTVKFRCPAAGNPTP IRWLKDGQAFHGENRIGGIRLRHQHW
SLVMESVVPSDRGTYTCLVENAVGSIRYNYLLDVLERSPHRP LQAGLPANTTAVVGSDVELLCKVYSD
AQPHIQWLKHIVINGSSFGADGFPYVQVLKTADINSSEVEVLYLRNVSAEDAGEYTCLAGNSIGLSYQS
AWLTVLPEEDPTWTAAAPEARYTDIILYASGSLALAVLLLLAGLYRGQALHGRHPRPPATVQKLSRFPL
ARQFSLESGSSGKSSSSLVRGVRLSSSGPALLAGLVSLDLPLDPLWEFPRDRLVLGKPLGEGCFGQVVR
AEAFGMDPARPDQASTVAVKMLKDNASDKDLADLVSEMEVMKLIGRHKNIINLLGVCTQEGPLYV VEC
AAKGNLREFLRARRPPGPDLSPDGPRSSEGPLSFPVLVSCAYQVARGMQYLESRKCIHRDLAARNVLVT
EDNVMKIADFGLARGVHHIDYYKKTSNGRLPVKWMAPEALFDRVYTHQSDVWSFGILLWEIFTLGGSPY
PGIPVEELFSLLREGHRMDRPPHCPPELYGLMRECWHAAPSQRPTFK<u>QLVEALDK</u>VLLAVSEEYLDLRL
TFGPYSPSGGDASSTCSSSDSVFSHDPLPLGSSS FPFGSGVQ

Mass Match Peptides (bold):

QLVEALDK [963]
QLVEALDK [963]

## Peptide Source: iTRAQ, Non-small cell lung cancer

MRLLLALLGVLLSVPGPPVLSLEASEEVELEPCLAPSLEQQEQELTVALGQPVRLCCGRAERGGHWYKE
GSRLAPAGRVRGWRGRLEIASFLPEDAGRYLCLARGSMIVLQNLTLITGDSLTSSNDDEDPKSHRDPSN
RHSYPQQAPYWTHPQRMEKKLHAVPAGNTVKFRCPAAGNPTP IRWLKDGQAFHGENRIGGIRLRHQHW
SLVMESVVPSDRGTYTCLVENAVGSIRYNYLLDVLERSPHRP LQAGLPANTTAVVGSDVELLCKVYSD
AQPHIQWLKHIVINGSSFGADGFPYVQVLKTADINSSEVEVLYLRNVSAEDAGEYTCLAGNSIGLSYQS
AWLTVLPEEDPTWTAAAPEARYTDIILYASGSLALAVLLLLAGLYRGQALIGRIIPRPPATVQKLSRFPL
ARQFSLESGSSGKSSSSLVRGVRLSSSGPALLAGLVSLDLPLDPLWEFPRDRLVLGKPLGEGCFGQVVR
AEAFGMDPARPDQASTVAVKMLKDNASDKDLADLVSEMEVMKLIGRHKNIINLLGVCTQEGPLYV VEC
AAKGNLREFLRARRPPGPDLSPDGPRSSEGPLSFPVLVSCAYQVARGMQYLESRKCIHRDLAARNVLVT
EDNVMKIADFGLARGVIIIIDYYKK<u>TSNGRLPVK</u>WMAPEALFDRVYTIIQSDVWSFGILLWEIFTLGGSPY
PGIPVEELFSLLREGHRMDRPPHCPPELYGLMRECWHAAPSQRPTFKQLVEALDKVLLAVSEEYLDLRL
TFGPYSPSGGDASSTCSSSDSVFSHDPLPLGSSS FPFGSGVQ

Mass Match Peptides (bold):

TSNGRLPVK [986]
TSNGRLPVK [986]

## Peptide Source: iTRAQ, Renal cell cancer

MRLLLALLGVLLSVPGPPVLSLEASEEVELEPCLAPSLEQQEQELTVALGQPVRLCCGRAERGGHWYKE
GSRLAPAGRVRGWRGRLEIASFLPEDAGRYLCLARGSMIVLQNLTLITGDSLTSSNDDEDPKSHRDPSN
RHSYPQQAPYWTHPQRMEKKLHAVPAGNTVKFRCPAAGNPTP IRWLKDGQAFHGENRIGGIRLRHQHW
SLVMESVVPSDRGTYTCLVENAVGSIRYNYLLDVLERSPHRP LQAGLPANTTAVVGSDVELLCKVYSD

AQPHIQWLKHIVINGSSFGADGFPYVQVLKTADINSSEVEVLYLRNVSAEDAGEYTCLAGNSIGLSYQS
AWLTVLPEEDPTWTAAAPEARYTDIILYASGSLALAVLLLLAGLYRGQALHGRHPRPPATVQKLSRFPL
ARQFSLESGSSGKSSSSLVRGVRLSSSGPALLAGLVSLDLPLDPLWEFPRDRLVLGKPLGEGCFGQVVR
**AEAFGMDPARPDQASTVAVK**MLKDNASDKDLADLVSEMEVMKLIGRHKNIINLLGVCTQEGPLYVIVEC
AAKGNLREFLRARRPPGPDLSPDGPRSSEGPLSFPVLVSCAYQVARGMQYLESRKCIHRDLAARNVLVT
EDNVMKIADFGLARGVHHIDYYKKTSNGRLPVKWMAPEALFDRVYTHQSDVWSFGILLWEIFTLGGSPY
PGIPVEELFSLLREGHRMDRPPHCPPELYGLMRECWHAAPSQRPTFKQLVEALDKVLLAVSEEYLDLRL
TFGPYSPSGGDASSTCSSSDSVFSHDPLPLGSSSFPFGSGVQT

**Mass Match Peptides (bold):**
AEAFGMDPARPDQASTVAVK [878]

**Tandem Peptides (underline):**
AEAFGMDPARPDQASTVAVK [878]


## Peptide Source: iTRAQ, Small cell lung cancer

MRLLLALLGVLLSVPGPPVLSLEASEEVELEPCLAPSLEQQEQELTVALGQPVRLCCGRAERGGHWYKE
GSRLAPAGRVRGWRGRLEIASFLPEDAGRYLCLARGSMIVLQNLTLITGDSLTSSNDDEDPKSHRDPSN
RHSYPQQAPYWTHPQRMEKKLHAVPAGNTVKFRCPAAGNPTPTIRWLKDGQAFHGENRIGGIRLRHQHW
SLVMESVVPSDRGTYTCLVENAVGSIRYNYLLDVLERSPHRPILQAGLPANTTAVVGSDVELLCKVYSD
AQPHIQWLKHIVINGSSFGADGFPYVQVLKTADINSSEVEVLYLRNVSAEDAGEYTCLAGNSIGLSYQS
AWLTVLPEEDPTWTAAAPEARYTDIILYASGSLALAVLLLLAGLYRGQALHGRHPRPPATVQKLSRFPL
ARQFSLESGSSGKSSSSLVRGVRLSSSGPALLAGLVSLDLPLDPLWEFPRDR<u>LVLGK</u>PLGEGCFGQVVR
AEAFGMDPARPDQASTVAVKMLKDNASDKDLADLVSEMEVMKLIGRHKNIINLLGVCTQEGPLYVIVEC
AAKGNLREFLRARRPPGPDLSPDGPRSSEGPLSFPVLVSCAYQVARGMQYLESRKCIHRDLAARNVLVT
EDNVMKIADFGLARGVHHIDYYKKTSNGRLPVKWMAPEALFDRVYTHQSDVWSFGILLWEIFTLGGSPY
PGIPVEELFSLLREGHRMDRPPHCPPELYGLMRECWHAAPSQRPTFKQLVEALDKVLLAVSEEYLDLRL
TFGPYSPSGGDASSTCSSSDSVFSHDPLPLGSSSFPFGSGVQT

**Mass Match Peptides (bold):**
LVLGK [951]

**Tandem Peptides (underline):**
LVLGK [951]


## OGTA247 (SEQ ID No: 44)

### Peptide Source: 1D-GE, Hepatocellular carcinoma

MVVALRYVWPLLLCSPCLLIQIPEEYEGHHVMEPPVITEQSPRRLVVFPTDDISLK**CEASGKPEVQFR**W
TRDGVHFKPKEELGVTVYQSPHSGSFTITGNNSNFAQR**FQGIYR**CFASNK<u>LGTAMSHEIR</u>LMAEGAPKW
PKETVKPVEVEEGESVVLPCNPPPSAEPLRIYWMNSKILHIKQDERVTMGQNGNLYFANVLTSDNHSDY
ICHAHFPGTRTIIQK**EPIDLR**VK**ATNSMIDR**KPRLLFPTNSSSHLVALQGQPLVLECIAEGFPTPTIK**W**
**LRPSGPMPADRVTYQNHNK**TLQLLK<u>VGEEDDGEYR</u>CLAENSLGSARHAYYVTVEAAPYWLHKPQSHLYG
PGETAR<u>LDCQVQGRPQPEVTWR</u>INGIPVEELAKDQKYRIQR**GALILSNVQPSDTMVTQCEAR**NRHGLLL
ANAYIYVVQLPAKILTADNQTYMAVQGSTAYLLCKAFGAPVPSVQWLDEDGTTVLQDER**FFPYANGTLG**
**IR**DLQANDTGRYFCLAANDQNNVTIMANLKVK**DATQITQGPRSTIEKK**GSR**VTFTCQASFDPSLQPSIT**
**WR**GDGR<u>DLQELGDSDK</u>**YFIEDGR**LVIHSLDYSDQGNYSCVASTELDVVESR**AQLLVVGSPGPVPR**LVLS
DLHLLTQSQVR**VSWSPAEDHNAPIEK**YDIEFEDKEMAPEKWYSLGKVPGNQTSTTLK**LSPYVHYTFR**VT
AINKYGPGEPSPVSETVVTPEAAPEKNPVDVKGEGNETTNMVITWKPLR**WMDWNAPQVQYRVQWRPQGT**
**R**GPWQEQIVSDPFLVVSNTSTFVPYEIKVQAVNSQGKGPEPQVTIGYSGEDYPQAIPELEGIEILNSSA
VLVKWRPVDLAQVKGHLR**GYNVTYWR**EGSQRKHSKRHIHKDHVVVPANTTSVILSGLRPYSSYHLEVQA
FNGRGSGPASEFTFSTPEGVPGHPEALHLECQSNTSLLLRWQPPLSHNGVLTGYVLSYHPLDEGGKGQL

SFNLRDPELRTHNLTDLSPHLRYRFQLQATTK**EGPGEAIVR**EGGTMALSGISDFGNISATAGENYSVVS
WVPKEGQCNFR**FHILFK**ALGEEKGGASLSPQYVSYNQSSYTQWDLQPDTDYEIHLFKERMFRH**QMAVK**T
NGTGRVRLPPAGFATEGWFIGFVSAIILLLLVLLILCFIKRSKGGKYSVKDK**EDTQVDSEARPMK**DETF
GEYRSLESDNEEKAFGSSQPSLNGDIKPLGSDDSLADYGGSVDVQFNEDGSFIGQYSGKKEKEAAGGND
SSGATSPINPAVALE

## Mass Match Peptides (bold):

AQLLVVGSPGPVPR [82]
ATNSMIDR [92]
CEASGKPEVQFR [101]
DATQITQGPR [109]
EDTQVDSEARPMK [170]
EGPGEAIVR [189]
EPIDLR [217]
FFPYANGTLGIR [251]
FHILFK [253]
FQGIYR [266]
GALILSNVQPSDTMVTQCEAR [284]
GYNVTYWR [330]
HQMAVK [342]
LGTAMSHEIR [463]
LSPYVHYTFR [489]
STIEKK [659]
VQWRPQGTR [795]
VSWSPAEDHNAPIEK [798]
VTFTCQASFDPSLQPSITWR [804]
VTYQNHNK [808]
WLRPSGPMPADR [828]
WMDWNAPQVQYR [829]
YFIEDGR [850]

## Tandem Peptides (underline):

AQLLVVGSPGPVPR [82]
DATQITQGPR [109]
DLQELGDSDK [137]
EGPGEAIVR [189]
INGIPVEELAK [382]
LDCQVQGRPQPEVTWR [445]
LGTAMSHEIR [463]
VGEEDDGEYR [762]
YDIEFEDK [842]
YFIEDGR [850]

## Peptide Source: 1D-GE, Melanoma

MVVALRYVWPLLLCSPCLLIQIPEEYEGHHVMEPPVITEQSPRRLVVFPTDDISLK**CEASGKPEVQFR**W
TR**DGVHFKPKEE**LGVIVYQSPHSGSFTITGNNSNTAQR**FQGIYR**CFASNK**LGTAMSHEIR**LMAEGAPKW
PKETVKPVEVEEGESVVLPCNPPPSAEPLRIYWMNSKILHIKQDERVTMGQNGNLYFANVLTSDNHSDY
ICHAHFPGTRTIIQK**EPIDLR**VKATNSMIDRKPRLLFPTNSSSHLVALQGQPLVLECIAEGFPTPTIK**W**
**LRPSGPMPADR**VTYQNHNKTLQLLK**VGEEDDGEYRCLAENSLGSAR**HAYYVTVEAAPYWLHKPQSHLYG
PGETARLDCQVQGRPQPEVTWR**INGIPVEELAK**DQKYRIQR**GALILSNVQPSDTMVTQCEAR**NRHGLLL
ANAYIYVVQLPAKILTADNQTYMAVQGSTAYLLCK**AFGAPVPSVQWLDEDGTTVLQDERFFPYANGTLG**
**IR**DLQANDTGRYFCLAANDQNNVTIMANLKVK**DATQITQGPR**STIEKKGSRVTFTCQASFDPSLQPSIT
WRGDGRDLQELGDSDK**YFIEDGR**LVIHSLDYSDQGNYSCVASTELDVVESR**AQLLVVGSPGPVPRLVLS**
**DLHLLTQSQVRVSWSPAEDHNAPIEK**YDIEFEDKEMAPEKWYSLGKVPGNQTSTTLK**LSPYVHYTFR**VT
AINKYGPGEPSPVSETVVTPEAAPEKNPVDVK**GEGNETTNMVITWKPLRWMDWNAPQVQYRVQWRPQGT**

RGPWQEQIVSDPFLVVSNTSTFVPYEIKVQAVNSQGKGPEPQVTIGYSGEDYPQAIPELEGIEILNSSA
VLVKWRPVDLAQVKGHLRGYNVTYWREGSQRKHSKRHIHKDHVVVPANTTSVILSGLRPYSSYHLEVQA
FNGRGSGPASEFTFSTPEGVPGHPEALHLECQSNTSLLLRWQPPLSHNGVLTGYVLSYHPLDEGGKGQL
SFNLRDPELRTHNLTDLSPHLRYRFQLQATTKEGPGEAIVREGGTMALSGISDFGNISATAGENYSVVS
WVPKEGQCNFRFHILFKALGEEKGGASLSPQYVSYNQSSYTQWDLQPDTDYEIHLFKERMFRHQMAVKT
NGTGRVRLPPPAGFATEGWFIGFVSAIILLLLVLLILCFIKRSKGGKYSVKDKEDTQVDSEARPMKDETF
GEYRSLESDNEEKAFGSSQPSLNGDIKPLGSDDSLADYGGSVDVQFNEDGSFIGQYSGKKEKEAAGGND
SSGATSPINPAVALE

## Mass Match Peptides (bold):

AFGAPVPSVQWLDEDGTTVLQDER [65]
AQLLVVGSPGPVPR [82]
CEASGKPEVQFR [101]
CLAENSLGSAR [105]
DATQITQGPR [109]
DGVHFKPK [126]
EGPGEAIVR [189]
EPIDLR [217]
FFPYANGTLGIR [251]
FHILFK [253]
FQGIYR [266]
GALILSNVQPSDTMVTQCEAR [284]
GEGNETTNMVITWKPLR [289]
GQLSFNLR [312]
INGIPVEELAK [382]
LGTAMSHEIR [463]
LSPYVHYTFR [489]
LVLSDLHLLTQSQVR [502]
VGEEDDGEYR [762]
VQWRPQGTR [795]
VSWSPAEDHNAPIEK [798]
WLRPSGPMPADR [828]
WMDWNAPQVQYR [829]
WRPVDLAQVK [831]
YFIEDGR [850]

## Tandem Peptides (underline):

AQLLVVGSPGPVPR [82]
DATQITQGPR [109]
DHVVVPANTTSVILSGLRPYSSYHLEVQAFNGR [128]
EELGVTVYQSPHSGSFTITGNNSNFAQR [178]
EGPGEAIVR [189]
VQWRPQGTR [795]
WMDWNAPQVQYR [829]
WRPVDLAQVK [831]


## Peptide Source: iTRAQ, Non-small cell lung cancer

MVVALRYVWPLLLCSPCLLIQIPEEYEGHHVMEPPVITEQSPRRLVVFPTDDISLKCEASGKPEVQFRW
TRDGVHFKPKEELGVTVYQSPHSGSFTITGNNSNFAQRFQGIYRCFASNKLGTAMSHEIRLMAEGAPKW
PKETVKPVEVEEGESVVLPCNPPPSAEPLRIYWMNSKILHIKQDERVTMGQNGNLYFANVLTSDNHSDY
ICHAHFPGTRTIIQKEPIDLRVKATNSMIDRKPRLLFPTNSSSHLVALQGQPLVLECIAEGFPTPTIKW
LRPSGPMPADRVTYQNHNKTLQLLKVGEEDDGEYRCLAENSLGSARHAYYVTVEAAPYWLHKPQSHLYG
PGETARLDCQVQGRPQPEVTWRINGIPVEELAKDQKYRIQRGALILSNVQPSDTMVTQCEARNRHGLLL
ANAYIYVVQLPAKILTADNQTYMAVQGSTAYLLCKAFGAPVPSVQWLDEDGTTVLQDERFFPYANGTLG
IRDLQANDTGRYFCLAANDQNNVTIMANLKVKDATQITQGPRSTIEKKGSRVTFTCQASFDPSLQPSIT

WRGDGRDLQELGDSDKYFIEDGRLVIHSLDYSDQGNYSCVASTELDVVESRAQLLVVGSPGPVPRLVLS
DLHLLTQSQVRVSWSPAEDHNAPIEKYDIEFEDKEMAPEKWYSLGKVPGNQTSTTLKLSPYVHYTFRVT
AINKYGPGEPSPVSETVVTPEAAPEKNPVDVKGEGNETTNMVITWKPLRWMDWNAPQVQYRVQWRPQGT
RGPWQEQIVSDPFLVVSNTSTFVPYEIKVQAVNSQGKGPEPQVTIGYSGEDYPQAIPELEGIEILNSSA
VLVK**WRPVDLAQVK**GHLRGYNVTYWREGSQRKHSKRHIHKDHVVVPANTTSVILSGLRPYSSYHLEVQA
FNGRGSGPASEFTFSTPEGVPGHPEALHLECQSNTSLLLRWQPPLSHNGVLTGYVLSYHPLDEGGKGQL
SFNLRDPELRTHNLTDLSPHLRYRFQLQATTKEGPGEAIVREGGTMALSGISDFGNISATAGENYSVVS
WVPKEGQCNFRFHILFKALGEEKGGASLSPQYVSYNQSSYTQWDLQPDTDYEIHLFK**ERMFR**HQMAVKT
**NGTGRVR**LPPAGFATEGWFIGFVSAIILLLLVLLILCFIKRSKGGKYSVKDKEDTQVDSEARPMKDETF
GEYRSLESDNEEKAFGSSQPSLNGDIKPLGSDDSLADYGGSVDVQFNEDGSFIGQYSGKKEKEAAGGND
SSGATSPINPAVALE

### Mass Match Peptides (bold):

    ERMFR [906]
    TNGTGRVR [984]
    WRPVDLAQVK [831]

### Tandem Peptides (underline):

    ERMFR [906]
    TNGTGRVR [984]
    WRPVDLAQVK [831]

## Peptide Source: iTRAQ, Small cell lung cancer

MVVALRYVWPLLLCSPCLLIQIPEEYEGHHVMEPPVITEQSPRRLVVFPTDDISLKCEASGKPEVQFRW
TRDGVHFKPKEELGVTVYQSPHSGSFTITGNNSNFAQRFQGIYRCFASNKLGTAMSHEIRLMAEGAPKW
PKETVKPVEVEEGESVVLPCNPPPSAEPLRIYWMNSKILHIKQDERVTMGQNGNLYFANVLTSDNHSDY
ICHAHFPGTRTIIQK**EPIDLR**VKATNSMIDRKPRLLFPTNSSSHLVALQGQPLVLECIAEGFPTPTIKW
LRPSGPMPADRVTYQNIINKTLQLLK**VGEEDDGEYR**CLAENSLGSARIIAYYVTVEAAPYWLIIKPQSIILYG
PGETARLDCQVQGRPQPEVTWRINGIPVEELAKDQKYRIQRGALILSNVQPSDTMVTQCEARNRHGLLL
ANAYIYVVQLPAKILTADNQTYMAVQGSTAYLLCKAFGAPVPSVQWLDEDGTTVLQDERFFPYANGTLG
IRDLQANDTGRYFCLAANDQNNVTIMANLKVK**DATQITQGPR**STIEKKGSRVTFTCQASFDPSLQPSIT
WRGDGR**DLQELGDSDK**YFIEDGRLVIHSLDYSDQGNYSCVASTELDVVESR**AQLLVVGSPGPVPR**LVLS
DLHLLTQSQVRVSWSPAEDHNAPIEKYDIEFEDKEMAPEKWYSLGKVPGNQTSTTLKLSPYVHYTFR**VT**
**AINKYGPGEPSPVSETVVTPEAAPEK**NPVDVKGEGNETTNMVITWKPLRWMDWNAPQVQYRVQWRPQGT
RGPWQEQIVSDPFLVVSNTSTFVPYEIK**VQAVNSQGK**GPEPQVTIGYSGEDYPQAIPELEGIEILNSSA
VLVKWRPVDLAQVKGHLRGYNVTYWREGSQRKHSKRHIHKDHVVVPANTTSVILSGLRPYSSYHLEVQA
FNGRGSGPASEFTFSTPEGVPGHPEALHLECQSNTSLLLRWQPPLSHNGVLTGYVLSYHPLDEGGKGQL
SFNLR**DPELR**THNLTDLSPHLRYRFQLQATTK**EGPGEAIVR**EGGTMALSGISDFGNISATAGENYSVVS
WVPKEGQCNFRFHILFKALGEEKGGASLSPQYVSYNQSSYTQWDLQPDTDYEIHLFKERMFRHQMAVKT
**NGTGRVR**LPPAGFATEGWFIGFVSAIILLLLVLLILCFIKRSKGGKYSVKDKEDTQVDSEARPMKDETF
GEYRSLESDNEEKAFGSSQPSLNGDIKPLGSDDSLADYGGSVDVQFNEDGSFIGQYSGKKEKEAAGGND
SSGATSPINPAVALE

### Mass Match Peptides (bold):

    AQLLVVGSPGPVPR [82]
    DATQITQGPR [109]
    DLQELGDSDK [137]
    DPELR [893]
    EGPGEAIVR [189]
    EPIDLR [217]
    NPVDVK [958]
    TNGTGRVR [984]
    VGEEDDGEYR [762]
    VQAVNSQGK [998]

VTAINK [1000]
YGPGEPSPVSETVVTPEAAPEK [1005]

Tandem Peptides (underline):

AQLLVVGSPGPVPR [82]
DATQITQGPR [109]
DLQELGDSDK [137]
DPELR [893]
EGPGEAIVR [189]
EPIDLR [217]
NPVDVK [958]
TNGTGRVR [984]
VGEEDDGEYR [762]
VQAVNSQGK [998]
VTAINK [1000]
YGPGEPSPVSETVVTPEAAPEK [1005]


## OGTA248 (SEQ ID No: 45)

### Peptide Source: 1D-GE, Renal cell cancer

MGCWGQLLVWFGAAGAILCSSPGSQETFLRSSPLPLASPSPQDPKVSAPPSILEPASPLNSPGTEGSWL
FSTCGASGRHGPTQTQCDGAYAGTSVVVTVGAAGQLRGVQLWRVPGPGQYLISAYGAAGGKGAKNHLSR
AHGVFVSAIFSLGLGESLYILVGQQGEDACPGGSPESQLVCLGESRAVEEHAAMDGSEGVPGSRRWAGG
GGGGGGATYVFRVRAGELEPLLVAAGGGGRAYLRPRDRGRTQASPEKLENRSEAPGSGGRGGAAGGGGG
WTSRAPSPQAGRSLQEGAEGGQGCSEAWATLGWAAAGGFGGGGGACTAGGGGGGYRGGDASETDNLWAD
GEDGVSFIHPSSELFLQPLAVTENHGEVEIRRHLNCSHCPLRDCQWQAELQLAECLCPEGMELAVDNVT
CMDLHKPPGPLVLMVAVVATSTLSLLMVCGVLILVKQKKWQGLQEMRLPSPELELSKLRTSAIRTAPNP
YYCQVGLGPAQSWPLPPGVTEVSPANVTLLRALGHGAFGEVYEGLVIGLPGDSSPLQVAIKTLPELCSP
QDELDFLMEALIISKFRHQNIVRCVGLSLRATPRLILLELMSGGDMKSFLRHSRPHLGQPSPLVMRDLL
QLAQDIAQGCHYLEENHFIHRDIAARNCLLSCAGPSRVAKIGDFGMARDIYRASYYRRGDRALLPVKWM
PPEAFLEGIFTSKTDSWSFGVLLWEIFSLGYMPYPGRTNQEVLDFVVGGGRMDPPRGCPGPVYRIMTQC
WQHEPELRPSFASILERLQYCTQDPDVLNSLLPMELGPTPEEEGTSGLGNRSLECLRPPQPQELSPEKL
KSWGGSPLGPWLSSGLKPLKSRGLQPQNLWNPTYRS

Mass Match Peptides (bold):
Tandem Peptides (underline):

TNQEVLDFVVGGGR [712]


### Peptide Source: iTRAQ, Non-small cell lung cancer

MGCWGQLLVWFGAAGAILCSSPGSQETFLRSSPLPLASPSPQDPKVSAPPSILEPASPLNSPGTEGSWL
FSTCGASGRHGPTQTQCDGAYAGTSVVVTVGAAGQLRGVQLWRVPGPGQYLISAYGAAGGKGAKNHLSR
AHGVFVSAIFSLGLGESLYILVGQQGEDACPGGSPESQLVCLGESRAVEEHAAMDGSEGVPGSRRWAGG
GGGGGGATYVFRVRAGELEPLLVAAGGGGRAYLRPRDRGRTQASPEKLENRSEAPGSGGRGGAAGGGGG
WTSRAPSPQAGRSLQEGAEGGQGCSEAWATLGWAAAGGFGGGGGACTAGGGGGGYRGGDASETDNLWAD
GEDGVSFIHPSSELFLQPLAVTENHGEVEIRRHLNCSHCPLRDCQWQAELQLAECLCPEGMELAVDNVT
CMDLHKPPGPLVLMVAVVATSTLSLLMVCGVLILVKQKKWQGLQEMRLPSPELELSKLRTSAIRTAPNP
YYCQVGLGPAQSWPLPPGVTEVSPANVTLLRALGHGAFGEVYEGLVIGLPGDSSPLQVAIKTLPELCSP
QDELDFLMEALIISKFRHQNIVRCVGLSLRATPRLILLELMSGGDMKSFLRHSRPHLGQPSPLVMRDLL
QLAQDIAQGCHYLEENHFIHRDIAARNCLLSCAGPSRVAKIGDFGMARDIYRASYYRRGDRALLPVKWM
PPEAFLEGIFTSKTDSWSFGVLLWEIFSLGYMPYPGRTNQEVLDFVVGGGRMDPPRGCPGPVYRIMTQC
WQHEPELRPSFASILERLQYCTQDPDVLNSLLPMELGPTPEEEGTSGLGNRSLECLRPPQPQELSPEKL
KSWGGSPLGPWLSSGLKPLKSRGLQPQNLWNPTYRS

**Mass Match Peptides (bold):**

LRTSAIR [945]

**Tandem Peptides (underline):**

LRTSAIR [945]

## OGTA249 (SEQ ID No: 46)

### Peptide Source: 1D-GE, Ovarian cancer

MSAGGASVPPPPNPAVSFPPPRVTLPAGPDILRTYSGAFVCLEILFGGLVWILVASSNVPLPLLQGWVM
FVSVTAFFFSLLFLGMFLSGMVAQIDANWNFLDFAYHFTVFVFYFGAFLLEAAATSLHDLHCNTTITGQ
PLLSDNQYNINVAASIFAFMTTACYGCSLGLALRRWRP

**Mass Match Peptides (bold):**

MSAGGASVPPPPNPAVSFPPPR [524]

**Tandem Peptides (underline):**

VTLPAGPDLLR [805]

### Peptide Source: iTRAQ, Non-small cell lung cancer

MSAGGASVPPPPNPAVSFPPPRVTLPAGPDILRTYSGAFVCLEILFGGLVWILVASSNVPLPLLQGWVM
FVSVTAFFFSLLFLGMFLSGMVAQIDANWNFLDFAYHFTVFVFYFGAFLLEAAATSLHDLHCNTTITGQ
PLLSDNQYNINVAASIFAFMTTACYGCSLGLALRRWRP

**Mass Match Peptides (bold):**

VTLPAGPDILR [1001]

**Tandem Peptides (underline):**

VTLPAGPDILR [1001]

### Peptide Source: iTRAQ, Renal cell cancer

MSAGGASVPPPPNPAVSFPPPRVTLPAGPDILRTYSGAFVCLEILFGGLVWILVASSNVPLPLLQGWVM
FVSVTAFFFSLLFLGMFLSGMVAQIDANWNFLDFAYHFTVFVFYFGAFLLEAAATSLHDLHCNTTITGQ
PLLSDNQYNINVAASIFAFMTTACYGCSLGLALRRWRP

**Mass Match Peptides (bold):**

VTLPAGPDILR [1001]

**Tandem Peptides (underline):**

VTLPAGPDILR [1001]

## OGTA257 (SEQ ID No: 47)

### Peptide Source: 1D-GE, Pancreatic cancer

```
MGLSLPKEKGLILCLWSKFCRWFQRRESWAQSRDEQNLLQQKRIWESPLLLAAKDNDVQALNKLLKYED
CKVHQRGAMGETALHIAALYDNLEAAMVLMEAAPELVFEPMTSELYEGQTALHIAVVNQNMNLVRALLA
RRASVSARATGTAFRRSPCNLIYFGEHPLSFAACVNSEEIVRLLIEHGADIRAQDSLGNTVLHILILQP
NKTFACQMYNLLLSYDRHGDHLQPLDLVPNHQGLTPFKLAGVEGNTVMFQHLMQKRKHTQWTYGPLTST
LYDLTEIDSSGDEQSLLELIITTKKREARQILDQTPVKELVSLKWKRYGRPYFCMLGAIYLLYIICFTM
CCIYRPLKPRTNNRTSPRDNTLLQQKLLQEAYMTPKDDIRLVGELVTVIGAIIILLVEVPDIFRMGVTR
FFGQTILGGPFHVLIITYAFMVLVTMVMRLISASGEVVPMSFALVLGWCNVMYFARGFQMLGPFTIMIQ
KMIFGDLMRFCWLMAVVILGFASAFYIIFQTEDPEELGHFYDYPMALFSTFELFLTIIDGPANYNVDLP
FMYSITYAAFAIIATLLMLNLLIAMMGDTHWRVAHERDELWRAQIVATTVMLERKLPRCLWPRSGICGR
EYGLGDRWFLRVEDRQDLNRQRIQRYAQAFHTRGSEDLDKDSVEKLELGCPFSPHLSLPMPSVSRSTSR
SSANWERLRQGTLRRDLRGIINRGLEDGESWEYQI
```

**Mass Match Peptides (bold):**
**Tandem Peptides (underline):**
LLQEAYMTPK [468]

## Peptide Source: iTRAQ, Colorectal cancer

```
MGLSLPKEKGLILCLWSKFCRWFQRRESWAQSRDEQNLLQQKRIWESPLLLAAKDNDVQALNKLLKYED
CKVHQRGAMGETALHIAALYDNLEAAMVLMEAAPELVFEPMTSELYEGQTALHIAVVNQNMNLVRALLA
RRASVSARATGTAFRRSPCNLIYFGEHPLSFAACVNSEEIVRLLIEHGADIRAQDSLGNTVLHILILQP
NKTFACQMYNLLLSYDRHGDHLQPLDLVPNHQGLTPFKLAGVEGNTVMFQHLMQKRKHTQWTYGPLTST
LYDLTEIDSSGDEQSLLELIITTKKREARQILDQTPVKELVSLKWKRYGRPYFCMLGAIYLLYIICFTM
CCIYRPLKPRTNNRTSPRDNTLLQQKLLQEAYMTPKDDIRLVGELVTVIGAIIILLVEVPDIFRMGVTR
FFGQTILGGPFHVLIITYAFMVLVTMVMRLISASGEVVPMSFALVLGWCNVMYFARGFQMLGPFTIMIQ
KMIFGDLMRFCWLMAVVILGFASAFYIIFQTEDPEELGHFYDYPMALFSTFELFLTIIDGPANYNVDLP
FMYSITYAAFAIIATLLMLNLLIAMMGDTHWRVAHERDELWRAQIVATTVMLERKLPRCLWPRSGICGR
EYGLGDRWFLRVEDRQDLNRQRIQRYAQAFHTRGSEDLDKDSVEKLELGCPFSPHLSLPMPSVSRSTSR
SSANWERLRQGTLRRDLRGIINRGLEDGESWEYQI
```

**Mass Match Peptides (bold):**
QILDQTPVK [962]
**Tandem Peptides (underline):**
QILDQTPVK [962]

## Peptide Source: iTRAQ, Hepatocellular cancer

```
MGLSLPKEKGLILCLWSKFCRWFQRRESWAQSRDEQNLLQQKRIWESPLLLAAKDNDVQALNKLLKYED
CKVHQRGAMGETALHIAALYDNLEAAMVLMEAAPELVFEPMTSELYEGQTALHIAVVNQNMNLVRALLA
RRASVSARATGTAFRRSPCNLIYFGEHPLSFAACVNSEEIVRLLIEHGADIRAQDSLGNTVLHILILQP
NKTFACQMYNLLLSYDRHGDHLQPLDLVPNHQGLTPFKLAGVEGNTVMFQHLMQKRKHTQWTYGPLTST
LYDLTEIDSSGDEQSLLELIITTKKREARQILDQTPVKELVSLKWKRYGRPYFCMLGAIYLLYIICFTM
CCIYRPLKPRTNNRTSPRDNTLLQQKLLQEAYMTPKDDIRLVGELVTVIGAIIILLVEVPDIFRMGVTR
FFGQTILGGPFHVLIITYAFMVLVTMVMRLISASGEVVPMSFALVLGWCNVMYFARGFQMLGPFTIMIQ
KMIFGDLMRFCWLMAVVILGFASAFYIIFQTEDPEELGHFYDYPMALFSTFELFLTIIDGPANYNVDLP
FMYSITYAAFAIIATLLMLNLLIAMMGDTHWRVAHERDELWRAQIVATTVMLERKLPRCLWPRSGICGR
EYGLGDRWFLRVEDRQDLNRQRIQRYAQAFHTRGSEDLDKDSVEKLELGCPFSPHLSLPMPSVSRSTSR
SSANWERLRQGTLRRDLRGIINRGLEDGESWEYQI
```

**Mass Match Peptides (bold):**
LRQGTLR [944]

**Tandem Peptides (underline):**

    LRQGTLR [944]

## Peptide Source: iTRAQ, Non-small cell lung cancer

MGLSLPKEKGLILCLWSKFCRWFQRRESWAQSRDEQNLLQQKRIWESPLLLAAKDNDVQALNKLLKYED
CKVHQRGAMGETALHIAALYDNLEAAMVLMEAAPELVFEPMTSELYEGQTALHIAVVNQNMNLVRALLA
RRASVSAR**ATGTAFRR**SPCNLIYFGEHPLSFAACVNSEEIVRLLIEHGADIRAQDSLGNTVLHILILQP
NKTFACQMYNLLLSYDRHGDHLQPLDLVPNHQGLTPFKLAGVEGNTVMFQHLMQKRKHTQWTYGPLTST
LYDLTEIDSSGDEQSLLELIITTKKREARQILDQTPVK**ELVSLK**WKRYGRPYFCMLGAIYLLYIICFTM
CCIYRPLKPRTNNRTSPRDNTLLQQKLLQEAYMTPKDDIRLVGELVTVIGAIIILLVEVPDIFRMGVTR
FFGQTILGGPFHVLIITYAFMVLVTMVMRLISASGEVVPMSFALVLGWCNVMYFARGFQMLGPFTIMIQ
KMIFGDLMRFCWLMAVVILGFASAFYIIFQTEDPEELGHFYDYPMALFSTFELFLTIIDGPANYNVDLP
FMYSITYAAFAIIATLLMLNLLIAMMGDTHWRVAHERDELWRAQIVATTVMLERKLPRCLWPRSGICGR
EYGLGDRWFLRVEDRQDLNRQRIQRYAQAFHTRGSEDLDKDSVEKLELGCPFSPHLSLPMPSVSRSTSR
SSANWERLRQGTLRRDLRGIINRGLEDGESWEYQI

**Mass Match Peptides (bold):**

    ATGTAFRR [884]
    ELVSLK [902]

**Tandem Peptides (underline):**

    ATGTAFRR [884]
    ELVSLK [902]

## Peptide Source: iTRAQ, Ovarian cancer

MGLSLPKEKGLILCLWSKFCRWFQRRESWAQSRDEQNLLQQKRIWESPLLLAAKDNDVQALNKLLKYED
CKVHQRGAMGETALHIAALYDNLEAAMVLMEAAPELVFEPMTSELYEGQTALHIAVVNQNMNLVRALLA
RRASVSARATGTAFRRSPCNLIYFGEHPLSFAACVNSEEIVR**LLIEHGADIR**AQDSLGNTVLHILILQP
NKTFACQMYNLLLSYDRHGDHLQPLDLVPNHQGLTPFKLAGVEGNTVMFQHLMQKRKHTQWTYGPLTST
LYDLTEIDSSGDEQSLLELIITTKKREARQILDQTPVKELVSLKWKRYGRPYFCMLGAIYLLYIICFTM
CCIYRPLKPRTNNRTSPRDNTLLQQKLLQEAYMTPKDDIRLVGELVTVIGAIIILLVEVPDIFRMGVTR
FFGQTILGGPFHVLIITYAFMVLVTMVMRLISASGEVVPMSFALVLGWCNVMYFARGFQMLGPFTIMIQ
KMIFGDLMRFCWLMAVVILGFASAFYIIFQTEDPEELGHFYDYPMALFSTFELFLTIIDGPANYNVDLP
FMYSITYAAFAIIATLLMLNLLIAMMGDTHWRVAHERDELWRAQIVATTVMLERKLPRCLWPRSGICGR
EYGLGDRWFLRVEDRQDLNRQRIQRYAQAFHTRGSEDLDKDSVEKLELGCPFSPHLSLPMPSVSRSTSR
SSANWERLRQGTLRRDLRGIINRGLEDGESWEYQI

**Mass Match Peptides (bold):**

    LLIEHGADIR [936]

**Tandem Peptides (underline):**

    LLIEHGADIR [936]

## OGTA271 (SEQ ID No: 48)

## Peptide Source: 1D-GE, Breast cancer

MAPEPAPGRTMVPLVPALVMLGLVAGAHGDSKPVFIK**VPEDQTGLSGGVASFVCQATGEPKPRITWMKK**
GKKVSSQR**FEVIEFDDGAGSVLR**IQPLRVQRDEAIYECTATNSLGEINTSAK**LSVLEEEQLPPGFPSID**
**MGPQLK**VVEKAR**TATMLCAAGGNPDPEISWFKDFLPVDPATSNGR**IKQLRSGALQIESSEESDQGKYEC

```
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEARTAQSTPSAPPQKVMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVEGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT
```

## Mass Match Peptides (bold):

```
AAGTEGPFQEVDGVATTR [50]
AALEYLGSFDHYAT [51]
ACNPLDAGPMVVHCSAGVGR [56]
AGLGEEFEK [71]
AHTDVGPGPESSPVLVR [72]
AYIATQGPLAESTEDFWR [98]
DFLPVDPATSNGR [117]
DHPPIPITDLADNIER [127]
DINSQQELQNITTDTR [129]
DSLLAHSSDPVEMRR [157]
EHSSWDLVGLEK [196]
ELPGELLGYR [206]
EQFGQDGPITVHCSAGVGR [220]
FDLSMPHVQDPSLVR [246]
FEVIEFDDGAGSVLR [249]
FTLTGLKPDTTYDIK [275]
GPPSEAVR [308]
GSGPLSPSIQSR [315]
GVEGSDYINASFLDGYR [326]
HVVDGISR [347]
ILYNGQSVEVDGHSMR [378]
ILYNGQSVEVDGHSMRK [379]
ITWMKK [399]
KLIADLQPNTEYSFVLMNR [424]
KQNAYIATQGPLPETMGDFWR [433]
LENGEPR [455]
LGQVPPGESVTAMELEFK [462]
LIADLQPNTEYSFVLMNR [467]
LNYQTPGMR [475]
LSVLEEEQLPPGFPSIDMGPQLK [492]
```

```
MLSASTMLVQWEPPEEPNGLVR [518]
MLWEHNSTIIVMLTK [519]
MVWEQR [529]
NGVITQYSVAYEAVDGEDR [537]
QFQFTDWPEQGVPK [572]
QHGQIR [578]
QNAYIATQGPLPETMGDFWR [581]
RLNYQTPGMR [606]
RPPNAWHK [609]
SANYTCVAISSLGMIEATAQVTVK [619]
SDMGVGVFTPTIEAR [621]
TATMLCAAGGNPDPEISWFK [674]
TATVVMMTR [676]
TDEDVPSGPPRK [683]
TGCFIVIDAMLER [692]
TGEGFIDFIGQVHK [693]
TSVLLSWEVPDSYK [732]
TVDIYGHVTCMR [735]
VLAVNSIGR [774]
VMCVSMGSTTVR [785]
VPEDQTGLSGGVASFVCQATGEPKPR [789]
VSWVPPPADSR [799]
YEGVVDMFQTVK [844]
YQYFVVDPMAEYNMPQYILR [869]
```

**Tandem Peptides (underline):**

```
AALEYLGSFDHYAT [51]
DDQHFTVTGLHK [111]
GVEGSDYINASFLDGYR [326]
SDMGVGVFTPTIEAR [621]
YEGVVDMFQTVK [844]
```

## Peptide Source: 1D-GE, Cervical cancer

```
MAPEPAPGRTMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTAINSLGEINTSAKLSVLEEEQLPPGFPSID
MGPQLKVVEKARTATMLCAAGGNPDPEISWFKDFLPVDPATSNGRIKQLRSGALQIESSEESDQGKYEC
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEARTAQSTPSAPPQKVMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVTGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
```

AFLRRVKACNPLDAGPMVVHCSAGVGRTGCFIVIDAMLERMKHEK**TVDIYGHVTCMR**SQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT

## Mass Match Peptides (bold):

                AAGTEGPFQEVDGVATTR [50]
                ADEARPNTIDFGK [58]
                AHTDVGPGPESSPVLVR [72]
                DDQHFTVTGLHK [111]
                DFLPVDPATSNGR [117]
                ELPGELLGYR [206]
                EPMDQKR [218]
                FDLSMPHVQDPSLVR [246]
                FEVIEFDDGAGSVLR [249]
                GSSAGGLQHLVSIR [321]
                NVLELSNVVR [558]
                QHGQIR [578]
                RPPNAWHK [609]
                SDMGVGVFTPTIEAR [621]
                TDEDVPSGPPR [682]
                TQQGVPAQPADFQAEVESDTR [725]
                TVDIYGHVTCMR [735]
                VLAVNSIGR [774]
                VSWVPPPADSR [799]
                VYYTPDSR [823]
                WTEYR [836]
                YSIGGLSPFSEYAFR [872]

## Tandem Peptides (underline):

                AAGTEGPFQEVDGVATTR [50]
                TDEDVPSGPPR [682]
                VYYTPDSR [823]

## Peptide Source: 1D-GE, Colorectal cancer

**MAPEPAPGR**TMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTATNSLGEINTSAKLSVLEEEQLPPGFPSID
MGPQLKVVEKARTATMLCAAGGNPDPEISWFKDFLPVDPATSNGRIKQLRSGALQIESSEESDQGKYEC
VATNSAGTR**YSAPANLYVR**VRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRAR**TGEQAPS
SPPR**RVQARMLSASTMLVQWEPPEEPNGLVRGYRVYYTPDSRRPPNAWHKHNTDAGLLTTVGSLLPGIT
YSLRVLAFTAVGDGPPSPTIQVK**TQQGVPAQPADFQAEVESDTR**IQLSWLLPPQERIIMYELVYWAAED
EDQQHKVTFDPTSSYTLEDLKPDTLYRFQLAAR**SDMGVGVFTPTIEAR**TAQSTPSAPPQKVMCVSMGST
TVRVSWVPPPADSRNGVITQYSVAYEAVDGEDRGRHVVDGISREHSSWDLVGLEKWTEYRVWVRAHTDV
GPGPESSPVLVR**TDEDVPSGPPR**KVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVTGLHKGTTYIFRLAAKNRAGLGEEFEK
EIRTPEDLPSGFPQNLHVTGLTTSTTELAWDPPVLAERNGRITSYTVVFRDINSQQELQNITTDTRFTL
TGLKPDTTYDIKVRAWTSK**GSGPLSPSIQSR**TMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRQAER
LKPYVAAQLDVLPETFTLGDKKNYRGFYNRPLSPDLSYQCFVLASLKEPMDQKRYASSPYSDEIVVQVT

PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVIAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT

**Mass Match Peptides (bold):**
    GSGPLSPSIQSR [315]
    MAPEPAPGR [509]
    SDMGVGVFTPTIEAR [621]
    TDEDVPSGPPR [682]
    TGEQAPSSPPR [694]
    TQQGVPAQPADFQAEVESDTR [725]

**Tandem Peptides (underline):**
    TDEDVPSGPPR [682]
    TGEQAPSSPPR [694]
    YSAPANLYVR [870]

## Peptide Source: 1D-GE, Hepatocellular carcinoma

MAPEPAPGRTMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTATNSLGEINTSAKLSVLEEEQLPPGFPSID
MGPQLKVVEKARTATMLCAAGGNPDPEISWFKDFLPVDPATSNGRIKQLRSGALQIESSEESDQGKYEC
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEARTAQSTPSAPPQKVMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVTGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVIAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT

**Mass Match Peptides (bold):**
YSAPANLYVR [870]

**Tandem Peptides (underline):**
YANVIAYDHSR [840]


## Peptide Source: 1D-GE, Lung cancer

MAPEPAPGRTMVPLVPALVMLGLVAGAHGDSKPVFIKVPEDQTGLSGGVASFVCQATGEPKPRITWMKK
GKKVSSQR**FEVIEFDDGAGSVLR**IQPLRVQRDEAIYECTATNSLGEINTSAKLSVLEEEQLPPGFPSID
MGPQLKVVEKAR**TATMLCAAGGNPDPEISWFK**DFLPVDPATSNGRIKQLR**SGAIQIESSEESDQGK**YEC
VATNSAGTR**YSAPANLYVR**VRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYR**AAGTEGPFQEVDGVATTR**YSIGGLSPFSEYAFRVLAVNSIGR**GPPSEAVR**AR**TGEQAPS
SPPRR**VQARMLSASTMLVQWEPPEEPNGLVRGYRVYYTPDSRRPPNAWHKHNTDAGLLTTVGSLLPGIT
YSLRVLAFTAVGDGPPSPTIQVK**TQQGVPAQPADFQAEVESDTR**IQLSWLLPPQER**IIMYELVYWAAED
EDQQHK**VTFDPTSSYTLEDLKPDTLYRFQLAARSDMGVGVFTPTIEARTAQSTPSAPPQKVMCVSMGST
TVRVSWVPPPADSRNGVITQYSVAYEAVDGEDRGRHVVDGISR**EHSSWDLVGLEK**WTEYRVWVR**AHTDV
GPGPESSPVLVR**TDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCR**ADEARPNTIDFGKDDQHFTVTGLHKGTTYIFR**LAAKNRAGLGEEFEK
EIRTPEDLPSGFPQNLHVTGLTTSTTELAWDPPVLAERNGRIISYTVVFR**DINSQQELQNITTDTR**FTL
TGLKPDTTYDIKVRAWTSKGSGPLSPSIQSR**TMPVEQVFAK**NFRVAAAMKTSVLLSWEVPDSYKSAVPF
K**ILYNGQSVEVDGHSMRKLIADLQPNTEYSFVLMNR**GSSAGGLQHLVSIR**TAPDLLPHKPLPASAYIED
GR**FDLSMPHVQDPSLVRWFYIVVVPIDRVGGSMLTPRWSTPEELELDELLEAIEQGGEEQRRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRK**RTHSPSSK**DEQSIGLK**DSLLAHSSDPVEMRR**LN
YQTPGMR**DHPPIPITDLADNIER**LKANDGLKFSQEYESIDPGQQFTWENSNLEVNKPKNR**YANVIAYDH
SR**VILTSIDGVPGSDYINANYIDGYR**KQNAYIATQGPLPETMGDFWRMVWEQR**TATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEK**TVDIYGHVTCMR**SQRNYMVQTEDQ
YVFIHEALLEAATCGHTEVPARNLYAHIQK**LGQVPPGESVTAMELEFK**LLASSKAHTSRFISANLPCNK
FKNRLVNIMPYELTRVCLQPIR**GVEGSDYINASFLDGYR**QQK**AYIATQGPLAESTEDFWRMLWEHNSTI
IVMLTK**LREMGREKCHQYWPAERSARYQYFVVDPMAEYNMPQYILREFKVTDARDGQSRTIR**QFQFTDW
PEQGVPK**TGEGFIDFIGQVHKTKEQFGQDGPITVHCSAGVGRTGVFITLSIVLERMR**YEGVVDMFQTVK**
TLR**TQRPAMVQTEDQYQLCYR**AALEYLGSFDHYAT


**Mass Match Peptides (bold):**
AAGTEGPFQEVDGVATTR [50]
ADEARPNTIDFGK [58]
AHTDVGPGPESSPVLVR [72]
AYIATQGPLAESTEDFWR [98]
DDQHFTVTGLHK [111]
DHPPIPITDLADNIER [127]
DINSQQELQNITTDTR [129]
DSLLAHSSDPVEMR [156]
DSLLAHSSDPVEMRR [157]
EHSSWDLVGLEK [196]
FEVIEFDDGAGSVLR [249]
GPPSEAVR [308]
GTTYIFR [324]
GVEGSDYINASFLDGYR [326]
IIMYELVYWAAEDEDQQHK [372]
ILYNGQSVEVDGHSMR [378]
ILYNGQSVEVDGHSMRK [379]

```
KLIADLQPNTEYSFVLMNR [424]
KQNAYIATQGPLPETMGDFWR [433]
LGQVPPGESVTAMELEFK [462]
MLSASTMLVQWEPPEEPNGLVR [518]
MLWEHNSTIIVMLTK [519]
MVWEQR [529]
QFQFTDWPEQGVPK [572]
QNAYIATQGPLPETMGDFWR [581]
RTHSPSSK [616]
SGALQIESSEESDQGK [631]
TAPDLLPHKPLPASAYIEDGR [671]
TATMLCAAGGNPDPEISWFK [674]
TGEQAPSSPPRR [695]
TMPVEQVFAK [708]
TQQGVPAQPADFQAEVESDTR [725]
TQRPAMVQTEDQYQLCYR [726]
TVDIYGHVTCMR [735]
YEGVVDMFQTVK [844]
YSAPANLYVR [870]
```

## Tandem Peptides (underline):

```
GSGPLSPSIQSR [315]
QFQFTDWPEQGVPK [572]
YANVIAYDHSR [840]
YEGVVDMFQTVK [844]
```

## Peptide Source: 1D-GE, Osteosarcoma

```
MAPEPAPGRTMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTAINSLGEINTSAKLSVLEEEQLPPGFPSID
MGPQLKVVEKARTATMLCAAGGNPDPEISWFKDFLPVDPATSNGRIKQLRSGALQIESSEESDQGKYEC
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEARTAQSTPSAPPQKVMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVTGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYTATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT
```

## Mass Match Peptides (bold):

AAGTEGPFQEVDGVATTR [50]
AHTDVGPGPESSPVLVR [72]
AYIATQGPLAESTEDFWR [98]
DDQHFTVTGLHK [111]
ELPGELLGYR [206]
FDLSMPHVQDPSLVR [246]
FEVIEFDDGAGSVLR [249]
FTLTGLKPDTTYDIK [275]
GYQVTYVR [331]
ILYNGQSVEVDGHSMR [378]
LIADLQPNTEYSFVLMNR [467]
MLSASTMLVQWEPPEEPNGLVR [518]
NGVITQYSVAYEAVDGEDR [537]
QHGQIR [578]
RPPNAWHK [609]
RRQAER [614]
RTHSPSSK [616]
SDMGVGVFTPTIEAR [621]
TQQGVPAQPADFQAEVESDTR [725]
VPEDQTGLSGGVASFVCQATGEPKPR [789]
VTFDPTSSYTLEDLKPDTLYR [803]
YEGVVDMFQTVK [844]
YQYFVVDPMAEYNMPQYILR [869]
YSIGGLSPFSEYAFR [872]

## Tandem Peptides (underline):

IQLSWLLPPQER [388]
NGVITQYSVAYEAVDGEDR [537]
VLAFTAVGDGPPSPTIQVK [770]


## Peptide Source: 1D-GE, Pancreatic cancer

MAPEPAPGRTMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTATNSLGEINTSAKLSVLEEEQLPPGFPSID
MGPQLKVVEKARTATMLCAAGGNPDPEISWFKDFLPVDPATSNGRIKQLRSGALQIESSEESDQGKYEC
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEARTAQSTPSAPPQKVMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVIWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVTGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL

AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRGVFITLSIVLERMRYEGVVDMFQTVK
TLRTQRPAMVQTEDQYQLCYRAALEYLGSFDHYAT        .

## Mass Match Peptides (bold):

AAGTEGPFQEVDGVATTR [50]
AYIATQGPLAESTEDFWR [98]
DDQHFTVTGLHK [111]
DEAIYECTATNSLGEINTSAK [112]
DFLPVDPATSNGR [117]
DHPPIPITDLADNIER [127]
DINSQQELQNITTDTR [129]
DSLLAHSSDPVEMR [156]
DSLLAHSSDPVEMRR [157]
EHSSWDLVGLEK [196]
ELPGELLGYR [206]
EQFGQDGPITVHCSAGVGR [220]
FDLSMPHVQDPSLVR [246]
FEVIEFDDGAGSVLR [249]
FQLAAR [267]
GPPSEAVR [308]
GSGPLSPSIQSR [315]
GTTYIFR [324]
GVEGSDYINASFLDGYR [326]
HNTDAGLLTTVGSLLPGITYSLR [341]
HVVDGISR [347]
IIMYELVYWAAEDEDQQHK [372]
LGQVPPGESVTAMELEFK [462]
LNYQTPGMR [475]
MLWEHNSTIIVMLTK [519]
MVWEQR [529]
QFQFMAWPDHGVPEYPTPILAFLR [571]
RLNYQTPGMR [606]
RPPNAWHK [609]
RTHSPSSK [616]
SDMGVGVFTPTIEAR [621]
TAQSTPSAPPQK [672]
TATMLCAAGGNPDPEISWFK [674]
TATVVMMTR [676]
TDEDVPSGPPRK [683]
TGCFIVIDAMLER [692]
TMPVEQVFAK [708]
TQQGVPAQPADFQAEVESDTR [725]
TQRPAMVQTEDQYQLCYR [726]
TSVLLSWEVPDSYK [732]
VEVEPLNSTAVHVYWK [756]
VGGSMLTPR [764]
VSWVPPPADSR [799]
WMMGAEELTK [830]
YANVIAYDHSR [840]
YEGVVDMFQTVK [844]
YQYFVVDPMAEYNMPQYILR [869]
YSAPANLYVR [870]
YSIGGLSPFSEYAFR [872]

**Tandem Peptides (underline):**

AAGTEGPFQEVDGVATTR [50]
NGVITQYSVAYEAVDGEDR [537]
YANVIAYDHSR [840]
YEGVVDMFQTVK [844]
YSAPANLYVR [870]

## Peptide Source: 1D-GE, Renal cell cancer

MAPEPAPGRTMVPLVPALVMLGLVAGAHGDSKPVFIKVPEDQTGLSGGVASFVCQATGEPKPRITWMKK
GKKVSSQR**FEVIEFDDGAGSVLR**IQPLRVQRDEAIYECTATNSLGEINTSAKLSVLEEEQLPPGFPSID
MGPQLKVVEKARTATMLCAAGGNPDPEISWFKDFLPVDPATSNGRIKQLRSGALQIESSEESDQGKYEC
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYR**AAGTEGPFQEVDGVATTR**YSIGGLSPFSEYAFRVLAVNSIGRGPPSEAVRAR**TGEQAPS**
**SPPR**RVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYR**FQLAARSDMGVGVFTPTIEAR**TAQSTPSAPPQKVMCVSMGST
TVRVSWVPPPADSRNGVITQYSVAYEAVDGEDRGRHVVDGISR**EHSSWDLVGLEK**WTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVTGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT

**Mass Match Peptides (bold):**

AAGTEGPFQEVDGVATTR [50]
EHSSWDLVGLEK [196]
FEVIEFDDGAGSVLR [249]
FQLAAR [267]
SDMGVGVFTPTIEAR [621]
TGEQAPSSPPR [694]

**Tandem Peptides (underline):**

TGEQAPSSPPR [694]

## Peptide Source: iTRAQ, Colorectal cancer

MAPEPAPGRTMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTATNSLGEINTSAKLSVLEEEQLPPGFPSID
MGPQLKVVEKARTATMLCAAGGNPDPEISWFKDFLPVDPATSNGR**IKQLR**SGALQIESSEESDQGKYEC

VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEAR**TAQSTPSAPPQK**VMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFIVTGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQR**TATVVMMTRLEEK**SRV
**KCDQYWPAR**GTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT

**Mass Match Peptides (bold):**

    IKQLR [922]
    TAQSTPSAPPQK [672]
    TATVVMMTRLEEK [979]
    VKCDQYWPAR [991]

**Tandem Peptides (underline):**

    IKQLR [922]
    TAQSTPSAPPQK [672]
    TATVVMMTRLEEK [979]
    VKCDQYWPAR [991]

## Peptide Source: iTRAQ, Hepatocellular cancer

MAPEPAPGRTMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTATNSLGEINTSAKLSVLEEEQLPPGFPSID
MGPQLKVVEKARTATMLCAAGGNPDPEISWFKDFLPVDPATSNGR**IKQLR**SGALQIESSEESDQGKYEC
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEARTAQSTPSAPPQKVMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFIVTGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED

GRFDLSMPHVQDPSLVRWFYIVVVPIDRVGGSMLTPRWSTPEELELDELLEAIEQGGEEQRRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT

**Mass Match Peptides (bold):**

    IKQLR [922]

**Tandem Peptides (underline):**

    IKQLR [922]


## Peptide Source: iTRAQ, Non-small cell lung cancer

MAPEPAPGRTMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTATNSLGEINTSAKLSVLEEEQLPPGFPSID
MGPQLKVVEKARTATMLCAAGGNPDPEISWFKDFLPVDPATSNGRIKQLRSGALQIESSEESDQGKYEC
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEAR<u>**TAQSTPSAPPQK**</u>VMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVTGLHKGTTYIFRLAAK<u>**NRAGLGEEFEK**</u>
<u>**EIR**</u>TPEDLPSGFPQNLHVTGLTTSTTELAWDPPVLAERNGRITSYTVVFRDINSQQELQNITTDTRFTL
TGLKPDTTYDIKVRAWTSKGSGPLSPSIQSR<u>**TMPVEQVFAK**</u>NFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRRQAER
LKPYVAAQLDVLPETFTLGDKKNYRGFYNRPLSPDLSYQCFVLASLKEPMDQKRYASSPYSDEIVVQVT
PAQQQEEPEMLWVTGPVLAVILIILIVIAILLFKRKRTHSPSSKDEQSIGLK<u>**DSLLAHSSDPVEMR**</u>RLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT

**Mass Match Peptides (bold):**

    DSLLAHSSDPVEMR [156]
    NRAGLGEEFEKEIR [960]
    TAQSTPSAPPQK [672]
    TMPVEQVFAK [708]

**Tandem Peptides (underline):**

DSLLAHSSDPVEMR [156]

NRAGLGEEFEKEIR [960]

TAQSTPSAPPQK [672]

TMPVEQVFAK [708]

## Peptide Source: iTRAQ, Renal cell cancer

MAPEPAPGRTMVPLVPALVMLGLVAGAHGDSKPVFIKVPEDQTGLSGGVASFVCQATGEPKPRITWMKK
GKKVSSQRFEVIEFDDGAGSVLRIQPLRVQRDEAIYECTATNSLGEINTSAKLSVLEEEQLPPGFPSID
MGPQLKVVEKARTATMLCAAGGNPDPEISWFKDFLPVDPATSNGRIKQLRSGALQIESSEESDQGKYEC
VATNSAGTRYSAPANLYVRVRRVAPRFSIPPSSQEVMPGGSVNLTCVAVGAPMPYVKWMMGAEELTKED
EMPVGRNVLELSNVVRSANYTCVAISSLGMIEATAQVTVKALPKPPIDLVVTETTATSVTLTWDSGNSE
PVTYYGIQYRAAGTEGPFQEVDGVATTRYSIGGLSPFSEYAFRVLAVNSIGRGPPSEAVRARTGEQAPS
SPPRRVQARMLSASTMLVQWEPPEEPNGLVRGYRVYYTPDSRRPPNAWHKHNTDAGLLTTVGSLLPGIT
YSLRVLAFTAVGDGPPSPTIQVKTQQGVPAQPADFQAEVESDTRIQLSWLLPPQERIIMYELVYWAAED
EDQQHKVTFDPTSSYTLEDLKPDTLYRFQLAARSDMGVGVFTPTIEAR**TAQSTPSAPPQK**VMCVSMGST
TVRVSWVPPPADSRNGVITQYSVAYEAVDGEDRGRHVVDGISREHSSWDLVGLEKWTEYRVWVRAHTDV
GPGPESSPVLVRTDEDVPSGPPRKVEVEPLNSTAVHVYWKLPVPSKQHGQIRGYQVTYVRLENGEPRGL
PIIQDVMLAEAQETTISGLTPETTYSVTVAAYTTKGDGARSKPKIVTTTGAVPGRPTMMISTTAMNTAL
LQWHPPKELPGELLGYRLQYCRADEARPNTIDFGKDDQHFTVTGLHKGTTYIFRLAAKNRAGLGEEFEK
EIRTPEDLPSGFPQNLHVTGLTTSTTELAWDPPVLAERNGRIISYTVVFRDINSQQELQNITTDTRFTL
TGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIED
GRFDLSMPHVQDPSLVRWFYIVVVPIDRVGGSMLTPRWSTPEELELDELLEAIEQGGEEQRRRRQAER
LKPYVAAQLDVLPETFTLGDKKNYRGFYNRPLSPDLSYQCFVLASLK**EPMDQKR**YASSPYSDEIVVQVT
PAQQQEEPEMLWVTGPVLAVILIILIVIAILLFKRKRTHSPSSKDEQSIGLKDSLLAHSSDPVEMRRLN
YQTPGMRDHPPIPITDLADNIERLKANDGLKFSQEYESIDPGQQFTWENSNLEVNKPKNRYANVIAYDH
SRVILTSIDGVPGSDYINANYIDGYRKQNAYIATQGPLPETMGDFWRMVWEQRTATVVMMTRLEEKSRV
KCDQYWPARGTETCGLIQVTLLDTVELATYTVRTFALHKSGSSEKRELRQFQFMAWPDHGVPEYPTPIL
AFLRRVKACNPLDAGPMVVHCSAGVGRTGCFIVIDAMLERMKHEKTVDIYGHVTCMRSQRNYMVQTEDQ
YVFIHEALLEAATCGHTEVPARNLYAHIQKLGQVPPGESVTAMELEFKLLASSKAHTSRFISANLPCNK
FKNRLVNIMPYELTRVCLQPIRGVEGSDYINASFLDGYRQQKAYIATQGPLAESTEDFWRMLWEHNSTI
IVMLTKLREMGREKCHQYWPAERSARYQYFVVDPMAEYNMPQYILREFKVTDARDGQSRTIRQFQFTDW
PEQGVPKTGEGFIDFIGQVHKTKEQFGQDGPITVHCSAGVGRTGVFITLSIVLERMRYEGVVDMFQTVK
TLRTQRPAMVQTEDQYQLCYRAALEYLGSFDHYAT

**Mass Match Peptides (bold):**

EPMDQKR [218]

TAQSTPSAPPQK [672]

**Tandem Peptides (underline):**

EPMDQKR [218]

TAQSTPSAPPQK [672]

## Figure 2: Box Plot data for OGTA066

**Oneway Analysis of AGP3 By Diagnosis**

Excluded Rows    16

**Quantiles**

| Level | Minimum | 10% | 25% | Median | 75% | 90% | Maximum |
|-------|---------|-----|-----|--------|-----|-----|---------|
| Colon | 0.23 | 0.23 | 0.7 | 1.945 | 3.58 | 4.37 | 4.37 |
| Normal | 1.7 | 1.7 | 2.8525 | 4.205 | 9.435 | 19.37 | 19.37 |

**Oneway Anova**

**Means for Oneway Anova**

| Level | Number | Mean | Std Error | Lower 95% | Upper 95% |
|-------|--------|------|-----------|-----------|-----------|
| Colon | 8 | 2.16375 | 1.5110 | -1.077 | 5.4044 |
| Normal | 8 | 6.64125 | 1.5110 | 3.401 | 9.8819 |

Std Error uses a pooled estimate of error variance

**Means Comparisons**

**Comparisons for each pair using Student's t**

| Level | -Level | Difference | Lower CL | Upper CL | p-Value | |
|-------|--------|------------|----------|----------|---------|---|
| Normal | Colon | 4.477500 | -0.105522 | 9.060522 | 0.0548 | |

687

# Figure 3: ROC curve data for OGTA066

| Curve | Area | SE | p | 95% CI of Area | | Colon vs Normals = colon cancer |
|-------|------|-----|---|--------|--------|---------|
| AGP3ng/ml | 0.863 | 0.0334 | <0.0001 | 0.797 | to 0.928 | have lower values |

# Figure 4: Box Plot data for OGTA066

| AGP3ng/ml by Diagnosis | n | Mean | SD | SE | 95% CI of Mean | | Median | IQR | 95% CI of Median | |
|---|---|---|---|---|---|---|---|---|---|---|
| colon cancer | 65 | 2.460 | 2.9292 | 0.3633 | 1.734 | to 3.186 | 1.656 | 1.944 | 1.203 | to 2.187 |
| normal | 61 | 7.248 | 7.1884 | 0.9204 | 5.407 | to 9.089 | 4.849 | 6.050 | 3.985 | to 7.451 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 09412168 B
- EP 320308 A
- US 6316409 B
- US 9002545 W
- US 4816567 A, Cabilly
- US 4816397 A, Boss
- US 5585089 A, Queen
- WO 8702671 A
- EP 184187 A
- EP 171496 A
- EP 173494 A
- WO 8601533 A
- EP 125023 A
- US 5225539 A
- US 5625126 A
- US 5633425 A
- US 5569825 A
- US 5661016 A
- US 5545806 A
- US 5571698 A, Ladner
- US 6057098 A
- GB 9101134 W
- WO 9002809 A
- WO 9110737 A
- WO 9201047 A
- WO 9218619 A
- WO 9311236 A
- WO 9515982 A
- WO 9520401 A
- US 5698426 A
- US 5223409 A
- US 5403484 A
- US 5580717 A
- US 5427908 A
- US 5750753 A
- US 5821047 A
- US 5516637 A
- US 5780225 A
- US 5658727 A
- US 5733743 A
- US 5969108 A
- WO 9222324 A
- US 4946778 A
- US 5258498 A

- WO 9308829 A
- WO 9404690 A
- US 5831012 A
- US 6291158 B
- US 6582915 B
- US 6593081 B
- US 6172197 B
- US 6696245 B
- US 20040110941 A
- EP 1433846 A
- EP 0368684 A
- EP 0616640 A
- WO 05035572 A
- WO 04101790 A
- WO 04081026 A
- WO 04058821 A
- WO 04003019 A
- WO 03002609 A
- WO 04041867 A
- US 6765087 B
- US 6838254 B
- WO 06079372 A
- WO 2007059782 A
- WO 8605807 A
- WO 8901036 A
- US 5122464 A
- US 4741900 A
- US 4676980 A
- WO 0061739 A1
- WO 0231140 A1
- WO 03085107 A1
- WO 9954342 A
- WO 03074679 A
- WO 2007039818 A
- US 4980286 A
- US 41216899 A
- WO 9636882 A
- US 6064754 A
- WO 9853323 A
- WO 09094996 A
- GB 08050125 W
- US 60903510 B
- US 60903509 B
- EP 07055537 W

### Non-patent literature cited in the description

- **SUMAR et al.** Lectins as Indicators of Disease-Associated Glycoforms. 158-159

- Lectins and Glycobiology. 1993, 158-174
- **INNIS et al.** PCR Protocols. Academic Press, Inc, 1990
- **OHARA et al.** *DNA Research,* 1997, vol. 4, 53-59
- Current Protocols in Molecular Biology. 1987
- **DAVIS et al.** *Basic Methods in Molecular Biology,* 1986
- **SAMBROOK et al.** Molecular Cloning: A Laboratory Manual. Cold Spring Harbour laboratory Press, 1989
- *Proc. Natl. Acad. Sci. U.S.A.,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Gene,* 1983, vol. 24, 255
- *Journal of Biochemistry,* 1984, vol. 95, 87
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proc. Natl. Acad. Sci. U.S.A.,* 1978, vol. 75, 1929
- *Cell Technology, separate,* vol. 8
- **SHUJUNSHA.** *New Cell Technology, Experimental Protocol.,* 1995, 263-267
- *Virology,* 1973, vol. 52, 456
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- Antibodies, A Laboratory Manual. Cold Spring Harbor Laboratory, 1988
- Antibody Engineering: A Practical Approach. Oxford University Press, 1995
- *J. Immunol.,* 1992, vol. 149, 3914-3920
- Guide to Protein Purification. Meth. Enzymol. 1990, vol. 182
- Solid Phase Peptide Synthesis. Meth. Enzymol. 1997, vol. 289
- **KISO et al.** *Chem. Pharm. Bull,* 1990, vol. 38, 1192-99
- **MOSTAFAVI et al.** *Biomed. Pept. Proteins Nucleic Acids,* 1995, vol. 1, 255-60
- **FUJIWARA et al.** *Chem. Pharm. Bull,* 1996, vol. 44, 1326-31
- **KOHLER ; MILSTEIN.** *Nature,* 1975, vol. 256, 495-497
- **KOZBOR et al.** *Immunology Today,* 1983, vol. 4, 72
- **COLE et al.** Monoclonal Antibodies and Cancer Therapy. Alan R. Liss, Inc, 1985, 77-96
- **BETTER et al.** *Science,* 1988, vol. 240, 1041-1043
- **LIU et al.** *Proc. Natl. Acad. Sci. USA,* 1987, vol. 84, 3439-3443
- **LIU et al.** *J. Immunol.,* 1987, vol. 139, 3521-3526
- **SUN et al.** *Proc. Natl. Acad. Sci. USA,* 1987, vol. 84, 214-218
- **NISHIMURA et al.** *Canc. Res.,* 1987, vol. 47, 999-1005
- **WOOD et al.** *Nature,* 1985, vol. 314, 446-449
- **SHAW et al.** *J. Natl. Cancer Inst.,* 1988, vol. 80, 1553-1559
- **MORRISON.** *Science,* 1985, vol. 229, 1202-1207
- **OI et al.** *Bio/Techniques,* 1986, vol. 4, 214
- **JONES et al.** *Nature,* 1986, vol. 321, 552-525
- **VERHOEYAN et al.** *Science,* 1988, vol. 239, 1534
- **BEIDLER et al.** *J. Immunol.,* 1988, vol. 141, 4053-4060
- **LONBERG ; HUSZAR.** *Int. Rev. Immunol.,* 1995, vol. 13, 65-93
- **JESPERS et al.** *Bio/technology,* 1994, vol. 12, 899-903
- **CWIRLA et al.** *Pro. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **BRINKMAN et al.** J. Immunol. Methods. 1995, vol. 182, 41-50
- **AMES et al.** *J. Immunol. Methods,* 1995, vol. 184, 177-186
- **KETTLEBOROUGH et al.** *Eur. J. Immunol.,* 1994, vol. 24, 952-958
- **PERSIC et al.** *Gene,* 1997, vol. 187, 9-18
- **BURTON et al.** *Advances in Immunology,* 1994, vol. 57, 191-280
- **MULLINAX et al.** *BioTechniques,* 1992, vol. 12 (6), 864-869
- **SAWAI et al.** *AJRI,* 1995, vol. 34, 26-34
- **HUSTON et al.** *Methods in Enzymology,* 1991, vol. 203, 46-88
- **SHU et al.** *PNAS,* 1993, vol. 90, 7995-7999
- **SKERRA et al.** *Science,* 1988, vol. 240, 1038-1040
- **MILSTEIN et al.** *Nature,* 1983, vol. 305, 537-539

- **TRAUNECKER et al.** *EMBO J.,* 1991, vol. 10, 3655-3659
- **SURESH et al.** *Methods in Enzymology,* 1986, vol. 121, 210
- **BIRD.** *Science,* 1988, vol. 242, 423-42
- **HUSTON et al.** *Proc. Natl. Acad. Sci. USA,* 1988, vol. 85, 5879-5883
- **WARD et al.** *Nature,* 1989, vol. 334, 544-54
- **SKERRA et al.** *Science,* 1988, vol. 242, 1038-1041
- **NORD K ; GUNNERIUSSON E ; RINGDAHL J ; STAHL S ; UHLEN M ; NYGREN PA.** Binding proteins selected from combinatorial libraries of an α-helical bacterial receptor domain. *Nat Biotechnol,* 1997, vol. 15, 772-7
- **RONMARK J ; GRONLUND H ; UHLEN M ; NYGREN PA.** Human immunoglobulin A (IgA)-specific ligands from combinatorial engineering of protein A. *Eur J Biochem,* 2002, vol. 269, 2647-55
- **RONMARK J ; HANSSON M ; NGUYEN T et al.** Construction and characterization of affibody-Fc chimeras produced in Escherichia coli. *J Immunol Methods,* 2002, vol. 261, 199-211
- **SANDSTORM K ; XU Z ; FORSBERG G ; NYGREN PA.** Inhibition of the CD28-CD80 co-stimulation signal by a CD28-binding Affibody ligand developed by combinatorial protein engineering. *Protein Eng,* 2003, vol. 16, 691-7
- **KUTMEIER et al.** *BioTechniques,* 1994, vol. 17, 242
- **HUSE et al.** *Science,* 1989, vol. 246, 1275-1281
- **CLACKSON et al.** *Nature,* 1991, vol. 352, 624
- **HANE et al.** *Proc. Natl. Acad. Sci. USA,* 1997, vol. 94, 4937
- **HUTCHINSON et al.** *J. Biol. Chem.,* 1978, vol. 253, 6551
- **MORRISON et al.** *Proc. Natl. Acad. Sci.,* 1984, vol. 81, 851-855
- **NEUBERGER et al.** *Nature,* 1984, vol. 312, 604-608
- **TAKEDA et al.** *Nature,* 1985, vol. 314, 452-454
- **SAMBROOK et al.** Molecular Cloning, A Laboratory Manual. Cold Spring Harbor Laboratory, 1990
- Current Protocols in Molecular Biology. John Wiley & Sons, 1998
- **FOECKING et al.** *Gene,* 1986, vol. 45, 101
- **COCKETT et al.** *Bio/Technology,* 1990, vol. 8, 2
- **RUTHER et al.** *EMBO J.,* 1983, vol. 2, 1791
- **INOUYE ; INOUYE.** *Nucleic Acids Res.,* 1985, vol. 13, 3101-3109
- **VAN HEEKE ; SCHUSTER.** *J. Biol. Chem.,* 1989, vol. 24, 5503-5509
- The use of vectors based on gene amplification for the expression of cloned genes in mammalian cells in. **BEBBINGTON ; HENTSCHEL.** DNA cloning. Academic Press, 1987, vol. 3
- **CROUSE et al.** *Mol. Cell. Biol.,* 1983, vol. 3, 257
- **PROUDFOOT.** *Nature,* 1986, vol. 322, 52
- **KOHLER.** *Proc. Natl. Acad. Sci. USA,* 1980, vol. 77, 2197
- **JANKNECHT et al.** *Proc. Natl. Acad. Sci. USA,* 1991, vol. 88, 8972-897
- **RONNMARK J ; GRONLUND H ; UHLE' N, M. ; NYGREN P.A°.** Human immunoglobulin A (IgA)-specific ligands from combinatorial engineering of protein A. *Eur. J. Biochem.,* 2002, vol. 269, 2647-2655
- **NORD, K. ; NILSSON, J. ; NILSSON, B. ; UHLE' N, M. ; NYGREN, P.A.** A combinatorial library of an a-helical bacterial receptor domain. *Protein Eng.,* 1995, vol. 8, 601-608
- **NORD, K. ; GUNNERIUSSON, E. ; RINGDAHL, J. ; STA° HL, S. ; UHLE' N, M. ; NYGREN, P.A°.** Binding proteins selected from combinatorial libraries of an a-helical bacterial receptor domain. *Nat. Biotechnol.,* 1997, vol. 15, 772-777
- Monoclonal Antibodies For Immunotargeting Of Drugs In Cancer Therapy. **ARNON et al.** Monoclonal Antibodies And Cancer Therapy. Alan R. Liss, Inc, 1985, 243-56
- Antibodies For Drug Delivery. **HELLSTROM et al.** Controlled Drug Delivery. Marcel Dekker, Inc, 1987, 623-53
- Antibody Carriers Of Cytotoxic Agents In Cancer Therapy: A Review. **THORPE et al.** Monoclonal Antibodies '84: Biological And Clinical Applications. 1985, 475-506
- Analysis, Results, And Future Prospective Of The Therapeutic Use Of Radiolabeled Antibody In Cancer Therapy. Monoclonal Antibodies For Cancer Detection And Therapy. Academic Press, 1985, 303-16
- **THORPE et al.** The Preparation And Cytotoxic Properties Of Antibody-Toxin Conjugates. *Immunol. Rev.,* 1982, vol. 62, 119-58
- **MORRISON et al.** *Proc. Natl. Acad. Sci.,* vol. 81, 851-855
- **JANEWAY JR. C.A. et al.** Immunobiology. Garland Publishing, 2001
- **PIER G.B et al.** *Immunology, Infection, and Immunity,* 2004, 246-5
- **ALBANELL J. et al.** *Advances in Experimental Medicine and Biology,* 2003, vol. 532, 2153-68
- **WENG, W.-K. et al.** *Journal of Clinical Oncology,* 2003, vol. 21, 3940-3947
- **BLOMBERG et al.** *Journal of Immunological Methods,* 1986, vol. 86, 225-9

- **BLOMBERG et al.** *Journal of Immunological Methods,* 1986, vol. 21 (92), 117-23
- **PATEL ; BOYD.** *Journal of Immunological Methods,* 1995, vol. 184, 29-38
- **SHINKAWA et al.** *J. Biol. Chem.,* 2003, vol. 278, 3466-34735
- **YAMANE-OHNUKI et al.** *Biotechnology and Bioengineering,* 2004, vol. 87, 614-22
- **LAZAR et al.** *Proceedings of the National Academy of Sciences,* 2006, vol. 103, 4005-4010
- **CAPECCHI.** *Science,* 1989, vol. 244, 1288-1292
- **WU ; WU.** *J. Biol. Chem.,* 1987, vol. 262, 4429-4432
- **LANGER.** *Science,* 1990, vol. 249, 1527-1533
- **TREAT et al.** Liposomes in the Therapy of Infectious Disease and Cancer. Liss, 1989, 353-365
- LIPOSOMES IN THE THERAPY OF INFECTIOUS DISEASE AND CANCER. 317-327
- **SEFTON.** *CRC Crit. Ref. Biomed. Eng.,* 1987, vol. 14, 201
- **BUCHWALD et al.** *Surgery,* 1980, vol. 88, 507
- **SAUDEK et al.** *N. Engl. J. Med.,* 1989, vol. 321, 574
- Medical Applications of Controlled Release. CRC Pres, 1974
- Controlled Drug Bioavailability, Drug Product Design and Performance. Wiley, 1984
- **RANGER ; PEPPAS, J.** *Macromol. Sci. Rev. Macromol. Chem.,* 1983, vol. 23, 61
- **LEVY et al.** *Science,* 1985, vol. 228, 190
- **DURING et al.** *Ann. Neurol.,* 1989, vol. 25, 351
- **HOWARD et al.** *J. Neurosurg.,* 1989, vol. 71, 105
- **GOODSON.** *Medical Applications of Controlled Release,* 1984, vol. 2, 115-138
- **JOLIOT et al.** *Proc. Natl. Acad. Sci. USA,* 1991, vol. 88, 1864-1868
- **BODEY B.** The significance of immunohistochemistry in the diagnosis and therapy of neoplasms. *Expert Opin Biol Ther.,* April 2002, vol. 2 (4), 371-93
- Current Protocols in Molecular Biology. Green Publishing Associates, Inc., and John Wiley & Sons, Inc, 1989, vol. II
- Development of a High-throughput Fluorescence Scanner Employing Internal Reflection Optics and Phase-sensitive Detection. **DAVID A. BASIJI.** Total Internal Reflection, Electrophoresis. University of Washington, 1997, vol. 58/12-B, 6686
- **ENG et al.** *J. Am. Soc. Mass Spectrom.,* 1994, vol. 5, 976-989
- **GASKELL et al.** *Rapid Commun. Mass Spectrom.,* 1992, vol. 6, 658-662
- **VENTER, J.C. et al.** The sequence of the human genome. *Science,* 2001, vol. 16, 1304-51
- Initial sequencing and analysis of the human genome. *Nature,* vol. 409, 860-921
- **H PEARSON.** What is a gene?. *Nature,* 2006, vol. 24, 399-401